# EUROPEAN PATENT SPECIFICATION

(11) **EP 3 810 606 B1**
(45) Date of publication and mention of the grant of the patent: **02.12.2026**
(21) Application number: 19733448.5
(22) Date of filing: 20.06.2019
(51) Int. Cl.: C07D 413/14, C07D 333/66, C07D 333/68, C07D 333/78, C07D 409/04, C07D 409/06, C07D 409/12, C07D 409/14, C07D 413/04, C07D 413/12, C07D 417/12, C07D 487/04, C07D 495/04, C07D 495/10, C07D 498/10, A61P 37/08, A61P 11/06, A61K 31/381

(54) **THIOPHENE DERIVATIVES FOR THE TREATMENT OF DISORDERS CAUSED BY IGE**
THIOPHEN-DERIVATE ZUR BEHANDLUNG VON ERKRANKUNGEN, DIE DURCH IGE VERURSACHT WERDEN
DÉRIVÉS DE THIOPHENE POUR LE TRAITMENT DE TROUBLES CAUSÉS PAR IGE

(30) Priority: 21.06.2018 EP 18179126
(43) Date of publication of application: 28.04.2021
(73) Proprietor: UCB Biopharma SRL, 1070 Brussels (BE)
(72) Inventor: CHOVATIA, Prafulkumar Tulshibhai, Abingdon Oxfordshire OX14 4RZ (GB); CONNELLY, Rickki Lee, Abingdon Oxfordshire OX14 4RZ (GB); FRANKLIN, Richard Jeremy, Slough Berkshire SL1 3WE (GB); HASLETT, Gregory William, Slough Berkshire SL1 3WE (GB); HENRY, Alistair James, Slough Berkshire SL1 3WE (GB); MADDEN, James, Abingdon Oxfordshire OX14 4RZ (GB); NEUSS, Judi Charlotte, Slough Berkshire SL1 3WE (GB); NORMAN, Timothy John, Slough Berkshire SL1 3WE (GB); PHILPS, Oliver, Abington, Oxfordshire OX14 4RZ (GB); PITT, William Ross, Slough Berkshire SL1 3WE (GB); RAMPALAKOS, Konstantinos, Abingdon Oxfordshire OX14 4RZ (GB); SELBY, Matthew Duncan, Slough Berkshire SL1 3WE (GB); SELVARATNAM, Suganthan, Abingdon Oxfordshire OX14 4RZ (GB); TRANI, Giancarlo, Abingdon Oxfordshire OX14 4RZ (GB); ZHU, Zhaoning, Slough Berkshire SL1 3WE (GB)
(74) Representative: J A Kemp LLP
(86) International application number: PCT/EP2019/066414
(87) International publication number: WO 2019/243550

(56) References cited:
- EP-A2- 0 317 295
- WO-A1-01/47874
- WO-A2-01/17516
- WO-A2-01/98290
- WO-A2-2005/023818
- WO-A2-2005/033102
- WO-A2-2005/044008
- WO-A2-2009/079373
- JP-A- 2001 151 780
- US-A1- 2009 018 114
- US-A1- 2015 045 327
- A P MKRTCHYAN ET AL: "-SUBSTITUTED TETRA- HYDROBENZO[b] THIENO[2,3-d]PYRIMIDIN-4-ONES AND THEIR ANTICONVULSANT ACTIVITY", HELV. CHIM. ACTA. J. AM. CHEM. SOC. J. AM. CHEM. SOC, 1 January 1963 (1963-01-01), pages 2658 - 2666, XP055498334, Retrieved from the Internet <URL:https://link.springer.com/content/pdf/10.1007/BF00763695.pdf> [retrieved on 20180809]
- SHIKHAR GUPTA ET AL: "Discovery of dual binding site acetylcholinesterase inhibitors identified by pharmacophore modeling and sequential virtual screening techniques", BIOORGANIC & MEDICINAL CHEMISTRY LETTERS, PERGAMON, AMSTERDAM, NL, vol. 21, no. 4, 28 December 2010 (2010-12-28), pages 1105 - 1112, XP028138651, ISSN: 0960-894X, [retrieved on 20110101], DOI: 10.1016/J.BMCL.2010.12.131
- HUNG THE DANG ET AL: "Syntheses and biological evaluation of 2-amino-3-acyl-tetrahydrobenzothiophene derivatives; antibacterial agents with antivirulence activity", ORGANIC & BIOMOLECULAR CHEMISTRY, vol. 12, no. 12, 1 January 2014 (2014-01-01), pages 1942 - 1956, XP055498237, ISSN: 1477-0520, DOI: 10.1039/C3OB42478B
- STEVEN E. VAN DER PLAS ET AL: "Discovery of N -(3-Carbamoyl-5,5,7,7-tetramethyl-5,7-dihydro-4 H -thieno[2,3- c ]pyran-2-yl)-l H -pyrazole-5-carboxamide (GLPG1837), a Novel Potentiator Which Can Open Class III Mutant Cystic Fibrosis Transmembrane Conductance Regulator (CFTR) Channels to a High Extent", JOURNAL OF MEDICINAL CHEMISTRY, vol. 61, no. 4, 17 November 2017 (2017-11-17), pages 1425 - 1435, XP055498730, ISSN: 0022-2623, DOI: 10.1021/acs.jmedchem.7b01288
- ANDERSEN H S ET AL: "Discovery and SAR of a Novel Selective and Orally Bioavailable Nonpeptide Classical Competitive Inhibitor Class of Protein-Tyrosine Phosphatase 1B", JOURNAL OF MEDICINAL CHEMISTRY, AMERICAN CHEMICAL SOCIETY, US, vol. 45, 1 January 2002 (2002-01-01), pages 4443 - 4459, XP002318790, ISSN: 0022-2623, DOI: 10.1021/JM0209026

## Description

### Technical Field

The present invention relates to thiophene derivatives of formula (I), processes for preparing them, pharmaceutical compositions containing them and their use in treating disorders caused by IgE (such as allergic responses, non-allergic mast cell responses or certain autoimmune responses), and in particular disorders caused by the interaction of IgE with the FcεRI receptor.

### Background of the Invention

IgE (immunoglobulin E) is a member of the immunoglobulin family and mediates allergic responses such as asthma, food allergies, type 1 hypersensitivity and the familiar sinus inflammation.

IgE is secreted by, and expressed on the surface of, B-cells. IgE synthesized by B-cells is anchored in the B-cell membrane by a transmembrane domain linked to the matu4=re IgE sequence by a short membrane binding region. IgE also is bound to B-cells (and monocytes, eosinophils and platelets) through its Fc region to a low affinity IgE receptor (FcεRII). Upon exposure of a mammal to an allergen, B-cells are clonally amplified which synthesize IgE that binds the allergen. This IgE in turn is released into the circulation by the B-cells where it is bound by B-cells (through FcεRII) and by mast cells and basophils through the so-called high affinity receptor (FcεRI) found on the surface of the mast cells and basophils. Such mast cells and basophils are thereby sensitized for allergen. The next exposure to the allergen cross-links the FcεRI on these cells and thus activate their release of histamine and other factors which are responsible for clinical hypersensitivity and anaphylaxis. Currently, allergic diseases , urticaria, and asthma are usually treated with one or more of the following drugs: (1) antihistamines and antileukotrienes which antagonize the inflammatory mediators histamine and leukotrienes, (2) local or systemic (oral or injectable) corticosteroids or immunosuppressants which suppress a broad spectrum of inflammatory mechanisms, (3) short or long-acting bronchodilators which relax smooth muscle of constricted airway in asthma, or (4) mast cell stabilizers which inhibit the degranulation of mast cells that is normally triggered by IgE-binding at FcεRI , (5) biologicals which prevent the binding of IgE at FcεRI.

However, there is still a need to identify compounds which have therapeutic utility in the treatment or prevention of disorders caused by IgE, particularly disorders caused by the interaction of IgE with the FcεRI receptor.

WO 01/17516 A1 describes a method of inhibiting a member of a family of Protein Tyrosine Phosphatases (PTPases, PTPs) such as PTP1B, TC-PTP, CD45, SHP-1, SHP-2, PTP alpha, PTP epsilon, PTP mu, PTP delta, PTP sigma, PTP zeta, PTP beta, PTPD1, PTPD2, PTPH1, PTP-MEG1, PTP-LAR, and HePTP by exposing said PTPase member by administration to a host or otherwise to at least one compound with certain structural, physical and spatial characteristics that allow for the interaction of said compound with specific residues of the active site of PTP1B and/or TC-PTP. These compounds are said to be indicated in the management or treatment of a broad range of diseases such as autoimmune diseases, acute and chronic inflammation, osteoporosis, various forms of cancer and malignant diseases, and type I diabetes and type II diabetes, as well as in the isolation of PTPases and in elucidation or further elucidation of their biological function.

### Summary of the Invention

Surprisingly, it has been found that compounds of formula (I) and their pharmaceutically acceptable salts can be used for this purpose.

The invention is set out in the appended set of claims.

### Detailed description

The present invention provides compounds of formula (I) and pharmaceutically acceptable salts thereof: 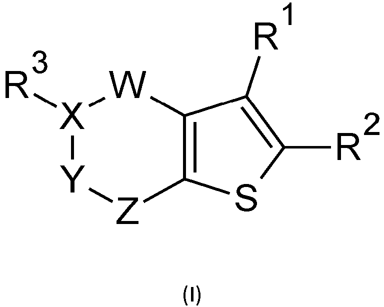 wherein
X represents CH, N, or C-R⁴, wherein when X is C-R⁴, R³ and R⁴ may be linked to form a 4-6 membered cycloalkyl or heterocyclic ring,
   any of which may be optionally substituted by one or more substituents selected from halogen, =O, OH, SMe, C₁-C₂ alkoxy, O-C₁-C₂ haloalkyl, C₁-C₂ alkyl, C₁-C₂ heteroalkyl, C₁-C₂ haloalkyl, NH₂, NHMe, NMe₂, aryl and heteroaryl, with aryl and heteroaryl optionally substituted by one or more substituents selected from halogen, OH, C₁-C₆ alkyl, C₁-C₆ alkoxy and C₁-C₂ haloalkyl;
when X represents N, then W, Y and Z are C or one of W, Y and Z is a single bond, the other two being C;
when X represents CH or C-R⁴, then W, Y and Z are C, or one of W, Y or Z is a single bond, O, S, S(O), S(O)₂ or N, the other two being C;
   when W, Y, or Z are C, each C may be substituted by 1 or 2 groups which are independently selected from H, halogen, SF₅, =O, =NOH, -OH, -CN, -C(O)Me, -C(O)OH, -C(O)NH₂, NH-C(O)O-C₁-C₄ alkyl, -NO₂, NH₂, NHMe, NMe₂, SH, SMe, SOMe, SO₂Me, C₁-C₄ alkyl, C₃-C₄ cycloalkyl, C₁-C₄ alkoxy, heterocycle, C₁-C₄ heteroalkyl, which may be linked to form a 3-6 membered cycloalkyl or 4-6 membered heterocyclic ring,
   any of which may be optionally substituted by one or more substituents selected from halogen, =O, OH, SMe, C₁-C₂ alkoxy, O-C₁-C₂ haloalkyl, C₁-C₂ alkyl, C₁-C₂ heteroalkyl, C₁-C₂ haloalkyl, NH₂, NHMe, NMe₂, which may be linked to form a 3-6 membered cycloalkyl or 4-6 membered heterocyclic ring;
   wherein
   when W, Y or Z is N, this N may be independently substituted by H, C₁-C₄ alkyl, C₃-C₄ cycloalkyl, heterocycle, -C(O)Me, -C(O)NH₂, -C(O)OH, -S(O)Me, -S(O)₂Me, -S(O)₂NH₂, any of which may be optionally substituted by one or more substituents selected from halogen, OH, SMe, C₁-C₂ alkoxy, O-C₁-C₂ haloalkyl, C₁-C₂ alkyl, C₁-C₂ heteroalkyl, C₁-C₂ haloalkyl, NH₂, NHMe, NMe₂;
   and W, X, Y and Z may be linked directly or via substituents to form a ring;
      R¹ represents 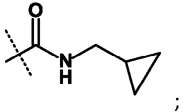
      R² represents 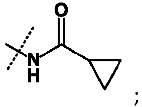
      R³ is selected from H, -OH, C₁-C₈ alkyl, -NR^{d}R^{c},
      -NH-R¹⁴-NH-R^{a}, -NMe-R¹⁴-NH-R^{a}, -NH-R¹⁴-NMe-R^{a}, -CO-NR^{d}R^{c}, -NH-COO-R^{c}, -NH-CO-R^{c}, -COOH, - COO-C₁-C₈ alkyl, -CO-C₁-C₈ alkyl, -C₁-C₈ alkylene-COO-C₁-C₈ alkyl, -C₁-C₈ alkylene-NR^{a} -COO-C₁-C₈ alkyl, -C₁-C₈ alkylene-NR^{d}R^{c}, -C₁-C₈ alkylene-NH-R¹⁴-NH-R^{a}, heteroaryl, -CN, -CH=N-OH, -CF₃, alkylamino, amino, -NH-SO-C₁-C₈ alkyl, -NH-SO₂-C₁-C₈ alkyl, heteroalkyl-amino, aryl, heterocycle, C₃-C₆ cycloalkyl, -NHS(O)(=NH)Me, -SO₂-C₁-C₈ alkyl, -NH-C(CH₃)=N-CN, -NH-CH=N-CN, -NH-S(=O)(=N)-C₁-C₂ alkyl,
   any of which may be optionally substituted by one or more substituents selected from halogen, -OH, =O, -COOH, -COH, -CN, SMe, -NR^{d}R^{c}, -OR^{c}, C₁-C₂ alkoxy, O-C₁-C₂ haloalkyl, C₁-C₂ alkyl, C₁-C₂ heteroalkyl, C₁-C₂ haloalkyl, C₁-C₂ alkylene-O-C₁-C₂ alkyl, C₁-C₂ alkylene-C₃-C₄ cycloalkyl, heterocycle, C₃-C₆ cycloalkyl, aryl, heteroaryl, C₃-C₆ cycloalkyl-amino, heterocyclo-amino, aryl-amino, heteroaryl-amino, -CONR^{d}R^{c} , -NHCO-R^{a}, -NHCOO-C₁-C₄ alkyl, -CO-C₁-C₂ alkyl, - COO-R^{a}, which may be linked to form a ring, and wherein -CONR^{d}R^{c} and -NR^{d}R^{c} may be linked to form a seven-membered ring fused to a six-membered aryl group;
   with aryl, heteroaryl, heterocycle and cycloalkyl optionally substituted by one or more substituents selected from halogen oxo, cyano, OH, -NOOH, C₁-C₆ alkyl, C₁-C₆ alkoxy, halo-C₁-C₆ alkoxy, C₃-C₆-cycloalkyl-C₁-C₃-alkoxy, -O-CO-C₁-C₆ alkyl, C₃-C₆ cycloalkyl, C₁-C₂ haloalkyl, C₂-C₄ heterocycle, acetyl, acetyl-phenyl, -CONR^{d}R^{c}, COO-C₁-C₈ alkyl, -S(O)NH-C₁-C₂-alkyl, -S(O)ₙ-C₁-C₂-alkyl, heteroaryl which may be optionally substituted with one or more halogen group, C₁-C₂-alkyl,
   with n equal 0, 1 or 2 ;
      R⁴ is selected from halogen, OH, CN, NO₂, NH₂, NHMe, NMe₂, SH, SMe, SOMe, SO₂Me, C₁-C₄ alkyl, C₃-C₄ cycloalkyl, C₁-C₃ alkoxy, C₁-C₃ heteroalkyl, heterocycle,
   any of which may be optionally substituted by one or more substituents selected from halogen, =O, OH, SMe, C₁-C₂ alkoxy, O-C₁-C₂ haloalkyl, C₁-C₂ alkyl, C₁-C₂ heteroalkyl, C₁-C₂ haloalkyl NH₂, NHMe, NMe₂, which may be linked to form a ring;
      R¹⁴ is C=O, C=S, C=N-OH, C=NR^{b}, C=CH-NO₂, SO₂, and C₃-C₆ cycloalkenyl which may be substituted with 1 or 2 =O;
      R^{a} is selected from H, C₁-C₈ alkyl, heteroalkyl, C₁-C₈ alkoxy, heterocycle, C₃-C₈ cycloalkyl, heteroaryl and aryl,
   any of which may be optionally substituted by one or more substituents selected from halogen, =O, OH, SMe, C₁-C₂ alkoxy, -O-C₁-C₂ haloalkyl, C₁-C₂ alkyl, C₃-C₆ cycloalkyl, C₃-C₆ heterocycloalkyl, C₁-C₂ heteroalkyl, C₁-C₂ haloalkyl, aryl, heteroaryl, NH₂, NHMe, NMe₂ which may be linked to form a ring;
      R^{b} is selected from CN, aryl, heteroaryl, -NO₂, -O-C₁-C₂ alkyl, -CO-O-C₁-C₂ alkyl,
   with aryl and heteroaryl optionally substituted by one or more C₁-C₂ alkyl;
      R^{C} is selected from H, aryl, heteroaryl, C₃-C₆ cycloalkenyl,C₃-C₈ cycloalkyl, heterocycle, C₁-C₈ alkyl, C₁-C₈ heteroalkyl, C₁-C₄ alkylene-C₃-C₆ cycloalkyl;
   any of which may be optionally substituted by one or more substituents selected from C(O)(R^{g}), C(O)N(R^{f})(R^{g}), halogen, SF₅, OH, =O, -COH, -COOH, -CO-O-R^{a}, CN, SMe, SO₂Me, SO₂R^{g}, C₁-C₄ alkoxy, O-C₁-C₄ haloalkyl, C₁-C₄ alkyl, C₁-C₂ heteroalkyl, C₁-C₄ haloalkyl, N(R^{f})(R^{g}), N(R^{f})SO₂(R^{g}), SO₂NH₂, SO₂NHMe, SO₂NMe₂, S(O)(NR^{f})R^{g}, C₃-C₆ cycloalkyl, heterocycle, C₁-C₄ alkylene-C₃-C₆ cycloalkyl, aryl and heteroaryl, which may be linked to form a ring
   with aryl, heteroaryl, heterocycle and cycloalkyl optionally substituted by one or more substituents selected from halogen, =O, =NH, =NMe, OH, C₁-C₂ alkyl, C₁-C₂ alkoxy, -COOH, -COO-C₁-C₂ alkyl, SO₂R^{a}, COR^{a}, SOR^{a}, S(O)(NH)R^{a}, CONHR^{a}, which may be linked to form a ring;
      R^{d} represents H or C₁-C₄ alkyl
      and R^{c} and R^{d} may be linked to form a heterocycle;
      R^{f} is selected from H, aryl, heteroaryl, C₃-C₆ cycloalkenyl, C₃-C₈ cycloalkyl, heterocycle, C₁-C₈ alkyl, C₁-C₈ heteroalkyl, C₁-C₄ alkylene-C₃-C₆ cycloalkyl ,
   any of which may be optionally substituted by one or more substituents selected from halogen, =O, =NH, =NMe, OH, aryl, heteroaryl, C₁-C₂ alkyl, C₁-C₂ haloalkyl, C₁-C₂ alkoxy, O-C₁-C₂ haloalkyl, -COOH, -COO- C₁-C₂ alkyl, SO₂R^{a}, COR^{a}, SOR^{a}, S(O)(NH)R^{a}, CONHR^{a};
      R^{g} is selected from H, aryl, heteroaryl, C₃-C₆ cycloalkenyl, C₃-C₈ cycloalkyl, heterocycle, C₁-C₈ alkyl, C₁-C₈ heteroalkyl, C₁-C₄ alkylene-C₃-C₆ cycloalkyl ,
   any of which may be optionally substituted by one or more substituents selected from halogen, =O, =NH, =NMe, OH, aryl, heteroaryl, C₁-C₂ alkyl, C₁-C₂ haloalkyl, C₁-C₂ alkoxy, O-C₁-C₂ haloalkyl, -COOH, -COO- C₁-C₂ alkyl, SO₂R^{a}, COR^{a}, SOR^{a}, S(O)(NH)R^{a}, CONHR^{a}
   R^{f} and R^{g} may be linked to form a ring.

According to another embodiment, compounds of the invention are according to formula (la) 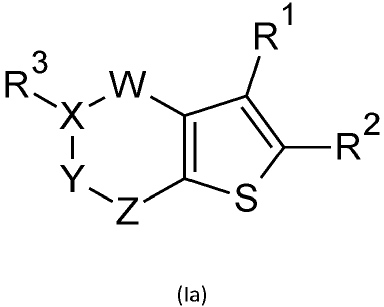 wherein
X represents CH, N, or C-R⁴, wherein when X is C-R⁴, R³ and R⁴ may be linked to form a 4-6 membered cycloalkyl or heterocyclic ring,
   any of which may be optionally substituted by one or more substituents selected from halogen, =O, OH, SMe, C₁-C₂ alkoxy, O-C₁-C₂ haloalkyl, C₁-C₂ alkyl, C₁-C₂ heteroalkyl, C₁-C₂ haloalkyl, NH₂, NHMe, NMe₂, aryl and heteroaryl, with aryl and heteroaryl optionally substituted by one or more substituents selected from halogen, OH, C₁-C₆ alkyl, C₁-C₆ alkoxy and C₁-C₂ haloalkyl;
when X represents N, then W, Y and Z are C or one of W, Y and Z is a single bond, the other two being C;
when X represents CH or C-R⁴, then W, Y and Z are C, or one of W, Y or Z is a single bond, O, S, S(O), S(O)₂ or N, the other two being C;
   when W, Y, or Z are C, each C may be substituted by 1 or 2 groups which are independently selected from H, halogen, SF₅, =O, =NOH, -OH, -CN, -C(O)Me, -C(O)OH, -C(O)NH₂, NH-C(O)O-C₁-C₄ alkyl, -NO₂, NH₂, NHMe, NMe₂, SH, SMe, SOMe, SO₂Me, C₁-C₄ alkyl, C₃-C₄ cycloalkyl, C₁-C₄ alkoxy, heterocycle, C₁-C₄ heteroalkyl, which may be linked to form a 3-6 membered cycloalkyl or 4-6 membered heterocyclic ring,
   any of which may be optionally substituted by one or more substituents selected from halogen, =O, OH, SMe, C₁-C₂ alkoxy, O-C₁-C₂ haloalkyl, C₁-C₂ alkyl, C₁-C₂ heteroalkyl, C₁-C₂ haloalkyl, NH₂, NHMe, NMe₂, which may be linked to form a 3-6 membered cycloalkyl or 4-6 membered heterocyclic ring;
   wherein
   when W, Y or Z is N, this N may be independently substituted by H, C₁-C₄ alkyl, C₃-C₄ cycloalkyl, heterocycle, -C(O)Me, -C(O)NH₂, -C(O)OH, -S(O)Me, -S(O)₂Me, -S(O)₂NH₂, any of which may be optionally substituted by one or more substituents selected from halogen, OH, SMe, C₁-C₂ alkoxy, O-C₁-C₂ haloalkyl, C₁-C₂ alkyl, C₁-C₂ heteroalkyl, C₁-C₂ haloalkyl, NH₂, NHMe, NMe₂;
   and W, X, Y and Z may be linked directly or via substituents to form a ring;
      R¹ represents 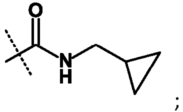
      R² represents 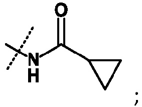
      R³ is selected from H, -OH, C₁-C₈ alkyl, -NR^{d}R^{c},
      -NH-R¹⁴-NH-R^{a}, -NMe-R¹⁴-NH-R^{a}, -NH-R¹⁴-NMe-R^{a}, -CO-NR^{d}R^{c}, -NH-COO-R^{c}, -NH-CO-R^{c}, -COOH, - COO-C₁-C₈ alkyl, -CO-C₁-C₈ alkyl, -C₁-C₈ alkylene-COO-C₁-C₈ alkyl, -C₁-C₈ alkylene-NR^{a} -COO-C₁-C₈ alkyl, -C₁-C₈ alkylene-NR^{d}R^{c}, -C₁-C₈ alkylene-NH-R¹⁴-NH-R^{a}, heteroaryl, -CN, -CH=N-OH, -CF₃, alkylamino, amino, -NH-SO-C₁-C₈ alkyl, -NH-SO₂-C₁-C₈ alkyl, heteroalkyl-amino, aryl, heterocycle, C₃-C₆ cycloalkyl, -NHS(O)(=NH)Me, -SO₂-C₁-C₈ alkyl, -NH-C(CH₃)=N-CN, -NH-CH=N-CN, -NH-S(=O)(=N)-C₁-C₂ alkyl,
   any of which may be optionally substituted by one or more substituents selected from halogen, -OH, =O, -COOH, -COH, -CN, SMe, -NR^{d}R^{c}, -OR^{c}, C₁-C₂ alkoxy, O-C₁-C₂ haloalkyl, C₁-C₂ alkyl, C₁-C₂ heteroalkyl, C₁-C₂ haloalkyl, C₁-C₂ alkylene-O-C₁-C₂ alkyl, C₁-C₂ alkylene-C₃-C₄ cycloalkyl, heterocycle, C₃-C₆ cycloalkyl, aryl, heteroaryl, C₃-C₆ cycloalkyl-amino, heterocycle-amino, aryl-amino, heteroaryl-amino, -CONR^{d}R^{c} , -NHCO-R^{a}, -NHCOO-C₁-C₄ alkyl, -CO-C₁-C₂ alkyl, -COO-R^{a}, which may be linked to form a ring,
   with aryl, heteroaryl, heterocycle and cycloalkyl optionally substituted by one or more substituents selected from halogen, OH, C₁-C₆ alkyl, C₁-C₆ alkoxy, -O-CO-C₁-C₆ alkyl, C₃-C₆ cycloalkyl, C₁-C₂ haloalkyl and C₂-C₄ heterocycle;
      R⁴ is selected from halogen, OH, CN, NO₂, NH₂, NHMe, NMe₂, SH, SMe, SOMe, SO₂Me, C₁-C₄ alkyl, C₃-C₄ cycloalkyl, C₁-C₃ alkoxy, C₁-C₃ heteroalkyl, heterocycle,
   any of which may be optionally substituted by one or more substituents selected from halogen, =O, OH, SMe, C₁-C₂ alkoxy, O-C₁-C₂ haloalkyl, C₁-C₂ alkyl, C₁-C₂ heteroalkyl,
      C₁-C₂ haloalkyl NH₂, NHMe, NMe₂, which may be linked to form a ring;
      R¹⁴ is C=O, C=S, C=N-OH, C=NR^{b}, C=CH-NO₂, SO₂, and C₃-C₆ cycloalkenyl which may be substituted with 1 or 2 =O;
      R^{a} is selected from H, C₁-C₈ alkyl, heteroalkyl, C₁-C₈ alkoxy, heterocycle, C₃-C₈ cycloalkyl, heteroaryl and aryl,
   any of which may be optionally substituted by one or more substituents selected from halogen, =O, OH, SMe, C₁-C₂ alkoxy, -O-C₁-C₂ haloalkyl, C₁-C₂ alkyl, C₃-C₆ cycloalkyl, C₁-C₂ heteroalkyl, C₁-C₂ haloalkyl, aryl, heteroaryl, NH₂, NHMe, NMe₂ which may be linked to form a ring; R^{b} is selected from CN, aryl, heteroaryl, -NO₂, -O-C₁-C₂ alkyl, -CO-O-C₁-C₂ alkyl,
   with aryl and heteroaryl optionally substituted by one or more C₁-C₂ alkyl;
      R^{C} is selected from H, aryl, heteroaryl, C₃-C₆ cycloalkenyl,C₃-C₈ cycloalkyl, heterocycle, C₁-C₈ alkyl, C₁-C₈ heteroalkyl, C₁-C₄ alkylene-C₃-C₆ cycloalkyl;
   any of which may be optionally substituted by one or more substituents selected from C(O)(R^{g}), C(O)N(R^{f})(R^{g}), halogen, SF₅, OH, =O, -COH, -COOH, -CO-O-R^{a}, CN, SMe, SO₂Me, SO₂R^{g}, C₁-C₄ alkoxy, O-C₁-C₄ haloalkyl, C₁-C₄ alkyl, C₁-C₂ heteroalkyl, C₁-C₄ haloalkyl, N(R^{f})(R^{g}), N(R^{f})SO₂(R^{g}), SO₂NH₂, SO₂NHMe, SO₂NMe₂, S(O)(NR^{f})R^{g}, C₃-C₆ cycloalkyl, heterocycle, C₁-C₄ alkylene-C₃-C₆ cycloalkyl, aryl and heteroaryl, which may be linked to form a ring
   with aryl, heteroaryl, heterocycle and cycloalkyl optionally substituted by one or more substituents selected from halogen, =O, =NH, =NMe, OH, C₁-C₂ alkyl, C₁-C₂ alkoxy, -COOH, -COO-C₁-C₂ alkyl, SO₂R^{a}, COR^{a}, SOR^{a}, S(O)(NH)R^{a}, CONHR^{a}, which may be linked to form a ring;
      R^{d} represents H or C₁-C₄ alkyl
      and R^{c} and R^{d} may be linked to form a heterocycle;
      R^{f} is selected from H, aryl, heteroaryl, C₃-C₆ cycloalkenyl, C₃-C₈ cycloalkyl, heterocycle, C₁-C₈ alkyl, C₁-C₈ heteroalkyl, C₁-C₄ alkylene-C₃-C₆ cycloalkyl ,
   any of which may be optionally substituted by one or more substituents selected from halogen, =O, =NH, =NMe, OH, aryl, heteroaryl, C₁-C₂ alkyl, C₁-C₂ haloalkyl, C₁-C₂ alkoxy, O-C₁-C₂ haloalkyl, -COOH, -COO- C₁-C₂ alkyl, SO₂R^{a}, COR^{a}, SOR^{a}, S(O)(NH)R^{a}, CONHR^{a};
      R^{g} is selected from H, aryl, heteroaryl, C₃-C₆ cycloalkenyl, C₃-C₈ cycloalkyl, heterocycle, C₁-C₈ alkyl, C₁-C₈ heteroalkyl, C₁-C₄ alkylene-C₃-C₆ cycloalkyl ,
   any of which may be optionally substituted by one or more substituents selected from halogen, =O, =NH, =NMe, OH, aryl, heteroaryl, C₁-C₂ alkyl, C₁-C₂ haloalkyl, C₁-C₂ alkoxy, O-C₁-C₂ haloalkyl, -COOH, -COO- C₁-C₂ alkyl, SO₂R^{a}, COR^{a}, SOR^{a}, S(O)(NH)R^{a}, CONHR^{a}
   R^{f} and R^{g} may be linked to form a ring.

In a preferred embodiment of the present invention, X, W, Y and Z are preferably C or alternatively, X, W and Y are preferably C and Z is preferably O, all of which may be substituted as described above.

More preferably X is CH and W, Y and Z are independently selected from CH₂, CH(C₁-C₂ alkyl), CH(C₁-C₂ haloalkyl), C(C₁-C₂ alkyl)₂ and spiro-cyclopropyl. Most preferably, X is CH and W, Y and Z are CH₂.

In this case, the preferred compounds are characterized by formula (II): 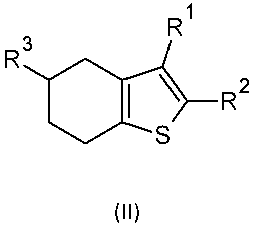 wherein R¹, R² and R³ are as defined in formula (I) or (la).

Preferred embodiments according to the invention are compounds of formula (I), (Ia) or (II) and salts thereof wherein R¹, R² and R³ are as defined below. It will be understood by those skilled in the art that the present invention also encompasses compounds of formula (I), (la) and (II) and salts thereof wherein various substituents are independently selected from these embodiment, preferred embodiment, more preferred embodiment, preferably, more preferably, even more preferably, most preferably, specific embodiment, preferred specific embodiment, specific preferred embodiment, specific more preferred embodiment, particularly preferred embodiment, best results. This also includes compounds of formula (III) to (XXIV) defined below.
R¹ is 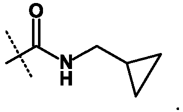
R² represents

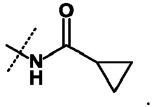

In a preferred embodiment, R³ is selected from -NHR^{c}, -NH-R¹⁴-NH-R^{a}, and heteroaryl, any of which may be substituted by one or more substituents selected from - NHR^{c}, -C(O)O-R^{a}, - C(O)NR^{d}R^{c}, aryl, heteroaryl, C₁-C₂ haloalkyl and -CN, where R^{c} is selected from H, optionally substituted heteroaryl, optionally substituted heterocycle and optionally substituted aryl,
wherein substituents of aryl, heterocycle and heteroaryl are selected from halogen, -CN, - C(O)OH, -C(O)O-C₁-C₂ alkyl, -C(O)Me, -S(O)Me, -S(O)(O)Me, -S(O)(=NH)Me, heterocycle, C₁-C₄ alkyl, C₁-C₂ heteroalkyl, C₁-C₂ haloalkyl, C₁-C₂ alkoxy, C₁-C₂ haloalkoxy, aryl, heteroaryl, C₃-C₈ cycloalkyl. In a specific preferred embodiment, R³ is heteroaryl, containing at least one nitrogen as heteroatom, which may be substituted by either -NR^{d}R^{c} or -NR^{d}R^{c} and -C(O)O-C₁-C₂ alkyl, R^{c} is selected from H, optionally substituted heteroaryl, optionally substituted heterocycle and optionally substituted aryl,
wherein substituents of aryl, heterocycle and heteroaryl are selected from -CN, -C(O)O-C₁-C₂ alkyl, C₁-C₄ alkyl, C₁-C₂ haloalkyl, C₃-C₆ cycloalkyl, C₁-C₂ haloalkoxy, -S(O)Me and -S(O)(O)Me and
R^{d} represents H.

In a specific more preferred embodiment, R³ is heteroaryl, containing at least two nitrogen as heteroatom, which may be substituted by either -NHR^{c} or -NHR^{c} and -C(O)O-C₁-C₂ alkyl, R^{c} is selected from H or optionally substituted heteroaryl, containing at least one nitrogen as heteroatom,
wherein substituents of heteroaryl are selected from C₁-C₃ alkyl, C₁-C₂ haloalkyl and C₁-C₂ haloalkoxy wherein at least one halogen is a fluorine atom.

The best results have been obtained with R³ being heteroaryl, containing three nitrogen as heteroatom, which may be substituted by -NHR^{c},
R^{c} is optionally substituted heteroaryl, containing at least one nitrogen as heteroatom,
wherein substituents of heteroaryl are selected from methyl, ethyl, difluoromethyl or trifluoromethyl.

Most preferably R³ is,

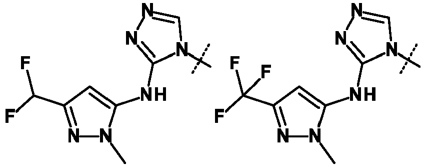

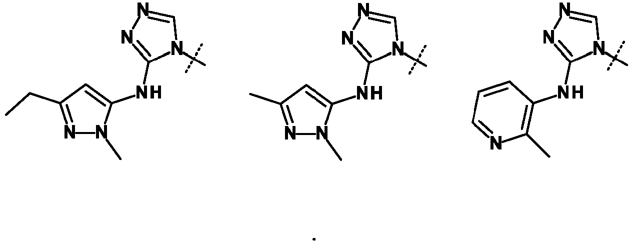

In a further embodiment, the compounds are characterized by formula (I) or (la) wherein X is C or N; Z is C, N, O or S; Y is C or S.

In a further embodiment, the compounds are characterized by formula (I) or (la) wherein X Y, W is C and Z is N wherein N is substituted with a group selected from methylsulfonyl, acetyl ethylcarbamoyl, cyano, other substituents are as defined above and below.

In a further embodiment, the compounds are characterized by formula (I) or (la) wherein X, Y, W, Z is C and wherein Z is substituted with a group selected from OH, methyl, ethylcarbamoyl, cyano, oxo, tert-butoxycarbonylamino, 1H-pyrazol-3-yl, other substituents are as defined above and below,

In another embodiment, the compounds are characterized by formula (I) or (la) wherein X, Y, W, Z is C and wherein W is substituted with a methyl group and R³ is H or a methy group, other substituents are as defined above and below.

In a further embodiment, compounds of the invention are characterized by formula (I) or (Ia) whererin X is C or N and R³ is selected from H, OH, methyl, amino, methylsulfonyl, acetyl, 2-methoxyacetyl, 2-phenylacetyl, 2-(tert-butoxycarbonylamino)ethyl, 2-phenylethyl, pyrimidin-2-ylamino, methanesufonamido, (4-methoxyphenyl)carbamothioylamino, (4-Methoxyphenyl)carbamoylamino, ethylcarbamoyl, [N'-Cyano-N-(p-tolyl)carbamimidoyl]amino, [N'-Cyano-N-ethyl-carbamimidoyl]amino, (1-oxoisoindolin-5-yl)carbamoyl, (3-sulfamoylphenyl)carbamoyl, (3-methylsulfonylphenyl)carbamoyl, 3-isoxazol-5-ylanilino, (2-ethoxy-3,4-dioxo-cyclobuten-1-yl)amino, ethylcarbonyl, carboxy, (1-oxoisoindolin-5-yl)aminocarbonyl, [(5-oxopyrrolidin-3-yl)phenyl]aminocarbonyl, [3-(3-methyl-5-oxo-4H-pyrazol-1-yl)phenyl]carbonylamino, (1H-indazol-4-yl)carbonylamino, 1H-imidazo[4,5-b]pyrazin-2-yl, indan-2-yl, [2-(4-methoxyphenyl)ethylaminocarbonyl, quinoxalin-6-yl, (thiazol-2-yl)aminocarbonyl, (1H-tetrazol-5-yl)aminocarbonyl, (4-pyridyl)aminocarbonyl, pyrazin-2-ylaminocarbonyl, (3-pyridyl)aminocarbonyl, 3-hydroxyazetidine-1-carbonyl, 1,1-dioxo-1,4-thiazinane-4-carbonyl, 2-(4-hydroxyphenyl)ethylaminocarbonyl, hydroxyiminomethyl, [(4-ethyl-1,2,4-triazol-3-yl)amino]methyl, (4-methylanilino)methyl, 2-Amino-3-pyridyl, 5-(p-tolyl)-1H-imidazol-2-yl, cyano, trifluoromethyl, 4-methyloxazol-2-yl, aminomethyl, (p-tolylcarbamoylamino)methyl, 3-methyl-1,2,4-oxadiazol-5-yl, 5-methyl-1,3,4-oxadiazol-2-yl, pyrrolidine-1-carbonyl, 3-pyridylcarbamothioylamino, 3-(3-pyridylamino)-1,2,4-triazol-4-yl, 1H-pyrazol-3-yl, 5-Amino-4-cyano-imidazol-1-yl, 3-(2,2-dimethylpropylamino)-1,2,4-triazol-4-yl, 3-[(2,5-dimethylpyrazol-3-yl)amino]-1,2,4-triazol-4-yl, 4-(2,5-dimethylpyrazol-3-yl)-1,2,4-triazol-3-yl]amino, [(2,5-dimethylpyrazol-3-yl)amino]-1,2,4-triazol-4-yl, N'-Cyano-N-phenyl-carbamimidoylamino, 3-(isobutylamino)-1,2,4-triazol-4-yl, 3-[(1-methylcyclopropyl)methylamino]-1,2,4-triazol-4-yl, 5-Acetamidoimidazol-1-yl, 3-[(5-methyl-3-pyridyl)amino]-1,2,4-triazol-4-yl, 3-(4-pyridylamino)-1,2,4-triazol-4-yl, N'-Cyano-N-(3,4-dimethoxyphenyl)carbamimidoyl]amino, 4-ethoxycarbony-5-(ethylamino)imidazole-1-yl, 3-(2-morpholinoethylamino)-1,2,4-triazol-4-yl, (5-oxo-4H-1,2,4-oxadiazol-3-yl)amino, 2-(ethylamino)-3,4-dioxo-cyclobuten-1-yl]amino, [N'-cyano-N-(2,2,2-trifluoroethyl)carbamimidoyl]amino, 3-(ethylamino)-1,2,4-triazol-4-yl, (N-ethyl-N'-nitro-carbamimidoyl)amino, 3-(3-pyridylamino)-1,2,4-triazol-4-yl, 3-[(1-oxidopyridin-1-ium-3-yl)amino, [N'-nitro-N-(p-tolyl)carbamimidoyl]amino, 2-(3-pyridylamino)imidazol-1-yl, 3-(3-pyridylamino)-1,2,4-triazol-4-yl, 3-(cyclopropylmethylamino, (2,5-dimethylpyrazol-3-yl)carbamothioylamino, 3-(pyrimidin-5-ylamino)-1,2,4-triazol-4-yl, 2-(cyclopropylmethylamino)imidazol-1-yl, [4-(ethylamino)-1,1-dioxo-1,2,5-thiadiazol-3-yl]amino, [1-(4-methylanilino)-2-nitro-vinyl]amino, (N'-cyano-N-ethyl-carbamimidoyl)-methyl-amino, [2-(4-methylanilino)-3,4-dioxo-cyclobuten-1-yl]amino, 3-(2-pyridylamino)-1,2,4-triazol-4-yl, 2-Acetamidoimidazol-1-yl, 5-(N-tert-butyloxycarbonylamino)-triazol-1-yl, 5-(3-pyridylamino)triazol-1-yl, ethylcarbamothioylamino, 3-[[(3S)-tetrahydrofuran-3-yl]amino]-1,2,4-triazol-4-yl, [(E)-N-ethyl-N'-nitro-carbamimidoyl]amino, 2-(ethylamino)imidazol-1-yl, 3-[(3,5-dimethylisoxazol-4-yl)amino, [[N'-ethoxycarbonyl-N-(p-tolyl)carbamimidoyl]amino], [2-[(2-methyl-3-pyridyl)amino]imidazol-1-yl, 1-[(ethylamino)-2-nitro-vinyl]amino, (4-methyl-6-oxo-1H-pyrimidin-2-yl)amino, 5-amino-4-(methylcarbamoyl)imidazol-1-yl, 5-amino-4-carbamoyl-imidazol-1-yl, 2-Anilinoimidazol-1-yl, ethylcarbamoylamino, (4-oxo-1H-pyridin-2-yl)amino, [6-(5-methyl-1,3,4-oxadiazol-2-yl)-3-pyridyl]amino, 5-(ethylamino)tetrazol-1-yl, 1H-1,2,4-triazol-3-ylamino, 4-(1-methylpyrazol-4-yl)-1,2,4-triazol-3-yl]amino, 4-(ethyloxy-carbonyl)imidazol-1-yl, [4-(4-fluorophenyl)-1,2,4-triazol-3-yl]amino, 1H-imidazol-2-ylamino, N'-methoxy-N-(p-tolyl)carbamimidoyl]amino, [4-(4-methylanilino)-1,1-dioxo-1,2,5-thiadiazol-3-yl]amino, [1-(3-pyridyl)imidazol-2-yl]amino, 5-aminotriazol-1-yl, (2-methoxyphenyl)carbamoylamino, 4-pyridine-carbonylamino, benzylamino, 1H-Benzimidazol-2-ylamino, [N'-hydroxycarbamimidoyl]amino, [N'-(1-methylpyrazol-3-yl)-N-(p-tolyl)carbamimidoyl]amino, 3-[(3,5-dimethylpyrazin-2-yl)amino, 3-(ethylamino)-5-(methoxymethyl)-1,2,4-triazol-4-yl, [5-(3-pyridyl)-1H-1,2,4-triazol-3-yl]amino, p-tolyl-carbonylamino, quinazolin-2-ylamino, 1,3-Benzoxazol-2-ylamino, 3-(ethylamino)-5-(hydroxymethyl)-1,2,4-triazol-4-yl, 2-aminoimidazol-1-yl, 3-amino-5-oxo-1,2,4-oxadiazol-4-yl, 3-(ethylamino)-5-methyl-1,2,4-triazol-4-yl, (2-ethyl-3,4-dioxo-cyclobuten-1-yl)amino, 1,3,4-oxadiazol-2-ylamino, 5-oxo-1-(p-tolyl)-4H-imidazol-2-yl]amino, [2-[ethyl(methyl)amino]-3,4-dioxo-cyclobuten-1-yl]amino, (methylsulfonimidoyl)amino, [N-Cyano-C-methyl-carbonimidoyl]amino, tetrazol-1-yl, methanesulfinamido, morpholino, 3-(ethylamino)-5-(trifluoromethyl)-1,2,4-triazol-4-yl, (4-oxo-1H-pyrimidin-2-yl)amino, isopropylsulfamoylamino, *N*'-Cyano-*N*-morpholino-carbamimidoyl]amino, acetamido, methanesulfonamido, [N'-Cyano-N-methoxy-carbamimidoyl]amino, (4-methoxyphenyl)carbamoylamino, [4-oxo-6-(trifluoromethyl)-1H-pyrimidin-2-yl]amino, [4-(trifluoromethoxy)phenyl]carbamoylamino, 3-(2-methylpropanoylamino)-1,2,4-triazol-4-yl, 3-[(2,5-dimethylpyrazol-3-yl)-methyl-amino]-1,2,4-triazol-4-yl, 5-amino-4-(5-methyl-1,3,4-oxadiazol-2-yl)imidazol-1-yl, 3-[(3-methyl-1H-pyrazol-5-yl)amino]-1,2,4-triazol-4-yl, 3-[(1,3-dimethylpyrazol-4-yl)amino]-1,2,4-triazol-4-yl -[(5-cyclopropyl-2-methyl-pyrazol-3-yl)amino]-1,2,4-triazol-4-yl, 3-[(5-isopropyl-2-methyl-pyrazol-3-yl)amino]-1,2,4-triazol-4-yl, 3-[[2-methyl-5-(trifluoromethyl)pyrazol-3-yl]amino]-1,2,4-triazol-4-yl, 3-[(2-isopropyl-5-methyl-pyrazol-3-yl)amino, 3-[(5-methyl-2-tetrahydrofuran-3-yl-pyrazol-3-yl)amino]-1,2,4-triazol-4-yl, 3-[(2-methylpyrazol-3-yl)amino]-1,2,4-triazol-4-yl, 3-[(2-ethyl-5-methyl-pyrazol-3-yl)amino]-1,2,4-triazol-4-yl, 3-[(2-cyclopropyl-5-methyl-pyrazol-3-yl)amino]-1,2,4-triazol-4-yl, 3-[(3-methoxy-1-methyl-pyrazol-4-yl)amino]-1,2,4-triazol-4-yl, 3-[(5-ethyl-2-methyl-pyrazol-3-yl)amino]-1,2,4-triazol-4-yl, 3-[[5-(difluoromethyl)-2-methyl-pyrazol-3-yl]amino, 3-[(1,4-dimethylpyrazol-3-yl)amino]-1,2,4-triazol-4-yl, [3-[(5-methoxycarbonyl-2-methyl-pyrazol-3-yl)amino]-1,2,4-triazol-4-yl], 2-[[2-methyl-5-(trifluoromethyl)pyrazol-3-yl]amino]imidazol-1-yl, [3-[(5-carboxy-2-methyl-pyrazol-3-yl)amino]-1,2,4-triazol-4-yl], other substituents are as defined above and below.

In a further embodiment, the compounds are characterized by formula (I) or (la) wherein W, X, Y, Z is C and Z is optionally substituted with a methyl group, other substituents are as defined above and below.

In a further embodiment, the compounds are characterized by formula (I) or (la) wherein R³ is selected from (1,1-dioxothiolan-3-yl)amino-1,2,4-triazol-4-yl, 3-[3-[methylsulfonimidoyl]anilino]-1,2,4-triazol-4-yl, 3-[(1-acetyl-3-methylpyrrolidin-3-yl)amino]-4H-1,2,4-triazol-4-yl, 3-{[1-(6-methylpyridazin-3-yl)pyrrolidin-3-yl]amino}-4H-1,2,4-triazol-4-yl, 3-[[-1-pyrazin-2-ylpyrrolidin-3-yl]amino]-1,2,4-triazol-4-yl, 3-[(4-methyl-1-methylsulfonyl-pyrrolidin-3-yl)amino]-1,2,4-triazol-4-yl, 3-[(1-oxo-2,3-dihydro-1H-isoindol-4-yl)amino]-4H-1,2,4-triazol-4-yl, 3-[[-1-(6-fluoropyridazin-3-yl)pyrrolidin-3-yl]amino]-1,2,4-triazol-4-yl, 3-[[1-pyrimidin-2-ylpyrrolidin-3-yl]amino]-1,2,4-triazol-4-yl, 5-amino-4-(2-methoxyethoxycarbonyl)imidazol-1-yl, 5-amino-4-(2-morpholinethoxycarbonyl)imidazol-1-yl, 3-(3-methylsulfanylanilino)-1,2,4-triazol-4-yl, 3-(3-methylsulfonylanilino)-1,2,4-triazol-4-yl, 3-(3-methylsulfinylanilino)-1,2,4-triazol-4-yl, 3-[3-(methylsulfonimidoyl)anilino]-1,2,4-triazol-4-yl, 3-[(6-methylsulfinyl-2-pyridyl)amino]-1,2,4-triazol-4-yl, 3-[(6-methylsulfonyl-2-pyridyl)amino]-1,2,4-triazol-4-yl, 8-methoxy-4-oxo-5,10-dihydroimidazo[4,5-c][1,5]benzodiazepin-1-yl, 8-methoxy-5-methyl-4-oxo-10H-imidazo[4,5-c][1,5]benzodiazepin-1-yl, 5-amino-4-[(2S)-2-methylpyrrolidine-1-carbonyl]imidazol-1-yl, 5-amino-4-[(3R)-3-hydroxypyrrolidine-1-carbonyl]imidazol-1-yl, 5-amino-4-[(2S)-2-methylpyrrolidine-1-carbonyl]imidazol-1-yl, 5-[[4-(dihydroxyamino)-2,5-dimethyl-3H-pyrazol-3-yl]amino]-4-ethoxycarbonyl-imidazol-1-yl, 5-[[4-(dihydroxyamino)-2,5-dimethyl-3H-pyrazol-3-yl]amino]-4-ethoxycarbonyl-imidazol-1-yl, 3-(2-methyl-3-methylsulfinyl-anilino)-1,2,4-triazol-4-yl, 3-(2-methyl-3-methylsulfonyl-anilino)-1,2,4-triazol-4-yl, 3-[(2-methyl-5-oxazol-2-yl-pyrazol-3-yl)amino]-1,2,4-triazol-4-yl, 1-acetylpyrrolidin-3-yl]amino]-1,2,4-triazol-4-yl, 1-methylsulfonylpyrrolidin-3-yl]amino]-1,2,4-triazol-4-yl, 3-[(2-methylcyclohexyl)amino]-1,2,4-triazol-4-yl, 3-[(5-oxopyrrolidin-3-yl)amino]-1,2,4-triazol-4-yl, 3-[(2-oxo-4-piperidyl)amino]-1,2,4-triazol-4-yl, 3-[(1-acetyl-2-methylpyrrolidin-3-yl)amino]-1,2,4-triazol-4-yl, 3-[(2-methyl-1-methylsulfonyl-pyrrolidin-3-yl)amino]-1,2,4-triazol-4-yl, -[3-[[3-(6-methyl-3-pyridyl)cyclobutyl]amino]-1,2,4-triazol-4-yl, , 3-[[5-(difluoromethoxy)-2-methyl-pyrazol-3-yl]amino]-1,2,4-triazol-4-yl, [[1-benzoyl-4,4-difluoro-pyrrolidin-3-yl]amino]-1,2,4-triazol-4-yl, 3-[(1-acetyl-4-piperidyl)amino]-1,2,4-triazol-4-yl, 3-[(1-methoxycarbonylpyrrolidin-3-yl)amino]-1,2,4-triazol-4-yl, 3-[[1-(ethylcarbamoyl)pyrrolidin-3-yl]amino]-1,2,4-triazol-4-yl, 3-{[2-(pyrimidin-2-yl)-2-azaspiro[4.4]nonan-7-yl]amino}-4H-1,2,4-triazol-4-yl, 3-[[2-methyl-5-(trifluoromethoxy)pyrazol-3-yl]amino]-1,2,4-triazol-4-yl, 3-[[5-[chloro(difluoro)methoxy]-2-methyl-pyrazol-3-yl]amino]-1,2,4-triazol-4-yl, 3-{[1-methyl-3-(5-methyl-1,3,4-oxadiazol-2-yl)-1H-pyrazol-5-yl]amino}-4H-1,2,4-triazol-4-yl, 3-[(5-methoxy-3-methyl-pyrazin-2-yl)amino]-1,2,4-triazol-4-yl, (5-methylthiazolo[5,4-b]pyridin-2-yl)amino, 3-[(5-cyano-2-methyl-3-pyridyl)amino]-1,2,4-triazol-4-yl, 3-[(6-cyano-2-methyl-3-pyridyl)amino]-1,2,4-triazol-4-yl, 3-[(1-methyl-2-oxo-4-pyridyl)amino]-1,2,4-triazol-4-yl, 3-[(6-chloro-2-methyl-3-pyridyl)amino]-1,2,4-triazol-4-yl, 3-[[2-methyl-6-(trifluoromethyl)-3-pyridyl]amino]-1,2,4-triazol-4-yl, 3-[(3-methylpyridazin-4-yl)amino]-1,2,4-triazol-4-yl, 3-[(5-chloro-2-methyl-pyrazol-3-yl)amino]-1,2,4-triazol-4-yl, 3-[(3-methylisothiazol-4-yl)amino]-1,2,4-triazol-4-yl, 3-[(3-methylisoxazol-4-yl)amino]-1,2,4-triazol-4-yl, 3-[[5-(cyclopropylmethoxy)-2-methyl-pyrazol-3-yl]amino]-1,2,4-triazol-4-yl, 3-[(5-ethoxy-2-methyl-pyrazol-3-yl)amino]-1,2,4-triazol-4-yl, 3-[(2,5-dimethylpyrazol-3-yl)amino]-1,2,4-triazol-4-yl, (5-amino-4-ethoxycarbonyl-imidazol-1-yl), 3-[[5-(difluoromethoxy)-2-methylpyrazol-3-yl]amino]-1,2,4-triazol-4-yl, 3-[[5-(difluoromethyl)-2-methylpyrazol-3-yl]amino]-1,2,4-triazol-4-yl, 3-[(5-methoxy-2-methylpyrazol-3-yl)amino]-1,2,4-triazol-4-yl, 3-[[5-(difluoromethyl)-2-(trideuteriomethyl)pyrazol-3-yl]amino, 3-[(5-Cyano-2-methyl-pyrazol-3-yl)amino]-1,2,4-triazol-4-yl, 3-[(2,2-difluoro-1,3-benzodioxol-5-yl)amino]-1,2,4-triazol-4-yl, 3-[(5-methoxy-2-methyl-pyrazol-3-yl)amino]-1,2,4-triazol-4-yl, 3-[[5-(difluoromethyl)-2-(trideuteriomethyl)pyrazol-3-yl]amino]-1,2,4-triazol-4-yl, 3-[(6-fluoro-2-methyl-3-pyridyl)amino]-1,2,4-triazol-4-yl, 3-[[5-(cyclopropoxy)-2-methyl-pyrazol-3-yl]amino]-1,2,4-triazol-4-yl, 3-[[2-methyl-5-(2,2,2-trifluoroethoxy)pyrazol-3-yl]amino]-1,2,4-triazol-4-yl, 3-[[6-(trifluoromethyl)-3-pyridyl]amino]-1,2,4-triazol-4-yl, 3-[(6-methoxy-3-pyridyl)amino]-1,2,4-triazol-4-yl, 3-[(2-methoxy-4-pyridyl)amino]-1,2,4-triazol-4-yl, 3-[(6-chloro-3-pyridyl)amino]-1,2,4-triazol-4-yl, 3-[(2-chloro-4-pyridyl)amino]-1,2,4-triazol-4-yl, 3-[(6-methoxy-2-methyl-3-pyridyl)amino]-1,2,4-triazol-4-yl, thiazolo[5,4-b]pyridin-2-ylamino, 3-[(3-methyltriazol-4-yl)amino]-1,2,4-triazol-4-yl, other substituents are as defined above and below.

In another preferred specific embodiment, the compound are characterized by formula (XXV) or (XXVI)

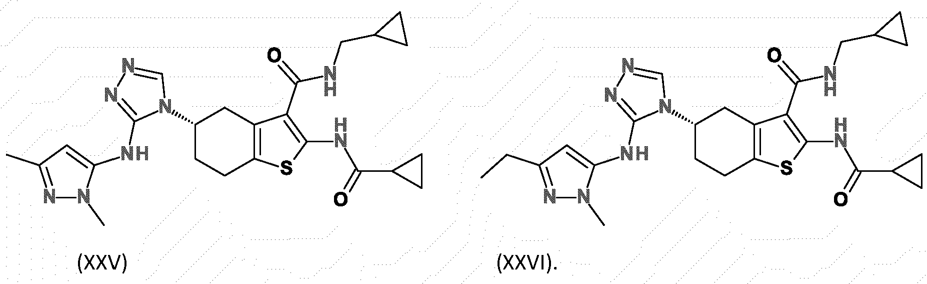

In another preferred specific embodiment, the compound are characterized by formula (XXVII) or (XXVIII)

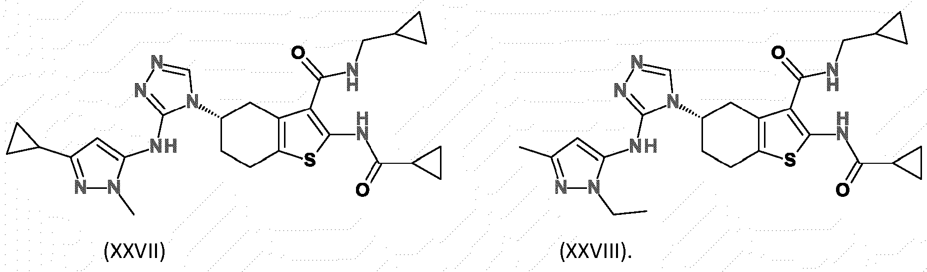

In another preferred specific embodiment, the compound are characterized by formula (XXIX) or (XXX)

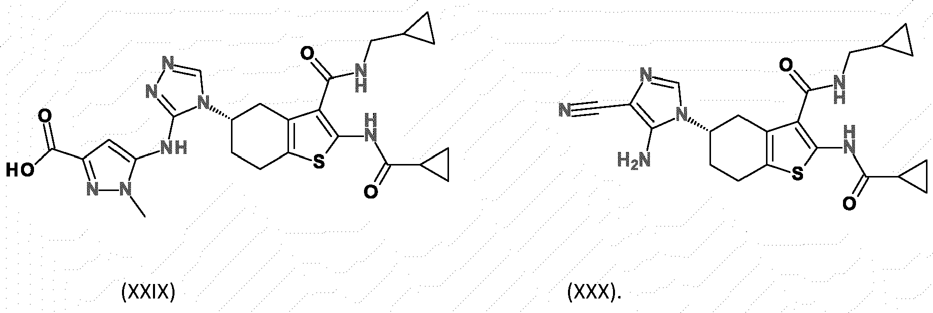

In another preferred specific embodiment, the compound are characterized by formula (XXXII)

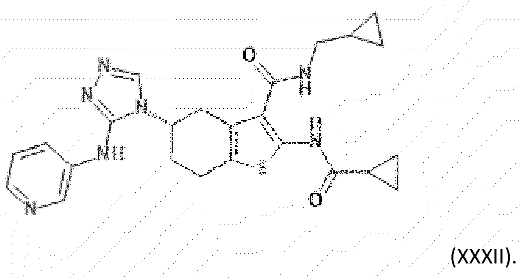

In another preferred specific embodiment, the compound are characterized by formula (XXXIII) or (XXXIV)

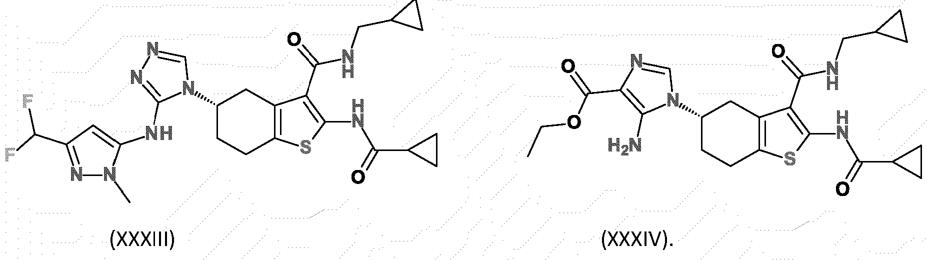

In another preferred specific embodiment, the compound are characterized by formula (XXXV) or (XXXVI)

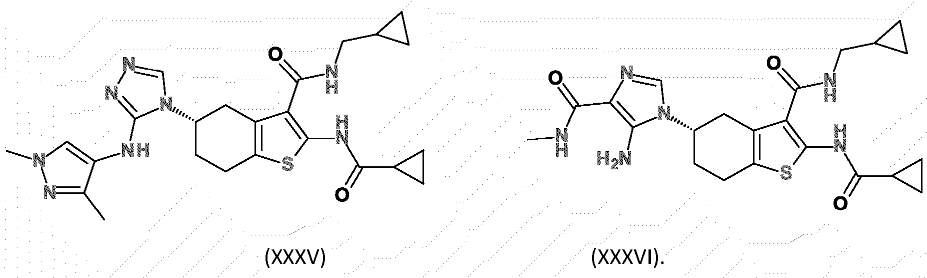

Specific compounds of formula (I) according to the present invention are selected from the group consisting of:
2-(Cyclopropanecarbonylamino)-*N*-(cyclopropylmethyl)-4,5,6,7-tetrahydrothieno[3,2-c]pyridine-3-carboxamide; hydrochloride ;
2-(Cyclopropanecarbonylamino)-*N*-(cyclopropylmethyl)-5-methylsulfonyl-6,7-dihydro-4H-thieno[3,2-c]pyridine-3-carboxamide;
5-Acetyl-2-(cyclopropanecarbonylamino)-*N*-(cyclopropylmethyl)-6,7-dihydro-4*H*-thieno[3,2-*c*]pyridine-3-carboxamide;
2-(Cyclopropanecarbonylamino)*-N*-(cyclopropylmethyl)-7-methylsulfonyl-5,6-dihydro-4*H*-thieno[2,3-*b*]pyridine-3-carboxamide ;
7-Acetyl-2-(cyclopropanecarbonylamino)-*N*-(cyclopropylmethyl)-5,6-dihydro-4*H*-thieno[2,3-b]pyridine-3-carboxamide ;
2-(Cyclopropanecarbonylamino)-*N*-(cyclopropylmethyl)-5-(2-methoxyacetyl)-6,7-dihydro-4*H-*thieno[3,2-*c*]pyridine-3-carboxamide ;
2-(Cyclopropanecarbonylamino)-*N*-(cyclopropylmethyl)-5-(2-phenylacetyl)-6,7-dihydro-4*H-*thieno[3,2-*c*]pyridine-3-carboxamide ;
*Tert-*butyl *N*-[2-[2-(cyclopropanecarbonylamino)-3-(cyclopropylmethylcarbamoyl)-6,7-dihydro-4*H-*thieno[3,2-*c*]pyridin-5-yl]ethyl]carbamate ;
2-(Cyclopropanecarbonylamino)-*N*-(cyclopropylmethyl)-5-(2-phenylethyl)-6,7-dihydro-4*H-*thieno[3,2-*c*]pyridine-3-carboxamide;
Methyl 2-[2-(cyclopropanecarbonylamino)-3-(cyclopropylmethylcarbamoyl)-6,7-dihydro-4*H-*thieno[3,2-*c*]pyridin-5-yl]acetate ;
(4*R*)-2-(Cyclopropanecarbonylamino)-*N*-(cyclopropylmethyl)-4-methyl-5,6-dihydro-4*H-*cyclopenta[*b*]thiophene-3-carboxamide ;
(4*S*)-2-(Cyclopropanecarbonylamino)-*N*-(cyclopropylmethyl)-4-methyl-5,6-dihydro-4*H-*cyclopenta[*b*]thiophene-3-carboxamide ;
Ethyl 2-(cyclopropanecarbonylamino)-3-(cyclopropylmethylcarbamoyl)-4,5,6,7-tetrahydrobenzothiophene-5-carboxylate;
2-(Cyclopropanecarbonylamino)-3-(cyclopropylmethylcarbamoyl)-4,5,6,7-tetrahydrobenzothiophene-5-carboxylic acid ;
2-(Cyclopropanecarbonylamino)-N3-(cyclopropylmethyl)-N5-(1-oxoisoindolin-5-yl)-4,5,6,7-tetrahydrobenzothiophene-3,5-dicarboxamide;
2-(Cyclopropanecarbonylamino)-N3-(cyclopropylmethyl)-N5-[3-(5-oxopyrrolidin-3-yl)phenyl]-4,5,6,7-tetrahydrobenzothiophene-3,5-dicarboxamide;
2-(Cyclopropanecarbonylamino)-N3-(cyclopropylmethyl)-N5-[3-(3-methyl-5-oxo-4H-pyrazol-1-yl)phenyl]-4,5,6,7-tetrahydrobenzothiophene-3,5-dicarboxamide;
2-(Cyclopropanecarbonylamino)-N3-(cyclopropylmethyl)-N5-(1H-indazol-4-yl)-4,5,6,7-tetrahydrobenzothiophene-3,5-dicarboxamide;2,2,2-trifluoroacetic acid;
2-(Cyclopropanecarbonylamino)-N3-(cyclopropylmethyl)-N5-(1H-indazol-5-yl)-4,5,6,7-tetrahydrobenzothiophene-3,5-dicarboxamide;2,2,2-trifluoroacetic acid;
2-(Cyclopropanecarbonylamino)-N-(cyclopropylmethyl)-5-(1H-imidazo[4,5-b]pyrazin-2-yl)-4,5,6,7-tetrahydrobenzothiophene-3-carboxamide;
2-(Cyclopropanecarbonylamino)-N3-(cyclopropylmethyl)-N5-indan-2-yl-4,5,6,7-tetrahydrobenzothiophene-3,5-dicarboxamide;
2-(Cyclopropanecarbonylamino)-N3-(cyclopropylmethyl)-N5-[2-(4-methoxyphenyl)ethyl]-4,5,6,7-tetrahydrobenzothiophene-3,5-dicarboxamide ;
2-(Cyclopropanecarbonylamino)-N3-(cyclopropylmethyl)-N5-quinoxalin-6-yl-4,5,6,7-tetrahydrobenzothiophene-3,5-dicarboxamide;
2-(Cyclopropanecarbonylamino)-N3-(cyclopropylmethyl)-N5-thiazol-2-yl-4,5,6,7-tetrahydrobenzothiophene-3,5-dicarboxamide ;
2-(Cyclopropanecarbonylamino)-N3-(cyclopropylmethyl)-N5-(1H-tetrazol-5-yl)-4,5,6,7-tetrahydrobenzothiophene-3,5-dicarboxamide ;
2-(Cyclopropanecarbonylamino)-N3-(cyclopropylmethyl)-N5-(4-pyridyl)-4,5,6,7-tetrahydrobenzothiophene-3,5-dicarboxamide ;
2-(Cyclopropanecarbonylamino)-N3-(cyclopropylmethyl)-N5-pyrazin-2-yl-4,5,6,7-tetrahydrobenzothiophene-3,5-dicarboxamide ;
2-(Cyclopropanecarbonylamino)-N3-(cyclopropylmethyl)-N5-(3-pyridyl)-4,5,6,7-tetrahydrobenzothiophene-3,5-dicarboxamide ;
2-(Cyclopropanecarbonylamino)-N-(cyclopropylmethyl)-5-(3-hydroxyazetidine-1-carbonyl)-4,5,6,7-tetrahydrobenzothiophene-3-carboxamide ;
2-(Cyclopropanecarbonylamino)-N-(cyclopropylmethyl)-5-(1,1-dioxo-1,4-thiazinane-4-carbonyl)-4,5,6,7-tetrahydrobenzothiophene-3-carboxamide ;
2-(Cyclopropanecarbonylamino)-N3-(cyclopropylmethyl)-N5-[2-(4-hydroxyphenyl)ethyl]-4,5,6,7-tetrahydrobenzothiophene-3,5-dicarboxamide ;
2-(cyclopropanecarbonylamino)-N-(cyclopropylmethyl)-5-(hydroxyiminomethyl)-4,5,6,7-tetrahydrobenzothiophene-3-carboxamide ;
2-(Cyclopropanecarbonylamino)-N-(cyclopropylmethyl)-5-[[(4-ethyl-1,2,4-triazol-3-yl)amino]methyl]-4,5,6,7-tetrahydrobenzothiophene-3-carboxamide ;
2-(Cyclopropanecarbonylamino)-N-(cyclopropylmethyl)-5-hydroxy-5-[(4-methylanilino)methyl]-6,7-dihydro-4H-benzothiophene-3-carboxamide ;
2-(Cyclopropanecarbonylamino)-N-(cyclopropylmethyl)-2'-oxo-3'-(p-tolyl)spiro[6,7-dihydro-4H-benzothiophene-5,5'-oxazolidine]-3-carboxamide ;
5-(2-Amino-3-pyridyl)-2-(cyclopropanecarbonylamino)-N-(cyclopropylmethyl)-4,5,6,7-tetrahydrobenzothiophene-3-carboxamide ;
2-(Cyclopropanecarbonylamino)-N-(cyclopropylmethyl)-5-[5-(p-tolyl)-1H-imidazol-2-yl]-4,5,6,7-tetrahydrobenzothiophene-3-carboxamide ;
5-Cyano-2-(cyclopropanecarbonylamino)-N-(cyclopropylmethyl)-4,5,6,7-tetrahydrobenzothiophene-3-carboxamide ;
7-Cyano-2-(cyclopropanecarbonylamino)-N-(cyclopropylmethyl)-4,5,6,7-tetrahydrobenzothiophene-3-carboxamide ;
(5R)-2-(Cyclopropanecarbonylamino)-N-(cyclopropylmethyl)-5-(trifluoromethyl)-4,5,6,7-tetrahydrobenzothiophene-3-carboxamide ;
(55)-2-(Cyclopropanecarbonylamino)-N-(cyclopropylmethyl)-5-(trifluoromethyl)-4,5,6,7-tetrahydrobenzothiophene-3-carboxamide ;
2-(Cyclopropanecarbonylamino)-N-(cyclopropylmethyl)-5-(4-methyloxazol-2-yl)-4,5,6,7-tetrahydrobenzothiophene-3-carboxamide ;
5-(Aminomethyl)-2-(cyclopropanecarbonylamino)-N-(cyclopropylmethyl)-5-hydroxy-6,7-dihydro-4H-benzothiophene-3-carboxamide ;
2-(Cyclopropanecarbonylamino)-N-(cyclopropylmethyl)-5-hydroxy-5-[(p-tolylcarbamoylamino)methyl]-6,7-dihydro-4H-benzothiophene-3-carboxamide ;
2-(Cyclopropanecarbonylamino)-N-(cyclopropylmethyl)-5-(3-methyl-1,2,4-oxadiazol-5-yl)-4,5,6,7-tetrahydrobenzothiophene-3-carboxamide ;
2-(Cyclopropanecarbonylamino)-N-(cyclopropylmethyl)-5-(5-methyl-1,3,4-oxadiazol-2-yl)-4,5,6,7-tetrahydrobenzothiophene-3-carboxamide ;
2-(Cyclopropanecarbonylamino)-N-(cyclopropylmethyl)-5-(pyrrolidine-1-carbonyl)-4,5,6,7-tetrahydrobenzothiophene-3-carboxamide ;
2-(Cyclopropanecarbonylamino)-N-(cyclopropylmethyl)-5-hydroxy-5-methyl-6,7-dihydro-4H-benzothiophene-3-carboxamide ;
2-(Cyclopropanecarbonylamino)-N-(cyclopropylmethyl)-4-methyl-4,5,6,7-tetrahydrobenzothiophene-3-carboxamide;
2-(Cyclopropanecarbonylamino)-N-(cyclopropylmethyl)-5,7-dimethyl-4,5,6,7-tetrahydrobenzothiophene-3-carboxamide;
2-(Cyclopropanecarbonylamino)-N-(cyclopropylmethyl)-5-methyl-4,5,6,7-tetrahydrobenzothiophene-3-carboxamide ;
2-(cyclopropanecarbonylamino)-N-(cyclopropylmethyl)-7-methyl-4,5,6,7-tetrahydrobenzothiophene-3-carboxamide ;
2-(Cyclopropanecarbonylamino)-N-(cyclopropylmethyl)-5,5-dimethyl-6,7-dihydro-4H-benzothiophene-3-carboxamide ;
2-(Cyclopropanecarbonylamino)-N-(cyclopropylmethyl)-5-methyl-7-oxo-5,6-dihydro-4H-benzothiophene-3-carboxamide ;
2'-(Cyclopropanecarbonylamino)-N-(cyclopropylmethyl)spiro[1,3-dioxolane-2,5'-6,7-dihydro-4H-benzothiophene]-3'-carboxamide ;
2-(Cyclopropanecarbonylamino)-N-(cyclopropylmethyl)-4,5-dimethyl-4,5,6,7-tetrahydrobenzothiophene-3-carboxamide ;
2-(Cyclopropanecarbonylamino)-N-(cyclopropylmethyl)-2',5'-dioxo-spiro[6,7-dihydro-4H-benzothiophene-5,4'-imidazolidine]-3-carboxamide ;
tert-Butyl N-[2-(cyclopropanecarbonylamino)-3-(cyclopropylmethylcarbamoyl)-4,5,6,7-tetrahydrobenzothiophen-7-yl]carbamate;
2-(Cyclopropanecarbonylamino)-N3-(cyclopropylmethyl)-N7-ethyl-4,5,6,7-tetrahydrobenzothiophene-3,7-dicarboxamide ;
2-(Cyclopropanecarbonylamino)-N-(cyclopropylmethyl)-5-(1H-pyrazol-3-yl)-4,5,6,7-tetrahydrobenzothiophene-3-carboxamide ;
2-(Cyclopropanecarbonylamino)-N-(cyclopropylmethyl)-7-(1H-pyrazol-3-yl)-4,5,6,7-tetrahydrobenzothiophene-3-carboxamide ;
(55)-5-(5-Amino-4-cyano-imidazol-1-yl)-2-(cyclopropanecarbonylamino)-N-(cyclopropylmethyl)-4,5,6,7-tetrahydrobenzothiophene-3-carboxamide ;
(55)-2-(Cyclopropanecarbonylamino)-N-(cyclopropylmethyl)-5-[3-(2,2-dimethylpropylamino)-1,2,4-triazol-4-yl]-4,5,6,7-tetrahydrobenzothiophene-3-carboxamide ;
(5S)-2-(Cyclopropanecarbonylamino)-N-(cyclopropylmethyl)-5-[3-[(2,5-dimethylpyrazol-3-yl)amino]-1,2,4-triazol-4-yl]-4,5,6,7-tetrahydrobenzothiophene-3-carboxamide ;
(5S)-2-(Cyclopropanecarbonylamino)-N-(cyclopropylmethyl)-5-[[4-(2,5-dimethylpyrazol-3-yl)-1,2,4-triazol-3-yl]amino]-4,5,6,7-tetrahydrobenzothiophene-3-carboxamide ;
5-Amino-2-(cyclopropanecarbonylamino)-N-(cyclopropylmethyl)-4,5,6,7-tetrahydrobenzothiophene-3-carboxamide ;
5-[[N'-Cyano-N-phenyl-carbamimidoyl]amino]-2-(cyclopropanecarbonylamino)-N-(cyclopropylmethyl)-4,5,6,7-tetrahydrobenzothiophene-3-carboxamide ;
(55)-2-(Cyclopropanecarbonylamino)-N-(cyclopropylmethyl)-5-[3-(isobutylamino)-1,2,4-triazol-4-yl]-4,5,6,7-tetrahydrobenzothiophene-3-carboxamide ;
(5S)-2-(Cyclopropanecarbonylamino)-N-(cyclopropylmethyl)-5-[3-[(1-methylcyclopropyl)methylamino]-1,2,4-triazol-4-yl]-4,5,6,7-tetrahydrobenzothiophene-3-carboxamide ;
(5S)-5-(5-Acetamidoimidazol-1-yl)-2-(cyclopropanecarbonylamino)-N-(cyclopropylmethyl)-4,5,6,7-tetrahydrobenzothiophene-3-carboxamide ;
(5S)-2-(cyclopropanecarbonylamino)-N-(cyclopropylmethyl)-5-[3-[(5-methyl-3-pyridyl)amino]-1,2,4-triazol-4-yl]-4,5,6,7-tetrahydrobenzothiophene-3-carboxamide ;
(5S)-2-(Cyclopropanecarbonylamino)-N-(cyclopropylmethyl)-5-[3-(4-pyridylamino)-1,2,4-triazol-4-yl]-4,5,6,7-tetrahydrobenzothiophene-3-carboxamide ;
5-[[N'-Cyano-N-(3,4-dimethoxyphenyl)carbamimidoyl]amino]-2-(cyclopropanecarbonylamino)-N-(cyclopropylmethyl)-4,5,6,7-tetrahydrobenzothiophene-3-carboxamide ;
Ethyl 5-amino-1-[(5S)-2-(cyclopropanecarbonylamino)-3-(cyclopropylmethylcarbamoyl)-4,5,6,7-tetrahydrobenzothiophen-5-yl]imidazole-4-carboxylate ;
(5S)-2-(Cyclopropanecarbonylamino)-N-(cyclopropylmethyl)-5-[3-(2-morpholinoethylamino)-1,2,4-triazol-4-yl]-4,5,6,7-tetrahydrobenzothiophene-3-carboxamide ;
2-(Cyclopropanecarbonylamino)-N-(cyclopropylmethyl)-5-[(5-oxo-4H-1,2,4-oxadiazol-3-yl)amino]-4,5,6,7-tetrahydrobenzothiophene-3-carboxamide ;
2-(Cyclopropanecarbonylamino)-N-(cyclopropylmethyl)-5-[[2-(ethylamino)-3,4-dioxo-cyclobuten-1-yl]amino]-4,5,6,7-tetrahydrobenzothiophene-3-carboxamide ;
5-[[N'-cyano-N-(2,2,2-trifluoroethyl)carbamimidoyl]amino]-2-(cyclopropanecarbonylamino)-N-(cyclopropylmethyl)-4,5,6,7-tetrahydrobenzothiophene-3-carboxamide ;
(5S)-2-(cyclopropanecarbonylamino)-N-(cyclopropylmethyl)-5-[3-(ethylamino)-1,2,4-triazol-4-yl]-4,5,6,7-tetrahydrobenzothiophene-3-carboxamide ;
2-(cyclopropanecarbonylamino)-N-(cyclopropylmethyl)-5-[(N-ethyl-N'-nitro-carbamimidoyl)amino]-4,5,6,7-tetrahydrobenzothiophene-3-carboxamide;
(5S)-2-(Cyclopropanecarbonylamino)-N-(cyclopropylmethyl)-5-[3-(3-pyridylamino)-1,2,4-triazol-4-yl]-4,5,6,7-tetrahydrobenzothiophene-3-carboxamide ;
(5S)-2-(Cyclopropanecarbonylamino)-N-(cyclopropylmethyl)-5-[3-[(1-oxidopyridin-1-ium-3-yl)amino]-1,2,4-triazol-4-yl]-4,5,6,7-tetrahydrobenzothiophene-3-carboxamide ;
2-(cyclopropanecarbonylamino)-N-(cyclopropylmethyl)-5-[[N'-nitro-N-(p-tolyl)carbamimidoyl]amino]-4,5,6,7-tetrahydrobenzothiophene-3-carboxamide ;
(5S)-2-(Cyclopropanecarbonylamino)-N-(cyclopropylmethyl)-5-[2-(3-pyridylamino)imidazol-1-yl]-4,5,6,7-tetrahydrobenzothiophene-3-carboxamide ;
(5S)-2-(Cyclopropanecarbonylamino)-N-(cyclopropylmethyl)-5-[3-(cyclopropylmethylamino)-1,2,4-triazol-4-yl]-4,5,6,7-tetrahydrobenzothiophene-3-carboxamide ;
(5S)-2-(Cyclopropanecarbonylamino)-N-(cyclopropylmethyl)-5-[(2,5-dimethylpyrazol-3-yl)carbamothioylamino]-4,5,6,7-tetrahydrobenzothiophene-3-carboxamide ;
(5S)-2-(Cyclopropanecarbonylamino)-N-(cyclopropylmethyl)-5-[3-(pyrimidin-5-ylamino)-1,2,4-triazol-4-yl]-4,5,6,7-tetrahydrobenzothiophene-3-carboxamide ;
(5S)-2-(Cyclopropanecarbonylamino)-N-(cyclopropylmethyl)-5-[2-(cyclopropylmethylamino)imidazol-1-yl]-4,5,6,7-tetrahydrobenzothiophene-3-carboxamide ;
2-(Cyclopropanecarbonylamino)-N-(cyclopropylmethyl)-5-[[4-(ethylamino)-1,1-dioxo-1,2,5-thiadiazol-3-yl]amino]-4,5,6,7-tetrahydrobenzothiophene-3-carboxamide ;
2-(cyclopropanecarbonylamino)-N-(cyclopropylmethyl)-5-[[1-(4-methylanilino)-2-nitro-vinyl]amino]-4,5,6,7-tetrahydrobenzothiophene-3-carboxamide ;
(5S)-5-[(N'-cyano-N-ethyl-carbamimidoyl)-methyl-amino]-2-(cyclopropanecarbonylamino)-N-(cyclopropylmethyl)-4,5,6,7-tetrahydrobenzothiophene-3-carboxamide ;
2-(Cyclopropanecarbonylamino)-N-(cyclopropylmethyl)-5-[[2-(4-methylanilino)-3,4-dioxo-cyclobuten-1-yl]amino]-4,5,6,7-tetrahydrobenzothiophene-3-carboxamide ;
(5S)-2-(Cyclopropanecarbonylamino)-N-(cyclopropylmethyl)-5-[3-(2-pyridylamino)-1,2,4-triazol-4-yl]-4,5,6,7-tetrahydrobenzothiophene-3-carboxamide ;
(5S)-5-(2-Acetamidoimidazol-1-yl)-2-(cyclopropanecarbonylamino)-N-(cyclopropylmethyl)-4,5,6,7-tetrahydrobenzothiophene-3-carboxamide ;
*tert-*Butyl N-[3-[2-(cyclopropanecarbonylamino)-3-(cyclopropylmethylcarbamoyl)-4,5,6,7-tetrahydrobenzothiophen-5-yl]triazol-4-yl]carbamate ;
2-(Cyclopropanecarbonylamino)-N-(cyclopropylmethyl)-5-[5-(3-pyridylamino)triazol-1-yl]-4,5,6,7-tetrahydrobenzothiophene-3-carboxamide ;
2-(Cyclopropanecarbonylamino)-N-(cyclopropylmethyl)-5-(ethylcarbamothioylamino)-4,5,6,7-tetrahydrobenzothiophene-3-carboxamide ;
(5S)-2-(cyclopropanecarbonylamino)-N-(cyclopropylmethyl)-5-[3-[[(3S)-tetrahydrofuran-3-yl]amino]-1,2,4-triazol-4-yl]-4,5,6,7-tetrahydrobenzothiophene-3-carboxamide;
5-[[N'-Cyano-N-ethyl-carbamimidoyl]amino]-2-(cyclopropanecarbonylamino)-N-(cyclopropylmethyl)-4,5,6,7-tetrahydrobenzothiophene-3-carboxamide;
(5S)-2-(Cyclopropanecarbonylamino)-N-(cyclopropylmethyl)-5-[[(E)-N-ethyl-N'-nitro-carbamimidoyl]amino]-4,5,6,7-tetrahydrobenzothiophene-3-carboxamide ;
(5R)-2-(Cyclopropanecarbonylamino)-N-(cyclopropylmethyl)-5-[[(E)-N-ethyl-N'-nitro-carbamimidoyl]amino]-4,5,6,7-tetrahydrobenzothiophene-3-carboxamide ;
(5S)-2-(Cyclopropanecarbonylamino)-N-(cyclopropylmethyl)-5-[2-(ethylamino)imidazol-1-yl]-4,5,6,7-tetrahydrobenzothiophene-3-carboxamide ;
(5S)-2-(Cyclopropanecarbonylamino)-N-(cyclopropylmethyl)-5-[3-[(3,5-dimethylisoxazol-4-yl)amino]-1,2,4-triazol-4-yl]-4,5,6,7-tetrahydrobenzothiophene-3-carboxamide ;
ethyl N-[[[2-(cyclopropanecarbonylamino)-3-(cyclopropylmethylcarbamoyl)-4,5,6,7-tetrahydrobenzothiophen-5-yl]amino]-(4-methylanilino)methylene]carbamate ;
(5S)-2-(Cyclopropanecarbonylamino)-N-(cyclopropylmethyl)-5-[2-[(2-methyl-3-pyridyl)amino]imidazol-1-yl]-4,5,6,7-tetrahydrobenzothiophene-3-carboxamide ;
2-(cyclopropanecarbonylamino)-N-(cyclopropylmethyl)-5-[[1-(ethylamino)-2-nitro-vinyl]amino]-4,5,6,7-tetrahydrobenzothiophene-3-carboxamide ;
2-(Cyclopropanecarbonylamino)-N-(cyclopropylmethyl)-5-[(4-methyl-6-oxo-1H-pyrimidin-2-yl)amino]-4,5,6,7-tetrahydrobenzothiophene-3-carboxamide ;
5-amino-1-[(5S)-2-cyclopropaneamido-3-[(cyclopropylmethyl)carbamoyl]-4,5,6,7-tetrahydro-1-benzothiophen-5-yl]-N-methyl-1H-imidazole-4-carboxamide ;
5-amino-1-[(5S)-2-cyclopropaneamido-3-[(cyclopropylmethyl)carbamoyl]-4,5,6,7-tetrahydro-1-benzothiophen-5-yl]-1H-imidazole-4-carboxamide;
5-(2-Anilinoimidazol-1-yl)-2-(cyclopropanecarbonylamino)-N-(cyclopropylmethyl)-4,5,6,7-tetrahydrobenzothiophene-3-carboxamide ;
(5S)-5-[[N'-cyano-N-ethyl-carbamimidoyl]amino]-2-(cyclopropanecarbonylamino)-N-(cyclopropylmethyl)-4,5,6,7-tetrahydrobenzothiophene-3-carboxamide ;
2-(Cyclopropanecarbonylamino)-N-(cyclopropylmethyl)-5-(ethylcarbamoylamino)-4,5,6,7-tetrahydrobenzothiophene-3-carboxamide;
2-(Cyclopropanecarbonylamino)-N-(cyclopropylmethyl)-5-[(4-oxo-1H-pyridin-2-yl)amino]-4,5,6,7-tetrahydrobenzothiophene-3-carboxamide;
(5S)-2-(cyclopropanecarbonylamino)-N-(cyclopropylmethyl)-5-[[6-(5-methyl-1,3,4-oxadiazol-2-yl)-3-pyridyl]amino]-4,5,6,7-tetrahydrobenzothiophene-3-carboxamide;
2-(Cyclopropanecarbonylamino)-N-(cyclopropylmethyl)-5-[5-(ethylamino)tetrazol-1-yl]-4,5,6,7-tetrahydrobenzothiophene-3-carboxamide;
2-(Cyclopropanecarbonylamino)-N-(cyclopropylmethyl)-5-(1H-1,2,4-triazol-3-ylamino)-4,5,6,7-tetrahydrobenzothiophene-3-carboxamide;
(5R)-2-(Cyclopropanecarbonylamino)-N-(cyclopropylmethyl)-5-[3-[(2,5-dimethylpyrazol-3-yl)amino]-1,2,4-triazol-4-yl]-4,5,6,7-tetrahydrobenzothiophene-3-carboxamide;
(5S)-2-(Cyclopropanecarbonylamino)-N-(cyclopropylmethyl)-5-[[4-(1-methylpyrazol-4-yl)-1,2,4-triazol-3-yl]amino]-4,5,6,7-tetrahydrobenzothiophene-3-carboxamide;
ethyl 1-[(5S)-2-cyclopropaneamido-3-[(cyclopropylmethyl)carbamoyl]-4,5,6,7-tetrahydro-1-benzothiophen-5-yl]-1H-imidazole-4-carboxylate;
2-(Cyclopropanecarbonylamino)-N-(cyclopropylmethyl)-5-[[4-(4-fluorophenyl)-1,2,4-triazol-3-yl]amino]-4,5,6,7-tetrahydrobenzothiophene-3-carboxamide ;
(5S)-2-(Cyclopropanecarbonylamino)-N-(cyclopropylmethyl)-5-(1H-imidazol-2-ylamino)-4,5,6,7-tetrahydrobenzothiophene-3-carboxamide ;
2-(Cyclopropanecarbonylamino)-N-(cyclopropylmethyl)-5-[[N'-methoxy-N-(p-tolyl)carbamimidoyl]amino]-4,5,6,7-tetrahydrobenzothiophene-3-carboxamide;
2-(Cyclopropanecarbonylamino)-N-(cyclopropylmethyl)-5-[[4-(4-methylanilino)-1,1-dioxo-1,2,5-thiadiazol-3-yl]amino]-4,5,6,7-tetrahydrobenzothiophene-3-carboxamide;
(5S)-2-(Cyclopropanecarbonylamino)-N-(cyclopropylmethyl)-5-[[1-(3-pyridyl)imidazol-2-yl]Jamino]-4,5,6,7-tetrahydrobenzothiophene-3-carboxamide ;
5-(5-Aminotriazol-1-yl)-2-(cyclopropanecarbonylamino)-N-(cyclopropylmethyl)-4,5,6,7-tetrahydrobenzothiophene-3-carboxamide ;
2-(Cyclopropanecarbonylamino)-N-(cyclopropylmethyl)-5-[(2-methoxyphenyl)carbamoylamino]-4,5,6,7-tetrahydrobenzothiophene-3-carboxamide ;
N-[2-(Cyclopropanecarbonylamino)-3-(cyclopropylmethylcarbamoyl)-4,5,6,7-tetrahydrobenzothiophen-5-yl]pyridine-4-carboxamide ;
5-(Benzylamino)-2-(cyclopropanecarbonylamino)-N-(cyclopropylmethyl)-4,5,6,7-tetrahydrobenzothiophene-3-carboxamide ;
5-(1H-Benzimidazol-2-ylamino)-2-(cyclopropanecarbonylamino)-N-(cyclopropylmethyl)-4,5,6,7-tetrahydrobenzothiophene-3-carboxamide ;
2-(Cyclopropanecarbonylamino)-N-(cyclopropylmethyl)-5-[[N'-hydroxycarbamimidoyl]amino]-4,5,6,7-tetrahydrobenzothiophene-3-carboxamide ;
2-(Cyclopropanecarbonylamino)-N-(cyclopropylmethyl)-5-[[N'-(1-methylpyrazol-3-yl)-N-(p-tolyl)carbamimidoyl]amino]-4,5,6,7-tetrahydrobenzothiophene-3-carboxamide;formic acid ;
(5S)-2-(Cyclopropanecarbonylamino)-N-(cyclopropylmethyl)-5-[3-[(3,5-dimethylpyrazin-2-yl)amino]-1,2,4-triazol-4-yl]-4,5,6,7-tetrahydrobenzothiophene-3-carboxamide ;
2-(Cyclopropanecarbonylamino)-N-(cyclopropylmethyl)-5-[3-(ethylamino)-5-(methoxymethyl)-1,2,4-triazol-4-yl]-4,5,6,7-tetrahydrobenzothiophene-3-carboxamide ;
2-(Cyclopropanecarbonylamino)-N-(cyclopropylmethyl)-5-[[5-(3-pyridyl)-1H-1,2,4-triazol-3-yl]amino]-4,5,6,7-tetrahydrobenzothiophene-3-carboxamide ;
p-Tolyl N-[2-(cyclopropanecarbonylamino)-3-(cyclopropylmethylcarbamoyl)-4,5,6,7-tetrahydrobenzothiophen-5-yl]carbamate ;
2-(Cyclopropanecarbonylamino)-N-(cyclopropylmethyl)-5-(quinazolin-2-ylamino)-4,5,6,7-tetrahydrobenzothiophene-3-carboxamide ;
5-(1,3-Benzoxazol-2-ylamino)-2-(cyclopropanecarbonylamino)-N-(cyclopropylmethyl)-4,5,6,7-tetrahydrobenzothiophene-3-carboxamide ;
2-(Cyclopropanecarbonylamino)-N-(cyclopropylmethyl)-5-[3-(ethylamino)-5-(hydroxymethyl)-1,2,4-triazol-4-yl]-4,5,6,7-tetrahydrobenzothiophene-3-carboxamide ;
(5S)-5-(2-Aminoimidazol-1-yl)-2-(cyclopropanecarbonylamino)-N-(cyclopropylmethyl)-4,5,6,7-tetrahydrobenzothiophene-3-carboxamide ;
5-(3-Amino-5-oxo-1,2,4-oxadiazol-4-yl)-2-(cyclopropanecarbonylamino)-N-(cyclopropylmethyl)-4,5,6,7-tetrahydrobenzothiophene-3-carboxamide ;
2-(Cyclopropanecarbonylamino)-N-(cyclopropylmethyl)-5-[3-(ethylamino)-5-methyl-1,2,4-triazol-4-yl]-4,5,6,7-tetrahydrobenzothiophene-3-carboxamide ;
(5R)-2-(Cyclopropanecarbonylamino)-N-(cyclopropylmethyl)-5-[(2-ethyl-3,4-dioxo-cyclobuten-1-yl)amino]-4,5,6,7-tetrahydrobenzothiophene-3-carboxamide ;
(5S)-2-(Cyclopropanecarbonylamino)-N-(cyclopropylmethyl)-5-[(2-ethyl-3,4-dioxo-cyclobuten-1-yl)amino]-4,5,6,7-tetrahydrobenzothiophene-3-carboxamide ;
2-(Cyclopropanecarbonylamino)-N-(cyclopropylmethyl)-5-(pyrimidin-2-ylamino)-4,5,6,7-tetrahydrobenzothiophene-3-carboxamide ;
2-(Cyclopropanecarbonylamino)-N-(cyclopropylmethyl)-5-(1,3,4-oxadiazol-2-ylamino)-4,5,6,7-tetrahydrobenzothiophene-3-carboxamide ;
2-(Cyclopropanecarbonylamino)-N-(cyclopropylmethyl)-5-[[5-oxo-1-(p-tolyl)-4H-imidazol-2-yl]amino]-4,5,6,7-tetrahydrobenzothiophene-3-carboxamide ;
2-(Cyclopropanecarbonylamino)-N-(cyclopropylmethyl)-5-[[2-[ethyl(methyl)amino]-3,4-dioxo-cyclobuten-1-yl]amino]-4,5,6,7-tetrahydrobenzothiophene-3-carboxamide ;
2-(Cyclopropanecarbonylamino)-N-(cyclopropylmethyl)-5-[(methylsulfonimidoyl)amino]-4,5,6,7-tetrahydrobenzothiophene-3-carboxamide ;
5-[[N-Cyano-C-methyl-carbonimidoyl]amino]-2-(cyclopropanecarbonylamino)-N-(cyclopropylmethyl)-4,5,6,7-tetrahydrobenzothiophene-3-carboxamide ;
2-(Cyclopropanecarbonylamino)-N-(cyclopropylmethyl)-5-(tetrazol-1-yl)-4,5,6,7-tetrahydrobenzothiophene-3-carboxamide ;
2-(Cyclopropanecarbonylamino)-N-(cyclopropylmethyl)-5-(methanesulfinamido)-4,5,6,7-tetrahydrobenzothiophene-3-carboxamide ;
2-(Cyclopropanecarbonylamino)-N-(cyclopropylmethyl)-5-morpholino-4,5,6,7-tetrahydrobenzothiophene-3-carboxamide ;
2-(Cyclopropanecarbonylamino)-N-(cyclopropylmethyl)-5-[3-(ethylamino)-5-(trifluoromethyl)-1,2,4-triazol-4-yl]-4,5,6,7-tetrahydrobenzothiophene-3-carboxamide;
2-(Cyclopropanecarbonylamino)-N-(cyclopropylmethyl)-5-[(4-oxo-1H-pyrimidin-2-yl)amino]-4,5,6,7-tetrahydrobenzothiophene-3-carboxamide ;
2-(Cyclopropanecarbonylamino)-N-(cyclopropylmethyl)-5-(isopropylsulfamoylamino)-4,5,6,7-tetrahydrobenzothiophene-3-carboxamide;
5-[[*N*'-Cyano-*N*-morpholino-carbamimidoyl]amino]-2-(cyclopropanecarbonylamino)-*N-*(cyclopropylmethyl)-4,5,6,7-tetrahydrobenzothiophene-3-carboxamide ;
5-Acetamido-2-(cyclopropanecarbonylamino)-N-(cyclopropylmethyl)-4,5,6,7-tetrahydrobenzothiophene-3-carboxamide;
2-(Cyclopropanecarbonylamino)-N-(cyclopropylmethyl)-5-(methanesulfonamido)-4,5,6,7-tetrahydrobenzothiophene-3-carboxamide ;
5-[[N'-Cyano-N-methoxy-carbamimidoyl]amino]-2-(cyclopropanecarbonylamino)-N-(cyclopropylmethyl)-4,5,6,7-tetrahydrobenzothiophene-3-carboxamide;
2-(Cyclopropanecarbonylamino)-N-(cyclopropylmethyl)-5-[[4-oxo-6-(trifluoromethyl)-1H-pyrimidin-2-yl]amino]-4,5,6,7-tetrahydrobenzothiophene-3-carboxamide;
(5S)-2-(cyclopropanecarbonylamino)-N-(cyclopropylmethyl)-5-[3-(2-methylpropanoylamino)-1,2,4-triazol-4-yl]-4,5,6,7-tetrahydrobenzothiophene-3-carboxamide ;
(5S)-2-(cyclopropanecarbonylamino)-N-(cyclopropylmethyl)-5-[3-[(2,5-dimethylpyrazol-3-yl)-methyl-amino]-1,2,4-triazol-4-yl]-4,5,6,7-tetrahydrobenzothiophene-3-carboxamide ;
(5S)-5-[5-amino-4-(5-methyl-1,3,4-oxadiazol-2-yl)imidazol-1-yl]-2-(cyclopropanecarbonylamino)-N-(cyclopropylmethyl)-4,5,6,7-tetrahydrobenzothiophene-3-carboxamide;
(5S)-2-(cyclopropanecarbonylamino)-N-(cyclopropylmethyl)-5-[3-[(3-methyl-1H-pyrazol-5-yl)amino]-1,2,4-triazol-4-yl]-4,5,6,7-tetrahydrobenzothiophene-3-carboxamide ;
(5S)-2-(cyclopropanecarbonylamino)-N-(cyclopropylmethyl)-5-[3-[(1,3-dimethylpyrazol-4-yl)amino]-1,2,4-triazol-4-yl]-4,5,6,7-tetrahydrobenzothiophene-3-carboxamide ;
(5S)-2-(cyclopropanecarbonylamino)-N-(cyclopropylmethyl)-5-[3-[(5-cyclopropyl-2-methyl-pyrazol-3-yl)amino]-1,2,4-triazol-4-yl]-4,5,6,7-tetrahydrobenzothiophene-3-carboxamide ;
(5S)-2-(cyclopropanecarbonylamino)-N-(cyclopropylmethyl)-5-[3-[(5-isopropyl-2-methyl-pyrazol-3-yl)amino]-1,2,4-triazol-4-yl]-4,5,6,7-tetrahydrobenzothiophene-3-carboxamide ;
(5S)-2-(cyclopropanecarbonylamino)-N-(cyclopropylmethyl)-5-[3-[[2-methyl-5-(trifluoromethyl)pyrazol-3-yl]amino]-1,2,4-triazol-4-yl]-4,5,6,7-tetrahydrobenzothiophene-3-carboxamide ;
(5S)-2-(cyclopropanecarbonylamino)-N-(cyclopropylmethyl)-5-[3-[(2-isopropyl-5-methyl-pyrazol-3-yl)amino]-1,2,4-triazol-4-yl]-4,5,6,7-tetrahydrobenzothiophene-3-carboxamide ;
(5S)-2-(cyclopropanecarbonylamino)-N-(cyclopropylmethyl)-5-[3-[(5-methyl-2-tetrahydrofuran-3-yl-pyrazol-3-yl)amino]-1,2,4-triazol-4-yl]-4,5,6,7-tetrahydrobenzothiophene-3-carboxamide ;
(5S)-2-(cyclopropanecarbonylamino)-N-(cyclopropylmethyl)-5-[3-[(2-methylpyrazol-3-yl)amino]-1,2,4-triazol-4-yl]-4,5,6,7-tetrahydrobenzothiophene-3-carboxamide ;
(5S)-2-(cyclopropanecarbonylamino)-N-(cyclopropylmethyl)-5-[3-[(2-ethyl-5-methyl-pyrazol-3-yl)amino]-1,2,4-triazol-4-yl]-4,5,6,7-tetrahydrobenzothiophene-3-carboxamide ;
(5S)-2-(cyclopropanecarbonylamino)-N-(cyclopropylmethyl)-5-[3-[(2-cyclopropyl-5-methyl-pyrazol-3-yl)amino]-1,2,4-triazol-4-yl]-4,5,6,7-tetrahydrobenzothiophene-3-carboxamide ;
(5S)-2-(cyclopropanecarbonylamino)-N-(cyclopropylmethyl)-5-[3-[(3-methoxy-1-methyl-pyrazol-4-yl)amino]-1,2,4-triazol-4-yl]-4,5,6,7-tetrahydrobenzothiophene-3-carboxamide ;
(5S)-2-(cyclopropanecarbonylamino)-N-(cyclopropylmethyl)-5-[3-[(5-ethyl-2-methyl-pyrazol-3-yl)amino]-1,2,4-triazol-4-yl]-4,5,6,7-tetrahydrobenzothiophene-3-carboxamide ;
(5S)-2-(Cyclopropanecarbonylamino)-N-(cyclopropylmethyl)-5-[3-[[5-(difluoromethyl)-2-methyl-pyrazol-3-yl]amino]-1,2,4-triazol-4-yl]-4,5,6,7-tetrahydrobenzothiophene-3-carboxamide ;
(5S)-2-(Cyclopropanecarbonylamino)-N-(cyclopropylmethyl)-5-[3-[(1,4-dimethylpyrazol-3-yl)amino]-1,2,4-triazol-4-yl]-4,5,6,7-tetrahydrobenzothiophene-3-carboxamide ;
Methyl 5-[[4-[(55)-2-(cyclopropanecarbonylamino)-3-(cyclopropylmethylcarbamoyl)-4,5,6,7-tetrahydrobenzothiophen-5-yl]-1,2,4-triazol-3-yl]amino]-1-methyl-pyrazole-3-carboxylate ;
(5S)-2-(Cyclopropanecarbonylamino)-N-(cyclopropylmethyl)-5-[2-[[2-methyl-5-(trifluoromethyl)pyrazol-3-yl]amino]imidazol-1-yl]-4,5,6,7-tetrahydrobenzothiophene-3-carboxamide ;
5-[[4-[(5S)-2-(Cyclopropanecarbonylamino)-3-(cyclopropylmethylcarbamoyl)-4,5,6,7-tetrahydrobenzothiophen-5-yl]-1,2,4-triazol-3-yl]amino]-1-methyl-pyrazole-3-carboxylic acid ;
(5S)-2-(cyclopropanecarbonylamino)-N-(cyclopropylmethyl)-5-[3-[[(3S*)-1,1-dioxothiolan-3-yl]amino]-1,2,4-triazol-4-yl]-4,5,6,7-tetrahydrobenzothiophene-3-carboxamide [*or R];
(5S)-2-cyclopropaneamido-N-(cyclopropylmethyl)-5-[3-(13-[imino(methyl)oxo-lambda6-sulfanyl]-2-methylphenyl}amino)-4H-1,2,4-triazol-4-yl]-4,5,6,7-tetrahydro-1-benzothiophene-3-carboxamide;
(5S)-2-(cyclopropanecarbonylamino)-N-(cyclopropylmethyl)-5-[3-[2-methyl-3-[(S)-methylsulfonimidoyl]anilino]-1,2,4-triazol-4-yl]-4,5,6,7-tetrahydrobenzothiophene-3-carboxamide
(5S)-2-(cyclopropanecarbonylamino)-N-(cyclopropylmethyl)-5-[3-[2-methyl-3-[(R)-methylsulfonimidoyl]anilino]-1,2,4-triazol-4-yl]-4,5,6,7-tetrahydrobenzothiophene-3-carboxamide;
(5S)-5-{3-[(1-acetyl-3-methylpyrrolidin-3-yl)amino]-4H-1,2,4-triazol-4-yl}-2-cyclopropaneamido-N-(cyclopropylmethyl)-4,5,6,7-tetrahydro-1-benzothiophene-3-carboxamide;
(5S)-5-[3-[[(3S*)-1-acetyl-3-methyl-pyrrolidin-3-yl]amino]-1,2,4-triazol-4-yl]-2-(cyclopropanecarbonylamino)-N-(cyclopropylmethyl)-4,5,6,7-tetrahydrobenzothiophene-3-carboxamide [* or R];
(5S)-2-cyclopropaneamido-N-(cyclopropylmethyl)-5-(3-{[(3S)-1-(6-methylpyridazin-3-yl)pyrrolidin-3-yl]amino}-4H-1,2,4-triazol-4-yl)-4,5,6,7-tetrahydro-1-benzothiophene-3-carboxamide;
(5S)-2-(cyclopropanecarbonylamino)-N-(cyclopropylmethyl)-5-[3-[[(3S)-1-pyrazin-2-ylpyrrolidin-3-yl]amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[(4-methyl-1-methylsulfonyl-pyrrolidin-3-yl)amino]-1,2,4-triazol-4-yl]-4,5,6,7-tetrahydrobenzothiophene-3-carboxamide;
(5S)-2-cyclopropaneamido-N-(cyclopropylmethyl)-5-{3-[(1-oxo-2,3-dihydro-1H-isoindol-4-yl)amino]-4H-1,2,4-triazol-4-yl}-4,5,6,7-tetrahydro-1-benzothiophene-3-carboxamide;
(5S)-2-(cyclopropanecarbonylamino)-N-(cyclopropylmethyl)-5-[3-[[(3S)-1-(6-fluoropyridazin-3-yl)pyrrolidin-3-yl]amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[[(3S)-1-pyrimidin-2-ylpyrrolidin-3-yl]amino]-1,2,4-triazol-4-yl]-4,5,6,7-tetrahydro-1-benzothiophene-3-carboxamide;
2-methoxyethyl 5-amino-1-[(5S)-2-(cyclopropanecarbonylamino)-3-(cyclopropylmethylcarbamoyl)-4,5,6,7-tetrahydro-1-benzothiophen-5-yl]imidazole-4-carboxylate;
2-morpholin-4-ylethyl 5-amino-1-[(55)-2-(cyclopropanecarbonylamino)-3-(cyclopropylmethylcarbamoyl)-4,5,6,7-tetrahydro-1-benzothiophen-5-yl]imidazole-4-carboxylate;
(5S)-2-(cyclopropanecarbonylamino)-N-(cyclopropylmethyl)-5-[3-(3-methylsulfanylanilino)-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-(3-methylsulfonylanilino)-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-(3-methylsulfinylanilino)-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[3-(methylsulfonimidoyl)anilino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[3-[(S)-methylsulfonimidoyl]anilino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[3-[(R)-methylsulfonimidoyl]anilino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-(8-methoxy-4-oxo-5,10-dihydroimidazo[4,5-c][1,5]benzodiazepin-1-yl)-4,5,6,7-tetrahydrobenzothiophene-3-carboxamide;
(5S)-2-(cyclopropanecarbonylamino)-N-(cyclopropylmethyl)-5-(8-methoxy-5-methyl-4-oxo-10H-imidazo[4,5-c][1,5]benzodiazepin-1-yl)-4,5,6,7-tetrahydrobenzothiophene-3-carboxamide;
(5S)-5-[5-amino-4-[(3R)-3-hydroxypyrrolidine-1-carbonyl]imidazol-1-yl]-2-(cyclopropanecarbonylamino)-N-(cyclopropylmethyl)-4,5,6,7-tetrahydrobenzothiophene-3-carboxamide;
(5S)-5-[5-amino-4-[(2S)-2-methylpyrrolidine-1-carbonyl]imidazol-1-yl]-2-(cyclopropanecarbonylamino)-N-(cyclopropylmethyl)-4,5,6,7-tetrahydrobenzothiophene-3-carboxamide;
Ethyl 1-[(5S)-2-(cyclopropanecarbonylamino)-3-(cyclopropylmethylcarbamoyl)-4,5,6,7-tetrahydrobenz othiophen-5-yl]-5-[(2,5-dimethyl-4-nitro-pyrazol-3-yl)amino]imidazole-4-carboxylate;
(5S)-2-(cyclopropanecarbonylamino)-N-(cyclopropylmethyl)-5-[3-(2-methyl-3-methylsulfinyl-anilino)-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-(2-methyl-3-methylsulfonyl-anilino)-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[(2-methyl-5-oxazo1-2-yl-pyrazol-3-yl)amino]-1,2,4-triazol-4-yl]-4,5,6,7-tetrahydrobenzothiophene-3-carboxamide;
(5S)-5-[3-[[(3S)-1-acetylpyrrolidin-3-yl]amino]-1,2,4-triazol-4-yl]-2-(cyclopropanecarbonylamino)-N-(cyclopropylmethyl)-4,5,6,7-tetrahydrobenzothiophene-3-carboxamide;
(5S)-2-(cyclopropanecarbonylamino)-N-(cyclopropylmethyl)-5-[3-[[(3S)-1-methylsulfonylpyrrolidin-3-yl]amino]-1,2,4-triazol-4-yl]-4,5,6,7-tetrahydrobenzothiophene-3-carboxamide ;
(5S)-2-(cyclopropanecarbonylamino)-N-(cyclopropylmethyl)-5-[3-[[(3R)-1-methylsulfonylpyrrolidin-3-yl]amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[(2-methylcyclohexyl)amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[(5-oxopyrrolidin-3-yljamino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[[(3R*)-5-oxopyrrolidin-3-yl]amino]-1,2,4-triazol-4-yl]-4,5,6,7-tetrahydrobenzothiophene-3-carboxamide (*or S);
5S)-2-(cyclopropanecarbonylamino)-N-(cyclopropylmethyl)-5-[3-[(2-oxo-4-piperidyl)amino]-1,2,4-triazol-4-yl]-4,5,6,7-tetrahydrobenzothiophene-3-carboxamide;
(5S)-5-[3-[(1-acetyl-2-methyl-pyrrolidin-3-yl)amino]-1,2,4-triazol-4-yl]-2-(cyclopropanecarbonylamino)-N-(cyclopropylmethyl)-4,5,6,7-tetrahydrobenzothiophene-3-carboxamide;
(5S)-2-(cyclopropanecarbonylamino)-N-(cyclopropylmethyl)-5-[3-[(2-methyl-1-methylsulfonyl-pyrrolidin-3-yl)amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[[3-(6-methyl-3-pyridyl)cyclobutyl]amino]-1,2,4-triazol-4-yl]-4,5,6,7-tetrahydrobenzothiophene-3-carboxamide
(5S)-2-(cyclopropanecarbonylamino)-N-(cyclopropylmethyl)-5-[3-[[3-(6-methyl-3-pyridyl)cyclobutyl]amino]-1,2,4-triazol-4-yl]-4,5,6,7-tetrahydrobenzothiophene-3-carboxamide;
(5S)-5-[3-[[(3S*)-1-benzoyl-4,4-difluoro-pyrrolidin-3-yl]amino]-1,2,4-triazol-4-yl]-2-(cyclopropanecarbonylamino)-N-(cyclopropylmethyl)-4,5,6,7-tetrahydrobenzothiophene-3-carboxamide [* or R];
(5S)-5-[3-[(1-acetyl-4-piperidyl)amino]-1,2,4-triazol-4-yl]-2-(cyclopropanecarbonylamino)-N-(cyclopropylmethyl)-4,5,6,7-tetrahydrobenzothiophene-3-carboxamide ;
Methyl (3S)-3-[[4-[(5S)-2-(cyclopropanecarbonylamino)-3-(cyclopropylmethylcarbamoyl)-4,5,6,7-tetrahydrobenzothiophen-5-yl]-1,2,4-triazol-3-yl]amino]pyrrolidine-1-carboxylate;
(3S)-3-[[4-[(5S)-2-(cyclopropanecarbonylamino)-3-(cyclopropylmethylcarbamoyl)-4,5,6,7-tetrahydrobenzothiophen-5-yl]-1,2,4-triazol-3-yl]amino]-N-ethyl-pyrrolidine-1-carboxamide ;
(5S)-2-cyclopropaneamido-N-(cyclopropylmethyl)-5-(3-{[2-(pyrimidin-2-yl)-2-azaspiro[4.4]nonan-7-yl]amino}-4H-1,2,4-triazol-4-yl)-4,5,6,7-tetrahydro-1-benzothiophene-3-carboxamide;
(5S)-2-(cyclopropanecarbonylamino)-N-(cyclopropylmethyl)-5-[3-[[2-methyl-5-(trifluoromethoxy)pyrazol-3-yl]amino]-1,2,4-triazol-4-yl]-4,5,6,7-tetrahydrobenzothiophene-3-carboxamide;
(5S)-5-[3-[[5-[chloro(difluoro)methoxy]-2-methyl-pyrazol-3-yl]amino]-1,2,4-triazol-4-yl]-2-(cyclopropanecarbonylamino)-N-(cyclopropylmethyl)-4,5,6,7-tetrahydrobenzothiophene-3-carboxamide;
(5S)-2-cyclopropaneamido-N-(cyclopropylmethyl)-5-(3-{[1-methyl-3-(5-methyl-1,3,4-oxadiazol-2-yl)-1H-pyrazol-5-yl]amino}-4H-1,2,4-triazol-4-yl)-4,5,6,7-tetrahydro-1-benzothiophene-3-carboxamide;
(5S)-2-(cyclopropanecarbonylamino)-N-(cyclopropylmethyl)-5-[3-[(5-methoxy-3-methyl-pyrazin-2-yl)amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[(5-methylthiazolo[5,4-b]pyridin-2-yl)amino]-4,5,6,7-tetrahydrobenzothiophene-3-carboxamide;
(5S)-5-[3-[(5-cyano-2-methyl-3-pyridyl)amino]-1,2,4-triazol-4-yl]-2-(cyclopropanecarbonylamino)-N-(cyclopropylmethyl)-4,5,6,7-tetrahydrobenzothiophene-3-carboxamide;
(5S)-5-[3-[(6-cyano-2-methyl-3-pyridyl)amino]-1,2,4-triazol-4-yl]-2-(cyclopropanecarbonylamino)-N-(cyclopropylmethyl)-4,5,6,7-tetrahydrobenzothiophene-3-carboxamide;
(5S)-2-(cyclopropanecarbonylamino)-N-(cyclopropylmethyl)-5-[3-[(1-methyl-2-oxo-4-pyridyl)amino]-1,2,4-triazol-4-yl]-4,5,6,7-tetrahydrobenzothiophene-3-carboxamide;
(5S)-5-[3-[(6-chloro-2-methyl-3-pyridyl)amino]-1,2,4-triazol-4-yl]-2-(cyclopropanecarbonylamino)-N-(cyclopropylmethyl)-4,5,6,7-tetrahydrobenzothiophene-3-carboxamide;
(5S)-2-(cyclopropanecarbonylamino)-N-(cyclopropylmethyl)-5-[3-[[2-methyl-6-(trifluoromethyl)-3-pyridyl]amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[(3-methylpyridazin-4-yl)amino]-1,2,4-triazol-4-yl]-4,5,6,7-tetrahydrobenzothiophene-3-carboxamide;
(5S)-5-[3-[(5-chloro-2-methyl-pyrazol-3-yl)amino]-1,2,4-triazol-4-yl]-2-(cyclopropanecarbonylamino)-N-(cyclopropylmethyl)-4,5,6,7-tetrahydrobenzothiophene-3-carboxamide;
(5S)-2-(cyclopropanecarbonylamino)-N-(cyclopropylmethyl)-5-[3-[(3-methylisothiazol-4-yl)amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[(3-methylisoxazol-4-yl)amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[(5-methoxy-2-methylpyrazol-3-yl)amino]-1,2,4-triazol-4-yl]-4,5,6,7-tetrahydro-1-benzothiophene-3-carboxamide;
(5S)-2-(cyclopropanecarbonylamino)-N-(cyclopropylmethyl)-5-[3-[[5-(difluoromethoxy)-2-methylpyrazol-3-yl]amino]-1,2,4-triazol-4-yl]-4,5,6,7-tetrahydro-1-benzothiophene-3-carboxamide;
(5S)-2-(cyclopropanecarbonylamino)-N-(cyclopropylmethyl)-5-[3-[[5-(difluoromethyl)-2-(trideuteriomethyl)pyrazol-3-yl]amino]-1,2,4-triazol-4-yl]-4,5,6,7-tetrahydrobenzothiophene-3-carboxamide;
(5S)-5-[3-[(5-Cyano-2-methyl-pyrazol-3-yl)amino]-1,2,4-triazol-4-yl]-2-(cyclopropanecarbonylamino)-N-(cyclopropylmethyl)-4,5,6,7-tetrahydrobenzothiophene-3-carboxamide; (5S)-2-(Cyclopropanecarbonylamino)-N-(cyclopropylmethyl)-5-[3-[(2,2-difluoro-1,3-benzodioxol-5-yl)amino]-1,2,4-triazol-4-yl]-4,5,6,7-tetrahydrobenzothiophene-3-carboxamide;
(4RS,5RS)-2-(cyclopropanecarbonylamino)-N-(cyclopropylmethyl)-4-methyl-5-[3-[[2-methyl-5-(trifluoromethyl)pyrazol-3-yl]amino]-1,2,4-triazol-4-yl]-4,5,6,7-tetrahydrobenzothiophene-3-carboxamide;
(4R*,5R*)-2-(cyclopropanecarbonylamino)-N-(cyclopropylmethyl)-4-methyl-5-[3-[[2-methyl-5-(trifluoromethyl)pyrazol-3-yl]amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[[6-(difluoromethyl)-3-pyridyl]amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[(6-fluoro-2-methyl-3-pyridyl)amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[[6-(trifluoromethyl)-3-pyridyl]amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[(6-methoxy-3-pyridyl)amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[(2-methoxy-4-pyridyl)amino]-1,2,4-triazol-4-yl]-4,5,6,7-tetrahydrobenzothiophene-3-carboxamide;
(5S)-5-[3-[(6-chloro-3-pyridyl)amino]-1,2,4-triazol-4-yl]-2-(cyclopropanecarbonylamino)-N-(cyclopropylmethyl)-4,5,6,7-tetrahydrobenzothiophene-3-carboxamide;
(5S)-5-[3-[(2-chloro-4-pyridyl)amino]-1,2,4-triazol-4-yl]-2-(cyclopropanecarbonylamino)-N-(cyclopropylmethyl)-4,5,6,7-tetrahydrobenzothiophene-3-carboxamide;
(5S)-2-(cyclopropanecarbonylamino)-N-(cyclopropylmethyl)-5-[3-[(6-methoxy-2-methyl-3-pyridyl)amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[(2-fluoro-3-pyridyl)amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[(3-methyltriazol-4-yl)amino]-1,2,4-triazol-4-yl]-4,5,6,7-tetrahydrobenzothiophene-3-carboxamide;
(3R*)-6-(cyclopropanecarbonylamino)-N-(cyclopropylmethyl)-3-[3-[[2-methyl-5-(trifluoromethyl)pyrazol-3-yl]amino]-1,2,4-triazol-4-yl]-3,4-dihydro-2H-thieno[2,3-b]pyran-5-carboxamide (* or S);
or their enantiomers and pharmaceutically acceptable salts thereof.

According to an embodiment, the compounds of the invention are selected from:
(5S)-2-(Cyclopropanecarbonylamino)-N-(cyclopropylmethyl)-5-[3-[(2,5-dimethylpyrazol-3-yl)amino]-1,2,4-triazol-4-yl]-4,5,6,7-tetrahydrobenzothiophene-3-carboxamide;
Ethyl 5-amino-1-[(5S)-2-(cyclopropanecarbonylamino)-3-(cyclopropylmethylcarbamoyl)-4,5,6,7-tetrahydrobenzothiophen-5-yl]imidazole-4-carboxylate;
(5S)-2-(cyclopropanecarbonylamino)-N-(cyclopropylmethyl)-5-[3-[(5-cyclopropyl-2-methyl-pyrazol-3-yl)amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[(5-isopropyl-2-methyl-pyrazol-3-yl)amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[[2-methyl-5-(trifluoromethyl)pyrazol-3-yl]amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[(5-ethyl-2-methyl-pyrazol-3-yl)amino]-1,2,4-triazol-4-yl]-4,5,6,7-tetrahydrobenzothiophene-3-carboxamide;
(5S)-2-(Cyclopropanecarbonylamino)-N-(cyclopropylmethyl)-5-[3-[[5-(difluoromethyl)-2-methyl-pyrazol-3-yl]amino]-1,2,4-triazol-4-yl]-4,5,6,7-tetrahydrobenzothiophene-3-carboxamide;
or their enantiomers and pharmaceutically acceptable salts thereof.

The following paragraphs provide definitions of the various chemical moieties that make up the compounds according to the invention and are intended to apply uniformly throughout the specification and claims unless a broader definition is explicitly set out.

The term "halogen" refers to fluorine, chlorine, bromine or iodine, and preferably fluorine.

The term "heterocyclic ring"refers to a saturated ring, containing at least one heteroatom, such as oxygen, sulphur and nitrogen; and includes a heteoaromatic or aromatic ring fused onto the heterocycle.

The term " alkyl" refers to a straight or branched, saturated, aliphatic hydrocarbon group.

The term "cycloalkyl" refers to monovalent groups derived from a saturated cyclic hydrocarbon and includes fused ring cycloalkyl and a heteroaromatic, heterocyclic or aromatic ring fused onto the carbocyle.

The term "fused ring cycloalkyl" refers to a cycloalkyl with two or more rings wherein the rings have at least one bond and two atoms in common. Examples of fused ring cycloalkyl include

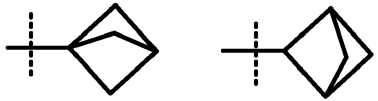

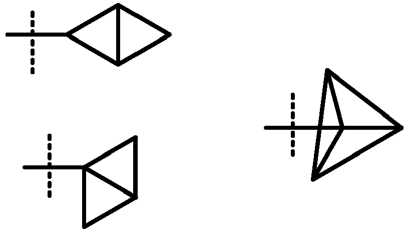

The term "heteroalkyl" refers to an alkyl group, where at least one carbon atom has been replaced with a heteroatom, such as oxygen, sulphur and nitrogen.

The term "heterocycle" refers to a ring structure that has atoms of at least two different elements as members of its ring, so some or all of the atoms in the molecules are joined in rings containing at least one atom of an element other than carbon, so for example oxygen, sulphur and nitrogen; and includes a heteoaromatic or aromatic ring fused onto the heterocycle.

The term "alkylene" refers to a bivalent saturated aliphatic radical derived from an unsaturated aliphatic hydrocarbon.

The term "haloalkyl" refers to an alkyl group as defined above, wherein at least one hydrogen atom is replaced by a halogen atom.

The term "alkenyl" refers to monovalent groups derived from a partially unsaturated, straight or branched carbon chain, such as ethenyl, propenyl, butenyl, pentenyl etc.

The term "cycloalkenyl" refers to monovalent groups derived from a partially unsaturated monocyclic hydrocarbon, such as cyclobutenyl.

The term "heteroalkenyl" refers to monovalent groups derived from a partially unsaturated straight or branched carbon chain containing at least one heteroatom such as oxygen, sulphur and nitrogen.

The term "heterocycloalkenyl" refers to monovalent groups derived from a partially unsaturated monocyclic hydrocarbon containing at least one heteroatom such as oxygen, sulphur and nitrogen.

The term "alkynyl" refers to an unsaturated, straight or branched carbon chain containing at least one carbon-carbon triple bond, such as ethynyl, propinyl and butinyl.

The term "heteroalkynyl" refers to an unsaturated, straight or branched carbon chain containing at least one carbon-carbon triple bond and containing at least one heteroatom such as oxygen, sulphur and nitrogen.

The term "aryl" refers to monovalent carbocyclic aromatic groups derived from a single aromatic ring or multiple condensed aromatic rings; for example phenyl, naphthyl.

The term "heteroaryl" refers to monovalent aromatic groups containing at least 5 atoms derived from a single ring or multiple condensed rings containing at least one heteroatom such as oxygen, sulphur and nitrogen.

The term "alkoxy" refers to a group -O-R' wherein R' is alkyl as defined above

The term "alkylamino" refers to the group -NH-alkyl, wherein alkyl is as defined above.The term "cycloalkyl-amino" refers to the group -NH- cycloalkyl, wherein cycloalkyl is as defined above.

The term "heterocycle-amino" refers to a heterocycle as defined above in which one or more carbon atoms is linked to -NH-, so refers to the group -NH-heterocycle.

The term "aryl-amino" refers to an aryl as defined above in which one or more carbon atoms is linked to -NH-, so refers to the group -NH-aryl.

The term "heteroaryl-amino", refers to a heteroaryl as defined above in which one or more carbon atoms is linked to -NH-, so refers to the group -NH-heteroaryl.

The term "heteroalkyl-amino" refers to the group -NH-heteroalkyl, wherein heteroalkyl is as defined above.

The term "pharmaceutically acceptable salt" according to the invention embraces salts of the compounds of formula (I) to (XXXVI) with a pharmaceutically acceptable acid or base, in particular an acid addition salt. The acid addition salt form of a compound of formula (I) to (XXXVI) that occurs in its free form as a base can be obtained by treating the free base with an appropriate acid such as an inorganic acid, for example, a hydrohalic acid such as hydrochloric acid or hydrobromic acid, sulfuric acid, nitric acid, phosphoric acid and the like; or an organic acid, such as, for example, acetic acid, trifluoroacetic acid, oxalic acid, hydroxyacetic acid, propanoic acid, lactic acid, pyruvic acid, malonic acid, succinic acid, maleic acid, fumaric acid, malic acid, tartaric acid, citric acid, methanesulfonic acid, ethanesulfonic acid, benzenesulfonic acid, p-toluenesulfonic acid, cyclamic acid, salicylic acid, p-aminosalicylic acid, pamoic acid and the like.

The invention also relates to all stereoisomeric forms such as enantiomeric and diastereoisomeric forms of the compounds of formula (I) to (XXXVI) or mixtures thereof (including all possible mixtures of stereoisomers such as racemates). With respect to the present invention reference to a compound or compounds is intended to encompass that compound in each of its possible isomeric forms and mixtures thereof, unless the particular isomeric form is referred to specifically.

Some of the compounds of formula (I) to (XXXVI) may also exist in tautomeric forms. Such forms although not explicitly indicated in the above formula are intended to be included within the scope of the present invention.

It is to be understood that each individual atom present in formula (I) to (XXXVI), or in formulae depicted herein, may in fact be present in the form of any of its naturally occurring isotopes, with the most abundant isotope(s) being preferred. Thus, by way of example, each individual hydrogen atom present in formula (I) to (XXXVI), or in the formulae depicted herein, may be present as a 1H, 2H (deuterium) or 3H (tritium) atom, preferably 1H. Similarly, by way of example, each individual carbon atom present in formula (I) to (XXXVI) , or in the formulae depicted herein, may be present as a 12C, 13C or 14C atom, preferably 12C.

The present invention includes within its scope solvates of the compounds of formula (I) to (XXXVI) above. Such solvates may be formed with common organic solvents or water.

The present invention also includes within its scope co-crystals of the compounds of formula (I) to (XXXVI) above. The technical term "co-crystal" is used to describe the situation where neutral molecular components are present within a crystalline compound in a definite stoichiometric ratio. The preparation of pharmaceutical co-crystals enables modifications to be made to the crystalline form of an active pharmaceutical ingredient, which in turn can alter its physicochemical properties without compromising its intended biological activity (see Pharmaceutical Salts and Co-crystals, ed. J. Wouters & L. Quere, RSC Publishing, 2012).

Compounds according to the present invention may exist in different polymorphic forms. Although not explicitly indicated in the above formula, such forms are intended to be included within the scope of the present invention.

The term "prodrug" means a compound metabolised in vivo to a compound of the invention or its salt. A prodrug may be identified by administering the prodrug to a mammal, such as rat, mouse, monkey or man, and identifying the compound or its salt, for example in blood or urine.

Another embodiment of the present invention concerns a pharmaceutical composition comprising a detectable amount of a compound of formula (I) to (XXXVI) or a pharmaceutically acceptable salt, solvate or co-crystal thereof in combination with a pharmaceutically acceptable diluent or carrier.

In yet another embodiment, the present invention concerns a compound of formula (I) to (XXXVI) a pharmaceutically acceptable salt, solvate or co-crystal thereof for use as a medicament, in particular for use in a method for the treatment or prevention of disorders caused by IgE, including allergy, type 1 hypersensitivity, familiar sinus inflammation, urticaria or related conditions, such as airway constriction in asthma, local inflammation in eczema, increased mucus secretion in allergic rhinitis, increased vascular permeability, eosinophilic granulomatosis with polyangiitis (also known as "Churg Strauss syndrome"), aspirin exacerbated respiratory disease, or cutaneous T-cell lymphoma.

The following examples illustrate how the compounds covered by formula (I) to (XXXVI) may be synthesized. They are provided for illustrative purposes only and are not intended, nor should they be construed, as limiting the invention in any manner. Those skilled in the art will appreciate that routine variations and modifications of the following examples can be made without exceeding the scope of the invention.

### EXAMPLES

The following examples illustrate how the compounds covered by formula I may be synthesized. They are provided for illustrative purposes only and are not intended, nor should they be construed, as limiting the invention in any manner.

### EXAMPLES

### Abbreviations

- DCM:: Dichloromethane
- MTBE:: tert-Butylmethyl ether
- Et₂O:: Diethyl ether
- THF:: Tetrahydrofuran
- EtOAc:: Ethyl acetate
- MeCN:: Acetonitrile
- MeOH:: Methanol
- h:: Hour
- b s.:: Broad singlet
- r.t.:: Room temperature
- M:: Mass
- Brine:: Saturated sodium chloride solution
- HPLC:: High performance liquid chromatography
- LCMS:: Liquid Chromatography Mass Spectrometry
- ES+:: Electrospray positive ionisation
- DIPEA:: *N,N*-di-iso-propylethylamine
- RT:: Retention time
- DMF:: *N,N'*-dimethylformamide
- NaOH:: Sodium hydroxide
- COMU:: (1-Cyano-2-ethoxy-2-oxoethylidenaminooxy)dimethylamino-morpholino-carbenium hexafluorophosphate
- mCPBA:: 3-Chloroperbenzoic acid
- DMAP:: 4-Dimethylaminopyridine
- NMP:: N-Methyl-2-pyrrolidone
- CV:: Column volume
- TFA:: Trifluoroacetic acid
- DMSO:: Dimethyl sulfoxide
- EDCI:: *N*-(3-Dimethylaminopropyl)-*N*'-ethylcarbodiimide hydrochloride
- TBTU:: *O*-(Benzotriazol-1-yl)-*N,N,N',N'*-tetramethyluronium tetrafluoroborate
- CDI:: 1,1'-Carbonyldiimidazole
- EtOH:: Ethanol

### Analytical Conditions

All NMRs were obtained either at 250 MHz, 300 MHz, 400 MHz or 500 MHz.

All reactions involving air- or moisture-sensitive reagents were performed under a nitrogen atmosphere using dried solvents and glassware.

All compound LCMS data was determined using the methods below.

### LCMS Methods

### Method 1: 6120B pH10 long (Slough)

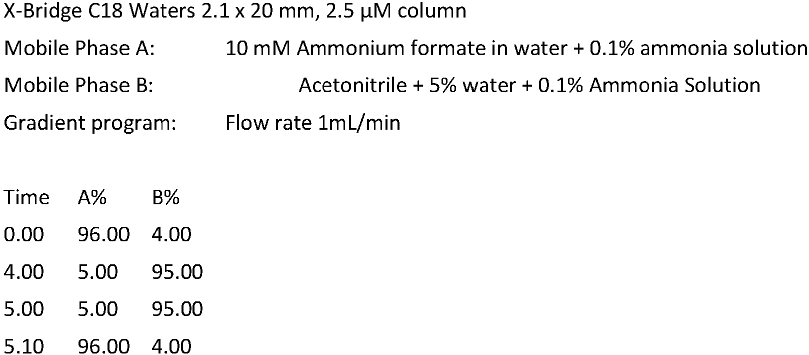

### Method 2: 6140 pH3 Registration (Slough)

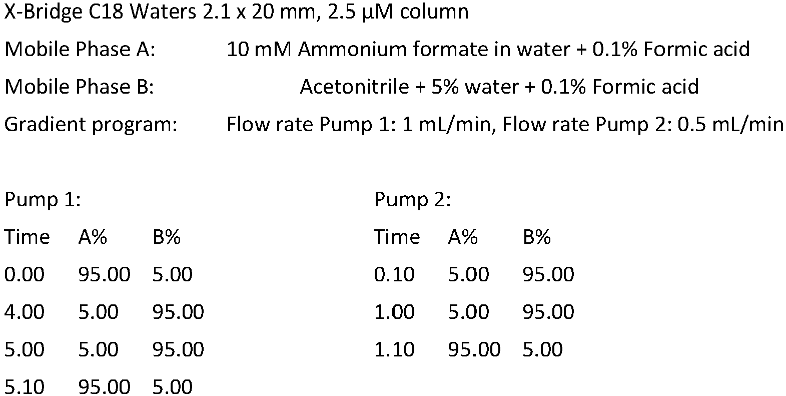

### Method 3: 6140 pH10 Registration (Slough)

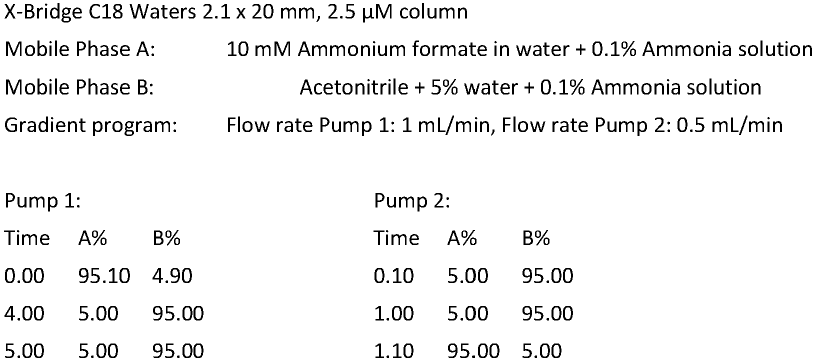

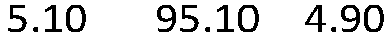

### Method 4: L102 pH10 short 3 min (Crude system 1?) (Slough)

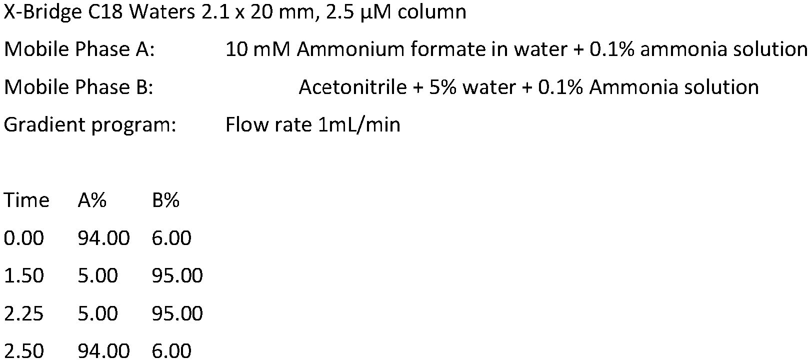

### Method 5: MS17 7 Min HiRes Evotec Shimadzu (Evotec) MET/CR/1416

| | |
|---|---|
| Mobile Phase A: | 0.1% Formic Acid in water |
| Mobile Phase B: | 0.1% Formic Acid in Acetonitrile |

### Waters Atlantis dC18, Part number 186001295, 2.1 mm x 100 mm, 3 µm column

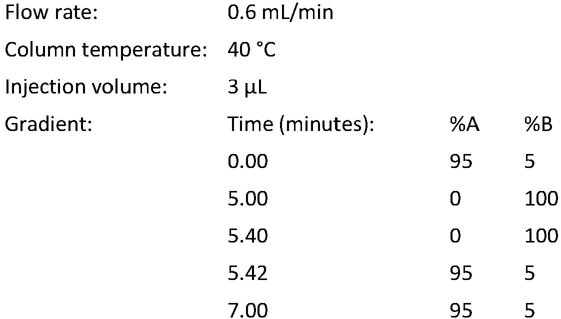

UV 215 nM, PDA spectrum 210 - 420 nm, step: 1 nm
MSD Scan Positive 100-1000

### Method 6: MS17 METCR1673 Generic 2 min (Evotec)

| | |
|---|---|
| Mobile Phase A: | 0.1% Formic Acid in water |
| Mobile Phase B: | 0.1% Formic Acid in Acetonitrile |

### Supelco Ascentis Express, Part Number: 53802-U, 2.1 mm x 30 mm, 2.7 µm column

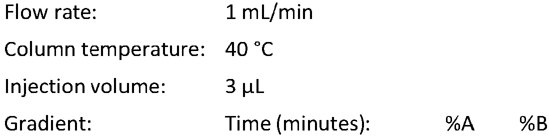

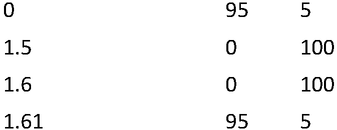

UV 215 nM, PDA spectrum 210 - 420 nm, step: 1 nm
MSD Scan Positive 100-1000

### Method 7: pH10 6120B Short (Slough)

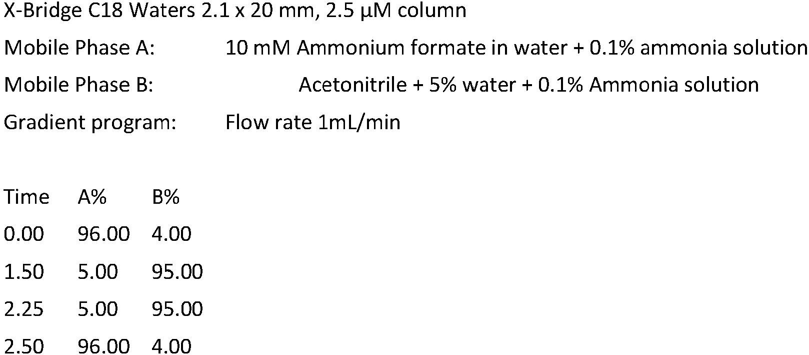

### Method 8: Braine Acidic QDA (Braine)

A QDA Waters simple quadrupole mass spectrometer is used for LC-MS analysis.

This spectrometer is equipped with an ESI source and an UPLC Acquity Hclass with diode array detector (210 to 400 nm).

Data are acquired in a full MS scan from *m*/*z* 50 to 1000 in positive mode with an acidic elution.

The reverse phase separation is carried out at 45°C on a Waters Acquity UPLC HSS T3 1.8 µm (2.1 x 50 mm) column for acidic elution.

### Gradient elution is performed with:

| | |
|---|---|
| Mobile Phase A: | Water |
| Mobile Phase B : | MeCN |
| Mobile Phase C: | Water/MeCN/formic acid 0.5% |

### Gradient program:

HPLC flow rate: 0.6 mL/minute to 0.7 mL/minute

| | |
|---|---|
| Injection volume: | 1 µL |

Full flow in MS.

| Time (minute) | A (%) | B (%) | C (%) | Flow (mL/minute) |
|---|---|---|---|---|
| 0 | 90 | 0 | 10 | 0.6 |
| 0.3 | 90 | 0 | 10 | 0.6 |
| 3.2 | 0 | 90 | 10 | 0.6 |
| 3.25 | 0 | 90 | 10 | 0.7 |
| 4 | 0 | 90 | 10 | 0.7 |
| 4.1 | 90 | 0 | 10 | 0.6 |
| 5.4 | 90 | 0 | 10 | 0.6 |

### Method 9: Braine Basic QDA (Braine)

A QDA Waters simple quadrupole mass spectrometer is used for LC-MS analysis.

This spectrometer is equipped with an ESI source and an UPLC Acquity Classic with diode array detector (210 to 400 nm).

Data are acquired in a full MS scan from *m*/*z* 50 to 1000 in positive mode with a basic elution.

The reverse phase separation is carried out at 45°C on a Waters Acquity UPLC BEH C18 1.7 µm (2.1 x 50 mm) column for basic elution.

### Gradient elution is performed with:

| | |
|---|---|
| Mobile Phase A: | H₂O/acetonitrile/ammonium formate (95/5/63 mg/L) + 50 µL NH₄OH |
| Mobile Phase B: | Acetonitrile/H₂O/ammonium formate (95/5/63 mg/L) + 50 µL NH₄OH |

### Gradient program:

HPLC flow rate: 0.4 mL/minute to 0.5 mL/minute

| | |
|---|---|
| Injection volume: | 1 µL |

Full flow in MS.

| Time (minute) | A (%) | B (%) | Flow (mL/minute) |
|---|---|---|---|
| 0 | 99 | 1 | 0.4 |
| 0.3 | 99 | 1 | 0.4 |
| 3.2 | 0 | 100 | 0.4 |
| 3.25 | 0 | 100 | 0.5 |
| 4 | 0 | 100 | 0.5 |
| 4.1 | 99 | 1 | 0.4 |
| 4.8 | 90 | 1 | 0.4 |

### Method 10: MSQ1 MET-uPLC-AB-101 7 min low pH (Evotec)

| | |
|---|---|
| Mobile Phase A: | 0.1% Formic Acid in water |
| Mobile Phase B: | 0.1% Formic Acid in Acetonitrile |

Phenomenex, Kinetex-XB C18, 2.1 mm x 100 mm, 1.7 µm column

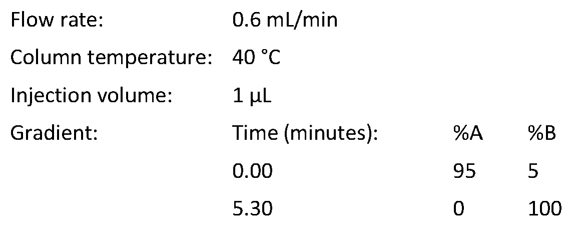

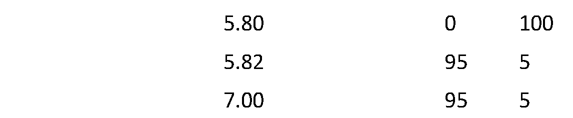

UV 215 nM, PDA spectrum 200 - 400 nm, step: 1 nm
MSD Scan Positive 150-850

### Method 11: MS17 METCR1410 Generic 2 min (Evotec)

| | |
|---|---|
| Mobile Phase A: | 0.1% Formic Acid in water |
| Mobile Phase B: | 0.1% Formic Acid in Acetonitrile |

Kinetex Core-Shell C18 50 x 2.1 mm, 5 µm column protected by Phenomenex 'Security Guard' column.

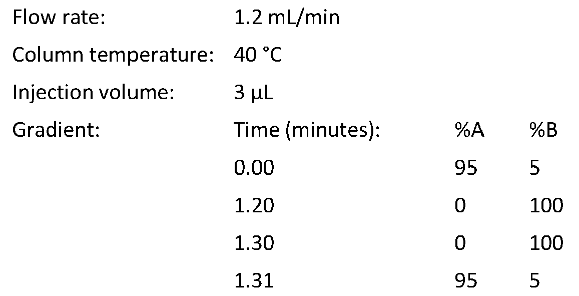

UV 215 nM, PDA spectrum 210 - 420 nm, step: 1 nm
MSD Scan Positive 100-1000

### Method 12: Agilent 6120 3 min pH3 (Evotec Insourcing lab-UCB machine)

| | |
|---|---|
| Column: | Waters X-Bridge, C18, 2.1 x 20 mm, 2.5 µm silica particle |
| Flow Rate: | 1.0 mL/min |
| Mobile Phase A: | 10 mM Ammonium Formate + 0.1% formic acid |
| Mobile Phase B: | Acetonitrile + 5% Water + 0.1% formic acid |
| Column Temp: | 40°C |
| Injection Volume: | 1 - 5 µL |
| UV Detection: | 230 to 400 nm and 215 nm |
| Mass Spec Detection: | Agilent 6120 Mass Spectrometer (ES) m/z 120 to 800 |
| Gradient: | 5% to 95% B over 1.5 minutes, hold 95% B for 0.5 minute, re-equilibrate at 5% B |

### Method 13: Braine Basic QDA Long

A QDA Waters simple quadrupole mass spectrometer is used for LC-MS analysis.

This spectrometer is equipped with an ESI source and an UPLC Acquity Classic with diode array detector (210 to 400 nm.)

Data are acquired in a full MS scan from m/z 70 to 800 in positive/negative mode with an acidic elution

The reverse phase separation is carried out at 45°C on a Waters Acquity UPLC BEH C18 1.7µm (2.1x100mm) column for basic elution

Gradient elution is done with $H 2 O / ACN / Ammonium _ formate \left(95/5/63mg/L\right) + 100 μl / L NH 4 OH$ $ACN / H 2 O / Ammonium _ formate \left(95/5/63mg/L\right) + 100 μl / L NH 4 OH$
HPLC flow rate: 0.4 ml/min to 0.5 ml/min,
injection volume: 1 µl
Full flow in MS.

| Time (min) | A (%) | B (%) | Flow (ml/min) |
|---|---|---|---|
| 0 | 99 | 1 | 0.4 |
| 0.8 | 99 | 1 | 0.4 |
| 5.30 | 0 | 100 | 0.4 |
| 5.35 | 0 | 100 | 0.5 |
| 7.30 | 0 | 100 | 0.5 |
| 7.35 | 99 | 1 | 0.4 |
| 9 | 90 | 1 | 0.4 |

### Method 14: METCR 1704 (MSQ2 1 min IPC - Evotec)

| | |
|---|---|
| Column: | Waters UPLC CSH C18, 2.1 x 100 mm, 1.7 µm silica particle |
| Flow Rate: | 0.9 mL/min |
| Mobile Phase A: | Water + 0.1% Formic acid |
| Mobile Phase B: | Acetonitrile + 0.1% Formic acid |
| Column Temp: | 40°C |
| Injection Volume: | 2 µL |
| UV Detection: | 200 to 400 nm and 215 nm |
| Mass Spec Detection | m/z 150 to 850 |
| Gradient: | 5% to 100% B over 1.1 minutes, hold 100% B for 0.25 minute, re-equilibrate at 5% B |

### Method 15: (Slough crude system pH10 short)

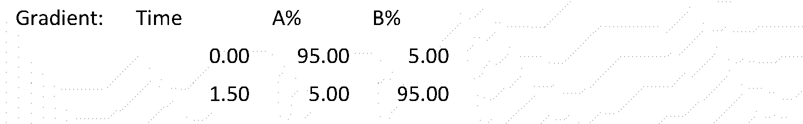

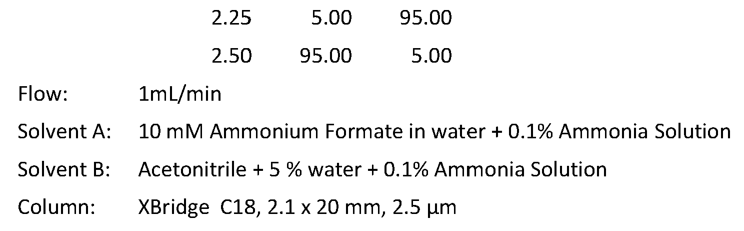

### Method 16: Agilent 6120 6 min pH10 (Evotec Insourcing lab-UCB machine)

| | |
|---|---|
| Column: | Waters X-Bridge, C18, 2.1 x 20 mm, 2.5 µm silica particle |
| Flow Rate: | 1.0 mL/min |
| Mobile Phase A: | 10 mM Ammonium Formate + 0.1% Ammonia solution |
| Mobile Phase B: | Acetonitrile + 5% Water + 0.1% Ammonia solution |
| Column Temp: | 40°C |
| Injection Volume: | 1 - 5 µL |
| UV Detection: | 230 to 400 nm and 215 nm |
| Mass Spec Detection: | Agilent 6120 Mass Spectrometer (ES) m/z 120 to 800 |
| Gradient: | 5% to 95% B over 4 minutes, hold 95% B for 1 minute, re-equilibrate at 5% B |

### Method 17: Agilent 6120 3 min pH10 (Evotec Insourcing lab-UCB machine)

| | |
|---|---|
| Column: | Waters X-Bridge, C18, 2.1 x 20 mm, 2.5 µm silica particle |
| Flow Rate: | 1.0 mL/min |
| Mobile Phase A: | 10 mM Ammonium Formate + 0.1% Ammonia solution |
| Mobile Phase B: | Acetonitrile + 5% Water + 0.1% Ammonia solution |
| Column Temp: | 40°C |
| Injection Volume: | 1 - 5 µL |
| UV Detection: | 230 to 400 nm and 215 nm |
| Mass Spec Detection: | Agilent 6120 Mass Spectrometer (ES) m/z 120 to 800 |
| Gradient: | 5% to 95% B over 1.5 minutes, hold 95% B for 0.5 minute, re-equilibrate at 5% B |

### Method 18: MS10 METCR0990 High pH 2.5 min (Evotec)

| | |
|---|---|
| Mobile Phase A: | 2 mM Ammonium bicarbonate pH10 |
| Mobile Phase B: | Acetonitrile |

Phenomenex Gemini-NX C18 2.0 mm x 50 mm, 3 µm column 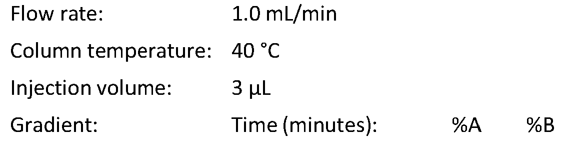 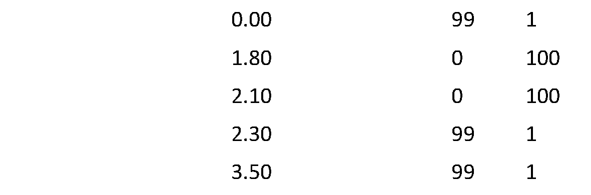
UV 215 nM, PDA spectrum 210 - 420 nm, step: 1 nm
MSD Scan Positive 150-850

### Method 19: SFC1AB (Evotec achiral SFC_1)

Column: Synergi 4u Polar RP, 4.6 x 250mm
Mobile phase: 15% Ethanol: 85%CO2
Flow rate: 4ml/min
Runtime: 15mins

### Method 20: Braine TOFA

| | |
|---|---|
| Column: | a Acquity UPLC HSS T3 C18 column (1.7µm, 2.1 x 100 mm) |
| Flow rate: | 0.4 ml/min to 0.5 ml/min |
| Solvent A: | Water |
| Solvent B: | Acetonitrile |
| Solvent C: | Formic Acid 5ml / L in Acetonitrile/water 50/50 (PH^{~}3) |
| Column Temp: | 45°C |
| Injection Volume: | 0.3 µl |

### Full flow in MS.

| Time (min) | %A | %B | %C | Flow (ml/min) |
|---|---|---|---|---|
| 0 | 88 | 2 | 10 | 0.4 |
| 0.5 | 88 | 2 | 10 | 0.4 |
| 5 | 2.5 | 90 | 7.5 | 0.4 |
| 5.1 | 2.5 | 90 | 7.5 | 0.5 |
| 7.30 | 2.5 | 90 | 7.5 | 0.5 |
| 7.5 | 88 | 2 | 10 | 0.4 |
| 10 | 88 | 2 | 10 | 0.4 |

| | |
|---|---|
| UV Detection: | 210 to 400 nm |

Mass Spec detection: LCT Waters Time of flight mass spectrometer m/z 50 to 950 in positive mode

### Method 21: Braine Acidic QDA Long

A QDA Waters simple quadrupole mass spectrometer is used for LC-MS analysis.

This spectrometer is equipped with an ESI source and an UPLC Acquity Hclass with diode array detector (210 to 400 nm.)

Data are acquired in a full MS scan from m/z 50 to 1000 in positive mode with an acidic elution

The reverse phase separation is carried out at 45°C on a Waters Acquity UPLC HSS T3 1.8µm (2.1x100mm) column for acidic elution
Gradient elution is done with
Water/ Formic Ac (solvent A)
Acetonitrile/ formic Ac (solvent B)
HPLC flow rate: 0.4 ml/min to 0.5 ml/min,
injection volume: 1 µl
Full flow in MS.

| Time (min) | A (%) | B (%) | Flow(ml/min) |
|---|---|---|---|
| 0 | 99 | 1 | 0.4 |
| 0.8 | 99 | 1 | 0.4 |
| 5.3 | 5 | 95 | 0.4 |
| 5.35 | 5 | 95 | 0.5 |
| 8.30 | 5 | 95 | 0.5 |
| 8.35 | 99 | 1 | 0.4 |
| 10 | 99 | 1 | 0.4 |

### Method 22: SFC1AB (Evotec achiral SFC_2)

Column: KR60-5DIOL, 4.6 x 250mm
Mobile phase: 25% Methanol: 75%CO2
Flow rate: 4ml/min
Runtime: 8mins

### Method 23: Chiral SFCMS Analysis (Evotec)

Column: Chiralpak AD-H 25cm
Mobile phase: 15% IPA: 85%CO2
Flow rate: 4ml/min
Runtime: 20mins

### Method 24: pH3 UPLC (Slough)

Low pH (approximately pH 3):

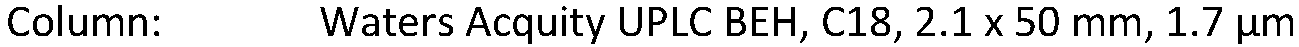

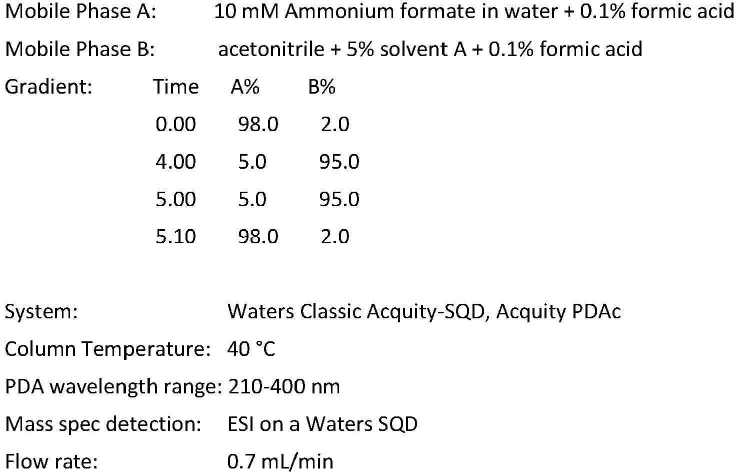

### Method 25: pH10 UPLC (Slough)

High pH (approximately pH 9.5)

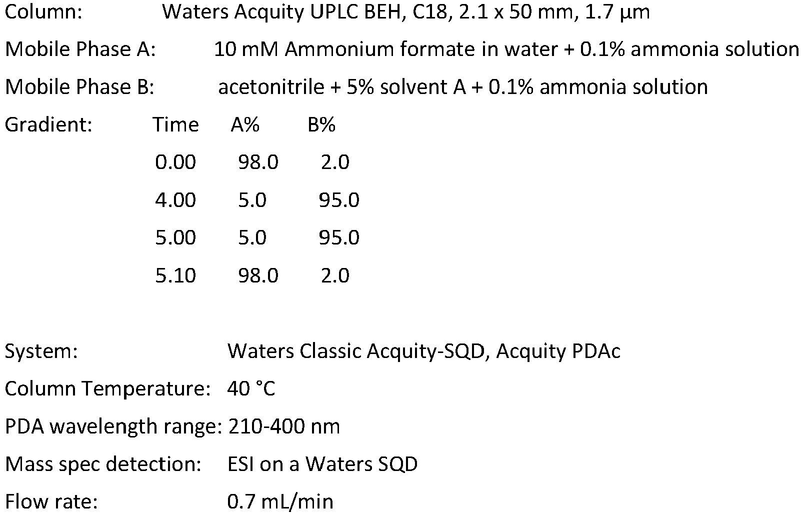

### Method 26: (Slough Registration 6140 pH 10, 2018)

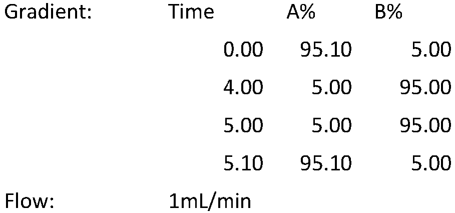

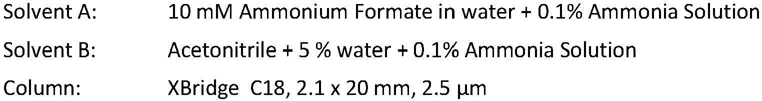

### Method 27: (Slough Registration 6140 pH 3, 2018)

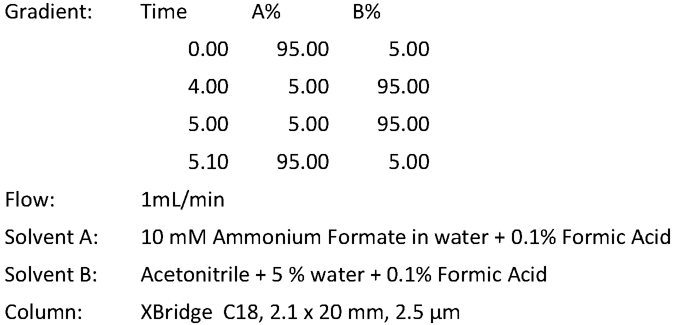

### Method 28: (Slough crude, long pH 3, 2018)

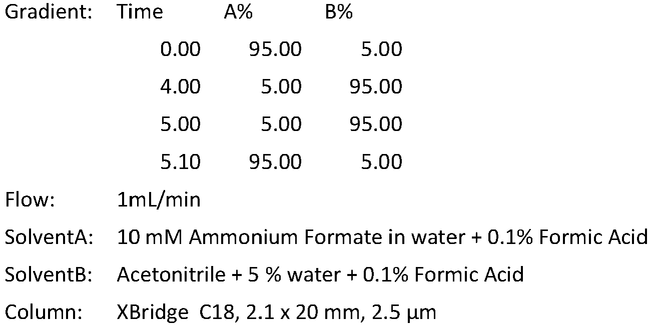

### Method 29: Agilent 6120 6 min pH3

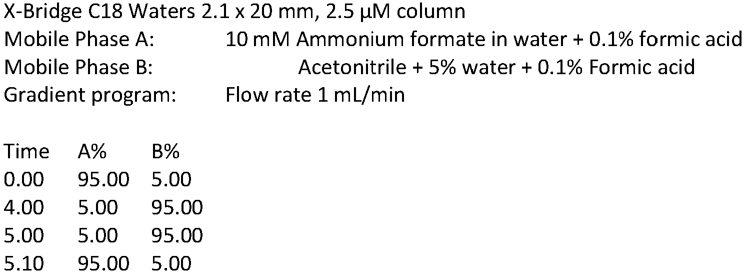

### Method 30: MET/uPLC/AB102

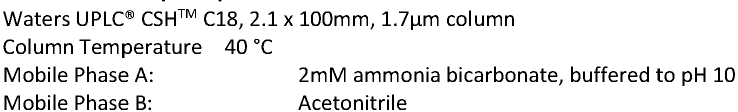

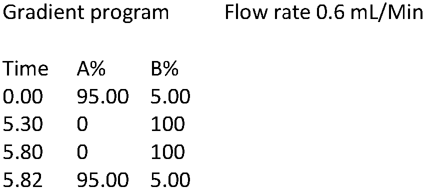

### Method 31: MET/CR/1600

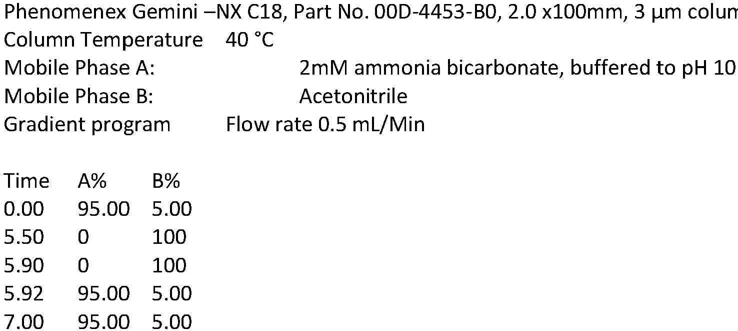

### Method 32: MET-uPLC-AB-105

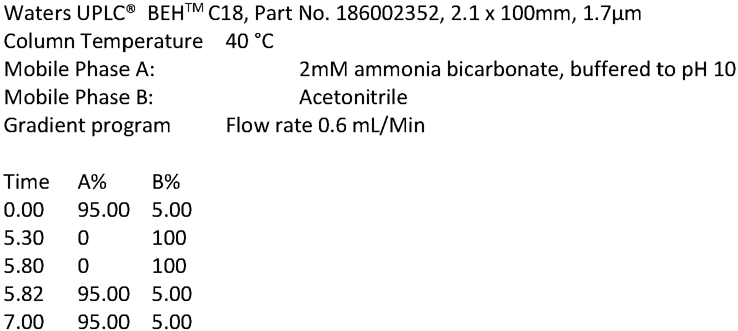

### Method 33: 6140 pH10 Short pH 3

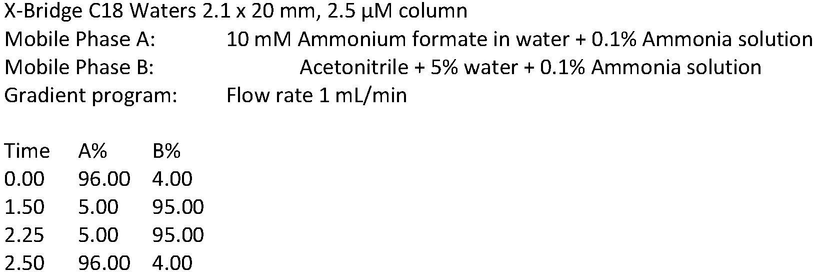

### Method 34: Agilent 1260-6120B

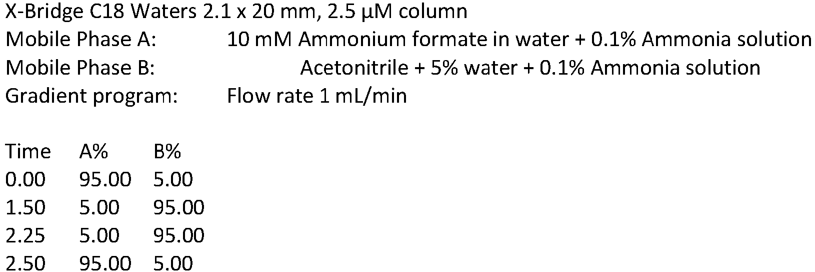

### Chiral SFC Analytical Methods

### Method 1: using conditions below unless otherwise stated in procedure

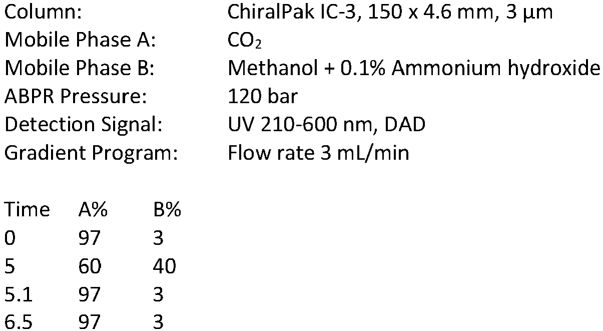

### HPLC Methods

### Method 1: Generic High pH prep method (Standard Method)

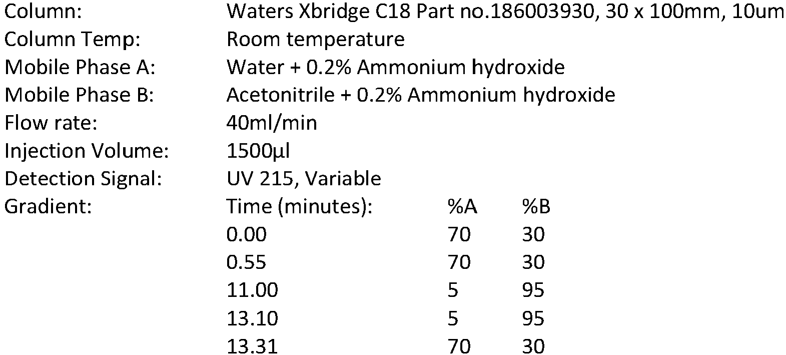

### Method 2: Generic High pH prep method (Early elute method)

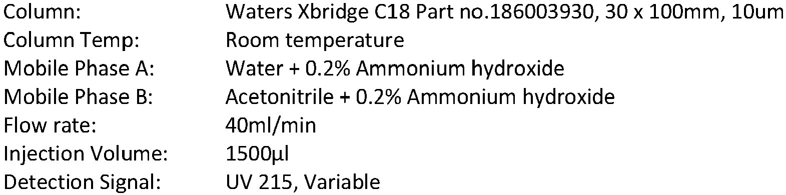

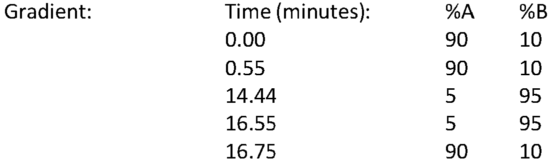

### Method 3: Generic Low pH prep method (Standard Method)

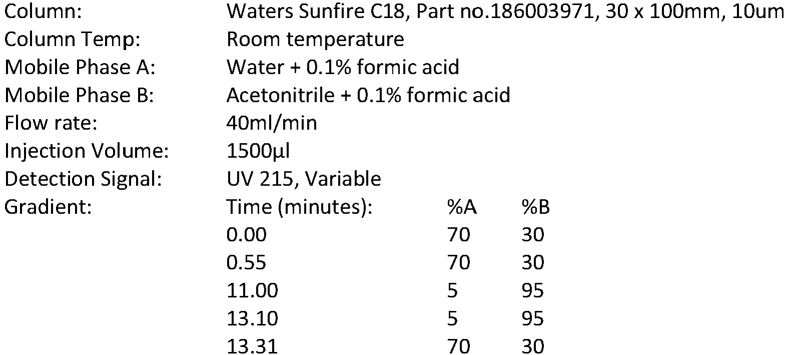

### Method 4: Generic Low pH prep method (Early elute method)

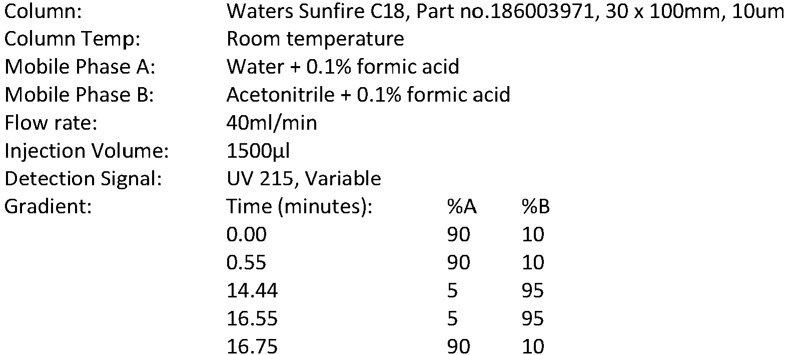

### Method 5: UCB pH10 Gilson Standard Method

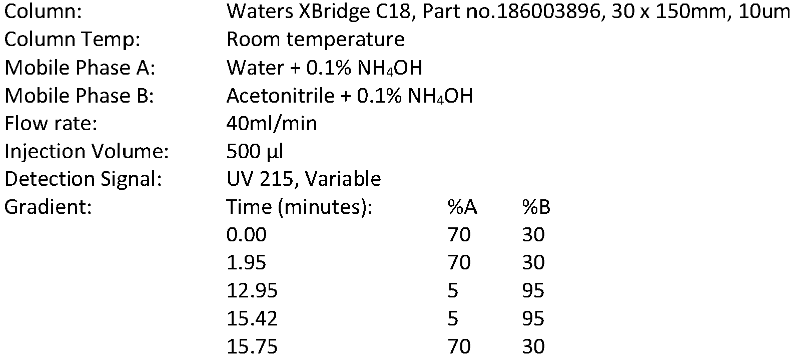

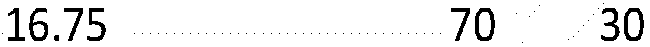

### General Experimental Methods

### General Method 1

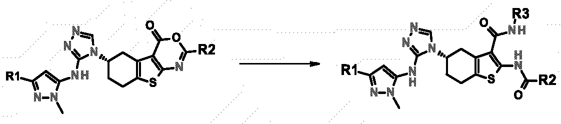

A mixture the tricycle and amine (1 - 10 equivalents) with or without an excess of base (triethylamine or DIPEA) was dissolved/suspended in either anhydrous DMF or acetonitrile. The reaction was then heated at ^{~}70 °C, 90 °C, or 140 °C for a period of 15 min to 1 hour in a sealed vial or under an atmosphere of N₂. The reaction mixture was then cooled to room temperature and either (1) filtered and purified by reverse phase HPLC or (2) worked up and purified by reverse phase HPLC or normal phase column chromatography unless otherwise noted.

### General method 2

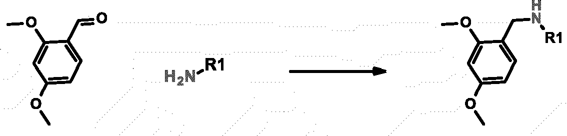

To a solution of amine (1 equivalent) in DCM was added 2,4-dimethoxybenzaldehyde (1 equivalent), followed by STAB (1.5 equivalent). The mixture was stirred at room temperature for 4 hours. The mixture was then quenched with saturated sodium bicarbonate. The aqueous layer was extracted with DCM and the organic fractions combined, dried (Na₂SO₄) and concentrated under vacuum. The resulting residue was purified by column chromatography.

### General Method 3

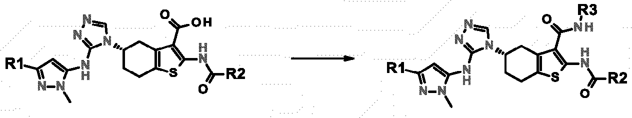

To a stirred solution of the acid (1 equivalent) in anhydrous DMF were added DIPEA (5-7 equivalents), relevant amine (2 - 6 equivalents), and HATU (1 - 3 equivalents). The reaction mixture was heated at 70 °C (unless otherwise stated) for a period of 1-7 hours (unless otherwise stated). Note: in some cases, the reagents were added in two or three portions throughout the duration of the reaction. The reaction mixture was then cooled to room temperature and either (1) purified by reverse phase HPLC or (2) worked up and purified by column chromatography.

### General method 4

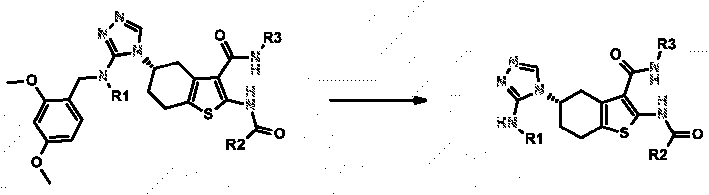

To a stirred solution of the 2,4-Dimethoxylbenzyl-protected amine (1 equivalent) in anhydrous dioxane was added 4 M HCl in dioxane (20 equivalents). The reaction mixture was stirred at RT for 16 hrs. After removing solvent, crude reaction mixture was used in the next stage as a HCl salt. Alternatively, the residue was dissolved in saturated aqueous NaHCO₃ and DCM, organic layer was separated and extracted with further DCM. Organic layers were combined, dried (Na₂SO₄) and concentrated under vacuum. Then purified either by column chromatography or reverse phase HPLC.

### General method 5

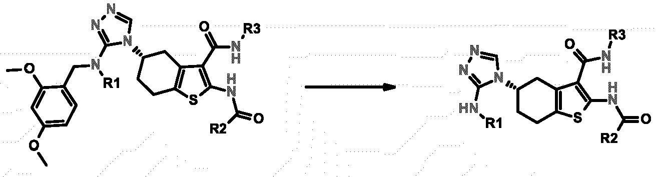

To a stirred solution of the 2,4-Dimethoxylbenzyl-protected amine (1 equivalent) in anhydrous DCM was added TFA (10 equivalents). The reaction mixture was stirred at RT for 16 hrs. After removing solvent, crude reaction mixture was used in the next stage as a TFA salt. Alternatively, the residue was dissolved in saturated aqueous NaHCO₃ and DCM, organic layer was separated and extracted with further DCM. Organics layers were combined, dried (Na₂SO₄) and concentrated under vacuum. Then purified either by column chromatography or reverse phase HPLC.

### General Method 6

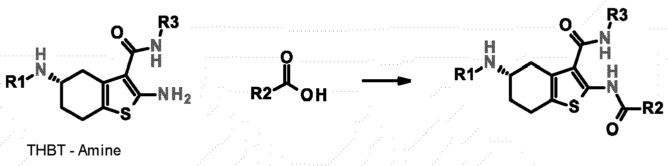

To a mixture of the THBT-amine (1 equivalent), acid (1 equivalent) and pyridine (5 equivalents) in DCM (5 mL), T3P (1-2 equivalents) was slowly added at 0 °C. The reaction mixture was slowly allowed to warm to RT and stirred for a period of several hours. Reaction mixture was worked up and purified by reverse phase HPLC.

### General method 7

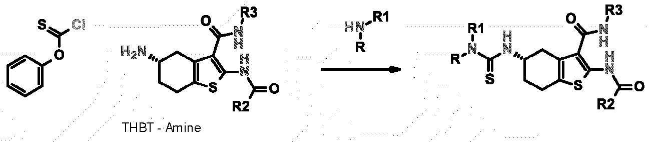

To a stirred solution of phenyl chloromethanethioate (1.1 equivalents) in DCM, at 0 °C was added THBT-amine (1 equivalent) and triethylamine (2-3 equivalents). The reaction was stirred whilst warming to room temperature for 1 hour, the relevant amine (1-2 equivalents) was then added as a solution in DCM. The mixture was stirred at room temperature until LCMS showed completion of the reaction. The mixture was then concentrated *in vacuo* and the residue purified.

### General method 8

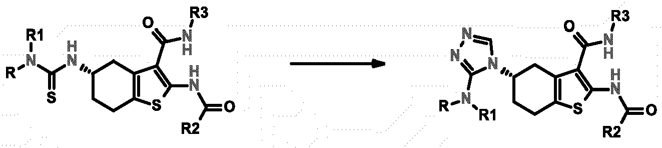

Thiourea (1 equivalent) was dissolved in DMF and formic hydrazide (2-3 equivalents) was added, followed by mercury dichloride (2-3 equivalents). The reaction mixture was stirred at room temperature for 5 minutes, then triethylamine (3 equivalents) was added. The mixture was stirred at 90 °C until reaction was complete. The mixture was cooled to room temperature, diluted with DCM and filtered through kieselguhr/Celite, washing with DCM. The solvent was removed *in vacuo* and the residue purified.

### General method 9

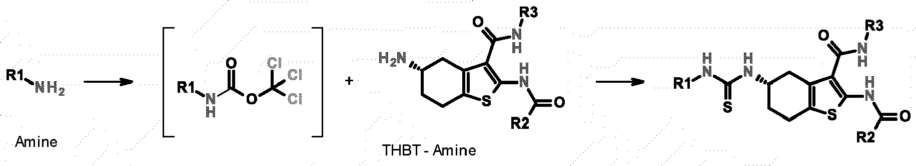

Sodium carbonate (5 equivalents) was stirred in mixture of DCM:Water (2:1, respectively) at 0 °C. Thiophosgene (1 equivalent) was added, followed by amine (1 equivalent). The reaction mixture was stirred at 0 °C for 5 minutes, then at room temperature for 1 hour. The mixture was then extracted using DCM, dried over sodium or magnesium sulphate, or passed through a hydrophobic frit. The solvent was removed *in vacuo* and the residue re-dissolved in DCM. THBT-amine (1.1 equivalents) was added and the mixture stirred at room temperature until the reaction was complete.

### Abbreviations

- sat.: saturated
- aq.: aqueous
- BOC: tert-Butoxycarbonyl
- tBuXPhos Pd G3: [(2-Di-tert-butylphosphino-2',4',6'-triisopropyl-1,1'-biphenyl)-2-(2'-amino-1,1' biphenyl)] palladium(II) methanesulfonate
- DCE: 1,2-Dichloroethane
- TEA: Triethylamine
- THBT: 4,5,6,7-tetrahydrobenzothiophene
- T3P: 2,4,6-Tripropyl-1,3,5,2,4,6-trioxatriphosphorinane-2,4,6-trioxide
- EDC: N-(3-Dimethylaminopropyl)-N'-ethylcarbodiimide hydrochloride
- STAB: Sodium triacetoxyborohydride
- HATU: 1-[Bis(dimethylamino)methylene]-1H-1,2,3-triazolo[4,5-b]pyridinium 3-oxid hexafluorophosphate
- DPPA: Diphenyl phosphoryl azide
- PTSA: para toluene sulfonic acid
- DEA: diethylamine
- DMA: N,N-dimethylacetamide
- TBME: tert-Butylmethyl ether (also abbreviated to MTBE)
- hr or hrs: hours
- KP-NH: Biotage^{®} SNAP KP-NH, Flash Chromatography Cartridge
- mCPBA: 3-Chloroperoxybenzoic acid
- o/n: overnight

It will be apparent to the person skilled in the art that different retention times (RT) may be obtained for LCMS if different analytical conditions are used.

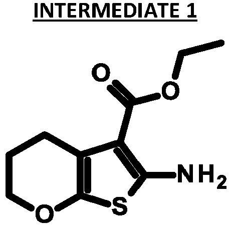

### Ethyl 6-amino-3,4-dihydro-2H-thieno[2,3-b]pyran-5-carboxylate

To sulfur [7704-34-9] (0.36 g, 11.2 mmol) was added dihydro-2H-pyran-3(4H)-one [23462-75-1] (1.04 g, 10.4 mmol), EtOH (30 mL), ethyl cyanoacetate [105-56-6] (1.2 mL, 11 mmol) and morpholine [110-91-8] (1.4 mL, 16 mmol). The reaction mixture was stirred at r.t. for 15 minutes under nitrogen before heating to 50 °C overnight. The reaction mixture was cooled to r.t. and filtered, washing with EtOAc. The crude residue was purified by flash column chromatography on silica (gradient elution with 100% isohexane/EtOAc to 30% EtOAc/isohexane) to afford the *title compound* (1.41 g, 60%) as a pale brown crystalline solid. δ_{H} (300 MHz, DMSO-*d*₆) 7.00 (s, 2H), 4.13 (q, J 7.1 Hz, 2H), 4.09 - 4.03 (m, 2H), 2.56 (t, J 6.4 Hz, 2H), 1.94 - 1.79 (m, 2H), 1.23 (t, J 7.1 Hz, 3H).

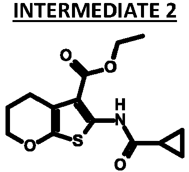

### ethyl 6-(cyclopropanecarbonylamino)-3,4-dihydro-2H-thieno[2,3-b]pyran-5-carboxylate

To *intermediate 1* (1.41 g, 6.20 mmol) dissolved in DCM (45 mL) was added DIPEA (3.2 mL, 18 mmol) and cyclopropanecarbonyl chloride [4023-34-1] (0.63 mL, 6.8 mmol) and the reaction mixture was stirred under nitrogen at r.t. for ^{~}2 h before quenching with water (15 mL) and diluting with DCM (25 mL). The reaction mixture was filtered through a phase separation cartridge and the mixture was concentrated *in vacuo* to afford the *title compound* (2.16 g, 118%) which was carried through crude to the following step. LCMS (ES+) [M+H]⁺ 296.1, RT 3.280 minutes, 94.2% purity (*Method 1*)*.*

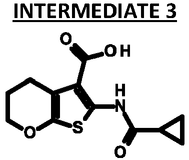

### 6-(Cyclopropanecarbonylamino)-3,4-dihydro-2H-thieno[2,3-b]pyran-5-carboxylic acid

To *intermediate 2* (2.16 g, 7.31 mmol) in 1,4-dioxane (20 mL) and water (4 mL) was added lithium hydroxide monohydrate [1310-66-3] (483 mg, 11.5 mmol) and the reaction mixture was heated to 70 °C under nitrogen for 3 h. Further lithium hydroxide monohydrate [1310-66-3] (179 mg, 4.25 mmol) was added and the mixture was heated to 70 °C under nitrogen for 2 h and cooled to r.t. overnight. The mixture was concentrated *in vacuo* and the residue left at r.t. for 2 nights. The residue was diluted with water (50 mL) and was washed with DCM (2 x 50 mL). The aqueous phase was acidified to ^{~}pH 4 with 2M aqueous hydrochloric acid solution and the solid formed was filtered and washed with water to afford the *title compound* (1.28 g, 65%) as a pale brown/orange solid. δ_{H} (300 MHz, DMSO-*d*₆) 13.06 (s, 1H), 11.31 (s, 1H), 4.14 (dd, J 5.9, 4.2 Hz, 2H), 2.67 (t, J 6.4 Hz, 2H), 2.03 - 1.78 (m, 3H), 0.98 - 0.77 (m, 4H).

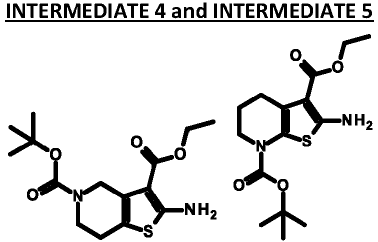

### Intermediate 4

### O5-Tert-butyl O3-ethyl 2-amino-6,7-dihydro-4H-thieno[3,2-c]pyridine-3,5-dicarboxylate and

### Intermediate 5

### O7-Tert-butyl O3-ethyl 2-amino-5,6-dihydro-4H-thieno[2,3-b]pyridine-3,7-dicarboxylate

1-Boc-3-piperidone [98977-36-7] (10.0 g, 48.7 mmol) was dissolved in EtOH (30 mL) and ethyl cyanoacetate [105-56-6] (6.06 g, 53.6 mmol), morpholine [110-91-8] (6.43 g, 73.0 mmol) and sulphur [7704-34-9] (1.72 g, 53.6 mmol) were added. The reaction mixture was stirred at r.t. for 16 h and heated at 50 °C for 24 h, before filtering and washing with EtOH. The filtrate was partitioned between DCM and water and the organic phase was washed with saturated sodium chloride solution and passed through a phase separation cartridge. The crude material was purified by flash column chromatography on silica (gradient elution with 10-50% EtOAc/hexane) to afford the *title compound intermediate 4* (2.28 g, 14%) as a yellow gum and *title compound intermediate 5* (1.74 g, 11%) as a pale yellow solid. *Intermediate 4* LCMS [M-H]⁻ 325.0, RT 2.613 minutes, 83.6% purity (*Method 1*)*. Intermediate 5* LCMS [M-H]⁻ 325.0, RT 2.774 minutes, 91.5% purity (*Method 1*)*.*

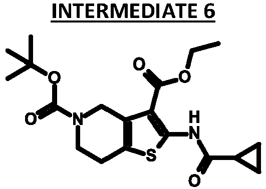

### O5-Tert-butyl O3-ethyl 2-(cyclopropanecarbonylamino)-6,7-dihydro-4H-thieno[3,2-c]pyridine-3,5-dicarboxylate

*Intermediate 4* (2.28 g, 6.99 mmol) was dissolved in DCM (50 mL) and DIPEA (1.81 g, 14.0 mmol) and cyclopropanecarbonyl chloride [4023-34-1] (0.820 g, 7.68 mmol) was added. The reaction mixture was stirred at r.t. for 16 h. The mixture was washed with water, passed through a phase separation cartridge and the organic phase concentrated *in vacuo.* The crude residue was purified by flash column chromatography on silica (gradient elution with 5-60% EtOAc/hexane) to afford the *title compound* (2.55 g, 93%) as an off-white solid. δ_{H} (400 MHz, DMSO-*d*₆) 11.20 (s, 1H), 4.54 (s, 2H), 4.32 (q, J = 7.3 Hz, 2H), 3.60 (t, J = 5.7 Hz, 2H), 2.66 (t, J = 5.8 Hz, 2H), 2.12 - 2.02 (m, 1H), 1.43 (s, 9H), 1.35 (t, J = 7.1 Hz, 3H), 1.01 - 0.83 (m, 4H). LCMS [M-Boc+H]⁺ 295.8, RT 2.904 minutes *(Method* 2).

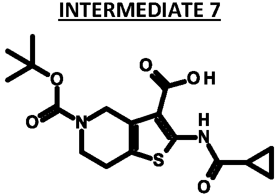

### 5-Tert-butoxycarbonyl-2-(cyclopropanecarbonylamino)-6,7-dihydro-4H-thieno[3,2-c]pyridine-3-carboxylic acid

*Intermediate* 6 (2.55 g, 6.46 mmol) was dissolved in 1,4-dioxane (100 mL) and a solution of lithium hydroxide monohydrate [1310-66-3] (0.73 g, 17.3 mmol) in water (5 mL) was added. The reaction mixture was stirred at 70 °C for 16 h before cooling to r.t. and concentrating *in vacuo.* The reaction was partitioned between water and DCM and the organic phase was separated and the aqueous phase extracted with DCM (2 x). The aqueous phase was acidified to pH < 4.5 by addition of aqueous citric acid solution and the resulting precipitate was filtered off washing with water (3 x) to afford the *title compound* (1.82g, 77%) as an off-white solid. δ_{H} (400 MHz, DMSO-*d*₆) 13.31 (s, 1H), 11.54 (s, 1H), 4.53 (s, 2H), 3.60 (t, J 5.6 Hz, 2H), 2.72 - 2.60 (m, 2H), 2.07 - 1.93 (m, 1H), 1.42 (s, 9H), 0.99 - 0.81 (m, 4H).

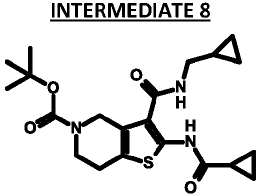

### Tert-butyl 2-(cyclopropanecarbonylamino)-3-(cyclopropylmethylcarbamoyl)-6,7-dihydro-4H-thieno[3,2-c]pyridine-5-carboxylate

*Intermediate* 7 (1.82 g, 4.97 mmol) was suspended in DCM (50 mL) and cyclopropylmethylamine [2516-47-4] (0.53 g, 7.5 mmol) and EDCI (1.18 g, 5.96 mmol) were added and the reaction mixture was stirred at r.t. for 16 h. The reaction mixture was washed with water and saturated sodium chloride solution, passed through a phase separation cartridge and the filtrate concentrated in *vacuo.* The crude residue was purified by flash column chromatography on silica (gradient elution with 10-60% EtOAc/hexane) to afford the *title compound* (873 mg, 42%) as an off-white solid. δ_{H} (400 MHz, DMSO-*d*₆) 10.75 (s, 1H), 7.60 (s, 1H), 4.23 (s, 2H), 3.36 (t, *J* 5.7 Hz, 2H), 2.91 (t, *J* 6.2 Hz, 2H), 2.52 - 2.31 (m, 2H), 1.81 - 1.60 (m, 1H), 1.17 (s, 9H), 0.87 - 0.69 (m, 1H), 0.68 - 0.48 (m, 4H), 0.26 - 0.11 (m, 2H), 0.07 - -0.10 (m, 2H). LCMS [M+H]⁺ 420.8, RT 2.582 minutes *(Method 2).* LCMS [M-H]⁻ 418.0, RT 2.471 minutes *(Method 3).*

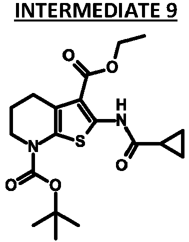

### O7-tert-Butyl O3-ethyl 2-(cyclopropanecarbonylamino)-5,6-dihydro-4H-thieno[2,3-b]pyridine-3,7-dicarboxylate

*Intermediate 5* (2.74 g, 8.39 mmol) was dissolved in DCM (50 mL) and DIPEA (2.17 g, 16.8 mmol) and cyclopropanecarbonyl chloride (0.99 g, 9.23 mmol) was added. The reaction mixture was stirred at r.t. for ^{~}16 h. The reaction mixture was washed with water and passed through a phase separation cartridge. The organic phase was concentrated *in vacuo* to give the crude residue which was purified by flash column chromatography on silica (gradient elution with 5-50% EtOAc/hexane) to afford the *title compound* (3.28 g, 99%) as an off-white solid. δ_{H} (400 MHz, DMSO-*d*₆) 11.09 (s, 1H), 4.30 (q, *J* 7.1 Hz, 2H), 3.69 (s, 2H), 2.77 (t, *J* 6.3 Hz, 2H), 2.06 - 1.93 (m, 1H), 1.93 - 1.82 (m, 2H), 1.49 (s, 9H), 1.33 (t, *J* 7.1 Hz, 3H), 1.01 - 0.82 (m, 4H). LCMS [M+H]⁺ 395.8, RT 3.141 minutes, 100.0% purity (*Method 2*)*.*

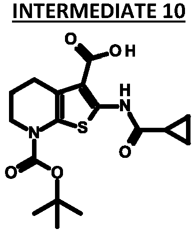

### 7-Tert-butoxycarbonyl-2-(cyclopropanecarbonylamino)-5,6-dihydro-4H-thieno[2,3-b]pyridine-3-carboxylic acid

*Intermediate 9* (3.28 g, 8.31 mmol) was dissolved in 1,4-dioxane (100 mL) and a solution of lithium hydroxide monohydrate [1310-66-3] (0.934 g, 12.5 mmol) in water (5 mL) was added. The reaction mixture was heated to 70 °C and was stirred for 16 h before cooling to r.t. and concentrating *in vacuo.* The reaction mixture was partitioned between water and DCM and the organic phase was separated and the aqueous phase extracted with further DCM (2 x). The aqueous phase was acidified to pH < 4.5 using aqueous citric acid solution and the resulting precipitated gum was extracted into EtOAc (3 x). The combined organic phases were washed with saturated sodium chloride solution and passed through a phase separation cartridge and the filtrate was concentrated *in vacuo* to afford the *title compound* (2.85 g, 94%) as a yellow sticky solid. δ_{H} (400 MHz, DMSO-*d*₆) 11.35 (s, 1H), 3.69 (br. s, J 6.2 Hz, 2H), 2.77 (t, J 6.4 Hz, 3H), 1.96 - 1.76 (m, 3H), 1.49 (s, 9H), 0.99 - 0.83 (m, 4H). LCMS [M+H]⁺ 367.2, RT 1.088 minutes (*Method 4*)*.* LCMS [M-H]⁻ 365.0, RT 2.351 minutes, 100.0% purity (*Method 2*)*.*

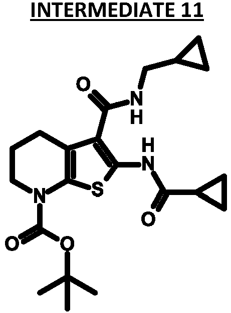

### tert-Butyl 2-(cyclopropanecarbonylamino)-3-(cyclopropylmethylcarbamoyl)-5,6-dihydro-4H-thieno[2,3-b]pyridine-7-carboxylate

*Intermediate 10* (1.43 g, 3.89 mmol) was suspended in DCM (50 mL) and cyclopropylmethylamine [2516-47-4] (0.42 g, 5.83 mmol) and EDCI (0.92 g, 4.67 mmol) were added and the reaction was stirred at r.t. for 16 h. The reaction mixture was partitioned between water and saturated sodium chloride solution, passed through a phase separator and the filtrate was concentrated *in vacuo* to yield the crude reaction mixture which was purified by flash column chromatography on silica (gradient elution with 20-80% EtOAc/isohexane) to afford the *title compound* (434 mg, 27%) as a pale yellow gum. δ_{H} (400 MHz, DMSO-*d*₆) 10.76 (s, 1H), 7.44 (s, 1H), 3.46 (s, 2H), 2.90 (t, *J* 6.2 Hz, 2H), 2.53 - 2.37 (m, 2H), 1.74 - 1.53 (m, 3H), 1.26 (s, 9H), 0.90 - 0.72 (m, 1H), 0.72 - 0.52 (m, 4H), 0.26 - 0.13 (m, 2H), 0.06 - -0.06 (m, 2H). LCMS [M+H]⁺ 420.8, [M+Na]⁺ 442.8, RT 2.645 minutes, 100.0% purity (*Method 3*)*.*

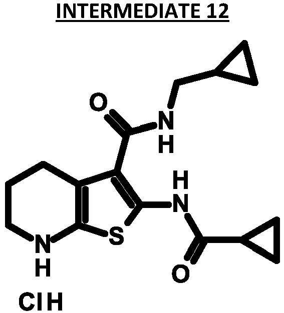

### 2-(Cyclopropanecarbonylamino)-N-(cyclopropylmethyl)-4,5,6,7-tetrahydrothieno[2,3-b]pyridine-3-carboxamide;hydrochloride

*Intermediate 11* (0.43 g, 1.03 mmol) was dissolved in 4 N hydrochloric acid solution in 1,4-dioxane (20 mL) and the reaction mixture was stirred at r.t. for 3 h, before concentrating *in vacuo* to afford the *title compound* (370 mg, 101%) as an off white solid. δ_{H} (400 MHz, DMSO-*d*₆) 10.98 (s, 1H), 7.41 (s, 1H), 3.05 - 2.94 (m, 2H), 2.90 (t, *J* 6.2 Hz, 2H), 2.43 (t, *J* 6.2 Hz, 2H), 1.73 - 1.53 (m, 3H), 0.86 - 0.69 (m, 1H), 0.68 - 0.51 (m, 4H), 0.25 - 0.16 (m, 2H), 0.05 - -0.06 (m, 2H), NH₂ not observed.

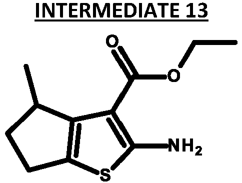

### Ethyl 2-amino-4-methyl-5,6-dihydro-4H-cyclopenta[b]thiophene-3-carboxylate

To a solution of 2-methylcyclopentanone [1120-72-5] (3.40 g, 34.6 mmol) in EtOH (10 mL) was added ethyl cyanoacetate [105-56-6] (4.31 g, 38.1 mmol), sulphur [7704-34-9] (1.22 g, 38.0 mmol) and diethylamine (3.82 g, 52.0 mmol). The reaction mixture was stirred at r.t. for 18 h and the mixture concentrated *in vacuo* to give the crude product which was purified by flash column chromatography on silica (gradient elution with 0-15% EtOAc in hexane) to afford the *title compound* (3.33 g, 43%) as a yellow solid. δ_{H} (400 MHz, DMSO-*d*₆) 7.21 (s, 2H), 4.26 - 4.04 (m, 2H), 3.14 (ddt, *J* 8.5, 6.6, 3.3 Hz, 1H), 2.72 (dtd, *J* 14.3, 8.1, 2.3 Hz, 1H), 2.55 (dd, *J* 9.3, 2.1 Hz, 1H), 2.44 (ddt, *J* 12.4, 9.3, 8.1 Hz, 1H), 1.85 (ddt, *J* 12.2, 8.1, 1.8 Hz, 1H), 1.25 (t, *J* 7.1 Hz, 3H), 1.09 (d, J 6.8 Hz, 3H).

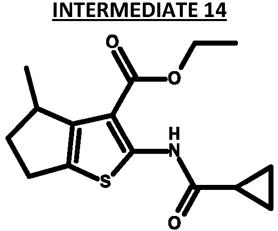

### Ethyl 2-(cyclopropanecarbonylamino)-4-methyl-5,6-dihydro-4H-cyclopenta[b]thiophene-3-carboxylate

To a solution of *intermediate 13* (1 g, 4.44 mmol) and triethylamine (1.0 mL, 7.18 mmol) in DCM (10 mL) at 0 °C was added cyclopropanecarbonyl chloride [4023-34-1] (0.6 mL, 6.61 mmol). The reaction mixture was stirred at 0 °C for 2 h, before diluting with DCM (20 mL). 1 M Aqueous hydrochloric acid solution (20 mL) was added and the aqueous phase was separated and extracted with DCM (2 x 10 mL) and washed with water (10 mL). The organic phases were combined, dried (MgSO₄), filtered and concentrated *in vacuo* to afford the *title compound* (1.45 g, 89%) as a brown oil which was utilised without further purification. δ_{H} (500 MHz, Chloroform-*d*) 11.27 (s, 1H), 4.42 - 4.35 (m, 1H), 4.31 (dq, *J* 10.8, 7.2 Hz, 1H), 3.39 - 3.28 (m, 1H), 2.91 (dtd, *J* 15.2, 8.7, 1.8 Hz, 1H), 2.72 (ddd, *J* 15.1, 9.4, 2.0 Hz, 1H), 2.58 (dq, *J* 12.6, 8.8 Hz, 1H), 2.00 (ddt, *J* 12.3, 7.9, 1.9 Hz, 1H), 1.66 (ddd, *J* 12.5, 7.9, 4.5 Hz, 1H), 1.39 (t, *J* 7.1 Hz, 3H), 1.16 (d, *J* 6.8 Hz, 3H), 1.13 (q, *J* 3.7, 3.2 Hz, 2H), 0.92 (dq, *J* 6.9, 3.7 Hz, 2H). 80% purity.

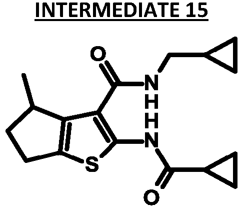

### 2-(Cyclopropanecarbonylamino)-N-(cyclopropylmethyl)-4-methyl-5,6-dihydro-4H-cyclopenta[b]thiophene-3-carboxamide

*Intermediate 14* (80% purity, 1.31 g, 3.57 mmol) was dissolved in a solution of MeOH (10 mL) and 2M aqueous NaOH solution (3.5 mL) was added. The reaction mixture was heated to reflux for 2 h and the reaction was ccoled to r.t. and concentrated *in vacuo.* The reaction mixture was acidified with 1 M aqueous hydrochloric acid solution and the mixture was extracted with EtOAc (2 x 20 mL). The combined organic phases were washed with water (10 mL), dried (MgSO₄), filtered and concentrated *in vacuo* to afford the acid: *2-(cyclopropanecarbonylamino)-4-methyl-5,6-dihydro-4H-cyclopenta[b]thiophene-3-carboxylic acid* (1 g, 74%, 70% purity) which was utilised without further purification.

EDCI (180 mg, 0.94 mmol) was added to a solution of *2-(cyclopropanecarbonylamino)-4-methyl-5,6-dihydro-4H-cyclopenta[b]thiophene-3-carboxylic acid* (200 mg, 0.75 mmol) and 1-cyclopropylmethanamine [2516-47-4] (100 µL, 1.15 mmol) in DCM (5 mL). The reaction mixture was stirred at r.t. for 4 h before diluting with DCM (10 mL) and washing with 1 M aqueous hydrochloric acid solution (10 mL). The organic phase was separated, dried (MgSO₄), filtered and concentrated *in vacuo* to give the crude product which was purified by flash column chromatography on silica (gradient elution with 0-30% EtOAc/heptane) to afford the *title compound* (40 mg, 16%) as a light brown solid. LCMS [M+H]⁺ 319.1, RT 4.58 minutes (*Method 5*)*.*

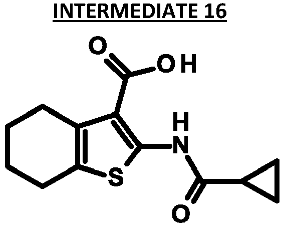

### 2-(Cyclopropanecarbonylamino)-4,5,6,7-tetrahydrobenzothiophene-3-carboxylic acid

Cyclopropanecarbonyl chloride [4023-34-1] (5.44 g, 51.0 mmol) was added to a solution of 2-amino-4,5,6,7-tetrahydro-benzo[B]thiophene-3-carboxylic acid methyl ester [108354-78-5] (10.0 g, 46.4 mmol) and DIPEA (15.0 g, 116 mmol) in DCM (100 mL) and the reaction was stirred at r.t. overnight. The reaction mixture was washed with water and brine, passed through a phase separator and evaporated *in vacuo* to yield a yellow sticky solid which was dissolved in 1,4-dioxane (100 mL). A solution of lithium hydroxide monohydrate [1310-66-3] (2.92 g, 69.6 mmol) in water (30 mL) was added and the mixture was heated at 70°C for ^{~}3 h. The reaction mixture was cooled to r.t. and was left to stand overnight and the 1,4-dioxane was removed *in vacuo.* The aqueous residue was diluted with water and washed with DCM (2 x). The aqueous phase was acidified to pH <4 by addition of concentrated hydrochloric acid. The resulting precipitate was filtered, washed with water (3 x) and was allowed to air dry to afford the *title compound* (12.1 g, 99%) as a cream solid. δ_{H} (400 MHz, DMSO-*d*₆) 13.04 (s, 1H), 11.46 (s, 1H), 2.81 - 2.63 (m, 2H), 2.63 - 2.54 (m, 2H), 1.95 (h, *J* 5.6 Hz, 1H), 1.80 - 1.60 (m, 4H), 1.02 - 0.76 (m, 4H).

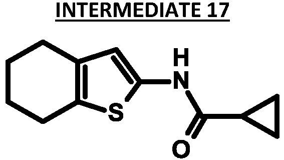

### N-(4,5,6,7-Tetrahydrobenzothiophen-2-yl)cyclopropanecarboxamide

A suspension of *intermediate 16* (500 mg, 1.89 mmol) in toluene (5 mL) was heated at 180 °C in a microwave for 8 h. The solvent was removed *in vacuo* and the residues were dissolved in hot EtOAc (20 mL). The mixture was filtered to afford the *title compound* (403 mg, 97%). δ_{H} (400 MHz, DMSO-*d*₆) 11.05 (s, 1H), 6.29 (s, 1H), 2.57 (t, J 5.8 Hz, 2H), 2.45 (t, J 5.9 Hz, 2H), 1.82 - 1.54 (m, 5H), 0.79 (d, J 6.2 Hz, 4H).

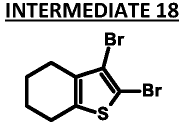

### 2,3-Dibromo-4,5,6,7-tetrahydrobenzothiophene

To a stirred solution of 4,5,6,7-tetrahydro-1-benzothiophene [13129-17-4] (3 g, 21.7 mmoL) in acetic acid (15 mL) and chloroform (15 mL) cooled to -5 °C was added N-bromosuccinimide [128-08-5] (8.5 g, 47.8 mmol) portionwise over 1.5 h and the resulting yellow/orange solution was stirred at -5 °C. The reaction mixture was quenched with 10% aqueous sodium sulphite (10 mL) and extracted with DCM (3 x 15 mL). The combined organic phases were washed with saturated aqueous sodium hydrogen carbonate solution (15 mL), brine (15 mL) and dried (MgSO₄) and filtered. The crude material was purified by filtration through silica (gradient elution with 0 to 10% DCM in heptane) to afford the *title compound* (260 mg, 64%) as a colourless oil. δ_{H} (250 MHz, Chloroform-d) 2.79 - 2.59 (m, 2H), 2.57 - 2.42 (m, 2H), 1.91 - 1.70 (m, 4H).

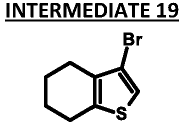

### 3-Bromo-4,5,6,7-tetrahydrobenzothiophene

To a stirred solution of *intermediate 18* (3.9 g, 13.2 mmoL) in Et₂O (100 mL) cooled to -78 °C was added n-butyllithium 1.6 M in hexanes (17.3 mL, 27.7 mmoL) dropwise over 20 minutes and the resulting solution was stirred at this temperature for 1.5 h. Water (30 mL) was added, the cooling bath was removed and the reaction mixture was warmed to r.t. over a couple of hours. EtOAc (30 mL) was added, the layers were separated and the aqueous phase was extracted with further EtOAc (40 mL). The combined organic phases were washed with brine (20 mL), dried (MgSO₄), filtered and concentrated *in vacuo* to afford the crude material (2.8 g) as an orange oil which was purified by flash column chromatography on silica (gradient elution with of 0 to 10% DCM in heptane) to afford the *title compound* (885 mg, 31%). δ_{H} (250 MHz, Chloroform-*d*) 7.03 (s, 1H), 2.81 - 2.69 (m, 2H), 2.57 - 2.46 (m, 2H), 1.92 - 1.73 (m, 4H).

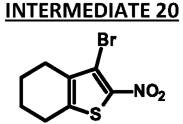

### 3-Bromo-2-nitro-4,5,6,7-tetrahydrobenzothiophene

To a solution of *intermediate 19* (660 mg, 3 mmol) in DCM (15 mL) cooled to -5 °C was added nitronium tetrafluoroborate [13826-86-3] (460 mg, 3.34 mmoL) in three portions (200 mg + 200 mg + 60 mg at 5 minutes intervals). After 1 h the mixture was poured onto ice, diluted with DCM and warmed to r.t.. The layers were separated and the aqueous phase was extracted with DCM (10 mL). The combined organic phases were washed with brine and dried (MgSO₄) and filtered. The crude product was combined with the crude material from another experiment (starting material *intermediate 17,* 200 mg, 0.92 mmol) concentrated *in vacuo* and purified by flash column chromatography on silica (gradient elution with 0 to 50% DCM in heptane) to afford the *title compound* (260 mg, 25%) as a red solid. δ_{H} (500 MHz, Chloroform-*d*) 2.87 - 2.68 (m, 2H), 2.62 - 2.49 (m, 2H), 1.96 - 1.74 (m, 4H).

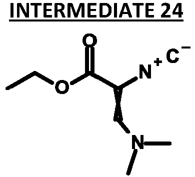

### Ethyl 3-(dimethylamino)-2-isocyanoprop-2-enoate

To a brown solution of ethyl isocyanoacetate [2999-46-4] (1 g, 8.84 mmol) in dry ethanol (10 mL) at 0 °C (ice bath) under N₂ was added DMF-DMA (1.53 mL, 11.49 mmol) dropwise. Reaction was allowed to warm to room temperature and stirred for 24 hours. The reaction was concentrated in *vacuo* and the material was purified by flash chromatography on silica (elution gradient using 0 - 60% ethyl acetate in heptane) to afford the *title compound* (780 mg, 52%) as a yellow oil which solidified on cooling. δ_{H} (500 MHz, Chloroform-d) δ 7.18 (s, 1H), 4.22 (q, J 7.1 Hz, 2H), 3.23 (s, 6H), 1.30 (t, J 7.1 Hz, 3H).

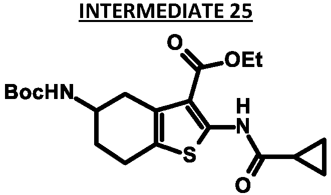

### Ethyl 5-(tert-butoxycarbonylamino)-2-(cyclopropanecarbonylamino)-4,5,6,7-tetrahydrobenzothiophene-3-carboxylate

To a stirred solution of *intermediate 102a* (15 g, 44.1 mmol) in DCM (250 mL) and triethylamine (12.3 mL, 88.2 mmol) was added cyclopropanecarbonyl chloride [4023-34-1] (6.9 g, 66.2 mmol) dropwise at 0 °C. The reaction mixture was stirred at r.t. for 16 h before quenching with 0.5 N aqueous hydrochloric acid solution. The organic phase was separated, washed with brine, dried (Na₂SO₄), filtered and concentrated *in vacuo* to give the crude product which was triturated with Et₂O and pentane to afford the *title compound* (14 g, 78%). δ_{H} (400 MHz, DMSO-*d*₆) δ 11.17 (s, 1H), 6.92 (d, *J* 7.3 Hz, 1H), 4.29 (q, *J* 7.1 Hz, 2H), 3.62 (s, 1H), 3.04 (dd, *J* 17.0, 4.3 Hz, 1H), 2.74-2.66 (m, 2H), 2.46 (d, *J* 9.3 Hz, 1H), 2.06 - 1.88 (m, 2H), 1.68-1.58 (m, 1H), 1.40 (s, 9H), 1.31 (t, *J* 7.1 Hz, 3H), 0.97 - 0.84 (m, 4H). LCMS [M-Boc+H]⁺ 309.8, RT 2.831 minutes, 96.2% purity (*Method 2*)*.*

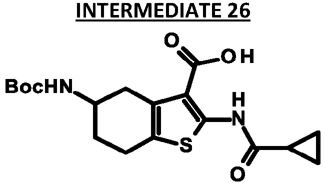

### 5-(tert-Butoxycarbonylamino)-2-(cyclopropanecarbonylamino)-4,5,6,7-tetrahydrobenzothiophene-3-carboxylic acid

To a stirred solution of *intermediate 25* (14 g, 34.3 mmol) in 1,4-dioxane (100 mL) and water (100 mL) was added lithium hydroxide (1.64 g, 68.6 mmol) and the reaction mixture was heated at 100 °C for 3 h. The reaction mixture was acidified with 1N hydrochloric acid solution to pH 6, stirred for 15 minutes and filtered. The residue obtained was dried *in vacuo* to afford the *title compound* (12 g, 91%). δ_{H} (400 MHz, Chloroform-d) 11.31 (s, 1H), 5.80 (s, 1H), 4.70 (s, 1H), 3.99 (s, 1H), 3.22-3.18 (m, 2H), 2.71 (s, 2H), 2.02-2.00 (m, 1H),1.78-1.67 (m, 2H), 1.45 (s, 9H), 1.18-1.12 (m, 2H), 0.96-0.92 (m, 2H). LCMS [M-H]⁻ 379.0, RT 1.267 minutes, 77.3% purity (*Method 7*)*.*

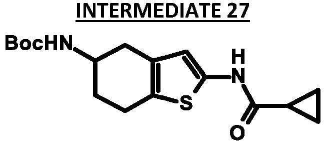

### tert-Butyl N-[2-(cyclopropanecarbonylamino)-4,5,6,7-tetrahydrobenzothiophen-5-yl]carbamate

To a suspension of *intermediate 26* (1.2 g, 2.71 mmoL, 86% purity) in quinoline [91-22-5] (10 mL) was added copper powder [7440-50-8] (259 mg, 4.0 mmol). The reaction mixture was heated to 150 °C for 1 h in the microwave before diluting with DCM (60 mL) and water (60 mL). The organic phase was washed with 0.75M aqueous hydrochloric acid solution, saturated sodium hydrogen carbonate solution and brine. The organic phase was separated, dried (Na₂SO₄), filtered and concentrated in *vacuo.* The crude residue was purified by flash column chromatography on silica (gradient elution with 5% to 50% EtOAc in heptane) to afford the 5-substituted *title compound* (640 mg, 50%) as an off-white solid mixed with the 7-regio isomer (carried through previous synthetic steps, originating from the synthesis of Intermediates 102 and 102a). LCMS [M+H]⁺ 337, RT 1.28 minutes, 71% (5-regio isomer), [M+Na]⁺ 359.15, [M-tBu+H]⁺ 280.95, RT 1.38 minutes, 28% (7-regio isomer) *(Method* 6).

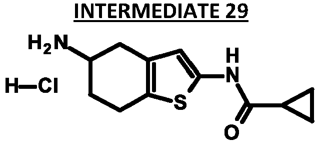

### N-(5-Amino-4,5,6,7-tetrahydrobenzothiophen-2-yl)cyclopropanecarboxamide;hydrochloride

*Intermediate 27* (640 mg, 1.35 mmol) was dissolved in 1,4-dioxane (5 mL) and 4M hydrochloric acid in 1,4-dioxane (10 mL, 40.5 mmol) was added. The reaction mixture was stirred at r.t. for 3 h. The reaction was concentrated *in vacuo* and the crude material (550 mg) used without further purification. LCMS [M+H]⁺ 236.95, RT 0.66 minutes, purity 31% (*Method 6*)*.*

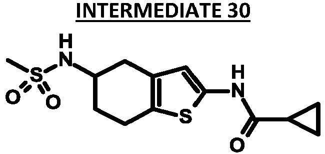

### N-[5-(Methanesulfonamido)-4,5,6,7-tetrahydrobenzothiophen-2-yl]cyclopropanecarboxamide

*Intermediate 29* (550 mg, 2.0 mmol) was dissolved in DCM (5 mL) and DIPEA (0.7 mL, 4.03 mmol) and methanesulfonyl chloride [124-63-0] (0.23 ml, 3.0 mmol) were added. The reaction mixture was stirred at r.t. for 5 h before diluting with DCM (50 mL) and water (50 mL). The organic phase was separated, dried (Na₂SO₄), filtered and concentrated *in vacuo.* The residue obtained was purified by flash column chromatography on silica (gradient elution with 12% to 100% EtOAc in heptane) to afford the *title compound* (270 mg) as an off-white solid. δ_{H} (500 MHz, CD₃OD) 6.33 (s, 1H), 3.72 - 3.63 (m, 1H), 2.99 (s, 3H), 2.92 (dd, J 15.9, 5.2 Hz, 1H), 2.83 - 2.76 (m, 2H), 2.49 (dd, J 15.9, 8.7 Hz, 1H), 2.18 - 2.10 (m, 1H), 1.88 - 1.79 (m, 1H), 1.74 - 1.66 (m, 1H), 0.96 - 0.91 (m, 2H), 0.86 (m, 2H). ^{~}90% purity.

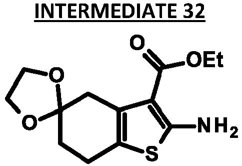

### Ethyl 2'-aminospiro[1,3-dioxolane-2,5'-6,7-dihydro-4H-benzothiophene]-3'-carboxylate

To a mixture of 1,4-dioxaspiro[4.5]decan-7-one [4969-01-1] (5 g, 32.0 mmol), sulphur [7664-93-9] (1.23 g, 38.4 mmol) and ethyl cyanoacetate [105-56-6] (4.34 g, 38.4 mmol) in EtOH (50 mL) was added triethylamine (8.92 ml, 64.0 mmol) and the reaction mixture was stirred at r.t. for 5 days before concentrating *in vacuo.* To the crude product was added water and the aqueous phase was extracted with EtOAc. The combined organic phases were washed with brine, dried (MgSO₄), filtered and concentrated *in vacuo* to yield a crude residue which was purified by flash column chromatography on silica (gradient elution with 100% DCM followed by 5% to 50% EtOAc in heptane) to afford the *title compound* (5.47 g, 59%) as yellow gum. δ_{H} (500 MHz, CD₃OD) 4.23 (q, *J* 7.1 Hz, 2H), 4.05 - 3.96 (m, 4H), 2.87 (s, 2H), 2.63 (tt, *J* 6.5, 1.6 Hz, 2H), 1.89 (t, *J* 6.5 Hz, 2H), 1.32 (t, *J* 7.1 Hz, 3H). LCMS [M+H]⁺ 283.95, RT 1.19 minutes *(Method 6).*

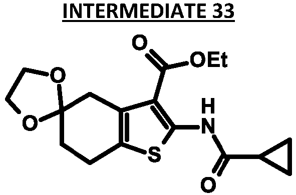

### Ethyl 2'-(cyclopropanecarbonylamino)spiro[1,3-dioxolane-2,5'-6,7-dihydro-4H-benzothiophene]-3'-carboxylate

To a solution of *intermediate 32* (5.47 g, 19.3 mmol) and DIPEA (6.7 mL, 38.6 mmol) in DCM (200 mL) at 0 °C was added cyclopropanecarbonyl chloride [4023-34-1] (2.6 ml, 28.9 mmol). The reaction mixture was stirred at r.t. for 2 h before diluting with DCM (200 mL). Water (200 mL) was added and the phases were separated. The aqueous phase was extracted with DCM (2 x 100 mL) and washed 0.5 M aqueous hydrochloric acid solution (100 mL), saturated sodium hydrogen carbonate solution (100 mL) and brine. The organic phase was dried (MgSO₄), filtered and concentrated *in vacuo* to yield the crude product, a pale yellow solid which was purified by flash column chromatography on silica (gradient elution with 5% to 50% EtOAc in heptane) to afford the *title compound* (6.5 g, 96%) as a pale yellow solid. LCMS [M+H]⁺ 352.05, RT 1.38 minutes *(Method 6).*

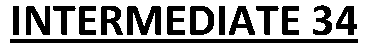

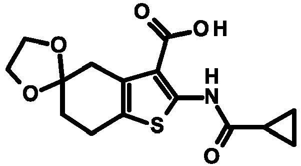

### 2'-(Cyclopropanecarbonylamino)spiro[1,3-dioxolane-2,5'-6,7-dihydro-4H-benzothiophene]-3'-carboxylic acid

To a suspension of *intermediate 33* (6.5 g, 18.5 mmoL) in 1,4-dioxane (120 mL) was added 2M lithium hydroxide monohydrate solution [1310-66-3] (18.5 mL, 37.0 mmoL) and the reaction mixture was heated to 90 °C for 2 h. The mixture was concentrated *in vacuo* and the residue was diluted with water (20 mL). The solution was acidified to pH 4-5 with 0.2M aqueous hydrochloric acid solution and the material was collected by filtration, washed with water and dried *in vacuo* to afford the *title compound* (5 g, 84%) as a brown solid. δ_{H} (500 MHz, CD₃OD) 4.03 - 3.99 (m, 4H), 2.99 (s, 2H), 2.80 (t, *J* 6.5 Hz, 2H), 1.93 (t, *J* 6.5 Hz, 2H), 1.85 - 1.77 (m, 1H), 1.05 - 0.94 (m, 4H). LCMS [M+H]⁺ 323.95, RT 1.14 minutes *(Method 6).*

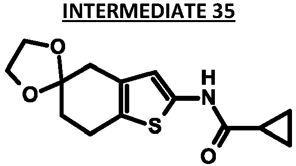

### N-Spiro[1,3-dioxolane-2,5'-6,7-dihydro-4H-benzothiophene]-2'-ylcyclopropanecarboxamide

To a suspension of *intermediate 34* (1.6 g, 4.94 mmoL) in quinolone [91-22-5] (15 mL) was added copper powder [7440-50-8] (472 mg, 7.42 mmol) and the mixture was heated to 150 °C for 1 h in the microwave. The reaction mixture was diluted with DCM (100 mL) and water (100 mL). The organic phase was washed with 0.75M aqueous hydrochloric acid solution, saturated sodium hydrogen carbonate solution and brine. The organic phase was dried (Na₂SO₄), filtered and concentrated in *vacuo.* The residue obtained was purified by flash column chromatography on silica (gradient elution with 5% to 50% EtOAc in heptane) to afford the *title compound* (1.1 g, 80%) as an off-white solid. LCMS [M+H]⁺ 279.95, RT 1.10 minutes *(Method 6).*

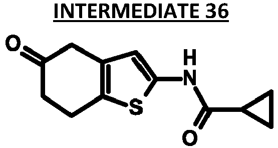

### N-(5-Oxo-6,7-dihydro-4H-benzothiophen-2-yl)cyclopropanecarboxamide

To *intermediate 35* (2 g, 7.15 mmol) dissolved in THF (80 mL) was added 1M aqueous hydrochloric acid solution (36 mL). The reaction mixture was heated at 50 °C for 5 h whilst stirring under nitrogen. After cooling to r.t. the reaction mixture was basified with saturated sodium hydrogen carbonate solution and diluted with EtOAc. The organic phase was separated, dried (Na₂SO₄), filtered and concentrated *in vacuo* to yield a pale yellow solid which was triturated with DCM/Et₂O to afford the *title compound* (1.42 g, 78%) as a pale yellow solid. LCMS [M+H]⁺ 235.90, RT 1.01 minutes *(Method 6).*

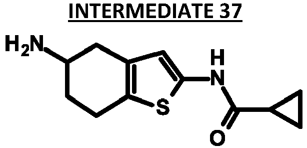

### N-(5-Amino-4,5,6,7-tetrahydrobenzothiophen-2-yl)cyclopropanecarboxamide

To a mixture of *intermediate 36* (1.42 g, 6.0 mmol), ammonium acetate (4.65 g, 60.3 mmol) and Na₂SO₄ (1.5 g) in MeOH (80 mL) was added sodium triacetoxyborohydride [56553-60-7] (2.55 g, 12.07 mmol). The reaction mixture was stirred at r.t. for 5 h. The mixture was filtered and the filtrate was diluted with EtOAc and washed with saturated sodium bicarbonate solution. The aqueous phase was extracted with isopropanol/chloroform (1:1). The organic phases were dried (MgSO₄), filtered and concentrated *in vacuo* to give the crude residue which was purified by flash column chromatography on silica (gradient elution with 1% to 70% MeOH in DCM) to afford the *title compound* (270 mg, 19%) as a light brown solid. LCMS [M+H]⁺ 236.95 *(Method 6).*

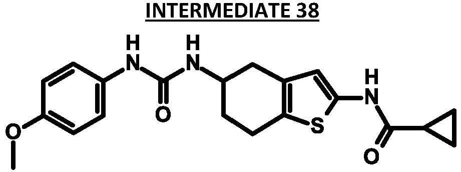

### N-[5-[(4-Methoxyphenyl)carbamoylamino]-4,5,6,7-tetrahydrobenzothiophen-2-yl]cyclopropanecarboxamide

To a solution of *intermediate 37* (130 mg, 0.55 mmol) in DCM (7 mL) was added 1-isocyanato-4-methoxybenzene [5416-93-3] (78.4 µL, 0.60 mmol). The solution was stirred at r.t. for 30 minutes and the reaction mixture was filtered and washed with DCM to afford the *title compound* (160 mg, 72%) as an off white solid. LCMS [M+H]⁺ 386.25, RT 1.17 minutes *(Method 6).*

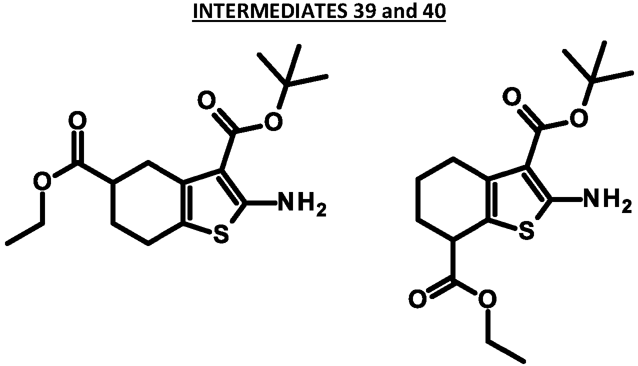

### Intermediate 39

### O3-tert-Butyl O5-ethyl 2-amino-4,5,6,7-tetrahydrobenzothiophene-3,5-dicarboxylate

### Intermediate 40

### O3-tert-Butyl O7-ethyl 2-amino-4,5,6,7-tetrahydrobenzothiophene-3,7-dicarboxylate

To a mixture of ethyl 3-oxocyclohexanecarboxylate [17159-79-4] (2 g, 11.75 mmol), sulfur (0.45 g, 14.1 mmol) and *tert-butyl* cyanoacetate [1116-98-9] (2.15 g, 15 mmol) in EtOH (20 mL) was added triethylamine (3.27 ml, 2.35 mmol). The reaction mixture was stirred at r.t. for 16 h before concentrating *in vacuo.* Water was added and the solution was extracted with EtOAc. The organic phases were combined, washed with brine, dried (MgSO₄), filtered and concentrated *in vacuo.* The crude residue was purified by flash column chromatography on silica (gradient elution with 5% to 40% EtOAc in heptane) to afford a 79:21 ratio of regioisomers of *title compound intermediate 39* and *title compound intermediate 40* (2.84 g, 74%). LCMS [M+H]⁺ 326.00, RT 1.49 and 1.52 minutes *(Method 6).*

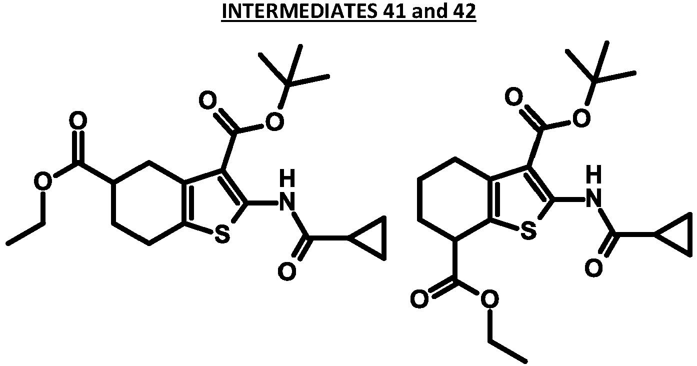

### Intermediate 41

### O3-tert-Butyl O5-ethyl 2-(cyclopropanecarbonylamino)-4,5,6,7-tetrahydrobenzothiophene-3,5-dicarboxylate

### Intermediate 42

### O3-tert-Butyl O7-ethyl 2-(cyclopropanecarbonylamino)-4,5,6,7-tetrahydrobenzothiophene-3,7-dicarboxylate

To a 79 : 21 solution of *intermediate 39* and *intermediate 40* (8 g, 24.5 mmol) and DIPEA (8.56 ml, 16.0 mmol) in DCM (300 mL) at 0 °C was added cyclopropanecarbonyl chloride (1.08 ml, 49.1 mmol). The reaction was stirred at r.t. for 2 h before diluting with DCM (200 mL). Water (200 mL) was added and the phases were separated. The aqueous phase was extracted with DCM (2 x 100 mL) and washed 0.5 M aqueous hydrochloric acid solution (100 mL), saturated sodium hydrogen carbonate solution (100 mL) and brine. The organic phase was separated, dried (MgSO₄), filtered and concentrated *in vacuo* to give a pale yellow solid as a mixture of regioisomers which was purified by flash column chromatography on silica (gradient elution with 5% to 50% EtOAc in heptane) to afford the solid product as a mixture of regioisomers. The solid was triturated with heptane/Et₂O to afford *title compound intermediate 41* (7 g, 72%) and the filtrate was concentrated *in vacuo* to afford the a 40 : 60 mixture of *title compound intermediate 41* and *title compound intermediate 42* (2 g, 21 %). *Intermediate 41:* δ_{H} (500 MHz, CD₃OD) 4.24 - 4.11 (m, 2H), 3.16 (dd, *J* 17.2, 5.4 Hz, 1H), 2.87 - 2.78 (m, 1H), 2.77 - 2.63 (m, 3H), 2.25 - 2.16 (m, 1H), 1.89 - 1.77 (m, 2H), 1.61 (s, 9H), 1.27 (t, *J* 7.1 Hz, 3H), 1.05 - 0.94 (m, 4H). LCMS [M+H]⁺ 394.10, RT 1.63 minutes *(Method 6).*

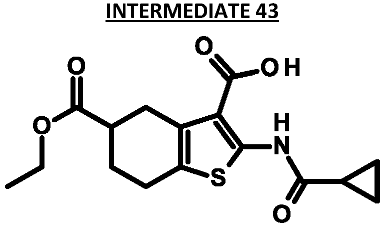

### 2-(Cyclopropanecarbonylamino)-5-ethoxycarbonyl-4,5,6,7-tetrahydrobenzothiophene-3-carboxylic acid

*Intermediate 41* (7.0 g, 17.7 mmol) was dissolved in 4M hydrochloric acid in 1,4-dioxane (88.95 mL).

The reaction mixture was stirred at 30 °C for 16 h. The solvent was removed *in vacuo* and the crude product was triturated with Et₂O to afford the *title compound* (5.3 g, 88%) as a white solid. LCMS [M+H]⁺ 338, RT 1.28 minutes *(Method 6).*

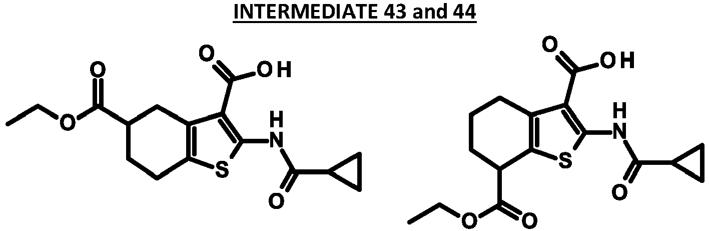

### Intermediate 43

### 2-(Cyclopropanecarbonylamino)-5-ethoxycarbonyl-4,5,6,7-tetrahydrobenzothiophene-3-carboxylic acid

### Intermediate 44

### 2-(Cyclopropanecarbonylamino)-7-ethoxycarbonyl-4,5,6,7-tetrahydrobenzothiophene-3-carboxylic acid

A 1:2 mixture of *intermediate 41 and 42* (90%, 2.7 g, 6.18 mmol) was dissolved in 4M hydrochloric acid in 1,4-dioxane (30 mL) and stirred at r.t. for 2 h, followed by 35 °C for 6 h, then r.t. for 16 h then 35 °C for 3 h. The reaction mixture was concentrated *in vacuo* and the residue was triturated with Et₂O to afford the 5-isomer *title compound intermediate 43* (220 mg, 11%) as an off white solid. The filtrate was concentrated *in vacuo* under reduced pressure to afford a 1:3 mixture of the 5-isomer *title compound intermediate 43* and the 7-isomer *title compound intermediate 44* (2 g, 88%) as a sticky yellow foam which was utilised without further purification. *Title compound intermediate 43:* δ_{H} (500 MHz, Chloroform-*d*) 11.27 (s, 1H), 4.25 - 4.12 (m, 2H), 3.24 (dd, *J* 17.4, 5.4 Hz, 1H), 3.03 - 2.90 (m, 1H), 2.82 - 2.63 (m, 3H), 2.28 - 2.18 (m, 1H), 1.96 - 1.84 (m, 1H), 1.80 - 1.71 (m, 2H), 1.29 (t, *J* 7.1 Hz, 3H), 1.20 - 1.13 (m, 2H), 1.02 - 0.94 (m, 2H).

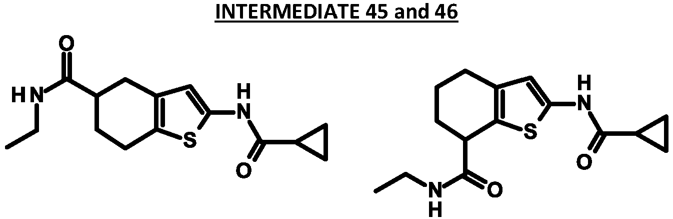

### Intermediate 45

### 2-(Cyclopropanecarbonylamino)-N-ethyl-4,5,6,7-tetrahydrobenzothiophene-5-carboxamide

### Intermediate 46

### 2-(Cyclopropanecarbonylamino)-N-ethyl-4,5,6,7-tetrahydrobenzothiophene-7-carboxamide

A 1:3 mixture of *intermediate 43* and *intermediate 44* (2 g, 5.45 mmol, 92% purity) was suspended in quinolone [91-22-5] (15 mL) and copper powder [7440-50-8] (520 mg, 8.18 mmol) was added. The mixture was heated to 150 °C for 1 h in the microwave and the reaction mixture was diluted with DCM (150 mL) and water (150 mL). The organic phase was washed with 1M aqueous hydrochloric acid solution (2 x 100 mL), followed by saturated aqueous sodium hydrogen carbonate (100 mL), dried (MgSO₄), filtered and concentrated *in vacuo.* Trituration with Et₂O afforded a 2:1 mixture of *ethyl 2-(cyclopropanecarbonylamino)-4,5,6,7-tetrahydrobenzothiophene-5-carboxylate* and *ethyl 2-(cyclopropanecarbonylamino)-4,5,6,7-tetrahydrobenzothiophene-7-carboxylate* (130 mg, 8%) as a light brown solid. The filtrate was concentrated *in vacuo* and purified by flash column chromatography on silica (gradient elution with 25 - 50% EtOAc/heptane) to afford a 1:3 mixture of *ethyl 2-(cyclopropanecarbonylamino)-4,5,6,7-tetrahydrobenzothiophene-5-carboxylate* and *ethyl 2-(cyclopropanecarbonylamino)-4,5,6,7-tetrahydrobenzothiophene-7-carboxylate* (1 g, 47%). *5-Isomer:* δ_{H} (500 MHz, CD₃OD) 6.35 (s, 1H), 4.16 (q, J 7.1 Hz, 2H), 2.82 - 2.65 (m, 5H), 2.25 - 2.17 (m, 1H), 1.92 - 1.83 (m, 1H), 1.74 - 1.67 (m, 1H), 1.26 (t, J 7.1 Hz, 3H), 0.96 - 0.91 (m, 2H), 0.89 -0.83 (m, 2H).

To a 1:3 mixture of ethyl *2-(cyclopropanecarbonylamino)-4,5,6,7-tetrahydrobenzothiophene-5-carboxylate* and *ethyl 2-(cyclopropanecarbonylamino)-4,5,6,7-tetrahydrobenzothiophene-7-carboxylate* (1 g, 3.41 mmol, 85% purity) dissolved in THF (20 mL), 2M aqueous lithium hydroxide solution (6 mL, 12 mmol) was added. The reaction mixture was stirred at r.t. for 20 h and the reaction mixture was concentrated *in vacuo* and the residue was acidified with 1M aqueous hydrochloric acid solution and the precipitate was filtered to afford a 1:3 mixture of 2-*(cyclopropanecarbonylamino)-4,5,6,7-tetrahydrobenzothiophene-5-carboxylic acid* and 2-*(cyclopropanecarbonylamino)-4,5,6,7-tetrahydrobenzothiophene-7-carboxylic acid* (600 mg, 60%). The mixture was utilised crude.

A 1:3 mixture of 2-(cyclopropanecarbonylamino)-4,5,6,7-tetrahydrobenzothiophene-5-carboxylic acid and *2-(cyclopropanecarbonylamino)-4,5,6,7-tetrahydrobenzothiophene-7-carboxylic acid (300* mg, 1.13 mmol) in DCM (5 mL) was treated with COMU (530 mg, 1.24 mmol) and DIPEA (240 µL, 1.38 mmol) and the mixture was stirred for 10 minutes at r.t. prior to addition of 2M ethanamine in THF (0.7 mL). The reaction mixture was stirred for a further 30 minutes and was diluted with DCM (20 mL) and washed with water (20 mL) followed by 1 M aqueous hydrochloric acid solution and dried (MgSO₄), filtered and concentrated *in vacuo.* The crude residue was purified by flash column chromatography on silica (gradient elution with 50 - 100% EtOAc/heptanes) to afford the 5-isomer *title compound intermediate 45* (100 mg, 20%, ^{~}65% purity) as a sticky dark brown oil and the 7-isomer *title compound intermediate 46* (150 mg, 45%) as a brown solid. 5-Isomer *intermediate 45:* LCMS [M+H]⁺ 293.00, RT 1.04 minutes (Method 11). 7-Isomer *intermediate 46:* δ_{H} (500 MHz, DMSO-*d*₆) 11.09 (s, 1H), 7.91 (t, *J* 5.5 Hz, 1H), 6.29 (s, 1H), 3.47 (t, *J* 6.7 Hz, 1H), 3.16 - 3.00 (m, 2H), 2.44 (t, *J* 5.5 Hz, 2H), 1.97 - 1.83 (m, 3H), 1.74 - 1.67 (m, 1H), 1.63 - 1.51 (m, 1H), 1.03 (t, *J* 7.2 Hz, 3H), 0.82 - 0.76 (m, 4H).

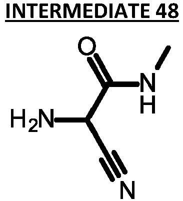

### 2-amino-2-cyano-N-methylacetamide

To a stirred solution of *Intermediate 131* (77%, 691 mg, 4.15 mmol) in TBME (10 mL) cooled in an ice bath was added 2M CH₃NH₂ in MeOH (7 mL). The reaction was stirred while warming to room temperature for 1.5 hours to give a yellow solution. The reaction was concentrated *in vacuo,* the orange residue was sonicated in ethanol: diethyl ether (1:9) to give an orange solid which was collected by vacuum filtration and washed with ethanol: diethyl ether (1:9) to afford the *title compound* (395 mg, 76% at 90% purity) as an orange solid. δ_{H} (250 MHz, DMSO-*d*₆) δ 8.09 (s, 1H), 4.47 (s, 1H), 2.81 (br s, 2H), 2.65 (d, J 4.7 Hz, 3H).

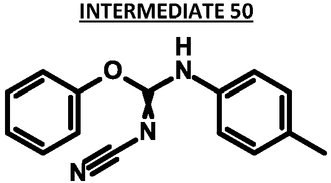

### 3-Cyano-2-phenyl-1-(p-tolyl)isourea

4-Methylaniline [106-49-0] (40 mg, 0.37 mmol) was dissolved in DCM (2 mL) and diphenyl cyanocarbonimidate [79463-77-7] (80.0 mg, 0.33 mmol) was added and the reaction mixture was stirred at r.t. for 6 h. The mixture was concentrated *in vacuo* and the *title compound* (94 mg, 84%) was used without further purification. LCMS [M+H]⁺ 251.95, RT 1.30 minutes, purity 72% *(Method* 6).

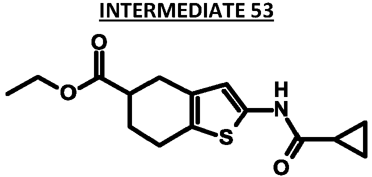

### Ethyl 2-(cyclopropanecarbonylamino)-4,5,6,7-tetrahydrobenzothiophene-5-carboxylate

*Intermediate 43* (4 g, 11.9 mmol) was split into 2 equal portions and each was suspended in quinoline (15 mL) and copper powder (555 mg, 8.73 mmol) was added. The mixtures were heated to 150 °C for 1 h in the microwave. The reaction mixtures were combined and diluted with DCM (250 mL) and washed with 1M aqueous hydrogen chloride solution (2 x 250 mL), followed by saturated aqueous sodium hydrogen carbonate solution (100 mL), dried (MgSO₄), filtered and concentrated *in vacuo.* The crude product was triturated with EtOAc and filtered afford the *title compound* (3.07 g, 88%) as a grey solid. δ_{H} (500 MHz, DMSO-*d*₆) 11.10 (s, 1H), 6.32 (s, 1H), 4.17 - 4.01 (m, 2H), 2.81 - 2.56 (m, 5H), 2.14 - 2.06 (m, 1H), 1.82 - 1.66 (m, 2H), 1.20 (t, J 7.1 Hz, 3H), 0.83 - 0.75 (m, 4H). LCMS [M+H]⁺ 294.0, RT 1.26 minutes, 98% purity (*Method 6*)*.*

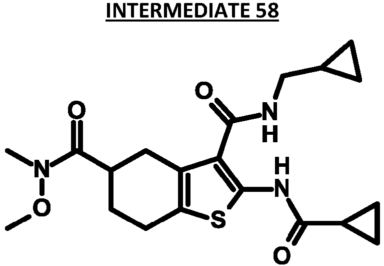

### 2-(Cyclopropanecarbonylamino)-N3-(cyclopropylmethyl)-N5-methoxy-N5-methyl-4,5,6,7-tetrahydrobenzothiophene-3,5-dicarboxamide

*Example 40* (4 g, 11.04 mmol) was suspended in DCM (100 mL) and EDCI (2.12 g, 11.04 mmol), methoxy(methyl)amine (1.35 g, 22.07 mmol), triethylamine (2.23 g, 22.07 mmol) and catalytic DMAP (30 mg) were added to this mixture. The reaction mixture was stirred at r.t. for 16 h and the reaction mixture was concentrated *in vacuo.* The crude residue was dissolved in saturated sodium hydrogen carbonate solution (2 x 10 mL) and extracted with DCM (40 mL). The organic phase was concentrated *in vacuo* and the residue was purified by flash column chromatography on silica (gradient elution with 0-50% DCM /MeOH) to afford the *title compound* (3.0 g, 60%) as a yellow solid. δ_{H} (500 MHz, Methylene Chloride-*d*₂) 12.11 (s, 1H), 6.00 (s, 1H), 3.70 (s, 3H), 3.29 - 3.22 (m, 2H), 3.20 (s, 3H), 2.97 - 2.83 (m, 2H), 2.82 - 2.70 (m, 2H), 2.13-2.08 (m, 1H), 1.89 - 1.76 (m, 1H), 1.65 (tt, J 7.9, 4.6 Hz, 1H), 1.09 - 0.98 (m, 3H), 0.90 - 0.81 (m, 2H), 0.60 - 0.44 (m, 2H), 0.29 - 0.18 (m, 2H). Missing an NH proton. LCMS [M+H]⁺ 406.20, RT 1.78 minutes, 91.76% purity (*Method 12*)*.*

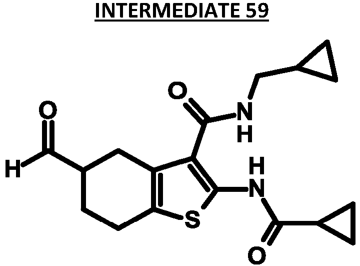

### 2-(Cyclopropanecarbonylamino)-N-(cyclopropylmethyl)-5-formyl-4,5,6,7-tetrahydrobenzothiophene-3-carboxamide

*Intermediate 58* (2.27 g, 5.6 mmol) was dissolved in DCM (70 mL) and the reaction mixture was stirred at r.t. for 5 minutes. 1M Hydrido[bis(2-methylpropyl)]aluminium in hexane (11.2 mL, 11.20 mmol) was added dropwise to the reaction mixture and stirred at -78 °C for 1 h. The reaction mixture was removed from the dry ice bath and quenched by addition of saturated sodium potassium tartrate (100 mL) and the combined organic phases were washed with brine (100 mL), dried (MgSO₄), filtered, and the filtrate was concentrated *in vacuo* to afford the *title compound* (1.46 g, 75%) which was utilised without further purification. LCMS [M+H]+ 347.20, RT 1.79 minutes, 80% purity (*Method 12*)*.*

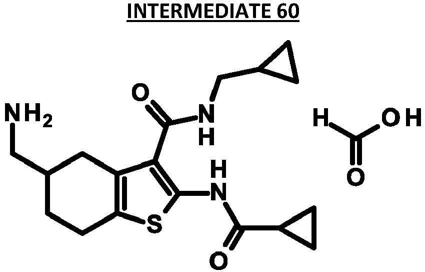

### 5-(Aminomethyl)-2-(cyclopropanecarbonylamino)-N-(cyclopropylmethyl)-4,5,6,7-tetrahydrobenzothiophene-3-carboxamide;formic acid

*Example 59* (300 mg, 0.83 mmol) was dissolved in MeOH (6 mL) and water (3 mL) and formic acid [64-18-6] (2.94 ml, 78.0 mmol) was added. The reaction mixture was cooled to 0 °C and zinc [7440-66-6] (109 mg, 1.66 mmol) was added. The reaction mixture was stirred at 0 °C and warmed to r.t. and stirred for 36 h and the reaction mixture was filtered, and the solid was washed with MeOH. The filtrate was concentrated *in vacuo* to give the desired product which was purified by flash column chromatography on silica (gradient elution with 0-60 % MeOH in DCM) to afford the *title compound* (300 mg, 92%) as a white solid. δ_{H} (250 MHz, Methylene Chloride-*d*₂) 12.07 (s, 1H), 6.46 (t, J 7.5 Hz, 1H), 3.36-3.33 (m, 1H), 3.21 - 3.08 (m, 1H), 3.07 - 2.85 (m, 3H), 2.73-2.68 (m, 2H), 2.57 - 2.40 (m, 1H), 2.18-2.15 (m, 1H), 2.02-1.96 (m, 1H), 1.71 - 1.46 (m, 2H), 1.14 - 0.96 (m, 3H), 0.90-0.86 (m, 2H), 0.49-0.45 (m, 2H), 0.29-0.23 (m, 2H), as a formate salt (1 equivalent).

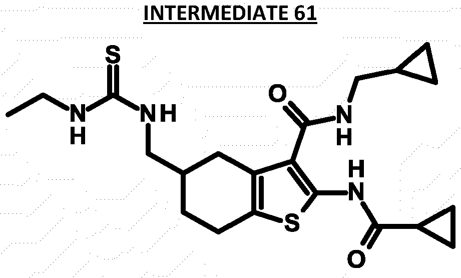

### 2-(Cyclopropanecarbonylamino)-N-(cyclopropylmethyl)-5-[(ethylcarbamothioylamino)methyl]-4,5,6,7-tetrahydrobenzothiophene-3-carboxamide

A solution of isothiocyanatoethane [542-85-8] (160.5 mg, 1.84 mmol) in DCM (5 mL) was stirred at r.t. prior to the addition of *intermediate 60* (320 mg, 0.92 mmol). Triethylamine (128 µl, 0.92 mmol) was added to the reaction mixture and the solution was stirred for 18 h under nitrogen. The reaction mixture was concentrated *in vacuo* to give a pale yellow solid which was purified by flash column chromatography on silica (gradient elution with 0% to 100% EtOAc in heptane) to afford the *title compound* (369 mg, 90%) as a white solid. δ_{H} (250 MHz, Methylene Chloride-*d*₂) 12.16 (s, 1H), 6.07 (s, 1H), 5.80 (br s, 2H), 3.73-3.70 (m, 1H), 3.64 - 3.49 (m, 1H), 3.48 - 3.32 (m, 2H), 3.26 (dd, *J* 7.0, 5.5 Hz, 2H), 2.90 (dd, *J* 15.0, 4.9 Hz, 1H), 2.75-2.70 (m, 2H), 2.61 - 2.43 (m, 1H), 2.13-2.08 (m, 1H), 2.03-1.97 (m, 1H), 1.69-1.66 (m, 1H), 1.22 (t, *J* 7.5 Hz , 3H), 1.11 - 0.96 (m, 3H), 0.91 - 0.81 (m, 2H), 0.63 - 0.50 (m, 2H), 0.30-0.25 (m, 2H); 1H obscured with solvent.

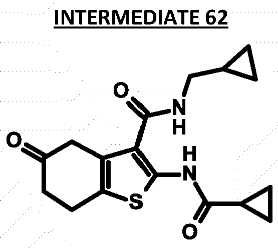

### 2-(Cyclopropanecarbonylamino)-N-(cyclopropylmethyl)-5-oxo-6,7-dihydro-4H-benzothiophene-3-carboxamide

*Example 86* (420 mg, 1.12 mmol) was dissolved in THF and 1M aqueous hydrochloric acid solution (7.81 mL) was added. The reaction mixture was heated at 70 °C for 3 h and then stirred at r.t. for 18 h. The reaction mixture was heated for a further 1 h and the solution was cooled to r.t. and the pH adjusted to pH 9 with saturated sodium hydrogen carbonate solution. The mixture was extracted with EtOAc (2 x 15 mL) and the combined organic phases were washed with brine (15 mL), dried (Na₂SO₄), filtered and the concentrated *in vacuo* to give a yellow solid which was purified by flash column chromatography on silica (gradient elution with 0-50% EtOAc/iso-hexane) to afford the *title compound* (264 mg, 71%) as an off white solid. LCMS [M+H]⁺ 333.2, RT 1.630 minutes, 92.8% purity (*Method 1*)*.*

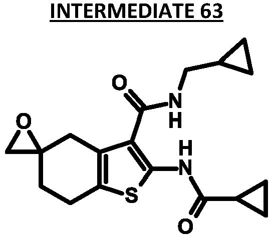

### 2-(Cyclopropanecarbonylamino)-N-(cyclopropylmethyl)spiro[6,7-dihydro-4H-benzothiophene-5,2'-oxirane]-3-carboxamide

Trimethylsulfoxonium iodide [1774-47-6] (2.00 g, 9.09 mmol) was poured into DMSO (13 mL) and cooled to 0 °C. Sodium hydride [7646-69-7] (650 mg, 16.3 mmol) was added and the reaction mixture was stirred at r.t. After 1.5 h, *intermediate 62* (1.95 g, 5.87 mmol) and tetrabutylammonium bromide [1643-19-2] (200 mg, 0.620 mmol) was added and the reaction mixture was stirred for 1 h. The reaction mixture was poured into water and ice (50 g) and was extracted with EtOAc (3 x 50 mL). The combined organic phases were dried (MgSO₄), filtered and concentrated *in vacuo* to afford a yellow solid which was purified by flash column chromatography on silica (gradient elution with 100% DCM to 95/5/0.5% DCM/MeOH/NH₄OH) to afford the *title compound* (1.73 g, 85%) as a yellow solid. LCMS [M+H]⁺ 347.11, RT 2.31 minutes, purity 96.42% (*Method 9*)*.*

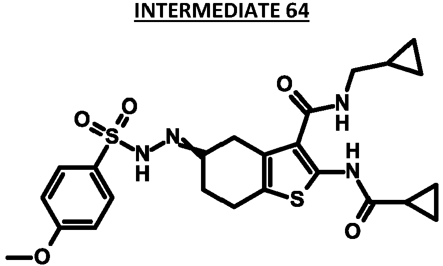

### 2-(cyclopropanecarbonylamino)-N-(cyclopropylmethyl)-5-[(4-methoxyphenyl)sulfonylhydrazono]-6,7-dihydro-4H-benzothiophene-3-carboxamide

*Intermediate 62* (200 mg, 0.6 mmol) was added to a solution of 4-methoxybenzenesulfonohydrazide [1950-68-1] (122 mg, 0.6 mmol) in MeOH (1.2 mL) and the suspension was stirred under nitrogen for 60 h at 20 °C. The reaction mixture was diluted with MeOH (10 mL) and the solids collected by filtration. The solids were washed with MeOH (2 x 20 mL) and dried in *vacuo* at 40 °C for 2 h to afford the *title compound* (146 mg, 44%) as a pale yellow powder. δ_{H} (500 MHz, DMSO-*d*₆) 10.96 (s, 1H), 10.16 (s, 1H), 7.90 (t, *J* 5.5 Hz, 1H), 7.83 - 7.76 (m, 2H), 7.14 - 7.08 (m, 2H), 3.83 (s, 3H), 3.54 (s, 2H), 3.17 (t, *J* 6.2 Hz, 2H), 2.73 (t, *J* 6.3 Hz, 2H), 2.49 - 2.44 (obs. m, 2H), 1.97 - 1.86 (m, 1H), 1.15 - 1.02 (m, 1H), 0.91 - 0.75 (m, 4H), 0.52 - 0.40 (m, 2H), 0.32 - 0.21 (m, 2H). LCMS [M+H]⁺ 517.2, RT 1.87 minutes, 95% purity *(Method 12).*

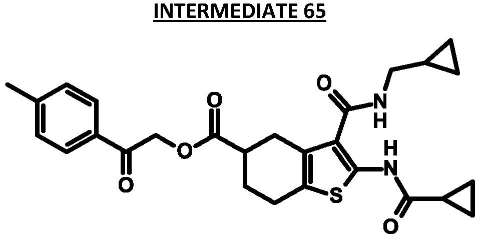

### [2-Oxo-2-(p-tolyl)ethyl] 2-(cyclopropanecarbonylamino)-3-(cyclopropylmethylcarbamoyl)-4,5,6,7-tetrahydrobenzothiophene-5-carboxylate

To a stirred solution of *Example 40* (100 mg, 0.28 mmol) in DMF (2 mL) was added potassium carbonate (55 mg, 2.26 mmol). The reaction mixture was stirred for 10 minutes and 2-bromo-1-(4-methylphenyl)ethanone [619-41-0] (65 mg, 0.3 mmol) was added and the mixture was stirred at 40 °C for 1h. The solvent was removed *in vacuo* and the remaining residue was quenched with water (50 mL) and extracted with EtOAc (3 x 50 mL). The organic phases were combined washed with water (3 x 50 mL), brine (50 mL), separated, dried (Na₂SO₄) and filtered and the solvent was removed *in vacuo* to yield the crude *title compound* (120 mg, 62%) as a yellow solid. LCMS [M+H]⁺ 517.10, RT 1.33 minutes, purity 70.3% (*Method 11*)*.*

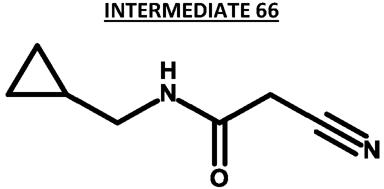

### 2-Cyano-N-(cyclopropylmethyl)acetamide

To cyclopropylmethylamine [2516-47-4] (3.63 g, 50.0 mmol) at 0 °C was added methyl cyanoacetate [105-34-0] (2.50 g, 25.0 mmol) dropwise. The reaction mixture was stirred for 90 minutes, and after 45 minutes a white solid precipitated. 1:1 Et₂O /iso-hexane (40 mL) was added to the reaction mixture which was stirred at 0 °C for 10 minutes. The solid was filtered and washed with a small amount of iso-hexane to afford the *title compound* (3.25 g, 95%) as a pure white solid. δ_{H} (300 MHz, Chloroform-*d*) 6.19 (s, 1H), 3.40 (s, 2H), 3.19 (dd, *J* 7.3, 5.4 Hz, 2H), 1.10 - 0.90 (m, 1H), 0.66 - 0.52 (m, 2H), 0.26 (dt, *J* 6.2, 4.7 Hz, 2H).

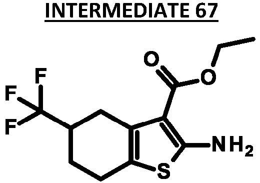

### Ethyl 2-amino-5-(trifluoromethyl)-4,5,6,7-tetrahydrobenzothiophene-3-carboxylate

To a solution of 3-(trifluoromethyl)cyclohexanone [585-36-4] (0.50 g, 3.0 mmol) in EtOH (3 mL) was added ethyl cyanoacetate [105-56-6] (0.37 g, 3.3 mmol) followed by sulphur [7704-34-9] (0.11 g, 3.4 mmol) and diethylamine (0.33 g, 4.5 mmol). The reaction mixture was stirred at r.t. for 1.5 h and the mixture was concentrated *in vacuo* and the crude residue was purified by flash column chromatography on silica (gradient elution with 0-15% EtOAc in hexanes) to afford the *title compound* (717 mg, 81%) as an off-white solid. LCMS [M+H]⁺ 294.0, RT 1.532 minutes (*Method 4*)*.*

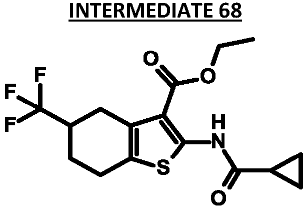

### Ethyl 2-(cyclopropanecarbonylamino)-5-(trifluoromethyl)-4,5,6,7-tetrahydrobenzothiophene-3-carboxylate

*Intermediate 67* (717 mg, 2.45 mmol) was dissolved in DCM (10 mL) and DIPEA (0.75 mL, 4.3 mmol) and cyclopropanecarbonyl chloride [4023-34-1] (0.22 mL, 2.4 mmol) were added. The reaction mixture was stirred at r.t. for ^{~}1 h before washing with saturated sodium hydrogen carbonate solution and brine. The mixture was passed through a phase separation cartridge and the organic layer was concentrated *in vacuo* to yield a yellow gum which was purified by flash column chromatography on silica (gradient elution with 10-50% EtOAc/hexane) to afford the *title compound* (0.75 g, 85%) as a white solid. δ_{H} (400 MHz, DMSO-*d*₆) 11.17 (s, 1H), 4.32 (qd, *J* 7.1, 2.0 Hz, 2H), 3.13 (dd, *J* 16.6, 5.0 Hz, 1H), 2.85 - 2.65 (m, 3H), 2.65 - 2.54 (m, 1H), 2.12 (d, *J* 12.9 Hz, 1H), 2.04 (tt, *J* 7.4, 4.9 Hz, 1H), 1.63 (qd, *J* 11.9, 5.7 Hz, 1H), 1.32 (t, *J* 7.1 Hz, 3H), 1.04 - 0.83 (m, 4H).

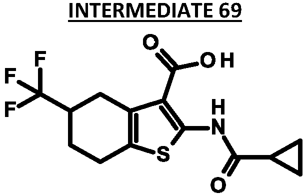

### 2-(Cyclopropanecarbonylamino)-5-(trifluoromethyl)-4,5,6,7-tetrahydrobenzothiophene-3-carboxylic acid

*Intermediate 68* (0.73 g, 2.0 mmol) was dissolved in 1,4-dioxane (7 mL) and a solution of lithium hydroxide monohydrate [1310-66-3] (236 mg, 3.15 mmol, 56 mass%) in water (1.5 mL) was added and the mixture was stirred at 50 °C for ^{~}16 h. The mixture was concentrated *in vacuo* and the residue was dissolved in water and washed with DCM (2 x). The aqueous phase was acidified to <pH 2 by addition of 2M aqueous hydrochloric acid solution and the resulting precipitate was filtered off and washed with water (2 x) to afford the *title compound* (665 mg, 99%) as an off white solid which was air dried. LCMS [M-H]⁻ 332.0, RT 2.374 minutes (*Method 2*)*.*

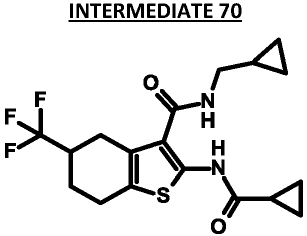

### 2-(Cyclopropanecarbonylamino)-N-(cyclopropylmethyl)-5-(trifluoromethyl)-4,5,6,7-tetrahydrobenzothiophene-3-carboxamide

To *intermediate 69* (100 mg, 0.30 mmol) was added cyclopropylmethylamine [2516-47-4] (32 mg, 0.45 mmol) and a suspension of EDCI (71.2 mg, 0.36 mmol) in DCM (1 mL). The reaction mixture was stirred at r.t. overnight before heating at 40 °C for ^{~}1 h. The mixture was diluted with DCM (5 mL), washed with water and passed through a phase separation cartridge before concentrating the filtrate *in vacuo.* The crude product was purified by flash column chromatography on silica (gradient elution with 10-50% EtOAc/hexane) followed by freeze drying from MeCN/water to afford the *title compound* (35 mg, 30%) as a white solid. LCMS [M+H]⁺ 387.8, RT 2.637 minutes (*Method 2*)*.*

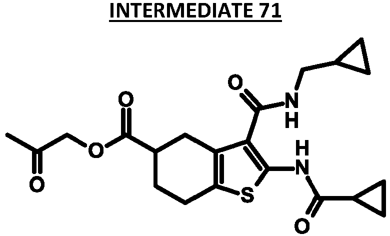

### Acetonyl 2-(cyclopropanecarbonylamino)-3-(cyclopropylmethylcarbamoyl)-4,5,6,7-tetrahydrobenzothiophene-5-carboxylate

*Example* 40 was dissolved in DMF (3 mL) and triethylamine (0.046 ml, 0.33 mmol) and chloroacetone [78-95-5] (0.026 ml, 0.33 mmol) were added. The reaction mixture was stirred at r.t. for 16 h. The reaction mixture was poured into water and the resulting solid was filtered off to afford the *title compound* (80 mg, 63%) which was utilised without further purification. LCMS [M+H]⁺ 419.05, RT 1.38 minutes, purity 91% (*Method 6*)*.*

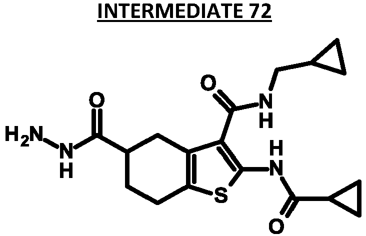

### 2-(Cyclopropanecarbonylamino)-N-(cyclopropylmethyl)-5-(hydrazinecarbonyl)-4,5,6,7-tetrahydrobenzothiophene-3-carboxamide

*Example 40* (100 mg, 0.26 mmol) was dissolved in EtOH (3 mL) and hydrazine hydrate (0.12 ml, 2.56 mmol) was added. The reaction mixture was stirred at 70 °C for 20 h. The mixture was concentrated *in vacuo* to afford the *title compound* (95 mg, 99%) as a white solid. LCMS [M+H]⁺ 377.05, RT 1.13 minutes, purity 100% (*Method 6*)*.*

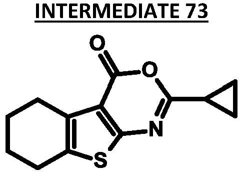

### 2-Cyclopropyl-5,6,7,8-tetrahydrobenzothiopheno[2,3-d][1,3]oxazin-4-one

To *intermediate 16* (200 mg, 0.75 mmol), DCM (4 mL) and DMF (0.01 mL) was added oxalyl chloride [79-37-8] (105 mg, 0.83 mmol). The reaction mixture was stirred at r.t. for 3 h before concentrating *in vacuo* to yield the crude product which was purified by LCMS reverse phase (basic mode) to afford the *title compound* (200 mg, 107%) as a beige solid. δ_{H} (400 MHz, DMSO-*d*₆) 2.75 (dt, J 11.2, 6.0 Hz, 4H), 2.05 - 1.95 (m, 1H), 1.87 - 1.70 (m, 4H), 1.17 - 1.07 (m, 4H).

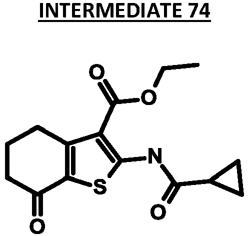

### Ethyl 2-(cyclopropanecarbonylamino)-7-oxo-5,6-dihydro-4H-benzothiophene-3-carboxylate

To ethyl 2-amino-7-oxo-4,5,6,7-tetrahydro-1-benzothiophene-3-carboxylate [96334-44-0] (506 mg, 2.12 mmol) was added DCM (15 mL) and DIPEA (1.1 mL, 6.3 mmol). The reaction mixture was stirred under nitrogen prior to the addition of cyclopropanecarbonyl chloride [4023-34-1] (0.21 mL, 2.30 mmol). The reaction mixture was stirred under nitrogen at r.t. overnight. Further DCM (24 mL) was added to solubilise the mixture, followed by a further cyclopropanecarbonyl chloride [4023-34-1] (0.21 mL, 2.30 mmol) and the reaction mixture was stirred for 1 h before adding further cyclopropanecarbonyl chloride [4023-34-1] (0.21 mL, 2.30 mmol), the mixture was left to stir under nitrogen overnight. Further cyclopropanecarbonyl chloride [4023-34-1] (0.21 mL, 2.30 mmol) was added and the reaction mixture was stirred for 2 h before addition of further cyclopropanecarbonyl chloride [4023-34-1] (0.85 mL, 9.19 mmol) and the reaction mixture was stirred at r.t. for 2 h. Water (25 mL) was added to the reaction mixture, which was stirred vigorously for 10 minutes. Further water (25 mL) and DCM (25 mL) was added and the organic layer was separated before washing with brine (50 mL). The organic layer was separated, passed through a phase separation frit and was concentrated *in vacuo* to yield a dark brown oil which was purified by flash column chromatography on silica (gradient elution with 20% EtOAc/isohexane to 95% EtOAc/isohexane) to afford the *title compound* (253 mg, 39%) as a pale yellow solid. LCMS [M+H]⁺ 308.8, RT 2.162 minutes, 100.0% purity (*Method 2*)*.* LCMS [M+H]⁺ 308.8, RT 2.205 minutes, 100.0% purity (*Method 3*)*.*

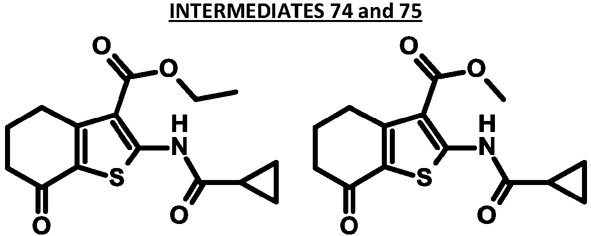

### Intermediate 74

### Ethyl 2-(cyclopropanecarbonylamino)-7-oxo-5,6-dihydro-4H-benzothiophene-3-carboxylate

### Intermediate 75

### Methyl 2-(cyclopropanecarbonylamino)-7-oxo-5,6-dihydro-4H-benzothiophene-3-carboxylate

To a stirred solution of cyclopropanecarboxylic acid [1759-53-1] (1.00 g, 11.6 mmol) in DCM (3 mL) was added oxalyl chloride [79-37-8] (1.50 g, 11.61 mmol) followed by DMF (1 drop). The reaction immediately evolved gas and was stirred at r.t. for 1 h to form cyclopropane carbonyl chloride [4023-34-1]. A mixture of ethyl 2-amino-7-oxo-5,6-dihydro-4H-benzothiophene-3-carboxylate and methyl 2-amino-7-oxo-5,6-dihydro-4H-benzothiophene-3-carboxylate [96334-44-0] *(commercially available ASINEX compound 73% ethyl and 27% methyl)* (Ratio 0.73 : 0.27, 2.736 g) was dissolved with heating in 1,4-dioxane (40 mL) and triethylamine (2.38 g, 23.2 mmol) was added. On cooling the amino ester crashes out of solution and the crude cyclopropane carbonyl chloride solution was added dropwise over 1 minute to this stirred, ^{~}30 °C solution. After 2 h at r.t., further commercial cyclopropane carbonyl chloride [4023-34-1] (364 mg, 3.48 mmol) was added and the mixture was stirred for 1 h at r.t. The mixture was concentrated *in vacuo* and the residues suspended in EtOAc, washed with water (2 x), 0.5 M aqueous hydrochloric acid solution, saturated sodium hydrogen carbonate solution (2 x) and water (2 x). The organic layer was separated, dried (MgSO₄), filtered and the mixture concentrated *in vacuo* to yield a crude mixture of *title products intermediate 74 and intermediate* 75 (2.84 g, 59%) as a yellow solid in ^{~} 3:1 ratio. *200mg was separated by prep HPLC to give 45 mg and 15 mg of intermediates 74 & 75 respectively. Intermediate 74:* LCMS [M+H]⁺ 308.8, RT 2.162 minutes, 100.0% purity (*Method 2*)*.* LCMS [M+H]⁺ 308.8, RT 2.205 minutes, 100.0% purity (*Method 3*)*. Intermediate 75:* LCMS [M+H]⁺ 294.6, RT 1.940 minutes, 100.0% purity (*Method 2*)*.* LCMS [M+H]⁺ 294.6, RT 1.868 minutes, 100.0% purity (*Method 3*)*.*

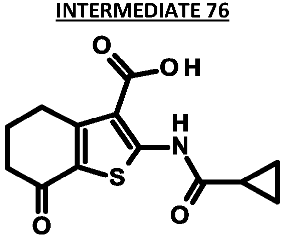

### 2-(Cyclopropanecarbonylamino)-7-oxo-5,6-dihydro-4H-benzothiophene-3-carboxylic acid

A mix of *Intermediates 74 and 75* (3:1 ratio) (2.63 g, 8.66 mmol) were dissolved with heating in 1,4-dioxane (40 mL). Lithium hydroxide monohydrate [1310-66-3] (0.55 g, 13.0 mmol) was dissolved with heating in water (3 mL) and this solution was added to the stirred solution of *intermediates 74 and 75.* The stirred solution was heated at 60 °C for 2 h followed by 18 h at r.t. The reaction mixture was heated at 70 °C for 2 h followed by 40 °C for 5 h. Further lithium hydroxide monohydrate [1310-66-3] (0.18 g, 4.33 mmol, 0.5 eq) dissolved in water (1 mL) was added and the solution stirred for 3 days at r.t. The reaction was heated for 4 h at 70 °C before removing the 1,4-dioxane under vacuum. The mixture was diluted with dilute sodium carbonate solution and the suspension was partitioned with EtOAc. The aqueous phase was separated and the organic phase extracted with dilute sodium carbonate solution and the aqueous extracts combined. 2M Aqueous hydrochloric acid solution was added to the aqueous extracts until the acid precipitated out of solution. The resulting suspension was filtered and the solids washed with 0.2 M aqueous hydrochloric acid solution and dried under vacuum to afford the *title compound* (890 mg, 37%) as a beige solid. δ_{H} (400 MHz, DMSO-*d*₆) 13.68 (s, 1H), 11.81 (s, 1H), 3.05 (t, J 6.0 Hz, 2H), 2.49 - 2.45 (m, 2H), 2.19 - 1.98 (m, 3H), 1.02 - 0.91 (m, 4H). LCMS [M+H]⁺ 280.7, RT 1.452 minutes, 96.8% purity (*Method 2*)*.* LCMS [M+H]⁺ 280.6, RT 0.600 minutes, 96.9% purity (*Method 3*)*.*

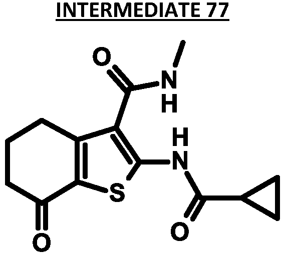

### 2-(Cyclopropanecarbonylamino)-N-methyl-7-oxo-5,6-dihydro-4H-benzothiophene-3-carboxamide

To a suspension of *intermediate 76* (320 mg, 1.15 mmol) in DCM (12 mL) was added 1-hydroxybenzotriazole hydrate [123333-53-9] (263 mg, 1.72 mmol) followed by EDCI (340 mg, 1.72 mmol). After stirring for 10 minutes 2M methylamine in THF (773 mg, 1.72 mmol, 0.86 mL) was added and the reaction stirred at r.t. overnight. The reaction mixture was concentrated *in vacuo* and the crude residue purified by flash column chromatography on silica (gradient elution with 0 - 100% EtOAc in hexane) to afford the *title compound* (250 mg, 75%) as a yellow solid. LCMS [M+H]⁺ 293.0, RT 1.092 minutes, 94.0% purity (*Method 4).*

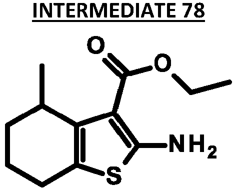

### Ethyl 2-amino-4-methyl-4,5,6,7-tetrahydrobenzothiophene-3-carboxylate

To a solution of 2-methylcyclohexanone [583-60-8] (1 g, 8.83 mmol) in EtOH (4 mL) was added ethyl cyanoacetate [105-56-6] (1.10 g, 9.71 mmol), sulphur [7704-34-9] (0.31 g, 9.71 mmol) and diethylamine [109-89-7] (0.973 g, 13.24 mmol). The reaction mixture was stirred at r.t. for 18 h, followed by heating at 50 °C for 7 h and then left at r.t. overnight. The solvent was removed *in vacuo* and the residue purified by flash column chromatography on silica (gradient elution with 0 - 10% EtOAc in hexane) to afford the *title compound* (520 mg, 25%) as a yellow solid. δ_{H} (400 MHz, DMSO-*d*₆) 7.23 (s, 2H), 4.27 - 4.07 (m, 2H), 3.22 - 3.10 (m, 1H), 2.47 - 2.32 (m, 2H), 1.87 - 1.62 (m, 3H), 1.61 - 1.47 (m, 1H), 1.26 (t, *J* 7.1 Hz, 3H), 1.09 (d, *J* 6.7 Hz, 3H).

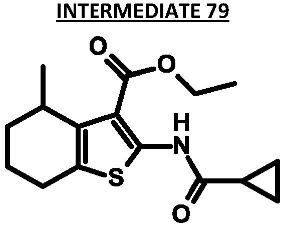

### Ethyl 2-(cyclopropanecarbonylamino)-4-methyl-4,5,6,7-tetrahydrobenzothiophene-3-carboxylate

Cyclopropanecarbonyl chloride [4023-34-1] (0.22 mL, 2.4 mmol) was added to a solution of *intermediate 78* (520 mg, 2.17 mmol) and DIPEA (0.75 mL, 4.3 mmol) in DCM (10 mL). The reaction mixture was stirred for 1.5 h, before diluting with DCM (10 mL) and washing with saturated aqueous sodium hydrogen carbonate solution (2 x 20 mL). The mixture was passed through a phase separator cartridge and the organic phase concentrated *in vacuo.* The crude product was dissolved into DCM and purified by flash chromatography on silica (gradient elution with 10% - 40% EtOAc in iso-hexane) to afford the *title compound* (676 mg, 101%) as a cream coloured solid. δ_{H} (300 MHz, DMSO-*d*₆) 11.23 (s, 1H), 4.22-4.41 (m, 2H), 3.24-3.31 (m, 1H), 2.54-2.68 (m, 2H), 1.94-2.03 (m, 1H), 1.58-1.84 (m, 4H), 1.33 (t, 3H, *J* 7.1 Hz), 1.12 (d, 3H, *J* 6.8 Hz), 0.84-0.96 (m, 4H).

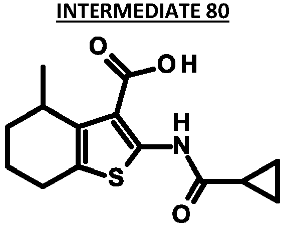

### 2-(Cyclopropanecarbonylamino)-4-methyl-4,5,6,7-tetrahydrobenzothiophene-3-carboxylic acid

Lithium hydroxide monohydrate [1310-66-3] (236 mg, 3.15 mmol) in water (1.5 mL) was added to a solution of *intermediate 79* (646 mg, 2.10 mmol) in 1,4-dioxane (7 mL). The reaction mixture was heated to 70 °C and stirred for 6.5 h, before cooling to r.t. and stirring overnight. The reaction mixture was heated to 70 °C and stirred for a further 6 h before cooling to r.t. overnight. Another portion of lithium hydroxide monohydrate [1310-66-3] (80 mg, 1.91 mmol) in water (0.5 mL) was added and the reaction mixture was heated to 70 °C for 6 h. The reaction mixture was cooled to r.t. and concentrated *in vacuo* before diluting with water (30 mL) and washing with DCM (2 x 20 mL). The aqueous phase was acidified to ~pH 5 with 2M aqueous hydrochloric acid solution. The cream solid formed was filtered off and washed with water followed by iso-hexane and was dried under vacuum to afford the *title compound* (454 mg, 77%) as a cream coloured solid. δ_{H} (400 MHz, DMSO-*d*₆) 13.12 (s, 1H), 11.60 (s, 1H), 2.51-2.65 (m, 2H), 1.88-1.95 (m, 1H), 1.66-1.82 (m, 4H), 1.58-1.63 (m, 1H), 1.12 (d, 3H, J 6.8 Hz), 0.85-0.93 (m, 4H).

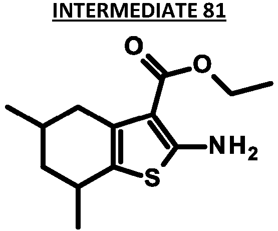

### Ethyl 2-amino-5,7-dimethyl-4,5,6,7-tetrahydrobenzothiophene-3-carboxylate

Morpholine [110-91-8] (1.04 mL, 11.7 mmol) was added to a suspension of 3,5-dimethylcyclohexanone [2320-30-1] (1.12 mL, 7.76 mmol), sulfur [7704-34-9] (274 mg, 8.55 mmol) and ethyl cyanoacetate [105-56-6] (0.91 mL, 8.5 mmol) in EtOH (30 mL). The reaction was stirred at r.t. overnight. The reaction mixture was heated to 50 °C overnight followed by a further 6 h at 50 °C. The reaction mixture was cooled to r.t. and stirred overnight. The mixture was concentrated in *vacuo,* diluted with EtOAc (20 mL) and washed with brine (2 x 20 mL), dried (MgSO₄), filtered and concentrated *in vacuo.* The crude product was purified by flash column chromatography on silica (gradient elution with 1% - 10% EtOAc in iso-hexane) to afford the *title compound* (1.23 g, 63%) as a cream solid. δ_{H} (400 MHz, DMSO-*d*₆) 7.18 (s, 2H), 4.15 (q, 2H, *J* 7.1 Hz), 2.84 (dd, 1H, *J* 17.2, 5.0 Hz), 2.64-2.74 (m, 1H), 2.00 (ddd, 1H, *J* 17.4, 11.2, 3.3 Hz), 1.80-1.85 (m, 1H), 1.64-1.77 (m, 1H), 1.24 (t, 3H, *J* 7.1 Hz), 1.08 (d, 3H, *J* 6.8 Hz), 1.00 (d, 3H, *J* 6.5 Hz), 0.91-0.99 (m, 1H).

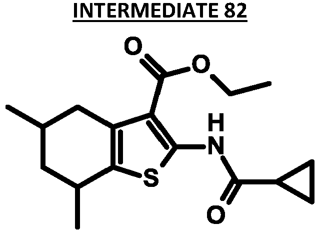

### Ethyl 2-(cyclopropanecarbonylamino)-5,7-dimethyl-4,5,6,7-tetrahydrobenzothiophene-3-carboxylate

Cyclopropanecarbonyl chloride [4023-34-1] (0.49 mL, 5.3 mmol) was added to a solution of *intermediate 81* (1.23 g, 4.85 mmol) and DIPEA (1.7 mL, 9.8 mmol) in DCM (20 mL). The reaction mixture was stirred for 1.5 h, diluted with DCM (10 mL), washed with saturated aqueous sodium hydrogen carbonate solution (20 mL). The mixture was passed through a phase separator cartridge and the organic phase concentrated *in vacuo* to yield the crude product which was purified by flash chromatography on silica (gradient elution with 1% - 10% EtOAc in iso-hexane) to afford the *title compound* (1.43 g, 91%) as a cream coloured solid. δ_{H} (300 MHz, DMSO-*d*₆) 11.17 (s, 1H), 4.30 (q, 2H, *J* 7.1 Hz), 2.94 (dd, 1H, *J* 17.2, 5.0 Hz), 2.77-2.89 (m, 1H), 2.11 (ddd, 1H, *J* 17.1, 11.2, 3.0 Hz), 1.95-2.04 (m, 1H), 1.83-1.90 (m, 1H), 1.69-1.82 (m, 1H), 1.32 (t, 3H, *J* 7.1 Hz), 1.19 (d, 3H, *J* 6.8 Hz), 1.03 (d, 3H, *J* 6.6 Hz), 0.98-1.06 (m, 1H), 0.85-0.96 (m, 4H).

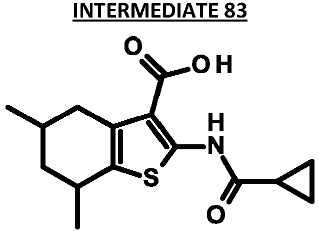

### 2-(Cyclopropanecarbonylamino)-5,7-dimethyl-4,5,6,7-tetrahydrobenzothiophene-3-carboxylic acid

Lithium hydroxide monohydrate [1310-66-3] (500 mg, 6.67 mmol) in water (5 mL) was added to a solution of *intermediate 82* (1.43 g, 4.43 mmol) in 1,4-dioxane (25 mL). The reaction mixture was heated to 70 °C and stirred overnight before cooling to r.t., diluting with water (30 mL) and washing with EtOAc (2 x 20 mL). The aqueous phase was acidified to ^{~}pH 5 with 2M aqueous hydrochloric acid solution. A peach coloured solid formed which was filtered off and washed with water and *iso-*hexane before drying *in vacuo.* The solid obtained was added to the EtOAc washings which were washed with the acidic aqueous phase. The acidic aqueous phase was extracted with EtOAc (20 mL) and the combined organic layers were dried (MgSO₄), filtered and concentrated *in vacuo* to a peach solid (1.40 g) which was partially dissolved in EtOAc and triturated with iso-hexane to give a cream coloured powder, which was washed with iso-hexane and dried. Trituration of the mother liquors with iso-hexane provided further further compound; total *title compound* (1.07 g, 82%). δ_{H} (400 MHz, DMSO-*d*₆) 13.07 (s, 1H), 11.49 (s, 1H), 2.98 (dd, 1H, *J* 17.3, 4.9 Hz), 2.78-2.90 (m, 1H), 2.09 (ddd, 1H, *J* 17.1, 11.3, 2.9 Hz), 1.90-1.99 (m, 1H), 1.83-1.89 (m, 1H), 1.69-1.82 (m, 1H), 1.18 (d, 3H, *J* 6.8 Hz), 0.95-1.04 (m, 1H), 1.02 (d, 3H, *J* 6.6 Hz), 0.85-0.93 (m, 4H).

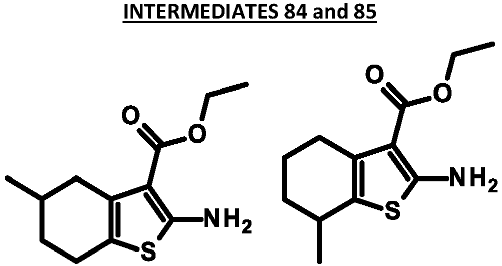

### Intermediate 84

### Ethyl 2-amino-5-methyl-4,5,6,7-tetrahydrobenzothiophene-3-carboxylate

### Intermediate 85

### Ethyl 2-amino-7-methyl-4,5,6,7-tetrahydrobenzothiophene-3-carboxylate

Morpholine [110-91-8] (1.2 mL, 14 mmol) was added to a suspension of 3-methylcyclohexanone [591-24-2] (1.1 mL, 8.7 mmol), sulfur [7704-34-9] (304 mg, 9.48 mmol) and ethyl cyanoacetate [105-56-6] (1.01 mL, 9.47 mmol) in EtOH (30 mL). The reaction was stirred at r.t. for 30 minutes and was left to stir at r.t. overnight before heating at 50 °C for 5 h. The reaction mixture was cooled to r.t. and concentrated *in vacuo,* diluting with EtOAc (20 mL) and washing with brine (2 x 20 mL). The organic phase was dried (MgSO₄), filtered and concentrated *in vacuo.* The crude product was purified by flash column chromatography on silica (gradient elution with 1% - 10% EtOAc in *iso-*hexane) to afford the *title compound* (1.78 g, 86%) as an off-white solid which was a ~6:1 mixture of 2 compounds 5-:7- methyl isomers. Major 5-isomer *intermediate 84:* δ_{H} (300 MHz, DMSO-*d*₆) 7.17 (s, 2H), 4.15 (qd, 2H, *J* 7.1, 0.6 Hz), 2.82 (ddd, 1H, *J* 17.3, 5.0, 0.7 Hz), 2.47-2.42 (m, 2H), 2.07 (ddt, 1H, *J* 17.2, 9.7, 2.2 Hz), 1.84-1.63 (m, 2H), 1.37-1.27 (m, 1H), 1.24 (t, 3H, *J* 7.1Hz), 1.00 (d, 3H, *J* 6.6 Hz). Minor 7-isomer *intermediate 85*: δ_{H} (300 MHz, DMSO-*d*₆) 7.19 (s, 2H), 4.14 (q, 2H, *J* 7.1Hz), 2.70-2.60 (m, 2H), 1.92-1.61 (m, 3H), 1.59-1.50 (m, 1H), 1.24 (t, 3H, *J* 7.1Hz), 1.10 (d, 3H, *J* 6.8 Hz); 1 proton is obscured.

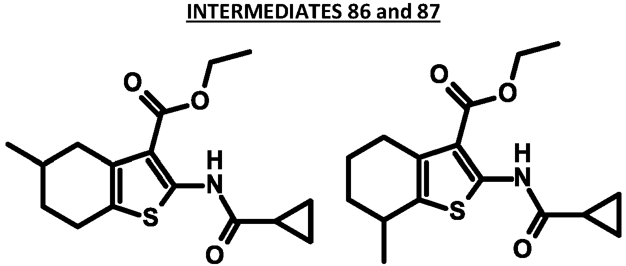

### Intermediate 86

### Ethyl 2-(cyclopropanecarbonylamino)-5-methyl-4,5,6,7-tetrahydrobenzothiophene-3-carboxylate

### Intermediate 87

### Ethyl 2-(cyclopropanecarbonylamino)-7-methyl-4,5,6,7-tetrahydrobenzothiophene-3-carboxylate

Cyclopropanecarbonyl chloride [4023-34-1] (0.76 mL, 8.2 mmol) was added to a solution of a 6: 1 mixture of *intermediate 84* and *intermediate 85* (1.78 g, 7.42 mmol) and DIPEA (2.6 mL, 15 mmol) in DCM (20 mL) and the reaction was stirred for 1.5 h. The reaction mixture was diluted with DCM (10 mL) and washed with saturated aqueous sodium hydrogen carbonate solution (20 mL). The mixture was passed through a phase separator cartridge and the organic phase concentrated *in vacuo.* The crude product was purified by flash column chromatography on silica (gradient elution with 1% - 10% EtOAc in iso-hexane) to give the product which was recrystallized from EtOAc/ iso-hexane to afford the *title compound* (141 mg, 0.459 mmol) as a white solid of 30:1 mixture of 5-:7- methyl isomers. The mother liquor was evaporated, re-dissolved in EtOAc, seeded with the product and allowed to crystallise overnight. The solid was washed with iso-hexane and dried under air to afford further product (0.52 g) as a white solid, ~30:1 mixture of 5-:7- methyl isomers; total 30:1 mixture of *title compounds intermediates 86 and 87* (0.66 g, 29%). The mother liquors were evaporated to a cream solid (1.495 g) to give a ~4:1 mixture of 5-:7- methyl isomer. Major *title compound, intermediate 86:* δ_{H} (400 MHz, DMSO-*d*₆) 11.17 (s, 1H), 4.26-4.34 (m, 2H), 2.92 (dd, 1H, *J* 17.2, 5.1 Hz), 2.54-2.68 (m, 2H), 2.15-2.23 (m, 1H), 1.96-2.03 (m, 1H), 1.70-1.86 (m, 2H), 1.29-1.39 (m, 1H), 1.32 (t, 3H, *J* 7.1 Hz), 1.03 (d, 3H, *J* 6.6 Hz), 0.86-0.94 (m, 4H).

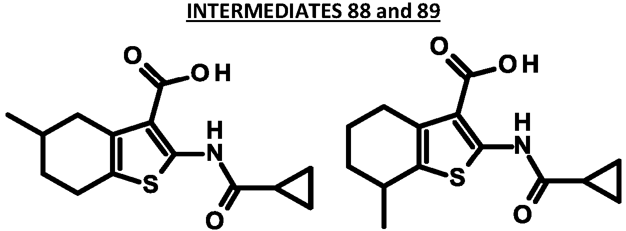

### Intermediate 88

### 2-(Cyclopropanecarbonylamino)-5-methyl-4,5,6,7-tetrahydrobenzothiophene-3-carboxylic acid

### Intermediate 89

### 2-(Cyclopropanecarbonylamino)-7-methyl-4,5,6,7-tetrahydrobenzothiophene-3-carboxylic acid

Lithium hydroxide monohydrate [1310-66-3] (240 mg, 3.20 mmol) in water (2 mL) was added to a solution of 30:1 *intermediate 86* and *intermediate 87* (623 mg, 2.03 mmol) in 1,4-dioxane (12 mL). The reaction mixture was heated to 70 °C and stirred overnight. The reaction was cooled to r.t. and concentrated *in vacuo* to yield the crude product which was treated with water (20 mL) and EtOAc (20 mL). 2M aqueous hydrochloric acid solution was added (5 mL) and the two phases were separated. The aqueous phase was extracted with EtOAc (20 mL) and the combined organic layers were dried (Na₂SO₄), filtered and concentrated *in vacuo.* The solid obtained was partially dissolved in EtOAc and triturated with iso-hexane, to give a terracotta coloured powder which was washed with iso-hexane and dried under a stream of air to afford the *title compound* (469 mg, 82%) as a ~30:1 mixture of *intermediate 88* 5-Me and *intermediate 89* 7-Me product. Major *title compound, intermediate 88:* δ_{H} (400 MHz, DMSO-*d*₆) 13.05 (s, 1H), 11.48 (s, 1H), 2.95 (dd, 1H, *J* 17.3, 5.0 Hz), 2.53-2.67 (m, 2H), 2.17 (dd, 1H, *J* 17.1, 10.0 Hz), 1.89-1.97 (m, 1H), 1.79-1.84 (m, 1H), 1.68-1.78 (m, 1H), 1.27-1.38 (m, 1H), 1.02 (d, 3H, J 6.6 Hz), 0.84-0.93 (m, 4H).

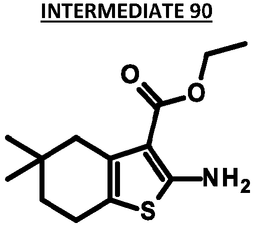

### Ethyl 2-amino-5,5-dimethyl-6,7-dihydro-4H-benzothiophene-3-carboxylate

To a solution of 3,3-dimethylcyclohexan-1-one [2979-19-3] (500 mg, 3.96 mmoL) and ethyl cyanoacetate [105-56-6] (493 mg, 4.36 mmoL) in EtOH (15 mL) was added morpholine [110-91-8] (0.5 mL, 5.9 mmoL) followed by sulphur [7704-34-9] (140 mg, 4.4 mmol) and the resulting suspension was stirred at r.t. under an atmosphere of nitrogen. The reaction mixture was stirred at r.t. for 20 h before heating to 50 °C for 4.5 h. The mixture was concentrated *in vacuo* and the residue was partitioned between EtOAc (15 mL) and water (10 mL). The layers were separated and the aqueous phase was extracted with further EtOAc (2 x 15 mL). The combined organic phases were washed with brine (10 mL), dried (MgSO₄), filtered and concentrated *in vacuo.* The crude material was purified by flash column chromatography on silica (gradient elution with 0 to 50% EtOAc in heptane), followed by further purification by flash column chromatography on silica (gradient elution with 0 to 100% DCM in heptane) to afford the *title compound* (555 mg, 55%) as an off-white solid. δ_{H} (500 MHz, Chloroform-d) 5.89 (s, 2H), 4.27 (q, *J* 7.1 Hz, 2H), 2.61 - 2.39 (m, 4H), 1.54 (t, 2H, partially obscured by water), 1.34 (t, *J* 7.1 Hz, 3H), 0.98 (s, 6H). LCMS [M+H]⁺ 254, RT 1.51 minutes, 100% purity *(Method 6).*

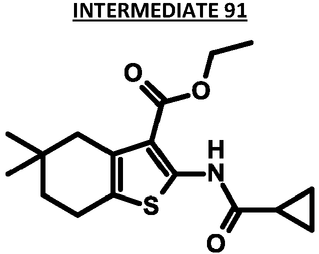

### Ethyl 2-(cyclopropanecarbonylamino)-5,5-dimethyl-6,7-dihydro-4H-benzothiophene-3-carboxylate

To a solution of *intermediate 90* (250 mg, 0.99 mmoL) and triethylamine (0.2 mL, 1.5 mmoL) in DCM (5 mL) cooled to 0 °C was added cyclopropanecarbonyl chloride [4023-34-1] (0.12 mL, 1.28 mmoL) and the solution was stirred under an atmosphere of nitrogen. After stirring for 15 minutes the mixture was diluted with DCM (15 mL) and 1M aqueous hydrochloric acid solution (10 mL) was added. The phases were separated and the aqueous phase was extracted with further DCM (10 mL). The combined organic phases were washed with brine (10 mL), dried (MgSO₄), filtered and concentrated *in vacuo* to afford the *title compound* (400 mg, quantitative, 80% purity) as a light brown solid. δ_{H} (500 MHz, Chloroform-d) 11.46 (s, 1H), 4.34 (q, *J* 7.1 Hz, 2H), 2.64 (t, *J* 6.4 Hz, 2H), 2.55 (s, 2H), 1.69 - 1.61 (m, 1H), 1.59 - 1.50 (m, 2H, partially obscured by water peak), 1.40 (t, *J* 7.1 Hz, 3H), 1.17 - 1.10 (m, 2H), 0.98 (s, 6H), 0.95 - 0.88 (m, 2H). LCMS [M+H]⁺ 322, [M+Na]⁺ 344, RT 1.66 minutes, 100% purity *(Method 6).*

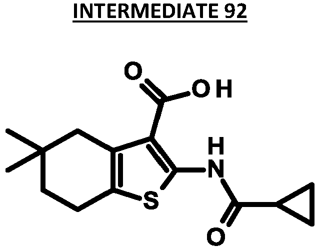

### 2-(Cyclopropanecarbonylamino)-5,5-dimethyl-6,7-dihydro-4H-benzothiophene-3-carboxylic acid

To a suspension of *intermediate 91* (320 mg, 1 mmoL) in 1,4-dioxane (3 mL) and water (3 mL) was added lithium hydroxide monohydrate [1310-66-3] (84 mg, 2 mmoL) and the mixture was heated to reflux. After heating for 2 h the reaction mixture was concentrated *in vacuo* and the residue was diluted with water (2 mL). The solution was acidified with 0.2M aqueous hydrochloric acid solution until pH 4-5 and the off-white precipitate was collected by filtration, washed with water and dried in the vacuum oven at 40 °C overnight to afford the *title compound* (310 mg, quantitative) as an off white solid. δ_{H} (250 MHz, Chloroform-*d*) 11.27 (s, 1H), 2.72 - 2.56 (m, 4H), 1.80 - 1.63 (m, 1H), 1.57 (t, *J* 6.3 Hz, 2H), 1.22 - 1.11 (m, 2H), 1.04 - 0.89 (m, 8H). LCMS [M+H]⁺ 294, RT 1.37 minutes, 98% purity (*Method 6*).

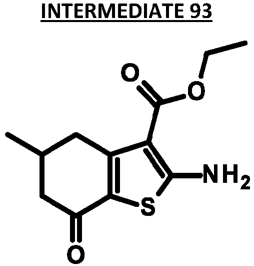

### Ethyl 2-amino-5-methyl-7-oxo-5,6-dihydro-4H-benzothiophene-3-carboxylate

To a solution of 5-methylcyclohexane-1,3-dione [4341-24-6] (2.46 g, 19.5 mmol, 2.66 mL) in EtOH (10 mL) was added ethyl cyanoacetate [105-56-6] (2.42 g, 21.4 mmol), sulphur [7704-34-9] (0.69 g, 21.4 mmol) and triethylamine (2.96 g, 29.2 mmol, 4.07 mL). The reaction was stirred at r.t. for 18 h before heating at reflux for 3 days. The reaction mixture was concentrated *in vacuo* and the residue purified by flash column chromatography on silica (gradient elution with 0 - 30% EtOAc in hexane) to afford the *title compound* (1.28 g, 26%). δ_{H} (400 MHz, DMSO-*d*₆) 8.20 (s, 2H), 4.24 (q, *J* 7.1 Hz, 2H), 3.23 (dd, *J* 17.8, 3.7 Hz, 1H), 2.48 - 2.30 (m, 2H), 2.30 - 2.13 (m, 2H), 1.29 (t, *J* 7.1 Hz, 3H), 1.06 (d, *J* 5.9 Hz, 3H).

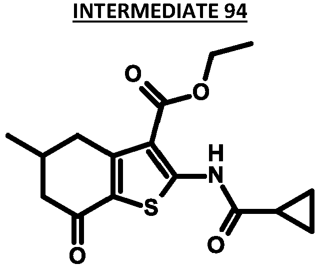

### Ethyl 2-(cyclopropanecarbonylamino)-5-methyl-7-oxo-5,6-dihydro-4H-benzothiophene-3-carboxylate

To a solution of *intermediate 93* (1.28 g, 4.80 mmol) and triethylamine (1 mL, 7.18 mmol) in DCM (15 mL) at 0 °C was added cyclopropanecarbonyl chloride [4023-34-1] (0.6 ml, 6.61 mmol). The reaction mixture was stirred at 0 °C for 4 h, before diluting with DCM (20 mL). 1 M Aqueous hydrochloric acid (20 mL) was added and the layers were separated. The aqueous phase was extracted with further DCM (2 x 10 mL) and washed with water (10 mL). The organic phase was separated, dried (MgSO₄), filtered and concentrated *in vacuo* to afford the *title compound* (1.8 g, 99%, ~85% purity) as a red/brown solid which was used in the next step without further purification. δ_{H} (500 MHz, Chloroform-*d*) 11.73 (s, 1H), 4.41 (q, *J* 7.1 Hz, 2H), 3.41 - 3.28 (m, 1H), 2.63 - 2.57 (m, 1H), 2.54 (dd, *J* 17.7, 10.1 Hz, 1H), 2.41 - 2.32 (m, 1H), 2.27 (dd, *J* 16.0, 12.0 Hz, 1H), 1.70 (tq, *J* 8.8, 4.5 Hz, 1H), 1.43 (t, *J* 7.1 Hz, 3H), 1.24 - 1.18 (m, 2H), 1.16 (d, *J* 6.5 Hz, 3H), 1.06 - 0.96 (m, 2H).

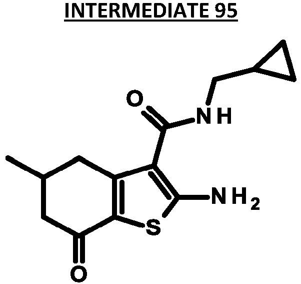

### 2-Amino-N-(cyclopropylmethyl)-5-methyl-7-oxo-5,6-dihydro-4H-benzothiophene-3-carboxamide

*Intermediate 94* (85% purity, 1.8 g, 4.76 mmol) was dissolved in a solution of MeOH (10 mL) and 2M aqueous NaOH (4.5 mL) and was heated to reflux for 4 h. After cooling to r.t., the mixture was left to stand over the weekend, before addition of 4M aqueous NaOH (1.25 mL) and the mixture was heated to reflux for 4 h. The reaction mixture was allowed to cool to r.t. and the MeOH was removed *in vacuo.* The reaction mixture was acidified with 1 M aqueous hydrochloric acid solution and the product was extracted with EtOAc (2 x 20 mL). The combined organic phases were washed with water (10 mL), dried (MgSO₄), filtered and concentrated *in vacuo* to afford *2-amino-5-methyl-7-oxo-5,6-dihydro-4H-benzothiophene-3-carboxylic acid* (1.4 g, 78%) as a dark brown solid which was used directly in the next step without further purification.

EDCI (640 mg, 3.34 mmol) was added to a solution of 1-cyclopropylmethanamine [2516-47-4] (0.29 mL, 3.34 mmol) and *2-amino-5-methyl-7-oxo-5,6-dihydro-4H-benzothiophene-3-carboxylic acid* (500 mg, 2.22 mmol ) in DCM (25 mL). The solution was stirred at r.t. for 16 h, before addition of 1-cyclopropylmethanamine [2516-47-4] (0.29 mL, 3.34 mmol) followed by EDCI (640 mg, 3.34 mmol) and stirring for a further 4 h. The reaction mixture was diluted with DCM and poured into 1 M aqueous hydrochloric acid solution (20 mL). The organic phase was dried (MgSO₄), filtered and concentrated *in vacuo* to give the crude product which was purified by flash column chromatography on silica (gradient elution with 30 - 80% EtOAc/Heptane) to afford the *title compound* (210 mg, 27%, 80% purity) as a bright yellow solid. δ_{H} (500 MHz, Chloroform-*d*) 5.67 (s, 1H), 3.30 - 3.24 (m, 2H), 3.05 (dd, *J* 15.3, 3.9 Hz, 1H), 2.61 (d, *J* 15.4 Hz, 1H), 2.54 - 2.38 (m, 2H), 2.36 - 2.26 (m, 1H), 1.19 (d, *J* 6.4 Hz, 3H), 1.07 (m, 1H), 0.58 (m, 2H), 0.28 (q, *J* 4.9 Hz, 2H).

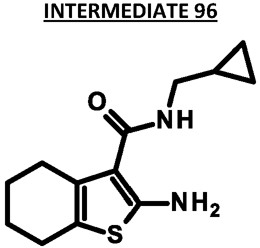

### 2-Amino-N-(cyclopropylmethyl)-4,5,6,7-tetrahydrobenzothiophene-3-carboxamide

To a solution of N1-cyclopropylmethyl-2-cyanoacetamide [114153-25-2] (98%, 660 mg, 4.68 mmol) and cyclohexanone [108-94-1] (0.53 ml, 5.11 mmol) in EtOH (20 mL) was added morpholine [110-91-8] (0.61 ml, 7.05 mmol) and sulphur [7704-34-9] (165 mg, 5.15 mmol). The yellow reaction mixture was stirred over the weekend at r.t., before heating to 60 °C for 4 h and then 50 °C for 16 h. The solvent was removed *in vacuo* and the residue dissolved in EtOAc (30 mL) and washed with water (2 x 10 mL) followed by brine (10 mL). The organic phase was dried (MgSO₄), filtered and concentrated *in vacuo.* The crude residue was purified by flash column chromatography on silica (gradient elution with 30 - 60% EtOAc/heptane) followed by purification by flash column chromatography on silica (gradient elution with 0 - 3% MeOH) to afford the *title compound* (400 mg, 27%) as a yellow solid, the material was used without further purification in the next step. TLC (1:1 EtOAc/heptane) DP Rf=0.34. δ_{H} (500 MHz, DMSO-*d*₆) 6.79 (t, *J* 5.6 Hz, 1H), 6.59 (s, 2H), 3.10 - 3.03 (m, 2H), 2.58 (t, *J* 5.7 Hz, 2H), 2.45 (t, *J* 5.8 Hz, 2H), 1.78 - 1.62 (m, 4H), 1.05 - 0.95 (m, 1H), 0.43 - 0.36 (m, 2H), 0.22 - 0.16 (m, 2H).

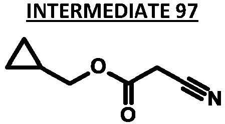

### Cyclopropylmethyl 2-cyanoacetate

Cyanoacetic acid [372-09-8] (1.00 g, 12 mmol) was dissolved in cyclopropanemethanol [2516-33-8] (4.20 g, 58 mmol) and hydrochloric acid (42 mg, 1.2 mmol) was added. The reaction mixture was stirred at r.t. for 16 h. The mixture was heated to 90 °C for ~2 h before cooling to r.t. and concentrating *in vacuo.* The crude product was partitioned between water and EtOAc, the organic phase was washed with saturated sodium hydrogen carbonate and brine. The organic layer was concentrated *in vacuo* to afford the *title compound* (1.58 g, 98%) as a pale yellow oil. δ_{H} (300 MHz, DMSO-*d*₆) 4.03 (s, 2H), 3.96 (d, *J* 7.3 Hz, 2H), 1.16 - 1.02 (m, 1H), 0.58 - 0.49 (m, 2H), 0.34 - 0.25 (m, 2H).

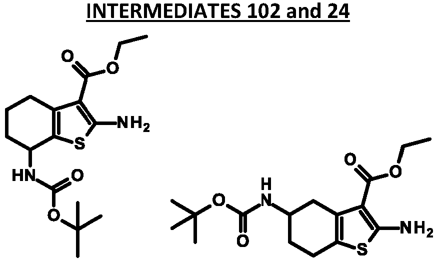

### Intermediate 102

### Ethyl 2-amino-7-(tert-butoxycarbonylamino)-4,5,6,7-tetrahydrobenzothiophene-3-carboxylate

### Intermediate 102a

### Ethyl 2-amino-5-(tert-butoxycarbonylamino)-4,5,6,7-tetrahydrobenzothiophene-3-carboxylate

To a mixture of *tert*-butyl *N*-(3-oxocyclohexyl)carbamate [885280-38-6] (4 g, 18.7 mmol), sulphur [7704-34-9] (0.722 g, 22.5 mmol) and ethyl cyanoacetate [105-56-6] (2.75 g, 24 mmol) in EtOH (40 mL) was added triethylamine (5.22 ml, 37.5 mmol). After stirring at r.t. for 16 h the reaction mixture was concentrated *in vacuo.* To the residue was added water and the mixture was extracted with EtOAc. The combined organic phases were washed with brine, dried (MgSO₄), filtered and concentrated *in vacuo* and the residue was purified by flash column chromatography on silica (gradient elution with 5% to 40% EtOAc in heptane) to afford the *7-isomer title compound intermediate 102* (700 mg, 11%) and the *5-isomer title compound intermediate 102a* (4.5 g, 70%) as white solids. *7-Isomer title compound intermediate 102:* δ_{H} (500 MHz, CD₃OD) 4.29 - 4.16 (m, 2H), 3.75 - 3.65 (m, 1H), 3.10 (dd, *J* 17.0, 5.0 Hz, 1H), 2.66 - 2.51 (m, 2H), 2.44 (dd, *J* 17.1, 9.2 Hz, 1H), 2.05 - 1.93 (m, 1H), 1.73 - 1.63 (m, 1H), 1.46 (s, 9H), 1.32 (t, *J* 7.1 Hz, 3H). LCMS [M+H]⁺ 341.10, [M+Na]⁺ 361.10, RT 1.35 minutes, 100% purity (*Method 6*). *5-Isomer title compound intermediate 102a :* δ_{H} (500 MHz, CD₃OD) 4.58 - 4.48 (m, 1H), 4.22 (q, *J* 7.1 Hz, 2H), 2.76 - 2.58 (m, 2H), 2.01 - 1.92 (m, 1H), 1.92 - 1.80 (m, 1H), 1.77 - 1.64 (m, 2H), 1.46 (s, 9H), 1.32 (t, *J* 7.1 Hz, 3H). LCMS [M+H]⁺ 341, [M+Na]⁺ 363.10, RT 1.38 minutes, 98% purity (*Method 6*).

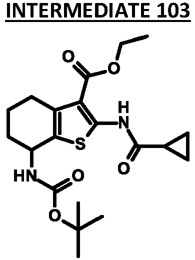

### Ethyl 7-(tert-butoxycarbonylamino)-2-(cyclopropanecarbonylamino)-4,5,6,7-tetrahydrobenzothiophene-3-carboxylate

To a solution of mixture of *intermediate 102* (710 mg, 2.08 mmol) and DIPEA (0.72 ml, 4.17 mol) in DCM (20 mL) at 0 °C was added cyclopropanecarbonyl chloride [4023-34-1] (0.28 mL, 3.12 mmol). The reaction mixture was stirred at r.t. for 2 h before diluting with DCM (30 mL). Water (20 mL) was added and the layers were separated, extracted with DCM (2 x 10 mL) and washed 0.5 M aqueous hydrochloric acid solution (20 mL), saturated sodium hydrogen carbonate solution (20 mL) and brine. The organic phase was separated, dried (MgSO₄), filtered and concentrated *in vacuo* to afford the *title compound* (850 mg, 100%) as a pale yellow solid which was used in the next step without further purification. δ_{H} (500 MHz, CD₃OD) 4.73 - 4.67 (m, 1H), 4.36 (q, *J* 7.1 Hz, 2H), 2.85 - 2.71 (m, 2H), 2.06 - 1.89 (m, 2H), 1.86 - 1.80 (m, 1H), 1.79 - 1.68 (m, 2H), 1.47 (s, 9H), 1.38 (t, *J* 7.1 Hz, 3H), 1.05 - 1.01 (m, 2H), 1.00 - 0.95 (m, 2H). LCMS [M+Na]⁺ 431.10, RT 1.56 minutes, 92% purity (*Method 6*).

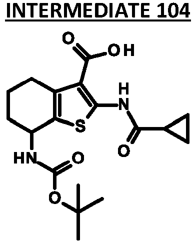

### 7-(tert-Butoxycarbonylamino)-2-(cyclopropanecarbonylamino)-4,5,6,7-tetrahydrobenzothiophene-3-carboxylic acid

*Intermediate 103* (850 mg, 2.08 mmol) was dissolved in 1,4-dioxane (20 mL) and 2M lithium hydroxide monohydrate [1310-66-3] (2.6 mL, 5.2 mmol) was added. The reaction mixture was heated at 90 °C for 2 h. 1,4-Dioxane was removed *in vacuo* and the residue was acidified with 0.5 M aqueous hydrochloric acid solution. The resulting solid was filtered off to afford the *title compound* (710 mg, 90%) which was dried and utilised in the following step without further purification. δ_{H} (500 MHz, CD₃OD) 4.75 - 4.61 (m, 1H), 2.90 - 2.74 (m, 2H), 2.09 - 1.86 (m, 2H), 1.84 - 1.65 (m, 3H), 1.47 (s, 9H), 1.06 - 0.91 (m, 4H). LCMS [M+Na]⁺ 403.05, RT 1.3 minutes, 95% purity (*Method 6*).

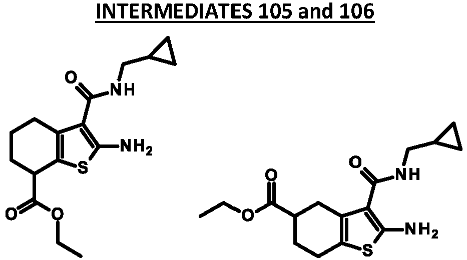

### Intermediate 105

### Ethyl 2-amino-3-(cyclopropylmethylcarbamoyl)-4,5,6,7-tetrahydrobenzothiophene-7-carboxylate

### Intermediate 106

### Ethyl 2-amino-3-(cyclopropylmethylcarbamoyl)-4,5,6,7-tetrahydrobenzothiophene-5-carboxylate

To a mixture of ethyl 3-oxocyclohexanecarboxylate [33668-25-6] (1.50 g, 8.81 mmol), sulphur [7704-34-9] (367 mg, 11.4 mmol) and N1-cyclopropylmethyl-2-cyanoacetamide [114153-25-2] (1.46 g, 10.58 mmol) in EtOH (15 mL) was added triethylamine (2.45 ml, 17.63 mmol). The reaction mixture was stirred at r.t. for 5 h before heating at 60 °C for 24 h. The reaction mixture was concentrated in *vacuo,* water was added and the solution was extracted with EtOAc. The combined organic layers were washed with brine, dried (MgSO₄), filtered and concentrated *in vacuo.* The residue was purified by flash column chromatography on silica (gradient elution with 5% to 70% EtOAc in heptane) to afford the *title compounds* (653 mg, 23%) as a mixture of regioisomers (5-isomer *intermediate 106* and 7-isomer *intermediate 105* in a ratio of 80 : 20). LCMS [M+H]⁺ 323.05, RT 1.25 minutes (*Method 6*). Intermediates 105 and 106 co-run by LCMS, but are both observable by ¹H NMR.

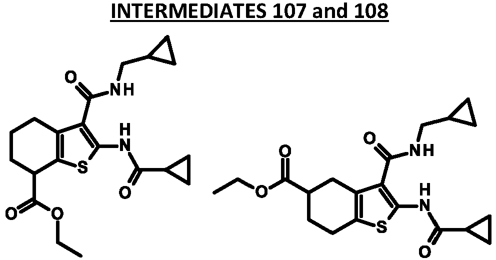

### Intermediate 107

### Ethyl 2-(cyclopropanecarbonylamino)-3-(cyclopropylmethylcarbamoyl)-4,5,6,7-tetrahydrobenzothiophene-7-carboxylate

### Intermediate 108

### Ethyl 2-(cyclopropanecarbonylamino)-3-(cyclopropylmethylcarbamoyl)-4,5,6,7-tetrahydrobenzothiophene-5-carboxylate

To a solution of mixture of *intermediates 105 and 106* (20:80 ratio) (653 mg, 2.02 mmol) and DIPEA (0.71 mL, 4.05 mmol) in DCM (30 mL) at 0 °C was added cyclopropanecarbonyl chloride [4023-34-1] (0.28 mL, 3.08 mmol). The reaction was stirred at r.t. for 2 h before diluting with DCM (50 mL). Water (50 mL) was added and the layers were separated. The aqueous phase was extracted with DCM (2 x 50 mL) and washed with 0.5 M aqueous hydrochloric acid solution (50 mL), saturated sodium hydrogen carbonate solution (50 mL) and brine. The organic layer was separated, dried (MgSO₄), filtered and concentrated *in vacuo* to give a pale yellow solid as a mixture of regio-isomers which was purified by flash column chromatography on silica (gradient elution with 55 to 50% EtOAc in heptane) to afford the *title compounds* as a mixture of 80% *5-isomer intermediate 108,* 20% 7-*isomer intermediates 107* (600 mg, 76%) as a white solid. LCMS [M+H]⁺ 391.0, RT 1.40 minutes, 100% purity (*Method 6*)*.*

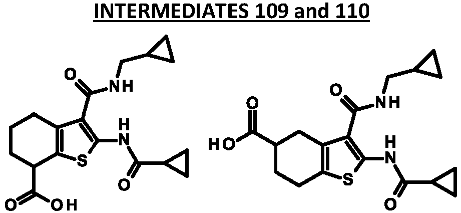

### Intermediate 109

### 2-(Cyclopropanecarbonylamino)-3-(cyclopropylmethylcarbamoyl)-4,5,6,7-tetrahydrobenzothiophene-7-carboxylic acid

### Intermediate 110

### 2-(Cyclopropanecarbonylamino)-3-(cyclopropylmethylcarbamoyl)-4,5,6,7-tetrahydrobenzothiophene-5-carboxylic acid

*Intermediate 107* and *intermediate 108* (20:80 ratio) (600 mg, 1.53 mmol) was dissolved in THF (10 mL) then 2M aqueous lithium hydroxide solution [1310-66-3] (2.3 mL, 4.6 mmol) was added. The reaction mixture was stirred at r.t. for 20 h and the THF was removed *in vacuo.* The residue was acidified with 1M aqueous hydrochloric acid solution and the resulting solid (75% 5-isomer *intermediate 110* and 22 % 7-isomer *intermediate 109*) was filtered off and dried. The mixture was separated by preparative HPLC to afford the *5-isomer title compound intermediate 110* (218 mg, 49%) and the *7-isomer title compound intermediate 109* (58.5 mg, 12%) with 92% purity containing 6% of the *5-isomer title compound intermediate 110. 5-Isomer title compound intermediate 110:* LCMS [M+H]⁺ 363.1, RT 2.78 minutes, 100% purity (*Method 10*). *7-Isomer title compound intermediate 109:* LCMS [M+H]⁺ 363.1, RT 2.90 minutes, 92% purity, 6% 5-regiosisomer present (*Method 10*).

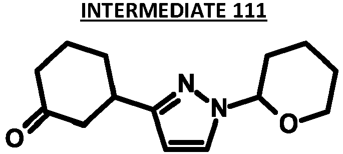

### 3-(1-Tetrahydropyran-2-ylpyrazol-3-yl)cyclohexanone

Hydroxy(cyclooctadiene)rhodium(I) dimer [73468-85-6] (12 mg, 0.025 mmol) and degassed 1,4-dioxane (1.2 mL) was stirred for 15 minutes at r.t. Potassium phosphate tribasic [7778-53-2] (4 mmol, 2 mL) was added and the mixture was stirred for 15 minutes. 2-Cyclohexen-1-one [930-68-7] (1.01 mmol, 100 mg) and 1-(tetrahydro-2H-pyran-2-yl)-3-pyrazole boronic acid mida ester (1.21 mmol, 383 mg) were added and the mixture was stirred at 60 °C overnight. The mixture was warmed at 70 °C overnight. The mixture was concentrated *in vacuo* and the crude product in water was extracted with Et₂O (2 x 3 mL) and DCM (2 x 3 mL). The organic phases were combined and concentrated *in vacuo* to yield the crude product which was purified by preparative TLC (silica plate) (elution with hexane/EtOAc 6/4). Extraction of the compound with EtOAc (100 mL) gave the *title compound* (22 mg, 8.7%) as a yellow oil. GCMS [M+H]⁺ 248.96, RT 14.31 minutes.

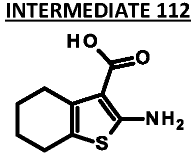

### 2-Amino-4,5,6,7-tetrahydrobenzothiophene-3-carboxylic acid

2M Aqueous NaOH solution (91 mL, 182 mmol) was added to a suspension of 2-amino-4,5,6,7-tetrahydro-benzo[B]thiophene-3-carboxylic acid methyl ester [108354-78-5] (13.1 g, 60.8 mmol) in EtOH (100 mL). The reaction mixture was heated to 60 °C and stirred for 4 h. The temperature was raised to 80 °C (reflux) and the reaction was stirred overnight. The reaction was left stirring at 80 °C for a further 7 h and was then cooled to r.t. and stirred overnight. The reaction mixture was concentrated *in vacuo* to remove the EtOH. The aqueous solution was washed with DCM (3 x 200 mL) and then acidified to pH 2 with concentrated hydrochloric acid. A brown solid was formed which was filtered off, washed with water, then hexane and dried to afford the *title compound* (7.55 g, 63%) as a dark brown solid. δ_{H} (400 MHz, DMSO-*d*₆) 11.8 (br. s, 1H), 7.16 (s, 2H), 2.57 - 2.61 (m, 2H), 2.40 - 2.44 (m, 2H), 1.63 - 1.72 (m, 4H).

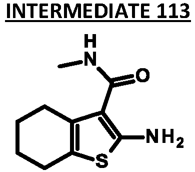

### 2-Amino-N-methyl-4,5,6,7-tetrahydrobenzothiophene-3-carboxamide

EDCI (5.0 g, 26 mmol) was added to a solution of *intermediate 112* (4 g, 20.28 mmol), 1-hydroxybenzotriazole hydrate (3.52 g, 22.3 mmol), DIPEA (14.5 mL, 83.2 mmol) and 2M solution of methylamine in THF (31 mL, 62 mmol) in DMF (100 mL) and the reaction was stirred overnight. The reaction mixture was concentrated *in vacuo* and the residue was partitioned between EtOAc (100 mL) and water (100 mL) and the organic layer was separated. The organic layer was washed with brine (2 x 100 mL), filtered to remove an insoluble dark brown solid, dried (Na₂SO₄), filtered and concentrated *in vacuo.* The crude product was dissolved in DCM and purified by flash column chromatography on silica (gradient elution with 10% - 40% EtOAc in iso-hexane). The product was recrystallised from EtOAc/ *iso*-hexane to afford the *title compound* (1.32 g, 31%) as a brown solid. δ_{H} (300 MHz, DMSO-*d*₆) 6.67 - 6.73 (m, 1H), 6.64 (s, 2H), 2.68 (d, J 4.6 Hz, 3H), 2.53 - 2.58 (m, 2H), 2.42 - 2.47 (m, 2H), 1.62 - 1.74 (m, 4H).

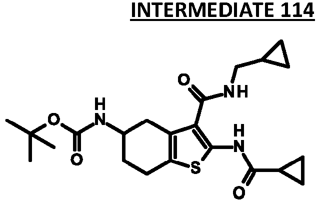

### tert-Butyl N-[2-(cyclopropanecarbonylamino)-3-(cyclopropylmethylcarbamoyl)-4,5,6,7-tetrahydrobenzothiophen-5-yl]carbamate

To a stirred solution of *intermediate 26* (12 g, 31.6 mmol) and cyclopropylmethanamine [2516-47-4] (3.36 g, 47.4 mmol) in DCM (80 mL) was added triethylamine (9.57 g, 94.7 mmol) and EDCI (15.2 g, 78.9 mmol) and the reaction mixture was stirred at r.t. for 20 h. The reaction mixture was quenched with water and extracted with DCM. The organic phase was separated, washed with brine, dried (Na₂SO₄), filtered and concentrated *in vacuo.* The crude product obtained was purified by flash column chromatography on silica (elution with 10% EtOAc/*n*-hexane) to afford the *title compound* (5.5 g, 40%). δ_{H} (400 MHz, Chloroform-*d*) 12.16 (s, 1H), 5.89 (s, 1H), 4.70 (s, 1H), 4.10 (s, 1H), 3.33 - 3.20 (m, 2H), 3.14 (dd, *J* 14.6, 5.0 Hz, 1H), 2.77 (d, *J* 17.3 Hz, 2H), 2.62 - 2.57 (m, 1H), 2.04-2.02 (m, 1H), 1.90-1.84 (m, 1H), 1.73 - 1.60 (m, 1H), 1.48 (s, 9H), 1.46 - 1.36 (m, 1H), 1.04- 0.96 (m, 2H), 0.90-0.89 (m, 2H), 0.61-0.57 (m, 2H), 0.28 - 0.23 (m, 2H).

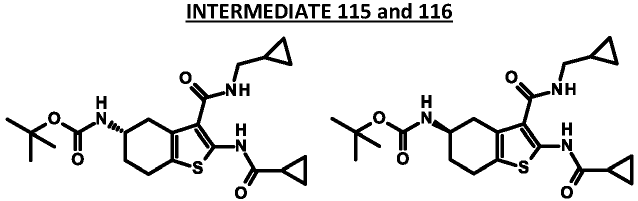

### Intermediate 115

### tert-Butyl N-[(5S)-2-(cyclopropanecarbonylamino)-3-(cyclopropylmethylcarbamoyl)-4,5,6,7-tetrahydrobenzothiophen-5-yl]carbamate

### Intermediate 116

### tert-Butyl N-[(5R)-2-(cyclopropanecarbonylamino)-3-(cyclopropylmethylcarbamoyl)-4,5,6,7-tetrahydrobenzothiophen-5-yl]carbamate

*Intermediate 114* (250 g) was separated by chiral preparative HPLC (Chiralpak AD, 50 x 252 mm, 30°C 360 ml/min, CO₂ + 35% MeOH) to afford the two enantiomers *Intermediate 115* (126 g) and *Intermediate 116* (122 g). *Intermediate 115 contained ~2% impurity of* tert-butyl N-[(5S)-2-(cyclopropanecarbonylamino)-3-(isobutylcarbamoyl)-4,5,6,7-tetrahydrobenzothiophen-5-yl]carbamate resulting from a 2-methylpropan-1-amine impurity in the cyclopropylmethanamine used in the preparation of *Intermediate 114.*

*Intermediate 115* δ_{H} (300 MHz, DMSO-*d*₆) 11.06 (s, 1H), 7.70 (t, *J* 5.6 Hz, 1H), 6.94 (d, *J* 7.5 Hz, 1H), 3.60 (s, 1H), 3.13 (ddq, *J* 19.8, 13.4, 6.6, 6.0 Hz, 2H), 2.88 (dd, *J* 16.1, 5.1 Hz, 1H), 2.79 - 2.58 (m, 2H), 2.48 - 2.39 (m, 1H), 1.98 - 1.82 (m, 2H), 1.80 - 1.57 (m, 1H), 1.39 (s, 9H), 1.14 - 0.93 (m, 1H), 0.90 - 0.70 (m, 4H), 0.48 - 0.34 (m, 2H), 0.30 - 0.15 (m, 2H). LCMS [M+H]⁺ 434.0, RT 2.43 minutes (*Method 2*). LCMS [M+H]⁺ 434.0, RT 2.37 minutes (*Method 3*). *Intermediate 116* LCMS [M+H]⁺ 434.0, RT 2.44 minutes (*Method 2*). LCMS [M+H]⁺ 434.0, RT 2.36 minutes (*Method 3*).

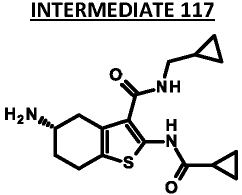

### (5S)-5-Amino-2-(cyclopropanecarbonylamino)-N-(cyclopropylmethyl)-4,5,6,7-tetrahydrobenzothiophene-3-carboxamide

To a stirred solution of *intermediate 115* (20 g, 43.1 mmol) in DCM (100 mL) was added TFA (67 ml, 876 mmol) dropwise over 15 minutes, keeping the temperature below 25 °C. The reaction mixture was stirred at r.t. for a further 15 minutes. Toluene (120 mL) was added and the solvent was removed *in vacuo.* EtOAc (40 mL) was added to the residue followed by diisopropyl ether (1.4 L) leading to the precipitation of the product. The slurry was stirred at r.t. for a further 15 minutes and the precipitate was collected by filtration. The filter cake was rinsed with diisopropyl ether (180 mL) and Et₂O (180 mL) and dried under a flow of nitrogen for 15 minutes to yield the crude product (25.4 g) as a beige solid. The precipitate was dissolved in water (800 mL) containing TFA (4 mL) and Et₂O (200 mL) and EtOAc (20 mL) were added. The aqueous phase was separated and washed with Et₂O (100 mL). Potassium carbonate (10.2 g, 36.9 mmol) was added and the aqueous phase extracted with EtOAc (2 x 400 mL). The aqueous phase was saturated with sodium chloride (250 g) and then further extracted with EtOAc (400 mL). The organic extracts were combined, dried (Na₂SO₄), filtered and the solvent removed *in vacuo* to afford the *title compound* (14.8 g, 93%) as a beige solid. δ_{H} (500 MHz, Chloroform-*d*) 12.07 (s, 1H), 6.01 (t, *J* 5.0 Hz, 1H), 3.34 - 3.23 (m, 3H), 3.04 (dd, *J* 14.5, 4.8 Hz, 1H), 2.86 - 2.57 (m, 4H), 2.53 (dd, *J* 14.5, 8.2 Hz, 1H), 2.08 - 2.01 (m, 1H), 1.79 - 1.69 (m, 1H), 1.69 - 1.62 (m, 1H), 1.12 - 1.03 (m, 3H), 0.91 - 0.85 (m, 2H), 0.61 - 0.50 (m, 2H), 0.32 - 0.21 (m, 2H).

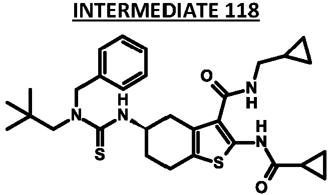

### (5S)-5-[[Benzyl(2,2-dimethylpropyl)carbamothioyl]amino]-2-(cyclopropanecarbonylamino)-N-(cyclopropylmethyl)-4,5,6,7-tetrahydrobenzothiophene-3-carboxamide

To a solution of *intermediate 117* (400 mg, 1.20 mmol) in DCM (20 mL) was added DIPEA (234 mg, 1.80 mmol) and phenyl chlorothionocarbonate [1005-56-7] (230 mg, 1.32 mmol). The reaction mixture was stirred for 2 h. DIPEA (239 mg, 1.84 mmol) and N-benzyl-2,2-dimethyl-propan-1-amine (217 mg, 1.23 mmol) were added and the reaction was stirred for 18 h. Water (10 mL) was added to the reaction mixture and the organic layer was separated and passed through a phase separator column. The organic phase was concentrated *in vacuo* to yield a yellow solid which was purified by flash column chromatography on silica (gradient elution with 0-45% EtOAc/iso-hexane) to afford the *title compound* (539 mg, 80%) as a yellow foam. LCMS [M+H]⁺ 553.0, RT 3.339 minutes, purity ~77% (*Method 1*).

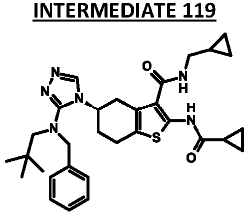

### (5S)-5-[3-[Benzyl(2,2-dimethylpropyl)aminol-1,2,4-triazol-4-yl]-2-(cyclopropanecarbonylamino)-N-(cyclopropylmethyl)-4,5,6,7-tetrahydrobenzothiophene-3-carboxamide

*Intermediate 118* (516 mg, 0.93 mmol) was dissolved in DMF (10 mL) and formic acid hydrazide [624-84-0] (125 mg, 1.87 mmol) was added followed by mercuric chloride [7487-94-7] (507 mg, 1.87 mmol) and finally triethylamine (190 mg, 1.87 mmol). The reaction mixture was stirred for 2 minutes at r.t. and then heated at 60 °C for 3 h. The mixture was cooled and diluted with MeCN (10 mL) and then filtered through a plug of Celite. The Celite pad was washed with excess MeCN (20 mL) and the filtrate was combined and concentrated *in vacuo* to give a yellow oil. The crude material was purified by flash column chromatography on silica (gradient elution with 0-5% MeOH/EtOAc) to afford the *title compound* (150 mg, 29%) as a white solid. LCMS [M+H]⁺ 561.0, RT 2.936 minutes (*Method 1*).

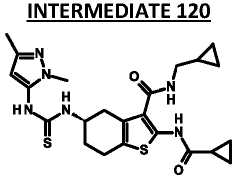

### (5S)-2-(Cyclopropanecarbonylamino)-N-(cyclopropylmethyl)-5-[(2,5-dimethylpyrazol-3-yl)carbamothioylaminol-4,5,6,7-tetrahydrobenzothiophene-3-carboxamide

To a stirred solution of *intermediate 117* (33.7 g, 101 mmol) in DCM (300 mL) was added DIPEA (27 mL, 150 mmol) followed by 5-isothiocyanato-1,3-dimethyl-1H-pyrazole [205246-65-7] (23 g, 150 mmol) in DCM (50 mL) and the reaction mixture was stirred at r.t. for 17 h. The reaction mixture was concentrated *in vacuo* to yield the crude product as a tan solid which was stirred in DCM (100 mL) for 1 h. Et₂O (150 mL) was added and the resulting solid filtered, washed with Et₂O (100 mL x 2) and dried under vacuum to afford the *title compound* (52 g, 100%) as an off-white solid. δ_{H} (300 MHz, DMSO-*d*₆) 11.09 (s, 1H), 9.11 (s, 1H), 7.95 (d, *J* 7.5 Hz, 1H), 7.75 (m, 1H), 5.92 (s, 1H), 4.53 (m, 1H), 3.53 (s, 3H), 3.14 (t, *J* 6.2 Hz, 2H), 3.05 (m, 1H), 2.79 - 2.56 (m, 3H), 2.09 (s, 3H), 2.05 - 1.80 (m, 3H), 1.11 - 0.95 (m, 1H), 0.93 - 0.74 (m, 4H), 0.50 - 0.36 (m, 2H), 0.32 - 0.16 (m, 2H).

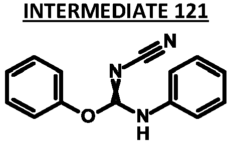

### 3-Cyano-1,2-diphenyl-isourea

To a solution of aniline [62-53-3] (500 mg, 5.37 mmol) in 2-propanol was added diphenyl N-cyanocarbonimidate [79463-77-7] (1.00 g, 6 mmol) and DIPEA (1.0 g, 8 mmol) and the reaction mixture was stirred at r.t. for 3 h. The white solid formed was filtered and washed with hexane, the solid was dried *in vacuo* to afford the *title compound* (1.0 g, 100%) as a white powder. LCMS [M+H]⁺ 238.0, RT 1.313 minutes, 100.0% purity (*Method 4*).

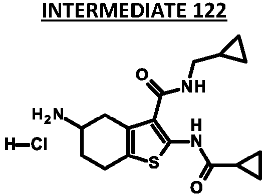

### 5-Amino-2-(cyclopropanecarbonylamino)-N-(cyclopropylmethyl)-4,5,6,7-tetrahydrobenzothiophene-3-carboxamide;hydrochloride

To a solution of *intermediate 114* (90% purity, 1.4 g, 2.91 mmol) in 1,4-dioxane (15 mL) was added 4M hydrogen chloride in 1,4-dioxane (11 mL) and the mixture was stirred for 2 h at r.t. The reaction mixture was concentrated *in vacuo* and the mixture was dissolved in 1,4-dioxane (10 mL). Further 4M hydrogen chloride in 1,4-dioxane (5 mL) was then added. After stirring for 3 h at r.t., the solvent was removed *in vacuo* to afford the *title compound* (1.2 g, 95%, 85% purity) as a beige solid. δ_{H} (500 MHz, CD₃OD) 3.62 - 3.54 (m, 1H), 3.24 (dd, *J* 7.0, 3.0 Hz, 2H), 3.18 - 3.12 (m, 1H), 2.91 - 2.85 (m, 2H), 2.80 - 2.71 (m, 1H), 2.30 - 2.20 (m, 1H), 2.01 - 1.91 (m, 1H), 1.85 - 1.78 (m, 1H), 1.14 - 1.05 (m, 1H), 1.02 - 0.96 (m, 2H), 0.96 - 0.90 (m, 2H), 0.59 - 0.50 (m, 2H), 0.32 - 0.26 (m, 2H). LCMS [M+H]⁺ 334.1, RT 0.88 minutes, 85% purity (*Method 6*).

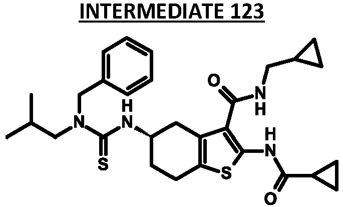

### (5S)-5-[[Benzyl(isobutyl)carbamothioyl]amino]-2-(cyclopropanecarbonylamino)-N-(cyclopropylmethyl)-4,5,6,7-tetrahydrobenzothiophene-3-carboxamide

To a solution of *intermediate 117* (400 mg, 1.20 mmol) in DCM (20 mL) was added DIPEA (234 mg, 1.80 mmol), phenyl chlorothionocarbonate [1005-56-7] (230 mg, 1.32 mmol) and the reaction mixture was stirred for 2 h. DIPEA (238.7 mg, 1.838 mmol) and N-benzyl-2-methyl-propan-1-amine [42882-36-0] (200 mg, 1.225 mmol) were added and the reaction mixture was stirred for 18 h. Water (10 mL) was added to the reaction mixture and the organic layer was separated and passed through a phase separator column. The organic phase was concentrated *in vacuo* to yield the crude material as a yellow solid which was purified by flash column chromatography on silica (gradient elution with 0-100% EtOAc/iso-hexane) to afford the *title compound* (617 mg, 93%) as a yellow solid. LCMS [M+H]⁺ 539.0, RT 3.24 minutes (*Method 1*)*.*

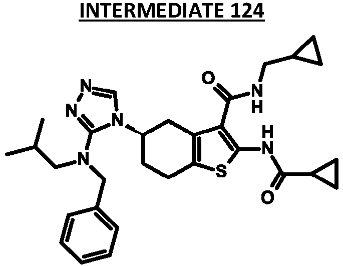

### (5S)-5-[3-[benzyl(isobutyl)amino]-1,2,4-triazol-4-yl]-2-(cyclopropanecarbonylamino)-N-(cyclopropylmethyl)-4,5,6,7-tetrahydrobenzothiophene-3-carboxamide

*Intermediate 123* (616 mg, 1.143 mmol) was dissolved in DMF (10 mL) and formic acid hydrazide [624-84-0] (153 mg, 2.29 mmol) was added followed by mercuric chloride [7487-94-7] (621 mg, 2.29 mmol) and finally triethylamine (233 mg, 2.29 mmol). The reaction mixture was stirred for 2 minutes at r.t. and then heated at 60 °C for 4 h. The reaction mixture was cooled and diluted with MeCN (10 mL) and filtered through Celite, washing with MeCN (20 mL). The filtrate was concentrated *in vacuo* to yield the crude product as a yellow solid which was triturated with EtOAc and filtered. The filtrate was concentrated *in vacuo* to yield a yellow oil which was purified by flash column chromatography on silica (gradient elution with 0-5% MeOH/EtOAc) to afford the *title compound* (270 mg, 43%) as a white solid. LCMS [M+H]⁺ 547.0, RT 2.83 minutes (*Method 1*)*.*

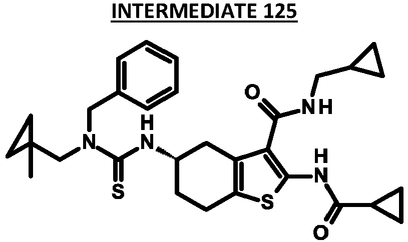

### (5S)-5-[[Benzyl-[(1-methylcyclopropyl)methyl]carbamothioyl]aminol-2-(cyclopropanecarbonylamino)-N-(cyclopropylmethyl)-4,5,6,7-tetrahydrobenzothiophene-3-carboxamide

Phenyl chloromethanethioate [1005-56-7] (0.46 mL, 3.3 mmol) was dissolved in DCM (15 mL) with stirring and cooled to 0 °C. *Intermediate 117* (1 g, 3 mmol) in DCM (15 mL) and triethylamine (1.25 mL, 9 mmol) was added dropwise and the reaction was stirred at 0 °C for 30 minutes. Benzyl[(1-methylcyclopropyl)methyl]amine (97%, 1.01 g, 5.61 mmol) in DCM (10 mL) was added and the reaction was stirred at r.t. for 2 h. The reaction was stirred for 18 h before diluting with water (40 mL) and extracted with DCM (3 x 30 mL). The organic phases were washed with brine, dried (Na₂SO₄), filtered and concentrated *in vacuo* to give a brown oil which was purified by flash column chromatography on silica (gradient elution with 0 - 100% EtOAc in heptane). The fractions containing the product were combined, washed with 0.5 M aqueous citric acid solution (2 x 25 mL) and the organic layer was dried (Na₂SO₄), filtered and concentrated *in vacuo* to afford the *title compound* (1.56 g, 94%) as a white solid. δ_{H} (500 MHz, DMSO-*d*₆) 11.01 (s, 1H), 7.70 (t, *J* 5.6 Hz, 1H), 7.31 (t, *J* 7.4 Hz, 2H), 7.28 - 7.22 (m, 2H), 7.20 (d, *J* 7.3 Hz, 2H), 5.21 - 5.06 (m, 2H), 4.74 - 4.60 (m, 1H), 3.65 - 3.46 (m, 2H), 3.17 (dt, *J* 12.7, 6.1 Hz, 1H), 3.10 (dt, J 13.4, 6.0 Hz, 1H), 3.03 (dd, *J* 15.7, 5.0 Hz, 1H), 2.71 - 2.65 (m, 2H), 2.59 (dd, *J* 15.7, 9.3 Hz, 1H), 2.03 - 1.95 (m, 1H), 1.95 - 1.87 (m, 1H), 1.87 - 1.78 (m, 1H), 1.04 - 0.96 (m, 4H), 0.89 - 0.78 (m, 4H), 0.44 - 0.38 (m, 2H), 0.39 - 0.31 (m, 2H), 0.32 - 0.27 (m, 2H), 0.24 - 0.17 (m, 2H).

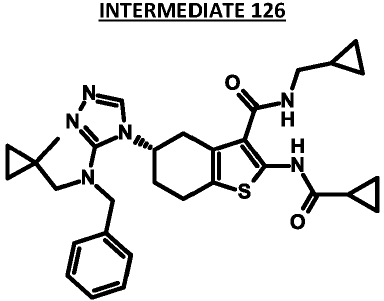

### (5S)-5-[3-[Benzyl-[(1-methylcyclopropyl)methyl]amino]-1,2,4-triazol-4-yl]-2-(cyclopropanecarbonylamino)-N-(cyclopropylmethyl)-4,5,6,7-tetrahydrobenzothiophene-3-carboxamide

*Intermediate 125* (735 mg, 1.33 mmol) was dissolved in DMF (10 mL) and formic hydrazide [624-84-0] (240 mg, 4 mmol) was added followed by mercury dichloride [7487-94-7] (1.09 g, 4 mmol). The reaction was stirred at r.t. for 5 minutes and triethylamine (0.56 mL, 4.00 mmol) was added. The reaction was heated to 90 °C with stirring for 2 h. Further formic hydrazide [624-84-0] (200 mg) was added and the reaction heated at 90 °C for 18 h. The reaction was allowed to cool, diluted with DCM and Kieselguhr added and the mixture was filtered through a plug of Kieselguhr washing through with DCM. The filtrate was concentrated *in vacuo* and the residue was dissolved in EtOAc (100 mL), washed with saturated ammonium chloride solution (40 mL), water (40 mL), saturated ammonium chloride solution (40 mL) and brine. The organic phase was dried (Na₂SO₄), filtered and concentrated *in vacuo.* The orange residue was purified by flash column chromatography on silica (gradient elution with 0 - 10 % MeOH in DCM) to afford the *title compound* (485 mg, 41% at 64% UV purity) as a yellow foam containing 31% urea starting material. LCMS [M+H]⁺ 559.2, RT 1.986 minutes, purity 64% *(Method 12).*

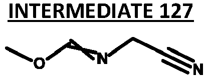

### Methyl N-(cyanomethyl)methanimidate

To a stirred solution of potassium carbonate (1.74 g, 12.6 mmol) in water (2 mL), was added cyanomethanaminium chloride [6011-14-9] (2.3 g, 25.1 mmol) portionwise with stirring. The aqueous mixture was extracted with EtOAc (5 x 30 mL). The organic phases were combined, dried (potassium carbonate), filtered and the solvent removed *in vacuo* to give the free base (766 mg, 54%) as a pale yellow oil which was utilised without further purification. The oil was diluted with trimethylorthoformate [149-73-5] (2 mL) and the solution added dropwise to a refluxing solution of trimethylorthoformate [149-73-5] (18 mL) containing Na₂SO₄ (2.0g) and sulfuric acid (1 drop) over 5 minutes. Stirring was continued for a further 30 minutes by which time the reaction had turned a yellow-orange colour. The mixture was concentrated *in vacuo* to afford the *title compound* (894 mg, 31%) as an orange oil. δ_{H} (500 MHz, DMSO-*d*₆) 7.91 (s, 1H), 4.37 (d, *J* 0.9 Hz, 2H), 3.65 (s, 3H) [~85% pure w/w].

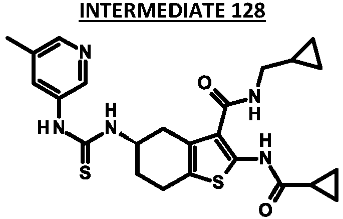

### (5S)-2-(Cyclopropanecarbonylamino)-N-(cyclopropylmethyl)-5-[(5-methyl-3-pyridyl)carbamothioylamino]-4,5,6,7-tetrahydrobenzothiophene-3-carboxamide

*Intermediate 117* (200 mg, 0.60 mmol) was dissolved in DCM (10 mL) and DIPEA (117 mg, 0.90 mmol) was added followed by 3-isothiocyanato-5-methyl-pyridine (135 mg, 0.90 mmol). The reaction was stirred for 2 h before concentrating *in vacuo* to yield the crude product which was purifed by flash column chromatography on silica (gradient elution with 0-100% EtOAc/iso-hexane) to afford the *title compound* (290 mg, 100%) as a white solid. LCMS [M+H]⁺ 483.9, RT 1.348 minutes, purity 100.0% (*Method 4*).

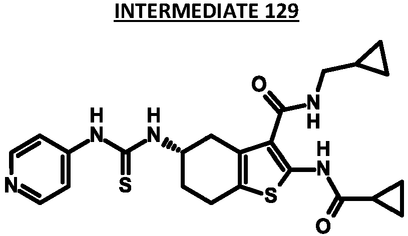

### (5S)-2-(Cyclopropanecarbonylamino)-N-(cyclopropylmethyl)-5-(4-pyridylcarbamothioylamino)-4,5,6,7-tetrahydrobenzothiophene-3-carboxamide

To a solution of pyridin-4-amine [90-25-5] (0.4 ml, 5.31 mmol) in THF (10 mL) was added di-1H-imidazol-1-ylmethanethione [6160-65-2] (1.14 g, 6.38 mmol) portionwise at 0 °C. DCM (10 mL) was added and the mixture was warmed to r.t. and stirred for 18 h. To the reaction mixture was added *intermediate 117* (1.75 g, 5.25 mmol) in DCM (10 mL) and the mixture was stirred for 18 h at r.t. The mixture was diluted with DCM (20 mL) and filtered, the filtrate was concentrated *in vacuo* to give the crude product which was purified by flash column chromatography on silica (gradient elution with 0% to 100% EtOAc in heptane, 0-50% MeOH in DCM) to afford the *title compound* (900 mg, 60%) as a brown solid. LCMS [M+H]⁺ 468.20/470.20, RT 1.67 minutes, purity 84% (*Method 12*).

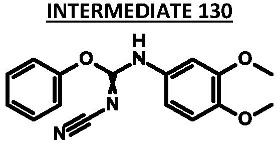

### 3-Cyano-1-(3,4-dimethoxyphenyl)-2-phenyl-isourea

Diphenyl N-cyanocarbonimidate [79463-77-7] (200 mg, 0.81 mmol) and 3,4-dimethoxyaniline [6315-89-5] (137 mg, 0.89 mmol) were dissolved in DCM (2 mL) and 2-propanol (2 mL) and DIPEA (159 mg, 1.22 mmol) were added and the reaction mixture was stirred at r.t. for ~1 h. The mixture was concentrated *in vacuo* and the residue was purified by flash column chromatography on silica (gradient elution with 15 - 100% EtOAc/hexane) to afford the *title compound* (198 mg, 82%) as a white solid. δ_{H} (300 MHz, DMSO-*d*₆) 10.65 (s, 1H), 7.51 7.37 (m, 2H), 7.36 - 7.22 (m, 3H), 7.09 (d, *J* 1.8 Hz, 1H), 7.03 - 6.91 (m, 2H), 3.75 (s, 6H).

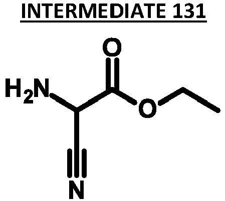

### Ethyl 2-amino-2-cyano-acetate

Ethyl (2*E*)-cyano(hydroxyimino)ethanoate [3849-21-6] (5 g, 35.2 mmol) was suspended in water (30 mL) and the reaction mixture was stirred. Aqueous saturated sodium hydrogen carbonate solution (36 mL) was added slowly in portions to give an orange solution (pH = 5). Sodium dithionite [7631-94-9] (85%, 17 g, 83.0 mmol) was added in portions over 20 minutes and the reaction was allowed to stir for an additional 30 minutes (the temperature of the solution rose to 39 °C). The reaction was extracted with DCM (4 x 50 mL), the organics were dried (Na₂SO₄), filtered and concentrated in *vacuo* to afford the *title compound* (2.82 g, 53% at 85% purity) as a red/brown oil which was stored in the fridge. δ_{H} (250 MHz, Chloroform-*d*) 4.44 (s, 1H), 4.34 (q, *J* 7.1 Hz, 2H), 2.55 (s, 2H), 1.35 (t, *J* 7.1 Hz, 3H.

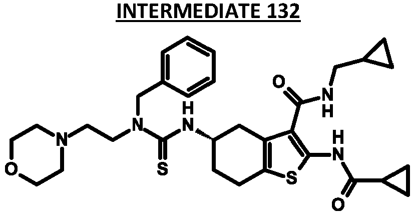

### (5S)-5-[[Benzyl(2-morpholinoethyl)carbamothioyl]amino]-2-(cyclopropanecarbonylamino)-N-(cyclopropylmethyl)-4,5,6,7-tetrahydrobenzothiophene-3-carboxamide

To a solution of *intermediate 117* (400 mg, 1.20 mmol) in DCM (20 mL) was added DIPEA (234 mg, 1.80 mmol) and phenyl chlorothionocarbonate [1005-56-7] (230 mg, 1.32 mmol) and the solution was stirred for 2 h. DIPEA (239 mg, 1.84 mmol) and N-benzyl-2-morpholino-ethanamine [2038-05-3] (270 mg, 1.23 mmol) were added and the reaction was stirred for 18 h. Water (10 mL) was added to the reaction mixture, the organic layer was separated and passed through a phase separator column. The organic phase was concentrated *in vacuo* to give a yellow solid which was purified by flash column chromatography on silica (gradient elution with 0 - 100% EtOAc/iso-hexane) to afford the *title compound* (505 mg, 69%) as a yellow gum. LCMS [M+H]⁺ 596.0, RT 2.75 minutes, 80.1% purity (*Method 1*)*.*

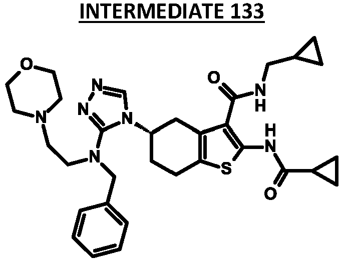

### (5S)-5-[3-[Benzyl(2-morpholinoethyl)amino]-1,2,4-triazol-4-yl]-2-(cyclopropanecarbonylamino)-N-(cyclopropylmethyl)-4,5,6,7-tetrahydrobenzothiophene-3-carboxamide

*Intermediate 132* (758 mg, 1.27 mmol) was dissolved in DMF (10 mL) and formic acid hydrazide [624-84-0] (153 mg, 2.29 mmol) was added followed by mercuric chloride [7487-94-7] (621 mg, 2.29 mmol) and finally triethylamine (233 mg, 2.29 mmol). The mixture was stirred for 2 minutes at r.t. and then heated at 80 °C for 5 h. The mixture was cooled and diluted with MeCN (10 mL) and then filtered through a plug of Celite which was washed with excess MeCN (20 mL). The reaction mixture was concentrated *in vacuo* to give a yellow solid which was purified by flash column chromatography on silica (gradient elution with 10% MeOH/DCM with approx 2% aqueous ammonia]/DCM) to afford the *title compound* (300 mg, 39%) as a white solid. LCMS [M+H]⁺ 604.0, RT 2.340 minutes (*Method 1*).

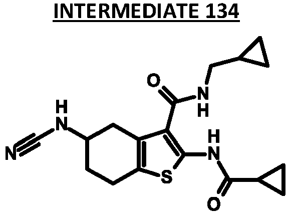

### 5-(Cyanoamino)-2-(cyclopropanecarbonylamino)-N-(cyclopropylmethyl)-4,5,6,7-tetrahydrobenzothiophene-3-carboxamide

To a stirred solution of *intermediate 122* (250 mg, 0.68 mmoL) and DIPEA (0.29 mL, 1.69 mmoL) in anhydrous DCM (3 mL) cooled to 0 °C in an ice bath, was added dropwise a solution of cyanogen bromide [506-68-3] (75 mg, 0.71 mmoL) in anhydrous DCM (2 mL) and the mixture was allowed to warm to r.t. overnight. The reaction mixture was diluted with DCM (20 mL), washed with saturated aqueous ammonium chloride solution (10 mL), saturated aqueous sodium bicarbonate solution (10 mL) and brine (10 mL), then dried (MgSO₄), filtered and concentrated *in vacuo.* The crude product was purified by flash column chromatography on silica (gradient elution with 0 - 100% of EtOAc in heptane) to afford the *title compound* (150 mg, 62%) as an off white solid. δ_{H} (250 MHz, Chloroform-d) 11.98 (s, 1H), 5.89 - 5.71 (m, 1H), 4.00 (d, *J* 5.2 Hz, 1H), 3.77 - 3.59 (m, 1H), 3.35 - 3.21 (m, 2H), 3.13 (dd, *J* 14.8, 4.9 Hz, 1H), 2.89 - 2.69 (m, 3H), 2.22 - 2.06 (m, 1H), 2.07 - 1.89 (m, 1H), 1.73 - 1.60 (m, 1H), 1.16 - 0.97 (m, 3H), 0.99 - 0.80 (m, 2H), 0.67 - 0.52 (m, 2H), 0.36 - 0.22 (m, 2H). LCMS [M+H]⁺ 359, RT 3.53 minutes, 96% purity (*Method 5*).

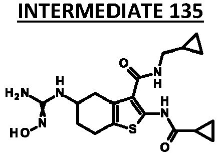

### 2-(cyclopropanecarbonylamino)-N-(cyclopropylmethyl)-5-[(N'-hydroxycarbamimidoyl)amino]-4,5,6,7-tetrahydrobenzothiophene-3-carboxamide

To a stirred solution of *intermediate 134* (140 mg, 0.39 mmoL) in EtOH (5 mL) was added hydroxylamine hydrochloride [5470-11-1] (29 mg, 0.41 mmoL) followed by potassium carbonate (108 mg, 0.78 mmoL) and the mixture was stirred at r.t. under an atmosphere of nitrogen for 18 h. Hydroxylamine hydrochloride [5470-11-1] (29 mg, 0.41 mmoL) followed by potassium carbonate (108 mg, 0.78 mmoL) were added and stirring was continued at r.t. for 1 h. The reaction mixture was filtered through a pad of celite, washed with EtOH and concentrated *in vacuo.* The crude material (210 mg) was purified by flash column chromatography on silica (gradient elution with 0 to 100% of EtOAc in heptane, followed by 0 to 10% of MeOH in EtOAc, followed by 10% ammonia (7N in MeOH) in EtOAc) to give the product which was further purified by preparative HPLC (low pH) to afford the *title compound* (2.2 mg, 1.4%) as a formate salt. The column waste was concentrated *in vacuo* to afford further *title compound* (30 mg, 13%, 66% purity). LCMS [M+H]⁺ 392, RT 2.76 minutes, 99% purity (*Method 5*).

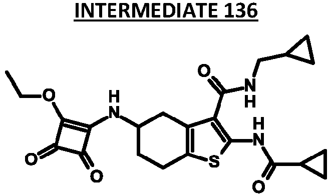

### 2-(Cyclopropanecarbonylamino)-N-(cyclopropylmethyl)-5-[(2-ethoxy-3,4-dioxo-cyclobuten-1-yl)aminol-4,5,6,7-tetrahydrobenzothiophene-3-carboxamide

*Intermediate 122* (90% purity, 100 mg, 0.24 mmol) was suspended in THF (1 mL) and DIPEA (50 µL, 0.29 mmol) was added. The solution was cooled to -78 °C and 3,4-diethoxycyclobut-3-ene-1,2-dione [5231-87-8] (42 mg, 0.25 mmol) was added. The reaction mixture was warmed to r.t. and stirred for 4 h. An additional reaction was carried out, *intermediate 122* (90%, 100 mg, 0.24 mmol) was dissolved in EtOH (1 mL) and DIPEA (50 µL, 0.29 mmol) was added, the solution was stirred at r.t. for 2 h. The two reactions were combined, resulting in a clear brown solution, a further equivalent of 3,4-diethoxycyclobut-3-ene-1,2-dione [5231-87-8] (42 mg, 0.25 mmol) and DIPEA (100 µL, 0.57 mmol) were added and the solution was stirred for 1 h at r.t. The reaction mixture was concentrated *in vacuo* and purified by flash column chromatography on silica (gradient elution with 0 - 100% EtOAc/heptane) to afford the *title compound* (200 mg, 85%) as a ~1:1 mixture of rotamers. δ_{H} (500 MHz, DMSO-*d*₆) 11.07 (d, *J* 7.4 Hz, 2H), 9.00 (d, *J* 7.8 Hz, 1H), 8.81 (d, *J* 8.1 Hz, 1H), 7.73 (br t, J 4.7 Hz, 2H), 4.69 - 4.62 (m, 4H), 4.22 (br, 1H), 3.83 (br, 1H), 3.12 (m, 4H), 2.97 (br d, *J* 16.5 Hz, 2H), 2.78 (br d, *J* 16.1 Hz, 2H), 2.70 (s, 4H), 2.04 (br, 2H), 1.95 - 1.88 (m, 2H), 1.84 (br, 2H), 1.41 - 1.30 (m, 6H), 1.07 - 0.97 (m, 2H), 0.89 - 0.79 (m, 8H), 0.44 - 0.37 (m, 4H), 0.24 - 0.18 (m, 4H).

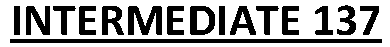

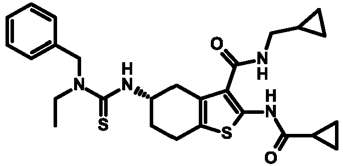

### (5S)-5-[[Benzyl(ethyl)carbamothioyl]amino]-2-(cyclopropanecarbonylamino)-N-(cyclopropylmethyl)-4,5,6,7-tetrahydrobenzothiophene-3-carboxamide

A solution of O-phenyl carbonochloridothioate [1005-56-7] (1.55 g, 9 mmol) in DCM (90 mL) was added over 20 minutes to an ice-cold stirred solution of *intermediate 117* (3.0 g, 9 mmol) and triethylamine [121-44-8] (1.87 mL, 13.5 mmol) in DCM (180 mL). The mixture was stirred at 0 °C for 2 h and then the ice bath was removed. The reaction was quenched by adding water (100 mL), the organic phase was separated and the aqueous phase was extracted with DCM (2 × 100 mL). The combined organic phases were washed with brine (100 mL), dried (MgSO₄), filtered and concentrated *in vacuo* to afford the crude *(55)-2-(cyclopropanecarbonylamino)-N-(cyclopropylmethyl)-5-isothiocyanato-4,5,6,7-tetrahydrobenzothiophene-3-carboxamide* (3.90 g, 94%, 81% purity).

A solution of N-benzylethanamine [14321-27-8] (1.36 g, 10.1 mmol) in Et₂O (70 mL) was added dropwise to a solution of crude *(5S)-2-(cyclopropanecarbonylamino)-N-(cyclopropylmethyl)-5-isothiocyanato-4,5,6,7-tetrahydrobenzothiophene-3-carboxamide* (81% purity, 3.90 g, 8.41 mmol) in isopropanol (70 mL). The reaction mixture was stirred for 4 h before concentrating *in vacuo* and the crude material was purified by flash column chromatography on silica (gradient elution with 0 - 100% EtOAc in heptane) to afford the *title compound* (1.52 g, 59%, 84% purity). LCMS [M+H]⁺ 511.20/509.20, RT 2.04 minutes, 84% purity (*Method 12*)*.*

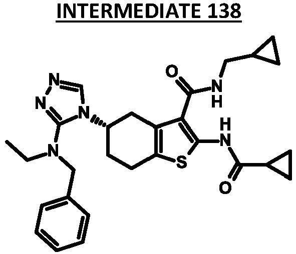

### (5S)-5-[3-[Benzyl(ethyl)aminol-1,2,4-triazol-4-yl]-2-(cyclopropanecarbonylamino)-N-(cyclopropylmethyl)-4,5,6,7-tetrahydrobenzothiophene-3-carboxamide

*Intermediate 137* (80%, 4.20 g, 6.58 mmol) and formic hydrazide [624-84-0] (1.19 g, 19.7 mmol) were dissolved in DMF (60 mL) and mercury dichloride [7487-94-7] (5.36 mg, 19.7 mmol) was added to the reaction mixture. The reaction mixture was stirred for 5 minutes and triethylamine [121-44-8] (2.74 ml, 19.7 mmol) was added. The reaction mixture was heated at 90 °C for 3 h before diluting with DCM (50 mL), filtering through a short pad of celite, and concentrating the filtrate *in vacuo.* The crude product was extracted with EtOAc (3 x 60 mL) and washed with water (60 mL). The organics were dried (MgSO₄), filtered, and the filtrate was concentrated *in vacuo* to afford the crude material which was purified by flash column chromatography on silica (gradient elution with 0 - 100%, 0 - 40% MeOH in DCM) to afford the desired product which was further purified by basic preparative HPLC to afford the *title compound* (1.7 g, 46%) as a yellow solid. δ_{H} (500 MHz, Methylene Chloride-*d*₂) 12.02 (s, 1H), 7.93 (s, 1H), 7.35 - 7.08 (m, 5H), 5.63-5.60 (m, 1H), 4.37-4.32 (m, 1H), 4.22 (s, 2H), 3.29 - 3.11 (m, 4H), 2.87 (dd, *J* 14.7, 5.1 Hz, 1H), 2.80 - 2.73 (m, 2H), 2.68 (dd, *J* 14.5, 9.5 Hz, 1H), 2.01-1.93 (m, 1H), 1.77 - 1.70 (m, 1H), 1.69-1.64 (m, 1H), 1.08 (t, *J* 7.1 Hz, 3H), 1.06-0.98 (m, 2H), 0.99-0.96 (m, 1H), 0.93 - 0.87 (m, 2H), 0.51 - 0.42 (m, 2H), 0.22-0.17 (m, 2H).

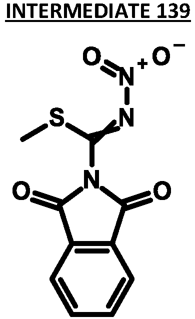

### methyl N-nitro-1,3-dioxo-isoindoline-2-carboximidothioate

To a solution of 2-methyl-1-nitroisothiourea (0.5 g, 3.7 mmol) in pyridine [110-86-1] (6.5 mL) was added dropwise O-phthaloyl dichloride [88-95-9] (0.80 mL, 5.55 mmol) at 0 °C over 20 minutes. On completion of addition the reaction was stirred at 0 °C for 30 minutes and poured into cold aqueous hydrochloric acid solution (5 mL of concentrated hydrochloric acid in 20 mL of water). The reaction mixture was filtered and the pale yellow solid was collected and recrystallized from EtOH to afford the *title compound* (0.76 g, 70%) as white needles. δ_{H} (500 MHz, CD₃OD) 8.05 - 7.98 (m, 2H), 7.97 - 7.92 (m, 2H), 2.6(s, 3H). LCMS [M+H]⁺ 265.95, RT 1.11 minutes, 90% purity (*Method 6*)*.*

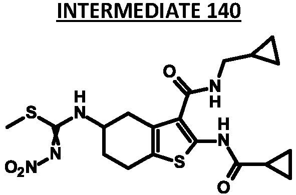

### 2-(cyclopropanecarbonylamino)-N-(cyclopropylmethyl)-5-[(C-methylsulfanyl-N-nitro-carbonimidoyl)amino]-4,5,6,7-tetrahydrobenzothiophene-3-carboxamide

To a solution of *intermediate 139* (107.5 mg, 0.40 mmol) in pyridine [88-95-9] (0.1 mL) and DCM (2 mL) was added dropwise *intermediate 122* (150 mg, 0.40 mmol, free based with DIPEA) in DCM (1.5 mL) at 0 °C over 10 minutes. The reaction was warmed to r.t. and stirred for 3 h. The reaction mixture was concentrated *in vacuo* to give a yellow oil, which was purified by flash column chromatography on silica (gradient elution with 10% to 100% EtOAc in heptane) to afford the *title compound* (170 mg, 93%) as a white solid. δ_{H} (500 MHz, CD₃OD) 4.39 - 4.14 (m, 1H), 3.26 - 3.13 (m, 3H), 2.91- 2.75 (m, 3H), 2.51 (s, 3H), 2.25 - 2.15 (m, 1H), 2.11 - 2.00 (m, 1H), 1.84 - 1. (m, 1H), 1.14 - 1.05 (m, 1H), 1.01 - 0.91 (m, 4H), 0.56 - 0.49 (m, 2H), 0.31 - 0.25 (m, 2H). LCMS [M+H]⁺ 452.1, RT 3.42 minutes, 100% purity (*Method 10*).

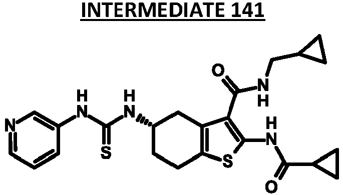

### (5S)-2-(Cyclopropanecarbonylamino)-N-(cyclopropylmethyl)-5-(3-pyridylcarbamothioylamino)-4,5,6,7-tetrahydrobenzothiophene-3-carboxamide

*Intermediate 117* (1.5 g, 4.5 mmol) was dissolved in DCM (20 mL) and DIPEA (880 mg, 6.7 mmol) was added. 3-Pyridyl isothiocyanate [17452-27-6] (940 mg, 6.7 mmol) was added and the reaction mixture was stirred at r.t. for ~1 h. The reaction was concentrated *in vacuo* and the residue purified by flash column chromatography on silica (gradient elution with 35 - 100% EtOAc/hexane to 0 - 5% MeOH/EtOAc) to afford the *title compound* (2.0 g, 95%) as a colourless gum. LCMS [M+H]⁺ 470.2, RT 1.836 minutes, 95.6% purity (*Method 1*)*.*

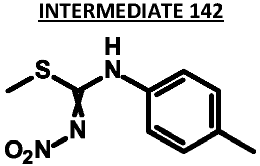

### 2-methyl-3-nitro-1-(p-tolyl)isothiourea

To a solution of *intermediate 140* (150 mg, 0.56 mmol) in pyridine [110-86-1] (0.1 mL) and DCM (2 mL) was added dropwise 4-methylaniline [106-49-0] (60.6 mg, 0.56 mmol) in DCM (2 mL) at 0 °C over 10 minutes. The reaction mixture was allowed to warm to r.t. and stirred for 16 h. The reaction mixture was concentrated *in vacuo* to give a yellow oil which was purified by flash column chromatography on silica (gradient elution with 10% to 100% EtOAc in heptane) to afford the *title compound* (110 mg, 76%) as a white solid. δ_{H} (500 MHz, Chloroform-d) 11.24 (s, 1H), 7.25 (d, *J* 6.7 Hz, 2H), 7.18 (d, *J* 8.3 Hz, 2H), 2.44 (s, 3H), 2.40 (s, 3H). LCMS [M-H]⁻ 223.95, RT 1.13 minutes, 88% purity (*Method 6*).

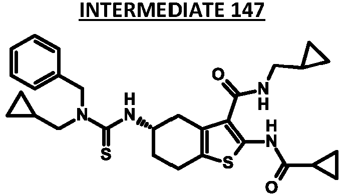

### (5S)-5-[[Benzyl(cyclopropylmethyl)carbamothioyl]amino]-2-(cyclopropanecarbonylamino)-N-(cyclopropylmethyl)-4,5,6,7-tetrahydrobenzothiophene-3-carboxamide

*Intermediate 117* (1.96 g, 5.88 mmol) was dissolved in DCM (20 mL) and DIPEA (1.15 g, 8.90 mmol) was added and the reaction mixture was cooled to 0 °C. Phenyl chlorothionocarbonate [1005-56-7] (1.13 g, 6.55 mmol) was added and the reaction mixture was stirred at 0 °C warming to r.t. over ~2 h. DIPEA (1.15 g, 8.82 mmol) and N-benzyl-1-cyclopropylmethanamine [116373-23-0] (998 mg, 5.88 mmol) was added and the reaction mixture was stirred at r.t. for ~4 h. Further N-benzyl-1-cyclopropylmethanamine [116373-23-0] (99.8 mg, 0.62 mmol) was added and the reaction mixture was stirred at r.t. for ~36 h. The reaction mixture was washed with water and brine, passed through a phase separation cartridge and concentrated *in vacuo.* The residue was purified by flash column chromatography on silica (gradient elution with 5-70% EtOAc/hexane) to afford the *title compound* (2.99 g, 95%) as a colourless foam. δ_{H} (500 MHz, DMSO-*d*₆) 11.02 (s, 1H), 7.70 (t, *J* 5.6 Hz, 1H), 7.31 (t, *J* 7.3 Hz, 2H), 7.27 - 7.20 (m, 3H), 7.18 (d, *J* 7.6 Hz, 1H), 5.16 - 5.02 (m, 2H), 4.72 - 4.58 (m, 1H), 3.60 - 3.45 (m, 2H), 3.21 -3.06 (m, 2H), 3.01 (dd, *J* 15.8, 5.3 Hz, 1H), 2.70 - 2.64 (m, 2H), 2.58 (dd, *J* 15.8, 9.4 Hz, 1H), 2.03 - 1.95 (m, 1H), 1.91 (p, *J* 6.4 Hz, 1H), 1.87 - 1.72 (m, 1H), 1.12 - 0.96 (m, 2H), 0.90 - 0.77 (m, 4H), 0.46 -0.34 (m, 4H), 0.26 - 0.14 (m, 4H). LCMS [M+H]⁺ 537.2, RT 2.097 minutes, 99% purity (*Method 12*). LCMS [M+H]⁺ 537.2, RT 2.871 minutes, 93.3% purity (*Method 3*).

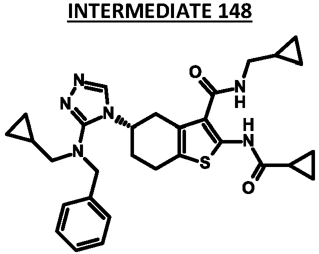

### (5S)-5-[3-[Benzyl(cyclopropylmethyl)aminol-1,2,4-triazol-4-yl]-2-(cyclopropanecarbonylamino)-N-(cyclopropylmethyl)-4,5,6,7-tetrahydrobenzothiophene-3-carboxamide

*Intermediate 147* (2.99 g, 5.57 mmol) was dissolved in DMF (10 mL) and formic acid hydrazide [624-84-0] (558 mg, 8.36 mmol) and mercuric chloride [7487-94-7] (1.97 g, 7.24 mmol) was added. The reaction mixture was stirred at r.t. for 5 minutes before addition of triethylamine (850 mg, 8.36 mmol). The reaction mixture was stirred at r.t. for ~30 minutes and then stirred at 60 °C for ~2.5 h. Further formic acid hydrazide [624-84-0] (279 mg, 4.65 mmol) was added and the mixture was stirred at 60 °C for 4 h, then at r.t. overnight. Further formic acid hydrazide [624-84-0] (186 mg, 3.10 mmol) and mercuric chloride [7487-94-7] (379 mg, 1.40 mmol) was added and the mixture was stirred at 60 °C for 4 h before cooling, diluting with MeCN (40 mL), filtering through a pad of celite and washing with MeCN (3 x 10 mL). The pale yellow filtrate was concentrated *in vacuo* to yield a tan solid which was purified by flash column chromatography on silica (gradient elution with 50-100% EtOAc/hexane to 20% MeOH/EtOAc) to afford the *title compound* (2.31 g, 76%) as a pale yellow gum. LCMS [M+H]⁺ 545.2, RT 2.348 minutes, 100.0% purity (*Method 2*).

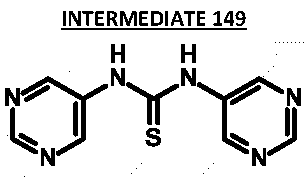

### 1,3-Di(pyrimidin-5-yl)thiourea

To a solution of pyrimidin-5-amine [591-55-9] (500 mg, 5.26 mmol) in THF (10 mL) was added di-1H-imidazol-1-ylmethanethione [6160-65-2] (1.12 g, 6.31 mmol) portionwise at 0 °C. The reaction mixture was warmed to r.t. and stirred for 18 h. The crude mixture was diluted with isopropanol (10 mL) and the solid was filtered and dried under reduced pressure to afford the *title compound* (0.34 g, 28%). δ_{H} (250 MHz, DMSO-*d*₆) 10.42 (s, 2H), 8.98 (s, 2H), 8.91 (s, 4H). LCMS [M+H]⁺ 233.20/231.20, RT 0.50 minutes, 100% purity (*Method 12*).

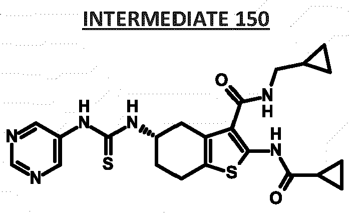

### (5S)-2-(Cyclopropanecarbonylamino)-N-(cyclopropylmethyl)-5-(pyrimidin-5-ylcarbamothioylamino)-4,5,6,7-tetrahydrobenzothiophene-3-carboxamide

A solution of *intermediate 149* (193.5 µl, 0.9 mmol) in DCM (5 mL) was stirred at r.t. prior to the addition of *intermediate 117* (300 mg, 0.9 mmol). The solution was stirred for 18 h under nitrogen and concentrated *in vacuo* to give a pale yellow solid which was purified by flash column chromatography on silica (gradient elution with 0% to 100% EtOAc in heptane, 0-60 % MeOH in DCM) to afford the product which was further purified by preparative HPLC (basic) to afford the *title compound* (220 mg, 52%) as a white solid. ¹H NMR (250 MHz, Methylene Chloride-*d*₂) δ 12.01 (s, 1H), 8.90 (s, 3H), 8.39 (s, 1H), 6.90 (d, *J* 6.8 Hz, 1H), 5.81 - 5.78 (m, 1H), 4.95 - 4.91 (m, 1H), 3.36 - 3.02 (m, 3H), 2.84 - 2.77 (m, 3H), 2.52 - 2.49 (m, 1H), 2.00 - 1.97 (m, 1H), 1.73 - 1.69 (m, 1H), 1.14 - 0.86 (m, 5H), 0.63 - 0.54 (m, 2H), 0.32 - 0.27 (m, 2H). LCMS [M+H]⁺ 471.20/469.20, RT 1.73 minutes, 100% purity (*Method 12*).

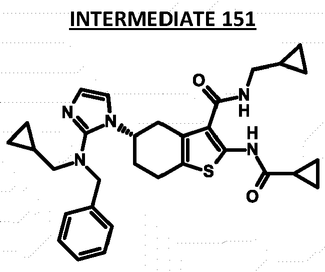

### (5S)-5-[2-[Benzyl(cyclopropylmethyl)amino]imidal-1-yl]-2-(cyclopropanecarbonylamino)-N-(cyclopropylmethyl)-4,5,6,7-tetrahydrobenzothiophene-3-carboxamide

*Intermediate 147* (541 mg, 1.01 mmol) was dissolved in DMF (10 mL), and 2,2-diethoxyethanamine [22483-09-6] (0.44 mL, 3.02 mmol) was added followed by mercury dichloride [7487-94-7] (821 mg, 3.02 mmol). The reaction was stirred at r.t. for 5 minutes then triethylamine (0.42 mL, 3.02 mmol) was added. The reaction was heated to 90 °C and stirred for 2 h. 4-Methylbenzene-1-sulfonic acid hydrate (PTSA hydrate) [6192-52-5] (1.15 g, 6.05 mmol) was added and the reaction was heated at 90 °C with stirring for 18 h. The reaction was allowed to cool, diluted with DCM and Kieselguhr added and the mixture was filtered through a plug of Kieselguhr washing with DCM. The filtrate was concentrated *in vacuo* and the residue was dissolved in EtOAc (100 mL). The organic layer was washed with water (3 x 20 mL), 5 % aqueous lithium chloride (20 mL), dried (Na₂SO₄), filtered and concentrated *in vacuo.* The orange / brown residue was purified by flash column chromatography on silica (gradient elution with 0 -100% EtOAc then 10 % MeOH in DCM) to afford the *title compound* (412 mg, 69 %, 92 % purity) as a brown foam. LCMS [M+H]⁺ 544.2, RT 1.824 minutes, 92% purity (*Method 12*).

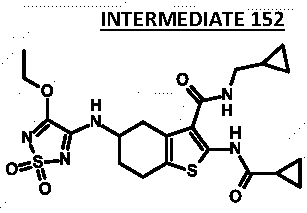

### 2-(Cyclopropanecarbonylamino)-N-(cyclopropylmethyl)-5-[(4-ethoxy-1,1-dioxo-1,2,5-thiadiazol-3-yl)amino]-4,5,6,7-tetrahydrobenzothiophene-3-carboxamide

*Intermediate 122* (50 mg, 0.13 mmol) was dissolved in EtOH (3 mL). 3,4-Diethoxy-1-{6},2,5-thiadiazole-1,1-dione [55904-84-2] (30.7 mg, 0.14 mmol) and DIPEA (0.033 ml, 0.19 mmol) were added and the reaction mixture was stirred at r.t. for 3 h. The reaction mixture was concentrated in *vacuo* and the crude residue, the *title compound* (66 mg, 85%, 86% purity) was used in the next stage without further purification. LCMS [M+H]⁺ 494.05, RT 1.27 minutes, 86% purity (*Method 6*).

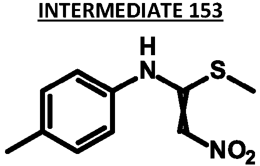

### 4-methyl-N-(1-methylsulfanyl-2-nitro-vinyl)aniline

1,1-Bis(methylthio)-2-nitroethylene [13623-94-4] (812 mg, 4.67 mmol) and p-toluidine [106-49-0] (500 mg, 4.67 mmol) were taken up in isopropanol (1.57 g, 26.12 mmol) and the reaction mixture was stirred at 100 °C for 2 h in the microwave before leaving to stand at r.t. overnight. The solid formed was filtered-off, washing with isopropanol (2 x) and EtOAc, to afford the *title compound* (553 mg, 53%) as a yellow crystalline solid. δ_{H} (500 MHz, Chloroform-*d*) 11.24 (s, 1H), 7.25 (d, *J* 6.7 Hz, 2H), 7.18 (d, *J* 8.3 Hz, 2H), 2.44 (s, 3H), 2.40 (s, 3H).

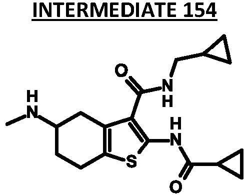

### 2-(Cyclopropanecarbonylamino)-N-(cyclopropylmethyl)-5-(methylamino)-4,5,6,7-tetrahydrobenzothiophene-3-carboxamide

To a stirred solution of *intermediate 62* (0.44 g, 1.33 mmol) in 1,2-DCE [107-06-2] (5 mL) at 30 °C was added methyl ammonium hydrochloride [593-51-1] (0.22 g, 3.32 mmol) and sodium triacetoxy borohydride [56553-60-7] (0.42 g, 1.99 mmol). The reaction mixture was stirred at r.t. for 3 h and then diluted with 1,2-DCE (10 mL), washed with water (10 mL) and washed with saturated aqueous sodium bicarbonate solution (10 mL). The organic layer was separated, dried (Na₂SO₄), filtered and concentrated *in vacuo* to give a pale green oil. The crude product was diluted with EtOAc (20 mL) and washed with 1M aqueous hydrochloric acid (2 x 10 mL). The combined acidic extracts were basified with sodium hydrogen carbonate to ~pH 7.5 and then the pH was adjusted to pH 9-10 with 2M aqueous sodium carbonate solution. The basic aqueous solution was extracted with EtOAc (2 x 15 mL) and the organic layers were dried (Na₂SO₄), filtered and concentrated *in vacuo* to give a pale yellow solid (0.13 g). The basic aqueous layer was then saturated with sodium chloride and extracted with EtOAc (3 x 10 mL) and 1:1 chloroform/isopropanol (2 x 10 mL). The combined organic extracts were dried (Na₂SO₄), filtered and concentrated *in vacuo* before combining with the previous batch to afford the *title compound* (0.14 g, 28%, 91% purity). δ_{H} (500 MHz, DMSO-*d*₆) 11.18 (s, 1H), 7.78 (s, 1H), 3.20 (dd, *J* 13.4, 6.4 Hz, 1H), 3.10 (dt, *J* 13.3, 6.0 Hz, 1H), 2.90 (dd, *J* 16.2, 4.4 Hz, 1H), 2.68 (m, 2H), 2.58 (m, 1H), 2.41 (dd, *J* 15.7, 8.4 Hz, 1H), 2.34 (s, 3H), 2.02 - 1.94 (m, 1H), 1.87 (p, *J* 6.2 Hz, 1H), 1.56 - 1.46 (m, 1H), 1.09 - 1.00 (m, 1H), 0.82 (m, 4H), 0.46 - 0.39 (m, 2H), 0.23 (q, J 4.5 Hz, 2H). LCMS [M+H]⁺ 348, RT 0.81 minutes, 91% purity (*Method 11*).

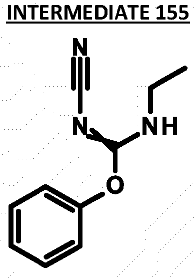

### 3-Cyano-1-ethyl-2-phenyl-isourea

To a solution of diphenyl N-cyanocarbonimidate [79463-77-7] (1.00 g, 4.20 mmol) in EtOH (40 mL) was added DIPEA (0.54 g, 4.20 mmol). 2.0M Ethylamine in THF (2.1 mL, 4.2 mmol) was added dropwise to the stirred solution at r.t. and the reaction was stirred at r.t. for 2 h. The reaction mixture was concentrated *in vacuo* and water (10 mL) was added to form a wax which was filtered and dried overnight at 40 °C. The solid was cooled to r.t. to afford the *title compound* (0.62 g, 78%) as an off-white solid. δ_{H} (500 MHz, Chloroform-*d*) 80%+ purity, 7.40 (t, *J* 7.6 Hz, 2H), 7.29 (d, *J* 7.3 Hz, 1H), 7.09 (d, *J* 7.9 Hz, 2H), 6.82 (s, 1H), 3.49 (p, *J* 6.8 Hz, 2H), 1.30 (t, *J* 7.1 Hz, 3H). ¹H NMR has broad second component that could be E/Z stereoisomer, rotational broadening or impurity. LCMS [M+H]⁺ 190, RT 0.98 minutes, 99% purity (*Method 6*).

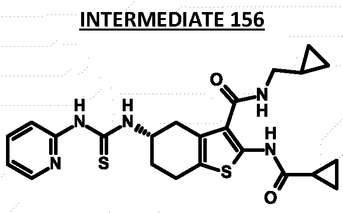

### (5S)-2-(Cyclopropanecarbonylamino)-N-(cyclopropylmethyl)-5-(2-pyridylcarbamothioylamino)-4,5,6,7-tetrahydrobenzothiophene-3-carboxamide

*Intermediate 117* (400 mg, 1.2 mmol) was dissolved in DCM (5 mL) and 2-isothiocyanatopyridine [52648-45-0] (196 mg, 1.44 mmol) in DCM (5 mL) was added and the solution was stirred for 18 h under nitrogen before stirring for a further 24h at r.t. The reaction mixture was concentrated in *vacuo* to give a pale yellow solid which was washed with DCM to afford the *title compound* (400 mg, 71%). δ_{H} (500 MHz, DMSO-*d*₆) 12.01 (d, J 7.9 Hz, 1H), 11.14 (s, 1H), 10.56 (s, 1H), 8.04 (d, *J* 3.9 Hz, 1H), 7.79 - 7.72 (m, 1H), 7.69 (t, *J* 4.4 Hz, 1H), 7.15 (d, *J* 8.4 Hz, 1H), 7.01 (dd, *J* 7.2, 5.9 Hz, 1H), 4.73 - 4.62 (m, 1H), 3.19 - 3.07 (m, 3H), 2.88 - 2.70 (m, 3H), 2.17 - 2.05 (m, 1H), 2.03 - 1.81 (m, 2H), 1.09 - 0.94 (m, 1H), 0.90 - 0.76 (m, 4H), 0.46 - 0.36 (m, 2H), 0.27 - 0.16 (m, 2H).

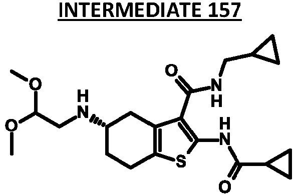

### (5S)-2-(Cyclopropanecarbonylamino)-N-(cyclopropylmethyl)-5-(2,2-dimethoxyethylamino)-4,5,6,7-tetrahydrobenzothiophene-3-carboxamide

To *intermediate 117* (1 g, 3 mmol) in MeOH (20 mL) was added dimethoxyacetaldehyde [51673-84-8] (60% aqueous, 452 µL, 2.99 mmol) and the reaction was stirred for 18 h to give a cream coloured suspension. Sodium borohydride [16940-66-2] (113 mg, 2.99 mmol) was added in portions, and the reaction was stirred at r.t. for 15 minutes. The reaction was concentrated *in vacuo* and diluted with EtOAc (60 mL). The organic layer was washed with water (2 x 20 mL), dried (Na₂SO₄), filtered and concentrated *in vacuo* to give a yellow oil (1.2 g) which was purified by flash column chromatography on silica (gradient elution with 0 - 10% MeOH in DCM) to afford the *title compound* (1.07g, 84%) as a yellow oil which solidified on standing. δ_{H} (500 MHz, DMSO-*d*₆) 11.20 (s, 1H), 7.66 - 7.57 (m, 1H), 4.40 (t, *J* 5.5 Hz, 1H), 3.27 (d, *J* 1.0 Hz, 6H), 3.24 - 3.15 (m, 1H), 3.15 - 3.05 (m, 1H), 2.87 (d, *J* 15.6 Hz, 1H), 2.85 - 2.74 (m, 1H), 2.70 (d, *J* 4.8 Hz, 2H), 2.69 - 2.64 (m, 1H), 2.63 - 2.53 (m, 1H), 2.42 (dd, *J* 15.4, 8.0 Hz, 1H), 2.01 - 1.93 (m, 1H), 1.93 - 1.84 (m, 1H), 1.59 - 1.39 (m, 2H), 1.10 - 0.99 (m, 1H), 0.88 - 0.77 (m, 4H), 0.47 - 0.39 (m, 2H), 0.27 - 0.21 (m, 2H).

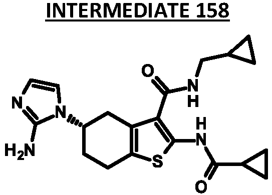

### (5S)-5-(2-Aminoimidazol-1-yl)-2-(cyclopropanecarbonylamino)-N-(cyclopropylmethyl)-4,5,6,7-tetrahydrobenzothiophene-3-carboxamide

Cyanamide [420-04-2] (134 mg, 3.2 mmol) was dissolved in 1,4-dioxane (10 mL) in a pressure tube and 4M hydrochloric acid in 1,4-dioxane (800 µl) was added to give a white suspension which was stirred for 5 minutes. *Intermediate* 157 (450 mg, 1.07 mmol) was added and the reaction was sealed and heated to 60 °C, stirring for 1 h. The reaction was heated and stirred at 60 °C for a further 17 h to give a brown oily solid. The reaction mixture was diluted with MeOH and was concentrated in *vacuo.* The residue was diluted with DCM (30 mL) and washed with saturated aqueous sodium hydrogen carbonate solution (20 mL). The aqueous layer was further extracted with 10% MeOH in DCM (3 x 20 mL). The organic layers were combined, dried (Na₂SO₄), filtered and concentrated in *vacuo* to give the crude product (574 mg) which was purified by flash column chromatography on silica (gradient elution with 0 - 10% MeOH in DCM) to give the crude product (184 mg) as a yellow oil. The material was dissolved in DMSO/ MeCN and purified by reverse phase preparative HPLC (basic) to give a gel which was sonicated in Et₂O to give a white solid which was collected by filtration and dried in a vacuum oven to afford the *title compound* (75 mg, 18%). δ_{H} (500 MHz, DMSO-*d*₆) 11.15 (s, 1H), 7.73 (s, 1H), 6.66 (d, *J* 1.5 Hz, 1H), 6.42 (d, *J* 1.5 Hz, 1H), 5.37 (s, 2H), 4.28 - 4.16 (m, 1H), 3.12 (qt, *J* 13.5, 6.2 Hz, 2H), 2.97 (dd, *J* 15.1, 4.0 Hz, 1H), 2.91 - 2.72 (m, 3H), 2.07 - 1.98 (m, 2H), 1.96 - 1.85 (m, 1H), 1.06 - 0.96 (m, 1H), 0.88 - 0.80 (m, 4H), 0.44 - 0.34 (m, 2H), 0.25 - 0.15 (m, 2H). LCMS [M+H]⁺ 400.2, RT 1.83 minutes, 100% purity (*Method 10*).

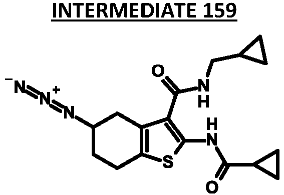

### 5-Azido-2-(cyclopropanecarbonylamino)-N-(cyclopropylmethyl)-4,5,6,7-tetrahydrobenzothiophene-3-carboxamide

To *Example 109* (1.24 g, 3.72 mmol) in MeOH (20 mL) was added potassium carbonate (1.80 g, 13.0 mmol), copper(II) sulfate pentahydrate (27.9 mg, 0.11 mmol) and 1H-imidazole-1-sulfonyl azide hydrochloride [952234-36-5] (1.07 g, 4.84 mmol). The reaction mixture was stirred at r.t. for 2 h and further 1H-imidazole-1-sulfonyl azide hydrochloride [952234-36-5] (82.3 mg, 0.39 mmol) was added and the reaction mixture was stirred for a further 30 minutes. The solvent was removed *in vacuo* and the solid was partitioned between water (10 mL) and EtOAc (30 mL). To this was added 2N aqueous hydrochloric acid solution (20 mL) and the organic layer was separated and the aqueous layer was further extracted with EtOAc (20 mL). The combined organic layers were dried (Na₂SO₄), filtered and the solvent removed *in vacuo* to give a red residue which was purified by flash column chromatography on silica (gradient elution with 0-30% EtOAc/iso-hexane) to afford the *title compound* (1.20 g, 90%) as a white solid. δ_{H} (300 MHz, DMSO-*d*₆) 11.08 (s, 1H), 7.75 (t, *J* 5.6 Hz, 1H), 4.03 (d, *J* 6.9 Hz, 1H), 3.26 - 2.92 (m, 4H), 2.72 (s, 3H), 2.10 - 1.76 (m, 2H), 1.12 - 0.98 (m, 1H), 0.89 - 0.81 (m, 2H), 0.83 (s, 2H), 0.50 - 0.37 (m, 2H), 0.29 - 0.18 (m, 2H).

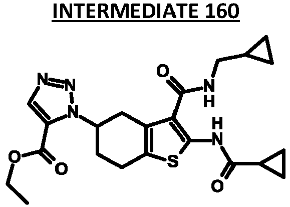

### Ethyl 3-[2-(cyclopropanecarbonylamino)-3-(cyclopropylmethylcarbamoyl)-4,5,6,7-tetrahydrobenzothiophen-5-yl]triazole-4-carboxylate

*Intermediate 159* (49 mg, 0.14 mmol) was dissolved in toluene (2 mL) and ethyl propiolate [623-47-2] (40.1 mg, 0.41 mmol) was added. The solution was heated at 110 °C for 18 h before cooling and removing the solvent *in vacuo.* The residue was purified by flash column chromatography on silica (gradient elution with 0-60% EtOAc/iso-hexane) to afford the *title compound* (13 mg, 21%). δ_{H} (300 MHz, Chloroform-*d*) 12.06 (s, 1H), 8.21 (s, 1H), 5.82 (s, 1H), 5.57 (d, *J* 11.3 Hz, 1H), 4.42 (q, *J* 7.1 Hz, 2H), 3.62 - 3.48 (m, 1H), 3.39 - 3.16 (m, 3H), 2.98 (s, 2H), 1.77 - 1.54 (m, 1H), 1.59 - 0.93 (m, 8H), 0.98 - 0.86 (m, 2H), 0.58 - 0.46 (m, 2H), 0.24 (dt, *J* 5.9, 4.5 Hz, 2H).

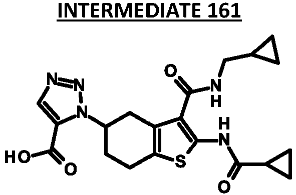

### 3-[2-(Cyclopropanecarbonylamino)-3-(cyclopropylmethylcarbamoyl)-4,5,6,7-tetrahydrobenzothiophen-5-yl]triazole-4-carboxylic acid

*Intermediate 160* (184 mg, 0.40 mmol) was dissolved in THF (1.76 g) and water (500 mg, 30 mmol). To this was added lithium hydroxide monohydrate [1310-66-3] (50.6 mg, 1.21 mmol) and the mixture was stirred for 18 h. The reaction mixture was concentrated *in vacuo* and the residue was redissolved in water (5 mL) and extracted with EtOAc (10 mL). The aqueous layer was acidified to pH 1 using 2M aqueous hydrochloric acid solution (5 - 10 mL). A white solid crashed out and was filtered to a solid which was suspended in MeOH and the solvent removed *in vacuo* to afford the *title compound* (127 mg, 74%) as a white solid. δ_{H} (300 MHz, DMSO-*d*₆) 11.12 (s, 1H), 8.24 (s, 1H), 7.76 (t, *J* 5.8 Hz, 1H), 5.46 (s, 1H), 3.16 (s, 2H), 3.26 - 2.97 (m, 2H), 2.86 (s, 3H), 2.80 - 2.66 (m, 1H), 2.35 (s, 1H), 1.99 - 1.88 (m, 1H), 0.98 (s, 1H), 0.90 - 0.81 (m, 4H), 0.41 - 0.29 (m, 2H), 0.16 (td, *J* 5.4, 3.9 Hz, 2H).

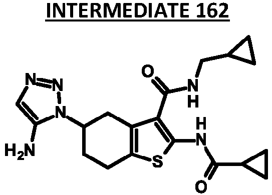

### 5-(5-Aminotriazol-1-yl)-2-(cyclopropanecarbonylamino)-N-(cyclopropylmethyl)-4,5,6,7-tetrahydrobenzothiophene-3-carboxamide

To a solution of *example 140* (81 mg, 0.16 mmol) in DCM (3 mL) at 0 °C was added TFA (3 mL, 39.7 mmol). The solution was stirred at 0 °C for 5 minutes then at r.t. for 2 h, the solvent was removed *in vacuo* and the excess TFA azetroped with isohexane. The residue was purified by flash column chromatography on silica (gradient elution with 0-100% MeOH/7M aqueous NH₃ in MeOH) to afford the *title compound* (40 mg, 61%) as a white solid. δ_{H} (300 MHz, Methanol-*d*₄) 6.97 (s, 1H), 4.57 (s, 1H), 3.33 - 3.09 (m, 2H), 2.96 (s, 3H), 2.43 (td, *J* 10.7, 10.1, 6.8 Hz, 1H), 2.34 (s, 1H), 1.81 (td, *J* 7.7, 3.8 Hz, 1H), 1.31 (s, 2H), 1.13 - 0.88 (m, 4H), 0.55 - 0.43 (m, 2H), 0.25 (dt, *J* 6.2, 4.5 Hz, 2H).

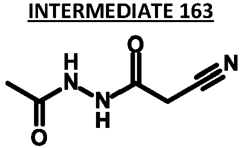

### N'-acetyl-2-cyano-acetohydrazide

2-cyanoacetohydrazide [140-87-4] (2 g, 20.18 mmol) was stirred in DCE (20 mL). Acetic anhydride (1.91 mL, 20.18 mmol) and pyridine (1.63 mL, 20.18 mmol) were added to the suspension. The reaction was stirred at 60 °C for 16 hours to give a fine light brown. The reaction was allowed to cool and the DCE was removed *in vacuo.* The residue was suspended in diethyl ether and collected by vacuum filtration to afford the *title compound* (2.82 g, 89% at 90% purity) as a yellow / pale brown solid. δ_{H} (500 MHz, DMSO-d₆) δ 10.18 - 10.13 (m, 1H), 9.95 (d, J 1.5 Hz, 1H), 3.73 (s, 2H), 1.86 (s, 3H).

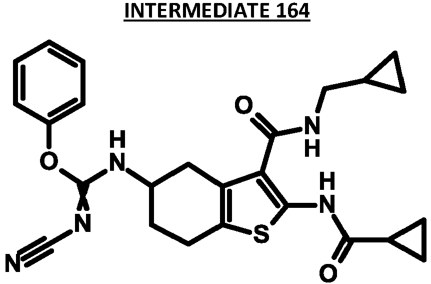

### 5-[(N-cyano-C-phenoxy-carbonimidoyl)amino]-2-(cyclopropanecarbonylamino)-N-(cyclopropylmethyl)-4,5,6,7-tetrahydrobenzothiophene-3-carboxamide

*Intermediate 122* (100 mg, 0.27 mmol) was dissolved in isopropanol (5 mL) and DIPEA (52.7 mg, 0.41 mmol) and diphenyl N-cyanocarbonimidate [79463-77-7] (79.7 mg, 0.32 mmol) were added. The reaction mixture was stirred at r.t. for ~1 h. p-Toluidine [106-49-0] (31.9 mg, 0.30 mmol) was added and the reaction mixture was stirred at r.t for ~4 h. The reaction mixture was heated at 80 °C for 30 minutes. The precipitate was filtered, washing with isopropanol and Et₂O to afford the *title compound* (70 mg, 0.15 mmol, 54%). LCMS [M+H]⁺ 478.2, [M+Na]⁺ 500.2, RT 2.174 minutes, 100.0% purity (*Method 1*)*.*

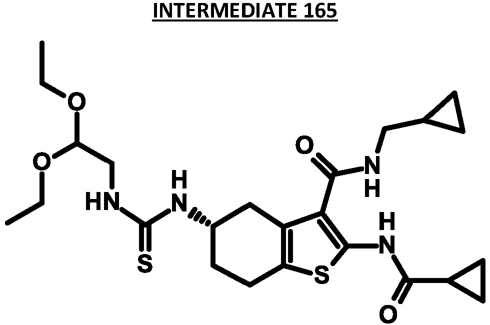

### (5S)-2-(Cyclopropanecarbonylamino)-N-(cyclopropylmethyl)-5-(2,2-diethoxyethylcarbamothioylamino)-4,5,6,7-tetrahydrobenzothiophene-3-carboxamide

To a stirred solution of phenyl chloromethanethioate [1005-56-7] (0.46 ml, 3.3 mmol) in DCM (30 mL), previously cooled to 0 °C was added a solution of *intermediate 117* (1.0 g, 3.0 mmol) and triethylamine (1.25 ml, 9.0 mmol) in DCM (15 mL) dropwise under an atmosphere of nitrogen. The reaction mixture was stirred at r.t. for a further 3 h and a solution of 2,2-diethoxyethanamine [645-36-3] (0.87 ml, 6.0 mmol) in DCM (5 mL) was added dropwise. The reaction was stirred at r.t. for 16 hours, the mixture diluted with EtOAc (150 mL) and washed with 0.5M aqueous citric acid solution (25 mL), water (20 mL), saturated aqueous sodium hydrogen carbonate (2 x 20 mL) and brine (20 mL). The organic layer was dried (Na₂SO₄), filtered and the solvent removed in vacuo. The residue was purified by flash column chromatography on silica (gradient elution with EtOAc in heptane 5% to 100%) to afford the *title compound* (1.12 g, 70%) as a pale yellow foamy solid. δ_{H} (500 MHz, DMSO-*d*₆) 11.12 (s, 1H), 7.74 - 7.59 (m, 2H), 7.28 (s, 1H), 4.56 (s, 1H), 4.50 (s, 1H), 3.64 - 3.57 (m, 2H), 3.56 - 3.43 (m, 3H), 3.20 - 3.07 (m, 2H), 3.02 (d, *J* 13.1 Hz, 1H), 2.69 (t, *J* 5.9 Hz, 2H), 2.55 (dd, *J* 16.2, 7.2 Hz, 1H), 1.98 - 1.87 (m, 2H), 1.87 - 1.75 (m, 1H), 1.15 - 1.09 (m, 6H), 1.07 - 0.99 (m, 1H), 0.87 - 0.81 (m, 5H), 0.45 - 0.39 (m, 2H), 0.25 - 0.20 (m, 2H). LCMS: [M-H]⁻ 507 , RT = 3.05 min, 96 % purity (*Method 16*).

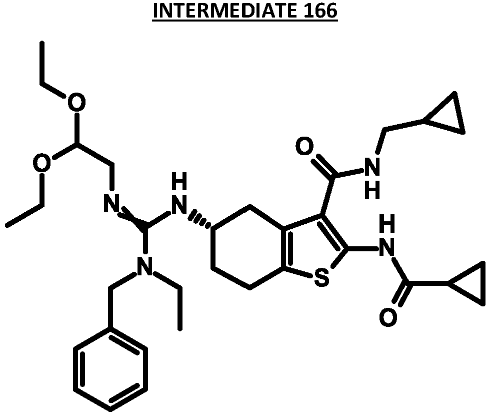

### (5S)-5-[[N-benzyl-N'-(2,2-diethoxyethyl)-N-ethyl-carbamimidoyl]amino]-2-(cyclopropanecarbonylamino)-N-(cyclopropylmethyl)-4,5,6,7-tetrahydrobenzothiophene-3-carboxamide

To a stirred solution of *intermediate 165* (500 mg, 0.98 mmol), triethylamine (0.41 ml, 2.95 mmol) and DMAP [1122-58-3] (12 mg, 0.1 mmol) in DCM (16 mL) previously cooled to 0 °C, was added a solution of methanesulfonyl chloride [124-63-0] (0.17 ml, 2.17 mmol) in DCM (2 mL) dropwise under an atmosphere of nitrogen. The reaction mixture was stirred at r.t. for a further 2 h and once the carbodiimide formation was complete, a solution of N-benzylethanamine [14321-27-8] (0.44 ml, 2.95 mmol) in DCM (2 mL) was added dropwise at r.t. and the reaction mixture was then stirred at r.t. for 3 h. A second portion of N-benzylethanamine [14321-27-8] (0.44 ml, 2.95 mmol) was added dropwise at r.t. and stirring continued at r.t. for another 16 h. The reaction mixture was heated under reflux for 48 h and the reaction mixture was left to cool to r.t., diluted with DCM (40 mL) and washed with saturated ammonium chloride solution (10 mL), 0.5M aqueous citric acid solution (10 mL), water (20 mL), 2M aqueous NaOH solution (20 mL) and brine (20 mL). The organic layer was dried (Na₂SO₄), filtered and the solvent removed in vacuo to afford the *title compound* (640 mg, 92%, 86% purity) as a yellow oil which was used without further purification. LCMS: [M-H]⁻ 608, RT = 1.92 min, 86 % purity (*Method 17*).

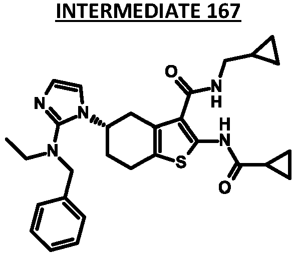

### (5S)-5-[2-[Benzyl(ethyl)amino]imidazol-1-yl]-2-(cyclopropanecarbonylamino)-N-(cyclopropylmethyl)-4,5,6,7-tetrahydrobenzothiophene-3-carboxamide

To a stirred solution of *intermediate 166* (640 mg, 0.89 mmol) in DCM (20 mL) was added 4-methylbenzenesulfonic acid hydrate (1:1) [6192-52-5] (1.35g, 7.14 mmol). The reaction mixture was stirred at r.t. for 2 h. The reaction mixture was diluted with DCM (20 mL) and washed with saturated aqueous sodium hydrogen carbonate solution (20 mL), water (20 mL) and brine (20 mL). The organic layer was dried (Na₂SO₄), filtered and concentrated *in vacuo* to give an orange oil. The residue was purified by flash column chromatography on silica (gradient elution with EtOAc in heptane 25% to 100%) to afford the *title compound* (238 mg, 50%) as a pale yellow foamy solid. δ_{H} (500 MHz, DMSO-*d*₆) 11.06 (s, 1H), 7.68 (t, *J* 5.3 Hz, 1H), 7.25 - 7.17 (m, 3H), 7.15 (d, *J* 6.8 Hz, 2H), 6.92 (d, *J* 1.2 Hz, 1H), 6.79 (d, J 0.9 Hz, 1H), 4.40 - 4.31 (m, 1H), 4.06 (d, J 12.7 Hz, 1H), 4.02 (d, *J* 12.7 Hz, 1H), 3.15 - 3.06 (m, 2H), 3.05 - 2.94 (m, 2H), 2.90 - 2.79 (m, 1H), 2.74 - 2.57 (m, 3H), 1.97 - 1.82 (m, 2H), 1.40 - 1.30 (m, 1H), 0.99 - 0.92 (m, 1H), 0.89 (t, *J* 7.1 Hz, 3H), 0.87 - 0.80 (m, 4H), 0.38 - 0.26 (m, 2H), 0.20 - 0.10 (m, 2H). LCMS: (M+H)⁺ 518, RT = 2.53 min, 98 % purity (*Method 10*).

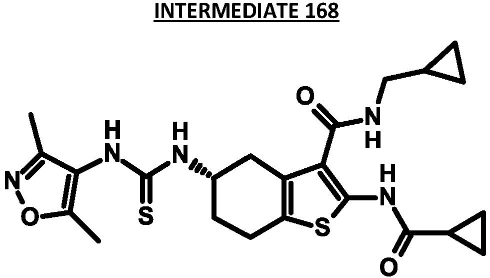

### (5S)-2-(Cyclopropanecarbonylamino)-N-(cyclopropylmethyl)-5-[(3,5-dimethylisoxazol-4-yl)carbamothioylamino]-4,5,6,7-tetrahydrobenzothiophene-3-carboxamide

*Intermediate 117* (400 mg, 1.2 mmol) was dissolved in DCM (5 mL) and 4-isothiocyanato-3,5-dimethyl-1,2-oxazole [321309-27-7] (222 mg, 1.44 mmol) in DCM was added to the reaction mixture and the solution was stirred for 18 h under nitrogen. The reaction mixture was concentrated in *vacuo* to give a pale yellow solid which was purified by flash column chromatography on silica (gradient elution with 0 - 100% EtOAc in heptane followed by 0-20% MeOH in DCM) to afford the *title compound* (475 mg, 81%). δ_{H} (500 MHz, DMSO-*d*₆) 11.04 (s, 1H), 8.64 (s, 1H), 7.85 (s, 1H), 7.73 (t, *J* 5.5 Hz, 1H), 4.52 (s, 1H), 3.19 - 3.09 (m, 2H), 3.03 (br d, *J* 12.4 Hz, 1H), 2.72 (br. s., 2H), 2.64 - 2.61 (m, 1H), 2.22 (s, 3H), 2.07 (s, 3H), 1.94 - 1.89 (m, 1H), 1.87 - 1.78 (m, 1H), 1.06 - 1.01 (m, 1H), 0.89 - 0.77 (m, 5H), 0.45 - 0.40 (m, 2H), 0.25 - 0.20 (m, 2H). LCMS [M+H]⁺ 488.20/486.20, RT 1.80 minutes, 99% purity (*Method 12*).

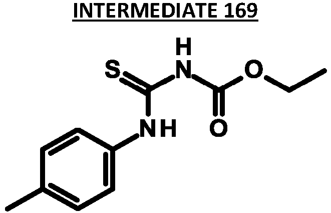

### Ethyl N-(p-tolylcarbamothioyl)carbamate

A solution of ethyl carbonisothiocyanatidate [16182-04-0] (0.22 mL, 1.86 mmol) in DCM (20 mL) was cooled to 0 °C before adding 4-methylaniline [106-49-0] (200 mg, 1.86 mmol). The ice bath was removed and the solution was stirred for 4 h under nitrogen and the solvent was removed *in vacuo* to yield a pale yellow solid which was triturated with heptane to afford the *title compound* (420 mg, 94%) as a white solid. LCMS [M+H]⁺ 238.95, RT 1.16 minutes, 100% purity (*Method 6*)*.*

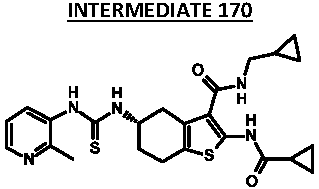

### (5S)-2-(Cyclopropanecarbonylamino)-N-(cyclopropylmethyl)-5-[(2-methyl-3-pyridyl)carbamothioylamino]-4,5,6,7-tetrahydrobenzothiophene-3-carboxamide

Phenyl chloromethanethioate [1005-56-7] (91 µL, 0.66 mmol) was stirred in DCM (5 mL) and cooled to 0 °C. *Intermediate 117* (200 mg, 0.6 mmol) in DCM (5 mL) and triethylamine (0.25 mL, 1.8 mmol) was added dropwise. The reaction was stirred for 30 minutes at r.t. and the reaction was concentrated *in vacuo* and the residue dissolved in 1,4-dioxane (5 mL). 2-Methylpyridin-3-amine [3430-10-2] (130 mg, 1.2 mmol) was added and the reaction stirred at 80 °C for 30 minutes followed by heating at 120 °C for 90 minutes. The reaction was diluted with EtOAc (40 mL) and washed with 0.5 M aqueous citric acid solution (20 mL) and saturated aqueous sodium hydrogen carbonate solution (20 mL), dried (Na₂SO₄), filtered and concentrated *in vacuo* to give a brown residue. The acidic aqueous phase was basified to pH 9 with saturated aqueous sodium hydrogen carbonate solution and was extracted with EtOAc (2 x 20 mL). The organic phases were combined and concentrated *in vacuo.* The brown residues were purified by flash column chromatography on silica (gradient elution with 0 - 100 % EtOAc followed by 0 - 10 % MeOH in DCM) to afford the *title compound* (172 mg, 54%, 91% purity) as a brown oil. LCMS [M+H]⁺ 484.2, RT 1.668 minutes, 90.5% purity (*Method 12*)*.*

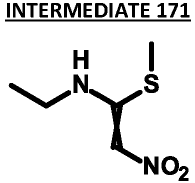

### N-ethyl-1-methylsulfanyl-2-nitro-ethenamine

1,1-Bis(methylthio)-2-nitroethylene [13623-94-4] (500 mg, 2.87 mmol) was taken up in DCM (2 mL) and 2.0M ethylamine solution (1.3 g, 3.2 mmol) was added. The reaction mixture was stirred at 80°C for 30 minutes in the microwave. The reaction mixture was concentrated *in vacuo* to afford the *title compound* (480 mg, 103%) as a brown oil which was utilised without purification. LCMS [M+H]⁺ 163.2, [M+Na]⁺ 185.2, RT 0.633 minutes, 73.7% purity (*Method 1*)*.*

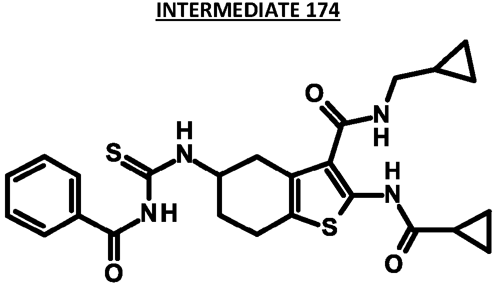

### 5-(Benzoylcarbamothioylamino)-2-(cyclopropanecarbonylamino)-N-(cyclopropylmethyl)-4,5,6,7-tetrahydrobenzothiophene-3-carboxamide

*Intermediate 122* (1.02 g, 2.75 mmol) was dissolved in DCM (50 mL) and cooled to 0 °C. DIPEA (0.71 mL, 4.06 mmol) was added followed by benzoyl isocyanate [4461-33-0] (0.56 mL, 4.08 mmol) and the mixture was stirred at r.t. for 20 h. The reaction mixture was washed with water and brine, passed through phase separator cartridge and the organic phase was concentrated *in vacuo* to afford the crude product as a golden orange solid. The residue was purified by flash column chromatography on silica (gradient elution with EtOAc/hexanes 0% 1 CV, 0-20% 11 CV, 20% 2 CV, 20-50% 6 CV, 50% 1 CV, 50-100% 5 CV, 100% 8 CV) to afford the purified material. The crude fractions were combined *in vacuo* and the sample was dissolved in DCM/MeOH and the material was concentrated *in vacuo.* To the residue was added hexane and EtOAc, the mixture was filtered and the solid washed with hexane and the trituration procedure repeated with the filtrate. The second trituration was purified by flash column chromatography on silica (gradient elution in DCM/MeOH 0% 1 CV, 0-10% 9 CV, 10% 1 CV, 10-25% 10 CV) to afford the *title compound* (1.12 g, 82% Yield). LCMS [M+H]⁺ 497.0, [M+Na]⁺ 519.0, RT 2.483 minutes, 100.0% purity (*Method 1*)*.*

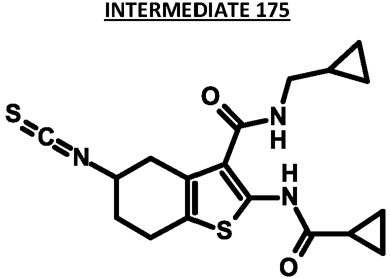

### 2-(Cyclopropanecarbonylamino)-N-(cyclopropylmethyl)-5-isothiocyanato-4,5,6,7-tetrahydrobenzothiophene-3-carboxamide

A solution of O-phenyl carbonochloridothioate [1005-56-7] (170 µL, 1.23 mmol) in DCM (10 mL) was added over 10 minutes to an ice-cold stirred solution of *example 109* (400 mg, 1.2 mmol) and triethylamine [121-44-8] (250 µL, 1.8 mmol) in DCM (25 mL). The mixture was stirred at 0 °C for 15 minutes and then the ice bath was removed. Water (50 mL) was added and the layers were separated. The aqueous phase was extracted with DCM (2 × 20 mL) and the combined organic extracts were dried (MgSO₄), filtered and concentrated *in vacuo* to give the crude intermediate which was purified by flash column chromatography on silica (gradient elution with 30-60% EtOAc/heptanes) to afford the *title compound* (360 mg, 32%, 40% purity) as a light yellow solid. δ_{H} (250 MHz, DMSO-*d*₆) 10.85 (s, 1H), 4.17 (s, 1H), 2.96 - 2.59 (m, 3H), 2.53 (d, *J* 8.1 Hz, 2H), 2.18 - 1.78 (m, 3H), 1.72 - 1.68 (m, 1H), 0.81 (d, *J* 6.1 Hz, 1H), 0.66 - 0.58 (m, 4H), 0.30 - 0.13 (m, 2H), 0.04 - 0.01 (m, 2H).

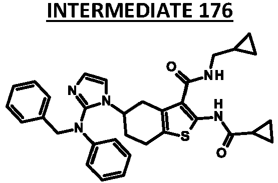

### 5-[2-(N-Benzylanilino)imidazol-1-yl]-2-(cyclopropanecarbonylamino)-N-(cyclopropylmethyl)-4,5,6,7-tetrahydrobenzothiophene-3-carboxamide

*Intermediate 175* (90%, 340 mg, 0.82 mmol) was dissolved in EtOH (10 mL) and 2,2-diethoxyethan-1-amine [645-36-3] (360 µL, 2.48 mmol) was added. After standing at r.t. for 16 h overnight and the solvent was removed *in vacuo* and the residue dissolved in EtOAc (20 mL) and washed with water (2 x 10 mL). The organic phase was dried (MgSO₄), filtered and concentrated *in vacuo* to afford *2-(Cyclopropanecarbonylamino)-N-(cyclopropylmethyl)-5-(2,2-diethoxyethylcarbamothioylamino)-4,5,6,7-tetrahydrobenzothiophene-3-carboxamide* (364 mg, 60.6%, 69% purity) as a yellow oil. LCMS (ES+) [M+Na]⁺ 531.0, RT 1.22 minutes, 69% purity (*Method 11*).

Methanesulfonyl chloride [124-63-0] (85 µL, 1.09 mmol) was added dropwise to a solution of 2-*(Cyclopropanecarbonylamino)-N-(cyclopropylmethyl)-5-(2,2-diethoxyethylcarbamothioylamino)-4,5,6,7-tetrahydrobenzothiophene-3-carboxamide* (69%, 364 mg, 0.49 mmol), triethylamine (225 µL, 1.61 mmol) and a catalytic amount of DMAP [1122-58-3] in DCM (20 mL), cooled in an ice bath. The resulting mixture was stirred for 10 minutes before removing the ice bath and stirring at r.t. for 1 h. N-Benzylaniline [103-32-2] (300 mg, 1.64 mmol) was added and stirring continued at r.t. for 16 h overnight. Further methanesulfonyl chloride [124-63-0] (113 mg, 0.99 mmol) was added prior to N-benzylaniline [103-32-2] addition (453 mg, 2.47 mmol) and further methanesulfonyl chloride [124-63-0] (85 µL, 1.09 mmol) was added, followed by N-benzylaniline [103-32-2] (200 mg, 1.09 mmol) and the reaction was stirred at r.t. for 3 days over the weekend. The reaction mixture was diluted with DCM (20 mL) and washed with saturated aqueous ammonium chloride (20 mL). The organic layer was dried (MgSO₄), filtered and concentrated *in vacuo* to afford *5-[[(E)-N-Benzyl-N'-(2,2-diethoxyethyl)-N-phenyl-carbamimidoyl]amino]-2-(cyclopropanecarbonylamino)-N-(cyclopropylmethyl)-4,5,6,7-tetrahydrobenzothiophene-3-carboxamide* (970 mg, 90%, 30% purity) as a yellow oil which was used directly in the next step without purification.

*5-[[(E)-N-Benzyl-N'-(2,2-diethoxyethyl)-N-phenyl-carbamimidoyl]amino]-2-(cyclopropanecarbonylamino)-N-(cyclopropylmethyl)-4,5,6,7-tetrahydrobenzothiophene-3-carboxamide* (30%, 970 mg, 0.44 mmol) was dissolved in DCM (30 mL) and *p*-toluene sulfonic acid [6192-52-5] (675 mg, 3.55 mmol) was added. The reaction mixture was stirred at r.t. for 2 h before diluting with DCM (30 mL) and washing with water (20 mL). The organic phase was dried (MgSO₄), filtered and concentrated *in vacuo* to give the crude product which was purified by flash column chromatography on silica (gradient elution with 30-60% EtOAc/heptane) to afford the *title compound* (229 mg, 81%, purity 89%) as a light yellow glass. LCMS [M+H]⁺ 566.2, RT 1.13 minutes, 89% purity (*Method 11*).

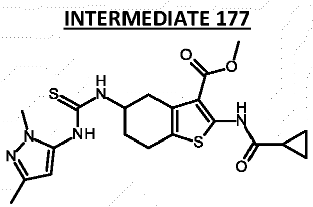

Methyl 2-(cyclopropanecarbonylamino)-5-[(2,5-dimethylpyrazol-3-yl)carbamothioylamino]-4,5,6,7-tetrahydrobenzothiophene-3-carboxylate. To a stirred solution of *intermediate 184* (1.5 g, 5.1 mmol) in dichloromethane (30 mL) was added N,N-diisopropylethylamine (1.3 mL, 7.5 mmol) and 5-isothiocyanato-1,3-dimethyl-pyrazole (1 g, 6.5274 mmol). The reaction mixture was stirred at room temperature for 2 hours and then evaporated *in vacuo.* The resulting residue was dissolved in DCM and washed with water. Purified by chromatography (Biotage SNAP Ultra 25 g, Isolera) with a gradient of 0% to 100% ethyl acetate in iso-hexane to afford the *title compound* (2.12 g, 4.74 mmol, 93% Yield) as a white solid. ¹H NMR (300 MHz, Chloroform-d) δ 11.42 (s, 1H), 7.17 (s, 1H), 6.10 (d, *J* = 8.3 Hz, 1H), 5.89 (s, 1H), 4.79 (s, 1H), 3.90 (s, 3H), 3.71 (s, 3H), 3.28 (dd, *J* = 17.1, 5.5 Hz, 1H), 2.95 - 2.75 (m, 1H), 2.65 (dt, *J* = 17.2, 5.9 Hz, 2H), 2.24 (s, 3H), 2.14 (d, *J* = 6.8 Hz, 1H), 1.99 - 1.83 (m, 1H), 1.69 (td, *J* = 8.0, 4.0 Hz, 1H), 1.16 (dt, *J* = 4.6, 3.3 Hz, 2H), 0.97 (dd, *J* = 7.8, 3.2 Hz, 2H). LCMS [M+H]⁺ 448.0, RT 1.39 minutes (*Method 15*)

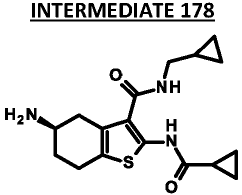

### (5R)-5-Amino-2-(cyclopropanecarbonylamino)-N-(cyclopropylmethyl)-4,5,6,7-tetrahydrobenzothiophene-3-carboxamide

To a stirred solution of *intermediate 116* (5 g, 10 mmol) in DCM (25 mL) was added TFA (20 mL) dropwise over 15 minutes, maintaining the temperature below 25 °C and the reaction mixture was stirred at r.t. for a further 15 minutes. Toluene (30 mL) was added to the reaction mixture and the solvent was removed *in vacuo.* To the residue was added EtOAc (10 mL) followed by diisopropyl ether (350 mL) and the resulting slurry was stirred for 15 minutes. The precipitate was filtered, and the solid washed with diisopropyl ether (45 mL), Et₂O (45 mL) and dried under a flow of nitrogen for 15 minutes to give a beige solid as a TFA salt (6.28 g). The solid was dissolved in water (200 mL) containing TFA (1 mL) and Et₂O (50 mL) and EtOAc (5 mL) were added. The aqueous phase was separated and washed with Et₂O (25 mL). Potassium carbonate (8 g, 60 mmol) was added in portions and the aqueous phase extracted with 10% MeOH in EtOAc (100 mL x 2), combined organic extracts dried (Na₂SO₄), filtered and concentrated *in vacuo* to afford the *title compound* (3.71 g, 100%) as a pale yellow solid. δ_{H} (300 MHz, Chloroform-d) 12.19 (s, 1H), 5.97 (s, 1H), 3.31 - 3.15 (m, 3H), 3.10 - 2.94 (m, 1H), 2.88 - 2.65 (m, 2H), 2.44 (ddt, *J* 14.3, 8.4, 2.0 Hz, 1H), 2.10 - 1.93 (m, 1H), 1.77 - 1.62 (m, 2H), 1.15 - 1.01 (m, 3H), 0.94 - 0.80 (m, 2H), 0.62 - 0.51 (m, 2H), 0.27 (dt, *J* 6.0, 4.6 Hz, 2H). NH₂ protons not visible.

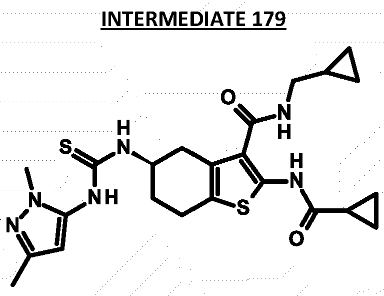

### (5R)-2-(Cyclopropanecarbonylamino)-N-(cyclopropylmethyl)-5-[(2,5-dimethylpyrazol-3-yl)carbamothioylamino]-4,5,6,7-tetrahydrobenzothiophene-3-carboxamide

To a stirred solution of *intermediate 178* (2.7 g, 8.1 mmol) in DCM (50 mL) was added DIPEA (2.1 mL, 12 mmol) and 5-isothiocyanato-1,3-dimethyl-1H-pyrazole [205246-65-7] (1.6 g, 10 mmol). The reaction mixture was stirred at r.t. for 17 h and concentrated *in vacuo.* The crude product was purified by flash column chromatography on silica (gradient elution with 0% to 100% EtOAc in *iso-*hexane) to afford the *title compound* (3.56 g, 90%) as an off-white solid. ¹H NMR (300 MHz, DMSO-*d*₆) 11.08 (s, 1H), 9.11 (s, 1H), 7.94 (d, *J* 7.7 Hz, 1H), 7.74 (t, *J* 5.6 Hz, 1H), 5.92 (s, 1H), 4.52 (m, 1H), 3.52 (s, 3H), 3.14 (t, *J* 6.1 Hz, 2H), 3.09 - 2.96 (m, 1H), 2.78 - 2.56 (m, 3H), 2.09 (s, 3H), 2.03 - 1.82 (m, 3H), 1.01 (m, 1H), 0.92 - 0.75 (m, 4H), 0.50 - 0.36 (m, 2H), 0.30 - 0.17 (m, 2H).

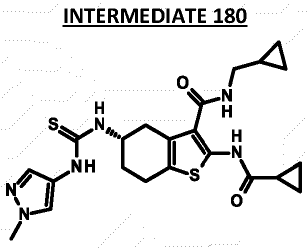

### (5S)-2-(Cyclopropanecarbonylamino)-N-(cyclopropylmethyl)-5-[(1-methylpyrazol-4-yl)carbamothioylamino]-4,5,6,7-tetrahydrobenzothiophene-3-carboxamide

*Intermediate 117* (400 mg, 1.2 mmol) was dissolved in DCM (5 mL) and 4-isothiocyanato-1-methyl-1H-pyrazole [1001500-53-3] (200 mg, 1.44 mmol) in DCM (5 mL) was added to this mixture and the solution was stirred for 54 h under nitrogen. The reaction mixture was concentrated *in vacuo* to give a pale yellow solid and the crude product was purified by flash column chromatography on silica (gradient elution with 0-100% EtOAc in heptane, 0-40% MeOH in DCM) to afford the *title compound* (274 mg, 37%, 77% purity). δ_{H} (250 MHz, Methylene Chloride-*d*₂) 12.11 (s, 1H), 7.35 (s, 2H), 7.10 (s, 1H), 6.00 (d, *J* 7.4 Hz, 1H), 5.87 (s, 1H), 4.82 (s, 1H), 3.85 (s, 3H), 3.35 - 3.19 (m, 3H), 2.84-2.80 (m, 1H), 2.69-2.63 (m, 2H), 2.11-2.07 (m, 1H), 1.98-1.94 (m, 1H), 1.74 - 1.60 (m, 1H), 1.08 - 0.97 (m, 3H), 0.94 - 0.83 (m, 2H), 0.60 - 0.50 (m, 2H), 0.31 - 0.19 (m, 2H). LCMS [M+H]⁺ 473.20/471.20, RT 1.73 minutes, 77% purity (*Method 12*).

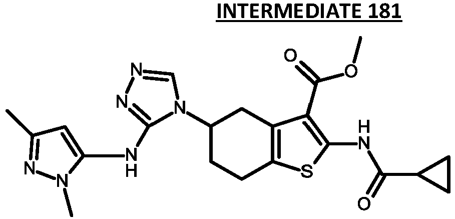

### Methyl 2-(cyclopropanecarbonylamino)-5-[3-[(2,5-dimethylpyrazol-3-yl)aminol-1,2,4-triazol-4-yl]-4,5,6,7-tetrahydrobenzothiophene-3-carboxylate

*Intermediate 177* (2.12g) was dissolved in N,N-dimethylformamide (40 mL) and formic acid hydrazide (853 mg, 14.2025 mmol) was added followed by mercuric chloride (3.88 mg, 0.0142 mmol) and triethylamine (2 mL, 14.3168 mmol). The resulting mixture was heated at 63 °C with a condenser for 5h. Volatiles and DMF were then evaporated. The resulting residue was diluted with MeCN (20 mL) and filtered through a pad of celite. MeCN was added to wash through celite and resulting clear yellow solution was concentrated *in vacuo* to give a yellow oil. This oil was re-dissolved in MeOH/DCM, added silica to it, and dry-loaded on column for purification. The crude product was purified by flash column chromatography on silica (gradient elution with 25g SNAP-Ultra, Isolera, MeOH/DCM, 0 to 10% in MeOH) to afford the *title compound* as white solid (0.882 g, 1.94 mmol, 40.9% Yield). ¹H NMR (300 MHz, DMSO-*d*₆) δ 11.16 (s, 1H), 8.40 (s, 1H), 5.91 (s, 1H), 5.76 (s, 1H), 4.44 (s, 1H), 3.83 (s, 3H), 3.63 - 3.46 (m, 3H), 3.37 (m, 1H), 2.83 (s, 3H), 2.21 (dd, *J* = 19.2, 11.0 Hz, 2H), 2.09 (d, *J* = 4.0 Hz, 3H), 1.08 - 0.82 (m, 4H). LCMS [M+H]⁺ 456.0, RT=1.51 minutes (*Method 28*).

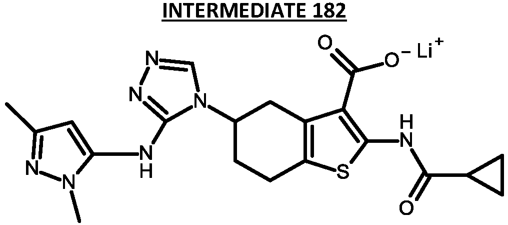

### lithium;2-(cyclopropanecarbonylamino)-5-[3-[(2,5-dimethylpyrazol-3-yl)amino]-1,2,4-triazol-4-yl]-4,5,6,7-tetrahydrobenzothiophene-3-carboxylate

*Intermediate 181* (453 mg, 0.9945 mmol) was suspended in THF (10 mL) and dissolved completely by adding water (1 mL) to it. Then lithium hydroxide monohydrate (66 mg, 1.57 mmol) was added and the reaction was stirred at rt for 2 days until complete. Volatiles were evaporated *in vacuo* until the solid was dry. Used without further purification in amide coupling reactions. ¹H NMR (300 MHz, DMSO-*d*₆) δ 14.54 (s, 1H), 7.72 (s, 1H), 5.78 (d, *J* = 13.3 Hz, 1H), 4.39 (s, 1H), 3.53 - 3.38 (m, 4H), 2.87 - 2.58 (m, 3H), 2.10 (dd, *J* = 11.1, 5.6 Hz, 1H), 1.99 (d, *J* = 12.1 Hz, 3H), 1.88 - 1.74 (m, 1H), 1.74 - 1.57 (m, 1H), 0.87 - 0.77 (m, 4H). LCMS [M+H]⁺ 442.0, RT=0.904 minutes (*Method 15*).

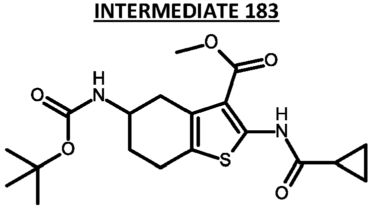

### Methyl-5-(tert-butoxycarbonylamino)-2-(cyclopropanecarbonylamino)-4,5,6,7-tetrahydrobenzothiophene-3-carboxylate

*Intermediate 26* (3 g, 7.884 mmol) was dissolved in methanol (30 mL) and toluene (60 mL) and (trimethylsilyl)diazomethane in hexanes (4.3 mL, 8.6 mmol, 2 mol/L) was added in portions at rt. Bubbling was observed for a few minutes, then ceased. The reaction was heated at 70 °C for 3h, then volatiles were evaporated *in vacuo.* The residue was dissolved in DCM and washed with water. The crude product was purified by flash column chromatography on silica (gradient elution with 100g SNAP-Ultra, Isolera, eluting with 0 to 50% EtOAc in i-Hexane) to afford the *title compound* (2.117 g, 5.366 mmol, 68.06% Yield) as a yellow oil. ¹H NMR (300 MHz, Chloroform-d) δ 11.44 (s, 1H), 4.00 (s, 1H), 3.89 (s, 3H), 3.16 (dd, *J* = 17.1, 5.4 Hz, 1H), 2.82 - 2.68 (m, 2H), 2.61 (dd, *J* = 17.2, 7.4 Hz, 1H), 2.05 (tdd, *J* = 8.3, 5.8, 2.5 Hz, 1H), 1.75 (s, 1H), 1.69 (tt, *J* = 7.8, 4.5 Hz, 1H), 1.60 (s, 3H), 1.48 (s, 9H), 1.21 - 1.10 (m, 2H), 0.97 (dt, *J* = 8.1, 3.5 Hz, 2H). LCMS [M+H]⁺ 395.0, RT 1.57 minutes *(Method 15).*

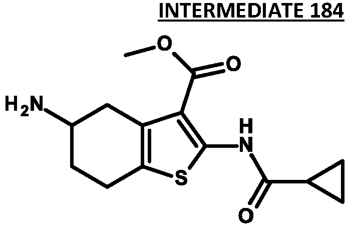

### Methyl 5-amino-2-(cyclopropanecarbonylamino)-4,5,6,7-tetrahydrobenzothiophene-3-carboxylate

*Intermediate 183* (2.170 g, 5.501 mmol) was dissolved in dichloromethane (50 mL) and trifluoroacetic acid (6 mL, 79.35 mmol) was added. The reaction was stirred at rt for 2h, then volatiles were evaporated *in vacuo* and the resulting residue was dissolved in methanol, loaded on 3*10g SCX-2 columns and eluted with 3N NH₃ in methanol. This purification afforded the *title compound* (1.5 g, 5.1 mmol, 93% Yield) of as yellow solid. ¹H NMR (300 MHz, DMSO-*d*₆) δ 11.13 (s, 1H), 3.84 (s, 3H), 3.04 - 2.88 (m, 2H), 2.75 - 2.54 (m, 2H), 2.38 - 2.20 (m, 1H), 2.04 (tt, *J* = 7.3, 5.1 Hz, 1H), 1.86 (d, *J* = 13.3 Hz, 3H), 1.47 (dtd, *J* = 12.4, 9.7, 5.9 Hz, 1H), 1.00 - 0.76 (m, 4H). LCMS [M+H]⁺ 295.0, RT 0.83 minutes *(Method 15).*

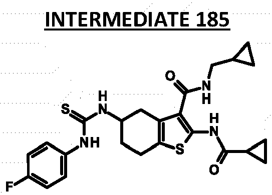

### 2-(Cyclopropanecarbonylamino)-N-(cyclopropylmethyl)-5-[(4-fluorophenyl)carbamothioylamino]-4,5,6,7-tetrahydrobenzothiophene-3-carboxamide

A solution of 1-fluoro-4-isothiocyanatobenzene [154257-75-7] (110 µl, 0.9 mmol) in DCM (5 mL) was stirred at r.t. before adding *example 109* (200 mg, 0.6 mmol). The solution was stirred for 18 h under nitrogen and the reaction mixture was concentrated *in vacuo* to give a pale yellow solid. The crude product was purified by flash column chromatography on silica (gradient elution with 0% to 100% EtOAc in heptane) to afford the *title compound* (267 mg, 91%) as a white solid. δ_{H} (250 MHz, Chloroform-d) 12.09 (s, 1H), 7.78 (s, 1H), 7.20 - 7.02 (m, 4H), 6.11 (d, *J* 7.5 Hz, 1H), 5.86 (s, 1H), 4.95 (s, 1H), 3.33 - 3.10 (m, 3H), 2.86-2.80 (m, 2H), 2.68 - 2.50 (m, 1H), 2.08 - 1.99 (m, 2H), 1.69-1.62 (m, 2H), 1.11-1.04 (m, 3H), 0.94 - 0.84 (m, 2H), 0.61-0.55 (m, 2H), 0.31-0.25 (m, 2H).

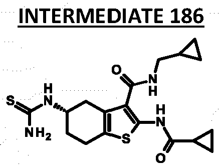

### (5S)-5-(Carbamothioylamino)-2-(cyclopropanecarbonylamino)-N-(cyclopropylmethyl)-4,5,6,7-tetrahydrobenzothiophene-3-carboxamide

Phenyl chloromethanethioate [1005-56-7] (0.23 mL, 1.65 mmol) was dissolved in DCM (10 mL) and the reaction mixture was cooled to 0 °C. *Intermediate 117* (500 mg, 1.5 mmol) in DCM (10 mL) and triethylamine (0.63 mL, 4.5 mmol) was added dropwise. The reaction was stirred at r.t. for 20 minutes and the reaction mixture was split in to two, and one portion carried through to the desired reaction. To (5S)-2-cyclopropaneamido-N-(cyclopropylmethyl)-5-[(phenoxymethanethioyl)amino]-4,5,6,7-tetrahydro-1-benzothiophene-3-carboxamide (352 mg, 0.75 mmol) in DCM (10 mL) was added aqueous ammonia (2 mL, 34.1 mmol) and the mixture was stirred at r.t. for 18 h. The reaction mixture was diluted with DCM (20 mL) and water (20 mL) followed by brine (50 mL) and the mixture was extracted with DCM (3 x 20 mL). The organic phases were washed with brine (20 mL), dried (Na₂SO₄), filtered and concentrated *in vacuo* to yield a yellow solid. The crude product was triturated with DCM / heptane and collected by filtration to afford the *title compound* (77 mg, 26 %) as a white solid. LCMS [M+H]⁺ 393.2, RT 1.659 minutes, 100% purity (*Method 12*)*.* The aqueous layer was extracted with isopropanol/chloroform (2 x 20 mL), and the organic phases were dried (Na₂SO₄), filtered, combined with the previous filtrate and concentrated *in vacuo* to afford a yellow oil (80 mg). The aqueous phase was neutralised to pH 7 using 1M aqueous hydrochloric acid and the aqueous phase was extracted with DCM (3 x 20 mL). The organic phases were dried (Na₂SO₄), filtered, combined with the above yellow oil and concentrated *in vacuo* to afford a yellow solid (300 mg). DCM and heptane were added to the crude solid, which was sonicated to give a white solid which was collected by filtration to afford the *title compound* (117 mg, 40 %) as a white solid. LCMS [M+H]⁺ 393.10, RT 1.05 minutes, 95% purity *(Method 11).*

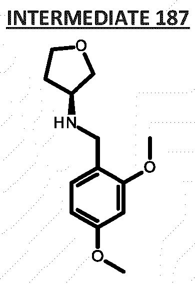

### (3S)-N-[(2,4-dimethoxyphenyl)methylltetrahydrofuran-3-amine

To a stirred solution of (3S)-tetrahydrofuran-3-amine (75 mg, 0.86 mmol) in DCM (3 mL) was added 2,4-dimethoxybenzaldehyde (143 mg, 0.86 mmol), followed by STAB (273 mg, 1.29 mmol). The mixture was stirred at r.t. for 2 hours. Saturated sodium bicarbonate solution was added and the organic phase taken. The aqueous phase was extracted with DCM (4 x 3 mL) and the organic phases combined, concentrated *in vacuo* then purified by flash column chromatography on aminopropyl silica (gradient elution with 0-2% methanol in TBME) to afford the *title compound* (175 mg, 75%) as a colourless oil. δ_{H} (250 MHz, DMSO-d₆) 7.17 (d, J 8.2 Hz, 1H), 6.51 (d, *J* 2.4 Hz, 1H), 6.46 (dd, *J* 8.2, 2.4 Hz, 1H), 3.76 (s, 3H), 3.74 - 3.58 (m, 7H), 3.58 - 3.53 (m, 2H), 3.42 - 3.37 (m, 1H), 3.26 - 3.16 (m, 1H), 2.00 - 1.83 (m, 1H), 1.70 - 1.56 (m, 1H). LCMS [M+H]⁺ 238.1, RT 0.4 minutes, purity 90% *(Method 14).*

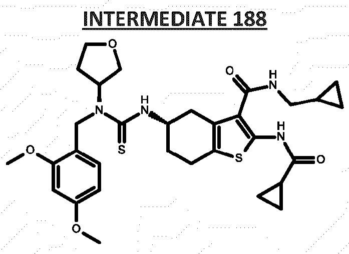

### (5S)-2-(cyclopropanecarbonylamino)-N-{cyclopropylmethyl)-5-[[(2,4-dimethoxyphenyl)methyl-[(3S)-tetrahydrofuran-3-yl]carbamothioyl]amino]-4,5,6,7-tetrahydrobenzothiophene-3-carboxamide

To a stirred solution of phenyl chloromethanethioate (45.7 µL, 0.33 mmol) in DCM (5 mL), at 0 °C was added *intermediate 117* (100 mg, 0.3 mmol) and triethylamine (0.13 ml, 0.9 mmol) in DCM (5 mL). The reaction was stirred at 0 °C for 30 minutes, then *intermediate 187* (85.4 mg, 0.36 mmol) was added. The mixture was stirred at 0 °C for 15 minutes, then warmed to room temperature and stirred for 18 h. The reaction was concentrated *in vacuo* and purified by flash column chromatography on silica (gradient elution with 0-100% TBME in heptane) to afford the *title compound* (182 mg, 91%) as a white powder. δ_{H} (500 MHz, DMSO-d₆) 11.06 (s, 1H), 7.69 - 7.59 (m, 1H), 6.87 (d, J 7.3 Hz, 1H), 6.75 (d, J 8.4 Hz, 1H), 6.52 (d, J 2.3 Hz, 1H), 6.44 (dd, J 8.4, 2.3 Hz, 1H), 5.69 (s, 1H), 4.72 - 4.60 (m, 3H), 3.82 (td, J 8.6, 3.9 Hz, 1H), 3.77 (s, 3H), 3.73 (s, 3H), 3.69 - 3.62 (m, 1H), 3.54 (dd, *J* 9.7, 4.3 Hz, 1H), 3.49 (q, J 8.5 Hz, 1H), 3.12 (t, J 6.2 Hz, 2H), 2.99 - 2.89 (m, 1H), 2.67 - 2.58 (m, 1H), 2.57 - 2.53 (m, 1H), 2.15 (dtd, J 12.7, 8.6, 3.9 Hz, 1H), 1.94 - 1.86 (m, 2H), 1.83 - 1.74 (m, 1H), 1.74 - 1.66 (m, 1H), 1.05 - 0.96 (m, 1H), 0.88 - 0.79 (m, 4H), 0.45 - 0.36 (m, 2H), 0.24 - 0.17 (m, 2H), 1 proton under DMSO. LCMS: [M+H]⁺ 613.2, RT 1.28 minutes, purity 92% *(Method 11).*

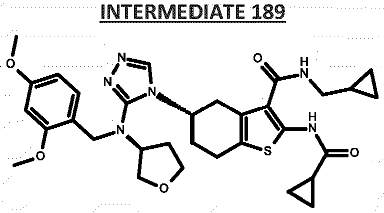

### (5S)-2-(cyclopropanecarbonylamino)-N-(cyclopropylmethyl)-5-[3-[(2,4-dimethoxyphenyl)methyl-[(3S)-tetrahydrofuran-3-yl]amino]-1,2,4-triazol-4-yl]-4,5,6,7-tetrahydrobenzothiophene-3-carboxamide

To a stirred solution of *intermediate 188* (75 mg, 0.11 mmol) in DMF (1 mL) was added formic hydrazide (14 mg, 0.23 mmol), followed by mercury dichloride (61 mg, 0.23 mmol). The reaction mixture was stirred at r.t. for 5 minutes, then triethylamine (47 µL, 0.34 mmol) was added and the suspension heated to 70 °C for 1 h. The suspension was cooled to r.t., diluted with DCM and kieselguhr added. The mixture was stirred for 1 minute then filtered. The filtrate was concentrated *in vacuo* to give a pale-yellow oil, which was purified by flash column chromatography on silica (gradient elution with 0-15% methanol in TBME) to afford the *title compound* (48 mg, 60%) as a white powder. LCMS [M+H]⁺ 621.3, RT 1.14 minutes, purity 89% *(Method 11).*

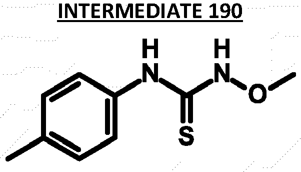

### 1-Methoxy-3-(p-tolyl)thiourea

A solution of 1-isothiocyanato-4-methylbenzene [622-59-3] (150 mg, 1.0 mmol) in THF (15mL) was cooled to 0°C prior to addition of (aminooxy)methane hydrochloride (1:1) (420 mg, 5.0 mmol) and DIPEA (1.0 mL, 6.0 mmol). The ice bath was removed and the solution was stirred for 16 h under nitrogen. The resulting solid was filtered off and the filtrate was concentrated *in vacuo* to yield the crude product as a pale yellow gum which was dissolved in DCM and washed with 1 M aqueous hydrochloric acid solution, saturated sodium hydrogen carbonate solution and brine. The organic phase was dried (MgSO₄), filtered and concentrated *in vacuo* to give a pale yellow solid which was purified by flash column chromatography on silica (gradient elution with 12 to 60% EtOAc in heptane) to afford the *title compound* (102 mg, 51%) as a white solid. LCMS [M+H]⁺ 196.9, RT 0.99 minutes, 99% purity *(Method 6).*

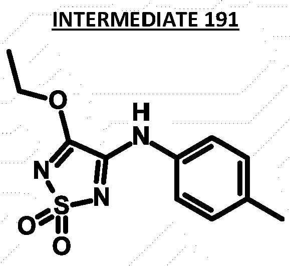

### 4-ethoxy-1,1-dioxo-N-(p-tolyl)-1,2,5-thiadiazol-3-amine

4-Methylaniline [106-49-0] (67.5 mg, 0.63 mmol) was dissolved in EtOH (30 ml) and 3,4-diethoxy-1-{6},2,5-thiadiazole-1,1-dione [55904-84-2] (130 mg, 0.63 mmol) was added and the reaction mixture was stirred at r.t. for 16 h. The reaction mixture was concentrated *in vacuo* to give the crude *title compound* (166 mg, 86%, 87% purity) which was utilised without further purification. LCMS [M+H]⁺ 267.95, RT 1.21 minutes, 87% purity *(Method 6).*

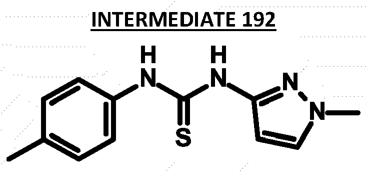

### 1-(1-Methylpyrazol-3-yl)-3-(p-tolyl)thiourea

A solution of 3-isothiocyanato-1-methyl-1H-pyrazole [114874-28-1] (195 mg, 1.4 mmol) in DCM (15 mL) was cooled to 0 °C before addition 4-methylaniline [106-49-0] (150 mg, 1.4 mmol). The ice bath was removed and the solution was stirred for 4 h under nitrogen. The reaction mixture was concentrated *in vacuo* to give the crude residue as a pale yellow solid which was triturated with Et₂O/heptane. The solid formed was collected by filtration and dried in vacuo to afford the *title compound* (310 mg, 90%) as a white solid. LCMS [M+H]⁺ 246.95, RT 1.09 minutes, 99% purity *(Method 6).*

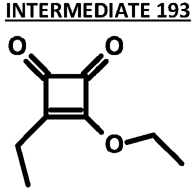

### 3-Ethoxy-4-ethyl-cyclobut-3-ene-1,2-dione

To a solution of diethoxycyclobut-3-ene-1,2-dione [5231-87-8] (0.2 mL, 1.35 mmol) in THF (7 mL) cooled to -78 °C and stirred under nitrogen, was added 3M ethyl magnesium bromide in Et₂O (0.47 mL, 1.42 mmoL) dropwise over a period of 30 seconds and the mixture was stirred for 1 h. Trifluoroacetic anhydride [407-25-0] (0.23 mL, 1.62 mmoL) was added followed by 10% aqueous ammonium chloride (1 mL) and the reaction mixture was warmed to r.t. and stirred for 1 h. The reaction mixture was partitioned between saturated sodium hydrogen carbonate solution (10 mL) and EtOAc (20 mL). The organic phase was separated and the aqueous phase was extracted with EtOAc (20 mL). The combined organic phases were washed with brine (10 mL), dried (MgSO₄), filtered and concentrated *in vacuo.* The pale yellow oil obtained was dissolved in DCM (5 mL), concentrated hydrochloric acid (2 drops) was added and the resulting mixture was stirred at r.t. for 15 minutes. The reaction mixture was dried (K₂CO₃), filtered and concentrated *in vacuo.* The crude residue was purified by flash column chromatography on silica (gradient elution with 0 to 50% of EtOAc in heptane) to afford the *title compound* (86 mg, 41%) as a pale yellow oil. δ_{H} (500 MHz, Chloroform-d) 4.78 (q, J 7.1 Hz, 2H), 2.63 (q, J 7.6 Hz, 2H), 1.49 (t, J 7.1 Hz, 3H), 1.27 (t, J 7.6 Hz, 3H). LCMS [M+H]⁺ 155, RT 0.90 minutes, 97% purity *(Method 11).*

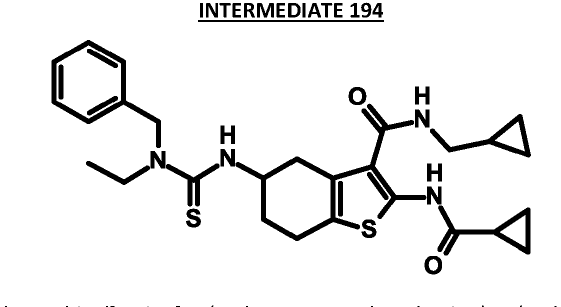

### 5-[[Benzyl(ethyl)carbamothioyl]amino]-2-(cyclopropanecarbonylamino)-N-(cyclopropylmethyl)-4,5,6,7-tetrahydrobenzothiophene-3-carboxamide

To *intermediate 122* (1.00 g, 2.70 mmol) in DCM (15 mL) was added triethylamine (0.82 g, 8.11 mmol). A solution of phenyl chloromethanethioate [1005-56-7] (513 mg, 2.97 mmol) in DCM (30 mL) was cooled to 0 °C and the amine / triethylamine solution was added dropwise at 0 °C under nitrogen. The reaction mixture was warmed to r.t. overnight. A solution of N-ethylbenzylamine [14321-27-8] (548 mg, 4.06 mmol) in DCM (5 mL) was added and the reaction was stirred at r.t. The reaction mixture was concentrated *in vacuo* and the residue was dissolved in EtOAc (60 mL) and washed with 1M aqueous citric acid solution (2 x 10 mL), saturated aqueous sodium hydrogen carbonate solution (2 x 10 mL) and brine (10 mL). The organic phase was dried (Na₂SO₄), filtered and the filtrate adsorbed onto silica for purification by flash column chromatography on silica (gradient elution with 200 mL of 8:2 heptane / EtOAc followed by 400 mL each of 7:3 - 6:4 - 5:5 - 4:6 - 3:7 and 2:8). Fractions were combined and concentrated *in vacuo* before azeotroping with EtOAc (10 mL). The residue was dried under high vacuum for 20 minutes to afford the *title compound* (865 mg, 62%) as an off-white foam. δ_{H} (250 MHz, CD₃OD) 12.17 (s, 1H), 7.27 - 7.24 (m, 3H), 7.14 - 7.06 (m, 2H), 5.77 (t, J 4.7 Hz, 1H), 5.47 (d, J 7.9 Hz, 1H), 5.10 - 5.00 (m, 1H), 4.71 - 4.63 (m, 2H), 3.93 (m, 2H), 3.26 (dd, J 7.2, 5.2 Hz, 2H), 3.03 (dd, J 15.5, 4.5 Hz, 1H), 2.68 (dt, J 19.0, 5.0 Hz, 1H), 2.58 (dd, J 15.2, 3.8 Hz, 1H), 2.34 - 2.23 (m, 1H), 2.02 - 1.97 (m, 1H), 1.89 - 1.81 (m, 1H), 1.71 - 1.66 (m, 1H), 1.25 (t, J 7.1 Hz, 3H), 1.14 - 1.09 (m, 2H), 1.08 - 1.00 (m, 1H), 0.94 - 0.88 (m, 2H), 0.61 - 0.56 (m, 2H), 0.27 (q, J 5.1 Hz, 2H). LCMS [M+H]⁺ 511, [M+Na]⁺ 533, RT 1.33 minutes, 99% purity *(Method 6).*

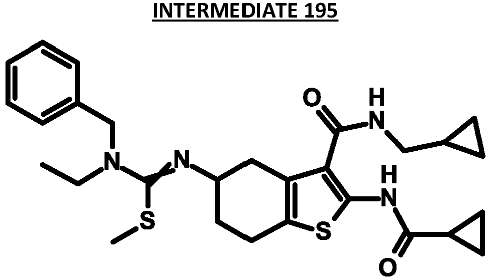

### Intermediate 195

### 5-[[[Benzyl(ethyl)aminol-methylsulfanyl-methylene]amino]-2-(cyclopropanecarbonylamino)-N-(cyclopropylmethyl)-4,5,6,7-tetrahydrobenzothiophene-3-carboxamide

To a solution of *intermediate 194* (865 mg, 1.69 mmol) in THF (18 mL) was added 1M potassium *tert-*butoxide in THF (5.08 mL, 5.08 mmol) dropwise at 0 °C and the reaction mixture was warmed to r.t. 5 minutes. The reaction mixture was cooled to 0 °C and iodomethane [74-88-4] (313 mg, 2.20 mmol) in THF (5 mL) was added. The reaction was then allowed to warm to r.t. and stirred for 30 minutes. The reaction mixture was diluted with EtOAc (150 mL) and washed with 1M aqueous citric acid solution (2 x 15 mL), saturated aqueous sodium hydrogen carbonate solution (2 x 15 mL) and brine (15 mL). The organic phase was dried (Na₂SO₄), filtered and concentrated *in vacuo* to afford the *title compound* (0.73 g, 75%, 92% purity) as a pale yellow foam. δ_{H} (500 MHz, Chloroform-d) 12.20 (s, 1H), 7.31- 7.20 (m, 5H), 5.97 (s, 1H), 4.60 - 4.45 (m, 2H), 4.06 (p, J 7.1, 6.6 Hz, 1H), 3.44 - 3.32 (m, 2H), 3.32 - 3.22 (m, 2H), 2.81 - 2.73 (m, 3H), 2.60 - 2.51 (m, 1H), 2.34 (s, 2.7H), 2.31 (s, 0.3 H), 1.89 - 1.79 (m, 2H), 1.66 (ddd, J 12.5, 7.9, 4.6 Hz, 1H), 1.15 - 1.07 (m, 5H), 1.07 - 0.99 (m, 1H), 0.87 (dp, J 10.3, 4.9 Hz, 2H), 0.56 - 0.50 (m, 2H), 0.25 (q, J 4.8 Hz, 2H). Peak broadening was observed at room temperature due to E/Z or conformational isomerism. LCMS [M+H]⁺ 525, RT 1.06 minutes, 92% purity *(Method 6).*

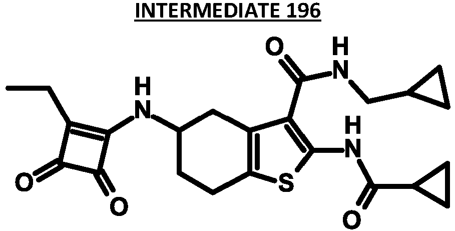

### 2-(Cyclopropanecarbonylamino)-N-(cyclopropylmethyl)-5-[(2-ethyl-3,4-dioxo-cyclobuten-1-yl)amino]-4,5,6,7-tetrahydrobenzothiophene-3-carboxamide

A solution of *example 109* (100 mg, 0.3 mmoL) and *intermediate 193* (56 mg, 0.36 mmoL) in EtOH was stirred at r.t. for 16 h. The reaction mixture was concentrated *in vacuo* and purified by preparative HPLC (low pH) to afford the *title compound* (86 mg, 65%) as a white solid. δ_{H} (250 MHz, Chloroform-d) 12.09 - 11.87 (m, 1H), 6.54 - 6.41 (m, 1H - Rotamer 1), 6.30 - 6.18 (m, 1H - Rotamer 2), 6.02 - 5.84 (m, 1H), 4.86 - 4.66 (m, 1H - Rotamer 2), 4.04 - 3.91 (m, 1H - Rotamer 1), 3.38 - 3.10 (m, 3H), 2.95 - 2.76 (m, 3H), 2.76 - 2.63 (m, 2H - Rotamer 1), 2.63 - 2.44 (m, 2H - Rotamer 2), 2.24 - 1.93 (m, 2H), 1.73 - 1.59 (m, 1H - part. obs. by water peak), 1.38 - 1.16 (m, 3H), 1.17 - 1.00 (m, 3H), 0.98 - 0.82 (m, 2H), 0.66 - 0.47 (m, 2H), 0.34 - 0.14 (m, 2H). LCMS [M+H]⁺ 442, RT 2.90 minutes, 100% purity (*Method 10*).

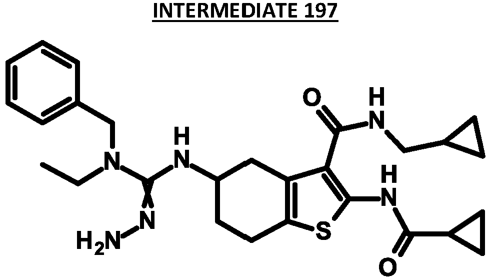

### 5-[(N-benzyl-N-ethyl-carbamohydrazonoyl)amino]-2-(cyclopropanecarbonylamino)-N-(cyclopropylmethyl)-4,5,6,7-tetrahydrobenzothiophene-3-carboxamide

To a solution of *intermediate 195* (627 mg, 1.23 mmol, 92% purity) in EtOH (10 mL) in a pressure tube was added hydrazine hydrate [10217-52-4] (1.2 mL, 23.90 mmol) and the reaction mixture was flushed with nitrogen and sealed. The mixture was warmed to 80 °C and stirred for 9 h before leaving to stand at r.t. overnight. The reaction mixture was concentrated *in vacuo* and the residue was dissolved in EtOAc (50 mL). The solution was washed with saturated aqueous sodium hydrogen carbonate solution (2 x 5 mL), water (2 x 10 mL) and brine (1 x 5 mL). The organic phase was dried (Na₂SO₄), filtered, the filtrate concentrated *in vacuo* to afford the *title compound* (455 mg, 55%, 74% purity) as a pale orange oil. LCMS [M+H]⁺ 509, RT 1.01 minutes, 74% purity *(Method 11).*

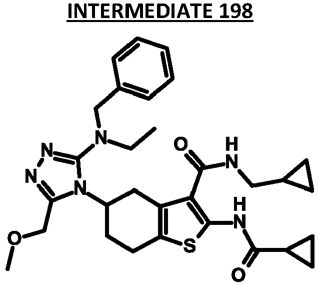

### 5-[3-[Benzyl(ethyl)amino]-5-(methoxymethyl)-1,2,4-triazol-4-yl]-2-(cyclopropanecarbonylamino)-N-(cyclopropylmethyl)-4,5,6,7-tetrahydrobenzothiophene-3-carboxamide

To a solution of *intermediate 197* (202 mg, 0.39 mmol) in DCM (8 mL) was added DIPEA (51 mg, 0.39 mmol) in DCM (0.5 mL). The reaction mixture was stirred under nitrogen and cooled to ~0 °C in an ice bath. To the stirred solution at ~ 0 °C was added 2-methoxyacetyl chloride [38870-89-2] (43 mg, 0.39 mmol) in DCM (0.5 mL) dropwise and the reaction mixture was warmed to r.t. overnight. The reaction mixture was concentrated *in vacuo* and dissolved in 1,4-dioxane (5 mL). TFA [76-05-1] (0.5 mL) was added and the reaction mixture was sealed in a pressure tube under nitrogen and the mixture was heated to 80 °C overnight. The reaction mixture was mixed with a previous experiment (20 mg, 0.039 mmol starting material) and was concentrated *in vacuo,* redissolved in 1:1 MeCN / water and purified by preparative HPLC (acidic) to afford the *title compound* (45.9 mg, 20%) which was azeotroped with MeCN (3 x 10 mL) to give a pale brown oil. δ_{H} (500 MHz, CD₃OD) 7.27 - 7.15 (m, 5H), 4.61 (d, J 13.1 Hz, 1H), 4.57 (d, J 13.1 Hz, 1H), 4.57 - 4.51 (m, 1H), 4.25 (d, J 12.8 Hz, 1H), 4.20 (d, J 12.8 Hz, 1H), 3.31 (s, 3H), 3.28 - 3.12 (m, 5H), 2.82 (dd, J 16.5, 5.6 Hz, 1H), 2.79 - 2.71 (m, 1H), 2.46 (dd, J 15.2, 5.0 Hz, 1H), 2.32 (ddt, J 18.3, 12.1, 5.9 Hz, 1H), 1.83 - 1.78 (m, 1H), 1.65 - 1.57 (m, 1H), 1.09 (t, J 7.1 Hz, 3H), 1.04 - 0.90 (m, 5H), 0.44 (m, 2H), 0.23 - 0.15 (m, 2H). LCMS [M+H]⁺ 563, RT 3.52 minutes, 96% purity (*Method 10*).

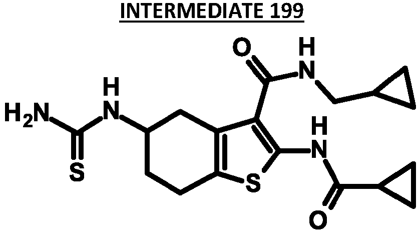

### 5-(Carbamothioylamino)-2-(cyclopropanecarbonylamino)-N-(cyclopropylmethyl)-4,5,6,7-tetrahydrobenzothiophene-3-carboxamide

To *intermediate 174* (0.11 g, 0.22 mmol) dissolved in MeOH (3.30 mL) was added potassium carbonate (70.1 mg, 0.507 mmol) and the reaction mixture was stirred for 6 h. The mixture was concentrated *in vacuo* to give the residue which was purified by flash column chromatography on silica (gradient elution with DCM/MeOH 0% 2 CV, 0-10% 8 CV, 10% 2 CV, 10-35% 10 CV) to afford the *title compound* (0.084 g, 99%) as a pale yellow oil. LCMS [M+H]⁺ 393.2, [M+Na]⁺ 415.0, RT 1.556 minutes, 100.0% purity (*Method 1*).

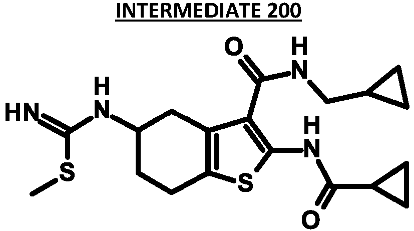

### 2-(Cyclopropanecarbonylamino)-N-(cyclopropylmethyl)-5-[(methylsulfanylcarbonimidoyl)amino]-4,5,6,7-tetrahydrobenzothiophene-3-carboxamide

To *intermediate 199* (0.32 g, 0.81 mmol) in THF (4.1 mL) was added iodomethane [74-88-4] (0.10 mL, 1.59 mmol) and the reaction mixture was heated in a microwave at 70 °C for 3 h. The reaction mixture was concentrated *in vacuo* to give a yellow residue which was purified by flash column chromatography on silica (DCM/MeOH 0% 2 CV, 0-10% 10 CV, 10% 4 CV) to afford the *title compound* (329 mg, 99%) as a pale yellow oil. LCMS [M+H]⁺ 407.2, RT 1.609 minutes, 100.0% purity *(Method 1).*

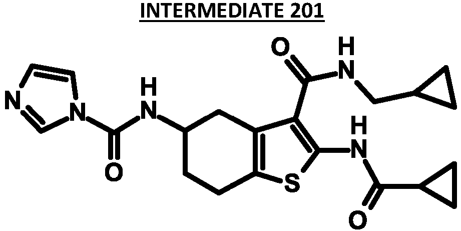

### N-[2-(Cyclopropanecarbonylamino)-3-(cyclopropylmethylcarbamoyl)-4,5,6,7-tetrahydrobenzothiophen-5-yl]imidazole-1-carboxamide

*Intermediate 122* (500 mg, 1.35 mmol) was dissolved in DCM (5 mL) and DIPEA (263 mg, 2.03 mmol) and 1,1'-carbonyldiimidazole [530-62-1] (241 mg, 1.49 mmol) were added. The reaction mixture was stirred at r.t. for ~1 h, the solution of *intermediate 201* was used directly without further purification. LCMS [M+H]⁺ 428.2, RT 1.283 minutes, 84.8% purity (*Method 4*).

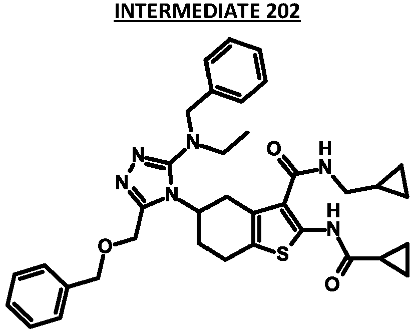

### 5-[3-[Benzyl(ethyl)aminol-5-(benzyloxymethyl)-1,2,4-triazol-4-yl]-2-(cyclopropanecarbonylamino)-N-(cyclopropylmethyl)-4,5,6,7-tetrahydrobenzothiophene-3-carboxamide

To a solution of *intermediate 197* (195 mg, 0.38 mmol) in DCM (1 mL) was added DIPEA (50 mg, 0.38 mmol) in DCM (0.5 mL) and the reaction mixture was flushed with nitrogen and cooled to ~0 °C in an ice bath. To the stirred solution at ~ 0 °C was added 2(benzyloxy)acetyl chloride [19810-31-2] (35 mg, 0.19 mmol) in DCM (0.5 mL) and the reaction mixture was warmed to r.t. overnight. The reaction mixture was concentrated *in vacuo* and the residue was dissolved in 1,4-dioxane (5 mL) and TFA [76-05-1] (0.5 mL) was added and the mixture transferred to a pressure tube. The reaction mixture was heated to 80°C overnight followed by a further 8 h. The reaction mixture was combined with a small scale reaction (20 mg, 0.039 mmol) and concentrated *in vacuo* and the residue purified by preparative HPLC (acidic) to afford the *title compound* (32 mg, 13%) as a pale brown oil which was azeotroped with MeCN (3 x 10 mL). δ_{H} (500 MHz, CD₃OD) 7.27 - 7.16 (m, 10H), 4.70 (d, J 12.7 Hz, 1H), 4.65 (d, J 12.7 Hz, 1H), 4.55 - 4.48 (m, 1H), 4.54 (d, J 11.5 Hz, 1H), 4.50 (d, J 11.5 Hz, 1H), 4.25 (d, J 12.9 Hz, 1H), 4.19 (d, J 12.8 Hz, 1H), 3.27 - 3.15 (m, 3H), 3.09 (qd, J 13.8, 7.1 Hz, 2H), 2.75 (dd, J 16.6, 5.7 Hz, 1H), 2.74 - 2.66 (m, 1H), 2.51 (dd, J 15.2, 5.3 Hz, 1H), 2.26 (ddt, J 18.2, 12.1, 6.0 Hz, 1H), 1.84 - 1.79 (m, 1H), 1.54 (br. d, J 12.0 Hz, 1H), 1.09 (t, J 7.1 Hz, 3H), 1.04 - 0.98 (m, 2H), 0.98 - 0.94 (m, 2H), 0.92 - 0.86 (m, 1H), 0.42 - 0.34 (m, 2H), 0.13-0.11 (m, 2H). LCMS [M+H]⁺ 639, RT 4.08 minutes, 96% purity (*Method 10*).

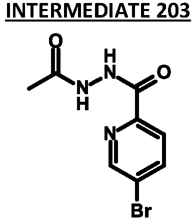

### N'-acetyl-5-bromopyridine-2-carbohydrazide

Acetohydrazide (0.37 g, 4.99 mmol) was stirred in DCM (10 mL) and DIPEA (0.87 mL, 4.99 mmol). 5-bromopyridine-2-carbonyl chloride (1 g, 4.54 mmol) was added in portions and the reaction was stirred for 15 minutes. The reaction was concentrated *in vacuo* to afford an oil. Water (10 mL) was added and the mixture sonicated which resulted in colloid formation, DCM (30 mL) was added and an emulsion formed, more water and DCM were added which resulted in flocculation. The mixture was filtered under vacuum to remove the brown solid. The filtrate was separated and the organic DCM layer was concentrated *in vacuo* to give a brown residue. The aqueous layer was added to this residue and the mixture sonicated, the resulting brown solid was collected by vacuum filtration and combined with the initial brown solid to afford the *title compound* (683 mg, 55% at 95% purity). δ_{H} (250 MHz, DMSO-d₆) δ 10.44 (s, 1H), 9.98 (s, 1H), 8.80 (d, J 1.8 Hz, 1H), 8.28 (dd, J 8.4, 2.3 Hz, 1H), 7.95 (dd, J 8.4, 0.5 Hz, 1H), 1.91 (s, 3H).

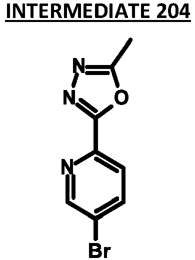

### 2-(5-bromo-2-pyridyl)-5-methyl-1,3,4-oxadiazole

*Intermediate 203* (90%, 856 mg, 2.99 mmol) was stirred in DCM (15 mL), NEt₃ (2.8 mL, 20 mmol) was added followed by 4-methylbenzenesulfonyl chloride (700 mg, 3.67 mmol). The reaction was stirred for 3 hours at room temperature. The reaction was quenched with saturated aq. NaHCO₃ (20 mL) and extracted with DCM (2 x 20 mL). The organic layer was dried over sodium sulfate and concentrated *in vacuo.* The resulting residue was purified by flash column chromatography on silica (gradient elution with 0 - 100% ethyl acetate in heptane) to afford the *title compound* (572mg, 80%) as a white solid. δ_{H} (250 MHz, DMSO-d₆) δ 8.91 (dd, J 2.3, 0.7 Hz, 1H), 8.31 (dd, J 8.5, 2.3 Hz, 1H), 8.09 (dd, J 8.5, 0.7 Hz, 1H), 2.61 (s, 3H).

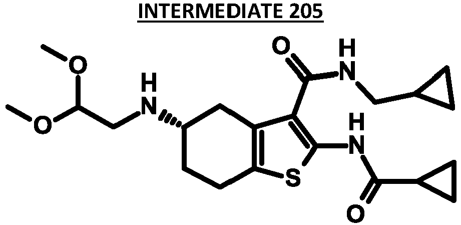

### (5S)-2-(Cyclopropanecarbonylamino)-N-(cyclopropylmethyl)-5-(2,2-dimethoxyethylamino)-4,5,6,7-tetrahydrobenzothiophene-3-carboxamide

To *intermediate 117* (1 g, 3 mmol) in MeOH (20 mL) was added dimethoxyacetaldehyde [51673-84-8] (60% aqueous, 452 µL, 2.99 mmol) and the reaction mixture was stirred for 18 h. Sodium borohydride [16940-66-2] (113 mg, 2.99 mmol) was added in portions and the reaction mixture was stirred at r.t. for 15 minutes. The reaction was concentrated *in vacuo* and diluted with EtOAc (60 mL). The organic phase was washed with water (2 x 20 mL), dried (Na₂SO₄), filtered and concentrated *in vacuo* to give the crude product (1.2 g) as a yellow oil which was purified by flash column chromatography on silica (gradient elution with 0 - 10% MeOH in DCM) to afford the *title compound* (1.07 g, 84%) as a yellow oil which solidified on standing. δ_{H} (500 MHz, DMSO-*d*₆) 11.20 (s, 1H), 7.66 - 7.57 (m, 1H), 4.40 (t, J 5.5 Hz, 1H), 3.27 (d, J 1.0 Hz, 6H), 3.24 - 3.15 (m, 1H), 3.15 - 3.05 (m, 1H), 2.87 (d, J 15.6 Hz, 1H), 2.85 - 2.74 (m, 1H), 2.70 (d, J 4.8 Hz, 2H), 2.69 - 2.64 (m, 1H), 2.63 - 2.53 (m, 1H), 2.42 (dd, J 15.4, 8.0 Hz, 1H), 2.01 - 1.93 (m, 1H), 1.93 - 1.84 (m, 1H), 1.59 - 1.39 (m, 2H), 1.10 - 0.99 (m, 1H), 0.88 - 0.77 (m, 4H), 0.47 - 0.39 (m, 2H), 0.27 - 0.21 (m, 2H).

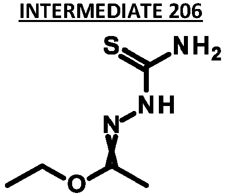

### Ethyl N-carbamothioylethanehydrazonate

To a solution of ethyl acetimdate hydrochloride [2208-07-3] (6.17 g, 49.9 mmol) in DMF (100 mL) under nitrogen was added thiosemicarbazide [79-19-6] (4.55 g, 49.9 mmol) at r.t. and the reaction mixture was stirred for 20 h. The mixture was poured into ice water (500 mL) and stirred at 0 °C for 1-2 h. The solid was filtered and the precipitate washed with ice cold water (20 mL) and air dried for 60 minutes and dried *in vacuo* overnight to afford the *title compound* (4.73 g, 59%) as a white amorphous solid. δ_{H} (500 MHz, DMSO-*d*₆) 8.94 (s, 1H), 7.95 (s, 1H), 7.39 (s, 1H), 4.15 (q, J 7.0 Hz, 2H), 2.06 (s, 3H), 1.28 (t, J 7.0 Hz, 3H). LCMS [M+H]⁺ 162, RT 1.06 minutes, 100% purity (*Method 18*)*.*

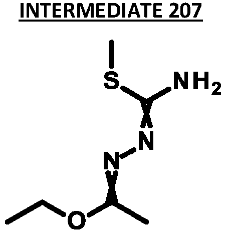

### ethyl N-[amino(methylsulfanyl)methylene]ethanehydrazonate

To a suspension of *intermediate 206* (1.00 g, 6.20 mmol) in DCM (5 mL) under nitrogen was added iodomethane [74-88-4] (1.76 g, 12.4 mmol) and the reaction mixture was stirred at r.t. for 4 h. The reaction mixture was concentrated *in vacuo,* azeotroped with DCM and the crude residue was suspended with EtOAc (40 mL) and washed with saturated aqueous sodium carbonate solution (6 mL). The organic phase was dried (Na₂SO₄), filtered and the filtrate concentrated *in vacuo* to give the *title compound* (1.09 g, 92%, 92% purity) as a pale yellow oil, mixture of isomers. ¹H NMR of major isomer: δ_{H} (500 MHz, Chloroform-d) 4.99 (br. s, 2H), 4.13 (q, J 7.1 Hz, 2H), 2.43 (s, 3H), 2.11 (s, 3H), 1.30 (t, J 7.1 Hz, 3H). Other minor configurantional isomers also observed by ¹H NMR. LCMS (ES+) [M+H]⁺ 176, RT 1.37 minutes, 92% purity (*Method 18*)*.*

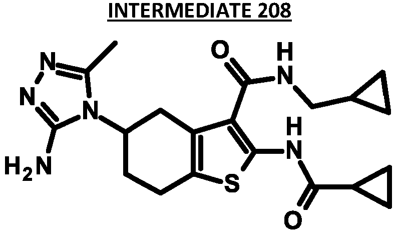

### 5-(3-Amino-5-methyl-1,2,4-triazol-4-yl)-2-(cyclopropanecarbonylamino)-N-(cyclopropylmethyl)-4,5,6,7-tetrahydrobenzothiophene-3-carboxamide

To *intermediate 122* (200 mg, 0.60 mmol) dissolved in DMF (0.5 mL) under nitrogen was added imidate *intermediate 207* (946 mg, 5.40 mmol) dissolved in DMF (0.5 mL). The reaction mixture was sealed in a pressure tube and heated to 120 °C for 2 h before cooling to r.t. The suspension was filtered and washed with MeCN (0.2 mL) to afford the *title compound* (111 mg, 44%) as a colourless solid. δ_{H} (500 MHz, DMSO-*d₆*) 11.23 (s, 1H), 7.67 (t, J 5.6 Hz, 1H), 5.50 (s, 2H), 4.35 - 4.15 (m, 1H), 3.18 - 3.07 (m, 3H), 2.88 - 2.79 (m, 3H), 2.41-2.36 (m, 1H), 2.32 (s, 3H), 2.00 (d, J 10.5 Hz, 1H), 1.92 (p, J 6.2, 5.7 Hz, 1H), 1.01 (tq, J 7.7, 4.0, 2.7 Hz, 1H), 0.92 - 0.79 (m, 4H), 0.44 - 0.32 (m, 2H), 0.25 - 0.14 (m, 2H). LCMS [M+H]⁺ 415, RT 2.18 minutes, 98% purity (*Method 16*).

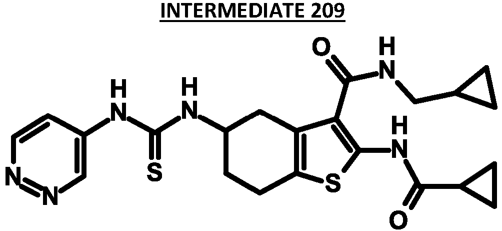

### 2-(Cyclopropanecarbonylamino)-N-(cyclopropylmethyl)-5-(pyridazin-4-ylcarbamothioylamino)-4,5,6,7-tetrahydrobenzothiophene-3-carboxamide

A solution of pyridazin-4-amine [20744-39-2] (134 mg, 1.41 mmol) in Et₂O (5 mL) was added dropwise to a solution of *intermediate 175* (70%, 378 mg, 0.7 mmol) in isopropanol (5 mL) and DCM (2 mL) and the reaction mixture was stirred for 18 h at r.t. The reaction mixture was concentrated in *vacuo* and the crude residue was purified by flash column chromatography on silica (gradient elution with 0-100 % EtOAc) to afford the *title compound* (136 mg, 41%) as a light yellow solid. δ_{H} (250 MHz, Chloroform-*d*) 12.13 (s, 1H), 7.47 - 7.35 (m, 2H), 7.16 - 7.01 (m, 2H), 5.89-5.85 ( br s, 1H), 4.77 (s, 1H), 3.35-3.28 (m, 3H), 2.91-2.85 (m, 3H), 2.20-2.16 (m, 1H), 1.77 - 1.61 (m, 1H), 1.32-1.27 (m, 1H), 1.15-1.09 (m, 3H), 0.98 - 0.81 (m, 3H), 0.63-0.57 (m, 2H), 0.34-0.26 (m, 2H).

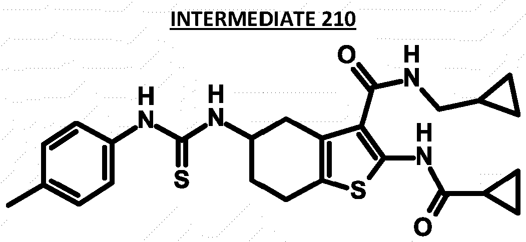

### 2-(Cyclopropanecarbonylamino)-N-(cyclopropylmethyl)-5-(p-tolylcarbamothioylamino)-4,5,6,7-tetrahydrobenzothiophene-3-carboxamide

A solution of 1-isothiocyanato-4-methylbenzene [622-59-3] (121 mg, 0.81 mmol) in DCM (20 mL) was cooled to 0 °C before addition of *intermediate 122* (300 mg, 0.81 mmol) and DIPEA (211.9 µL, 1.217 mmol). The ice bath was removed and the reaction mixture was stirred for 4 h under nitrogen. The mixture was diluted with DCM (30 mL) and washed with 1 M aqueous hydrochloric acid, saturated sodium hydrogen carbonate solution and brine. The organic phase was separated, dried (MgSO₄), filtered and concentrated *in vacuo* to give the crude product as a pale yellow solid which was purified by flash column chromatography on silica (gradient elution with 12 to 60% EtOAc in heptane) to afford the *title compound* (340 mg, 84%) as a white solid. LCMS [M+H]⁺ 483.05, RT 1.23 minutes, 97% purity *(Method 6).*

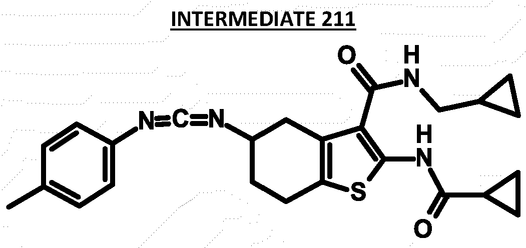

### 2-(Cyclopropanecarbonylamino)-N-(cyclopropylmethyl)-5-(p-tolyliminomethyleneamino)-4,5,6,7-tetrahydrobenzothiophene-3-carboxamide

Methanesulfonyl chloride [124-63-0] (32.3 µl, 0.41 mmol) was added over a period of 10 minutes to a solution of *intermediate 210* (100 mg, 0.20 mmol), triethylamine (86.6 µl, 0.62 mmol), and DMAP [1122-58-3] (2.5 mg, catalytic amount) in DCM (4 mL) cooled in an ice bath. The reaction mixture was stirred for 15 minutes before concentrating *in vacuo* to give the crude product which was purified by flash column chromatography on silica (gradient elution with 5% to 50% EtOAc in heptane) to afford the *title compound* (75 mg, 70%, 87% purity). LCMS [M+H]⁺ 449.2, [M+Na]+ 471.05 RT 1.39 minutes, 87% purity *(Method 6).*

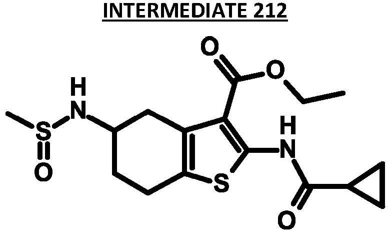

### Ethyl 2-(cyclopropanecarbonylamino)-5-(methanesulfinamido)-4,5,6,7-tetrahydrobenzothiophene-3-carboxylate

The following two reactions were carried out, differing in the starting amine *intermediate 122* and *example 109.*

Reaction 1: To *intermediate 122* (126 mg, 0.37 mmoL), sodium methanesulfinate [20277-69-4] (45 mg, 0.44 mmoL) and 1-ethyl-3-(3-dimethylaminopropyl)carbodiimide (84 mg, 0.44 mmoL) in DCM (5 mL) was added DIPEA (0.15 mL, 0.88 mmoL) and the resulting solution was stirred at r.t. under nitrogen. After stirring for 60 h at r.t. sodium methanesulfinate [20277-69-4] (45 mg, 0.44 mmoL), EDCI (84 mg, 0.44 mmoL) and DIPEA (0.15 mL, 0.88 mmoL) were added and stirring was continued at r.t. for further 6 h. The reaction mixture was combined with Reaction 2 for work up.

Reaction 2: To *example 109* (158 mg, 0.512 mmoL), sodium methanesulfinate [20277-69-4] (63 mg, 0.62 mmoL) and EDCI (118 mg, 0.62 mmoL) in DCM (5 mL) was added DIPEA (0.21 mL, 1.23 mmoL) and the resulting solution was stirred at r.t. under nitrogen for 60 h. The reaction mixture was combined with Reaction 1 for work up. The combined reaction mixtures were diluted with DCM and washed with saturated aqueous sodium hydrogen carbonate solution (10 mL), saturated aqueous ammonium chloride (10 mL), water (10 mL) and brine (10 mL). The organic phase was dried (MgSO₄), filtered and concentrated *in vacuo* to give the crude product which was purified by flash column chromatography on silica (gradient elution with 0 to 100% of EtOAc in heptane, followed by 0 to 20% MeOH in EtOAc) to afford the *title compound* (183 mg, 56%) as an off-white solid. δ_{H} (250 MHz, Chloroform-d) 11.44 (s, 1H), 4.35 (q, J 7.0 Hz, 2H), 3.90 - 3.71 (m, 2H), 3.34 - 3.16 (m, 1H), 2.85 - 2.68 (m, 3H), 2.69 - 2.47 (m, 3H), 2.20 - 2.00 (m, 1H), 1.95 - 1.78 (m, 1H), 1.74 - 1.59 (m, 1H), 1.40 (t, *J* 7.1 Hz, 3H), 1.21 - 1.07 (m, 2H), 1.02 - 0.85 (m, 2H). LCMS [M+H]⁺ 371, RT 1.20 minutes, 94% purity *(Method 6).*

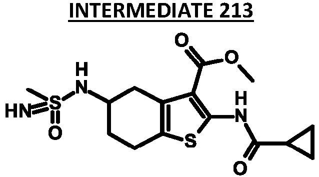

### Methyl 2-(cyclopropanecarbonylamino)-5-[(methylsulfonimidoyl)aminol-4,5,6,7-tetrahydrobenzothiophene-3-carboxylate

To a solution of *intermediate 212* (180 mg, 0.49 mmoL) in DCM (5 mL) was added 2,2,2-trifluoroacetamide [354-38-1] (110 mg, 0.97 mmoL), magnesium oxide [1309-48-4] (78 mg, 1.9 mmol), rhodium diacetate dimer [15956-28-2] (5 mg, 0.012 mmoL) and iodobenzene diacetate [3240-34-4] (235 mg, 0.73 mmoL) and the resulting suspension was stirred at r.t. for 16 h under nitrogen. The reaction mixture was filtered through a pad of celite, washed with DCM and the filtrate was concentrated *in vacuo* to afford a mixture of *ethyl 2-(cyclopropanecarbonylamino)-5-(methanesulfonamido)-4,5,6,7-tetrahydrobenzothiophene-3-carboxylate* and *ethyl 2-(cyclopropanecarbonylamino)-5-[[S-methyl-N-(2,2,2-trifluoroacetyl)sulfonimidoyl]amino]-4,5,6,7-tetrahydrobenzothiophene-3-carboxylate* which was utilised without further purification. To this mixture dissolved in MeOH (5 mL) was added potassium carbonate (335 mg, 2.43 mmoL) and the reaction mixture was stirred at r.t. overnight. The mixture was concentrated *in vacuo* and the crude residue was purified by flash column chromatography on silica (gradient elution with 0 to 100% of EtOAc in heptane, followed by 0 to 10% of MeOH in EtOAc) to afford the desired *title compound* (44 mg, 19%) as a yellow oil. LCMS [M+H]⁺ 372.00, RT 1.00 minutes, 94% purity *(Method 6).*

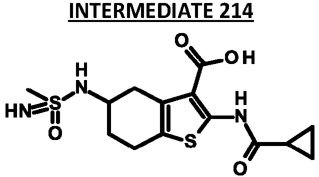

### 2-(Cyclopropanecarbonylamino)-5-[(methylsulfonimidoyl)aminol-4,5,6,7-tetrahydrobenzothiophene-3-carboxylic acid

To a solution of *intermediate 213* (40 mg, 0.11 mmoL) in 1,4-dioxane (3 mL) and water (3 mL) was added lithium hydroxide monohydrate [1310-66-3] (9 mg, 0.22 mmoL) and the reaction mixture was stirred at r.t. for 6 h. The reaction mixture was concentrated *in vacuo* and the residue was diluted with water (5 mL). The aqueous solution was acidified with 0.2M aqueous hydrochloric acid solution until pH 2 and the mixture was extracted with EtOAc (3 x 30 mL) followed by chloroform/isopropanol (1:1, 3 x 20 mL). The organic phases were dried (MgSO₄), filtered and concentrated *in vacuo* to afford the *title compound* (30 mg, 54%, 69% purity) as a yellow film. LCMS [M+H]⁺ 358, RT 0.93 minutes, 69% purity *(Method 6).*

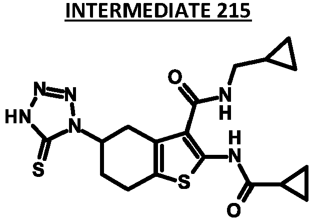

### 2-(Cyclopropanecarbonylamino)-N-(cyclopropylmethyl)-5-(5-thioxo-1H-tetrazol-4-yl)-4,5,6,7-tetrahydrobenzothiophene-3-carboxamide

*Intermediate 175* (1.13 g, 3.01 mmol) was added to a solution of sodium azide (196 mg, 3.01 mmol) in 2-propanol (80 mL) and water (8 mL) and the reaction mixture was heated to reflux for 1 h. The cooled suspension was filtered through Celite and concentrated *in vacuo* to afford the *title compound* (1.12 g, 49%, 55% purity) as a pale yellow solid which was utilised in the next step without purification. LCMS [M+H]⁺ 419.05, [M+Na]⁺ 440.95, RT 1.11 minutes, 55% purity (*Method* 11).

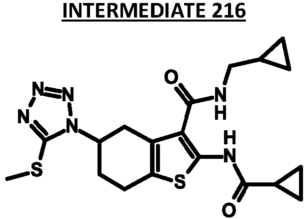

### 2-(Cyclopropanecarbonylamino)-N-(cyclopropylmethyl)-5-(5-methylsulfanyltetrazol-1-yl)-4,5,6,7-tetrahydrobenzothiophene-3-carboxamide

To a solution of *intermediate 215* (1.12 g, 2.68 mmol) in THF (30 mL) was added 2M potassium 2-methylpropan-2-olate (4.01 mL) at r.t. The reaction mixture was stirred for 10 minutes and iodomethane [74-88-4] (0.46 mL, 3.21 mmol) was added dropwise. The mixture was stirred for 1 h, concentrated *in vacuo,* diluted with 10% aqueous citric acid (300 mL) and extracted with DCM (3 × 50 mL). The combined organic phases were dried (Na₂SO₄), filtered and concentrated *in vacuo* to give the crude product which was purified by flash column chromatography on silica (gradient elution with 0-100% EtOAc in heptane followed by 0-40% MeOH in DCM) to afford the *title compound* (1 g, 81%, 94% purity). δ_{H} (250 MHz, Chloroform-d) 12.01 (s, 1H), 5.74 (s, 1H), 4.59 (s, 1H), 3.49 - 3.13 (m, 4H), 2.99 (s, 2H), 2.89 (s, 3H), 2.65 - 2.25 (m, 2H), 1.75 - 1.64 (m, 2H), 1.21 - 1.11 (m, 2H), 0.99-0.91 (m, 2H), 0.59-0.51 (m, 2H), 0.31 - 0.13 (m, 2H).

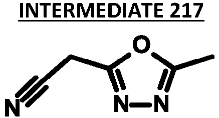

### 2-(5-methyl-1,3,4-oxadiazol-2-yl)acetonitrile

*Intermediate 163* (90%, 2.8 g, 17.86 mmol) was stirred in toluene (75 mL). POCl₃ (4.55 g, 29.68 mmol) was added and the suspension was heated at 80 °C for 2.5 hours. The reaction was allowed to cool and the suspension was added to water (100 mL) then taken to pH7 using saturated aq. Na₂CO₃. The aqueous solution was extracted with ethyl acetate (3 x 100 mL), the organics were dried over sodium sulfate and concentrated *in vacuo* to afford the *title compound* (980 mg, 44%) as a red solid. δ_{H} (500 MHz, Chloroform-d) δ 4.02 (s, 2H), 2.58 (s, 3H). δH (500 MHz, DMSO-d₆) δ 4.57 (s, 2H), 2.51 (s, 3H).

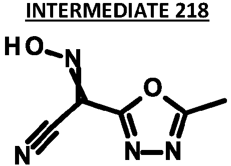

### 2-hydroxyimino-2-(5-methyl-1,3,4-oxadiazol-2-yl)acetonitrile

*Intermediate 217* (880 mg, 7.15 mmol) was stirred in dry ethanol (10 mL) and powdered KOH (441 mg, 7.86 mmol) was added followed by ^{t}butyl nitrite (935 µl, 7.86 mmol). An exotherm was observed and the reaction was cooled in an ice bath for 10 minutes then stirred at room temperature for 18 hours. Further ^{t}butyl nitrite (250 µl, 2.1 mmol) was added and the reaction stirred for 2 hours at room temperature. The brown suspension was diluted with diethyl ether and the solid was collected by vacuum filtration to give an orange solid which was dissolved in water (50 mL) and acidified to pH1 using 1M aq. HCl. The product was extracted into EtOAc (3 x 50 mL), dried over sodium sulfate and concentrated *in vacuo* to afford the *title compound* as a 1:1 mixture of E/Z isomers, (912 mg, 80% at 95% purity) as a red solid. δ_{H} (250 MHz, DMSO-d₆) δ 15.02 (s, 2H Isomer A+B), 2.61 (s, 3H Isomer A), 2.58 (s, 3H Isomer B).

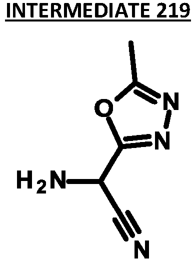

### 2-amino-2-(5-methyl-1,3,4-oxadiazol-2-yl)acetonitrile

*Intermediate 218* (0.98 g, 6.44 mmol) was stirred in water (6 mL) and saturated NaHCO₃ (6 mL) was added slowly. The mixture was stirred until all material had dissolved. Disodium dithionite (85%, 3.17 g, 15.46 mmol) was added in portions over 15 minutes, reaction reached 29 °C. The reaction was stirred for 1 hour then extracted with DCM (3 x 20 mL). The organics were dried over sodium sulfate and concentrated *in vacuo* to afford the *title compound* (208 mg, 16% at 68% purity) as an orange oil which solidified on standing. δ_{H} (500 MHz, DMSO-d₆) δ 5.53 (s, 1H), 2.53 (s, 3H). δ_{H} (500 MHz, Chloroform-d) δ 5.22 (s, 1H), 2.61 (s, 3H).

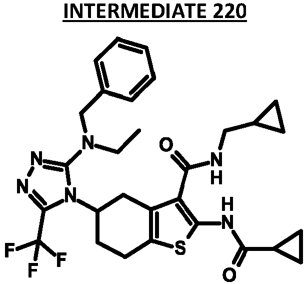

### 5-[3-[Benzyl(ethyl)amino]-5-(trifluoromethvyl)-1,2,4-triazol-4-yl]-2-(cyclopropanecarbonylamino)-N-(cyclopropylmethyl)-4,5,6,7-tetrahydrobenzothiophene-3-carboxamide

To a solution of *intermediate 197* (198 mg at 57% purity - estimated 113 mg product, 0.22 mmol) in 1,4-dioxane (2 mL) in a pressure tube was added trifluoroacetic anhydride [407-25-0] (46 mg in 0.2 mL 1,4-dioxane from a 230 mg in 1 mL stock solution, 0.22 mmol) and the reaction mixture was stirred at 30 °C under nitrogen for 60 minutes. The reaction mixture was retreated with further trifluoroacetic anhydride stock solution [407-25-0] (0.2 mL, 0.22 mmol) and the reaction mixture was stirred at r.t. overnight. TFA [76-05-1] (251 mg, 2.20 mol) was added under nitrogen and the reaction mixture was warmed to 80 °C for 50 minutes. Further TFA [76-05-1] (0.1 mL) was added and the reaction mixture was warmed at 80 °C for a further 4 h. The reaction mixture was concentrated *in vacuo* and the crude product was purified by preparative HPLC to afford the *title compound* (32 mg, 25%) which was azeotroped from MeCN (3 x 5 mL) to give a brown oil. LCMS [M+H]⁺ 587, RT 1.36 minutes, 100% purity *(Method 11).*

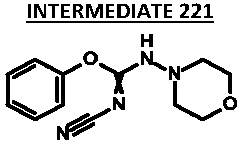

### 3-Cyano-1-morpholino-2-phenyl-isourea

Diphenyl N-cyanocarbonimidate [79463-77-7] (200 mg, 0.81 mmol, 97 mass%) and 4-aminomorpholine [4319-49-7] (100 mg, 0.98 mmol) were dissolved in DCM (2 mL) and 2-propanol (1.57 g, 26.1 mmol) and DIPEA (159 mg, 1.22 mmol) were added. The reaction mixture was stirred at r.t. for ^{~}1 h before concentrating *in vacuo.* The crude residue was purified by flash column chromatography on silica (gradient elution with 15-100% EtOAc/hexane) to afford the *title compound* (170 mg, 85%) as a white solid. δ_{H} (300 MHz, DMSO-*d*₆) 10.39 (s, 1H), 7.42 (t, J 7.8 Hz, 2H), 7.28 (t, J 7.4 Hz, 1H), 7.23 - 7.08 (m, 2H), 3.68 (s, 4H), 2.89 (s, 4H).

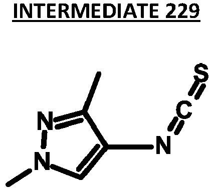

### 4-Isothiocyanato-1,3-dimethyl-pyrazole

1,3-Dimethyl-1H-pyrazol-4amine dihydrochloride [1692-25-7] (291.0 mg, 1.502 mmol,) was dissolved in DCM (4.5 mL) under nitrogen. 1,1'-Thiocarbonyldiimidazole (310.3 mg, 1.654 mmol) was added and the reaction was stirred at r.t. for 5.5 h. The crude reaction mixture was then concentrated in *vacuo* to give a yellow solid which was purified by flash column chromatography on silica (gradient elution with 0% to 100% EtOAc in isohexane) to afford the *title compound* (194.0 mg, 1.266 mmol, 84% Yield) as a pale yellow liquid. LCMS [M+H]⁺ 154.2, RT 0.839 minutes *(Method 15).*

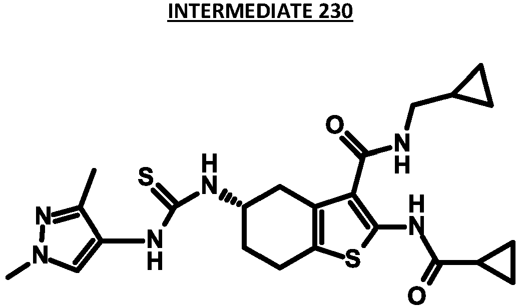

### (5S)-2-(cyclopropanecarbonylamino)-N-(cyclopropylmethyl)-5-[(1,3-dimethylpyrazol-4-yl)carbamothioylamino]-4,5,6,7-tetrahydrobenzothiophene-3-carboxamide

To a stirred solution of *intermediate 117* (313.5 mg, 0.9403 mmol) dissolved in DCM (3.7 mL) under nitrogen was added DIPEA (0.23 mL, 1.3 mmol) followed by a solution of *intermediate 229* (194.0 mg, 1.266 mmol) in DCM (1.0 mL). The reaction was stirred at r.t. for 17.5 h before being concentrated in vacuo to give a brownish grey oil. This oil was purified by flash column chromatography on silica (gradient elution with 0% to 100% EtOAc in isohexane, 0% to 20% MeOH in EtOAc) to afford the *title compound* (478.3 mg, 0.9338 mmol, 95 mass%, assumed quantitative yield and that the extra 5 mass% was due to solvent) as a brownish grey oil. LCMS [M+H]⁺ 487.0, RT 1.317 minutes, purity 98% *(Method 15).*

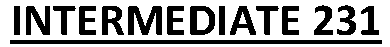

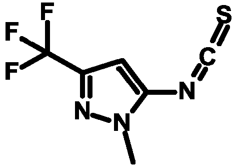

### 5-isothiocyanato-1-methyl-3-(trifluoromethyl)pyrazole

Hydrochloric acid in 1,4-dioxane (10.5 mL, 42.0 mmol, 4 mol/L) followed by 1,1'-thiocarbonyldiimidazole (4.38 g, 23.3 mmol) were added to a stirred solution of 1-methyl-3-(trifluoromethyl)-1H-pyrazol-5-amine [149978-43-8] (3.49 g, 21.1 mmol) in DCM (63 mL) under nitrogen. The reaction mixture was stirred at rt for 4.5 h before being left without stirring for a further 15.5 h. The reaction mixture was filtered and the pale yellow solid was washed with DCM (2 x 40 mL). The filtrate and washings were concentrated *in vacuo* to give an orange liquid which was purified by flash column chromatography on silica (gradient elution with 0% to 15% EtOAc in isohexane) to afford the *title compound* (3.0430 g, 14.688 mmol, 70%) as a colourless liquid. To confirm the presence of the *title compound,* a few drops of isobutylamine were added to the LCMS sample of the product before analysis to give the thiourea, LCMS [M+H]⁺ 281.0, RT 1.336 minutes, purity 100% *(Method 15).*

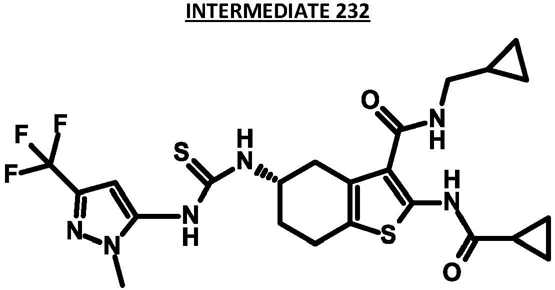

### (5S)-2-(cyclopropanecarbonylamino)-N-(cyclopropylmethyl)-5-[[2-methyl-5-(trifluoromethyl)pyrazol-3-yl]carbamothioylaminol-4,5,6,7-tetrahydrobenzothiophene-3-carboxamide

DIPEA (0.57 mL, 3.3 mmol) followed by a solution of *intermediate 231* (684.5 mg, 3.304 mmol) in DCM (5.0 mL) were added to a stirred solution of *intermediate 117* (1.000 g, 2.999 mmol) in DCM (10 mL) under nitrogen. The reaction mixture was stirred at rt for 4 h before being concentrated in vacuo to give a yellow oil. This oil was purified by flash column chromatography on silica (gradient elution with 0% to 100% EtOAc in isohexane) to afford impure *title compound* as a pale peach oil which was re-purified by flash column chromatography on silica (gradient elution with 20% to 60% EtOAc in isohexane) to give the *title compound* (1.191 g, 2.203 mmol, 73% Yield) as a white foam. LCMS [M+H]⁺ 541.0, RT 1.478 minutes, purity 99% *(Method 15).*

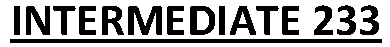

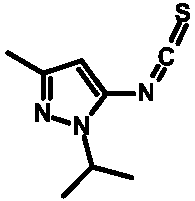

### 1-isopropyl-5-isothiocyanato-3-methyl-pyrazole

Hydrochloric acid in 1,4-dioxane (1.25 mL, 5.00 mmol, 4 mol/L) followed by 1,1'-thiocarbonyldiimidazole (520.1 mg, 2.773 mmol) were added to a stirred solution of 1-isopropyl-3-methyl-1H-pyrazol-5-amine [1124-16-9] (368.2 mg, 2.513 mmol) in DCM (7.5 mL) under nitrogen. The reaction mixture was stirred at rt for 19 h. The reaction mixture was filtered and the white solid was washed with DCM (2 x 15 mL). The filtrate and washings were concentrated *in vacuo* to give a golden yellow oil which was purified by flash column chromatography on silica (gradient elution with 0% to 50% EtOAc in isohexane) to afford the *title compound* (167.6 mg, 0.9246 mmol, 37%) as a pale yellow liquid. To confirm the presence of the *title compound,* a few drops of isobutylamine were added to the LCMS sample of the product before analysis to give the thiourea, LCMS [M+H]⁺ 255.1, RT 1.256 minutes, purity 99.6% *(Method 15).*

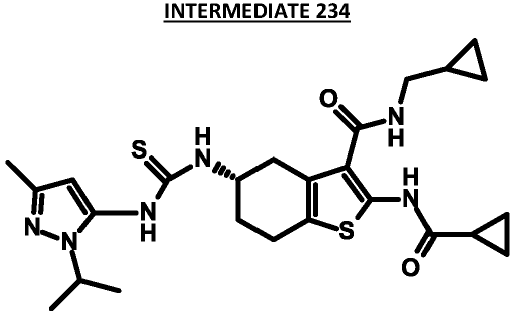

### (5S)-2-(cyclopropanecarbonylamino)-N-(cyclopropylmethyl)-5-[(2-isopropyl-5-methyl-pyrazol-3-yl)carbamothioylaminol-4,5,6,7-tetrahydrobenzothiophene-3-carboxamide

DIPEA (0.17 mL, 0.98 mmol) followed by a solution of *intermediate* 233 (167.6 mg, 0.9246 mmol) in DCM (1.0 mL) were added to a stirred solution of *intermediate 117* (250.0 mg, 0.7499 mmol) in DCM (2.75 mL) under nitrogen. The reaction mixture was stirred at rt for 17 h before being concentrated in vacuo to give a pale yellow oily solid which was purified by flash column chromatography on silica (gradient elution with 0% to 100% EtOAc in isohexane) to give the *title compound* (422.0 mg, 0.7461 mmol, 91 mass%, assumed quantitative yield and that the extra 9 mass% was due to solvent) as a white foam. LCMS [M+H]⁺ 515.0, RT 1.432 minutes, purity 99% *(Method 15).*

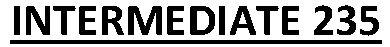

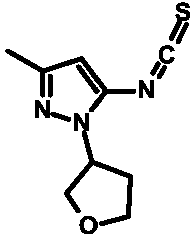

### 5-isothiocyanato-3-methyl-1-tetrahydrofuran-3-yl-pyrazole

Hydrochloric acid in 1,4-dioxane (1.25 mL, 5.00 mmol, 4 mol/L) followed by 1,1'-thiocarbonyldiimidazole (294.2 mg, 1.568 mmol) were added to a stirred solution of 3-methyl-1-(oxolan-3-yl)-1H-pyrazol-5-amine (250.0 mg, 1.420 mmol) in DCM (7.5 mL) under nitrogen. The reaction mixture was stirred at rt for 19 h. The reaction mixture was filtered and the white solid was washed with DCM (2 x 15 mL). The filtrate and washings were concentrated *in vacuo* to give a golden yellow oil which was purified by flash column chromatography on silica (gradient elution with 0% to 50% EtOAc in isohexane) to afford the *title compound* (219.0 mg, 1.046 mmol, 74%) as a pale yellow liquid. To confirm the presence of the *title compound,* a few drops of isobutylamine were added to the LCMS sample of the product before analysis to give the thiourea, LCMS [M+H]⁺ 283.0, RT 1.186 minutes, purity 99% *(Method 15).*

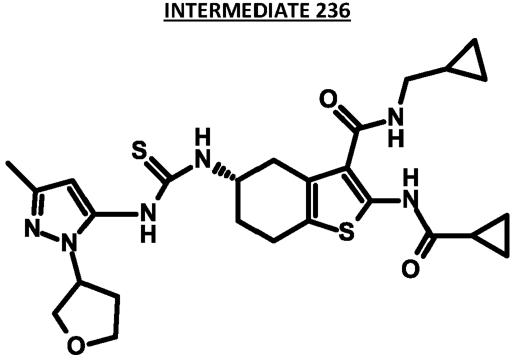

### (5S)-2-(cyclopropanecarbonylamino)-N-(cyclopropylmethyl)-5-[(5-methyl-2-tetrahydrofuran-3-yl-pyrazol-3-yl)carbamothioylamino]-4,5,6,7-tetrahydrobenzothiophene-3-carboxamide

DIPEA (0.17 mL, 0.98 mmol) followed by a solution of *intermediate 235* (219.0 mg, 1.046 mmol) in DCM (1.0 mL) were added to a stirred solution of *intermediate 117* (250.0 mg, 0.7499 mmol) in DCM (2.75 mL) under nitrogen. The reaction mixture was stirred at rt for 17 h before being concentrated in vacuo to give a pale yellow oily solid which was purified by flash column chromatography on silica (gradient elution with 0% to 100% EtOAc in isohexane) to give the *title compound* (385.4 mg, 0.7102 mmol, 95%) as a white foam. LCMS [M+H]⁺ 543.0, RT 1.389 minutes, purity 99% *(Method 15).*

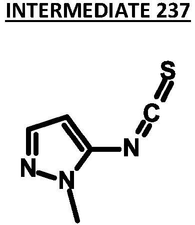

### 5-isothiocyanato-1-methyl-pyrazole

Hydrochloric acid in 1,4-dioxane (10.5 mL, 42.0 mmol, 4 mol/L) followed by 1,1'-thiocarbonyldiimidazole (4.361 g, 23.25 mmol) were added to a stirred solution of 1-methyl-1H-pyrazol-5-ylamine [1192-21-8] (2.110 g, 21.07 mmol) in DCM (63 mL) under nitrogen. The reaction mixture was stirred at rt for 24.5 h. The reaction mixture was filtered and the pale yellow solid was washed with DCM (2 x 40 mL). The filtrate and washings were concentrated *in vacuo* to give a yellow liquid which was purified by flash column chromatography on silica (gradient elution with 0% to 50% EtOAc in isohexane) to afford the *title compound* (1.7164 g, 12.332 mmol, 59%) as a yellow liquid. To confirm the presence of the *title compound,* a few drops of isobutylamine were added to the LCMS sample of the product before analysis to give the thiourea, LCMS [M+H]⁺ 213.0, RT 1.021 minutes *(Method 15).*

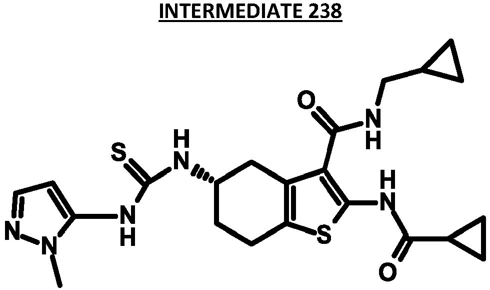

### (5S)-2-(cyclopropanecarbonylamino)-N-(cyclopropylmethyl)-5-[(2-methylpyrazol-3-yl)carbamothioylaminol-4,5,6,7-tetrahydrobenzothiophene-3-carboxamide

DIPEA (0.70 mL, 4.0 mmol) followed by a solution of *intermediate 237* (565.4 mg, 4.062 mmol) in DCM (5.0 mL) were added to a stirred solution of *intermediate 117* (1.034 g, 3.101 mmol) in DCM (10.5 mL) under nitrogen. The reaction mixture was stirred at rt for 3 h before being concentrated in vacuo to give a pale yellow solid which was purified by flash column chromatography on silica (gradient elution with 0% to 100% EtOAc in isohexane) to give the *title compound* (1.3368 g, 2.829 mmol, 91%) as a white foam. LCMS [M+H]⁺ 473.0, RT 1.306 minutes, purity 100% *(Method 15).*

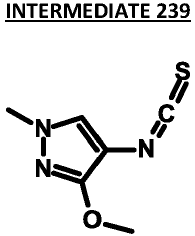

### 4-isothiocyanato-3-methoxy-1-methyl-pyrazole

1,1'-thiocarbonyldiimidazole (745.1 mg, 3.972 mmol) was added to a stirred solution of 3-methoxy-1-methyl-1H-pyrazol-4-amine hydrochloride [1431962-46-7] (610.5 mg, 3.545 mmol) in DCM (10.5 mL) under nitrogen. The reaction mixture was stirred at rt for 4.5 h. The reaction mixture was concentrated *in vacuo* to give an orange solid which was purified by flash column chromatography on silica (gradient elution with 0% to 100% EtOAc in isohexane) to afford the *title compound* (385.0 mg, 2.275mmol, 64%) as a white solid. To confirm the presence of the *title compound,* a few drops of isobutylamine were added to the LCMS sample of the product before analysis to give the thiourea, LCMS [M+H]⁺ 243.0, RT 1.106 minutes, purity 100% *(Method 15).*

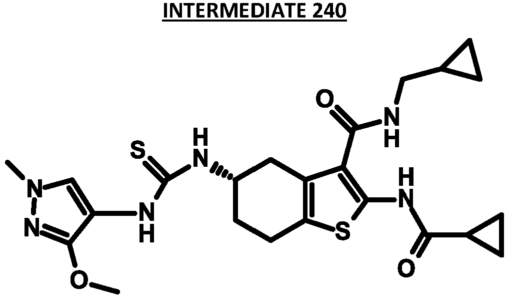

### (5S)-2-(cyclopropanecarbonylamino)-N-(cyclopropylmethyl)-5-[(3-methoxy-1-methyl-pyrazol-4-yl)carbamothioylaminol-4,5,6,7-tetrahydrobenzothiophene-3-carboxamide

DIPEA (0.17 mL, 0.98 mmol) followed by a solution of *intermediate 239* (165.1 mg, 0.9758 mmol) in DCM (1.0 mL) were added to a stirred solution of *intermediate 117* (249.3 mg, 0.7478 mmol) in DCM (2.75 mL) under nitrogen. The reaction mixture was stirred at rt for 45 min before being concentrated in vacuo to give an off white solid which was purified by flash column chromatography on silica (gradient elution with 0% to 100% EtOAc in isohexane) to give the *title compound* (376.0 mg, 0.7481 mmol, quantitative yield) as a white foam. LCMS [M+H]⁺ 503.0, RT 1.361 minutes, purity *100% (Method 15).*

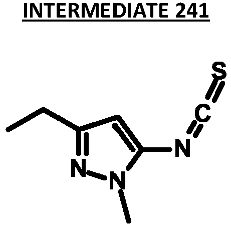

### 3-ethyl-5-isothiocyanato-1-methyl-pyrazole

Hydrochloric acid in 1,4-dioxane (1.25 mL, 5.00 mmol, 4 mol/L) followed by 1,1'-thiocarbonyldiimidazole (560.1 mg, 2.986 mmol) were added to a stirred solution of 3-ethyl-1-methyl-1H-pyrazol-5-amine [3524-46-7] (329.1 mg, 2.498 mmol) in DCM (7.5 mL) under nitrogen. The reaction mixture was stirred at rt for 2.5 h. The reaction mixture was concentrated *in vacuo* to give a golden yellow solid which was purified by flash column chromatography on silica (gradient elution with 0% to 50% EtOAc in isohexane) to afford the *title compound* (205.2 mg, 1.227 mmol, 49%) as a yellow liquid. To confirm the presence of the *title compound,* a few drops of isobutylamine were added to the LCMS sample of the product before analysis to give the thiourea, LCMS [M+H]⁺ 241.1, RT 1.222 minutes, purity 100% *(Method 15).*

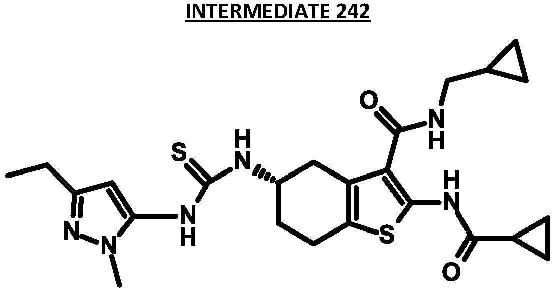

### (5S)-2-(cyclopropanecarbonylamino)-N-(cyclopropylmethyl)-5-[(5-ethyl-2-methyl-pyrazol-3-yl)carbamothioylaminol-4,5,6,7-tetrahydrobenzothiophene-3-carboxamide

DIPEA (0.17 mL, 0.98 mmol) followed by a solution of *intermediate 241* (205.2 mg, 1.227 mmol) in DCM (1.0 mL) were added to a stirred solution of *intermediate 117* (249.3 mg, 0.7478 mmol) in DCM (2.75 mL) under nitrogen. The reaction mixture was stirred at rt for 6 h before being concentrated in vacuo to give a yellow solid which was purified by flash column chromatography on silica (gradient elution with 0% to 100% EtOAc in isohexane) to give the *title compound* (327.6 mg, 0.6543 mmol, 88%) as a white solid. LCMS [M+H]⁺ 501.0, RT 1.378 minutes, purity 100% *(Method 15).*

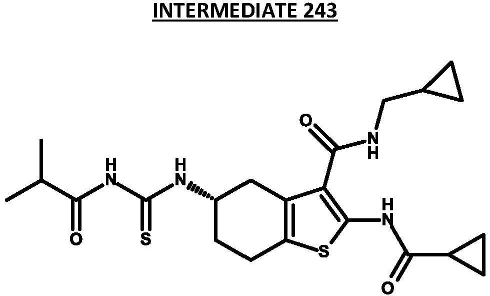

### (5S)-2-(cyclopropanecarbonylamino)-N-(cyclopropylmethyl)-5-(2-methylpropanoylcarbamothioylamino)-4,5,6,7-tetrahydrobenzothiophene-3-carboxamide

To a stirred solution of *intermediate 117* (150 mg, 0.450 mmol) dissolved in DCM (5 mL) under nitrogen was added DIPEA (0.12 mL, 0.675 mmol) followed by 2-methylpropanecarbonyl isothiocyanate (92 mg, 0.675 mmol).

The reaction was stirred at r.t. for 65 h before being concentrated in vacuo to give a gum. This was purified by flash column chromatography on silica (gradient elution with 10% to 70% EtOAc in isohexane) to afford the *title compound* (144 mg, 0.311 mmol, 69%) as an off-white solid. LCMS [M+H]⁺ 463.0, RT 2.39 minutes, purity 87% (*Method 1*)*.*

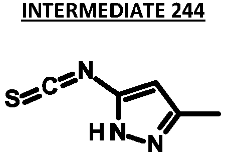

### 5-isothiocyanato-3-methyl-1H-pyrazole

To a solution of sodium carbonate (10.9 g, 103 mmol) and thiophosgene (4.74 g, 41.2 mmol) in water (30 ml) was added a solution of 3-methyl-1H-pyrazol-5-amine [31230-17-8] (291.0 mg, 1.502 mmol,) in DCM (15 mL). The mixture was stirred at room temperature for 2h then added to DCM (100 mL) and water (100 mL) and the layers separated. The aqueous layer was extracted with DCM (2 x 25 mL). The combined organic layers were washed with brine (50 ml) and passed through a phase separation cartridge and concentrated *in vacuo* to give a brown gum. This was purified by flash column chromatography on silica (gradient elution with 10% to 100% EtOAc in isohexane) to afford the *title compound* (0.65 g, 4.70 mmol, 23% Yield) as a pale yellow liquid. LCMS [M+H]⁺ 140.4, RT 0.58 minutes, purity 61% *(Method 7).*

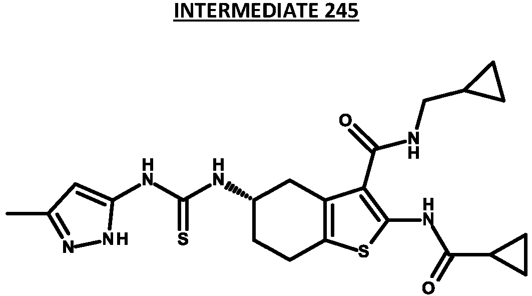

### (5S)-2-(cyclopropanecarbonylamino)-N-(cyclopropylmethyl)-5-[(3-methyl-1H-pyrazol-5-yl)carbamothioylaminol-4,5,6,7-tetrahydrobenzothiophene-3-carboxamide

To a stirred solution of *intermediate 117* (400 mg, 1.20 mmol) dissolved in DCM (20 mL) under nitrogen was added DIPEA (0.84 mL, 4.80 mmol) followed by *intermediate 244* (250 mg, 1.80 mmol).

The reaction was stirred at r.t. for 16 h. 3-Methyl-1H-pyrazol-5-amine (200 mg, 2.10 mmol) added as a solution in acetonitrile (5 mL). The reaction was stirred at r.t. for 1 h before being concentrated in vacuo to remove the DCM. Acetonitrile (10 mL) added and the mixture was stirred at 90 °C for 16 h. The mixture was cooled to r.t. before being concentrated in vacuo to give a gum. This was purified by flash column chromatography on silica (gradient elution with 25% to 100% EtOAc in isohexane) to afford impure *title compound* (186 mg) as a yellow gum. This was further purified by flash column chromatography on silica (gradient elution with 30% to 80% EtOAc in isohexane) to afford the *title compound* (124 mg, 0.262 mmol, 22%) as a yellow gum. LCMS [M+H]⁺ 473.0, RT 1.87 minutes, purity *80% (Method 3).*

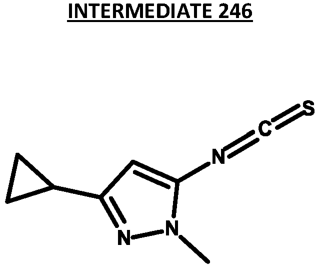

### 3-cyclopropyl-5-isothiocyanato-1-methyl-pyrazole

3-Cyclopropyl-1-methyl-1H-pyrazol-5-amine [118430-74-3] (2.50 g, 18.2 mmol,) was dissolved in DCM (50 mL) under nitrogen. 1,1'-Thiocarbonyldiimidazole (3.97 g, 20.0 mmol) was added and the reaction was stirred at r.t. for 64 h. The crude reaction mixture was then concentrated *in vacuo* to give a yellow solid which was purified by flash column chromatography on silica (gradient elution with 20% to 100% EtOAc in isohexane) to afford the *title compound* (135 mg, 0.753 mmol, 21% Yield) as a pale yellow liquid. LCMS [M+H]⁺ 180.2, RT 1.02 minutes, purity 99% *(Method 7).*

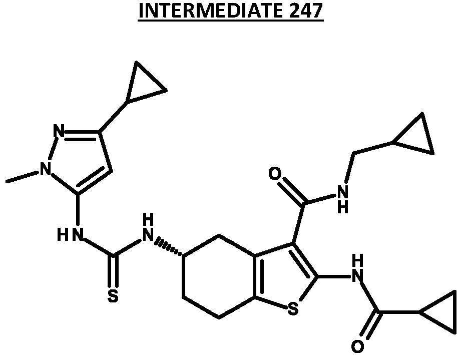

### (5S)-2-(cyclopropanecarbonylamino)-N-(cyclopropylmethyl)-5-[{5-cyclopropyl-2-methyl-pyrazol-3-yl)carbamothioylaminol-4,5,6,7-tetrahydrobenzothiophene-3-carboxamide

To a stirred solution of *intermediate 117* (1.0 g, 3.00 mmol) dissolved in DCM (10 mL) under nitrogen was added DIPEA (0.79 mL, 4.50 mmol) followed by *intermediate 246* (591 mg, 3.30 mmol).

The reaction was stirred at r.t. for 1 h before being concentrated in vacuo to give a gum. This was purified by flash column chromatography on silica (gradient elution with 30% to 100% EtOAc in isohexane) to afford the *title compound* (1.38 g, 2.69 mmol, 90%) as a white solid. LCMS [M+H]⁺ 513.2, RT 1.38 minutes, purity 99% (*Method 7*)*.*

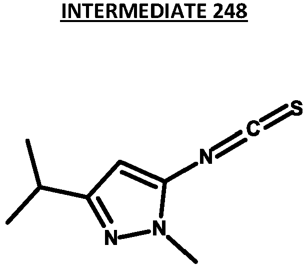

### 3-isopropyl-5-isothiocyanato-1-methyl-pyrazole

3-Isopropyl-1-methyl-1H-pyrazol-5-amine [3702-12-3] (0.50 g, 3.59 mmol,) was dissolved in DCM (10 mL) under nitrogen. 1,1'-Thiocarbonyldiimidazole (782 mg, 3.95 mmol) was added and the reaction was stirred at r.t. for 1 h. The crude reaction mixture was then concentrated *in vacuo* to give a yellow solid which was purified by flash column chromatography on silica (gradient elution with 5% to 75% EtOAc in isohexane) to afford the *title compound* (213 mg, 1.18 mmol, 33% Yield) as a pale yellow liquid. LCMS [M+H]⁺ 182.2, RT 1.10 minutes, purity 99% (*Method 7*)*.*

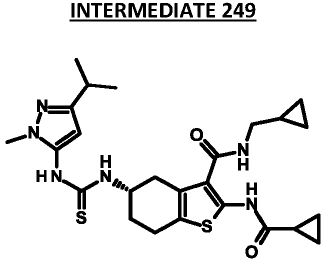

### (5S)-2-(cyclopropanecarbonylamino)-N-(cyclopropylmethyl)-5-[(5-isopropyl-2-methyl-pyrazol-3-yl)carbamothioylaminol-4,5,6,7-tetrahydrobenzothiophene-3-carboxamide

To a stirred solution of *intermediate 117* (200 mg, 0.600 mmol) dissolved in DCM (10 mL) under nitrogen was added DIPEA (0.16 mL, 0.900 mmol) followed by *intermediate 248* (163 mg, 0.900 mmol). The reaction was stirred at r.t. for 2 h before being concentrated in vacuo to give a gum. This was purified by flash column chromatography on silica (gradient elution with 30% to 100% EtOAc in isohexane) to afford the *title compound* (283 mg, 0.548 mmol, 91%) as a white solid. LCMS [M+H]⁺ 515.2, RT 2.00 minutes, purity 100% (*Method 1*)*.*

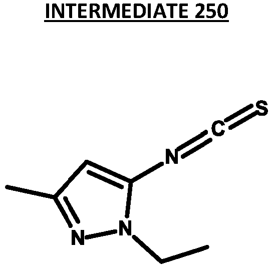

### 1-ethyl-5-isothiocyanato-3-methyl-pyrazole

1-Ethyl-3-methyl-1H-pyrazol-5-amine [3524-33-2] (0.50 g, 3.80 mmol,) was dissolved in DCM (10 mL) under nitrogen. 1,1'-Thiocarbonyldiimidazole (830 mg, 4.20 mmol) was added and the reaction was stirred at r.t. for 4 h. The crude reaction mixture was then concentrated *in vacuo* to give a yellow solid which was purified by flash column chromatography on silica (gradient elution with 30% to 100% EtOAc in isohexane) to afford the *title compound* (144 mg, 0.861 mmol, 23% Yield) as a pale yellow liquid. LCMS [M+H]⁺ 168.0, RT 1.39 minutes, purity 100% (*Method 7*)*.*

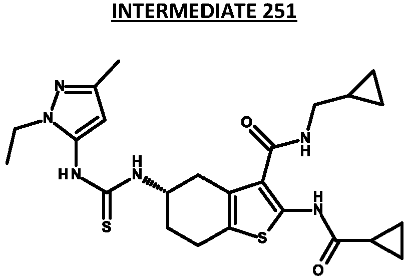

### (5S)-2-(cyclopropanecarbonylamino)-N-(cyclopropylmethyl)-5-[(2-ethyl-5-methyl-pyrazol-3-yl)carbamothioylaminol-4,5,6,7-tetrahydrobenzothiophene-3-carboxamide

To a stirred solution of *intermediate 117* (200 mg, 0.600 mmol) dissolved in DCM (10 mL) under nitrogen was added DIPEA (0.16 mL, 0.900 mmol) followed by *intermediate 250* (145 mg, 0.870 mmol).

The reaction was stirred at r.t. for 16 h before being concentrated in vacuo to give a gum. This was purified by flash column chromatography on silica (gradient elution with 30% to 100% EtOAc in isohexane) to afford the *title compound* (300 mg, 0.870 mmol, 100%) as a white solid. LCMS [M+H]⁺ 501.0, RT 2.00 minutes, purity 100% *(Method 3).*

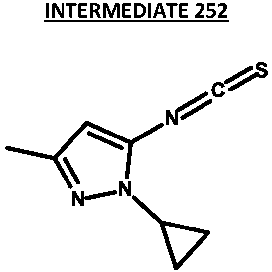

### 1-cyclopropyl-5-isothiocyanato-3-methyl-pyrazole

2-cyclopropyl-5-methyl-pyrazol-3-amine (352 mg, 2.57 mmol,) was dissolved in DCM (10 mL) under nitrogen. 1,1'-Thiocarbonyldiimidazole (559 mg, 2.82 mmol) was added and the reaction was stirred at r.t. for 4 h. The crude reaction mixture was then concentrated *in vacuo* to give a yellow solid which was purified by flash column chromatography on silica (gradient elution with 30% to 100% EtOAc in isohexane) to afford the *title compound* (105 mg, 0.586 mmol, 23% Yield) as a pale yellow liquid. LCMS [M+H]⁺ 180.0, RT 1.41 minutes, purity 100% *(Method 7).*

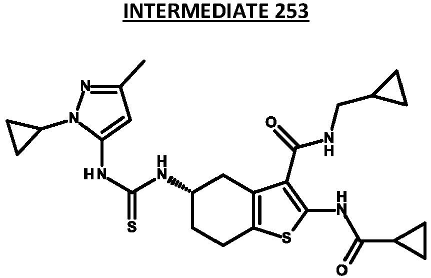

### (5S)-2-(cyclopropanecarbonylamino)-N-(cyclopropylmethyl)-5-[(2-cyclopropyl-5-methyl-pyrazol-3-yl)carbamothioylaminol-4,5,6,7-tetrahydrobenzothiophene-3-carboxamide

To a stirred solution of *intermediate 117* (200 mg, 0.600 mmol) dissolved in DCM (10 mL) under nitrogen was added DIPEA (0.16 mL, 0.900 mmol) followed by *intermediate 252* (105 mg, 0.600 mmol). The reaction was stirred at r.t. for 16 h before being concentrated in vacuo to give a gum. This was purified by flash column chromatography on silica (gradient elution with 30% to 100% EtOAc in isohexane) to afford the *title compound* (282 mg, 0.550 mmol, 92%) as a white solid. LCMS [M+H]⁺ 513.0, RT 2.03 minutes, purity 100% *(Method 3).*

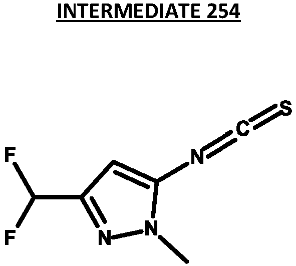

### 3-(difluoromethyl)-5-isothiocyanato-1-methyl-pyrazole

5-(Difluoromethyl)-2-methyl-pyrazol-3-amine (2.00 g, 13.6 mmol,) was dissolved in DCM (50 mL) under nitrogen. 1,1'-Thiocarbonyldiimidazole (2.96 mg, 15.0 mmol) was added. Hydrochloric acid in 1,4-dioxane (6.80 mL, 27.2 mmol, 4 mol/L) was added and the reaction was stirred at r.t. for 2 h. The crude reaction mixture was filtered and then concentrated *in vacuo* to give a yellow solid which was purified by flash column chromatography on silica (gradient elution with 5% to 50% EtOAc in isohexane) to afford the *title compound* (817 mg, 4.32 mmol, 32% Yield) as a pale yellow liquid. LCMS RT 0.95 minutes, purity 100% *(Method 7).*

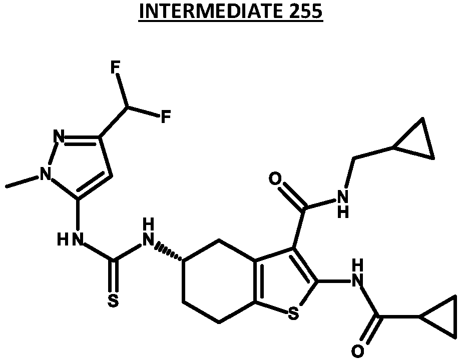

### (5S)-2-(Cyclopropanecarbonylamino)-N-(cyclopropylmethyl)-5-[[5-(difluoromethyl)-2-methyl-pyrazol-3-yl]carbamothioylamino]-4,5,6,7-tetrahydrobenzothiophene-3-carboxamide

To a stirred solution of *intermediate 117* (1.2 g, 3.60 mmol) dissolved in DCM (10 mL) under nitrogen was added DIPEA (0.95 mL, 5.40 mmol) followed by *intermediate 254* (885 mg, 4.68 mmol).

The reaction was stirred at r.t. for 40 minutes before being concentrated in vacuo to give a gum. This was purified by flash column chromatography on silica (gradient elution with 50% to 100% EtOAc in isohexane) to afford the *title compound* (1.75 g, 3.35 mmol, 93%) as a white solid. LCMS [M+H]⁺ 523.2, RT 1.43 minutes, purity 99% *(Method 7).*

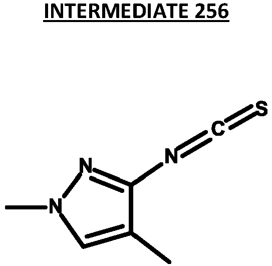

### 3-Isothiocyanato-1,4-dimethyl-pyrazole

1,4-Dimethyl-1H-pyrazol-3-amine [85485-61-6] (550 mg, 4.75 mmol,) was dissolved in DCM (10 mL) under nitrogen. 1,1'-Thiocarbonyldiimidazole (941 mg, 4.75 mmol) was added and the reaction was stirred at r.t. for 16 h. The crude reaction mixture was then concentrated *in vacuo* to give a yellow solid which was purified by flash column chromatography on silica (gradient elution with 20% to 100% EtOAc in isohexane) to afford the *title compound* (470 mg, 4.75 mmol, 100% Yield) as a white solid. LCMS [M+H]⁺ 154.2, RT 0.90 minutes, purity 100% *(Method 7).*

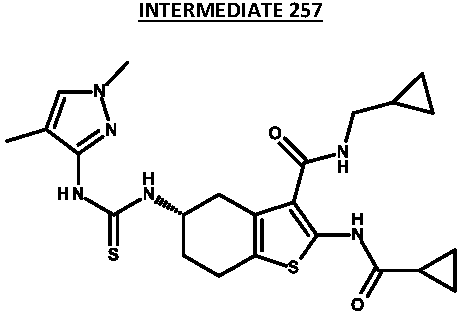

### (5S)-2-(Cyclopropanecarbonylamino)-N-(cyclopropylmethyl)-5-[(1,4-dimethylpyrazol-3-yl)carbamothioylaminol-4,5,6,7-tetrahydrobenzothiophene-3-carboxamide

To a stirred solution of *intermediate 117* (210 mg, 0.630 mmol) dissolved in DCM (10 mL) under nitrogen was added DIPEA (0.17 mL, 0.900 mmol) followed by *intermediate 256* (120 mg, 0.780 mmol).

The reaction was stirred at r.t. for 2 h before being concentrated in vacuo to give a gum. This was purified by flash column chromatography on silica (gradient elution with 30% to 100% EtOAc in isohexane) to afford the *title compound* (301 mg, 0.619 mmol, 98%) as a white solid. LCMS [M+H]⁺ 487.0, RT 2.19 minutes, purity 99% *(Method 3).*

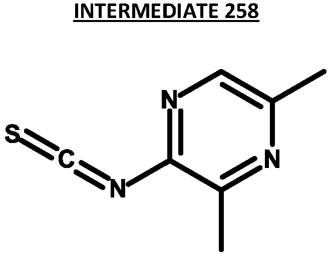

### 2-Isothiocyanato-3,5-dimethyl-pyrazine

To a solution of pyridine (0.51 ml, 6.20 mmol) and 3,5-dimethylpyrazin-2-amine [91678-81-8] (308 mg, 2.50 mmol) in DCM (4.5 ml) was added a solution of thiophosgene (0.21 ml, 2.70 mmol) in DCM (3 mL). The mixture was stirred at room temperature for 1 h then concentrated *in vacuo* to give a brown gum. This was purified by flash column chromatography on silica (gradient elution with 0% to 50% EtOAc in isohexane) to afford the *title compound* (303 mg, 1.83 mmol, 73% Yield) as a dark red liquid.

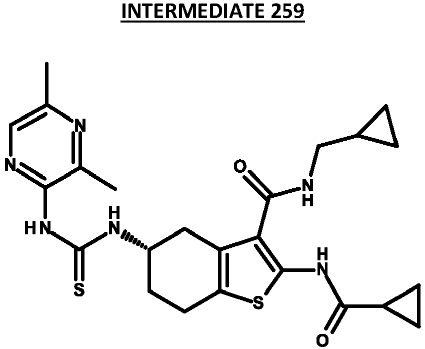

### (5S)-2-(Cyclopropanecarbonylamino)-N-(cyclopropylmethyl)-5-[(3,5-dimethylpyrazin-2-yl)carbamothioylaminol-4,5,6,7-tetrahydrobenzothiophene-3-carboxamide

To a stirred solution of *intermediate 117* (205 mg, 0.615 mmol) dissolved in DCM (10 mL) under nitrogen was added DIPEA (0.16 mL, 0.922 mmol) followed by *intermediate 258* (132 mg, 0.799 mmol).

The reaction was stirred at r.t. for 1 h before being concentrated in vacuo to give a gum. This was purified by flash column chromatography on silica (gradient elution with 30% to 100% EtOAc in isohexane) to afford the *title compound* (306 mg, 0.799 mmol, 100%) as a white solid. LCMS [M+H]⁺ 499.2, RT 1.45 minutes, purity 99% *(Method 7).*

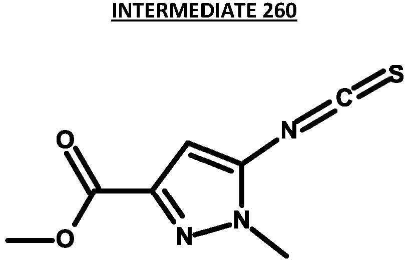

### Methyl 5-isothiocyanato-1-methyl-pyrazole-3-carboxylate

Methyl 5-amino-1-methyl-pyrazole-3-carboxylate [92406-53-6] (500 mg, 3.22 mmol,) was dissolved in DCM (10 mL) under nitrogen. 1,1'-Thiocarbonyldiimidazole (702 mg, 3.55 mmol) was added and the reaction was stirred at r.t. for 16 h. The crude reaction mixture was then concentrated *in vacuo* to give a yellow solid which was purified by flash column chromatography on silica (gradient elution with 20% to 100% EtOAc in isohexane) to afford the *title compound* (177 mg, 0.900 mmol, 28% Yield) as a white solid. LCMS [M+H]⁺ 198.0, RT 0.49 minutes, purity 91% *(Method 7).*

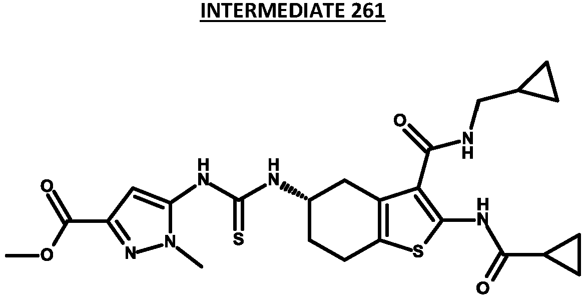

### Methyl 5-[[(5S)-2-(cyclopropanecarbonylamino)-3-(cyclopropylmethylcarbamoyl)-4,5,6,7-tetrahydrobenzothiophen-5-yl]carbamothioylamino]-1-methyl-pyrazole-3-carboxylate

To a stirred solution of *intermediate 117* (200 mg, 0.600 mmol) dissolved in DCM (10 mL) under nitrogen was added DIPEA (0.16 mL, 0.900 mmol) followed by *intermediate 260* (177 mg, 0.900 mmol).

The reaction was stirred at r.t. for 64 h before being concentrated in vacuo to give a gum. This was purified by flash column chromatography on silica (gradient elution with 30% to 100% EtOAc in isohexane) to afford the *title compound* (295 mg, 0.556 mmol, 93%) as a white solid. LCMS [M+H]⁺ 531.0, RT 1.94 minutes, purity 100% *(Method 3).*

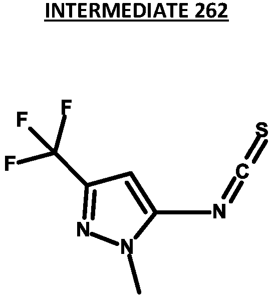

### 5-Isothiocyanato-1-methyl-3-(trifluoromethyl)pyrazole

1-Methyl-3-(trifluoromethyl)-1H-pyrazol-5-amine [149978-43-8 ] (3.49 g, 21.1 mmol,) was dissolved in DCM (63 mL) under nitrogen and hydrochloric acid in 1,4-dioxane (10.5 mL, 42.0 mmol, 4 mol/L) was added. 1,1'-Thiocarbonyldiimidazole (4.38 mg, 23.3 mmol) was added and the reaction was stirred at r.t. for 40 h. The crude reaction mixture was filtered and then concentrated *in vacuo* to give a yellow gum which was purified by flash column chromatography on silica (gradient elution with 0% to 50% EtOAc in isohexane) to afford the *title compound* (3.04 g, 14.7 mmol, 70% Yield) as a pale yellow liquid. LCMS (ES+) [M+H]⁺ 208.0, RT 1.34 minutes, purity 97% *(Method 7).*

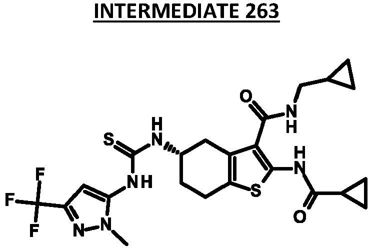

### (5S)-2-(Cyclopropanecarbonylamino)-N-(cyclopropylmethyl)-5-[[2-methyl-5-(trifluoromethyl)pyrazol-3-yl]carbamothioylamino]-4,5,6,7-tetrahydrobenzothiophene-3-carboxamide

To a stirred solution of *intermediate 117* (1.00 g, 3.00 mmol) dissolved in DCM (15 mL) under nitrogen was added DIPEA (0.57 mL, 3.30 mmol) followed by *intermediate 262* (685 mg, 3.30 mmol).

The reaction was stirred at r.t. for 4 h before being concentrated in vacuo to give a gum. This was purified by flash column chromatography on silica (gradient elution with 0% to 50% EtOAc in isohexane) to afford the *title compound* (1.19 g, 2.20 mmol, 73%) as a white solid. LCMS [M+H]⁺ 541.0, RT 1.48 minutes, purity 99% *(Method 7).*

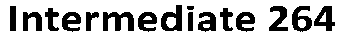

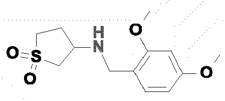

### N-[(2,4-dimethoxyphenyl)methyl]-1,1-dioxo-thiolan-3-amine

To a solution of 1,1-dioxothiolan-3-amine hydrochloride (100 mg, 0.58 mmol) and TEA (0.11 mL, 0.81 mmol) in DCM (2 mL) was added 2,4-dimethoxybenzaldehyde (123 mg, 0.74 mmol), followed by STAB (235 mg, 1.11 mmol). The suspension was stirred at room temperature for 1 hour, then at 40 °C for 3 hours. 1,1-dioxothiolan-3-amine hydrochloride (20 mg, 0.12 mmol) and TEA (0.02 mL, 0.15 mmol) were added as a solution in DCM (0.5 mL), followed by STAB (92 mg, 0.44 mmol) and the mixture stirred at 40 °C for 16 hours. STAB (185 mg, 0.87 mmol) was added and the mixture stirred at 40 °C for 4 hours. The mixture was quenched with saturated sodium bicarbonate and the organic layer separated. The aqueous layer was extracted with DCM (4 x 5 mL) and the organic fractions combined, passed through a hydrophobic frit and concentrated *in vacuo* to give an oil. The oil was purified *via* column chromatography, using a gradient of 50 - 100% TBME in heptanes, followed by 0 - 10% methanol in TBME to give the title compound (167 mg, 84% yield). δ_{H} (500 MHz, d-Chloroform) 7.08 (d, J = 8.1 Hz, 1H), 6.48 - 6.42 (m, 2H), 3.82 (s, 3H), 3.81 (s, 3H), 3.74 (d, J = 2.2 Hz, 2H), 3.58 - 3.51 (m, 1H), 3.32 - 3.22 (m, 2H), 3.04 - 2.97 (m, 1H), 2.86 (dd, J = 13.2, 6.7 Hz, 1H), 2.42 - 2.34 (m, 1H), 2.11 - 2.02 (m, 1H). LCMS [M+H]⁺ 286, RT 1.42 (Method 18) .

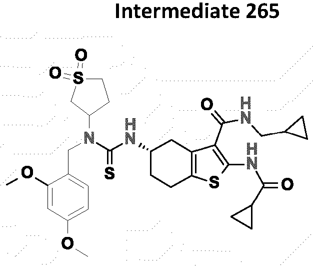

### (5S)-2-(cyclopropanecarbonylamino)-N-(cyclopropylmethyl)-5-[[(2,4-dimethoxyphenyl)methyl-(1,1-dioxothiolan-3-yl)carbamothioyl]amino]-4,5,6,7-tetrahydrobenzothiophene-3-carboxamide

The title compound was prepared according to **general method 7,** using **intermediate 117** (133 mg, 0.4 mmol) and **intermediate 264** (125 mg, 0.44 mmol). Purification via column chromatography, using a gradient of 0 - 100% TBME in heptanes, followed by 0 - 10% methanol in TBME gave the title compound (280 mg, 96% yield). LCMS (M+H)⁺ 661, RT 1.2 (Method 11).

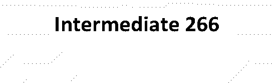

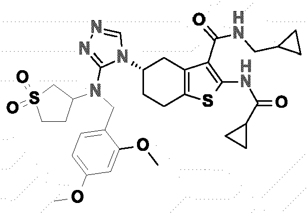

### (5S)-2-(cyclopropanecarbonylamino)-N-{cyclopropylmethyl)-5-[3-[(2,4-dimethoxyphenyl)methyl-(1,1-dioxothiolan-3-yl)amino]-1,2,4-triazol-4-yl]-4,5,6,7-tetrahydrobenzothiophene-3-carboxamide

The title compound was prepared according to **general method 8,** using **intermediate 265** (280 mg, 0.38 mmol). Purificaion *via* column chromatography (KP-NH), using a gradient of 0 - 20% methanol in TBME gave the title compound (240 mg, 85% yield). LCMS (M+H)⁺ 669, RT 1.09 (Method 11).

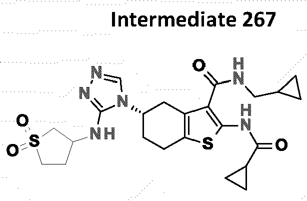

### (5S)-2-(cyclopropanecarbonylamino)-N-(cyclopropylmethyl)-5-[3-[(1,1-dioxothiolan-3-yl)amino]-1,2,4-triazol-4-yl]-4,5,6,7-tetrahydrobenzothiophene-3-carboxamide

**Intermediate 266** (240 mg, 0.32 mmol) was dissolved in DCM (5 mL) and TFA (247 µL, 3.23 mmol) was added. The reaction was stirred at room temperature overnight, then retreated with TFA (247 µL, 3.23 mmol). The reaction mixture was stirred at room temperature for 4 hours, then quenched with saturated sodium bicarbonate and the organic layer separated. The aqueous layer was extracted with DCM (3 x 5 mL) and the organic fractions combined, passed through a phase separator and concentrated *in vacuo.* The resulting oil was purified *via* column chromatography (KP-NH), eluting with a 0 - 20% gradient of methanol in TBME to give the title compound (145 mg, 85% yield). δ_{H} (500 MHz, DMSO-d₆) 11.29 - 11.19 (m, 1H), 8.17 (s, 1H), 7.71 - 7.60 (m, 1H), 6.43 (d, J = 6.3 Hz, 1H), 4.48 - 4.37 (m, 1H), 4.29 - 4.19 (m, 1H), 3.58 - 3.49 (m, 1H), 3.22 - 3.13 (m, 2H), 3.12 - 2.98 (m, 3H), 2.92 - 2.79 (m, 3H), 2.49 - 2.44 (m, 1H), 2.27 - 2.08 (m, 3H), 1.96 - 1.87 (m, 1H), 1.07 - 0.96 (m, 1H), 0.92 - 0.79 (m, 4H), 0.43 - 0.33 (m, 2H), 0.25 - 0.14 (m, 2H). LCMS (M+H)⁺ 519, RT 1.96 (Method 10) .

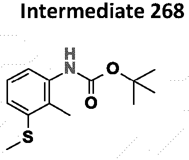

### tert-butyl N-(2-methyl-3-methylsulfanyl-phenyl)carbamate

A solution of 2-methyl-3-methylsulfanyl-aniline (500 mg, 3.26 mmol) and tert-butoxycarbonyl tert-butyl carbonate (783 mg, 3.59 mmol) in THF (10 mL) was stirred under an atmosphere of nitrogen overnight at 60 °C. The solution was retreated with tert-butoxycarbonyl tert-butyl carbonate (107 mg, 0.49 mmol) and stirred at 60 °C for another 3 hours. The mixture was concentrated *in vacuo* and purified via column chromatography, using a gradient of 0 - 20% TBME in heptanes to give the title compound (1 g, 98% yield) as an orange oil. δ_{H} (250 MHz, d-Chloroform) 7.56 (d, J = 8.0 Hz, 1H), 7.17 (t, J = 8.0 Hz, 1H), 6.98 (d, J = 7.8 Hz, 1H), 6.26 (s, 1H), 2.44 (s, 3H), 2.27 (s, 3H), 1.55 - 1.48 (m, 9H).

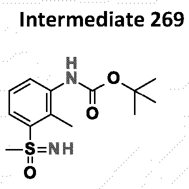

### tert-butyl N-[2-methyl-3-(methylsulfonimidoyl)phenyl]carbamate

To a stirring solution of **Intermediate 268** (900 mg, 2.88 mmol) in anhydrous Methanol (7 mL) was added ammonium carbamate (899 mg, 11.5 mmol) followed by (Diacetoxyiodo)benzene (2.78 g, 8.63 mmol) and the mixture was stirred at room temperature in air for 15 hours. The mixture was concentrated *in vacuo,* then diluted with water (10 mL) and extracted with DCM (4 x 10 mL). The organic fractions were combined, passed through a hydrophobic frit and concentrated *in vacuo.* The residue was purified via column chromatography, using a gradient of 50 - 100% TBME in heptanes, followed by 0 - 10% methanol in TBME to give the title compound (620 mg, 58 %). δ_{H} (250 MHz, DMSO-d₆) 8.78 (s, 1H), 7.83 (dd, J = 7.9, 1.3 Hz, 1H), 7.57 - 7.50 (m, 1H), 7.35 (t, J = 7.9 Hz, 1H), 4.30 (s, 1H), 3.09 - 3.05 (m, 3H), 2.56 (s, 3H), 1.46 (s, 9H). LCMS (M+H)⁺ 285, RT 1.55 (Method 12).

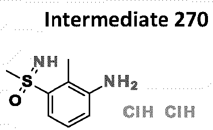

### 2-methyl-3-(methylsulfonimidoyl)aniline dihydrochloride

To a stirred solution of **intermediate 269** (694 mg, 1.95 mmol) in dioxane (5 ml) was added 4 M HCl in dioxane (5 mL). The mixture was stirred at 50 °C for 3 hours. The reaction mixture was concentrated *in vacuo* and the residue was further *dried in vacuo* at 40 °C for 1 hour to afford the desired product (684 mg, 25% dioxane w/w, 95% yield). δ_{H} (250 MHz, DMSO-d6) 7.45 - 7.22 (m, 3H), 3.91 (s, 3H), 2.42 (s, 3H).

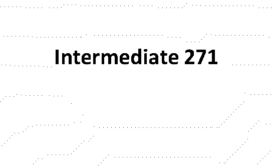

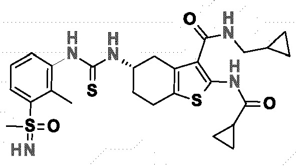

### (5S)-2-(cyclopropanecarbonylamino)-N-(cyclopropylmethyl)-5-[[2-methyl-3-(methylsulfonimidoyl)phenyl]carbamothioylamino]-4,5,6,7-tetrahydrobenzothiophene-3-carboxamide

To stirring solution of sodium carbonate (474 mg, 4.47 mmol) in water (4.5 mL) cooled to 0 °C in an ice bath was added DCM (9 mL), followed by thiophosgene (0.14 mL, 1.79 mmol). To the biphasic mixture was added **intermediate 270** (230 mg, 0.89 mmol) and resulting mixture was stirred at room temperature for 1 hour. The layers were separated and the aqueous phase was extracted with DCM (2 x 10 mL). The combined organics were washed with brine (10 mL), dried (MgSO₄) and the concentrated *in vacuo.* The residue obtained was then dissolved in DCM (10 mL) and **intermediate 117** (298 mg, 0.89 mmol) added, followed by triethylamine (0.62 mL, 4.47 mmol). The mixture was stirred at room temperature for 3 hours, then diluted with water (10 mL) and the organic layer separated. The aqueous layer was extracted with DCM (1 x 10 mL) and the organic fractions combined, passed through a hydrophobic frit and concentrated *in vacuo.* The residue was purified *via* column chromatography using a gradient of 0 - 20% methanol in TBME to give a powder. The powder was further purified *via* column chromatography, using a gradient of 0 - 8% methanol in DCM to give the title compound (217 mg, 39% yield). δ_{H} (250 MHz, DMSO-*d*₆) 11.08 (s, 1H), 9.09 (s, 1H), 7.95 - 7.80 (m, 2H), 7.78 - 7.69 (m, 1H), 7.63 - 7.51 (m, 1H), 7.37 (t, J = 7.9 Hz, 1H), 4.57 (s, 1H), 4.31 (s, 1H), 3.15 - 3.10 (m, 2H), 3.11 - 3.03 (m, 3H), 2.81 - 2.64 (m, 3H), 2.53 (s, 3H), 2.14 - 1.80 (m, 3H), 1.09 - 0.96 (m, 1H), 0.89 - 0.79 (m, 4H), 0.48 - 0.38 (m, 2H), 0.27 - 0.18 (m, 2H). LCMS (M+H)⁺ 560, RT 1.73 (Method 12).

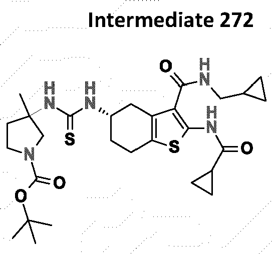

### tert-butyl 3-[[(5S)-2-(cyclopropanecarbonylamino)-3-(cyclopropylmethylcarbamoyl)-4,5,6,7-tetrahydrobenzothiophen-5-yl]carbamothioylamino]-3-methyl-pyrrolidine-1-carboxylate

The title compound was prepared according to **general method 7,** using **intermediate 117** (46 mg, 0.14 mmol) and tert-butyl 3-amino-3-methyl-pyrrolidine-1-carboxylate (25 mg, 0.12 mmol). The reaction mixture was purified *via* column chromatography, using a gradient of 30 - 100% TBME in heptanes to give the title compound (56 mg, 70% yield). δ_{H} (250 MHz, d-Chloroform) 12.13 (s, 1H), 6.49 - 6.30 (m, 2H), 5.93 - 5.82 (m, 1H), 5.00 - 4.84 (m, 1H), 3.37 - 3.22 (m, 4H), 3.16 - 3.01 (m, 1H), 2.89 - 2.67 (m, 3H), 2.23 - 2.07 (m, 1H), 2.06 - 1.92 (m, 1H), 1.89 - 1.73 (m, 1H), 1.69 - 1.59 (m, 4H), 1.46 - 1.35 (m, 10H), 1.11 - 1.00 (m, 3H), 0.95 - 0.78 (m, 4H), 0.63 - 0.53 (m, 2H), 0.30 - 0.22 (m, 2H). LCMS (M+H)⁺ 576, RT 2.03 (Method 12).

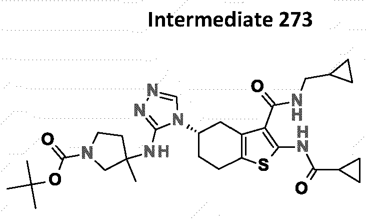

### tert-butyl 3-[[4-[(5S)-2-(cyclopropanecarbonylamino)-3-(cyclopropylmethylcarbamoyl)-4,5,6,7-tetrahydrobenzothiophen-5-yl]-1,2,4-triazol-3-yl]amino]-3-methyl-pyrrolidine-1-carboxylate

**Intermediate 272** (720 mg, 0.9 mmol) was dissolved in DMF (25 mL) and formic hydrazide (162 mg, 2.7 mmol) was added, followed by dichloromercury (509 mg, 1.87 mmol). The reaction mixture was stirred at room temperature for 5 minutes and then DIPEA (369 µL, 2.65 mmol) was added. The reaction was stirred at 90 °C overnight. The suspension was retreated with formic hydrazide (80 mg, 1.33 mmol), stirred at 90 °C for 6 hours, before being stirred at 100 °C for 24 hours. The mixture was filtered through Celite, washing with DCM. The solution was concentrated *in vacuo,* then purified *via* column chromatography, using a gradient of 0 - 14% methanol in DCM to give the title compound (182 mg, 35%). δ_{H} (500 MHz, DMSO-*d*₆) 11.24 (s, 1H), 8.13 (s, 1H), 7.66 (s, 1H), 5.89 - 5.80 (m, 1H), 4.35 - 4.23 (m, 1H), 3.78 - 3.70 (m, 1H), 3.28 - 3.13 (m, 2H), 3.11 - 2.98 (m, 2H), 2.88 - 2.77 (m, 3H), 2.47 - 2.31 (m, 1H), 2.21 - 2.05 (m, 2H), 1.94 - 1.88 (m, 1H), 1.88 - 1.80 (m, 1H), 1.47 - 1.42 (m, 3H), 1.41 - 1.33 (m, 9H), 1.05 - 0.96 (m, 1H), 0.90 - 0.80 (m, 4H), 0.42 - 0.33 (m, 2H), 0.22 - 0.15 (m, 2H). LCMS (M+H)⁺ 584, RT 2.8 (Method 29).

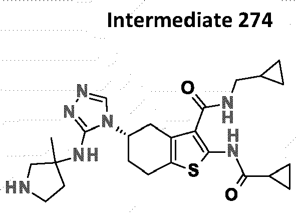

### (5S)-2-(cyclopropanecarbonylamino)-N-(cyclopropylmethyl)-5-[3-[(3-methylpyrrolidin-3-yl)aminol-1,2,4-triazol-4-yl]-4,5,6,7-tetrahydrobenzothiophene-3-carboxamide

To a stirred solution of **intermediate 273** (180 mg, 0.31 mmol) in dioxane (4 mL) was added 4 M hydrogen chloride in dioxane (2 mL, 8 mmol). The reaction mixture was then diluted with methanol (1 mL) and stirred at room temperature for 4 hours. The mixture was then concentrated under reduced pressure and purified *via* an SCX-2 column, washing with methanol, then eluting product with 7 M ammonia in methanol, to give the title compound (142 mg, 92% yield). δ_{H} (500 MHz, DMSO-*d*₆) 8.61 (s, 1H), 8.12 (s, 1H), 5.81 - 5.74 (m, 1H), 4.32 - 4.23 (m, 1H), 3.35 - 3.31 (m, 1H), 3.19 - 3.11 (m, 2H), 3.10 - 2.98 (m, 2H), 2.95 - 2.89 (m, 1H), 2.85 - 2.71 (m, 4H), 2.29 -2.20 (m, 1H), 2.18 - 2.02 (m, 2H), 1.85 - 1.78 (m, 1H), 1.75 - 1.67 (m, 1H), 1.49 - 1.44 (m, 3H), 1.04 - 0.94 (m, 1H), 0.84 - 0.74 (m, 4H), 0.45 - 0.32 (m, 2H), 0.24 - 0.12 (m, 2H). LCMS (M+H)⁺ 484, RT 1.99 (Method 29).

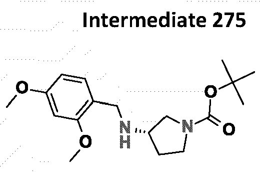

### tert-butyl (3S)-3-[(2,4-dimethoxyphenyl)methylamino]pyrrolidine-1-carboxylate

To a solution of tert-butyl (3S)-3-aminopyrrolidine-1-carboxylate (1 g, 5.37 mmol) in DCM (15 mL) was added 2,4-dimethoxybenzaldehyde (892 mg, 5.37 mmol) and STAB (3.41 g, 16.1 mmol). The reaction was stirred at room temperature for 3 h. The mixture was diluted with saturated NaHCO₃ solution (10 mL) and the organic layer was separated. The aqueous layer was extracted with DCM (2 x 10 mL) and the combined organic layers were dried (MgSO₄). The solvent was removed *in vacuo* to give an oil, which was purified *via* column chromatography, using a gradient of 0 - 10% methanol in DCM, to give the title compound (638 mg, 35% yield). LCMS (M+H)⁺ 337, RT 1.57 (Method 12).

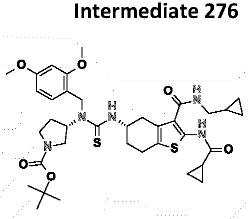

### tert-butyl (3S)-3-[[(55)-2-(cyclopropanecarbonylamino)-3-(cyclopropylmethylcarbamoyl)-4,5,6,7-tetrahydrobenzothiophen-5-yl]carbamothioyl-[(2,4-dimethoxyphenyl)methyl]amino]pyrrolidine-1-carboxylate

To a solution of phenyl chloromethanethioate (183 µL, 1.32 mmol) in DCM (6 mL) at 0 °C was added a solution of **intermediate 117** (400 mg, 1.2 mmol) and triethylamine (502 µL, 3.6 mmol) in DCM (10 mL) over 5 minutes. The solution was stirred at 0 °C for 30 minutes then a solution of **intermediate 275** (626.9 µl, 1.92 mmol) in DCM (10 mL) was added. The reaction was stirred for 20 hours. The reaction was heated at 40 °C for 8 hours, then at room temperature for 20 hours. The reaction was diluted with DCM (10 mL), washed with water (30 mL), saturated aqueous NaHCO₃ (30 mL) and then dried

(MgSO₄). The solvent was removed *in vacuo* to give an oil. Purification *via* column chromatography, using a gradient of 0 - 50% ethyl acetate in iso-hexane gave the title compound (700 mg, 75% yield). LCMS (M+H)⁺ 712, RT 2.16 (Method 12).

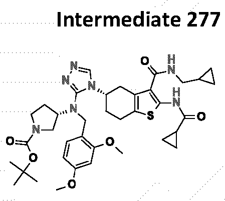

### tert-butyl (3S)-3-[[4-[(55)-2-(cyclopropanecarbonylamino)-3-(cyclopropylmethylcarbamoyl)-4,5,6,7-tetrahydrobenzothiophen-5-yl]-1,2,4-triazol-3-yl]-[(2,4-dimethoxyphenyl)methyl]amino]pyrrolidine-1-carboxylate

The title compound was prepared according to **general method 8,** using thiourea **intermediate 276** (960 mg, 1.35 mmol). The residue was purified *via* column chromatography, using a gradient of 0 - 10% MeOH in ethyl acetate to give a yellow solid. The solid was purified *via* column chromatography (KP-NH), using a gradient of 0 - 12% methanol in TBME to give the title compound (648 mg, 67% yield). LCMS (M+H)⁺ 720, RT 1.27 (Method 11).

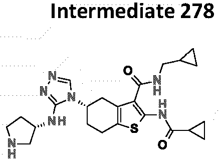

### (5S)-2-(cyclopropanecarbonylamino)-N-(cyclopropylmethyl)-5-[3-[[(3S)-pyrrolidin-3-yl]amino]-1,2,4-triazol-4-yl]-4,5,6,7-tetrahydrobenzothiophene-3-carboxamide

To a stirred solution of **intermediate 277** (648 mg, 0.9 mmol) in DCM (10 mL) at room temperature was added TFA (0.83 mL, 10.8 mmol). The reaction mixture was stirred at room temperature for 2 hours, then was retreated with TFA (0.5 mL, 6.53 mmol) and stirred at 40 °C for 1 hour. The resulting pink suspension was concentrated *in vacuo,* then purified *via* an SCX-2 column, washing with methanol, then eluting product with 7 M ammonia in methanol, to give the title compound (444 mg, 97% yield). δ_{H} (500 MHz, DMSO-*d*₆) 8.39 (s, 1H), 8.10 (s, 1H), 6.03 (d, J = 6.2 Hz, 1H), 4.27 - 4.19 (m, 1H), 4.12 - 4.05 (m, 1H), 3.16 - 3.03 (m, 4H), 3.00 - 2.93 (m, 1H), 2.86 - 2.73 (m, 5H), 2.18 - 2.11 (m, 1H), 2.11 - 1.98 (m, 2H), 1.88 - 1.81 (m, 1H), 1.74 - 1.66 (m, 1H), 1.04 - 0.95 (m, 1H), 0.84 - 0.77 (m, 4H), 0.43 - 0.34 (m, 2H), 0.24 - 0.14 (m, 2H). LCMS (M+H)⁺ 470, RT 1.4 (Method 12).

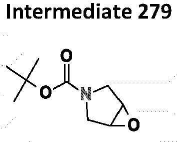

### tert-butyl 6-oxa-3-azabicyclo[3.1.0]hexane-3-carboxylate

To a stirred solution of tert-butyl 2,5-dihydro-1H-pyrrole-1-carboxylate (3 g, 17.73 mmol) in DCM (45 mL), at room temperature under nitrogen was added mCPBA (7.04 g, 40.77 mmol). The mixture was stirred at room temperature overnight. 1 M aqueous NaOH (30 mL) was added and the organic layer separated. The aqueous layer was extracted with DCM (3 x 20 mL) and the organic fractions were combined, dried (Na₂SO₄) and concentrated *in vacuo.* The residue was purified *via* column chromatography, using a gradient of 0 - 30% ethyl acetate in heptanes, to give the title compound (2.17 g, 63% yield). δ_{H} (250 MHz, d-Chloroform) 3.82 (d, J = 12.9 Hz, 1H), 3.74 (d, J = 12.8 Hz, 1H), 3.69 - 3.63 (m, 2H), 3.38 - 3.25 (m, 2H), 1.44 (s, 9H).

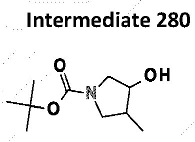

### tert-butyl 3-hydroxy-4-methyl-pyrrolidine-1-carboxylate

To a stirred suspension of copper(i) iodide (4.83 g, 25.38 mmol) in diethyl ether (60 mL), at -10 °C, under nitrogen was added 1.6 M methyllithium in diethyl ether (31.7 mL, 50.72 mmol). The reaction mixture was stirred between -5 °C and -10 °C for 20 minutes, then **intermediate 279** (2 g, 10.1 mmol) was added as a solution in diethyl ether (20 mL). The reaction mixture was stirred at -5 °C for 1 hour, then quenched carefully with water and DCM (10 mL) added. The mixture was filtered and the aqueous layer adjusted to pH 10 using saturated ammonium chloride. The organic layer was separated and the aqueous layer extracted with DCM (3 x 30 mL). The organic fractions were combined, dried (Na₂SO₄) and concentrated *in vacuo* to give the title compound (2.03 g, 84% yield). δ_{H} (500 MHz, d-Chloroform) 3.98 - 3.90 (m, 1H), 3.68 - 3.55 (m, 2H), 3.29 - 3.17 (m, 1H), 3.09 - 2.95 (m, 1H), 2.17 - 2.06 (m, 1H), 1.73 (s, 1H), 1.46 (s, 9H), 1.02 (d, J = 6.9 Hz, 3H).

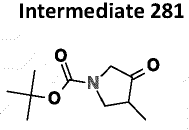

### Tert-butyl 3-methyl-4-oxo-pyrrolidine-1-carboxylate

To a stirred solution of ethanedioyl dichloride (1.28 mL, 15.13 mmol) in DCM (6 mL), at -78 °C was added DMSO (2.15 mL, 30.26 mmol) as a solution in DCM (12 mL). The reaction mixture was stirred at -78 °C for 5 minutes, then **intermediate 280** (2.21 g, 10.97 mmol) was added as a solution in DCM (18 mL). The mixture was stirred at this temperature for 2 hours, then DIPEA (7.03 mL, 50.43 mmol) was added. The mixture was stirred at -78 °C for 1 hour, then diluted with DCM (10 mL) and stirred at room temperature for 2 hours. The mixture was quenched with saturated sodium bicarbonate solution and extracted with DCM (3 x 20 mL). The organic fractions were combined, passed through a hydrophobic frit and concentrated under reduced pressure to give a yellow oil (2.9 g). The oil was purified via column chromatography, using a gradient of 0 - 60% TBME in heptanes to give the title compound (1.6 g, 73% yield). δ_{H} (250 MHz, DMSO-*d*₆) 4.03 - 3.92 (m, 1H), 3.80 (dd, J = 18.7, 1.1 Hz, 1H), 3.57 (d, J = 18.7 Hz, 1H), 3.18 - 2.98 (m, 1H), 2.80 - 2.60 (m, 1H), 1.42 (s, 9H), 1.02 (d, J = 7.1 Hz, 3H).

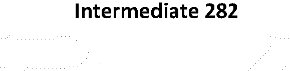

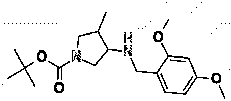

### tert-butyl 3-[(2,4-dimethoxyphenyl)methylamino]-4-methyl-pyrrolidine-1-carboxylate

To a solution of **intermediate 281** (1.5 g, 7.53 mmol) in DCM (30 mL) was added 1-(2,4-dimethoxyphenyl)methanamine (1.26 g, 7.53 mmol). The mixture was stirred at room temperature for 10 minutes, then STAB (2.39 g, 11.29 mmol) was added. The mixture was stirred at room temperature for 3 days, then quenched with saturated sodium bicarbonate and the organic layer separated. The aqueous layer was extracted with DCM (3 x 5 mL) and the organic fractions combined, passed through a hydrophobic frit and purified *via* column chromatography (KP-NH), using a gradient of 0 - 100% TBME in heptanes, to give the title compound (2.16 g, 81% yield). δ_{H} (500 MHz, DMSO-*d*₆) 7.18 (d, J = 8.2 Hz, 1H), 6.52 (d, J = 2.3 Hz, 1H), 6.46 (dd, J = 8.2, 2.2 Hz, 1H), 3.75 (d, J = 13.5 Hz, 6H), 3.57 (s, 2H), 3.31 - 3.20 (m, 2H), 3.10 - 3.00 (m, 2H), 3.01 - 2.94 (m, 1H), 2.27 - 2.17 (m, 1H), 1.80 (s, 1H), 1.38 (s, 9H), 0.94 - 0.86 (m, 3H). LCMS (M+H)⁺ 351, RT 1.59 (Method 12).

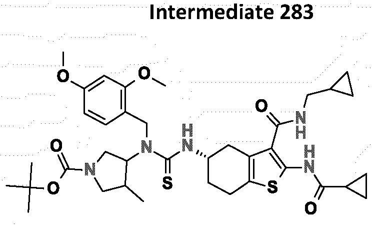

### tert-butyl 3-[[(5S)-2-(cyclopropanecarbonylamino)-3-(cyclopropylmethylcarbamoyl)-4,5,6,7-tetrahydrobenzothiophen-5-yl]carbamothioyl-[(2,4-dimethoxyphenyl)methyl]amino]-4-methyl-pyrrolidine-1-carboxylate

The title compound was prepared according to **general method 7,** using **intermediate 117** (1.67 g, 5 mmol) and **intermediate 282** (1.93 g, 5.5 mmol). The residue was purified *via* column chromatography, using a gradient of 0 - 100% TBME in heptanes, to give the title compound (3.39 g, 72% yield). LCMS (M+H)⁺ 726, RT 2.14 (Method 18).

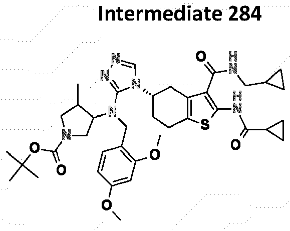

### tert-butyl 3-[[4-[(5S)-2-(cyclopropanecarbonylamino)-3-(cyclopropylmethylcarbamoyl)-4,5,6,7-tetrahydrobenzothiophen-5-yl]-1,2,4-triazol-3-yl]-[(2,4-dimethoxyphenyl)methyl]amino]-4-methyl-pyrrolidine-1-carboxylate

The title compound was prepared according to **general method 8,** using **intermediate 283** (3.39 g, 3.6 mmol). The residue was purified *via* column chromatography, using a gradient of 0 - 6% methanol in DCM, to give the title compound (2.59 g, 91% yield). LCMS (M+H)⁺ 734, RT 1.28 (Method 11).

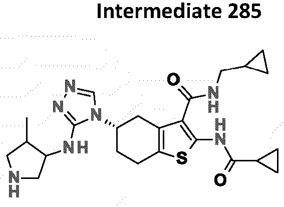

### (5S)-2-(cyclopropanecarbonylamino)-N-(cyclopropylmethyl)-5-[3-[(4-methylpyrrolidin-3-yl)amino]-1,2,4-triazol-4-yl]-4,5,6,7-tetrahydrobenzothiophene-3-carboxamide

To a stirred solution of **intermediate 284** (2.59 g, 3.32 mmol) in DCM (40 mL) at room temperature was added TFA (2.54 mL, 33.2 mmol). The reaction mixture was stirred overnight at room temperature. TFA (0.5 mL, 6.53 mmol) was added and the mixture stirred at room temperature for 2 hours, then at 40 °C for 2 hours. The reaction mixture was cooled to room temperature, then quenched with saturated sodium bicarbonate and filtered, to give the title compound (930 mg, 57% yield). LCMS (M+H)⁺ 484, RT 0.84 (Method 11).

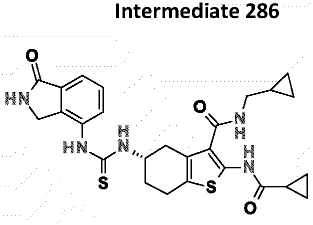

### (5S)-2-(cyclopropanecarbonylamino)-N-(cyclopropylmethyl)-5-[(1-oxoisoindolin-4-yl)carbamothioylaminol-4,5,6,7-tetrahydrobenzothiophene-3-carboxamide

To a stirred solution of di-1H-imidazol-1-ylmethanethione (118 mg, 0.59 mmol) and DIPEA (188 µL, 1.08 mmol) in DCM (4 mL) was added 4-amino-2,3-dihydro-1H-isoindol-1-one (88 mg, 0.59 mmol). The reaction was stirred for 1 hour, then **intermediate 117** (200 mg, 0.54 mmol) was added. The reaction mixture was stirred at room temperature for 1 hour, then concentrated *in vacuo.* The residue was purified via column chromatography, using 0 - 15% methanol in TBME to give the title compound (244 mg, 73% yield). LCMS (M+H)+ 524, RT 1.75 (Method 12).

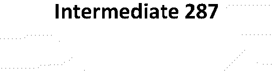

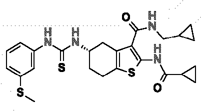

### (5S)-2-(cyclopropanecarbonylamino)-N-(cyclopropylmethyl)-5-[(3-methylsulfanylphenyl)carbamothioylamino]-4,5,6,7-tetrahydrobenzothiophene-3-carboxamide

To a stirring solution of 1-isothiocyanato-3-methylsulfanyl-benzene (113 mg, 0.62 mmol) in anhydrous DCM (2 mL) was added a solution of **intermediate 117** (200 mg, 0.6 mmol) in DCM (3 mL) dropwise under an atmosphere of nitrogen and the mixture was stirred overnight at room temperature. The reaction mixture was concentrated to dryness and purified by flash column chromatography eluting with a gradient of 0 to 100% ethyl acetate in heptane to afford the title compound (300 mg, 97% yield). δ_{H} (500 MHz, Chloroform-d) 12.13 (s, 1H), 7.58 (s, 1H), 7.31- 7.24 (m, 1H), 7.12 (d, J = 8.1 Hz, 1H), 6.97 (t, J = 1.8 Hz, 1H), 6.83 (dd, J = 7.8, 1.5 Hz, 1H), 6.18 (d, J = 7.9 Hz, 1H), 5.86 (t, J = 4.9 Hz, 1H), 4.98 - 4.88 (m, 1H), 3.30 - 3.26 (m, 2H), 3.22 (dd, J = 14.9, 5.0 Hz, 1H), 2.89 - 2.77 (m, 2H), 2.66 - 2.56 (m, 1H), 2.43 (s, 3H), 2.11 - 1.99 (m, 2H), 1.69 - 1.63 (m, 1H), 1.13 - 1.05 (m, 3H), 0.92 - 0.88 (m, 2H), 0.61 - 0.55 (m, 2H), 0.28 (q, J = 4.8 Hz, 2H). LCMS [M+H]⁺ 515, RT 1.98 minutes (Method 12).

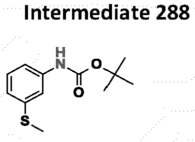

### tert-butyl N-(3-methylsulfanylphenyl)carbamate

A solution of 3-methylsulfanylaniline (0.44 mL, 3.59 mmol) and tert-butoxycarbonyl tert-butyl carbonate (862 mg, 3.95 mmol) in THF (10 mL) was stirred under an atmosphere of nitrogen at 60 °C overnight. The mixture was concentrated to dryness and the crude was purified by flash column chromatography eluting with a 0 to 50% ethyl acetate in heptane gradient to afford the title compound (900 mg, 99% yield) as a colourless oil. δ_{H} (500 MHz, DMSO-d6) 9.33 (s, 1H), 7.43 (s, 1H), 7.22 - 7.13 (m, 2H), 6.84 (dt, J = 6.7, 1.9 Hz, 1H), 2.42 (s, 3H), 1.47 (s, 9H).

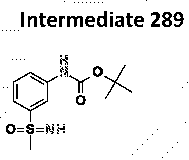

### tert-butyl N-[3-(methylsulfonimidoyl)phenyl]carbamate

To a stirring solution of **Intermediate 288** (300 mg, 1.25 mmol) in anhydrous methanol (2.5 mL) was added ammonium carbamate (391 mg, 5.01 mmol) followed by (diacetoxyiodo)benzene (1211 mg, 3.76 mmol) and the mixture was stirred at room temperature in air for 15 hours. The mixture was concentrated to dryness and the crude was purified by flash column chromatography eluting with a 0 to 100% ethyl acetate in heptane gradient, followed by a 0 to 20% methanol in ethyl acetate gradient to afford the title compound (252 mg, 74% yield) as a white solid. δ_{H} (500 MHz, DMSO-d6) 9.68 (s, 1H), 8.18 (s, 1H), 7.58 (dd, J = 7.9, 1.9 Hz, 1H), 7.51 (dt, J = 7.7, 1.4 Hz, 1H), 7.46 (t, J = 7.8 Hz, 1H), 4.11 (s, 1H), 3.01 (d, J = 1.0 Hz, 3H), 1.48 (s, 9H). LCMS [M+H]⁺ 271, RT 1.54 minutes (Method 12).

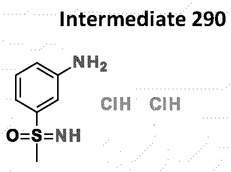

### 3-(methylsulfonimidoyl)aniline dihydrochloride

To a stirring solution of **Intermediate 289** (285 mg, 1.05 mmol) in 1,4-Dioxane (2 mL) was added 4 M HCl in dioxane (2 mL). The reaction was stirred at room temperature for 30 minutes, then at 50 °C for 3 hours. The reaction mixture was concentrated *in vacuo* and the residue was further dried *in vacuo* at 40 °C for 2 hours to afford the title compound (280 mg, 91% purity, 98% yield) as a solid._δ_{H} (500 MHz, DMSO-d6) 7.44 (t, J = 8.0 Hz, 1H), 7.24 (s, 1H), 7.21 (d, J = 7.9 Hz, 1H), 7.05 (d, J = 8.2 Hz, 1H), 3.86 (s, 3H), NH2 and NH masked by water peak.

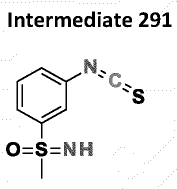

### 3-(methylsulfonimidoyl)phenylisothiocyanate

To a stirring solution of disodium carbonate (403 mg, 3.8 mmol) in water (3.7 mL) cooled to 0°C in an ice bath was added DCM (7.4 mL) followed by carbonothioyl dichloride (0.12 mL, 1.52 mmol). To the biphasic mixture was **Intermediate 290** (185 mg, 0.76 mmol), the ice bath was removed and the resulting mixture was stirred at room temperature for 1 hour. The layers were separated and the aqueous phase was extracted with DCM (2 x 10 mL). The combined organic layers were washed with brine (10 mL), dried over magnesium sulfate, filtered and the volume was reduced to about 10 mL *in vacuo* at 30 °C to afford the title compound as a solution in DCM. LCMS [M+H]⁺ 213, RT 1.49 minutes (Method 12).

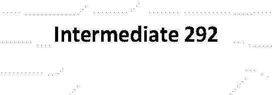

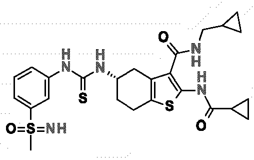

### (5S)-2-(cyclopropanecarbonylamino)-N-(cyclopropylmethyl)-5-[[3-(methylsulfonimidoyl)phenyl]carbamothioylamino]-4,5,6,7-tetrahydrobenzothiophene-3-carboxamide

To a stirring solution of **Intermediate 291** (164 mg, 0.75 mmol) in DCM (10 mL) was added **Intermediate 117** (250 mg, 0.75 mmol) portion wise under an atmosphere of nitrogen and the mixture was stirred at room temperature for 2 hours. Triethylamine (0.52 mL, 3.75 mmol) was added and stirring was continued at room temperature over the weekend. The reaction mixture was diluted with DCM (10 mL), washed with brine (10 mL), dried over magnesium sulfate, filtered and concentrated. The crude material was purified by flash column chromatography eluting with a 0 to 100% ethyl acetate in heptane gradient, followed by a 0 to 20% methanol in ethyl acetate gradient to afford the title compound (258 mg, 60% yield). δ_{H} (500 MHz, DMSO-d6) 11.12 (s, 1H), 9.73 (s, 1H), 8.10 (s, 1H), 7.98 (s, 1H), 7.85 - 7.78 (m, 1H), 7.71 (t, J = 5.7 Hz, 1H), 7.60 (d, J = 7.7 Hz, 1H), 7.51 (t, J = 7.9 Hz, 1H), 4.61 (s, 1H), 4.15 (s, 1H), 3.20 - 3.06 (m, 3H), 3.03 (s, 3H), 2.79 - 2.70 (m, 2H), 2.67 (d, J = 6.9 Hz, 1H), 2.09 - 2.00 (m, 1H), 1.96 - 1.86 (m, 2H), 1.11 - 0.98 (m, 1H), 0.93 - 0.78 (m, 4H), 0.43 (q, J = 5.4 Hz, 2H), 0.23 (q, J = 4.7 Hz, 2H). LCMS [M+H]⁺ 546, RT 1.74 minutes (Method 12).

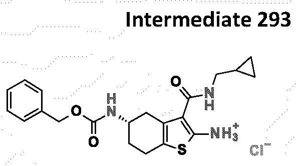

### [(5S)-5-(benzyloxycarbonylamino)-3-(cyclopropylmethylcarbamoyl)-4,5,6,7-tetrahydrobenzothiophen-2-yllammonium chloride

To a stirring solution of **intermediate 564** (1400 mg, 2.80 mmol) in anhydrous 1,4-dioxane (5.6 mL) cooled to 0 °C under an atmosphere of nitrogen was added 4 M hydrogen chloride in 1,4 dioxane (14 mL, 56 mmol) and the resulting mixture was stirred for 10 minutes at this temperature, then the bath was removed and it was allowed to reach room temperature over 3 hours. The mixture was concentrated *in vacuo* maintaining the temperature of the bath at 30 °C to afford the title compound (1.43 g, 99% yield). LCMS [M+H]⁺ 400, RT 1.89 minutes (Method 12).

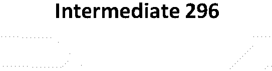

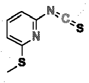

### 2-isothiocyanato-6-methylsulfanyl-pyridine

To stirring solution of disodium carbonate (1890 mg, 17.83 mmol) in water (5 mL) was added DCM (5 mL) followed by carbonothioyl dichloride (0.54 mL, 7.13 mmol). The biphasic mixture was cooled to 0 °C in an ice-bath and a solution of 6-methylsulfanylpyridin-2-amine (500 mg, 3.57 mmol) in DCM (5 mL) was added dropwise. The ice bath was removed and the resulting mixture was allowed to reach room temperature and stirred for 1 hour. The layers were separated and the aqueous phase was extracted with DCM (2 x 10 mL). The combined organic layers were washed with brine (10 mL), dried over magnesium sulfate, filtered and concentrated *in vacuo* to afford the title compound (334 mg, 51% yield) as an oil. δ_{H} (500 MHz, Chloroform-d) 7.47 (t, J = 7.8 Hz, 1H), 7.08 (dd, J = 8.0, 0.6 Hz, 1H), 6.77 (dd, J = 7.7, 0.6 Hz, 1H), 2.55 (s, 3H).

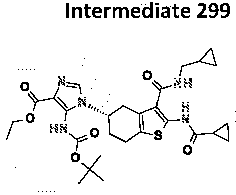

### ethyl 5-(tert-butoxycarbonylamino)-1-[(5S)-2-(cyclopropanecarbonylamino)-3-(cyclopropylmethylcarbamoyl)-4,5,6,7-tetrahydrobenzothiophen-5-yl]imidazole-4-carboxylate

Sodium hydride (60%, 255 mg, 6.38 mmol) was added in one portion to the stirred solution of **Example 117** (1.0 g, 2.12 mmol) in dry DMF (15 mL) at room temperature. The mixture was stirred for 5 minutes, then tert-butoxycarbonyl tert-butyl carbonate (509 mg, 2.33 mmol) was added (Note: Initially a gel formed with poor stirring which was broken down after sonication and as the reaction time progressed). The mixture was diluted with dry DMF (5 mL), sonicated for 5 minutes and stirred at room temperature for 1 h. The reaction was treated with tert-butoxycarbonyl tert-butyl carbonate (100 mg, 0.46 mmol) and stirred for 1 h. The reaction was cooled to 0 °C, quenched with saturated aqueous NH₄Cl (5 mL), diluted with DCM (50 mL) and washed with aq. saturated NH₄Cl (50 mL) followed by water (3 x 50 mL). The organic layers were dried over sodium sulfate, filtered and concentrated under vacuum to afford solid. The crude solid was purified by column chromatography, eluting with 0-100% Ethyl acetate in heptane to give the title compound (400 mg, 26% yield). LCMS [M+H]⁺ 572, RT 1.90 minutes (Method 12).

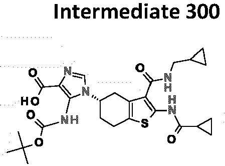

### 5-(tert-butoxycarbonylamino)-1-[(5S)-2-(cyclopropanecarbonylamino)-3-(cyclopropylmethylcarbamoyl)-4,5,6,7-tetrahydrobenzothiophen-5-yl]imidazole-4-carboxylic acid

A solution of **Intermediate 299** (78% purity, 400 mg, 0.55 mmol) in THF (4 mL) was treated with 1 M sodium hydroxide (2.7 mL, 2.7 mmol) at room temperature and stirred for 2 days. The mixture was concentrated under reduced pressure (NB water bath kept 25°C due to concern of decarboxylation), then adjusted to pH 5 with 1 M HCl and extracted with DCM (3 x 10 mL), dried over sodium sulfate, concentrated under reduced pressure at 25 °C to give the title compound (267 mg, 78% yield). LCMS [M+H]⁺ 544, RT 1.74 minutes (Method 12).

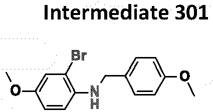

### 2-bromo-4-methoxy-N-[(4-methoxyphenyl)methyl]aniline

A solution of 4-methoxybenzaldehyde (60 µL, 0.49 mmol), acetic acid (30 µL, 0.52 mmol) and 4-methoxybenzaldehyde (60 µL, 0.494 mmol) in DCE (1.5 mL) was stirred for 10 min then sodium triacetoxyboranuide (210 mg, 0.99 mmol) was added and stirred at room temperature for 18 h. The reaction mixture diluted with water (10 mL) and extracted with DCM (2 x 10 mL). The combined organic layers were dried over sodium sulfate, filtered and evaporated to dryness to afford the crude. This was purified by column chromatography, eluting with 0-100% ethyl acetate in heptane to give the title compound (145 mg, 84% yield). δ_{H} (500 MHz, Chloroform-d) 7.33 - 7.29 (m, 2H), 7.10 (d, *J* = 2.8 Hz, 1H), 6.94 - 6.88 (m, 2H), 6.79 (dd, *J* = 8.9, 2.9 Hz, 1H), 6.61 (d, *J* = 8.9 Hz, 1H), 4.35 (s, 1H), 4.30 (s, 2H),3.83 (s, 3H), 3.75 (s, 3H). LCMS [M+H]⁺ Doesn't ionised, RT 2.10 minutes (Method 12).

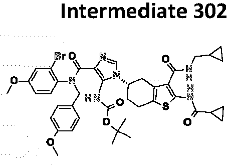

### tert-butyl N-[5-[(2-bromo-4-methoxy-phenyl)-[(4-methoxyphenyl)methyl]carbamoyl]-3-[(5S)-2-(cyclopropanecarbonylamino)-3-(cyclopropylmethylcarbamoyl)-4,5,6,7-tetrahydrobenzothiophen-5-yllimidazol-4-yllcarbamate

T3P (50% w/w in ethyl acetate, 2.4 mL, 4.03 mmol) was added dropwise to the stirred solution of **Intermediate 300** (300 mg, 0.52 mmol), **Intermediate 301** (587 mg, 1.73 mmol) and pyridine (225 µL, 2.79 mmol) in dry DMF (0.2 mL) at 0 °C. The mixture was then gradually warmed up to room temperature and stirred for 18 h. The reaction mixture neutralized with saturated aqueous NaHCO₃ and extracted with DCM (3 x 5 mL). The combined organic layers were dried over sodium sulfate, filtered and evaporated to dryness to afford the crude. This was purified by column chromatography, eluting with 0-100% ethyl acetate in heptane to give the title compound (240 mg, 54% yield). δ_{H} (500 MHz, Chloroform-d) 12.31 - 12.02 (m, 1H), 8.28 - 7.77 (m, 1H), 7.27 - 6.95 (m, 4H), 6.85 - 6.61 (m, 4H), 5.91 - 5.71 (m, 1H), 5.63 (dd, J = 14.3, 5.2 Hz, 1H), 4.67 - 4.44 (m, 1H), 4.21 - 4.08 (m,1H), 3.86 - 3.71 (m, 6H), 3.55 - 3.15 (m, 3H), 3.03 - 2.69 (m, 3H), 2.47 - 2.23 (m, 1H), 2.21 - 2.07 (m, 1H), 1.63 - 1.44 (m, 9H), 1.17 - 0.99 (m, 3H), 0.96 - 0.78 (m, 3H), 0.63 - 0.50 (m, 2H), 0.35 - 0.17 (m, 2H). LCMS [M+H]⁺ 847/849 (Br-isotope), RT 2.17 minutes (Method 12).

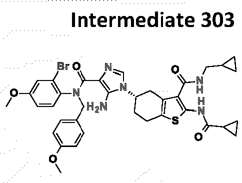

### 5-amino-N-(2-bromo-4-methoxy-phenyl)-1-[(55)-2-(cyclopropanecarbonylamino)-3-(cyclopropylmethyl carbamoyl)-4,5,6,7-tetrahydrobenzothiophen-5-yl]-N-[(4-methoxyphenyl)methyl]imidazole-4-carboxamide

4 M HCl in 1,4-dioxane (708 µL, 2.83 mmol) was added to the stirred solution of **Intermediate 302** (240 mg, 0.283 mmol) in DCM (0.5 mL) at room temperature and the mixture stirred for 5 h. The reaction mixture was concentrated under reduced pressure and the crude material purified by column chromatography, eluting with 0-20% methanol in DCM to give the title compound (200 mg, 94% yield). δ_{H} (500 MHz, DMSO-d6) 11.03 (s, 1H), 7.60 - 7.34 (m, 1H), 7.06 - 6.86 (m, 3H), 6.75 - 6.58 (m, 3H), 6.58 - 6.38 (m, 2H), 6.21 - 5.99 (m, 2H), 4.23 - 3.95 (m, 1H), 3.83 - 3.66 (m, 1H), 3.54 (s, 3H), 3.51 (s,3H), 2.87 (d, J = 49.9 Hz, 4H), 2.72 - 2.46 (m, 3H), 2.12 - 1.79 (m, 2H), 1.75 - 1.63 (m, 1H), 0.93 - 0.72 (m, 1H), 0.73 - 0.56 (m, 4H), 0.31 - 0.10 (m, 2H), 0.10 - -0.13 (m, 2H). LCMS [M+H]⁺ 747/749 (Br-isotope), RT 2.09 minutes (Method 12).

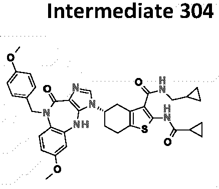

### (5S)-2-(cyclopropanecarbonylamino)-N-(cyclopropylmethyl)-5-[8-methoxy-5-[(4-methoxyphenyl)methyl]-4-oxo-10H-imidazo[4,5-c][1,5]benzodiazepin-1-yl]-4,5,6,7-tetrahydrobenzothiophene-3-carboxamide

**Intermediate 303** (140 mg, 0.187 mmol) and sodium 2-methylpropan-2-olate (84 mg, 0.874 mmol) were suspended in dry tert-butanol (2 mL) and 1,4-Dioxane (1 mL). The mixture was de-gassed for 5 minutes, then added tBuXPhos Pd G3 (14 mg, 0.0176 mmol) was added. The mixture was heated at 90 °C in sealed tube for 24 h. The mixture was cooled to room temperature, diluted with water (20 mL) and extracted with 10% methanol in DCM (3 x 20 mL). The combine organic layers were diluted with brine (20 mL, pH= ^{~}14) and adjusted to pH 7 using acetic acid. The layers were separated and the organic layer dried over sodium sulfate, filtered, evaporated to dryness to afford the crude. The crude material was purified by column chromatography, eluting with 0-20% methanol in DCM to give the title compound (80 mg, 64% yield). δ_{H} (500 MHz, DMSO-d6) 11.24 (s, 1H), 8.20 (s, 1H), 7.72 - 7.63 (m, 1H), 7.51 (s, 1H), 7.20 - 7.13 (m, 3H), 6.84 - 6.76 (m, 2H), 6.68 (d, J = 2.9 Hz, 1H), 6.55 (dd, J = 9.0, 2.9 Hz, 1H), 5.09 - 4.90 (m,2H), 4.58 - 4.48 (m, 1H), 3.68 (s, 3H), 3.65 (s, 3H), 3.23 - 3.15 (m, 1H), 3.15 - 3.05 (m, 1H), 3.05 - 2.98 (m, 1H), 2.98 - 2.75 (m, 3H), 2.34 - 2.01 (m, 2H), 1.97 - 1.84 (m, 1H), 1.35 - 1.20 (m, 4H), 0.91 - 0.80 (m, 1H),0.38 - 0.24 (m, 2H), 0.24 - 0.10 (m, 2H). LCMS [M+H]⁺ 667, RT 1.89 minutes (Method 12).

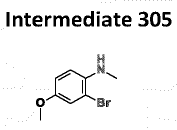

### 2-bromo-4-methoxy-N-methyl-aniline

1.6 M methyllithium in Et₂O (1.7 mL, 2.72 mmol) was added dropwise to the stirred solution of 2-bromo-4-methoxy-aniline (500 mg, 2.47 mmol) in dry THF (5 mL) at -78 °C. The reaction mixture stirred for 1h at -78 °C, then iodomethane (185 µL, 2.97 mmol) in dry THF (3 mL) was added. The mixture was gradually warmed up to room temperature and stirred for 4 h. The reaction mixture was quenched with saturated aqueous NH₄Cl (25 mL) and extracted with ethyl acetate (3 x 25 mL). The combined organic layers were washed with brine (25 mL), dried over sodium sulfate, filtered and concentrated to dryness to afford the crude. The crude was purified by column chromatography, eluting with 0-100% TBME in heptane to give the title compound (416 mg, 78% yield). LCMS [M+H]⁺ 216/218, RT 1.75 minutes (Method 12).

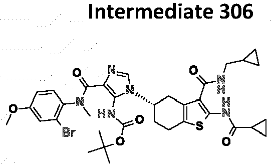

### tert-butyl N-[5-[(2-bromo-4-methoxy-phenyl)-methyl-carbamoyl]-3-[(5S)-2-(cyclopropanecarbonylamino)-3-(cyclopropylmethylcarbamoyl)-4,5,6,7-tetrahydrobenzothiophen-5-yl]imidazol-4-yl]carbamate

T3P (50% w/w in ethyl acetate, 800 µL, 1.34 mmol) was added dropwise to a stirred solution of **Intermediate 300** (100 mg, 0.17 mmol), **Intermediate 305** (160 mg, 0.74 mmol) and pyridine (75 µL, 0.931 mmol) in dry DMF (2 mL) at 0 °C. The mixture was gradually warmed up to room temperature and stirred for 4 h. The reaction mixture was diluted with water (25 mL), neutralized with sat. aq. NaHCO₃ and extracted with DCM (3 x 25 mL). The combined organic layers were dried over sodium sulfate, filtered and evaporated to dryness to afford the crude. This was purified by column chromatography, eluting with 0-100% ethyl acetate in heptane to give the title compound (110 mg, 64% yield). LCMS [M+H]⁺ 741/743, RT 2.03 minutes (Method 12).

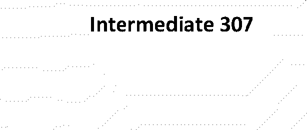

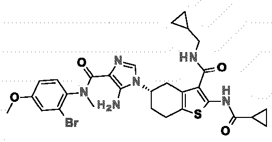

### 5-amino-N-(2-bromo-4-methoxy-phenyl)-1-[(5S)-2-(cyclopropanecarbonylamino)-3-(cyclopropylmethyl carbamoyl)-4,5,6,7-tetrahydrobenzothiophen-5-yl]-N-methyl-imidazole-4-carboxamide

2,2,2-Trifluoroacetic acid (0.30 mL, 4.04 mmol) was added to the stirred solution of **Intermediate 306** (100 mg, 0.101 mmol) in DCM (1 mL) at room temperature. The mixture was stirred for 3 h. The reaction mixture concentrated under reduced pressure and purified by SCX chromatography, eluting with 7 M ammonia in methanol to provide a mixture of acid and the title compound. This crude material was dissolved in DCM:MeOH (9:1) (25 mL) and washed with sat. aq. NaHCO₃ (2 x 10 mL). The organic layer was dried over sodium sulfate, filtered and evaporated to dryness to afford the crude. This was further purified by column chromatography, eluting with 0-20% methanol in DCM to give the title compound (60 mg, 90% yield). δ_{H} (500 MHz, DMSO-d6) 11.23 (s, 1H), 7.66 (br s, 1H), 7.39 - 7.10 (m, 2H), 7.09 - 6.74 (m, 2H), 6.22 (s, 2H), 4.47 - 4.13 (m, 1H), 3.78 (s, 3H), 3.26 - 2.88 (m, 6H), 2.94 - 2.66 (m, 3H), 2.29 - 1.99 (m, 2H),1.95 - 1.85 (m, 1H), 1.01 (s, 1H), 0.90 - 0.77 (m, 4H), 0.45 - 0.31 (m, 2H), 0.25 - 0.14 (m, 2H). LCMS [M+H]⁺ 641/643, RT 3.16 minutes (Method 10).

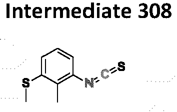

### 1-isothiocyanato-2-methyl-3-methylsulfanyl-benzene

To a stirring solution of sodium carbonate (881 mg, 8.16 mmol) in water (2.5 mL) previously cooled in an ice/water bath was added DCM (2.5 mL) followed by thiophosgene (0.25 mL, 3.26 mmol). The biphasic mixture was then treated with a solution of 2-methyl-3-methylsulfanyl-aniline (250 mg, 1.63 mmol) in DCM (2.5 mL) dropwise. The ice bath was removed and the resulting mixture was allowed to reach room temperature and stirred for 1 h. The layers were separated and the aqueous phase was extracted with DCM (20 mL). The combined organics were dried over magnesium sulfate, filtered and concentrated under reduced pressure. The crude material was purified by flash column chromatography eluting with a 0-10% ethyl acetate in heptane gradient to give the title compound (300 mg, 92% Yield). The material was used immediately in the next step. LCMS [no ionisation], RT 2.15 minutes (Method 12).

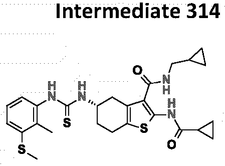

### (5S)-2-(cyclopropanecarbonylamino)-N-(cyclopropylmethyl)-5-[(2-methyl-3-methylsulfanyl-phenyl)carbamothioylamino]-4,5,6,7-tetrahydrobenzothiophene-3-carboxamide

**Intermediate 117** (250 mg, 0.75 mmol) was dissolved in DCM (5 mL) and **Intermediate 308** (146 mg, 0.75 mmol) was added. The mixture was stirred at room temperature for 16 h. Concentrated under reduced pressure and purified by flash column chromatography eluting with a 30-60% ethyl acetate in heptane gradient to give the title compound (317 mg, 72% Yield). δ_{H} (500 MHz, Chloroform-d) 12.13 (s, 1H), 7.41 (s, 1H), 7.20 (t, J = 7.9 Hz, 1H), 7.12 (d, J = 7.7 Hz, 1H), 6.90 (d, J = 7.7 Hz, 1H), 5.84 (t, J = 4.9 Hz, 1H), 5.69 (d, J = 8.1 Hz, 1H), 4.94 - 4.85 (m, 1H), 3.33 - 3.24 (m, 2H), 3.21 (dd, J = 14.8, 5.0 Hz, 1H), 2.81 (dt, J = 17.0, 6.4 Hz, 1H), 2.70 (dd, J = 14.8, 6.2 Hz, 1H), 2.52 (dt, J = 17.0, 6.2 Hz, 1H), 2.47 (s, 3H), 2.19 (s, 3H), 2.08 - 1.99 (m, 1H), 1.94 - 1.85 (m, 1H), 1.69 - 1.62 (m, 1H), 1.13 - 1.02 (m, 3H), 0.92 - 0.87 (m, 2H), 0.62 - 0.56 (m, 2H), 0.31 - 0.23 (m, 2H). LCMS [M+H]⁺ 529, RT 1.97 minutes (Method 12).

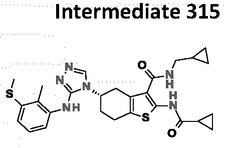

### (5S)-2-(cyclopropanecarbonylamino)-N-(cyclopropylmethyl)-5-[3-(2-methyl-3-methylsulfanyl-anilino)-1,2,4-triazol-4-yl]-4,5,6,7-tetrahydrobenzothiophene-3-carboxamide

Prepared according to **general procedure 8** using **intermediate 314** (317 mg, 0.60 mmol). Purified by flash column chromatography eluting with a 0-10% methanol in DCM gradient to give the title compound (75 mg, 21% Yield) as a white solid. δ_{H} (500 MHz, DMSO-d₆) 11.19 (s, 1H), 8.40 (s, 1H), 7.75 (s, 1H), 7.71 (t, J = 5.6 Hz, 1H), 7.10 (t, J = 8.0 Hz, 1H), 6.92 - 6.83 (m, 2H), 4.45 - 4.35 (m, 1H), 3.16 - 2.96 (m, 4H), 2.84 - 2.77 (m, 2H), 2.44 (s, 3H), 2.27 - 2.15 (m, 2H), 2.14 (s, 3H), 1.95 - 1.89 (m, 1H), 1.03 - 0.94 (m, 1H), 0.88 - 0.81 (m, 4H), 0.39 - 0.32 (m, 2H), 0.21 - 0.16 (m, 2H). LCMS [M+H]⁺ 537, RT 2.83 minutes (Method 29).

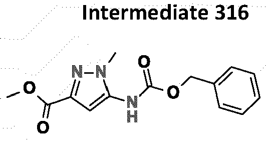

### Methyl 5-(benzyloxycarbonylamino)-1-methyl-pyrazole-3-carboxylate

Methyl 5-amino-1-methyl-1H-pyrazole-3-carboxylate (500 mg, 3.22 mmol) was dissolved in THF (10 mL) and sodium hydrogen carbonate (298 mg, 3.54 mmol) was added followed by benzyl carbonochloridate (506 µL, 3.54 mmol). The solution was stirred at room temperature for 2 h, to give a cloudy mixture. The mixture was extracted with ethyl acetate (2 x 40 mL) and the combined organic extracts were washed with brine (20 mL), dried over MgSO₄ and filtered. Concentrated under reduced pressure and purified by flash column chromatography eluting with a 50-80% ethyl acetate in heptane gradient to give the title compound (710 mg, 66% Yield). δ_{H} (500 MHz, Chloroform-d) 7.34 (m, 5H), 6.91 (s, 1H), 6.69 (s, 1H), 5.19 (s, 2H), 3.87 (s, 3H), 3.76 (s, 3H). LCMS [M+H]⁺ 290, RT 1.65 minutes (Method 12).

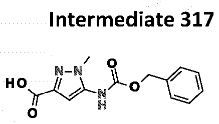

### 5-(benzyloxycarbonylamino)-1-methyl-pyrazole-3-carboxylic acid

**Intermediate 316** (816 mg, 2.45 mmol) was dissolved in 1,4-dioxane (5 mL) and 2 M aqueous lithium hydroxide (2.5 mL, 4.91 mmol) was added. The mixture was stirred at room temperature for 16 h before removing solvent under reduced pressure. The solution was acidified with conc. HCl to give a white precipitate which was collected by vacuum filtration, washing with water to afford the title compound (460 mg, 68% Yield). δ_{H} (500 MHz, DMSO-d₆) 12.56 (br s, 1H), 9.93 (s, 1H), 7.46 - 7.32 (m, 5H), 6.52 (s, 1H), 5.17 (s, 2H), 3.72 (s, 3H). LCMS [M+H]⁺ 276, RT 1.51 minutes (Method 12).

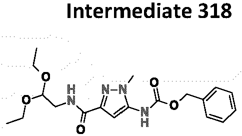

### Benzyl N-[5-(2,2-diethoxyethylcarbamoyl)-2-methyl-pyrazol-3-yl]carbamate

A suspension of **Intermediate 317** (460 mg, 1.64 mmol) in DCM (10 mL) was treated with N-ethyl-N-isopropyl-propan-2-amine (0.63 mL, 3.60 mmol) to give a solution. HATU (685 mg, 1.80 mmol) was added and the mixture was stirred at room temperature for 10 minutes before adding 2,2-diethoxyethanamine (0.25 mL, 1.80 mmol). Stirring was continued at room temperature for 2 h before concentrating under reduced pressure. Purified by flash column chromatography eluting with a 60-80% ethyl acetate in heptane gradient to give the title compound (650 mg, 100% Yield) as a colourless oil. LCMS [M-H]⁻ 389, RT 1.74 minutes (Method 12).

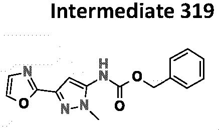

### Benzyl N-(2-methyl-5-oxazol-2-yl-pyrazol-3-yl)carbamate

**Intermediate 318** (655 mg, 1.63 mmol) was dissolved in THF (10 mL) and 2 M aqueous HCl (5.0 mL, 10.0 mmol) was added. The mixture was stirred at room temperature for 16 h. The mixture was diluted with water (50 mL) and extracted with ethyl acetate (2 x 50 mL). The combined organic extracts were dried over MgSO₄, filtered and concentrated under reduced pressure. The residue was dissolved in THF (10 mL) and treated with Burgess reagent (969 mg, 4.07 mmol). The solution was stirred at room temperature for 20 h. Diluted with ethyl acetate (50 mL) then washed with water (50 mL). The aqueous was extracted further with ethyl acetate (50 mL) and the combined organic extracts were washed with brine (25 mL), dried over MgSO₄ and filtered. Concentrated under reduced pressure and purified by flash column chromatography eluting with a 0-10% methanol in DCM gradient to give the title compound (30 mg, 6% Yield). δ_{H} (250 MHz, DMSO-d₆) 10.00 (s, 1H), 8.12 (d, J = 0.8 Hz, 1H), 7.48 - 7.34 (m, 5H), 7.31 (d, J = 0.8 Hz, 1H), 6.62 (s, 1H), 5.19 (s, 2H), 3.75 (s, 3H). LCMS [M+H]⁺ 299, RT 1.64 minutes (Method 12).

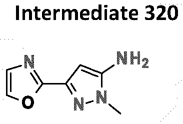

### 2-methyl-5-oxazol-2-yl-pyrazol-3-amine

**Intermediate 319** (30 mg, 0.10 mmol) was dissolved in ethanol (2 mL) and 10% Pd/C (50% wet) (21 mg, 0.01 mmol) was added. The mixture was stirred under an atmosphere of H₂ at room temperature for 2 h. After this time the mixture was filtered through Celite and concentrated under reduced pressure to give the title compound (20 mg, 99% Yield). LCMS [M+H]⁺ 165, RT 0.27 minutes (Method 12).

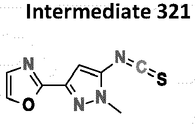

### 2-(5-isothiocyanato-1-methyl-pyrazol-3-yl)oxazole

To a stirring solution of sodium carbonate (53 mg, 0.487 mmol) in water (0.25 mL) cooled to 0 °C was added DCM (0.5 mL) followed by thiophosgene (15 µL, 0.195 mmol). The biphasic mixture was then treated with a solution of **Intermediate 320** (16 mg, 0.0975 mmol). The ice bath was removed and the resulting mixture was allowed to reach room temperature and stirred for 1 h. The layers were separated and the aqueous phase was extracted with DCM (10 mL). The combined organics were dried over MgSO₄, filtered and concentrated under reduced pressure. The crude material was purified by column chromatography eluting with a 50-65% ethyl acetate in heptane gradient to give the title compound (20 mg, quantitative). LCMS [M+H]⁺ 207, RT 1.62 minutes (Method 12).

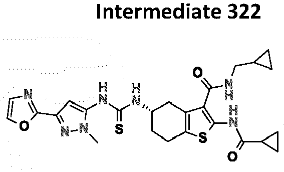

### (5S)-2-(cyclopropanecarbonylamino)-N-(cyclopropylmethyl)-5-[(2-methyl-5-oxazo1-2-yl-pyrazol-3-yl)carbamothioylamino]-4,5,6,7-tetrahydrobenzothiophene-3-carboxamide

**Intermediate 117** (32 mg, 0.097 mmol) was dissolved in DCM (2 mL) and **Intermediate 321** (20 mg, 0.097 mmol) was added. The mixture was stirred at room temperature for 16 h. Concentrated under reduced pressure and purified by flash column chromatography eluting with a 5-8% methanol in DCM gradient to give the title compound (18 mg, 32% Yield). δ_{H} (500 MHz, Chloroform-d) 12.04 (s, 1H), 7.70 (s, 1H), 7.46 (d, J = 2.1 Hz, 1H), 7.21 (s, 1H), 6.66 (s, 1H), 6.30 (d, J = 6.7 Hz, 1H), 5.81 (t, J = 5.1 Hz, 1H), 4.90 - 4.82 (m, 1H), 3.83 (s, 3H), 3.31 - 3.21 (m, 3H), 2.91 - 2.80 (m, 1H), 2.77 - 2.63 (m, 2H), 2.16 - 2.07 (m, 1H), 2.07 - 1.98 (m, 1H), 1.70 - 1.63 (m, 1H), 1.12 - 1.03 (m, 3H), 0.94 - 0.88 (m, 2H), 0.62 - 0.54 (m, 2H), 0.29 - 0.24 (m, 2H). LCMS [M+H]⁺ 540, RT 1.77 minutes (Method 12).

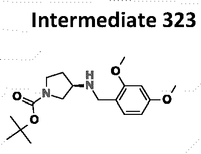

### tert-butyl (3R)-3-[(2,4-dimethoxyphenyl)methylamino]pyrrolidine-1-carboxylate

The title compound was obtained following **general method 2** with tert-butyl (3R)-3-aminopyrrolidine-1-carboxylate (300 mg, 1.61 mmol), 2,4-dimethoxybenzaldehyde (267 mg, 1.61 mmol) and STAB (512 mg, 2.41 mmol). Reaction time 4 h. The residue was purified by column chromatography eluting with 0 - 20 % MeOH in DCM to give the title compound (495 mg, 91% yield). LCMS [M+H]⁺ 337, RT 0.91 minutes (Method 6).

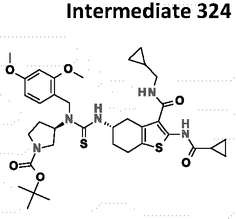

### tert-butyl (3R)-3-[[(5S)-2-(cyclopropanecarbonylamino)-3-(cyclopropylmethylcarbamoyl)-4,5,6,7-tetrahydrobenzothiophen-5-yl]carbamothioyl-[(2,4-dimethoxyphenyl)methyl]amino]pyrrolidine-1-carboxylate

The title compound was obtained following **general method 7** with **intermediate 117** (290 mg, 0.87 mmol), triethylamine (0.364 ml, 2.60 mmol), phenyl chloromethanethioate (0.133 ml, 0.95 mmol) and **intermediate 323** (497.0 mg, 1.47 mmol). Crude was purified by column chromatography eluting with 0 - 100% ethyl acetate in heptane to give the title compound (582 mg, 86% yield). LCMS [M+H]⁺ 712, RT 1.4 minutes (Method 6).

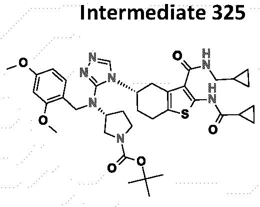

### tert-butyl (3R)-3-[[4-[(5S)-2-(cyclopropanecarbonylamino)-3-(cyclopropylmethylcarbamoyl)-4,5,6,7-tetrahydrobenzothiophen-5-yl]-1,2,4-triazol-3-yl]-[(2,4-dimethoxyphenyl)methyl]amino]pyrrolidine-1-carboxylate

The title compound was obtained following **general method 8** with **intermediate 324** (582 mg, 0.81 mmol), formic hydrazide (147.2 mg, 2.45 mmol), mercury dichloride (665.8 mg, 2.45 mmol) and triethylamine (0.34 ml, 2.45 mmol). Reaction time 2 hours. The residue was purified by column chromatography eluting with 0 - 20 % MeOH in DCM to give the title compound (554 mg, 93% Yield). LCMS [M+H]⁺ 720, RT 1.24 minutes (Method 11).

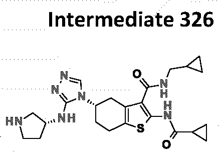

### (5S)-2-(cyclopropanecarbonylamino)-N-(cyclopropylmethyl)-5-[3-[[(3R)-pyrrolidin-3-yl]amino]-1,2,4-triazol-4-yl]-4,5,6,7-tetrahydrobenzothiophene-3-carboxamide

The title compound was obtained following **general method 4** with **intermediate 325** (554 mg, 0.53 mmol) and 4 M HCl in dioxane (3.88 ml, 15.3 mmol). Reaction time 3 h. After removing solvent crude (420 mg, bis HCl salt) material was used in the next stage without further purification. δ_{H} (500 MHz, Methanol-d4) 8.15 (s, 1H), 4.41 - 4.32 (m, 1H), 4.28 - 4.21 (m, 1H), 3.31 - 3.15 (m, 4H), 3.15 - 3.06 (m, 1H), 3.03 - 2.84 (m, 5H), 2.35 - 2.17 (m, 3H), 1.91 - 1.77 (m, 2H), 1.14 -1.05 (m, 1H), 1.04 - 0.93 (m, 4H), 0.54 - 0.47 (m, 2H), 0.30 - 0.24 (m, 2H). LCMS [M+H]⁺ 470, RT 1.33 minutes (Method 10).

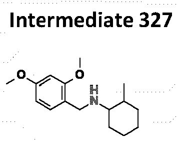

### N-[(2,4-dimethoxyphenyl)methyl]-2-methyl-cyclohexanamine

The title compound was obtained following **general method** 2 with 2-methylcyclohexanamine (100 mg, 0.88 mmol), 2, 4-dimethoxybenzaldehyde (147 mg, 0.88 mmol) and STAB (281 mg, 1.32 mmol). Reaction time 4 h. The residue was purified by column chromatography eluting with 0 - 20 % MeOH in DCM to give the title compound (169 mg, 71%). LCMS [M+H]⁺ 264, RT 0.95 minutes (Method 6).

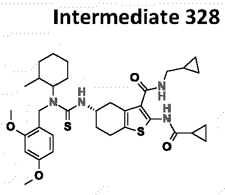

### (5S)-2-(cyclopropanecarbonylamino)-N-(cyclopropylmethyl)-5-[[(2,4-dimethoxyphenyl)methyl-(2-methylcyclohexyl)carbamothioyl]amino]-4,5,6,7-tetrahydrobenzothiophene-3-carboxamide

The title compound was obtained following **general method** 7 with **intermediate** 117 (100 mg, 0.3 mmol), triethylamine (0.125 ml, 0.9 mmol), phenyl chloromethanethioate (0.045 ml, 0.33 mmol) and intermediate 327 (134 mg, 0.51 mmol). Crude was purified by column chromatography eluting with 0 - 100% ethyl acetate in heptane to give the title compound (166 mg, 84% Yield). LCMS [M+H]⁺ 639, RT 1.51 minutes (Method 6).

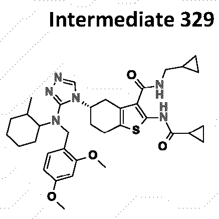

### (5S)-2-(cyclopropanecarbonylamino)-N-(cyclopropylmethyl)-5-[3-[(2,4-dimethoxyphenyl)methyl-(2-methylcyclohexyl)amino]-1,2,4-triazol-4-yl]-4,5,6,7-tetrahydrobenzothiophene-3-carboxamide

The title compound was obtained following **general method** 8 with **intermediate 328** (166 mg, 0.25 mmol), formic hydrazide (45.8 mg, 0.76 mmol), mercury dichloride (207.3 mg, 0.76 mmol) and triethylamine (0.10 ml, 0.76 mmol). Reaction time 2 hours. The orange residue was purified by column chromatography eluting with 0 - 20 % MeOH in DCM to give the title compound (146 mg, 85% yield). LCMS [M+H]⁺ 647, RT 1.43 minutes (Method 11).

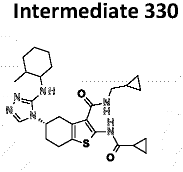

### (5S)-2-(cyclopropanecarbonylamino)-N-(cyclopropylmethyl)-5-[3-[(2-methylcyclohexyl)amino]-1,2,4-triazol-4-yl]-4,5,6,7-tetrahydrobenzothiophene-3-carboxamide

The title compound was obtained following **general method 5** with **intermediate 329** (146 mg, 0.24 mmol) and TFA (172.7 µl, 2.25 mmol). Reaction stirred for 3 h at room temperature. The crude was purified by reverse phase column chromatography eluting with 5 - 95 % Acetonitrile (0. 1 % formic acid) in water (0. 1 % formic acid) gave the title compound (54 mg, 48% Yield) as a mixture of 4 diastereomers. δ_{H} (500 MHz, Methanol-d4) 8.14 (s, 1H, isomer 1), 8.13 (s, 1H, isomer 2), 8.11 (s, 1H, isomer 3), 8.09 (s, 1H, isomer 4), 4.52 (m, 1H, isomers), 4.38 (m, 1H, isomers), 3.86 - 3.78 (m, 1H, isomer), 3.31 - 3.13 (m, 4H), 2.95 (m, 3H), 2.37 - 2.25 (m, 2H), 2.17 (m, 1H, isomer), 2.10 (m, 1H, isomer), 1.88 - 1.76 (m, 3H, isomers), 1.72 (m, 1H), 1.68 - 1.38 (m, 3H, isomers), 1.37 - 1.21 (m, 2H, isomers), 1.21 - 1.13 (m, 1H, isomer), 1.08 (m, 1H), 1.01 (m, 4H), 0.96 (m, 3H), 0.54 - 0.46 (m, 2H), 0.27 (m, 2H). LCMS [M+H]⁺ 497, RT 2.25 minutes (Method 10).

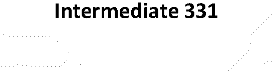

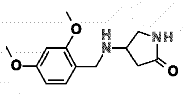

### 4-[(2,4-dimethoxyphenyl)methylamino]pyrrolidin-2-one

To a solution of 4-aminopyrrolidin-2-one hydrochloride (100 mg, 0.73 mmol) in DCM (10 mL) were added DIPEA (0.19 ml, 1.09 mmol) and 2,4-dimethoxybenzaldehyde (122 mg, 0.73 mmol), followed by STAB (233 mg, 1.09 mmol). The mixture was stirred at room temperature for 4 h. Then heated at 40 °C for 16 h. Reaction mixture was cooled to 0 °C and NaBH₄ (41.5 mg, 1.09 mmol) added and stirred at room temperature for 16 h. The mixture was quenched with saturated sodium bicarbonate and the organic layer separated. The aqueous layer was extracted with DCM (contains ^{~}5% MeOH) (2 x 20 mL) and the organic fractions combined, dried over sodium sulfate and concentrated under vacuum. The residue was purified by column chromatography eluting with 0 - 50 % MeOH in DCM to give the title compound (168 mg, 91% yield). LCMS [M+Na]⁺ 273, RT 0.61 minutes (Method 6).

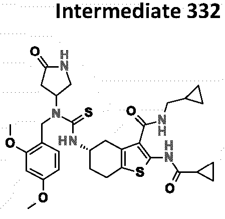

### (5S)-2-(cyclopropanecarbonylamino)-N-(cyclopropylmethyl)-5-[[(2,4-dimethoxyphenyl)methyl-(5-oxopyrrolidin-3-yl)carbamothioyl]amino]-4,5,6,7-tetrahydrobenzothiophene-3-carboxamide

The title compound was obtained following **general method** 7 with **intermediate 117** (130 mg, 0.39 mmol), triethylamine (0.16 ml, 1.17 mmol), phenyl chloromethanethioate (0.059 ml, 0.42 mmol) and **intermediate 331** (166 mg, 0.66 mmol). Crude was purified by column chromatography using 0 - 100% ethyl acetate in heptane then 0% to 10% MeOH in DCM to give the title compound (208 mg, 84%). LCMS [M+H]⁺ 626, RT 1.11 minutes (Method 6).

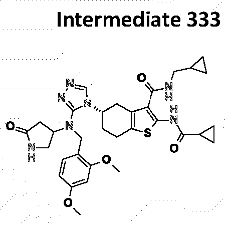

### (5S)-2-(cyclopropanecarbonylamino)-N-(cyclopropylmethyl)-5-[3-[(2,4-dimethoxyphenyl)methyl-(5-oxopyrrolidin-3-yl)amino]-1,2,4-triazol-4-yl]-4,5,6,7-tetrahydrobenzothiophene-3-carboxamide

The title compound was obtained following **general method 8** with intermediate **332** (208 mg, 0.33 mmol), formic hydrazide (59.8 mg, 0.99 mmol), mercury dichloride (270.7 mg, 0.99 mmol) and triethylamine (0.14 ml, 0.99 mmol). Reaction time 2 hours. The residue was purified by column chromatography eluting with 0 - 20 % MeOH in DCM to give the title compound (192 mg, 91% yield). LCMS [M+H]⁺ 634, RT 1.03 minutes (Method 11).

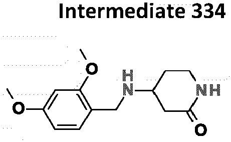

### 4-[(2,4-dimethoxyphenyl)methylamino]piperidin-2-one

To a solution of 4-aminopiperidin-2-one TFA (400 mg, 1.75 mmol) in EtOH (35 mL) were added DIPEA (0.458 ml, 2.63 mmol) and 2,4-dimethoxybenzaldehyde (291.3 mg, 1.75 mmol). Reaction mixture was heated at 70 °C for 6 h. Reaction mixture was cooled to 0 °C and NaBH₄ (94.4 mg, 2.63 mmol) was added and stirred at room temperature for 16 h. Solvent was removed water and DCM were added. The aqueous layer was extracted with DCM (2 x 20 mL) and the organic extracts combined, dried over sodium sulfate and concentrated under vacuum. The residue was purified by column chromatography eluting with 0 - 50 % MeOH in DCM to give the title compound (413 mg, 89% yield). LCMS [M+Na]⁺ 287, RT 0.71 minutes (Method 12).

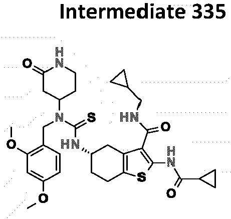

### (5S)-2-(cyclopropanecarbonylamino)-N-(cyclopropylmethyl)-5-[[(2,4-dimethoxyphenyl)methyl-(2-oxo-4-piperidyl)carbamothioyl]amino]-4,5,6,7-tetrahydrobenzothiophene-3-carboxamide

The title compound was obtained following **general method 7** with **intermediate 117** (300 mg, 0.9 mmol), triethylamine (0.37 ml, 2.69 mmol), phenyl chloromethanethioate (0.137 ml, 0.99 mmol) and **intermediate 334** (404 mg, 1.52 mmol). Crude was purified by column chromatography eluting with 0 - 100% ethyl acetate in heptane then 0% to 10% MeOH in DCM to give the title compound (481 mg, 83% Yield). LCMS [M+H]⁺ 640, RT 1.85 minutes (Method 12).

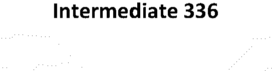

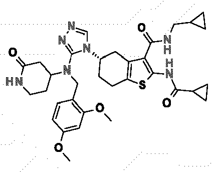

### (5S)-2-(cyclopropanecarbonylamino)-N-(cyclopropylmethyl)-5-[3-[(2,4-dimethoxyphenyl)methyl-(2-oxo-4-piperidyl)amino]-1,2,4-triazol-4-yl]-4,5,6,7-tetrahydrobenzothiophene-3-carboxamide

The title compound was obtained following **general method 8** with intermediate **335** (200 mg, 0.31 mmol), formic hydrazide (56 mg, 0.93 mmol), mercury dichloride (255 mg, 0.93 mmol) and triethylamine (0.13 ml, 0.93 mmol). Reaction time 4 hours. The residue was purified by column chromatography eluting with 0 - 20 % MeOH in DCM to give the title compound (184 mg, 88% Yield). LCMS [M+H]⁺ 648, RT 1.74 minutes (Method 12).

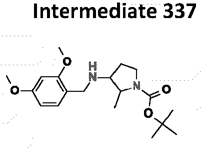

### tert-butyl 3-[(2,4-dimethoxyphenyl)methylamino]-2-methyl-pyrrolidine-1-carboxylate

The title compound was obtained following **general method 2** with tert-butyl 3-amino-2-methyl-pyrrolidine-1-carboxylate (500 mg, 2.49 mmol), 2,4-dimethoxybenzaldehyde (415 mg, 2.49 mmol) and STAB (794 mg, 3.74 mmol). Reaction time 4 h. The residue was purified by column chromatography eluting with 0 - 100 % TBME in heptane to give the title compound (832 mg, 95% Yield). LCMS [M+H]⁺ 351, RT 1.64 minutes (Method 12).

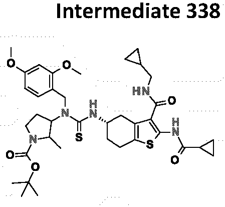

### tert-butyl 3-[[(5S)-2-(cyclopropanecarbonylamino)-3-(cyclopropylmethylcarbamoyl)-4,5,6,7-tetrahydrobenzothiophen-5-yl]carbamothioyl-[(2,4-dimethoxyphenyl)methyl]amino]-2-methyl-pyrrolidine-1-carboxylate

The title compound was obtained following **general method 7** with **intermediate 117** (550 mg, 1.64 mmol), triethylamine (0.69 ml, 3.59 mmol), phenyl chloromethanethioate (0.251 ml, 1.81 mmol) and **intermediate 337** (867.0 mg, 2.4 mmol). Crude was purified by column chromatography eluting with 0 - 100% ethyl acetate in heptane to give the title compound (804 mg, 67% Yield). LCMS [M+H]⁺ 726, RT 2.18 minutes (Method 6).

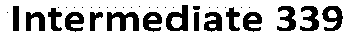

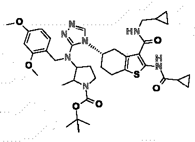

### 3-[[4-[(5S)-2-(cyclopropanecarbonylamino)-3-(cyclopropylmethylcarbamoyl)-4,5,6,7-tetrahydrobenzothiophen-5-yl]-1,2,4-triazol-3-yl]-[(2,4-dimethoxyphenyl)methyl]amino]-2-methyl-pyrrolidine-1-carboxylate

The title compound was obtained following **general method** 8 with **intermediate 338** (804 mg, 1.10 mmol), formic hydrazide (199 mg, 3.32 mmol), mercury dichloride (902 mg, 3.32 mmol) and triethylamine (0.46 mL, 3.32 mmol). Reaction time 2 hours. The residue was purified by column chromatography eluting with 0 - 20 % MeOH in DCM to give the title compound (702 mg, 84% yield). LCMS [M+H]⁺ 734, RT 2.02 minutes (Method 12).

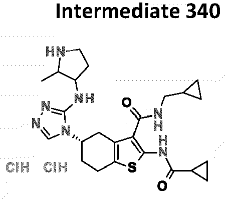

### (5S)-2-(cyclopropanecarbonylamino)-N-(cyclopropylmethyl)-5-[3-[(2-methylpyrrolidin-3-yl)amino]-1,2,4-triazol-4-yl]-4,5,6,7-tetrahydrobenzothiophene-3-carboxamide dihydrochloride

The title compound was obtained following **general method 4** with intermediate **339** (702 mg, 0.95 mmol) and 4 M HCl in dioxane (4.78 mL, 19.13 mmol). Reaction time 16 h. After removing solvent crude (690 mg, bis HCl salt) material was used in the next stage without further purification. LCMS [M+H]⁺ 484, RT 1.51 minutes (Method 12).

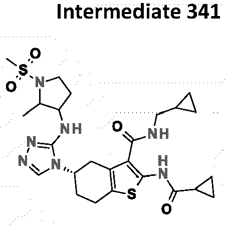

### (5S)-2-(cyclopropanecarbonylamino)-N-(cyclopropylmethyl)-5-[3-[(2-methyl-1-methylsulfonyl-pyrrolidin-3-yl)amino]-1,2,4-triazol-4-yl]-4,5,6,7-tetrahydrobenzothiophene-3-carboxamide

Suspension of **intermediate 340** (265 mg, 0.45 mmol) was stirred in DCM (20 mL) was cooled to 0 °C then DIPEA (0.23 mL, 1.35 mmol) was added followed by methanesulfonyl methanesulfonate (70.9 mg, 0.40 mmol). Reaction stirred for 2 h at 0 °C. Then warmed to room temperature. Reaction mixture was diluted with DCM (20 mL) then water (15 mL) was added and organic layer separated. The aqueous layer was extracted with DCM (2 x 10 mL) and the organic fractions combined, dried over sodium sulphate and concentrated under vacuum. The residue purified by column chromatography eluting with 0 - 100 % TBME in heptane then 0 to 30% MeOH in DCM to give the title compound (137 mg, 51% Yield). LCMS [M+H]⁺ 562, RT 2.05 minutes (Method 10).

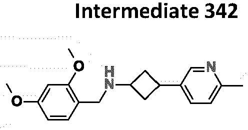

### N-[(2,4-dimethoxyphenyl)methyl]-3-(6-methyl-3-pyridyl)cyclobutanamine

To a solution of 3-(6-methyl-3-pyridyl)cyclobutanone (207 mg, 1.28 mmol) in DCM (25 mL) was added (2,4-dimethoxyphenyl)methanamine (322 mg, 1.92 mmol), followed by STAB (408 mg, 1.92 mmol). The mixture was stirred at room temperature for 16 hours. The mixture was quenched with saturated sodium bicarbonate and the organic layer separated. The aqueous layer was extracted with DCM (2 x 30 mL) and the organic fractions combined, dried over sodium sulfate and concentrated under vacuum. The residue was purified by column chromatography eluting with 0 - 10 % MeOH in DCM to give the title compound (318 mg, 75% Yield). ^{~}60:40 mixture of cis:trans or trans:cis isomers present. LCMS [M+H]⁺ 313, RT 4.49 and 4.54 minutes (Method 31).

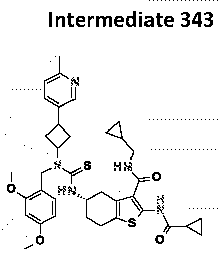

### (5S)-2-(cyclopropanecarbonylamino)-N-(cyclopropylmethyl)-5-[[(2,4-dimethoxyphenyl)methyl-[3-(6-methyl-3-pyridyl)cyclobutyl]carbamothioyl]amino]-4,5,6,7-tetrahydrobenzothiophene-3-carboxamide

The title compound was obtained following **general method** 7 with **intermediate 117** (225 mg, 0.67 mmol), triethylamine (0.28 ml, 2.02 mmol), phenyl chloromethanethioate (0.10 ml, 0.74 mmol) and intermediate **342** (316.2 mg, 1.01 mmol). Crude was purified by column chromatography eluting with 0 - 100% ethyl acetate in heptane to give the title compound (411 mg, 83% Yield). LCMS [M+H]⁺ 688, RT 1.89 minutes (Method 6).

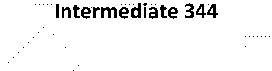

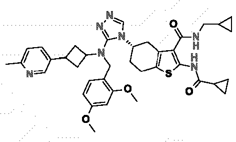

### (5S)-2-(cyclopropanecarbonylamino)-N-(cyclopropylmethyl)-5-[3-[(2,4-dimethoxyphenyl)methyl-[3-(6-methyl-3-pyridyl)cyclobutyl]amino]-1,2,4-triazol-4-yl]-4,5,6,7-tetrahydrobenzothiophene-3-carboxamide

The title compound was obtained following **general method 8** with **intermediate 343** (411 mg, 0.59 mmol), formic hydrazide (108 mg, 1.79 mmol), mercury dichloride (487 mg, 1.79 mmol) and triethylamine (0.25 mL, 1.79 mmol). Reaction time 2 hours. The residue was purified by column chromatography eluting with 0 - 20 % MeOH in DCM to give the title compound (177 mg, 43% Yield). LCMS [M+H]⁺ 696, RT 1.79 minutes (Method 12).

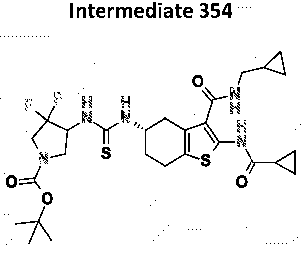

### tert-butyl 4-[[(5S)-2-(cyclopropanecarbonylamino)-3-(cyclopropylmethylcarbamoyl)-4,5,6,7-tetrahydrobenzothiophen-5-yl]carbamothioylamino]-3,3-difluoro-pyrrolidine-1-carboxylate

To a solution of sodium carbonate (510 mg, 4.72 mmol) in water (3 mL) at 0 °C was added DCM (4 mL) followed by thiocarbonyl dichloride (0.14 mL, 1.89 mmol). To this was added dropwise tert-butyl 4-amino-3,3-difluoro-pyrrolidine-1-carboxylate (210 mg, 0.94 mmol) in DCM (3 mL) over 1 minute. The mixture was vigorously stirred at 0 °C for 5 minutes then at room temperature for 40 minutes. The DCM layer was separated, dried (MgSO₄) and the solvent was removed to give the intermediate isothiocyanate as an oil. The oil was dissolved in DCM (5 mL) and **Intermediate 117** (268 mg, 0.8 mmol) in DCM (3 mL) was added. The solution was stirred for 30 minutes. The solvent was then removed to give a residue. Purification by flash column chromatography eluting with a gradient of 0 to 60% EtOAc in heptane gave the title compound (520 mg, 84% Yield). δ_{H} (250 MHz, Chloroform-d) 12.01 (s, 1H), 6.87 - 6.60 (m, 2H), 5.73 (s, 1H), 5.57 - 5.25 (m, 1H), 4.98 - 4.79 (m, 1H), 3.78 - 3.57 (m, 2H), 3.37 - 3.17 (m, 2H), 3.11 - 2.94 (m, 2H), 2.81 - 2.64 (m, 3H), 2.58 - 2.38 (m, 1H), 2.00 - 1.82 (m, 1H), 1.69 - 1.60 (m, 1H), 1.55 (s, 9H), 1.12 - 0.73 (m, 6H), 0.64 - 0.47 (m, 2H), 0.32 - 0.13 (m, 2H). LCMS [M+H]⁺ 598, RT 2.05 minutes (Method 12).

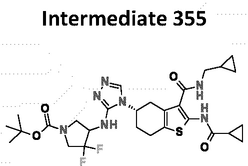

### tert-butyl 4-[[4-[(5S)-2-(cyclopropanecarbonylamino)-3-(cyclopropylmethylcarbamoyl)-4,5,6,7-tetrahydrobenzothiophen-5-yl]-1,2,4-triazol-3-yl]amino]-3,3-difluoro-pyrrolidine-1-carboxylate

To a solution of **Intermediate 354** (520 mg, 0.87 mmol) in DMF (20 mL) were added formohydrazide (157 mg, 2.61 mmol) and dichloromercury (708 mg, 2.61 mmol). The reaction was stirred for 5 minutes then N,N-diethylethanamine (364 µL, 2.61 mmol) was added and the reaction was heated at 90 °C for 2 hours. The reaction was allowed to cool to room temperature and then was diluted with DCM (25 mL) and Kieselguhr was added. The mixture stirred for 5 minutes then filtered through a plug of Kieselguhr washing through with DCM. The filtrate was concentrated under vacuum to give an orange residue. Purification by flash column chromatography eluting with a gradient of 0 to 20% MeOH in DCM gave the title compound (350 mg, 63 % Yield). LCMS [M+H]⁺ 606, RT 2.87 minutes (Method 12).

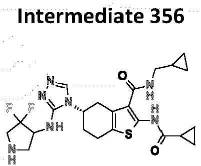

### (5S)-2-(cyclopropanecarbonylamino)-N-(cyclopropylmethyl)-5-[3-[(4,4-difluoropyrrolidin-3-yl)amino]-1,2,4-triazol-4-yl]-4,5,6,7-tetrahydrobenzothiophene-3-carboxamide

To a solution of **Intermediate 355** (297 mg, 0.45 mmol) in DCM (3 mL) was added TFA (2 mL). The solution was stirred at room temperature for 1 hour. The solvent was removed azeotroping the excess TFA with DCM/heptane (1:1, 2 x 20 mL) to give a brown oil. Purification by SCX column chromatography eluting with 0 to 100% 7 M NH₃ in MeOH gradient gave the title compound (230 mg, 93% Yield). δ_{H} (500 MHz, DMSO-d6) 11.21 (d, J = 4.1 Hz, 1H), 8.15 - 8.04 (m, 1H), 7.72 - 7.57 (m, 1H), 6.26 (d, J = 8.9 Hz, 1H), 4.42 - 4.22 (m, 2H), 3.43 - 3.35 (m, 1H), 3.23 - 2.94 (m, 5H), 2.94 - 2.78 (m, 3H), 2.74 (dd, J = 11.6, 8.5 Hz, 1H), 2.24 - 2.04 (m, 2H), 1.96 - 1.86 (m, 1H), 1.09 - 0.95 (m, 1H), 0.91 - 0.75 (m, 4H), 0.43 - 0.28 (m, 2H), 0.25 - 0.08 (m, 2H). LCMS [M+H]⁺ 506, RT 1.54 minutes (Method 12).

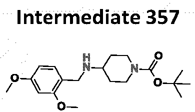

### tert-butyl 4-[(2,4-dimethoxyphenyl)methylamino]piperidine-1-carboxylate

To a solution of tert-butyl 4-aminopiperidine-1-carboxylate (300 mg, 1.5 mmol) and 2,4-dimethoxybenzaldehyde (249 mg, 1.5 mmol) in DCM (10 mL) was added sodium triacetoxyborohydride (476 mg, 2.25 mmol). The reaction was stirred at room temperature for 18 hours. Another portion of sodium triacetoxyborohydride (476 mg, 2.25 mmol) was added followed by 3 drops of AcOH. The mixture was stirred for a further 5 hours. The mixture was diluted with sat. aq. NaHCO₃ solution (10 mL) and the organic layer was separated. The aqueous layer was further extracted with DCM (2 x 10 mL) and the combined organic layers were dried (MgSO₄). The solvent was concentrated *in vacuo* to give an oil. Purification by flash column chromatography eluting with 0 to 10% 7M NH₃ MeOH in DCM gradient gave the title compound (379 mg, 72% Yield). δ_{H} (250 MHz, Chloroform-d) 7.12 (d, J = 7.9 Hz, 1H), 6.48 - 6.38 (m, 2H), 4.07 -3.92 (m, 2H), 3.80 (d, J = 3.1 Hz, 6H), 3.75 (s, 2H), 2.87 - 2.68 (m, 2H), 2.68 - 2.50 (m, 1H), 1.91 - 1.75 (m, 2H), 1.45 (s, 9H), 1.40 - 1.19 (m, 2H). LCMS [M+H]⁺ 351, RT 1.61 minutes (Method 12).

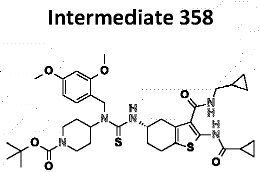

### tert-butyl 4-[[(5S)-2-(cyclopropanecarbonylamino)-3-(cyclopropylmethylcarbamoyl)-4,5,6,7-tetrahydrobenzothiophen-5-yl]carbamothioyl-[(2,4-dimethoxyphenyl)methyl]amino]piperidine-1-carboxylate

A solution of **intermediate 117** (200 mg, 0.6 mmol) and triethylamine (251 µl, 1.8 mmol) in DCM (8 mL) was added dropwise over 5 minutes to a solution of phenylchloromethanethioate (91.4 µl, 0.66 mmol) in DCM (8 mL) at 0 °C. The solution was stirred for 30 minutes at 0 °C and then **Intermediate 357** (357.34 mg, 1.02 mmol) in DCM (6 mL) was added at 0 °C. The reaction was allowed to warm to room temperature and stirred for 20 hours. The reaction was diluted with DCM (10 mL), washed with water (30 mL), saturated aq. NaHCO₃ solution (30 mL) and then dried (MgSO₄). The organic layer was concentrated *in vacuo* to give an oil. Purification by flash column chromatography eluting with 0 to 60% EtOAc in heptane gradient gave the title compound (400 mg, 92% Yield). LCMS [M+H]⁺ 726, RT 2.19 minutes (Method 12).

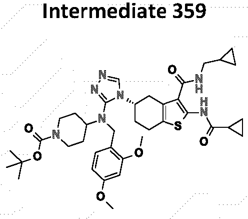

### tert-butyl 4-[[4-[(5S)-2-(cyclopropanecarbonylamino)-3-(cyclopropylmethylcarbamoyl)-4,5,6,7-tetrahydrobenzothiophen-5-yl]-1,2,4-triazol-3-yl]-[(2,4-dimethoxyphenyl)methyl]amino]piperidine-1-carboxylate

To a solution of **Intermediate 358** (400 mg, 0.55 mmol) in DMF (5 mL) was added formic hydrazide (99 mg, 1.65 mmol) and mercury dichloride (449 mg, 1.65 mmol). The mixture was stirred for 5 minutes then triethylamine (230 µl, 1.65 mmol) was added. The mixture was then heated at 90 °C for 3 hours. The reaction was allowed to cool, diluted with DCM (10 mL) and ^{~}5 g Kieselguhr was added. The mixture was stirred for 5 minutes then filtered through a plug of Kieselguhr washing the Kieselguhr with DCM (20 mL). The filtrate was concentrated under vacuum to give an orange oil. Purification by flash column chromatography eluting with 0 to 10% MeOH in DCM gradient gave the title compound (290 mg, 72% yield). δ_{H} (500 MHz, Chloroform-d) 12.09 (s, 1H), 7.99 (s, 1H), 6.78 (d, J = 8.3 Hz, 1H), 6.29 (d, J = 2.3 Hz, 1H), 6.24 (dd, J = 8.2, 2.4 Hz, 1H), 5.53 - 5.38 (m, 1H), 4.30 - 4.08 (m, 4H), 3.72 (s, 3H), 3.62 (s, 3H), 3.38 - 3.11 (m, 3H), 2.81 - 2.60 (m, 4H), 2.60 - 2.39 (m, 2H), 2.06 - 1.89 (m, 2H), 1.87 - 1.74 (m, 1H), 1.74 - 1.62 (m, 1H), 1.44 (s, 9H), 1.16 - 1.04 (m, 2H), 1.00 - 0.82 (m, 3H), 0.57 - 0.45 (m, 2H), 0.26 - 0.12 (m, 2H). LCMS [M+H]⁺ 734, RT 2.04 minutes (Method 12).

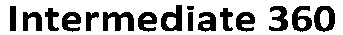

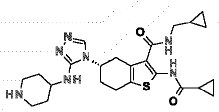

### (5S)-2-(cyclopropanecarbonylamino)-N-(cyclopropylmethyl)-5-[3-(4-piperidylamino)-1,2,4-triazol-4-yl]-4,5,6,7-tetrahydrobenzothiophene-3-carboxamide

To a solution of **Intermediate 359** (290 mg, 0.4 mmol) in DCM (3 mL) was added TFA (0.6 mL, 7.90 mmol). The solvent was removed azeotroping the excess TFA with 1:1 DCM/heptane to give an off white residue. Purification by SCX column chromatography eluting with 0 to 100% 7 M NH₃ in MeOH gradient gave the title compound (165 mg, 84% Yield). δ_{H} (500 MHz, Methanol-d4) 8.10 (s, 1H), 4.43 - 4.30 (m, 1H), 3.73 - 3.59 (m, 1H), 3.30 - 3.12 (m, 3H), 3.12 - 3.02 (m, 2H), 3.00 - 2.82 (m, 3H), 2.71 (td, J = 12.5, 2.4 Hz, 2H), 2.32 - 2.22 (m, 2H), 2.10 - 2.00 (m, 2H), 1.86 - 1.77 (m, 1H), 1.56 - 1.42 (m, 2H), 1.12 - 1.03 (m, 1H), 1.03 - 0.91 (m, 4H), 0.53 - 0.45 (m, 2H), 0.32 - 0.22 (m, 2H). LCMS [M+H]⁺ 484, RT 1.24 minutes (Method 10).

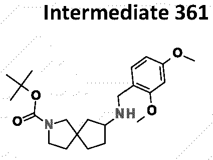

### tert-butyl 7-{[(2,4-dimethoxyphenyl)methyl]amino}-2-azaspiro[4.4]nonane-2-carboxylate

To a solution of tert-butyl 7-oxo-2-azaspiro[4.4]nonane-2-carboxylate (542 mg, 2.26 mmol, preparation as described in Tetrahedron Letters 2011, 52, 4204) in dichloromethane (8 mL) was introduced acetic acid (1.36 g, 22.6 mmol) and 2,4-dimethoxybenzylamine (757 mg, 4.53 mmol). After 30 minutes, sodium triacetoxyborohydride (4.80 g, 22.6 mmol) was introduced (portion-wise, approximately 1.1 g every 30 minutes). The reaction mixture was stirred at room temperature for 8 hours under an atmosphere of nitrogen then saturated aqueous potassium carbonate (20 mL) introduced. After 20 minutes, the two-phase mixture was diluted with dichloromethane (40 mL) and water (15 mL), and the two phases separated. The organic phase was washed with saturated aqueous potassium carbonate (20 mL) and brine (20 mL) then dried over sodium sulfate. Following filtration, the filtrate was concentrated in-vacuo and purified by flash column chromatography (dichloromethane containing 1% by volume of 7 M ammonia in methanol and a 0-6% gradient of methanol) to furnish the title compound (0.589 g, 48% Yield). LCMS [M+H]⁺ 391, RT 2.10 and 2.16 mins (diastereomers) (Method 10).

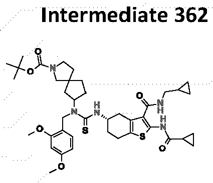

### tert-butyl 8-[[(5S)-2-(cyclopropanecarbonylamino)-3-(cyclopropylmethylcarbamoyl)-4,5,6,7-tetrahydrobenzothiophen-5-yl]carbamothioyl-[(2,4-dimethoxyphenyl)methyl]amino]-2-azaspiro[4.4]nonane-2-carboxylate

To a solution of O-phenyl chlorothioformate (286 mg, 1.66 mmol) in anhydrous dichloromethane (10 mL) at 0 °C was introduced a solution of **Intermediate 117** (503 mg, 1.51 mmol) and triethylamine (458 mg, 4.52 mmol) in anhydrous dichloromethane (5 mL). After 40 minutes, a solution of **Intermediate 361** (589 mg, 1.09 mmol) in anhydrous dichloromethane (5 mL) was introduced and the mixture warmed to room temperature for 14 hours. The reaction mixture was diluted with dichloromethane (20 mL), washed with saturated aqueous sodium bicarbonate (20 mL) and dried over sodium sulfate. Following filtration, the filtrate was concentrated in-vacuo and the residue purified by flash column chromatography (10-90% gradient of ethyl acetate in heptane) to furnish the title compound (831 mg, 64% Yield) as a mixture of diastereomers. LCMS [M+H]⁺ 766, RT 4.85 mins (Method 10).

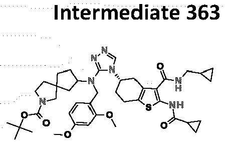

### tert-butyl 8-[[4-[(5S)-2-(cyclopropanecarbonylamino)-3-(cyclopropylmethylcarbamoyl)-4,5,6,7-tetrahydrobenzothiophen-5-yl]-1,2,4-triazol-3-yl]-[(2,4-dimethoxyphenyl)methyl]amino]-2-azaspiro[4.4] nonane-2-carboxylate

To a solution of **Intermediate 362** (831 mg, 89% purity, 0.96 mmol) in anhydrous N,N-dimethylformamide (14 mL) under an atmosphere of nitrogen was introduced formyl hydrazine (195 mg, 3.25 mmol) and mercury(II) chloride (883 mg, 3.25 mmol). After 5 minutes stirring at room temperature, triethylamine (0.45 mL, 3.25 mmol) was introduced and the reaction mixture warmed to 90 °C for 2 hours. After cooling to room temperature, the resulting suspension was diluted with dichloromethane (40 mL) and kieselguhr (2 g) introduced with vigorous stirring for 30 minutes. The suspension was then filtered and the filtrate concentrated in-vacuo. The residue was dissolved in 1:1 ethyl acetate / tetrahydrofuran (40 mL), washed with water (2 x 5 mL) and brine (5 mL), then dried over sodium sulfate. After filtration the filtrate was concentrated in-vacuo to furnish the title compound (838 mg, 99% Yield) as a mixture of diastereomers. LCMS [M+H]⁺ 774, RT 4.12-4.17 mins (part resolved diastereomers) (Method 10).

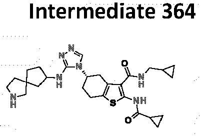

### (5S)-5-[3-({2-azaspiro[4.4]nonan-7-yl}amino)-4H-1,2,4-triazol-4-yl]-2-cyclopropaneamido-N-(cyclopropylmethyl)-4,5,6,7-tetrahydro-1-benzothiophene-3-carboxamide

To a solution of **Intermediate 363** (838 mg, 88% purity, 0.95 mmol) in 1,4-dioxane (20 mL) was introduced hydrogen chloride (2.71 mL of a 4 M solution in 1,4-dioxane, 10.83 mmol) then concentrated aqueous hydrogen chloride (1 mL). The resulting solution was stirred at room temperature for 60 minutes then re-treated with concentrated aqueous hydrogen chloride (1 mL). After 30 minutes the solution was concentrated in-vacuo to furnish an aqueous residue which was basified with saturated aqueous sodium carbonate resulting in a suspension of a black solid. Tetrahydrofuran (50 mL) was introduced to this suspension then the two-phase mixture filtered. The filtrate (two-phase) was diluted with ethyl acetate (30 mL), the organic phase separated, then washed with saturated aqueous sodium bicarbonate (2 x 5 mL) and brine (5 mL). The organic phase was concentrated in-vacuo to furnish the title compound (917 mg, quantitative) as a mixture of diastereosiomers, which was carried forward to the next synthetic step without purification. LCMS [M+H]⁺ 524, RT 2.05 mins (Method 12).

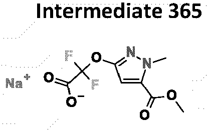

### Sodium 2,2-difluoro-2-{[5-(methoxycarbonyl)-1-methyl-1H-pyrazol-3-yl]oxy}acetate

To a solution of methyl 3-hydroxy-1-methyl-1H-pyrazole-5-carboxylate (2.00 g, 12.81 mmol) in anhydrous 1,4-dioxane (30 mL) was introduced (dropwise addition) sodium hydride (680 mg of a 50% dispersion in mineral oil suspended in 5 mL dry 1,4-dioxane) under a stream of nitrogen. The suspension was stirred for 30 minutes at room temperature under an atmosphere of nitrogen. Sodium bromodifluoroacetate (2.77 g, 14.1 mmol) was then introduced (portion-wise over 5 minutes) and the reaction mixture warmed to 60 °C for 20 hours. After a further treatment with sodium bromodifluoroacetate (1.00 g, 5.10 mmol, portion-wise addition over 5 minutes) and warming to 80 °C for 20 hours, the reaction mixture was diluted with 1,4-dioxane (30 mL) and the suspension filtered (at room temperature). The filter cake was washed with 1,4-dioxane (30 mL) and the combined filtrates concentrated in-vacuo to furnish the title compound (3.4 g, 97% Yield). δ_{H} (500 MHz, D₂O) 6.70 (s, 1H), 4.06 (s, 3H), 3.94 (s, 3H); δ_{F} (H-decoupled) - 78.71.

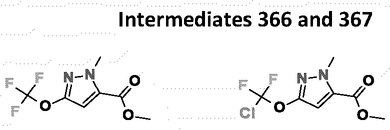

### methyl 1-methyl-3-(trifluoromethoxy)-1H-pyrazole-5-carboxylate & methyl 1-methyl-3-(chlorodifluoromethoxy)-1H-pyrazole-5-carboxylate

To a solution of **Intermediate 365** (1187 mg, 4.36 mmol) in 1,4-dioxane (15 mL) was introduced hydrogen chloride (1.5 mL of a 4 M solution in 1,4-dioxane, 6 mmol) and the solution immediately concentrated in-vacuo. The residue was re-dissolved in deuterochloroform (20 mL pre-dried over magnesium sulfate and filtered) then transferred to a PTFE bottle. Xenon difluoride (2.5 g, 14.99 mmol) was introduced in one portion and the bottle flushed with nitrogen and capped. The reaction mixture was subjected to sonication for 2 minutes, then stirred at room temperature for a further 20 minutes. After diluting with chloroform (50 mL), the solution was washed with a 2:1 mixture of saturated aqueous sodium bicarbonate and saturated aqueous calcium hydroxide (6 mL). The organic phase was dried over magnesium sulfate, filtered and concentrated in-vacuo to furnish a 67:33 mixture of the title compounds (1.56 g, 49 % total purity LCMS-UV₂₁₅, ^{~}78% total Yield). LCMS [M+H]⁺ 225, RT 3.12 mins and LCMS [M+H]⁺ 241/243, RT 3.29 mins (Method 10).

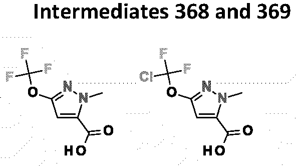

### 1-methyl-3-(trifluoromethoxy)-1H-pyrazole-5-carboxylic acid & 1-methyl-3-(chlorodifluoromethoxy)-1H-pyrazole-5-carboxylic acid

A mixture of **Intermediates 366 and 367** (1.565 g at 33% total purity LCMS-UV₂₁₅, ^{~}2.30 mmol) was dissolve in methanol (50 mL) and treated with sodium hydroxide (0.84 g, 20.95 mmol, dissolved in 25 mL of water). After 12 hours at room temperature, the pH of the reaction mixture was adjusted to 7 with 6 M aqueous hydrogen chloride and the reaction mixture concentrated in-vacuo to remove methanol. The residual aqueous solution was acidified to pH 3 (with 2 M aqueous hydrogen chloride) and extracted with ethyl acetate (3 x 50 mL). After saturating the aqueous phase with sodium chloride, the brine solution was extracted with 1:1 tetrahydrofuran/ethyl acetate (2 x 50 mL extractions). The combined organic extracts were concentrated in-vacuo, re-dissolved in ethyl acetate and dried over magnesium sulfate. Following filtration, the filtrate was concentrated in-vacuo to furnish a 71:29 mixture of the title compounds (1.113 g at 63% total purity LCMS-UV₂₁₅, quantitative). LCMS [M-H]⁻ 209, RT 2.38 mins and LCMS [M-H]⁻ 225/227, RT 2.61 mins (Method 10).

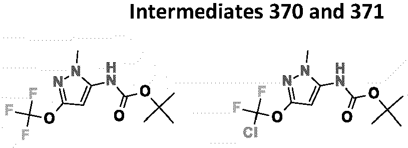

### tert-butyl N-[1-methyl-3-(trifluoromethoxy)-1H-pyrazol-5-yl]carbamate (370) & tert-butyl N-[1-methyl-3-(chlorodifluoromethoxy)-1H-pyrazol-5-yl]carbamate (371)

A mixture of **Intermediates** 368 and 369 (1.113 g at 45% purity LCMS-UV₂₁₅, ^{~}2.38 mmol) was dissolved in tert-butanol (25 mL) and treated with diisopropylethylamine (0.95 mL, 5.83 mmol) and diphenylphosphoryl azide (4.37 g, 15.89 mmol). The reaction mixture was warmed to 85 °C under an atmosphere of nitrogen for 5 hours. After the reaction mixture was concentrated in-vacuo, the residue was purified by flash column chromatography (5-40% gradient of ethyl acetate in heptane) to furnish a 2:1 mixture of the title compounds **370** and **371** respectively (0.552 g, ^{~}78% Yield).

**Intermediate 370:** δ_{H} (400 MHz, d-chloroform) 6.41 (s, 1H), 5.96 (s, 1H), 3.67 (s, 3H), 1.51 (s, 9H); δ_{F} (H-decoupled, 376 MHz, d-chloroform) -59.2; LCMS [M+H]⁺ 282, RT 3.26 mins (Method 10).

**Intermediate 371:** δ_{H} (400 MHz, d-chloroform) 6.41 (s, 1H), 6.01 (s, 1H), 3.68 (s, 3H), 1.51 (s, 9H); δ_{F} (H-decoupled, 376 MHz, d-chloroform) -27.6; LCMS [M+H]⁺ 298/300, RT 3.39 mins (Method 10).

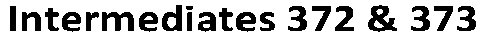

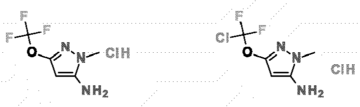

### 1-methyl-3-(trifluoromethoxy)-1H-pyrazol-5-amine hydrochloride (372) & 1-methyl-3-(chlorodifluoromethoxy)-1H-pyrazol-5-amine hydrochloride (373)

To solution of hydrogen chloride (5.4 mL of a 4 M solution in 1,4-dioxane) at 0 °C a solution of **intermediates 370** & **371** (2:1 respectively, 0.552 g, ^{~}1.86 mmol) in 1,4-dioxane (2 mL) was added drop-wise over 5 minutes. The reaction mixture was warmed to room temperature for 4 hours, then concentrated in-vacuo to furnish a 2:1 mixture of the title compounds **372** and **373** respectively (0.318 g, ^{~}72% Yield ).

**Intermediates 372:** δ_{H} (500 MHz, d₆-dmso) 5.10 (q, J = 1.0 Hz, 1H), 3.443 (s, 3H) ; δ_{F} (376 MHz, d6-dmso) -57.77; LCMS [M+H]⁺ 181, RT 0.92 mins (Method 17).

**Intermediates 373:** δ_{H} (500 MHz, d₆-dmso) 5.12 (t, J = 1.0 Hz, 1H), 3.449 (s, 3H); δ_{F} (376 MHz, d6-dmso) -25.98; LCMS [M+H]⁺ 198/200, RT 1.32 mins (Method 17).

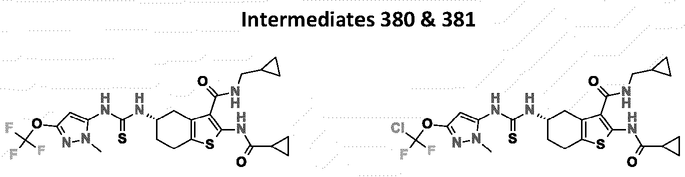

### (5S)-2-(cyclopropanecarbonylamino)-N-(cyclopropylmethyl)-5-[[2-methyl-5-(trifluoromethoxy)pyrazol-3-yl]carbamothioylamino]-4,5,6,7-tetrahydrobenzothiophene-3-carboxamide (380) &

### (5S)-5-[[5-[chloro(difluoro)methoxy]-2-methyl-pyrazol-3-yl]carbamothioylamino]-2-(cyclopropanecarbonylamino)-N-(cyclopropylmethyl)-4,5,6,7-tetrahydrobenzothiophene-3-carboxamide (381)

A 2:1 mixture of **Intermediates 372** and **373** respectively (100 mg, 0.42 mmol) was dissolved in anhydrous dichloromethane (2.5 mL) and treated with diisopropylethylamine (0.16 mL, 0.96 mmol). After 10 minutes at room temperature, thiocarbonyldiimidazole (86 mg, 0.48 mmol) was added and the solution stirred under an atmosphere of nitrogen for 4 hours. This solution was introduced (dropwise) to a solution of **intermediate 117** (122 mg, 0.37 mmol) in dry dichloromethane (2 mL) and the reaction stirred under a nitrogen atmosphere for 7 hours. After adsorbing the reaction mixture onto silica in-vacuo, the dry-loaded material was purified by flash column chromatography (10-100% gradient of ethyl acetate in heptane). The title compounds were isolated as a 2:1 mixture of **380** and **381** respectively (105 mg, 38% Yield). LCMS [M+H]⁺ 557, RT 3.65 and LCMS [M+H]⁺ 573/575, RT 3.74 mins (Method 32).

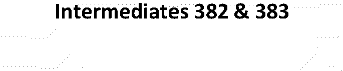

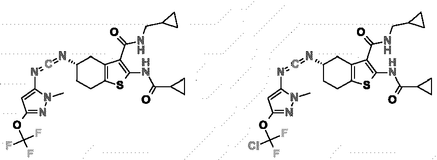

### (5S)-2-(cyclopropanecarbonylamino)-N-(cyclopropylmethyl)-5-[[2-methyl-5-(trifluoromethoxy)pyrazol-3-yl]iminomethyleneamino]-4,5,6,7-tetrahydrobenzothiophene-3-carboxamide (382) &

### (5S)-5-[[5-[chloro(difluoro)methoxy]-2-methyl-pyrazol-3-yl]iminomethyleneamino]-2-(cyclopropanecarbonylamino)-N-(cyclopropylmethyl)-4,5,6,7-tetrahydrobenzothiophene-3-carboxamide (383)

A 2:1 mixture of **380** and **381** respectively (105 mg, 0.16 mmol) was dissolved in anhydrous dichloromethane (3 mL) and cooled to 0 °C. Trimethylamine (0.16 mL, 1.14 mmol) was then added followed by dropwise addition of a methanesulfonyl chloride (39 mg dissolved in 0.2 mL dry dichloromethane, 0.33 mmol). After warming to room temperature for 60 minutes, the reaction mixture was diluted with dichloromethane (25 mL) and washed with saturated aqueous ammonium chloride (2 x 5 mL). The organic extract was dried over sodium sulfate, filtered and concentrated in-vacuo to furnish a mixture of the title compounds (121 mg, quantitative) which was used immediately in the next synthetic step without further purification. LCMS [M+H]⁺ 523 and 539/541, RT 2.09 mins (identical retention times) (Method 12).

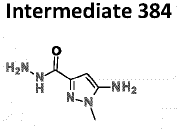

### 5-amino-1-methyl-1H-pyrazole-3-carbohydrazide

To a solution of methyl 5-amino-1-methylpyrrazole-2-carboxylate (1 g, 6.44 mmol) in methanol (10 mL) was introduced hydrazine hydrate (1.6 g, 32.2 mmol) and the reaction warmed to 80 °C for 16 hours. After concentration in-vacuo, the residue was dissolved in 1-butanol (5 mL) and concentrated in-vacuo (twice) to furnish the title compound (1.231 g, quantitative) which was used in the next synthetic step without purification. δ_{H} (500 MHz, d₄-methanol) 5.86 (1H, s), 4.83 (2H, s), 3.63 (3H, s); LCMS [M+H]⁺ 156, RT 0.40 mins (Method 18).

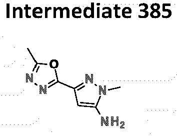

### 1-methyl-3-(5-methyl-1,3,4-oxadiazol-2-yl)-1H-pyrazol-5-amine

To a solution of **Intermediate 384** (1.231 g, 6.44 mmol) in 1-butanol (40 mL) was introduced trimethyl orthoacetate (15.5 g, 128.9 mmol). The reaction mixture was warmed to 145 °C for 10 minutes, cooled to room temperature and DBU (981 mg, 6.44 mmol) introduced. After warming the reaction mixture to 145 °C for 70 minutes, the reaction mixture was concentrated in-vacuo. The residue was re-dissolved in acetonitrile (20 mL) and the pH adjusted to 1 (dropwise addition of aqueous 1 M HCl). The solution was then diluted with water (15 mL) and the acetonitrile evaporated in-vacuo. After adjusting the pH of the aqueous solution to 7 (dropwise addition of saturated aqueous sodium bicarbonate) the solution was subjected to freeze drying to furnish the crude product containing sodium chloride residues. The crude product was dissolved in water (5 mL) then applied onto a plug of reverse phase silica and eluted with a gradient of methanol (0-50 %) in water. Product fractions were combined, concentrated in-vacuo and the resulting aqueous solution freeze-dried to furnish the title compound (0.384 g, 33% Yield): δ_{H} (500 MHz, d₆-dmso) 5.79 (s, 1H), 5.53 (s, 2H), 3.61 (s, 3H), 2.51 (s, 3H); LCMS [M+H]⁺ 180, RT 1.01 mins (Method 18).

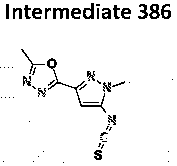

### 2-(5-isothiocyanato-1-methyl-1H-pyrazol-3-yl)-5-methyl-1,3,4-oxadiazole

To a vigorously stirred solution of **Intermediate 385** (571 mg, 2.23 mmol) in water (8 mL) was introduced thiophosgene (256 mg, dropwise addition). After 5 minutes, the precipitated solid was collected by filtration and partially dried at the filter to furnish the (damp) title compound which was used immediately in the next synthetic step without further purification. LCMS [M+H]⁺ 222, RT 1.59 mins (Method 12).

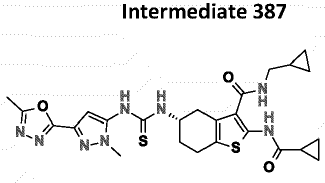

### (5S)-2-(cyclopropanecarbonylamino)-N-(cyclopropylmethyl)-5-[[2-methyl-5-(5-methyl-1,3,4-oxadiazol-2-yl)pyrazol-3-yl]carbamothioylamino]-4,5,6,7-tetrahydrobenzothiophene-3-carboxamide

To a solution of **intermediate 117** (366 mg, 1.10 mmol) in acetonitrile (5.0 mL) was introduced **Intermediate 386** (243 mg [damp], <1.10 mmol, as a solution in minimum volume of acetonitrile required to solubilise). After stirring the reaction mixture for 90 minutes at room temperature, a second portion of **Intermediate 386** (150 mg [damp], <0.68 mmol) was introduced and the reaction continued for 16 hours at room temperature. The reaction mixture was adsorbed onto silica in-vacuo, and the dry-loaded material purified by flash column chromatography (0-100% gradient of ethyl acetate in heptane) to furnish the title compound (281 mg, 41% Yield) δ_{H} (500 MHz, d₆-dmso) 11.08 (s, 1H), 9.28 (s, 1H), 8.36 - 8.24 (m, 1H), 7.74 (t, J = 5.5 Hz, 1H), 6.81 (s, 1H), 4.54 (s, 1H), 3.74 (s, 3H), 3.19 - 3.04 (m, 3H), 2.74 (br. s, 2H), 2.67-2.55 (m, 1H), 2.55 (s, 3H), 2.07 - 2.00 (m, 1H), 1.96 - 1.87 (m, 2H), 1.08 - 0.98 (m, 1H), 0.89 - 0.80 (m, 4H), 0.46 - 0.40 (m, 2H), 0.23 (q, J = 4.8 Hz, 2H); LCMS [M+H]⁺ 555, RT 2.65 mins (Method 29).

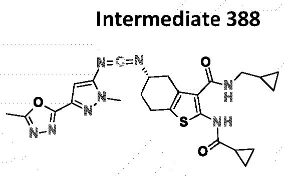

### (5S)-2-(cyclopropanecarbonylamino)-N-(cyclopropylmethyl)-5-[[2-methyl-5-(5-methyl-1,3,4-oxadiazol-2-yl)pyrazol-3-yl]iminomethyleneamino]-4,5,6,7-tetrahydrobenzothiophene-3-carboxamide

A solution of **Intermediate 387** (256 mg, 0.41 mmol) in anhydrous dichloromethane (20 mL) under an atmosphere of nitrogen was cooled to 10 °C. A solution of triethylamine (280 mg, 2.77 mmol) in anhydrous dichloromethane (1 mL) and a solution of methanesulfonyl chloride (106 mg, 0.92 mmol) in anhydrous dichloromethane (1 mL) were then added and the reaction stirred at this temperature for 30 minutes. The reaction mixture was diluted with dichloromethane (40 mL), washed with saturated aqueous ammonium chloride (5 mL) and brine (5 mL), then dried over sodium sulfate. After filtration, the filtrate was concentrated in-vacuo to furnish the crude title compound (344 mg, quantitative) which was used immediately in the next synthetic step. LCMS [M+H]⁺ 521, RT 1.86 mins (Method 12)

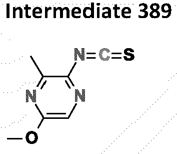

### 2-isothiocyanato-5-methoxy-3-methyl-pyrazine

1,1'-Thiocarbonyldiimidazole (226 mg, 1.21 mmol) and then hydrochloric acid in dioxane (0.55 mL, 2.2 mmol, 4 mol/L) were added to a solution of 5-methoxy-3-methyl-pyrazin-2-amine (153 mg, 1.10 mmol) in DCM (5.5 mL) under air. The reaction was stirred at r.t. for 4 hours, filtered and the solid washed with DCM (2 x 10 mL). The filtrate was concentrated in vacuo and purified by flash column chromatography on silica (gradient elution with 0% to 100% EtOAc in isohexanes) to give the title compound (127 mg, 40 % Yield). A few drops of isobutylamine were added to the LCMS sample of the product before analysis LCMS [M+H]⁺ 255, RT 1.46 minutes [M = 1-isobutyl-3-(5-methoxy-3-methyl-pyrazin-2-yl)thiourea] (Method 4).

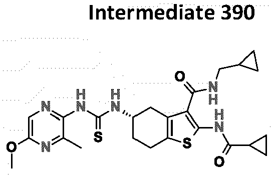

### (5S)-2-(cyclopropanecarbonylamino)-N-(cyclopropylmethyl)-5-[(5-methoxy-3-methyl-pyrazin-2-yl)carbamothioylaminol-4,5,6,7-tetrahydrobenzothiophene-3-carboxamide

**Intermediate 117** (200 mg, 0.600 mmol) was added to a solution of **intermediate 389** (127 mg, 0.702 mmol) dissolved in DCM (3.0 mL) under air. The reaction was stirred at r.t. for 4.5 hours, concentrated in vacuo and purified by flash column chromatography on silica (gradient elution with 0% to 100% EtOAc in isohexanes then 0 % to 20 % MeOH in EtOAc) to give the title compound (180 mg, 58 % Yield). LCMS [M+H]⁺ 515, RT 1.56 minutes (Method 4).

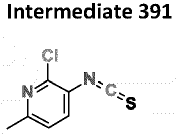

### 2-chloro-3-isothiocyanato-6-methyl-pyridine

1,1'-Thiocarbonyldiimidazole (426 mg, 2.27 mmol, 95 mass%) and then hydrochloric acid in dioxane (1.0 mL, 4.0 mmol, 4 mol/L) were added to a solution of 3-amino-2-chloro-6-picoline (300 mg, 2.06 mmol, 98 mass%) in DCM (10.3 mL) under air. The reaction was stirred at r.t. for 20 hours, filtered and the solid washed with DCM (2 x 15 mL). The filtrate was concentrated in vacuo and purified by flash column chromatography on silica (gradient elution with 0 % to 100 % EtOAc in isohexanes) to give the title compound (343 mg, 90 % Yield). A few drops of isobutylamine were added to the LCMS sample of the product before analysis, LCMS [M+H]⁺ 258, RT 0.82 minutes [M = 1-(2-chloro-6-methyl-3-pyridyl)-3-isobutyl-thiourea] (Method 7).

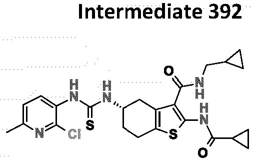

### (5S)-5-[(2-chloro-6-methyl-3-pyridyl)carbamothioylamino]-2-(cyclopropanecarbonylamino)-N-(cyclopropylmethyl)-4,5,6,7-tetrahydrobenzothiophene-3-carboxamide

**Intermediate 117** (500 mg, 1.50 mmol) was added to a solution of **intermediate 391** (332 mg, 1.80 mmol) dissolved in DCM (15 mL) under air. The reaction was stirred at r.t. for 3.5 hours, concentrated in vacuo and purified by flash column chromatography on silica (gradient elution with 40% to 100% EtOAc in isohexanes) to give the title compound (255 mg, 33 % Yield). δ_{H} (400 MHz, DMSO-d6) 11.12 (s, 1H), 9.13 (s, 1H), 8.29 (d, J = 7.6 Hz, 1H), 8.11 (d, J = 8.1 Hz, 1H), 7.75 (t, J = 5.7 Hz, 1H), 7.26 (d, J = 8.1 Hz, 1H), 4.65 - 4.52 (m, 1H), 3.17 - 3.07 (m, 3H), 2.81 - 2.71 (m, 2H), 2.70 - 2.58 (m, 1H), 2.43 (s, 3H), 2.09 - 2.01 (m, 1H), 1.97 - 1.85 (m, 2H), 1.10 - 0.97 (m, 1H), 0.88 - 0.81 (m, 4H), 0.50 - 0.38 (m, 2H), 0.29 - 0.18 (m, 2H). Product reacts in the LCMS to generate an 5-methylthiazolo[5,4-b]pyridin-2-amine instead of the thiourea, LCMS [M+H]⁺ 482, RT 1.47 minutes (Method 4).

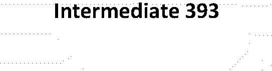

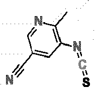

### 5-isothiocyanato-6-methyl-pyridine-3-carbonitrile

1,1'-Thiocarbonyldiimidazole (368 mg, 1.96 mmol, 95 mass%) and then hydrochloric acid in dioxane (0.89 mL, 3.6 mmol, 4 mol/L) were added to a solution of 5-amino-6-methylnicotinonitrile (250 mg, 1.78 mmol, 95 mass%) in DCM (8.9 mL) under air. The reaction was stirred at r.t. for 17.5 hours and then at 40 °C for a further 6.5 hours. The reaction was concentrated in vacuo and purified by flash column chromatography on silica (gradient elution with 0% to 100% EtOAc in isohexanes) to give the title compound (92 mg, 29 % Yield). LCMS [M+H]⁺ 176, RT 0.87 minutes (Method 7).

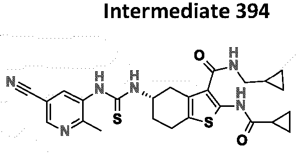

### (5S)-5-[(5-cyano-2-methyl-3-pyridyl)carbamothioylamino]-2-(cyclopropanecarbonylamino)-N-(cyclopropylmethyl)-4,5,6,7-tetrahydrobenzothiophene-3-carboxamide

**Intermediate 117** (167 mg, 0.501 mmol) was added to a solution of **intermediate 393** (92 mg, 0.52 mmol) dissolved in DCM (5.0 mL) under air. The reaction was stirred at r.t. for 5 hours, concentrated in vacuo and purified by flash column chromatography on silica (gradient elution with 0% to 100% EtOAc in isohexanes) to give the title compound (254 mg, 99 % Yield). LCMS [M+H]⁺ 509, RT 0.99 minutes (Method 7).

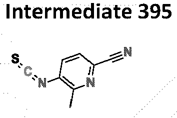

### 5-isothiocyanato-6-methyl-pyridine-2-carbonitrile

1,1'-Thiocarbonyldiimidazole (368 mg, 1.96 mmol, 95 mass%) and then hydrochloric acid in dioxane (0.89 mL, 3.6 mmol, 4 mol/L) were added to a solution of 5-amino-6-methylpicolinonitrile (250 mg, 1.78 mmol, 95 mass%) in DCM (8.9 mL) under air. The reaction was stirred at r.t. for 24.5 hours before being concentrated in vacuo and purified by flash column chromatography on silica (gradient elution with 0% to 100% EtOAc in isohexanes) to give the title compound (176 mg, 56 % Yield). LCMS [M+H]⁺ 176, RT 0.93 minutes (Method 7).

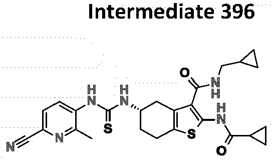

### (5S)-5-[(6-cyano-2-methyl-3-pyridyl)carbamothioylamino]-2-(cyclopropanecarbonylamino)-N-(cyclopropylmethyl)-4,5,6,7-tetrahydrobenzothiophene-3-carboxamide

**Intermediate 117** (256 mg, 0.795 mmol) was added to a solution of **intermediate 395** (176 mg, 1.01 mmol) dissolved in DCM (8.0 mL) under air. The reaction was stirred at r.t. for 5 hours, concentrated in vacuo and purified by flash column chromatography on silica (gradient elution with 0% to 100% EtOAc in isohexanes) to give the title compound (421 mg, quantitative yield). LCMS [M+H]⁺ 509, RT 1.05 minutes (Method 7).

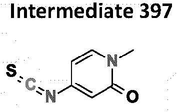

### 4-isothiocyanato-1-methyl-pyridin-2-one

1,1'-Thiocarbonyldiimidazole (516 mg, 2.75 mmol, 95 mass%) was added to a solution of 4-amino-1-methylpyridin-2(1H)-one (327 mg, 2.50 mmol, 95 mass%) in DCM (7.5 mL) under air. The reaction was stirred at r.t. for 24.5 hours and then at 40 °C for a further 2 hours. The reaction was cooled to r.t., concentrated in vacuo and purified by flash column chromatography on silica (gradient elution with 0% to 100% EtOAc in isohexanes) to give the title compound (210 mg, 51 % Yield). A few drops of isobutylamine were added to the LCMS sample of the product before analysis, LCMS [M+H]⁺ 240, RT 1.01 minutes [M = 1-isobutyl-3-(1-methyl-2-oxo-4-pyridyl)thiourea] (Method 4).

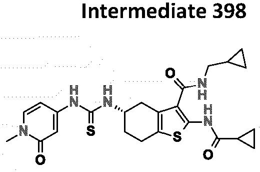

### (5S)-2-(cyclopropanecarbonylamino)-N-(cyclopropylmethyl)-5-[(1-methyl-2-oxo-4-pyridyl)carbamothioylaminol-4,5,6,7-tetrahydrobenzothiophene-3-carboxamide

**Intermediate 117** (278 mg, 0.834 mmol) was added to a solution of **Intermediate 397** (152 mg, 0.917 mmol) dissolved in DCM (4.2 mL) under air. The reaction was stirred at r.t. for 4 hours, diluted with DCM (20 mL) and filtered and washed with further DCM. The solid was product. The filtrate was concentrated in vacuo and purified by flash column chromatography on silica (gradient elution with 50% to 100% EtOAc in isohexanes then 0% to 20% MeOH in EtOAc) to give more product. Both batches of product were combined to give the title compound (320 mg, 77 % Yield). LCMS [M+H]⁺ 500, RT 1.30 minutes (Method 4).

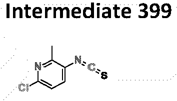

### 6-chloro-3-isothiocyanato-2-methyl-pyridine

1,1'-Thiocarbonyldiimidazole (516 mg, 2.75 mmol, 95 mass%) and then hydrochloric acid in dioxane (1.25 mL, 5.00 mmol, 4 mol/L) were added to a solution of 3-amino-6-chloro-2-picoline (368 mg, 2.50 mmol, 97 mass%) in DCM (7.5 mL) under air. The reaction was stirred at r.t. for 5 hours, concentrated in vacuo and purified by flash column chromatography on silica (gradient elution with 0% to 50% EtOAc in isohexanes) to give the title compound (326 mg, 71 % Yield). A few drops of isobutylamine were added to the LCMS sample of the product before analysis, LCMS [M+H]⁺ 258, RT 1.24 minutes [M = 1-(6-chloro-2-methyl-3-pyridyl)-3-isobutyl-thiourea] (Method 4).

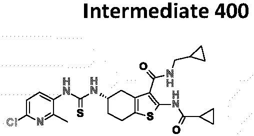

### (5S)-5-[(6-chloro-2-methyl-3-pyridyl)carbamothioylamino]-2-(cyclopropanecarbonylamino)-N-(cyclopropylmethyl)-4,5,6,7-tetrahydrobenzothiophene-3-carboxamide

**Intermediate 117** (284 mg, 0.852 mmol) was added to a solution of **intermediate 399** (326 mg, 1.77 mmol) dissolved in DCM (4.3 mL) under air. The reaction was stirred at r.t. for 1.5 hours, concentrated in vacuo and purified by flash column chromatography on silica (gradient elution with 0% to 100% EtOAc in isohexanes) to give the title compound (459 mg, quantitative yield). LCMS [M+H]⁺ 518, RT 1.45 minutes (Method 4).

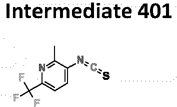

### 3-isothiocyanato-2-methyl-6-(trifluoromethyl)pyridine

1,1'-Thiocarbonyldiimidazole (516 mg, 2.75 mmol, 95 mass%) and then hydrochloric acid in dioxane (1.25 mL, 5.00 mmol, 4 mol/L) were added to a solution of 2-methyl-6-(trifluoromethyl)pyridin-3-amine (464 mg, 2.50 mmol, 95 mass%) in DCM (7.5 mL) under air. The reaction was stirred at r.t. for 4 hours, concentrated in vacuo and purified by flash column chromatography on silica (gradient elution with 0% to 50% EtOAc in isohexanes) to give the title compound (360 mg, 66 % Yield). A few drops of isobutylamine were added to the LCMS sample of the product before analysis, LCMS [M+H]⁺ 292, RT 1.39 minutes [M = 1-isobutyl-3-[2-methyl-6-(trifluoromethyl)-3-pyridyl]thiourea] (Method 4).

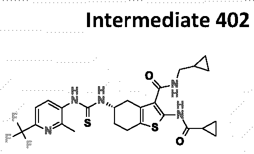

### (5S)-2-(cyclopropanecarbonylamino)-N-(cyclopropylmethyl)-5-[[2-methyl-6-(trifluoromethyl)-3-pyridyl]carbamothioylamino]-4,5,6,7-tetrahydrobenzothiophene-3-carboxamide

**Intermediate 117** (200 mg, 0.600 mmol) was added to a solution of **intermediate 401** (144 mg, 0.660 mmol) dissolved in DCM (3.0 mL) under air. The reaction was stirred at r.t. for 1.5 hours, concentrated in vacuo and purified by flash column chromatography on silica (gradient elution with 0% to 100 % EtOAc in isohexanes) to give the title compound (368 mg, quantitative). LCMS [M+H]⁺ 552, RT 1.55 minutes (Method 4).

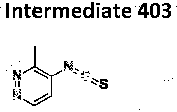

### 4-isothiocyanato-3-methyl-pyridazine

DIPEA (0.57 mL, 3.3 mmol) followed by 1,1'-thiocarbonyldiimidazole (516 mg, 2.75 mmol, 95 mass%) were added to a solution of 3-methylpyridazin-4-amine hydrochloride (383 mg, 2.50 mmol, 95 mass%) in DCM (7.5 mL) under air. The reaction was stirred at r.t. for 24 hours, concentrated in vacuo and purified by flash column chromatography on silica (gradient elution with 0 % to 50 % EtOAc in isohexanes) to give the title compound (48 mg, 13 % Yield). A few drops of isobutylamine were added to the LCMS sample of the product before analysis, LCMS [M+H]⁺ 225, RT 1.07 minutes [M = 1-isobutyl-3-(3-methylpyridazin-4-yl)thiourea] (Method 4).

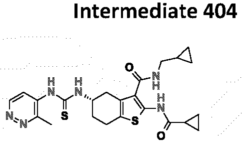

### (5S)-2-(cyclopropanecarbonylamino)-N-(cyclopropylmethyl)-5-[(3-methylpyridazin-4-yl)carbamothioylamino]-4,5,6,7-tetrahydrobenzothiophene-3-carboxamide

**Intermediate 117** (105 mg, 0.315 mmol) was added to a solution of **intermediate 403** (48 mg, 0.32 mmol) dissolved in DCM (1.6 mL) under air. The reaction was stirred at r.t. for 1 hour, concentrated in vacuo and purified by flash column chromatography on silica (gradient elution with 0 % to 100 % EtOAc in isohexanes then 0 % to 20 % MeOH in EtOAc) to give the title compound (152 mg, quantitative). LCMS [M+H]⁺ 485, RT 0.92 minutes (Method 7).

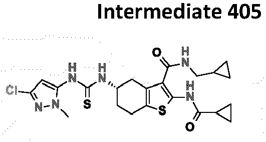

### (5S)-5-[(5-chloro-2-methyl-pyrazol-3-yl)carbamothioylamino]-2-(cyclopropanecarbonylamino)-N-(cyclopropylmethyl)-4,5,6,7-tetrahydrobenzothiophene-3-carboxamide

DIPEA (0.42 mL, 2.4 mmol) followed by 1,1'-thiocarbonyldiimidazole (207 mg, 1.10 mmol, 95 mass%) were added to a solution of 3-chloro-1-methyl-1H-pyrazol-5-amine dihydrochloride (232 mg, 1.10 mmol, 97 mass%) in DCM (3.3 mL) under nitrogen. The reaction was stirred at r.t for 2.5 hours. DIPEA (0.21 mL, 1.2 mmol) followed by **intermediate 117** (205 mg, 0.615 mmol) were then added and the reaction was stirred at r.t. for a further 24 hours before being concentrated in vacuo and purified by flash column chromatography on silica (gradient elution with 0% to 100% EtOAc in isohexanes) to give the title compound (258 mg, 83 % Yield). LCMS [M+H]⁺ 507, RT 1.07 minutes (Method 7).

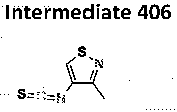

### 4-isothiocyanato-3-methyl-isothiazole

DIPEA (0.57 mL, 3.3 mmol) followed by 1,1'-thiocarbonyldiimidazole (516 mg, 2.75 mmol, 95 mass%) were added to a solution of 3-methylisothiazol-4-amine hydrochloride (396 mg, 2.50 mmol, 95 mass%) in DCM (7.5 mL) under air. The reaction was stirred at r.t. for 22 hours, concentrated in vacuo and purified by flash column chromatography on silica (gradient elution with 0 % to 50 % EtOAc in isohexanes) to give the title compound (292 mg, 75 % Yield). A few drops of isobutylamine were added to the LCMS sample of the product before analysis, LCMS [M+H]⁺ 230, RT 1.04 minutes [M = 1-isobutyl-3-(3-methylisothiazol-4-yl)thiourea] (Method 4).

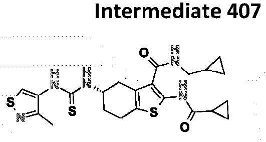

### (5S)-2-(cyclopropanecarbonylamino)-N-(cyclopropylmethyl)-5-[(3-methylisothiazol-4-yl)carbamothioylamino]-4,5,6,7-tetrahydrobenzothiophene-3-carboxamide

**Intermediate 117** (300 mg, 0.900 mmol) was added to a solution of **intermediate 406** (155 mg, 0.990 mmol) dissolved in DCM (4.5 mL) under air. The reaction was stirred at r.t. for 3.5 hours, concentrated in vacuo and purified by flash column chromatography on silica (gradient elution with 0% to 100% EtOAc in isohexanes) to give the title compound (393 mg, 89 % Yield). LCMS [M+H]⁺490, RT 1.37 minutes (Method 4).

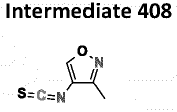

### 4-isothiocyanato-3-methyl-isoxazole

1,1'-Thiocarbonyldiimidazole (516 mg, 2.75 mmol, 95 mass%) and then hydrochloric acid in dioxane (1.25 mL, 5.00 mmol, 4 mol/L) were added to a solution of 3-methylisoxazol-4-amine (258 mg, 2.50 mmol, 95 mass%) in DCM (7.5 mL) under air. The reaction was stirred at r.t. for 3 hours 15 mins, concentrated in vacuo and purified by flash column chromatography on silica (gradient elution with 0% to 50% EtOAc in isohexanes) to give the title compound (250 mg, 71 % Yield). A few drops of isobutylamine were added to the LCMS sample of the product before analysis, LCMS [M+H]⁺ 214, RT 1.13 minutes [M = 1-isobutyl-3-(3-methylisoxazol-4-yl)thiourea] (Method 4).

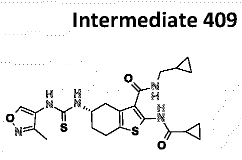

### (5S)-2-(cyclopropanecarbonylamino)-N-(cyclopropylmethyl)-5-[(3-methylisoxazol-4-yl)carbamothioylamino]-4,5,6,7-tetrahydrobenzothiophene-3-carboxamide

DIPEA (0.17 mL, 0.98 mmol) followed by **Intermediate 408** (112 mg, 0.799 mmol) were added to a solution of **Intermediate 117** (205 mg, 0.615 mmol) in DCM (3.8 mL) under air. The reaction was stirred at r.t. for 18.5 hours, concentrated in vacuo and purified by flash column chromatography on silica (gradient elution with 0% to 100% EtOAc in isohexanes) to give the title compound (313 mg, quantitative yield). LCMS [M+H]⁺474, RT 1.41 minutes (Method 4).

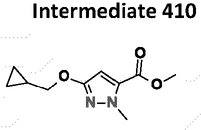

### methyl 5-(cyclopropylmethoxy)-2-methyl-pyrazole-3-carboxylate

To a stirred suspension of methyl 5-hydroxy-2-methyl-pyrazole-3-carboxylate (3.00 g, 19.0 mmol) and K₂CO₃ (2.90 g, 21.0 mmol) in DMF (19 mL) at room temperature was added cyclopropylmethyl bromide (2.0 mL, 21.0 mmol) and the reaction mixture stirred at 50 °C for 16 h. The reaction mixture was diluted with water (190 mL) and the aqueous layer extracted with ethyl acetate (3 x 100 mL). The combined organic layers were washed with water (2 x 100 mL), passed through a phase separator, and concentrated in vacuo. The crude material was purified by flash chromatography using a gradient of 0-50% EtOAc in isohexanes to afford the title compound (3.14 g, 78% Yield) as a colourless oil. δ_{H} (300 MHz, d-Chloroform) 6.18 (s, 1H), 4.02 (s, 3H), 3.94 (d, J = 7.1 Hz, 2H), 3.85 (s, 3H), 1.33-1.20 (m, 1H), 0.64-0.57 (m, 2H), 0.36-0.31 (m, 2H).

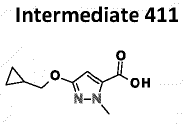

### 5-(cyclopropylmethoxy)-2-methyl-pyrazole-3-carboxylic acid

To a solution of **intermediate 410** (3.14 g, 14.9 mmol) in MeOH (75 mL) was added NaOH (aq. 2.0 M, 15 mL) and the reaction mixture stirred at 50 °C for 1 h. The reaction mixture was concentrated in vacuo, and then acidified to pH 1 with HCl (aq. 2M). The mixture was diluted with water (200 mL) and the aqueous layer extracted with EtOAc (3 x 150 mL). The combined organic extracts were passed through a phase separator, and concentrated in vacuo to give the title compound (2.62 g, 89% Yield). δ_{H} (300 MHz, d-DMSO) 13.34 (s, 1H), 6.19 (s, 1H), 3.92 (s, 3H), 3.88 (d, J = 7.1 Hz, 2H), 1.27-1.13 (m, 1H), 0.57-0.51 (m, 2H), 0.32-0.27 (m, 2H).

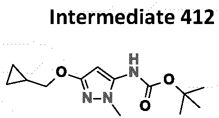

### tert-butyl N-[5-(cyclopropylmethoxy)-2-methyl-pyrazol-3-yl]carbamate

To a stirred solution of **intermediate 411** (1.00 g, 5.10 mmol) and triethylamine (2.13 mL, 15.3 mmol) in THF (25 mL) at 0 °C was added diphenylphosphoryl azide (1.65 mL, 7.65 mmol). After 10 min the reaction mixture was allowed to warm to room temperature and stirred for 1 h. *t*-Butyl alcohol (10 mL) was added and the reaction mixture heated to 80 °C and stirred for 16 h. The reaction mixture was concentrated in vacuo and the crude material was purified by flash chromatography [EtOAc/isohexanes (0-80%)] to afford the title compound (1.35 g, 99% Yield) as a colourless oil. δ_{H} (300 MHz, d-Chloroform) 6.36 (br. s, 1H), 5.59 (s, 1H), 3.89 (d, J = 7.1 Hz, 2H), 3.57 (s, 3H), 1.49 (s, 9H), 1.31-1.17 (m, 1H), 0.61-0.55 (m, 2H), 0.33-0.28 (m, 2H).

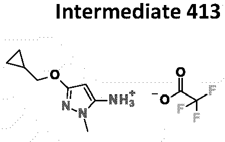

### [5-(cyclopropylmethoxy)-2-methyl-pyrazol-3-yl]ammonium 2,2,2-trifluoroacetate

To a solution of **intermediate 412** (1.35 g, 5.05 mmol) in CH₂Cl₂ (16 mL) was added trifluoroacetic acid (4 mL) and the reaction mixture stirred at room temperature for 16 h. The reaction mixture was concentrated in vacuo and the crude material azeotroped with toluene (3 x 20 mL) to give the title compound (1.60 g, quantitative) as an orange oil. δ_{H} (300 MHz, d-Chloroform) 5.02 (br. s, 1H), 3.96 (d, J = 7.3 Hz, 2H), 3.57 (s, 3H), 1.30-1.17 (m, 1H), 0.70-0.63 (m, 2H), 0.38-0.32 (m, 2H).

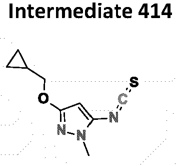

### 3-(cyclopropylmethoxy)-5-isothiocyanato-1-methyl-pyrazole

To a solution of **intermediate 413** (1.42 mg, 5.05 mmol) and N,N-diisopropylethylamine (1.14 mL, 6.56 mmol) in CH₂Cl₂ (15 mL) was added 1,1'-thiocarbonyldiimidazole (1.04 g, 5.56 mmol) and the reaction mixture stirred at room temperature for 16 h. The reaction mixture was concentrated in vacuo and the crude material was purified by flash chromatography [EtOAc/isohexanes (0-50%)] to afford the title compound (253 mg, 24% Yield) as a yellow oil. δ_{H} (300 MHz, d-Chloroform) 5.64 (s, 1H), 3.92 (d, J = 7.1 Hz, 2H), 3.64 (s, 3H), 1.31-1.18 (m, 1H), 0.63-0.57 (m, 2H), 0.35-0.30 (m, 2H).

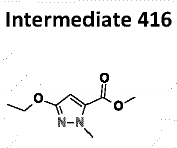

### methyl 5-ethoxy-2-methyl-pyrazole-3-carboxylate

To a stirred suspension of methyl 5-hydroxy-2-methyl-pyrazole-3-carboxylate (3.00 g, 19.0 mmol) and K₂CO₃ (2.90 g, 21.0 mmol) in DMF (19 mL) at room temperature was added iodoethane (1.71 mL, 21.0 mmol) and the reaction mixture stirred at 50 °C for 16 h. The reaction mixture was diluted with water (190 mL) and the aqueous layer extracted with ethyl acetate (3 x 100 mL). The combined organic layers were washed with water (2 x 100 mL), passed through a phase separator and concentrated in vacuo. The crude material was purified by flash chromatography [EtOAc/isohexanes (0-50%)] to afford the title compound (3.33 g, 94% Yield) as a colourless oil. δ_{H} (300 MHz, d-Chloroform) 6.17 (s, 1H), 4.16 (q, J = 7.1 Hz, 2H), 4.03 (s, 3H), 3.86 (s, 3H), 1.38 (t, J = 7.1 Hz, 3H).

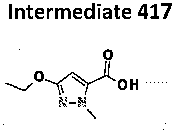

### 5-ethoxy-2-methyl-pyrazole-3-carboxylic acid

To a solution of **intermediate 416** (3.33 g, 18.1 mmol) in MeOH (90 mL) was added NaOH (aq. 2.0 M, 18 mL) and the reaction mixture stirred at 50 °C for 1 h. The reaction mixture was concentrated in vacuo, and then acidified to pH 1 with HCl (aq. 2 M). The mixture was diluted with water (200 mL) and the aqueous layer extracted with EtOAc (3 x 150 mL). The combined organic extracts were passed through a phase separator, and concentrated in vacuo to give the title compound (2.91 g, 94% Yield) as a colourless crystalline solid. δ_{H} (300 MHz, d-DMSO) 13.36 (s, 1H), 6.19 (s, 1H), 4.09 (q, J = 7.1 Hz, 2H), 3.92 (s, 3H), 1.28 (t, J = 7.1 Hz, 3H).

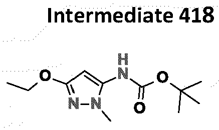

### tert-butyl N-(5-ethoxy-2-methyl-pyrazol-3-yl)carbamate

To a stirred solution of **intermediate 417** (1.00 g, 5.88 mmol) and triethylamine (2.46 mL, 17.6 mmol) in THF (29 mL) at 0 °C was added diphenylphosphoryl azide (1.90 mL, 8.81 mmol). After 10 min the reaction mixture was allowed to warm to room temperature and stirred for 1 h. t-Butyl alcohol (10 mL) was added and the reaction mixture heated to 80 °C and stirred for 16 h. The reaction mixture was concentrated in vacuo and the crude material was purified by flash chromatography [EtOAc/isohexanes (0-80%)] to afford the title compound (1.01 g, 77% Yield). δ_{H} (300 MHz, d-Chloroform) 6.19 (s, 1H), 5.59 (s, 1H), 4.14 (q, J = 7.1 Hz, 2H), 3.58 (s, 3H), 1.50 (s, 9H), 1.36 (t, J = 7.1 Hz, 3H).

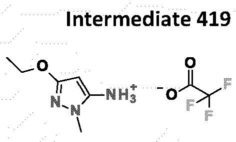

### (5-ethoxy-2-methyl-pyrazol-3-yl)ammonium 2,2,2-trifluoroacetate

To a solution of **intermediate 418** (1.00 g, 4.10 mmol) in CH₂Cl₂ (16 mL) was added trifluoroacetic acid (4 mL) and the reaction mixture stirred at room tempearture for 16 h. The reaction mixture was concentrated in vacuo and the crude material azeotroped with toluene (3 x 20 mL) to give the title compound (1.51 g, quantitative yield). δ_{H} (300 MHz, d-DMSO) 5.08 (br. s, 1H), 4.15 (q, J = 7.1 Hz, 2H), 3.44 (s, 3H), 1.30 (t, J = 7.1 Hz, 3H).

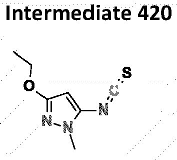

### 3-ethoxy-5-isothiocyanato-1-methyl-pyrazole

To a solution of **intermediate 419** (1.1 g, 4.3 mmol) and N,N-diisopropylethylamine (0.97 mL, 5.6 mmol) in CH₂Cl₂ (13 mL) was added 1,1'-thiocarbonyldiimidazole (890 mg, 4.7 mmol) and the reaction mixture stirred at room temperature for 16 h. The reaction mixture was concentrated in vacuo and the crude material was purified by flash chromatography [EtOAc/isohexanes (0-50%)] to afford the title compound (199 mg, 25% Yield). δ_{H} (300 MHz, d-Chloroform) 5.62 (s, 1H), 4.13 (q, J = 7.1 Hz, 2H), 3.65 (s, 3H), 1.36 (t, J = 7.1 Hz, 3H).

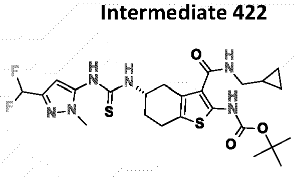

### tert-butyl N-[(5S)-3-(cyclopropylmethylcarbamovyl)-5-[[5-(difluoromethyl)-2-methyl-pyrazol-3-yl]carbamothioylamino]-4,5,6,7-tetrahydrobenzothiophen-2-yl]carbamate

A solution of **intermediate 559** (1.0 g, 2.74 mmol) was stirred in DCM (50 mL) and cooled to 0 °C. **Intermediate 254** (517.63 mg, 2.74 mmol) was then added. Reaction stirred at 0 °C for 30 min then at RT for 5 h. Reaction mixture was diluted with DCM then water (30 mL) was added and organic layer separated. The aqueous layer was extracted with DCM (2 x 20 mL) and the organic fractions combined, dried over sodium sulfate and concentrated under vacuum. The residue was purified by column chromatography using 0 - 70 % EtOAc in heptane to give the title compound (1480 mg, 97 % Yield). LCMS [M+H]⁺555, RT 2.04 minutes (Method 12).

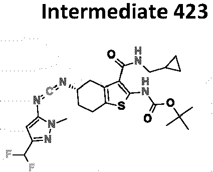

### tert-butyl N-[(5S)-3-(cyclopropylmethylcarbamoyl)-5-[[5-(difluoromethyl)-2-methyl-pyrazol-3-yl]iminomethyleneamino]-4,5,6,7-tetrahydrobenzothiophen-2-yl]carbamate

To a solution of **intermediate 422** (1790 mg, 3.23 mmol) in DCM (60 mL) was added triethylamine (1349 µL, 9.68 mmol). The solution was cooled to 0 °C then methanesulfonyl chloride (275 µL, 3.55 mmol) was added. Reaction stirred for 30 min at 0 °C. Reaction mixture was diluted with DCM (20 mL) then sat. NH₄Cl solution (30 mL) was added and organic layer separated. The aqueous layer was extracted with DCM (2 x 25 mL) and the organic fractions combined, dried over sodium sulfate and concentrated under vacuum to give the title compound (1720 mg, 95% Yield) which was used in the next stage without further purification. LCMS [M+H]⁺ 521, RT 2.13 minutes (Method 12).

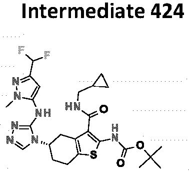

### tert-butyl N-[(5S)-3-(cyclopropylmethylcarbamoyl)-5-[3-[[5-(difluoromethyl)-2-methyl-pyrazol-3-yl]amino]-1,2,4-triazol-4-yl]-4,5,6,7-tetrahydrobenzothiophen-2-yl]carbamate

1 M formic hydrazide (9.68 mL, 9.68 mmol) in MeOH was added to **intermediate 423** (1680 mg, 3.23 mmol) followed by MeOH (10 mL) and stirred for 30 mins. Aqueous disodium carbonate (0.92 M, 10.52 mL, 9.68 mmol) was added and reaction heated at 50 °C with stirring for 16 hours. Reaction allowed to cool, MeOH was removed and water was added (20 mL). The mixture was extracted with DCM (2 X 30 mL). The solvent of the combined organic layers was dried (MgSO₄) before concentration to dryness. The residue was purified by column chromatography using 0 to 100% EtOAc in heptane to give the title compound (1300 mg, 71 % Yield). LCMS [M+H]⁺ 563, RT 1.91 minutes (Method 12).

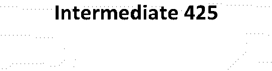

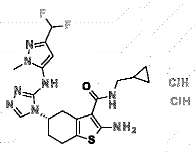

### (5S)-2-amino-N-(cyclopropylmethyl)-5-[3-[[5-(difluoromethyl)-2-methyl-pyrazol-3-yl]amino]-1,2,4-triazol-4-yl]-4,5,6,7-tetrahydrobenzothiophene-3-carboxamide dihydrochloride

**Intermediate 424** (75 mg, 0.13 mmol) was dissolved in 1,4-Dioxane (1.5 mL) then 4 M 1,4-dioxane hydrochloride (0.67 mL, 2.67 mmol) was added. Reaction stirred for 5 h at room temperature. Reaction mixture was concentrated under vacuum to give the title compound (70 mg, 98 % Yield) which was used in the next stage without further purification. LCMS [M+H]⁺463, RT 1.57 minutes (Method 12).

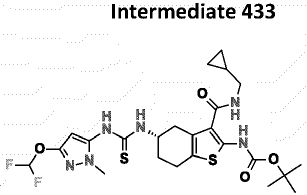

### tert-butyl N-[(5S)-3-(cyclopropylmethylcarbamoyl)-5-[[5-(difluoromethoxy)-2-methyl-pyrazol-3-yl]carbamothioylamino]-4,5,6,7-tetrahydrobenzothiophen-2-yl]carbamate

To a solution of **intermediate 559** (600 mg, 1.64 mmol) in DCM (20 mL) at 0 °C, **Intermediate 644** (336.85 mg, 1.64 mmol) was added. Reaction was stirred for 30 min at 0 °C then at r.t. for 3 h. The reaction mixture was diluted with DCM then water (30 mL) was added and organic layer separated. The aqueous layer was extracted with DCM (2 x 20 mL) and the organic fractions combined, dried over sodium sulfate and concentrated under vacuum. The residue was purified by column chromatography using 0 - 80 % EtOAc in heptane to give the title compound (903 mg, 96 % Yield). LCMS [M+H]⁺571, RT 2.12 minutes (Method 12).

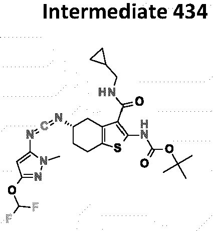

### tert-butyl N-[(5S)-3-(cyclopropylmethylcarbamoyl)-5-[[5-(difluoromethoxy)-2-methyl-pyrazol-3-yl]iminomethyleneamino]-4,5,6,7-tetrahydrobenzothiophen-2-yl]carbamate

To a solution of **intermediate 433** (903 mg, 1.58 mmol) in DCM (10 mL) was added triethylamine (662 µL, 4.75 mmol). The solution was cooled to 0 °C then methanesulfonyl chloride (135 µL, 1.74 mmol) was added and the reaction stirred for 30 min at 0 °C. The reaction mixture was diluted with DCM (10 mL) then saturated aqueous NH₄Cl (20 mL) was added and organic layer separated. The aqueous layer was extracted with DCM (2 x 20 mL) and the organic fractions combined, dried over sodium sulfate and concentrated under vacuum to give the title compound (840 mg, 93 % Yield) which was used in the next stage without further purification. LCMS [M+H]⁺ 537, RT 2.22 minutes (Method 12).

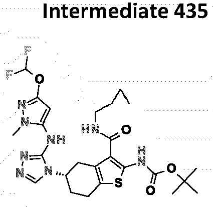

### tert-butyl N-[(5S)-3-(cyclopropylmethylcarbamoyl)-5-[3-[[5-(difluoromethoxy)-2-methyl-pyrazol-3-yl]amino]-1,2,4-triazol-4-yl]-4,5,6,7-tetrahydrobenzothiophen-2-yl]carbamate

1 M formic hydrazide (4.4 mL, 4.41 mmol) in MeOH was added to **intermediate 434** (840 mg, 1.47 mmol) followed by MeOH (10 mL). The reaction mixture was stirred for 30 mins then aqueous 0.92 M disodium carbonate (4.4 mL, 4.41 mmol) was added and reaction heated to 50 °C with stirring for 6 hours. Reaction allowed to cool, MeOH was removed and water was added (20 mL). The aqueous layer was extracted with DCM (2 x 30 mL) and the organic fractions combined, dried over sodium sulfate and concentrated under vacuum. The residue was purified by column chromatography using 0 - 100 % EtOAc in heptane to give the title compound (585 mg, 67 % Yield). δ_{H} (500 MHz, DMSO-d6) 12.04 (s, 1H, major rotamer), 10.39 (s, 1H), 8.73 (s, 1H, minor rotamer), 8.40 and 8.19 (2 x s, 1H, isomers), 7.48 (s, 1H), 7.35 - 6.93 (m, 1H), 5.90 and 5.65 (2 x s, 1H, isomers), 4.53 - 4.29 (m, 1H), 3.57 and 3.48 (2 x s, 3H, isomers), 3.22 - 2.96 (m, 4H), 2.92 - 2.74 (m, 2H), 2.28 - 2.13 (m, 2H), 1.47 (s, 9H), 1.04 - 0.93 (m, 1H), 0.39 - 0.31 (m, 2H), 0.22 - 0.14 (m, 2H). LCMS [M+H]⁺ 579, RT 1.96 minutes (Method 12).

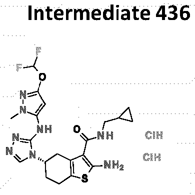

### (5S)-2-amino-N-(cyclopropylmethyl)-5-[3-[[5-(difluoromethoxy)-2-methyl-pyrazol-3-yl]amino]-1,2,4-triazol-4-yl]-4,5,6,7-tetrahydrobenzothiophene-3-carboxamide dihydrochloride

**Intermediate 435** (40 mg, 0.07 mmol) was dissolved in 1,4-Dioxane (1 mL) then 4 M 1,4-dioxane hydrochloride (0.35 mL, 1.38 mmol) was added. Reaction was stirred for 16 h at room temperature. Reaction mixture was concentrated under vacuum to give the title compound (38 mg, 99 % Yield) which was used in the next stage without further purification. LCMS [M+H]⁺ 479, RT 1.63 minutes (Method 12).

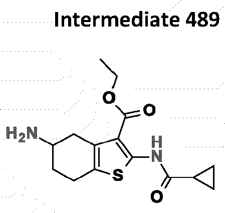

### Ethyl 5-amino-2-(cyclopropanecarbonylamino)-4,5,6,7-tetrahydrobenzothiophene-3-carboxylate

2,2,2-Trifluoroacetic acid (10 mL, 0.135 mol) was added drop wise to a stirred solution of **intermediate 25** (3.30 g, 8.08 mmol) in DCM (10 mL) at room temperature and stirred for 1 h. The reaction mixture was evaporated to dryness and residue dissovled in DCM (100 mL). The organic layer was washed with saturated aqueous NaHCO₃ (2 x 25 mL), followed by brine (25 mL), dried over sodium sulfate, filtered, evaporated to dryness to give the title compound (2.50 g, 97% Yield). LCMS [M+H]⁺ 309, RT 1.55 minutes (Method 12).

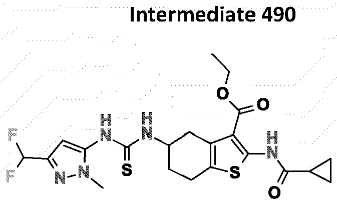

### Ethyl 2-(cyclopropanecarbonylamino)-5-[[5-(difluoromethyl)-2-methyl-pyrazol-3-yl]carbamothioylamino]-4,5,6,7-tetrahydrobenzothiophene-3-carboxylate

To a stirred solution of **intermediate 489** (2.58 g, 8.11 mmol) in anhydrous DCM (2 mL) was added dropwise solution of **intermediate 254** (1.68 g, 8.89 mmol) in DCM (1 mL) at room temperature and mixture was stirred for 2 h. A second portion of **intermediate 254** (0.5 g, 2.64 mmol) was added and stirred for 2 h. The reaction mixture was diluted with DCM (50 mL), washed with water (50 mL), dried over sodium sulfate, filtered, and concentrated to dryness. The crude residue was purified by column chromatography, eluting with 0-100% ethyl acetate in heptane to give the title compound (2.95 g, 68% Yield). δ_{H} (500 MHz, Chloroform-d) 11.43 (s, 1H), 7.45 (s, 1H), 6.60 (t, J = 55.0 Hz, 1H), 6.36 (s, 1H), 6.17 (br s, 1H), 4.78 (br s, 1H), 4.45 - 4.29 (m, 2H), 3.81 (s, 3H), 3.29 (dd, J = 17.0, 5.0 Hz, 1H), 2.91 - 2.79 (m, 1H), 2.79 - 2.62 (m, 2H), 2.20 - 2.11 (m, 1H), 2.03 - 1.92 (m, 1H), 1.73 - 1.65 (m, 1H), 1.41 (t, J = 7.1 Hz, 3H), 1.18 - 1.13 (m, 2H), 1.01 - 0.94 (m, 2H). LCMS [M+H]⁺ 498, RT 1.9 minutes (Method 12).

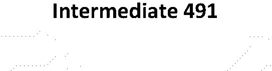

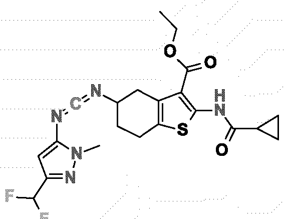

### Ethyl 2-(cyclopropanecarbonylamino)-5-[[5-(difluoromethyl)-2-methyl-pyrazol-3-yl]iminomethyleneamino]-4,5,6,7-tetrahydrobenzothiophene-3-carboxylate

Methanesulfonyl chloride (0.47 mL, 6.01 mmol) was added to a stirred suspension of **intermediate 490** (2.60 g, 5.23 mmol) and triethylamine (2.2 mL, 15.7 mmol) in dry DCM (50 mL) at ^{~}5 °C and mixture was stirred for 30 min. The reaction mixture was diluted with DCM (50 mL), washed with water (50 mL), saturated aqueous NH₄Cl (50 mL) and brine (50 mL). The organic layer was dried over sodium sulfate, filtered, concentrated to dryness to give the title compound (2.40 g, 95% Yield). LCMS [M+H]⁺ 464, RT 2.15 minutes (Method 12).

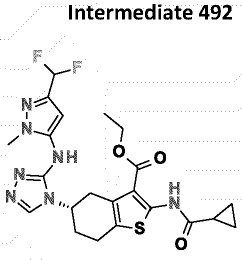

### ethyl (5S)-2-(cyclopropanecarbonylamino)-5-[3-[[5-(difluoromethyl)-2-methyl-pyrazol-3-yl]amino]-1,2,4-triazol-4-yl]-4,5,6,7-tetrahydrobenzothiophene-3-carboxylate

Formic hydrazide (0.90 g, 14.9 mmol) was added to a stirred solution of **intermediate 491** (2.40 g, 4.97 mmol) in ethanol (50 mL) and stirred at room temperature for 30 min. Aqueous 2 M disodium carbonate (10 mL, 19.9 mmol) was added and the reaction mixture heated at 45 °C for 16 h. The reaction mixture was cooled to room temperature and the inorganic solid removed by filtration. The filtrate was concentrated to dryness, diluted with water (50 mL), neutralized to pH 7, and extracted with 10% methanol in DCM (2 x 50 mL), followed by (1:1) IPA:CHCL₃ (2 x 50 mL). The combined organic layers were washed with brine (50 mL), dried over sodium sulfate, filtered, conc. to dryness to afford crude material. The crude material was dissolved in DCM (20 mL) and triturated with diethyl ether (100 mL) to afford precipitation which was filtered off and washed with TBME (2 x 10 mL) to afford crude material. The crude material was purified twice by column chromatography, eluting with 0-20% methanol in DCM to afford racemic mixture (1.70 g, 68% Yield). This was purified by chiral SFC to afford title compound (850 mg, 49%). LCMS [M+H]⁺ 506, RT 1.82 minutes (Method 12). Chiral LC* RT 10.54 minutes (Peak 1).

* Chiral analysis was carried out using CHIRALPAK IG 4.6 x 250 mm, 5 µm column, 2.4 mL/min, 200 Bar, eluting with a gradient of 60/40 CO₂/MEOH + 0.5% Isopropylamine, using a 30 minutes run time on SFC Berger.

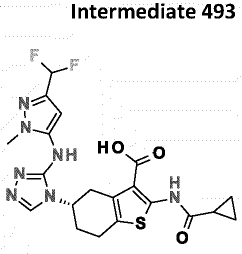

### (5S)-2-(cyclopropanecarbonylamino)-5-[3-[[5-(difluoromethyl)-2-methyl-pyrazol-3-yl]amino]-1,2,4-triazol-4-yl]-4,5,6,7-tetrahydrobenzothiophene-3-carboxylic acid

1 M aqueous sodium hydroxide (6.7 mL, 6.73 mmol) was added to a stirred solution of **intermediate 492** (850 mg, 1.68 mmol) in THF (7 mL) and mixture was stirred at room temperature over the weekend. The reaction mixture evaporated to dryness, acidified to pH ^{~}5 using 1 M aqueous HCl to afford white percipitated. This was filtered off, washed with water (2 mL), and dried in a vaccum oven to give the title compound (590 mg, 73% yield). δ_{H} (500 MHz, DMSO-d6) 12.54 (br s, 1H), 12.06 (s, 1H), 8.60 - 8.05 (m, 1H), 7.05 - 6.56 (m, 1H), 6.50 - 6.10 (m, 1H), 4.48 (s, 1H), 3.77 - 3.58 (m, 5H), 2.91 - 2.76 (m, 3H), 2.33 - 2.05 (m, 2H), 1.89 - 1.79(m, 1H), 0.95 - 0.81 (m, 4H). LCMS [M+H]⁺ 478, RT 1.65 minutes (Method 12).

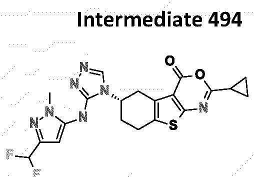

### (6S)-2-cyclopropyl-6-[3-[[5-(difluoromethyl)-2-methyl-pyrazol-3-yl]amino]-1,2,4-triazol-4-yl]-5,6,7,8-tetrahydrobenzothiopheno[2,3-d][1,3]oxazin-4-one

3-(ethyliminomethyleneamino)-N,N-dimethyl-propan-1-amine hydrochloride (270 mg, 1.41 mmol) was added to a stirred suspension of **intermediate 493** (560 mg, 1.17 mmol) in acetonitrile (15 mL) and the mixture heated at 50 °C for 5 h. The reaction mixture was stirred at room temperature over the weekend and then treated with 3-(ethyliminomethyleneamino)-N,N-dimethyl-propan-1-amine hydrochloride (50 mg, 0.261 mmol) and heated at 50 °C for 7 h. The reaction mixture was cooled to room temperature, evaporated to dryness, dissolved in 10% methanol in DCM (50 mL) and washed with water (20 mL) followed by brine (20 mL). The combined organic layers were dried over sodium sulfate, filtered, evaporated to dryness and purified by column chromatography, eluting with 0-100% ethyl acetate in heptane followed by 60% methanol in ethyl acetate to give the title compound (289 mg, 54% Yield). LCMS [M+H]⁺ 460, RT 1.75 minutes (Method 12) δδδδ

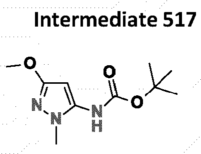

### tert-butyl N-(5-methoxy-2-methyl-pyrazol-3-yl)carbamate

To a solution of 5-methoxy-2-methyl-pyrazole-3-carboxylic acid (1.0 g, 6.40 mmol) in tert-butanol (50 mL) was introduced diisopropylethylamine (1.14 mL, 6.53 mmol) and diphenylphosphoryl azide (4.14 mL, 19.21 mmol). The reaction was warmed to 85 °C for 5 hours. After concentrating in-vacuo, the residue was purified by flash column chromatography (0-100% ethyl acetate gradient in heptane) to furnish the title compound (1.05 g, 68% Yield). δ_{H} (500 MHz, d₆-dmso) 9.28 (s, 1H), 5.51 (s, 1H), 3.69 (s, 3H), 3.47 (s, 3H), 1.45 (s, 9H); LCMS [M+H]⁺ 228, RT 1.37 minutes (Method 18).

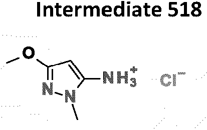

### (5-methoxy-2-methyl-pyrazol-3-yl)ammonium chloride

To a solution of **intermediate 517** (1270 mg, 5.59 mmol) in anhydrous 1,4-Dioxane (11 mL) was added 4 M HCl in dioxane (14 mL, 55.9 mmol) and the resulting solution stirred at room temperature for 16 hours. 4 M HCl in dioxane (3 mL, 12 mmol) was added and stirring was continued at room temperature for 3 hours. The reaction mixture was concentrated to dryness to afford the *title compound* (930 mg, quantitative). δ_{H} (500 MHz, DMSO-d6) 5.14 (s, 1H), 3.87 (s, 3H), 3.48 (s, 3H). Note: 3 exchangeable protons missing.

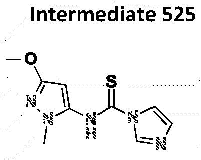

### N-(5-methoxy-2-methyl-pyrazol-3-yl)imidazole-1-carbothioamide

To a mixture of di(imidazol-1-yl)methanethione (90%, 890 mg, 4.49 mmol) and N-ethyl-N-isopropyl-propan-2-amine (1.6 mL, 8.98 mmol) in DCM (20 mL), (5-methoxy-2-methyl-pyrazol-3-yl)ammonium chloride (700 mg, 4.28 mmol) was added portion-wise and the resulting solution stirred at room temperature for 18 hours. The reaction mixture was used directly in the next step (1015 mg, quantitative). LCMS [M+H]⁺ 238, RT 1.69 minutes (Method 12).

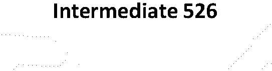

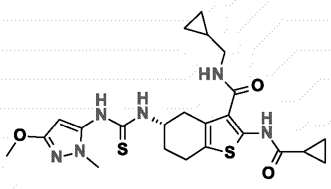

### (5S)-2-(cyclopropanecarbonylamino)-N-(cyclopropylmethyl)-5-[(5-methoxy-2-methyl-pyrazol-3-yl)carbamothioylamino]-4,5,6,7-tetrahydrobenzothiophene-3-carboxamide

To a solution of **intermediate 117** (940 mg, 2.82 mmol) in DCM (10 mL) a solution of **intermediate 525** (1003 mg, 4.23 mmol) in DCM (20 mL) was added dropwise. The mixture was stirred at room temperature for 18 hours. The reaction mixture was diluted with DCM (30 mL) and washed with saturated aqueous NH₄Cl (30 mL), saturated aqueous NaHCO₃ (30 mL), water (30 mL), brine (30 mL), and then dried over magnesium sulfate. The solvent was then concentrated under reduced pressure and the residue purified by flash column chromatography (0 to 100% of ethyl acetate in heptane gradient) to afford the title compound (1398 mg, 93% Yield). δ_{H} (400 MHz, DMSO-d6) 11.09 (s, 1H), 9.16 (s, 1H), 8.00 (d, J = 7.5 Hz, 1H), 7.79 - 7.59 (m, 1H), 5.61 (s, 1H), 4.63 - 4.41 (m, 1H), 3.72 (s, 3H), 3.46 (s, 3H), 3.21 - 3.00 (m, 3H), 2.82 - 2.59 (m, 3H), 2.07 0.98 (m, 1H), 1.95 - 1.83 (m, 2H), 1.09 - 0.98 (m, 1H), 0.93 - 0.80 (m, 4H), 0.51 - 0.40 (m, 2H), 0.29 - 0.16 (m, 2H). LCMS [M+H]⁺ 503, RT 1.80 minutes (Method 12).

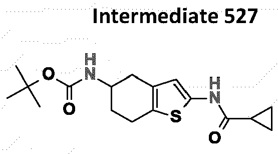

### tert-butyl N-[2-(cyclopropanecarbonylamino)-4,5,6,7-tetrahydrobenzothiophen-5-yl]carbamate

A solution of **intermediate 26** (27.60 g, 72.54 mmol, 1.0 eq.) in N,N-dimethylacetamide (240 mL) was heated at 170 °C for 2 hours. The reaction mixture was left to cool down to ambient temperature and the solution diluted with ethyl acetate (1 L). The organic phase was washed with water (500 mL) and brine (500 mL), dried over anhydrous sodium sulfate, filtered and the solvent removed in vacuo. The residue was purified by column chromatography using a gradient of ethyl acetate in heptane (5% to 80%) to afford the title compound (14.2 g, 58% yield). δ_{H} (250 MHz, Chloroform-d) 8.30 (s, 1H), 6.26 (s, 1H), 4.66 (d, J = 7.7 Hz, 1H), 3.96 (s, 1H), 2.85 (dd, J = 15.8, 5.0 Hz, 1H), 2.74 (t, J = 6.1 Hz, 2H), 2.36 (dd, J = 15.9, 7.3 Hz, 1H), 2.09 - 1.95 (m, 1H), 1.88 - 1.73 (m, 1H), 1.59 - 1.49 (m, 1H), 1.45 (s, 9H), 1.16 - 1.02 (m, 2H), 0.89 - 0.79 (m, 2H). LCMS [M+H]⁺ 337, RT 2.77 minutes (Method 12)

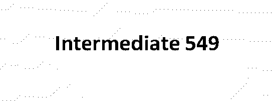

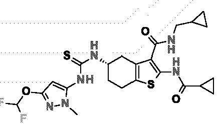

### (5S)-2-(cyclopropanecarbonylamino)-N-(cyclopropylmethyl)-5-[[5-(difluoromethoxy)-2-methyl-pyrazol-3-yl]carbamothioylamino]-4,5,6,7-tetrahydrobenzothiophene-3-carboxamide

A solution of the **intermediate 117** (40.0 mg, 0.12 mmol) in DCM (4 mL) was cooled to 0 °C. **Intermediate 644** (24.6 mg, 0.12 mmol) then was added. The reaction mixture was stirred for 30 min at 0 °C then RT for 5 h. DCM was added followed by water (30 mL). The aqueous layer was extracted with DCM (2 x 20 mL) and the organic fractions combined, dried over (Na₂SO₄) and concentrated under vacuum. The residue was purified by column chromatography eluting with 0 - 100 % EtOAc in heptane to afford product the title compound (41 mg, 63 % Yield). LCMS [M+H]⁺ 539, RT 1.89 minutes (Method 12).

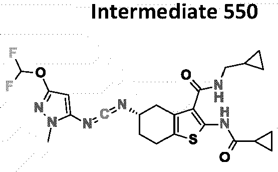

### (5S)-2-(cyclopropanecarbonylamino)-N-(cyclopropylmethyl)-5-[[5-(difluoromethoxy)-2-methyl-pyrazol-3-yl]iminomethyleneamino]-4,5,6,7-tetrahydrobenzothiophene-3-carboxamide

To a solution of **intermediate 549** (41 mg, 0.08 mmol) in DCM (2 mL) triethylamine (32 µL, 0.23 mmol) was added. The solution was cooled to 0 °C then methanesulfonyl chloride (6 µL, 0.08 mmol) was added. The reaction mixture was stirred for 30 min at 0 °C. DCM (10 mL) was then added followed by a saturated aqueous ammonium chloride solution (10 mL). The aqueous layer was extracted with DCM (2 x 15 ml) and the organic fractions combined, dried over (Na₂SO₄) and concentrated under vacuum to give the title compound (38 mg, 98 % Yield). LCMS [M+H]⁺ 505, RT 1.99 minutes (Method 12).

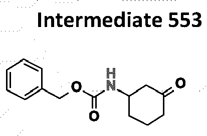

### Benzyl N-(3-oxocyclohexyl)carbamate

To a solution of benzyl carbamate (80 g, 530 mmol) and 2-cyclohexen-1-one (50 g, 510 mmol) in DCM (50 mL) was added bismuth(III) nitrate pentahydrate (25 g, 51 mmol) portion-wise over 15 min. The mixture was stirred for 16 h at ambient temperature. The reaction mixture was diluted with DCM (400 ml), filtered through celite and then washed with saturated aqueous sodium bicarbonate solution (twice), water, and then brine, and dried over MgSO₄. The solvent was removed in vacuo to give a yellow/orange gum that solidified. This was triturated with ether and stirred for ^{~}1 h. The slurry was filtered, washing with ether ( x 3 ) to give the title compound (62 g, 48 % Yield). δ_{H} (300 MHz, DMSO-d6) 7.49 (d, *J* = 7.6 Hz, 1H), 7.42 - 7.27 (m, 5H), 5.01 (s, 2H), 3.76 - 3.62 (m, 1H), 2.48 - 2.11 (m, 4H), 1.97 - 1.85 (m, 2H), 1.66 - 1.46 (m, 2H). LCMS [M+H]⁺ 248, RT 0.84 minutes (Method 7).

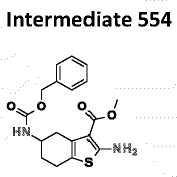

### Methyl 2-amino-5-(benzyloxycarbonylamino)-4,5,6,7-tetrahydrobenzothiophene-3-carboxylate

To a solution of methyl cyanoacetate (25.0 g, 250 mmol), **intermediate 553** (61.8 g, 250 mmol) and morpholine (32.6 g, 375 mmol) in 1,4-dioxane (200 mL) was added sulfur (8.81 g, 275 mmol). The mixture was stirred for 30 min at ambient temperature then at 70°C for 16 h. The reaction mixture was allowed to cool and the solvent removed in vacuo. The residue was stirred in diethyl ether (750 mL) for 4 h and the crude product filtered off. This was recrystallised from methanol / water to give the title compound (40.7 g, 45% Yield). LCMS [M+H]⁺ 361, RT 1.10 minutes (Method 7).

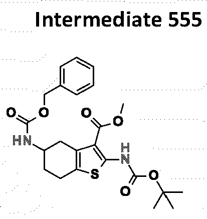

### Methyl 5-(benzyloxycarbonylamino)-2-(tert-butoxycarbonylamino)-4,5,6,7-tetrahydrobenzothiophene-3-carboxylate

To a solution of **intermediate 554** (25.0 g, 69.4 mmol), N,N-diisopropylethylamine (13.5 g, 104 mmol) and 4-dimethylaminopyridine (0.437 g, 3.47 mmol) in DCM (500 mL) was added di-tert-butyl dicarbonate (15.9 g, 72.8 mmol). The mixture was stirred 2 h at reflux. The reaction mixture was allowed to cool, and then washed with water and then brine. The organic phase was passed through a phase separator cartridge and the solvent removed in vacuo. The crude product was purified by flash chromatography eluting with 8-40% EtOAc in iso-hexane to afford the title compound (13.8 g, 43%) as a colourless gum. δ_{H} (300 MHz, DMSO-d6) 10.16 (s, 1H), 7.44 - 7.27 (m, 6H), 5.03 (s, 2H), 3.78 (s, 3H), 3.70 (s, 1H), 3.02 (dd, J = 16.8, 5.4 Hz, 1H), 2.77 - 2.57 (m, 2H), 2.55 - 2.43 (m, 1H), 1.97 - 1.87 (m, 1H), 1.72 - 1.57 (m, 1H), 1.48 (s, 9H). LCMS [M-H]⁻ 459 , RT 1.60 minutes (Method 7).

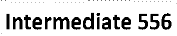

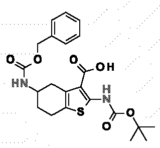

### 5-(Benzyloxycarbonylamino)-2-(tert-butoxycarbonylamino)-4,5,6,7-tetrahydrobenzothiophene-3-carboxylic acid

To a solution of **intermediate 555** (13.8 g, 29.9 mmol) in 1,4-dioxane (200 mL) was added a solution of lithium hydroxide monohydrate (3.84 g, 89.7 mmol) in water (100 mL). The mixture was heated at 50 °C for 12 h. The reaction mixture was allowed to cool and the solvent removed in vacuo. The residue was portioned between EtOAc and water. The aqueous phase was further extracted with EtOAc (x2). Combined organic extracts were washed with water and then brine, dried (MgSO₄) and the solvent removed in vacuo to give the title compound (12.2 g, 91%). LCMS [M+H]⁺ 447 , RT 0.89 minutes (Method 7).

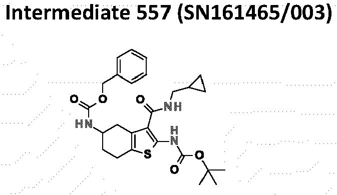

### tert-Butyl N-[5-(benzyloxycarbonylamino)-3-(cyclopropylmethylcarbamoyl)-4,5,6,7-tetrahydrobenzothiophen-2-yl]carbamate

To a solution of **intermediate 556** (12.2 g, 27.3 mmol), N,N-diisopropylethylamine (7.42 g, 57.4 mmol) and cyclopropylmethylamine (3.17 g, 43.7 mmol) in DMF (10 mL) at 0 °C was added HATU (20.8 g, 54.7 mmol). The mixture was stirred for 3 h and then the solvent was removed in vacuo. The residue was portioned between EtOAc and water. The aqueous phase was further extracted with EtOAc (x2). Combined organic extracts were washed with water and then brine, dried (MgSO4) and the solvent removed in vacuo to give a brown gum. The crude product was purified by flash chromatography eluting with 5-35% EtOAc in iso-hexane to afford the title compound (10.2g, 75%) as a colourless solid. LCMS [M+H]⁺ 500 , RT 2.94 minutes (Method 3).

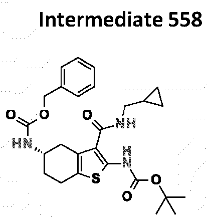

### tert-Butyl N-[(5S)-5-(benzyloxycarbonylamino)-3-(cyclopropylmethylcarbamoyl)-4,5,6,7-tetrahydrobenzothiophen-2-yl]carbamate

The title compound was obtained by chiral separation of **intermediate 557** (16.9 g, 33.9 mmol) to give the title compound. Peak 1 (9.24 g, 55% Yield). δ_{H} (400 MHz, DMSO-d6) 10.23 (s, 1H), 7.53 - 7.41 (m, 2H), 7.40 - 7.28 (m, 5H), 5.04 (s, 2H), 3.76 - 3.66 (m, 1H), 3.17 - 3.05 (m, 2H), 2.94 (dd, J = 16.0, 5.2 Hz, 1H), 2.79 - 2.63 (m, 2H), 2.62 - 2.53 (m, 1H), 1.98 - 1.89 (m, 1H), 1.78 - 1.65 (m, 1H), 1.46 (s, 9H), 1.06 - 0.95 (m, 1H), 0.45 - 0.36 (m, 2H), 0.26 - 0.17 (m, 2H). LCMS [M+H]⁺ 500, RT 3.00 minutes (Method 3). Chiral LC* RT 3.29 minutes,

* Chiral analysis was performed using a Chiralpak IC, eluted using an isocratic 50% IPA in heptane (+ 0.1% DEA) method, flow rate of 1.5 mL/min, 100 bar and an 8 minute run time on an Agilent Infinity II 1290.

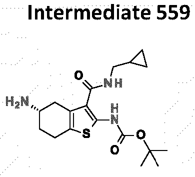

### tert-Butyl N-[(5S)-5-amino-3-(cyclopropylmethylcarbamoyl)-4,5,6,7-tetrahydrobenzothiophen-2-yl]carbamate

To a solution of **intermediate 558** (4.0 g, 8.00 mmol) in EtOAc (75 mL) was added 10% palladium on carbon (0.80 g). Stirred under an atmosphere of hydrogen for 5 days. The reaction mixture was filtered through celite, washing with methanol and the solvent removed in vacuo to give the title compound (2.44g, 83% Yield) as a green/yellow sticky solid. δ_{H} (300 MHz, DMSO-d6) 7.77 (s, 1H), 3.72 - 3.37 (m, 3H), 3.18 - 2.98 (m, 3H), 2.89 (dd, J = 15.9, 5.0 Hz, 1H), 2.75 - 2.54 (m, 2H), 2.48 - 2.38 (m, 1H), 1.96 - 1.85 (m, 1H), 1.59 - 1.50 (m, 1H), 1.46 (s, 9H), 1.08 - 0.96 (m, 1H), 0.46 - 0.38 (m, 2H), 0.27 - 0.19 (m, 2H). LCMS [M+H]⁺ 366 , RT 1.88 minutes (Method 3).

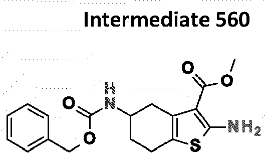

### methyl(5S)-2-amino-5-(benzyloxycarbonylamino)-4,5,6,7-tetrahydrobenzothiophene-3-carboxylate, hydrochloride

The title compound was prepared from benzyl N-(3-oxocyclohexyl)carbamate, methyl cyanoacetate and sulphur, using a method analogous to that used to prepare intermediate 567. The title compound was isolated after salt formation in *iso*-propanol as an off-white solid (89.5 g, 25% Yield). LCMS [M+H]⁺ 361, RT 1.90 minutes (Method 12).

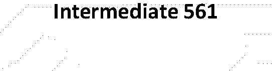

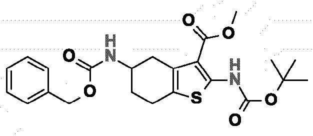

### methyl(5S)-5-(benzyloxycarbonylamino)-2-(tert-butoxycarbonylamino)-4,5,6,7-tetrahydrobenzothiophene-3-carboxylate

A solution of **intermediate 560** (31.2 g, 76.9 mmol), in DCM (450 mL) was treated with triethylamine (53 mL, 308 mmol) and 4-dimethylaminopyidine (1.9 g 15 mmol). Tert-butoxycarbonyl-tert-butyl-carbonate (36.9 g, 169.2 mmol) was added portionwise and the resulting mixture was stirred at reflux for 4 days during which further charges of tert-butoxycarbonyl-tert-butyl-carbonate (total of 25 g 114.5 mmol) were added. The reaction mixture was washed with water and brine and concentrated *in vacuo.* The crude residue was purified by chromatography on a pad of silica gel eluting with a gradient of EtOAc in hexane (5 to 20%) and by trituration with hexane to yield the title compound as a cream-coloured solid (24.6 g, 70% Yield). LCMS [M+Na]⁺ 483, RT 2.23 minutes (Method 12).

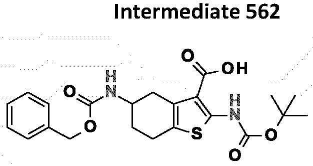

### 5-(benzyloxycarbonylamino)-2-(tert-butoxycarbonylamino)-4,5,6,7-tetrahydrobenzothiophene-3-carboxylic acid

A solution of **intermediate 561** (24.5 g, 53 mmol) in 1,4-dioxane (250 mL) was treated with lithium hydroxide (3.97 g, 159.3 mmol in 125 mL water). The resulting mixture was treated with methanol until a single phase solution was achieved and was heated to 40 °C for 1 day, after which time the reaction mixture was concentrated *in vacuo* and suspended in 1,4-dioxane (250 mL) and water (100 mL) and treated with citric acid (10 % aqueous solution, approximately 250 mL added) until the mixture reached pH4. The mixture was filtered and solid washed with water and dried *in vacuo* to give the title compound as a cream-coloured solid (22 g, 76% Yield). LCMS [M-H]⁻ 445, RT 2.08 minutes (Method 12).

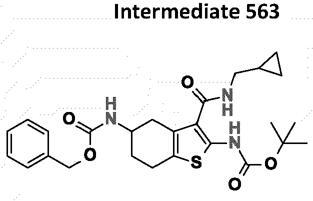

### tert-butylN-[5-(benzyloxycarbonylamino)-3-(cyclopropylmethylcarbamoyl)-4,5,6,7-tetrahydrobenzothiophen-2-yl]carbamate

A solution of **intermediate 562** (34.5 g, 77.3 mmol) in DMF (345 mL) was treated with DIPEA (15 g, 116 mmol) and cyclopropylmethylamine (6.2 g, 85 mmol), followed by HATU (38.2 g, 100.4 mmol) and the resulting stirred for 15 minutes and then poured into ice-water. After 18 h the resulting solid was removed and taken up in tert-butyl-methyl ether, the resulting solution was wahed with water and brine and filtered through a phase-separation cartridge, washing with EtOAc. The resulting solution was concentrated *in vacuo* and the residue recrystalised from a mixture of heptane and EtOAc to give the title compound as a pale yellow solid (22.3 g, 58% Yield). LCMS [M+H]⁺ 499, RT 4.45 minutes (Method 10).

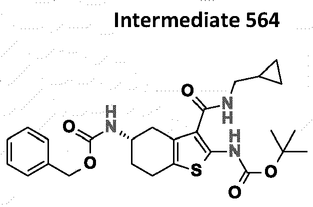

### tert-butylN-[(55)-5-(benzyloxycarbonylamino)-3-(cyclopropylmethylcarbamoyl)-4,5,6,7-tetrahydrobenzothiophen-2-yl]carbamate

The title compound was prepared by chiral SFC separation of racemic **intermediate 563** (30 g), using a 50 x 229 - 10µm GS-NO2 column, eluted with 20% MeOH in CO₂ at a flow rate of 360 ml/min to give the title compound as a brown solid (13 g). LCMS [M+H]⁺ 500, RT 1.56 minutes (Method system 3, pH10 short). ${\left[α\right]}_{D}^{20}$ 50.8 (c 2.0, CH₃OH).

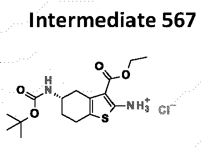

### [(5S)-5-(tert-butoxycarbonylamino)-3-ethoxycarbonyl-4,5,6,7-tetrahydrobenzothiophen-2-yllammonium chloride

The title compound was prepared by a method analogous to that used to prepare intermediate 102a. A solution of tert-butyl N-[(1S)-3-oxocyclohexyl]carbamate (100 g, 70% w/w, 328 mmol) in ethanol (600 mL) was treated with elemental sulphur (18 g, 561 mmol) followed by ethyl cyanoacetate (65 mL, 609 mmol). The resulting suspension was treated with triethylamine (30 mL, 215 mmol, added over minutes whilst maintaining internal temperature below 20 °C) and stirred at 20 °C for 18 h then filtered and the filtrate concentrated to dryness. The residue was taken up in EtOAc (750 mL) and treated with HCl (110 mL, 5.5 M in iso-propanol, added slowly whilst maintaining the internal temperature below 30 °C) and the resulting suspension stirred at 20 °C for 18 h. The title compound was obtained as a solid (89 g, 72% Yield) after filtration and washing with EtOAc (250 mL). LCMS [M+H]⁺ 341, RT 1.13 minutes (Method 7).

Note: tert-butyl N-[(1S)-3-oxocyclohexyl]carbamate (also called: (S)-3-Boc-aminocyclohexanone) may be purchased commercially from a number of suppliers including J&W Pharmlab Ltd of Levittown, PA, USA (CAS [1803033-61-5])

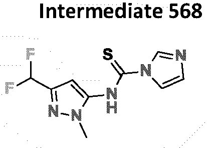

### N-[5-(difluoromethyl)-2-methyl-pyrazol-3-yl]imidazole-1-carbothioamide

A suspension of thiocarbonyl diimidazole (107.3 g, 572 mmol) in DCM (275 mL) was cooled to -10 °C and treated with a solution of 5-(Difluoromethyl)-2-methyl-pyrazol-3-amine (42.0 g, 285 mmol) in DCM (210 mL) whilst maintaining internal temperature between -8 °C and -10 °C. The resulting mixture was stirred at -10 C for 18 h and filtered and the solid washed with DCM (300 mL) to provide the title compound as a pale solid (71.4 g, 49 % Yield). LCMS ion observed results from solvolysis by MeOH to give C₇H₉F₂N₃OS [M+H]⁺ 222, RT 0.34 minutes (Method7).

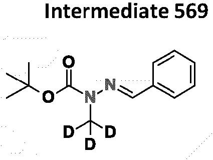

### tert-Butyl N-[(E)-benzylideneamino]-N-(trideuteriomethyl)carbamate

A solution of tert-Butyl N-[(E)-benzylideneamino]carbamate (68 g, 308 mmol) in THF (680 mL) was treated with t-BuOK (41.6 g, 370.5 mmol) followed by CD₃I (53.7 g, 370.5 mmol, added dropwise). The resulting mixture was stirred at 25 °C for 12 h after which time the mixture was diluted with water (500 mL) and extracted with EtOAc (three portions of 500 mL). The crude organic extracts were dried over Na₂SO₄ and concentrated *in vacuo* to afford the title compound as a yellow solid (68 g, 93% Yield). δ_{H} (400 MHz, CDCl3) 7.68 - 7.74 (m, 2 H), 7.65 (s, 1 H), 7.29 - 7.44 (m, 3 H), 1.59 (s, 9 H).

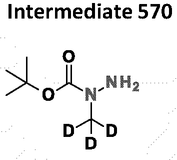

### tert-Butyl N-amino-N-(trideuteriomethyl)carbamate

A suspension of **intermediate 569** (68 g, 286.5 mmol) and Palladium on carbon (6.8 g) in MeOH (680 mL) was degassed *in vacuo* and purged with H₂ three times and then stirred un an atmosphere of H₂ (50 psi, 344.7 kPa) at 50 °C for 12 h. The reaction mixture was filtered and concentrated *in vacuo* to yield the title product as a yellow oil (40 g, 93% Yield). δ_{H} (400 MHz, DMSO-d6) 4.48 (s, 2H), 1.40 (s, 9H).

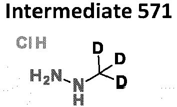

### Trideuteriomethylhydrazine hydrochloride

A solution of **intermediate 570** (40 g, 268.1 mmol) in EtOAc (40 mL) was treated with HCl (4 N in EtOAc, 200mL, 800 mmol) and stirred at 25 C for 12 h, after which time the reaction mixture was concentrated in vacuo to give the title compound as a yellow solid (20 g, 47% Yield). δ_{H} (400 MHz, DMSO-d6) 7.84 (br s, 3H).

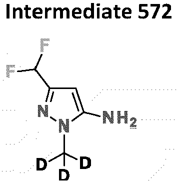

### 5-(Difluoromethyl)-2-(trideuteriomethyl)pyrazol-3-amine

A solution of 4,4-difluoro-3-oxo-butanenitrile (22.5 g, 189.3 mmol) in EtOH (105 mL) was treated with **intermediate 571** (20 g, 126.2 mmol) followed by Et₃N (51.1 g, 504.8 mmol) and heated to 80 C with stirring for 2 h. The resulting mixture was concentrated *in vacuo* and the residue diluted with water (100 mL) and extracted with EtOAc (three portions of 100 mL). The combined organic extracts were dried over Na₂SO₄, filtered and concentrated *in vacuo.* The residue was purified by flash column chromatography on silica eluting with EtOAc to yield the title compound as a yellow oil (5.50 g, 28 % Yield). δ_{H} (400 MHz, DMSO-d6) 6.48 - 6.90 (t, J =54.8 Hz, 1H), 5.43 (br d, *J* =16 Hz, 3H).

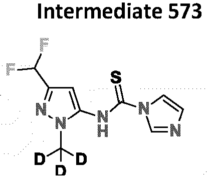

### N-[5-(difluoromethyl)-2-(trideuteriomethyl)pyrazol-3-yl]imidazole-1-carbothioamide

The title compound was prepared by a method analogous to that used to prepare **intermediate 572** using **intermediate 571.** The title compound was obtained as a solid (3.61 g, 74% Yield). δ_{H} (400 MHz, D₂O) 8.51 (s,1H), 7.83 (s,1H), 6.96 (s,1H), 6.72 (t,1H), 6.62 (s,1H).

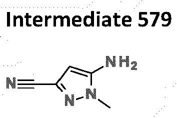

### 5-Amino-1-methyl-pyrazole-3-carbonitrile

1-Methyl-5-nitro-1H-pyrazole-3-carbonitrile (1000 mg, 6.25 mmol) was dissolved in tetrahydrofuran (15 mL) and methanol (15 mL). Ammonium chloride (4.68 g, 87.4 mmol) added. Cooled to 0°C. Zinc (5840 mg, 87.4 mmol) as filings added portion-wise. Ice bath removed after 60 mins. Stirred at room temperature for a further 1h. Filtered through celite washing with MeOH (10 mL x 2). Evaporated to leave a pale yellow solid. Used directly in the next step without purification. δ_{H} (300 MHz, DMSO-d6) 5.81 (s, 1H), 5.71 (s, 2H), 3.60 (s, 3H).

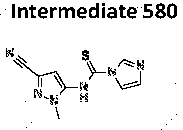

### N-(5-Cyano-2-methyl-pyrazol-3-yl)imidazole-1-carbothioamide

1,1'-Thiocarbonyldiimidazole (653 mg, 3.30 mmol) was added to a stirred solution of **intermediate 579** (403 mg, 3.30 mmol) in DCM (20 mL) at -10 °C and the reaction stirred for 90 min at -10 °C. The mixture was filtered and the solvent was removed in vacuo to give the crude product as a beige foam. Used directly in the next step without purification.

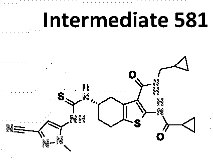

### (5S)-5-[(5-Cyano-2-methyl-pyrazol-3-yl)carbamothioylamino]-2-(cyclopropanecarbonylamino)-N-(cyclopropylmethyl)-4,5,6,7-tetrahydrobenzothiophene-3-carboxamide

**Intermediate 117** (1100 mg, 3.30 mmol) was added to a stirred solution of **intermediate 580** (766 mg, 3.30 mmol) in DCM (10 mL) and the reaction stirred for 90 min at ambient temperature. The solvent was removed in vacuo to give the crude product as a beige foam. Used directly in the next step without purification.

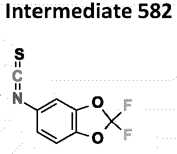

### 2,2-Difluoro-5-isothiocyanato-1,3-benzodioxole

2,2-Difluoro-5-aminobenzodioxole (1.0 g, 5.70 mmol) was dissolved in DCM (20 mL) and 4 N HCl in dioxane (2.8 mL, 11.0 mmol) was added. 1,1'-Thiocarbonyldiimidazole (1100 mg, 5.70 mmol) was added. The reaction was stirred at ambient temperature for 2 h, filtered and concentrated in vacuo to afford the crude title compound, which was carried through to the next stage without purification.

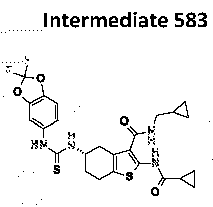

### (55)-2-(Cyclopropanecarbonylamino)-N-(cyclopropylmethyl)-5-[(2,2-difluoro-1,3-benzodioxol-5-yl)carbamothioylamino]-4,5,6,7-tetrahydrobenzothiophene-3-carboxamide

**Intermediate 117** (300 mg, 0.900 mmol) was added to a stirred solution of **intermediate 582** (232 mg, 1.08 mmol) in DCM (20 mL) and the reaction stirred for 40 min at ambient temperature. The solvent was removed in vacuo. The crude product was purified by flash chromatography eluting with 5-100% EtOAc in iso-hexane to afford the title compound (455 mg, 92%) as a colourless powder. δ_{H} (300 MHz, DMSO-d6) 11.11 (s, 1H), 9.54 (s, 1H), 7.91 (s, 1H), 7.91 - 7.66 (m, 2H), 7.34 (d, *J* = 8.6 Hz, 1H), 7.09 (dd, *J* = 8.6, 2.1 Hz, 1H), 4.57 (s, 1H), 3.24 - 3.00 (m, 3H), 2.78 - 2.58 (m, 3H), 2.10 - 1.81 (m, 3H), 1.10 - 0.97 (m, 1H), 0.91 - 0.78 (m, 4H), 0.48 - 0.38 (m, 2H), 0.27 - 0.19 (m, 2H). LCMS [M+H]⁺ 549 , RT 1.06 minutes (Method 7).

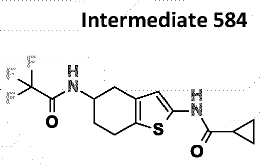

### N-[5-[(2,2,2-trifluoroacetyl)aminol-4,5,6,7-tetrahydrobenzothiophen-2-yl]cyclopropanecarboxamide

A solution of the trifluoroacetamide salt of **intermediate 29** (83.0 g, 237 mmol) in methanol (1500 mL) was stirred at 20 °C and treated with DIPEA (87 mL, 498 mmol) followed by ethyl trifluoacetate (56.6 mL, 474 mmol). After 16 h the reaction mixture was cooled to 0 °C and poured into ice-water (3 L). The resulting solid was collected by filtration, washed with water (4 portions of 300 mL) and cyclohexane (300 mL). The solid was further dried by azeotrope with toluene and drying in vacuo to yield the title compound as a beige solid (76 g, 95% Yield). δ_{H} (250 MHz, DMSO-d6) 11.12 (s, 1H), 9.46 (d, J = 7.5 Hz, 1H), 6.30 (s, 1H), 4.13-3.88 (m, 1H), 2.84-2.61 (m, 3H), 2.56-2.41 (m, 1H), 2.04-1.89 (m, 1H), 1.89-1.62 (m, 2H), 0.8 (d, J = 6.1 Hz, 4H). LCMS [M+H]⁺ 333 RT 1.66 minutes (Method 12).

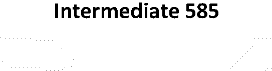

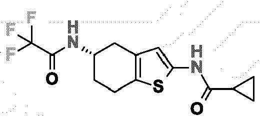

### N-[(S5)-5-[(2,2,2-trifluoroacetyl)amino]-4,5,6,7-tetrahydrobenzothiophen-2-yl]cyclopropanecarboxamide

Purification of **intermediate 584** by chiral-SFC using a 50x250 mm, 20 µm ChiralPak IG column, eluting with 25% EtOH in CO₂, yielded the title compound (the second peak eluted form the column) as a beige solid (2.196 g, 44% Yield). LCMS [M+H]⁺ 333 RT 0.91 minutes (Method 7). ${\left[α\right]}_{D}^{20}$ 28.4 (c 1.0, CH₃OH)

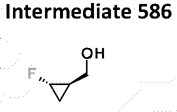

### [(1R,2S)-2-fluorocyclopropyl]methanol

To a stirred solution of (1R,2S)-2-fluorocyclopropanecarboxylic acid (5 g, 48.0 mmol) in anhydrous THF (25 mL) under a N₂ atmosphere and cooled in an ice bath was added 2 M lithium aluminum hydride in THF (31 mL, 62 mmol) dropwise. Initial effervescence subsided and a white precipitate was observed during the addition. The reaction was warmed to ambient temperature over 90 min and was stirred at this temperature overnight, during which time the precipitate went back into solution. The reaction was cooled with an ice bath prior to dropwise addition of water (3 mL) then 2 M sodium hydroxide (3 mL). THF (30 mL) was added to the resulting thick precipitate and stirring continued for 25 min. The mixture was filtered, washed with THF (3 x 25 mL) and the filtrate concentrated under reduced pressure to afford the title compound (5 g, 67% Yield) as a colourless oil (~42% THF w/w/ present). δ_{H} (300 MHz, d-Chloroform) 4.46 (ddt, J = 64.1, 6.3, 2.2 Hz, 1H), 3.55 - 3.36 (m, 2H), 1.56 (ddqd, J = 19.9, 11.0, 6.9, 1.9 Hz, 1H), 1.08 (dddd, J = 21.0, 10.9, 7.0, 2.6 Hz, 1H), 0.61 (dq, J = 10.3, 6.7 Hz, 1H).

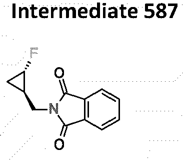

### 2-[[(1R,2S)-2-fluorocyclopropyl]methyl]isoindoline-1,3-dione

**Intermediate** 586 (5 g, 32 mmol), phthalimide (5.74 g, 38 mmol) and triphenylphosphine (12.8 g, 48 mmol) were suspended in anhydrous THF (25 mL) and stirred under N₂ at 0 °C prior to dropwise addition of diethyl azodicarboxylate (6.3 mL, 40 mmol). Upon completion of addition, the suspension went into solution. The reaction was warmed to ambient temperature and stirred for 23 h. The reaction was concentrated under reduced pressure to remove volatiles and diluted with EtOAc (75 mL). The solution was washed with sat aqueous NaHCO₃ (50 mL) followed by aqueous 2 M HCl (50 mL) and then brine (25 mL). The organic phase was dried (Na₂SO₄) and concentrated in vacuo. White needles had crystallised overnight. Upon breaking these up for filtration, a further crystalline solid crashed out. Combined solids were filtered, washing with diethyl ether. The residue & filtrate both contained product. These were combined, and purified by column chromatography eluting with 0-100% EtOAc in iso-hexanes to give the title compound (4.83 g, 68% Yield). δ_{H} (300 MHz, d-DMSO) 8.00 - 7.73 (m, 4H), 4.71 (dddd, J = 64.3, 6.2, 2.6, 1.9 Hz, 1H), 3.56 - 3.36 (m, 2H), 1.72 - 1.43 (m, 1H), 1.06 (dddd, J = 21.9, 11.0, 7.0, 2.7 Hz, 1H), 0.72 (dq, J = 10.8, 6.7 Hz, 1H).

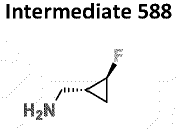

### [(1R,2S)-2-fluorocyclopropyl]methanamine

A suspension of **intermediate 587** (4.3 g, 20 mmol) in ethanol (20 mL) was treated with 24% hydrazine hydrate (4.5 mL, 22 mmol). The reaction was sealed and heated to 60 °C with stirring for 3 hours. A second aliquot of 24 % hydrazine hydrate (1 mL, 4.9 mmol) was added and heating increased to 80 °C for a further 90 min. The reaction was filtered to remove colourless precipitate (washing with DCM). A solution of the crude product was obtained by distillation (120 mBar to 10 mBar at 40°C). 4 M HCl in dioxane (2.5 mL) was added to the solution which was subsequently concentrated in vacuo. The resulting colourless powder was dissolved in DMSO (10 mL) and purified via column chromatography (KP-NH) eluting with a 0-100% gradient of MeOH in DCM to give the title compound as a 6% w/v solution in DMSO (10.5 g, 36% Yield). δ_{H} (300 MHz, Chloroform-d) 4.38 (ddt, J = 64.2, 6.2, 2.1 Hz, 1H), 2.55 - 2.49 (m, 2H), 1.52 - 1.26 (m, 1H), 1.10 - 0.92 (m, 1H), 0.50 (dq, J = 10.0, 6.7 Hz, 1H).

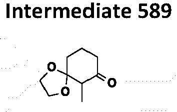

### 6-methyl-1,4-dioxaspiro[4.5]decan-7-one

ethylene glycol (43 mL, 769 mmol), 2-methyl-1,3-cyclohexanedione (25 g, 192 mmol) and PTSA (100 mg, 0.58 mmol) were suspended in toluene (350 mL) in a 1 L round bottom flask equipped with Dean-Stark condenser, and the mixture refluxed (set temp 130 °C) overnight with stirring. The mixture was cooled and concentrated in vacuo. The crude product was redissolved in DCM (250 mL) and treated with silica (40 g) and 3 M aqueous sulfuric acid (6.0 mL). The resulting slurry was stirred vigorously overnight at ambient temperature. The reaction was filtered and washed with saturated aqueous NaHCO₃ solution (2 x 250 mL). The organic layer was dried over Na₂SO₄ and the solvent removed in vacuo. The crude product was purified using column chromatography (eluting with a 0-60% gradient of Et₂O in isohexane) to afford the title compound (12.5 g, 38.2% Yield). δ_{H} (300 MHz, Chloroform-d) 4.02 - 3.86 (m, 4H), 2.73 (qd, *J* = 6.7, 1.1 Hz, 1H), 2.49 - 2.37 (m, 1H), 2.33 - 2.17 (m, 1H), 2.02 - 1.92 (m, 1H), 1.92 - 1.64 (m, 3H), 1.04 (d, *J* = 6.7 Hz, 3H).

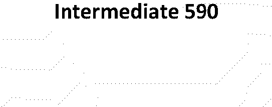

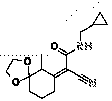

### (2Z)-2-cyano-N-(cyclopropylmethyl)-2-(6-methyl-1,4-dioxaspiro[4.5]decan-7-ylidene)acetamide

To a stirred solution of **intermediate 589** (10.2 g, 74.0 mmol) and 6-methyl-1,4-dioxaspiro[4.5]decan-7-one (12000 mg, 70.5 mmol) in anhydrous THF (200 mL) at r.t. were added pyridine (10.7 g, 133.95 mmol) and titanium (IV) isopropoxide (62.0 g, 211.50 mmol). The reaction was stirred at ambient temperature overnight. The reaction was stirred vigorously as it was quenched by dropwise addition of water (200 mL). The organic layer was decanted, passing through a phase separator cartridge and the TiO₂ residues slurried with DCM (2 x 150 mL), also passing through phase separator cartridge. The combined organic layers were concentrated in vacuo. The crude product was purified by column chromatography (eluting with a 0-80% gradient of EtOAc in isohexane) to afford the title compound (10.95 g, 40% Yield). δ_{H} (300 MHz, Chloroform-d) 6.48 - 6.09 (m, 1H), 4.02 - 3.88 (m, 4H), 3.23 - 3.11 (m, 2H), 2.89 - 2.74 (m, 1H), 2.52 - 2.35 (m, 1H), 2.00 - 1.79 (m, 2H), 1.75 - 1.51 (m, 2H), 1.21 (d, J = 7.1 Hz, 3H), 1.10 - 0.88 (m, 1H), 0.62 - 0.47 (m, 2H), 0.23 (dt, J = 5.8, 4.6 Hz, 2H).

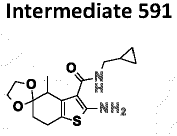

### 2'-amino-N-(cyclopropylmethyl)-4'-methyl-spiro[1,3-dioxolane-2,5'-6,7-dihydro-4H-benzothiophene]-3'-carboxamide

To a solution of **intermediate 590** (10.95 g, 37.7 mmol) in ethanol (200 mL) was added morpholine (6.70 mL, 75.4 mmol) and sulfur (1.81 g 56.6 mmol). The reaction was heated to 100 °C with stirring for 5.5 h. The volatiles were removed in vacuo to afford the crude title compound (24 g), which was used in the next stage without purification.

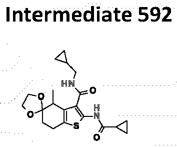

### 2'-(cyclopropanecarbonylamino)-N-(cyclopropylmethyl)-4'-methyl-spiro[1,3-dioxolane-2,5'-6,7-dihydro-4H-benzothiophene]-3'-carboxamide

Crude **intermediate 591** (24 g) was dissolved in DCM (300.0 mL) and DIPEA (26.0 mL, 148.9 mmol) was added. The solution was cooled in an ice bath with stirring prior to dropwise addition of cyclopropanecarbonyl chloride (7.58 mL, 81.89 mmol). Upon completion of addition the ice bath was removed and stirring continued for 30 min. The reaction was diluted with water (200 mL) and partitioned. The organic layer was passed through a phase separator and concentrated in vacuo. The crude product was purified by column chromatography (eluting with a 0-100% gradient of EtOAc in isohexane) to afford the title compound (8.8g, 30% Yield). δ_{H} (300 MHz, DMSO-*d*₆) 10.68 (s, 1H), 8.09 (t, J = 5.7 Hz, 1H), 3.98 - 3.86 (m, 4H), 3.22 - 3.00 (m, 3H), 2.80 - 2.57 (m, 2H), 2.01 - 1.86 (m, 2H), 1.69 (dt, J = 12.9, 4.4 Hz, 1H), 1.10 - 0.97 (m, 4H), 0.88 - 0.76 (m, 4H), 0.67 - 0.56 (m, 1H), 0.46 - 0.35 (m, 2H), 0.26 - 0.18 (m, 2H).

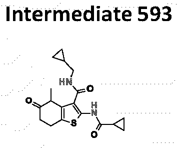

### 2-(cyclopropanecarbonylamino)-N-(cyclopropylmethyl)-4-methyl-5-oxo-6,7-dihydro-4H-benzothiophene-3-carboxamide

To a solution of **intermediate 592** (10.0 g, 25.6 mmol) in THF (200 mL) was degassed using a stream of N₂ for 10 min prior to addition of 1 M HCl (25 mL). The solution was degassed for 2 more minutes and then stirred at 70 °C under N₂ until complete. The reaction mixture was basified with solid NaHCO₃ until effervescence ceased and then diluted with EtOAc until 2 layers formed. The top layer was partitioned and dried over Na₂SO₄, then concentrated in vacuo to afford the title compound (9.31 g, 89 % Yield). LCMS [M+H]⁺ 347, RT 1.11 minutes (Method 33).

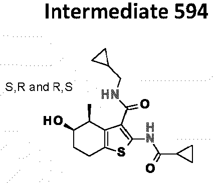

### (4SR,5RS)-2-(cyclopropanecarbonylamino)-N-(cyclopropylmethyl)-5-hydroxy-4-methyl-4,5,6,7-tetrahydrobenzothiophene-3-carboxamide

To an ice-cold stirred solution **intermediate 593** (9.1 g, 26 mmol) in MeOH (200 mL) and DCM (40.00 mL) was added NaBH₄ (1500 mg, 39 mmol) portion-wise over 30 min. The reaction mixture was stirred at this temperature for 2.5 h. The reaction mixture was quenched by addition aqueous AcOH (1 mL AcOH in 25 mL H₂O) and concentrated in vacuo. The resulting slurry was diluted with DCM (150 mL), MeOH (10 mL) and water (50 mL). The organic layer was partitioned and the aqueous layer extracted with DCM/MeOH (150 mL). The organic layer was concentrated and then purified by column chromatography (eluting with a 0-100% gradient of EtOAc in isohexane followed by 0-20% MeOH in EtOAc) to afford the title compound (4.5 g, 49 % Yield). δ_{H} (400 MHz, Chloroform-d) 12.21 (s, 1H), 6.24 - 6.11 (m, 1H), 4.18 (ddd, J = 11.6, 5.2, 3.9 Hz, 1H), 3.41 - 3.19 (m, 2H), 3.18 - 3.09 (m, 1H), 2.84 - 2.78 (m, 2H), 2.03 - 1.85 (m, 2H), 1.69 - 1.63 (m, 2H), 1.26 (d, J = 6.9 Hz, 3H), 1.17 - 1.00 (m, 3H), 0.89 (dt, J = 7.8, 3.5 Hz, 2H), 0.65 - 0.57 (m, 2H), 0.28 (dt, J = 5.7, 4.5 Hz, 2H).

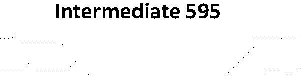

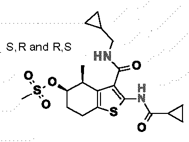

### [(4SR,5RS)-2-(cyclopropanecarbonylamino)-3-(cyclopropylmethylcarbamoyl)-4-methyl-4,5,6,7-tetrahydrobenzothiophen-5-yl] methanesulfonate

**Intermediate 594** (4.5g, 13 mmol) was dissolved in DCM (200 mL). TEA (2.7 mL, 19 mmol) was added and the mixture cooled with an ice bath. Methanesulfonyl chloride (1.1 mL, 14 mmol) was added dropwise and the reaction sealed and stirred at this temp for 150 min. Additional methanesulfonyl chloride (0.4 mL) was added at ambient temperature and reaction stirred at this temp overnight. The reaction mixture was concentrated in vacuo and triturated in Et₂O (100 mL) with sonication. The resulting solids were filtered and washed with ether, air drying under suction to afford the title compound (4.52 g, 82 % Yield). δ_{H} (300 MHz, Chloroform-d) 11.98 (s, 1H), 6.01 (m, 1H), 5.13 (ddd, J = 12.0, 5.3, 3.8 Hz, 1H), 3.51 - 3.17 (m, 3H), 3.10 (s, 3H), 2.92 - 2.82 (m, 2H), 2.31 - 2.05 (m, 2H), 1.70 - 1.57 (m, 1H), 1.33 (d, J = 6.9 Hz, 3H), 1.13 - 1.05 (m, 3H), 0.90 (dt, J = 8.0, 3.4 Hz, 2H), 0.66 - 0.51 (m, 2H), 0.29 (dt, J = 5.9, 4.6 Hz, 2H).

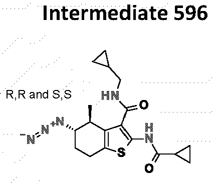

### (4SR,5SR)-5-azido-2-(cyclopropanecarbonylamino)-N-(cyclopropylmethyl)-4-methyl-4,5,6,7-tetrahydrobenzothiophene-3-carboxamide

**Intermediate 595** (2.5 g, 5.9 mmol) was dissolved in DMF (30.0 mL) and NaN₃ (0.96 g, 15 mmol) added. The reaction was sealed and stirred at 75 °C for 2.5 h. Additional NaN₃ (250 mg) was added and stirring resumed at 75°C for 4 h. The reaction was cooled and diluted with water (50 mL) and extracted with EtOAc (3 x 50 mL). The combined organic extracts were dried over Na₂SO₄ and concentrated *in vacuo.* The resulting beige powder was purified using column chromatography eluting with a 0-40% gradient of EtOAc in isohexane to afford after dry down the title compound (572 mg, 26% Yield). δ_{H} (300 MHz, Chloroform-*d*) 11.72 (s, 1H), 6.16 - 5.71 (m, 1H), 3.77 (ddd, *J* = 6.3, 3.8, 2.8 Hz, 1H), 3.48 - 3.16 (m, 2H), 3.00 - 2.64 (m, 3H), 2.19 - 1.95 (m, 2H), 1.72 - 1.55 (m, 1H), 1.31 (d, *J* = 7.0 Hz,3H), 1.17 - 0.99 (m, 3H), 0.89 (dt, *J* = 8.0, 3.4 Hz, 2H), 0.68 - 0.52 (m, 2H), 0.28 (dt, *J* = 5.9, 4.6 Hz, 2H).

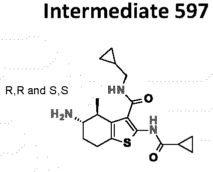

### (4SR,5SR)-5-amino-2-(cyclopropanecarbonylamino)-N-(cyclopropylmethyl)-4-methyl-4,5,6,7-tetrahydrobenzothiophene-3-carboxamide

**Intermediate 596** (0.57 g, 1.53 mmol) was dissolved in EtOH (2 mL) and the solution degassed with 2 cycles of vacuum/N₂ prior to addition of 10% w/w Pd on carbon (50 mg). The solution was degassed with a further cycle of vacuum/N₂ before regassing with 3 cycles of vacuum/H₂. The reaction was stirred under balloon pressure H₂ at ambient temperature for 3 h. The reaction mixture was degassed and filtered through celite, washing with EtOAc (50 mL). The solvents were removed *in vacuo* to afford a beige solid, which was dissolved in MeOH (12 mL) and absorbed on an SCX2 cartridge. The absorbed crude was washed with MeOH (40 mL) and the product eluted with 4 M NH₃ in MeOH (60 mL). Solvents were removed *in vacuo* to afford the title compound (498 mg, 94% Yield). δ_{H} (300 MHz, Chloroform-d) 11.94 (s, 1H), 6.26 (t, J = 4.8 Hz, 1H), 3.41 - 3.13 (m, 2H), 3.07 (dt, J = 5.7, 2.9 Hz, 1H), 2.86 - 2.60 (m, 3H), 2.09 - 1.94 (m, 1H), 1.82 - 1.58 (m, 4H), 1.27 (d, J = 6.9 Hz, 3H), 1.21 - 0.98 (m, 3H), 0.87 (dt, J = 8.0, 3.4 Hz, 2H), 0.63 - 0.50 (m, 2H), 0.27 (dt, J = 5.9, 4.5 Hz, 2H).

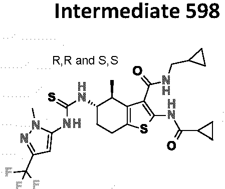

### (4SR,5SR)-2-(cyclopropanecarbonylamino)-N-(cyclopropylmethyl)-4-methyl-5-[[2-methyl-5-(trifluoromethyl)pyrazol-3-yl]carbamothioylamino]-4,5,6,7-tetrahydrobenzothiophene-3-carboxamide

Synthesised in the same manner as intermediate 168, using **intermediate 597** (75 mg, 0.22 mmol) and **intermediate 262** (49 mg, 0.24 mmol). The product was purified by column chromatography eluting with a 0-100% gradient of EtOAc in isohexane to afford the title compound (105 mg, 88 % Yield). δ_{H} (300 MHz, Chloroform-d) 12.59 (s, 1H), 8.41 (s, 1H), 7.24 (s, 1H), 6.54 (s, 1H), 6.25 (s, 1H), 4.69 - 4.55 (m, 1H), 3.77 (s, 3H), 3.49 - 3.32 (m, 2H), 3.17 (ddd, *J* = 13.7, 7.2, 4.3 Hz, 1H), 2.84 - 2.45 (m,2H), 2.28 - 2.07 (m, 2H), 1.75 - 1.64 (m, 1H), 1.37 (d, *J* = 7.0 Hz, 3H), 1.17 - 0.80 (m, 5H), 0.68 - 0.59 (m, 2H), 0.35 - 0.24 (m, 2H).

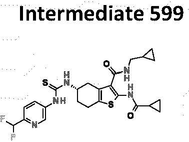

### (5S)-2-(cyclopropanecarbonylamino)-N-(cyclopropylmethyl)-5-[[6-(difluoromethyl)-3-pyridyl]carbamothioylamino]-4,5,6,7-tetrahydrobenzothiophene-3-carboxamide

A solution of 2-(difluoromethyl)-5-isothiocyanato-pyridine (200 mg, 1.07 mmol) in DCM (2 mL) was added dropwise to a solution of **intermediate 117** (286 mg, 0.86 mmol) in DCM (10 mL). The reaction was stirred at ambient temperature for 20 min and then concentrated in vacuo. The crude product was purified by column chromatography eluting with a 0-100% gradient of EtOAc in isohexane. Product-containing fractions were combined and concentrated to afford the title compound (403 mg, 72.2% Yield). δ_{H} (300 MHz, Chloroform-*d*) 12.01 (s, 1H), 8.91 (s, 1H), 8.52 (dd, *J* = 8.5, 2.5 Hz, 1H), 8.42 (d, *J* = 2.5 Hz, 1H), 7.56 (d, *J* = 8.5 Hz, 1H), 7.06 (d, *J* = 7.0 Hz, 1H), 6.57 (t, *J* = 55.6 Hz, 1H), 5.74 (t, *J* = 5.2 Hz, 1H), 5.10 - 4.87 (m, 1H), 3.38 - 3.17 (m, 2H), 3.06 (dd, *J* = 15.4, 4.5 Hz, 1H), 2.92 - 2.49 (m, 4H), 1.87 (dq, *J* = 13.7, 6.1, 3.7 Hz, 1H), 1.77 - 1.60 (m, 2H), 1.11 - 0.78 (m, 4H), 0.67 - 0.51 (m, 2H), 0.27 (dt, *J* = 5.9, 4.6 Hz, 2H).

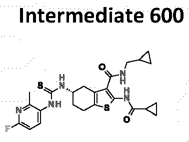

### (5S)-2-(cyclopropanecarbonylamino)-N-(cyclopropylmethyl)-5-[(6-fluoro-2-methyl-3-pyridyl)carbamothioylamino]-4,5,6,7-tetrahydrobenzothiophene-3-carboxamide

A solution of 6-fluoro-3-isothiocyanato-2-methyl-pyridine (200 mg, 1.19 mmol) in DCM (2 mL) was added dropwise to a solution of **Intermediate 117** (285 mg, 0.86 mmol) in DCM (10 mL). The reaction was stirred at ambient temperature for 20 min and then concentrated in vacuo. The crude product was purified by column chromatography eluting with a 0-100% gradient of EtOAc in isohexane. Product-containing fractions were combined and concentrated to afford the title compound (469 mg, 78 % Yield). δ_{H} (300 MHz, Chloroform-d) 11.99 (s, 1H), 7.98 (s, 1H), 7.94 - 7.82 (m, 1H), 6.76 (dd, *J* = 8.6, 3.5 Hz, 1H), 6.70 (d, *J* = 7.1 Hz, 1H), 5.78 (t, *J* = 5.2 Hz, 1H), 5.06 - 4.81 (m, 1H), 3.36 - 3.15 (m, 2H), 3.10 (dd, *J* = 15.2, 4.7 Hz, 1H), 2.83 - 2.61 (m, 3H), 2.49 - 2.40 (m, 1H), 2.37 (s, 3H), 1.98 - 1.84 (m, 1H), 1.65 (tt, *J* = 7.6, 4.7 Hz, 1H), 1.09 - 0.80 (m, 5H), 0.64 - 0.52 (m, 2H), 0.26 (dt, *J* = 5.9, 4.6 Hz, 2H).

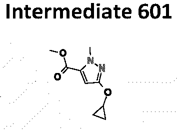

### methyl 5-(cyclopropoxy)-2-methyl-pyrazole-3-carboxylate

Cyclopropanol (413 mg, 6.40 mmol) and methyl 5-hydroxy-2-methyl-pyrazole-3-carboxylate (1.0 g, 6.40 mmol) were suspended in anhydrous toluene (10.0 mL) prior to addition of triphenylphosphine (1.69 g, 6.40 mmol) and then di-tert-butylazodicarboxylate (1.47 g, 6.40 mmol). The reaction was heated to 110 °C with stirring in a sealed vessel for 4.5 h, cooled & diluted with EtOAc (10 mL) and washed with water (10 mL). The organic layer was dried over Na₂SO₄ and concentrated in vacuo. The crude product was purified by column chromatography eluting with a 0-100% gradient of EtOAc in isohexane. Product-containing fractions were combined and concentrated to afford the title compound (730 mg, 58% Yield). δ_{H} (300 MHz, Chloroform-d) 6.31 (s, 1H), 4.04 (s, 3H), 3.91 (tt, J = 6.0, 3.0 Hz, 1H), 3.86 (s, 3H), 0.82 - 0.66 (m, 4H).

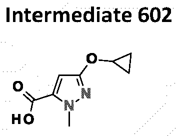

### 5-(cyclopropoxy)-2-methyl-pyrazole-3-carboxylic acid

**Intermediate 601** (730 mg, 3.7 mmol) was dissolved in MeOH (15 mL) and 2 M aqueous NaOH (3.7 mL). The reaction was stirred at 50 °C for 1.25 h. The reaction mixture was concentrated to remove MeOH, acidified to pH^{~}1 with 2 N HCl and extracted with EtOAc (4 x 15 mL) The organic extracts were dried over Na₂SO₄ and concentrated in vacuo and concentrated to afford the title compound (730 mg, 97% Yield) as a colourless powder. δ_{H} (300 MHz, DMSO-*d*6) 13.20 (s, 1H), 6.31 (s, 1H), 3.98 - 3.91 (m, 4H), 0.76 - 0.56 (m, 4H).

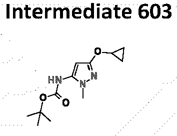

### tert-butyl N-[5-(cyclopropoxy)-2-methyl-pyrazol-3-yl]carbamate

**Intermediate 602** (730 mg, 4.0 mmol) was dissolved in THF (10 mL) and TEA (1.68 mL, 12.0 mmol) was added and the solution cooled in an ice bath. DPPA (1.65 g, 6.01 mmol) was added dropwise and the reaction stirred at this temp for 0.25 h, then allowed to warm to ambient temperature for 1 h. Tert-butanol (10 mL, 110 mmol) was added and the reaction heated to 85 °C for 6 h. The reaction mixture was concentrated in vacuo and the crude product purified by column chromatography eluting with a 0-100% gradient of EtOAc in isohexane. Product-containing fractions were combined and concentrated to afford the title compound (670 mg, 66% Yield). δ_{H} (300 MHz, Chloroform-d) 6.24 (s, 1H), 5.73 (s, 1H), 3.90 (tt, J = 6.0, 3.0 Hz, 1H), 3.60 (s, 3H), 1.50 (s, 9H), 0.81 - 0.64 (m, 4H).

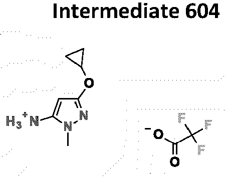

### [5-(cyclopropoxy)-2-methyl-pyrazol-3-yl]ammonium 2,2,2-trifluoroacetate

**Intermediate 603** (530 mg, 2.1 mmol) was dissolved in 20% TFA/DCM (10mL) and stirred at ambient temperature for 2 h The reaction mixture was concentrated in vacuo and then azeotroped with toluene and concentrated in vacuo to afford the title compound as a brown oil. Yield not recorded. δ_{H} (300 MHz, Methanol-d4) 4.15 - 4.07 (m, 1H), 3.53 (s, 3H), 0.88 - 0.81 (m, 4H).

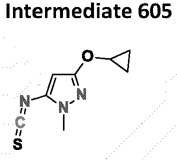

### 3-(cyclopropoxy)-5-isothiocyanato-1-methyl-pyrazole

DIPEA (600 mg, 4.6 mmol) was added to a solution of **intermediate 604** (560 mg, 2.1 mmol) and 1,1'-thiocarbonyldiimidazole (470 mg, 2.5 mmol) in DCM (10 mL) under N₂ and the reaction stirred for 90 min. The crude product was used directly in the next stage without any purification.

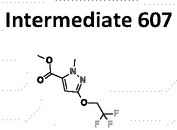

### methyl 2-methyl-5-(2,2,2-trifluoroethoxy)pyrazole-3-carboxylate

methyl 5-hydroxy-2-methyl-pyrazole-3-carboxylate (2.7 g, 17.0 mmol) and 2-iodo-1,1,1-trifluoroethane (2.1 mL, 21 mmol) were dissolved in anhydrous DMF (20 mL). The reaction was cooled in an ice bath prior to addition of sodium hydride (1.20 g, 29 mmol, 60 mass% in mineral oil) portionwise over 10 min. Upon completion of addition, the reaction was sealed and heated to 100 °C with stirring for 2 h. The reaction was diluted with water (20 mL) and extracted with EtOAc (3 x 20 mL). The organic layer was dried over Na₂SO₄ and concentrated in vacuo. The crude product was purified by column chromatography eluting with a 0-50% gradient of EtOAc in isohexane. Product-containing fractions were combined and concentrated to afford the title compound (660 mg, 16% Yield). δ_{H} (300 MHz, Chloroform-d) 6.25 (s, 1H), 4.55 (q, *J* = 8.4 Hz, 2H), 4.03 (s, 3H), 3.87 (s, 3H).

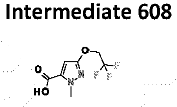

### 2-methyl-5-(2,2,2-trifluoroethoxy)pyrazole-3-carboxylic acid

**Intermediate 607** (660 mg, 2.77 mmol) was dissolved in MeOH (13 mL) and 2 M aqueous sodium hydroxide (3.4 mL). The reaction was stirred at 50 °C for 1.25 h. The reaction mixture was acidified to pH 1 with 2 M HCl and concentrated to remove most of the MeOH. The resulting aqueous was extracted with EtOAc (3 x 20mL) and the combined organic extracts dried over Na₂SO₄ and concentrated in vacuo to afford the title compound (559 mg, 81 % Yield). δ_{H} (300 MHz, DMSO-d6) 13.52 (s, 1H), 6.38 (s, 1H), 4.79 (q, *J* = 9.0 Hz, 2H), 3.94 (s, 3H).

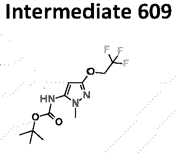

### tert-butyl N-[2-methyl-5-(2,2,2-trifluoroethoxy)pyrazol-3-yl]carbamate

**Intermediate 608** (559 mg, 2.49 mmol) was dissolved in THF (10.0 mL) and TEA (1.0 mL, 7.49 mmol) was added and the solution cooled in an ice bath. DPPA (0.81 mL, 3.74 mmol) was added dropwise and the reaction stirred at this temperature for 0.25 h, then allowed to warm to ambient temperature for 1. Tert-butanol (10 mL) was added and the reaction heated to 85 °C for 3 h. The reaction mixture was concentrated in vacuo and purified by column chromatography eluting with a 0-100% gradient of EtOAc in isohexane. Product-containing fractions were combined and concentrated to afford the title compound (530 mg, 72 % Yield). δ_{H} (300 MHz, Chloroform-d) 6.27 (s, 1H), 5.66 (s, 1H), 4.51 (q, J = 8.4 Hz, 2H), 3.58 (s, 3H), 1.50 (s, 9H).

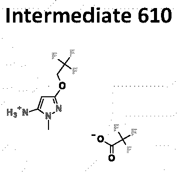

### [2-methyl-5-(2,2,2-trifluoroethoxy)pyrazol-3-yl]ammonium 2,2,2-trifluoroacetate

**Intermediate 609** (530 mg, 1.8 mmol) was dissolved in TFA/DCM (10 mL) and stirred at ambient temperature for 2 h. The reaction mixture was concentrated in vacuo and then azeotroped with toluene and concentrated in vacuo to afford the title compound as a brown oil. Yield not recorded. δ_{H} (300 MHz, Methanol-d4) 4.68 (q, J = 8.4 Hz, 2H), 3.51 (s, 3H).

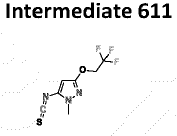

### 5-isothiocyanato-1-methyl-3-(2,2,2-trifluoroethoxy)pyrazole

DIPEA (510 mg, 4.0 mmol) was added to a solution of **intermediate 610** (560 mg, 1.8 mmol) and 1,1'-thiocarbonyldiimidazole (410 mg, 2.2 mmol) in DCM (10 mL) under N₂. The reaction was stirred for 90 min. The crude product was used directly in the next stage without any purification.

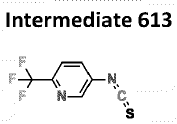

### 5-isothiocyanato-2-(trifluoromethyl)pyridine

5-Amino-2-(trifluoromethyl)pyridine (0.2 g) was dissolved in a solution of 1,1'-thiocarbonyldiimidazole (284 mg, 1.59 mmol) in DCM (2 mL) and 4 N HCl in dioxane (0.35 mL) was added. The reaction was stirred at ambient temperature for 2.5 h, filtered and concentrated in vacuo to afford the crude title compound, which was carried through to the next stage without purification.

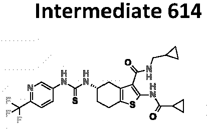

### (5S)-2-(cyclopropanecarbonylamino)-N-(cyclopropylmethyl)-5-[[6-(trifluoromethyl)-3-pyridyl]carbamothioylamino]-4,5,6,7-tetrahydrobenzothiophene-3-carboxamide

**Intermediate 613** (0.165 mmol) was dissolved in DCM (2 mL) and added to a solution of **intermediate 117** (50 mg, 0.15 mmol) in DCM (0.5 mL). The reaction was stirred at ambient temperature for 45 min and then absorbed directly silica and purified by column chromatography eluting with a 0-100% gradient of EtOAc in isohexane, followed by 0-20% MeOH in EtOAc. Product-containing fractions were combined and concentrated to afford the title compound (78 mg, 97%). δ_{H} (300 MHz, Chloroform-d) 12.02 (s, 1H), 8.99 (s, 1H), 8.78 - 8.65 (m, 1H), 8.41 (d, *J* = 2.5 Hz, 1H), 7.62 (d, *J* = 8.6 Hz, 1H), 7.06 (d, *J* = 6.9 Hz, 1H), 5.71 (t, *J* = 5.2 Hz, 1H), 5.00 (s, 1H), 3.40 - 3.15 (m,2H), 3.15 - 2.91 (m, 1H), 2.89 - 2.57 (m, 4H), 1.88 (q, *J* = 11.7, 11.2 Hz, 1H), 1.76 - 1.64 (m, 1H), 1.11 - 0.87 (m, 5H), 0.67 - 0.55 (m, 2H), 0.34 - 0.20 (m, 2H).

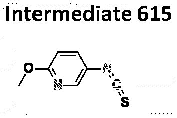

### 5-isothiocyanato-2-methoxy-pyridine

The title compound was prepared according to the same procedure described for intermediate 613 using 5-amino-2-methoxypyridine (0.2 g), 1,1'-thiocarbonyldiimidazole (284 mg, 1.59 mmol) in DCM (2 mL) and 4 N HCl in dioxane (0.35 mL). The crude product was carried through to the next stage without purification.

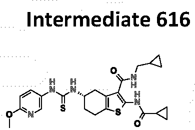

### (5S)-2-(cyclopropanecarbonylamino)-N-(cyclopropylmethyl)-5-[(6-methoxy-3-pyridyl)carbamothioylamino]-4,5,6,7-tetrahydrobenzothiophene-3-carboxamide

**Intermediate 117** (50 mg, 0.15 mmol) and **Intermediate 615** (0.15 mmol) were combined in DCM (2.5 mL) using to the same procedure described for intermediate 614. The crude product was purified by column chromatography eluting with a 0-100% gradient of EtOAc in isohexane, followed by 0-20% MeOH in EtOAc to afford, after dry down, the title compound (80 mg, 100%). δ_{H} (300 MHz, Chloroform-d) 12.10 (s, 1H), 8.02 (d, *J* = 2.7 Hz, 1H), 7.55 - 7.47 (m, 1H), 7.44 (dd, *J* = 8.8, 2.7 Hz, 1H), 6.77 (d, *J* = 8.7 Hz, 1H), 6.00 - 5.75 (m, 2H), 5.04 - 4.75 (m, 1H), 3.93 (s, 3H), 3.28 (dd, *J* = 7.2, 5.2 Hz, 3H), 2.93 - 2.52 (m, 3H), 2.08 - 2.01 (m, 2H), 1.65 (td, *J* = 7.9, 4.0 Hz, 1H), 1.13 - 1.02 (m, 3H), 0.91 (dt, *J* = 8.0, 3.4 Hz, 2H), 0.66 - 0.53 (m, 2H), 0.36 - 0.16 (m, 2H).

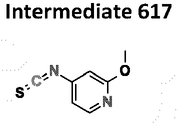

### 4-isothiocyanato-2-methoxy-pyridine

The title compound was prepared according to the same procedure described for intermediate 613 using 4-amino-2-methoxypyridine (0.2 g), 1,1'-thiocarbonyldiimidazole (1.59 mmol, 284 mg) in DCM (2 mL) and 4 N HCl in dioxane (0.35 mL). The crude product was carried through to the next stage without purification.

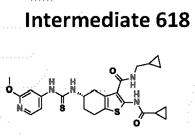

### (5S)-2-(cyclopropanecarbonylamino)-N-(cyclopropylmethyl)-5-[(2-methoxy-4-pyridyl)carbamothioylamino]-4,5,6,7-tetrahydrobenzothiophene-3-carboxamide

**Intermediate 117** (50 mg, 0.15 mmol) and **intermediate 617** (0.15 mmol) were combined in DCM (2.5 mL) according to the same procedure described for intermediate 614. The crude product was purified by column chromatography eluting with a 0-100% gradient of EtOAc in isohexane, followed by 0-20% MeOH in EtOAc to afford, after dry down, the title compound (11 mg, 15% Yield). δ_{H} (300 MHz, Chloroform-d) 12.09 (s, 1H), 8.04 (d, *J* = 5.5 Hz, 1H), 6.73 (s, 3H), 5.86 - 5.76 (m, 1H), 5.05 - 4.89 (m, 1H), 3.90 (s, 3H), 3.36 - 3.23 (m, 2H), 3.23 - 3.07 (m, 1H), 2.97 - 2.64 (m, 4H), 2.37 - 2.11 (m, 1H), 1.67 (tt, *J* = 8.1, 4.3 Hz, 1H), 1.15 - 1.00 (m, 3H), 0.97 - 0.81 (m, 3H), 0.67 - 0.53 (m, 2H), 0.35 - 0.21 (m, 2H).

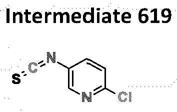

### 2-chloro-5-isothiocyanato-pyridine

The title compound was prepared according to the same procedure described for intermediate 613 using 5-amino-2-chloropyridine (0.2 g), 1,1'-thiocarbonyldiimidazole (1.59 mmol, 284 mg) in DCM (2 mL) and 4 N HCl in dioxane (0.35 mL). The crude product was carried through to the next stage without purification.

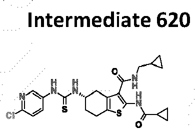

### (5S)-5-[(6-chloro-3-pyridyl)carbamothioylamino]-2-(cyclopropanecarbonylamino)-N-(cyclopropylmethyl)-4,5,6,7-tetrahydrobenzothiophene-3-carboxamide

**Intermediate117** (50 mg, 0.15 mmol) and **intermediate 619** (0.15mmol) were combined in DCM (2.5 mL) according to the same procedure described for intermediate 614. The crude product was purified by column chromatography eluting with a 0-100% gradient of EtOAc in isohexane, followed by 0-20% MeOH in EtOAc to afford, after dry down, the title compound (62 mg, 82%). δ_{H} 300 MHz, Chloroform-*d*) 12.01 (s, 1H), 8.52 (s, 1H), 8.22 - 8.15 (m, 2H), 7.28 (dd, *J* = 8.6, 1.0 Hz, 1H), 6.86 (d, *J* = 7.2 Hz, 1H), 5.75 (t, *J* = 5.2 Hz, 1H), 4.98 (s, 1H), 3.36 - 3.16 (m, 2H), 3.15 - 3.00 (m, 1H), 2.87 - 2.64 (m, 3H), 2.59 - 2.42 (m, 1H), 1.99 - 1.83 (m, 1H), 1.72 - 1.61 (m, 1H), 1.14 - 0.83 (m, 5H), 0.67 - 0.54 (m, 2H), 0.27 (dt, *J* = 5.9, 4.6 Hz, 2H).

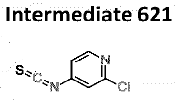

### 2-chloro-4-isothiocyanato-pyridine

The title compound was prepared according to the same procedure described for intermediate 613 using 4-amino-2-chloropyridine (0.2 g), 1,1'-thiocarbonyldiimidazole (1.59 mmol, 284 mg) in DCM (2 mL) and 4 N HCl in dioxane (0.35 mL). The crude product was carried through to the next stage without purification.

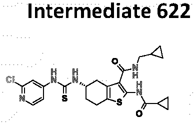

### (5S)-5-[(2-chloro-4-pyridyl)carbamothioylamino]-2-(cyclopropanecarbonylamino)-N-(cyclopropylmethyl)-4,5,6,7-tetrahydrobenzothiophene-3-carboxamide

**Intermediate 117** (50 mg, 0.15 mmol) and **intermediate 621** (0.15 mmol) were combined in DCM (2.5 mL) according to the same procedure described for intermediate 614. The crude product was purified by column chromatography eluting with a 0-100% gradient of EtOAc in isohexane, followed by 0-20% MeOH in EtOAc to afford, after dry down, the title compound (59 mg, 78%). δ_{H} (300 MHz, Chloroform-d) 12.08 (s, 1H), 9.04 (s, 1H), 8.21 - 8.06 (m, 1H), 7.75 - 7.66 (m, 1H), 7.51 (dd, *J* = 5.7, 2.1 Hz, 1H), 7.11 (d, *J* = 7.0 Hz, 1H), 5.69 (t, *J* = 5.2 Hz, 1H), 5.06 - 4.82 (m, 1H), 3.45 - 3.14 (m, 2H), 3.14 - 2.96 (m, 1H), 2.86 - 2.56 (m, 4H), 1.96 - 1.78 (m, 1H), 1.79 - 1.66 (m, 1H), 1.13 - 0.80 (m, 5H), 0.69 - 0.55 (m, 2H), 0.28 (dt, *J* = 5.9, 4.6 Hz, 2H).

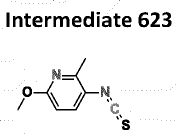

### 3-isothiocyanato-6-methoxy-2-methyl-pyridine

The title compound was prepared according to the same procedure described for intermediate 613 using 3-amino-6-methoxy-2-picoline (0.2 g), 1,1'-thiocarbonyldiimidazole (1.59 mmol, 284 mg) in DCM (2 mL) and 4N HCl in dioxane (0.35 mL). The crude product was carried through to the next stage without purification.

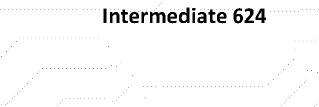

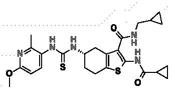

### (5S)-2-(cyclopropanecarbonylamino)-N-(cyclopropylmethyl)-5-[(6-methoxy-2-methyl-3-pyridyl)carbamothioylamino]-4,5,6,7-tetrahydrobenzothiophene-3-carboxamide

**Intermediate 117** (50 mg, 0.15 mmol) and **intermediate 623** (0.15 mmol) were combined in DCM (2.5 mL) according to the same procedure described for intermediate 614. The crude product was purified by column chromatography eluting with a 0-100% gradient of EtOAc in isohexane, followed by 0-20% MeOH in EtOAc to afford, after dry down, the title compound (47 mg, 61% Yield). δ_{H} (300 MHz, Chloroform-*d*) 12.10 (s, 1H), 7.34 - 7.27 (m, 2H), 6.59 (d, *J* = 8.5 Hz, 1H), 5.83 (t, *J* = 5.2 Hz, 1H), 5.59 (d, *J* = 8.0 Hz, 1H), 4.95 - 4.79 (m, 1H), 3.92 (s, 3H), 3.32 - 3.19 (m, 3H), 2.91 - 2.50 (m, 3H), 2.36 (s, 3H), 2.11 - 1.85 (m, 2H), 1.71 - 1.60 (m, 1H), 1.13 - 0.99 (m, 3H), 0.90 (dt, *J* = 8.0, 3.5 Hz, 2H), 0.64 - 0.54 (m, 2H), 0.27 (dt, *J* = 5.9, 4.6 Hz, 2H).

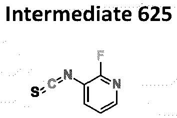

### 2-fluoro-3-isothiocyanato-pyridine

The title compound was prepared according to the same procedure described for intermediate 613 using 3-amino-2-fluoropyridine (0.2 g), 1,1'-thiocarbonyldiimidazole (1.59 mmol, 284 mg) in DCM (2 mL) and 4 N HCl in dioxane (0.35 mL). The crude product was carried through to the next stage without purification.

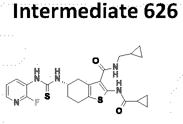

### (55)-2-(cyclopropanecarbonylamino)-N-(cyclopropylmethyl)-5-[(2-fluoro-3-pyridyl)carbamothioylamino]-4,5,6,7-tetrahydrobenzothiophene-3-carboxamide

**Intermediate 117** (50 mg, 0.15 mmol) and **intermediate 625** (0.15 mmol) were combined in DCM (2.5 mL) according to the same procedure described for intermediate 614. The crude product was purified by column chromatography eluting with a 0-100% gradient of EtOAc in isohexane, followed by 0-20% MeOH in EtOAc to afford, after dry down, the title compound (25 mg, 34% Yield). δ_{H} (300 MHz, Chloroform-*d*) 12.00 (s, 1H), 8.83 (t, *J* = 9.4 Hz, 1H), 8.50 - 8.32 (m, 1H), 7.89 (d, *J* = 4.9 Hz, 1H), 7.24 - 7.13 (m, 2H), 5.73 (t, *J* = 5.2 Hz, 1H), 5.10 - 4.85 (m, 1H), 3.38 - 3.15 (m, 2H), 3.16 - 3.00 (m, 1H), 2.93 - 2.66 (m, 3H), 2.63 - 2.49 (m, 1H), 2.00 - 1.83 (m, 1H), 1.81 - 1.48 (m, 1H), 1.13 - 0.98 (m, 3H), 0.98 - 0.77 (m, 2H), 0.67 - 0.56 (m, 2H), 0.37 - 0.23 (m, 2H).

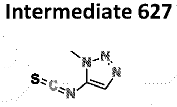

### 5-isothiocyanato-1-methyl-triazole

The title compound was prepared according to the same procedure described for intermediate 613 using 5-amino-1-methyl-1,2,3-triazole (240 mg, 2.32 mmol), 1,1'-thiocarbonyldiimidazole (479 mg, 2.56 mmol), 4 M HCl in 1,4-dioxane (1.2 mL) in DCM (7 mL). The crude product was purified by column chromatogrpahy eluting with a 0-100% gradient of EtOAc in isohexane. Product-containing fractions were combined and concentrated to afford the title compound (72 mg, 22 % Yield). δ_{H} (300 MHz, Chloroform-d) 7.65 (s, 1H), 3.99 (s, 3H).

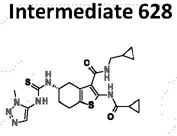

### (5S)-2-(cyclopropanecarbonylamino)-N-(cyclopropylmethyl)-5-[(3-methyltriazol-4-yl)carbamothioylamino]-4,5,6,7-tetrahydrobenzothiophene-3-carboxamide

**Intermediate 117** (100 mg, 0.30 mmol) was added to a stirred solution of **intermediate 627** (72 mg, 0.51 mmol) in DCM (12 mL) and the reaction stirred for 45 min at ambient temperature. The solvent was removed in vacuo and the crude product purified by column chromatography eluting with a 0-100% gradient of EtOAc in isohexane followed by 0-20% MeOH in EtOAc to afford, after dry down, the title compound (213 mg, 87 % Yield) as a beige foam. δ_{H} (300 MHz, Chloroform-*d*) 12.08 (s, 1H), 8.60 (s, 1H), 7.69 (s, 1H), 7.30 (d, *J* = 7.5 Hz, 1H), 5.87 (t, *J* = 5.3 Hz, 1H), 5.03 - 4.84 (m, 1H), 3.86 (s, 3H), 3.36 - 3.18 (m, 2H), 3.12 (dd, *J* = 15.3, 4.7 Hz, 1H), 2.84 (dd, *J* = 15.5, 4.0 Hz, 1H), 2.79 - 2.59 (m, 2H), 2.45 - 2.30 (m, 1H), 2.01 - 1.88 (m, 1H), 1.77 - 1.61 (m, 2H), 1.13 - 0.87 (m, 4H), 0.64 - 0.53 (m, 2H), 0.27 (dt, *J* = 6.1, 4.6 Hz, 2H).

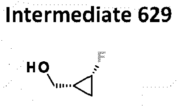

### [(1R,2R)-2-fluorocyclopropyllmethanol

To a stirred suspension of (1R,2R)-2-fluorocyclopropanecarboxylic acid (5.00 g, 48 mmol) in THF (25 mL) in an ice-bath under N₂ was added lithium aluminum hydride (32 mL, 64 mmol) dropwise over 15-20 min. [Note: Effervescence observed upon immediate addition and a white precipitate formed after 8 mL of the reagent were added]. Reaction mixture slowly warmed to r.t. o/n. The reaction mixture was then cooled in an ice-bath and water (3 mL) was added followed by 2 M aqueous sodium hydroxide (3 mL). Diethyl ether (30 mL) was then added and the reaction mixture stirred for 1 h at room temperature [Note: quench was exothermic and formed a thick white copious precipitate which at times was not stirring]. The reaction mixture was filtered and washed with diethyl ether (100 mL). The solution was then concentrated under reduced pressure to remove most of the solvent. The residual solution was dried (MgSO₄) and concentrated in vacuo to give the title compound in THF (56% wt) (9 g, 92 % Yield). δ_{H} (300 MHz, d-Chloroform) 4.73 (dtd, *J* = 65.1, 5.9, 2.7 Hz, 1H), 3.94 (ddd, *J* = 11.7, 5.9, 1.4 Hz, 1H), 3.64 (ddd, *J* = 11.7, 8.7, 1.1 Hz, 1H), 1.59 (ddd, *J* = 7.6, 4.8, 2.7 Hz, 1H), 1.33 - 1.16 (m, 1H), 0.94 - 0.68 (m, 2H).

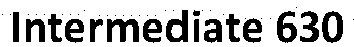

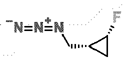

### (1R,2R)-1-(azidomethyl)-2-fluoro-cyclopropane

To a stirred solution of **intermediate 629** (8.0 g, 39 mmol) in DCM (30 mL, 468 mmol) in an ice-bath were added triethylamine (8 mL, 57.4 mmol) and methansulfonyl chloride (3.6 mL, 46 mmol). The ice-bath was removed and the reaction mixture allowed to stir at room temperature. A second portion of DCM (10 mL) was added. After 1 hour the reaction mixture was diluted with aqueous 0.2 N HCl (50 mL) and the aqueous phase extracted with DCM (2 x 10 mL). The organics were combined dried and concentrated in vacuo. The residue was then dissolved in anhydrous DMF (20 mL, 259 mmol) at room temperature. Sodium azide (3.0 g, 46 mmol) was added and the reaction mixture stirred at room temperature for 48 hours. The reaction mixture was diluted with diethyl ether (50 mL) and washed with brine (200 mL). The aqueous layer was extracted with diethyl ether (50 mL) and the combined organics dried and concentrated in vacuo. DMF (10 mL) was then added along with sodium azide (1g, 15 mmol). The reaction mixture was stirred a r.t. for 72 hours. Diethyl ether (50 mL) was added and the solution washed with brine (200 mL). The aqueous layer was extracted with diethyl ether (50 mL) and the combined organics dried and concentrated in vacuo to give the title compound (6.3 g, 84% Yield). δ_{H} (300 MHz, d-Chloroform) 4.74 (dtd, J = 64.6, 5.9, 2.7 Hz, 1H), 3.36 (td, J = 13.7, 13.2, 7.5 Hz, 2H), 1.21 (t, J = 7.0 Hz, 1H), 1.02 - 0.68 (m, 2H).

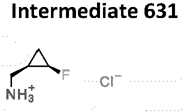

### [(1R,2R)-2-fluorocyclopropylimethylammonium chloride

To a solution of **intermediate 630** (6.33 g, 33 mmol) in methanol (10 mL) was added a slurry of palladium on carbon (200 mg) in MeOH (4 mL). The reaction mixture was subjected to vacuum and backfilled with H₂ (twice). The reaction mixture was then stirred at room temperature overnight. A second portion of palladium on carbon (50 mg) was added and the reaction mixture stirred under an atmosphere of H₂ for 1 hour. The reaction mixture was then filtered through a pre-packed cartridge of celite (1 g) eluting with MeOH. A solution of 1 M HCl in diethyl ether (50 mL) was then added and the resulting solution concentrated in vacuo to give the title compound (3.95 g, 92% Yield). δ_{H} (400 MHz, d-MeOH) 4.84 (dtd, *J* = 64.8, 5.9, 2.7 Hz, 1H), 3.14 (dd, *J* = 13.4, 7.3 Hz, 1H), 3.04 (dd, *J* = 13.4, 8.2 Hz, 1H), 1.30 - 1.16 (m, 1H), 1.02 (dddd, *J* = 12.5, 9.8, 7.4, 5.9 Hz, 1H), 0.88 (dtd, *J* = 23.4, 7.3, 2.7 Hz, 1H).

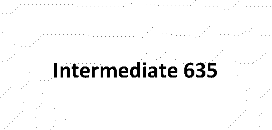

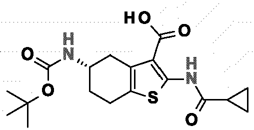

### (55)-5-(tert-butoxycarbonylamino)-2-(cyclopropanecarbonylamino)-4,5,6,7-tetrahydrobenzothiophene-3-carboxylic acid

**Intermediate** 26 was separated by chiral SFC (using an IG column 250 x 50 mm, 20 µm, eluting with isocratic 25 % MeOH in supercritical CO₂, 30 °C, 360 mL/min, run time of 15 min) to give the title compound. Chiral SFC* RT = 3.36 minutes.

* Chiral analysis was preformed using SFC, Lux Cellulose-4 150 x 4.6 mm, 3 µm, eluting with isocratic 100 % MeOH with 0.1% DEA in supercritical CO₂, 1.5 mL/min, 8 min run time, 30 °C, 100 bar.

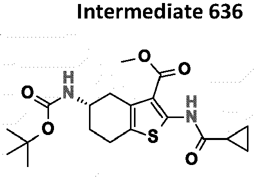

### methyl (55)-5-(tert-butoxycarbonylamino)-2-(cyclopropanecarbonylamino)-4,5,6,7-tetrahydrobenzothiophene-3-carboxylate

**Intermediate 635** (2.5 g, 6.6 mmol) was dissolved in MeOH (30 mL) and toluene (60 mL). 2 M (trimethylsilyl)diazomethane in hexanes (5.3 mL, 11 mmol) was added in portions to the stirred solution at ambient temperature and the reaction stirred for 15 min. 2 M (trimethylsilyl)diazomethane in hexanes (1.0 mL, 11 mmol) was added and the reaction stirred for a further 10 min. AcOH was added until effervescence ceased and the volatiles evaporated to afford a brown solid, which was recrystallised from IPA to afford the title compound (2.10g, 81% Yield). δ_{H} (300 MHz, Chloroform-*d*) 11.41 (s, 1H), 4.71 - 4.50 (m, 1H), 4.07 - 3.91 (m, 1H), 3.87 (s, 3H), 3.14 (dd, *J* = 17.2, 5.4 Hz, 1H), 2.78 - 2.68 (m, 2H), 2.59 (dd, *J* = 17.2, 7.3 Hz, 1H), 2.10 - 1.96 (m, 1H),1.86 - 1.72 (m, 1H), 1.67 (tt, *J* = 7.9, 4.6 Hz, 1H), 1.17 - 1.10 (m, 2H), 0.94 (dt, *J* = 8.0, 3.4 Hz, 2H)

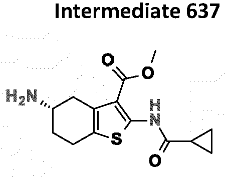

### methyl (55)-5-amino-2-(cyclopropanecarbonylamino)-4,5,6,7-tetrahydrobenzothiophene-3-carboxylate

**Intermediate 636** (2.1 g, 5.32 mmol) was dissolved in DCM (30 mL) and trifluoroacetic acid (6 mL, 79 mmol) was added. The reaction was stirred at ambient temperature for 45 min. The reaction mixture was concentrated in vacuo and taken back up in MeOH (5 mL). Upon dissolution in MeOH, the product crashed out of solution. The solvent was removed under vacuum and the resulting residue suspended in EtOAc (40 mL). The solution was stirred vigorously during addition of saturated aqueous K₂CO₃ (15 mL). After 15 min, the organic phase was separated. The aqueous layer was extracted with EtOAc (20 mL) and the combined organic extracts dried (Na₂SO₄). The solvent was then removed in vacuo to afford the title compound (2.5 g, quantitative). δ_{H} (400 MHz, DMSO-d6) 11.12 (s, 1H), 3.83 (s, 3H), 3.03 - 2.91 (m, 2H), 2.73 - 2.52 (m, 2H), 2.35 - 2.23 (m, 1H), 2.03 (tt, J = 7.5, 4.9 Hz, 1H), 1.91 - 1.81 (m, 3H), 1.53 - 1.37 (m, 1H), 0.96 - 0.82 (m, 4H).

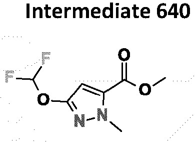

### methyl 5-(difluoromethoxy)-2-methyl-pyrazole-3-carboxylate

Methyl 3-hydroxy-1-methylpyrazole-5-carboxylate (10 g, 60.8 mmol) was dissolved in DMF (200 mL). sodium chlorodifluoroacetate (23.2 g, 152 mmol) was added followed by potassium carbonate (25.2 g, 182 mmol). The reaction mixture was heated to 80 °C with stirring under N₂ overnight. The reaction was cooled & poured into water (200 mL). The mixture was then extracted with EtOAc (3 x 200 mL) and the combined organic extracts dried (Na₂SO₄) and concentrated under reduced pressure. The residue was purified by column chromatography eluting with a gradient of 0-25% EtOAc in iso-hexanes to give the title compound (6.41 g, 51 % Yield). δ_{H} (300 MHz, Chloroform-d) 6.76 (t, J = 73.1 Hz, 1H), 6.41 (s, 1H), 4.08 (s, 3H), 3.88 (s, 3H).

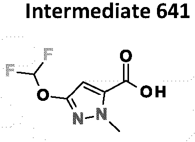

### 5-(difluoromethoxy)-2-methyl-pyrazole-3-carboxylic acid

**Intermediate 640** (6.4 g, 31 mmol) was diluted in methanol (130 mL) and 2 M aqueous sodium hydroxide (31 mL, 62 mmol). The reaction was stirred at 50 °C for 1.25 h. The reaction mixture was cooled to room temperature and acidified to pH 1 with 2 M HCl (30 mL). The reaction mixture was concentrated under vacuum to remove most of the MeOH. The resulting aqueous solution was extracted with EtOAc (3 x 60 mL) and the combined organic extracts dried (Na₂SO₄) and concentrated in vacuo to afford the title compound (5.87 g, 98% Yield). δ_{H} (300 MHz, DMSO-d6) 13.70 (s, 1H), 7.26 (t, J = 73.2 Hz, 1H), 6.51 (s, 1H), 3.99 (s, 3H).

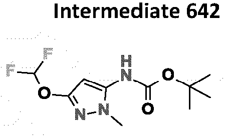

### tert-butyl N-[5-(difluoromethoxy)-2-methyl-pyrazol-3-yl]carbamate

**Intermediate 641** (6.00 g, 31.2 mmol) was dissolved in tert-butyl alcohol (100 mL, 1100 mmol) and triethylamine (13.1 mL, 93.7 mmol) was added. The reaction mixture was cooled using an ice bath and diphenylphosphoryl azide (10.1 mL, 46.8 mmol) added dropwise. The reaction mixture was stirred at 0 °C for 30 minutes. The ice bath was then removed and the reaction mixture heated to 85 °C for 3 h. The reaction was cooled to room temperature and poured into water (150 mL). The mixture was extracted with EtOAc (3 x 150 mL) and the combined organic extracts dried (Na₂SO₄) and concentrated in vacuo. The resulting residue was purified by column chromatography eluting with a gradient of 0-40% EtOAc in iso-hexanes to give the title compound (6.1 g, 74 % Yield). δ_{H} (300 MHz, Chloroform-d) 6.75 (t, J = 73.5 Hz, 1H), 6.37 (s, 1H), 5.82 (s, 1H), 3.63 (s, 3H), 1.50 (s, 9H).

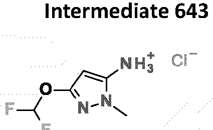

### [5-(difluoromethoxy)-2-methyl-pyrazol-3-yl]ammonium chloride

**Intermediate 642** (3.0 g, 11.4 mmol) was suspended in 1,4-dioxane (25 mL) and hydrochloric acid (4.0 mol/L) in dioxane (5.7 mL, 23 mmol) was added. The suspension was stirred for 45 min. Methanol (5 mL) was added and the reaction mixture stirred for 5 h at room temperature. The reaction mixture was then stored at -21 °C for 2 days. Reaction mixture was defrosted and a second portion of hydrochloric acid (4.0 mol/L) in dioxane (5 mL, 20 mmol) was added and stirred reaction mixture heated to 45 °C for 90 min. The reaction mixture was then cooled to room temperature and concentrated in vacuo. The resulting oil was stirred vigorously with diethyl ether (25 mL) for 15 min. The ether layer was then decanted and the residual oil concentrated under reduced pressure to give the title compound (2.55 g, quantitative). δ_{H} (300 MHz, DMSO-d6) 8.00 (s, 3H), 7.13 (t, J = 73.9 Hz, 1H), 5.09 (s, 1H), 3.44 (s, 3H).

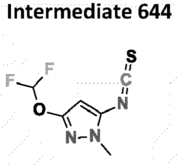

### 3-(difluoromethoxy)-5-isothiocyanato-1-methyl-pyrazole

A solution of **Intermediate 643** (2.5 g, 15 mmol) in water (25 mL) was added dropwise to a vigorously stirred mixture of sodium carbonate (8.1 g, 77 mmol), DCM (50 mL), water (25 mL) and thiophosgene (3.5 g, 31 mmol) at 0 °C. After the addition was complete, the reaction was stirred at this temperature for 10 min, the ice bath was then removed and the reaction mixture was stirred at room temperature for 45 min. The reaction mixture was partitioned with phase separator cartridge. The aqueous layer was then extracted with DCM (30 mL). The combined organic extracts were concentrated under reduced pressure and purified by column chromatography eluting with a gradient of 0-40 % ethyl acetate in iso-hexanes to afford the title compound (1.37 g, 44 % Yield). δ_{H} (300 MHz, Chloroform-d) 6.76 (t, J = 73.0 Hz, 1H), 5.87 (s, 1H), 3.70 (s, 3H).

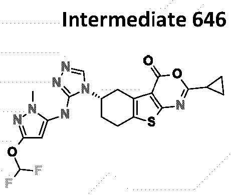

### (6S)-2-cyclopropyl-6-[3-[[5-(difluoromethoxy)-2-methyl-pyrazol-3-yl]amino]-1,2,4-triazol-4-yl]-5,6,7,8-tetrahydrobenzothiopheno[2,3-d][1,3]oxazin-4-one

**Intermediate 660** (10.5 g, 2.13 mmol) was suspended in MeCN (42 mL) and heated to 55 °C with stirring prior to addition of EDC (499 mg, 2.55 mmol). The reaction was heated at this temperature for 40 min. The reaction mixture was concentrated in vacuo and the residue purified by column chromatography eluting with a 0-100% gradient of EtOAc in isohexane to give the title compound (650 mg, 64 % Yield). δ_{H} (300 MHz, d-Chloroform) 7.54 (s, 1H), 6.79 (t, J = 73.9 Hz, 1H), 5.42 (s, 1H), 4.78 - 4.57 (m, 1H), 3.66 - 3.54 (m, 4H), 3.15 - 2.83 (m, 3H), 2.38 - 2.24 (m, 2H), 2.01 - 1.91 (m, 1H), 1.33 - 1.06 (m, 4H).

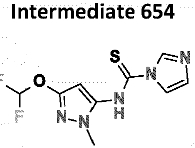

### N-[5-(difluoromethoxy)-2-methyl-pyrazol-3-yl]imidazole-1-carbothioamide

To a stirred suspension of 5-(difluoromethoxy)-2-methyl-pyrazol-3-amine hydrochloride (1.00 g, 5.01 mmol) in DCM (15 mL) at room temperature were added DIPEA (1.3 mL, 7.5 mmol) and 1,1'-thiocarbonyldiimidazole (1.05 g, 5.60 mmol). The reaction mixture was used directly in the next step. LCMS [M-H]⁻ 272, RT 1.42 minutes (Method 7).

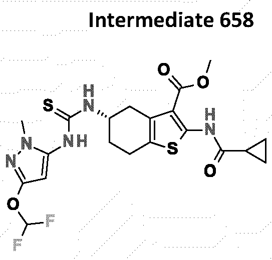

### methyl (55)-2-(cyclopropanecarbonylamino)-5-[[5-(difluoromethoxy)-2-methyl-pyrazol-3-yl]carbamothioylamino]-4,5,6,7-tetrahydrobenzothiophene-3-carboxylate

To a stirred solution of **intermediate 636** (2.0 g, 6.8 mmol) in DCM (20 mL) was added a solution of **Intermediate** 644 (1.4 g, 6.8 mmol) in DCM (20 mL). The reaction mixture was stirred at room temperature for 10 minutes. The reaction mixture was concentrated under reduced pressure and purified by column chromatography eluting with a gradient of 0-100% ethyl acetate in iso-hexanes to give the title compound (2.54 g, 75 % Yield). δ_{H} (300 MHz, Chloroform-d) 11.40 (s, 1H), 7.55 (s, 1H), 6.78 (t, J = 72.9 Hz, 1H), 6.29 (d, J = 8.0 Hz, 1H), 5.81 (s, 1H), 4.87 - 4.65 (m, 1H), 3.88 (s, 3H), 3.67 (s, 3H), 3.24 (dd, J = 17.1, 5.4 Hz, 1H), 2.91 - 2.58 (m, 3H), 2.18 - 1.91 (m, 1H), 1.67 (tt, J = 8.2, 4.5 Hz, 1H), 1.12 (dt, J = 4.6, 3.3 Hz, 2H), 0.97 (dt, J = 8.0, 3.4 Hz, 2H).

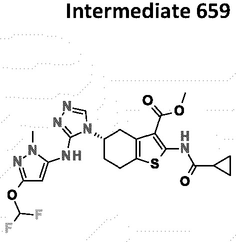

### methyl (55)-2-(cyclopropanecarbonylamino)-5-[3-[[5-(difluoromethoxy)-2-methyl-pyrazol-3-yl]amino]-1,2,4-triazol-4-yl]-4,5,6,7-tetrahydrobenzothiophene-3-carboxylate

Synthesised in the same manner as Example 229, using **intermediate 658** (2.5 g, 5.0 mmol) and comparable stoichiometries of reagents. The product was purified by column chromatography (eluting with a 0-100% gradient of EtOAc in isohexane) to afford the title compound (1.5 g, 59 % Yield) as a pale brown oil. δ_{H} (300 MHz, Chloroform-*d*) 11.42 (s, 1H), 7.50 (s, 1H), 6.79 (t, *J* = 73.9 Hz, 1H), 5.45 (s, 1H), 4.74 - 4.58 (m, 1H), 3.88 (s, 3H), 3.64 (s, 3H), 3.43 (dd, *J* = 17.2, 5.7 Hz, 1H), 3.01 - 2.66 (m, 2H), 2.32 - 2.14 (m, 2H), 1.69 (tt, *J* = 7.8, 4.5 Hz, 1H), 1.15 (dt, *J* = 4.4, 3.3 Hz, 2H), 1.03 - 0.90 (m, 2H).

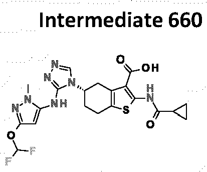

### (5S)-2-(cyclopropanecarbonylamino)-5-[3-[[5-(difluoromethoxy)-2-methyl-pyrazol-3-yl]amino]-1,2,4-triazol-4-yl]-4,5,6,7-tetrahydrobenzothiophene-3-carboxylic acid

**Intermediate 659** (1.51 g, 2.98 mmol) was dissolved in THF (25 mL) and 2 M aqueous sodium hydroxide (5.95 mL, 11.9 mmol) added. Reaction heated to 50 °C with stirring for 6 h. The volatiles were removed in vacuo and the aqueous solution acidified to pH^{~}5 with 2 M HCl. A thick purple precipitate formed, which was diluted with water and filtered, washing with diethyl ether. The residue was dried in vacuum pistol (^{~}3 mBar, 50 °C) overnight to afford the title compound (1.05 g, 71 % Yield). δ_{H} (400 MHz, DMSO-d6) 13.24 (s, 1H), 12.04 (s, 1H), 11.43 (s, 1H), 8.84 - 8.39 (m, 1H), 8.21 (s, 1H), 7.42 - 6.94 (m, 1H), 5.99 - 5.60 (m, 1H), 4.53 - 4.32 (m, 1H), 3.63 - 3.34 (m, 3H), 2.94 - 2.73 (m, 3H), 2.30 - 2.06 (m, 2H), 1.99 (tt, J = 7.7, 5.2 Hz, 1H), 0.97 - 0.85 (m, 4H). LCMS [M+H]⁺ 494, RT 0.96 minutes (Method 26).

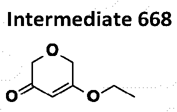

### 3-ethoxy-2H-pyran-5-one

2H-Pyran-3,5(4H,6H)-dione (1.36 g, 11.6 mmol) was dissolved in EtOH (50 mL) and conc. sulfuric acid (1.5 mL) added. The reaction was stirred at ambient temperature overnight. The reaction was concentrated to remove the EtOH and basified (pH ^{~}8) with saturated aqueous NaHCO₃ solution. The resulting brown solution was extracted with EtOAc (3 x 50 mL). The combined organic extracts were dried over Na₂SO₄ and concentrated in vacuo. The resulting crude oil was purified by column chromatography eluting with a 0-100% gradient of EtOAc in isohexane. Product-containing fractions were combined and concentrated to afford the title compound (331 mg, 20 % Yield). δ_{H} (300 MHz, Chloroform-*d*) 5.46 (s, 1H), 4.25 (s, 2H), 4.08 (d, *J* = 0.8 Hz, 2H), 3.99 (q, *J* = 7.0 Hz, 2H), 1.39 (t, *J* = 7.0 Hz, 3H).

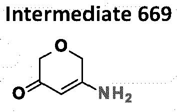

### 3-amino-2H-pyran-5-one

**Intermediate 668** (331 mg, 2.33 mmol) was dissolved in EtOH (25 mL) and the solution cooled to -78 °C. Ammonia gas was bubbled through the solution for 5 min. The cooling was removed, and the reaction allowed to warm to ambient temperature with stirring under a positive pressure of N₂ gas. The reaction was stirred overnight and then concentrated in vacuo and the crude product purified by column chromatography eluting with a 0-20 % gradient of MeOH in DCM. Product-containing fractions were combined and concentrated to afford the title compound (223 mg, 85% Yield). δ_{H} (300 MHz, DMSO-d6) 7.00 (s, 2H), 5.01 (s, 1H), 4.18 (s, 2H), 3.80 (s, 2H).

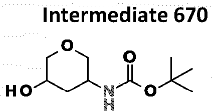

### tert-butyl N-(5-hydroxytetrahydropyran-3-yl)carbamate

A solution of **intermediate 669** (1.50 g, 13 mmol) in EtOH (100 mL) was treated with nickel aluminide (1.10 g, 6.6 mmol, 50% slurry in water). The reaction was sealed in a stainless-steel pressure vessel and evacuated then placed under 4 bar H₂ and heated to 60 °C with stirring for 3 days. The reaction was cooled and degassed and the slurry filtered through a pad of celite, washing with EtOH (20 mL). The ethanolic solution was concentrated in vacuo to afford a brown oily solid, which was dissolved in a mixture of TEA (5.5 mL, 40 mmol), DCM (2.5 mL) and di-tert-butyl dicarbonate (1 mol/L) in THF (9.9 mL, 20 mmol). The reaction stirred overnight at ambient temperature. The reaction was filtered to removed undissolved solids and concentrated in vacuo. The crude product was purified by column chromatography eluting with a 30-100% gradient of EtOAc in isohexane. Product-containing fractions were combined and concentrated to afford the title compound (741 mg, 26% Yield). δ_{H} (300 MHz, Chloroform-*d*) 5.71 - 4.51 (m, 1H), 4.03 - 3.82 (m, 2H), 3.82 - 3.55 (m, 3H), 3.54 - 3.26 (m, 2H), 1.99 - 1.68 (m, 2H), 1.44 (s, 9H).

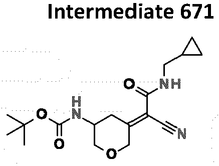

### tert-butyl N-[(5E)-5-[1-cyano-2-(cyclopropylmethylamino)-2-oxo-ethylidene]tetrahydropyran-3-yllcarbamate

**Intermediate 670** (350 mg, 1.61 mmol) was dissolved in DCM (4 mL) and the solution cooled in an ice bath under a N₂ atmosphere prior to addition of Dess-Martin Periodinane (1.02 g, 2.42 mmol). The reaction was stirred for 1 h at 0 °C. The reaction was quenched by addition of saturated aqueous Na₂S₂O₇ solution (1.5 mL) with vigorous stirring. Saturated aqueous NaHCO₃ (10 mL) was added followed by portions of solid NaHCO₃ until pH~8 was achieved. The organic layer was partitioned, and the aqueous layer extracted with DCM (10 mL). The combined organic extracts were dried over Na₂SO₄ and the solvents removed in vacuo. The resulting crude ketone was dissolved in in THF (10 mL) and 2-cyano-N-(cyclopropylmethyl)acetamide (510 mg, 3.7 mmol), pyridine (530 mg, 6.6 mmol) and titanium (IV) isopropoxide (3.0 g, 10 mmol) were added and the reaction stirred at ambient temperature overnight. The reaction was diluted with DCM (10 mL) and quenched with water (7 mL) with vigorous stirring. The organic layer was partitioned and the aqueous extracted with DCM (2 x 10 mL). The combined organic extracts were passed through a plug of celite and the solvents removed in vacuo. The crude product was purified by column chromatography eluting with a 0-100 % gradient of EtOAc in isohexane. Product-containing fractions were combined and concentrated to afford the title compound (303 mg, 45% Yield). LCMS [M-H]⁻ 334, RT 0.88 minutes (Method 7).

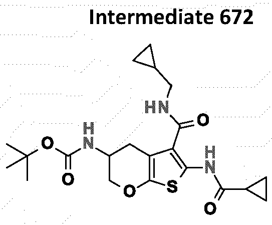

### tert-butyl N-[6-amino-5-(cyclopropylmethylcarbamoyl)-3,4-dihydro-2H-thieno[2,3-b]pyran-3-yl]carbamate

To a solution of **Intermediate 671** (700 mg, 2.09 mmol) in EtOH (7.5 mL) were added morpholine (0.37 mL, 4.17 mmol) and sulfur (100 mg, 3.13 mmol). The vessel was sealed and heated to 70 °C for 1 h in a microwave reactor with stirring. The crude product mixture was filtered and concentrated to give a residue, which was dissolved in a mixture of DCM (30 mL) and DIPEA (0.43 mL, 2.45 mmol).

The solution was stirred prior to dropwise addition of cyclopropanecarbonyl chloride (125 µL, 1.35 mmol) and the reaction allowed to stir overnight at ambient temperature. The reaction was washed with water (10 mL) and passed through a phase separator cartridge prior to removal of volatiles in vacuo. The crude product was purified by column chromatography eluting with a 0-60 % gradient of EtOAc in isohexane. Product-containing fractions were combined and concentrated to afford the title compound (317 mg, 50 % Yield). δ_{H} (400 MHz, Chloroform-d) 12.15 (s, 1H), 5.79 - 5.68 (m, 1H), 5.10 - 4.93 (m, 1H), 4.35 - 4.14 (m, 2H), 3.33 - 3.20 (m, 2H), 3.04 (dd, J = 14.9, 5.6 Hz, 1H), 2.79 - 2.67 (m, 1H), 1.69 - 1.59 (m, 1H), 1.45 (s, 9H), 1.18 - 0.97 (m, 3H), 0.96 - 0.82 (m, 2H), 0.64 - 0.54 (m, 2H), 0.32 - 0.20 (m, 2H).

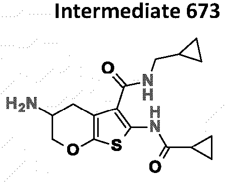

### 3-amino-6-(cyclopropanecarbonylamino)-N-(cyclopropylmethyl)-3,4-dihydro-2H-thieno[2,3-b]pyran-5-carboxamide

**Intermediate 672** (300 mg, 0.69 mmol) was dissolved in DCM (5 mL) and TFA (1 mL) was added. The reaction was stirred at ambient temperature for 2 h. The reaction was concentrated in vacuo and the crude product dissolved in MeOH (3 mL) and absorbed on to an SCX2 cartridge. The absorbed product was washed with MeOH (20 mL) and then eluted using 4 M NH₃ in MeOH (30 mL). The methanolic ammonia was removed in vacuo to afford the title compound (175 mg, 75 % Yield). δ_{H} (400 MHz, Chloroform-d) 12.08 (s, 1H), 5.90 (s, 1H), 4.14 (dd, J = 10.5, 2.5 Hz, 1H), 3.94 (dd, J = 10.4, 6.6 Hz, 1H), 3.51 - 3.44 (m, 1H), 3.25 (dd, J = 7.1, 5.3 Hz, 2H), 3.05 (dd, J = 14.6, 5.6 Hz, 1H), 2.59 (dd, J = 15.3, 5.4 Hz, 1H), 2.53 - 1.81 (m, 2H), 1.63 (tt, J = 8.0, 4.6 Hz, 1H), 1.15 - 0.99 (m, 3H), 0.88 (dq, J = 7.3, 4.1 Hz, 2H), 0.63 - 0.48 (m, 2H), 0.29 - 0.20 (m, 2H).

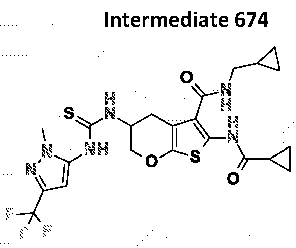

### 6-(cyclopropanecarbonylamino)-N-(cyclopropylmethyl)-3-[[2-methyl-5-(trifluoromethyl)pyrazol-3-yl]carbamothioylamino]-3,4-dihydro-2H-thieno[2,3-b]pyran-5-carboxamide

To a stirred solution of **intermediate 673** (175 mg, 0.52 mmol) in DCM (3.5 mL) was added a solution of **intermediate 262** (113 mg, 0.559 mmol) in DCM (1.5 mL). The reaction mixture was stirred at room temperature for 10 min. The reaction mixture was dry loaded onto silica and purified by column chromatography with a gradient of 0-100% EtOAc in isohexane. Product-containing fractions were combined and concentrated to afford the title compound (215 mg, 75.9 % Yield). δ_{H} (400 MHz, Chloroform-d) 11.97 (s, 1H), 8.56 (s, 1H), 7.95 (d, *J* = 7.3 Hz, 1H), 6.49 (s, 1H), 5.84 (t, *J* = 5.2 Hz, 1H), 5.24 - 5.10 (m, 1H), 4.49 - 4.37 (m, 1H), 4.16 - 4.11 (m, 1H), 3.81 (s, 3H), 3.25 (dddd, *J* = 43.4, 13.7, 7.2, 5.1 Hz, 2H), 3.08 - 2.96 (m, 2H), 1.58 - 1.48 (m, 1H), 1.16 - 0.80 (m, 5H), 0.67 - 0.53 (m, 2H), 0.36 - 0.18 (m, 2H).

### EXAMPLES

### EXAMPLE 3

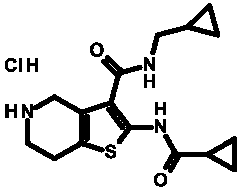

### 2-(Cyclopropanecarbonylamino)-N-(cyclopropylmethyl)-4,5,6,7-tetrahydrothieno[3,2-c]pyridine-3-carboxamide; hydrochloride

*Intermediate 8* (0.87 g, 2.08 mmol) was dissolved in 4 N hydrochloric acid solution in 1,4-dioxane (20 mL) and the reaction was stirred at r.t. for 16 h. Further 4 N hydrochloric acid solution in 1,4-dioxane (6 mL) was added and the reaction was stirred at r.t. for 4 h. The reaction mixture was concentrated *in vacuo* to afford the *title compound* (820 mg, 111%) as an off white solid. δ_{H} (400 MHz, DMSO-*d*₆) 10.73 (s, 1H), 9.07 (s, 2H), 7.87 (t, *J* 5.6 Hz, 1H), 3.96 (q, *J* 6.4, 4.3 Hz, 2H), 3.13 (d, *J* 6.4 Hz, 2H), 2.90 (dd, *J* 6.9, 5.6 Hz, 2H), 2.70 (t, *J* 6.0 Hz, 2H), 1.81 - 1.71 (m, 1H), 0.91 - 0.73 (m, 1H), 0.73 - 0.55 (m, 4H), 0.26 - 0.16 (m, 2H), 0.08 - -0.08 (m, 2H). LCMS (ES+) [M+H]⁺ 320.8, RT 1.008 minutes (method 6140, pH 3 *Method 2*). LCMS (ES+) [M+H]⁺ 320.8, RT 1.372 minutes *(Method 3).*

### EXAMPLE 4

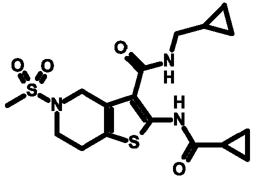

### 2-(Cyclopropanecarbonylamino)-N-(cyclopropylmethyl)-5-methylsulfonyl-6,7-dihydro-4H-thieno[3,2-c]pyridine-3-carboxamide

*Example 3* (56 mg, 0.16 mmol) was dissolved in DCM (6.6 mg, 78.0 mmol) and DIPEA (51.1 mg, 0.39 mmol) and methanesulfonyl chloride [124-63-0] (23.5 mg, 0.20 mmol) was added and the reaction mixture was stirred at r.t. for 16 h. The reaction mixture was washed with water, passed through a phase separation cartridge and the filtrate evaporated *in vacuo.* The crude reaction mixture was purified by flash column chromatography on silica (gradient elution 30-100% EtOAc/hexane) to afford the *title compound* (48 mg, 77%) as a white solid. LCMS (ES+) [M+H]⁺ 398.6, RT 1.779 minutes *(Method 3).* LCMS (ES+) [M+H]⁺ 398.7, RT 1.848 minutes *(Method 2).*

### EXAMPLE 5

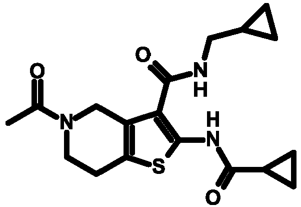

### 5-Acetyl-2-(cyclopropanecarbonylamino)-N-(cyclopropylmethyl)-6,7-dihydro-4H-thieno[3,2-c]pyridine-3-carboxamide

*Example 3* (56 mg, 0.16 mmol) was dissolved in DCM (5 mL) and DIPEA (51 mg, 0.39 mmol) and acetyl chloride (16 mg, 0.20 mmol) was added and the reaction mixture was stirred at r.t. for 16 h. The reaction mixture was washed with water, passed through a phase separation cartridge and the filtrate was concentrated *in vacuo* to give the crude product which was purified by flash column chromatography on silica (gradient elution with 30-100% EtOAc/hexane) to afford the *title compound* (37 mg, 65%) as a white solid. δ_{H} (400 MHz, DMSO-*d*₆) 11.07 - 10.83 (m, 1H), 7.97 - 7.75 (m, 1H), 4.57 (d, *J* 2.0 Hz, 2H), 3.78 - 3.62 (m, 2H), 3.16 (q, *J* 5.5 Hz, 2H), 2.81 - 2.70 (m, 1H), 2.70 - 2.61 (m, 1H), 2.14 - 2.02 (m, 3H), 2.00 - 1.86 (m, 1H), 1.14 - 1.00 (m, 1H), 0.94 - 0.77 (m, 4H), 0.52 - 0.36 (m, 2H), 0.28 - 0.18 (m, 2H). LCMS (ES+) [M+H]⁺ 362.8, RT 1.406 minutes *(Method 3).* LCMS [M-H]⁻ 360.0, RT 1.661 minutes *(Method 2).*

### EXAMPLE 6

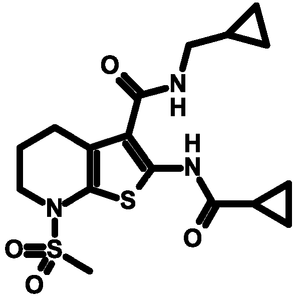

### 2-(Cyclopropanecarbonylamino)-N-(cyclopropylmethyl)-7-methylsulfonyl-5,6-dihydro-4H-thieno[2,3-b]pyridine-3-carboxamide

*Intermediate 12* (75 mg, 0.21 mmol) was dissolved in DCM (5 mL) and DIPEA (68 mg, 0.53 mmol) and methanesulfonyl chloride [124-63-0] (32 mg, 0.27 mmol) were added and the reaction mixture was stirred at r.t. for 16 h. The reaction mixture was washed with water, passed through a phase separation cartridge and the filtrate was concentrated *in vacuo.* The crude residue was purified by flash column chromatography on silica (gradient elution 30-100% EtOAc/hexane) to afford the *title compound* (58 mg, 69%) as a white solid. δ_{H} (400 MHz, DMSO-*d*₆) 10.91 (s, 1H), 7.58 (t, *J* 5.7 Hz, 1H), 3.49 - 3.39 (m, 2H), 2.98 - 2.86 (m, 2H), 2.81 (s, 3H), 2.48 (t, *J* 6.4 Hz, 2H), 1.76 - 1.64 (m, 3H), 0.88 - 0.77 (m, 1H), 0.69 - 0.55 (m, 4H), 0.28 - 0.18 (m, 2H), 0.07 - -0.04 (m, 2H). LCMS (ES+) [M+H]⁺ 398.6, RT 1.956 minutes *(Method 3).* LCMS (ES+) [M+H]⁺ 398.6, RT 1.991 minutes *(Method 2).*

### EXAMPLE 7

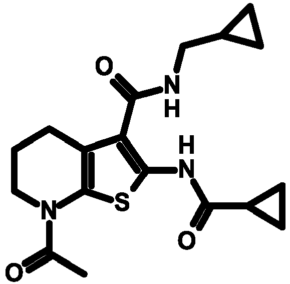

### 7-Acetyl-2-(cyclopropanecarbonylamino)-N-(cyclopropylmethyl)-5,6-dihydro-4H-thieno[2,3-b]pyridine-3-carboxamide

*Intermediate 12* (75 mg, 0.21 mmol) was dissolved in DCM (5 mL) and DIPEA (68 mg, 0.53 mmol) and acetyl chloride (22 mg, 0.27 mmol) were added. The reaction mixture was stirred at r.t. for 16 h. The mixture was washed with water, passed through a phase separation cartridge and the filtrate concentrated *in vacuo* to yield the crude product which was purified by flash column chromatography on silica (gradient elution 30-100% EtOAc/hexane) to afford the *title compound (60* mg, 79%) as a white solid. δ_{H} (400 MHz, DMSO-*d*₆) 10.71 (s, 1H), 7.46 (t, *J* 5.7 Hz, 1H), 3.58 - 3.50 (m, 2H), 2.91 (t, *J* 6.2 Hz, 2H), 2.49 (t, *J* 6.2 Hz, 2H), 1.99 (s, 3H), 1.71 (s, 2H), 1.65 - 1.57 (m, 1H), 0.87 - 0.72 (m, 1H), 0.63 - 0.54 (m, 4H), 0.25 - 0.15 (m, 2H), 0.05 - -0.07 (m, 2H). LCMS (ES+) [M+H]⁺ 362.8, RT 1.744 minutes *(Method 3).* LCMS (ES+) [M+H]⁺ 362.8, RT 1.797 minutes *(Method 2).*

### EXAMPLE 8

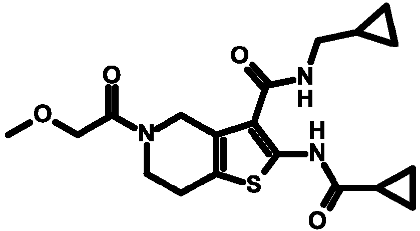

### 2-(Cyclopropanecarbonylamino)-N-(cyclopropylmethyl)-5-(2-methoxyacetyl)-6,7-dihydro-4H-thieno[3,2-c]pyridine-3-carboxamide

To *example 3* (36 mg, 0.10 mmol) and N,N-(diisopropyl)aminomethylpolystyrene (40 mg, 3.53 mmol/g, 0.30 mmol) in DCM (0.8 mL) was added methoxyacetyl chloride (14 mg, 0.13 mmol) and the reaction mixture was stirred at r.t. overnight. To the reaction mixture was added methoxyacetyl chloride (11 mg, 0.10 mmol) and was stirred for 4 h at r.t. prior to addition of further methoxyacetyl chloride (11 mg, 0.10 mmol) and the mixture was stirred overnight. To the sample was added N,N-(diisopropyl)aminomethylpolystyrene (40 mg, 3.53 mmol/g, 0.30 mmol) and the mixture was stirred at r.t. for 2 h then MP-trisamine (55 mg, 0.21 mmol) was added and the mixture was stirred for 1 h at r.t. The reaction mixture was filtered, washed with MeCN and concentrated *in vacuo* to give the crude product which was purified by reverse phase HPLC (basic) to afford the *title compound* (0.011 g, 27%). LCMS [M+H]⁺ 391.2, RT 3.77 minutes *(Method 8).*

### EXAMPLE 9

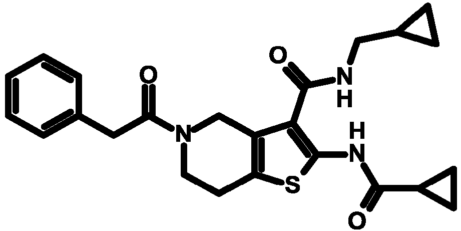

### 2-(Cyclopropanecarbonylamino)-N-(cyclopropylmethyl)-5-(2-phenylacetyl)-6,7-dihydro-4H-thieno[3,2-c]pyridine-3-carboxamide

To *example 3* (36 mg, 0.10 mmol) and N,N-(diisopropyl)aminomethylpolystyrene (40 mg, 3.53 mmol/g, 0.30 mmol) in DCM (0.8 mL) was added phenylacetyl chloride (20 mg, 0.13 mmol) and the reaction mixture was stirred at r.t. overnight. To the reaction mixture was added further phenylacetyl chloride (16 mg, 0.10 mmol) and the mixture was stirred overnight. To the sample was added N,N-(diisopropyl)aminomethylpolystyrene (40 mg, 3.53 mmol/g, 0.30 mmol) and the mixture was stirred at r.t. for 2 h then MP-trisamine (55 mg, 0.21 mmol) was added and the mixture was stirred for 1 h at r.t. The reaction mixture was filtered, washed with MeCN and concentrated in *vacuo* to give the crude product which was purified by reverse phase HPLC (basic) to afford the *title compound* (0.0087 g, 20%). LCMS [M+H]⁺ 437.2, RT 4.48 minutes *(Method 8).*

### EXAMPLE 10

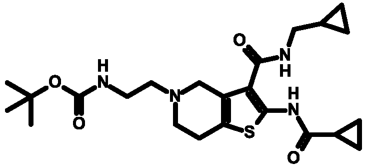

### Tert-butyl N-[2-[2-(cyclopropanecarbonylamino)-3-(cyclopropylmethylcarbamoyl)-6,7-dihydro-4H-thieno[3,2-c]pyridin-5-yl]ethyl]carbamate

To *example 3* (36 mg, 0.10 mmol) in THF (1 mL) and EtOH (0.5 mL) was added MP-carbonate (75 mg, 0.25 mmol, 3.36 mmol/g). The mixture was stirred for 1 h at r.t., filtered and rinsed with EtOH (2 x 200 µL). To the filtrate was added N-Boc-2-aminoacetaldehyde [89711-08-0] (20 mg, 0.13 mmol), MP-cyanoborohydride (100 mg, 0.25 mmol, 2.49 mmol/g) and acetic acid (0.1 mL, 2 mmol). The reaction mixture was stirred at r.t. overnight. The reaction mixture was filtered, rinsed with EtOH and the filtrate concentrated *in vacuo* reaction mixture. To the reaction mixture was added DCM (1 mL) and a portion of the reaction mixture containing the product (20 mg, 0.043 mmol) was removed and purified by reverse phase HPLC (basic) to afford the *title compound* (4.4 mg, 9.5%). LCMS [M+H]⁺ 463.1, RT 4.67 minutes, 95.55% purity (*Method 9*).

### EXAMPLE 11

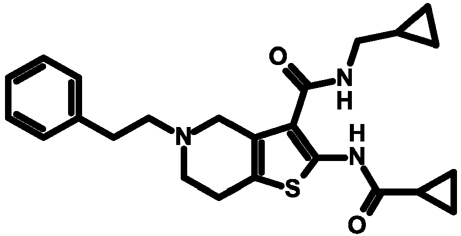

### 2-(Cyclopropanecarbonylamino)-N-(cyclopropylmethyl)-5-(2-phenylethyl)-6,7-dihydro-4H-thieno[3,2-c]pyridine-3-carboxamide

To *example 3* (36 mg, 0.10 mmol) in THF (1 mL) and EtOH (0.5 mL) was added MP-carbonate (75 mg, 0.25 mmol, 3.36 mmol/g). The mixture was stirred for 1 h at r.t., filtered and rinsed with EtOH (2 x 200 µL). To the filtrate was added phenylacetaldehyde [122-78-1] (14 mg, 0.12 mmol), MP-cyanoborohydride (100 mg, 0.25 mmol, 2.49 mmol/g) and acetic acid (0.1 mL, 2 mmol). The reaction mixture was stirred at r.t. overnight. The reaction mixture was filtered, rinsed with EtOH and the filtrate concentrated *in vacuo* reaction to give the product which was purified by reverse phase HPLC (basic) to afford the *title compound* (4.1 mg, 10%). LCMS [M+H]⁺ 424.19, RT 5.10 minutes, 95.60% purity *(Method 9).*

### EXAMPLE 12

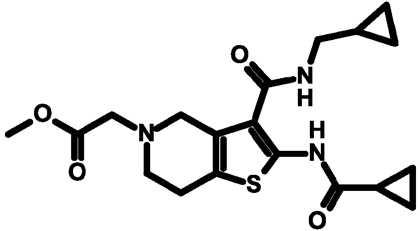

### Methyl 2-[2-(cyclopropanecarbonylamino)-3-(cyclopropylmethylcarbamoyl)-6,7-dihydro-4H-thieno[3,2-c]pyridin-5-yl]acetate

To *example 3* (36 mg, 0.100 mmol) in THF (1 mL) and EtOH (0.5 mL) was added MP-carbonate (75 mg, 0.25 mmol, 3.36 mmol/g). The mixture was stirred for 1 h at r.t., filtered and rinsed with EtOH (2 x 200 µL). To the filtrate was added methyl glyoxylate (11 mg, 0.12 mmol), MP-cyanoborohydride (100 mg, 0.25 mmol, 2.49 mmol/g) and acetic acid (0.1 mL, 2 mmol). The reaction mixture was stirred at r.t. overnight. The reaction mixture was filtered, rinsed with EtOH and the filtrate concentrated *in vacuo.* Reaction mixture was purified by reverse phase HPLC (basic) to afford the *title compound* (0.0048 g, 12%). LCMS [M+H]⁺ 376.205, RT 2.93 minutes, 100% purity *(Method 20).*

### EXAMPLE 13 and EXAMPLE 14

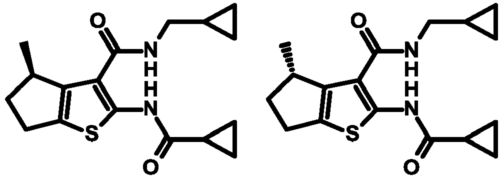

### Example13

### (4R)-2-(Cyclopropanecarbonylamino)-N-(cyclopropylmethyl)-4-methyl-5,6-dihydro-4H-cyclopenta[b]thiophene-3-carboxamide

### Example 14

### (4S)-2-(Cyclopropanecarbonylamino)-N-(cyclopropylmethyl)-4-methyl-5,6-dihydro-4H-cyclopenta[b]thiophene-3-carboxamide

The 2 enantiomers of *intermediate 15* were separated by chiral preparative chromatography (Chiralpak IC, 200 mL/min 1:1 hexane:iPrOH) to afford two isomers *isomer A, example 13* and *Isomer B, example 14.* The absolute stereochemistry of the two isomers was arbitrarily assigned. *Isomer A, example 13* was repurified by flash column chromatography on silica (gradient elution with 20 - 80% EtOAc/hexane) and was freeze-dried from MeCN/water to afford *isomer A, example 13* (6.5 mg, 20%) as a white solid. δ_{H} (400 MHz, DMSO-*d*₆) 11.44 (s, 1H), 7.19 (t, *J* 5.9 Hz, 1H), 3.33 (t, *J* 7.3 Hz, 1H), 3.14 - 3.08 (m, 1H), 2.73 (ddd, *J* 13.6, 7.0, 5.1 Hz, 1H), 2.68 - 2.53 (m, 1H), 2.53 - 2.41 (m, 1H), 2.39 - 2.29 (m, 1H), 1.77 - 1.67 (m, 1H), 1.67 - 1.56 (m, 1H), 0.88 - 0.73 (m, 4H), 0.68 - 0.54 (m, 4H), 0.24 - 0.08 (m, 2H), 0.06 - -0.08 (m, 2H). *Isomer B, example 14* (11 mg, 34%). δ_{H} (400 MHz, DMSO-*d*₆) 11.44 (s, 1H), 7.19 (t, *J* 5.9 Hz, 1H), 3.33 (t, *J* 7.5 Hz, 1H), 3.15 - 3.08 (m, 1H), 2.79 - 2.67 (m, 1H), 2.68 - 2.54 (m, 1H), 2.53 - 2.40 (m, 1H), 2.39 - 2.29 (m, 1H), 1.77 - 1.66 (m, 1H), 1.66 - 1.56 (m, 1H), 0.89 - 0.77 (m, 4H), 0.67 - 0.54 (m, 4H), 0.22 - 0.13 (m, 2H), 0.00 (dd, *J* 4.5, 2.2 Hz, 2H).

### EXAMPLE 39

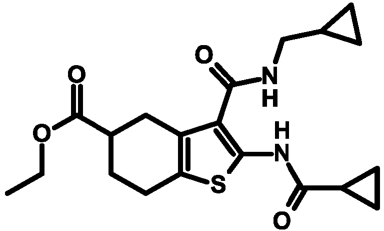

### Ethyl 2-(cyclopropanecarbonylamino)-3-(cyclopropylmethylcarbamoyl)-4,5,6,7-tetrahydrobenzothiophene-5-carboxylate

*Intermediate 43* (5.3 g, 15.7 mmol) was dissolved in DCM (100 mL) and 1-cyclopropylmethanamine (2.7 mL, 31.42 mmol) and EDCI (4.5 g, 23.56 mmol) was added and the reaction mixture was stirred at r.t. for 16 h. The reaction mixture was diluted with DCM (100 mL) then washed with water, 1M aqueous hydrochloric acid solution and saturated sodium hydrogen carbonate solution. The organic phase was washed with brine, dried (MgSO₄), filtered and concentrated *in vacuo* to give the crude residue which was purified by flash column chromatography on silica (gradient elution with 5% to 50% EtOAc in heptane) to afford the *title compound* (3.11 g, 51%) as a white solid. δ_{H} (500 MHz, DMSO-*d*₆) 11.03 (s, 1H), 7.74 (t, *J* 5.6 Hz, 1H), 4.10 (q, *J* 7.1 Hz, 2H), 3.25 - 3.16 (m, 1H), 3.13 - 3.05 (m, 1H), 2.90 - 2.76 (m, 2H), 2.75 - 2.64 (m, 3H), 2.18 - 2.08 (m, 1H), 1.94 - 1.87 (m, 1H), 1.82 - 1.70 (m, 1H), 1.20 (t, *J* 7.1 Hz, 3H), 1.09 - 0.97 (m, 1H), 0.87 - 0.80 (m, 4H), 0.45 - 0.39 (m, 2H), 0.26 - 0.20 (m, 2H). LCMS (ES+) [M+H]⁺ 391.05, [M+Na]⁺ 413.10, RT 1.41 minutes, 100% purity (*Method 6*). LCMS (ES+) [M+H]⁺ 391.1, RT 3.73 minutes, purity 97.5%, 1.2% 7-regioisomer present (*Method 10*).

### EXAMPLE 40

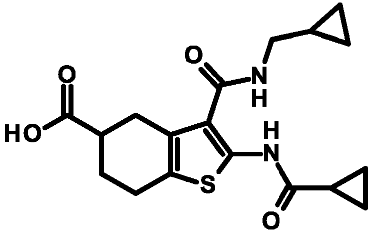

### 2-(Cyclopropanecarbonylamino)-3-(cyclopropylmethylcarbamoyl)-4,5,6,7-tetrahydrobenzothiophene-5-carboxylic acid

*Example 39* (3.5 g, 8.96 mmol) was dissolved in THF (35 mL) and 2M lithium hydroxide monohydrate solution was added (13.44 mL). The reaction mixture was stirred at r.t. for 48 h before concentrating *in vacuo* and the crude residue was dissolved in water (5 mL) and sonicated for 2 minutes. The resulting solution was acidified with 1M aqueous hydrochloric acid solution and the resulting solid was filtered off, washed with water and dried *in vacuo* to afford the title compound (3.2 g, 90%). δ_{H} (500 MHz, DMSO-*d*₆) 12.29 (s, 1H), 11.07 (s, 1H), 7.72 (s, 1H), 3.21 - 3.06 (m, 2H), 2.89 - 2.76 (m, 2H), 2.72 - 2.56 (m, 3H), 2.16 - 2.06 (m, 1H), 1.94 - 1.86 (m, 1H), 1.80 - 1.69 (m, 1H), 1.09 - 0.98 (m, 1H), 0.88 - 0.79 (m, 4H), 0.46 - 0.38 (m, 2H), 0.26 - 0.19 (m, 2H). LCMS (ES+) [M+H]+ 363.10, RT 1.18 minutes, 98% purity (*Method 6*).

### EXAMPLE 41

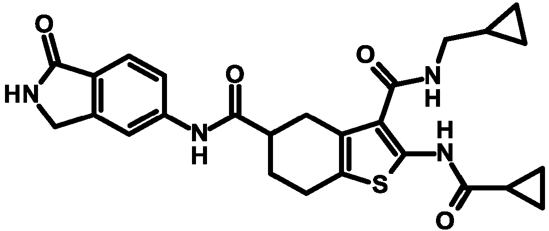

### 2-(Cyclopropanecarbonylamino)-N3-(cyclopropylmethyl)-N5-(1-oxoisoindolin-5-yl)-4,5,6,7-tetrahydrobenzothiophene-3,5-dicarboxamide

To the reaction vessel was added *example 40* (47.1 mg, 0.13 mmol), 5-aminoisoindolin-1-one [222036-66-0] (19.3 mg, 0.13 mmol), COMU (69 mg, 0.16 mmol), DCM (1.3 mL) and DIPEA (17 mg, 0.13 mmol). The reaction mixture was shaken at r.t. for 3 nights. To the dry reaction mixture was added DCM (2 mL) and water (2 mL) and the mixture was filtered through a phase separator and washed with DCM (2 x 2 mL). The combined organic phases were concentrated *in vacuo* and purified by preparative HPLC (basic) to afford the *title compound* (0.0041 g, 6.3%). LCMS (ES+) [M+H]⁺ 493.1893, RT 4.28 minutes *(Method 20).*

### EXAMPLE 42

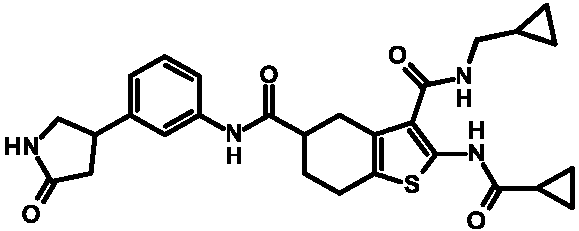

### 2-(Cyclopropanecarbonylamino)-N3-(cyclopropylmethyl)-N5-[3-(5-oxopyrrolidin-3-yl)phenyl]-4,5,6,7-tetrahydrobenzothiophene-3,5-dicarboxamide

The method for *example 41* was utilised substituting 4-(3-aminophenyl)-2-pyrrolidinone (23 mg, 0.130 mmol) for 5-aminoisoindolin-1-one to afford the *title compound* (0.027 g, 39%). LCMS (ES+) [M+H]⁺ 521.2209, RT 4.42 minutes *(Method 20).*

### EXAMPLE 43

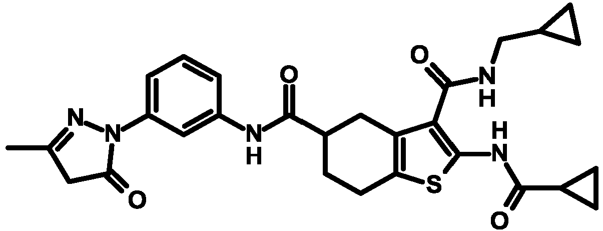

### 2-(Cyclopropanecarbonylamino)-N3-(cyclopropylmethyl)-N5-[3-(3-methyl-5-oxo-4H-pyrazol-1-yl)phenyl]-4,5,6,7-tetrahydrobenzothiophene-3,5-dicarboxamide

The method for *example 41* was utilised substituting 2-(3-aminophenyl)-5-methyl-2,4-dihydro-pyrazol-3-one hydrochloride [292151-88-3] (29 mg, 0.13 mmol) for 5-aminoisoindolin-1-one and adding an additional equivalent of DIPEA (17 mg, 0.13 mmol) to afford the *title compound* (0.018 g, 26%). LCMS (ES+) [M+H]⁺ 534.2139, RT 4.69 minutes *(Method 20).*

### EXAMPLE 44

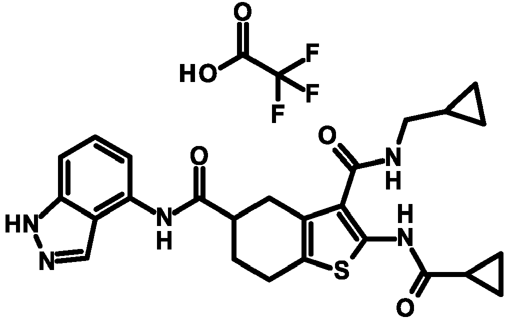

### 2-(Cyclopropanecarbonylamino)-N3-(cyclopropylmethyl)-N5-(1H-indazol-4-yl)-4,5,6,7-tetrahydrobenzothiophene-3,5-dicarboxamide;2,2,2-trifluoroacetic acid

To the reaction vessel was added *example 40* (54 mg, 0.15 mmol), COMU (67 mg, 0.15 mmol), DCM (0.8 mL) and DIPEA (58 mg, 0.45 mmol). The reaction mixture was stirred for 5 minutes prior to addition of *tert*-butyl 4-aminoindazole-1-carboxylate (35 mg, 0.15 mmol) and the reaction was stirred at r.t. overnight before heating to 40 °C for a further night. To the reaction mixture was added water (1 mL) and the mixture was filtered through a phase separation cartridge and the aqueous phase was washed with further DCM (1 mL). The organic phases were combined and to this mixture was added TFA (0.5 mL, 6 mmol) and the mixture was shaken for 2 h at r.t. before concentrating *in vacuo* to yield the crude product which was purified by preparative HPLC (acidic) to afford the *title compound* (12.4 mg, 14%). LCMS (ES+) [M+H]⁺ 478.17, RT 3.98 minutes (*Method 13*).

### EXAMPLE 45

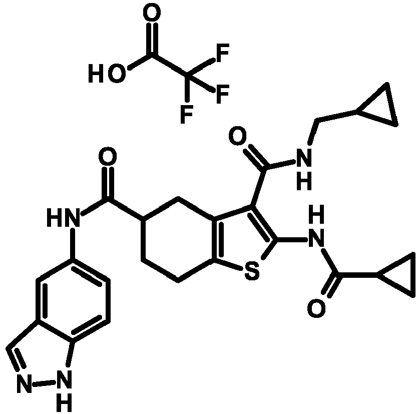

### 2-(Cyclopropanecarbonylamino)-N3-(cyclopropylmethyl)-N5-(1H-indazol-5-yl)-4,5,6,7-tetrahydrobenzothiophene-3,5-dicarboxamide;2,2,2-trifluoroacetic acid

To a reaction vessel cooled in an ice bath was added *example 40* (55 mg, 0.15 mmol), 5-aminoindazole [19335-11-6] (21 mg, 0.15 mmol), TBTU [125700-67-6] (49 mg, 0.15 mmol), DMF (0.8 mL) and DIPEA (78 µL, 0.45 mmol). The reaction mixture was stirred and allowed to warm to r.t. for 1 h. The reaction mixture was purified by preparative HPLC (basic) followed by preparative HPLC (acidic) to afford the *title compound* (28 mg, 31%) as a white solid. LCMS (ES+) [M+H]⁺ 478.20, RT 2.36 minutes *(Method 8).*

### EXAMPLE 46

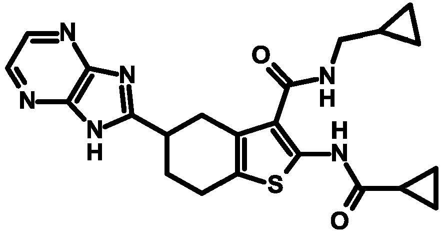

### 2-(Cyclopropanecarbonylamino)-N-(cyclopropylmethyl)-5-(1H-imidazo[4,5-b]pyrazin-2-yl)-4,5,6,7-tetrahydrobenzothiophene-3-carboxamide

To *example 40* (0.080 mmol, 30 mg) and pyrazine-2,3-diamine [13134-31-1] (9.5 mg, 0.086 mmol) was added pyridine (0.6 mL) and triphenyl phosphite (31 mg, 0.10 mmol). The reaction mixture was heated at 220 °C in the microwave for 1 h. The solvent was removed *in vacuo* and the crude product was purified by reverse phase HPLC (basic) to afford the *title compound* (0.0092 g, 26%). LCMS (ES+) [M+H]⁺ 437.1735, RT 4.18 minutes *(Method 20).*

### EXAMPLE 47

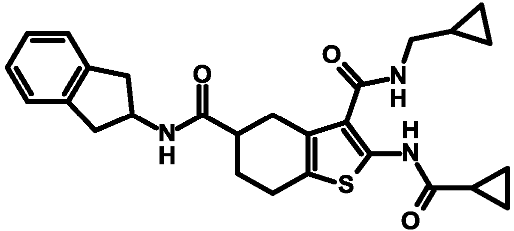

### 2-(Cyclopropanecarbonylamino)-N3-(cyclopropylmethyl)-N5-indan-2-yl-4,5,6,7-tetrahydrobenzothiophene-3,5-dicarboxamide

To *example 40* (36 mg, 0.10 mmol), COMU (53 mg, 0.12 mmol) and DCM (1 mL) was added 2-aminoindanhydrochloride [2338-18-3] (17 mg, 0.10 mmol) and a solution of DIPEA (26 mg, 0.20 mmol) in DCM (300µL). The reaction mixture was stirred overnight at r.t. and to the reaction mixture was added DCM (2 mL) and water (4 mL) and the mixture was filtered through a phase separation cartridge. The aqueous phase was extracted with further DCM (2 x 2 mL) and the organic phases were concentrated *in vacuo* and purified by reverse phase HPLC (basic) to afford the *title compound* (0.025 mg, 53%). LCMS (ES+) [M+H]⁺ 478.2157, RT 5.28 minutes, 89% Purity *(Method 20).*

### EXAMPLE 48

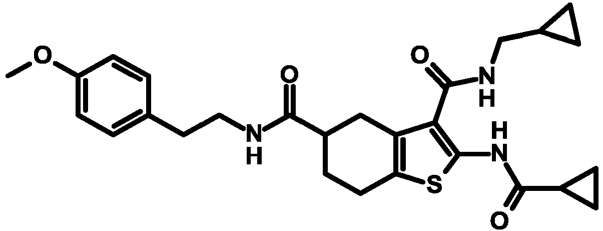

### 2-(Cyclopropanecarbonylamino)-N3-(cyclopropylmethyl)-N5-[2-(4-methoxyphenyl)ethyl]-4,5,6,7-tetrahydrobenzothiophene-3,5-dicarboxamide

The method for *example* 47 was utilised substituting 2-(4-methoxyphenyl)ethylamine [55-81-2] (15 mg, 0.10 mmol) for 2-aminoindanhydrochloride and adding DIPEA (13 mg, 0.10 mmol) rather than DIPEA (26 mg, 0.20 mmol) to afford the *title compound* (0.028 g, 55%). LCMS (ES+) [M+H]⁺ 496.2283, RT 5.14 minutes, Purity 98% *(Method 20).*

### EXAMPLE 49

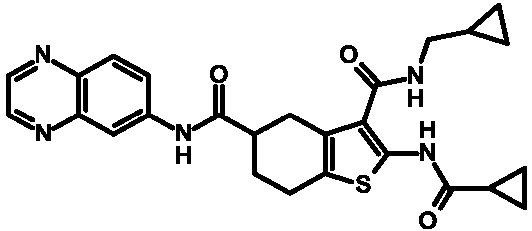

### 2-(Cyclopropanecarbonylamino)-N3-(cyclopropylmethyl)-N5-quinoxalin-6-yl-4,5,6,7-tetrahydrobenzothiophene-3,5-dicarboxamide

To the reaction vessel was added *example 40* (36 mg, 0.10 mmol), 6-aminoquinoxaline [6298-37-9] (15 mg, 0.10 mmol), COMU (53 mg, 0.12 mmol), DCM (1.3 mL) and DIPEA (17 µL, 0.10 mmol). The reaction mixture was stirred at r.t. overnight. To the reaction mixture was added water (1.2 mL) and the mixture was filtered through a phase separator. The aqueous phase was further extracted with DCM (2 x 400 µL), the organic phases were combined and concentrated *in vacuo* to yield the crude product which was purified by preparative HPLC (basic) to afford the *title compound* (0.0092g, 19%). LCMS (ES+) [M+H]⁺ 490.1892, RT 4.72 minutes *(Method 20).*

### EXAMPLE 50

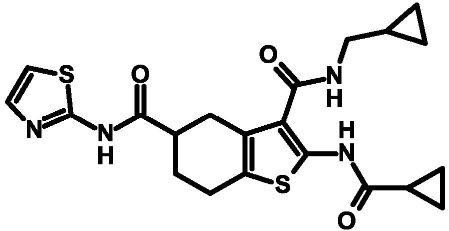

### 2-(Cyclopropanecarbonylamino)-N3-(cyclopropylmethyl)-N5-thiazol-2-yl-4,5,6,7-tetrahydrobenzothiophene-3,5-dicarboxamide

The method for *example* 49 was utilised substituting 2-aminothiazole [96-50-4] (10 mg, 0.10 mmol) for 6-aminoquinoxaline [6298-37-9] to afford the *title compound* (0.0032 g, 7.2%). LCMS (ES+) [M+H]⁺ 445.1337, RT 4.84 minutes, Purity 93.51% *(Method 20).*

### EXAMPLE 51

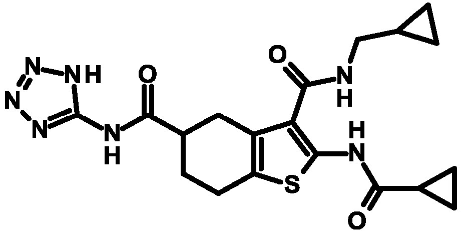

### 2-(Cyclopropanecarbonylamino)-N3-(cyclopropylmethyl)-N5-(1H-tetrazol-5-yl)-4,5,6,7-tetrahydrobenzothiophene-3,5-dicarboxamide

The method for *example* 49 was utilised substituting 5-amino-1H-tetrazole [4418-61-5] (8.5 mg, 0.10 mmol) for 6-aminoquinoxaline [6298-37-9] to afford the *title compound* (0.0019 g, 4.5%). LCMS (ES+) [M+H]⁺ 430.1638, RT 4.18 minutes, purity 81.79% *(Method 20).*

### EXAMPLE 52

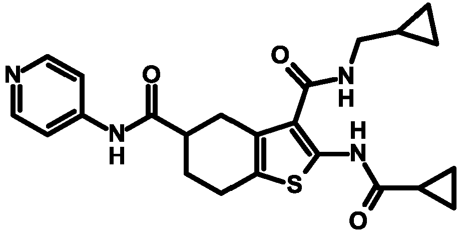

### 2-(Cyclopropanecarbonylamino)-N3-(cyclopropylmethyl)-N5-(4-pyridyl)-4,5,6,7-tetrahydrobenzothiophene-3,5-dicarboxamide

The method for *example* 49 was utilised substituting 4-aminopyridine [504-24-5] (9.5 mg, 0.10 mmol) for 6-aminoquinoxaline [6298-37-9] to afford the *title compound* (0.0075 g, 17%). LCMS (ES+) [M+H]⁺ 439.1781, RT 3.56 minutes, purity 96.16% *(Method 20).*

### EXAMPLE 53

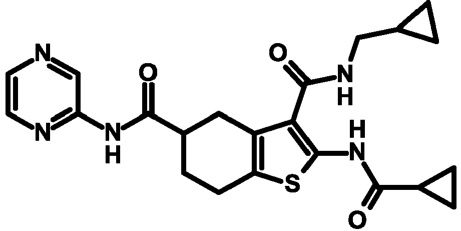

### 2-(Cyclopropanecarbonylamino)-N3-(cyclopropylmethyl)-N5-pyrazin-2-yl-4,5,6,7-tetrahydrobenzothiophene-3,5-dicarboxamide

The method for *example* 49 was utilised substituting 2-aminopyrazine [5049-61-6] (9.5 mg, 0.10 mmol) for 6-aminoquinoxaline [6298-37-9] to afford the *title compound* (0.0018 g, 4.1%). LCMS (ES+) [M+H]+ 439.1781, RT 3.56 minutes, Purity 96.16% *(Method 20).*

### EXAMPLE 54

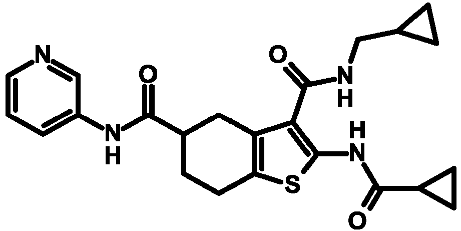

### 2-(Cyclopropanecarbonylamino)-N3-(cyclopropylmethyl)-N5-(3-pyridyl)-4,5,6,7-tetrahydrobenzothiophene-3,5-dicarboxamide

The method for *example* 49 was utilised substituting 3-aminopyridine [462-08-8] (9.4 mg, 0.10 mmol) for 6-aminoquinoxaline [6298-37-9] to afford the *title compound* (0.011 g, 24%). LCMS (ES+) [M+H]⁺ 439.1788, RT 3.89 minutes, purity 91.94% *(Method 20).*

### EXAMPLE 55

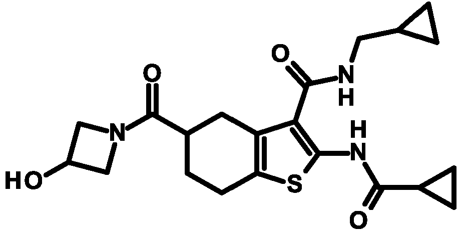

### 2-(Cyclopropanecarbonylamino)-N-(cyclopropylmethyl)-5-(3-hydroxyazetidine-1-carbonyl)-4,5,6,7-tetrahydrobenzothiophene-3-carboxamide

The method for *example* 49 was utilised substituting 3-hydroxyazetidine hydrochloride [18621-18-6] (11 mg, 0.10 mmol) for 6-aminoquinoxaline [6298-37-9] and adding an additional equivalent of DIPEA (13 mg, 0.10 mmol). The reaction mixture afforded the *title compound* (0.028 g, 66%). LCMS (ES+) [M+H]⁺ 418.1791, RT 3.97 minutes, purity 98.93% *(Method 20).*

### EXAMPLE 56

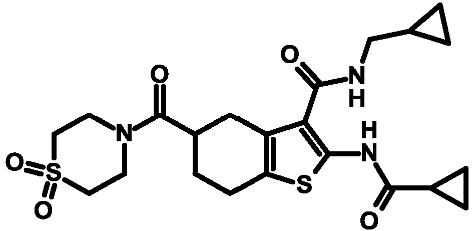

### 2-(Cyclopropanecarbonylamino)-N-(cyclopropylmethyl)-5-(1,1-dioxo-1,4-thiazinane-4-carbonyl)-4,5,6,7-tetrahydrobenzothiophene-3-carboxamide

The method for *example* 49 was utilised substituting thiomorpholine 1,1-dioxide [39093-93-1] (14 mg, 0.10 mmol) for 6-aminoquinoxaline [6298-37-9] to afford the *title compound* (0.026 g, 55%). LCMS (ES+) [M+H]⁺ 430.1638, RT 4.18 minutes, purity 81.79% *(Method 20).*

### EXAMPLE 57

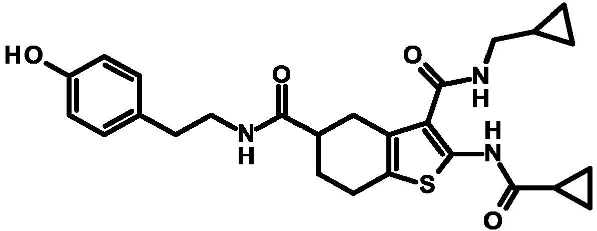

### 2-(Cyclopropanecarbonylamino)-N3-(cyclopropylmethyl)-N5-[2-(4-hydroxyphenyl)ethyl]-4,5,6,7-tetrahydrobenzothiophene-3,5-dicarboxamide

To *example 40* (36 mg, 0.10 mmol), COMU (53 mg, 0.12 mmol) and DCM (1 mL) was added tyramine [51-67-2] (14 mg, 0.10 mmol) and a solution of DIPEA (13 mg, 0.10 mmol) in DCM (300 µL). The reaction mixture was stirred at r.t. overnight and to the mixture was added DCM (2 mL) and water (4 mL) and the mixture was filtered through a phase separation cartridge. The aqueous phase was further extracted with DCM (2 x 2 mL). The organic phases were combined and concentrated in *vacuo* to yield the crude product which was purified by reverse phase HPLC (basic) to afford the *title compound* (0.017 g, 34 %). LCMS (ES+) [M+H]⁺ 481.2, RT 4.14 minutes, purity 93.89% *(Method 20).*

### EXAMPLE 58

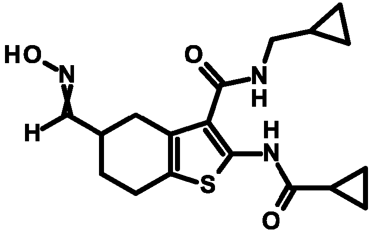

### 2-(cyclopropanecarbonylamino)-N-(cyclopropylmethyl)-5-(hydroxyiminomethyl)-4,5,6,7-tetrahydrobenzothiophene-3-carboxamide

*Intermediate 59* (1.46 g, 4.21 mmol) was dissolved in EtOH (30 mL) and hydroxylamine (1.11 g, 33.7 mmol) was added to the reaction mixture. The reaction mixture was stirred at r.t. for 16 h and then diluted with 1N aqueous hydrochloric acid solution and extracted with DCM (20 mL). The organic phase was dried (MgSO₄), filtered and the filtrate was concentrated *in vacuo* to give the crude product (1.2 g) as a yellow solid. The oxime was purified by flash column chromatography on silica (gradient elution with 0-100 % EtOAc in heptane) to afford the *title compound* (1.0 g, 68%) as a white solid. δ_{H} (500 MHz, DMSO-*d*₆) 11.15 (s, 1H), 10.54 (s, 1H), 7.68 (t, J 5.6 Hz, 1H), 7.38 (d, J 5.2 Hz, 1H), 3.16-3.11 (m, 2H), 2.79 (dd, J 15.9, 4.5 Hz, 1H), 2.68-2.63 (m, 3H), 2.59-2.57 (m, 1H), 2.06 - 1.95 (m, 1H), 1.94 - 1.84 (m, 1H), 1.72 - 1.59 (m, 1H), 1.05-1.03 (m, 1H), 0.90 - 0.78 (m, 4H), 0.46 - 0.36 (m, 2H), 0.26-0.21 (m, 2H). LCMS (ES+) [M+H]⁺ 362.18, RT 2.87 minutes, 100.0% purity (*Method 10*).

### EXAMPLE 59

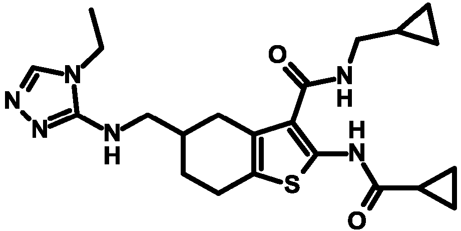

### 2-(Cyclopropanecarbonylamino)-N-(cyclopropylmethyl)-5-[[(4-ethyl-1,2,4-triazol-3-yl)aminolmethyl]-4,5,6,7-tetrahydrobenzothiophene-3-carboxamide

*Intermediate 61* (300 mg, 0.72 mmol) and formic hydrazide [624-84-0] (129 mg, 2.15 mmol) were dissolved in DMF (3 mL). Mercury dichloride [7487-94-7] (584 mg, 2.15 mmol) was added to the reaction mixture and the mixture stirred for 5 minutes. Triethylamine (0.3 mL, 2.15 mmol) was added to the reaction mixture which was heated at 90 °C for 2 h. The mixture was diluted with DCM (20 mL), filtered, and the filtrate was concentrated *in vacuo* to yield crude material which was extracted with EtOAc (3 x 20 mL) and washed with water (20 mL). The organics phase was separated, dried (MgSO₄), filtered and the filtrate was concentrated *in vacuo* to afford the crude product which was purified by flash column chromatography on silica (gradient elution with 0-100% EtOAc followed by 0-50% MeOH in DCM) to afford the product which was further purified by preparative HPLC to afford the *title compound* (21 mg, 6.4%). δ_{H} (500 MHz, DMSO-*d*₆) 11.20 (s, 1H), 7.94 (s, 1H), 7.61 (s, 1H), 6.05 (t, *J* 5.5 Hz, 1H), 3.77 (q, *J* 7.2 Hz, 2H), 3.22-3.16 (m, 3H), 3.11-3.08 (m, 1H), 2.76-2.72 (m, 2H), 2.65 - 2.55 (m, 1H), 2.45 - 2.32 (m, 1H), 2.10-2.06 (s, 1H), 2.03 - 1.95 (m, 1H), 1.92-1.88 (m, 1H), 1.48-1.42 (m, 1H), 1.23 (t, *J* 7.2 Hz, 3H), 1.04-1.00 (m, 1H), 0.87 - 0.78 (m, 4H), 0.45 - 0.34 (m, 2H), 0.24-0.20 (m, 2H). LCMS : RT 6.79 minutes, MH+ 443.3 (*Method 19*).

### EXAMPLE 60

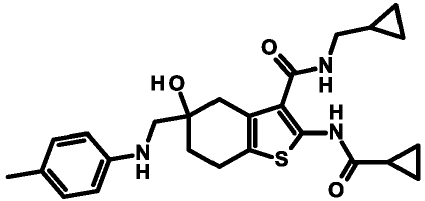

### 2-(Cyclopropanecarbonylamino)-N-(cyclopropylmethyl)-5-hydroxy-5-[(4-methylanilino)methyl]-6,7-dihydro-4H-benzothiophene-3-carboxamide

To *intermediate 63* (400 mg, 1.15 mmol) was added p-toluidine [106-49-0] (150 mg, 1.40 mmol) in EtOH (1.16 mL). The reaction mixture was heated to 70 °C overnight before concentrating *in vacuo* to yield the crude product which was purified by flash column chromatography on silica (gradient elution with DCM/MeOH [NH₄OH 10%] 5% to 10%) to afford the *title compound* (272 mg, 46%). ¹H NMR (400 MHz, Chloroform-*d*) δ 12.15 (s, 1H), 6.99 (d, *J* = 7.9 Hz, 2H), 6.60 (d, *J* = 7.9 Hz, 2H), 5.81 (s, 1H), 3.92 (s, 1H), 3.35 - 3.12 (m, 4H), 2.83 (m, 4H), 2.23 (s, 3H), 2.16 (d, *J* = 1.1 Hz, 1H), 2.04 (dt, *J* = 12.1, 6.0 Hz, 1H), 1.91 (dt, *J* = 13.6, 7.4 Hz, 1H), 1.66 (tt, *J* = 8.3, 4.7 Hz, 1H), 1.11 (t, *J* = 3.4 Hz, 2H), 0.98 (dd, *J* = 9.5, 5.2 Hz, 1H), 0.93 - 0.85 (m, 2H), 0.55 - 0.47 (m, 2H), 0.21 (d, *J* = 4.8 Hz, 2H). LCMS (ES+) [M+H]⁺ 454.22, RT 2.72 minutes, purity 91.65% (*Method 9*).

### EXAMPLE 61

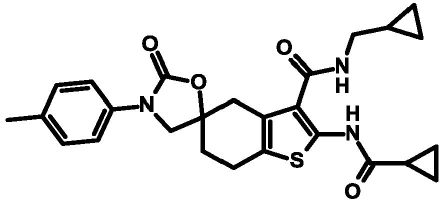

### 2-(Cyclopropanecarbonylamino)-N-(cyclopropylmethyl)-2'-oxo-3'-(p-tolyl)spiro[6,7-dihydro-4H-benzothiophene-5,5'-oxazolidine]-3-carboxamide

To *example 60* (75 mg, 0.17 mmol) in MeCN was added 1,8-diazabicyclo[5.4.0]undec-7-ene [6674-22-2] (67 mg, 0.43 mmol) and 1,1'-carbonyldiimidazole [530-62-1] (40.2 mg, 0.248 mmol) and the reaction mixture was heated at 80 °C overnight. Further 1,1'-carbonyldiimidazole [530-62-1] (20 mg, 0.123 mmol) was added and the reaction was heated at 80 °C before leaving to cool for 2 nights. The reaction mixture was concentrated *in vacuo* and purified by preparative TLC to afford the *title compound* (18 mg, 23%). LCMS (ES+) [M+H]⁺ 480.15, RT 2.98 minutes, purity 93.32% *(Method 8).*

### EXAMPLE 62

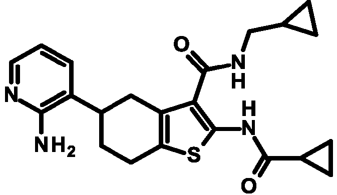

### 5-(2-Amino-3-pyridyl)-2-(cyclopropanecarbonylamino)-N-(cyclopropylmethyl)-4,5,6,7-tetrahydrobenzothiophene-3-carboxamide

A sealed tube was charged with *intermediate 64* (146 mg, 0.26 mmol), {2-[(*tert-*butoxycarbonyl)amino]pyridin-3-yl}boronic acid [863753-35-9] (94 mg, 0.39 mmol) and cesium carbonate (130 mg, 0.40 mmol) and was placed under vacuum for 10 minutes. The tube was backfilled with nitrogen and the solids suspended in 1,4-dioxane (1.05 mL). The mixture was degassed with nitrogen for 5 minutes before sealing and heating at 110 °C for 16 h. The reaction mixture was cooled to r.t. and partitioned between EtOAc (20 mL) and saturated aqueous sodium hydrogen carbonate solution (10 mL). The organic phase was separated and the aqueous phase extracted with EtOAc (2 x 20 mL). The combined organic phases were washed with brine (20 mL), dried (MgSO₄), filtered and concentrated in *vacuo* to afford the crude product (127 mg) as a dark orange semi-solid. The crude product was purified by flash column chromatography on silica (gradient elution with 0 to 100% EtOAc in heptane) to afford the product (14 mg) as a red gum which was further purified by preparative HPLC (low pH) to afford the *title compound* (2.2 mg, 2%) as a white powder. δ_{H} (500 MHz, DMSO-*d*₆) 11.20 (s, 1H), 7.81 (dd, *J* 4.9, 1.7 Hz, 1H), 7.63 (dd, *J* 7.4, 1.4 Hz, 1H), 7.33 (dd, *J* 7.4, 1.4 Hz, 1H), 6.55 (dd, *J* 7.4, 4.9 Hz, 1H), 5.75 (s, 2H), 3.19 - 3.13 (m, 1H), 3.12 - 3.06 (m, 1H), 2.98 - 2.86 (m, 2H), 2.86 - 2.78 (m, 1H), 2.76 - 2.64 (m, 2H), 2.03 - 1.94 (m, 1H), 1.94 - 1.86 (m, 1H), 1.87 - 1.77 (m, 1H), 1.04 - 0.95 (m, 1H), 0.92 - 0.77 (m, 4H), 0.40 - 0.34 (m, 2H), 0.22 - 0.16 (m, 2H). LCMS ES+ [M+H]⁺ 411.2, RT 2.00 minutes, purity 100% (*Method 10*).

### EXAMPLE 63

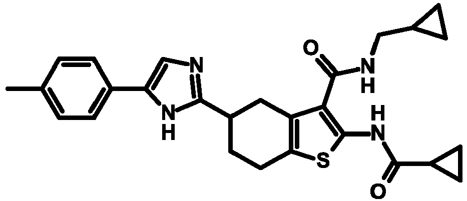

### 2-(Cyclopropanecarbonylamino)-N-(cyclopropylmethyl)-5-[5-(p-tolyl)-1H-imidazol-2-yl]-4,5,6,7-tetrahydrobenzothiophene-3-carboxamide

*Intermediate 65* (100 mg, 0.2 mmol) was suspended in toluene (2 mL) and ammonium acetate (0.31 g, 4.04 mmol) and acetic acid (0.24 ml, 4.04 mmol) were added. The reaction mixture was heated at 120 °C for 1.5 h. The reaction mixture was diluted with EtOAc and washed with water followed by brine. The organic phase was separated, dried (Na₂SO₄), filtered and concentrated *in vacuo* to yield the crude product which was purified by preparative HPLC (basic) to afford the product which was further purified by re-crystallisation with IPA/water (2 x) to afford the *title compound* (16 mg, 17%) as a tautomeric mixture (20:80). δ_{H} (500 MHz, DMSO-*d*₆) 12.03 (s, 0.2H-isomer 1), 11.83 (s, 0.8H-isomer 2), 11.15 (s, 1H), 7.72 (s, 1H), 7.65 (d, *J* 8.0 Hz, 1.6H-isomer 2), 7.51 (d, *J* 7.6 Hz, 0.4H-isomer 1), 7.46 (d, *J* 1.7 Hz, 0.8H-isomer 2), 7.20 (d, *J* 8.2 Hz, 0.4H-isomer 1), 7.17 (s, 0.2H-isomer 1), 7.13 (d, *J* 8.0 Hz, 1.6H-isomer 2), 3.22 - 2.94 (m, 5H), 2.81-2.77 (m, 2H), 2.32-2.27 (m, 4H), 1.95-1.90 (m, 2H), 1.06-1.03 (m, 1H), 0.90 - 0.79 (m, 4H), 0.43-0.39 (m, 2H), 0.23 (s, 2H) as mixture of tautomers (20:80). LCMS (ES+) [M+H]⁺ 475.05, RT 3.48 minutes, purity 100% (*Method 5*).

### EXAMPLE 64 and 65

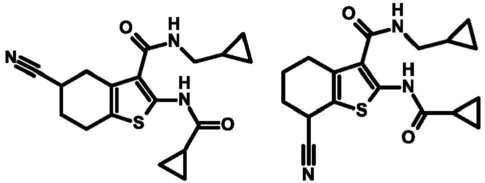

### Example 64

### 5-Cyano-2-(cyclopropanecarbonylamino)-N-(cyclopropylmethyl)-4,5,6,7-tetrahydrobenzothiophene-3-carboxamide

### Example 65

### 7-Cyano-2-(cyclopropanecarbonylamino)-N-(cyclopropylmethyl)-4,5,6,7-tetrahydrobenzothiophene-3-carboxamide

To 3-oxocyclohexane-1-carbonitrile [17983-30-1] (63 mg, 0.49 mmol) and 2-cyano-N-(cyclopropylmethyl)acetamide [114153-25-2] (79 mg, 0.55 mmol) in THF (1 mL) was added ammonium acetate (52 mg, 0.675 mmol) and acetic acid (30 µL). The reaction mixture was stirred at 70 °C for 3 h. To the reaction mixture was added sulphur [7704-34-9] (26 mg, 0.81 mmol), EtOH (3 mL) and triethylamine (210 µL, 1.49 mmol) and the mixture was warmed at 65 °C overnight and the mixture was concentrated *in vacuo.* To the residue was added EtOAc (5 mL) and 10% potassium bisulfate (2 mL), the organic layer was separated and washed with saturated sodium hydrogen carbonate solution followed by brine. The organic phase was separated, dried (Na₂SO₄), filtered and concentrated *in vacuo* to yield a crude brown oil. To the residue was added DCM (2.5 mL) and DIPEA (130 µL, 0.75 mmol) and the mixture was cooled to 0 °C in an ice-bath and a solution of cyclopropanecarbonyl chloride [4023-34-1] (75 mg, 0.70 mmol) in DCM (0.5 mL) was added. The reaction mixture was stirred at r.t. for 4 h before addition of saturated sodium hydrogen carbonate solution. The organic phase was separated with a phase separation cartridge and the aqueous was further extracted with DCM (2 mL). The combined organic layers were concentrated *in vacuo* to yield the crude products which were purified by preparative HPLC (basic), to afford *title compound example 64* (15 mg, 9%). LCMS [M+H]⁺ 344.10, RT 4.11 minutes, (Method13). Preparative HPLC purification (acidic) of impure fractions from the initial purification (basic) afforded *title compound example 65* (1.4 mg, 0.8%). LCMS [M+H]⁺ 344.10, [M+Na]⁺ 366.13, RT 4.60 minutes, 100.0% purity *(Method 21).* LCMS [M+H]⁺ 344.10, [M+Na]⁺ 366.08, [M-H]⁻ 342.06, RT 4.30 minutes, 97.08% purity *(Method 13).*

### EXAMPLES 66 and 67

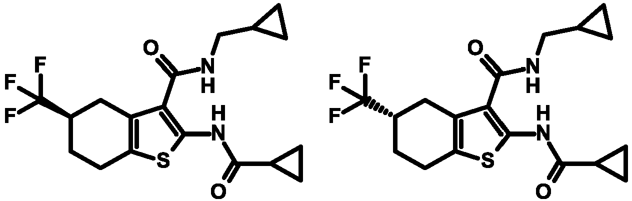

### Example 66

### (5R)-2-(Cyclopropanecarbonylamino)-N-(cyclopropylmethyl)-5-(trifluoromethyl)-4,5,6,7-tetrahydrobenzothiophene-3-carboxamide

### Example 67

### (5S)-2-(Cyclopropanecarbonylamino)-N-(cyclopropylmethyl)-5-(trifluoromethyl)-4,5,6,7-tetrahydrobenzothiophene-3-carboxamide

*Intermediate 70* (23 mg) was purified by chiral separation on preparative HPLC (solvent CO₂+20% EtOH) to afford the two separated *title chiral compounds* as *title compound example 66* (7.5 mg, 33%) and *title compound example 67* (8.2 mg, 36%). *Example 66* LCMS [M-H]⁻ 385.0, RT 2.683 minutes *(Method 2). Example 67* LCMS (ES+) [M+H]⁺ 387.8, RT 2.671 minutes *(Method 2).*

### EXAMPLE 68

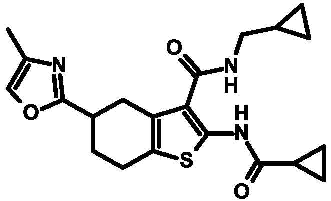

### 2-(Cyclopropanecarbonylamino)-N-(cyclopropylmethyl)-5-(4-methyloxazol-2-yl)-4,5,6,7-tetrahydrobenzothiophene-3-carboxamide

*Intermediate 71* (80 mg, 0.19 mmol) was dissolved in acetic acid (3 mL) and acetic acid ammoniate (1:1) (59 mg, 0.76 mmol) was added and the reaction mixture was stirred at 100 °C for 24 h. The acetic acid was removed *in vacuo* and EtOAc (50 mL) and water (50 mL) were added. The organic phases were separated and dried (MgSO₄), filtered and concentrated *in vacuo* to yield the crude product which was purified by preparative HPLC (low pH) to afford the *title compound* (20 mg, 26%) as an off-white solid. δ_{H} (500 MHz, CD₃OD) 7.56 (s, 1H), 3.29 - 3.19 (m, 3H), 3.16 - 3.09 (m, 1H), 3.08 - 2.98 (m, 1H), 2.88 - 2.76 (m, 2H), 2.40 - 2.31 (m, 1H), 2.15 (s, 3H), 2.10 - 1.99 (m, 1H), 1.84 - 1.76 (m, 1H),1.17 - 1.07 (m, 1H), 1.02 - 0.97 (m, 2H), 0.96 - 0.91 (m, 2H), 0.55 - 0.49 (m, 2H), 0.31 - 0.26 (m, 2H). LCMS (ES+) [M+H]⁺ 400.2, RT 3.52 minutes, purity 100% *(Method 10).* Peak was split (51.23% and 48.77%).

### EXAMPLE 69

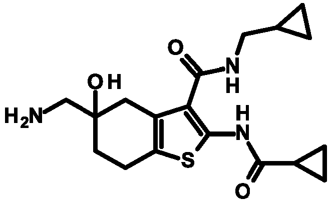

### 5-(Aminomethyl)-2-(cyclopropanecarbonylamino)-N-(cyclopropylmethyl)-5-hydroxy-6,7-dihydro-4H-benzothiophene-3-carboxamide

*Intermediate 63* (450 mg, 1.30 mmol) and ammonia (1.43 g, 13.0 mmol) were heated at 70 °C overnight. The reaction mixture was concentrated *in vacuo* and the residue was purified by flash column chromatography on silica (gradient elution with DCM/MeOH (NH₄OH 10%) 5% to 10%) to afford the *title compound* (292 mg, 62%) as a white solid. LCMS (ES+) [M+H]⁺ 364.18, RT 1.69 minutes *(Method 8).*

### EXAMPLE 70

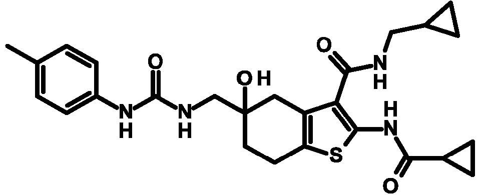

### 2-(Cyclopropanecarbonylamino)-N-(cyclopropylmethyl)-5-hydroxy-5-[(p-tolylcarbamoylamino)methyl]-6,7-dihydro-4H-benzothiophene-3-carboxamide

To *example 69* (292 mg, 0.80 mmol) in DCM (0.80 mL) was added triethylamine (123 mg, 1.21 mmol). The mixture was cooled to 0 °C and p-tolyl isocyanate [622-58-2] (162 mg, 1.21 mmol) was added dropwise. The reaction was allowed to warm to r.t. and was stirred overnight before concentrating *in vacuo.* To the crude material was added EtOH (10 mL) and the mixture was heated in the microwave at 150 °C for 20 minutes. The reaction mixture was concentrated *in vacuo* and purified by flash column chromatography on silica (gradient elution with DCM/0-10% MeOH [NH₄OH 10%] to afford the *title compound* (0.0043 g, 0.87%). LCMS (ES+) [M+H]⁺ 497.2, RT 2.36 minutes, purity 87.07% *(Method 9).*

### EXAMPLE 71

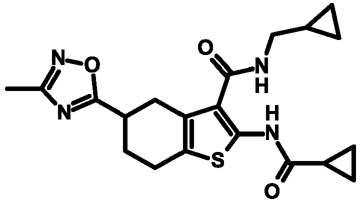

### 2-(Cyclopropanecarbonylamino)-N-(cyclopropylmethyl)-5-(3-methyl-1,2,4-oxadiazol-5-yl)-4,5,6,7-tetrahydrobenzothiophene-3-carboxamide

To a solution of *example 40* (30 mg, 0.083 mmoL) and N-hydroxyacetamidine [22059-22-9] (9 mg, 0.12 mmoL) in anhydrous DCM (2 mL) was added EDCI (25 mg, 0.13 mmoL) and the resulting solution was stirred at r.t. Further N-hydroxyacetamidine [22059-22-9] (9 mg, 0.12 mmoL) and EDCI (25 mg, 0.13 mmoL) were added and stirring was continued for a further 24 h. The solvent was removed *in vacuo* and the residue was taken up in toluene and heated to 100 °C for 5 h. The reaction mixture was diluted with EtOAc (10 mL) and water (10 mL). The layers were separated and the aqueous layer was extracted with further EtOAc (10 mL). The combined organics were washed with brine (10 mL), dried (MgSO₄), filtered and concentrated *in vacuo* to yield the crude product which was purified by flash column chromatography on silica (gradient elution with EtOAc/heptane 0 to 100%) to afford the *title compound* (11 mg, 33%) as a white solid. δ_{H} (250 MHz, Chloroform-d) 12.07 (s, 1H), 5.96 - 5.79 (m, 1H), 3.53 - 3.17 (m, 4H), 3.17 - 2.97 (m, 1H), 2.93 - 2.79 (m, 2H), 2.51 - 2.36 (m, 4H), 2.23 - 1.97 (m, 1H), 1.75 - 1.53 (m, 1H), 1.20 - 1.01 (m, 3H), 0.98 - 0.81 (m, 2H), 0.63 - 0.49 (m, 2H), 0.36 - 0.18 (m, 2H). LCMS (ES+) [M+H]⁺ 401, RT 3.36 minutes *(Method 10).*

### EXAMPLE 72

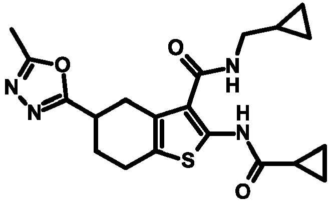

### 2-(Cyclopropanecarbonylamino)-N-(cyclopropylmethyl)-5-(5-methyl-1,3,4-oxadiazol-2-yl)-4,5,6,7-tetrahydrobenzothiophene-3-carboxamide

*Intermediate 72* (95 mg, 0.25 mmol) was dissolved in acetic acid (2 mL) and 1,1,1-triethoxyethane [78-39-7] (0.23 ml, 1.26 mmol) was added. The reaction mixture was stirred at 120 °C for 3 h before concentrating *in vacuo.* The crude product was purified by preparative HPLC (low pH) to afford the *title compound* (74 mg, 73%) as a white solid. δ_{H} (500 MHz, CD₃OD) 3.42 - 3.34 (m, 1H), 3.29 - 3.15 (m, 3H), 3.11 - 3.03 (m, 1H), 2.90 - 2.80 (m, 2H), 2.53 (s, 3H), 2.45 - 2.37 (m, 1H), 2.14 - 2.02 (m, 1H), 1.84 - 1.76 (m, 1H), 1.16 - 1.06(m, 1H), 1.02 - 0.96 (m, 2H), 0.96 - 0.91 (m, 2H), 0.58 - 0.48 (m, 2H), 0.33 - 0.24 (m, 2H). LCMS (ES+) [M+H]⁺ 401.2, RT 2.93 minutes, purity 99% *(Method 10),* peak split (50.19% and 48.91%).

### EXAMPLE 73

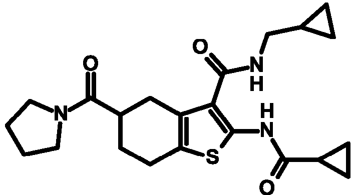

### 2-(Cyclopropanecarbonylamino)-N-(cyclopropylmethyl)-5-(pyrrolidine-1-carbonyl)-4,5,6,7-tetrahydrobenzothiophene-3-carboxamide

*Example 40* (50 mg, 0.13 mmol) was dissolved in DCM (4 mL) and EDCI (40 mg, 0.20 mmol) was added and the reaction mixture was stirred for 1 h. Pyrrolidine [123-75-1] (0.023 mL, 0.28 mmol) was added and the reaction mixture was stirred at r.t. for 16 h. The mixture was diluted with DCM (20 mL) and washed with water, 1M aqueous hydrochloric acid solution and saturated sodium hydrogen carbonate solution. The organic layer was washed with brine, dried (MgSO₄), filtered and concentrated *in vacuo.* The crude residue was purified by preparative HPLC (acidic) to afford the *title compound* (22 mg, 38%) as a white solid. δ_{H} (500 MHz, CD₃OD) 3.60 (t, *J* 6.8 Hz, 2H), 3.49 - 3.43 (m, 2H), 3.30 - 3.25 (m, 1H), 3.21 - 3.15 (m, 1H), 2.94 - 2.75 (m, 5H), 2.13 - 2.06 (m, 1H), 2.04 - 1.97 (m, 2H), 1.94 - 1.84 (m, 3H),1.82 - 1.75 (m, 1H), 1.14 - 1.05 (m, 1H), 1.02 - 0.90 (m, 4H), 0.55 - 0.49 (m, 2H), 0.30 - 0.25 (m, 2H). LCMS (ES+) [M+H]⁺ 416.2, RT 3.10 minutes, purity 100% *(Method 10).*

### EXAMPLE 74

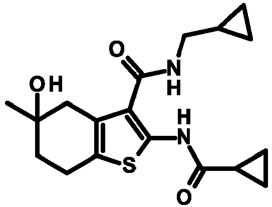

### 2-(Cyclopropanecarbonylamino)-N-(cyclopropylmethyl)-5-hydroxy-5-methyl-6,7-dihydro-4H-benzothiophene-3-carboxamide

*Intermediate 63* (50 mg, 0.14 mmol) in THF (0.30 mL) was cooled to 0 °C and lithium triethylborohydride [22560-16-3] (0.11 mL, 0.19 mmol) was added dropwise. After stirring for 5 minutes the reaction was allowed to warm to r.t. and stirred over the weekend. Further lithium triethylborohydride [22560-16-3] (0.11 mL, 0.19 mmol) was added dropwise at 0 °C and the reaction mixture was allowed to warm to r.t. and stirred for 2 h. The reaction mixture was quenched with water at 0 °C and was extracted with DCM. The organic phase was separated, dried (MgSO₄), filtered and concentrated *in vacuo* to yield the crude product which was purified by TLC prep (eluted with DCM/MeOH (NH₄OH10%) 5%) to afford the *title compound* (20 mg, 40%). LCMS (ES+) [M+H]⁺ 249.20, RT 2.16 minutes, purity 98.83% *(Method 8).*

### EXAMPLE 80

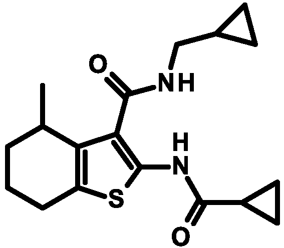

### 2-(Cyclopropanecarbonylamino)-N-(cyclopropylmethyl)-4-methyl-4,5,6,7-tetrahydrobenzothiophene-3-carboxamide

To a solution of *intermediate 80* (181 mg, 0.65 mmol) in DCM (4 mL) was added cyclopropylmethylamine [2516-47-4] (92 mg, 1.30 mmol) followed by EDCI (186 mg, 0.97 mmol). The reaction was stirred at r.t. for 45 minutes before leaving for 3 days at r.t. The reaction was poured into EtOAc, washed with saturated sodium hydrogen carbonate solution (2 x), 0.1M aqueous hydrochloric acid solution and dried (MgSO₄). The mixture was filtered and concentrated *in vacuo* to yield a brown oil/gum which was purified by flash column chromatography on silica (gradient elution with 0 - 20% EtOAc in heptane) to afford the *title compound* (43 mg, 20%). δ_{H} (500 MHz, DMSO-*d*₆) 10.64 (s, 1H), 8.04 (s, 1H), 3.24 - 3.10 (m, 2H), 3.04 (dt, *J* 13.0, 6.2 Hz, 1H), 2.62 - 2.54 (m, 2H), 1.97 - 1.87 (m, 1H), 1.87 - 1.64 (m, 3H), 1.58 - 1.42 (m, 1H), 1.05 (d, *J* 6.9 Hz, 4H), 0.81 (d, *J* 4.9 Hz, 4H), 0.47 - 0.37 (m, 2H), 0.31 - 0.14 (m, 2H).

### EXAMPLE 81

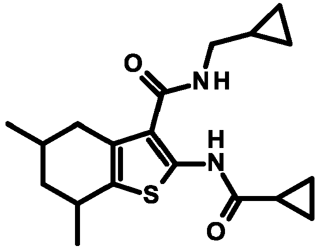

### 2-(Cyclopropanecarbonylamino)-N-(cyclopropylmethyl)-5,7-dimethyl-4,5,6,7-tetrahydrobenzothiophene-3-carboxamide

A mixture of *intermediate 83* (103 mg, 0.35 mmol), EDCI (67 mg, 0.35 mmol) and cyclopropylmethylamine [2516-47-4] (37 mg, 0.52 mmol) in DCM (5 mL) were stirred overnight. Water (5 mL) was added to the reaction mixture and the mixture was passed through a phase separator cartridge and the organic phase concentrated *in vacuo* onto silica. The reaction mixture was purified by flash column chromatography on silica (gradient elution with 5% - 40% EtOAc in *iso*hexane) to afford the *title compound* (31 mg, 26%) as a white solid which was freeze-dried overnight from MeCN/water. δ_{H} (400 MHz, DMSO-*d*₆) 11.12 (s, 1H), 7.65 (s, 1H), 3.15-3.23 (m, 1H), 3.05-3.12 (m, 1H), 2.80-2.90 (m, 1H), 2.60-2.68 (m, 1H), 2.20-2.31 (m, 1H), 1.84-1.93 (m, 2H), 1.70-1.82 (m, 1H), 1.18 (d, *J* 6.7 Hz, 3H), 0.97-1.07 (m, 2H), 1.03 (d, *J* 6.4 Hz, 3H), 0.78-0.89 (m, 4H), 0.40-0.45 (m, 2H), 0.21-0.25 (m, 2H).

### EXAMPLES 82 and 83

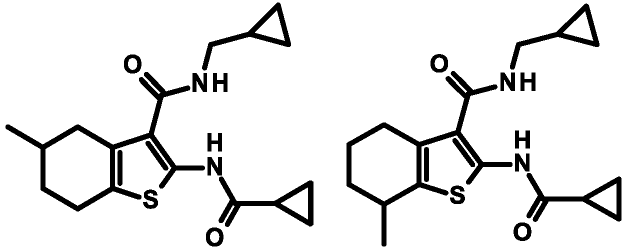

### Example 82

### 2-(Cyclopropanecarbonylamino)-N-(cyclopropylmethyl)-5-methyl-4,5,6,7-tetrahydrobenzothiophene-3-carboxamide

### Example 83

### 2-(cyclopropanecarbonylamino)-N-(cyclopropylmethyl)-7-methyl-4,5,6,7-tetrahydrobenzothiophene-3-carboxamide

The method for *example 81* was utilised substituting a ^{~}25:1 mixture of *intermediates 88* 5-isomer and 89 7-isomer (98 mg, 0.350 mmol) for *intermediate 83* to afford the *title compounds* (34 mg, 0.10 mmol) as a ~60:1 mixture of *example 82,* 5-isomer *: example 83,* 7-isomer. *Example 82:* δ_{H} (400 MHz, DMSO-*d*₆) 11.19 (s, 1H), 7.57-7.63 (m, 1H), 3.15-3.22 (m, 1H), 3.07-3.13 (m, 1H), 2.56-2.73 (m, 3H), 2.28 (m, 1H), 1.70-1.92 (m, 3H), 1.30-1.41 (m, 1H), 1.00-1.10 (m, 1H), 1.03 (d, *J* 6.6 Hz, 3H), 0.79-0.88 (m, 4H), 0.40-0.45 (m, 2 H), 0.21-0.25 (m, 2 H). ). LCMS [M+H]⁺ 333.8, RT 2.72 minutes, (*Method 2*), LCMS [M+H]⁺ 333.8, RT 2.70 minutes, (*Method 3*)*.*

### EXAMPLE 84

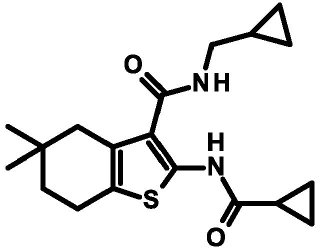

### 2-(Cyclopropanecarbonylamino)-N-(cyclopropylmethyl)-5,5-dimethyl-6,7-dihydro-4H-benzothiophene-3-carboxamide

To a solution of *intermediate 92* (150 mg, 0.51 mmoL) and cyclopropylmethanamine [2516-47-4] (0.067 mL, 0.77 mmoL) in DCM (5 mL) was added EDCI (157 mg, 0.82 mmoL) and the resulting solution was stirred at r.t. under an atmosphere of nitrogen. After stirring for 20 h the mixture was diluted with DCM (15 mL) and 1M aqueous hydrochloric acid solution (10 mL). The layers were separated and the aqueous phase was further extracted with DCM (10 mL). The combined organics were washed with brine (10 mL), dried (MgSO₄), filtered and concentrated *in vacuo.* The crude material was purified by flash column chromatography on silica (elution with 100% DCM followed by a gradient of EtOAc 0 to 50% in DCM) to afford the *title compound* (60 mg, 34%) as a white solid. δ_{H} (250 MHz, Chloroform-*d*) 12.16 (s, 1H), 5.97 (s, 1H), 3.37 - 3.24 (m, 2H), 2.68 (t, *J* 6.5 Hz, 2H), 2.46 (s, 2H), 1.74 - 1.56 (m, 3H), 1.17 - 1.05 (m, 3H), 1.02 (s, 6H), 0.95 - 0.82 (m, 2H), 0.67 - 0.52 (m, 2H), 0.34 - 0.23 (m, 2H). LCMS [M+H]⁺ 347, RT 4.22 minutes, 98% purity *(Method 10).*

### EXAMPLE 85

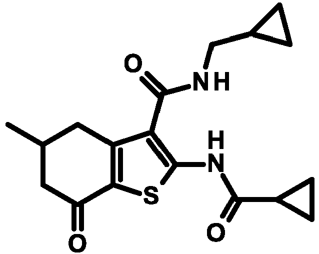

### 2-(Cyclopropanecarbonylamino)-N-(cyclopropylmethyl)-5-methyl-7-oxo-5,6-dihydro-4H-benzothiophene-3-carboxamide

To a solution of *intermediate 95* (290 mg, 0.99 mmol) and triethylamine (0.2 mL, 1.44 mmol) in DCM (5 mL) at 0 °C was added cyclopropanecarbonyl chloride [4023-34-1] (0.12ml, 1.32 mmol). The reaction mixture was stirred at 0 °C for 2 h. Further cyclopropanecarbonyl chloride [4023-34-1] (0.12 ml, 1.32 mmol) was added and the reaction was stirred for 1 h at r.t. The reaction mixture was diluted with DCM (20 mL) and 1 M aqueous hydrochloric acid solution (20 mL) was added. The organic layer was separated and the remaining aqueous phase extracted with further DCM (2 x 10 mL) and washed with water (10 mL). The combined organic phases were dried (MgSO₄), filtered and concentrated *in vacuo* and purified by flash column chromatography on silica (gradient elution with 30 - 60% EtOAc/heptane) to afford the *title compound* (90 mg, 34%) as an orange solid. δ_{H} (500 MHz, Chloroform-d) 12.31 (s, 1H), 5.90 (s, 1H), 3.37 - 3.30 (m, 2H), 3.10 (dd, *J* 15.3, 4.3 Hz, 1H), 2.64 (dd, *J* 16.3, 2.9 Hz, 1H), 2.57 (dd, *J* 15.3, 9.8 Hz, 1H), 2.51 - 2.41 (m, 1H), 2.32 (dd*, J* 16.2, 11.9 Hz, 1H), 1.74 - 1.67 (m, 1H), 1.21 (d, *J* 6.5 Hz, 3H), 1.19 - 1.15 (m, 2H), 1.14 - 1.06 (m, 1H), 0.99 - 0.94 (m, 2H), 0.65 - 0.58 (m, 2H), 0.34 - 0.29 (m, 2H).

### EXAMPLE 86

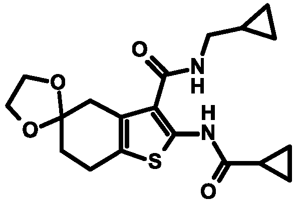

### 2'-(Cyclopropanecarbonylamino)-N-(cyclopropylmethyl)spiro[1,3-dioxolane-2,5'-6,7-dihydro-4H-benzothiophene]-3'-carboxamide

*Intermediate 34* (540 mg, 1.67 mmol) was dissolved in DMF (10 mL) and triethylamine (425 mg, 4.17 mmol) and cyclopropanemethylamine hydrochloride [7252-53-1] (290 mg, 2.67 mmol) were added prior to addition of EDCI (512 mg, 2.67 mmol) and the reaction mixture was stirred at r.t. for 72 h. The mixture was diluted with EtOAc (15 mL) and washed with water (3 x 15 mL). The organic phase was separated, dried (Na₂SO₄), filtered and the concentrated *in vacuo* to give a yellow oil which was purified by flash column chromatography on silica (gradient elution with 0-50% EtOAc/iso-hexane) to afford the *title compound* (420 mg, 67%) as a yellow gum. LCMS (ES+) [M+H]⁺ 377.0, RT 1.367 minutes, purity 98.0% (*Method 4*)*.*

### EXAMPLE 89

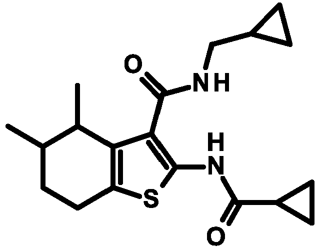

### 2-(Cyclopropanecarbonylamino)-N-(cyclopropylmethyl)-4,5-dimethyl-4,5,6,7-tetrahydrobenzothiophene-3-carboxamide

### 2-(Cyclopropanecarbonylamino)}-4,5-dimethyl-4,5,6,7-tetrahydrobenzothiophene-3-carboxylic acid

To a solution of 2,3-dimethylcyclohexanone (MFCD11223163) (2.43 g, 19.2 mmol) in EtOH (10 mL) was added ethyl cyanoacetate [105-56-6] (2.25 mL, 21.1 mmol), sulphur [7704-34-9] (0.68 g, 21.1 mmol) and diethylamine (2.97 mL, 28.8 mmol). The reaction was stirred at r.t. for 18 h followed by heating at 50 °C for 7 h. The reaction was left to cool to r.t. overnight and the mixture was concentrated *in vacuo.* The crude product was purified by flash column chromatography on silica (gradient elution with heptane/EtOAc 100:0 to 0:100) to afford *ethyl 2-amino-4,5-dimethyl-4,5,6,7-tetrahydrobenzothiophene-3-carboxylate* (1.29 g, 26%) which was used in the following step.

To a solution of *ethyl 2-amino-4,5-dimethyl-4,5,6,7-tetrahydrobenzothiophene-3-carboxylate* (1.29 g, 5.09 mmol) and DIPEA (1.68 mL, 10.17 mol) in DCM (24 mL) was added cyclopropanecarbonyl chloride [4023-34-1] (0.51 mL, 5.60 mmol). The reaction mixture was stirred at r.t. for 1.5 h before diluting with DCM (15 mL) and washing with saturated aqueous sodium hydrogen carbonate solution (2 x 30 mL). The combined organic phases were dried (brine and MgSO₄), filtered and the organic phase was concentrated *in vacuo.* The crude product was purified by flash column chromatography on silica (gradient elution with heptane/EtOAc 100:0 to 60:40) to afford *ethyl 2-(cyclopropanecarbonylamino)-4,5-dimethyl-4,5,6,7-tetrahydrobenzothiophene-3-carboxylate* (1.47 g, 90%).

1M Aqueous NaOH solution (2 mL) was added to a solution of *ethyl 2-(cyclopropanecarbonylamino)-4,5-dimethyl-4,5,6,7-tetrahydrobenzothiophene-3-carboxylate* (1.47 g, 4.57 mmol) in an 8:1 mixture of THF:water (9 mL). The reaction mixture was stirred at r.t. for 12.5 h. A further portion of 1M aqueous NaOH solution (2 mL) was added and stirring was continued for a further 6 h. The reaction mixture was concentrated *in vacuo,* diluted with water (30 mL) and washed with DCM (2 x 20 mL). The aqueous layer was acidified to pH 5 with 2M aqueous hydrochloric acid solution and the solid was filtered off and washed with water and heptane to afford *2-(cyclopropanecarbonylamino)-4,5-dimethyl-4,5,6,7-tetrahydrobenzothiophene-3-carboxylic acid* which was utilised in the following step. LCMS [M+H]⁺ 294.0, RT 3.749 minutes, 98.9% purity.

To a solution of *2-(cyclopropanecarbonylamino)-4,5-dimethyl-4,5,6,7-tetrahydrobenzothiophene-3-carboxylic acid* (200 mg, 0.68 mmol) in DCM (4 mL) was added cyclopropylmethylamine [2516-47-4] (97.0 mg, 1.36 mmol) followed by EDCI (196 mg, 1.02 mmol). The reaction was stirred at r.t. for 3 h before heating at 80°C in a microwave for 2 h and leaving overnight at r.t. The reaction was poured into EtOAc, washed with saturated sodium hydrogen carbonate solution (2 x), 0.1M aqueous hydrochloric acid solution (1 x) and dried (MgSO₄). The mixture was filtered and the solvent was removed *in vacuo* to yield a brown oil/gum which was purified by flash column chromatography on silica (gradient elution with 0 - 20% EtOAc in heptane) to afford the *title compound* (46 mg, 19%) as a white solid. δ_{H} (500 MHz, Chloroform-d) 12.28 (s, 1H), 6.26 (s, 1H), 3.40 (ddd, *J* 13.0, 7.2, 5.5 Hz, 1H), 3.27 (ddd, *J* 13.7, 7.2, 4.7 Hz, 1H), 2.84 (p, *J* 6.8 Hz, 1H), 2.80 - 2.64 (m, 2H), 2.13 - 2.02 (m, 1H), 1.72 - 1.62 (m, 3H), 1.18 - 1.04 (m, 8H), 0.90 (dq, *J* 7.1, 3.5 Hz, 2H), 0.61 (dt, *J* 7.9, 4.7 Hz, 2H), 0.31 (q, *J* 4.6 Hz, 2H).

### EXAMPLE 92

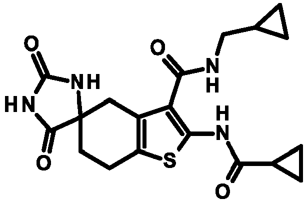

### 2-(Cyclopropanecarbonylamino)-N-(cyclopropylmethyl)-2',5'-dioxo-spiro[6,7-dihydro-4H-benzothiophene-5,4'-imidazolidine]-3-carboxamide

*Intermediate 62* (420 mg, 1.26 mmol) was dissolved in EtOH (2.5 mL) and water (2.5 mL). Potassium cyanide [151-50-8] (123 mg, 1.90 mmol) was added followed by ammonium carbonate (850 mg, 8.84 mmol), the reaction mixture was stirred at 65 °C for 1 h before cooling to r.t. overnight. The reaction mixture was concentrated *in vacuo,* water was added and the mixture was filtered to afford a solid. The filtrate was extracted with n-butanol, and the organic phase was washed with brine, dried (MgSO₄), filtered and concentrated *in vacuo.* The residue was triturated with Et₂O, filtered and dried in an oven at 40 °C to afford the *title compound* (410 mg, 81%). ¹H NMR (400 MHz, DMSO-*d*₆) δ 10.77 (s, 2H), 8.11 (s, 1H), 2.89 (td, *J* = 14.6, 13.7, 8.1 Hz, 3H), 2.50 (s, 1H), 2.30 (s, 3H), 1.66 (q, *J* = 13.2, 11.5 Hz, 3H), 0.94 - 0.33 (m, 5H), 0.20 (d, *J* = 7.9 Hz, 2H), -0.00 (d, *J* = 4.9 Hz, 2H). LCMS [M+H]⁺ 403.19, RT 1.77 minutes (*Method 8*)*.*

### EXAMPLE 97

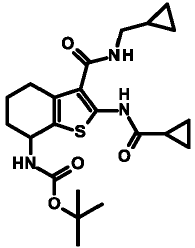

### tert-Butyl N-[2-(cyclopropanecarbonylamino)-3-(cyclopropylmethylcarbamoyl)-4,5,6,7-tetrahydrobenzothiophen-7-yl]carbamate

To *intermediate 104* (700 mg, 1.84 mmol) dissolved in DCM (50 mL) was added 1-cyclopropylmethanamine [2516-47-4] (0.63 mL, 7.36 mmol) and EDCI (600 mg, 3.12 mmol). The reaction mixture was stirred at r.t. for 16 h before diluting with DCM (50 mL) and washing with water, 0.5M aqueous hydrochloric acid solution and saturated sodium hydrogen carbonate solution. The organic layer was separated and washed with brine, dried (MgSO₄), filtered and concentrated in *vacuo.* The crude residue was purified by flash column chromatography on silica to afford the *title compound* (319 mg, 40%) as a white solid. 25 mg was purified by preparative HPLC (basic) to afford the pure compound (5 mg) for analysis. δ_{H} (500 MHz, CD₃OD) 4.79 - 4.67 (m, 1H), 3.24 (d, *J* 6.9 Hz, 2H), 2.76 - 2.64 (m, 2H), 2.08 - 1.91 (m, 2H), 1.78 (m, 3H), 1.47 (s, 9H), 1.10 (m, 1H), 1.01 - 0.91 (m, 4H), 0.56 - 0.51 (m, 2H), 0.31 - 0.26 (m, 2H). LCMS [M+H]⁺ 434.2, RT 3.87 minutes, 96% purity (*Method 10*)*.*

### EXAMPLES 98 and 99

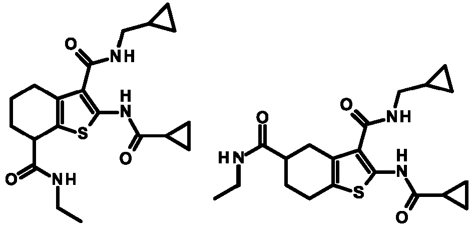

### 2-(Cyclopropanecarbonylamino)-N3-(cyclopropylmethyl)-N7-ethyl-4,5,6,7-tetrahydrobenzothiophene-3,7-dicarboxamide

*A mixture of intermediates 109 and 110* (30 mg, 0.083 mmol) (6% *intermediate 110*) was dissolved in DCM (3 mL) then EDCI (24 mg, 0.12 mmol) was added and the reaction mixture was stirred for 20 minutes. 2M Ethyl amine in THF (0.083 mL, 0.16 mmol) was added and the reaction mixture was stirred at r.t. for 16 h. The reaction mixture was diluted with DCM (20 mL) and washed with water, 1M aqueous hydrochloric acid solution and saturated sodium hydrogen carbonate solution. The organic layer was washed with brine, dried (MgSO₄), filtered and concentrated *in vacuo.* The residue was purified by preparative HPLC (acidic) to afford the *title compound* (16 mg, 50%) as a white solid which contained 91% of the *7-isomer title compound example 98* and 9% of the *5-isomer title compound example 99.* δ_{H} (500 MHz, CD₃OD) 3.62 (t, J 6.3 Hz, 1H, 7-isomer), 3.29 - 3.16 (m, 4H), 2.87 - 2.66 (m, 2H, 7-isomer, 4H, 5-isomer), 2.60 - 2.47 (m, 1H, 5-isomer), 2.12 - 1.95 (m, 3H, 7-isomer, 1H, 5-isomer), 1.93 - 1.87 (m, 1H, 5-isomer), 1.85 - 1.67 (m, 2H, 7-isomer, 1H, 5-isomer), 1.22 - 1.05 (m, 4H), 1.03 - 0.89 (m, 4H), 0.59 - 0.49 (m, 2H), 0.32 - 0.25 (m, 2H). 9% 5-regio isomer present. LCMS [M+H]⁺ 390.2, RT 2.82 minutes, 90% 7-isomer, 9% 5-isomer *(Method 10).*

### EXAMPLES 100 and 101

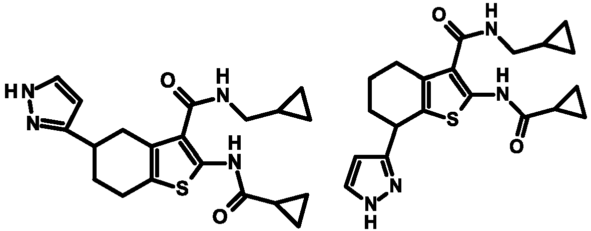

### Example 100

### 2-(Cyclopropanecarbonylamino)-N-(cyclopropylmethyl)-5-(1H-pyrazol-3-yl)-4,5,6,7-tetrahydrobenzothiophene-3-carboxamide

### Example 101

### 2-(Cyclopropanecarbonylamino)-N-(cyclopropylmethyl)-7-(1H-pyrazol-3-yl)-4,5,6,7-tetrahydrobenzothiophene-3-carboxamide

To *intermediate 111* (22 mg, 0.089 mmol) and 2-cyano-N-(cyclopropylmethyl)acetamide [114153-25-2] (15 mg, 0.0977 mmol) in THF (0.6 mL) was added ammonium acetate (7 mg, 0.0908 mmol) and acetic acid (5.4 mg, 0.089 mmol). The reaction mixture was stirred at 60 °C overnight. To the reaction mixture was added sulphur [7704-34-9] (8 mg, 0.250 mmol), EtOH (1.8 mL) and triethylamine (30 mg, 0.294 mmol) and the mixture was warmed at 65 °C for 2 h. The reaction mixture was concentrated *in vacuo* and EtOAc (5 mL) and 5% potassium bisulfate (2 mL) was added. The organic layer was separated and washed with saturated sodium hydrogen carbonate solution and brine. The organic layer was dried (Na₂SO₄), filtered and concentrated *in vacuo* to yield a brown oil. To this oil was added DCM (0.8 mL), DIPEA (17 mg, 0.132 mmol) and cyclopropanecarbonyl chloride [4023-34-1] (11 mg, 0.1031 mmol). The reaction mixture was stirred at r.t. overnight. To the reaction mixture was added saturated sodium hydrogen carbonate solution (2 mL) and the mixture was filtered through a phase separation cartridge. The aqueous layer was further extracted with DCM (2 mL) and the combined organic phases were concentrated *in vacuo.* To this was added DCM (2 mL) and TFA (1 mL) and the reaction mixture was stirred at r.t. for 2 h and then concentrated *in vacuo.* Purification was carried out by reverse phase HPLC (basic) to afford the *title compounds examples 100 and 101* (2.1 mg, 6.2%) in the ratio of 8/2 respectively. LCMS [M+H]⁺ 385.06, RT 3.87 minutes, 82.13%; [M+H]⁺ 385.00, RT 3.63 minutes, 17.87% (*Method 13*)*.* LCMS [M+H]⁺ 385.15, [M+Na]⁺ 407.08, RT 3.87 minutes, 84.06%; [M+H]⁺ 385.15, RT 3.95 minutes, 15.94% *(Method 21).*

### EXAMPLE 104

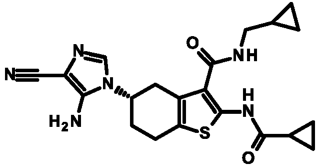

### (55)-5-(5-Amino-4-cyano-imidazol-1-yl)-2-(cyclopropanecarbonylamino)-N-(cyclopropylmethyl)-4,5,6,7-tetrahydrobenzothiophene-3-carboxamide

To aminopropanedinitrile 4-methylbenzenesulfonate (1:1) [5098-14-6] (0.15 g, 0.6 mmol) stirring in MeCN (2 mL) was added triethylamine (84 µL, 0.6 mmol) and triethylorthoformate [122-51-0] (100 µL, 0.6 mmol). The reaction was sealed and heated to 90 °C for 1 h. *Intermediate 117* (0.2 g, 0.6 mmol) was added followed by further MeCN (3 mL). The suspension was sonicated and stirred for 15 minutes, the reaction was heated to 60 °C for 45 minutes before concentrating *in vacuo.* The residue was dissolved in DCM (50 mL) and washed with water (50 mL). An emulsion was obtained and the mixture was diluted with DCM, water and brine. The organic phase was collected and the aqueous phase extracted with further DCM (2 x 50 mL). Aqueous layer was adjusted to pH 8 using 1M aqueous sodium hydroxide solution and was extracted with EtOAc (2 x 50 mL). The organics were dried (Na₂SO₄), filtered and concentrated *in vacuo* to give the crude product as a light brown solid (280 mg) which was purified by flash column chromatography on silica (gradient elution with 0 -10 % MeOH in DCM) to afford the *title compound* as a solid which was triturated with EtOAc and heptane (1:1) to afford the purified *title compound* (65 mg, 26%) as a cream coloured solid. δ_{H} (500 MHz, DMSO-*d*₆) 11.22 (s, 1H), 7.68 (t, *J* 5.7 Hz, 1H), 7.34 (s, 1H), 6.24 (s, 2H), 4.35 - 4.23 (m, 1H), 3.20 - 3.08 (m, 2H), 3.08 - 3.01 (m, 1H), 2.99 - 2.89 (m, 1H), 2.89 - 2.74 (m, 2H), 2.21 - 2.05 (m, 2H), 1.96 - 1.87 (m, 1H), 1.07 - 0.97 (m, 1H), 0.90 - 0.79 (m, 4H), 0.44 - 0.32 (m, 2H), 0.26 - 0.12 (m, 2H). LCMS (ES+) [M+H]⁺ 425.1, RT 2.59 minutes, purity 97% *(Method 10).*

### EXAMPLE 105

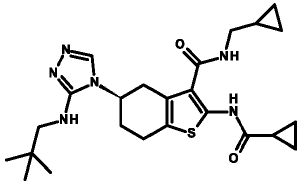

### (5S)-2-(Cyclopropanecarbonylamino)-N-(cyclopropylmethyl)-5-[3-(2,2-dimethylpropylamino)-1,2,4-triazol-4-yl]-4,5,6,7-tetrahydrobenothiophene-3-carboxamide

*Intermediate 119* (150 mg, 0.268 mmol) was dissolved in EtOH (10 mL) and formic acid [64-18-6] (10 mL). The reaction mixture was stirred under nitrogen and a slurry of 10% palladium on carbon (100 mg, 0.94 mmol) was added and the reaction was heated at 80 °C for 3 h. The reaction mixture was cooled and further 10% palladium on carbon (50 mg) was added. The mixture was then heated at 80 °C for a further 3 h before cooling and filtering through Celite. The solvent was removed *in vacuo* to give a pink gum which was purified by flash column chromatography on silica (gradient elution with 0-5% EtOAc/iso-hexane) to give the desired product as an impure solid which was repurified by flash column chromatography on silica (gradient elution with 0-3% EtOAc/iso-hexane) to afford the *title compound* (65 mg, 51%) as a white solid. δ_{H} (400 MHz, DMSO-*d*₆) 11.23 (s, 1H), 8.04 (s, 1H), 7.69 (t, *J* 5.7 Hz, 1H), 5.85 (t, *J* 6.4 Hz, 1H), 4.36 (p, *J* 5.0 Hz, 1H), 3.23 - 3.02 (m, 5H), 2.83 (s, 3H), 2.14 (s, 2H), 1.97 - 1.89 (m, 1H), 1.12 - 0.92 (m, 1H), 0.90 (s, 11H), 0.43 - 0.33 (m, 2H), 0.20 (ddd, *J* 5.6, 4.7, 3.6 Hz, 2H). LCMS (ES+) [M+H]⁺ 471.2, RT 2.11 minutes, 100.0% purity *(Method 3).*

### EXAMPLE 106 and 107

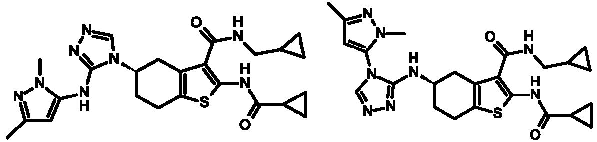

### Example 106

### (5S)-2-(Cyclopropanecarbonylamino)-N-(cyclopropylmethyl)-5-[3-[(2,5-dimethylpyrazol-3-yl)amino]-1,2,4-triazol-4-yl]-4,5,6,7-tetrahydrobenzothiophene-3-carboxamide

### Example 107

### (5S)-2-(Cyclopropanecarbonylamino)-N-(cyclopropylmethyl)-5-[[4-(2,5-dimethylpyrazol-3-yl)-1,2,4-triazol-3-yl]amino]-4,5,6,7-tetrahydrobenzothiophene-3-carboxamide

### Reference Example 108

### (5S)-2-(Cyclopropanecarbonylamino)-5-[3-[(2,5-dimethylpyrazol-3-yl)aminol-1,2,4-triazol-4-yl]-N-isobutyl-4,5,6,7-tetrahydrobenzothiophene-3-carboxamide

*Intermediate 120* (3.50 g, 7.19 mmol) was dissolved in DMF (94 g, 1.29 mol). Formic acid hydrazide [624-84-0] (0.62 g, 9.35 mmol) was added followed by mercuric chloride [7487-94-7] (2.54 g, 9.35 mmol). The reaction mixture was stirred at r.t. for ~10 minutes and triethylamine (0.81 g, 7.91 mmol) was added and the mixture was stirred at 80 °C for ~2.5 h. Further formyl hydrazine [624-84-0] (0.22 g, 3.60 mmol) and mercuric chloride [7487-94-7] (1.75 g, 3.60 mmol) were added and the reaction mixture was stirred at 80 °C for ~1 h. The mixture was cooled to r.t. and left to stand over the weekend. Further triethylamine (0.81 g, 7.96 mmol) was added and the mixture was heated at 80 °C for ~1 h. The reaction mixture was cooled to r.t. and diluted with MeCN (100 mL), filtered through a pad of celite and washed with MeCN (2 x 50 mL). The filtrate was concentrated *in vacuo* to yield the crude product as an orange/brown gum which was dissolved in 5% MeOH/DCM, washed with water and brine, passed through a phase separation cartridge and the organic phase concentrated *in vacuo* to yield an orange/brown solid (~5.5 g) which was purified by flash column chromatography on silica (gradient elution with 2-15 % MeOH/DCM) to afford *title compound 106* (contaminated with *reference title compound 108)* and *title compound 107. Reference title compound 108* was present as a result of a 2-methylpropan-1-amine impurity in the cyclopropylmethanamine used in the preparation of *intermediate 114) Title compound 106 was* further purified by reverse phase chromatography on C18 silica to afford the *title compound 106* (1.57 g, 44.1% as a 2:1 mixture of rotamers) as a white solid. Reverse phase fractions containing *reference title compound 108 were* further purified by preparative HPLC to afford *reference title compound example 108* (15 mg, 0.030 mmol, 0.42%) as a ^{~}2:1 mixture of rotamers. LCMS (ES+) [M+H]⁺ 497.36, RT 1.91 minutes, purity 97.80% (*Method 24*)*.* LCMS (ES+) [M+H]⁺ 497.36, RT 1.94 minutes, purity 97.52% (*Method 25*)*.*

*Title compound example 106:* δ_{H} (500 MHz, DMSO-*d*₆) 11.22 (s, 1H), 8.42 (s, 1H), 8.33 (s, 1H), 7.74 (s, 1H), 5.89 (s, 1H), 4.45-4.40 (m, 1H), 3.55 (s, 3H), 3.19 - 3.05 (m, 3H), 2.99-2.94 (m, 1H), 2.86-2.80 (m, 2H), 2.25-2.17 (m, 2H), 2.06 (s, 3H), 1.93-1.90 (m, 1H), 1.04-0.98 (m, 1H), 0.86-0.82 (m, 4H), 0.40-0.36 (m, 2H), 0.22-0.17 (m, 2H), mixture of rotamers (2:1). LCMS (ES+) [M+H]⁺ 495.2, RT 1.68 minutes, 100.0% purity (*Method 3*)*.* LCMS (ES+) [M+H]⁺ 495.2, RT 1.63 minutes, 100.0% purity (*Method 2*)*.*

*Title compound example 107:* δ_{H} (500 MHz, DMSO-*d*₆) 11.00 (br s, 1H), 8.15 (s, 1H), 7.70 (s, 1H), 6.27 (s, 1H), 6.16 (d, *J* 7.2 Hz, 1H), 3.87-3.84 (m, 1H), 3.49 (s, 3H), 3.13-3.08 (m, 3H), 2.75-2.68 (m, 2H), 2.62 - 2.55 (m, 1H), 2.18 (s, 3H), 2.08-2.05 (m, 1H), 1.89-1.82 (m, 2H), 1.04-0.98 (m, 1H), 0.88-0.79 (m, 4H), 0.41-0.37 (m, 2H), 0.24-0.19 (m, 2H).

### EXAMPLE 109

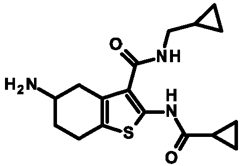

### 5-Amino-2-(cyclopropanecarbonylamino)-N-(cyclopropylmethyl)-4,5,6,7-tetrahydrobenzothiophene-3-carboxamide

To a stirred solution of *intermediate 114* (10 g, 23.1 mmol) in 1,4-dioxane (120 mL), 4M hydrogen chloride in 1,4-dioxane (120 mL) was added at 0°C. The reaction mixture was stirred at r.t. for 16 h. The reaction mixture was concentrated *in vacuo* and the crude product obtained was washed with sodium hydrogen carbonate solution and extracted with 10% MeOH:DCM. The organic layer was dried (Na₂SO₄), filtered and concentrated *in vacuo* to afford the *title compound* (6.5 g, 84%) which was triturated using *n*-pentane. δ_{H} (400 MHz, DMSO-*d*₆) 3.12-3.09 (m, 4H), 3.07 (s, 1H), 2.97 - 2.88 (m, 1H), 2.70-2.60 (d, *J* 16.1 Hz, 2H), 2.49 - 2.38 (m, 2H), 1.92 - 1.86 (m, 2H), 1.57 - 1.51 (m, 1H), 1.26 - 1.13 (m, 1H), 1.04 - 0.99 (m, 1H), 0.80-0.79 (m, 4H), 0.46 - 0.41 (m, 2H), 0.23-.21 (q, *J* 4.8 Hz, 2H).

### EXAMPLE 110

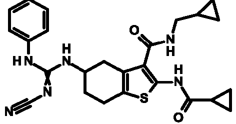

### 5-[[N'-Cyano-N-phenyl-carbamimidoyl]amino]-2-(cyclopropanecarbonylamino)-N-(cyclopropylmethyl)-4,5,6,7-tetrahydrobenzothiophene-3-carboxamide

*Intermediate 122* (50 mg, 0.14 mmol) was dissolved in DCM (4 mL) and DIPEA (26 mg, 0.20 mmol) and *intermediate 121* (64 mg, 0.270 mmol calculated not actual) was added. The reaction mixture was stirred at 100 °C in the microwave for 1 h. The mixture was purified by flash column chromatography on silica (gradient elution was 15-100% EtOAc/hexane) to afford the *title compound* (52 mg) as a tan solid which was purified by preparative HPLC to afford the *title compound* (2.9 mg, 4.5%) as a white solid. LCMS (ES+) [M+H]⁺ 477.8, RT 2.197 minutes, purity 100.0% *(Method 3).*

### EXAMPLE 111

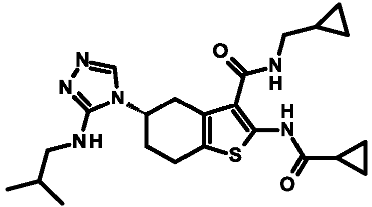

### (5S)-2-(Cyclopropanecarbonylamino)-N-(cyclopropylmethyl)-5-[3-(isobutylamino)-1,2,4-triazol-4-yl]-4,5,6,7-tetrahydrobenzothiophene-3-carboxamide

*Intermediate 124* (266 mg, 0.49 mmol) was dissolved in EtOH (10 mL) and formic acid [64-18-6] (10 mL). To the reaction mixture stirred under a nitrogen atmosphere was added a slurry of 10% palladium on carbon (200 mg, 1.88 mmol) and the reaction was heated at 80 °C for 5 h. The reaction mixture was cooled and further 10% Palladium on carbon (200 mg, 1.88 mmol) in EtOH (1.5 mL) was added. The reaction mixture was stirred under nitrogen and heated at 80 °C for a further 3 h. The mixture was cooled and stirred for 18 h at r.t. Further 10% Palladium on carbon (100 mg, 0.94 mmol) was added and the mixture was stirred under nitrogen and heated at 80 °C for a further 2 h. The reaction mixture was filtered through Celite washing with EtOH (2 x 10 mL). The solvent was removed *in vacuo* to give a pale yellow solid which was purified by flash column chromatography on silica (gradient elution with 0-3% MeOH/DCM) to afford the *title compound* (70 mg, 32%) as a white solid. δ_{H} (400 MHz, DMSO-*d*₆) 11.23 (s, 1H), 8.05 (s, 1H), 7.69 (t, *J* 5.7 Hz, 1H), 6.06 (t, *J* 5.7 Hz, 1H), 4.25 (dq, *J* 10.0, 4.9 Hz, 1H), 3.23 - 2.79 (m, 6H), 2.83 (s, 2H), 2.14 (td, *J* 9.9, 6.0 Hz, 2H), 1.92 (pd, *J* 6.7, 5.7, 3.4 Hz, 2H), 1.07 - 0.95 (m, 1H), 0.88 (dd, *J* 12.1, 5.3 Hz, 10H), 0.43 - 0.34 (m, 2H), 0.24 - 0.16 (m, 2H).

### EXAMPLE 112

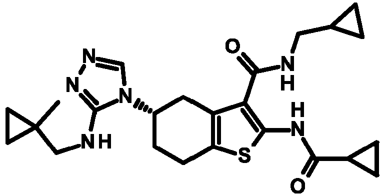

### (5S)-2-(Cyclopropanecarbonylamino)-N-(cyclopropylmethyl)-5-[3-[(1-methylcyclopropyl)methylaminol-1,2,4-triazol-4-yl]-4,5,6,7-tetrahydrobenzothiophene-3-carboxamide

*Intermediate 126* (64% purity, 485 mg, 0.56 mmol) was dissolved in EtOH (20 mL) and formic acid [64-18-6] (20 mL). 10% Palladium on carbon (50% wet) (5%, 1.18 g, 0.56 mmol) was added and the reaction was heated at 90 °C for 2.5 h. The reaction was heated at 90 °C for a further 16 h before filtering and washing with EtOH (50 mL). The filtrate was concentrated *in vacuo* and the oily residue (500 mg) was dissolved in formic acid (20 mL) and 10% palladium on carbon (50% wet) (5%, 1.18 g) added. The reaction was heated at 100 °C for 2 h. The reaction was allowed to cool and filtered, washing with EtOH : formic acid (1:1, 40 mL). The filtrate was concentrated *in vacuo* to yield the crude product as an oil. EtOAc (80 mL) was added and the organic layer was washed with saturated aqueous sodium hydrogen carbonate (2 x 20 mL) and brine. The organic phase was dried (Na₂SO₄), filtered and concentrated *in vacuo* to yield the crude product which was purified by flash column chromatography on silica (gradient elution with 0 - 10% MeOH in DCM) to afford the crude *title compound* which was purified by preparative HPLC to afford the *title compound* (28 mg, 11%) as a white solid. δ_{H} (500 MHz, DMSO-*d*₆) 11.23 (s, 1H), 8.04 (s, 1H), 7.68 (s, 1H), 5.96 (t, *J* 5.9 Hz, 1H), 4.32 (tt, *J* 10.2, 5.1 Hz, 1H), 3.16 (td, *J* 12.5, 11.6, 5.9 Hz, 3H), 3.12 - 3.02 (m, 2H), 2.90 - 2.75 (m, 3H), 2.19 -2.06 (m, 2H), 1.96 - 1.88 (m, 1H), 1.07 (s, 3H), 1.05 - 0.96 (m, 1H), 0.88 - 0.81 (m, 4H), 0.50 - 0.43 (m, 2H), 0.41 - 0.33 (m, 2H), 0.25 - 0.21 (m, 2H), 0.21 - 0.16 (m, 2H).

### EXAMPLE 113

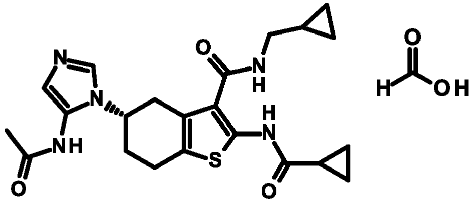

### (5S)-5-(5-Acetamidoimidazol-1-yl)-2-(cyclopropanecarbonylamino)-N-(cyclopropylmethyl)-4,5,6,7-tetrahydrobenzothiophene-3-carboxamide

To a stirred solution of *intermediate 127* (85%, 42 mg, 0.36 mmol) in anhydrous DCM (2 mL) was added *intermediate 117* (100 mg, 0.30 mmol). The reaction mixture was heated in a microwave (up to 100W) at 50 °C for 1 h. The reaction mixture was left to cool to r.t. and a solution of acetic anhydride (0.071 ml, 0.75 mmol) in DCM (0.5 mL) was added dropwise and the reaction mixture was stirred at r.t. for 2 h. The reaction mixture was diluted with DCM (5 mL) and washed with water (2 mL), 1M aqueous sodium hydrogen carbonate (2 mL) and brine (2 mL). The organic layer was dried (Na₂SO₄), filtered and the solvent was removed *in vacuo* to yield the crude product which was purified by preparative HPLC (low pH) to afford the *title compound* (22 mg, 16%) as a clear glass. δ_{H} (500 MHz, DMSO-*d*₆) 11.16 (s, 1H), 9.53 (s, 1H), 8.16 (s, 1H, *formate*)*,* 7.78 - 7.67 (m, 1H), 7.63 (s, 1H), 6.76 (s, 1H), 4.27 - 4.13 (m, 1H), 3.17 - 3.06 (m, 2H), 3.02 (d, *J* 7.5 Hz, 2H), 2.85 - 2.73 (m, 2H), 2.21 - 2.09 (m, 2H), 2.02 (s, 3H), 1.96 - 1.87 (m, 1H), 1.06 - 0.94 (m, 1H), 0.88 - 0.79 (m, 4H), 0.43 - 0.33 (m, 2H), 0.23 - 0.15 (m, 2H). LCMS [M-H]⁻ 442, RT 1.61 minutes, purity 100% (*Method 10*)*.*

### EXAMPLE 114

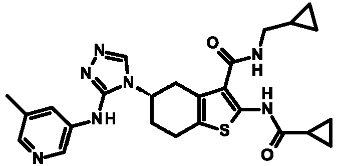

### (5S)-2-(cyclopropanecarbonylamino)-N-(cyclopropylmethyl)-5-[3-[(5-methyl-3-pyridyl)amino]-1,2,4-triazol-4-yl]-4,5,6,7-tetrahydrobenothiophene-3-carboxamide

*Intermediate 128* (290 mg, 0.60 mmol) was dissolved in DMF (10 mL) and formic acid hydrazide [624-84-0] (80 mg, 1.20 mmol) was added followed by mercuric chloride [7487-94-7] (326 mg, 1.20 mmol) and triethylamine (122 mg, 1.20 mmol). The mixture was stirred for 2 minutes at r.t. and then heated at 60 °C for 2 hrs. The reaction mixture was cooled and diluted with MeCN (10 mL) and then filtered through a plug of Celite, washing with MeCN (20 mL). The filtrate was concentrated *in vacuo* to yield a yellow oil which was dissolved in 10% MeOH/DCM and washed with water (2 x 5 mL). The organic phase was dried (Na₂SO₄), filtered and concentrated *in vacuo* to yield the crude product which was purified by flash column chromatography on silica (gradient elution with 0-20% MeOH/DCM) to afford the *title compound* (158 mg, 53%) as a white solid. δ_{H} (400 MHz, DMSO-*d*₆) 11.26 (s, 1H), 8.77 (s, 1H), 8.54 (d, *J* 2.5 Hz, 1H), 8.41 (s, 1H), 7.96 (d, *J* 6.6 Hz, 2H), 7.68 (s, 1H), 4.53 (s, 1H), 3.21 - 3.01 (m, 3H), 2.92 (d, *J* 28.9 Hz, 3H), 2.28 (s, 6H), 1.04 - 0.75 (m, 5H), 0.34 - 0.27 (m, 2H), 0.16 (t, *J* 4.6 Hz, 2H).

### EXAMPLE 115

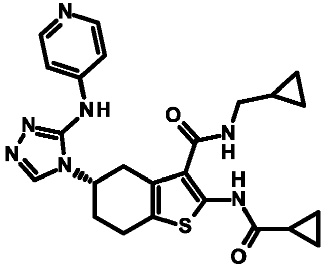

### (5S)-2-(Cyclopropanecarbonylamino)-N-(cyclopropylmethyl)-5-[3-(4-pyridylamino)-1,2,4-triazol-4-yl]-4,5,6,7-tetrahydrobenzothiophene-3-carboxamide

*Intermediate 129* (80%, 150 mg, 0.26 mmol) and formic hydrazide [624-84-0] (46 mg, 0.77 mmol) were dissolved in DMF (3 mL). Mercury dichloride [7487-94-7] (208 mg, 0.77 mmol) was added to the reaction mixture. The mixture was stirred for 5 minutes and triethylamine (0.11 ml, 0.77 mmol) was added. The reaction mixture was heated at 60 °C for 1h. The reaction mixture was stirred for 1 h at 90 °C and the reaction mixture was diluted with DCM (20 mL), filtered, and the filtrate concentrated *in vacuo.* The crude product was extracted with EtOAc (3 x 20 mL), washed with water (20 mL) and the organic phases were dried (MgSO₄), filtered, and the filtrate was evaporated to afford the crude product. The crude material was purified by flash column chromatography on silica (gradient elution with EtOAc then 0-50 % MeOH in DCM). The relevant fractions were concentrated *in vacuo* to give the crude desired product which was further purified by preparative HPLC (acidic) to afford the *title compound* (21 mg, 17%) as a partial formate salt, white solid. δ_{H} (500 MHz, CD₃OD) 8.52 (s, 1H), 8.31 (d, *J* 5.7 Hz, 2H), 8.26 (s, 1H), 7.50 (s, 2H), 4.61-4.56 (m, 1H), 3.27 - 3.18 (m, 2H), 3.12 (dd, *J* 13.8, 7.0 Hz, 1H), 3.05 (dd, *J* 15.4, 9.8 Hz, 1H), 3.01 - 2.85 (m, 2H), 2.42 - 2.26 (m, 2H), 1.80 (tt, *J* 8.1, 4.6 Hz, 1H), 1.06 - 0.85 (m, 5H), 0.47 - 0.35 (m, 2H), 0.23-0.14 (m, 2H). LCMS (ES+) [M+H]⁺ 478.14, RT 1.66 minutes (*Method 10*)*.* Chiral analysis 100% ee.

### EXAMPLE 116

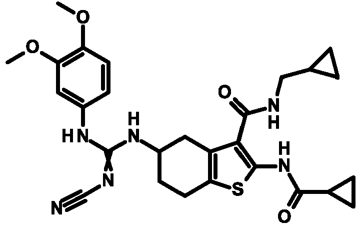

### 5-[[N'-Cyano-N-(3,4-dimethoxyphenyl)carbamimidoyl]amino]-2-(cyclopropanecarbonylamino)-N-(cyclopropylmethyl)-4,5,6,7-tetrahydrobenzothiophene-3-carboxamide

*Intermediate 122* (50 mg, 0.14 mmol) was dissolved in DCM (4 mL) and DIPEA (26 mg, 0.20 mmol) and *intermediate 130* (60 mg, 0.20 mmol) was added. The reaction mixture was stirred at 100 °C in the microwave for 3 h before leaving at r.t. overnight. The mixture was concentrated *in vacuo* and the residue was purified by flash column chromatography on silica (gradient elution with 15 - 100% EtOAc/hexane). The residue was freeze dried from MeCN/water to afford the product as a tan solid which was purified by preparative HPLC and freeze dried from MeCN/water to afford the *title compound* (10 mg, 14%) as a white solid. δ_{H} (300 MHz, DMSO-*d*₆) 10.83 (s, 1H), 8.65 (s, 1H), 7.59 - 7.41 (m, 1H), 6.78 - 6.56 (m, 3H), 6.52 (dd, *J* 8.7, 2.4 Hz, 1H), 3.79 (br. s, 1H), 3.50 (d, *J* 4.3 Hz, 6H), 2.91 (t, *J* 6.4 Hz, 2H), 2.80 - 2.63 (m, 1H), 2.58 - 2.44 (m, 2H), 2.43 - 2.31 (m, 1H), 1.82 - 1.44 (m, 3H), 0.79 (s, 1H), 0.59 (s, 4H), 0.27 - 0.14 (m, 2H), 0.04 - -0.06 (m, 2H).

### EXAMPLE 117

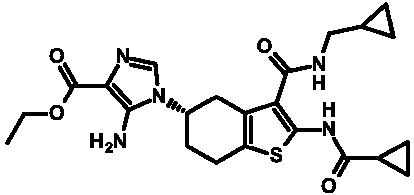

### Ethyl 5-amino-1-[(5S)-2-(cyclopropanecarbonylamino)-3-(cyclopropylmethylcarbamoyl)-4,5,6,7-tetrahydrobenzothiophen-5-yl]imidazole-4-carboxylate

To *intermediate 131* (85% purity, 23 mg, 0.15 mmol) in MeCN (2 mL) was added triethylorthoformate [122-51-0] (25 µL, 0.15 mmol) in a pressure tube. The reaction was sealed and stirred and heated at 90 °C for 1 h. The reaction was allowed to cool to r.t. and *intermediate 117* (50 mg, 0.15 mmol) was added and the reaction was stirred at r.t. for 18 h. The reaction mixture was concentrated *in vacuo* and the residue was purified by flash column chromatography on silica (gradient elution with 0 - 10 % MeOH in DCM) to afford the *title compound* (10 mg, 13 % at 94 % purity) as a grey solid. δ_{H} (500 MHz, DMSO-*d*₆) 11.21 (s, 1H), 7.70 (t, *J* 5.6 Hz, 1H), 7.28 (s, 1H), 6.09 (s, 2H), 4.39 - 4.24 (m, 1H), 4.17 (q, *J* 7.1 Hz, 2H), 3.22 - 3.02 (m, 3H), 3.02 - 2.92 (m, 1H), 2.91 - 2.74 (m, 2H), 2.25 - 2.05 (m, 2H), 2.00 - 1.83 (m, 1H), 1.24 (t, *J* 7.1 Hz, 3H), 1.08 - 0.93 (m, 1H), 0.93 - 0.75 (m, 4H), 0.44 - 0.31 (m, 2H), 0.20 (q, *J* 5.0 Hz, 2H).

### EXAMPLE 118

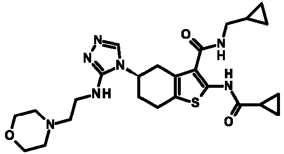

### (5S)-2-(Cyclopropanecarbonylamino)-N-(cyclopropylmethyl)-5-[3-(2-morpholinoethylamino)-1,2,4-triazol-4-yl]-4,5,6,7-tetrahydrobenothiophene-3-carboxamide

To a solution of *intermediate 133* (300 mg, 0.50 mmol) in EtOH (10 mL) was added formic acid [64-18-6] (10 mL, 100 mass%). The reaction mixture was placed under a nitrogen atmosphere and a slurry of 10% palladium on carbon (200 mg, 0.19 mmol) in EtOH (2 mL) was added and the solution was stirred for 5 h at 80 °C. Further 10% palladium on carbon (200 mg, 0.19 mmol) was added and the reaction heated for a further 2 h at 80 °C then 72 h at r.t. The reaction mixture was cooled and further 10% palladium on carbon (150 mg, 0.14 mmol) was added. The reaction was evacuated and under a nitrogen atmosphere was heated at 80 °C for 4 h. The reaction was cooled, filtered through Celite and washed with excess EtOH (2 x 10 mL). The solvent was removed *in vacuo* to give a yellow gum which was purified by flash column chromatography on silica (gradient elution with 0 - 100% [10% v/v MeOH/DCM with 2% v/v aqueous ammonia]/DCM) to give the product which was freeze dried from MeCN/water to afford the *title compound* (92 mg, 36%) as a white solid. δ_{H} (400 MHz, DMSO-*d*₆) 11.21 (s, 1H), 8.07 (s, 1H), 7.69 (t, *J* 5.7 Hz, 1H), 5.97 (t, *J* 5.7 Hz, 1H), 4.22 (dt, *J* 10.0, 5.0 Hz, 1H), 3.60 - 3.53 (m, 5H), 3.38 - 3.27 (m, 4H), 3.22 - 2.98 (m, 3H), 2.94 - 2.76 (m, 3H), 2.45 - 2.36 (m, 3H), 2.12 (s, 2H), 1.92 (q, *J* 6.8, 6.3 Hz, 1H), 1.06 - 0.98 (m, 1H), 0.92 - 0.83 (m, 4H), 0.39 (dt, *J* 8.1, 2.9 Hz, 2H), 0.25 - 0.16 (m, 2H).

### EXAMPLE 119

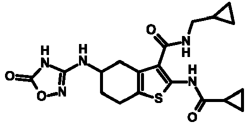

### 2-(Cyclopropanecarbonylamino)-N-(cyclopropylmethyl)-5-[(5-oxo-4H-1,2,4-oxadiazol-3-yl)amino]-4,5,6,7-tetrahydrobenzothiophene-3-carboxamide

To a stirred solution of *intermediate 135* (30 mg, 0.077 mmoL) in THF (3 mL) was added CDI (15 mg, 0.092 mmoL) and the mixture was stirred at r.t. under an atmosphere of nitrogen for 20 h. The reaction mixture was concentrated *in vacuo* and the residue was purified by preparative HPLC (low pH) to afford the *title compound* (1 mg, 3%, 82% purity). δ_{H} (250 MHz, CD₃OD) 3.69 - 3.53 (m, 1H), 3.17 - 3.02 (m, 3H, part. obs. by MeOD peak), 2.77 - 2.65 (m, 2H), 2.66 - 2.47 (m, 1H), 2.13 - 1.97 (m, 1H), 1.94 - 1.76 (m, 1H), 1.77 - 1.62 (m, 1H), 1.08 - 0.92 (m, 1H), 0.97 - 0.73 (m, 4H), 0.51 - 0.32 (m, 2H), 0.24 - 0.11 (m, 2H). LCMS [M+H]⁺ 418, RT 2.61 minutes, 82% purity *(Method 10).*

### EXAMPLE 120

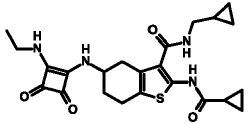

### 2-(Cyclopropanecarbonylamino)-N-(cyclopropylmethyl)-5-[[2-(ethylamino)-3,4-dioxo-cyclobuten-1-yl]amino]-4,5,6,7-tetrahydrobenzothiophene-3-carboxamide

*Intermediate 136* (95% purity, 50 mg, 0.1 mmol) was suspended in EtOH (4 mL) and 2M ethanamine in THF (60 µL) was added. The reaction mixture was warmed to ^{~}50 °C until the solids dissolved and the solution was left to stand for 16 h at r.t. The precipitate was filtered and washed with EtOH to afford the *title compound* (25 mg, 50 %) as a white solid. δ_{H} (500 MHz, CD₃OD) 4.49 (br, 1H), 3.68 - 3.61 (m, 2H), 3.28 - 3.14 (m, 3H), 2.85 (br t, *J* 5.8 Hz, 2H), 2.78 (dd, *J* 15.9, 6.5 Hz, 1H), 2.20 - 2.10 (m, 1H), 2.10 - 2.00 (m, 1H), 1.84 - 1.76 (m, 1H), 1.25 (t, *J* 7.2 Hz, 3H), 1.14 - 1.05 (m, 1H), 1.02 - 0.89 (m, 4H), 0.55 - 0.49 (m, 2H), 0.31 - 0.24 (m, 2H). LCMS[M+H]⁺ 457.3, RT 2.57 minutes, 95% purity (*Method 10*)*.*

### EXAMPLE 121

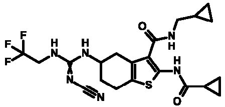

### 5-[[N'-cyano-N-(2,2,2-trifluoroethyl)carbamimidoyl]amino]-2-(cyclopropanecarbonylamino)-N-(cyclopropylmethyl)-4,5,6,7-tetrahydrobenzothiophene-3-carboxamide

To diphenyl N-cyanocarbonimidate [79463-77-7] (40 mg, 0.17 mmol) was added 2,2,2-trifluoroethylamine [753-90-2] (18 mg, 0.19 mmol) and 1:1 DCM/isopropanol (1 mL). To this was added DIPEA (44 mg, 0.34 mmol) and the reaction was stirred at r.t. overnight. To the mixture was added *intermediate 122* (40 mg, 0.11 mmol) and the mixture was stirred at 100 °C for 5 h. The reaction mixture was concentrated *in vacuo,* solubilised in 7:3 MeCN/water (700 µL) and the solvent was removed *in vacuo,* followed by solubilisation in DMF (700 µL) and MeCN/water (200 µL). The compound was purified by reverse phase HPLC (basic) followed by reverse phase HPLC (acidic) to afford the *title compound* (5.1 mg, 9.8%). LCMS [M+H]⁺ 483.1762, RT 4.69 minutes, 87.03% purity *(Method 20).*

### EXAMPLE 122

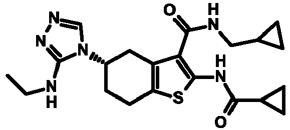

### (5S)-2-(cyclopropanecarbonylamino)-N-(cyclopropylmethyl)-5-[3-(ethylamino)-1,2,4-triazol-4-yl]-4,5,6,7-tetrahydrobenzothiophene-3-carboxamide

*Intermediate 138* (92%, 1.71 g, 3.03 mmol) was dissolved in EtOH (70 mL) and formic acid [64-18-6] (70 mL). Palladium on carbon (3.22 g, 30.3 mmol) was added to the reaction mixture and the mixture was refluxed for 2 h at 90 °C. Further palladium on carbon (2 g) was added to the reaction mixture and the mixture was refluxed for 3 h at 90 °C. The crude reaction mixture was filtered through a short pad of celite and washed with EtOH. The solvent was removed under reduced pressure to give the crude solid which was dissolved in EtOAc and neutralised with sodium hydrogen carbonate to pH 7. The organic phase was separated, dried (MgSO₄), filtered, and the filtrate was concentrated *in vacuo* to give the product which was purified by flash column chromatography on silica (gradient elution with 0 - 25% MeOH in DCM) to afford the *title compound* (407 mg, 32%). δ_{H} (500 MHz, Methylene Chloride-*d*₂) 11.97 (s, 1H), 7.76 (s, 1H), 5.82 (s, 1H), 4.15-4.10 (m, 1H), 3.89 (t, *J* 5.5 Hz, 1H), 3.50 - 3.37 (m, 2H), 3.32-3.26 (m, 2H), 3.22 - 3.15 (m, 1H), 2.91-2.81 (m, 3H), 2.34 - 2.15 (m, 2H), 1.68-1.58 (m, 1H), 1.28 (t, *J* 7.2 Hz, 3H), 1.08-1.01 (m, 3H), 0.96 - 0.83 (m, 2H), 0.57 - 0.44 (m, 2H), 0.28-0.22 (m, 2H). LCMS [M+H]⁺ 429.20, RT 1.81 minutes, 96% purity *(Method 10).*

### EXAMPLE 123

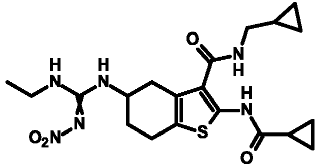

### 2-(cyclopropanecarbonylamino)-N-(cyclopropylmethyl)-5-[(N-ethyl-N'-nitro-carbamimidoyl)amino]-4,5,6,7-tetrahydrobenzothiophene-3-carboxamide

A mixture of *intermediate 140* (60 mg, 0.13 mmol) and 2M ethanamine in THF (99.6 µL, 0.19 mmol) in MeOH (1.5mL) was stirred and heated at 70 °C for 1 h in a microwave. The reaction mixture was concentrated *in vacuo* and the residue was purified by acidic HPLC to afford the *title compound* (45 mg, 76%) as a white solid. δ_{H} (500 MHz, CD₃OD) 4.31- 3.91 (m, 1H), 3.36 (q, *J* 7.2 Hz, 2H), 3.27 - 3.12 (m, 3H), 2.92 - 2.79 (m, 2H), 2.78 - 2.63 (m, 1H), 2.20 - 2.10 (m, 1H), 2.01 - 1.90 (m, 1H), 1.84 - 1.76 (m, 1H),1.31 - 1.18 (m, 3H), 1.14 - 1.05 (m, 1H), 1.01 - 0.90 (m, 4H), 0.56 - 0.49 (m, 2H), 0.32 - 0.24 (m, 2H). LCMS [M+H]⁺ 449.2, RT 2.96 minutes, 100% purity (*Method 10*)*.*

### EXAMPLE 124

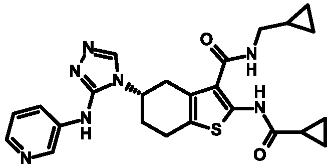

### (5S)-2-(Cyclopropanecarbonylamino)-N-(cyclopropylmethyl)-5-[3-(3-pyridylamino)-1,2,4-triazol-4-yl]-4,5,6,7-tetrahydrobenzothiophene-3-carboxamide

*Intermediate 141* (2.00 g, 4.26 mmol) was dissolved in DMF (25 mL). Formic acid hydrazide [624-84-0] (313 mg, 4.68 mmol) and mercuric chloride [7487-94-7] (1.27 g, 4.68 mmol) was added. The reaction mixture was stirred at r.t. for 5 minutes. Triethylamine (476 mg, 4.68 mmol) was added and the reaction mixture was stirred at r.t. for ^{~}30 minutes before heating to 60 °C for ^{~}2.5 h. Further triethylamine (216 mg, 2.14 mmol) and formic acid hydrazide [624-84-0] (83 mg, 1.38 mmol) was added and the reaction mixture was stirred at 60 °C for a further ^{~}1 h. The reaction mixture was cooled, diluted with MeCN (20 mL), filtered through a pad of celite and washed with MeCN (3 x 10 mL). The pale yellow filtrate was concentrated *in vacuo* to give a tan solid which was dissolved in 5% MeOH/DCM and washed with water (2 x) and brine. The mixture was passed through a phase separation cartridge and concentrated *in vacuo* to give a tan solid which was purified by flash column chromatography on silica (gradient elution with 5 - 15% MeOH/DCM) followed by further purification by flash column chromatography on silica (gradient elution with 2 - 15% MeOH/DCM) to afford the *title compound* (736 mg, 36%) which was freeze-dried from MeCN/water to yield a white powder. δ_{H} (300 MHz, DMSO-*d*₆) 11.26 (s, 1H), 8.85 (s, 1H), 8.76 (dd, *J* 2.7, 0.7 Hz, 1H), 8.42 (s, 1H), 8.17 - 8.05 (m, 2H), 7.70 (t, *J* 5.7 Hz, 1H), 7.35 - 7.24 (m, 1H), 4.62 - 4.44 (m, 1H), 3.22 - 2.99 (m, 3H), 2.99 - 2.80 (m, 3H), 2.35 - 2.13 (m, 2H), 2.01 - 1.85 (m, 1H), 1.03 - 0.90 (m, 1H), 0.90 - 0.80 (m, 4H), 0.35 - 0.22 (m, 2H), 0.20 - 0.08 (m, 2H). LCMS [M+H]⁺ 478.2, RT 1.385 minutes, 97.5% purity (*Method 2*)*.* LCMS [M+H]+ 478.2, RT 1.644 minutes, 96.9% purity (*Method 3*)*.*

### EXAMPLE 125

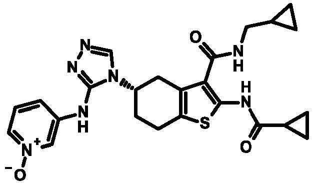

### (5S)-2-(Cyclopropanecarbonylamino)-N-(cyclopropylmethyl)-5-[3-[(1-oxidopyridin-1-ium-3-yl)amino]-1,2,4-triazol-4-yl]-4,5,6,7-tetrahydrobenzothiophene-3-carboxamide

*Example 124* (72 mg, 0.15 mmol) was dissolved in DCM (5 mL) with a few drops of MeOH. m-CPBA [937-14-4] (37 mg, 0.17 mmol) was added and the reaction mixture was stirred at r.t. for ^{~}2 h. Further mCPBA [937-14-4] (17 mg, 0.075 mmol) and the reaction was stirred at r.t. for ^{~}16 h. The reaction mixture was washed with saturated sodium hydrogen carbonate solution and brine before passing through a phase separation cartridge. The filtrate was concentrated *in vacuo* and the residue was purified by flash column chromatography on silica (gradient elution with 5 - 25% MeOH/DCM) to afford the *title compound* (21 mg, 28%) which was freeze-dried from MeCN/water to give a white solid. δ_{H} (300 MHz, DMSO-*d*₆) 11.27 (s, 1H), 9.09 (s, 1H), 8.77 (t, *J* 1.9 Hz, 1H), 8.46 (s, 1H), 7.80 (ddd, *J* 6.2, 1.7, 0.9 Hz, 1H), 7.67 (t, *J* 5.6 Hz, 1H), 7.49 (ddd, *J* 8.6, 2.1, 0.9 Hz, 1H), 7.30 (dd, *J* 8.6, 6.2 Hz, 1H), 4.51 (br. s, 1H), 3.21 - 2.77 (m, 6H), 2.36 - 2.10 (m, 2H), 2.01 - 1.82 (m, 1H), 1.03 - 0.72 (m, 5H), 0.36 - 0.23 (m, 2H), 0.22 - 0.08 (m, 2H).

### EXAMPLE 126

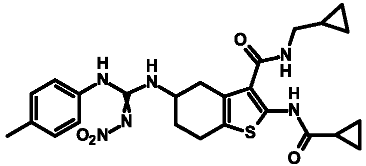

### 2-(cyclopropanecarbonylamino)-N-(cyclopropylmethyl)-5-[[N'-nitro-N-(p-tolyl)carbamimidoyl]amino]-4,5,6,7-tetrahydrobenzothiophene-3-carboxamide

A mixture of *intermediate 122* (60 mg, 0.16 mmol), *intermediate 142* (44 mg, 0.19 mmol) and DIPEA (42.3 µl, 0.24 mmol) in isopropanol (3 mL) was stirred and heated at 80 °C for 3 h in a microwave. The reaction mixture was concentrated *in vacuo* and the residue was purified by acidic HPLC to afford the *title compound* (25 mg, 30%) as a white solid. δ_{H} (500 MHz, CD₃OD) 7.24 (d, *J* 7.9 Hz, 2H), 7.17 (d, *J* 8.3 Hz,2H), 4.33 - 4.17 (m, 1H), 3.27 - 3.19 (m, 2H), 3.17 - 3.08 (m, 1H), 2.93 - 2.57 (m,3H), 2.35 (s, 3H), 2.21 - 2.04 (m, 1H), 1.99 -1.86 (m, 1H), 1.84 - 1.75 (m, 1H), 1.14 - 1.04 (m, 1H), 1.00 - 0.91 (m, 4H), 0.56 -0.48 (m, 2H), 0.31 - 0.24 (m, 2H). LCMS [M+H]⁺ 511.2, RT 3.6 minutes, 98% purity *(Method 10).*

### EXAMPLE 127

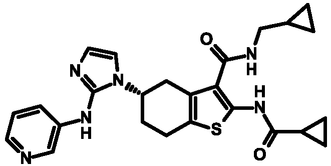

### (5S)-2-(Cyclopropanecarbonylamino)-N-(cyclopropylmethyl)-5-[2-(3-pyridylamino)imidazol-1-yl]-4,5,6,7-tetrahydrobenzothiophene-3-carboxamide

To a stirred solution of *intermediate 141* (222 mg, 0.47 mmol) and 2,2-diethoxyethanamine [645-36-3] (0.21 ml, 1.42 mmol) in DMF (4.5 mL) was added mercury dichloride [7487-94-7] (385 mg, 1.42 mmol). The reaction mixture was stirred at ambient temperature for 5 minutes then triethylamine (0.197 ml, 1.42 mmol) was added. The reaction mixture was heated at 90 °C for 1 h. 4-Methylbenzenesulfonic acid hydrate (1:1) [6192-52-5] (539 mg, 2.84 mmol) was added and heated continued at 90 °C for a further 2.5 h. The reaction mixture was diluted with DCM (40 mL), filtered through a pad of celite, and the filtrate concentrated *in vacuo.* The residue was diluted with water (20 mL) and extracted with EtOAc (3 x 20 mL). The organic extracts were combined, washed with water (10 mL) and brine (10 mL), dried (Na₂SO₄), filtered and the reaction mixture concentrated in *vacuo.* The residue was purified by flash column chromatography on silica (gradient elution with 1% to 10% MeOH/DCM) to afford the *title compound* (71.5 mg, 31%) as an off-white solid. δ_{H} (500 MHz, DMSO-*d*₆) 11.18 (s, 1H), 8.59 (d, *J* 2.6 Hz, 1H), 8.48 (s, 1H), 7.99 (dd, *J* 4.6, 1.4 Hz, 1H), 7.90 (ddd, *J* 8.4, 2.7, 1.4 Hz, 1H), 7.70 (t, J = 5.5 Hz, 1H), 7.20 (dd, J = 8.4, 4.6 Hz, 1H), 7.03 (d, J = 1.5 Hz, 1H), 6.76 (d, *J* 1.4 Hz, 1H), 4.57 - 4.47 (m, 1H), 3.16 - 2.99 (m, 3H), 2.99 - 2.90 (m, 1H), 2.89 - 2.79 (m, 2H), 2.24 - 2.14 (m, 1H), 2.13 - 2.05 (m, 1H), 1.96 - 1.87 (m, 1H), 1.00 - 0.91 (m, 1H), 0.90 - 0.82 (m, 4H), 0.37 - 0.26 (m, 2H), 0.20 - 0.11 (m, 2H). LCMS: M+H⁺ 477, RT = 1.73 min (97 %) (*Method 10*)*.*

### EXAMPLE 130

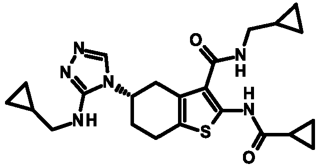

### (5S)-2-(Cyclopropanecarbonylamino)-N-(cyclopropylmethyl)-5-[3-(cyclopropylmethylamino)-1,2,4-triazol-4-yl]-4,5,6,7-tetrahydrobenothiophene-3-carboxamide

*Intermediate 148* (2.31 g, 4.24 mmol) was dissolved in EtOH (30 mL) and formic acid [64-18-6] (30 mL) was added. The reaction mixture was degassed (2 x) and 10% Pd/C (1.2 g, 1.13 mmol) was added. The reaction mixture was stirred at 80 °C for ^{~}2 h. Further 10% Pd/C (1.2 g, 1.13 mol) was added and the reaction mixture was stirred at 80 °C for ^{~}5 h. Further 10% Pd/C (1.0 g, 0.94 mmol) was added and the mixture was stirred at 80 °C for ^{~}1 h followed by r.t. overnight. Further 10% Pd/C (1.0 g, 0.94 mmol) was added and the mixture was stirred at 80 °C for ^{~}1 h. The mixture was cooled, filtered through a pad of celite, washed with EtOH (4 x) and the filtrate was concentrated *in vacuo.* The residue was purified by flash column chromatography on silica (gradient elution with 2-15% MeOH/DCM) to afford the crude material which was further purified by flash column chromatography on silica (gradient elution with 0-10% MeOH/DCM) and freeze dried to afford the *title compound* (720 mg, 37%) as a white solid. δ_{H} (300 MHz, DMSO-*d*₆) 11.22 (s, 1H), 8.06 (s, 1H), 7.69 (t, *J* = 5.4 Hz, 1H), 6.09 (t, *J* = 5.7 Hz, 1H), 4.36 - 4.16 (m, 1H), 3.23 - 3.00 (m, 5H), 3.00 - 2.69 (m, 3H), 2.17 - 2.03 (m, 2H), 2.03 - 1.77 (m, 1H), 1.20 - 0.94 (m, 2H), 0.94 - 0.77 (m, 4H), 0.53 - 0.31 (m, 4H), 0.28 - 0.15 (m, 4H).

### EXAMPLE 131

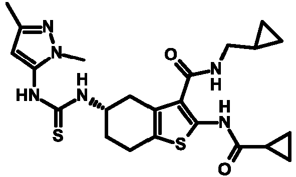

### (5S)-2-(Cyclopropanecarbonylamino)-N-(cyclopropylmethyl)-5-[(2,5-dimethylpyrazol-3-yl)carbamothioylamino]-4,5,6,7-tetrahydrobenzothiophene-3-carboxamide

*Intermediate 117* (2.5 g, 7.5 mmol) was dissolved in DCM (20 mL) and DIPEA (1.50 g, 11 mmol). 5-Isothiocyanato-1,3-dimethyl-1H-pyrazole [2052446-65-7] (1.50 g, 9.0 mmol) was added and the reaction mixture was stirred at r.t. for ^{~}18 h. The mixture was concentrated *in vacuo* and the residue was purified by flash column chromatography on silica (gradient elution with 50-100% EtOAc/hexane) to afford the *title compound* (3.54 g, 97%) as a white solid which was freeze dried from MeCN/water. δ_{H} (400 MHz, DMSO-*d*₆) 11.09 (s, 1H), 9.11 (s, 1H), 7.95 (d, *J* 7.8 Hz, 1H), 7.74 (t, *J* 5.7 Hz, 1H), 5.93 (s, 1H), 4.53 (s, 1H), 3.53 (s, 3H), 3.35 (s, 6H), 3.15 (t, *J* 6.2 Hz, 2H), 3.05 (dd, *J* 16.1, 5.1 Hz, 1H), 2.79 - 2.69 (m, 2H), 2.63 (dd, *J* 15.9, 7.7 Hz, 1H), 2.10 (s, 3H), 2.06 - 1.96 (m, 1H), 1.96 - 1.82 (m, 2H), 1.12 - 0.95 (m, 1H), 0.92 - 0.73 (m, 4H), 0.54 - 0.36 (m, 2H), 0.28 - 0.16 (m, 2H). LCMS (ES+) [M+H]- 485.0, RT 1.973 minutes, 97.4% purity *(Method 2).*

### EXAMPLE 132

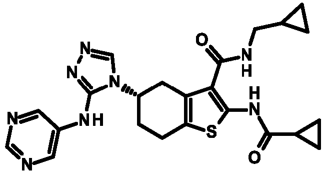

### (5S)-2-(Cyclopropanecarbonylamino)-N-(cyclopropylmethyl)-5-[3-(pyrimidin-5-ylamino)-1,2,4-triazol-4-yl]-4,5,6,7-tetrahydrobenzothiophene-3-carboxamide

*Intermediate 150* (141 mg, 0.3 mmol) and formic hydrazide [624-84-0] (54 mg, 0.9 mmol) were dissolved in DMF (3 mL) and mercury dichloride [7487-94-7] (244 mg, 0.9 mmol) was added. The reaction mixture was stirred for 5 minutes and triethylamine (0.12 ml, 0.9 mmol) was added. The reaction mixture was heated at 90 °C for 3 h before diluting with DCM (20 mL) and the reaction mixture was filtered and concentrated *in vacuo.* The crude residue was extracted with EtOAc (3 x 20 mL), washed with water (20 mL) and the organic layer was separated and dried (MgSO₄), filtered, and the filtrate was concentrated *in vacuo* to afford the crude product which was purified by flash column chromatography on silica (gradient elution with 0-100% EtOAc then 0-50% MeOH in DCM) to yield the product which was further purified by preparative HPLC to afford the *title compound* (14 mg, 9.8%) as a white solid. LCMS [M+H]⁺ 479.16, RT 2.22 minutes, 100.0% purity *(Method 10).*

### EXAMPLE 133

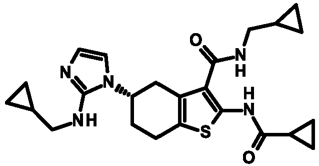

### (5S)-2-(Cyclopropanecarbonylamino)-N-(cyclopropylmethyl)-5-[2-(cyclopropylmethylamino)imidazol-1-yl]-4,5,6,7-tetrahydrobenzothiophene-3-carboxamide

*Intermediate 151* (92%, 412 mg, 0.7 mmol) was dissolved in EtOH (20 mL) and formic acid [624-84-0] (20 mL). 10% Pd/C (50% wet) (5%, 0.74 g, 0.35 mmol) was added and the reaction was heated at 90 °C for 2.5 h. The reaction was heated at 90 °C for a further 18 h before filtering through glass filter paper to remove Pd/C and washed with EtOH (50 mL). The filtrate was concentrated *in vacuo* and the residue (400 mg) was dissolved in formic acid [624-84-0] (20 mL) and 10% Pd/C (50% wet) (5%, 0.74 g, 0.35 mmol) added. The reaction was heated at 100 °C for 2 h before addition of further 10% Pd/C (50% wet) (740 mg) and the reaction was heated at 110 °C for 75 minutes. The reaction was allowed to cool and filtered through glass filter paper washing though with formic acid [624-84-0] (30 mL). The filtrate was concentrated *in vacuo* to give an oil, EtOAc (80 mL) was added and the organic layer washed with saturated aqueous sodium hydrogen carbonate solution (2 x 20 mL), brine, dried (Na₂SO₄), filtered and concentrated *in vacuo* to give an oil which was purified by flash column chromatography on silica (gradient elution with 0 - 10% MeOH in DCM) to give the product (25 mg) as a yellow oil. The product was dissolved in EtOH (0.5 mL) and precipitated by addition of water (a few drops) to give a white solid which was collected by filtration, washing with water. The solid and filtrate were combined in MeCN and concentrated *in vacuo.* The residue (an oil) was dissolved in MeCN (0.5 mL) and drops of water added until a white precipitate formed, Et₂O was added and the mixture concentrated *in vacuo.* The solid was dried under vacuum at 50 °C to afford the *title compound* (20 mg, 6%) as a white solid. δ_{H} (500 MHz, DMSO-*d*₆) 11.16 (s, 1H), 7.70 (s, 1H), 6.70 (d, *J* 1.5 Hz, 1H), 6.46 (d, *J* 1.2 Hz, 1H), 5.71 (s, 1H), 4.34 - 4.21 (m, 1H), 3.12 (dh, *J* 19.8, 6.3 Hz, 2H), 3.03 (t, *J* = 6.2 Hz, 2H), 3.01 - 2.92 (m, 1H), 2.91 - 2.71 (m, 3H), 2.13 - 1.99 (m, 2H), 1.97 - 1.85 (m, 1H), 1.14 - 1.04 (m, 1H), 1.05 - 0.95 (m, 1H), 0.92 - 0.77 (m, 4H), 0.43 - 0.34 (m, 4H), 0.26 - 0.10 (m, 4H). LCMS [M+H]⁺ 454.1, RT 2.21 minutes, 95% purity (*Method 10*)*.*

### EXAMPLE 134

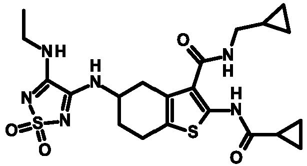

### 2-(Cyclopropanecarbonylamino)-N-(cyclopropylmethyl)-5-[[4-(ethylamino)-1,1-dioxo-1,2,5-thiadiazol-3-yl]amino]-4,5,6,7-tetrahydrobenzothiophene-3-carboxamide

*Intermediate 152* (67 mg, 0.13 mmol) was dissolved in EtOH (4 ml). 2M Ethyl amine in THF (2.7 mL, 5.4 mmol) was added and the reaction mixture was stirred at r.t for 3 h. The reaction mixture was concentrated *in vacuo* and the crude product was purified by preparative HPLC (acidic) to afford the *title compound* (35 mg, 52%) as a white solid. δ_{H} (500 MHz, CD₃OD) 4.29 - 4.22 (m, 1H), 3.44 (q,*J* 7.3 Hz, 2H), 3.29 - 3.23 (m, 2H), 3.22 - 3.17 (m, 1H), 2.88 - 2.80 (m, 2H), 2.81 - 2.73 (m, 1H), 2.20 - 2.10 (m, 2H), 1.85 - 1.77 (m, 1H), 1.28 (t, *J* 7.3 Hz, 3H), 1.15 - 1.05 (m, 1H), 1.02 - 0.90 (m, 4H), 0.59 - 0.49 (m, 2H), 0.32 - 0.22 (m, 2H). LCMS [M+H]⁺ 493.4, RT 2.71 minutes, 100% purity (*Method 10*)*.*

### EXAMPLE 135

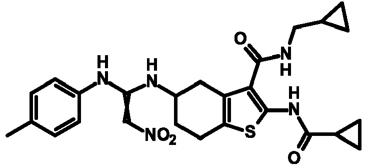

### 2-(cyclopropanecarbonylamino)-N-(cyclopropylmethyl)-5-[[1-(4-methylanilino)-2-nitro-vinyl]amino]-4,5,6,7-tetrahydrobenzothiophene-3-carboxamide

*Intermediate 122* (50 mg, 0.14 mmol) was dissolved in DCM (4 mL) and DIPEA (53 mg, 0.41 mmol). *Intermediate 153* (36 mg, 0.16 mmol), silver nitrate [7761-88-8] (25 mg, 0.15 mmol) and 2-propanol (785 mg, 13.1 mmol) was added. The reaction mixture was stirred at r.t. for ^{~}1.5 h followed by 100 °C in the microwave for 30 minutes. The reaction mixture was concentrated *in vacuo* and the residue purified by flash column chromatography on silica (gradient elution with 15-100% EtOAc/hexane) to afford the *title compound* (7.7 mg, 11%) as a yellow solid. δ_{H} (400 MHz, DMSO-*d*₆) 11.14 (s, 1H), 10.41 (br.s, 1H), 9.54 - 9.07 (m, 1H), 7.77 (t, *J* 5.6 Hz, 1H), 7.27 (d, *J* 8.0 Hz, 2H), 7.15 (d, *J* 8.1 Hz, 2H), 6.03 (br.s , 1H), 4.18 (br.s, 1H), 3.26 - 3.04 (m, 3H), 2.95 - 2.69 (m, 3H), 2.34 (s, 3H), 2.23 - 2.11 (m, 1H), 1.96 - 1.85 (m, 2H), 0.89 - 0.82 (m, 5H), 0.54 - 0.31 (m, 2H), 0.31 - 0.10 (m, 2H).

### EXAMPLE 136

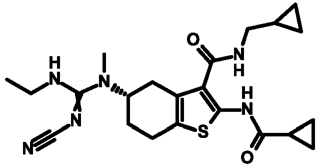

### (5S)-5-[(N'-cyano-N-ethyl-carbamimidoyl)-methyl-amino]-2-(cyclopropanecarbonylamino)-N-(cyclopropylmethyl)-4,5,6,7-tetrahydrobenzothiophene-3-carboxamide

To a solution of *intermediate 154* (439 mg, 1.26 mmol) in DMF (6.0 mL) in a pressure tube was added *intermediate 155* (239 mg, 1.26 mmol) and DIPEA (490 mg, 3.79 mmol). The reaction mixture was heated to 140 °C for 4 h and then left to stand at r.t. overnight. The reaction mixture was concentrated *in vacuo* and the residue was dissolved in EtOAc (50 mL) then washed with water (2 x 20 mL), saturated aqueous sodium hydrogen carbonate solution (2 x 20 mL) and brine (20 mL). The organic layer was separated, dried (Na₂SO₄), filtered and the filtrate concentrated *in vacuo* to give a brown foam (0.638 g). The product was pooled with smaller batches (42 mg), (55 mg) and (127 mg) and evaporated to yield a single batch (0.87 g) which was purified by preparative HPLC (basic) to afford the racemic *title compound* (373 mg). The product was purified by chiral preparative HPLC to afford the *title compound* (103 mg, 18%). δ_{H} (500 MHz, Chloroform-*d*) 11.95 (s, 1H), 6.08 (t, *J* 5.1 Hz, 1H), 4.95 (t, *J* 4.2 Hz, 1H), 4.49 (tt, *J* 10.3, 4.8 Hz, 1H), 3.64 - 3.51 (m, 2H), 3.36 - 3.20 (m, 2H), 3.06 (dd, *J* 14.4, 5.1 Hz, 1H), 2.94 (s, 3H), 2.88 - 2.81 (m, 2H), 2.74 - 2.64 (m, 1H), 2.04 - 1.90 (m, 2H), 1.65 (tt, *J* 8.0, 4.6 Hz, 1H), 1.27 (t, *J* 7.2 Hz, 3H), 1.13 - 1.04 (m, 3H), 0.93 - 0.85 (m, 2H), 0.58 - 0.52 (m, 2H), 0.31 - 0.23 (m, 2H). LCMS [M+H]⁺ 443, RT 2.85 minutes, 100% purity (*Method 10*).

### EXAMPLE 137

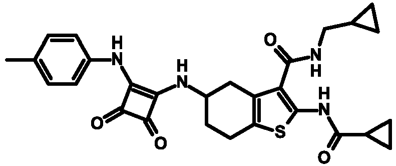

### 2-(Cyclopropanecarbonylamino)-N-(cyclopropylmethyl)-5-[[2-(4-methylanilino)-3,4-dioxo-cyclobuten-1-yl]amino]-4,5,6,7-tetrahydrobenzothiophene-3-carboxamide

*Intermediate 136* (95%, 50 mg, 0.1 mmol) was suspended in EtOH (1 mL) and 4-methylaniline [106-49-0] (50 mg, 0.47 mmol) was added. The mixture was heated to 160 °C in the microwave for 2 h. The solvent was removed *in vacuo* and the residue purified by preparative HPLC (basic) to afford the *title compound* (16.4 mg, 29%) as a white solid. δ_{H} (500 MHz, DMSO-*d*₆) 11.14 (br. s, 1H), 9.55 (br. s, 1H), 7.86 - 7.66 (m, 2H), 7.32 (d, *J* 8.0 Hz, 2H), 7.13 (d, *J* 8.3 Hz, 2H), 4.41 (br. s, 1H), 3.18 - 3.07 (m, 3H), 2.76 (br. s, 3H), 2.25 (s, 3H), 2.12 - 2.02 (m, 1H), 2.01 - 1.86 (m, 2H), 1.08 - 0.98 (m, 1H), 0.84 (s, 4H), 0.45 - 0.35 (m, 2H), 0.25 - 0.17 (m, 2H). LCMS [M+H]⁺ 519.3, RT 3.32 minutes, 95% purity (*Method 10*)*.*

### EXAMPLE 138

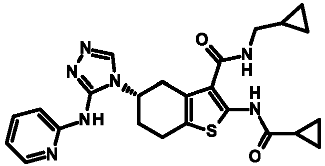

### (5S)-2-(Cyclopropanecarbonylamino)-N-(cyclopropylmethyl)-5-[3-(2-pyridylamino)-1,2,4-triazol-4-yl]-4,5,6,7-tetrahydrobenzothiophene-3-carboxamide

To a stirred solution of *intermediate 156* (92 mg, 0.20 mmol) and formic hydrazide [624-84-0] (35 mg, 0.59 mmol) in DMF (2.0 mL) was added mercury dichloride [7487-94-7] (159 mg, 0.59 mmol). The reaction mixture was stirred at r.t. for 5 minutes and triethylamine (0.082 ml, 0.59 mmol) was added. The reaction mixture was heated at 90 °C for 1 h before diluting with DCM (40 mL), filtering over a pad of celite and concentrating the filtrate *in vacuo.* The residue was diluted with water (20 mL) and extracted with EtOAc (3 x 20 mL). The organic extracts were combined, washed with water (10 mL) and brine (10 mL), dried (Na₂SO₄), filtered and the solvent removed *in vacuo.* The residue was purified by flash column chromatography on silica (gradient elution with 1 - 10% MeOH in DCM) followed by preparative HPLC (acidic) to afford the *title compound* (34 mg, 36%) as an off white solid. δ_{H} (500 MHz, DMSO-*d*₆) 11.19 (s, 1H), 9.34 (s, 1H), 8.52 (s, 1H), 8.11 (d, *J* 3.7 Hz, 1H), 7.73 (s, 1H), 7.65 (t, *J* 7.5 Hz, 1H), 7.40 (d, *J* 8.2 Hz, 1H), 6.84 (dd, *J* 6.0, 5.4 Hz, 1H), 4.42 (s, 1H), 3.17 - 3.03 (m, 3H), 3.01 - 2.93 (m, 1H), 2.79 (s, 2H), 2.18 (s, 2H), 1.95 - 1.86 (m, 1H), 1.02 - 0.91 (m, 1H), 0.89 - 0.78 (m, 4H), 0.39 - 0.29 (m, 2H), 0.20 - 0.13 (m, 2H). LCMS [M+H]⁺ 478, RT 2.05 minutes, purity 100 *% (Method 10).*

### EXAMPLE 139

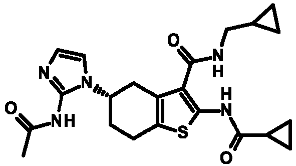

### (5S)-5-(2-Acetamidoimidazol-1-yl)-2-(cyclopropanecarbonylamino)-N-(cyclopropylmethyl)-4,5,6,7-tetrahydrobenzothiophene-3-carboxamide

*Intermediate* 158 (30 mg, 0.08 mmol) was dissolved in DCM (3 mL), triethylamine (12.6 µl, 0.09 mmol) was added followed by a solution of 1.41 M acetyl chloride in DCM (53 µL, 1 eq). The reaction was stirred at r.t. for 5 minutes. Further 1.41 M acetyl chloride in DCM (5 µL) was added and the reaction was stirred for 15 minutes. The reaction was quenched with saturated aqueous sodium hydrogen carbonate solution (2 mL), separated and extracted with DCM (2 x 2 mL). The organic layers were washed with brine (1 mL), dried (Na₂SO₄), filtered and concentrated *in vacuo* to yield a clear oil (30 mg) which was purified by silica flash column chromatography (gradient elution with 0 - 10 % MeOH in DCM) followed by silica flash column chromatography (gradient elution 0 - 3 % MeOH in DCM) to give the product. To the product was added Et₂O and the mixture was sonicated to give a white solid. The Et₂O was removed and the material dried in a vacuum oven to afford the *title compound* (14 mg, 42%) as a white solid. δ_{H} (500 MHz, DMSO-*d*₆) 11.09 (s, 1H), 9.87 (s, 1H), 7.73 (t, *J* 5.5 Hz, 1H), 7.20 (s, 1H), 6.84 (s, 1H), 4.25 - 4.09 (m, 1H), 3.11 (t, *J* 6.0 Hz, 2H), 3.04 - 2.89 (m, 2H), 2.85 - 2.72 (m, 2H), 2.18 - 1.84 (m, 6H), 1.04 - 0.95 (m, 1H), 0.91 - 0.75 (m, 4H), 0.38 (d, *J* 8.0 Hz, 2H), 0.19 (d, *J* 4.3 Hz, 2H). LCMS [M+H]⁺ 442.1, RT 1.81 minutes, 100% purity (*Method 10*).

### EXAMPLE 140

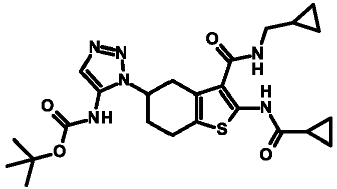

### tert-Butyl N-[3-[2-(cyclopropanecarbonylamino)-3-(cyclopropylmethylcarbamoyl)-4,5,6,7-tetrahydrobenzothiophen-5-yl]triazol-4-yl]carbamate

*Intermediate 161* (40 mg, 0.093 mmol) was suspended in toluene (2 mL) and triethylamine (28 mg, 0.28 mmol, *tert*-butyl alcohol (15 mg, 0.21 mmol) and diphenylphosphoryl azide [26386-88-9] (77 mg, 0.28 mmol) were added and the mixture was heated at 90 °C for 3 h. The solution was allowed to cool and the solvent removed *in vacuo* to give the residue which was purified by flash column chromatography on silica (gradient elution with 0-80% EtOAc/iso-hexane) to afford the *title compound* (26mg, 56%) as a colourless gum. LCMS (ES+) [M+H]⁺ 501.2, RT 2.237 minutes, 100.0% purity *(Method 2).* LCMS (ES+) [M+H]⁺ 501.2, RT 2.247 minutes, 95.9% purity *(Method 3).*

### EXAMPLE 141

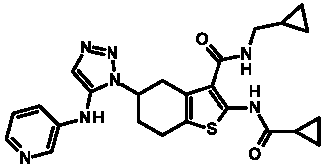

### 2-(Cyclopropanecarbonylamino)-N-(cyclopropylmethyl)-5-[5-(3-pyridylamino)triazol-1-yl]-4,5,6,7-tetrahydrobenzothiophene-3-carboxamide

To a microwave vial was added 2-(dicyclohexylphosphino)-2',4',6'-triisopropylbiphenyl XPhos (1 mg, 0.0021 mmol) and tBuXPhos (0.01 equiv., 0.00015 mmol). A solution of *intermediate 162* (6 mg, 0.015 mmol) in *tert*-butyl alcohol (1.56 g, 20.9 mmol) was added, followed by 3-bromopyridine [626-55-1] (3.08 mg, 0.019 mmol) and potassium tert-butoxide (3.60 mg, 0.031 mmol). The reaction was heated at 85 °C for 3 h. The reaction mixture was cooled and left to stand overnight. The solvent was removed *in vacuo* and the residue partitioned between water (3 mL) and DCM (5 mL). The organic layer was separated, dried (Na₂SO₄), filtered and the solvent removed to give a residue which was purified by flash column chromatography on silica (gradient elution with 0-10% MeOH/DCM) to afford the *title compound* (3.8 mg, 53%) which was freeze-dried to give an off white solid. δ_{H} (300 MHz, CD₃OD) 8.24 (d, *J* 2.8 Hz, 1H), 8.12 - 7.97 (m, 1H), 7.65 (s, 1H), 7.45 - 7.21 (m, 2H), 4.76 (s, 1H), 3.36 (s, 1H), 3.33 - 3.06 (m, 1H), 2.96 (d, *J* 7.1 Hz, 2H), 2.66 - 2.47 (m, 1H), 2.37 (d, *J* 12.7 Hz, 1H), 1.81 (tt, *J* 7.7, 4.7 Hz, 1H), 1.30 (s, 2H), 1.10 - 0.86 (m, 4H), 0.51 - 0.38 (m, 2H), 0.28 - 0.16 (m, 2H). LCMS (ES+) [M+Na]⁺ 500.167, RT 1.593 minutes, 100.0% purity *(Method 2).* LCMS (ES+) [M+H]⁺ 478.196, RT 1.829 minutes (*Method 3*)*.*

### EXAMPLE 142

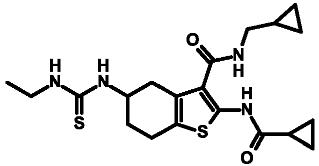

### 2-(Cyclopropanecarbonylamino)-N-(cyclopropylmethyl)-5-(ethylcarbamothioylamino)-4,5,6,7-tetrahydrobenzothiophene-3-carboxamide

*Intermediate 122* (50 mg, 0.14 mmol) was dissolved in DCM (5 mL) and DIPEA (26 mg, 0.20 mmol) and ethyl isothiocyanate [542-85-8] (18 mg, 0.21 mmol) were added. The reaction mixture was stirred at r.t. for ~16 h. The reaction mixture was directly purified by flash column chromatography on silica (gradient elution with 30-90% EtOAc/hexane) followed by further flash column chromatography on silica (gradient elution with 35-80% EtOAc/hexane). The product was freeze-dried from MeCN/water to afford the *title compound* (6.5 mg, 11%) as a white solid. δ_{H} (400 MHz, DMSO-*d*₆) 11.12 (s, 1H), 7.68 (t, *J* 5.7 Hz, 1H), 7.51 - 7.16 (m, 2H), 4.42 (s, 1H), 3.45 - 3.34 (m, 2H), 3.22 - 3.06 (m, 2H), 3.01 (dd, *J* 16.1, 5.1 Hz, 1H), 2.75 - 2.64 (m, 2H), 2.61 - 2.52 (m, 1H), 2.02 - 1.87 (m, 2H), 1.86 - 1.73 (m, 1H), 1.06 (t, *J* 7.2 Hz, 4H), 0.90 - 0.76 (m, 4H), 0.47 - 0.37 (m, 2H), 0.28 - 0.18 (m, 2H).

### EXAMPLE 143

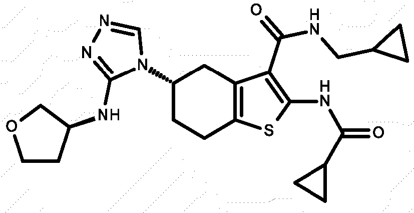

### (5S)-2-(cyclopropanecarbonylamino)-N-(cyclopropylmethyl)-5-[3-[[(3S)-tetrahydrofuran-3-yl]amino]-1,2,4-triazol-4-yl]-4,5,6,7-tetrahydrobenzothiophene-3-carboxamide

*Intermediate 189* (47.5 mg, 0.07 mmol) was dissolved in DCM (2 mL) and TFA (52 µL, 0.68 mmol) was added. The reaction was stirred at r.t. for 18 h, then 2M sodium carbonate solution was added and the organic phase taken. The aqueous phase was extracted with DCM (4 x 3 mL) and the organic phases combined, passed through a phase separator and concentrated *in vacuo.* The residue was suspended in 1:1 DMSO:MeOH (1 mL), filtered and purified via low pH, reverse-phase HPLC to afford a white powder. The powder was dissolved in 1:1 water:MeCN and freeze-dried to afford a white powder, which was subsequently triturated with heptane, then dissolved in acetonitrile and filtered under vacuum. The filtrate was concentrated *in vacuo* to afford the formic acid salt of the *title compound* (8.3 mg, 23%) as a colourless glass. δ_{H} (500 MHz, DMSO-d₆) 11.22 (s, 1H), 8.12 (s, 1H), 7.67 (s, 1H), 6.15 (d, *J* 5.9 Hz, 1H), 4.31 - 4.23 (m, 1H), 4.23 - 4.16 (m, 1H), 3.86 (dd, *J* 9.0, 6.0 Hz, 1H), 3.84 - 3.78 (m, 1H), 3.77 - 3.66 (m, 2H), 3.59 - 3.55 (m, 1H), 3.21 - 3.13 (m, 1H), 3.11 - 3.00 (m, 2H), 2.88 - 2.77 (m, 3H), 2.20 - 2.06 (m, 3H), 1.94 - 1.82 (m, 2H), 1.05 - 0.96 (m, 1H), 0.89 - 0.81 (m, 4H), 0.41 - 0.35 (m, 2H), 0.21 - 0.17 (m, 2H).

LCMS: [M+H]⁺ 471.1, RT 1.73 minutes, purity 97% *(Method 10).*

### EXAMPLE 144

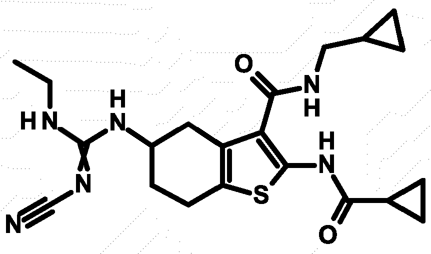

### 5-[[N'-Cyano-N-ethyl-carbamimidoyl]amino]-2-(cyclopropanecarbonylamino)-N-(cyclopropylmethyl)-4,5,6,7-tetrahydrobenzothiophene-3-carboxamide

*Intermediate 164* (35 mg, 0.073 mmol) was dissolved in DCM (3 mL) and MeOH (60 mL) and 2.0 M ethylamine in THF (1.62 g, 4.0 mmol, 2.0 mol/L) was added. The reaction mixture was stirred at r.t. for ~5 h before concentrating onto silica. The crude product was purified by flash column chromatography on silica (gradient elution with 35-100% EtOAc/hexane to 20% MeOH/EtOAc) before freeze drying from MeCN/water to afford the *title compound* (19 mg, 60.5%) as a white solid. δ_{H} (400 MHz, DMSO-*d*₆) 11.08 (s, 1H), 7.73 (s, 1H), 6.98 (t, *J* 5.6 Hz, 1H), 6.74 (d, *J* 8.1 Hz, 1H), 3.86 (s, 1H), 3.25 - 3.06 (m, 4H), 2.94 (dd, *J* 16.0, 5.0 Hz, 1H), 2.80 - 2.65 (m, 2H), 2.60 (dd, *J* 15.8, 9.5 Hz, 1H), 2.02 - 1.65 (m, 3H), 1.10 - 0.97 (m, 4H), 0.91 - 0.73 (m, 4H), 0.55 - 0.31 (m, 2H), 0.31 - 0.11 (m, 2H).

### EXAMPLES 145 and 146

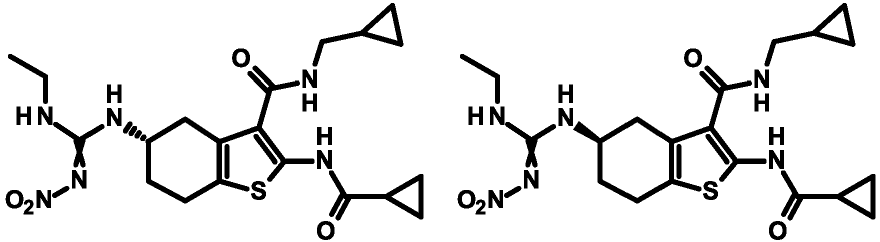

### Example 145

### (5S)-2-(Cyclopropanecarbonylamino)-N-(cyclopropylmethyl)-5-[[(E)-N-ethyl-N'-nitro-carbamimidoyl]amino]-4,5,6,7-tetrahydrobenzothiophene-3-carboxamide

### Example 146

### (5R)-2-(Cyclopropanecarbonylamino)-N-(cyclopropylmethyl)-5-[[(E)-N-ethyl-N'-nitro-carbamimidoyl]amino]-4,5,6,7-tetrahydrobenzothiophene-3-carboxamide

To a solution of *intermediate 140* (224 mg, 0.50 mmol) in MeOH (6 mL) in a pressure tube was added 2M ethylamine in THF (0.37 mL, 0.75 mmol). The reaction mixture was flushed with nitrogen and warmed to 70 °C with stirring for 60 minutes before leaving to cool to r.t. overnight. The reaction mixture was concentrated *in vacuo* and re-suspended in MeOH (3 mL). The mixture was transferred to a pressure tube and warmed to 80 °C and then cooling to r.t. The reaction mixture was diluted with - 0.5 mL MeOH and filtered under vacuum and washed with further MeOH (0.5 mL) to afford a colourless solid (0.15 g) which was chirally separated (85% heptane:15% ethanol, Chiral OD-H, 25cm, 18ml/min) and freeze dried from 1:1 MeCN/water to afford *title compound example 145* and *title compound example 146.* Each compound was repurified by flash column chromatography on silica (gradient elution with 1:1 heptane / EtOAc (10 mL) - 1:3 heptane / EtOAc (20 mL) - neat EtOAc (100 mL)) to afford *title compound example 145* and *title compound example 146* as colourless solids which were repurified by flash column chromatography on silica [gradient elution with 1:1 heptane / EtOAc (10 mL) - 1:3 heptane / EtOAc (20 mL) - neat EtOAc (100 mL)] to afford the *title compound example 145* (24 mg, 11%) and *title compound example 146* (18 mg, 8%) as colourless solids. *Title compound example 145:* δ_{H} (500 MHz, DMSO-*d*₆) 11.09 (s, 1H), 9.18 (s, 1H), 7.73 (s, 1H), 3.96 (s, 1H), 3.29 - 3.23 (m, 2H), 3.18 (dt, *J* 11.5, 6.0 Hz, 1H), 3.10 (dt, *J* 13.2, 6.1 Hz, 1H), 3.00 (d, *J* 13.7 Hz, 1H), 2.75 (s, 2H), 2.69 - 2.62 (m, 1H), 2.01 (d, *J* 8.8 Hz, 1H), 1.96 - 1.80 (m, 2H), 1.11 (t, *J* 7.0 Hz, 3H), 1.03 (dt, *J* 12.2, 5.5 Hz, 1H), 0.91 - 0.75 (m, 4H), 0.45 - 0.36 (m, 2H), 0.22 (q, J 4.8 Hz, 2H). LCMS [M+H]⁺ 449, RT 2.90 minutes, 99% purity (*Method 10*). *Title compound example 146*: δ_{H} (500 MHz, DMSO-*d*₆) 11.09 (s, 1H), 9.16 (s, 1H), 7.73 (s, 1H), 3.96 (s, 1H), 3.29 - 3.23 (m, 2H), 3.22 - 3.14 (m, 1H), 3.14 - 3.06 (m, 1H), 3.00 (d, *J* 14.0 Hz, 1H), 2.75 (s, 2H), 2.67 (d, *J* 10.7 Hz, 1H), 2.00 (s, 1H), 1.96 - 1.80 (m, 2H), 1.11 (t, *J* 7.0 Hz, 3H), 1.02 (td, *J* 12.5, 6.5 Hz, 1H), 0.85 (d, *J* 7.8 Hz, 4H), 0.46 - 0.36 (m, 2H), 0.22 (q, *J* 4.8 Hz, 2H). LCMS [M+H]⁺ 449, RT 2.90 minutes, 100% purity (*Method 10*)*.*

### EXAMPLE 147

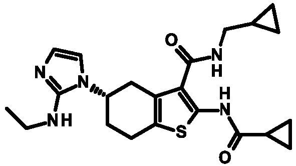

### (5S)-2-(Cyclopropanecarbonylamino)-N-(cyclopropylmethyl)-5-[2-(ethylamino)imidazol-1-yl]-4,5,6,7-tetrahydrobenzothiophene-3-carboxamide

To a stirred solution of *intermediate 167* (235 mg, 0.45 mmol) in a mixture of formic acid [64-18-6] (88% purity, 12 mL) and EtOH (12 mL) was added 10% palladium on charcoal (50% wet, 483 mg). The reaction mixture was heated at 90 °C for 5 h. A second aliquot of 10% palladium on charcoal (50% wet, 483 mg) was added and heating at 90 °C was continued for a further 16 h. The reaction mixture was cooled to r.t. and filtered through a pad of celite. The filter cake was extracted with EtOH (20 mL) and formic acid [64-18-6] (20 mL). The filtrate and extracts were combined and the solvent removed *in vacuo.* The residue was dissolved in EtOAc (50 mL), washed with saturated aqueous sodium hydrogen carbonate (2 x 10 mL) and brine (10 mL), dried (Na₂SO₄), filtered and the solvent removed *in vacuo.* The residue was purified by flash column chromatography on silica (gradient elution with MeOH in DCM 1% to 10%) to afford the *title compound* (94 mg, 47%) as an off-white solid. δ_{H} (500 MHz, DMSO-*d*₆) 11.16 (s, 1H), 7.70 (s, 1H), 6.72 (d, *J* 1.3 Hz, 1H), 6.49 (d, *J* 1.3 Hz, 1H), 5.67 (s, 1H), 4.23 (dq, *J* 9.4, 5.0 Hz, 1H), 3.22 - 3.06 (m, 4H), 2.96 (dd, *J* 15.3, 4.8 Hz, 1H), 2.87 - 2.75 (m, 3H), 2.09 - 1.99 (m, 2H), 1.91 (p, *J* 6.2 Hz, 1H), 1.12 (t, *J* 7.1 Hz, 3H), 1.05 - 0.95 (m, 1H), 0.87 - 0.82 (m, 4H), 0.41 - 0.34 (m, 2H), 0.22 - 0.15 (m, 2H). LCMS: M+H⁺ 428, RT = 1.95 min (97 %) (*Method 10*)*.*

### EXAMPLE 148

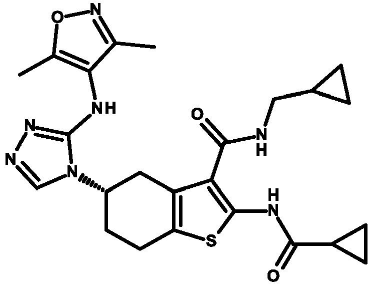

### (5S)-2-(Cyclopropanecarbonylamino)-N-(cyclopropylmethyl)-5-[3-[(3,5-dimethylisoxazol-4-yl)amino]-1,2,4-triazol-4-yl]-4,5,6,7-tetrahydrobenzothiophene-3-carboxamide

*Intermediate 168* (250 mg, 0.51 mmol), formic hydrazide [624-84-0] (92 mg, 1.54 mmol) were dissolved in DMF (3 mL). Mercury dichloride [7487-94-7] (418 mg, 1.54 mmol) was added to the reaction mixture. The mixture was stirred for 5 minutes and triethylamine [121-44-8] (0.21 mL, 1.54 mmol) was added to this mixture and the reaction mixture was heated at 90 °C for 1 h. The reaction mixture was diluted with DCM (20 mL), filtered, and the filtrate was concentrated *in vacuo.* The crude material was extracted with EtOAc (3 x 20 mL) and washed with water (20 mL). The organic layers were dried (MgSO₄), filtered and the filtrate was concentrated to afford the desired product. The crude product was purified by flash column chromatography on silica (gradient elution with 0-100% EtOAc then 0-50% MeOH in DCM) to afford the desired product which was further purified by preparative HPLC (acidic) to afford the *title compound* (37 mg, 15%). δ_{H} (500 MHz, DMSO-*d*₆) 11.26 (s, 1H), 8.23 (s, 1H), 7.74 (s, 1H), 7.73-7.70 (m, 1H), 4.38 (d, *J* 15.5 Hz, 1H), 3.22-3.16 (m, 2H), 3.09-3.05 (m, 1H), 3.01 - 2.91 (m, 1H), 2.90-2.85 (m, 2H), 2.26 (s, 3H), 2.25-2.20 (m, 2H), 2.06 (s, 3H), 1.95-1.91 (m, 1H), 1.03-0.98 (m, 1H), 0.91 - 0.77 (m, 4H), 0.42 - 0.32 (m, 2H), 0.22-0.18 (m, 2H). LCMS [M+H]⁺ 496.22, RT 2.17 minutes, 99% purity *(Method 10).*

### EXAMPLE 149

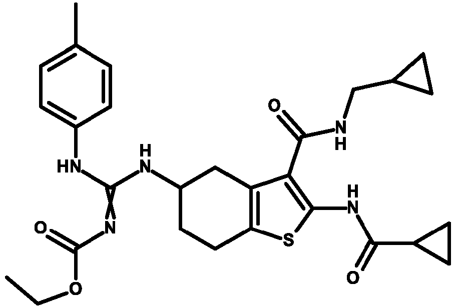

### ethyl N-[[[2-(cyclopropanecarbonylamino)-3-(cyclopropylmethylcarbamoyl)-4,5,6,7-tetrahydrobenzothiophen-5-yl]amino]-(4-methylanilino)methylene]carbamate

A stirred solution of *intermediate 169* (35 mg, 0.14 mmol), *intermediate 122* (60 mg, 0.16 mmol) and DIPEA (0.057 mL, 0.32 mmol) in DCM (4 mL) was cooled to 0 °C. EDCI [1892-57-5] (37 mg, 0.19 mmol) was added and the solution was stirred at r.t. overnight. The reaction mixture was washed with water (20 mL), brine, dried (Na₂SO₄), filtered and concentrated *in vacuo.* The crude residue was purified by acidic preparative HPLC to afford the *title compound* (50 mg, 57%) as a white solid. δ_{H} (500 MHz, CD₃OD) 7.21 (d, *J* 8.1 Hz, 2H), 7.12 (d, *J* 8.1 Hz,2H), 4.36 - 4.21 (m, 1H), 4.11 (q, J 7.1 Hz, 2H), 3.29 - 3.19 (m, 2H), 3.15 - 3.(m, 1H), 2.90 - 2.81 (m, 1H), 2.80 - 2.71 (m,1H), 2.69 - 2.55 (m, 1H), 2.34 (s, 3H), 2.17 - 2.05 (m, 1H), 1.97 - 1.83 (m, 1H),1.83 - 1.75 (m, 1H), 1.25 (t, *J* 7.1 Hz, 3H), 1.13 - 1.05 (m, 1H), 1.01 - 0.91 (m,4H), 0.58 - 0.47 (m, 2H), 0.33 - 0.23 (m, 2H). 2.5% w/w HCOOH, 0.4% w/w DMSO and 0.3% w/w. LCMS [M+H]⁺ 538.2, RT 2.58 minutes, 100% purity *(Method 10).*

### EXAMPLE 150

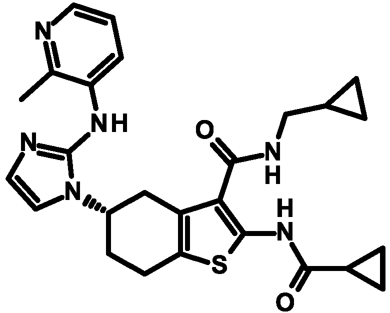

### (5S)-2-(Cyclopropanecarbonylamino)-N-(cyclopropylmethyl)-5-[2-[(2-methyl-3-pyridyl)amino]imidazol-1-yl]-4,5,6,7-tetrahydrobenzothiophene-3-carboxamide

*Intermediate 170* (225 mg, 0.47 mmol) was dissolved in DMF (4.5 mL) and 2,2-diethoxyethanamine [645-36-3] (0.2 mL, 1.4 mmol) was added followed by mercury dichloride [7487-94-7] (380 mg, 1.4 mmol). The reaction was stirred at r.t. for 5 minutes and triethylamine (0.19 mL, 1.4 mmol) was added. The reaction mixture was heated to 90 °C and stirred for 1 h. p-Toluene sulfonic acid (531 mg, 2.79 mmol) was added and the reaction heated at 90 °C for 18 h. The reaction mixture was allowed to cool and filtered through kieselguhr washing with DCM. The filtrate was concentrated in *vacuo* and the residue was dissolved in EtOAc (60 mL). The organics were washed with water (3 x 20 mL), dried (Na₂SO₄), filtered and concentrated *in vacuo.* The aqueous layer was basified to pH 9 using 1M aqueous NaOH and extracted with DCM (2 x 20 mL). The organic phases were dried (Na₂SO₄), filtered and concentrated *in vacuo* to afford a yellow liquid which was purified by flash column chromatography on silica (gradient elution with 0 - 10% MeOH in DCM) to give a yellow solid which was further purified by preparative HPLC (acidic) and again by flash column chromatography on silica (gradient elution with 0 - 10% MeOH in DCM) to afford the *title compound* (35 mg, 15%) as a yellow solid. δ_{H} (500 MHz, DMSO-*d*₆) 11.10 (s, 1H), 7.97 - 7.91 (m, 1H), 7.73 (t, *J* 5.7 Hz, 1H), 7.55 (s, 1H), 7.26 - 7.17 (m, 1H), 7.12 (d, *J* 1.4 Hz, 1H), 7.03 (dd, *J* 8.1, 4.7 Hz, 1H), 6.81 (d, *J* 1.3 Hz, 1H), 4.55 - 4.40 (m, 1H), 3.20 - 3.02 (m, 2H), 3.03 - 2.92 (m, 2H), 2.85 - 2.73 (m, 2H), 2.37 (s, 3H), 2.21 - 2.01 (m, 2H), 1.96 - 1.84 (m, 1H), 1.02 - 0.90 (m, 1H), 0.91 - 0.76 (m, 4H), 0.40 - 0.28 (m, 2H), 0.23 - 0.10 (m, 2H). LCSM [M+H]⁺ 491.3, RT 1.69 minutes, 97% purity (*Method 10*)*.*

### EXAMPLE 151

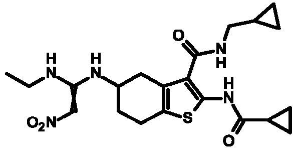

### 2-(cyclopropanecarbonylamino)-N-(cyclopropylmethyl)-5-[[1-(ethylamino)-2-nitro-vinyl]amino]-4,5,6,7-tetrahydrobenzothiophene-3-carboxamide

*Intermediate 122* (50 mg, 0.14 mmol) was dissolved in DCM (4 mL) and DIPEA (53 mg, 0.41 mmol). *Intermediate 171* (33 mg, 0.20 mmol), silver nitrate [7761-88-8] (25 mg, 0.15 mmol) and isopropanol (1 mL, 785 mg, 13.06 mmol) were added and the reaction mixture was stirred at 100 °C in the microwave for 60 minutes. Further silver nitrate [7761-88-8] (23 mg, 0.14 mmol) was added and the reaction mixture was stirred at r.t. for ^{~}16 h. Further *intermediate 171* (33 mg, 0.20 mmol) in DCM (1 mL) was added and the reaction mixture was stirred at r.t. for ^{~}16 h. Further silver nitrate [7761-88-8] (23 mg, 0.14 mmol) was added and the reaction mixture was stirred at r.t. for 6 days. The reaction mixture was concentrated *in vacuo* and partitioned between DCM and water. The mixture was passed through a phase separation cartridge and was concentrated *in vacuo* to give a residue which was purified by flash column chromatography on silica (gradient elution with 15-100% EtOAc/hexane to 0-20% MeOH/EtOAc). The material was freeze dried from MeCN/water to afford the *title compound* (8.3 mg, 14%) as a yellow solid. LCMS [M+H]⁺ 448.2, [M+Na]⁺ 470.0, RT 1.725 minutes, 100.0% purity *(Method 2).*

### EXAMPLE 152

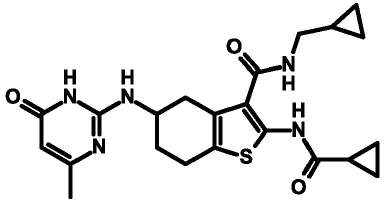

### 2-(Cyclopropanecarbonylamino)-N-(cyclopropylmethyl)-5-[(4-methyl-6-oxo-1H-pyrimidin-2-yl)amino]-4,5,6,7-tetrahydrobenzothiophene-3-carboxamide

A mixture of *intermediate 122* (40 mg, 0.1 mmoL), 6-methyl-2-(methylsulfanyl)-3,4-dihydropyrimidin-4-one [6328-58-1] (20 mg, 0.13 mmoL) and DIPEA (0.038 mL, 0.22 mmoL) in *tert-*butanol (2 mL) was heated to 120 °C in a microwave reactor. The reaction mixture was heated at 140 °C for 1 h, followed by 160 °C for 7 h. Further 6-methyl-2-(methylsulfanyl)-3,4-dihydropyrimidin-4-one [6328-58-1] (20 mg, 0.13 mmoL) was added and stirring was continued at 160 °C in the microwave reactor for 16 h. The reaction mixture was concentrated *in vacuo* and purified by preparative HPLC (acidic) to afford the *title compound* (2.5 mg, 5%) as a white solid. δ_{H} (250 MHz, CD₃OD) 5.61 (s, 1H), 4.44 - 4.31 (m, 1H), 3.27 - 3.10 (m, 3H), 2.84 (t, *J* 6.0 Hz, 2H), 2.75 - 2.60 (m, 1H), 2.15 (s, 3H), 2.13 - 2.05 (m, 1H), 2.05 - 1.92 (m, 1H), 1.86 - 1.70 (m, 1H), 1.16 - (m, 1H), 1.03 - 0.89 (m, 4H), 0.59 - 0.42 (m, 2H), 0.32 - 0.21 (m, 2H). LCMS [M+H]⁺ 442, RT 2.23 minutes, 100% purity (*Method 10*)*.*

### EXAMPLE 153

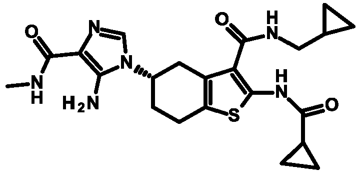

### 5-amino-1-[(5S)-2-cyclopropaneamido-3-[(cyclopropylmethyl)carbamoyl]-4,5,6,7-tetrahydro-1-benzothiophen-5-yl]-N-methyl-1H-imidazole-4-carboxamide

*Intermediate 48* (92%, 37 mg, 0.3 mmol) was stirred in dry MeCN (3 mL) in a 20 mL pressure tube. Triethylorthoformate (50 µl, 0.3 mmol) was added, the reaction was sealed and heated to 90 °C for 1 hour to give a yellow solution. The reaction was allowed to cool to room temperature and *intermediate 117* (100 mg, 0.3 mmol) was added. The solution was stirred for 17 hours to give a light brown suspension. The solid was collected by vacuum filtration and purified by flash column chromatography on silica (gradient elution with 0-100% ethyl acetate in heptane followed by 0 -10% MeOH in ethyl acetate) followed by purification by preparative HPLC (H₂O, MeCN, formic acid) and then trituration with MeCN then EtOH afford the *title compound* (40 mg, 29% at 98% UV purity) as a white solid. δ_{H} (500 MHz, DMSO-d₆) δ 11.20 (s, 1H), 7.71 (t, J 5.5 Hz, 1H), 7.34 (q, J 4.7 Hz, 1H), 7.24 (s, 1H), 5.81 (s, 2H), 4.33 - 4.24 (m, 1H), 3.19 - 3.04 (m, 3H), 3.01 - 2.93 (m, 1H), 2.90 - 2.75 (m, 2H), 2.69 (d, J 4.8 Hz, 3H), 2.24 - 2.10 (m, 2H), 1.97 - 1.88 (m, 1H), 1.07 - 0.96 (m, 1H), 0.91 - 0.79 (m, 4H), 0.44 - 0.32 (m, 2H), 0.26 - 0.15 (m, 2H). LCMS [M+H]⁺ 457.3, RT 2.21 minutes, 98% purity *(Method 10).*

### EXAMPLE 154

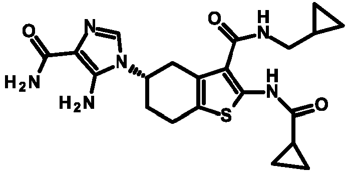

### 5-amino-1-[(5S)-2-cyclopropaneamido-3-[(cyclopropylmethyl)carbamoyl]-4,5,6,7-tetrahydro-1-benzothiophen-5-yl]-1H-imidazole-4-carboxamide

2-amino-2-cyanoacetamide (30 mg, 0.3 mmol) was stirred in dry MeCN (3 mL) in a 20 mL pressure tube. Triethylorthoformate (50 µl, 0.3 mmol) was added, the reaction was sealed and heated at 90 °C for 1 hour with stirring. The reaction was allowed to cool to room temperature, *intermediate 117* (100 mg, 0.3 mmol) was added and the reaction mixture was stirred at room temperature for 17 hours. The reaction solution was concentrated *in vacuo* and purified by flash column chromatography on silica (gradient elution using 0 - 100% ethyl acetate in heptane followed by 0 - 10% MeOH in ethyl acetate). The material obtained was purified further by preparative HPLC (H₂O, MeCN, formic acid) then by flash column chromatography on silica (gradient elution using 0 - 10% MeOH in DCM). Further purification by reverse phase column chromatography (gradient elution with 10-100% MeCN in water) afforded the *title compound* (10 mg, 7.5%) as a white solid. δ_{H} (500 MHz, DMSO-d₆) δ 11.21 (s, 1H), 7.71 (s, 1H), 7.23 (s, 1H), 6.75 (s, 1H), 6.61 (s, 1H), 5.87 (s, 2H), 4.37 - 4.15 (m, 1H), 3.21 - 3.03 (m, 3H), 3.03 - 2.92 (m, 1H), 2.90 - 2.75 (m, 2H), 2.22 - 2.08 (m, 2H), 1.97 - 1.87 (m, 1H), 1.07 - 0.95 (m, 1H), 0.92 - 0.75 (m, 4H), 0.44 - 0.34 (m, 2H), 0.26 - 0.14 (m, 2H). LCMS [M+H]⁺ 443.3, RT 2.16 minutes, 99% purity (*Method 10*)

### EXAMPLE 155

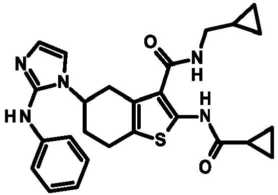

### 5-(2-Anilinoimidazol-1-yl)-2-(cyclopropanecarbonylamino)-N-(cyclopropylmethyl)-4,5,6,7-tetrahydrobenzothiophene-3-carboxamide

*Intermediate 176* (89%, 120 mg, 0.19 mmol) was dissolved in EtOH (5 mL) and a drop of concentrated sulphuric acid was added, followed by 20% Pd(OH)₂/C (30 mg, 0.21 mmol). The reaction mixture was stirred at 60 °C under ^{~}7 bar hydrogen for 24 h in a pressure vessel. Additional 20% Pd(OH)₂/C (30 mg, 0.21 mmol) was added and the reaction was returned to 60 °C under ^{~}7 bar hydrogen for a further 24 h. The reaction mixture was filtered through a pad of Celite and concentrated *in vacuo* to give the crude residue which was purified by flash column chromatography on silica (gradient elution with 0-10% MeOH/DCM) followed by preparative HPLC (basic) to afford the *title compound* (9.5 mg, 10%). δ_{H} (500 MHz, DMSO-*d*₆) 11.16 (s, 1H), 8.17 (s, 1H), 7.70 (t, *J* 5.8 H 1H), 7.30 (d, *J* 7.9 Hz, 2H), 7.20 - 7.12 (m, 2H), 7.01 (d, *J* 1.5 Hz, 1H), 6.80 - 6.69 (m, 2H), 4.49 (br. s, 1H), 3.16 - 3.03 (m, 2H), 3.03 - 2.90 (m, 2H), 2.87 - 2.78 (m, 2H), 2.21 - 2.10 (m, 1H), 2.10 - 2.03 (m, 1H), 1.96 - 1.88 (m, 1H), 1.00 - 0.90 (m, 1H), 0.89 - 0.80 (m, 4H), 0.35 - 0.30 (m, 2H), 0.18 - 0.14 (m, 2H). LCMS (ES+) [M+H]⁺ 476.1, RT 2.17 minutes, 95% purity *(Method 10).*

### EXAMPLE 156

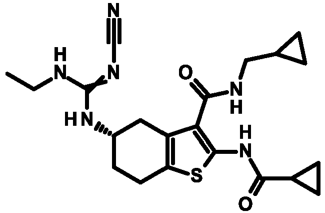

### (5S)-5-[[N'-cyano-N-ethyl-carbamimidoyl]amino]-2-(cyclopropanecarbonylamino)-N-(cyclopropylmethyl)-4,5,6,7-tetrahydrobenzothiophene-3-carboxamide

Diphenyl cyanocarbonimidate [79463-77-7] (210 mg, 0.88 mmol) was added to a solution of *intermediate 117* (265 mg, 0.79 mmol) in isopropanol (10 mL). The reaction mixture was stirred at r.t. for 1 h. DIPEA (140 µL, 0.8 mmol) was added and the reaction was stirred at r.t. for 1 h. 2M Ethanamine (1 mL) was added, and then solution was stirred at r.t. for 3 days. The solution was heated in the microwave at 100 °C for 2 h. The reaction mixture was concentrated *in vacuo* and the residue purified by flash column chromatography on silica (EtOAc), followed by further purification by flash column chromatography on silica (gradient elution with 0-5% MeOH/DCM) to afford the *title compound* (200 mg, 95% purity) as a light yellow foam which was further purified by acid prep-HPLC and freeze dried from 1:1 MeCN/H₂O to afford the *title compound* (120 mg, 35%) as a fluffy white solid. δ_{H} (500 MHz, DMSO-*d*₆) 11.07 (s, 1H), 7.75 - 7.67 (m, 1H), 7.01 - 6.94 (m, 1H), 6.73 (d, J 8.1 Hz, 1H), 3.84 (br. s, 1H), 3.23 - 3.07 (m, 4H), 2.93 (dd, J 16.0, 4.1 Hz, 1H), 2.75 - 2.68 (m, 2H), 2.59 (dd, *J* 15.7, 9.4 Hz, 1H), 1.98 - 1.87 (m, 2H), 1.86 - 1.76 (m, 1H), 1.06 (t, *J* 7.1 Hz, 3H), 1.04 - 0.98 (m, 1H), 0.88 - 0.78 (m, 4H), 0.45 - 0.39 (m, 2H), 0.25 - 0.20 (m, 2H). LCMS (ES+) [M+H]+ 429.1, RT 2.82 minutes, 99% purity *(Method 10).*

### EXAMPLE 157

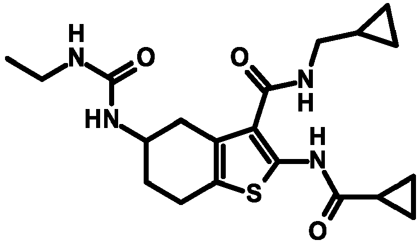

### 2-(Cyclopropanecarbonylamino)-N-(cyclopropylmethyl)-5-(ethylcarbamoylamino)-4,5,6,7-tetrahydrobenzothiophene-3-carboxamide

*Intermediate 122* (50 mg, 0.14 mmol) was dissolved in DCM (5 mL) and DIPEA (26 mg, 0.20 mmol). Ethyl isocyanate [109-90-0] (15 mg, 0.20 mmol) was added and the reaction mixture was stirred at r.t. for ^{~}16 h. The crude residue was pre-adsorbed onto silica from DCM/MeOH and the material was purified by flash column chromatography on silica (gradient elution with 30-100% EtOAc/hexane to 0-20%MeOH/EtOAc) to give the product which was freeze-dried from MeCN/water to afford the *title compound* (14 mg, 25%) as a white solid. δ_{H} (400 MHz, DMSO-*d*₆) 11.13 (s, 1H), 7.66 (t, *J* 5.6 Hz, 1H), 5.87 (d, *J* 7.7 Hz, 1H), 5.74 (t, *J* 5.6 Hz, 1H), 3.91 - 3.75 (m, 1H), 3.14 (td, *J* 7.0, 2.6 Hz, 2H), 3.01 (qd, *J* 7.1, 5.5 Hz, 2H), 2.91 (dd, *J* 16.1, 5.1 Hz, 1H), 2.77 - 2.61 (m, 2H), 2.47 - 2.40 (m, 1H), 2.01 - 1.77 (m, 2H), 1.77 - 1.57 (m, 1H), 1.11 - 1.00 (m, 1H), 0.98 (t, *J* 7.2 Hz, 3H), 0.90 - 0.78 (m, 4H), 0.51 - 0.31 (m, 2H), 0.27 - 0.15 (m, 2H).

### EXAMPLE 158

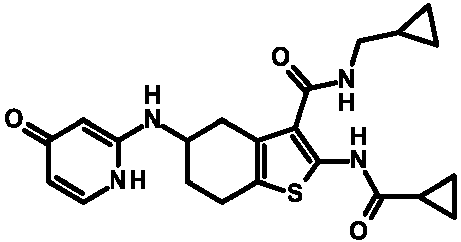

### 2-(Cyclopropanecarbonylamino)-N-(cyclopropylmethyl)-5-[(4-oxo-1H-pyridin-2-yl)amino]-4,5,6,7-tetrahydrobenzothiophene-3-carboxamide

*Intermediate 122* (46 mg, 0.12 mmol) and 2-chloro-4-hydroxypyridine [17368-12-6] (23 mg, 0.17 mmol) were dissolved in *tert*-butyl alcohol (0.4 mL) and DIPEA (60 µL, 0.35 mmol) was added. The reaction was sealed and heated in microwave at 160 °C for 36 h. The reaction mixture was concentrated *in vacuo* to give the crude residue as a red oil which was purified by flash column chromatography on silica (gradient elution with 0-20% MeOH/DCM) to give the crude product. The crude material was repurified by flash column chromatography on silica (gradient elution with 0-30% MeOH/DCM) to afford the product which was repurified by preparative HPLC to afford the *title compound* (2 mg, 3.8%) as an off white solid. LCMS [M+H]⁺ 427.21, RT 1.26 minutes, 98.53% (*Method 24*). LCMS [M+H]⁺ 427.21, RT 1.19 minutes, 98.94% (*Method 25*).

### EXAMPLE 159

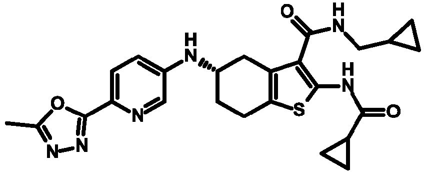

### (5S)-2-(cyclopropanecarbonylamino)-N-(cyclopropylmethyl)-5-[[6-(5-methyl-1,3,4-oxadiazol-2-yl)-3-pyridyl]amino]-4,5,6,7-tetrahydrobenzothiophene-3-carboxamide

Dry dioxane (3 mL) and dry ^{t}BuOH (1.5 mL) were added to *intermediate 117* (100 mg, 0.3 mmol), *intermediate 204* (101 mg, 0.42 mmol), NaO^{t}Bu (86 mg, 0.9 mmol) and ^{t}BuXPhos Pd G3 (36 mg, 0.04 mmol). The reaction mixture was sonicated for 1 minute and stirred at room temperature for 2 hours to give a light brown suspension. The reaction was quenched with saturated aq. NH₄Cl (20 mL) and extracted with DCM (2 x 20 mL). The organics were dried over sodium sulfate and concentrated *in vacuo* followed by purification by flash chromatography on silica (elution gradient using 0 - 100% ethyl acetate in heptane followed by 0 - 10% MeOH in ethyl acetate) then further purification by preparative HPLC (H₂O, MeCN, formic acid) to afford the *title compound* (42 mg, 28%) as an off white solid. δ_{H} (500 MHz, DMSO-*d*₆) δ 11.05 (s, 1H), 8.15 (d, J 2.7 Hz, 1H), 7.83 (s, 1H), 7.75 (s, 1H), 7.15 (dd, J 8.8, 2.8 Hz, 1H), 6.73 (d, J 7.7 Hz, 1H), 3.86 - 3.74 (m, 1H), 3.17 - 3.04 (m, 3H), 2.84 - 2.73 (m, 2H), 2.61 - 2.55 (m, 1H), 2.54 (s, 3H), 2.12 - 2.04 (m, 1H), 1.95 - 1.88 (m, 1H), 1.81 - 1.71 (m, 1H), 1.05 - 0.94 (m, 1H), 0.88 - 0.79 (m, 4H), 0.40 - 0.31 (m, 2H), 0.24 - 0.12 (m, 2H). LCMS [M+H]⁺ 493.3, RT 3.00 minutes, 100% purity (*Method 10*)

### EXAMPLE 160

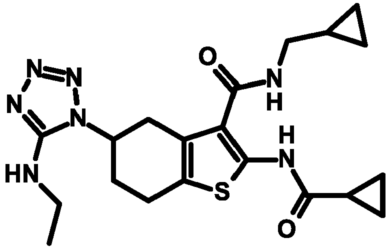

### 2-(Cyclopropanecarbonylamino)-N-(cyclopropylmethyl)-5-[5-(ethylamino)tetrazol-1-yl]-4,5,6,7-tetrahydrobenzothiophene-3-carboxamide

*Example 142* (150 mg, 0.36 mmol) was added to a stirred solution of mercury dichloride [7487-94-7] (290 mg, 1.08 mmol) and triethylamine (0.14 ml, 1.08 mmol) in DMF (4 mL). The reaction mixture was stirred for 5 minutes and sodium azide [26628-22-8] (70 mg, 1.06 mmol) was slowly added to the reaction mixture with continued stirring for 1 h at r.t. The reaction mixture was quenched with sodium hydrogen carbonate solution (10 mL) and the aqueous layer was extracted with EtOAc (3 x 20 mL). The organic phases were dried (MgSO₄), filtered and the filtrate concentrated *in vacuo* to yield the crude residue which was purified by preparative HPLC (acidic) to afford the *title compound* (22 mg, 14%) as a white solid. ¹H NMR (500 MHz, Chloroform-*d*) δ 11.95 (s, 1H), 5.76 (s, 1H), 4.28-4.23 (m, 2H), 3.60-3.55 (m, 2H), 3.38 - 3.27 (m, 2H), 3.27 - 3.18 (m, 1H), 3.18-3.13 (m, 1H), 2.99-2.93 (m, 2H), 2.51 - 2.38 (m, 1H), 2.34-2.29 (m, 1H), 1.69-1.61 (m, 1H), 1.33 (t, *J* 7.2 Hz, 3H), 1.14-1.08 (m, 2H), 1.06-1.02 (m, 1H), 0.96-0.89 (m, 2H), 0.58-0.51 (m, 2H), 0.28-0.21 (m, 2H). LCMS [M+H]⁺ 430.13, RT 2.87 minutes, 99% purity (*Method 10*).

### EXAMPLE 161

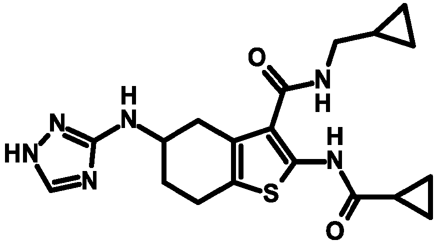

### 2-(Cyclopropanecarbonylamino)-N-(cyclopropylmethyl)-5-(1H-1,2,4-triazol-3-ylamino)-4,5,6,7-tetrahydrobenzothiophene-3-carboxamide

To a mixture of *intermediate 62* (70 mg, 0.21 mmol) and 1H-1,2,4-triazol-3-amine [61-82-5] (17.7 mg, 0.21 mmol) in acetic acid (3 mL) was gradually added sodium triacetoxyborohydride [56553-60-7] (67 mg, 0.31 mmol) whilst cooling in ice. The reaction mixture was stirred at r.t. for 16 h and the reaction mixture was diluted with DCM and washed with saturated sodium bicarbonate. The organic phase was dried (MgSO₄), filtered and concentrated *in vacuo* to give the crude residue which was purified by preparative HPLC (acidic) to afford the *title compound* (8 mg, 9%) as white solid. δ_{H} (500 MHz, CD₃OD) 7.64 (s, 1H), 3.94 - 3.84 (m, 1H), 3.27 - 3.16 (m, 3H), 2.89 - 2.79 (m, 2H), 2.65 (dd, *J* 15.7, 8.4 Hz, 1H), 2.23 - 2.13 (m, 1H), 1.95 - 1.84 (m, 1H), 1.83 - 1.74 (m, 1H), 1.12 -1.03 (m, 1H), 1.01 - 0.91 (m, 4H), 0.53 - 0.47 (m, 2H), 0.28 - 0.22 (m, 2H). LCMS [M+H]⁺ 401.2, RT 1.87 minutes, 98% purity *(Method 10).*

### EXAMPLE 162

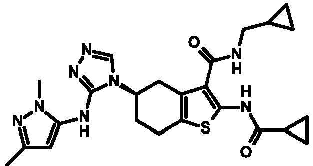

### (5R)-2-(Cyclopropanecarbonylamino)-N-(cyclopropylmethyl)-5-[3-[(2,5-dimethylpyrazol-3-yl)amino]-1,2,4-triazol-4-yl]-4,5,6,7-tetrahydrobenzothiophene-3-carboxamide

To a stirred solution of *intermediate 179* (3.4 g, 7.0 mmol) in DMF (35 mL) was added formic acid hydrazide [624-84-0] (1.4 g, 21 mmol) followed by mercuric chloride [7487-94-7] (3.8 g, 14 mmol). The mixture was stirred for 10 minutes at r.t. before the addition of triethylamine (2.9 mL, 21 mmol). The reaction mixture was stirred at 80 °C for 4 h and the reaction mixture was cooled to r.t. The mixture was diluted with MeCN (35 mL), filtered through a pad of celite and washed with MeCN (35 mL x 3). The filtrate was diluted with DCM (70 mL), washed with water (70 mL x 2), brine (70 mL), dried (Na₂SO₄), filtered and concentrated *in vacuo.* To the resulting residue was added water (70 mL) and the precipitated solid filtered, washed with water (70 mL x 3) and dried *in vacuo* to give crude product (5.9 g) as a beige solid. The solid was dissolved in DCM (20 mL), passed through a phase separator and purified by flash column chromatography on silica (gradient elution with 0% to 15% MeOH in DCM) to afford the product (960 mg) as a yellow foam which was further purified by reverse phase chromatography on silica (gradient elution with 0 to 100% MeCN [containing 0.1% ammonia] in water [containing 0.1% ammonia]) to give the product fractions which were diluted with DCM. The aqueous phase was separated, extracted with 10% MeOH in DCM (2 x) and the combined organic phases were passed through a phase separator and concentrated *in vacuo* to afford the product (625 mg) as a pale green solid. The crude product was dissolved in MeOH (20 mL) and the resulting pale green solution was treated with QuadraSil methylthiourea (1 g). The mixture was heated at 40 °C for 4 h and a few drops of water were added followed by further QuadraSil methylthiourea (0.5 g). The mixture was heated at 40 °C for 3 h before cooling to r.t. The cooled solution was filtered through celite, washed with MeOH (20 mL x 3) and the filtrate concentrated in *vacuo* to give the product which was further purified by flash column chromatography on silica (gradient elution with 0% to 15% MeOH in DCM) to afford the *title compound* (566 mg, 16%) as a white solid. δ_{H} (300 MHz, DMSO-*d*₆) 11.74 (s, 0.3H), 11.22 (m, 1H), 8.43 (s, 0.7H), 8.34 (s, 0.7H), 8.12 (s, 0.3H), 7.72 (t, *J* 6.1 Hz, 1H), 5.89 (s, 0.7H), 5.70 (s, 0.3H), 4.53 - 4.30 (m, 1H), 3.51 (m, 3H), 3.25 - 3.05 (m, 3H), 3.05 - 2.90 (m, 1H), 2.83 (m, 2H), 2.33 - 2.13 (m, 2H), 2.06 (m, 3H), 1.93 (p, *J* 6.3 Hz, 1H), 1.09 - 0.92 (m, 1H), 0.90 - 0.77 (m, 4H), 0.36 (ddd, *J* 8.0, 4.0, 1.7 Hz, 2H), 0.24 - 0.13 (m, 2H), compound partially rotameric. LCMS (ES+) [M+H]⁺ 495.0, RT 1.643 minutes, 100.0% purity (*Method 3*).

### EXAMPLE 163

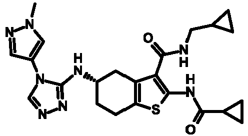

### (5S)-2-(Cyclopropanecarbonylamino)-N-(cyclopropylmethyl)-5-[[4-(1-methylpyrazol-4-yl)-1,2,4-triazol-3-yl]amino]-4,5,6,7-tetrahydrobenzothiophene-3-carboxamide

*Intermediate 180* (77%, 274 mg, 0.45 mmol) and formic hydrazide [624-84-0] (80 mg, 1.34 mmol) were dissolved in DMF (3 mL). Mercury dichloride [7487-94-7] (364 mg, 1.34 mmol) was added and the reaction mixture was stirred for 5 minutes. Triethylamine (0.19 ml, 1.34 mmol) was added to the reaction mixture, which was stirred for 1 h at 90 °C. The reaction mixture was diluted with DCM (20 mL), filtered, and the filtrate was concentrated *in vacuo* and the crude product was extracted with EtOAc (3 x 20 mL) and washed with water (20 mL). The organic phases were dried (MgSO₄), filtered and concentrated *in vacuo* to afford the product which was purified by flash column chromatography on silica (gradient elution with 0-100% EtOAc in DCM followed by 0-50% MeOH in DCM). The product was further purified twice by preparative HPLC to afford the *title compound* (50 mg, 23%) as a white solid. δ_{H} (500 MHz, DMSO-*d*₆) 11.01 (s, 1H), 8.08 (s, 1H), 8.07 (s, 1H), 7.70 (s, 1H), 7.65 (s, 1H), 5.71 (d, *J* 7.0 Hz, 1H), 3.87 (s, 3H), 3.83-3.80 (m, 1H), 3.15 - 2.99 (m, 3H), 2.75 - 2.68 (m, 2H), 2.61 (dd, *J* 16.0, 9.3 Hz, 1H), 2.21 - 2.02 (m, 1H), 1.97 - 1.63 (m, 2H), 1.03 - 0.96 (m, 1H), 0.90 - 0.73 (m, 4H), 0.47 - 0.30 (m, 2H), 0.22-0.19 (m, 2H). LCMS [M+H]⁺ 481.24, RT 4.08 minutes, 98% purity (*Method 22*)*.*

### EXAMPLE 164

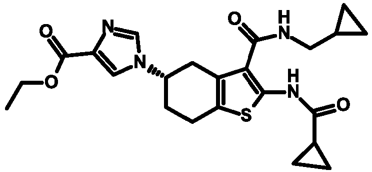

### ethyl 1-[(5S)-2-cyclopropaneamido-3-[(cyclopropylmethyl)carbamoyl]-4,5,6,7-tetrahydro-1-benzothiophen-5-yl]-1H-imidazole-4-carboxylate

*Intermediate 117* (100 mg, 0.3 mmol) and *intermediate 24* (50 mg, 0.3 mmol) were heated in ⁿBuOH (1 mL) in a sealed tube with stirring at 70 °C for 1.5 hours then at 120 °C for 17 hours with stirring. Reaction was allowed to cool to room temperature and purified by SCX column (2 g, washed with MeOH and eluted with 3M NH₃ in MeOH). The brown residue obtained was purified further by preparative HPLC (H₂O, MeCN, NH₄OH) to afford the *title compound* (5 mg, 3% at 85% purity) as a yellow solid. δ_{H} (500 MHz, Methanol-d4) δ 7.95 (s, 1H), 7.87 (s, 1H), 4.68 - 4.59 (m, 1H), 4.32 (q, J 7.1 Hz, 2H), 3.29 - 3.22 (m, 2H), 3.21 - 3.10 (m, 2H), 3.01 - 2.80 (m, 2H), 2.38 - 2.22 (m, 2H), 1.85 - 1.73 (m, 1H), 1.35 (t, J 7.1 Hz, 3H), 1.13 - 1.03 (m, 1H), 1.03 - 0.96 (m, 2H), 0.96 - 0.88 (m, 2H), 0.53 - 0.43 (m, 2H), 0.27 - 0.21 (m, 2H). LCMS [M+H]⁺ 457.2, RT 2.83 minutes, 93% purity *(Method 10).*

### EXAMPLE 165

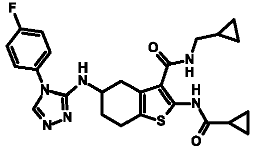

### 2-(Cyclopropanecarbonylamino)-N-(cyclopropylmethyl)-5-[[4-(4-fluorophenyl)-1,2,4-triazol-3-yl]amino]-4,5,6,7-tetrahydrobenzothiophene-3-carboxamide

*Intermediate 185* (167 mg, 0.34 mmol) and formic hydrazide [624-84-0] (62 mg, 1.03 mmol) were dissolved in DMF (3 mL). Mercury dichloride [7487-94-7] (280 mg, 1.03 mmol) was added to the reaction mixture which was stirred for 5 minutes. Triethylamine (0.12 ml, 0.87 mmol) was added to this mixture which was heated at 90 °C for 1 h. The reaction mixture was diluted with DCM (20 mL), filtered, and the filtrate was concentrated *in vacuo* to give the crude residue which was extracted with EtOAc (3 x 20 mL) and washed with water (20 mL). The organic phases were dried (MgSO₄), filtered and the filtrate was concentrated *in vacuo* to afford the product which was purified by preparative HPLC (basic) to afford the *title compound* (0.018 g, 11%) as a white solid. δ_{H} (500 MHz, Methylene Chloride-*d*₂) 12.06 (s, 1H), 7.83 (s, 1H), 7.35 - 7.30 (m, 2H), 7.27 - 7.18 (m, 2H), 5.98 (s, 1H), 4.29 - 4.24 (m, 1H), 3.96 (d, *J* 7.4 Hz, 1H), 3.29 (dd, *J* 14.7, 4.7 Hz, 1H), 3.24 (dd, *J* 7.0, 5.5 Hz, 2H), 2.88 - 2.2.70 (m, 3H), 2.19 - 2.13 (m, 1H), 2.04 - 1.93 (m, 1H), 1.67 - 1.62 (m, 1H), 1.09 - 0.98 (m, 3H), 0.93 - 0.85 (m, 2H), 0.57 - 0.47 (m, 2H), 0.28 - 0.20 (m, 2H). LCMS [M+H]⁺ 495, RT 2.39 minutes, 100% purity *(Method 10).*

### EXAMPLE 166

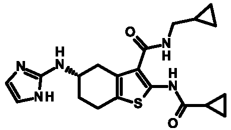

### (5S)-2-(Cyclopropanecarbonylamino)-N-(cyclopropylmethyl)-5-(1H-imidazol-2-ylamino)-4,5,6,7-tetrahydrobenzothiophene-3-carboxamide

*Intermediate 186* (194 mg, 0.49 mmol) was stirred in DMF (4.5 mL) and 2,2-diethoxyethanamine [645-36-3] (0.22 mL, 1.48 mmol) was added followed by mercury dichloride [7487-94-7] (403 mg, 1.48 mmol). The reaction mixture was stirred at r.t. for 5 minutes and triethylamine (0.21 mL, 1.48 mmol) was added. The reaction was heated at 90 °C whilst stirring for 2 h. 4-Methylbenzene-1-sulfonic acid hydrate (PTSA hydrate) [6192-52-5] (564 mg, 2.97 mmol) was added and the reaction mixture was stirred at 90 °C for 20 h. The mixture was diluted with DCM (10 mL) and filtered through kieselguhr washing with DCM. The filtrate was concentrated *in vacuo* and the orange residue was dissolved in EtOAc (100 mL), washed with water (3 x 20 mL) and 5% aqueous lithium chloride (20 mL). The organic phases were dried (Na₂SO₄), filtered and concentrated *in vacuo* to give an orange oil. The aqueous layer was basified to pH 9 using 1M aqueous NaOH solution and was extracted with EtOAc (100 mL). The organic phases were washed with water (2 x 20 mL), 5% aqueous lithium chloride solution (20 mL), dried (Na₂SO₄), filtered and combined with the initial extracted material. The combined organic phases were concentrated *in vacuo* to afford the crude product which was purified by flash column chromatography on silica (gradient elution with 0 - 10% MeOH in DCM) to afford a brown solid (50 mg) which was further purified by preparative HPLC (basic) to afford the *title compound* (14.4 mg, 6%) as a white solid. δ_{H} (500 MHz, DMSO-*d*₆) 11.09 (s, 1H), 10.18 (s, 1H), 7.66 (s, 1H), 6.47 (s, 2H), 5.55 (d, *J* 7.7 Hz, 1H), 3.79 - 3.70 (m, 1H), 3.11 (hept, *J* 6.7, 6.0 Hz, 2H), 3.03 (dd, *J* 15.9, 4.4 Hz, 1H), 2.76 - 2.66 (m, 2H), 2.59 - 2.53 (m, 1H), 2.12 - 2.00 (m, 1H), 1.90 (p, J 6.3 Hz, 1H), 1.82 - 1.62 (m, 1H), 1.09 - 0.92 (m, 1H), 0.89 - 0.76 (m, 4H), 0.44 - 0.32 (m, 2H), 0.20 (q, J 5.2 Hz, 2H).

### EXAMPLE 168

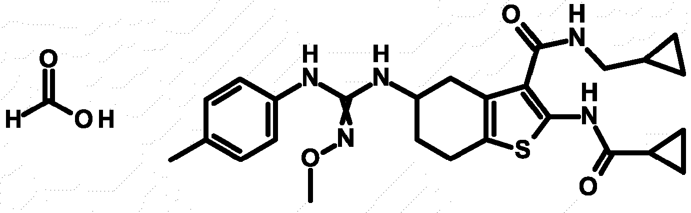

### 2-(Cyclopropanecarbonylamino)-N-(cyclopropylmethyl)-5-[[N'-methoxy-N-(p-tolyl)carbamimidoyl]amino]-4,5,6,7-tetrahydrobenzothiophene-3-carboxamide

A stirred solution of *intermediate 190* (30 mg, 0.15 mmol), *intermediate 122* (57 mg, 0.15 mmol) and DIPEA (0.053 ml, 0.30 mmol) in DCM (4 mL) was cooled to 0 °C. EDCI [1892-57-5] (35 mg, 0.18 mmol) was added and the solution was stirred at r.t. overnight. The reaction mixture was washed with water (20 mL), brine and dried (Na₂SO₄). The mixture was filtered and concentrated *in vacuo* and the crude product was purified by preparative HPLC (acidic) to afford the *title compound* (40 mg, 53%) as a white solid. δ_{H} (500 MHz, CD₃OD) 8.37 (s, 1H), 7.13 (d, J 8.3 Hz, 2H), 7.08 (d, J 8.3 Hz, 2H), 3.87 - 3.77 (m, 1H), 3.70 (s, 3H), 3.28 - 3.18 (m, 2H), 3.05 (dd, J 15.5, 5.0 Hz, 1H), 2.83 - 2.73 (m, 2H), 2.71- 2.64 (m, 1H), 2.30 (s, 3H), 2.15 - 2.06 (m, 1H), 1.93 - 1.84 (m, 1H), 1.83 - 1.75 (m, 1H), 1.14 - 1.04 (m, 1H), 1.00 - 0.90 (m, 4H), 0.57 - 0.50 (m, 2H), 0.31 - 0(m, 2H). 5.1% w/w HCOOH present (Formate salt with stoichiometry of 0.6). LCMS [M+H]⁺ 496.2, RT 2.36 minutes, 100% purity *(Method* 10).

### EXAMPLE 169

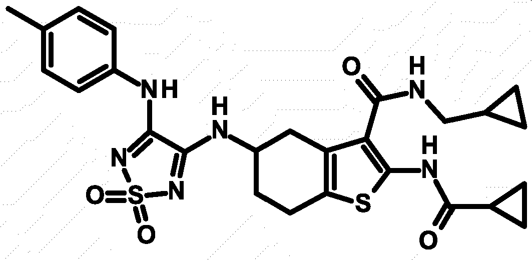

### 2-(Cyclopropanecarbonylamino)-N-(cyclopropylmethyl)-5-[[4-(4-methylanilino)-1,1-dioxo-1,2,5-thiadiazol-3-yl]amino]-4,5,6,7-tetrahydrobenzothiophene-3-carboxamide

*Intermediate 191* (84 mg, 0.31 mmol) was dissolved in EtOH (4 mL). *Intermediate 122* (116 mg, 0.31 mmol) was added and the reaction mixture was stirred at 80 °C in the microwave for 30 minutes. The reaction mixture was concentrated *in vacuo* and the resulting crude residue was purified by preparative HPLC (acidic) to afford the *title compound* (70 mg, 40%) as a white solid. δ_{H} (500 MHz, CD₃OD) 7.60 (d, J 8.4 Hz, 2H), 7.23 (d, J 8.3 Hz, 2H), 4.37 - 4.28 (m, 1H), 3.34 - 3.33 (m, 1H), 3.28 - 3.24 (m, 1H), 3.24 - 3.18 (m, 1H), 2.92 - 2.79 (m, 3H), 2.34 (s, 3H), 2.24 -2.16 (m, 2H), 1.86 - 1.77 (m, 1H), 1.15 - 1.06 (m, 1H), 1.01 - 0.92 (m, 4H), 0.58 - 0.50 (m, 2H), 0.31 - 0.24 (m, 2H). LCMS [M+H]⁺ 555.2, RT 3.38 minutes, 100% purity *(Method 10).*

### EXAMPLE 170

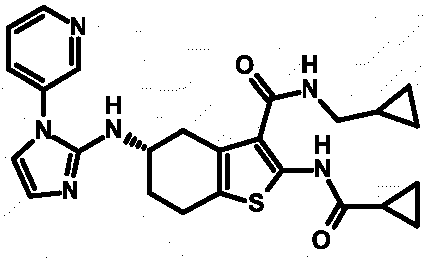

### (5S)-2-(Cyclopropanecarbonylamino)-N-(cyclopropylmethyl)-5-[[1-(3-pyridyl)imidazol-2-yl]amino]-4,5,6,7-tetrahydrobenzothiophene-3-carboxamide

To a stirred solution of *intermediate 141* (222 mg, 0.47 mmol) and 2,2-diethoxyethanamine [645-36-3] (0.21 ml, 1.42 mmol) in DMF (4.5 mL) was added mercury dichloride [7487-94-7] (385 mg, 1.42 mmol). The reaction mixture was stirred at r.t. for 5 minutes and triethylamine (0.20 ml, 1.42 mmol) was added and the reaction mixture was heated at 90 °C for 1 h. 4-Methylbenzenesulfonic acid hydrate (1:1) [6192-52-5] (539 mg, 2.84 mmol) was added and heating continued at 90 °C for a further 2.5 h. The reaction mixture was diluted with DCM (40 mL), filtered over a pad of Celite, and the filtrate concentrated *in vacuo.* The crude residue was diluted with water (20 mL) and extracted with EtOAc (3 x 20 mL). The organic phases were combined, washed with water (10 mL) and brine (10 mL), dried (Na₂SO₄), filtered and concentrated *in vacuo.* The residue was purified by flash column chromatography on silica (gradient elution with 1% to 10% MeOH in DCM) to afford the *title compound* (19 mg, 8.3%) as a beige solid. δ_{H} (500 MHz, DMSO-*d*₆) 11.04 (s, 1H), 8.70 (d, *J* 2.2 Hz, 1H), 8.55 (dd, *J* 4.7, 1.4 Hz, 1H), 7.89 (ddd, *J* 8.2, 2.6, 1.5 Hz, 1H), 7.66 (t, *J* 5.5 Hz, 1H), 7.53 (ddd, *J* 8.2, 4.8, 0.6 Hz, 1H), 6.98 (d, *J* 1.6 Hz, 1H), 6.67 (d, *J* 1.6 Hz, 1H), 5.68 (d, *J* 6.4 Hz, 1H), 3.91 - 3.81 (m, 1H), 3.16 - 3.03 (m, 3H), 2.74 - 2.66 (m, 2H), 2.57 (dd, *J* 16.0, 9.3 Hz, 1H), 2.16 - 2.09 (m, 1H), 1.93 - 1.86 (m, 1H), 1.81 - 1.69 (m, 1H), 1.01 (s, 1H), 0.86 - 0.80 (m, 4H), 0.42 - 0.37 (m, 2H), 0.22 - 0.17 (m, 2H). LCMS [M+H]⁺ 477, RT 1.83 minutes, 97% purity *(Method 10).*

### EXAMPLE 171

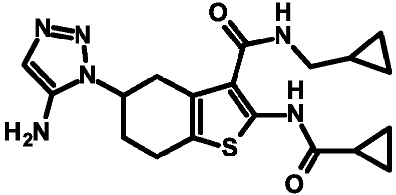

### 5-(5-Aminotriazol-1-yl)-2-(cyclopropanecarbonylamino)-N-(cyclopropylmethyl)-4,5,6,7-tetrahydrobenzothiophene-3-carboxamide

To a solution of *example 140* (81 mg, 0.16 mmol) in DCM (3 mL) at 0 °C was added TFA [76-05-1] (3 mL, 40 mmol). The solution was stirred at 0 °C for 5 minutes and then r.t. for 2 h. The solvent was removed and the excess TFA azetroped with isohexane. The residue was purified by flash column chromatography on silica (gradient elution with 0 - 100% MeOH/7M aqueous NH₃ in MeOH) to afford the *title compound* (40 mg, 61%) as a white solid. δ_{H} (300 MHz, CD₃OD) 6.97 (s, 1H), 4.57 (s, 1H), 3.33 - 3.09 (m, 2H), 2.96 (s, 3H), 2.43 (td, *J* 10.7, 10.1, 6.8 Hz, 1H), 2.34 (s, 1H), 1.81 (td, *J* 7.7, 3.8 Hz, 1H), 1.31 (s, 2H), 1.13 - 0.88 (m, 4H), 0.55 - 0.43 (m, 2H), 0.25 (dt, *J* 6.2, 4.5 Hz, 2H).

### EXAMPLE 172

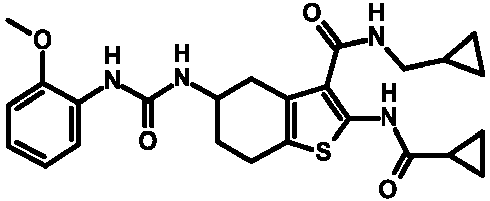

### 2-(Cyclopropanecarbonylamino)-N-(cyclopropylmethyl)-5-[(2-methoxyphenyl)carbamoylaminol-4,5,6,7-tetrahydrobenzothiophene-3-carboxamide

*Intermediate 122* (50 mg, 0.14 mmol) was dissolved in DCM (5 mL) and DIPEA (26 mg, 0.20 mmol). 2-Methoxyphenyl isocyanate [700-87-8] (31 mg, 0.20 mmol) was added and the reaction mixture was stirred at r.t. for ^{~}16 h. The precipitate was filtered off, washing with DCM (2 x), to afford the *title compound* (17 mg, 26%) as a white solid. δ_{H} (400 MHz, DMSO-*d*₆) 11.17 (s, 1H), 8.10 (dd, *J* 7.5, 2.2 Hz, 1H), 7.94 (s, 1H), 7.68 (t, *J* 5.7 Hz, 1H), 7.01 (d, *J* 7.5 Hz, 1H), 6.95 (dd, *J* 7.5, 2.0 Hz, 1H), 6.88 - 6.75 (m, 2H), 4.03 - 3.88 (m, 1H), 3.82 (s, 3H), 3.14 (qt, *J* 13.3, 6.2 Hz, 2H), 3.00 (dd, *J* 16.1, 5.1 Hz, 1H), 2.78 - 2.69 (m, 2H), 2.58 - 2.52 (m, 1H), 2.01 - 1.85 (m, 2H), 1.85 - 1.68 (m, 1H), 1.15 - 0.97 (m, 1H), 0.95 - 0.82 (m, 4H), 0.47 - 0.33 (m, 2H), 0.28 - 0.14 (m, 2H). LCMS (ES+) [M+H]⁺ 483.8, RT 2.429 minutes, 99.2% purity *(Method 2).* LCMS (ES+) [M+H]⁺ 483.8, RT 2.408 minutes, 99.2% purity *(Method 3).*

### EXAMPLE 173

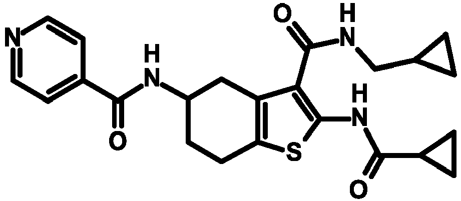

### N-[2-(Cyclopropanecarbonylamino)-3-(cyclopropylmethylcarbamoyl)-4,5,6,7-tetrahydrobenzothiophen-5-yl]pyridine-4-carboxamide

To *intermediate 122* (18 mg, 0.050 mmol) in DCM (0.8 mL) was added DIPEA (26 µL, 0.075 mmol). DIPEA (26 µL, 0.075 mmol), isonicotinic acid [55-22-1] (8 mg, 0.065 mmol) and COMU [1075198-30-9] (27 mg, 0.060 mmol) were added and the reaction mixture was stirred at 30°C. After 1 h, water (1 mL) was added and the reaction mixture was passed through a phase separator. To the filtrate was added DCM (1 mL) and the reaction mixture was concentrated to give the crude residue which was purified by reverse phase HPLC (basic) to afford the *title compound* (6.8 mg, 32%). LCMS [M+H]⁺ 439.30, RT 2.20 minutes, 96.63% purity (*Method 9*). LCMS [M+H]⁺ 439.26, RT 1.56 minutes, 100% purity (*Method 8*).

### EXAMPLE 174

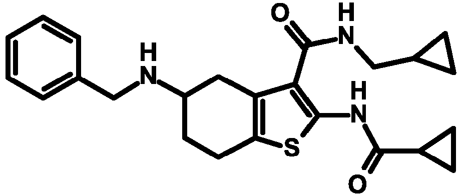

### 5-(Benzylamino)-2-(cyclopropanecarbonylamino)-N-(cyclopropylmethyl)-4,5,6,7-tetrahydrobenzothiophene-3-carboxamide

To *intermediate 122* (37 mg, 0.10 mmol) in THF (1 mL) and EtOH (0.5 mL) was added MP-carbonate (75 mg, 0.25 mmol, 3.36 mmol/g). The mixture was shaken for 1 h, filtered and rinsed with EtOH (2 x 200µL). To the filtrate was added benzaldehyde [100-52-7] (13 mg, 0.12 mmol), MP-cyanoborohydride (100 mg, 0.25 mmol, 2.49 mmol/g) and acetic acid (0.1 mL, 2 mmol). The reaction mixture was shaken at r.t. for 3 h, filtered and rinsed with EtOH. The filtrate was concentrated in *vacuo* to give the crude residue which was purified by preparative HPLC (basic) to afford the *title compound* (9.6 mg, 23%). LCMS [M+H]⁺ 423.2, RT 3.73 minutes, 95.9% purity (*Method 8*).

### EXAMPLE 175

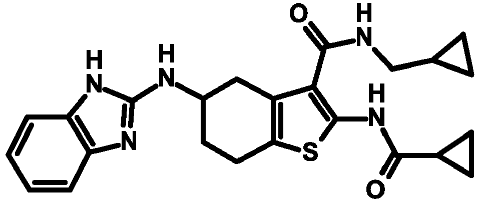

### 5-(1H-Benzimidazol-2-ylamino)-2-(cyclopropanecarbonylamino)-N-(cyclopropylmethyl)-4,5,6,7-tetrahydrobenzothiophene-3-carboxamide

A solution of *intermediate 62* (60 mg, 0.18 mmol) and 1H-1,3-benzodiazol-2-amine (25 mg, 0.19 mmol) in THF (1 mL) was treated with titanium(IV) isopropoxide [546-68-9] (95 µL, 0.32 mmol). The reaction mixture was stirred at r.t. for 3.5 h prior to addition of sodium triacetoxyborohydride [56553-60-7] (80 mg, 0.38 mmol) and the reaction mixture was then stirred at r.t. for 16 h. The reaction mixture was quenched with sodium hydrogen carbonate solution (2 mL) and diluted with EtOAc (5 mL). A solid precipitate formed which was filtered off and extracted with EtOAc (2 x 5 mL). The combined organic phases were dried (MgSO₄), filtered and concentrated *in vacuo* and the residue purified by preparative HPLC (acidic) followed by preparative HPLC (basic) to afford the *title compound* (24 mg, 29%) as a white solid. δ_{H} (500 MHz, DMSO-*d*₆) 11.10 (s, 1H), 10.64 (br s, 1H), 7.70 (t, *J* 5.6 Hz, 1H), 7.16 - 7.09 (m, 2H), 6.86 (dd, *J* 5.6, 3.2 Hz, 2H), 6.70 (br d, *J* 5.9 Hz, 1H), 4.06 - 3.95 (m, 1H), 3.18 - 3.08 (m, 3H), 2.82 - 2.73 (m, 2H), 2.71 - 2.61 (m, 1H), 2.17 - 2.05 (m, 1H), 1.99 - 1.79 (m, 2H), 1.06 - 0.94 (m, 1H), 0.91 - 0.79 (m, 4H), 0.41 - 0.30 (m, 2H), 0.24 - 0.13 (m, 2H). LCMS [M+H]⁺ 450.2, RT 2.04 minutes, 99% purity *(Method 10).*

### EXAMPLE 176

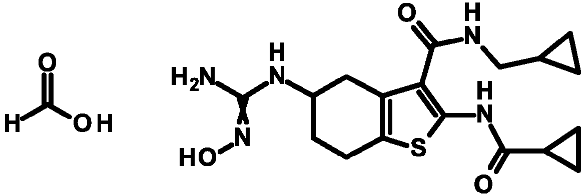

### 2-(Cyclopropanecarbonylamino)-N-(cyclopropylmethyl)-5-[[N'-hydroxycarbamimidoyllaminol-4,5,6,7-tetrahydrobenzothiophene-3-carboxamide

To a stirred solution of *intermediate 134* (140 mg, 0.39 mmoL) in EtOH (5 mL) was added hydroxylamine hydrochloride [5470-11-1] (29 mg, 0.41 mmoL) followed by potassium carbonate (108 mg, 0.78 mmoL) and the reaction mixture was stirred at r.t. under nitrogen. After stirring for 18 h further hydroxylamine hydrochloride [5470-11-1] (29 mg, 0.41 mmol) and potassium carbonate (108 mg, 0.78 mmoL) were added and the reaction mixture was stirred at r.t. for 1 h. The reaction mixture was filtered through a pad of celite, washed with EtOH and concentrated *in vacuo* to give the crude residue (210 mg) which was purified by flash column chromatography on silica (gradient elution with 0 to 100% of EtOAc in heptane, followed by 0 to 10% MeOH in EtOAc, followed by 10% NH₃ (7N in MeOH) in EtOAc). The product was further purified by preparative HPLC (low pH x 2) to afford the *title compound* (2.2 mg, 1.4%) as a white formate salt. δ_{H} (500 MHz, CD₃OD) 8.55 (s, 1H), 3.90 - 3.80 (m, 1H), 3.30 - 3.17 (m, 2H), 3.10 (dd, *J* 15.7, 5.1 Hz, 1H), 2.88 - 2.81 (m, 2H), 2.74 - 2.64 (m, 1H), 2.17 - 2.06 (m, 1H), 2.01 - 1.87 (m, 1H), 1.86 - 1.75 (m, 1H), 1.14 - 1.04 (m, 1H), 1.03 - 0.86 (m, 4H), 0.60 - 0.47 (m, 2H), 0.32 - 0.23 (m, 2H). LCMS [M+H]⁺ 392, RT 2.76 minutes, 99% purity *(Method 5).*

### EXAMPLE 177

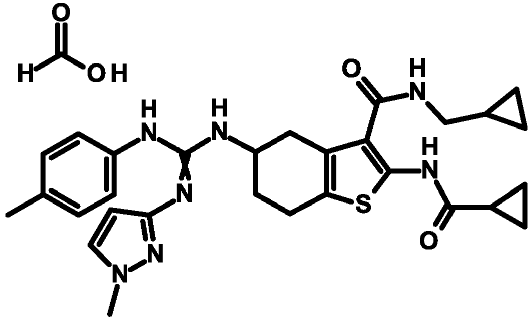

### 2-(Cyclopropanecarbonylamino)-N-(cyclopropylmethyl)-5-[[N'-(1-methylpyrazol-3-yl)-N-(p-tolyl)carbamimidoyl]aminol-4,5,6,7-tetrahydrobenzothiophene-3-carboxamide;formic acid

A stirred solution of *intermediate 192* (36 mg, 0.14 mmol), *intermediate 122* (60 mg, 0.16 mmol), and DIPEA (0.057 ml, 0.32 mmol) in DCM (4 mL) was cooled to 0 °C. EDCI (37 mg, 0.19 mmol) was added and the solution was stirred at r.t. overnight. The reaction mixture was washed with water (20 mL), followed by brine and dried (Na₂SO₄). The mixture was filtered and concentrated *in vacuo* to give the crude residue which was purified by preparative HPLC (acidic) to afford the *title compound* (55 mg, 57%) as a white solid (formate salt). δ_{H} (500 MHz, CD₃OD) 8.48 (s, 1H), 7.49 (d, *J* 2.2 Hz, 1H), 7.33 (d, J8.1 Hz, 2H), 7.23 (d, *J* 8.3 Hz, 2H), 5.95 (s, 1H), 4.45 - 4.30 (m, 1H), 3.70 (s,3H), 3.29 - 3.18 (m, 3H), 3.00 - 2.84 (m, 3H),2.40 (s, 3H), 2.25 - 2.13 (m, 2H), 1.85 - 1.78 (m, 1H), 1.14 - 1.04 (m, 1H), 1.01 -0.92 (m, 4H), 0.55 - 0.48 (m, 2H), 0.32 - 0.24 (m, 2H). LCMS [M+H]⁺ 546.3, RT 2.56 minutes, 99% purity *(Method 10).*

### EXAMPLE 178

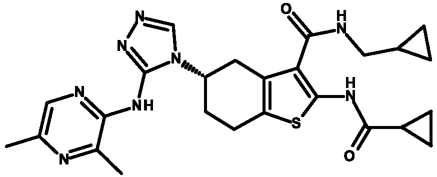

### (55)-2-(Cyclopropanecarbonylamino)-N-(cyclopropylmethyl)-5-[3-[(3,5-dimethylpyrazin-2-yl)aminol-1,2,4-triazol-4-yl]-4,5,6,7-tetrahydrobenzothiophene-3-carboxamide

To a stirred solution of *intermediate 259* (290 mg, 0.582 mmol) in DMF (10 mL) under nitrogen was added formic acid hydrazide [624-84-0] (116 mg, 1.75 mmol) and mercuric chloride [7487-94-7] (237 mg, 0.872 mmol). The reaction mixture was stirred at r.t. for 15 min before the addition of triethylamine (0.24 mL, 1.75 mmol) and the mixture was stirred at 85 °C for 16 h. The mixture was cooled to r.t. and diluted with MeCN (10 mL), filtered through a pad of celite and washed with MeCN (15 mL). The filtrate was concentrated *in vacuo* to yield the crude product as a yellow gum which was dissolved in DCM (50 mL), washed with water (30 mL) and the layers separated. The Aq layer was extracted with DCM (2 x 15 mL). The combined organic layers were washed with brine (30 ml) and passed through a phase separation cartridge and concentrated *in vacuo* to give a yellow gum. This was purified by flash column chromatography on silica (gradient elution with 2% to 20% MeOH in DCM) to afford impure *title compound* as a white solid. This was dissolved in MeOH (10 mL) and QuadraSil^{®} MTU (130 mg) was added and the suspension was stirred at 40 °C for 1 h before being filtered and washed with MeOH (2 x 10 mL). The filtrate was concentrated *in vacuo* to give a gum. This was further purified by flash column chromatography on silica (gradient elution with 4% to 20% MeOH in EtOAc) product containing fractions were freeze dried to afford the *title compound* (169 mg, 0.334 mmol, 57%) as a white solid. LCMS (ES+) [M+H]⁺ 507.0, RT 1.73 minutes, purity 99% *(Method 3).* LCMS (ES+) [M+H]⁺ 507.0, RT 1.71 minutes, purity 99% *(Method 2).* 1H NMR (300 MHz, DMSO-d6) (contained 2 tautomers in an approximate ratio of 0.6:0.4) δ 12.88 (s, 0.4H), 11.22 (s, 0.4H), 11.10 (s, 0.6H), 8.73 (s, 0.6H), 8.64 (s, 0.6H), 8.29 (s, 0.4H), 7.85 - 7.66 (m, 2H), 4.70 - 4.46 (m, 0.4H), 4.19 - 3.94 (m, 0.6H), 3.29 - 2.57 (m, 6H), 2.44 (s, 1.8H), 2.40 (s, 1.2H), 2.36 - 2.04 (m, 5H), 2.01-1.83 (m, 1H), 1.10 - 0.93 (m, 1H), 0.91 - 0.77 (m, 4H), 0.47 - 0.29 (m, 2H), 0.26 - 0.13 (m, 2H).

### EXAMPLE 179

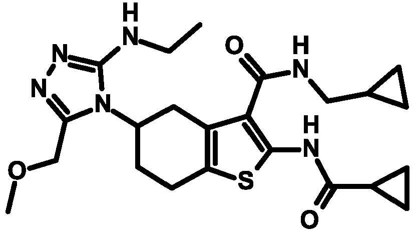

### 2-(Cyclopropanecarbonylamino)-N-(cyclopropylmethyl)-5-[3-(ethylamino)-5-(methoxymethyl)-1,2,4-triazol-4-yl]-4,5,6,7-tetrahydrobenzothiophene-3-carboxamide

To a solution of *intermediate 198* (46 mg, 0.082 mmol) in 88% formic acid [64-18-6] (6 mL) was added 10% Pd on carbon (50 % wet type) (30 mg) under nitrogen. The reaction mixture was warmed to 80 °C and stirred for 60 minutes. The reaction was filtered through Celite and washed with formic acid [64-18-6] (2 mL). The filtrate was concentrated *in vacuo* to give the crude product as a colourless oil which was purified by preparative HPLC (basic) to afford the *title compound* (21 mg, 51%) which was freeze dried from 1:1 MeCN/water overnight. δ_{H} (500 MHz, Chloroform-*d*) 12.07 (s, 1H), 5.88 (t, *J* 5.0 Hz, 1H), 4.56 (d, *J* 13.0 Hz, 1H), 4.52 (d, *J* 13.0 Hz, 1H), 4.40 - 4.33 (m, 1H), 4.01 (s, 1H), 3.51 - 3.42 (m, 2H), 3.35 (s, 3H), 3.32 - 3.18 (m, 3H), 3.01 (dd, *J* 14.6, 5.5 Hz, 1H), 2.94 - 2.83 (m, 2H), 2.39 (tdd, *J* 12.3, 9.8, 7.2 Hz, 1H), 2.24 - 2.18 (m, 1H), 1.66 (tt, *J* 8.0, 4.6 Hz, 1H), 1.27 (t, J 7.2 Hz, 3H), 1.10 (p, *J* 3.7, 3.3 Hz, 2H), 1.06 - 0.99 (m, 1H), 0.95 - 0.87 (m, 2H), 0.57 - 0.46 (m, 2H), 0.23 (q, *J* 4.7 Hz, 2H). LCMS (ES+) [M+H]⁺ 473, RT 2.05 minutes, 97% purity *(Method 10).*

### EXAMPLE 180

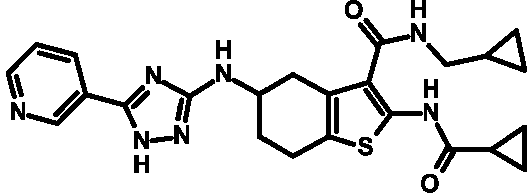

### 2-(Cyclopropanecarbonylamino)-N-(cyclopropylmethyl)-5-[[5-(3-pyridyl)-1H-1,2,4-triazol-3-yl]amino]-4,5,6,7-tetrahydrobenzothiophene-3-carboxamide

To *intermediate 200* (120 mg, 0.29 mmol) in DCM (2.5 mL) and 2-propanol (2.5 mL) was added nicotinic acid hydrazide [553-53-7] (133 mg, 0.94 mmol), triethylamine (0.06 mL, 0.4 mmol) and silver nitrate [7761-88-8] (59 mg, 0.35 mmol). The reaction mixture was heated in a microwave at 130 °C for 9 h before filtering through a phase separator cartridge. The aqueous phase was washed with DCM/MeOH, concentrated *in vacuo* to give the crude residue as a yellow oil which was purified by flash column chromatography on silica (gradient elution with 0-20% MeOH/DCM) to give the product which was purified by preparative HPLC to afford the *title compound* (11 mg, 8%) as a white solid. δ_{H} (300 MHz, DMSO-*d*₆) 12.41 (br. s, 1H), 11.05 (br. s, 1H), 9.06 (dd, *J* 2.2, 0.9 Hz, 1H), 8.54 (dd, J 4.8, 1.7 Hz, 1H), 8.19 (dt, *J* 7.9, 1.9 Hz, 1H), 7.77 (br. s, 1H), 7.50 - 7.37 (m, 1H), 6.88 (br. s, 1H), 3.89 - 3.68 (m, 1H), 3.18 - 3.02 (m, 3H), 2.85 - 2.69 (m, 2H), 2.61 (dd, *J* 16.1, 8.7 Hz, 1H), 2.14 - 2.01 (m, 1H), 1.96 - 1.72 (m, 2H), 1.07 - 0.90 (m, 1H), 0.89 - 0.78 (m, 4H), 0.42 - 0.28 (m, 2H), 0.22 - 0.13 (m, 2H).

### EXAMPLE 181

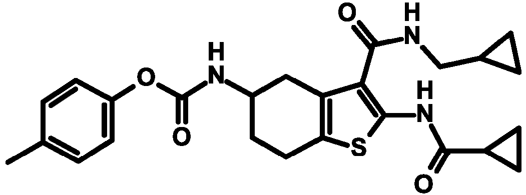

### p-Tolyl N-[2-(cyclopropanecarbonylamino)-3-(cyclopropylmethylcarbamoyl)-4,5,6,7-tetrahydrobenzothiophen-5-yl]carbamate

To a solution of the reaction mixture of *intermediate 201* in DCM (5 mL) was added *p*-cresol [106-44-5] (38 mg, 0.35 mmol) and the mixture was stirred at 100 °C for 60 minutes in the microwave. The reaction mixture was purified directly by flash column chromatography on silica (gradient elution with 20-100% EtOAc/hexane) and the product was freeze-dried from MeCN/water to afford the *title compound* (35 mg, 63%) as a white solid. δ_{H} (400 MHz, DMSO-*d*₆) 11.06 (s, 1H), 7.87 (d, *J* 7.4 Hz, 1H), 7.74 (t, *J* 5.6 Hz, 1H), 7.23 - 7.12 (m, 2H), 7.03 - 6.95 (m, 2H), 3.74 (br s, 1H), 3.24 - 3.07 (m, 2H), 2.97 (dd, *J* 15.9, 5.2 Hz, 1H), 2.86 - 2.55 (m, 3H), 2.29 (s, 3H), 2.08 - 1.96 (m, 1H), 1.95 - 1.87 (m, 1H), 1.86 - 1.70 (m, 1H), 1.09 - 0.98 (m, 1H), 0.93 - 0.77 (m, 4H), 0.51 - 0.36 (m, 2H), 0.30 - 0.17 (m, 2H).

### EXAMPLE 182

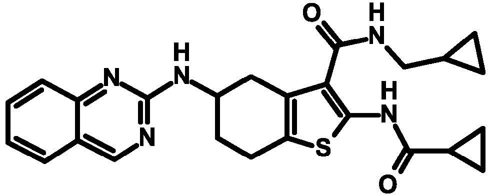

### 2-(Cyclopropanecarbonylamino)-N-(cyclopropylmethyl)-5-(quinazolin-2-ylamino)-4,5,6,7-tetrahydrobenzothiophene-3-carboxamide

*Intermediate 122* (50 mg, 0.13 mmol) was dissolved in butanol (2 mL). 2-Chloroquinazoline [6141-13-5] (33 mg, 0.20 mmol) and DIPEA (0.035 ml, 0.2 mmol) were added and the reaction mixture was stirred at 100 °C in the microwave for 2 h. The reaction mixture was concentrated *in vacuo* and the crude residue was purified by preparative HPLC (acidic) to afford the *title compound* (18 mg, 28%) as an off white solid. δ_{H} (500 MHz, CD₃OD) 9.05 (s, 1H), 7.81 - 7.74 (m, 1H), 7.74 - 7.67 (m, 1H), 7.55 (d, *J* 8.4 Hz, 1H), 7.30 - 7.23 (m, 1H), 4.46 - 4.36 (m, 1H), 3.29 - 3.22 (m, 2H), 3.21 - 3.14 (m, 1H), 2.95 - 2.84(m, 2H), 2.78 - 2.70 (m, 1H), 2.30 - 2.20 (m, 1H), 2.06 - 1.95 (m, 1H), 1.84 - 1.75 (m, 1H), 1.08 - 0.91 (m, 5H), 0.46 - 0.34 (m, 2H), 0.26 - 0.15 (m, 2H). LCMS (ES+) [M+H]⁺ 462.3, RT 3.01 minutes, 96% purity *(Method 10).*

### EXAMPLE 183

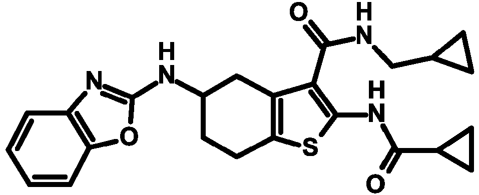

### 5-(1,3-Benzoxazol-2-ylamino)-2-(cyclopropanecarbonylamino)-N-(cyclopropylmethyl)-4,5,6,7-tetrahydrobenzothiophene-3-carboxamide

*Intermediate 122* (90%, 50 mg, 0.12 mmol) was suspended in MeCN (1 mL) and DIPEA (45 µL, 0.26 mmol) and 2-chloro-1,3-benzoxazole [615-18-9] (16 µL, 0.14 mmol) were added. The reaction mixture was heated to 100 °C in the microwave for 30 minutes followed by heating at 120 °C in the microwave for 30 minutes. The reaction mixture was concentrated *in vacuo* to give the crude residue which was purified by preparative HPLC to afford the *title compound* (26 mg, 48%) as a white solid. δ_{H} (500 MHz, DMSO-*d*₆) 11.07 (s, 1H), 8.06 (d, *J* 6.8 Hz, 1H), 7.78 - 7.68 (m, 1H), 7.33 (d, *J* 7.8 Hz, 1H), 7.24 (d, *J* 7.2 Hz, 1H), 7.11 (td, *J* 7.7, 1.0 Hz, 1H), 6.97 (td, *J* 7.8, 1.2 Hz, 1H), 3.97 (br. s, 1H), 3.20 - 3.02 (m, 3H), 2.86 - 2.66 (m, 3H), 2.19 - 2.10 (m, 1H), 1.97 - 1.83 (m, 2H), 1.06 - 0.93 (m, 1H), 0.84 (m, 4H), 0.42 - 0.32 (m, 2H), 0.23 - 0.13 (m, 2H). LCMS (ES+) [M+H]⁺ 451.3, RT 3.45 minutes, 100% purity *(Method 10).*

### EXAMPLE 184

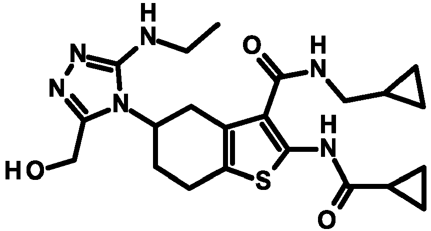

### 2-(Cyclopropanecarbonylamino)-N-(cyclopropylmethyl)-5-[3-(ethylamino)-5-(hydroxymethyl)-1,2,4-triazol-4-yl]-4,5,6,7-tetrahydrobenzothiophene-3-carboxamide

To a solution of *intermediate 202* (32 mg, 0.050 mmol) in 88% formic acid [64-18-6] (4 mL) was added 10% Pd on carbon (50 % wet type, 53 mg, 0.050 mmol). The reaction mixture was heated to 80 °C under nitrogen for 60 minutes. The reaction mixture was filtered through a glass fibre filter paper and washed with formic acid [64-18-6] (1 mL). The filtrate was concentrated *in vacuo* and the crude residue dried under vacuum to give a colourless oil. The crude product was dissolved in THF (3 mL) and 1M lithium hydroxide solution (0.1 mL, ^{~} 2 equiv) was added. The reaction mixture was stirred at r.t. for 60 minutes. The pH was adjusted to pH7 by dropwise addition of 1M aqueous hydrochloric acid solution and the mixture was then concentrated *in vacuo.* The residue was partitioned between EtOAc (10 mL) and saturated aqueous sodium hydrogen carbonate solution (3 mL). The organic phase was separated and washed with brine (3 mL), dried (Na₂SO₄), filtered and concentrated *in vacuo* to give the crude product which was purified by preparative HPLC to afford the *title compound* (13 mg, 55%) as a colourless solid. δ_{H} (500 MHz, Chloroform-*d*) 12.14 (s, 1H), 6.46 - 6.32 (m, 1H), 4.72 (d, *J* 13.9 Hz, 1H), 4.65 (d, *J* 13.9 Hz, 1H), 4.52 - 4.42 (m, 1H), 4.42 - 4.19 (m, 1H), 3.47 - 3.39 (m, 3H), 3.31 - 3.26 (m, 1H), 3.26 - 3.15 (m, 2H), 2.94 - 2.87 (m, 2H), 2.53 - 2.42 (m, 1H), 2.29 - 2.20 (m, 1H), 1.68 (tt, *J* 8.0, 4.6 Hz, 1H), 1.26 (t, *J* 7.2 Hz, 3H), 1.11 (dt, *J* 6.7, 3.3 Hz, 2H), 1.09 - 1.01 (m, 1H), 0.92 (dq, *J* 7.3, 4.0 Hz, 2H), 0.50 - 0.44 (m, 2H), 0.21 (q, J 4.7 Hz, 2H). LCMS (ES+) [M+H]⁺ 459, RT 1.77 minutes, 100% purity *(Method 10).*

### EXAMPLE 186

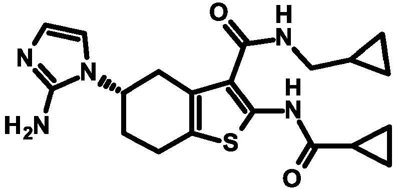

### (5S)-5-(2-Aminoimidazol-1-yl)-2-(cyclopropanecarbonylamino)-N-(cyclopropylmethyl)-4,5,6,7-tetrahydrobenzothiophene-3-carboxamide

Cyanamide [420-04-2] (134 mg, 3.2 mmol) was dissolved in 1,4-dioxane (10 mL) and 4M hydrogen chloride in 1,4-dioxane (800 µl) was added and the reaction mixture was stirred for 5 minutes. *Intermediate 205* (450 mg, 1.07 mmol) was added and the reaction was sealed and heated to 60°C for 18 h. The reaction mixture was diluted with MeOH and concentrated *in vacuo.* The crude residue was diluted with DCM (30 mL) and saturated aqueous sodium hydrogen carbonate solution (20 mL) was added. The mixture was extracted with 10% MeOH in DCM (3 x 20 mL), the organic phases were combined, dried (Na₂SO₄), filtered and concentrated *in vacuo* to give a yellow oil (574 mg). The crude product was purified by flash column chromatography on silica (gradient elution with 0 - 10% MeOH in DCM) to give impure product (184 mg) which was purified by preparative HPLC (basic) to give the desired product which was sonicated in Et₂O to afford the *title compound* (75 mg, 18%) as a white solid which was collected by filtration and dried *in vacuo.* δ_{H} (500 MHz, DMSO-*d*₆) 11.15 (s, 1H), 7.73 (s, 1H), 6.66 (d, *J* 1.5 Hz, 1H), 6.42 (d, *J* 1.5 Hz, 1H), 5.37 (s, 2H), 4.28 - 4.16 (m, 1H), 3.12 (qt, *J* 13.5, 6.2 Hz, 2H), 2.97 (dd, *J* 15.1, 4.0 Hz, 1H), 2.91 - 2.72 (m, 3H), 2.07 - 1.98 (m, 2H), 1.96 - 1.85 (m, 1H), 1.06 - 0.96 (m, 1H), 0.88 - 0.80 (m, 4H), 0.44 - 0.34 (m, 2H), 0.25 - 0.15 (m, 2H). LCMS (ES+) [M+H]⁺ 400.2, RT 1.83 minutes, 100% purity *(Method 10).*

### EXAMPLE 187

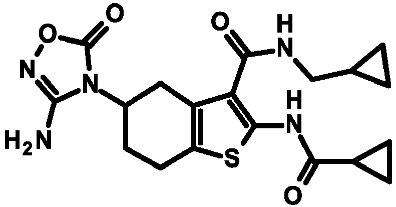

### 5-(3-Amino-5-oxo-1,2,4-oxadiazol-4-yl)-2-(cyclopropanecarbonylamino)-N-(cyclopropylmethyl)-4,5,6,7-tetrahydrobenzothiophene-3-carboxamide

To a stirred solution of *example 176* (30 mg, 0.077 mmoL) in THF (3 mL) was added 1,1'-carbonyldiimidazole [530-62-1] (15 mg, 0.092 mmoL) and the reaction mixture was stirred at r.t. under nitrogen for 20 h. The mixture was concentrated *in vacuo* and the residue was purified by preparative HPLC (acidic) to afford the *title compound* (4 mg, 12%) as a white solid. δ_{H} (250 MHz, Chloroform-d) 11.98 (s, 1H), 5.90 - 5.67 (m, 1H), 4.72 (s, 2H), 4.14 - 3.96 (m, 1H), 3.52 - 3.31 (m, 1H), 3.36 - 3.11 (m, 2H), 2.94 - 2.74 (m, 3H), 2.77 - 2.56 (m, 1H), 2.18 - 2.02 (m, 1H), 1.76 - 1.59 (m, 1H, part. obs. by water peak), 1.15 - 0.85 (m, 5H), 0.70 - 0.48 (m, 2H), 0.33 - 0.19 (m, 2H). LCMS (ES+) [M+H]⁺ 418, RT 2.74 minutes, 99% purity *(Method 10).*

### EXAMPLE 188

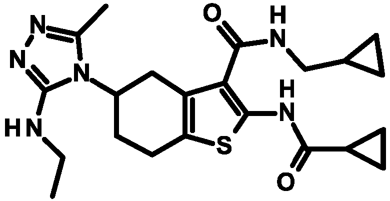

### 2-(Cyclopropanecarbonylamino)-N-(cyclopropylmethyl)-5-[3-(ethylamino)-5-methyl-1,2,4-triazol-4-yl]-4,5,6,7-tetrahydrobenzothiophene-3-carboxamide

To a solution of *intermediate 208* (70 mg, 0.17 mmol) in DCM (6 mL) was added acetic acid (0.7 mL of a 290 mg acetic acid / 10 mL DCM stock solution) and acetaldehyde [75-07-0] (0.7 mL of a 106 mg / 10 mL DCM stock solution). The reaction mixture was stirred for 10 minutes prior to the addition of sodium triacetoxyborohydride [56553-60-7] (72 mg, 0.34 mmol) in ~3 equal portions over 10 minute intervals. The reaction mixture was stirred at r.t. under nitrogen for 4 h. The reaction mixture was retreated with further acetaldehyde [75-07-0] (0.7 mL of a 106 mg / 10 mL DCM stock solution) and after 15 minutes stirring further sodium triacetoxyborohydride [56553-60-7] (72 mg, 0.34 mmol) was added portionwise over 15 minutes and the reaction mixture was stirred for 4 h. The reaction mixture was retreated with further acetaldehyde [75-07-0] (0.7 mL of a 106 mg / 10 mL DCM stock solution) and sodium triacetoxyborohydride [56553-60-7] (72 mg, 0.34 mmol) was added in three portions over 15 minutes and the reaction mixture was stirred at r.t. overnight. The reaction mixture was retreated with further acetaldehyde [75-07-0] (0.7 mL of a 106 mg / 10 mL DCM stock solution) and after 15 minutes stirring further sodium triacetoxyborohydride [56553-60-7] (72 mg, 0.34 mmol) was added in three portions over 15 minutes and then stirred at r.t. for 4 h before being retreated with further acetaldehyde [75-07-0] (0.7 mL of a 106 mg / 10 mL DCM stock solution) and after 15 minutes stirring further sodium triacetoxyborohydride [56553-60-7] (72 mg, 0.34 mmol) was added in three portions over 15 minutes. The reaction mixture was cooled to 4 °C overnight, concentrated *in vacuo,* combined with another reaction (10 mg, 0.024 mmol starting material) and dissolved in MeOH (10 mL). The pH was adjusted to pH13 with aqueous 1M sodium hydroxide solution and the mixture was stirred at r.t. for 15 minutes before concentrating *in vacuo* to remove the MeOH and adjusting to pH7 with 1M aqueous hydrochloric acid solution. To the solution was added saturated sodium bicarbonate solution (3 mL) and the aqueous phase was extracted with EtOAc (3 x 15 mL). The combined organic phases were washed with water (10 mL) and brine (10 mL), separated and dried (Na₂SO₄), filtered and concentrated *in vacuo* to give a colourless oil which was combined with a another reaction (10 mg, 0.024 mmol starting material). The crude product was purified by preparative HPLC (basic) to afford the *title compound* (13.4 mg, 18%). δ_{H} (500 MHz, DMSO-*d*₆) 11.19 (s, 1H), 7.67 (s, 1H), 5.69 (t, *J* 5.2 Hz, 1H), 4.29 - 4.15 (m, 1H), 3.14 (dtd, *J* 22.9, 13.7, 6.7 Hz, 5H), 2.93 - 2.75 (m, 3H), 2.43 - 2.34 (m, 1H), 2.32 (s, 3H), 2.00 (d, *J* 11.1 Hz, 1H), 1.89 (s, 1H), 1.13 (t, *J* 7.1 Hz, 3H), 1.05 - 0.94 (m, 1H), 0.91 - 0.74 (m, 4H), 0.43 - 0.34 (m, 2H), 0.23 - 0.15 (m, 2H). LCMS (ES+) [M+H]⁺ 443, RT 1.90 minutes, 99% purity *(Method 10).*

### EXAMPLES 189 and 190

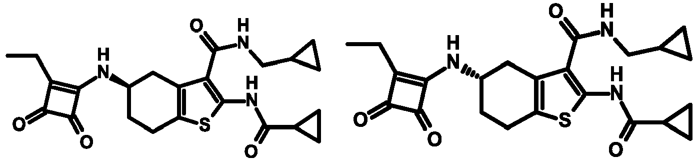

### Example 189

### (5R)-2-(Cyclopropanecarbonylamino)-N-(cyclopropylmethyl)-5-[(2-ethyl-3,4-dioxo-cyclobuten-1-yl)aminol-4,5,6,7-tetrahydrobenzothiophene-3-carboxamide

### Example 190

### (5S)-2-(Cyclopropanecarbonylamino)-N-(cyclopropylmethyl)-5-[(2-ethyl-3,4-dioxo-cyclobuten-1-yl)aminol-4,5,6,7-tetrahydrobenzothiophene-3-carboxamide

*Intermediate 196* (79 mg, 0.18 mmol) was purified by chiral HPLC *(Column - Chiralcel OD-H 25 cm at 15 mL*/*min, eluent - 35% IPA: 65% CO₂)* and the 2 enantiomers were isolated in high purity: first eluting enantiomer (*Example 189*) (30 mg, 38%), second eluting enantiomer (*Example 190*) (30 mg, 38%).

*Example 189:* δ_{H} (250 MHz, Choroform-*d*) 12.14 - 11.73 (m, 1H), 6.30 - 6.16 (m, 1H - Rotamer 1), 6.04 - 5.92 (m, 1H - Rotamer 2), 5.91 - 5.79 (m, 1H), 4.84 - 4.56 (m, 1H - Rotamer 2), 4.06 - 3.90 (m, 1H - Rotamer 1), 3.39 - 3.10 (m, 3H), 2.93 - 2.75 (m, 3H), 2.75 - 2.64 (m, 2H - Rotamer 1), 2.63 - 2.43 (m, 2H - Rotamer 2), 2.19 - 2.00 (m, 2H), 1.73 - 1.59 (m, 1H, part. obs. by water peak), 1.39 - 1.19 (m, 3H), 1.16 - 1.01 (m, 3H), 0.98 - 0.82 (m, 2H), 0.70 - 0.47 (m, 2H), 0.33 - 0.17 (m, 2H). LCMS [M+H]⁺ 442, RT 2.89 minutes, 98% purity *(Method 10).*

*Example 190:* δ_{H} (250 MHz, Chloroform-d) 12.18 - 11.77 (m, 1H), 6.44 - 6.31 (m, 1H - Rotamer 1), 6.25 - 6.06 (m, 1H - Rotamer 2), 6.03 - 5.82 (m, 1H), 4.85 - 4.67 (m, 1H - Rotamer 2), 4.09 - 3.84 (m, 1H - Rotamer 1), 3.37 - 3.12 (m, 3H), 2.96 - 2.76 (m, 3H), 2.75 - 2.64 (m, 2H, Rotamer 1), 2.62 - 2.46 (m, 2H - Rotamer 2), 2.21 - 1.98 (m, 2H), 1.73 - 1.56 (m, 1H, part. obs. by water peak), 1.40 - 1.17 (m, 3H), 1.08 (m, 3H), 0.97 - 0.84 (m, 2H), 0.64 - 0.48 (m, 2H), 0.34 - 0.15 (m, 2H). LCMS [M+H]⁺ 442, RT 2.89 minutes, 94% purity *(Method 10).*

### EXAMPLE 191

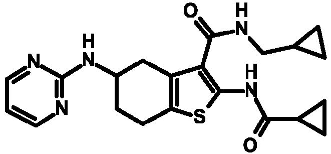

### 2-(Cyclopropanecarbonylamino)-N-(cyclopropylmethyl)-5-(pyrimidin-2-ylamino)-4,5,6,7-tetrahydrobenzothiophene-3-carboxamide

*Intermediate 122* (50 mg, 0.14 mmol) and 2-chloropyrimidine [1722-12-9] (30 mg, 0.26 mmol) were dissolved in MeCN (1 mL) and DIPEA (85 µL, 0.49 mmol) was added. The reaction mixture was heated at 150 °C for 1 h in the microwave. The solvent was removed *in vacuo* and the crude material was purified by preparative HPLC (basic) to afford the *title compound* (10 mg, 17%) as a light brown solid. δ_{H} (500 MHz, CD₃OD) 8.28 (d, *J* 4.8 Hz, 2H), 6.61 (t, *J* 4.9 Hz, 1H), 4.28 - 4.17 (m, 1H), 3.26 (dd, *J* 13.8, 7.0 Hz, 1H), 3.23 - 3.15 (m, 2H), 2.84 (t, *J* 5.9 Hz, 2H), 2.72 - 2.64 (m, 1H), 2.21 - 2.14 (m, 1H), 1.97 - 1.87 (m, 1H), 1.82 - 1.75 (m, 1H), 1.12 - 1.03 (m, 1H), 1.01 - 0.96 (m, 2H), 0.96 - 0.91 (m, 2H), 0.52 - 0.45 (m, 2H), 0.28 - 0.21 (m, 2H). LCMS (ES+) [M+H]⁺ 412.2, RT 2.87 minutes, 95% purity *(Method 10).*

### EXAMPLE 192

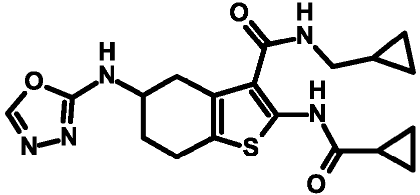

### 2-(Cyclopropanecarbonylamino)-N-(cyclopropylmethyl)-5-(1,3,4-oxadiazol-2-ylamino)-4,5,6,7-tetrahydrobenzothiophene-3-carboxamide

*Intermediate 209* (136 mg, 0.29 mmol) and formic hydrazide [624-84-0] (52 mg, 0.87 mmol) were dissolved in DMF (3 mL). Mercury dichloride [7487-94-7] (235 mg, 0.87 mmol) was added and the mixture was stirred for 5 minutes. Triethylamine (0.12 ml, 0.87 mmol) was added and the reaction mixture was heated at 90 °C for 1 h. The mixture was quenched with saturated sodium hydrogen carbonate solution (10 mL) and the aqueous layer was extracted with EtOAc (3 x 20 mL), dried (MgSO₄), filtered and concentrated *in vacuo* to give the crude product which was purified by preparative HPLC (basic) to afford the *title compound* (2 mg, 1.7%). δ_{H} (500 MHz, Methylene Chloride-*d*₂) 11.97 (s, 1H), 7.80 (s, 1H), 5.80 (s, 1H), 4.93 (d, *J* 6.8 Hz, 1H), 4.13-4.09 (m, 1H), 3.25-3.15 (m, 3H), 2.80-2.69 (m, 3H), 2.11-2.06 (m, 1H), 2.04 - 1.93 (m, 1H), 1.62 - 1.54 (m, 1H), 1.02 - 0.91 (m, 3H), 0.85-0.78 (m, 2H), 0.50 - 0.42 (m, 2H), 0.21 - 0.12 (m, 2H). LCMS (ES+) [M+H]⁺ 402.11, RT 2.48 minutes, 97% purity *(Method 10).*

### EXAMPLE 194

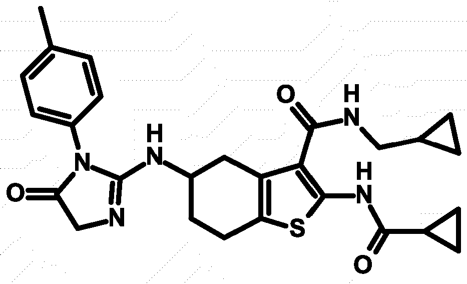

### 2-(Cyclopropanecarbonylamino)-N-(cyclopropylmethyl)-5-[[5-oxo-1-(p-tolyl)-4H-imidazol-2-yl]amino]-4,5,6,7-tetrahydrobenzothiophene-3-carboxamide

*Intermediate 211* (75 mg, 0.16 mmol) in MeCN (3 mL) was added to a stirred solution of methyl glycinate hydrochloride (1:1) [5680-79-5] (21 mg, 0.16 mmol) and triethylamine (0.035 mL, 0.25 mmol) in MeCN (1 mL). The reaction mixture was stirred at r.t. for 16 h and the mixture was concentrated *in vacuo* to give the crude product which was purified by preparative HPLC (basic) to afford the *title compound* (15 mg, 18%) as a white solid. δ_{H} (500 MHz, Chloroform-*d*) 12.08 (s, 1H), 7.30 (d, *J* 8.1 Hz, 2H), 7.09 (d, J 8.1 Hz, 2H), 5.97 - 5.82 (m, 1H), 4.35 (s, 1H), 4.21 (s, 2H), 4.15 - 4.01 (m, 1H), 3.35 - 3.14 (m, 3H), 2.87 - 2.77 (m, 1H), 2.76 - 2.60 (m, 2H), 2.40 (s, 3H), 2.13 - 2.02 (m, 1H), 1.99 - 1.89 (m, 1H), 1.68 - 1.65 (m, 1H), 1.15 - 1.01 (m, 3H), 0.94 - 0.87 (m, 2H), 0.61 - 0.51 (m, 2H), 0.31 - 0.22 (m, 2H). LCMS (ES+) [M+H]⁺ 506.3, RT 2.18 minutes, 100% purity *(Method 10).*

### EXAMPLE 195

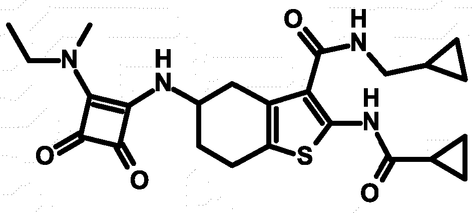

### 2-(Cyclopropanecarbonylamino)-N-(cyclopropylmethyl)-5-[[2-[ethyl(methyl)amino]-3,4-dioxo-cyclobuten-1-yl]amino]-4,5,6,7-tetrahydrobenzothiophene-3-carboxamide

*Intermediate 136* (93%, 50 mg, 0.1 mmol) was suspended in EtOH (3 mL) and ethyl(methyl)amine [624-78-2] (20 µL, 0.23 mmol) was added. The reaction mixture was stirred for 16 h at r.t. before adding further ethyl(methyl)amine [624-78-2] (20 µL, 0.23 mmol) and stirring at r.t. for 4 h. The precipitate was filtered and washed with EtOH to afford the *title compound* (42.9 mg, 57%) as a white solid. δ_{H} (500 MHz, DMSO-*d*₆) 11.04 (s, 1H), 7.76 (t, *J* 5.6 Hz, 1H), 7.61 (d, *J* 8.2 Hz, 1H), 4.48 - 4.36 (m, 1H), 3.57 (s, 2H), 3.19 (s, 3H), 3.13 (t, *J* 6.2 Hz, 2H), 2.97 (dd, *J* 15.4, 4.8 Hz, 1H), 2.82 - 2.65 (m, 3H), 2.11 - 2.01 (m, 1H), 1.95 - 1.79 (m, 2H), 1.15 (t, *J* 7.1 Hz, 3H), 1.07 - 0.97 (m, 1H), 0.90 - 0.76 (m, 4H), 0.45 - 0.37 (m, 2H), 0.24 - 0.17 (m, 2H). LCMS (ES+) [M+H]⁺ 471.1, RT 2.66 minutes, 96% purity *(Method 10).*

### EXAMPLE 196

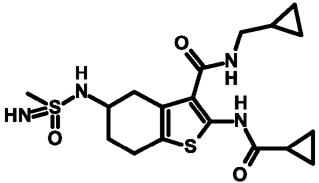

### 2-(Cyclopropanecarbonylamino)-N-(cyclopropylmethyl)-5-[(methylsulfonimidoyl)aminol-4,5,6,7-tetrahydrobenzothiophene-3-carboxamide

To a suspension of *intermediate 214* (32 mg, 0.09 mmoL) and cyclopropylmethanamine [2516-47-4] (0.012 mL, 0.13 mmoL) in DCM (3 mL) was added EDCI (28 mg, 0.14 mmoL) and the reaction mixture was stirred at r.t. under nitrogen for 20 h. Cyclopropylmethanamine [2516-47-4] (0.047 mL, 0.54 mmoL) was added and stirring was continued at 40 °C before diluting with chloroform/isopropanol (2:1, 15 mL) and saturated aqueous ammonium chloride (10 mL). The phases were separated and the aqueous phase was extracted with further chloroform/isopropanol (2:1, 10 mL). The combined organic phases were washed with brine (10 mL), dried (MgSO₄), filtered and concentrated *in vacuo* to give the crude product which was purified by preparative HPLC (acidic) to afford the *title compound* (2.5 mg, 7%) as a thin film. δ_{H} (250 MHz, Chloroform-*d*) 12.18 - 11.93 (m, 1H), 5.93 - 5.79 (m, 1H), 3.95 - 3.81 (m, 1H), 3.36 - 3.22 (m, 2H), 3.25 - 3.03 (m, 4H), 2.92 - 2.77 (m, 2H), 2.79 - 2.65 (m, 1H), 2.61 - 2.33 (2H, obscured by water peak), 2.19 - 2.03 (m, 1H), 2.02 - 1.83 (m, 1H), 1.74 - 1.58 (m, 1H), 1.18 - 1.00 (m, 3H), 0.99 - 0.82 (m, 2H), 0.69 - 0.47 (m, 2H), 0.35 - 0.21 (m, 2H). LCMS (ES+) [M+H]⁺ 411, RT 1.92 minutes, 97% purity *(Method 10).*

### EXAMPLE 197

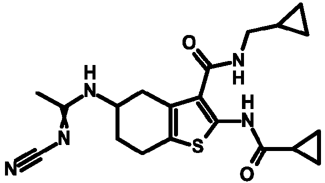

### 5-[[N-Cyano-C-methyl-carbonimidoyl]amino]-2-(cyclopropanecarbonylamino)-N-(cyclopropylmethyl)-4,5,6,7-tetrahydrobenzothiophene-3-carboxamide

*Intermediate 122* (50 mg, 0.13 mmol) was dissolved in EtOH (3 ml) and ethyl (1Z)-N-cyanoethanimidoate [1558-82-3] (20 mg, 0.17 mmol) and DIPEA (0.035 ml, 0.2 mmol) was added. The reaction mixture was stirred at 100 °C in the microwave for 4 h. The mixture was concentrated *in vacuo* to give the crude product which was purified by preparative HPLC (acidic) to afford the *title compound* (20 mg, 37%) as a white solid. δ_{H} (500 MHz, CD₃OD) 4.27 - 4.15 (m, 1H), 3.27 - 3.19 (m, 2H), 3.12 (dd, *J* 15.9, 5.1 Hz, 1H), 2.85 - 2.73 (m, 2H), 2.67 - 2.57 (m, 1H), 2.29 (s, 3H), 2.13 - 2.04 (m, 1H), 1.99 - 1.89 (m, 1H), 1.84 - 1.76 (m, 1H), 1.15 - 1.05 (m, 1H), 1.01 - 0.90 (m, 4H), 0.59 - 0.50 (m, 2H), 0.33 - 0.24 (m, 2H). LCMS (ES+) [M+H]⁺ 400.3, RT 2.75 minutes, 100% *(Method 10).*

### EXAMPLE 198

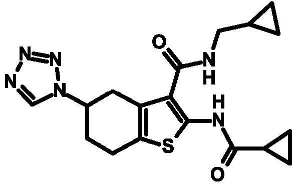

### 2-(Cyclopropanecarbonylamino)-N-(cyclopropylmethyl)-5-(tetrazol-1-yl)-4,5,6,7-tetrahydrobenzothiophene-3-carboxamide

*Intermediate 216* (100 mg, 0.23 mmol) was dissolved in 1,4-dioxane (2 mL) and water (2 mL). Potassium hydrogen sulfate sulfate sulfodioxidanide (5:1:1:2) (255 mg, 0.42 mmol) was added portionwise over 5 minutes. The reaction mixture was stirred for 2 h at r.t. and the mixture was concentrated *in vacuo.* The crude residue was dissolved in EtOAc (2 x 30 mL) and washed with water and brine, dried (MgSO₄), filtered and concentrated *in vacuo* to afford a mixture of the crude sulfoxide intermediate *2-(cyclopropanecarbonylamino)-N-(cyclopropylmethyl)-5-(5-methylsulfinyltetrazol-1-yl)-4,5,6,7-tetrahydrobenzothiophene-3-carboxamide* (43%) and *Intermediate 216* (27%). The crude material was dissolved in dimethylacetamide [127-19-5] (1 mL) and cyclopropanamine [765-30-0] (36 mg, 0.62 mmol) was added to the mixture which was heated at 140 °C for 8 h. The crude material was purified by preparative HPLC to afford the *title compound* (6 mg, 13%). δ_{H} (500 MHz, Methylene Chloride-*d*₂) 11.95 (s, 1H), 8.64 (s, 1H), 5.73 (s, 1H), 5.08 - 4.98 (m, 1H), 3.44 (dd, J = 15.0, 5.2 Hz, 1H), 3.30 - 3.17 (m, 3H), 3.04 - 2.92 (m, 1H), 2.86 (dt, J = 17.0, 5.0 Hz, 1H), 2.53 - 2.39 (m, 2H), 1.70-1.65 (m, 1H), 1.07 - 1.00 (m, 3H), 0.93 - 0.87 (m, 2H), 0.55 - 0.48 (m, 2H), 0.26 - 0.20 (m, 2H).

### EXAMPLE 199

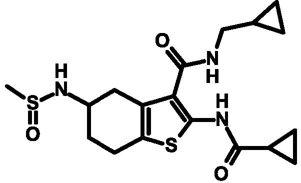

### 2-(Cyclopropanecarbonylamino)-N-(cyclopropylmethyl)-5-(methanesulfinamido)-4,5,6,7-tetrahydrobenzothiophene-3-carboxamide

To *intermediate 122* (150 mg, 0.41 mmoL), sodium methanesulfinate [20277-69-4] (50 mg, 0.49 mmoL) and EDCI (93 mg, 0.49 mmoL) in DCM (5 mL) was added DIPEA (0.17 mL, 0.97 mmoL) and the reaction mixture was stirred at r.t. under nitrogen for 18 h. Sodium methanesulfinate [20277-69-4] (50 mg, 0.49 mmoL), EDCI (93 mg, 0.49 mmoL) and DIPEA (0.17 mL, 0.97 mmoL) were again added and stirring continued at r.t. for a further 5 h. The reaction mixture was diluted with DCM (10 mL) and washed with saturated aqueous sodium hydrogen carbonate (10 mL), saturated aqueous ammonium chloride (10 mL), water (10 mL), brine (10 mL), dried (MgSO₄), filtered and concentrated *in vacuo.* The crude product was purified by flash column chromatography on silica (gradient elution with 0 to 100% EtOAc in heptane, followed by 0 to 20% MeOH in EtOAc) to afford the *title compound* (125 mg, 78%) as an off-white solid, a mixture of two diastereomers. δ_{H} (250 MHz, Chloroform-d) 12.18 - 11.85 (m, 1H), 6.24 - 5.81 (m, 1H), 4.04 - 3.77 (m, 2H), 3.33 - 3.22 (m, 2H), 3.22 - 3.05 (m, 1H), 2.98 - 2.69 (m, 3H), 2.70 - 2.61 (m, 3H), 2.21 - 2.03 (m, 1H), 2.03 - 1.83 (m, 1H), 1.75 - 1.57 (m, 1H, partially obs. by water), 1.17 - 0.99 (m, 3H), 0.96 - 0.82 (m, 2H), 0.64 - 0.51 (m, 2H), 0.32 - 0.22 (m, 2H). LCMS (ES+) [M+H]⁺ 396, RT 1.12 minutes, 96% purity (*Method 6*). LCMS (ES+) [M+H]⁺ 396, RT 2.49 minutes, 95% purity *(Method 10).*

### EXAMPLE 201

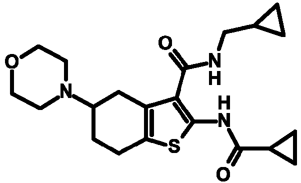

### 2-(Cyclopropanecarbonylamino)-N-(cyclopropylmethyl)-5-morpholino-4,5,6,7-tetrahydrobenzothiophene-3-carboxamide

To a solution of *intermediate 62* (50 mg, 0.15 mmoL) and morpholine [110-91-8] (0.02 mL, 0.23 mmoL) in 1,2-dichloroethane [107-06-2] (3 mL) was added acetic acid [64-19-7] (0.009 mL, 0.15 mmoL) followed by sodium triacetoxyborohydride [56553-60-7] (48 mg, 0.23 mmoL) and the mixture was stirred at r.t. under nitrogen for 21 h. Further morpholine [110-91-8] (0.02 mL, 0.23 mmoL) and sodium triacetoxyborohydride [56553-60-7] (48 mg, 0.23 mmoL) were added and stirring was continued at r.t. for 6 h. The reaction mixture was partitioned between DCM (10 mL) and saturated sodium hydrogen carbonate solution (10 mL). The phases were separated and the aqueous phase was extracted with DCM (15 mL). The combined organic phases were washed with water (10 mL), brine (10 mL) and dried (MgSO₄), filtered and concentrated *in vacuo* to give the crude product which was purified by flash column chromatography on silica (gradient elution with 0 to 100% EtOAc in heptane followed by a gradient 0 to 20% MeOH in EtOAc) to give the product which was purified by preparative HPLC (basic) to afford the *title compound* (23 mg, 38%). δ_{H} (250 MHz, CD₃OD) 3.74 (t, *J* 4.7 Hz, 4H), 3.42 - 3.28 (m, 1H, partially obscured by MeOD signal), 3.15 (dd, *J* 13.7, 7.0 Hz, 1H), 2.98 - 2.82 (m, 1H), 2.85 - 2.60 (m, 8H), 2.30 - 2.12 (m, 1H), 1.87 - 1.59 (m, 2H), 1.18 - 1.02 (m, 1H), 1.02 - 0.85 (m, 4H), 0.63 - 0.46 (m, 2H), 0.37 - 0.21 (m, 2H). LCMS (ES+) [M+H]⁺ 404, RT 1.53 minutes, 98% purity *(Method 10).*

### EXAMPLE 202

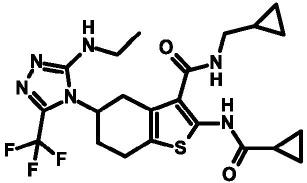

### 2-(Cyclopropanecarbonylamino)-N-(cyclopropylmethyl)-5-[3-(ethylamino)-5-(trifluoromethyl)-1,2,4-triazol-4-yl]-4,5,6,7-tetrahydrobenzothiophene-3-carboxamide

To a solution of *intermediate 220* (31 mg, 0.053 mmol) in 88% formic acid [64-18-6] (6 mL) was added 10% Pd on carbon (50 % wet type, 30 mg, 0.028 mmol) under nitrogen. The reaction mixture was warmed to 80 °C and stirred for 120 minutes. The reaction mixture was treated with additional 10% Pd/C (50% wet type, 30 mg, 0.028 mmol) and was heated at 80 °C for 60 minutes. The reaction mixture was filtered through Celite and the filtrate was concentrated *in vacuo* to give the crude product as a colourless glass which was purified by preparative HPLC (basic) to afford the *title compound* (18 mg, 70%) as a colourless solid. δ_{H} (500 MHz, Chloroform-d) 12.04 (s, 1H), 5.82 (t, *J* 4.9 Hz, 1H), 4.49 (s, 1H), 4.16 (s, 1H), 3.52 (p, *J* 6.9 Hz, 2H), 3.34 - 3.18 (m, 3H), 3.07 (dd, *J* 14.8, 5.8 Hz, 1H), 2.97 - 2.87 (m, 2H), 2.45 - 2.32 (m, 1H), 2.30 - 2.21 (m, 1H), 1.68 (ddd, *J* 12.5, 7.9, 4.6 Hz, 1H), 1.29 (t, *J* 7.2 Hz, 3H), 1.14 - 1.09 (m, 2H), 1.05 (ddd, *J* 12.7, 8.7, 5.1 Hz, 1H), 0.96 - 0.90 (m, 2H), 0.56 - 0.51 (m, 2H), 0.24 (q, *J* 4.6 Hz, 2H). LCMS (ES+) [M+H]⁺ 497, RT 3.19 minutes, 100% purity *(Method* 10).

### EXAMPLE 203

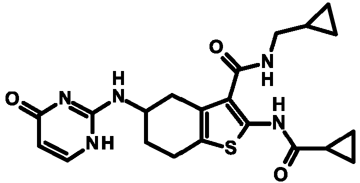

### 2-(Cyclopropanecarbonylamino)-N-(cyclopropylmethyl)-5-[(4-oxo-1H-pyrimidin-2-yl)amino]-4,5,6,7-tetrahydrobenzothiophene-3-carboxamide

*Intermediate 122* (156 mg, 0.42 mmol) and 2-methylthiopyrimidin-4-ol [124700-70-5] (72 mg, 0.51 mmol) was dissolved in *tert-butyl* alcohol (1.5 mL) and DIPEA (0.18 mL, 1.03 mmol) was added. The reaction was heated in the microwave at 160 °C for 16 h before concentrating *in vacuo.* The crude product was purified by flash column chromatography on silica (gradient elution with 0-20% MeOH in DCM) to afford the *title compound* (63 mg, 35%) as an off white solid. δ_{H} (400 MHz, DMSO-*d*₆) 11.12 (s, 1H), 10.42 (s, 1H), 7.71 (t, *J* 5.7 Hz, 1H), 7.60 (d, *J* 6.5 Hz, 1H), 6.71 - 6.44 (m, 1H), 5.54 (d, *J* 6.5 Hz, 1H), 4.31 - 4.03 (m, 1H), 3.14 (t, *J* 6.2 Hz, 2H), 3.05 (dd, *J* 16.3, 5.1 Hz, 1H), 2.79 - 2.69 (m, 2H), 2.61 (dd, *J* 16.2, 7.2 Hz, 1H), 2.09 - 1.74 (m, 3H), 1.12 - 0.96 (m, 1H), 0.95 - 0.72 (m, 4H), 0.51 - 0.32 (m, 2H), 0.30 - 0.14 (m, 2H). LCMS [M-H]⁻ 426.0, RT 1.530 minutes, 94.8% purity *(Method 2).* LCMS [M-H]⁻ 426.0, RT 1.598 minutes, 96.9% purity *(Method 3).*

### EXAMPLE 204

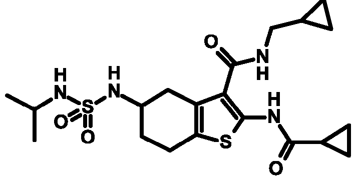

### 2-(Cyclopropanecarbonylamino)-N-(cyclopropylmethyl)-5-(isopropylsulfamoylamino)-4,5,6,7-tetrahydrobenzothiophene-3-carboxamide

*Intermediate 122* (50 mg, 0.14 mmol) was dissolved in DCM (5 mL) and DIPEA (26 mg, 0.20 mmol) and isopropylsulfamoyl chloride [26118-67-2] (32 mg, 0.20 mmol) was added. The reaction mixture was stirred at r.t. for 3 days. Further isopropylsulfamoyl chloride [26118-67-2] was added (32 mg, 0.20 mmol) and the reaction mixture was stirred at r.t. for 4 days. The reaction mixture was directly purified by flash column chromatography on silica (gradient elution with 35-100% EtOAc/hexane) to give the product which was freeze-dried from MeCN/water to afford the *title compound* (9 mg, 15%) as a white solid. LCMS [M-H]⁻ 453.0, RT 2.084 minutes, 100.0% purity *(Method 3).* LCMS [M-H]⁻ 453.0, RT 2.156 minutes, 100.0% purity *(Method 2).*

### EXAMPLE 205

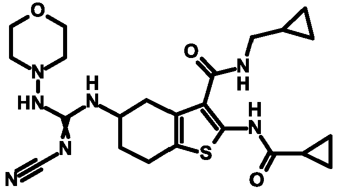

### 5-[[N'-Cyano-N-morpholino-carbamimidoyl]amino]-2-(cyclopropanecarbonylamino)-N-(cyclopropylmethyl)-4,5,6,7-tetrahydrobenzothiophene-3-carboxamide

*Intermediate 122* (100 mg, 0.27 mmol) was dissolved in DCM (4 mL) and DIPEA (88 mg, 0.68 mmol) and *intermediate 221* (100 mg, 0.41 mmol) was added. The reaction mixture was stirred at 130 °C in the microwave for 4 h. The reaction mixture was concentrated *in vacuo* and the crude residue was purified by flash column chromatography on silica (gradient elution with 30-100% EtOAc/hexane to 20% MeOH/EtOAc) to give the product which was freeze dried from MeCN/water to afford the product which was purified by preparative HPLC to afford the *title compound* (5.5 mg, 4.2%) as a white solid. δ_{H} (400 MHz, DMSO-*d*₆) 11.01 (s, 1H), 8.72 (s, 1H), 7.75 (t, *J* 5.6 Hz, 1H), 7.00 (d, *J* 8.3 Hz, 1H), 3.93 (br. s, 1H), 3.65 (br. d, *J* 61.3 Hz, 4H), 3.19 - 3.06 (m, 2H), 2.97 - 2.61 (m, 8H), 2.05 - 1.68 (m, 3H), 1.10 - 0.96 (m, 1H), 0.92 - 0.76 (m, 4H), 0.48 - 0.36 (m, 2H), 0.30 - 0.17 (m, 2H). LCMS (ES+) [M+H]⁺ 486.20, RT 1.890 minutes, purity 100.0% *(Method 2).*

### EXAMPLE 206

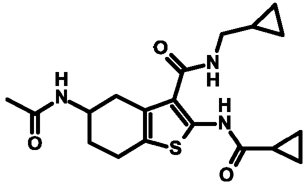

### 5-Acetamido-2-(cyclopropanecarbonylamino)-N-(cyclopropylmethyl)-4,5,6,7-tetrahydrobenzothiophene-3-carboxamide

*Intermediate 122* (50 mg, 0.14 mmol) was dissolved in DCM (5 mL) and DIPEA (44 mg, 0.34 mmol) and acetyl chloride [75-36-5] (14 mg, 0.18 mmol) were added. The reaction mixture was stirred at r.t. for ^{~}4 h. The reaction mixture was washed with water and passed through a phase separation cartridge and concentrated *in vacuo.* The crude residue was purified by flash column chromatography on silica (gradient elution with 30-100% EtOAc/hexane to 20% MeOH/EtOAc) to give the product which was freeze-dried from MeCN/water to afford the *title compound* (16 mg, 32%) as a white solid. δ_{H} (400 MHz, DMSO-*d*₆) 10.85 (s, 1H), 7.67 (d, *J* 7.4 Hz, 1H), 7.47 (s, 1H), 3.80 - 3.59 (m, 1H), 2.96 - 2.82 (m, 2H), 2.67 (d, *J* 16.9 Hz, 1H), 2.54 - 2.36 (m, 2H), 1.76 - 1.62 (m, 2H), 1.59 (s, 3H), 1.46 (br. s, 1H), 0.93 (d, *J* 6.7 Hz, 1H), 0.85 - 0.71 (m, 1H), 0.68 - 0.48 (m, 4H), 0.29 - 0.13 (m, 2H), 0.09 - -0.08 (m, 2H). LCMS [M-H]⁻ 374.0, [M+H]⁺ 376.8, RT 1.693 minutes, 94.4% purity *(Method 2).* LCMS (ES+) [M+H]⁺ 376.8, [M+Na]⁺ 398.6, RT 1.621 minutes, 94.1% purity *(Method 3).*

### EXAMPLE 207

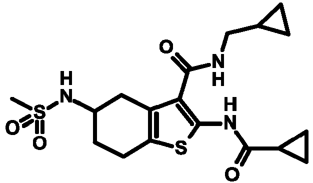

### 2-(Cyclopropanecarbonylamino)-N-(cyclopropylmethyl)-5-(methanesulfonamido)-4,5,6,7-tetrahydrobenzothiophene-3-carboxamide

*Intermediate 122* (50 mg, 0.14 mmol) was dissolved in DCM (5 mL) and DIPEA (44 mg, 0.34 mmol) and methanesulfonyl chloride [124-63-0] (20 mg, 0.18 mmol) were added. The reaction mixture was stirred at r.t. for ^{~}4 h. The reaction mixture was washed with water, passed through a phase separation cartridge and concentrated *in vacuo* to give the crude residue which was purified by flash column chromatography on silica (gradient elution with 30-100% EtOAc/hexane). The product was freeze dried from MeCN/water to afford the *title compound* (21 mg, 38%) as a white solid. δ_{H} (400 MHz, DMSO-*d*₆) 10.78 (s, 1H), 7.49 (s, 1H), 6.95 (d, *J* 6.7 Hz, 1H), 3.31 (s, 1H), 2.96 - 2.85 (m, 2H), 2.73 (s, 4H), 2.53 - 2.45 (m, 2H), 2.37 (dd, *J* 16.1, 8.6 Hz, 1H), 1.82 - 1.74 (m, 1H), 1.67 (s, 1H), 1.55 - 1.42 (m, 1H), 0.82 - 0.72 (m, 1H), 0.68 - 0.50 (m, 4H), 0.24 - 0.09 (m, 2H), 0.06 - -0.10 (m, 2H). LCMS [M+H]⁺ 412.8, [M-H]⁻ 410.0, RT 1.812 minutes, 100.0% purity *(Method 2).* LCMS (ES+) [M+H]⁺ 412.7, [M+Na]⁺ 434.6, RT 1.735 minutes, 100.0% purity *(Method 3).*

### EXAMPLE 208

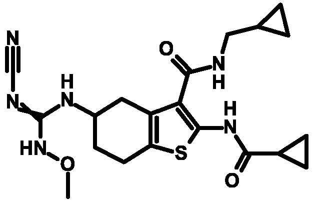

### 5-[[N'-Cyano-N-methoxy-carbamimidoyl]amino]-2-(cyclopropanecarbonylamino)-N-(cyclopropylmethyl)-4,5,6,7-tetrahydrobenzothiophene-3-carboxamide

To diphenyl N-cyanocarbonimidate [76463-77-7] (40 mg, 0.17 mmol) was added O-methylhydroxylamine [67-62-9] (15 mg, 0.18 mmol) and 1:1 DCM/isopropanol (1 mL) followed by DIPEA (44 mg, 0.34 mmol). The reaction mixture was stirred at r.t. overnight. To the reaction mixture was added *intermediate 122* (40 mg, 0.11 mmol) and the reaction mixture was stirred at 100 °C for 5 h. The reaction mixture was concentrated *in vacuo* and purified by preparative HPLC (basic) to afford the *title compound* (4.1 mg, 8.8%). LCMS [M+H]⁺ 431.1855, RT 4.41 minutes, 90.56% purity *(Method 20).*

### EXAMPLE 210

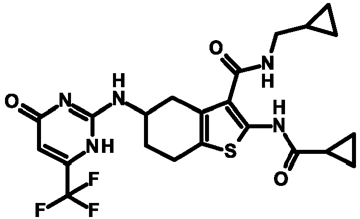

### 2-(Cyclopropanecarbonylamino)-N-(cyclopropylmethyl)-5-[[4-oxo-6-(trifluoromethyl)-1H-pyrimidin-2-yl]amino]-4,5,6,7-tetrahydrobenzothiophene-3-carboxamide

To *intermediate 122* (144 mg, 0.39 mmol) and 4-hydroxy-2-(methylthio)-6-(trifluoromethyl)pyrimidine [16097-62-4] (109 mg, 0.50 mmol) dissolved in *tert*-butyl alcohol (1.5 mL) was added DIPEA (0.18 mL, 1.03 mmol). The reaction mixture was heated in the microwave at 160 °C for 60 h, concentrated *in vacuo* and purified by preparative HPLC to afford the *title compound* (17 mg, 8.7%) as a white solid. LCMS (ES+) [M+H]⁺ 496.0, [M+Na]⁺ 518.0, RT 2.293 minutes, 100.0% purity *(Method 2).* LCMS [M-H]⁻ 494.0, RT 2.108 minutes, 100.0% purity *(Method 3).*

### EXAMPLE 213

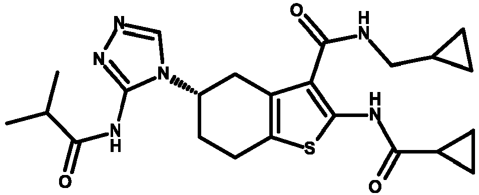

### (5S)-2-(cyclopropanecarbonylamino)-N-(cyclopropylmethyl)-5-[3-(2-methylpropanoylamino)-1,2,4-triazol-4-yl]-4,5,6,7-tetrahydrobenzothiophene-3-carboxamide

To a stirred solution of *intermediate 243* (128 mg, 0.277 mmol) in DMF (7 mL) under nitrogen was added formic acid hydrazide [624-84-0] (46 mg, 0.692 mmol) and mercuric chloride [7487-94-7] (98 mg, 0.360 mmol). The reaction mixture was stirred at r.t. for 5 min before the addition of triethylamine (0.058 mL, 0.415 mmol) and the mixture was stirred at 80 °C for 17 h. The mixture was cooled to r.t. and diluted with MeCN (5 mL), filtered through a pad of celite and washed with MeCN (15 mL). The filtrate was concentrated *in vacuo* to yield the crude product as an tan solid. This solid was purified by flash column chromatography on silica (gradient elution with 50% to 100% EtOAc in isohexanes and then 0% to 20% EtOAc in DCM) to afford impure *title compound* as an off-white solid (14 mg). This solid was further purified by reverse phase chromatography on C18 silica (5% to 100% MeCN (w/ 0.1% NH3) in water (w/ 0.1% NH3)) and the product containing fractions were freeze dried to afford the *title compound* (3.0 mg, 0.006 mmol, 2%) as a white solid. LCMS (ES+) [M+H]⁺ 471.2, RT 1.60 minutes, purity 100% *(Method 3).* LCMS (ES+) [M+H]⁺ 471.2, RT 1.63 minutes, purity *100% (Method 2).*

### EXAMPLE 214

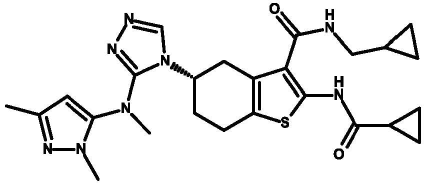

### (5S)-2-(cyclopropanecarbonylamino)-N-(cyclopropylmethyl)-5-[3-[(2,5-dimethylpyrazol-3-yl)-methyl-amino]-1,2,4-triazol-4-yl]-4,5,6,7-tetrahydrobenzothiophene-3-carboxamide

To a stirred solution of *example 106* (70 mg, 0.142 mmol) dissolved in acetonitrile (5 mL) was added cesium carbonate (69 mg, 0.212 mmol) followed by iodomethane (11 µl, 0.170 mmol). The reaction was stirred at r.t. for 17 h before being concentrated in vacuo to give a gum. This gum was dissolved in DCM (50 mL), washed with water (30 mL) and the layers separated. The Aq layer was extracted with DCM (2 x 25 mL). The combined organic layers were passed through a phase separation cartridge and concentrated *in vacuo* to give a tan gum. This was purified by flash column chromatography on silica (gradient elution with 1% to 20% MeOH in DCM) to afford the *title compound RF3* (15 mg, 0.029 mmol, 21%) as a white solid. LCMS [M+H]⁺ 509.2, RT 1.67 minutes, purity 95.6% *(Method 3).* LCMS [M+H]⁺ 509.2, RT 1.60 minutes, purity 95.1% *(Method 3).* 1H NMR (400 MHz, Methanol-d4) δ 8.43 (s, 1H), 5.85 (s, 1H), 3.92 - 3.78 (m, 1H), 3.67 (s, 3H), 3.33 (s, 3H), 3.26 (dd, J = 13.8, 7.0 Hz, 1H), 3.18 (dd, J = 13.8, 7.0 Hz, 1H), 3.05 - 2.96 (m, 1H), 2.92 (dd, J = 15.6, 5.6 Hz, 1H), 2.85 - 2.76 (m, 1H), 2.72 - 2.60 (m, 1H), 2.20 - 2.01 (m, 5H), 1.86 - 1.77 (m, 1H), 1.15 - 1.03 (m, 1H), 1.03 - 0.89 (m, 4H), 0.55 - 0.48 (m, 2H), 0.31 - 0.24 (m, 2H).

### EXAMPLE 216

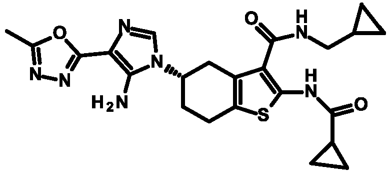

### (5S)-5-[5-amino-4-(5-methyl-1,3,4-oxadiazol-2-yl)imidazol-1-yl]-2-(cyclopropanecarbonylamino)-N-(cyclopropylmethyl)-4,5,6,7-tetrahydrobenzothiophene-3-carboxamide

*Intermediate 219* (68%, 61 mg, 0.3 mmol) was dissolved in dry MeCN (4 mL) in a 20 mL pressure tube. Triethylorthoformate (50 µl, 0.3 mmol) was added, the reaction was sealed and heated to 90 °C for 1 hour. The reaction was allowed to cool to room temperature and *intermediate 117* (100 mg, 0.3 mmol) was added. The suspension was stirred for 3 days at room temperature. The pale brown suspension was concentrated *in vacuo* and the solid purified by flash chromatography on silica (gradient elution using 0 - 10% MeOH in DCM) followed by trituration with ethyl acetate: heptane (1 : 1) to afford the *title compound* (20.5mg, 14%) as a pink solid. δ_{H} (500 MHz, DMSO-*d*₆) δ 11.21 (s, 1H), 7.72 (t, J 5.7 Hz, 1H), 7.44 (s, 1H), 5.96 (s, 2H), 4.45 - 4.35 (m, 1H), 3.20 - 3.05 (m, 3H), 3.05 - 2.96 (m, 1H), 2.93 - 2.76 (m, 2H), 2.55 - 2.44 (m, 3H, obscured by DMSO, observed in HSQC), 2.27 - 2.12 (m, 2H), 1.97 - 1.88 (m, 1H), 1.07 - 0.95 (m, 1H), 0.91 - 0.80 (m, 4H), 0.43 - 0.33 (m, 2H), 0.26 - 0.14 (m, 2H). LCMS [M+H]⁺ 482.2, RT 2.49 minutes, 98% purity *(Method 10).*

### EXAMPLE 217

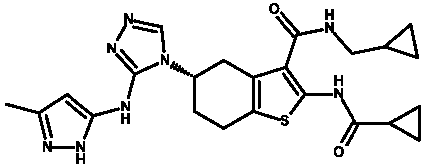

### (5S)-2-(cyclopropanecarbonylamino)-N-(cyclopropylmethyl)-5-[3-[(3-methyl-1H-pyrazol-5-yl)amino]-1,2,4-triazol-4-yl]-4,5,6,7-tetrahydrobenzothiophene-3-carboxamide

To a stirred solution of *intermediate 245* (105 mg, 0.222 mmol) in DMF (5 mL) under nitrogen was added formic acid hydrazide [624-84-0] (22 mg, 0.333 mmol) and mercuric chloride [7487-94-7] (78 mg, 0.289 mmol). The reaction mixture was stirred at r.t. for 5 min before the addition of triethylamine (0.046 mL, 0.333 mmol) and the mixture was stirred at 80 °C for 2 h. The mixture was cooled to r.t. and diluted with MeCN (5 mL), filtered through a pad of celite and washed with MeCN (15 mL). The filtrate was concentrated *in vacuo* to yield the crude product as a tan solid. This solid was purified by flash column chromatography on silica (gradient elution with 90% to 100% EtOAc in isohexanes and then 0% to 20% MeOH in EtOAc) to afford impure *title compound* as an off-white solid (23 mg). This solid was further purified by reverse phase chromatography on C18 silica (5% to 100% MeCN (w/ 0.1% NH3) in water (w/ 0.1% NH3)) and the product containing fractions were freeze dried to afford the *title compound* (9.0 mg, 0.019 mmol, 8.4%) as a white solid. LCMS (ES+) [M+H]⁺ 481.0, RT 1.53 minutes, purity 100% *(Method 3).* LCMS (ES+) [M+H]⁺ 481.0, RT 1.50 minutes, purity 100% *(Method 2).* 1H NMR (400 MHz, DMSO-d6) δ 11.72 (s, 1H), 11.24 (s, 1H), 8.93 (s, 1H), 8.27 (s, 1H), 7.89 - 7.50 (m, 1H), 6.21 (s, 1H), 4.68 - 4.28 (m, 1H), 3.20 - 3.03 (m, 3H), 3.00 - 2.75 (m, 3H), 2.23 - 2.09 (m, 5H), 1.99 - 1.88 (m, 1H), 1.04 - 0.95 (m, 1H), 0.91 - 0.81 (m, 4H), 0.40 - 0.31 (m, 2H), 0.21 - 0.15 (m, 2H).

### EXAMPLE 218

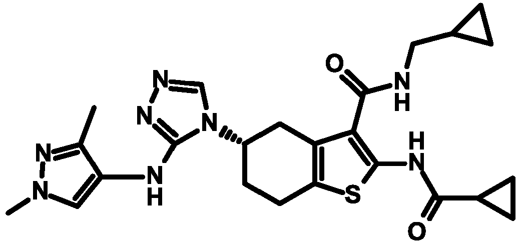

### (5S)-2-(cyclopropanecarbonylamino)-N-(cyclopropylmethyl)-5-[3-[(1,3-dimethylpyrazol-4-yl)aminol-1,2,4-triazol-4-yl]-4,5,6,7-tetrahydrobenzothiophene-3-carboxamide

To a stirred solution of *intermediate* 230(478.3 mg, 0.9338 mmol, 95 mass%) in DMF (6.6 mL) under nitrogen was added formic acid hydrazide [624-84-0] (170.1 mg, 2.832 mmol) and mercuric chloride [7487-94-7] (770.5 mg, 2.824 mmol). The reaction mixture was stirred at r.t. for 5 min before the addition of triethylamine (0.40 mL, 2.9 mmol) and the mixture was stirred at 60 °C for 19.5 h. The mixture was cooled to r.t. and diluted with MeCN (20 mL), filtered through a pad of celite and washed with MeCN (25 mL). The filtrate was concentrated *in vacuo* to yield the crude product as an orange oil which was dissolved in 5% MeOH in DCM (50 mL), washed with water (30 mL) and the layers separated. The aqueous layer was extracted with 5% MeOH in DCM (2 x 25 mL). The combined organic layers were passed through a phase separation cartridge and concentrated in *vacuo* to give an orange oil. This oil was purified by flash column chromatography on silica (gradient elution with 0% to 20% MeOH in DCM) to afford impure *title compound* as a white residue (116.0 mg). This residue was dissolved in MeOH (4 mL), QuadraSil^{®} MTU (120 mg) was added and the suspension was stirred at r.t. for 4.5 h before being filtered and washed with MeOH (2 x 5 mL). The filtrate was concentrated *in vacuo* to give a white solid which was further purified by reverse phase chromatography on C18 silica (5% to 100% MeCN (w/ 0.1% NH3) in water (w/ 0.1% NH3)) and the product containing fractions were freeze dried to afford the *title compound* (51.5 mg, 0.104 mmol, 11%) as a white solid. LCMS (ES+) [M+H]⁺ 494.8, RT 1.91 minutes, purity 97% *(Method 24).* LCMS (ES+) [M+H]⁺ 494.8, RT 1.97 minutes, purity 97% (*Method 25*). 1H NMR (400 MHz, DMSO-*d*₆) δ 11.25 (s, 1H), 8.21 (s, 1H), 7.80 (s, 1H), 7.72 (t, J = 5.9 Hz, 1H), 7.64 (s, 1H), 4.53 - 4.41 (m, 1H), 3.72 (s, 3H), 3.21 3.04 (m, 3H), 3.00 - 2.78 (m, 3H), 2.26 - 2.13 (m, 2H), 2.08 (s, 3H), 1.98 - 1.88 (m, 1H), 1.06 - 0.94 (m, 1H), 0.92 - 0.77 (m, 4H), 0.43 - 0.30 (m, 2H), 0.22 - 0.15 (m, 2H).

### EXAMPLE 219

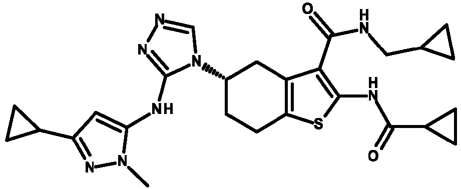

### (5S)-2-(cyclopropanecarbonylamino)-N-(cyclopropylmethyl)-5-[3-[(5-cyclopropyl-2-methyl-pyrazol-3-yl)amino]-1,2,4-triazol-4-yl]-4,5,6,7-tetrahydrobenzothiophene-3-carboxamide

To a stirred solution of *intermediate* 247 (1.38 g, 2.69 mmol) in DMF (20 mL) under nitrogen was added formic acid hydrazide [624-84-0] (539 mg, 8.07 mmol) and mercuric chloride [7487-94-7] (1.1 g, 4.04 mmol). The reaction mixture was stirred at r.t. for 15 min before the addition of triethylamine (1.13 mL, 8.07 mmol) and the mixture was stirred at 60 °C for 16 h. The mixture was cooled to r.t. and diluted with MeCN (20 mL), filtered through a pad of celite and washed with MeCN (30 mL). The filtrate was concentrated *in vacuo* to yield the crude product as a yellow gum which was dissolved in DCM (100 mL), washed with water (50 mL) and the layers separated. The Aq layer was extracted with DCM (2 x 25 mL). The combined organic layers were washed with brine (50 ml) and passed through a phase separation cartridge and concentrated *in vacuo* to give a yellow gum. This was purified by flash column chromatography on silica (gradient elution with 2% to 20% MeOH in DCM) to afford impure *title compound* as an off-white solid. This solid was dissolved in MeOH (50 mL) and QuadraSil^{®} MTU (2.0 g) was added and the suspension was stirred at r.t. for 2 h before being filtered and washed with MeOH (2 x 15 mL). The filtrate was concentrated *in vacuo* to give a colourless gum. This was further purified by flash column chromatography on silica (gradient elution with 5% to 25% MeOH in EtOAc) product containing fractions were freeze dried to afford the *title compound* (673 mg, 1.29 mmol, 48%) as a white solid. LCMS (ES+) [M+H]⁺ 521.0, RT 1.71 minutes, purity 100% *(Method 3).* LCMS (ES+) [M+H]⁺ 521.0, RT 1.68 minutes, purity 100% *(Method 2).* 1H NMR (300 MHz, DMSO-d6) (contained 2 tautomers in an approximate ratio of 0.7:0.3) δ 11.71 (s, 0.3H), 11.25 (s, 0.7H), 11.20 (s, 0.3H), 8.43 (s, 0.7H), 8.35 (s, 0.7H), 8.12 (s, 0.3H), 7.87 - 7.59 (m, 1H), 5.80 (s, 0.7H), 5.60 (s, 0.3H), 4.55 - 4.26 (m, 1H), 3.55 (s, 2H), 3.47 (s, 1H), 3.22 - 2.74 (m, 6H), 2.29 - 2.10 (m, 2H), 2.01-1.86 (m, 1H), 1.83 - 1.63 (m, 0.7H), 1.08 - 0.93 (m, 0.3H), 0.91 - 0.72 (m, 6H), 0.62 - 0.54 (m, 2H), 0.42 - 0.33 (m, 2H), 0.23 - 0.15 (m, 2H).

### EXAMPLE 220

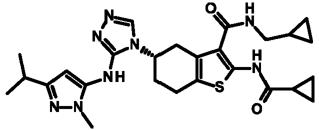

### (5S)-2-(cyclopropanecarbonylamino)-N-(cyclopropylmethyl)-5-[3-[(5-isopropyl-2-methyl-pyrazol-3-yl)amino]-1,2,4-triazol-4-yl]-4,5,6,7-tetrahydrobenzothiophene-3-carboxamide

To a stirred solution of *intermediate 249* (282 mg, 0.548 mmol) in DMF (10 mL) under nitrogen was added formic acid hydrazide [624-84-0] (110 mg, 1.64 mmol) and mercuric chloride [7487-94-7] (446 mg, 1.64 mmol). The reaction mixture was stirred at r.t. for 15 min before the addition of triethylamine (0.23 mL, 1.64 mmol) and the mixture was stirred at 60 °C for 16 h. The mixture was cooled to r.t. and diluted with MeCN (10 mL), filtered through a pad of celite and washed with MeCN (15 mL). The filtrate was concentrated *in vacuo* to yield the crude product as a yellow gum which was dissolved in DCM (50 mL), washed with water (30 mL) and the layers separated. The Aq layer was extracted with DCM (2 x 15 mL). The combined organic layers were washed with brine (30 ml) and passed through a phase separation cartridge and concentrated *in vacuo* to give a yellow gum. This was purified by flash column chromatography on silica (gradient elution with 1% to 20% MeOH in DCM) to afford impure *title compound* as a pale yellow gum. This was purified again by flash column chromatography on silica (gradient elution with 5% to 25% MeOH in EtOAc) to give a white solid. This solid was dissolved in MeOH (10 mL) and QuadraSil^{®} MTU (130 mg) was added and the suspension was stirred at r.t. for 2 h before being filtered and washed with MeOH (2 x 10 mL). The filtrate was concentrated *in vacuo* to give a colourless gum. This was further purified by flash column chromatography on silica (gradient elution with 5% to 25% MeOH in EtOAc) product containing fractions were freeze dried to afford the *title compound* (98 mg, 0.188 mmol, 34%) as a white solid. LCMS (ES+) [M+H]⁺ 523.0, RT 1.80 minutes, purity 100% *(Method 3).* LCMS (ES+) [M+H]⁺ 523.0, RT 1.75 minutes, purity 100% *(Method 2).* 1H NMR (300 MHz, DMSO-d6) (contained 2 tautomers in an approximate ratio of 0.7:0.3) δ 11.74 (s, 0.3H), 11.37 - 11.07 (m, 1H), 8.43 (s, 0.7H), 8.36 (s, 0.7H), 8.12 (s, 0.3H), 7.84 - 7.62 (m, 1H), 5.93 (s, 0.7H), 5.79 (s, 0.3H), 4.47 - 4.31 (m, 1H), 3.58 (s, 2H), 3.50 (s, 1H), 3.22 - 2.66 (m, 7H), 2.30 - 2.11 (m, 2H), 1.99 - 1.85 (m, 1H), 1.16 (d, J = 6.9 Hz, 6H), 0.90 - 0.78 (m, 4H), 0.42 - 0.31 (m, 2H), 0.23 - 0.15 (m, 2H).

### EXAMPLE 221

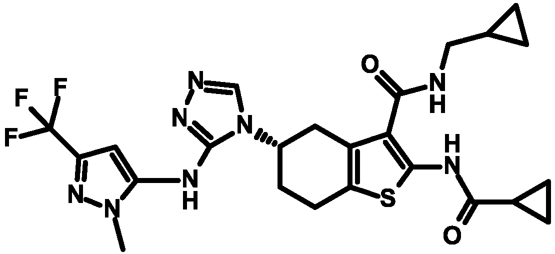

### (5S)-2-(cyclopropanecarbonylamino)-N-(cyclopropylmethyl)-5-[3-[[2-methyl-5-(trifluoromethyl)pyrazol-3-yl]amino]-1,2,4-triazol-4-yl]-4,5,6,7-tetrahydrobenzothiophene-3-carboxamide

To a stirred solution of *intermediate 232* (1.040 g, 1.924 mmol) in DMF (13.5 mL) under nitrogen was added formic acid hydrazide [624-84-0] (347.1 mg, 5.779 mmol) and mercuric chloride [7487-94-7] (630.2 mg, 2.310 mmol). The reaction mixture was stirred at r.t. for 5 min before the addition of triethylamine (0.80 mL, 5.7 mmol) and the mixture was stirred at 80 °C for 21.5 h. The mixture was cooled to r.t. and diluted with MeCN (60 mL), filtered through a pad of celite and washed with MeCN (30 mL). The filtrate was concentrated *in vacuo* to yield the crude product as an orange oil which was dissolved in 5% MeOH in DCM (100 mL), washed with water (100 mL) and the layers separated. The aqueous layer was extracted with 5% MeOH in DCM (2 x 50 mL). The combined organic layers were passed through a phase separation cartridge and concentrated *in vacuo* to give an orange oil. This oil was purified by flash column chromatography on silica (gradient elution with 0% to 15% MeOH in DCM) to afford impure *title compound* as an orange oil (1.443 g). This residue was dissolved in MeOH (13.5 mL), QuadraSil^{®} MTU (2.00 g) was added and the suspension was stirred at 40 °C for 4 h before being filtered and washed with MeOH (2 x 10 mL). The filtrate was concentrated *in vacuo* to give an orange oil which was further purified by preparative SFC (Waters Prep 100, Column - Viridis BEH 2-ethylpyridine (2-EP), 5-15% MeOH + 0.1 % NH4OH in CO₂) and the product containing fractions were freeze dried to afford the *title compound* (505.8mg, 0.9220 mmol, 48%) as a white solid. LCMS (ES+) [M+H]⁺ 549.0, RT 2.101 minutes, purity 99.7% *(Method 2).* LCMS (ES+) [M+H]⁺ 549.0, RT 2.020 minutes, purity 99.7% *(Method 3).* 1H NMR (300 MHz, DMSO-d6) (contained 2 tautomers in an approximate ratio of 0.8:0.2) δ 12.19 (s, 0.8H), 11.21 (s, 1H), 8.90 (s, 0.2H), 8.42 (s, 0.2H), 8.24 (s, 0.8H), 7.75 (s, 1H), 6.60 (s, 0.2H), 6.36 (s, 0.8H), 4.62 - 4.32 (m, 1H), 3.86 - 3.49 (m, 3H), 3.25 - 2.76 (m, 6H), 2.32 - 2.12 (m, 2H), 2.01-1.84 (m, 1H), 1.08 - 0.93 (m, 1H), 0.92 - 0.76 (m, 4H), 0.43 - 0.30 (m, 2H), 0.22 - 0.10 (m, 2H).

### EXAMPLE 222

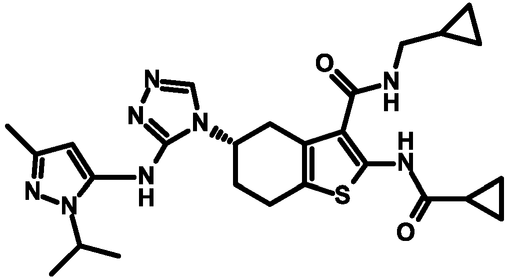

### (5S)-2-(cyclopropanecarbonylamino)-N-(cyclopropylmethyl)-5-[3-[(2-isopropyl-5-methyl-pyrazol-3-yl)amino]-1,2,4-triazol-4-yl]-4,5,6,7-tetrahydrobenzothiophene-3-carboxamide

To a stirred solution of *intermediate 234* (385.9 mg, 0.7498 mmol) in DMF (5.3 mL) under nitrogen was added formic acid hydrazide [624-84-0] (136.1 mg, 2.266 mmol) and mercuric chloride [7487-94-7] (615.2 mg, 2.255 mmol). The reaction mixture was stirred at r.t. for 5 min before the addition of triethylamine (0.31 mL, 2.2 mmol) and the mixture was stirred at 80 °C for 5 h and then at 60 °C for a further 17 h. The mixture was cooled to r.t. and diluted with MeCN (20 mL), filtered through a pad of celite and washed with MeCN (25 mL). The filtrate was concentrated *in vacuo* to yield the crude product as an orange oil that solidified upon standing which was dissolved in 5% MeOH in DCM (50 mL), washed with water (30 mL) and the layers separated. The aqueous layer was extracted with 5% MeOH in DCM (2 x 25 mL). The combined organic layers were passed through a phase separation cartridge and concentrated *in vacuo* to give an orange oil. This oil was purified by flash column chromatography on silica (gradient elution with 0% to 20% MeOH in DCM) to afford impure *title compound* as a pale yellow glass (196.0 mg). The impure product was dissolved in a mixture of MeOH (3 mL) and MeCN (1.0 mL), QuadraSil^{®} MTU (200 mg) was added and the suspension was stirred at 40 °C for 2 h before being filtered and washed with MeOH (2 x 5 mL). The filtrate was concentrated *in vacuo* to give a pale yellow oil which was further purified by flash column chromatography on silica (gradient elution with 0% to 20% MeOH in EtOAc) and the product containing fractions were concentrated then the material was freeze dried to afford the *title compound* (96.4 mg, 0.184 mmol, 25%) as a white solid. LCMS (ES+) [M+H]+ 523.0, RT 1.675 minutes, purity 96.6% (Method 2). LCMS (ES+) [M+H]+ 523.0, RT 1.742 minutes, purity 96.1% (Method 3). 1H NMR (400 MHz, DMSO-d6) (contained 2 tautomers in an approximate ratio of 0.6:0.4) δ 11.71 (s, 0.4H), 11.23 (s, 0.6H), 11.20 (s, 0.4H), 8.33 (s, 0.6H), 8.27 (s, 0.6H), 8.10 (s, 0.4H), 7.74 (s, 1H), 5.84 (s, 0.6H), 5.69 (s, 0.4H), 4.71 - 4.62 (m, 0.4H), 4.48 - 4.30 (m, 1.6H), 3.24 - 3.05 (m, 3H), 3.04 - 2.90 (m, 1H), 2.87 - 2.78 (m, 2H), 2.27 - 2.14 (m, 2H), 2.11 (s, 1.8H), 2.07 (s, 1.2H), 1.99 - 1.87 (m, 1H), 1.29 (d, J = 6.6 Hz, 3.6H), 1.25 (d, J = 6.7 Hz, 1.2H), 1.22 (d, J = 6.6 Hz, 1.2H), 1.07 - 0.94 (m, 1H), 0.93 - 0.79 (m, 4H), 0.41 - 0.32 (m, 2H), 0.23 - 0.13 (m, 2H).

### EXAMPLE 223

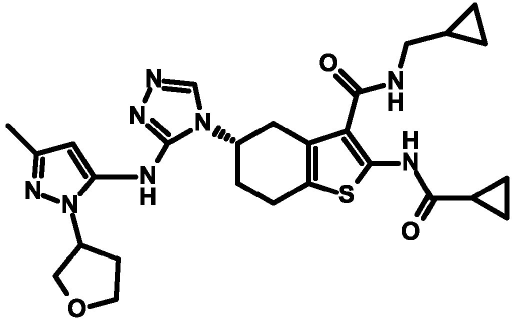

### (5S)-2-(cyclopropanecarbonylamino)-N-(cyclopropylmethyl)-5-[3-[(5-methyl-2-tetrahydrofuran-3-yl-pyrazol-3-yl)amino]-1,2,4-triazol-4-yl]-4,5,6,7-tetrahydrobenzothiophene-3-carboxamide

To a stirred solution of *intermediate 236* (385.4 mg, 0.7102 mmol) in DMF (5.0 mL) under nitrogen was added formic acid hydrazide [624-84-0] (130.1 mg, 2.166 mmol) and mercuric chloride [7487-94-7] (595.1 mg, 2.181 mmol). The reaction mixture was stirred at r.t. for 5 min before the addition of triethylamine (0.30 mL, 2.1 mmol) and the mixture was stirred at 80 °C for 5 h and then at 60 °C for a further 18 h. The mixture was cooled to r.t. and diluted with MeCN (20 mL), filtered through a pad of celite and washed with MeCN (25 mL). The filtrate was concentrated *in vacuo* to yield the crude product as an orange oil that solidified upon standing which was dissolved in 5% MeOH in DCM (50 mL), washed with water (30 mL) and the layers separated. The Aq layer was extracted with 5% MeOH in DCM (2 x 25 mL). The combined organic layers were passed through a phase separation cartridge and concentrated *in vacuo* to give an orange oil. This oil was purified by flash column chromatography on silica (gradient elution with 0% to 20% MeOH in DCM) to afford impure *title compound* as a pale yellow glass (190.6 mg). The impure product was dissolved in a mixture of MeOH (3 mL) and MeCN (1.0 mL), QuadraSil^{®} MTU (200 mg) was added and the suspension was stirred at 40 °C for 2 h before being filtered and washed with MeOH (2 x 5 mL). The filtrate was concentrated *in vacuo* to give a very pale yellow solid which was further purified by flash column chromatography on silica (gradient elution with 0% to 20% MeOH in EtOAc) and the product containing fractions were concentrated then the material was freeze dried to afford the *title compound* (104.0 mg, 0.1889 mmol, 27%) as a white solid. LCMS (ES+) [M+H]+ 551.0, RT 1.613 minutes, purity 98.2% (Method 2). LCMS (ES+) [M+H]+ 551.0, RT 1.646 minutes, purity 96.6% (Method 3). 1H NMR (400 MHz, DMSO-d6) (contained 2 tautomers in an approximate ratio of 0.5:0.5) δ 11.79 (s, 0.5H), 11.24 (s, 0.5H), 11.20 (d, J = 5.9 Hz, 0.5H), 8.40 (s, 0.5H), 8.35 (s, 0.5H), 8.13 (d, J = 2.0 Hz, 0.5H), 7.80 - 7.67 (m, 1H), 5.91 (d, J = 1.5 Hz, 0.5H), 5.74 (s, 0.5H), 5.16 - 5.02 (m, 0.5H), 4.94 - 4.82 (m, 0.5H), 4.49 - 4.33 (m, 1H), 4.04 - 3.83 (m, 2H), 3.81 - 3.61 (m, 2H), 3.23 - 3.05 (m, 3H), 3.04 - 2.77 (m, 3H), 2.33 - 2.13 (m, 4H), 2.12 (s, 1.5H), 2.08 (s, 1.5H), 2.00 - 1.85 (m, 1H), 1.07 - 0.95 (m, 1H), 0.93 - 0.76 (m, 4H), 0.43 - 0.31 (m, 2H), 0.26 - 0.14 (m, 2H).

### EXAMPLE 224

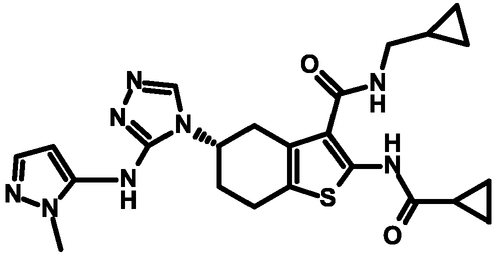

### (5S)-2-(cyclopropanecarbonylamino)-N-(cyclopropylmethyl)-5-[3-[(2-methylpyrazol-3-yl)aminol-1,2,4-triazol-4-yl]-4,5,6,7-tetrahydrobenzothiophene-3-carboxamide

To a stirred solution of *intermediate 238* (1.3368 g, 2.829 mmol) in DMF (20 mL) under nitrogen was added formic acid hydrazide [624-84-0] (550.4 mg, 9.164 mmol) and mercuric chloride [7487-94-7] (1.201 g, 4.402 mmol). The reaction mixture was stirred at r.t. for 20 min before the addition of triethylamine (1.2 mL, 8.6 mmol) and the mixture was stirred at 80 °C for 4 h. The mixture was cooled to r.t. and diluted with MeCN (50 mL), filtered through a pad of celite and washed with MeCN (40 mL). The filtrate was concentrated *in vacuo* to yield the crude product as a grey oil. This grey oil was purified by flash column chromatography on silica (dry loaded, gradient elution with 0% to 20% MeOH in DCM) to afford impure *title compound* as a pale yellow oil (1.8369 g). This oil was dissolved in 5% MeOH in DCM (150 mL), washed with water (125mL) and the layers separated. The Aq layer was extracted with 5% MeOH in DCM (2 x 50 mL). The combined organic layers were passed through a phase separation cartridge and concentrated *in vacuo* to give a pale yellow oil (1.0345 g) that solidified upon standing. The impure product was dissolved in MeOH (20 mL), QuadraSil^{®} MTU (2.0 g) was added and the suspension was stirred at 40 °C for 3.5 h before being filtered and washed with MeOH (2 x 10 mL). The filtrate was concentrated *in vacuo* to give a colourless oil which was further purified by flash column chromatography on silica (gradient elution with 0% to 20% MeOH in EtOAc) and the product containing fractions were concentrated then the material was freeze dried to afford the *title compound* (801.0 mg, 1.667 mmol, 59%) as a white solid. LCMS (ES+) [M+H]+ 481.0, RT 1.473 minutes, purity 99% (Method 2). LCMS (ES+) [M+H]+ 481.0, RT 1.495 minutes, purity 99% (Method 3). 1H NMR (300 MHz, DMSO-d6) (contained 2 tautomers in an approximate ratio of 0.6:0.4) δ 11.83 (s, 0.4H), 11.25 (s, 0.6H), 11.21 (s, 0.4H), 8.51 (s, 0.6H), 8.36 (s, 0.6H), 8.15 (s, 0.4H), 7.83 - 7.63 (m, 1H), 7.30 (d, J = 1.9 Hz, 0.6H), 7.19 (d, J = 2.0 Hz, 0.4H), 6.11 (d, J = 1.9 Hz, 0.6H), 5.91 (d, J = 2.0 Hz, 0.4H), 4.53 - 4.34 (m, 1H), 3.65 (s, 1.8H), 3.57 (s, 1.2H), 3.25 - 2.77 (m, 6H), 2.27 - 2.11 (m, 2H), 1.99 - 1.88 (m, 1H), 1.08 - 0.94 (m, 1H), 0.92 - 0.78 (m, 4H), 0.43 - 0.31 (m, 2H), 0.25 - 0.10 (m, 2H).

### EXAMPLE 225

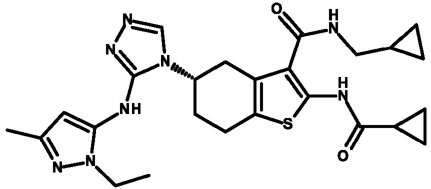

### (5S)-2-(cyclopropanecarbonylamino)-N-(cyclopropylmethyl)-5-[3-[(2-ethyl-5-methyl-pyrazol-3-yl)amino]-1,2,4-triazol-4-yl]-4,5,6,7-tetrahydrobenzothiophene-3-carboxamide

To a stirred solution of *intermediate 251* (303 mg, 0.605 mmol) in DMF (10 mL) under nitrogen was added formic acid hydrazide [624-84-0] (121 mg, 1.82 mmol) and mercuric chloride [7487-94-7] (493 mg, 1.82 mmol). The reaction mixture was stirred at r.t. for 15 min before the addition of triethylamine (0.25 mL, 1.82 mmol) and the mixture was stirred at 60 °C for 4 h. The mixture was cooled to r.t. and diluted with MeCN (10 mL), filtered through a pad of celite and washed with MeCN (15 mL). The filtrate was concentrated *in vacuo* to yield the crude product as a yellow gum which was dissolved in DCM (50 mL), washed with water (30 mL) and the layers separated. The Aq layer was extracted with DCM (2 x 15 mL). The combined organic layers were washed with brine (30 ml) and passed through a phase separation cartridge and concentrated *in vacuo* to give a yellow gum. This was purified by flash column chromatography on silica (gradient elution with 1% to 20% MeOH in DCM) to afford impure *title compound* as a pale yellow gum. This was dissolved in MeOH (10 mL) and QuadraSil^{®} MTU (130 mg) was added and the suspension was stirred at 50 °C for 2 h before being filtered and washed with MeOH (2 x 10 mL). The filtrate was concentrated *in vacuo* to give a colourless gum. This was further purified by flash column chromatography on silica (gradient elution with 5% to 25% MeOH in EtOAc) product containing fractions were freeze dried to afford the *title compound* (83 mg, 0.163 mmol, 27%) as a white solid. LCMS (ES+) [M+H]⁺ 509.0, RT 1.63 minutes, purity 100% *(Method 3).* LCMS (ES+) [M+H]⁺ 509.0, RT 1.60 minutes, purity 100% *(Method 2).* 1H NMR (300 MHz, DMSO-d6) (contained 2 tautomers in an approximate ratio of 0.6:0.4) δ 11.73 (s, 0.4H), 11.36 - 11.08 (m, 1H), 8.43 - 8.25 (m, 1.2H), 8.11 (s, 0.4H), 7.86 - 7.62 (m, 1H), 5.87 (s, 0.6H), 5.70 (s, 0.4H), 4.51 - 4.23 (m, 1H), 3.90 (q, J = 7.2 Hz, 2H), 3.24 - 2.76 (m, 5.6H), 2.32 - 2.14 (m, 2H), 2.10 (s, 2H), 2.06 (s, 1H), 2.00 - 1.87 (m, 1H), 1.29 - 1.13 (m, 3H), 1.07 - 0.94 (m, 0.3H), 0.93 - 0.79 (m, 4H), 0.42 - 0.30 (m, 2H), 0.24 - 0.15 (m, 2H).

### EXAMPLE 226

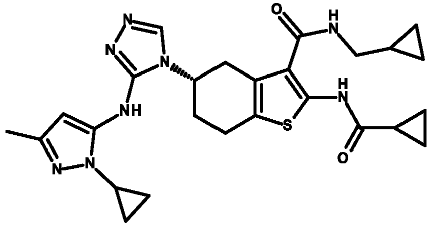

### (5S)-2-(cyclopropanecarbonylamino)-N-(cyclopropylmethyl)-5-[3-[(2-cyclopropyl-5-methyl-pyrazol-3-yl)amino]-1,2,4-triazol-4-yl]-4,5,6,7-tetrahydrobenzothiophene-3-carboxamide

To a stirred solution of *intermediate 253* (292 mg, 0.570 mmol) in DMF (10 mL) under nitrogen was added formic acid hydrazide [624-84-0] (114 mg, 1.71 mmol) and mercuric chloride [7487-94-7] (464 mg, 1.71 mmol). The reaction mixture was stirred at r.t. for 15 min before the addition of triethylamine (0.24 mL, 1.71 mmol) and the mixture was stirred at 60 °C for 4 h. The mixture was cooled to r.t. and diluted with MeCN (10 mL), filtered through a pad of celite and washed with MeCN (15 mL). The filtrate was concentrated *in vacuo* to yield the crude product as a yellow gum which was dissolved in DCM (50 mL), washed with water (30 mL) and the layers separated. The Aq layer was extracted with DCM (2 x 15 mL). The combined organic layers were washed with brine (30 ml) and passed through a phase separation cartridge and concentrated *in vacuo* to give a yellow gum. This was purified by flash column chromatography on silica (gradient elution with 1% to 20% MeOH in DCM) to afford impure *title compound* as a pale yellow gum. This was dissolved in MeOH (10 mL) and QuadraSil^{®} MTU (130 mg) was added and the suspension was stirred at 50 °C for 2 h before being filtered and washed with MeOH (2 x 10 mL). The filtrate was concentrated *in vacuo* to give a gum. This was further purified by flash column chromatography on silica (gradient elution with 5% to 25% MeOH in EtOAc) product containing fractions were freeze dried to afford the *title compound* (46 mg, 0.088 mmol, 16%) as a white solid. LCMS (ES+) [M+H]⁺ 521.0, RT 1.65 minutes, purity 100% *(Method* 3). LCMS (ES+) [M+H]⁺ 521.0, RT 1.61 minutes, purity 100% *(Method 2).* 1H NMR (300 MHz, DMSO-d6) (contained 2 tautomers in an approximate ratio of 0.7:0.3) δ 11.75 (s, 0.3H), 11.34 - 11.04 (m, 1H), 8.45 (s, 0.7H), 8.35 (s, 0.7H), 8.13 (s, 0.3H), 7.84 - 7.62 (m, 1H), 5.89 (s, 0.7H), 5.69 (s, 0.3H), 4.60 - 4.29 (m, 1H), 3.25 - 2.76 (m, 6H), 2.30 - 2.15 (m, 2H), 2.06 (s, 2H), 2.03 (s, 1H), 1.99 - 1.83 (m, 1H), 1.07 - 0.69 (m, 8H), 0.42 - 0.33 (m, 2H), 0.25 - 0.15 (m, 2H).

### EXAMPLE 227

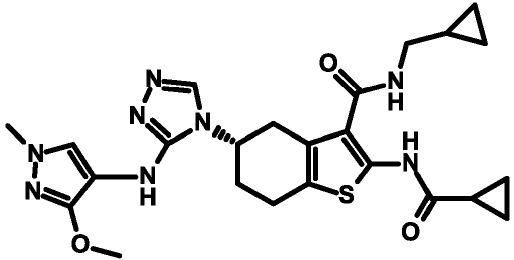

### (5S)-2-(cyclopropanecarbonylamino)-N-(cyclopropylmethyl)-5-[3-[(3-methoxy-1-methyl-pyrazol-4-yl)amino]-1,2,4-triazol-4-yl]-4,5,6,7-tetrahydrobenzothiophene-3-carboxamide

To a stirred solution of *intermediate* 240(376.0 mg, 0.7481 mmol) in DMF (5.2 mL) under nitrogen was added formic acid hydrazide [624-84-0] (134.0 mg, 2.231 mmol) and mercuric chloride [7487-94-7] (640.5 mg, 2.347 mmol). The reaction mixture was stirred at r.t. for 5 min before the addition of triethylamine (0.31 mL, 2.2 mmol) and the mixture was stirred at 80 °C for 2.5 h. The mixture was cooled to r.t. and diluted with MeCN (20 mL), filtered through a pad of celite and washed with MeCN (25 mL). The filtrate was concentrated *in vacuo* to yield the crude product as a yellow oil that solidified upon standing which was dissolved in 5% MeOH in DCM (50 mL), washed with water (30 mL) and the layers separated. The Aq layer was extracted with 5% MeOH in DCM (2 x 25 mL). The combined organic layers were passed through a phase separation cartridge and concentrated in *vacuo* to give a yellow oil. This oil was purified by flash column chromatography on silica (gradient elution with 0% to 20% MeOH in DCM) to afford impure *title compound* as a colourless oil (59.5 mg). The impure product was dissolved in MeOH (4 mL), QuadraSil^{®} MTU (250 mg) was added and the suspension was stirred at 40 °C for 0.5 h before being filtered and washed with MeOH (2 x 5 mL). The filtrate was concentrated *in vacuo* to give a colourless oil which was further purified by flash column chromatography on silica (gradient elution with 0% to 20% MeOH in EtOAc) and the product containing fractions were concentrated then the material was freeze dried to afford the *title compound* (27.7 mg, 0.0542 mmol, 7%) as a white solid. LCMS (ES+) [M+H]+ 511.0, RT 1.404 minutes, purity 100% (Method 2). LCMS (ES+) [M+H]+ 511.0, RT 1.541 minutes, purity 100% (Method 3). 1H NMR (400 MHz, DMSO-d6) δ 11.23 (s, 1H), 8.20 (s, 1H), 7.80 (s, 1H), 7.78 (s, 1H), 7.72 (t, J = 5.7 Hz, 1H), 4.56 - 4.40 (m, 1H), 3.80 (s, 3H), 3.66 (s, 3H), 3.21 - 3.02 (m, 3H), 2.98 - 2.74 (m, 3H), 2.27 - 2.03 (m, 2H), 2.03 - 1.82 (m, 1H), 1.08 - 0.94 (m, 1H), 0.93 - 0.77 (m, 4H), 0.46 - 0.29 (m, 2H), 0.26 - 0.11 (m, 2H).

### EXAMPLE 228

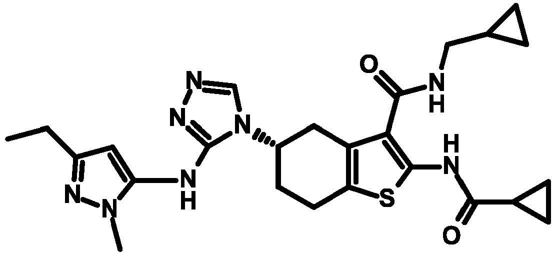

### (5S)-2-(cyclopropanecarbonylamino)-N-(cyclopropylmethyl)-5-[3-[(5-ethyl-2-methyl-pyrazol-3-yl)amino]-1,2,4-triazol-4-yl]-4,5,6,7-tetrahydrobenzothiophene-3-carboxamide

To a stirred solution of *intermediate 243* (327.6 mg, 0.6543 mmol) in DMF (4.6 mL) under nitrogen was added formic acid hydrazide [624-84-0] (140.1 mg, 2.333 mmol) and mercuric chloride [7487-94-7] (562.3 mg, 2.061 mmol). The reaction mixture was stirred at r.t. for 5 min before the addition of triethylamine (0.27 mL, 1.9 mmol) and the mixture was stirred at 80 °C for 4 h. The mixture was cooled to r.t. and diluted with MeCN (20 mL), filtered through a pad of celite and washed with MeCN (25 mL). The filtrate was concentrated *in vacuo* to yield the crude product a yellow oil which was dissolved in 5% MeOH in DCM (50 mL), washed with water (30 mL) and the layers separated. The Aq layer was extracted with 5% MeOH in DCM (2 x 25 mL). The combined organic layers were passed through a phase separation cartridge and concentrated *in vacuo* to give a yellow oil. This oil was purified by flash column chromatography on silica (gradient elution with 0% to 20% MeOH in DCM) to afford impure *title compound* as a grey oil (229.8 mg). The impure product was dissolved in MeOH (4 mL), QuadraSil^{®} MTU (500 mg) was added and the suspension was stirred at 40 °C for 0.5 h before being filtered and washed with MeOH (2 x 5 mL). The filtrate was concentrated *in vacuo* to give a grey oil which was further purified by flash column chromatography on silica (gradient elution with 0% to 20% MeOH in EtOAc) and the product containing fractions were concentrated then the material was freeze dried to afford the *title compound* (157.3 mg, 0.3093 mmol, 47%) as a white solid. LCMS (ES+) [M+H]+ 509.0, RT 1.644 minutes, purity 99% (Method 2). LCMS (ES+) [M+H]+ 509.0, RT 1.680 minutes, purity 99% (Method 3). 1H NMR (300 MHz, DMSO-d6) (contained 2 tautomers in an approximate ratio of 0.7:0.3) δ 11.75 (s, 0.3H), 11.25 (s, 0.7H), 11.20 (s, 0.3H), 8.44 (s, 0.7H), 8.35 (s, 0.7H), 8.12 (s, 0.3H), 7.81 - 7.65 (m, 1H), 5.93 (s, 0.7H), 5.76 (s, 0.3H), 4.53 - 4.33 (m, 1H), 3.57 (s, 2.1H), 3.50 (s, 0.9H), 3.32 (s, 3H), 3.24 - 2.75 (m, 6H), 2.48 - 2.37 (m, 2H), 2.31 - 2.10 (m, 2H), 1.98 - 1.85 (m, 1H), 1.20 - 1.08 (m, 3H), 1.07 - 0.93 (m, 1H), 0.93 - 0.77 (m, 4H), 0.43 - 0.31 (m, 2H), 0.23 - 0.14 (m, 2H).

### EXAMPLE 229

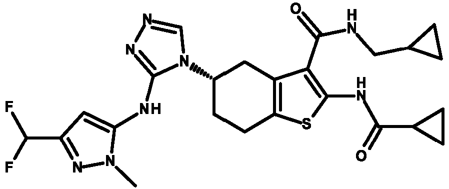

### (5S)-2-(Cyclopropanecarbonylamino)-N-(cyclopropylmethyl)-5-[3-[[5-(difluoromethyl)-2-methyl-pyrazol-3-yl]amino]-1,2,4-triazol-4-yl]-4,5,6,7-tetrahydrobenzothiophene-3-carboxamide

To a stirred solution of *intermediate* 255 (1.08 g, 2.07 mmol) in DCM (15 mL) at 0 °C under nitrogen was added triethylamine (0.865 mL, 6.19 mmol) and methanesulfonyl chloride [124-63-0] (0.17 ml, 2.19 mmol). The reaction mixture was stirred at 0 °C for 1 h then transferred to a separating funnel, diluting with DCM (80ml), washed with saturated NH4Cl solution (50 ml) and brine (50 ml). The organic layer was passed through a phase separation cartridge and concentrated *in vacuo* to give a yellow gum. This was dissolved in DMF (10ml) under nitrogen and formic acid hydrazide [624-84-0] (372 mg, 6.19 mmol) added. The reaction mixture was stirred at r.t. for 30 min. Sodium carbonate (481 mg, 4.54 mmol) was added and the mixture stirred at 45°C under nitrogen for 16 h then allowed to cool to room temperature. Sodium carbonate (240 mg, 2.27 mmol) as a solution in water (2 ml) was added and the mixture stirred at 55°C under nitrogen for 5 h. The mixture was cooled to r.t. and concentrated *in vacuo.* The residue was dissolved in 5% MeOH/DCM (100 mL), washed with water (50 mL) and the layers separated. The Aq layer was extracted with DCM (2 x 25 mL). The combined organic layers were washed with brine (50 ml) and passed through a phase separation cartridge and concentrated *in vacuo* to give a brown gum. This was purified by flash column chromatography on silica (gradient elution with 1% to 10% MeOH in DCM) to afford impure *title compound* as an off-white solid. This was further purified by flash column chromatography on silica (gradient elution with 60% to 100% EtOAc in isohexane) product containing fractions were freeze dried to afford the *title compound* (613 mg, 1.16 mmol, 56%) as a white solid. LCMS (ES+) [M+H]⁺ 531.0, RT 1.92 minutes, purity 99% *(Method 3).* LCMS (ES+) [M+H]⁺ 531.0, RT 1.86 minutes, purity *99% (Method 2).* 1H NMR (300 MHz, DMSO-d6) (contained 2 tautomers in an approximate ratio of 0.6:0.4) δ 12.08 (s, 0.6H), 11.32 - 11.12 (m, 1H), 8.75 (s, 0.4H), 8.41 (s, 0.4H), 8.20 (s, 0.6H), 7.84 - 7.65 (m, 1H), 7.07 - 6.50 (m, 1H), 6.41 (s, 0.4H), 6.20 (s, 0.6H), 4.47 (dt, J = 9.9, 5.0 Hz, 0.6H), 3.71 (s, 1H), 3.61 (s, 2H), 3.24 - 2.75 (m, 4H), 2.33 - 2.12 (m, 3H), 2.02 - 1.86 (m, 1H), 1.08 - 0.94 (m, 1H), 0.92 - 0.78 (m, 5H), 0.35 (dq, J = 7.7, 3.5, 2.7 Hz, 2H), 0.24 - 0.11 (m, 2H).

### EXAMPLE 230

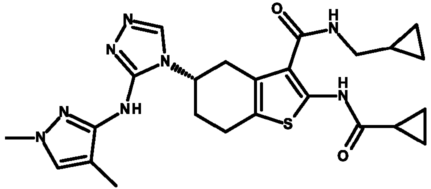

### (5S)-2-(Cyclopropanecarbonylamino)-N-(cyclopropylmethyl)-5-[3-[(1,4-dimethylpyrazol-3-yl)amino]-1,2,4-triazol-4-yl]-4,5,6,7-tetrahydrobenzothiophene-3-carboxamide

To a stirred solution of *intermediate* 257 (288 mg, 0.592 mmol) in DMF (10 mL) under nitrogen was added formic acid hydrazide [624-84-0] (119 mg, 1.78 mmol) and mercuric chloride [7487-94-7] (241 mg, 0.888 mmol). The reaction mixture was stirred at r.t. for 15 min before the addition of triethylamine (0.25 mL, 1.78 mmol) and the mixture was stirred at 60 °C for 16 h. The mixture was cooled to r.t. and diluted with MeCN (10 mL), filtered through a pad of celite and washed with MeCN (15 mL). The filtrate was concentrated *in vacuo* to yield the crude product as a yellow gum which was dissolved in DCM (50 mL), washed with water (30 mL) and the layers separated. The Aq layer was extracted with DCM (2 x 15 mL). The combined organic layers were washed with brine (30 ml) and passed through a phase separation cartridge and concentrated *in vacuo* to give a yellow gum. This was purified by flash column chromatography on silica (gradient elution with 2% to 20% MeOH in DCM) to afford impure *title compound* as a white solid. This was dissolved in MeOH (10 mL) and QuadraSil^{®} MTU (130 mg) was added and the suspension was stirred at 40 °C for 1 h before being filtered and washed with MeOH (2 x 10 mL). The filtrate was concentrated *in vacuo* to give a gum. This was further purified by flash column chromatography on silica (gradient elution with 4% to 20% MeOH in EtOAc) product containing fractions were freeze dried to afford the *title compound* (46 mg, 0.093 mmol, 16%) as a white solid. LCMS (ES+) [M+H]⁺ 495.0, RT 1.63 minutes, purity 100% *(Method* 3). LCMS (ES+) [M+H]⁺ 495.0, RT 1.52 minutes, purity 100% *(Method 2).* 1H NMR (300 MHz, DMSO-d6) δ 11.17 (s, 1H), 8.31 (s, 1H), 8.17 (s, 1H), 7.76 (t, J = 5.6 Hz, 1H), 7.31 (s, 1H), 4.38 - 4.20 (m, 1H), 3.63 (s, 3H), 3.21 - 2.70 (m, 6H), 2.22 - 2.10 (m, 2H), 2.00 - 1.85 (m, 1H), 1.79 (s, 3H), 1.08 - 0.96 (m, 1H), 0.90 - 0.80 (m, 4H), 0.43 - 0.34 (m, 2H), 0.26 - 0.15 (m, 2H).

### EXAMPLE 231

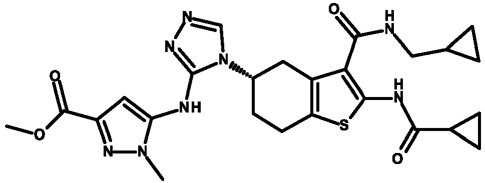

### Methyl 5-[[4-[(5S)-2-(cyclopropanecarbonylamino)-3-(cyclopropylmethylcarbamoyl)-4,5,6,7-tetrahydrobenzothiophen-5-yl]-1,2,4-triazol-3-yl]amino]-1-methyl-pyrazole-3-carboxylate

To a stirred solution of *intermediate 261* (261 mg, 0.492 mmol) in DMF (10 mL) under nitrogen was added formic acid hydrazide [624-84-0] (98 mg, 1.48 mmol) and mercuric chloride [7487-94-7] (200 mg, 0.738 mmol). The reaction mixture was stirred at r.t. for 15 min before the addition of triethylamine (0.21 mL, 1.48 mmol) and the mixture was stirred at 80 °C for 16 h. The mixture was cooled to r.t. and diluted with MeCN (10 mL), filtered through a pad of celite and washed with MeCN (15 mL). The filtrate was concentrated *in vacuo* to yield the crude product as a yellow gum which was dissolved in DCM (50 mL), washed with water (30 mL) and the layers separated. The Aq layer was extracted with DCM (2 x 15 mL). The combined organic layers were washed with brine (30 ml) and passed through a phase separation cartridge and concentrated *in vacuo* to give a yellow gum. This was purified by flash column chromatography on silica (gradient elution with 2% to 20% MeOH in DCM) to afford impure *title compound* as a yellow gum. This was dissolved in MeOH (10 mL) and QuadraSil^{®} MTU (130 mg) was added and the suspension was stirred at 40 °C for 1 h before being filtered and washed with MeOH (2 x 10 mL). The filtrate was concentrated *in vacuo* to give a gum. This was further purified by flash column chromatography on silica (gradient elution with 4% to 20% MeOH in EtOAc) product containing fractions were freeze dried to afford the *title compound* (134 mg, 0.248 mmol, 50%) as a white solid. LCMS (ES+) [M+H]⁺ 539.0, RT 1.63 minutes, purity 100% *(Method 3).* LCMS (ES+) [M+H]⁺ 539.0, RT 1.71 minutes, purity 100% *(Method 2).* 1H NMR (300 MHz, DMSO-d6) (contained 2 tautomers in an approximate ratio of 0.7:0.3) δ 12.14 (s, 0.7H), 11.38 - 11.09 (m, 1H), 8.78 (s, 0.3H), 8.41 (s, 0.3H), 8.20 (s, 0.7H), 7.85 - 7.64 (m, 1H), 6.67 (s, 0.3H), 6.48 (s, 0.7H), 4.60 - 4.30 (m, 1H), 3.83 - 3.73 (m, 3.9H), 3.66 (s, 2.1H), 3.24 - 2.77 (m, 6H), 2.32 - 2.12 (m, 2H), 2.00 - 1.86 (m, 1H), 1.08 - 0.94 (m, 1H), 0.93 - 0.79 (m, 4H), 0.42 - 0.30 (m, 2H), 0.23 - 0.14 (m, 2H).

### EXAMPLE 232

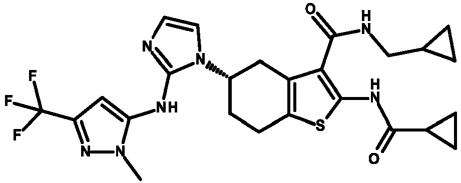

### (5S)-2-(Cyclopropanecarbonylamino)-N-(cyclopropylmethyl)-5-[2-[[2-methyl-5-(trifluoromethyl)pyrazol-3-yl]aminolimidazol-1-yl]-4,5,6,7-tetrahydrobenzothiophene-3-carboxamide

To a stirred solution of *intermediate 263* (148 mg, 0.274 mmol) in DMF (10 mL) under nitrogen was added aminoacetaldehyde diethyl acetal [645-36-3] (109 mg, 0.821 mmol) and mercuric chloride [7487-94-7] (112 mg, 0.411 mmol). The reaction mixture was stirred at r.t. for 15 min before the addition of triethylamine (0.11 mL, 0.821 mmol) and the mixture was stirred at 90 °C for 16 h. p-Toluenesulfonic acid monohydrate (319 mg, 1.64 mmol) was added and the mixture was stirred at 90 °C for 7 h. An additional amount of p-toluenesulfonic acid monohydrate (160 mg, 0.820 mmol) was added and the mixture was stirred at 90 °C for a further 16 h. The mixture was cooled to r.t. and diluted with MeCN (10 mL), filtered through a pad of celite and washed with MeCN (15 mL). The filtrate was concentrated *in vacuo* to yield the crude product as a yellow gum which was dissolved in DCM (50 mL), washed with water (30 mL) and the layers separated. The Aq layer was extracted with DCM (2 x 15 mL). The combined organic layers were washed with brine (30 ml) and passed through a phase separation cartridge and concentrated *in vacuo* to give a yellow gum. This was purified by flash column chromatography on silica (gradient elution with 2% to 20% MeOH in DCM) to afford impure *title compound* as a yellow gum. This was dissolved in MeOH (10 mL) and QuadraSil^{®} MTU (130 mg) was added and the suspension was stirred at 40 °C for 1 h before being filtered and washed with MeOH (2 x 10 mL). The filtrate was concentrated *in vacuo* to give a gum. This was further purified by flash column chromatography on silica (gradient elution with 4% to 20% MeOH in EtOAc) product containing fractions were freeze dried to afford the *title compound* (22 mg, 0.040 mmol, 15%) as a white solid. LCMS (ES+) [M+H]⁺ 548.0, RT 2.25 minutes, purity 100% *(Method 3).* LCMS (ES+) [M+H]⁺ 548.0, RT 1.84 minutes, purity 100% *(Method 2).* 1H NMR (300 MHz, DMSO-*d*₆) ) (contained 2 tautomers in an approximate ratio of 0.7:0.3) δ 11.25 - 11.10 (m, 1H), 10.37 (s, 0.7H), 8.52 (s, 0.3H), 7.83 - 7.67 (m, 1H), 7.05 (s, 0.3H), 6.83 (s, 0.7H), 6.75 - 6.60 (m, 1H), 6.39 (s, 0.3H), 6.19 (s, 0.7H), 4.71 - 4.37 (m, 1H), 3.71 (s, 0.9H), 3.60 (s, 2.1H), 3.22 - 2.76 (m, 6H), 2.24 - 2.03 (m, 2H), 1.99 - 1.87 (m, 1H), 1.08 - 0.92 (m, 1H), 0.92 - 0.81 (m, 4H), 0.43 - 0.30 (m, 2H), 0.24 - 0.13 (m, 2H).

### EXAMPLE 233

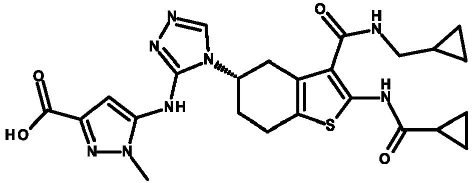

### 5-[[4-[(5S)-2-(Cyclopropanecarbonylamino)-3-(cyclopropylmethylcarbamoyl)-4,5,6,7-tetrahydrobenzothiophen-5-yl]-1,2,4-triazol-3-yl]amino]-1-methyl-pyrazole-3-carboxylic acid

To a stirred solution of *example 231* (88 mg, 0.163 mmol) in 1,4-dioxane (10 mL) was added a solution of lithium hydroxide monohydrate [1310-66-3] (21 mg, 0.490 mmol) in water (2 mL). The reaction mixture was stirred at r.t. for 5 h and then concentrated *in vacuo.* The residue was dissolved in DCM (50 mL) and water (50 mL) and the layers separated. The Aq layer was washed with DCM (2 x 15 mL). The Aq layer was acidified to pH 1 by addition of 0.5M HCl (aq) and then extracted with EtOAc (4 x 30 mL). The combined organic layers were washed with brine (30 ml) and passed through a phase separation cartridge and concentrated *in vacuo* to give a white solid. This was purified by flash column chromatography on silica (gradient elution with 5% to 25% MeOH in DCM) to afford impure *title compound* as a white solid. This solid was further purified by reverse phase chromatography on C18 silica (5% to 100% MeCN (w/ 0.1% NH3) in water (w/ 0.1% NH3)) and the product containing fractions were freeze dried to afford the *title compound* (20 mg, 0.038 mmol, 23%) as a white solid. LCMS (ES+) [M+H]⁺ 525.0, RT 1.18 minutes, purity 100% *(Method 3).* LCMS (ES+) [M+H]⁺ 525.0, RT 1.50 minutes, purity 100% *(Method 2).* 1H NMR (300 MHz, DMSO-d6) (contained 2 tautomers in an approximate ratio of 0.7:0.3) δ 12.25 (brs, 1H), 12.09 (s, 0.7H), 11.23 (s, 1H), 8.72 (s, 0.3H), 8.40 (s, 0.3H), 8.19 (s, 0.7H), 7.90 - 7.57 (m, 1H), 6.58 (s, 0.3H), 6.42 (s, 0.7H), 4.62 - 4.25 (m, 1H), 3.74 (s, 0.9H), 3.65 (s, 2.1H), 3.25 - 2.77 (m, 6H), 2.31 - 2.11 (m, 2H), 1.99 - 1.86 (m, 1H), 1.10 - 0.94 (m, 1H), 0.93 - 0.80 (m, 4H), 0.42 - 0.31 (m, 2H), 0.25 - 0.13 (m, 2H).

### Example 234

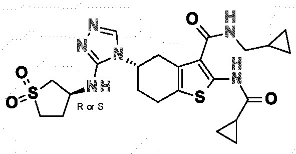

### (5S)-2-(cyclopropanecarbonylamino)-N-(cyclopropylmethyl)-5-[3-[[(3S*)-1,1-dioxothiolan-3-yl]aminol]-1,2,4-triazol-4-yl]-4,5,6,7-tetrahydrobenzothiophene-3-carboxamide [*or R]

**Intermediate 267** (140 mg, 0.27 mmol) was purified *via* chiral chromatography (Ethanol with Cellulose-4 25 cm column at 6 mL/min) to give the title compound (21 mg, 15% yield). δ_{H} (500 MHz, DMSO-*d*₆) 11.28 (s, 1H), 8.17 (s, 1H), 7.74 (s, 1H), 6.45 (d, J = 6.3 Hz, 1H), 4.47 - 4.37 (m, 1H), 4.27 - 4.19 (m, 1H), 3.54 (dd, J = 13.4, 7.5 Hz, 1H), 3.35 - 3.29 (m, 1H), 3.21 - 3.13 (m, 2H), 3.12 - 2.99 (m, 3H), 2.91 - 2.86 (m, 1H), 2.86 - 2.79 (m, 2H), 2.48 - 2.44 (m, 1H), 2.28 - 2.07 (m, 3H), 1.95 - 1.87 (m, 1H), 1.07 - 0.96 (m, 1H), 0.90 - 0.78 (m, 4H), 0.43 - 0.32 (m, 2H), 0.24 - 0.14 (m, 2H). LCMS (M+H)⁺ 519, RT 2.53 (Method 10). Chiral LC **RT = 36.89 min
** Chiral analysis was carried out using a Cellulose-4 (4.6 x 250 mm 5 µm) column, flow rate 0.2 mL/min eluting with ethanol, using a 60 min run time on a Waters 2795.

### Example 235

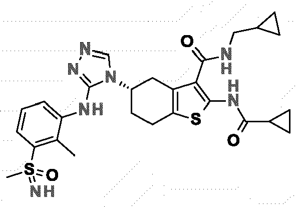

### (5S)-2-cyclopropaneamido-N-(cyclopropylmethyl)-5-[3-({3-[imino(methyl)oxo-lambda6-sulfanyl]-2-methylphenyl}amino)-4H-1,2,4-triazol-4-yl]-4,5,6,7-tetrahydro-1-benzothiophene-3-carboxamide

The title compound was prepared according to **general method 8,** using **intermediate 271** (195 mg, 0.35 mmol). The residue was purified *via* column chromatography (KP-NH), eluting with a gradient of 0 - 8% methanol in DCM to give a pale-yellow solid (95 mg). The solid was dissolved in DMSO:MeCN (1:1, 2 mL) and purified *via* HPLC (Method 4) to give the title compound (43.5 mg, 22% yield). δ_{H} (500 MHz, DMSO-*d*₆) 11.19 (s, 1H), 8.49 - 8.43 (m, 1H), 8.14 (s, 1H), 7.87 (s, 1H), 7.75 - 7.69 (m, 1H), 7.69 - 7.64 (m, 1H), 7.36 - 7.25 (m, 2H), 4.49 - 4.41 (m, 1H), 4.28 (s, 1H), 3.18 - 2.99 (m, 7H), 2.85 - 2.78 (m, 2H), 2.58 (s, 3H), 2.27 - 2.14 (m, 2H), 1.95 - 1.89 (m, 1H), 1.02 - 0.94 (m, 1H), 0.88 - 0.81 (m, 4H), 0.38 - 0.33 (m, 2H), 0.21 - 0.15 (m, 2H). LCMS (M+H)⁺ 568, RT 1.98 (Method 10).

### Examples 236 & 237

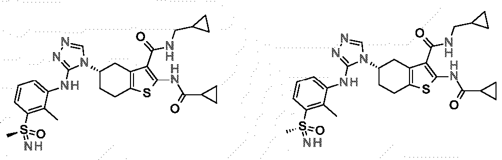

### (5S)-2-(cyclopropanecarbonylamino)-N-(cyclopropylmethyl)-5-[3-[2-methyl-3-[(S)-methylsulfonimidoyl]anilino]-1,2,4-triazol-4-yl]-4,5,6,7-tetrahydrobenzothiophene-3-carboxamide

### (5S)-2-(cyclopropanecarbonylamino)-N-(cyclopropylmethyl)-5-[3-[2-methyl-3-[(R)-methylsulfonimidoyl]anilino]-1,2,4-triazol-4-yl]-4,5,6,7-tetrahydrobenzothiophene-3-carboxamide

**Example 235** (39 mg, 0.07 mmol) was purified via chiral chromatography, to give the title compounds examples:
Peak 1 (3.5 mg). δ_{H} (500 MHz, DMSO-*d*₆) 11.18 (s, 1H), 8.47 (s, 1H), 7.87 (s, 1H), 7.72 (t, J = 5.7 Hz, 1H), 7.66 (dd, J = 7.5, 1.5 Hz, 1H), 7.35 - 7.23 (m, 2H), 4.49 - 4.37 (m, 1H), 4.28 (s, 1H), 3.19 - 2.98 (m, 7H), 2.85 - 2.78 (m, 2H), 2.58 (s, 3H), 2.28 - 2.15 (m, 2H), 1.96 - 1.88 (m, 1H), 1.03 - 0.93 (m, 1H), 0.89 - 0.80 (m, 4H), 0.40 - 0.32 (m, 2H), 0.23 - 0.15 (m, 2H). LCMS (M+H)⁺ 568, RT 2.01 (Method 10). Chiral SFC* RT = 28.28 min.
Peak 2 (4.5 mg). δ_{H} (500 MHz, DMSO-*d*₆) 11.19 (s, 1H), 8.47 (s, 1H), 7.87 (s, 1H), 7.72 (t, J = 5.6 Hz, 1H), 7.66 (dd, J = 7.7, 1.3 Hz, 1H), 7.36 - 7.25 (m, 2H), 4.49 - 4.41 (m, 1H), 4.28 (s, 1H), 3.19 - 2.98 (m, 7H), 2.86 - 2.78 (m, 2H), 2.58 (s, 3H), 2.29 - 2.13 (m, 2H), 1.96 - 1.88 (m, 1H), 1.03 - 0.94 (m, 1H), 0.89 - 0.82 (m, 4H), 0.39 - 0.32 (m, 2H), 0.20 - 0.16 (m, 2H). LCMS (M+H)⁺ 568, RT 2.01 (Method 10). Chiral SFC* RT = 33.04 min.
* Chiral analysis was carried out using a Chiralcel OJ-H 4.6 x 250 mm, 5 µm column, flow rate 4 mL/min eluting with 25% Ethanol + 0.2% DEA (diethylamine): 75% CO2, using a 50 min run time on a WatersThar Resolution-3100.

### Example 238

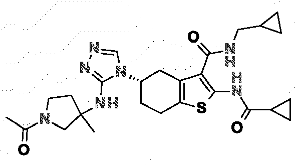

### (5S)-5-{3-[(1-acetyl-3-methylpyrrolidin-3-yl)aminol-4H-1,2,4-triazol-4-yl}-2-cyclopropaneamido-N-(cyclopropylmethyl)-4,5,6,7-tetrahydro-1-benzothiophene-3-carboxamide

To a stirred solution of **intermediate 274** (37 mg, 0.07 mmol) in DCM (1 mL) was added acetyl chloride (6 µL, 0.08 mmol) and DIPEA (26 µL, 0.15 mmol). The reaction mixture was stirred at room temperature for 2 hours, then retreated with DIPEA (26 µL, 0.15 mmol) and acetyl chloride (6 µL, 0.08 mmol) before being left to stand as a solution overnight. The solution was purified *via* column chromatography, using a gradient of 0 - 15% methanol in DCM. Product was eluted using 0 - 15% methanol in DCM to give an off-white powder. The powder was dissolved in DCM (3 mL) and 0.5 M HCl (1 mL) added. The organic layer was separated and the aqueous extracted with DCM (3 x 3 mL) and 1:1 IPA:CHCl₃ (3 x 3 mL). The aqueous layer was then adjusted to pH 7 using saturated sodium bicarbonate and extracted with DCM (3 x 3 mL). The organic fractions were combined, passed through a hydrophobic frit and concentrated *in vacuo.* The residue was purified *via* reverse-phase column chromatography, using a gradient of 10 - 30% acetonitrile in water (+ 0.1% formic acid) to give the title compound (19 mg, 49% yield). δ_{H} (500 MHz, DMSO-*d*₆) 11.25 - 11.18 (m, 1H), 8.17 - 8.11 (m, 1H), 7.66 (s, 1H), 5.92 - 5.83 (m, 1H), 4.34 - 4.25 (m, 1H), 3.97 - 3.77 (m, 1H), 3.53 - 3.44 (m, 2H), 3.39 - 3.37 (m, 1H), 3.27 - 3.22 (m, 1H), 3.22 - 3.14 (m, 1H), 3.10 - 2.98 (m, 2H), 2.89 - 2.77 (m, 3H), 2.21 - 2.05 (m, 2H), 1.96 - 1.79 (m, 5H), 1.48 - 1.43 (m, 3H), 1.05 - 0.95 (m, 1H), 0.89 - 0.81 (m, 4H), 0.41 - 0.32 (m, 2H), 0.23 - 0.14 (m, 2H). LCMS (M+H)⁺ 526, RT 1.97 & 2.01 (two diastereomers), (Method 10).

### Example 239

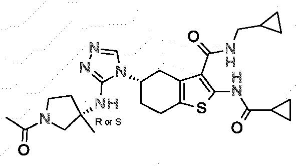

### (5S)-5-[3-[[(3S*)-1-acetyl-3-methyl-pyrrolidin-3-yl]amino]-1,2,4-triazol-4-yl]-2-(cyclopropanecarbonylamino)-N-(cyclopropylmethyl)-4,5,6,7-tetrahydrobenzothiophene-3-carboxamide [* or R]

**Example 238** (15 mg, 0.03 mmol) was separated *via* SFC (90:10 CO₂: Ethanol + 0.2% DEA using a Chiralcel OJ-H 25 cm column at 15 mL/min) to give the title compound (4.7 mg, 31%). δ_{H} (500 MHz, d-Chloroform) 11.94 - 11.78 (m, 1H), 7.88 - 7.79 (m, 1H), 6.29 - 5.81 (m, 1H), 4.53 - 4.38 (m, 1H), 4.32 - 4.18 (m, 1H), 4.13 - 3.62 (m, 1H), 3.57 - 3.13 (m, 6H), 2.91 - 2.73 (m, 3H), 2.44 - 1.84 (m, 6H), 1.75 - 1.54 (m, 4H), 1.12 - 1.00 (m, 3H), 0.95 - 0.87 (m, 3H), 0.56 - 0.47 (m, 2H), 0.28 - 0.20 (m, 2H). LCMS (M+H)⁺ 526, RT 1.89 (Method 10). Chiral SFC** RT = 11.77 min.

** Chiral analysis was carried out using a Cellulose-3 (4.6 x 250 mm 5µm) column, flow rate 4 mL/min eluting with 70:30 CO₂:Ethanol + 0.2% DEA, using a 20 minute run time on a WatersThar Resolution-3100.

### Example 240

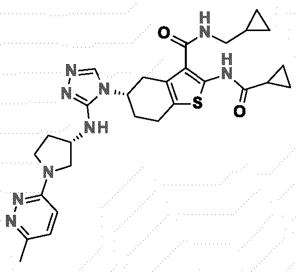

### (5S)-2-cyclopropaneamido-N-(cyclopropylmethyl)-5-(3-{[(3S)-1-(6-methylpyridazin-3-yl)pyrrolidin-3-yl]amino}-4H-1,2,4-triazol-4-yl)-4,5,6,7-tetrahydro-1-benzothiophene-3-carboxamide

**Intermediate 278** (25 mg, 0.05 mmol), cesium carbonate (34 mg, 0.11 mmol) and 3-fluoro-6-methylpyridazine (7 mg, 0.06 mmol) were suspended in DMSO (0.5 mL). The mixture was stirred at 100 °C for 5 hours, then diluted with methanol (0.5 mL) and purified HPLC (Method 2) to give a brown solid (4 mg). The solid was then purified *via* HPLC, using a gradient of 5-95 % acetonitrile in water with 0.1 % formic acid to give the title compound (2.9 mg, 10% yield). δ_{H}(500 MHz, MeOD-d₄) 8.31 (s, 1H), 8.17 (s, 1H), 7.31 (d, J = 9.3 Hz, 1H), 6.95 (d, J = 9.3 Hz, 1H), 4.49 - 4.43 (m, 1H), 4.43 - 4.35 (m, 1H), 3.89 (dd, J = 10.9, 6.0 Hz, 1H), 3.72 - 3.65 (m, 1H), 3.65 - 3.59 (m, 1H), 3.55 (dd, J = 10.9, 4.1 Hz, 1H), 3.25 - 3.15 (m, 2H), 3.14 - 3.09 (m, 1H), 3.00 - 2.84 (m, 3H), 2.47 (s, 3H), 2.45 - 2.38 (m, 1H), 2.31 - 2.24 (m, 2H), 2.24 - 2.18 (m, 1H), 1.83 - 1.76 (m, 1H), 1.06 - 0.96 (m, 3H), 0.96 - 0.91 (m, 2H), 0.45 - 0.39 (m, 2H), 0.22 - 0.17 (m, 2H). LCMS (M+H)⁺ 562, RT 1.45 (Method 10).

### Example 241

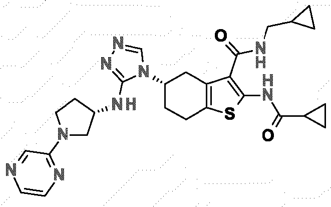

### (5S)-2-(cyclopropanecarbonylamino)-N-(cyclopropylmethyl)-5-[3-[[(3S)-1-pyrazin-2-ylpyrrolidin-3-yl]amino]-1,2,4-triazol-4-yl]-4,5,6,7-tetrahydrobenzothiophene-3-carboxamide

To a stirred solution of **intermediate 278** (92% purity, 45 mg, 0.088 mmol) and cesium carbonate (58 mg, 0.18 mmol) in DMSO (1 mL) was added 2-fluoropyrazine (10 mg, 0.11 mmol). The mixture was stirred at 100 °C for 4 hours, then purified by HPLC (Method 4) to give the title compound (19 mg, 38% yield). δ_{H} (500 MHz, DMSO-*d*₆) 11.21 (s, 1H), 8.14 (s, 1H), 8.01 (dd, J = 2.7, 1.5 Hz, 1H), 7.93 (d, J = 1.4 Hz, 1H), 7.74 (d, J = 2.7 Hz, 1H), 7.64 (s, 1H), 6.24 (d, J = 5.9 Hz, 1H), 4.39 - 4.31 (m, 1H), 4.30 - 4.22 (m, 1H), 3.78 (dd, J = 11.0, 6.2 Hz, 1H), 3.61 - 3.49 (m, 2H), 3.45 (dd, J = 11.0, 4.0 Hz, 1H), 3.16 - 3.09 (m, 1H), 3.07 - 3.00 (m, 2H), 2.87 - 2.77 (m, 3H), 2.32 - 2.23 (m, 1H), 2.22 - 2.07 (m, 3H), 1.95 - 1.86 (m, 1H), 0.98 - 0.91 (m, 1H), 0.88 - 0.80 (m, 4H), 0.34 - 0.28 (m, 2H), 0.18 - 0.12 (m, 2H). LCMS: (M+H)⁺ 548, RT 1.89 (Method 10).

### Examples 242 and 243

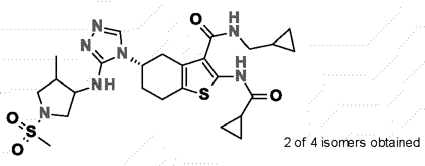

### (5S)-2-(cyclopropanecarbonylamino)-N-(cyclopropylmethyl)-5-[3-[(4-methyl-1-methylsulfonyl-pyrrolidin-3-yl)amino]-1,2,4-triazol-4-yl]-4,5,6,7-tetrahydrobenzothiophene-3-carboxamide

### *Absolute stereochemistry of the (4-methyl-1-methylsulfonyl-pyrrolidin-3-yl)amino group in the separated isomers is unknown

To a stirred solution of **intermediate 285** (300 mg, 0.61 mmol) and DIPEA (0.32 mL, 1.84 mmol) in DCM (6 mL), at 0 °C was added methanesulfonyl chloride (0.04 mL, 0.55 mmol). The mixture was stirred at room temperature for 1 hour. Methanesulfonyl chloride (4.2 µL, 0.05 mmol) was added and the mixture was stirred at room temperature for 30 minutes. The mixture was concentrated *in vacuo* and purified via column chromatography (KP-NH) using a gradient of 0 - 30% methanol in TBME to give an off-white powder (114 mg). The powder was separated by SFC (Methanol with Lux Cellulose-4 25 cm column at 5 mL/min), to give the following 2 diastereoisomers (2 out of 4):
Peak 1 (1.9 mg). δ_{H} (500 MHz, DMSO-*d*₆) 11.31 - 11.13 (m, 1H), 8.14 (s, 1H), 7.67 (s, 1H), 6.18 (d, J = 6.2 Hz, 1H), 4.31 - 4.21 (m, 1H), 3.85 - 3.78 (m, 1H), 3.70 (dd, J = 10.2, 6.9 Hz, 1H), 3.56 - 3.50 (m, 1H), 3.18 - 3.14 (m, 2H), 3.11 - 3.03 (m, 3H), 2.94 - 2.88 (m, 4H), 2.87 - 2.77 (m, 3H), 2.36 - 2.30 (m, 1H), 2.23 - 2.08 (m, 2H), 1.99 - 1.84 (m, 1H), 1.07 - 1.03 (m, 3H), 1.03 - 0.96 (m, 1H), 0.89 - 0.79 (m, 4H), 0.42 - 0.36 (m, 2H), 0.22 - 0.17 (m, 2H). LCMS (M+H)⁺ 562, RT 2.06 (Method 10). Chiral SFC** RT = 19.22 min.
Peak 4 (22 mg). δ_{H} (500 MHz, DMSO-*d*₆) 11.26 (s, 1H), 8.12 (s, 1H), 7.66 (s, 1H), 6.02 (d, J = 7.6 Hz, 1H), 4.42 - 4.34 (m, 1H), 4.33 - 4.27 (m, 1H), 3.58 (dd, J = 10.3, 6.6 Hz, 1H), 3.45 (dd, J = 9.9, 7.1 Hz, 1H), 3.23 (dd, J = 10.4, 5.2 Hz, 1H), 3.19 - 3.13 (m, 1H), 3.13 - 3.01 (m, 3H), 2.93 - 2.76 (m, 6H), 2.59 - 2.53 (m, 1H), 2.19 - 2.07 (m, 2H), 1.96 - 1.87 (m, 1H), 1.06 - 0.97 (m, 1H), 0.94 - 0.89 (m, 3H), 0.88 - 0.81 (m, 4H), 0.43 - 0.34 (m, 2H), 0.23 - 0.16 (m, 2H). LCMS (M+H)⁺ 562, RT 2.06 (Method 10). Chiral SFC* RT = 25.05 min
** Chiral analysis was carried out using a Lux Cellulose-4 4.6 x 250 mm, 5 µm column, flowrate 0.3 mL/min eluting with methanol, using a 40 min run time on a Waters 2795.

### Example 244

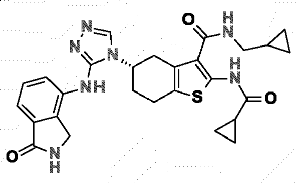

### (5S)-2-cyclopropaneamido-N-(cyclopropylmethyl)-5-{3-[(1-oxo-2,3-dihydro-1H-isoindol-4-yl)aminol-4H-1,2,4-triazol-4-yl}-4,5,6,7-tetrahydro-1-benzothiophene-3-carboxamide

The title compound was prepared according to **general method 8,** using of **intermediate 286** (130 mg, 0.25 mmol). The resulting residue was dissolved in 2:1 MeOH:DMSO (3 mL). The solution was purified by HPLC (Method 4) to give the title compound (36 mg, 26% yield). δ_{H} (500 MHz, DMSO-*d*₆) 11.22 (s, 1H), 8.50 - 8.47 (m, 2H), 8.31 (s, 1H), 7.69 (s, 1H), 7.46 (d, J = 7.5 Hz, 1H), 7.37 (t, J = 7.7 Hz, 1H), 7.25 (d, J = 6.8 Hz, 1H), 4.57 - 4.44 (m, 1H), 4.21 (s, 2H), 3.15 - 2.96 (m, 4H), 2.86 - 2.79 (m, 2H), 2.28 - 2.14 (m, 2H), 1.94 - 1.86 (m, 1H), 0.98 - 0.90 (m, 1H), 0.88 - 0.81 (m, 4H), 0.35 - 0.27 (m, 2H), 0.18 - 0.13 (m, 2H). LCMS (M+H)⁺ 532, RT 2.12 (Method 10) .

### Example 245

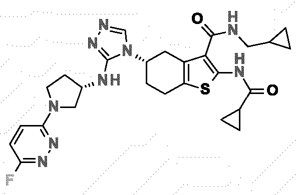

### (5S)-2-(cyclopropanecarbonylamino)-N-(cyclopropylmethyl)-5-[3-[[(3S)-1-(6-fluoropyridazin-3-yl)pyrrolidin-3-yl]amino]-1,2,4-triazol-4-yl]-4,5,6,7-tetrahydrobenzothiophene-3-carboxamide

To a stirred solution of **intermediate 278** (50 mg, 0.1 mmol) in DMSO (1 mL) was added dipotassium carbonate (41 mg, 0.29 mmol) and 3,6-difluoropyridazine (14 mg, 0.12 mmol). The mixture was stirred at room temperature for 1 hour, then left to stand overnight. The mixture was diluted with water (1 mL) and extracted with DCM (3 x 3 mL). The organic fractions were combined, passed through a hydrophobic frit and purified *via* column chromatography (KP-NH), using a gradient of 0 - 25% methanol in TBME to give an off-white powder. The powder was triturated using DCM and the filtrate taken to give the title compound (20.5 mg, 35% yield). δ_{H} (500 MHz, DMSO-d6) 11.21 (s, 1H), 8.14 (s, 1H), 7.64 (t, J = 5.5 Hz, 1H), 7.34 (dd, J = 9.6, 2.1 Hz, 1H), 7.14 (dd, J = 9.6, 6.8 Hz, 1H), 6.24 (d, J = 5.9 Hz, 1H), 4.41 - 4.32 (m, 1H), 4.31 - 4.22 (m, 1H), 3.79 (dd, J = 11.0, 6.2 Hz, 1H), 3.62 - 3.49 (m, 2H), 3.46 (dd, J = 10.9, 4.0 Hz, 1H), 3.17 - 3.09 (m, 1H), 3.07 - 3.00 (m, 2H), 2.87 - 2.78 (m, 3H), 2.33 - 2.24 (m, 1H), 2.22 - 2.08 (m, 3H), 1.95 - 1.87 (m, 1H), 0.99 - 0.90 (m, 1H), 0.88 - 0.81 (m, 4H), 0.34 - 0.28 (m, 2H), 0.17 - 0.11 (m, 2H). LCMS (M+H)⁺ 566, RT 2.61 (Method 10).

### Example 246

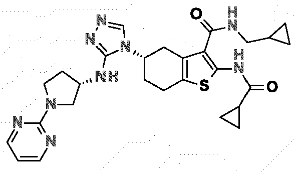

### (5S)-2-(cyclopropanecarbonylamino)-N-(cyclopropylmethyl)-5-[3-[[(3S)-1-pyrimidin-2-ylpyrrolidin-3-yl]amino]-1,2,4-triazol-4-yl]-4,5,6,7-tetrahydro-1-benzothiophene-3-carboxamide

To a stirred solution of **intermediate 278** (30 mg, 0.06 mmol) and DIPEA (31 µL, 0.18 mmol) in DMF (0.5 mL) was added 2-chloropyrimidine (9 mg, 0.08 mmol). The mixture was stirred at 120 °C for 2 hours and then concentrated *in vacuo.* The residue was purified *via* column chromatography (KP-NH), using a gradient of 0 - 20% methanol in TBME to give the title compound (21.5 mg, 65% yield). δ_{H} (500 MHz, DMSO-d6) 11.22 (s, 1H), 8.31 (d, J = 4.7 Hz, 2H), 8.13 (s, 1H), 7.64 (t, J = 5.4 Hz, 1H), 6.57 (t, J = 4.7 Hz, 1H), 6.22 (d, J = 5.8 Hz, 1H), 4.34 - 4.23 (m, 2H), 3.81 (dd, J = 11.6, 6.2 Hz, 1H), 3.65 - 3.54 (m, 2H), 3.51 (dd, J = 11.6, 4.1 Hz, 1H), 3.17 - 3.10 (m, 1H), 3.07 - 3.01 (m, 2H), 2.86 - 2.78 (m, 3H), 2.29 - 2.20 (m, 1H), 2.20 - 2.03 (m, 3H), 1.95 - 1.87 (m, 1H), 1.00 - 0.91 (m, 1H), 0.89 - 0.80 (m, 4H), 0.37 - 0.28 (m, 2H), 0.21 - 0.10 (m, 2H). LCMS (M+H)⁺ 548, RT 1.86 (Method 10).

### Example 247

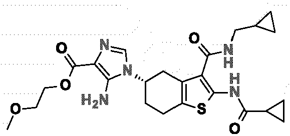

### 2-methoxyethyl 5-amino-1-[(5S)-2-(cyclopropanecarbonylamino)-3-(cyclopropylmethylcarbamoyl)-4,5,6,7-tetrahydro-1-benzothiophen-5-yl]imidazole-4-carboxylate

To a pressure tube containing 2-methoxyethanol (0.5 mL, 6.37 mmol) was added sodium hydride (17 mg, 0.42 mmol) and the resulting mixture was stirred under a flow of nitrogen for 5 minutes. **Example 117** (20 mg, 0.04 mmol) was added and stirring was continued until it was in solution. The pressure tube was then sealed and heated to 50 °C for 4 hours. The reaction mixture was then diluted with ethyl acetate (10 mL) and quenched with sat. aq. NH₄Cl solution (5 mL) and water (5 mL). The layers were separated and the aqueous phase was extracted further with ethyl acetate (10 mL). The combined organic layers were washed with brine (10 mL), dried over magnesium sulfate, filtered and concentrated. The residual solid was purified by reverse phase column chromatography (Method 4) to afford the title compound (12 mg, 25% yield). δ_{H} (500 MHz, DMSO-d6) 11.21 (s, 1H), 7.70 (s, 1H), 7.28 (s, 1H), 6.10 (s, 2H), 4.38 - 4.27 (m, 1H), 4.27 - 4.22 (m, 2H), 3.61 - 3.55 (m, 2H), 3.28 (s, 3H), 3.19 - 3.03 (m, 3H), 3.01 - 2.92 (m, 1H), 2.91 - 2.75 (m, 2H), 2.23 - 2.14 (m, 1H), 2.12 (s, 1H), 1.96 - 1.86 (m, 1H), 1.06 - 0.95 (m, 1H), 0.92 - 0.78 (m, 4H), 0.42 - 0.35 (m, 2H), 0.23 - 0.17 (m, 2H). LCMS [M+H]⁺ 502, RT 2.05 minutes (Method 10).

### Example 248

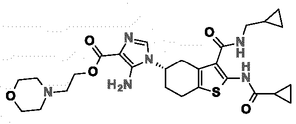

### 2-morpholin-4-ylethyl 5-amino-1-[(55)-2-(cyclopropanecarbonylamino)-3-(cyclopropylmethylcarbamoyl)-4,5,6,7-tetrahydro-1-benzothiophen-5-yl]imidazole-4-carboxylate

To a stirring mixture of 2-morpholinoethanol (500 µL, 4.13 mmol) and THF (300 µL) in a vial was added sodium (15 mg, 0.64 mmol) and the resulting mixture was stirred under a flow of nitrogen until completely in solution (1 hour at room temperature and 1 hour at 40 °C). **Example 117** (30 mg, 0.06 mmol) was added, the vial was sealed and the mixture was heated to 50 °C for 2 hours. The reaction mixture was diluted with ethyl acetate (10 mL) and washed with water (10 mL). The layers were separated and the aqueous phase was extracted with ethyl acetate (10 mL). The combined organic layers were dried over magnesium sulfate, filtered and concentrated *in vacuo.* The residual solid was purified by reverse phase column chromatography (Method 4) to afford the title compound (13 mg, 36% yield). δ_{H} (500 MHz, DMSO-d6) 11.21 (s, 1H), 7.71 (s, 1H), 7.29 (s, 1H), 6.20 (s, 2H), 4.36 - 4.28 (m, 1H), 4.22 (t, J = 5.8 Hz, 2H), 3.59 - 3.55 (m, 4H), 3.18 - 3.08 (m, 2H), 3.08 - 3.03 (m, 1H), 2.99 - 2.90 (m, 1H), 2.90 - 2.76 (m, 2H), 2.59 (t, J = 5.9 Hz, 2H), 2.46 - 2.38 (m, 4H), 2.23 - 2.16 (m, 1H), 2.16 - 2.08 (m, 1H), 1.95 - 1.89 (m, 1H), 1.06 - 0.97 (m, 1H), 0.89 - 0.81 (m, 4H), 0.41 - 0.35 (m, 2H), 0.22 - 0.16 (m, 2H). LCMS [M+H]⁺557, RT 1.66 minutes (Method 10).

### Example 249

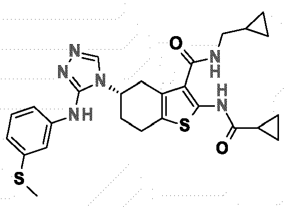

### (5S)-2-(cyclopropanecarbonylamino)-N-(cyclopropylmethyl)-5-[3-(3-methylsulfanylanilino)-1,2,4-triazol-4-yl]-4,5,6,7-tetrahydrobenzothiophene-3-carboxamide

To a solution of **intermediate 287** (275 mg, 0.53 mmol) in anhydrous DMF (5 mL) was added formic hydrazide (96 mg, 1.6 mmol) followed by mercury dichloride (435 mg, 1.60 mmol) and triethylamine (0.22 mL, 1.6 mmol). The resulting mixture was stirred at 90 °C for 2.5 hours. Celite was added to the stirring mixture and it was diluted with ethyl acetate (10 mL). The mixture was then filtered through a pad of celite, washing through with more ethyl acetate, and the solvent was evaporated *in vacuo.* The crude material was purified by flash column chromatography eluting with a 0 to 10% methanol in DCM gradient to afford the title compound (115 mg, 42% yield). δ_{H} (500 MHz, DMSO-d6) 11.25 (s, 1H), 8.61 (s, 1H), 8.38 (s, 1H), 7.67 (t, J = 5.4 Hz, 1H), 7.57 (t, J = 1.9 Hz, 1H), 7.37 - 7.32 (m, 1H), 7.19 (t, J = 8.0 Hz, 1H), 6.76 (dd, J = 7.8, 0.9 Hz, 1H), 4.56 - 4.46 (m, 1H), 3.17 - 3.02 (m, 3H), 2.98 - 2.82 (m, 3H), 2.45 (s, 3H), 2.31 - 2.13 (m, 2H), 1.96 - 1.88 (m, 1H), 1.00 - 0.90 (m, 1H), 0.90 - 0.82 (m, 4H), 0.35 - 0.27 (m, 2H), 0.18 - 0.10 (m, 2H). LCMS [M+H]⁺ 523, RT 2.93 minutes (Method 10).

### Example 250

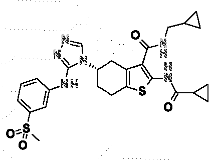

### (55)-2-(cyclopropanecarbonylamino)-N-(cyclopropylmethyl)-5-[3-(3-methylsulfonylanilino)-1,2,4-triazol-4-yl]-4,5,6,7-tetrahydrobenzothiophene-3-carboxamide

To a stirring solution of **Example 249** (21 mg, 0.04 mmol) in anhydrous DCM (1 mL) was added 3-chlorobenzenecarboperoxoic acid (10 mg, 0.046 mmol) and the resulting solution was stirred at room temperature for 1.5 hours. Another portion of 3-chlorobenzenecarboperoxoic acid (18 mg, 0.08 mmol) was added and stirring was continued at room temperature for 2 hours. The reaction mixture was concentrated in vacuo and the residue was purified by reverse phase column chromatography (Method 4) to afford the title compound (2.3 mg, 10% yield). δ_{H} (500 MHz, Methanol-d4) 8.42 (s, 1H), 8.04 (t, J = 1.8 Hz, 1H), 7.73 - 7.68 (m, 1H), 7.57 - 7.48 (m, 2H), 4.65 - 4.57 (m, 1H), 3.29 - 3.21 (m, 2H), 3.13 (s, 4H), 3.10 - 3.03 (m, 1H), 3.03 - 2.88 (m, 2H), 2.39 - 2.33 (m, 2H), 1.84 - 1.77 (m, 1H), 1.05 - 0.97 (m, 3H), 0.97 - 0.91 (m, 2H), 0.43 - 0.38 (m, 2H), 0.22 - 0.17 (m, 2H). LCMS [M+H]⁺ 555, RT 2.58 minutes (Method 10).

### Example 251

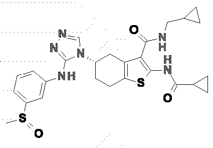

### (5S)-2-(cyclopropanecarbonylamino)-N-(cyclopropylmethyl)-5-[3-(3-methylsulfinylanilino)-1,2,4-triazol-4-yl]-4,5,6,7-tetrahydrobenzothiophene-3-carboxamide

To a solution of **Example 249** (88 mg, 0.168 mmol) in anhydrous DCM (4 mL) was added 3-chlorobenzenecarboperoxoic acid (38 mg, 0.168 mmol) and the resulting solution was stirred at room temperature for 45 minutes. The reaction mixture was diluted with DCM (10 mL) and washed with sat. aq. NaHCO₃ solution (10 mL). The aqueous layer was extracted further with DCM (10 mL), the combined organic layers were washed with brine (10 mL), dried over magnesium sulphate, filtered and concentrated. The crude material was purified by flash column chromatography with a 0 to 10% methanol in DCM gradient to afford the title compound (65 mg, 68% yield). δ_{H} (500 MHz, DMSO-d6) 11.25 (d, J = 4.1 Hz, 1H), 8.98 (s, 1H), 8.42 (s, 1H), 7.93 (t, J = 1.7 Hz, 1H), 7.83 - 7.75 (m, 1H), 7.67 (t, J = 5.3 Hz, 1H), 7.44 (t, J = 7.9 Hz, 1H), 7.11 (d, J = 7.6 Hz, 1H), 4.60 - 4.48 (m, 1H), 3.18 - 3.10 (m, 2H), 3.09 - 3.01 (m, 1H), 2.99 - 2.90 (m, 1H), 2.90 - 2.83 (m, 2H), 2.71 (s, 3H), 2.32 - 2.24 (m, 1H), 2.23 - 2.16 (m, 1H), 1.95 - 1.88 (m, 1H), 0.99 - 0.91 (m, 1H), 0.90 - 0.81 (m, 4H), 0.29 (d, J = 7.8 Hz, 2H), 0.18 - 0.09 (m, 2H). LCMS [M+H]⁺ 539, RT 2.29 minutes (Method 10).

### Example 252

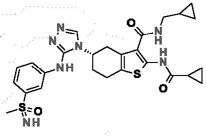

### (5S)-2-(cyclopropanecarbonylamino)-N-{cyclopropylmethyl)-5-[3-[3-(methylsulfonimidoyl)anilinol-1,2,4-triazol-4-yl1-4,5,6,7-tetrahydrobenzothiophene-3-carboxamide

To a solution of **Intermediate 292** (255 mg, 0.46 mmol) in anhydrous DMF (4.3 mL) was added formic hydrazide (82 mg, 1.37 mmol) followed by mercury dichloride (371 mg, 1.37 mmol) and triethylamine (0.19 mL, 1.37 mmol) and the resulting mixture was stirred at 90 °C overnight. Celite was added to the stirring mixture and it was diluted with ethyl acetate (10 mL). The mixture was then filtered through a pad of celite, washing through with more ethyl acetate, and the solvent was evaporated *in vacuo.* The crude material was purified by reverse phase column chromatography (Method 4) to afford the title compound (formic acid salt) (55 mg, 20% yield). δ_{H} (250 MHz, DMSO-d6) 11.25 (s, 1H), 9.03 (s, 1H), 8.43 (s, 1H), 8.20 - 8.11 (m, 2H), 8.00 - 7.93 (m, 1H), 7.69 (t, J = 5.8 Hz, 1H), 7.54 - 7.38 (m, 2H), 4.63 - 4.46 (m, 1H), 4.10 (s, 1H), 3.16 - 3.05 (m, 3H), 3.02 (s, 3H), 3.00 - 2.93 (m, 1H), 2.92 - 2.82 (m, 2H), 2.35 - 2.14 (m, 2H), 2.00 - 1.84 (m, 1H), 1.03 - 0.89 (m, 1H), 0.89 - 0.80 (m, 4H), 0.36 - 0.26 (m, 2H), 0.19 - 0.10 (m, 2H). LCMS [M+H]⁺ 554, RT 2.15 minutes (Method 10).

### Examples 253 & 254

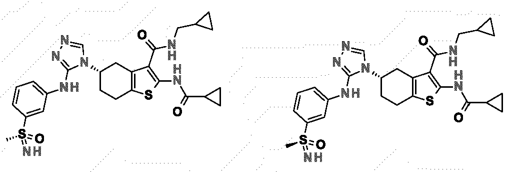

### (5S)-2-(cyclopropanecarbonylamino)-N-(cyclopropylmethyl)-5-[3-[3-[(S)-methylsulfonimidoyl]anilino]-1,2,4-triazol-4-yl]-4,5,6,7-tetrahydrobenzothiophene-3-carboxamide

### (5S)-2-(cyclopropanecarbonylamino)-N-(cyclopropylmethyl)-5-[3-[3-[(R)-methylsulfonimidoyl]anilino]-1,2,4-triazol-4-yl]-4,5,6,7-tetrahydrobenzothiophene-3-carboxamide

The title compounds were obtained by separating the two diastereoisomers of **Example 252** (45 mg) by chiral chromatography (Ethanol + 0.2 % DEA with Amylose-2 25 cm at 7 mL/min). Each sample after separation was dissolved in DCM (5 mL), washed with saturated aqueous NH₄Cl (2 x 5 mL), water (5 mL), then brine (5 mL). The organic layer was filtered through a phase separator and concentrated under reduced pressure. The residue was dissolved in acetonitrile:water (1:1) and freeze dried to afford the final compounds:
Isomer 1 (Peak 1, 11.5 mg). δ_{H} (500 MHz, DMSO-d6) 11.25 (s, 1H), 9.03 (s, 1H), 8.43 (s, 1H), 8.16 (t, J = 1.9 Hz, 1H), 8.02 - 7.92 (m, 1H), 7.69 (t, J = 5.7 Hz, 1H), 7.49 (t, J = 7.9 Hz, 1H), 7.45 - 7.37 (m, 1H), 4.62 - 4.46 (m, 1H), 4.09 (br s, 1H), 3.18 - 3.10 (m, 2H), 3.10 - 3.04 (m, 1H), 3.03 (s, 3H), 3.00 - 2.91 (m, 1H), 2.91 - 2.82 (m, 2H), 2.33 - 2.23 (m, 1H), 2.23 - 2.15 (m, 1H), 1.98 - 1.87 (m, 1H), 1.01 - 0.93 (m, 1H), 0.92 - 0.82 (m, 4H), 0.38 - 0.26 (m, 2H), 0.21 - 0.10 (m, 2H). LCMS [M+H]⁺ 554, RT 2.15 minutes (Method 10). Chiral LC* RT = 9.99 minutes.
Isomer 2 (Peak 2, 14 mg). δ_{H} (500 MHz, DMSO-d6) 11.24 (s, 1H), 9.05 (s, 1H), 8.44 (s, 1H), 8.16 (t, J = 2.0 Hz, 1H), 8.00 - 7.93 (m, 1H), 7.69 (t, J = 5.7 Hz, 1H), 7.53 - 7.46 (m, 1H), 7.46 - 7.37 (m, 1H), 4.61 - 4.50 (m, 1H), 3.18 - 3.11 (m, 2H), 3.10 - 3.06 (m, 1H), 3.04 (s, 3H), 3.00 - 2.92 (m, 1H), 2.91 - 2.82 (m, 2H), 2.32 - 2.23 (m, 1H), 2.23 - 2.15 (m, 1H), 1.96 - 1.89 (m, 1H), 1.00 - 0.91 (m, 1H), 0.89 - 0.81 (m, 4H), 0.35 - 0.26 (m, 2H), 0.20 - 0.09 (m, 2H), NH proton missing. LCMS [M+H]⁺ 554, RT 2.17 minutes (Method 10). Chiral LC* RT = 15.70 minutes.
* Chiral analysis using Amylose-2 (4.6 x 250 mm 5 µm) column, flow rate 0.5 mL/min, eluting with Ethanol + 0.2% DEA, 30 minutes run time on a Waters 2795.

### Example 257

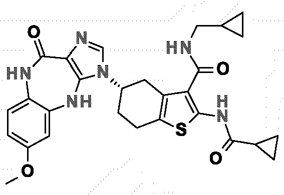

### (5S)-2-(cyclopropanecarbonylamino)-N-(cyclopropylmethyl)-5-(8-methoxy-4-oxo-5,10-dihydroimidazo[4,5-c][1,5]benzodiazepin-1-yl)-4,5,6,7-tetrahydrobenzothiophene-3-carboxamide

2,2,2-Trifluoroacetic acid (133 µL, 1.79 mmol) was added to the stirred solution of **Intermediate 304** (60 mg, 0.09 mmol) in DCM (0.7 mL) and the mixture stirred at room temperature for 24 h. The reaction mixture was evaporated to dryness and purified by column chromatography, eluting with 0-20% methanol in DCM to afford the title compound (30 mg, 61% yield). δ_{H} (500 MHz, DMSO-d6) 11.08 (s, 1H), 8.64 (br s, 1H), 7.91 (br s, 1H), 7.53 (t, J = 5.6 Hz, 1H), 7.47 (br s, 1H), 6.67 (d, J = 8.7 Hz, 1H), 6.47 (d, J = 2.7 Hz, 1H), 6.34 (dd, J = 8.7, 2.7 Hz, 1H), 4.41 - 4.31 (m,1H), 3.50 (s, 3H), 3.04 - 2.85 (m, 3H), 2.82 - 2.65 (m, 3H), 2.15 - 1.99 (m, 2H), 1.82 - 1.73 (m, 1H), 0.87 - 0.78 (m, 1H), 0.74 - 0.66 (m, 4H), 0.23 - 0.11 (m, 2H), 0.05 - -0.05 (m, 2H). LCMS [M+H]⁺ 547, RT 2.38 minutes (Method 10).

### Example 258

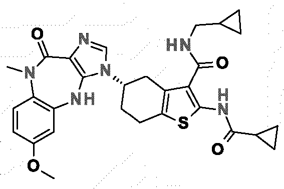

### (5S)-2-(cyclopropanecarbonylamino)-N-(cyclopropylmethyl)-5-(8-methoxy-5-methyl-4-oxo-10H-imidazo[4,5-c][1,5]benzodiazepin-1-yl)-4,5,6,7-tetrahydrobenzothiophene-3-carboxamide

**Intermediate 307** (42 mg, 0.066 mmol) and sodium 2-methylpropan-2-olate (29 mg, 0.306 mmol) were suspended in dry tert-butanol (0.84 mL) and dioxane (0.4 mL) and de-gassed for 5 minutes. tBuXPhos Pd G3 (5.2 mg, 6.55 µmol) was added and the mixture heated at 90 °C in a sealed tube for 11 h. The mixture was cooled to room temperature, diluted with water (5 mL), neutralized with 1 M HCl and extracted with IPA:CHCl₃ (1:1, 3 x 5 mL). The combined organic layers were dried over sodium sulfate, filtered and evaporated to dryness to afford the crude. This was dissolved in DMSO and purified by HPLC (Method 2) to give the title compound (5.0 mg, 14% yield). δ_{H} (500 MHz, DMSO-d6) 11.25 (s, 1H), 8.20 (s, 1H), 7.75 - 7.59 (m, 1H), 7.47 (s, 1H), 7.11 (d, J = 8.9 Hz, 1H), 6.72 (d, J = 2.9 Hz, 1H), 6.65 (dd, J = 8.9, 2.9 Hz, 1H), 4.59 - 4.45 (m, 1H), 3.70 (s, 3H), 3.18(s, 3H), 3.17 - 3.12 (m, 1H), 3.11 - 3.00 (m, 2H), 2.98 - 2.91 (m, 1H), 2.91 - 2.79 (m, 2H), 2.30 - 2.14 (m, 2H), 1.99 - 1.86 (m, 1H), 1.03 - 0.92 (m, 1H), 0.92 - 0.76 (m, 4H), 0.38 - 0.24 (m, 2H), 0.21 - 0.08 (m, J =4.9 Hz, 2H). LCMS [M+H]⁺ 561, RT 2.50 minutes (Method 10).

### Example 260

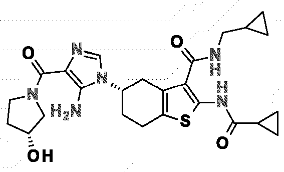

### (5S)-5-[5-amino-4-[(3R)}-3-hydroxypyrrolidine-1-carbonyl]imidazol-1-yl]-2-(cyclopropanecarbonylamino)-N-(cyclopropylmethyl)-4,5,6,7-tetrahydrobenzothiophene-3-carboxamide

HATU (25 mg, 0.07 mmol) was added to a stirred solution of **Intermediate 300** (30 mg, 0.06 mmol), (3R)-pyrrolidin-3-ol (10 µL, 0.124 mmol) and DIPEA (30 µL, 0.17 mmol) in dry DMF (0.6 mL) at room temperature. The reaction mixture was stirred for 2 h, diluted with water (5 mL) and extracted with DCM (3 x 5 mL). The combined organic layers were dried over sodium sulfate, filtered and evaporated to dryness to afford the crude. The crude material was purified by column chromatography, eluted with 0-20% methanol in DCM to afford crude material. This was treated with 4 M HCl in 1,4-dioxane (222 µL, 0.889 mmol) and stirred for 3 h. The mixture was diluted with saturated aqueous NaHCO₃ (5 mL) and extracted with DCM (3 x 5 mL). The combined organic layers were dried over sodium sulfate, filtered, evaporated to dryness and purified by HPLC (Method 1) to give the title compound (8.0 mg, 28% yield). δ_{H} (500 MHz, DMSO-d6) 11.20 (s, 1H), 7.71 (br s, 1H), 7.24 (s, 1H), 6.14 (s, 2H), 4.84 (d, J = 3.3 Hz, 1H), 4.35 - 4.27 (m, 1H), 4.27 - 4.01 (m, 1H), 4.01 - 3.74 (m, 2H), 3.64 - 3.43 (m, 2H), 3.19 - 3.03 (m,3H), 3.03 - 2.89 (m, 1H), 2.89 - 2.74 (m, 2H), 2.24 - 2.07 (m, 2H), 1.96 - 1.89 (m, 1H), 1.89 - 1.65 (m, 2H), 1.06 - 0.95 (m, 1H), 0.94 - 0.74 (m, 4H), 0.43 - 0.33 (m, 2H), 0.25 - 0.14 (m, 2H). LCMS [M+H]⁺ 513, RT 2.00 minutes (Method 10).

### Example 261

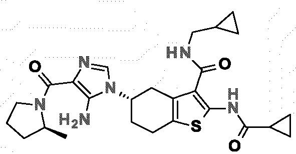

### (5S)-5-[5-amino-4-[(2S)-2-methylpyrrolidine-1-carbonyl]imidazol-1-yl]-2-(cyclopropanecarbonylamino)-N-(cyclopropylmethyl)-4,5,6,7-tetrahydrobenzothiophene-3-carboxamide

HATU (24 mg, 0.06 mmol) was added to a stirred solution of **Intermediate 300** (30 mg, 0.05 mmol), (2{S})-2-methylpyrrolidine (10 mg, 0.117 mmol) and DIPEA (27 µL, 0.15 mmol) in dry DMF (0.6 mL) at room temperature. The reaction mixture was stirred for 30 min. The reaction mixture was diluted with water (5 mL) and extracted with DCM (3 x 5 mL). The combined organic layers were dried over sodium sulfate, filtered and evaporated to dryness to afford the crude. The crude material was treated with 4 M HCl in 1,4-dioxane (200 µL, 0.800 mmol) and stirred for 2 h. The reaction mixture was diluted with saturated aqueous NaHCO₃ (5 mL) and extracted with DCM (3 x 5 mL). The combined organic layers were dried over sodium sulfate, filtered, evaporated to dryness and purified by column chromatography, eluting with 20% methanol in DCM, followed by HPLC (Method 1) to give the title compound (18 mg, 67% yield). δ_{H} (500 MHz, DMSO-d6) 11.20 (s, 1H), 7.71 (t, J = 5.3 Hz, 1H), 7.24 (s, 1H), 6.17 (s, 2H), 4.37 - 4.28 (m, 1H), 4.18 (br s, 1H), 3.96 (br s, 1H), 3.61 - 3.35 (m, 1H), 3.17 - 3.10 (m, 2H), 3.09 - 3.02 (m, 1H),3.02 - 2.91 (m, 1H), 2.91 - 2.71 (m, 2H), 2.23 - 2.06 (m, 2H), 1.98 - 1.83 (m, 3H), 1.83 - 1.72 (m, 1H), 1.70 - 1.37 (m, 1H), 1.15 (d, J = 6.3 Hz, 3H), 1.08 - 0.95 (m, 1H), 0.91 - 0.79 (m, 4H), 0.43 - 0.34 (m, 2H), 0.26- 0.14 (m, 2H). LCMS [M+H]⁺ 511, RT 2.71 minutes (Method 10).

### Example 262

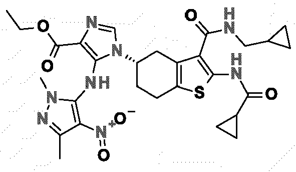

### Ethyl 1-[(5S)-2-(cyclopropanecarbonylamino)-3-(cyclopropylmethylcarbamoyl)-4,5,6,7-tetrahydrobenz othiophen-5-yl]-5-[(2,5-dimethyl-4-nitro-pyrazol-3-yl)amino]imidazole-4-carboxylate

5-chloro-1,3-dimethyl-4-nitro-1H-pyrazole (179 mg, 1.02 mmol), **Example 117** (400 mg, 0.85 mmol) and cesium carbonate (1.38 g, 4.24 mmol) were suspended in dry DMF (7 mL) at room temperature. The reaction mixture was heated at 100 °C for 5 h, then cooled to room temperature. The cesium carbonate was filtered, washing with ethyl acetate. The filtrate was diluted with water and neutralized with saturated aqueous NH₄Cl then extracted with ethyl acetate (3 x 25 mL). The combined organic layers were washed with brine (25 mL), dried over sodium sulfate, filtered, concentrated under reduced pressure and purified by HPLC (Method 3) to give the title compound (120 mg, 22% yield). δ_{H} (500 MHz, Chloroform-d) 11.96 (br s, 1H), 8.48 (s, 1H), 7.63 (s, 1H), 5.64 (s, 1H), 4.75 - 4.64 (m, 1H), 4.24 (q, J = 7.1 Hz, 2H), 3.38 - 3.22 (m, 3H), 3.19 (s, 3H), 3.11 - 2.77 (m, 3H), 2.49 (s, 3H), 2.44 -2.22 (m, 2H), 1.71 - 1.63 (m, 1H), 1.22 - 1.17 (m, 3H), 1.15 - 1.08 (m, 2H), 1.07 - 0.96 (m, 1H), 0.95 - 0.90 (m, 2H), 0.60 - 0.45 (m, 2H), 0.30 - 0.19 (m, 2H). LCMS [M+H]⁺ 611, RT 3.15 minutes (Method 10).

### Example 263

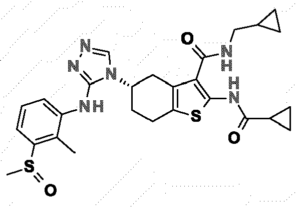

### (55)-2-(cyclopropanecarbonylamino)-N-(cyclopropylmethyl)-5-[3-(2-methyl-3-methylsulfinyl-anilino)-1,2,4-triazol-4-yl]-4,5,6,7-tetrahydrobenzothiophene-3-carboxamide

To a solution of **Intermediate 315** (33 mg, 0.061 mmol) in anhydrous DCM (2 mL) was added 3-chlorobenzenecarboperoxoic acid (14 mg, 0.061 mmol) and the resulting solution was stirred at room temperature for 30 minutes. Concentrated under reduced pressure and purified by flash column chromatography eluting with a 0-20% methanol in DCM gradient, followed by reverse phase HPLC (Method 4) to give the title compound (14 mg, 40% Yield). δ_{H} (500 MHz, DMSO-d₆) 11.27 - 11.09 (m, 1H), 8.51 - 8.43 (m, 1H), 7.88 - 7.83 (m, 1H), 7.81 - 7.66 (m, 1H), 7.43 (d, J = 7.8 Hz, 1H), 7.37 (t, J = 7.8 Hz, 1H), 7.27 - 7.20 (m, 1H), 4.49 - 4.38 (m, 1H), 3.20 - 2.94 (m, 4H), 2.87 - 2.76 (m, 2H), 2.66 - 2.65 (m, 3H), 2.26 - 2.14 (m, 5H), 1.95 - 1.88 (m, 1H), 1.02 - 0.92 (m, 1H), 0.89 - 0.79 (m, 4H), 0.39 - 0.30 (m, 2H), 0.24 - 0.13 (m, 2H). LCMS [M+H]⁺ 553, RT 2.07 minutes (Method 10).

### Example 264

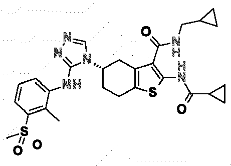

### (55)-2-(cyclopropanecarbonylamino)-N-(cyclopropylmethyl)-5-[3-(2-methyl-3-methylsulfonyl-anilino)-1,2,4-triazol-4-yl]-4,5,6,7-tetrahydrobenzothiophene-3-carboxamide

To a solution of **Example 263** (33 mg, 0.061 mmol) in anhydrous DCM (2 mL) was added 3-chlorobenzenecarboperoxoic acid (41 mg, 0.18 mmol) and the resulting solution was stirred at room temperature for 2 h. Further 3-chlorobenzenecarboperoxoic acid (41 mg, 0.18 mmol) was added and the mixture was stirred at room temperature for a further 20 mins. Concentrated under reduced pressure and first purified by flash column chromatography eluting with a 0-10% methanol in DCM gradient, then by HPLC (Method 4), and finally by HPLC (Method 2) to give the title compound (1.5 mg, 4.2% Yield). δ_{H} (500 MHz, Methanol-d₄) 8.42 (s, 1H), 7.84 (dd, J = 6.8, 2.4 Hz, 1H), 7.48 - 7.38 (m, 2H), 4.58 (s, 1H), 3.32 - 3.25 (m, 2H), 3.23 - 3.16 (m, 4H), 3.12 (dd, J = 15.6, 9.6 Hz, 1H), 3.02 - 2.89 (m, 2H), 2.61 (s, 3H), 2.42 - 2.34 (m, 2H), 1.87 - 1.79 (m, 1H), 1.13 - 1.04 (m, 1H), 1.04 - 0.99 (m, 2H), 0.99 - 0.93 (m, 2H), 0.53 - 0.47 (m, 2H), 0.29 - 0.24 (m, 2H). LCMS [M+H]⁺ 569, RT 2.36 minutes (Method 10).

### Example 265

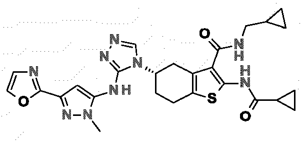

### (55)-2-(cyclopropanecarbonylamino)-N-(cyclopropylmethyl)-5-[3-[(2-methyl-5-oxazol-2-yl-pyrazol-3-yl)aminol-1,2,4-triazol-4-yl]-4,5,6,7-tetrahydrobenzothiophene-3-carboxamide

Methanesulfonyl chloride (7.7 µL, 0.10 mmol) was added to a stirred suspension of **Intermediate** 322 (18 mg, 0.033 mmol) and triethylamine (42 µL, 0.30 mmol) in DCM (1 mL) at 0 °C and stirred at this temperature for 20 minutes. Further methanesulfonyl chloride (7.7 µL, 0.10 mmol) and triethylamine (42 µL, 0.30 mmol) were added and the mixture was stirred at 0 °C for 30 minutes. Further methanesulfonyl chloride (7.7 µL, 0.10 mmol) and triethylamine (42 µL, 0.30 mmol) were added and the mixture was stirred at 0 °C for 15 minutes. The reaction mixture was diluted with DCM (10 mL) and washed with sat. aq. NH₄Cl (2 x 10 mL), brine (10 mL), dried over MgSO₄, filtered and concentrated under reduced pressure to afford intermediate carbodiimide. To this was added a solution of formic hydrazide (8.0 mg, 0.13 mmol) in dry methanol (1 mL) and the reaction mixture was stirred at room temperature for 30 minutes. Sodium carbonate (14 mg, 0.13 mmol) was added and the mixture was stirred and heated to 50 °C for 18 h. The reaction mixture was cooled to room temperature, concentrated under reduced pressure, diluted with water (10 mL) and extracted with DCM:methanol (9:1, 3 x 10 mL). The combined organic layers were dried over MgSO₄, filtered, concentrated under reduced pressure and purified by flash column chromatography eluting with a 4-10% methanol in DCM gradient, followed by HPLC (Method 2). The residue was then dissolved in methanol and passed through an SCX-2 cartridge (eluting with methanol followed by 3.5 M ammonia in methanol). The fractions containing the product were concentrated under reduced pressure and purified by flash column chromatography eluting with a 2-8% methanol in DCM gradient to give the title compound (3.4 mg, 18% Yield). δ_{H} (500 MHz, DMSO-d₆) 12.15 (s, 1H, R1), 11.31 - 11.16 (m, 1H), 8.77 (s, 1H, R2), 8.41 (s, 1H, R2), 8.19 (s, 1H, R1), 8.12 (s, 1H, R2), 8.08 (s, 1H, R1), 7.73 (s, 1H), 7.31 (s, 1H, R2), 7.28 (s, 1H, R1), 6.66 (s, 1H, R2), 6.49 (s, 1H, R1), 4.55 - 4.41 (m, 1H), 3.80 - 3.72 (m, 3H, R2), 3.70 - 3.62 (m, 3H, R1), 3.22 - 3.05 (m, 3H), 3.04 - 2.91 (m, 1H), 2.91 - 2.78 (m, 2H), 2.30 - 2.15 (m, 2H), 1.96 - 1.89 (m, 1H), 1.04 - 0.96 (m, 1H), 0.89 - 0.79 (m, 4H), 0.38 - 0.32 (m, 2H), 0.21 - 0.15 (m, 2H). 1 Contains ~2:1 rotamers (R1 and R2). LCMS [M+H]⁺ 548, RT 2.45 minutes (Method 10).

### Example 266

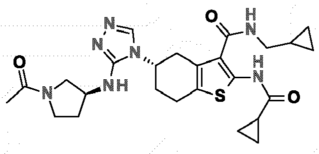

### (5S)-5-[3-[[(3S)-1-acetylpyrrolidin-3-yl]amino]-1,2,4-triazol-4-yl]-2-(cyclopropanecarbonylamino)-N-(cyclopropylmethyl)-4,5,6,7-tetrahydrobenzothiophene-3-carboxamide

Suspension of **intermediate 278** (50 mg, 0.092 mmol) was stirred in DCM (5 mL) and cooled to 0 °C. DIPEA (48.1 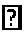 µL, 0.27 mmol) followed by acetic anhydride (7.8 µL, 0.083 mmol) were then added. Reaction stirred for 1 h at 0 °C then warmed to room temperature. Reaction mixture was diluted with DCM then water (15 mL) was added and organic layer separated. The aqueous layer was extracted with DCM (2 x 10 mL) and the organic extracts combined, dried over sodium sulphate and concentrated under vacuum. The residue was purified by revers phase column chromatography eluting with 5 - 95 % acetonitrile (0. 2 % ammonium hydroxide) in water (0. 2 % ammonium hydroxide) to give the title compound (15 mg, 32% Yield). δ_{H} (500 MHz, Methanol-d4) 8.18 (s, 1H, rotamer), 8.17 (s, 1H, rotamer), 4.46 - 4.28 (m, 2H), 3.93 (dd, J = 11.0, 6.0 Hz, 1H, rotamer), 3.77 (dd, J = 12.5, 6.2 Hz, 1H, rotamer), 3.72 - 3.43 (m, 3H), 3.31 - 3.15 (m, 3H), 3.05 - 2.84 (m, 3H), 2.41 - 2.32 (m, 1H, rotamer), 2.32 - 2.24 (m, 2H and1H rotamer), 2.19 - 2.08 (m, 1H), 2.07 (s, 3H, rotamer), 2.06 (s, 3H, rotamer), 1.13 - 1.05 (m, 1H), 1.84 - 1.79 (m, 1H), 1.03 - 0.93 (m, 4H), 0.53 - 0.48 (m, 2H), 0.29 - 0.24 (m, 2H). LCMS [M+H]⁺ 512, RT 1.68 minutes (Method 10).

### Example 267

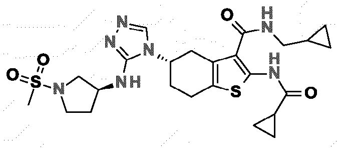

### (55)-2-(cyclopropanecarbonylamino)-N-(cyclopropylmethyl)-5-[3-[[(3S)-1-methylsulfonylpyrrolidin-3-yl]amino]-1,2,4-triazol-4-yl]-4,5,6,7-tetrahydrobenzothiophene-3-carboxamide

Suspension of **intermediate 278** (50 mg, 0.092 mmol) was stirred in DCM (5 mL) was cooled to 0 °C. DIPEA (48.1 µL, 0.27 mmol) followed by methanesulfonyl methanesulfonate (14.4 mg, 0.083 mmol) were then added. Reaction stirred for 1 h at 0 °C. Then warm to room temperature. Reaction mixture was diluted with DCM (20 mL) then water (15 mL) was added and organic layer separated. The aqueous layer was extracted with DCM (2 x 10 mL) and the organic extracts combined, dried over sodium sulphate and concentrated under vacuum. The residue was purified by reverse phase column chromatography eluting with 5 - 95 % Acetonitrile (0. 2 % ammonium hydroxide) in water (0. 2 % ammonium hydroxide) to give the title compound (16 mg, 31% Yield). δ_{H}(500 MHz, Methanol-d4) 8.18 (s, 1H), 4.45 - 4.37 (m, 1H), 4.36 - 4.30 (m, 1H), 3.68 (dd, J = 10.6, 6.0 Hz, 1H), 3.59 - 3.52 (m, 1H), 3.45 - 3.39 (m, 1H), 3.37 - 3.34 (m, 1H), 3.31 - 3.17 (m, 3H), 3.03 - 2.86 (m, 6H), 2.42 - 2.33 (m, 1H), 2.33 - 2.25 (m, 2H), 2.16 - 2.07 (m, 1H), 1.87 - 1.78 (m, 1H), 1.15 - 1.06 (m, 1H), 1.04 - 0.93 (m, 4H), 0.55 - 0.48 (m, 2H), 0.30 - 0.23 (m, 2H). LCMS [M+H]⁺ 548, RT 1.90 minutes (Method 10).

### Example 268

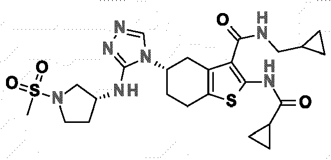

### (5S)-2-(cyclopropanecarbonylamino)-N-(cyclopropylmethyl)-5-[3-[[(3R)-1-methylsulfonylpyrrolidin-3-yl]aminol-1,2,4-triazol-4-yl]-4,5,6,7-tetrahydrobenzothiophene-3-carboxamide

Suspension of **intermediate 326** (50 mg, 0.092 mmol) was stirred in DCM (5 mL) was cooled to 0 °C then DIPEA (48.1 µl, 0.27 mmol) followed by methanesulfonyl methanesulfonate (14.4 mg, 0.083 mmol) were added. Reaction stirred for 2 h at 0 °C, then warmed to room temperature. Reaction mixture was diluted with DCM (20 mL) then water (15 mL) was added and organic layer separated. The aqueous layer was extracted with DCM (2 x 10 mL) and the organic fractions combined, dried over sodium sulphate and concentrated under vacuum. The residue was purified by HPLC (Method 4) to give the title compound (12 mg, 23% Yield). δ_{H} (500 MHz, Methanol-d4) 8.21 (s, 1H), 4.45 - 4.37 (m, 1H), 4.36 - 4.30 (m, 1H), 3.67 (dd, J = 10.6, 5.9 Hz, 1H), 3.59 - 3.52 (m, 1H), 3.45 - 3.39 (m, 1H), 3.36 (dd, J = 10.6, 4.2 Hz, 1H), 3.32 - 3.17 (m, 3H), 3.07 - 2.84 (m, 6H), 2.42 - 2.33 (m, 1H), 2.33 - 2.25 (m, 2H), 2.17 - 2.06 (m, 1H), 1.87 - 1.77 (m, 1H), 1.14 - 1.04 (m, 1H), 1.04 - 0.91 (m, 4H), 0.56 - 0.46 (m, 2H), 0.34 - 0.22 (m, 2H). LCMS [M+H]⁺ 548, RT 1.90 minutes (Method 10) .

### Example 269

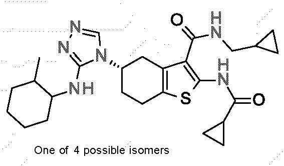

### (5S)-2-(cyclopropanecarbonylamino)-N-(cyclopropylmethyl)-5-[3-[(2-methylcyclohexyl)amino]-1,2,4-triazol-4-yl]-4,5,6,7-tetrahydrobenzothiophene-3-carboxamide *

*Absolute stereochemistry of the 2-methylcyclohexanamine group is unknown

**Intermediate 330** (49.0 mg, 0.099 mmol) was separated by chiral SFC (using 28% Methanol: 72% CO₂ with Chiralpak IC 25cm column at 15 mL/min) to give the title compound as one of 4 possible diastereoisomers (Peak 3, 12.9 mg, 19.6 % Yield). δ_{H} (500 MHz, Methanol-d4) 8.10 (s, 1H), 4.54 - 4.49 (m, 1H), 3.89 - 3.81 (m, 1H), 3.31 - 3.27 (m, 1H), 3.24 (dd, J = 15.5, 4.9 Hz, 1H), 3.18 (dd, J = 13.8, 7.0 Hz, 1H), 3.05 - 2.93 (m, 2H), 2.92 - 2.83 (m, 1H), 2.33 - 2.22 (m, 2H), 2.22 - 2.10 (m, 1H), 1.87 - 1.73 (m, 2H), 1.71 - 1.54 (m, 5H), 1.51 - 1.37 (m, 2H), 1.15 - 1.05 (m, 1H), 1.04 - 0.99 (m, 2H), 0.99 - 0.92 (m, 5H), 0.53 - 0.48 (m, 2H), 0.29 - 0.25 (m, 2H). LCMS [M+H]⁺ 497, RT 2.92 minutes (Method 10). Chiral SFC** RT = 34.91 minutes.

** Chiral analysis using Chiralpak IC 4.6 x 250 mm, 5 µm column, flow rate 4 mL/min, eluting with 72:28 CO₂:Methanol, 50 minutes run time on a WatersThar Resolution - 3100 MS.

### Example 270

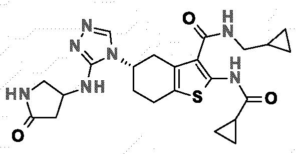

### (5S)-2-(cyclopropanecarbonylamino)-N-(cyclopropylmethyl)-5-[3-[(5-oxopyrrolidin-3-yl)amino]-1,2,4-triazol-4-yl]-4,5,6,7-tetrahydrobenzothiophene-3-carboxamide

The title compound was obtained following **general method** 5 with **intermediate 333** (192 mg, 0.30 mmol) and TFA (231.8 µl, 3.0 mmol). Purification by HPLC (Method 4) gave the title compound (78 mg, 47% Yield). δ_{H} (500 MHz, Methanol-d4) 8.20 (s, 1H), 4.54 - 4.45 (m, 1H), 4.44 - 4.36 (m, 1H), 3.86 - 3.79 (m, 1H), 3.40 - 3.34 (m, 1H), 3.30 - 3.14 (m, 3H), 3.05 - 2.79 (m, 4H), 2.46 - 2.35 (m, 1H), 2.34 - 2.20 (m, 2H), 1.87 - 1.79 (m, 1H), 1.13 - 1.05 (m, 1H), 1.04 - 0.99 (m, 2H), 0.99 - 0.92 (m, 2H), 0.54 - 0.48 (m, 2H), 0.30 - 0.23 (m, 2H). LCMS [M+H]⁺ 484, RT 1.64 minutes (Method 10).

### Example 271

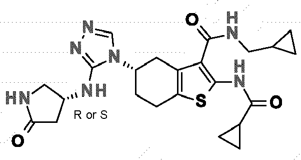

### (5S)-2-(cyclopropanecarbonylamino)-N-(cyclopropylmethyl)-5-[3-[[(3R*)-5-oxopyrrolidin-3-yl]amino]-1,2,4-triazol-4-yl]-4,5,6,7-tetrahydrobenzothiophene-3-carboxamide (*or S)

**Example 270** (65 mg, 0.134 mmol) was separated by chiral chromatography (60:40 Ethanol: Methanol with a Cellulose-4 25 cm column at 8 mL/min) to give the title compound (18.3 mg, 28 % Yield). δ_{H} (500 MHz, Methanol-d4) 8.18 (s, 1H), 4.54 - 4.46 (m, 1H), 4.43 - 4.32 (m, 1H), 3.83 (dd, J = 10.7, 6.8 Hz, 1H), 3.36 (dd, J = 10.7, 3.7 Hz, 1H), 3.31 - 3.14 (m, 3H), 3.06 - 2.87 (m, 3H), 2.83 (dd, J =17.3, 8.1 Hz, 1H), 2.40 (dd, J = 17.3, 4.5 Hz, 1H), 2.33 - 2.23 (m, 2H), 1.86 - 1.78 (m, 1H), 1.14 - 1.04 (m, 1H), 1.04 - 0.99 (m, 2H), 0.99 - 0.92 (m, 2H), 0.55 - 0.47 (m, 2H), 0.29 - 0.23 (m, 2H). LCMS [M+H]⁺ 484, RT 1.63 minutes (Method 10).Chiral LC** RT = 6.03 minutes.

** Chiral analysis using Cellulose-4 (4.6 x 250 mm 5 µm) column, flow rate 1 mL/min, eluting with 60:40 Ethanol:Methanol, 10 minutes run time on a Waters 2795.

### Example 272

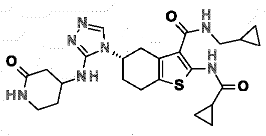

### (5S)-2-(cyclopropanecarbonylamino)-N-(cyclopropylmethyl)-5-[3-[(2-oxo-4-piperidyl)amino]-1,2,4-triazol-4-yl]-4,5,6,7-tetrahydrobenzothiophene-3-carboxamide

The title compound was obtained following **general method 5** with **intermediate 336** (184 mg, 0.27 mmol) and TFA (210.8 µl, 3.0 mmol). Purification by HPLC (Method 4) gave the title compound (52 mg, 38% Yield). δ_{H} (500 MHz, Methanol-d4) 8.20 (s, 1H), 4.48 - 4.36 (m, 1H), 4.13 - 3.99 (m, 1H), 3.43 - 3.35 (m, 2H), 3.31 - 3.15 (m, 3H), 3.07 - 2.86 (m, 3H), 2.80 (dd, J = 17.5, 4.6 Hz, 1H), 2.47 - 2.35 (m, 1H), 2.33 - 2.20 (m, 3H), 1.90 - 1.77 (m, 2H), 1.14 - 1.06 (m, 1H), 1.03 - 0.99 (m, 2H), 0.98 - 0.94 (m, 2H), 0.55 - 0.48 (m, 2H), 0.30 - 0.24 (m, 2H). LCMS [M+H]⁺ 498, RT 1.60 minutes (Method 10).

### Example 273

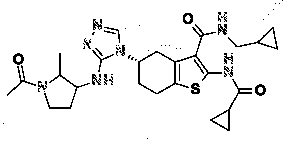

### (5S)-5-[3-[(1-acetyl-2-methyl-pyrrolidin-3-yl)Jamino]-1,2,4-triazol-4-yl]-2-(cyclopropanecarbonylamino)-N-(cyclopropylmethyl)-4,5,6,7-tetrahydrobenzothiophene-3-carboxamide

Suspension of **intermediate 340** (265 mg, 0.452 mmol) was stirred in DCM (20 mL) was cooled to 0 °C. DIPEA (236.3 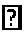 µL, 1.35 mmol) followed by acetic anhydride (38.4 µL, 0.407 mmol) were then added. Reaction stirred for 1 h at 0 °C then warmed to room temperature. Reaction mixture was diluted with DCM then water (25 mL) was added and the organic layer separated. The aqueous layer was extracted with DCM (2 x 20 mL) and the organic extracts combined, dried over sodium sulphate and concentrated under vacuum. The residue was purified by column chromatography eluting with 0 to 30% MeOH in DCM to give the title compound (169 mg, 68% Yield). δ_{H} (500 MHz, Methanol-d4) 8.23 - 8.06 (m, 1H), 4.58 - 4.46 (m, 1H), 4.42 (dt, J = 9.2, 4.3 Hz, 1H), 4.27 - 4.08 (m, 1H), 3.98 (dd, J = 28.0, 3.6 Hz, 1H), 3.76 - 3.60 (m, 2H), 3.57 - 3.41 (m, 1H), 3.29 -3.12 (m, 3H), 3.05 - 2.80 (m, 3H), 2.28 (tt, J = 14.1, 5.9 Hz, 3H), 2.21 - 2.07 (m, 2H), 2.07 - 1.99 (m, 2H), 1.81 (td, J = 7.7, 4.3 Hz, 1H), 1.39 - 1.25 (m, 5H), 1.08 - 0.92 (m, 7H), 0.53 - 0.44 (m, 2H), 0.28 - 0.20 (m, 2H). LCMS [M+H]⁺ 526, RT 1.76 minutes (Method 10).

### Example 274

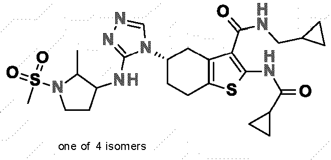

### (5S)-2-(cyclopropanecarbonylamino)-N-(cyclopropylmethyl)-5-[3-[(2-methyl-1-methylsulfonyl-pyrrolidin-3-yl)aminol-1,2,4-triazol-4-yl]-4,5,6,7-tetrahydrobenzothiophene-3-carboxamide

*Absolute stereochemistry of the 2-methyl-1-methylsulfonyl-pyrrolidin-3-amine group is unknown

**Intermediate 341** (135 mg, 0.24 mmol) was separated by SFC (Chiralcel OJ-H 25cm column, with 10% Methanol + 0.2% DEA : 90% CO₂ at 15 mL/min) to give the title compound as a single isomer (8.5 mg, 6 % Yield). δ_{H} (500 MHz, Methanol-d4) 8.18 (s, 1H), 4.49 - 4.35 (m, 1H), 4.26 - 4.14 (m, 2H), 3.53 - 3.45 (m, 1H), 3.30 - 3.24 (m, 2H), 3.20 (dd, J = 15.4, 5.1 Hz, 1H), 3.14 (dd, J = 13.8, 7.0 Hz, 1H), 3.03 - 2.98 (m, 1H), 2.97 (s, 3H), 2.96 - 2.85 (m, 2H), 2.36 - 2.24 (m, 3H), 2.21 - 2.08 (m, 1H), 1.84 - 1.76 (m, 1H), 1.10 - 1.02 (m, 4H), 1.02 - 0.98 (m, 2H), 0.97 - 0.89 (m, 2H), 0.56 - 0.41 (m, 2H), 0.28 - 0.21 (m, 2H). LCMS [M+H]⁺ 562, RT 2.09 minutes (Method 10). Chiral SFC** RT = 10.36 minutes.

** Chiral analysis using Chiralpak AS-H 4.6 x 250 mm, 5 µm column, flow rate 4 mL/min, eluting with 30% Methanol: 70% CO₂, 30 minutes run time on a WatersThar Resolution - 3100 MS.

### Example 275

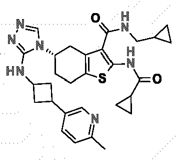

### (5S)-2-(cyclopropanecarbonylamino)-N-(cyclopropylmethyl)-5-[3-[[3-(6-methyl-3-pyridyl)cyclobutyl]amino]-1,2,4-triazol-4-yl]-4,5,6,7-tetrahydrobenzothiophene-3-carboxamide

The title compound was obtained following **general method 5** with **intermediate 344** (160 mg, 0.23 mmol) and TFA (175.9 µl, 2.29 mmol). Purification by HPLC (Method 4) gave the title compound (91 mg, 72% Yield). δ_{H} (500 MHz, Methanol-d4) 8.35 (d, J = 2.3 Hz, 1H, isomer), 8.31 (d, J = 2.2 Hz, 1H, isomer), 8.17 (s, 1H, isomer), 8.15 (s, 1H, isomer), 7.83 (dd, J = 8.1, 2.3 Hz, 1H, isomer), 7.71 (dd, J = 8.1, 2.3 Hz,1H, isomer), 7.33 (d, J = 8.1 Hz, 1H, isomer), 7.28 (d, J = 8.1 Hz, 1H, isomer), 4.50 - 4.40 (m, 1H, isomer), 4.40 - 4.30 (m, 1H), 4.27 - 4.17 (m, 1H, isomer), 3.74 - 3.66 (m, 1H, isomer), 3.30 - 3.20 (m, 2H, 1H, isomer), 3.20 - 3.12 (m, 1H), 3.05 - 2.86 (m, 4H), 2.65 - 2.54 (m, 2H), 2.53 (s, 3H, isomer), 2.50 (s, 3H, isomer), 2.37 - 2.22 (m, 2H), 2.18 - 2.07 (m, 1H), 1.86 - 1.76 (m, 1H), 1.13 - 1.03 (m, 1H), 1.02 - 0.91 (m, 4H), 0.52 - 0.43 (m, 2H), 0.31 - 0.18 (m, 2H). LCMS [M+H]⁺ 546, RT 1.38 minutes (Method 10).

### Example 276 and 277

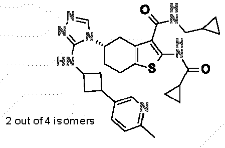

### (5S)-2-(cyclopropanecarbonylamino)-N-(cyclopropylmethyl)-5-[3-[[3-(6-methyl-3-pyridyl)cyclobutyl]amino]-1,2,4-triazol-4-yl]-4,5,6,7-tetrahydrobenzothiophene-3-carboxamid.

* Absolute stereochemistry of the 3-(6-methyl-3-pyridyl)cyclobutanamine group in both isolated isomers is unknown

**Example 275** (49.0 mg, 0.089 mmol) was separated by chiral liquid chromatography (eluting with 85:15 Heptane: Ethanol using Chiralcel OD-H 25 cm column at 18 mL/min) to give two out of four possible diastereosiomers.

Isomer 1 (Peak 1, 7.5 mg, 15 % Yield). δ_{H} 500 MHz, Methanol-d4) 8.29 (d, J = 2.2 Hz, 1H), 8.12 (s, 1H), 7.67 (dd, J = 8.0, 2.3 Hz, 1H), 7.24 (d, J = 8.0 Hz, 1H), 4.43 - 4.30 (m, 1H), 4.27 - 4.14 (m, 1H), 3.29 - 3.20 (m, 3H), 3.15 (dd, J = 13.8, 7.0 Hz, 1H), 3.03 - 2.84 (m, 5H), 2.48 (s, 3H), 2.34 - 2.24 (m, 2H), 2.18 - 2.05 (m, 2H), 1.85 - 1.75 (m, 1H), 1.10 - 1.03 (m, 1H), 1.04 - 0.98 (m, 2H), 0.97 - 0.92 (m, 2H), 0.51 - 0.43 (m, 2H), 0.27 - 0.19 (m, 2H). LCMS [M+H]⁺ 546, RT 1.37 minutes (Method 10). Chiral LC** RT = 26.23 minutes.

Isomer 2 (peak 2, 8.7 mg, 18 % Yield). δ_{H} (500 MHz, Methanol-d4) 8.32 (d, J = 2.3 Hz, 1H), 8.14 (s, 1H), 7.78 (dd, J = 8.1, 2.3 Hz, 1H), 7.29 (d, J = 8.1 Hz, 1H), 4.46 - 4.39 (m, 1H), 4.38 - 4.32 (m, 1H), 3.73 - 3.62 (m, 1H), 3.30 - 3.23 (m, 2H), 3.16 (dd, J = 13.8, 7.0 Hz, 1H), 3.04 - 2.94 (m, 2H), 2.93 - 2.86 (m, 1H), 2.65 - 2.52 (m, 4H), 2.51 (s, 3H), 2.35 - 2.25 (m, 2H), 1.87 - 1.76 (m, 1H), 1.11 - 1.05 (m, 1H), 1.02 - 0.97 (m, 2H), 0.97 - 0.93 (m, 2H), 0.53 - 0.45 (m, 2H), 0.31 - 0.21 (m, 2H). LCMS [M+H]⁺ 546, RT 1.40 minutes (Method 10). Chiral LC** RT = 31.22 minutes.

** Chiral analysis using Chiralcel OD-H (4.6 x 250 mm 5 µm) column, flow rate 1 mL/min, eluting with 85:15 Heptane:Ethanol, 120 minutes run time on a Waters 2795

### Example 285

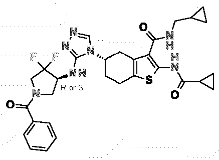

### (5S)-5-[3-[[(3S*)-1-benzoyl-4,4-difluoro-pyrrolidin-3-yl]amino]-1,2,4-triazol-4-yl]-2-(cyclopropanecarbonylamino)-N-(cyclopropylmethyl)-4,5,6,7-tetrahydrobenzothiophene-3-carboxamide [* or R]

To a solution of **Intermediate 356** (69 mg, 0.14 mmol) in DCM (2 mL) and DMF (0.2 mL) was added DIPEA (0.07 mL, 0.41 mmol) and benzoyl chloride (0.01 mL, 0.12 mmol). The solution was stirred at room temperature for 20 minutes. The solvent was removed to give a pale brown oil. Purification by flash column chromatography eluting with 0 to 10% MeOH in DCM gradient gave the racemic compound (58 mg, 70 % Yield). The two diastereosiomers were separated by reverse phase chiral chromatography (Waters Xbridge column 100 x 19 mm, 5 µm, 5-95% CH₃CN with 0.2 % NH₄OH in water over 27.5 minutes) to give the title compound (15.4 mg, 30% Yield). δ_{H} (500 MHz, Chloroform-d) 11.89 (s, 1H), 7.81 (s, 1H), 7.51 - 7.32 (m, 5H), 5.97 - 5.60 (2 x m, 1H, rotamers), 5.04 - 4.48 (m, 2H, rotamers), 4.35 - 3.79 (m, 4H, rotamers), 3.77 - 3.46 (m, 1H), 3.39 - 3.09 (m, 3H), 2.95 - 2.76 (m, 3H), 2.35 - 2.11 (m, 2H), 1.65 (tt, J = 8.0, 4.6 Hz, 1H), 1.14 - 1.06 (m, 2H), 1.06 - 0.93 (m, 1H), 0.94 - 0.76 (m, 2H), 0.58 - 0.42 (m, 2H), 0.22 (d, J = 4.8 Hz, 2H). LCMS [M+H]⁺ 610, RT 2.61 minutes (Method 10).

### Example 286

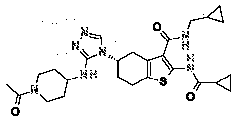

### (5S)-5-[3-[(1-acetyl-4-piperidyl)amino]-1,2,4-triazol-4-yl]-2-(cyclopropanecarbonylamino)-N-(cyclopropylmethyl)-4,5,6,7-tetrahydrobenzothiophene-3-carboxamide

To a solution of **Intermediate 360** (41 mg, 0.08 mmol) in DCM (3 mL) and DMF (0.2 mL) was added DIPEA (0.04 mL, 0.25 mmol) and acetic anhydride (0.01 mL, 0.08 mmol). The reaction was stirred for 30 minutes. The DCM was removed to give an oil. Purification by flash column chromatography eluting with 0 to 10% 7 M NH₃ in MeOH in DCM gradient gave the title compound (41 mg, 89% Yield). δ_{H} (500 MHz, Methanol-d4) 8.12 (s, 1H), 4.51 - 4.43 (m, 1H), 4.40 - 4.28 (m, 1H), 4.00 - 3.88 (m, 1H), 3.83 - 3.76 (m, 1H), 3.30 - 3.13 (m, 4H), 3.03 - 2.92 (m, 2H), 2.92 - 2.78 (m, 2H), 2.32 - 2.23 (m, 2H), 2.22 - 2.15 (m, 1H), 2.15 - 2.06 (m, 4H), 1.87 - 1.75 (m, 1H), 1.55 - 1.36 (m, 2H), 1.13 - 1.03 (m, 1H), 1.03 - 0.90 (m, 4H), 0.54 - 0.43 (m, 2H), 0.31 - 0.20 (m, 2H). LCMS [M+H]⁺ 526, RT 2.35 minutes (Method 32).

### Example 287

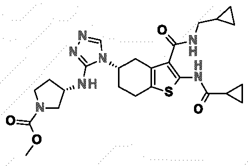

### Methyl (3S)-3-[[4-[(5S)-2-(cyclopropanecarbonylamino)-3-(cyclopropylmethylcarbamoyl)-4,5,6,7-tetrahydrobenzothiophen-5-yl]-1,2,4-triazol-3-yl]amino]pyrrolidine-1-carboxylate

To a solution of **Intermediate 278** (62 mg, 0.07 mmol) in DCM (5 mL) was added DIPEA (35 µl, 0.2 mmol) and dimethyl dicarbonate (5.66 µl, 0.05 mmol). The mixture was stirred for 18 hours. The reaction mixture was wet loaded directly onto a column and purified by flash column chromatography eluting with 0 to 10% 7 M NH₃ in MeOH in DCM gradient to give the title compound as a gum (40 mg). LCMS showed it to be a mixture of two products. Further purification by HPLC (Method 4) gave the title compound (2.6 mg, 7% Yield). δ_{H} (500 MHz, Methanol-d4) 8.16 (s, 1H), 4.44 - 4.34 (m, 1H), 4.34 - 4.24 (m, 1H), 3.77 - 3.70 (m, 1H), 3.68 (s, 3H), 3.59 - 3.45 (m, 2H), 3.43 - 3.36 (m, 1H), 3.28 - 3.14 (m, 3H), 2.99 - 2.84 (m, 3H), 2.31 - 2.22 (m, 3H), 2.10 - 1.98 (m, 1H), 1.81 (ddd, J = 12.6, 7.9, 4.6 Hz, 1H), 1.07 (ddd, J = 12.5, 7.6, 4.9 Hz, 1H), 0.97 (ddt, J = 23.8, 5.9, 3.8 Hz, 4H), 0.54 - 0.42 (m, 2H), 0.25 (q, J = 4.6 Hz, 2H). LCMS [M+H]⁺ 528, RT 2.56 minutes (Method 32).

### Example 288

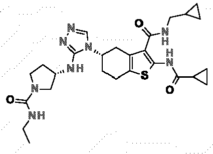

### (3S)-3-[[4-[(5S)-2-(cyclopropanecarbonylamino)-3-(cyclopropylmethylcarbamoyl)-4,5,6,7-tetrahydrobenzothiophen-5-yl]-1,2,4-triazol-3-yl]amino]-N-ethyl-pyrrolidine-1-carboxamide

To a solution of **Intermediate 278** (30 mg, 0.06 mmol) in DCM (3 mL) was added triethylamine (19 mg, 0.19 mmol). The solution was cooled to 0 °C then isocyanatomethane (3.96 µl, 0.06 mmol) was added. The reaction was stirred at 0 °C for 5 minutes then at room temperature for 1 hour. The solvent was removed to give a brown solid. Purification by flash column chromatography eluting with 0 to 20% 7 M NH₃ in MeOH in DCM gradient gave the title compound (25 mg, 68% Yield). δ_{H} (500 MHz, Methanol-d4) 8.15 (s, 1H), 6.14 (t, J = 5.5 Hz, 1H), 4.43 - 4.35 (m, 1H), 4.35 - 4.22 (m, 1H), 3.75 - 3.65 (m, 1H), 3.54 - 3.38 (m, 2H), 3.27 - 3.21 (m, 2H), 3.21 - 3.11 (m, 4H), 3.03 - 2.83 (m, 3H), 2.32 - 2.21 (m, 3H), 2.10 - 2.02 (m, 1H), 1.86 - 1.76 (m, 1H), 1.14 - 1.03 (m, 4H), 1.03 - 0.90 (m, 4H), 0.55 - 0.43 (m, 2H), 0.28 - 0.18 (m, 2H). LCMS [M+H]⁺ 541, RT 2.37 minutes (Method 32).

### Examples 289, 290 & 291

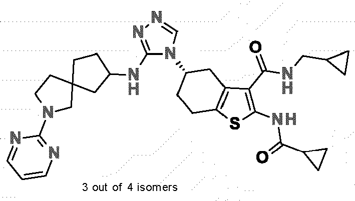

### (5S)-2-cyclopropaneamido-N-(cyclopropylmethyl)-5-(3-H2-(pyrimidin-2-yl)-2-azaspiro[4.4]nonan-7-yl]amino}-4H-1,2,4-triazol-4-yl)-4,5,6,7-tetrahydro-1-benzothiophene-3-carboxamide

To a solution of **Intermediate 364** (200 mg, 0.38 mmol) in N,N-dimethylformamide (5 mL) was introduced diisopropylethylamine (148 mg, 1.14 mmol) and 2-chloropyrimidine (58 mg, 0.49 mmol, pre-dissolved in N,N-dimethylformamide [1 mL]). The resulting solution was warmed to 110 °C for 2 hours, concentrated in-vacuo and the residue suspended in a mixture of acetonitrile/water/DMSO (95:5:5 ratio, 3 mL). The insoluble solid was removed by filtration and the filtrate was purified by low pH achiral preparative-LC chromatography (Method 3) to furnish the title compound (100 mg at 93% purity LCMS-UV₂₁₅, 0.155 mmol) as a mixture of four diastereomers. Diastereomer separation by chiral chromatography (Cellulose 4 [25 cm column] with 100% methanol followed by a Chiralcel-OD [25 cm column] using 1:1 methanol/ethanol) furnished all four diastereomers (three are reported below):
**Example 289** - Peak 2 from initial separation on the cellulose column (5.5 mg, 2% Yield). 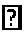 δ_{H} [500 MHz, d₄-Methanol] 8.27 (d, J = 4.8 Hz, 2H), 8.11 (s, 1H), 6.56 (t, J = 4.8 Hz, 1H), 4.43 - 4.35 (m, 1H), 4.20 (p, J = 6.9 Hz, 1H), 3.64 - 3.54 (m, 2H), 3.51 (d, J = 10.8 Hz, 1H), 3.47 (d, J = 10.8 Hz, 1H), 3.30 - 3.26 (m, 1H), 3.23 (dd, J = 15.1, 4.9 Hz, 1H), 3.15 (dd, J = 13.8, 7.0 Hz, 1H), 2.97 (dt, J = 17.5, 9.1 Hz, 2H), 2.87 (td, J = 16.4, 5.1 Hz, 1H), 2.31 - 2.23 (m, 3H), 2.20 (dd, J = 13.3, 7.3 Hz, 1H), 2.01 -1.92 (m, 2H), 1.85 - 1.77 (m, 2H), 1.76 - 1.67 (m, 3H), 1.11 - 1.04 (m, 1H), 1.01 - 0.97 (m, 2H), 0.97 - 0.93 (m, 2H), 0.51 - 0.46 (m, 2H), 0.24 (q, J = 4.7 Hz, 2H). LCMS [M+H]+ 602, RT 1.94 mins (Method 10). Chiral LC* RT = 34.12 minutes.
**Example 290** - Peak 1 from initial separation on the cellulose column and peak 2 on the Chiralcel-OD column (7.1 mg, 3% Yield). δ_{H} [500, d₄-Methanol] 8.29 (d, J = 4.8 Hz, 2H), 8.10 (s, 1H), 6.57 (t, J = 4.8 Hz, 1H), 4.43 - 4.30 (m, 1H), 4.18 (p, J = 7.2 Hz, 1H), 3.56 (tt, J = 11.1, 5.5 Hz, 2H), 3.48 (d, J = 10.7 Hz, 1H), 3.43 (d, J =10.7 Hz, 1H), 3.27 (dd, J = 13.8, 7.1 Hz, 1H), 3.21 (dd, J = 15.3, 5.0 Hz, 1H), 3.15 (dd, J = 13.8, 6.9 Hz, 1H), 2.95 (dt, J = 15.3, 7.8 Hz, 2H), 2.87 (d, J = 16.2 Hz, 1H), 2.30 - 2.20 (m, 4H), 2.00 (tq, J = 9.6, 5.2 Hz, 2H), 1.87 - 1.70 (m, 4H), 1.65 (dd, J = 13.2, 7.4 Hz, 1H), 1.06 (dt, J = 12.0, 6.2 Hz, 1H), 1.01 - 0.90 (m, 4H), 0.47 (q, J = 5.2 Hz, 2H), 0.23 (q, J = 4.8 Hz, 2H); LCMS [M+H]⁺ 602, RT 1.94 mins (Method 10) Chiral LC** RT = 6.46 minutes.
**Example 291** Peak 1 from initial separation on the cellulose column and peak 1 on the Chiralcel-OD column (1.1 mg, 0.5% Yield). δ_{H} [500 MHz, d₄-Methanol] 8.29 (d, J = 4.8 Hz, 2H), 8.10 (s, 1H), 6.58 (t, J = 4.8 Hz, 1H), 4.43 - 4.32 (m, 1H), 4.19 (p, J = 7.2 Hz, 1H), 3.58 (tq, J = 8.7, 4.1 Hz, 2H), 3.48 (d, J = 10.8 Hz, 1H), 3.44 (d, J = 10.7 Hz, 1H), 3.28 (d, J = 7.1 Hz, 1H), 3.23 (dd, J = 15.1, 5.1 Hz, 1H), 3.16 (dd, J = 13.8, 7.0 Hz, 1H), 2.99 - 2.93 (m, 2H), 2.88 (dt, J = 16.2, 4.5 Hz, 1H), 2.31 - 2.22 (m, 4H), 2.07 - 1.95 (m, 2H), 1.87 - 1.70 (m, 4H), 1.66 (dd, J = 13.3, 7.4 Hz, 1H), 1.11 - 1.06 (m, 1H), 1.02 - 0.97 (m, 2H), 0.95 (dt, J = 7.9, 3.0 Hz, 2H), 0.52 - 0.46 (m, 2H), 0.28 - 0.23 (m, 2H); LCMS [M+H]⁺ 602, RT 1.97 mins (Method 10). Chiral LC** RT = 5.02 minutes.
* Chiral analysis using Cellulose 4 - 4.6 x 250 mm, 5µm column, flow rate 0.3 mL/min eluting with methanol, 50 minute run time on a Waters 2795
** Chiral analysis using Chiralcel OD - 4.6 x 250 mm, 5µm column, flow rate 1 mL/min eluting with 1:1 ethanol/methanol, 15 minute run time on a Waters 2795

### Examples 294 and 295

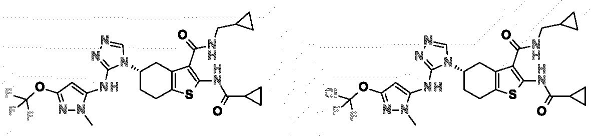

### (5S)-2-(cyclopropanecarbonylamino)-N-(cyclopropylmethyl)-5-[3-[[2-methyl-5-(trifluoromethoxy)pyrazol-3-yl]amino]-1,2,4-triazol-4-yl]-4,5,6,7-tetrahydrobenzothiophene-3-carboxamide (294) &

### (5S)-5-[3-[[5-[chloro(difluoro)methoxy]-2-methyl-pyrazol-3-yl]amino]-1,2,4-triazol-4-yl]-2-(cyclopropanecarbonylamino)-N-(cyclopropylmethyl)-4,5,6,7-tetrahydrobenzothiophene-3-carboxamide (295)

A solution of formic hydrazide (35 mg, 0.57 mmol) in ethanol (3 mL) was introduced in one portion to a mixture of **Intermediates 382** and **383** (121 mg, 0.18 mmol). The resulting solution was stirred at room temperature for 90 minutes. 1 M aqueous sodium carbonate (0.57 mL, 0.57 mmol) was introduced in one portion and the reaction mixture stirred at 40 °C for 16 hours. The reaction mixture was concentrated in-vacuo, then the residue was diluted with water (10 mL) and the precipitated solid collected by filtration and washed with water (3 mL). Purification of the solid by HPLC eluting with 5 - 95 % acetonitrile (with 0.1 % formic acid) in water (with 0.1 % formic acid) gave the title compounds:
**Example 294** (9.7 mg, 9 % Yield). δ_{H} (500 MHz, d-chloroform) 11.98 (s, 1H), 7.47 (s, 1H), 5.79 (t, J = 5.2 Hz, 1H), 5.55 (s, 1H), 4.66 - 4.56 (m, 1H), 3.64 (s, 3H), 3.37 (dd, J = 14.6, 4.8 Hz, 1H), 3.33 - 3.21 (m, 2H), 2.96 - 2.87 (m, 2H), 2.82 (dt, J = 16.8, 5.1 Hz, 1H), 2.34 (m, 1H), 2.28 - 2.19 (m, 1H), 1.67 (td, J = 7.9, 4.0 Hz, 1H), 1.10 (h,J = 4.6 Hz, 2H), 1.03 (dddd, J = 15.1, 12.3, 7.5, 4.9 Hz, 1H), 0.92 (dt, J = 7.8, 3.7 Hz, 2H), 0.56 - 0.49 (m, 2H), 0.23 (q, J = 4.7 Hz, 2H); δ_{F} (376 MHz, d-chloroform, H-decoupled) - 58.93; LCMS [M+H]⁺ 565, RT 3.06 mins (Method 10).
**Example 295** (3.2 mg, 3 % Yield). δ_{H} (500 MHz, d-chloroform) 12.03 (s, 1H), 9.17 (s, 1H), 7.43 (s, 1H), 5.75 (t, J = 5.2 Hz, 1H), 5.55 (s, 1H), 4.65 (d, J = 6.3 Hz, 1H), 3.67 (s, 3H), 3.41 (dd,J = 14.7, 4.8 Hz, 1H), 3.34 - 3.21 (m, 2H), 2.95 - 2.89 (m, 2H), 2.82 (dt, J = 16.9, 5.4 Hz, 1H), 2.40 - 2.33 (m, 1H), 2.28 - 2.19 (m, 1H), 1.68 (ddd, J = 12.5, 8.0, 4.6 Hz, 1H), 1.12 (ddd, J = 7.0, 4.3, 3.0 Hz, 2H), 1.03 (tt, J = 7.9, 5.1 Hz, 1H), 0.96 - 0.89 (m, 2H), 0.57 - 0.48 (m, 2H), 0.24 (q, J = 4.7 Hz, 2H); δ_{F} (376 MHz, d-chloroform, H-decoupled) -27.10; LCMS [M+H]⁺ 581/583, RT 3.16 mins (Method 10).

### Example 296

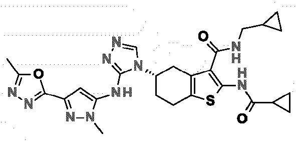

### (5S)-2-cyclopropaneamido-N-(cyclopropylmethyl)-5-(3-(5-methyl-3-(5-methyl-1,3,4-oxadiazol-2-yl)-1H-pyrazol-5-yl]amino}-4H-1,2,4-triazol-4-yl)-4,5,6,7-tetrahydro-1-benzothiophene-3-carboxamide

To a solution of **Intermediate 388** (240 mg, 0.41 mmol) in methanol (16 mL) was introduced formic hydrazide (111 mg, 1.84 mmol). After 40 minutes at room temperature, 1 M aqueous sodium carbonate (1.5 mL, 1.5 mmol) was introduced and the solution stirred at 50 °C for 4 hours. The reaction mixture was concentrated in-vacuo and the residue suspended in water (8 mL) and extracted with a mixture of tetrahydrofuran and ethyl acetate (1:1, 3 x 20 mL). The combined organic extracts were washed with brine (10 mL) and dried over sodium sulfate. After filtration, the filtrate was adsorbed onto silica in-vacuo and the dry-loaded material partially purified by flash column chromatography (dichloromethane containing 0-5% methanol and 0-3% 7 M ammonia in methanol). The product from flash column chromatography was further purified by HPLC (Method 2) to furnish the title compound (35 mg, 15% Yield) δ_{H} (500 MHz, d₄-methanol) 8.15 (s, 1H), 6.60 (s, 1H), 4.62 - 4.53 (m, 1H), 3.80 (s, 3H), 3.37 - 3.32 (m, 1H), 3.27 (dd, J = 13.8, 7.0 Hz, 1H), 3.18 (dd, J = 13.8, 7.0 Hz, 1H), 3.07 (dd, J = 15.4, 9.2 Hz, 1H), 2.98 (dt, J = 16.0, 8.0 Hz, 1H), 2.90 (dt, J = 17.1, 4.6 Hz, 1H), 2.60 (s, 3H), 2.39 - 2.31 (m, 2H), 1.81 (ddd, J = 12.6, 7.9, 4.7 Hz, 1H), 1.12 - 1.02 (m, 1H), 1.02 - 0.97 (m, 2H), 0.94 (ddt, J = 7.5, 4.7, 2.2 Hz, 2H), 0.52 - 0.43 (m, 2H), 0.28 - 0.21 (m, 2H); LCMS [M+H]⁺ 563, RT 2.41 mins (Method 10).

### Example 297

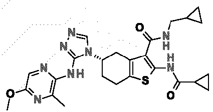

### (5S)-2-(cyclopropanecarbonylamino)-N-(cyclopropylmethyl)-5-[3-[(5-methoxy-3-methyl-pyrazin-2-yl)aminol-1,2,4-triazol-4-yl]-4,5,6,7-tetrahydrobenzothiophene-3-carboxamide

To a stirred solution of **intermediate 390** (180 mg, 0.349 mmol) in DMF (3.5 mL) under nitrogen was added formic acid hydrazide (63 mg, 1.1 mmol) and mercuric chloride (114 mg, 0.419 mmol). The reaction mixture was stirred at room temperature for 15 min before the addition of triethylamine (0.15 mL, 1.1 mmol) and the mixture was stirred at 60 °C for 3 hours. Additional mercuric chloride (114 mg, 0.419 mmol) was added and the reaction stirred for a further 16 hours at 60 °C. The mixture was cooled to r.t. and diluted with MeCN (15 mL), filtered through a pad of celite and washed with MeCN (20 mL). The filtrate was concentrated in vacuo to yield the crude product which was dissolved in 5% MeOH in DCM (50 mL), washed with water (30 mL) and the layers separated. The aqueous layer was extracted with 5% MeOH in DCM (2 x 25 mL). The combined organic layers were passed through a phase separation cartridge and concentrated in vacuo to give crude product which was purified by reverse phase HPLC to afford the title compound (27 mg, 15 % Yield). δ_{H} (400 MHz, d-Chloroform) 12.13 (s, 1H), 7.69 (s, 1H), 7.56 (s, 1H), 5.95 - 5.86 (m, 1H), 4.90 - 4.77 (m, 1H), 3.94 (s, 3H), 3.56 (dd, J = 14.9, 5.5 Hz, 1H), 3.33 - 3.25 (m, 2H), 3.06 - 2.91 (m, 2H), 2.86 (dt, J = 16.8, 5.2 Hz, 1H), 2.57 (s, 3H), 2.49 - 2.38 (m, 1H), 2.35 - 2.25 (m, 1H), 1.71 (tt, J = 8.2, 4.5 Hz, 1H), 1.18 - 1.12 (m, 2H), 1.11 - 0.99 (m, 1H), 0.98 - 0.91 (m, 2H), 0.56 - 0.48 (m, 2H), 0.28 - 0.20 (m, 2H). LCMS [M+H]⁺ 523, RT 1.93 minutes (Method 26).

### Example 298

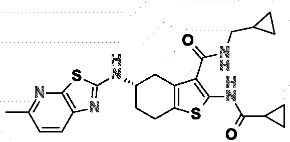

### (5S)-2-(cyclopropanecarbonylamino)-N-(cyclopropylmethyl)-5-[(5-methylthiazolo[5,4-b]pyridin-2-yl)amino]-4,5,6,7-tetrahydrobenzothiophene-3-carboxamide

Methanesulfonyl chloride (0.042 mL, 0.54 mmol) was added dropwise to a solution of **intermediate 392** (255 mg, 0.493 mmol) and triethylamine (0.21 mL, 1.5 mmol) in DCM (4.9 mL) at 0 °C and under nitrogen. The reaction was stirred with warming to room temperature for 0.5 hours. The reaction was diluted with DCM (20 mL) and washed with sat. aqueous NH₄Cl (20 mL). The layers were separated and the aqueous layer extracted with DCM (2 x 20 mL). The combined organic layers were passed through a phase separation cartridge and concentrated in vacuo. This material was dissolved in DMF (2.5 mL) and formic acid hydrazide (33 mg, 0.54 mmol) was added. The reaction was stirred at room temperature for 2 hours. Water (20 mL) and DCM (20 mL) were added and the layers separated. The aqueous layer was extracted with DCM (2 x 20 mL). The combined organic layers were passed through a phase separation cartridge and concentrated in vacuo to give crude product which was purified by flash column chromatography on silica (gradient elution with 0% to 100% EtOAc in isohexanes then 0% to 20% MeOH in EtOAc) to afford the title compound (78 mg, 33 % Yield). δ_{H} (300 MHz, DMSO-d6) 11.11 (s, 1H), 8.32 (d, J = 6.9 Hz, 1H), 7.73 (t, J = 5.6 Hz, 1H), 7.57 (d, J = 8.1 Hz, 1H), 7.10 (d, J = 8.4 Hz, 1H), 4.28 - 4.10 (m, 1H), 3.25 - 3.05 (m, 3H), 2.87 - 2.62 (m, 3H), 2.45 (s, 3H), 2.20 - 2.06 (m, 1H), 2.01 - 1.81 (m, 2H), 1.08 - 0.93 (m, 1H), 0.92 - 0.75 (m, 4H), 0.42 - 0.31 (m, 2H), 0.24 - 0.12 (m, 2H). LCMS [M+H]⁺ 482, RT 2.19 minutes (Method 26).

### Example 299

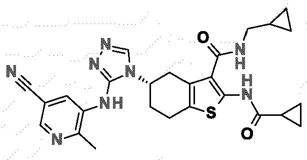

### (5S)-5-[3-[(5-cyano-2-methyl-3-pyridyl)amino]-1,2,4-triazol-4-yl]-2-(cyclopropanecarbonylamino)-N-(cyclopropylmethyl)-4,5,6,7-tetrahydrobenzothiophene-3-carboxamide

A solution of methanesulfonyl chloride (0.043 mL, 0.55 mmol) in DCM (1.0 mL) was added dropwise to a solution of **intermediate 394** (254 mg, 0.498 mmol) and triethylamine (0.21 mL, 1.5 mmol) in DCM (4.0 mL) at 0 °C. The reaction was stirred at 0 °C for 1.5 hours. The reaction was diluted with DCM (20 mL) and washed with sat. aqueous NH₄Cl (20 mL). The layers were separated and the aq. layer extracted with DCM (2 x 20 mL). The combined organic layers were passed through a phase separation cartridge and concentrated in vacuo. This material was dissolved in DMF (2.5 mL) and formic acid hydrazide (45 mg, 0.75 mmol) was added. The reaction was stirred at room temperature for 1.5 hours. Water (1.2 mL) followed by sodium carbonate (158 mg, 1.49 mmol) were added and the reaction was stirred for 16 h at 45 °C. Water (10 mL) and DCM (10 mL) were then added and the layers separated. The organic layer was concentrated in vacuo and re-dissolved in 5% MeOH in DCM (50 mL). The initial aqueous layer and additional water (20 mL) were added and the layers separated. The aqueous layer was extracted with 5% MeOH in DCM (2 x 25 mL). The combined organic layers were passed through a phase separation cartridge and concentrated in vacuo to give crude product which was purified using HPLC (Method 5) to give the title compound (155 mg, 60 % Yield). δ_{H} (300 MHz, DMSO-d6) 11.25 (s, 1H), 8.57 (s, 1H), 8.46 (d, J = 1.9 Hz, 1H), 8.19 (s, 1H), 8.07 (d, J = 1.9 Hz, 1H), 7.70 (t, J = 5.8 Hz, 1H), 4.62 - 4.45 (m, 1H), 3.21 - 2.94 (m, 4H), 2.91 - 2.78 (m, 2H), 2.55 (s, 3H), 2.35 - 2.13 (m, 2H), 2.00 - 1.86 (m, 1H), 1.05 - 0.91 (m, 1H), 0.91 - 0.76 (m, 4H), 0.39 - 0.26 (m, 2H), 0.22 - 0.10 (m, 2H). LCMS [M+H]⁺ 517, RT 1.69 minutes (Method 26).

### Example 300

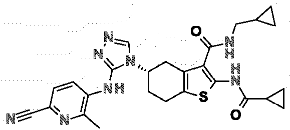

### (5S)-5-[3-[(6-cyano-2-methyl-3-pyridyl)amino]-1,2,4-triazol-4-yl]-2-(cyclopropanecarbonylamino)-N-(cyclopropylmethyl)-4,5,6,7-tetrahydrobenzothiophene-3-carboxamide

A solution of methanesulfonyl chloride (0.068 mL, 0.88 mmol) in DCM (1.0 mL) was added dropwise to a solution of **intermediate 396** (421 mg, 0.795 mmol) and triethylamine (0.33 mL, 2.4 mmol) in DCM (7 mL) at 0 °C. The reaction was stirred at 0 °C for 1 hours. The reaction was diluted with DCM (20 mL) and washed with sat. aqueous NH₄Cl (20 mL). The layers were separated and the aqueous layer extracted with DCM (2 x 20 mL). The combined organic layers were passed through a phase separation cartridge and concentrated in vacuo. This material was dissolved in DMF (4.0 mL) and formic acid hydrazide (72 mg, 1.2 mmol) was added. The reaction was stirred at room temperature for 1.5 hours. Water (2 mL) followed by sodium carbonate (253 mg, 2.39 mmol) were added and the reaction was stirred for 16 h at 45 °C. Water (10 mL) and DCM (10 mL) were then added and the layers separated. The organic layer was concentrated in vacuo and re-dissolved in 5% MeOH in DCM (50 mL). The initial aqueous layer and additional water (20 mL) were added and the layers separated. The aqueous layer was extracted with 5% MeOH in DCM (2 x 25 mL). The combined organic layers were passed through a phase separation cartridge and concentrated in vacuo to give crude product which was purified using HPLC (method 5) to give the title compound (181 mg, 44 % Yield). δ_{H} (300 MHz, DMSO-d6) 11.18 (s, 1H), 8.65 (s, 1H), 8.38 (s, 1H), 7.72 (d, J = 8.4 Hz, 2H), 7.60 (d, J = 8.5 Hz, 1H), 4.59 - 4.40 (m, 1H), 3.18 - 2.92 (m, 4H), 2.89 - 2.76 (m, 2H), 2.51 (obs. s, 3H), 2.34 - 2.09 (m, 2H), 2.00 - 1.81 (m, 1H), 1.00 - 0.89 (m, 1H), 0.89 - 0.77 (m, 4H), 0.40 - 0.25 (m, 2H), 0.21 - 0.10 (m, 2H). LCMS [M+H]⁺ 517, RT 1.75 minutes (Method 26).

### Example 301

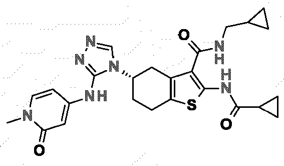

### (5S)-2-(cyclopropanecarbonylamino)-N-(cyclopropylmethyl)-5-[3-[(1-methyl-2-oxo-4-pyridyl)amino]-1,2,4-triazol-4-yl]-4,5,6,7-tetrahydrobenzothiophene-3-carboxamide

A solution of methanesulfonyl chloride (0.055 mL, 0.71 mmol) in DCM (1.0 mL) was added dropwise to a solution of **intermediate 398** (320 mg, 0.641 mmol) and triethylamine (0.27 mL, 1.9 mmol) in DCM (5.2 mL) at 0 °C and under nitrogen. The reaction was stirred at 0 °C for 45 mins. The reaction was diluted with DCM (20 mL) and washed with sat. aqueous NH₄Cl (20 mL). The layers were separated and the aqueous layer extracted with 5% MeOH in DCM (2 x 20 mL). The combined organic layers were passed through a phase separation cartridge and concentrated in vacuo. To this material was added MeOH (3.2 mL), MeCN (3.2 mL) and formic acid hydrazide (42 mg, 0.71 mmol). The reaction was stirred at room temperature for 1 hour 45 mins. Water (1.6 mL) was added and the reaction stirred for a further 30 mins at room temperature before adding DCM (1.6 mL) and then stirring at room temperature for another 1 hour 15 mins. Added more formic acid hydrazide (116 mg, 1.92 mmol) and stirred for a further 16.5 hours at room temperature before adding sodium carbonate (204 mg, 1.92 mmol) and stirring for 4 h at 45 °C. Water (20 mL) and 5% MeOH in DCM (50 mL) were added and the layers separated. The aqueous layer was extracted with 5% MeOH in DCM (2 x 25 mL). The combined organic layers were passed through a phase separation cartridge and concentrated in vacuo to give crude product which was purified by flash column chromatography on silica (gradient elution with 0% to 20% MeOH in DCM) to give the title compound (76 mg, 23 % Yield). δ_{H} (300 MHz, DMSO-d6) 11.26 (s, 1H), 8.92 (s, 1H), 8.50 (s, 1H), 7.69 (t, J = 5.7 Hz, 1H), 7.52 (d, J = 7.5 Hz, 1H), 6.56 (d, J = 2.4 Hz, 1H), 6.29 (dd, J = 7.5, 2.5 Hz, 1H), 4.57 - 4.42 (m, 1H), 3.32 (s, 4H), 3.22 - 2.75 (m, 6H), 2.33 - 2.11 (m, 2H), 2.00 - 1.87 (m, 1H), 1.06 - 0.91 (m, 1H), 0.91 - 0.74 (m, 4H), 0.41 - 0.28 (m, 2H), 0.23 - 0.09 (m, 2H). LCMS [M+H]⁺ 508, RT 1.51 minutes (Method 26).

### Example 302

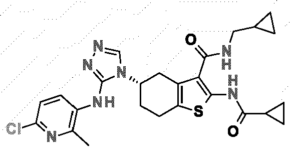

### (5S)-5-[3-[(6-chloro-2-methyl-3-pyridyl)amino]-1,2,4-triazol-4-yl]-2-(cyclopropanecarbonylamino)-N-(cyclopropylmethyl)-4,5,6,7-tetrahydrobenzothiophene-3-carboxamide

A solution of methanesulfonyl chloride (0.073 mL, 0.94 mmol) in DCM (1.0 mL) was added dropwise to a solution of **intermediate 400** (459 mg, 0.851 mmol) and triethylamine (0.35 mL, 2.5 mmol) in DCM (7.5 mL) at 0 °C and under nitrogen. The reaction was stirred at 0 °C for 45 mins. Additional methanesulfonyl chloride (0.023 mL, 0.30 mmol) was added and the reaction stirred for a further 15 mins at 0 °C before adding formic acid hydrazide (153 mg, 2.55 mmol). The reaction was stirred at room temperature for a further 6 hours and then at 40 °C for another 1.5 hours. The reaction was diluted with 5% MeOH in DCM (50 mL) and washed with sat. aq. NH₄Cl (20 mL). The layers were separated and the aqueous layer extracted with 5% MeOH in DCM (2 x 25 mL). The combined organic layers were passed through a phase separation cartridge and concentrated in vacuo. To this material was added MeOH (4.3 mL), water (2.1 mL), MeCN (4.3 mL), formic acid hydrazide (153 mg, 2.55 mmol) and sodium carbonate (361 mg, 3.40 mmol). The reaction was stirred for 15 h at 45 °C. Water (20 mL) and 5% MeOH in DCM (50 mL) were added and the layers separated. The aqueous layer was extracted with 5% MeOH in DCM (2 x 25 mL). The combined organic layers were passed through a phase separation cartridge and concentrated in vacuo to give crude product which was purified by flash column chromatography on silica (gradient elution with 0% to 20% MeOH in DCM) followed by further flash column chromatography on silica (gradient elution with 0% to 20% MeOH in EtOAc) to give the title compound (114 mg, 25 % Yield). δ_{H} (300 MHz, DMSO-d6) 11.22 (s, 1H), 8.48 (s, 1H), 7.98 (s, 1H), 7.82 - 7.63 (m, 2H), 7.25 (d, J = 8.5 Hz, 1H), 4.60 - 4.38 (m, 1H), 3.21 - 2.91 (m, 4H), 2.91 - 2.77 (m, 2H), 2.40 (s, 3H), 2.30 - 2.13 (m, 2H), 1.99 - 1.85 (m, 1H), 1.03 - 0.91 (m, 1H), 0.91 - 0.77 (m, 4H), 0.39 - 0.28 (m, 2H), 0.22 - 0.12 (m, 2H). LCMS [M+H]⁺ 526, RT 1.95 minutes (Method 26).

### Example 303

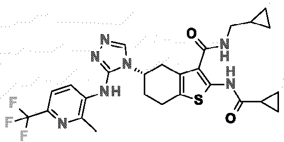

### (5S)-2-(cyclopropanecarbonylamino)-N-(cyclopropylmethyl)-5-[3-[[2-methyl-6-(trifluoromethyl)-3-pyridyl]amino]-1,2,4-triazol-4-yl]-4,5,6,7-tetrahydrobenzothiophene-3-carboxamide

A solution of methanesulfonyl chloride (0.048 mL, 0.62 mmol) in DCM (1.0 mL) was added dropwise to a solution of **intermediate 402** (348 mg, 0.568 mmol, 90 mass%) and triethylamine (0.24 mL, 1.7 mmol) in DCM (4.7 mL) at 0 °C. The reaction was stirred at 0 °C for 45 mins. Additional methanesulfonyl chloride (0.009 mL, 0.1 mmol) in DCM (0.5 mL) was added dropwise and the reaction stirred for a further 20 mins at 0 °C before adding formic acid hydrazide (102 mg, 1.70 mmol). The reaction was stirred at room temperature for a further 5.5 hours. The reaction was diluted with 5% MeOH in DCM (50 mL) and washed with sat. aqueous NH₄Cl (20 mL). The layers were separated and the aqueous layer extracted with 5% MeOH in DCM (2 x 25 mL). The combined organic layers were passed through a phase separation cartridge and concentrated in vacuo. To this material was added MeOH (3.0 mL), water (1.5 mL), MeCN (3 mL) and sodium carbonate (255 mg, 2.41 mmol). The reaction was stirred for 16 h at 45 °C. The reaction was cooled to room temperature then water (20 mL) and 5% MeOH in DCM (50 mL) were added and the layers separated. The aqueous layer was extracted with 5% MeOH in DCM (2 x 25 mL). The combined organic layers were passed through a phase separation cartridge and concentrated in vacuo to give crude product which was purified by flash column chromatography on silica (gradient elution with 0% to 20% MeOH in DCM) followed by further flash column chromatography on silica (gradient elution with 0% to 20% MeOH in EtOAc) to give the title compound (97 mg, 31 % Yield). δ_{H} (300 MHz, DMSO-d6) 11.19 (s, 1H), 8.61 (s, 1H), 8.22 (s, 1H), 7.78 - 7.67 (m, 2H), 7.60 (d, J = 8.5 Hz, 1H), 4.64 - 4.42 (m, 1H), 3.19 - 2.91 (m, 4H), 2.91 - 2.77 (m, 2H), 2.53 (s, 3H), 2.35 - 2.10 (m, 2H), 1.98 - 1.82 (m, 1H), 1.01 - 0.89 (m, 1H), 0.91 - 0.78 (m, 4H), 0.37 - 0.24 (m, 2H), 0.22 - 0.10 (m, 2H). LCMS [M+H]⁺ 560, RT 2.20 minutes (Method 26).

### Example 304

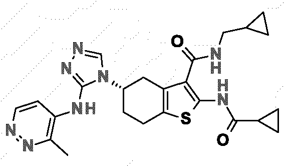

### (5S)-2-(cyclopropanecarbonylamino)-N-(cyclopropylmethyl)-5-[3-[(3-methylpyridazin-4-yl)amino]-1,2,4-triazol-4-yl]-4,5,6,7-tetrahydrobenzothiophene-3-carboxamide

Methanesulfonyl chloride (0.027 mL, 0.35 mmol) was added dropwise to a solution of **intermediate 404** (152 mg, 0.314 mmol) and triethylamine (0.13 mL, 0.93 mmol) in DCM (3.2 mL) at 0 °C and under nitrogen. The reaction was stirred at 0 °C for 15 mins and then at room temperature for a further 1.5 hours. Formic acid hydrazide (57 mg, 0.94 mmol) was added as a solution in DMF (1.0 mL). The reaction was stirred at room temperature for 1 hour before additional formic acid hydrazide (57 mg, 0.94 mmol) was added and the reaction stirred at room temperature for a further 2 hours. The reaction was diluted with DCM (20 mL) and washed with water (20 mL). The layers were separated and the aqueous layer extracted with DCM (2 x 20 mL). The combined organic layers were washed with sat. aqueous NH₄Cl (20 mL), then brine (20 mL), passed through a phase separation cartridge and concentrated in vacuo. To this material was added DMF (0.5 mL) and sodium carbonate (73 mg, 0.69 mmol). The reaction was stirred for 15.5 h at 45 °C. The reaction was cooled to room temperature then filtered, the filter was washed with DCM (20 mL). The filtrate was collected and washed with water (2 x 20 mL), then brine (20 mL), passed through a phase separation cartridge and concentrated in vacuo to give crude product. The combined water washings were also concentrated in vacuo to give further crude product. The combined crude product was purified by flash column chromatography on silica (gradient elution with 0% to 20% MeOH in DCM) followed by further flash column chromatography on silica (gradient elution with 0% to 40% MeOH in EtOAc) to give the title compound (15 mg, 10 % Yield). δ_{H} (300 MHz, DMSO-d6) 12.95 (s, 1H), 11.21 (s, 1H), 8.30 (s, 1H), 8.12 (d, J = 7.5 Hz, 1H), 7.97 (d, J = 7.4 Hz, 1H), 7.72 (t, J = 5.6 Hz, 1H), 4.65 - 4.53 (m, 1H), 3.21 - 2.94 (m, 4H), 2.86 - 2.77 (m, 2H), 2.29 (s, 3H), 2.23 - 2.15 (m, 2H), 1.97 - 1.89 (m, 1H), 1.02 - 0.92 (m, 1H), 0.90 - 0.75 (m, 4H), 0.35 - 0.29 (m, 2H), 0.18 - 0.12 (m, 2H). LCMS [M+H]⁺ 493, RT 1.68 minutes (Method 26).

### Example 305

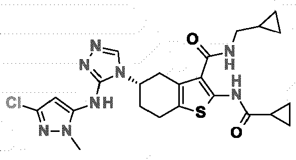

### (5S)-5-[3-[(5-chloro-2-methyl-pyrazol-3-yl)amino]-1,2,4-triazol-4-yl]-2-(cyclopropanecarbonylamino)-N-(cyclopropylmethyl)-4,5,6,7-tetrahydrobenzothiophene-3-carboxamide

To a stirred solution of **intermediate 405** (258 mg, 0.509 mmol) in DMF (3.6 mL) under nitrogen was added formic acid hydrazide (92 mg, 1.5 mmol) and mercuric chloride (190 mg, 0.697 mmol). The reaction mixture was stirred at r.t. for 5 min before the addition of triethylamine (0.21 mL, 1.5 mmol) and the mixture was stirred at 80 °C for 18 hours. The mixture was cooled to r.t. and diluted with MeCN (20 mL), filtered through a pad of celite and washed with MeCN (25 mL). The filtrate was concentrated in vacuo to yield the crude product which was dissolved in 5% MeOH in DCM (50 mL), washed with water (30 mL) and the layers separated. The aqueous layer was extracted with 5% MeOH in DCM (2 x 25 mL). The combined organic layers were passed through a phase separation cartridge and concentrated in vacuo to give crude product which was purified by flash column chromatography on silica (gradient elution with 0% to 10% MeOH in DCM). The product containing fractions were concentrated in vacuo and dissolved in MeOH (4 mL), QuadraSil^{®} MTU (500 mg) was added and the suspension was stirred at 40 °C for 2.5 h before being filtered and washed with MeOH (2 x 5 mL). The filtrate was concentrated in vacuo and was further purified by flash column chromatography on silica (gradient elution with 0% to 20% MeOH in EtOAc) to give the title compound (103 mg, 39 % Yield). δ_{H} (300 MHz, DMSO-d6) (contained 2 tautomers in an approximate ratio of 0.7:0.3) 12.07 (s, 0.7H), 11.29 - 11.15 (m, 1H), 8.79 (s, 0.3H), 8.41 (s, 0.3H), 8.21 (s, 0.7H), 7.82 - 7.66 (m, 1H), 6.21 (s, 0.3H), 5.96 (s, 0.7H), 4.53 - 4.34 (m, 1H), 3.63 (s, 0.9H), 3.53 (s, 2.1H), 3.23 - 3.04 (m, 3H), 3.03 - 2.76 (m, 3H), 2.33 - 2.12 (m, 2H), 1.99 - 1.87 (m, 1H), 1.08 - 0.94 (m, 1H), 0.92 - 0.78 (m, 4H), 0.41 - 0.32 (m, 2H), 0.23 - 0.15 (m, 2H). LCMS [M+H]⁺ 515, RT 1.80 minutes (Method 26).

### Example 307

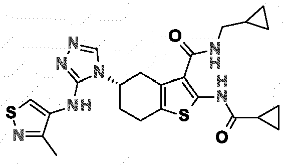

### (55)-2-(cyclopropanecarbonylamino)-N-(cyclopropylmethyl)-5-[3-[(3-methylisothiazol-4-yl)aminol-1,2,4-triazol-4-yl]-4,5,6,7-tetrahydrobenzothiophene-3-carboxamide

A solution of methanesulfonyl chloride (0.076 mL, 0.98 mmol) in DCM (1 mL) was added dropwise to a solution of **intermediate 407** (393 mg, 0.803 mmol) and triethylamine (0.37 mL, 2.6 mmol) in DCM (8 mL) at 0 °C and under nitrogen. The reaction was stirred at 0 °C for 1 hour 10 mins. Additional methanesulfonyl chloride (0.014 mL, 0.18 mmol) as a solution in DCM (1 mL) was added and the reaction stirred at 0 °C for a further 10 mins. The reaction was diluted with DCM (20 mL) and washed with sat. aq. NH₄Cl (20 mL). The layers were separated and the aquous layer extracted with 5% MeOH in DCM (2 x 20 mL). The combined organic layers were passed through a phase separation cartridge and concentrated in vacuo. To this material was added MeOH (4.5 mL), MeCN (4.5 mL) and formic acid hydrazide (81 mg, 1.3 mmol). The reaction was stirred at room temperature for 3 hours, DMF (2.2 mL) was added and the reaction stirred for a further 1.5 hours at room temperature. Water (2.2 mL) followed by sodium carbonate (284 mg, 2.68 mmol) were added and the reaction was stirred for 16 h at 45 °C. The reaction was concentrated in vacuo and re-dissolved in 5% MeOH in DCM (50 mL). Water (20 mL) was added and the layers separated. The Aq layer was extracted with 5% MeOH in DCM (2 x 25 mL). The combined organic layers were passed through a phase separation cartridge and concentrated in vacuo to give the crude product which was purified by flash column chromatography on silica (gradient elution with 0% to 20% MeOH in DCM) followed by further flash column chromatography on silica (gradient elution with 0% to 40% MeOH in EtOAc) and then HPLC (Method 5) to give the title compound (32 mg, 8 % Yield). δ_{H} (300 MHz, DMSO-d6) 11.25 (s, 1H), 8.84 (s, 1H), 8.39 (s, 1H), 8.27 (s, 1H), 7.73 (t, J = 5.7 Hz, 1H), 4.64 - 4.50 (m, 1H), 3.23 - 2.91 (m, 4H), 2.91 - 2.78 (m, 2H), 2.40 (s, 3H), 2.31 - 2.14 (m, 2H), 1.94 (p, J = 6.4 Hz, 1H), 1.06 - 0.92 (m, 1H), 0.91 - 0.78 (m, 4H), 0.42 - 0.27 (m, 2H), 0.23 - 0.10 (m, 2H). LCMS [M+H]⁺ 498, RT 1.79 minutes (Method 26).

### Example 308

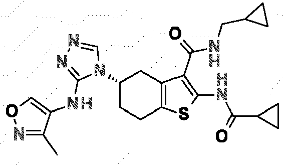

### (5S)-2-(cyclopropanecarbonylamino)-N-{cyclopropylmethyl)-5-[3-[(3-methylisoxazol-4-yl)amino]-1,2,4-triazol-4-yl]-4,5,6,7-tetrahydrobenzothiophene-3-carboxamide

To a stirred solution of **intermediate 409** (313 mg, 0.614 mmol, 93 mass%) in DMF (4.3 mL) under nitrogen was added formic acid hydrazide [624-84-0] (111 mg, 1.84 mmol) and mercuric chloride [7487-94-7] (201 mg, 0.734 mmol). The reaction mixture was stirred at r.t. for 5 min before the addition of triethylamine (0.26 mL, 1.9 mmol) and the mixture was stirred at 80 °C for 4.5 hours. The mixture was cooled to r.t. and diluted with MeCN (20 mL), filtered through a pad of celite and washed with MeCN (25 mL). The filtrate was concentrated in vacuo to yield the crude product which was dissolved in 5% MeOH in DCM (50 mL), washed with water (30 mL) and the layers separated. The aq. layer was extracted with 5% MeOH in DCM (2 x 25 mL). The combined organic layers were passed through a phase separation cartridge and concentrated in vacuo. The crude product was dissolved in MeOH (6 mL) and MeCN (3 mL), QuadraSil^{®} MTU (600 mg) was added and the suspension was stirred at 40 °C for 4.5 h before being filtered and washed with MeOH (2 x 5 mL). The filtrate was concentrated in vacuo and was purified by flash column chromatography on silica (gradient elution with 0% to 20% MeOH in DCM) followed by flash column chromatography on silica (gradient elution with 0% to 20% MeOH in EtOAc) and then by SFC to give the title compound (83 mg, 28 % Yield). δ_{H} (300 MHz, DMSO-d6) 11.26 (s, 1H), 9.02 (s, 1H), 8.35 (s, 1H), 8.16 (s, 1H), 7.72 (t, J = 5.7 Hz, 1H), 4.62 - 4.48 (m, 1H), 3.21 - 3.03 (m, J = 6.3 Hz, 3H), 3.03 - 2.79 (m, 3H), 2.28 (s, 3H), 2.26 - 2.13 (m, 2H), 1.99 - 1.88 (m, 1H), 1.06 - 0.95 (m, 1H), 0.92 - 0.80 (m, 4H), 0.40 - 0.29 (m, 2H), 0.23 - 0.13 (m, 2H). LCMS [M+H]⁺ 482, RT 1.64 minutes (Method 26).

### Example 337

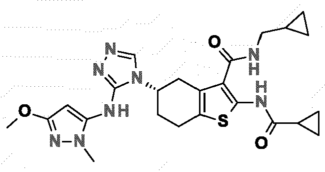

### (5S)-2-(cyclopropanecarbonylamino)-N-(cyclopropylmethyl)-5-[3-[(5-methoxy-2-methylpyrazol-3-yl)amino]-1,2,4-triazol-4-yl]-4,5,6,7-tetrahydro-1-benzothiophene-3-carboxamide

To a mixture of **intermediate 526** (90%, 631 mg, 1.13 mmol) and triethylamine (0.47 mL, 3.39 mmol) in DCM (14 mL) at 0 °C, methanesulfonyl chloride (0.10 mL, 1.36 mmol) was added. The solution was stirred at 0 °C for 10 minutes. An additional portion of methanesulfonyl chloride (0.022 mL, 0.282 mmol) was added and the reaction was stirred for another 20 minutes. The reaction was diluted with DCM (10 mL) and washed with saturated NH₄Cl (20 mL). The organic layer was dried (MgSO₄) and the solvent removed to give the intermediate carbodiimide as a yellow oil. The oil was dissolved in a solution of formic hydrazide (204 mg, 3.39 mmol) in MeOH (5 mL) and then diluted with MeOH (15 mL). The reaction was stirred for 10 minutes. Another portion of formic hydrazide (100 mg, 1.70 mmol) in MeOH (2.5 mL) was added and the reaction was stirred for a further 10 minutes. 1 M sodium carbonate (3.4 mL, 3.39 mmol) was added to the solution and this was then heated at 40 °C for 21 hours. The solvent was removed and the residue partitioned between DCM (20 mL) and water (20 mL). The organic layer was separated, and the aqueous layer extracted with DCM (2 x 10 mL). The combined organic layers were dried (MgSO₄) and the solvent was removed. Purification of the residue by flash column chromatography eluting with 0 to 10% of methanol in DCM gradient gave the title compound (316 mg, 49% Yield). δ_{H} (500 MHz, DMSO-d6) 11.83 (s, 0.5H, rotamer), 11.24 (s, 0.5H, rotamer), 11.20 (s, 0.5H, rotamer), 8.54 (s, 0.5H, rotamer), 8.37 (s, 0.5H, rotamer), 8.14 (s, 0.5H, rotamer), 7.79 - 7.63 (m, 1H), 5.57 (s, 0.5H, rotamer), 5.39 (s, 0.5H, rotamer), 4.52 - 4.32 (m, 1H), 3.72 (s, 1.5H, rotamer), 3.69 (s, 1.5H, rotamer), 3.50 (s, 1.5H, rotamer), 3.44 (s, 1.5H, rotamer), 3.26 - 3.07 (m, 3H), 3.02 - 2.88 (m, 1H), 2.88 - 2.76 (m, 2H), 2.24 - 2.06 (m, 2H), 1.96 - 1.86 (m, 1H), 1.09 - 0.95 (m, 1H), 0.93 - 0.79 (m, 4H), 0.44 - 0.30 (m, 2H), 0.24 - 0.07 (m, 2H). LCMS [M+H]⁺ 511, RT 2.27 minutes, Method 10.

### Example 351

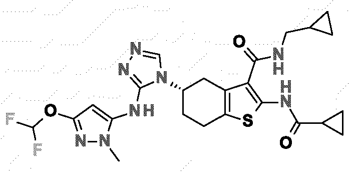

### (5S)-2-(cyclopropanecarbonylamino)-N-(cyclopropylmethyl)-5-[3-[[5-(difluoromethoxy)-2-methylpyrazol-3-yl]amino]-1,2,4-triazol-4-yl]-4,5,6,7-tetrahydro-1-benzothiophene-3-carboxamide

1 M formic hydrazide (0.23 mL, 0.23 mmol) in MeOH was added to **intermediate 550** (38.0 mg, 0.08 mmol). MeOH (2 mL) was added and the reaction mixture stirred for 30 min. Then aqueous 0.92 M sodium carbonate (0.25 mL, 0.23 mmol) was added and reaction heated to 50 °C with stirring for 16 hours. Reaction allowed to cool to room temperature. The MeOH was removed under vacuum and water added (10 mL). The mixture was extracted with DCM (2 x 10 mL) and the combined organic layers dried (MgSO₄) before concentration under reduced pressure. The residue was purified by reverse phase HPLC eluting with 5 - 95 % Acetonitrile (0. 1 % formic acid) in Water (0. 1 % formic acid) to give the title compound (16 mg, 38 % Yield). δ_{H} (500 MHz, DMSO-d6) 12.03 (s, 1H, Major rotamer), 11.23 and11.19 (2 x s, 1H), 8.74 (s, 1H, minor rotamer), 8.40 and 8.19 (2 x s, 1H), 7.81 - 7.54 (m, 1H), 7.45 - 6.87 (m, 1H), 5.90 and 5.65 (2 x s, 1H), 4.62 - 4.27 (m, 1H), 3.57 and 3.48 (s, 3H), 3.24 - 3.02 (m, 3H), 3.03 - 2.89 (m, 1H), 2.89 - 2.78 (m, 2H), 2.28 - 2.12 (m, 2H), 1.98 - 1.87 (m, 1H), 1.07 - 0.95 (m, 1H), 0.90 - 0.80 (m, 4H), 0.40 - 0.31 (m, 2H), 0.23 - 0.15 (m, 2H). LCMS [M+H]⁺ 547, RT 2.78 minutes (Method 10).

### Example 403

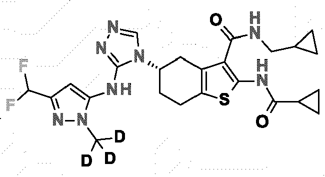

### (5S)-2-(cyclopropanecarbonylamino)-N-(cyclopropylmethyl)-5-[3-[[5-(difluoromethyl)-2-(trideuteriomethyl)pyrazol-3-yl]amino]-1,2,4-triazol-4-yl]-4,5,6,7-tetrahydrobenzothiophene-3-carboxamide

The title compound was prepared by a method analogous to that used to prepare Example 108, using **intermediate 573, intermediate 117,** and formyl hydrazine. Crystallization of the crude material from methanol afforded the title compound (2.96 g, 47% Yield). δ_{H} (400 MHz, DMSO-d6)12.07 and 8.74 (s, 1H rotamer), 11.26 and 11.21 (s,1H rotamer), 8.40 and 8.19 (s,1H rotamers 40:60), 7.73 (m, 1H), 6.86 and 6.75 (t, 1H rotamer), 6.41 and 6.20 (s, 1H rotamer), 4.46 (m, 1H), 3.13 (m, 2H), 2.99 (m, 2H), 2.85 (m, 2H), 2.20 (m, 2H), 1.93 (m, 1H), 1.00 (m, 1H), 0.85 (m, 4H), 0.36 (m, 2H), 0.19 (m, 2H). HRMS [M+H⁺] 534.2290 (C₂₄H₂₅D₃F₂N₈O₂S= 533.1789).

### Example 418

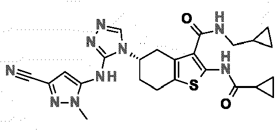

### (5S)-5-[3-[(5-Cyano-2-methyl-pyrazol-3-yl)amino]-1,2,4-triazol-4-yl]-2-(cyclopropanecarbonylamino)-N-(cyclopropylmethyl)-4,5,6,7-tetrahydrobenzothiophene-3-carboxamide

Synthesised using general procedure **general method 8,** using **intermediate 581** (500 mg, 1.01 mmol) and comparable stoichiometries of reagents. The product was purified by flash chromatography eluting with 0-15% MeOH/DCM to afford the title compound (8 mg, 1.6%) as a colourless powder. δ_{H} (400 MHz, DMSO-d6) 11.48 - 10.95 (m, 2H), 8.23 (s, 1H), 7.99 - 7.56 (m, 1H), 6.54 (s, 1H), 4.52 - 4.37 (m, 1H), 3.66 (s, 3H), 3.25 - 3.04 (m, 3H), 2.98 - 2.71 (m, 4H), 2.28 - 2.09 (m, 3H), 1.94 - 1.77 (m, 1H), 1.05 - 0.91 (m, 1H), 0.90 - 0.68 (m, 4H), 0.43 - 0.30 (m, 1H), 0.25 - 0.14 (m, 1H). LCMS [M+H]⁺ 506 , RT 1.80 minutes (Method 25).

### Example 419

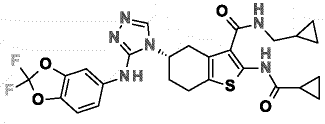

### (5S)-2-(Cyclopropanecarbonylamino)-N-(cyclopropylmethyl)-5-[3-[(2,2-difluoro-1,3-benzodioxol-5-yl)aminol-1,2,4-triazol-4-yl]-4,5,6,7-tetrahydrobenzothiophene-3-carboxamide

Synthesised using general procedure **general method 8,** using **intermediate 583** (431 mg, 0.786 mmol) and comparable stoichiometries of reagents. The product was purified by flash chromatography eluting with 0-10% MeOH/DCM to afford the title compound (149 mg, 34 % Yield) as a colourless powder. δ_{H} (300 MHz, DMSO-d6) 11.26 (s, 1H), 8.85 (s, 1H), 8.41 (s, 1H), 7.81 (s, 1H), 7.69 (t, J = 5.7 Hz, 1H), 7.40 - 7.25 (m, 2H), 4.58 - 4.43 (m, 1H), 3.21 - 2.80 (m, 6H), 2.35 - 2.12 (m, 2H), 1.94 (p, J = 6.5 Hz, 1H), 1.03 - 0.78 (m, 5H), 0.36 - 0.25 (m, 2H), 0.19 - 0.10 (m, 2H). LCMS [M+H]⁺ 557 , RT 2.44 minutes (Method 25).

### Example 421

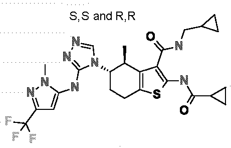

(4RS,5RS)-2-(cyclopropanecarbonylamino)-N-(cyclopropylmethyl)-4-methyl-5-[3-[[2-methyl-5-(trifluoromethyl)pyrazol-3-yl]amino]-1,2,4-triazol-4-yl]-4,5,6,7-tetrahydrobenzothiophene-3-carboxamide Synthesised in the same manner as Example 229, using **intermediate 598** (95 mg, 0.17 mmol) and comparable stoichiometries of reagents. The product was purified by HPLC to afford the title compound (27 mg, 27% Yield). δ_{H} (400 MHz, DMSO-d6) 12.16 (s, 1H), 10.65 (s, 1H), 8.35 - 8.12 (m, 1H), 8.12 - 7.78 (m, 1H), 6.47 - 6.22 (m, 1H), 4.29 (ddd, J = 8.9, 6.1, 2.9 Hz, 1H), 3.67 (s, 3H), 3.43 (s, 1H), 3.11 (qt, J = 13.4, 6.2 Hz,2H), 2.83 - 2.70 (m, 1H), 2.62 - 2.54 (m, 1H), 2.31 - 2.20 (m, 1H), 2.12 - 1.89 (m, 2H), 1.16 (d, J = 6.8 Hz, 3H), 1.07 - 0.94 (m, 1H), 0.93 - 0.70 (m, 5H), 0.44 - 0.35 (m, 2H), 0.25 - 0.17 (m, 2H). LCMS [M+H]⁺ 563, RT 1.73 minutes (Method 26).

### Example 422

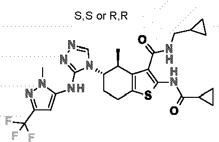

### (4R*,5R*)-2-(cyclopropanecarbonylamino)-N-(cyclopropylmethyl)-4-methyl-5-[3-[[2-methyl-5-(trifluoromethyl)pyrazol-3-yl]amino]-1,2,4-triazol-4-yl]-4,5,6,7-tetrahydrobenzothiophene-3-carboxamide

Chiral separation by SFC of **Example 421** (20 mg) afforded the title compound (6 mg, 6 % Yield) as a colourless powder. δ_{H} (400 MHz, DMSO-d6) 12.16 (s, 1H), 10.64 (s, 1H), 8.20 (s, 1H), 7.95 (s, 1H), 6.36 (s, 1H), 4.36 - 4.21 (m, 1H), 3.67 (s, 3H), 3.42 (s, 1H), 3.11 (qt, J = 13.4, 6.2 Hz, 2H), 2.84 - 2.72 (m, 1H), 2.61 - 2.55(m, 1H), 2.29 - 2.20 (m, 1H), 2.13 - 1.91 (m, 2H), 1.22 - 1.07 (m, 3H), 1.07 - 0.94 (m, 1H), 0.92 - 0.71 (m, 5H), 0.46 - 0.31 (m, 2H), 0.24 - 0.13 (m, 2H). LCMS [M+H]⁺ 563, RT 1.73 minutes (Method 26). Chiral SFC* RT = 4.93 minutes

* Using chiral SFC Method 1 with a Chiralpak IB column.

### Example 423

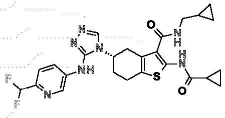

### (5S)-2-(cyclopropanecarbonylamino)-N-(cyclopropylmethyl)-5-[3-[[6-(difluoromethyl)-3-pyridyl]amino]-1,2,4-triazol-4-yl]-4,5,6,7-tetrahydrobenzothiophene-3-carboxamide

Synthesised in the same manner as Example 229, using **intermediate 599** (403 mg, 0.78 mmol) and comparable stoichiometries of reagents. The product was purified by HPLC (Method 5) to afford the title compound (140 mg, 34% Yield). δ_{H} (300 MHz, DMSO-d6) 11.26 (s, 1H), 9.20 (s, 1H), 8.82 (d, J = 2.6 Hz, 1H), 8.47 (s, 1H), 8.30 (dd, J = 8.6, 2.6 Hz, 1H), 7.68 (t, J = 5.7 Hz, 1H), 7.63 (d, J = 8.7 Hz, 1H), 6.87 (t, J = 55.4 Hz, 1H), 4.69 -4.44 (m, 1H), 3.22 - 2.79 (m, 6H), 2.38 - 2.12 (m, 2H), 2.03 - 1.83 (m, 1H), 1.02 - 0.79 (m, 5H), 0.28 (dt, J= 8.3, 2.8 Hz, 2H), 0.18 - 0.10 (m, 2H). LCMS [M+H]⁺ 528, RT 1.92 minutes (Method 26).

### Example 424

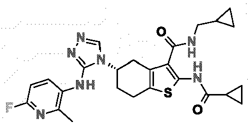

### (5S)-2-(cyclopropanecarbonylamino)-N-(cyclopropylmethyl)-5-[3-[(6-fluoro-2-methyl-3-pyridyl)amino]-1,2,4-triazol-4-yl]-4,5,6,7-tetrahydrobenzothiophene-3-carboxamide

Synthesised in the same manner as Example 229, using **intermediate 600** (446 mg, 0.89 mmol) and comparable stoichiometries of reagents. The product was purified by HPLC (Method 5) to afford the title compound (100 mg, 22 % Yield). δ_{H} (300 MHz, DMSO-d6) 11.22 (s, 1H), 8.39 (s, 1H), 7.94 (s, 1H), 7.85 (dd, J = 8.6, 7.5 Hz, 1H), 7.73 (t, J = 5.7 Hz, 1H), 6.94 (dd, J = 8.6, 3.6 Hz, 1H), 4.49 (dt, J = 9.8, 4.9 Hz, 1H), 3.23 - 2.91 (m, 4H), 2.91 - 2.76 (m, 2H), 2.33 (s, 3H), 2.31 - 2.13 (m, 2H), 1.93 (dt, J = 12.5, 6.5 Hz, 1H), 1.06 - 0.92 (m, 1H), 0.90 - 0.81 (m, 4H), 0.42 - 0.28 (m, 2H), 0.25 - 0.10 (m, 2H). LCMS [M+H]⁺ 510, RT 1.84 minutes (Method 26).

### Example 427

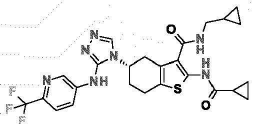

### (5S)-2-(cyclopropanecarbonylamino)-N-(cyclopropylmethyl)-5-[3-[[6-(trifluoromethyl)-3-pyridyl]amino]-1,2,4-triazol-4-yl]-4,5,6,7-tetrahydrobenzothiophene-3-carboxamide

Synthesised in the same manner as Example 229, using **Intermediate 614** (78mg) and comparable stoichiometries of reagents. The product was purified by SFC to afford the title compound (3 mg) as a colourless powder. δ_{H} (400 MHz, Methanol-d4) 8.74 (d, J = 2.6 Hz, 1H), 8.45 (s, 1H), 8.20 (dd, J = 8.8, 2.6 Hz, 1H), 7.73 (d, J = 8.7 Hz, 1H), 4.67 - 4.56 (m, 1H), 3.30 - 2.84 (m, 6H), 2.42 - 2.30 (m, 2H), 1.85 - 1.75 (m, 1H),1.07 - 0.84 (m, 5H), 0.46 - 0.36 (m, 2H), 0.25 - 0.16 (m, 2H). LCMS [M+H]⁺ 546, RT 2.13 minutes (Method 26).

### Example 428

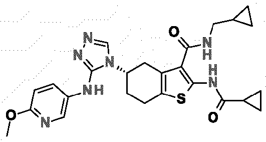

### (5S)-2-(cyclopropanecarbonylamino)-N-(cyclopropylmethyl)-5-[3-[(6-methoxy-3-pyridyl)amino]-1,2,4-triazol-4-yl]-4,5,6,7-tetrahydrobenzothiophene-3-carboxamide

Synthesised in the same manner as Example 229, using **intermediate 616** (80 mg) and comparable stoichiometries of reagents. The product was purified by HPLC to afford the title compound (9 mg) as a colourless powder. δ_{H} (300 MHz, Methanol-d4) 8.31 (s, 1H), 8.21 (d, J = 2.8 Hz, 1H), 7.84 (dd, J = 8.9, 2.9 Hz, 1H), 6.84 - 6.72 (m, 1H), 4.63 - 4.44 (m, 1H), 3.88 (s, 3H), 3.30 - 2.86 (m, 6H), 2.42 - 2.28 (m, 2H), 1.92 - 1.73 (m, 1H), 1.19 - 0.83 (m, 5H), 0.56 - 0.35 (m, 2H), 0.22 (dt, J = 6.1, 4.5 Hz, 2H). LCMS [M+H]⁺ 508, RT 1.86 minutes (Method 26).

### Example 429

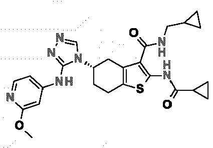

### (5S)-2-(cyclopropanecarbonylamino)-N-(cyclopropylmethyl)-5-[3-[(2-methoxy-4-pyridyl)amino]-1,2,4-triazol-4-yl]-4,5,6,7-tetrahydrobenzothiophene-3-carboxamide

Synthesised in the same manner as Example 229, using **intermediate 618** (11mg) and comparable stoichiometries of reagents. The product was purified by HPLC to afford the title compound (2 mg) as a colourless powder. δ_{H} (300 MHz, Methanol-d4) 8.47 (s, 1H), 7.89 (d, J = 5.9 Hz, 1H), 6.89 (dd, J = 5.9, 2.0 Hz, 1H), 6.81 (d, J = 1.9 Hz, 1H), 4.55 (dq, J = 9.8, 5.8, 5.3 Hz, 1H), 3.86 (s, 3H), 3.30 - 2.87 (m, 6H), 2.44 - 2.24 (m, 2H), 1.86 - 1.73 (m, 1H), 1.06 - 0.87 (m, 5H), 0.49 - 0.36 (m, 2H), 0.20 (dt, J = 6.1, 4.4 Hz, 2H). LCMS [M+H]⁺ 508, RT 1.80 minutes (Method 26).

### Example 430

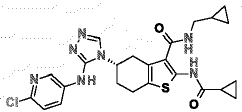

### (5S)-5-[3-[(6-chloro-3-pyridyl)amino]-1,2,4-triazol-4-yl]-2-(cyclopropanecarbonylamino)-N-(cyclopropylmethyl)-4,5,6,7-tetrahydrobenzothiophene-3-carboxamide

Synthesised in the same manner as Example 229, using **Intermediate 620** (62 mg) and comparable stoichiometries of reagents. The product was purified by HPLC (Method 5) to afford the title compound (19 mg) as a colourless powder. δ_{H} (300 MHz, Methanol-d4) 8.49 (dd, J = 3.0, 0.6 Hz, 1H), 8.40 (s, 1H), 8.03 (dd, J = 8.7, 3.0 Hz, 1H), 7.37 (dd, J = 8.7, 0.6 Hz, 1H), 4.66 - 4.48 (m, 1H), 3.30 - 2.85 (m, 6H), 2.44 - 2.29 (m, 2H), 1.92 - 1.73 (m, 1H), 1.11 - 0.86 (m, 5H), 0.50 - 0.33 (m, 2H), 0.21 (dt, J = 6.1, 4.4 Hz, 2H). LCMS [M+H]⁺ 512, RT 1.95 minutes (Method 26).

### Example 431

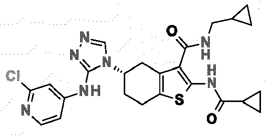

### (5S)-5-[3-[(2-chloro-4-pyridyl)amino]-1,2,4-triazol-4-yl]-2-(cyclopropanecarbonylamino)-N-(cyclopropylmethyl)-4,5,6,7-tetrahydrobenzothiophene-3-carboxamide

Synthesised in the same manner as Example 229, using **Intermediate 622** (59 mg) and comparable stoichiometries of reagents. The product was HPLC (Method 5) to afford the title compound (7 mg) as a colourless glass. δ_{H} (400 MHz, DMSO-d6) 11.26 (s, 1H), 9.50 (s, 1H), 8.53 (s, 1H), 8.14 (d, J = 5.7 Hz, 1H), 7.72 (d, J = 2.0 Hz, 1H), 7.67 (t, J = 5.7 Hz, 1H), 7.41 (dd, J = 5.8, 2.0 Hz, 1H), 4.62 - 4.44 (m, 1H), 3.19 - 2.90 (m, 4H), 2.90 - 2.82 (m, 2H), 2.31 - 2.15 (m, 2H), 1.99 - 1.86 (m, 1H), 1.00 - 0.90 (m, 1H), 0.90 - 0.79 (m, 4H), 0.36 - 0.24 (m, 2H), 0.20 - 0.07 (m, 2H). LCMS [M+H]⁺ 512, RT 1.82 minutes (Method 26).

### Example 432

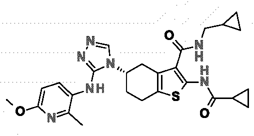

### (55)-2-(cyclopropanecarbonylamino)-N-(cyclopropylmethyl)-5-[3-[(6-methoxy-2-methyl-3-pyridyl)amino]-1,2,4-triazol-4-yl]-4,5,6,7-tetrahydrobenzothiophene-3-carboxamide

Synthesised in the same manner as Example 229, using **Intermediate 624** (47 mg) and comparable stoichiometries of reagents. The product was purified by HPLC to afford the title compound (7 mg) as a colourless powder. δ_{H} (400 MHz, Methanol-d4) 7.66 - 7.48 (m, 1H), 6.85 - 6.72 (m, 1H), 4.07 - 3.98 (m, 1H), 3.96 (s, 3H), 3.26 - 3.13 (m, 3H), 2.91 - 2.74 (m, 2H), 2.69 - 2.54 (m, 1H), 2.29 - 2.15 (m, 4H), 1.99 - 1.69 (m, 2H), 1.17 - 1.03 (m, 1H), 1.03 - 0.89 (m, 4H), 0.57 - 0.39 (m, 2H), 0.34 - 0.19 (m, 2H). LCMS [M+H]⁺ 522, RT 1.92 minutes (Method 26).

### Example 433

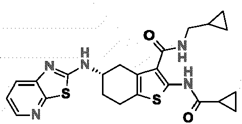

### (5S)-2-(cyclopropanecarbonylamino)-N-(cyclopropylmethyl)-5-[3-[(2-fluoro-3-pyridyl)amino]-1,2,4-triazol-4-yl]-4,5,6,7-tetrahydrobenzothiophene-3-carboxamide

Synthesised in the same manner as Example 229, using **intermediate 626** (25mg) and comparable stoichiometries of reagents. The product was purified by HPLC (Method 5) to afford the title compound (31 mg). δ_{H} (400 MHz, DMSO-d6) 11.11 (s, 1H), 8.47 (d, J = 6.9 Hz, 1H), 8.10 (dd, J = 4.8, 1.5 Hz, 1H), 7.73 (t, J = 5.7 Hz, 1H), 7.68 (dd, J = 8.0, 1.5 Hz, 1H), 7.25 (dd, J = 8.1, 4.8 Hz, 1H), 4.28 - 4.17 (m, 1H), 3.21 - 3.08 (m, 3H), 2.87 - 2.75 (m, 2H), 2.70 (dd, J = 16.1, 7.4 Hz, 1H), 2.18 - 2.08 (m, 1H), 1.98 - 1.85 (m, 2H), 1.06 - 0.95 (m, 1H), 0.92 - 0.79 (m, 4H), 0.36 (dt, J = 8.6, 2.8 Hz, 2H), 0.28 - 0.12 (m, 2H). LCMS [M+H]⁺ 468, RT 2.16 minutes (Method 26).

### Example 434

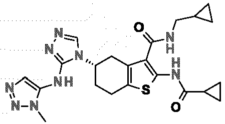

### (5S)-2-(cyclopropanecarbonylamino)-N-(cyclopropylmethyl)-5-[3-[(3-methyltriazol-4-yl)aminol-1,2,4-triazol-4-yl]-4,5,6,7-tetrahydrobenzothiophene-3-carboxamide

Synthesised in the same manner as Example 229, using **intermediate 628** (210 mg, 0.44 mmol) and comparable stoichiometries of reagents. The product was purified by HPLC (Method 5) to afford the title compound (78 mg, 16% Yield) as a colourless powder. δ_{H} (300 MHz, DMSO-d6) 12.11 (s, 1H), 11.29 - 11.13 (m, 1H), 8.23 (d, J = 1.1 Hz, 1H), 7.80 - 7.67 (m, 1H), 7.45 (s, 1H), 4.57 - 4.39 (m, 1H), 3.88 - 3.71 (m, 3H), 3.22 - 2.79 (m, 6H), 2.29 - 2.08 (m, 2H), 1.99 - 1.86 (m, 1H), 1.06 - 0.93 (m, 1H), 0.91 - 0.75 (m, 4H), 0.43 - 0.27 (m, 2H), 0.17 (dt, J = 6.0, 4.3 Hz, 2H). LCMS [M+H]⁺ 482, RT 1.62 minutes (Method 26).

### Example 501

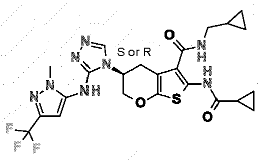

### (3R*)-6-(cyclopropanecarbonylamino)-N-(cyclopropylmethyl)-3-[3-[[2-methyl-5-(trifluoromethyl)pyrazol-3-yl]amino]-1,2,4-triazol-4-yl]-3,4-dihydro-2H-thieno[2,3-b]pyran-5-carboxamide (* or S)

To a stirred solution of **intermediate 673** (200 mg, 0.37 mmol) in DCM (10 mL) at 0 °C under nitrogen was added triethylamine (0.15 mL, 1.10 mmol) and methanesulfonyl chloride [124-63-0] (0.03 mL, 0.39 mmol). The reaction mixture was stirred at 0 °C for 1 h and then treated with a solution of formic acid hydrazide [624-84-0] (46 mg, 0.74 mmol) in DMF (1.5 mL). Stirring was continued at 0 °C for 1.5 h. The organic layer was washed with water and dried over Na₂SO₄. Volatiles were removed in vacuo and the resulting mixture diluted with DMF (1.5 mL) and treated with sodium carbonate (97 mg, 0.92 mmol). The mixture stirred at 45 °C under nitrogen for 5 h then allowed to cool to room temperature. The reaction mixture was filtered and concentrated in vacuo. The crude product was purified by column chromatography eluting with a 0-100 % gradient of DCM in isohexane followed by 0-10 % MeOH in DCM. Product-containing fractions were concentrated and purified by HPLC (Method 5) to afford a racemic mixture of the title compound (33 mg). Chiral separation employing SFC with a 10-25% gradient of MeOH (+ 0.1 % NH₄OH) in CO₂, on a CHIRALCEL OJ-H column (20 x 250 mm 5 µm, 100 mL/min) gave the title compound (Peak 2, 11.5 mg, 5.7% yield). δ_{H} (400 MHz, DMSO-d6) 12.24 (s, 1H), 11.27 (s, 1H), 7.89 (s, 1H), 7.81 - 7.49 (m, 1H), 6.37 (s, 1H), 4.96 - 4.70 (m, 1H), 4.62 - 4.24 (m, 2H), 3.69 (s, 3H), 3.30 - 3.20 (m, 1H), 3.18 - 2.96 (m, 3H), 2.01 -1.78 (m, 1H), 1.09 - 0.96 (m, 1H), 0.93 - 0.72 (m, 4H), 0.44 - 0.34 (m, 2H), 0.24 - 0.16 (m, 2H). LCMS [M+H]⁺ 551, RT 2.02 minutes (Method 3). Chiral SFC** RT = 5.71 minutes.

** Chiral analysis using CHIRALCEL OJ-H (4.6 x 150 mm 3 µm) column, eluting 10-25% gradient of MeOH (+ 0.1 % NH₄OH) in CO₂, flow rate 1 mL/min.

### Y131A FRET Assay

### Protocol for preparation of IgE-Tb reagent

86 nmoles of IgE-Fc(N265Q, N371Q) (Young *et al.,* 1995) at 172 uM in 100 mM NaHCO₃, pH 9.5 was added to 1 mg of LanthaScreen^{™} Amine Reactive Tb Chelate (ThermoFisher catalogue number PV3583) and incubated for 16 hours at 20 °C. The material was then buffer exchanged into Phosphate Buffered Saline (being, 137 mM NaCl, 2.7 mM KCl, 10 mM Na₂HPO₄, 1.8 mM KH₂HPO₄, pH 7.4) and the material quantified and the degree of Tb conjugation determined by measuring the absorption at 280 nm and 343 nm.

The integrity of the conjugated material was determined by analytical size exclusion chromatography on a S200 HR 10x300 column (GE Healthcare). Typical conjugation ratios were 4:1 Tb:IgE-Fc.

Young RJ., Owens, RJ., MacKay GA., Chan CMW., Shi J., Hide M., Francis DM., Henry AJ., Sutton BJ., and Gould HJ (1995) Protein Engineering 8:193-199

### Protocol for preparation of sFcεRIα-Y131A-AF488 reagent

400 nmoles FcεRIα (Y131A mutant) (Cook *et al.,* 1997) at 400 uM in 100 mM NaOAc pH 5.5 was reacted with 1 mM sodium periodate (in 100 mM NaOAc, pH 5.5) for 60 minutes at 22 ^{O}C. Oxidation was quenched with the addition of 40 uL of ethanediol and incubation for 60 minutes at 22 ^{O}C. The protein was buffer exchanged in to conjugation buffer (50 mM NaHCO₃ 150 mM NaCl, pH 9.5) and concentrated to 750 uM.

175 nmoles of protein was added to 1 mg of Alexa Fluor^{™} 488 hydrazide (Invitrogen) and incubated for 16 hours at 22 ^{O}C. Sodium cyanoborohydride (at 100 mM in conjugation buffer) was added to a final concentration of 1 mM and incubated for 60 minutes on ice. The protein was buffer exchanged into Phosphate Buffered Saline (being, 137 mM NaCl, 2.7 mM KCI, 10 mM Na₂HPO₄, 1.8 mM KH₂HPO₄, pH 7.4) and the material quantified and the degree of Alexa Fluor^{™}488 conjugation determined by measuring the absorption at 280 nm and 495 nm.

The integrity of the conjugated material was determined by analytical size exclusion chromatography on a S200 HR 10x300 column (GE Healthcare). Typical conjugation ratios were 2:1 Alexa Fluor^{™}488: FcεRIα

Cook JPD., Henry AJ., McDonnell JM., Owens RJ., Sutton BJ., and Gould HJ (1997) Biochemistry 36:15579-15588

### In vitro Biochemical Assay:

The aim was to measure binding of IgE-Tb to receptor, and the inhibition thereof by compounds, using an in vitro Fluorescence Resonance Energy Transfer (FRET) Assay.

### Reagents

FRET reagents used were IgE labelled with Terbium (FRET donor), and soluble IgE receptor FcεRIα with a Y131A mutation, labelled with Alexa Fluor^{™} 488 (FRET acceptor). Unlabelled FcεRIα was also used to generate a background control. The assay buffer consisted of 20mM Tris pH7.2, 150mM NaCl, and 0.002% Tween.

### Assay Reaction

### Examples 1- 233:

For examples 1 to 233, the assay was conducted according to the following: Each assay reaction was conducted in a volume of 25µl in a 384-well half-volume plate. 10 point compound serial dilutions (3-fold) were generated in DMSO at a concentration of x50 that of the final assay concentration (FAC). Compound solutions were then prepared by IgE-Tb diluting 10-fold in assay buffer. For the assay, 5µl of diluted compound was added to 10µl of, followed by addition of 10µl FcεRIα-Y131A-AF488. FRET reagents FACs were 5nM IgE-Tb, 25nM FcεRIα-Y131A-AF488. Usually the top FAC of compound in the assay was 10µM. The final DMSO concentration was 2%. The minimum signal (MIN) was measured by adding 5µl unlabelled FcεRIα at 1µM (FAC = 200nM) to the FRET reagents. The maximum FRET signal (MAX) was measured in wells containing FRET reagents but no compound. The assay was incubated for 2 hours at room temperature, protected from light and evaporation, and with gentle agitation.

### FRET measurement

Measurement of FRET for each well was carried out by exciting at 330nm and measuring emission at 495/520nm using an Envision plate reader (Perkin Elmer). FRET ratio was calculated as follows: $Emission at 520 / Emission at 495 \times 1000 .$

The FRET ratio was used for the data analysis.

### Data Analysis

Z' was calculated as follows (σ = standard deviation and µ = mean): $1 - \left(\left(3\times {σ}_{MAX}\right)+\left(3\times {σ}_{MIN}\right)\right) / \left({μ}_{MAX}-{μ}_{MIN}\right)$

Z' above 0.5 was considered a good assay.

Background signal (MIN) was subtracted from all wells. Using the background subtracted values, the percent inhibition by compound in each test-well was calculated as follows: $100 - Test - well FRET ratio / MAX FRET ratio \times 100 .$

### Examples 234-501:

For examples 234 to 501, the assay was conducted according to the following: Each assay reaction was conducted in a volume of 25µl in a 384-well half-volume plate. 10 point compound serial dilutions (3-fold) were generated in DMSO at a concentration of x50 that of the final assay concentration (FAC). Compound solutions were then prepared by diluting 10-fold in assay buffer. For the assay, 5µl of diluted compound was added to 10µl of IgE-Tb and incubated for 30 minutes before the addition of 10µl sFcER1α-Y131A-AF488. FRET reagents FACs were 5nM IgE-Tb, 25nM sFcER1α-Y131A-AF488. Usually the top FAC of compound in the assay was 10µM. The final DMSO concentration was 2%. The minimum signal (MIN) was measured by adding 5µl unlabelled sFcER1a at 1µM (FAC = 200nM) to the FRET reagents. The maximum FRET signal (MAX) was measured in wells containing FRET reagents but no compound.

The assay was incubated for 18 hours at room temperature, protected from light and evaporation, and with gentle agitation.

### FRET measurement

Measurement of FRET for each well was carried out by exciting at 337nm and measuring emission at 490/520nm using a PHERAstar FSX plate reader (BMG Labtech). FRET ratio was calculated as follows: $Emission at 520 / Emission at 490 \times 1000 .$

The FRET ratio was used for the data analysis.

### Data Analysis

Z' was calculated as follows (σ = standard deviation and µ = mean): $1 - \left(\left(3\times {σ}_{MAX}\right)+\left(3\times {σ}_{MIN}\right)\right) / \left({μ}_{MAX}-{μ}_{MIN}\right)$

Z' above 0.5 was considered a good assay.

Background signal (MIN) was subtracted from all wells. Using the background subtracted values, the percent inhibition by compound in each test-well was calculated as follows: $100 - Test - well FRET ratio / MAX FRET ratio \times 100 .$

Percent inhibition was plotted against compound concentration. IC50 values for each compound were determined using four parameter logistic fit model using the XLFIT5 software package.

The results are as follows:

| Example Number | Y131A FRET IC₅₀ range |
|---|---|
| 3,4,5,7,8,9,10,11,12,14,40,55,56,69,74, 98, 109,146,174. | 10-50 micromolar |
| 6,13, 39,58,60,64,65,66,68,70,71,72,73, 80,81,82,84,85,89, 92, 97,107, | 1-10 micromolar |
| 151,157,163,170,173,177,179,194,196,199,201,202,204,205,206, 214. | |
| 41,42,43,44,45,46,47,48,49,50,51,52,53,54,57,59,61,62,63,67,86, 100,104,105,106, | 0-1 micromolar |
| 110,111,112,113,114,115,116,117,118,119,120,121,122,123,124,125, 126,127, | |
| 130,131,132,133,134,135,136,137,138,139,140,141,142,143,144,145, 147,148,149,150,152,153,154,155,156,158,159,160,161,162,164,165, | |
| 166, 168,169,171,172,175,176,178,180,181,182,183,184, | |
| 186,187,188,189,190,191,192, 195,197,198, 203,207,208, | |
| 210,213,216,217,218,219,220,221,222,223,224,225,226,227,228,229, 230,231,232,233, 247, 260, 421, 234, 235, 236, 237, 238, 239, 240, 241, 242, 243, 244, 245, 246, 247, 248, 249, 250, 251, 252, 253, 254, 257, 258, 260, 261, 262, 263, 264, 265, 266, 267, 268, 269, 270, 271, 272, 273, 274, 275, 276, 277, 285, 286, 287, 288, 289,290, 291, 294, 295, 296, 297, 298, 299, 300, 301, 302, 303, 304, 305,, 307, 308, 337, 351, 403, 418, 419, 422, 423, 424, 427, 428, 429, 430, 431, 432, 433, 434, 501. | |

## Claims

1. Compounds of formula (I) and pharmaceutically acceptable salts thereof: 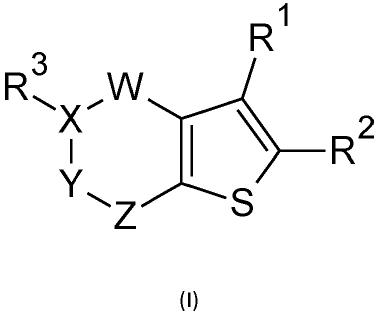 wherein
X represents CH, N, or C-R⁴, wherein when X is C-R⁴, R³ and R⁴ may be linked to form a 4-6 membered cycloalkyl or heterocyclic ring,
any of which may be optionally substituted by one or more substituents selected from halogen, =O, OH, SMe, C₁-C₂ alkoxy, O-C₁-C₂ haloalkyl, C₁-C₂ alkyl, C₁-C₂ heteroalkyl, C₁-C₂ haloalkyl, NH₂, NHMe, NMe₂, aryl and heteroaryl, with aryl and heteroaryl optionally substituted by one or more substituents selected from halogen, OH, C₁-C₆ alkyl, C₁-C₆
alkoxy and C₁-C₂ haloalkyl;
when X represents N, then W, Y and Z are C or one of W, Y and Z is a single bond, the other two being C;
when X represents CH or C-R⁴, then W, Y and Z are C, or one of W, Y or Z is a single bond, O, S, S(O), S(O)₂ or N, the other two being C;
when W, Y, or Z are C, each C may be substituted by 1 or 2 groups which are independently selected from H, halogen, SF₅, =O, =NOH, -OH, -CN, -C(O)Me, -C(O)OH, -C(O)NH₂, NH-C(O)O-C₁-C₄ alkyl, -NO₂, NH₂, NHMe, NMe₂, SH, SMe, SOMe, SO₂Me, C₁-C₄ alkyl, C₃-C₄ cycloalkyl, C₁-C₄ alkoxy, heterocycle, C₁-C₄ heteroalkyl, which may be linked to form a 3-6 membered cycloalkyl or 4-6 membered heterocyclic ring,
any of which may be optionally substituted by one or more substituents selected from halogen, =O, OH, SMe, C₁-C₂ alkoxy, O-C₁-C₂ haloalkyl, C₁-C₂ alkyl, C₁-C₂ heteroalkyl, C₁-C₂ haloalkyl, NH₂, NHMe, NMe₂, which may be linked to form a 3-6 membered cycloalkyl or 4-6 membered heterocyclic ring;
wherein
when W, Y or Z is N, this N may be independently substituted by H, C₁-C₄ alkyl, C₃-C₄ cycloalkyl, heterocycle, -C(O)Me, -C(O)NH₂, -C(O)OH, -S(O)Me, -S(O)₂Me, -S(O)₂NH₂, any of which may be optionally substituted by one or more substituents selected from halogen, OH, SMe, C₁-C₂ alkoxy, O-C₁-C₂ haloalkyl, C₁-C₂ alkyl, C₁-C₂ heteroalkyl, C₁-C₂ haloalkyl, NH₂, NHMe, NMe₂;
and W, X, Y and Z may be linked directly or via substituents to form a ring;
R¹ represents 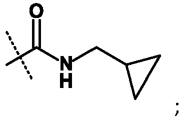
R² represents 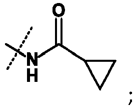
R³ is selected from H, -OH, C₁-C₈ alkyl, -NR^{d}R^{c},
-NH-R¹⁴-NH-R^{a}, -NMe-R¹⁴-NH-R^{a}, -NH-R¹⁴-NMₑ-R^{a}, -CO-NR^{d}R^{c}, -NH-COO-R^{c}, -NH-CO-R^{c}, -COOH, - COO-C₁-C₈ alkyl, -CO-C₁-C₈ alkyl, -C₁-C₈ alkylene-COO-C₁-C₈ alkyl, -C₁-C₈ alkylene-NR^{a} -COO-C₁-C₈ alkyl, -C₁-C₈ alkylene-NR^{d}R^{c}, -C₁-C₈ alkylene-NH-R¹⁴-NH-R^{a}, heteroaryl, -CN, -CH=N-OH, -CF₃, alkylamino, amino, -NH-SO-C₁-C₈ alkyl, -NH-SO₂-C₁-C₈ alkyl, heteroalkyl-amino, aryl, heterocycle, C₃-C₆ cycloalkyl, -NHS(O)(=NH)Me, -SO₂-C₁-C₈ alkyl, -NH-C(CH₃)=N-CN, -NH-CH=N-CN, -NH-S(=O)(=N)-C₁-C₂ alkyl,
any of which may be optionally substituted by one or more substituents selected from halogen, -OH, =O, -COOH, -COH, -CN, SMe, -NR^{d}R^{c}, -OR^{c}, C₁-C₂ alkoxy, O-C₁-C₂ haloalkyl, C₁-C₂ alkyl, C₁-C₂ heteroalkyl, C₁-C₂ haloalkyl, C₁-C₂ alkylene-O-C₁-C₂ alkyl, C₁-C₂ alkylene-C₃-C₄ cycloalkyl, heterocycle, C₃-C₆ cycloalkyl, aryl, heteroaryl, C₃-C₆ cycloalkyl-amino, heterocyclo-amino, aryl-amino, heteroaryl-amino, -CONR^{d}R^{c} , -NHCO-R^{a}, -NHCOO-C₁-C₄ alkyl, -CO-C₁-C₂ alkyl, - COO-R^{a}, which may be linked to form a ring, and wherein -CONR^{d}R^{c} and -NR^{d}R^{c} may be linked to form a seven-membered ring fused to a six-membered aryl group;
with aryl, heteroaryl, heterocycle and cycloalkyl optionally substituted by one or more substituents selected from halogen oxo, cyano, OH, -NOOH, C₁-C₆ alkyl, C₁-C₆ alkoxy, halo-C₁-C₆ alkoxy, C₃-C₆-cycloalkyl-C₁-C₃-alkoxy, -O-CO-C₁-C₆ alkyl, C₃-C₆ cycloalkyl, C₁-C₂ haloalkyl, C₂-C₄ heterocycle, acetyl, acetyl-phenyl, -CONR^{d}R^{c}, COO-C₁-C₈ alkyl, -S(O)NH-C₁-C₂-alkyl, -S(O)ₙ-C₁-C₂-alkyl, heteroaryl which may be optionally substituted with one or more halogen group, C₁-C₂-alkyl,
with n equal 0, 1 or 2 ;
R⁴ is selected from halogen, OH, CN, NO₂, NH₂, NHMe, NMe₂, SH, SMe, SOMe, SO₂Me, C₁-C₄ alkyl, C₃-C₄ cycloalkyl, C₁-C₃ alkoxy, C₁-C₃ heteroalkyl, heterocycle,
any of which may be optionally substituted by one or more substituents selected from halogen, =O, OH, SMe, C₁-C₂ alkoxy, O-C₁-C₂ haloalkyl, C₁-C₂ alkyl, C₁-C₂ heteroalkyl,
C₁-C₂ haloalkyl NH₂, NHMe, NMe₂, which may be linked to form a ring;
R¹⁴ is C=O, C=S, C=N-OH, C=NR^{b}, C=CH-NO₂, SO₂, and C₃-C₆ cycloalkenyl which may be substituted with 1 or 2 =O;
R^{a} is selected from H, C₁-C₈ alkyl, heteroalkyl, C₁-C₈ alkoxy, heterocycle, C₃-C₈ cycloalkyl, heteroaryl and aryl,
any of which may be optionally substituted by one or more substituents selected from halogen, =O, OH, SMe, C₁-C₂ alkoxy, -O-C₁-C₂ haloalkyl, C₁-C₂ alkyl, C₃-C₆ cycloalkyl, C₃-C₆ heterocycloalkyl, C₁-C₂ heteroalkyl, C₁-C₂ haloalkyl, aryl, heteroaryl, NH₂, NHMe, NMe₂ which may be linked to form a ring;
R^{b} is selected from CN, aryl, heteroaryl, -NO₂, -O-C₁-C₂ alkyl, -CO-O-C₁-C₂ alkyl,
with aryl and heteroaryl optionally substituted by one or more C₁-C₂ alkyl;
R^{c} is selected from H, aryl, heteroaryl, C₃-C₆ cycloalkenyl,C₃-C₈ cycloalkyl, heterocycle, C₁-C₈ alkyl, C₁-C₈ heteroalkyl, C₁-C₄ alkylene-C₃-C₆ cycloalkyl;
any of which may be optionally substituted by one or more substituents selected from C(O)(R^{g}), C(O)N(R^{f})(R^{g}), halogen, SF₅, OH, =O, -COH, -COOH, -CO-O-R^{a}, CN, SMe, SO₂Me, SO₂R^{g}, C₁-C₄ alkoxy, O-C₁-C₄ haloalkyl, C₁-C₄ alkyl, C₁-C₂ heteroalkyl, C₁-C₄ haloalkyl, N(R^{f})(R^{g}), N(R^{f})SO₂(R^{g}), SO₂NH₂, SO₂NHMe, SO₂NMe₂, S(O)(NR^{f})R^{g}, C₃-C₆ cycloalkyl, heterocycle, C₁-C₄ alkylene-C₃-C₆ cycloalkyl, aryl and heteroaryl, which may be linked to form a ring
with aryl, heteroaryl, heterocycle and cycloalkyl optionally substituted by one or more substituents selected from halogen, =O, =NH, =NMe, OH, C₁-C₂ alkyl, C₁-C₂ alkoxy, -COOH, -COO-C₁-C₇ alkyl, SO₂R^{a}, COR^{a}, SOR^{a}, S(O)(NH)R^{a}, CONHR^{a}, which may be linked to form a ring;
R^{d} represents H or C₁-C₄ alkyl
and R^{c} and R^{d} may be linked to form a heterocycle;
R^{f} is selected from H, aryl, heteroaryl, C₃-C₆ cycloalkenyl, C₃-C₈ cycloalkyl, heterocycle, C₁-C₈ alkyl, C₁-C₈ heteroalkyl, C₁-C₄ alkylene-C₃-C₆ cycloalkyl ,
any of which may be optionally substituted by one or more substituents selected from halogen, =O, =NH, =NMe, OH, aryl, heteroaryl, C₁-C₅ alkyl, C₁-C₇ haloalkyl, C₁-C₇ alkoxy, O-C₁-C₂ haloalkyl, -COOH, -COO- C₁-C₂ alkyl, SO₂R^{a}, COR^{a}, SOR^{a}, S(O)(NH)R^{a}, CONHR^{a};
R^{g} is selected from H, aryl, heteroaryl, C₃-C₆ cycloalkenyl, C₃-C₈ cycloalkyl, heterocycle, C₁-C₈ alkyl, C₁-C₈ heteroalkyl, C₁-C₄ alkylene-C₃-C₆ cycloalkyl ,
any of which may be optionally substituted by one or more substituents selected from halogen, =O, =NH, =NMe, OH, aryl, heteroaryl, C₁-C₂ alkyl, C₁-C₂ haloalkyl, C₁-C₇ alkoxy, O-C₁-C₂ haloalkyl, -COOH, -COO- C₁-C₇ alkyl, SO₂R^{a}, COR^{a}, SOR^{a}, S(O)(NH)R^{a}, CONHR^{a}
R^{f} and R^{g} may be linked to form a ring.

2. A compound according to claim 1 or a pharmaceutically acceptable salt thereof, which is a compound of formula (Ia): 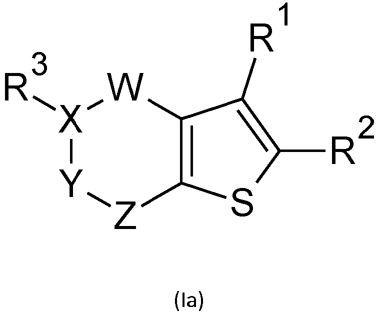 wherein
X represents CH, N, or C-R⁴, wherein when X is C-R⁴, R³ and R⁴ may be linked to form a 4-6 membered cycloalkyl or heterocyclic ring,
any of which may be optionally substituted by one or more substituents selected from halogen, =O, OH, SMe, C₁-C₂ alkoxy, O-C₁-C₂ haloalkyl, C₁-C₂ alkyl, C₁-C₂ heteroalkyl, C₁-C₂ haloalkyl, NH₂, NHMe, NMe₂, aryl and heteroaryl, with aryl and heteroaryl optionally substituted by one or more substituents selected from halogen, OH, C₁-C₆ alkyl, C₁-C₆
alkoxy and C₁-C₂ haloalkyl;
when X represents N, then W, Y and Z are C or one of W, Y and Z is a single bond, the other two being C;
when X represents CH or C-R⁴, then W, Y and Z are C, or one of W, Y or Z is a single bond, O, S, S(O), S(O)₂ or N, the other two being C;
when W, Y, or Z are C, each C may be substituted by 1 or 2 groups which are independently selected from H, halogen, SF₅, =O, =NOH, -OH, -CN, -C(O)Me, -C(O)OH, -C(O)NH₂, NH-C(O)O-C₁-C₄ alkyl, -NO₂, NH₂, NHMe, NMe₂, SH, SMe, SOMe, SO₂Me, C₁-C₄ alkyl, C₃-C₄ cycloalkyl, C₁-C₄ alkoxy, heterocycle, C₁-C₄ heteroalkyl, which may be linked to form a 3-6 membered cycloalkyl or 4-6 membered heterocyclic ring,
any of which may be optionally substituted by one or more substituents selected from halogen, =O, OH, SMe, C₁-C₂ alkoxy, O-C₁-C₂ haloalkyl, C₁-C₂ alkyl, C₁-C₂ heteroalkyl, C₁-C₂ haloalkyl, NH₂, NHMe, NMe₂, which may be linked to form a 3-6 membered cycloalkyl or 4-6 membered heterocyclic ring;
wherein
when W, Y or Z is N, this N may be independently substituted by H, C₁-C₄ alkyl, C₃-C₄ cycloalkyl, heterocycle, -C(O)Me, -C(O)NH₂, -C(O)OH, -S(O)Me, -S(O)₂Me, -S(O)₂NH₂, any of which may be optionally substituted by one or more substituents selected from halogen, OH, SMe, C₁-C₂ alkoxy, O-C₁-C₂ haloalkyl, C₁-C₂ alkyl, C₁-C₂ heteroalkyl, C₁-C₂ haloalkyl, NH₂, NHMe, NMe₂;
and W, X, Y and Z may be linked directly or via substituents to form a ring;
R¹ represents 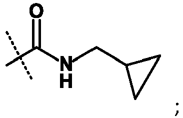
R² represents 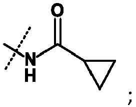
R³ is selected from H, -OH, C₁-C₈ alkyl, -NR^{d}R^{c},
-NH-R¹⁴-NH-R^{a}, -NMe-R¹⁴-NH-R^{a}, -NH-R¹⁴-NMe-R^{a}, -CO-NR^{d}R^{c}, -NH-COO-R^{c}, -NH-CO-R^{c}, -COOH, - COO-C₁-C₈ alkyl, -CO-C₁-C₈ alkyl, -C₁-C₈ alkylene-COO-C₁-C₈ alkyl, -C₁-C₈ alkylene-NR^{a} -COO-C₁-C₈ alkyl, -C₁-C₈ alkylene-NR^{d}R^{c}, -C₁-C₈ alkylene-NH-R¹⁴-NH-R^{a}, heteroaryl, -CN, -CH=N-OH, -CF₃, alkylamino, amino, -NH-SO-C₁-C₈ alkyl, -NH-SO₂-C₁-C₈ alkyl, heteroalkyl-amino, aryl, heterocycle, C₃-C₆ cycloalkyl, -NHS(O)(=NH)Me, -SO₂-C₁-C₈ alkyl, -NH-C(CH₃)=N-CN, -NH-CH=N-CN, -NH-S(=O)(=N)-C₁-C₂ alkyl,
any of which may be optionally substituted by one or more substituents selected from halogen, -OH, =O, -COOH, -COH, -CN, SMe, -NR^{d}R^{c}, -OR^{c}, C₁-C₂ alkoxy, O-C₁-C₂ haloalkyl, C₁-C₂ alkyl, C₁-C₂ heteroalkyl, C₁-C₂ haloalkyl, C₁-C₂ alkylene-O-C₁-C₂ alkyl, C₁-C₂ alkylene-C₃-C₄ cycloalkyl, heterocycle, C₃-C₆ cycloalkyl, aryl, heteroaryl, C₃-C₆ cycloalkyl-amino, heterocycle-amino, aryl-amino, heteroaryl-amino, -CONR^{d}R^{c} , -NHCO-R^{a}, -NHCOO-C₁-C₄ alkyl, -CO-C₁-C₂ alkyl, -COO-R^{a}, which may be linked to form a ring,
with aryl, heteroaryl, heterocycle and cycloalkyl optionally substituted by one or more substituents selected from halogen, OH, C₁-C₆ alkyl, C₁-C₆ alkoxy, -O-CO-C₁-C₆ alkyl, C₃-C₆ cycloalkyl, C₁-C₂ haloalkyl and C₂-C₄ heterocycle;
R⁴ is selected from halogen, OH, CN, NO₂, NH₂, NHMe, NMe₂, SH, SMe, SOMe, SO₂Me, C₁-C₄ alkyl, C₃-C₄ cycloalkyl, C₁-C₃ alkoxy, C₁-C₃ heteroalkyl, heterocycle,
any of which may be optionally substituted by one or more substituents selected from halogen, =O, OH, SMe, C₁-C₂ alkoxy, O-C₁-C₂ haloalkyl, C₁-C₂ alkyl, C₁-C₂ heteroalkyl,
C₁-C₂ haloalkyl NH₂, NHMe, NMe₂, which may be linked to form a ring;
R¹⁴ is C=O, C=S, C=N-OH, C=NR^{b}, C=CH-NO₂, SO₂, and C₃-C₆ cycloalkenyl which may be substituted with 1 or 2 =O;
R^{a} is selected from H, C₁-C₈ alkyl, heteroalkyl, C₁-C₈ alkoxy, heterocycle, C₃-C₈ cycloalkyl, heteroaryl and aryl,
any of which may be optionally substituted by one or more substituents selected from halogen, =O, OH, SMe, C₁-C₂ alkoxy, -O-C₁-C₂ haloalkyl, C₁-C₂ alkyl, C₃-C₆ cycloalkyl, C₁-C₂ heteroalkyl, C₁-C₂ haloalkyl, aryl, heteroaryl, NH₂, NHMe, NMe₂ which may be linked to form a ring; R^{b} is selected from CN, aryl, heteroaryl, -NO₂, -O-C₁-C₂ alkyl, -CO-O-C₁-C₂ alkyl,
with aryl and heteroaryl optionally substituted by one or more C₁-C₂ alkyl;
R^{C} is selected from H, aryl, heteroaryl, C₃-C₆ cycloalkenyl,C₃-C₈ cycloalkyl, heterocycle, C₁-C₈ alkyl, C₁-C₈ heteroalkyl, C₁-C₄ alkylene-C₃-C₆ cycloalkyl;
any of which may be optionally substituted by one or more substituents selected from C(O)(R^{g}), C(O)N(R^{f})(R^{g}), halogen, SF₅, OH, =O, -COH, -COOH, -CO-O-R^{a}, CN, SMe, SO₂Me, SO₂R^{g}, C₁-C₄ alkoxy, O-C₁-C₄ haloalkyl, C₁-C₄ alkyl, C₁-C₂ heteroalkyl, C₁-C₄ haloalkyl, N(R^{f})(R^{g}), N(R^{f})SO₂(R^{g}), SO₂NH₂, SO₂NHMe, SO₂NMe₂, S(O)(NR^{f})R^{g}, C₃-C₆ cycloalkyl, heterocycle, C₁-C₄ alkylene-C₃-C₆ cycloalkyl, aryl and heteroaryl, which may be linked to form a ring
with aryl, heteroaryl, heterocycle and cycloalkyl optionally substituted by one or more substituents selected from halogen, =O, =NH, =NMe, OH, C₁-C₂ alkyl, C₁-C₂ alkoxy, -COOH, -COO-C₁-C₂ alkyl, SO₂R^{a}, COR^{a}, SOR^{a}, S(O)(NH)R^{a}, CONHR^{a}, which may be linked to form a ring;
R^{d} represents H or C₁-C₄ alkyl
and R^{c} and R^{d} may be linked to form a heterocycle;
R^{f} is selected from H, aryl, heteroaryl, C₃-C₆ cycloalkenyl, C₃-C₈ cycloalkyl, heterocycle, C₁-C₈ alkyl, C₁-C₈ heteroalkyl, C₁-C₄ alkylene-C₃-C₆ cycloalkyl ,
any of which may be optionally substituted by one or more substituents selected from halogen, =O, =NH, =NMe, OH, aryl, heteroaryl, C₁-C₂ alkyl, C₁-C₂ haloalkyl, C₁-C₂ alkoxy, O-C₁-C₂ haloalkyl, -COOH, -COO- C₁-C₂ alkyl, SO₂R^{a}, COR^{a}, SOR^{a}, S(O)(NH)R^{a}, CONHR^{a};
R^{g} is selected from H, aryl, heteroaryl, C₃-C₆ cycloalkenyl, C₃-C₈ cycloalkyl, heterocycle, C₁-C₈ alkyl, C₁-C₈ heteroalkyl, C₁-C₄ alkylene-C₃-C₆ cycloalkyl ,
any of which may be optionally substituted by one or more substituents selected from halogen, =O, =NH, =NMe, OH, aryl, heteroaryl, C₁-C₂ alkyl, C₁-C₂ haloalkyl, C₁-C₂ alkoxy, O-C₁-C₂ haloalkyl, -COOH, -COO- C₁-C₂ alkyl, SO₂R^{a}, COR^{a}, SOR^{a}, S(O)(NH)R^{a}, CONHR^{a}
R^{f} and R^{g} may be linked to form a ring.

3. A compound according to claim 1 or 2, or a pharmaceutically acceptable salt thereof, which is a compound of formula (II): 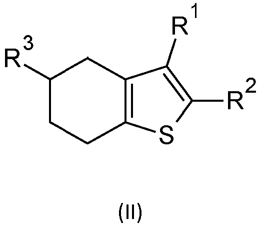 wherein R¹, R² and R³ are as defined in claim 1.

4. A compound according to any of claims 1 or 2 or a pharmaceutically acceptable salt thereof, wherein X is C or N; Z is C, N, O or S; Y is C or S.

5. A compound according to anyone of claims 1 or 2 or a pharmaceutically acceptable salt thereof, wherein X Y, W is C and Z is N wherein N is substituted with a group selected from methylsulfonyl, acetyl ethylcarbamoyl, cyano.

6. A compound according to anyone of claims 1 or 2 or a pharmaceutically acceptable salt thereof, wherein X, Y, W, Z is C and wherein Z is substituted with a group selected from OH, methyl, ethylcarbamoyl, cyano, oxo, tert-butoxycarbonylamino, 1H-pyrazol-3-yl,

7. A compound according to claim 1 or 2 or a pharmaceutically acceptable salt thereof, wherein X, Y, W, Z is C and wherein W is substituted with a methyl group and R³ is H or a methyl group.

8. A compound according to anyone of claims 1 to 4 or a pharmaceutically acceptable salt thereof, wherein X is C or N and R³ is selected from H, OH, methyl, amino, methylsulfonyl, acetyl, 2-methoxyacetyl, 2-phenylacetyl, 2-(tert-butoxycarbonylamino)ethyl, 2-phenylethyl, pyrimidin-2-ylamino, methanesufonamido, (4-methoxyphenyl)carbamothioylamino, (4-Methoxyphenyl)carbamoylamino, ethylcarbamoyl, [N'-Cyano-N-(p-tolyl)carbamimidoyl]amino, [N'-Cyano-N-ethyl-carbamimidoyl]amino, (1-oxoisoindolin-5-yl)carbamoyl, (3-sulfamoylphenyl)carbamoyl, (3-methylsulfonylphenyl)carbamoyl, 3-isoxazol-5-ylanilino, (2-ethoxy-3,4-dioxo-cyclobuten-1-yl)amino, ethylcarbonyl, carboxy, (1-oxoisoindolin-5-yl)aminocarbonyl, [(5-oxopyrrolidin-3-yl)phenyl]aminocarbonyl, [3-(3-methyl-5-oxo-4H-pyrazol-1-yl)phenyl]carbonylamino, (1H-indazol-4-yl)carbonylamino, 1H-imidazo[4,5-b]pyrazin-2-yl, indan-2-yl, [2-(4-methoxyphenyl)ethylaminocarbonyl, quinoxalin-6-yl, (thiazol-2-yl)aminocarbonyl, (1H-tetrazol-5-yl)aminocarbonyl, (4-pyridyl)aminocarbonyl, pyrazin-2-ylaminocarbonyl, (3-pyridyl)aminocarbonyl, 3-hydroxyazetidine-1-carbonyl, 1,1-dioxo-1,4-thiazinane-4-carbonyl, 2-(4-hydroxyphenyl)ethylaminocarbonyl, hydroxyiminomethyl, [(4-ethyl-1,2,4-triazol-3-yl)amino]methyl, (4-methylanilino)methyl, 2-Amino-3-pyridyl, 5-(p-tolyl)-1H-imidazol-2-yl, cyano, trifluoromethyl, 4-methyloxazol-2-yl, aminomethyl, (p-tolylcarbamoylamino)methyl, 3-methyl-1,2,4-oxadiazol-5-yl, 5-methyl-1,3,4-oxadiazol-2-yl, pyrrolidine-1-carbonyl, 3-pyridylcarbamothioylamino, 3-(3-pyridylamino)-1,2,4-triazol-4-yl, 1H-pyrazol-3-yl, 5-Amino-4-cyano-imidazol-1-yl, 3-(2,2-dimethylpropylamino)-1,2,4-triazol-4-yl, 3-[(2,5-dimethylpyrazol-3-yl)amino]-1,2,4-triazol-4-yl, 4-(2,5-dimethylpyrazol-3-yl)-1,2,4-triazol-3-yl]amino, [(2,5-dimethylpyrazol-3-yl)amino]-1,2,4-triazol-4-yl, N'-Cyano-N-phenyl-carbamimidoylamino, 3-(isobutylamino)-1,2,4-triazol-4-yl, 3-[(1-methylcyclopropyl)methylamino]-1,2,4-triazol-4-yl, 5-Acetamidoimidazol-1-yl, 3-[(5-methyl-3-pyridyl)amino]-1,2,4-triazol-4-yl, 3-(4-pyridylamino)-1,2,4-triazol-4-yl, N'-Cyano-N-(3,4-dimethoxyphenyl)carbamimidoyl]amino, 4-ethoxycarbony-5-(ethylamino)imidazole-1-yl, 3-(2-morpholinoethylamino)-1,2,4-triazol-4-yl, (5-oxo-4H-1,2,4-oxadiazol-3-yl)amino, 2-(ethylamino)-3,4-dioxo-cyclobuten-1-yl]amino, [N'-cyano-N-(2,2,2-trifluoroethyl)carbamimidoyl]amino, 3-(ethylamino)-1,2,4-triazol-4-yl, (N-ethyl-N'-nitro-carbamimidoyl)amino, 3-(3-pyridylamino)-1,2,4-triazol-4-yl, 3-[(1-oxidopyridin-1-ium-3-yl)amino, [N'-nitro-N-(p-tolyl)carbamimidoyl]amino, 2-(3-pyridylamino)imidazol-1-yl, 3-(3-pyridylamino)-1,2,4-triazol-4-yl, 3-(cyclopropylmethylamino, (2,5-dimethylpyrazol-3-yl)carbamothioylamino, 3-(pyrimidin-5-ylamino)-1,2,4-triazol-4-yl, 2-(cyclopropylmethylamino)imidazol-1-yl, [4-(ethylamino)-1,1-dioxo-1,2,5-thiadiazol-3-yl]amino, [1-(4-methylanilino)-2-nitro-vinyl]amino, (N'-cyano-N-ethyl-carbamimidoyl)-methyl-amino, [2-(4-methylanilino)-3,4-dioxo-cyclobuten-1-yl]amino, 3-(2-pyridylamino)-1,2,4-triazol-4-yl, 2-Acetamidoimidazol-1-yl, 5-(N-tert-butyloxycarbonylamino)-triazol-1-yl, 5-(3-pyridylamino)triazol-1-yl, ethylcarbamothioylamino, 3-[[(3S)-tetrahydrofuran-3-yl]amino]-1,2,4-triazol-4-yl, [(E)-N-ethyl-N'-nitro-carbamimidoyl]amino, 2-(ethylamino)imidazol-1-yl, 3-[(3,5-dimethylisoxazol-4-yl)amino, [[N'-ethoxycarbonyl-N-(p-tolyl)carbamimidoyl]amino], [2-[(2-methyl-3-pyridyl)amino]imidazol-1-yl, 1-[(ethylamino)-2-nitro-vinyl]amino, (4-methyl-6-oxo-1H-pyrimidin-2-yl)amino, 5-amino-4-(methylcarbamoyl)imidazol-1-yl, 5-amino-4-carbamoyl-imidazol-1-yl, 2-Anilinoimidazol-1-yl, ethylcarbamoylamino, (4-oxo-1H-pyridin-2-yl)amino, [6-(5-methyl-1,3,4-oxadiazol-2-yl)-3-pyridyl]amino, 5-(ethylamino)tetrazol-1-yl, 1H-1,2,4-triazol-3-ylamino, 4-(1-methylpyrazol-4-yl)-1,2,4-triazol-3-yl]amino, 4-(ethyloxy-carbonyl)imidazol-1-yl, [4-(4-fluorophenyl)-1,2,4-triazol-3-yl]amino, 1H-imidazol-2-ylamino, N'-methoxy-N-(p-tolyl)carbamimidoyl]amino, [4-(4-methylanilino)-1,1-dioxo-1,2,5-thiadiazol-3-yl]amino, [1-(3-pyridyl)imidazol-2-yl]amino, 5-aminotriazol-1-yl, (2-methoxyphenyl)carbamoylamino, 4-pyridine-carbonylamino, benzylamino, 1H-Benzimidazol-2-ylamino, [N'-hydroxycarbamimidoyl]amino, [N'-(1-methylpyrazol-3-yl)-N-(p-tolyl)carbamimidoyl]amino, 3-[(3,5-dimethylpyrazin-2-yl)amino, 3-(ethylamino)-5-(methoxymethyl)-1,2,4-triazol-4-yl, [5-(3-pyridyl)-1H-1,2,4-triazol-3-yl]amino, p-tolyl-carbonylamino, quinazolin-2-ylamino, 1,3-Benzoxazol-2-ylamino, 3-(ethylamino)-5-(hydroxymethyl)-1,2,4-triazol-4-yl, 2-aminoimidazol-1-yl, 3-amino-5-oxo-1,2,4-oxadiazol-4-yl, 3-(ethylamino)-5-methyl-1,2,4-triazol-4-yl, (2-ethyl-3,4-dioxo-cyclobuten-1-yl)amino, 1,3,4-oxadiazol-2-ylamino, 5-oxo-1-(p-tolyl)-4H-imidazol-2-yl]amino, [2-[ethyl(methyl)amino]-3,4-dioxo-cyclobuten-1-yl]amino, (methylsulfonimidoyl)amino, [N-Cyano-C-methyl-carbonimidoyl]amino, tetrazol-1-yl, methanesulfinamido, morpholino, 3-(ethylamino)-5-(trifluoromethyl)-1,2,4-triazol-4-yl, (4-oxo-1H-pyrimidin-2-yl)amino, isopropylsulfamoylamino, N'-Cyano-N-morpholino-carbamimidoyl]amino, acetamido, methanesulfonamido, [N'-Cyano-N-methoxy-carbamimidoyl]amino, (4-methoxyphenyl)carbamoylamino, [4-oxo-6-(trifluoromethyl)-1H-pyrimidin-2-yl]amino, [4-(trifluoromethoxy)phenyl]carbamoylamino, 3-(2-methylpropanoylamino)-1,2,4-triazol-4-yl, 3-[(2,5-dimethylpyrazol-3-yl)-methyl-amino]-1,2,4-triazol-4-yl, 5-amino-4-(5-methyl-1,3,4-oxadiazol-2-yl)imidazol-1-yl, 3-[(3-methyl-1H-pyrazol-5-yl)amino]-1,2,4-triazol-4-yl, 3-[(1,3-dimethylpyrazol-4-yl)amino]-1,2,4-triazol-4-yl -[(5-cyclopropyl-2-methyl-pyrazol-3-yl)amino]-1,2,4-triazol-4-yl, 3-[(5-isopropyl-2-methyl-pyrazol-3-yl)amino]-1,2,4-triazol-4-yl, 3-[[2-methyl-5-(trifluoromethyl)pyrazol-3-yl]amino]-1,2,4-triazol-4-yl, 3-[(2-isopropyl-5-methyl-pyrazol-3-yl)amino, 3-[(5-methyl-2-tetrahydrofuran-3-yl-pyrazol-3-yl)amino]-1,2,4-triazol-4-yl, 3-[(2-methylpyrazol-3-yl)amino]-1,2,4-triazol-4-yl, 3-[(2-ethyl-5-methyl-pyrazol-3-yl)amino]-1,2,4-triazol-4-yl, 3-[(2-cyclopropyl-5-methyl-pyrazol-3-yl)amino]-1,2,4-triazol-4-yl, 3-[(3-methoxy-1-methyl-pyrazol-4-yl)amino]-1,2,4-triazol-4-yl, 3-[(5-ethyl-2-methyl-pyrazol-3-yl)amino]-1,2,4-triazol-4-yl, 3-[[5-(difluoromethyl)-2-methyl-pyrazol-3-yl]amino, 3-[(1,4-dimethylpyrazol-3-yl)amino]-1,2,4-triazol-4-yl, [3-[(5-methoxycarbonyl-2-methyl-pyrazol-3-yl)amino]-1,2,4-triazol-4-yl], 2-[[2-methyl-5-(trifluoromethyl)pyrazol-3-yl]amino]imidazol-1-yl, [3-[(5-carboxy-2-methyl-pyrazol-3-yl)amino]-1,2,4-triazol-4-yl].

9. Compounds according to claim 1 or a pharmaceutically acceptable salt thereof, wherein W, X, Y, Z is C and Z is optionally substituted with a methyl group.

10. Compounds according to claims 1 to 4 or a pharmaceutically acceptable salt thereof, wherein
R³ is selected from (1,1-dioxothiolan-3-yl)amino-1,2,4-triazol-4-yl, 3-[3-[methylsulfonimidoyl]anilino]-1,2,4-triazol-4-yl, 3-[(1-acetyl-3-methylpyrrolidin-3-yl)amino]-4H-1,2,4-triazol-4-yl, 3-{[1-(6-methylpyridazin-3-yl)pyrrolidin-3-yl]amino}-4H-1,2,4-triazol-4-yl, 3-[[-1-pyrazin-2-ylpyrrolidin-3-yl]amino]-1,2,4-triazol-4-yl, 3-[(4-methyl-1-methylsulfonyl-pyrrolidin-3-yl)amino]-1,2,4-triazol-4-yl, 3-[(1-oxo-2,3-dihydro-1H-isoindol-4-yl)amino]-4H-1,2,4-triazol-4-yl, 3-[[-1-(6-fluoropyridazin-3-yl)pyrrolidin-3-yl]amino]-1,2,4-triazol-4-yl, 3-[[1-pyrimidin-2-ylpyrrolidin-3-yl]amino]-1,2,4-triazol-4-yl, 5-amino-4-(2-methoxyethoxycarbonyl)imidazol-1-yl, 5-amino-4-(2-morpholinethoxycarbonyl)imidazol-1-yl, 3-(3-methylsulfanylanilino)-1,2,4-triazol-4-yl, 3-(3-methylsulfonylanilino)-1,2,4-triazol-4-yl, 3-(3-methylsulfinylanilino)-1,2,4-triazol-4-yl, 3-[3-(methylsulfonimidoyl)anilino]-1,2,4-triazol-4-yl, 3-[(6-methylsulfinyl-2-pyridyl)amino]-1,2,4-triazol-4-yl, 3-[(6-methylsulfonyl-2-pyridyl)amino]-1,2,4-triazol-4-yl, 8-methoxy-4-oxo-5,10-dihydroimidazo[4,5-c][1,5]benzodiazepin-1-yl, 8-methoxy-5-methyl-4-oxo-10H-imidazo[4,5-c][1,5]benzodiazepin-1-yl, 5-amino-4-[(2S)-2-methylpyrrolidine-1-carbonyl]imidazol-1-yl, 5-amino-4-[(3R)-3-hydroxypyrrolidine-1-carbonyl]imidazol-1-yl, 5-amino-4-[(2S)-2-methylpyrrolidine-1-carbonyl]imidazol-1-yl, 5-[[4-(dihydroxyamino)-2,5-dimethyl-3H-pyrazol-3-yl]amino]-4-ethoxycarbonyl-imidazol-1-yl, 5-[[4-(dihydroxyamino)-2,5-dimethyl-3H-pyrazol-3-yl]amino]-4-ethoxycarbonyl-imidazol-1-yl, 3-(2-methyl-3-methylsulfinyl-anilino)-1,2,4-triazol-4-yl, 3-(2-methyl-3-methylsulfonyl-anilino)-1,2,4-triazol-4-yl, 3-[(2-methyl-5-oxazol-2-yl-pyrazol-3-yl)amino]-1,2,4-triazol-4-yl, 1-acetylpyrrolidin-3-yl]amino]-1,2,4-triazol-4-yl, 1-methylsulfonylpyrrolidin-3-yl]amino]-1,2,4-triazol-4-yl, 3-[(2-methylcyclohexyl)amino]-1,2,4-triazol-4-yl, 3-[(5-oxopyrrolidin-3-yl)amino]-1,2,4-triazol-4-yl, 3-[(2-oxo-4-piperidyl)amino]-1,2,4-triazol-4-yl, 3-[(1-acetyl-2-methyl-pyrrolidin-3-yl)amino]-1,2,4-triazol-4-yl, 3-[(2-methyl-1-methylsulfonyl-pyrrolidin-3-yl)amino]-1,2,4-triazol-4-yl, -[3-[[3-(6-methyl-3-pyridyl)cyclobutyl]amino]-1,2,4-triazol-4-yl, , 3-[[5-(difluoromethoxy)-2-methyl-pyrazol-3-yl]amino]-1,2,4-triazol-4-yl, [[1-benzoyl-4,4-difluoro-pyrrolidin-3-yl]amino]-1,2,4-triazol-4-yl, 3-[(1-acetyl-4-piperidyl)amino]-1,2,4-triazol-4-yl, 3-[(1-methoxycarbonylpyrrolidin-3-yl)amino]-1,2,4-triazol-4-yl, 3-[[1-(ethylcarbamoyl)pyrrolidin-3-yl]amino]-1,2,4-triazol-4-yl, 3-{[2-(pyrimidin-2-yl)-2-azaspiro[4.4]nonan-7-yl]amino}-4H-1,2,4-triazol-4-yl, 3-[[2-methyl-5-(trifluoromethoxy)pyrazol-3-yl]amino]-1,2,4-triazol-4-yl, 3-[[5-[chloro(difluoro)methoxy]-2-methyl-pyrazol-3-yl]amino]-1,2,4-triazol-4-yl, 3-{[1-methyl-3-(5-methyl-1,3,4-oxadiazol-2-yl)-1H-pyrazol-5-yl]amino}-4H-1,2,4-triazol-4-yl, 3-[(5-methoxy-3-methyl-pyrazin-2-yl)amino]-1,2,4-triazol-4-yl, (5-methylthiazolo[5,4-b]pyridin-2-yl)amino, 3-[(5-cyano-2-methyl-3-pyridyl)amino]-1,2,4-triazol-4-yl, 3-[(6-cyano-2-methyl-3-pyridyl)amino]-1,2,4-triazol-4-yl, 3-[(1-methyl-2-oxo-4-pyridyl)amino]-1,2,4-triazol-4-yl, 3-[(6-chloro-2-methyl-3-pyridyl)amino]-1,2,4-triazol-4-yl, 3-[[2-methyl-6-(trifluoromethyl)-3-pyridyl]amino]-1,2,4-triazol-4-yl, 3-[(3-methylpyridazin-4-yl)amino]-1,2,4-triazol-4-yl, 3-[(5-chloro-2-methyl-pyrazol-3-yl)amino]-1,2,4-triazol-4-yl, 3-[(3-methylisothiazol-4-yl)amino]-1,2,4-triazol-4-yl, 3-[(3-methylisoxazol-4-yl)amino]-1,2,4-triazol-4-yl, 3-[[5-(cyclopropylmethoxy)-2-methyl-pyrazol-3-yl]amino]-1,2,4-triazol-4-yl, 3-[(5-ethoxy-2-methyl-pyrazol-3-yl)amino]-1,2,4-triazol-4-yl, 3-[(2,5-dimethylpyrazol-3-yl)amino]-1,2,4-triazol-4-yl, (5-amino-4-ethoxycarbonyl-imidazol-1-yl), 3-[[5-(difluoromethoxy)-2-methylpyrazol-3-yl]amino]-1,2,4-triazol-4-yl, 3-[[5-(difluoromethyl)-2-methylpyrazol-3-yl]amino]-1,2,4-triazol-4-yl, 3-[(5-methoxy-2-methylpyrazol-3-yl)amino]-1,2,4-triazol-4-yl, 3-[[5-(difluoromethyl)-2-(trideuteriomethyl)pyrazol-3-yl]amino, 3-[(5-Cyano-2-methyl-pyrazol-3-yl)amino]-1,2,4-triazol-4-yl, 3-[(2,2-difluoro-1,3-benzodioxol-5-yl)amino]-1,2,4-triazol-4-yl, 3-[(5-methoxy-2-methyl-pyrazol-3-yl)amino]-1,2,4-triazol-4-yl, 3-[[5-(difluoromethyl)-2-(trideuteriomethyl)pyrazol-3-yl]amino]-1,2,4-triazol-4-yl, 3-[(6-fluoro-2-methyl-3-pyridyl)amino]-1,2,4-triazol-4-yl, 3-[[5-(cyclopropoxy)-2-methyl-pyrazol-3-yl]amino]-1,2,4-triazol-4-yl, 3-[[2-methyl-5-(2,2,2-trifluoroethoxy)pyrazol-3-yl]amino]-1,2,4-triazol-4-yl, 3-[[6-(trifluoromethyl)-3-pyridyl]amino]-1,2,4-triazol-4-yl, 3-[(6-methoxy-3-pyridyl)amino]-1,2,4-triazol-4-yl, 3-[(2-methoxy-4-pyridyl)amino]-1,2,4-triazol-4-yl, 3-[(6-chloro-3-pyridyl)amino]-1,2,4-triazol-4-yl, 3-[(2-chloro-4-pyridyl)amino]-1,2,4-triazol-4-yl, 3-[(6-methoxy-2-methyl-3-pyridyl)amino]-1,2,4-triazol-4-yl, thiazolo[5,4-b]pyridin-2-ylamino, 3-[(3-methyltriazol-4-yl)amino]-1,2,4-triazol-4-yl.

11. A compound according to claim 1 or a pharmaceutically acceptable salt thereof, wherein the compound is selected amongst:
2-(Cyclopropanecarbonylamino)-*N*-(cyclopropylmethyl)-4,5,6,7-tetrahydrothieno[3,2-*c*]pyridine-3-carboxamide; hydrochloride ;
2-(Cyclopropanecarbonylamino)-*N*-(cyclopropylmethyl)-5-methylsulfonyl-6,7-dihydro-4*H*-thieno[3,2-c]pyridine-3-carboxamide;
5-Acetyl-2-(cyclopropanecarbonylamino)-N-(cyclopropylmethyl)-6,7-dihydro-4H-thieno[3,2-c]pyridine-3-carboxamide;
2-(Cyclopropanecarbonylamino)-*N*-(cyclopropylmethyl)-7-methylsulfonyl-5,6-dihydro-4*H*-thieno[2,3-b]pyridine-3-carboxamide ;
7-Acetyl-2-(cyclopropanecarbonylamino)-N-(cyclopropylmethyl)-5,6-dihydro-4H-thieno[2,3-b]pyridine-3-carboxamide ;
2-(Cyclopropanecarbonylamino)-*N*-(cyclopropylmethyl)-5-(2-methoxyacetyl)-6,7-dihydro-4*H-*thieno[3,2-*c*]pyridine-3-carboxamide ;
2-(Cyclopropanecarbonylamino)-*N*-(cyclopropylmethyl)-5-(2-phenylacetyl)-6,7-dihydro-4*H-*thieno[3,2-*c*]pyridine-3-carboxamide ;
*Tert-butyl N*-[2-[2-(cyclopropanecarbonylamino)-3-(cyclopropylmethylcarbamoyl)-6,7-dihydro-4*H-*thieno[3,2-c]pyridin-5-yl]ethyl]carbamate ;
2-(Cyclopropanecarbonylamino)-*N*-(cyclopropylmethyl)-5-(2-phenylethyl)-6,7-dihydro-4*H-*thieno[3,2-*c*]pyridine-3-carboxamide ;
Methyl 2-[2-(cyclopropanecarbonylamino)-3-(cyclopropylmethylcarbamoyl)-6,7-dihydro-4*H-*thieno[3,2-c]pyridin-5-yl]acetate ;
(4*R*)-2-(Cyclopropanecarbonylamino)-*N*-(cyclopropylmethyl)-4-methyl-5,6-dihydro-4*H-*cyclopenta[b]thiophene-3-carboxamide ;
(4*S*)-2-(Cyclopropanecarbonylamino)-*N*-(cyclopropylmethyl)-4-methyl-5,6-dihydro-4*H-*cyclopenta[b]thiophene-3-carboxamide ;
Ethyl 2-(cyclopropanecarbonylamino)-3-(cyclopropylmethylcarbamoyl)-4,5,6,7-tetrahydrobenzothiophene-5-carboxylate;
2-(Cyclopropanecarbonylamino)-3-(cyclopropylmethylcarbamoyl)-4,5,6,7-tetrahydrobenzothiophene-5-carboxylic acid ;
2-(Cyclopropanecarbonylamino)-N3-(cyclopropylmethyl)-N5-(1-oxoisoindolin-5-yl)-4,5,6,7-tetrahydrobenzothiophene-3,5-dicarboxamide;
2-(Cyclopropanecarbonylamino)-N3-(cyclopropylmethyl)-N5-[3-(5-oxopyrrolidin-3-yl)phenyl]-4,5,6,7-tetrahydrobenzothiophene-3,5-dicarboxamide;
2-(Cyclopropanecarbonylamino)-N3-(cyclopropylmethyl)-N5-[3-(3-methyl-5-oxo-4H-pyrazol-1-yl)phenyl]-4,5,6,7-tetrahydrobenzothiophene-3,5-dicarboxamide;
2-(Cyclopropanecarbonylamino)-N3-(cyclopropylmethyl)-N5-(1H-indazol-4-yl)-4,5,6,7-tetrahydrobenzothiophene-3,5-dicarboxamide;2,2,2-trifluoroacetic acid;
2-(Cyclopropanecarbonylamino)-N3-(cyclopropylmethyl)-N5-(1H-indazol-5-yl)-4,5,6,7-tetrahydrobenzothiophene-3,5-dicarboxamide;2,2,2-trifluoroacetic acid;
2-(Cyclopropanecarbonylamino)-N-(cyclopropylmethyl)-5-(1H-imidazo[4,5-b]pyrazin-2-yl)-4,5,6,7-tetrahydrobenzothiophene-3-carboxamide;
2-(Cyclopropanecarbonylamino)-N3-(cyclopropylmethyl)-N5-indan-2-yl-4,5,6,7-tetrahydrobenzothiophene-3,5-dicarboxamide;
2-(Cyclopropanecarbonylamino)-N3-(cyclopropylmethyl)-N5-[2-(4-methoxyphenyl)ethyl]-4,5,6,7-tetrahydrobenzothiophene-3,5-dicarboxamide ;
2-(Cyclopropanecarbonylamino)-N3-(cyclopropylmethyl)-N5-quinoxalin-6-yl-4,5,6,7-tetrahydrobenzothiophene-3,5-dicarboxamide;
2-(Cyclopropanecarbonylamino)-N3-(cyclopropylmethyl)-N5-thiazol-2-yl-4,5,6,7-tetrahydrobenzothiophene-3,5-dicarboxamide ;
2-(Cyclopropanecarbonylamino)-N3-(cyclopropylmethyl)-N5-(1H-tetrazol-5-yl)-4,5,6,7-tetrahydrobenzothiophene-3,5-dicarboxamide ;
2-(Cyclopropanecarbonylamino)-N3-(cyclopropylmethyl)-N5-(4-pyridyl)-4,5,6,7-tetrahydrobenzothiophene-3,5-dicarboxamide ;
2-(Cyclopropanecarbonylamino)-N3-(cyclopropylmethyl)-N5-pyrazin-2-yl-4,5,6,7-tetrahydrobenzothiophene-3,5-dicarboxamide ;
2-(Cyclopropanecarbonylamino)-N3-(cyclopropylmethyl)-N5-(3-pyridyl)-4,5,6,7-tetrahydrobenzothiophene-3,5-dicarboxamide ;
2-(Cyclopropanecarbonylamino)-N-(cyclopropylmethyl)-5-(3-hydroxyazetidine-1-carbonyl)-4,5,6,7-tetrahydrobenzothiophene-3-carboxamide ;
2-(Cyclopropanecarbonylamino)-N-(cyclopropylmethyl)-5-(1,1-dioxo-1,4-thiazinane-4-carbonyl)-4,5,6,7-tetrahydrobenzothiophene-3-carboxamide ;
2-(Cyclopropanecarbonylamino)-N3-(cyclopropylmethyl)-N5-[2-(4-hydroxyphenyl)ethyl]-4,5,6,7-tetrahydrobenzothiophene-3,5-dicarboxamide ;
2-(cyclopropanecarbonylamino)-N-(cyclopropylmethyl)-5-(hydroxyiminomethyl)-4,5,6,7-tetrahydrobenzothiophene-3-carboxamide ;
2-(Cyclopropanecarbonylamino)-N-(cyclopropylmethyl)-5-[[(4-ethyl-1,2,4-triazol-3-yl)amino]methyl]-4,5,6,7-tetrahydrobenzothiophene-3-carboxamide ;
2-(Cyclopropanecarbonylamino)-N-(cyclopropylmethyl)-5-hydroxy-5-[(4-methylanilino)methyl]-6,7-dihydro-4H-benzothiophene-3-carboxamide ;
2-(Cyclopropanecarbonylamino)-N-(cyclopropylmethyl)-2'-oxo-3'-(p-tolyl)spiro[6,7-dihydro-4H-benzothiophene-5,5'-oxazolidine]-3-carboxamide ;
5-(2-Amino-3-pyridyl)-2-(cyclopropanecarbonylamino)-N-(cyclopropylmethyl)-4,5,6,7-tetrahydrobenzothiophene-3-carboxamide ;
2-(Cyclopropanecarbonylamino)-N-(cyclopropylmethyl)-5-[5-(p-tolyl)-1H-imidazol-2-yl]-4,5,6,7-tetrahydrobenzothiophene-3-carboxamide ;
5-Cyano-2-(cyclopropanecarbonylamino)-N-(cyclopropylmethyl)-4,5,6,7-tetrahydrobenzothiophene-3-carboxamide ;
7-Cyano-2-(cyclopropanecarbonylamino)-N-(cyclopropylmethyl)-4,5,6,7-tetrahydrobenzothiophene-3-carboxamide ;
(5R)-2-(Cyclopropanecarbonylamino)-N-(cyclopropylmethyl)-5-(trifluoromethyl)-4,5,6,7-tetrahydrobenzothiophene-3-carboxamide ;
(5S)-2-(Cyclopropanecarbonylamino)-N-(cyclopropylmethyl)-5-(trifluoromethyl)-4,5,6,7-tetrahydrobenzothiophene-3-carboxamide ;
2-(Cyclopropanecarbonylamino)-N-(cyclopropylmethyl)-5-(4-methyloxazol-2-yl)-4,5,6,7-tetrahydrobenzothiophene-3-carboxamide ;
5-(Aminomethyl)-2-(cyclopropanecarbonylamino)-N-(cyclopropylmethyl)-5-hydroxy-6,7-dihydro-4H-benzothiophene-3-carboxamide ;
2-(Cyclopropanecarbonylamino)-N-(cyclopropylmethyl)-5-hydroxy-5-[(p-tolylcarbamoylamino)methyl]-6,7-dihydro-4H-benzothiophene-3-carboxamide ;
2-(Cyclopropanecarbonylamino)-N-(cyclopropylmethyl)-5-(3-methyl-1,2,4-oxadiazol-5-yl)-4,5,6,7-tetrahydrobenzothiophene-3-carboxamide ;
2-(Cyclopropanecarbonylamino)-N-(cyclopropylmethyl)-5-(5-methyl-1,3,4-oxadiazol-2-yl)-4,5,6,7-tetrahydrobenzothiophene-3-carboxamide ;
2-(Cyclopropanecarbonylamino)-N-(cyclopropylmethyl)-5-(pyrrolidine-1-carbonyl)-4,5,6,7-tetrahydrobenzothiophene-3-carboxamide ;
2-(Cyclopropanecarbonylamino)-N-(cyclopropylmethyl)-5-hydroxy-5-methyl-6,7-dihydro-4H-benzothiophene-3-carboxamide ;
2-(Cyclopropanecarbonylamino)-N-(cyclopropylmethyl)-4-methyl-4,5,6,7-tetrahydrobenzothiophene-3-carboxamide;
2-(Cyclopropanecarbonylamino)-N-(cyclopropylmethyl)-5,7-dimethyl-4,5,6,7-tetrahydrobenzothiophene-3-carboxamide;
2-(Cyclopropanecarbonylamino)-N-(cyclopropylmethyl)-5-methyl-4,5,6,7-tetrahydrobenzothiophene-3-carboxamide ;
2-(cyclopropanecarbonylamino)-N-(cyclopropylmethyl)-7-methyl-4,5,6,7-tetrahydrobenzothiophene-3-carboxamide ;
2-(Cyclopropanecarbonylamino)-N-(cyclopropylmethyl)-5,5-dimethyl-6,7-dihydro-4H-benzothiophene-3-carboxamide ;
2-(Cyclopropanecarbonylamino)-N-(cyclopropylmethyl)-5-methyl-7-oxo-5,6-dihydro-4H-benzothiophene-3-carboxamide ;
2'-(Cyclopropanecarbonylamino)-N-(cyclopropylmethyl)spiro[1,3-dioxolane-2,5'-6,7-dihydro-4H-benzothiophene]-3'-carboxamide ;
2-(Cyclopropanecarbonylamino)-N-(cyclopropylmethyl)-4,5-dimethyl-4,5,6,7-tetrahydrobenzothiophene-3-carboxamide ;
2-(Cyclopropanecarbonylamino)-N-(cyclopropylmethyl)-2',5'-dioxo-spiro[6,7-dihydro-4H-benzothiophene-5,4'-imidazolidine]-3-carboxamide ;
*tert-Butyl* N-[2-(cyclopropanecarbonylamino)-3-(cyclopropylmethylcarbamoyl)-4,5,6,7-tetrahydrobenzothiophen-7-yl]carbamate;
2-(Cyclopropanecarbonylamino)-N3-(cyclopropylmethyl)-N7-ethyl-4,5,6,7-tetrahydrobenzothiophene-3,7-dicarboxamide ;
2-(Cyclopropanecarbonylamino)-N-(cyclopropylmethyl)-5-(1H-pyrazol-3-yl)-4,5,6,7-tetrahydrobenzothiophene-3-carboxamide ;
2-(Cyclopropanecarbonylamino)-N-(cyclopropylmethyl)-7-(1H-pyrazol-3-yl)-4,5,6,7-tetrahydrobenzothiophene-3-carboxamide ;
(5S)-5-(5-Amino-4-cyano-imidazol-1-yl)-2-(cyclopropanecarbonylamino)-N-(cyclopropylmethyl)-4,5,6,7-tetrahydrobenzothiophene-3-carboxamide ;
(5S)-2-(Cyclopropanecarbonylamino)-N-(cyclopropylmethyl)-5-[3-(2,2-dimethylpropylamino)-1,2,4-triazol-4-yl]-4,5,6,7-tetrahydrobenzothiophene-3-carboxamide ;
(5S)-2-(Cyclopropanecarbonylamino)-N-(cyclopropylmethyl)-5-[3-[(2,5-dimethylpyrazol-3-yl)amino]-1,2,4-triazol-4-yl]-4,5,6,7-tetrahydrobenzothiophene-3-carboxamide ;
(5S)-2-(Cyclopropanecarbonylamino)-N-(cyclopropylmethyl)-5-[[4-(2,5-dimethylpyrazol-3-yl)-1,2,4-triazol-3-yl]amino]-4,5,6,7-tetrahydrobenzothiophene-3-carboxamide ;
5-Amino-2-(cyclopropanecarbonylamino)-N-(cyclopropylmethyl)-4,5,6,7-tetrahydrobenzothiophene-3-carboxamide ;
5-[[N'-Cyano-N-phenyl-carbamimidoyl]amino]-2-(cyclopropanecarbonylamino)-N-(cyclopropylmethyl)-4,5,6,7-tetrahydrobenzothiophene-3-carboxamide ;
(5S)-2-(Cyclopropanecarbonylamino)-N-(cyclopropylmethyl)-5-[3-(isobutylamino)-1,2,4-triazol-4-yl]-4,5,6,7-tetrahydrobenzothiophene-3-carboxamide ;
(5S)-2-(Cyclopropanecarbonylamino)-N-(cyclopropylmethyl)-5-[3-[(1-methylcyclopropyl)methylamino]-1,2,4-triazol-4-yl]-4,5,6,7-tetrahydrobenzothiophene-3-carboxamide ;
(5S)-5-(5-Acetamidoimidazol-1-yl)-2-(cyclopropanecarbonylamino)-N-(cyclopropylmethyl)-4,5,6,7-tetrahydrobenzothiophene-3-carboxamide ;
(5S)-2-(cyclopropanecarbonylamino)-N-(cyclopropylmethyl)-5-[3-[(5-methyl-3-pyridyl)amino]-1,2,4-triazol-4-yl]-4,5,6,7-tetrahydrobenzothiophene-3-carboxamide ;
(5S)-2-(Cyclopropanecarbonylamino)-N-(cyclopropylmethyl)-5-[3-(4-pyridylamino)-1,2,4-triazol-4-yl]-4,5,6,7-tetrahydrobenzothiophene-3-carboxamide ;
5-[[N'-Cyano-N-(3,4-dimethoxyphenyl)carbamimidoyl]amino]-2-(cyclopropanecarbonylamino)-N-(cyclopropylmethyl)-4,5,6,7-tetrahydrobenzothiophene-3-carboxamide ;
Ethyl 5-amino-1-[(5S)-2-(cyclopropanecarbonylamino)-3-(cyclopropylmethylcarbamoyl)-4,5,6,7-tetrahydrobenzothiophen-5-yl]imidazole-4-carboxylate ;
(5S)-2-(Cyclopropanecarbonylamino)-N-(cyclopropylmethyl)-5-[3-(2-morpholinoethylamino)-1,2,4-triazol-4-yl]-4,5,6,7-tetrahydrobenzothiophene-3-carboxamide ;
2-(Cyclopropanecarbonylamino)-N-(cyclopropylmethyl)-5-[(5-oxo-4H-1,2,4-oxadiazol-3-yl)amino]-4,5,6,7-tetrahydrobenzothiophene-3-carboxamide ;
2-(Cyclopropanecarbonylamino)-N-(cyclopropylmethyl)-5-[[2-(ethylamino)-3,4-dioxo-cyclobuten-1-yl]amino]-4,5,6,7-tetrahydrobenzothiophene-3-carboxamide ;
5-[[N'-cyano-N-(2,2,2-trifluoroethyl)carbamimidoyl]amino]-2-(cyclopropanecarbonylamino)-N-(cyclopropylmethyl)-4,5,6,7-tetrahydrobenzothiophene-3-carboxamide ;
(5S)-2-(cyclopropanecarbonylamino)-N-(cyclopropylmethyl)-5-[3-(ethylamino)-1,2,4-triazol-4-yl]-4,5,6,7-tetrahydrobenzothiophene-3-carboxamide ;
2-(cyclopropanecarbonylamino)-N-(cyclopropylmethyl)-5-[(N-ethyl-N'-nitro-carbamimidoyl)amino]-4,5,6,7-tetrahydrobenzothiophene-3-carboxamide;
(5S)-2-(Cyclopropanecarbonylamino)-N-(cyclopropylmethyl)-5-[3-(3-pyridylamino)-1,2,4-triazol-4-yl]-4,5,6,7-tetrahydrobenzothiophene-3-carboxamide ;
(5S)-2-(Cyclopropanecarbonylamino)-N-(cyclopropylmethyl)-5-[3-[(1-oxidopyridin-1-ium-3-yl)amino]-1,2,4-triazol-4-yl]-4,5,6,7-tetrahydrobenzothiophene-3-carboxamide ;
2-(cyclopropanecarbonylamino)-N-(cyclopropylmethyl)-5-[[N'-nitro-N-(p-tolyl)carbamimidoyl]amino]-4,5,6,7-tetrahydrobenzothiophene-3-carboxamide ; (5S)-2-(Cyclopropanecarbonylamino)-N-(cyclopropylmethyl)-5-[2-(3-pyridylamino)imidazol-1-yl]-4,5,6,7-tetrahydrobenzothiophene-3-carboxamide ;
(5S)-2-(Cyclopropanecarbonylamino)-N-(cyclopropylmethyl)-5-[3-(cyclopropylmethylamino)-1,2,4-triazol-4-yl]-4,5,6,7-tetrahydrobenzothiophene-3-carboxamide ;
(5S)-2-(Cyclopropanecarbonylamino)-N-(cyclopropylmethyl)-5-[(2,5-dimethylpyrazol-3-yl)carbamothioylamino]-4,5,6,7-tetrahydrobenzothiophene-3-carboxamide ;
(5S)-2-(Cyclopropanecarbonylamino)-N-(cyclopropylmethyl)-5-[3-(pyrimidin-5-ylamino)-1,2,4-triazol-4-yl]-4,5,6,7-tetrahydrobenzothiophene-3-carboxamide ;
(5S)-2-(Cyclopropanecarbonylamino)-N-(cyclopropylmethyl)-5-[2-(cyclopropylmethylamino)imidazol-1-yl]-4,5,6,7-tetrahydrobenzothiophene-3-carboxamide ;
2-(Cyclopropanecarbonylamino)-N-(cyclopropylmethyl)-5-[[4-(ethylamino)-1,1-dioxo-1,2,5-thiadiazol-3-yl]amino]-4,5,6,7-tetrahydrobenzothiophene-3-carboxamide ;
2-(cyclopropanecarbonylamino)-N-(cyclopropylmethyl)-5-[[1-(4-methylanilino)-2-nitro-vinyl]amino]-4,5,6,7-tetrahydrobenzothiophene-3-carboxamide ;
(5S)-5-[(N'-cyano-N-ethyl-carbamimidoyl)-methyl-amino]-2-(cyclopropanecarbonylamino)-N-(cyclopropylmethyl)-4,5,6,7-tetrahydrobenzothiophene-3-carboxamide ;
2-(Cyclopropanecarbonylamino)-N-(cyclopropylmethyl)-5-[[2-(4-methylanilino)-3,4-dioxo-cyclobuten-1-yl]amino]-4,5,6,7-tetrahydrobenzothiophene-3-carboxamide ;
(5S)-2-(Cyclopropanecarbonylamino)-N-(cyclopropylmethyl)-5-[3-(2-pyridylamino)-1,2,4-triazol-4-yl]-4,5,6,7-tetrahydrobenzothiophene-3-carboxamide ;
(5S)-5-(2-Acetamidoimidazol-1-yl)-2-(cyclopropanecarbonylamino)-N-(cyclopropylmethyl)-4,5,6,7-tetrahydrobenzothiophene-3-carboxamide ;
*tert-Butyl* N-[3-[2-(cyclopropanecarbonylamino)-3-(cyclopropylmethylcarbamoyl)-4,5,6,7-tetrahydrobenzothiophen-5-yl]triazol-4-yl]carbamate ;
2-(Cyclopropanecarbonylamino)-N-(cyclopropylmethyl)-5-[5-(3-pyridylamino)triazol-1-yl]-4,5,6,7-tetrahydrobenzothiophene-3-carboxamide ;
2-(Cyclopropanecarbonylamino)-N-(cyclopropylmethyl)-5-(ethylcarbamothioylamino)-4,5,6,7-tetrahydrobenzothiophene-3-carboxamide ;
(5S)-2-(cyclopropanecarbonylamino)-N-(cyclopropylmethyl)-5-[3-[[(3S)-tetrahydrofuran-3-yl]amino]-1,2,4-triazol-4-yl]-4,5,6,7-tetrahydrobenzothiophene-3-carboxamide;
5-[[N'-Cyano-N-ethyl-carbamimidoyl]amino]-2-(cyclopropanecarbonylamino)-N-(cyclopropylmethyl)-4,5,6,7-tetrahydrobenzothiophene-3-carboxamide;
(5S)-2-(Cyclopropanecarbonylamino)-N-(cyclopropylmethyl)-5-[[(E)-N-ethyl-N'-nitro-carbamimidoyl]amino]-4,5,6,7-tetrahydrobenzothiophene-3-carboxamide ;
(5R)-2-(Cyclopropanecarbonylamino)-N-(cyclopropylmethyl)-5-[[(E)-N-ethyl-N'-nitro-carbamimidoyl]amino]-4,5,6,7-tetrahydrobenzothiophene-3-carboxamide ;
(5S)-2-(Cyclopropanecarbonylamino)-N-(cyclopropylmethyl)-5-[2-(ethylamino)imidazol-1-yl]-4,5,6,7-tetrahydrobenzothiophene-3-carboxamide ;
(5S)-2-(Cyclopropanecarbonylamino)-N-(cyclopropylmethyl)-5-[3-[(3,5-dimethylisoxazol-4-yl)amino]-1,2,4-triazol-4-yl]-4,5,6,7-tetrahydrobenzothiophene-3-carboxamide ;
ethyl N-[[[2-(cyclopropanecarbonylamino)-3-(cyclopropylmethylcarbamoyl)-4,5,6,7-tetrahydrobenzothiophen-5-yl]amino]-(4-methylanilino)methylene]carbamate ;
(5S)-2-(Cyclopropanecarbonylamino)-N-(cyclopropylmethyl)-5-[2-[(2-methyl-3-pyridyl)amino]imidazol-1-yl]-4,5,6,7-tetrahydrobenzothiophene-3-carboxamide ;
2-(cyclopropanecarbonylamino)-N-(cyclopropylmethyl)-5-[[1-(ethylamino)-2-nitro-vinyl]amino]-4,5,6,7-tetrahydrobenzothiophene-3-carboxamide ;
2-(Cyclopropanecarbonylamino)-N-(cyclopropylmethyl)-5-[(4-methyl-6-oxo-1H-pyrimidin-2-yl)amino]-4,5,6,7-tetrahydrobenzothiophene-3-carboxamide ;
5-amino-1-[(5S)-2-cyclopropaneamido-3-[(cyclopropylmethyl)carbamoyl]-4,5,6,7-tetrahydro-1-benzothiophen-5-yl]-N-methyl-1H-imidazole-4-carboxamide ;
5-amino-1-[(5S)-2-cyclopropaneamido-3-[(cyclopropylmethyl)carbamoyl]-4,5,6,7-tetrahydro-1-benzothiophen-5-yl]-1H-imidazole-4-carboxamide;
5-(2-Anilinoimidazol-1-yl)-2-(cyclopropanecarbonylamino)-N-(cyclopropylmethyl)-4,5,6,7-tetrahydrobenzothiophene-3-carboxamide ;
(5S)-5-[[N'-cyano-N-ethyl-carbamimidoyl]amino]-2-(cyclopropanecarbonylamino)-N-(cyclopropylmethyl)-4,5,6,7-tetrahydrobenzothiophene-3-carboxamide ;
2-(Cyclopropanecarbonylamino)-N-(cyclopropylmethyl)-5-(ethylcarbamoylamino)-4,5,6,7-tetrahydrobenzothiophene-3-carboxamide;
2-(Cyclopropanecarbonylamino)-N-(cyclopropylmethyl)-5-[(4-oxo-1H-pyridin-2-yl)amino]-4,5,6,7-tetrahydrobenzothiophene-3-carboxamide;
(5S)-2-(cyclopropanecarbonylamino)-N-(cyclopropylmethyl)-5-[[6-(5-methyl-1,3,4-oxadiazol-2-yl)-3-pyridyl]amino]-4,5,6,7-tetrahydrobenzothiophene-3-carboxamide;
2-(Cyclopropanecarbonylamino)-N-(cyclopropylmethyl)-5-[5-(ethylamino)tetrazol-1-yl]-4,5,6,7-tetrahydrobenzothiophene-3-carboxamide;
2-(Cyclopropanecarbonylamino)-N-(cyclopropylmethyl)-5-(1H-1,2,4-triazol-3-ylamino)-4,5,6,7-tetrahydrobenzothiophene-3-carboxamide;
(5R)-2-(Cyclopropanecarbonylamino)-N-(cyclopropylmethyl)-5-[3-[(2,5-dimethylpyrazol-3-yl)amino]-1,2,4-triazol-4-yl]-4,5,6,7-tetrahydrobenzothiophene-3-carboxamide;
(5S)-2-(Cyclopropanecarbonylamino)-N-(cyclopropylmethyl)-5-[[4-(1-methylpyrazol-4-yl)-1,2,4-triazol-3-yl]amino]-4,5,6,7-tetrahydrobenzothiophene-3-carboxamide;
ethyl 1-[(5S)-2-cyclopropaneamido-3-[(cyclopropylmethyl)carbamoyl]-4,5,6,7-tetrahydro-1-benzothiophen-5-yl]-1H-imidazole-4-carboxylate;
2-(Cyclopropanecarbonylamino)-N-(cyclopropylmethyl)-5-[[4-(4-fluorophenyl)-1,2,4-triazol-3-yl]amino]-4,5,6,7-tetrahydrobenzothiophene-3-carboxamide ;
(5S)-2-(Cyclopropanecarbonylamino)-N-(cyclopropylmethyl)-5-(1H-imidazol-2-ylamino)-4,5,6,7-tetrahydrobenzothiophene-3-carboxamide ;
2-(Cyclopropanecarbonylamino)-N-(cyclopropylmethyl)-5-[[N'-methoxy-N-(p-tolyl)carbamimidoyl]amino]-4,5,6,7-tetrahydrobenzothiophene-3-carboxamide;
2-(Cyclopropanecarbonylamino)-N-(cyclopropylmethyl)-5-[[4-(4-methylanilino)-1,1-dioxo-1,2,5-thiadiazol-3-yl]amino]-4,5,6,7-tetrahydrobenzothiophene-3-carboxamide;
(5S)-2-(Cyclopropanecarbonylamino)-N-(cyclopropylmethyl)-5-[[1-(3-pyridyl)imidazol-2-yl]amino]-4,5,6,7-tetrahydrobenzothiophene-3-carboxamide ;
5-(5-Aminotriazol-1-yl)-2-(cyclopropanecarbonylamino)-N-(cyclopropylmethyl)-4,5,6,7-tetrahydrobenzothiophene-3-carboxamide ;
2-(Cyclopropanecarbonylamino)-N-(cyclopropylmethyl)-5-[(2-methoxyphenyl)carbamoylamino]-4,5,6,7-tetrahydrobenzothiophene-3-carboxamide ;
N-[2-(Cyclopropanecarbonylamino)-3-(cyclopropylmethylcarbamoyl)-4,5,6,7-tetrahydrobenzothiophen-5-yl]pyridine-4-carboxamide ;
5-(Benzylamino)-2-(cyclopropanecarbonylamino)-N-(cyclopropylmethyl)-4,5,6,7-tetrahydrobenzothiophene-3-carboxamide ;
5-(1H-Benzimidazol-2-ylamino)-2-(cyclopropanecarbonylamino)-N-(cyclopropylmethyl)-4,5,6,7-tetrahydrobenzothiophene-3-carboxamide ;
2-(Cyclopropanecarbonylamino)-N-(cyclopropylmethyl)-5-[[N'-hydroxycarbamimidoyl]amino]-4,5,6,7-tetrahydrobenzothiophene-3-carboxamide ;
2-(Cyclopropanecarbonylamino)-N-(cyclopropylmethyl)-5-[[N'-(1-methylpyrazol-3-yl)-N-(p-tolyl)carbamimidoyl]amino]-4,5,6,7-tetrahydrobenzothiophene-3-carboxamide;formic acid ;
(5S)-2-(Cyclopropanecarbonylamino)-N-(cyclopropylmethyl)-5-[3-[(3,5-dimethylpyrazin-2-yl)amino]-1,2,4-triazol-4-yl]-4,5,6,7-tetrahydrobenzothiophene-3-carboxamide ;
2-(Cyclopropanecarbonylamino)-N-(cyclopropylmethyl)-5-[3-(ethylamino)-5-(methoxymethyl)-1,2,4-triazol-4-yl]-4,5,6,7-tetrahydrobenzothiophene-3-carboxamide ;
2-(Cyclopropanecarbonylamino)-N-(cyclopropylmethyl)-5-[[5-(3-pyridyl)-1H-1,2,4-triazol-3-yl]amino]-4,5,6,7-tetrahydrobenzothiophene-3-carboxamide ;
p-Tolyl N-[2-(cyclopropanecarbonylamino)-3-(cyclopropylmethylcarbamoyl)-4,5,6,7-tetrahydrobenzothiophen-5-yl]carbamate ;
2-(Cyclopropanecarbonylamino)-N-(cyclopropylmethyl)-5-(quinazolin-2-ylamino)-4,5,6,7-tetrahydrobenzothiophene-3-carboxamide ;
5-(1,3-Benzoxazol-2-ylamino)-2-(cyclopropanecarbonylamino)-N-(cyclopropylmethyl)-4,5,6,7-tetrahydrobenzothiophene-3-carboxamide ;
2-(Cyclopropanecarbonylamino)-N-(cyclopropylmethyl)-5-[3-(ethylamino)-5-(hydroxymethyl)-1,2,4-triazol-4-yl]-4,5,6,7-tetrahydrobenzothiophene-3-carboxamide ;
(5S)-5-(2-Aminoimidazol-1-yl)-2-(cyclopropanecarbonylamino)-N-(cyclopropylmethyl)-4,5,6,7-tetrahydrobenzothiophene-3-carboxamide ;
5-(3-Amino-5-oxo-1,2,4-oxadiazol-4-yl)-2-(cyclopropanecarbonylamino)-N-(cyclopropylmethyl)-4,5,6,7-tetrahydrobenzothiophene-3-carboxamide ;
2-(Cyclopropanecarbonylamino)-N-(cyclopropylmethyl)-5-[3-(ethylamino)-5-methyl-1,2,4-triazol-4-yl]-4,5,6,7-tetrahydrobenzothiophene-3-carboxamide ;
(5R)-2-(Cyclopropanecarbonylamino)-N-(cyclopropylmethyl)-5-[(2-ethyl-3,4-dioxo-cyclobuten-1-yl)amino]-4,5,6,7-tetrahydrobenzothiophene-3-carboxamide ;
(5S)-2-(Cyclopropanecarbonylamino)-N-(cyclopropylmethyl)-5-[(2-ethyl-3,4-dioxo-cyclobuten-1-yl)amino]-4,5,6,7-tetrahydrobenzothiophene-3-carboxamide ;
2-(Cyclopropanecarbonylamino)-N-(cyclopropylmethyl)-5-(pyrimidin-2-ylamino)-4,5,6,7-tetrahydrobenzothiophene-3-carboxamide ;
2-(Cyclopropanecarbonylamino)-N-(cyclopropylmethyl)-5-(1,3,4-oxadiazol-2-ylamino)-4,5,6,7-tetrahydrobenzothiophene-3-carboxamide ;
2-(Cyclopropanecarbonylamino)-N-(cyclopropylmethyl)-5-[[5-oxo-1-(p-tolyl)-4H-imidazol-2-yl]amino]-4,5,6,7-tetrahydrobenzothiophene-3-carboxamide ;
2-(Cyclopropanecarbonylamino)-N-(cyclopropylmethyl)-5-[[2-[ethyl(methyl)amino]-3,4-dioxo-cyclobuten-1-yl]amino]-4,5,6,7-tetrahydrobenzothiophene-3-carboxamide ;
2-(Cyclopropanecarbonylamino)-N-(cyclopropylmethyl)-5-[(methylsulfonimidoyl)amino]-4,5,6,7-tetrahydrobenzothiophene-3-carboxamide ;
5-[[N-Cyano-C-methyl-carbonimidoyl]amino]-2-(cyclopropanecarbonylamino)-N-(cyclopropylmethyl)-4,5,6,7-tetrahydrobenzothiophene-3-carboxamide ;
2-(Cyclopropanecarbonylamino)-N-(cyclopropylmethyl)-5-(tetrazol-1-yl)-4,5,6,7-tetrahydrobenzothiophene-3-carboxamide ;
2-(Cyclopropanecarbonylamino)-N-(cyclopropylmethyl)-5-(methanesulfinamido)-4,5,6,7-tetrahydrobenzothiophene-3-carboxamide ;
2-(Cyclopropanecarbonylamino)-N-(cyclopropylmethyl)-5-morpholino-4,5,6,7-tetrahydrobenzothiophene-3-carboxamide ;
2-(Cyclopropanecarbonylamino)-N-(cyclopropylmethyl)-5-[3-(ethylamino)-5-(trifluoromethyl)-1,2,4-triazol-4-yl]-4,5,6,7-tetrahydrobenzothiophene-3-carboxamide;
2-(Cyclopropanecarbonylamino)-N-(cyclopropylmethyl)-5-[(4-oxo-1H-pyrimidin-2-yl)amino]-4,5,6,7-tetrahydrobenzothiophene-3-carboxamide ;
2-(Cyclopropanecarbonylamino)-N-(cyclopropylmethyl)-5-(isopropylsulfamoylamino)-4,5,6,7-tetrahydrobenzothiophene-3-carboxamide;
5-[[N'-Cyano-N-morpholino-carbamimidoyl]amino]-2-(cyclopropanecarbonylamino)-N-(cyclopropylmethyl)-4,5,6,7-tetrahydrobenzothiophene-3-carboxamide ;
5-Acetamido-2-(cyclopropanecarbonylamino)-N-(cyclopropylmethyl)-4,5,6,7-tetrahydrobenzothiophene-3-carboxamide;
2-(Cyclopropanecarbonylamino)-N-(cyclopropylmethyl)-5-(methanesulfonamido)-4,5,6,7-tetrahydrobenzothiophene-3-carboxamide ;
5-[[N'-Cyano-N-methoxy-carbamimidoyl]amino]-2-(cyclopropanecarbonylamino)-N-(cyclopropylmethyl)-4,5,6,7-tetrahydrobenzothiophene-3-carboxamide;
2-(Cyclopropanecarbonylamino)-N-(cyclopropylmethyl)-5-[[4-oxo-6-(trifluoromethyl)-1H-pyrimidin-2-yl]amino]-4,5,6,7-tetrahydrobenzothiophene-3-carboxamide;
(5S)-2-(cyclopropanecarbonylamino)-N-(cyclopropylmethyl)-5-[3-(2-methylpropanoylamino)-1,2,4-triazol-4-yl]-4,5,6,7-tetrahydrobenzothiophene-3-carboxamide ;
(5S)-2-(cyclopropanecarbonylamino)-N-(cyelopropylmethyl)-5-[3-[(2,5-dimethylpyrazol-3-yl)-methyl-amino]-1,2,4-triazol-4-yl]-4,5,6,7-tetrahydrobenzothiophene-3-carboxamide ;
(5S)-5-[5-amino-4-(5-methyl-1,3,4-oxadiazol-2-yl)imidazol-1-yl]-2-(cyclopropanecarbonylamino)-N-(cyclopropylmethyl)-4,5,6,7-tetrahydrobenzothiophene-3-carboxamide;
(5S)-2-(cyclopropanecarbonylamino)-N-(cyclopropylmethyl)-5-[3-[(3-methyl-1H-pyrazol-5-yl)amino]-1,2,4-triazol-4-yl]-4,5,6,7-tetrahydrobenzothiophene-3-carboxamide ;
(5S)-2-(cyclopropanecarbonylamino)-N-(cyclopropylmethyl)-5-[3-[(1,3-dimethylpyrazol-4-yl)amino]-1,2,4-triazol-4-yl]-4,5,6,7-tetrahydrobenzothiophene-3-carboxamide ;
(5S)-2-(cyclopropanecarbonylamino)-N-(cyclopropylmethyl)-5-[3-[(5-cyclopropyl-2-methyl-pyrazol-3-yl)amino]-1,2,4-triazol-4-yl]-4,5,6,7-tetrahydrobenzothiophene-3-carboxamide ;
(5S)-2-(cyclopropanecarbonylamino)-N-(cyclopropylmethyl)-5-[3-[(5-isopropyl-2-methyl-pyrazol-3-yl)amino]-1,2,4-triazol-4-yl]-4,5,6,7-tetrahydrobenzothiophene-3-carboxamide ;
(5S)-2-(cyclopropanecarbonylamino)-N-(cyclopropylmethyl)-5-[3-[[2-methyl-5-(trifluoromethyl)pyrazol-3-yl]amino]-1,2,4-triazol-4-yl]-4,5,6,7-tetrahydrobenzothiophene-3-carboxamide ;
(5S)-2-(cyclopropanecarbonylamino)-N-(cyclopropylmethyl)-5-[3-[(2-isopropyl-5-methyl-pyrazol-3-yl)amino]-1,2,4-triazol-4-yl]-4,5,6,7-tetrahydrobenzothiophene-3-carboxamide ;
(5S)-2-(cyclopropanecarbonylamino)-N-(cyclopropylmethyl)-5-[3-[(5-methyl-2-tetrahydrofuran-3-yl-pyrazol-3-yl)amino]-1,2,4-triazol-4-yl]-4,5,6,7-tetrahydrobenzothiophene-3-carboxamide ;
(5S)-2-(cyclopropanecarbonylamino)-N-(cyclopropylmethyl)-5-[3-[(2-methylpyrazol-3-yl)amino]-1,2,4-triazol-4-yl]-4,5,6,7-tetrahydrobenzothiophene-3-carboxamide ;
(5S)-2-(cyclopropanecarbonylamino)-N-(cyclopropylmethyl)-5-[3-[(2-ethyl-5-methyl-pyrazol-3-yl)amino]-1,2,4-triazol-4-yl]-4,5,6,7-tetrahydrobenzothiophene-3-carboxamide ;
(5S)-2-(cyclopropanecarbonylamino)-N-(cyclopropylmethyl)-5-[3-[(2-cyclopropyl-5-methyl-pyrazol-3-yl)amino]-1,2,4-triazol-4-yl]-4,5,6,7-tetrahydrobenzothiophene-3-carboxamide ;
(5S)-2-(cyclopropanecarbonylamino)-N-(cyclopropylmethyl)-5-[3-[(3-methoxy-1-methyl-pyrazol-4-yl)amino]-1,2,4-triazol-4-yl]-4,5,6,7-tetrahydrobenzothiophene-3-carboxamide ;
(5S)-2-(cyclopropanecarbonylamino)-N-(cyclopropylmethyl)-5-[3-[(5-ethyl-2-methyl-pyrazol-3-yl)amino]-1,2,4-triazol-4-yl]-4,5,6,7-tetrahydrobenzothiophene-3-carboxamide ;
(5S)-2-(Cyclopropanecarbonylamino)-N-(cyclopropylmethyl)-5-[3-[[5-(difluoromethyl)-2-methyl-pyrazol-3-yl]amino]-1,2,4-triazol-4-yl]-4,5,6,7-tetrahydrobenzothiophene-3-carboxamide ;
(5S)-2-(Cyclopropanecarbonylamino)-N-(cyclopropylmethyl)-5-[3-[(1,4-dimethylpyrazol-3-yl)amino]-1,2,4-triazol-4-yl]-4,5,6,7-tetrahydrobenzothiophene-3-carboxamide ;
Methyl 5-[[4-[(5S)-2-(cyclopropanecarbonylamino)-3-(cyclopropylmethylcarbamoyl)-4,5,6,7-tetrahydrobenzothiophen-5-yl]-1,2,4-triazol-3-yl]amino]-1-methyl-pyrazole-3-carboxylate ;
(5S)-2-(Cyclopropanecarbonylamino)-N-(cyclopropylmethyl)-5-[2-[[2-methyl-5-(trifluoromethyl)pyrazol-3-yl]amino]imidazol-1-yl]-4,5,6,7-tetrahydrobenzothiophene-3-carboxamide ;
5-[[4-[(5S)-2-(Cyclopropanecarbonylamino)-3-(cyclopropylmethylcarbamoyl)-4,5,6,7-tetrahydrobenzothiophen-5-yl]-1,2,4-triazol-3-yl]amino]-1-methyl-pyrazole-3-carboxylic acid ;
their enantiomers and pharmaceutically acceptable salts thereof.

12. A compound according to claim 1 or a pharmaceutically acceptable salt thereof, wherein the compound is selected amongst:
(5S)-2-(cyclopropanecarbonylamino)-N-(cyclopropylmethyl)-5-[3-[[(3S*)-1,1-dioxothiolan-3-yl]amino]-1,2,4-triazol-4-yl]-4,5,6,7-tetrahydrobenzothiophene-3-carboxamide [*or R];
(5S)-2-cyclopropaneamido-N-(cyclopropylmethyl)-5-[3-({3-[imino(methyl)oxo-lambda6-sulfanyl]-2-methylphenyl}amino)-4H-1,2,4-triazol-4-yl]-4,5,6,7-tetrahydro-1-benzothiophene-3-carboxamide;
(5S)-2-(cyclopropanecarbonylamino)-N-(cyclopropylmethyl)-5-[3-[2-methyl-3-[(S)-methylsulfonimidoyl]anilino]-1,2,4-triazol-4-yl]-4,5,6,7-tetrahydrobenzothiophene-3-carboxamide
(5S)-2-(cyclopropanecarbonylamino)-N-(cyclopropylmethyl)-5-[3-[2-methyl-3-[(R)-methylsulfonimidoyl]anilino]-1,2,4-triazol-4-yl]-4,5,6,7-tetrahydrobenzothiophene-3-carboxamide;
(5S)-5-{3-[(1-acetyl-3-methylpyrrolidin-3-yl)amino]-4H-1,2,4-triazol-4-yl}-2-cyclopropaneamido-N-(cyclopropylmethyl)-4,5,6,7-tetrahydro-1-benzothiophene-3-carboxamide;
(5S)-5-[3-[[(3S*)-1-acetyl-3-methyl-pyrrolidin-3-yl]amino]-1,2,4-triazol-4-yl]-2-(cyclopropanecarbonylamino)-N-(cyclopropylmethyl)-4,5,6,7-tetrahydrobenzothiophene-3-carboxamide [* or R];
(5S)-2-cyclopropaneamido-N-(cyclopropylmethyl)-5-(3-{[(3S)-1-(6-methylpyridazin-3-yl)pyrrolidin-3-yl]amino}-4H-1,2,4-triazol-4-yl)-4,5,6,7-tetrahydro-1-benzothiophene-3-carboxamide;
(5S)-2-(cyclopropanecarbonylamino)-N-(cyclopropylmethyl)-5-[3-[[(3S)-1-pyrazin-2-ylpyrrolidin-3-yl]amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[(4-methyl-1-methylsulfonyl-pyrrolidin-3-yl)amino]-1,2,4-triazol-4-yl]-4,5,6,7-tetrahydrobenzothiophene-3-carboxamide;
(5S)-2-cyclopropaneamido-N-(cyclopropylmethyl)-5-{3-[(1-oxo-2,3-dihydro-1H-isoindol-4-yl)amino]-4H-1,2,4-triazol-4-yl}-4,5,6,7-tetrahydro-1-benzothiophene-3-carboxamide;
(5S)-2-(cyclopropanecarbonylamino)-N-(cyclopropylmethyl)-5-[3-[[(3S)-1-(6-fluoropyridazin-3-yl)pyrrolidin-3-yl]amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[[(3S)-1-pyrimidin-2-ylpyrrolidin-3-yl]amino]-1,2,4-triazol-4-yl]-4,5,6,7-tetrahydro-1-benzothiophene-3-carboxamide;
2-methoxyethyl 5-amino-1-[(5S)-2-(cyclopropanecarbonylamino)-3-(cyclopropylmethylcarbamoyl)-4,5,6,7-tetrahydro-1-benzothiophen-5-yl]imidazole-4-carboxylate;
2-morpholin-4-ylethyl 5-amino-1-[(55)-2-(cyclopropanecarbonylamino)-3-(cyclopropylmethylcarbamoyl)-4,5,6,7-tetrahydro-1-benzothiophen-5-yl]imidazole-4-carboxylate;
(5S)-2-(cyclopropanecarbonylamino)-N-(cyclopropylmethyl)-5-[3-(3-methylsulfanylanilino)-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-(3-methylsulfonylanilino)-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-(3-methylsulfinylanilino)-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[3-(methylsulfonimidoyl)anilino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[3-[(S)-methylsulfonimidoyl]anilino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[3-[(R)-methylsulfonimidoyl]anilino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-(8-methoxy-4-oxo-5,10-dihydroimidazo[4,5-c][1,5]benzodiazepin-1-yl)-4,5,6,7-tetrahydrobenzothiophene-3-carboxamide;
(5S)-2-(cyclopropanecarbonylamino)-N-(cyclopropylmethyl)-5-(8-methoxy-5-methyl-4-oxo-10H-imidazo[4,5-c][1,5]benzodiazepin-1-yl)-4,5,6,7-tetrahydrobenzothiophene-3-carboxamide;
(5S)-5-[5-amino-4-[(3R)-3-hydroxypyrrolidine-1-carbonyl]imidazol-1-yl]-2-(cyclopropanecarbonylamino)-N-(cyclopropylmethyl)-4,5,6,7-tetrahydrobenzothiophene-3-carboxamide;
(5S)-5-[5-amino-4-[(2S)-2-methylpyrrolidine-1-carbonyl]imidazol-1-yl]-2-(cyclopropanecarbonylamino)-N-(cyclopropylmethyl)-4,5,6,7-tetrahydrobenzothiophene-3-carboxamide;
Ethyl 1-[(5S)-2-(cyclopropanecarbonylamino)-3-(cyclopropylmethylcarbamoyl)-4,5,6,7-tetrahydrobenz othiophen-5-yl]-5-[(2,5-dimethyl-4-nitro-pyrazol-3-yl)amino]imidazole-4-carboxylate;
(5S)-2-(cyclopropanecarbonylamino)-N-(cyclopropylmethyl)-5-[3-(2-methyl-3-methylsulfinyl-anilino)-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-(2-methyl-3-methylsulfonyl-anilino)-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[(2-methyl-5-oxazol-2-yl-pyrazol-3-yl)amino]-1,2,4-triazol-4-yl]-4,5,6,7-tetrahydrobenzothiophene-3-carboxamide;
(5S)-5-[3-[[(3S)-1-acetylpyrrolidin-3-yl]amino]-1,2,4-triazol-4-yl]-2-(cyclopropanecarbonylamino)-N-(cyclopropylmethyl)-4,5,6,7-tetrahydrobenzothiophene-3-carboxamide;
(5S)-2-(cyclopropanecarbonylamino)-N-(cyclopropylmethyl)-5-[3-[[(3S)-1-methylsulfonylpyrrolidin-3-yl]amino]-1,2,4-triazol-4-yl]-4,5,6,7-tetrahydrobenzothiophene-3-carboxamide ;
(5S)-2-(cyclopropanecarbonylamino)-N-(cyclopropylmethyl)-5-[3-[[(3R)-1-methylsulfonylpyrrolidin-3-yl]amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[(2-methylcyclohexyl)amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[(5-oxopyrrolidin-3-yl)amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[[(3R*)-5-oxopyrrolidin-3-yl]amino]-1,2,4-triazol-4-yl]-4,5,6,7-tetrahydrobenzothiophene-3-carboxamide (*or S);
5S)-2-(cyclopropanecarbonylamino)-N-(cyclopropylmethyl)-5-[3-[(2-oxo-4-piperidyl)amino]-1,2,4-triazol-4-yl]-4,5,6,7-tetrahydrobenzothiophene-3-carboxamide;
(5S)-5-[3-[(1-acetyl-2-methyl-pyrrolidin-3-yl)amino]-1,2,4-triazol-4-yl]-2-(cyclopropanecarbonylamino)-N-(cyclopropylmethyl)-4,5,6,7-tetrahydrobenzothiophene-3-carboxamide;
(5S)-2-(cyclopropanecarbonylamino)-N-(cyclopropylmethyl)-5-[3-[(2-methyl-1-methylsulfonyl-pyrrolidin-3-yl)amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[[3-(6-methyl-3-pyridyl)cyclobutyl]amino]-1,2,4-triazol-4-yl]-4,5,6,7-tetrahydrobenzothiophene-3-carboxamide
(5S)-2-(cyclopropanecarbonylamino)-N-(cyclopropylmethyl)-5-[3-[[3-(6-methyl-3-pyridyl)cyclobutyl]amino]-1,2,4-triazol-4-yl]-4,5,6,7-tetrahydrobenzothiophene-3-carboxamide;
(5S)-5-[3-[[(3S*)-1-benzoyl-4,4-difluoro-pyrrolidin-3-yl]amino]-1,2,4-triazol-4-yl]-2-(cyclopropanecarbonylamino)-N-(cyclopropylmethyl)-4,5,6,7-tetrahydrobenzothiophene-3-carboxamide [* or R];
(5S)-5-[3-[(1-acetyl-4-piperidyl)amino]-1,2,4-triazol-4-yl]-2-(cyclopropanecarbonylamino)-N-(cyclopropylmethyl)-4,5,6,7-tetrahydrobenzothiophene-3-carboxamide ;
Methyl (3S)-3-[[4-[(5S)-2-(cyclopropanecarbonylamino)-3-(cyclopropylmethylcarbamoyl)-4,5,6,7-tetrahydrobenzothiophen-5-yl]-1,2,4-triazol-3-yl]amino]pyrrolidine-1-carboxylate;
(3S)-3-[[4-[(5S)-2-(cyclopropanecarbonylamino)-3-(cyclopropylmethylcarbamoyl)-4,5,6,7-tetrahydrobenzothiophen-5-yl]-1,2,4-triazol-3-yl]amino]-N-ethyl-pyrrolidine-1-carboxamide ;
(5S)-2-cyclopropaneamido-N-(cyclopropylmethyl)-5-(3-{[2-(pyrimidin-2-yl)-2-azaspiro[4.4]nonan-7-yl]amino}-4H-1,2,4-triazol-4-yl)-4,5,6,7-tetrahydro-1-benzothiophene-3-carboxamide;
(5S)-2-(cyclopropanecarbonylamino)-N-(cyclopropylmethyl)-5-[3-[[2-methyl-5-(trifluoromethoxy)pyrazol-3-yl]amino]-1,2,4-triazol-4-yl]-4,5,6,7-tetrahydrobenzothiophene-3-carboxamide;
(5S)-5-[3-[[5-[chloro(difluoro)methoxy]-2-methyl-pyrazol-3-yl]amino]-1,2,4-triazol-4-yl]-2-(cyclopropanecarbonylamino)-N-(cyclopropylmethyl)-4,5,6,7-tetrahydrobenzothiophene-3-carboxamide;
(5S)-2-cyclopropaneamido-N-(cyclopropylmethyl)-5-(3-{[1-methyl-3-(5-methyl-1,3,4-oxadiazol-2-yl)-1H-pyrazol-5-yl]amino}-4H-1,2,4-triazol-4-yl)-4,5,6,7-tetrahydro-1-benzothiophene-3-carboxamide;
(5S)-2-(cyclopropanecarbonylamino)-N-(cyclopropylmethyl)-5-[3-[(5-methoxy-3-methyl-pyrazin-2-yl)amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[(5-methylthiazolo[5,4-b]pyridin-2-yl)amino]-4,5,6,7-tetrahydrobenzothiophene-3-carboxamide;
(5S)-5-[3-[(5-cyano-2-methyl-3-pyridyl)amino]-1,2,4-triazol-4-yl]-2-(cyclopropanecarbonylamino)-N-(cyclopropylmethyl)-4,5,6,7-tetrahydrobenzothiophene-3-carboxamide;
(5S)-5-[3-[(6-cyano-2-methyl-3-pyridyl)amino]-1,2,4-triazol-4-yl]-2-(cyclopropanecarbonylamino)-N-(cyclopropylmethyl)-4,5,6,7-tetrahydrobenzothiophene-3-carboxamide;
(5S)-2-(cyclopropanecarbonylamino)-N-(cyclopropylmethyl)-5-[3-[(1-methyl-2-oxo-4-pyridyl)amino]-1,2,4-triazol-4-yl]-4,5,6,7-tetrahydrobenzothiophene-3-carboxamide;
(5S)-5-[3-[(6-chloro-2-methyl-3-pyridyl)amino]-1,2,4-triazol-4-yl]-2-(cyclopropanecarbonylamino)-N-(cyclopropylmethyl)-4,5,6,7-tetrahydrobenzothiophene-3-carboxamide;
(5S)-2-(cyclopropanecarbonylamino)-N-(cyclopropylmethyl)-5-[3-[[2-methyl-6-(trifluoromethyl)-3-pyridyl]amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[(3-methylpyridazin-4-yl)amino]-1,2,4-triazol-4-yl]-4,5,6,7-tetrahydrobenzothiophene-3-carboxamide;
(5S)-5-[3-[(5-chloro-2-methyl-pyrazol-3-yl)amino]-1,2,4-triazol-4-yl]-2-(cyclopropanecarbonylamino)-N-(cyclopropylmethyl)-4,5,6,7-tetrahydrobenzothiophene-3-carboxamide;
(5S)-2-(cyclopropanecarbonylamino)-N-(cyclopropylmethyl)-5-[3-[(3-methylisothiazol-4-yl)amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[(3-methylisoxazol-4-yl)amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[(5-methoxy-2-methylpyrazol-3-yl)amino]-1,2,4-triazol-4-yl]-4,5,6,7-tetrahydro-1-benzothiophene-3-carboxamide;
(5S)-2-(cyclopropanecarbonylamino)-N-(cyclopropylmethyl)-5-[3-[[5-(difluoromethoxy)-2-methylpyrazol-3-yl]amino]-1,2,4-triazol-4-yl]-4,5,6,7-tetrahydro-1-benzothiophene-3-carboxamide;
(5S)-2-(cyclopropanecarbonylamino)-N-(cyclopropylmethyl)-5-[3-[[5-(difluoromethyl)-2-(trideuteriomethyl)pyrazol-3-yl]amino]-1,2,4-triazol-4-yl]-4,5,6,7-tetrahydrobenzothiophene-3-carboxamide;
(5S)-5-[3-[(5-Cyano-2-methyl-pyrazol-3-yl)amino]-1,2,4-triazol-4-yl]-2-(cyclopropanecarbonylamino)-N-(cyclopropylmethyl)-4,5,6,7-tetrahydrobenzothiophene-3-carboxamide;
(5S)-2-(Cyclopropanecarbonylamino)-N-(cyclopropylmethyl)-5-[3-[(2,2-difluoro-1,3-benzodioxol-5-yl)amino]-1,2,4-triazol-4-yl]-4,5,6,7-tetrahydrobenzothiophene-3-carboxamide;
(4RS,5RS)-2-(cyclopropanecarbonylamino)-N-(cyclopropylmethyl)-4-methyl-5-[3-[[2-methyl-5-(trifluoromethyl)pyrazol-3-yl]amino]-1,2,4-triazol-4-yl]-4,5,6,7-tetrahydrobenzothiophene-3-carboxamide;
(4R*,5R*)-2-(cyclopropanecarbonylamino)-N-(cyclopropylmethyl)-4-methyl-5-[3-[[2-methyl-5-(trifluoromethyl)pyrazol-3-yl]amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[[6-(difluoromethyl)-3-pyridyl]amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[(6-fluoro-2-methyl-3-pyridyl)amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[[6-(trifluoromethyl)-3-pyridyl]amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[(6-methoxy-3-pyridyl)amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[(2-methoxy-4-pyridyl)amino]-1,2,4-triazol-4-yl]-4,5,6,7-tetrahydrobenzothiophene-3-carboxamide;
(5S)-5-[3-[(6-chloro-3-pyridyl)amino]-1,2,4-triazol-4-yl]-2-(cyclopropanecarbonylamino)-N-(cyclopropylmethyl)-4,5,6,7-tetrahydrobenzothiophene-3-carboxamide;
(5S)-5-[3-[(2-chloro-4-pyridyl)amino]-1,2,4-triazol-4-yl]-2-(cyclopropanecarbonylamino)-N-(cyclopropylmethyl)-4,5,6,7-tetrahydrobenzothiophene-3-carboxamide;
(5S)-2-(cyclopropanecarbonylamino)-N-(cyclopropylmethyl)-5-[3-[(6-methoxy-2-methyl-3-pyridyl)amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[(2-fluoro-3-pyridyl)amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[(3-methyltriazol-4-yl)amino]-1,2,4-triazol-4-yl]-4,5,6,7-tetrahydrobenzothiophene-3-carboxamide;
(3R*)-6-(cyclopropanecarbonylamino)-N-(cyclopropylmethyl)-3-[3-[[2-methyl-5-(trifluoromethyl)pyrazol-3-yl]amino]-1,2,4-triazol-4-yl]-3,4-dihydro-2H-thieno[2,3-b]pyran-5-carboxamide (* or S).

13. A compound according to claim 1 or a pharmaceutically acceptable salt thereof, wherein the compound is selected amongst:
(5S)-2-(Cyclopropanecarbonylamino)-N-(cyclopropylmethyl)-5-[3-[(2,5-dimethylpyrazol-3-yl)amino]-1,2,4-triazol-4-yl]-4,5,6,7-tetrahydrobenzothiophene-3-carboxamide;
Ethyl 5-amino-1-[(5S)-2-(cyclopropanecarbonylamino)-3-(cyclopropylmethylcarbamoyl)-4,5,6,7-tetrahydrobenzothiophen-5-yl]imidazole-4-carboxylate;
(5S)-2-(cyclopropanecarbonylamino)-N-(cyclopropylmethyl)-5-[3-[(5-cyclopropyl-2-methyl-pyrazol-3-yl)amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[(5-isopropyl-2-methyl-pyrazol-3-yl)amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[[2-methyl-5-(trifluoromethyl)pyrazol-3-yl]amino]-1,2,4-triazol-4-yl]-4,5,6,7-tetrahydrobenzothiophene-3-carboxamide;
(5S)-2-(cyclopropanecarbonylamino)-N-(cyclopropylmethyl)-5-[3-[(5-ethyl-2-methyl-pyrazol-3-yl)amino]-1,2,4-triazol-4-yl]-4,5,6,7-tetrahydrobenzothiophene-3-carboxamide;
(5S)-2-(Cyclopropanecarbonylamino)-N-(cyclopropylmethyl)-5-[3-[[5-(difluoromethyl)-2-methyl-pyrazol-3-yl]amino]-1,2,4-triazol-4-yl]-4,5,6,7-tetrahydrobenzothiophene-3-carboxamide.

14. A compound according to claim 1 or a pharmaceutically acceptable salt thereof, wherein the compound is selected amongst: 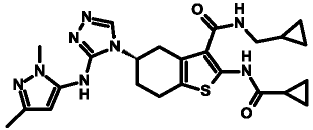
(5S)-2-(Cyclopropanecarbonylamino)-N-(cyclopropylmethyl)-5-[3-[(2,5-dimethylpyrazol-3-yl)amino]-1,2,4-triazol-4-yl]-4,5,6,7-tetrahydrobenzothiophene-3-carboxamide; 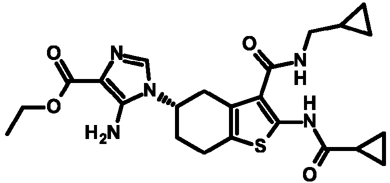
Ethyl 5-amino-1-[(5S)-2-(cyclopropanecarbonylamino)-3-(cyclopropylmethylcarbamoyl)-4,5,6,7-tetrahydrobenzothiophen-5-yl]imidazole-4-carboxylate; 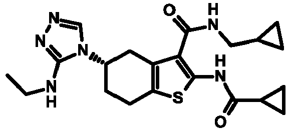
(5S)-2-(cyclopropanecarbonylamino)-N-(cyclopropylmethyl)-5-[3-(ethylamino)-1,2,4-triazol-4-yl]-4,5,6,7-tetrahydrobenzothiophene-3-carboxamide; 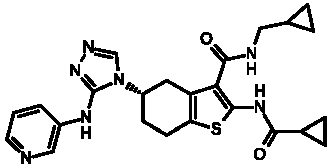
(5S)-2-(Cyclopropanecarbonylamino)-N-(cyclopropylmethyl)-5-[3-(3-pyridylamino)-1,2,4-triazol-4-yl]-4,5,6,7-tetrahydrobenzothiophene-3-carboxamide; 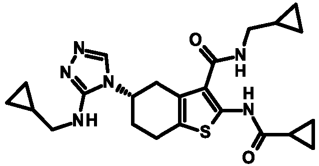
(5S)-2-(Cyclopropanecarbonylamino)-N-(cyclopropylmethyl)-5-[3-(cyclopropylmethylamino)-1,2,4-triazol-4-yl]-4,5,6,7-tetrahydrobenzothiophene-3-carboxamide; 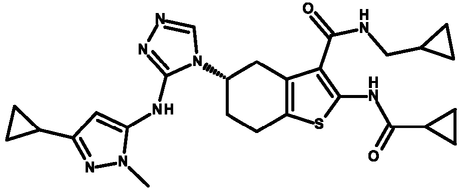
(5S)-2-(cyclopropanecarbonylamino)-N-(cyclopropylmethyl)-5-[3-[(5-cyclopropyl-2-methyl-pyrazol-3-yl)amino]-1,2,4-triazol-4-yl]-4,5,6,7-tetrahydrobenzothiophene-3-carboxamide; 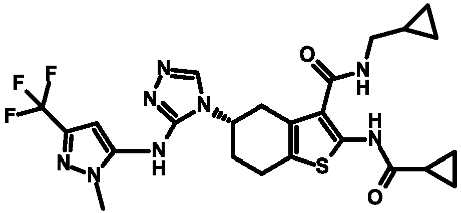
(5S)-2-(cyclopropanecarbonylamino)-N-(cyclopropylmethyl)-5-[3-[[2-methyl-5-(trifluoromethyl)pyrazol-3-yl]amino]-1,2,4-triazol-4-yl]-4,5,6,7-tetrahydrobenzothiophene-3-carboxamide; 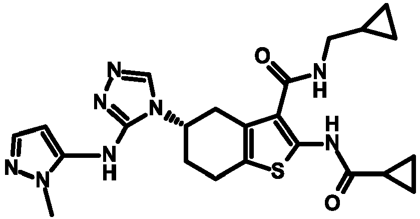
(5S)-2-(cyclopropanecarbonylamino)-N-(cyclopropylmethyl)-5-[3-[(2-methylpyrazol-3-yl)amino]-1,2,4-triazol-4-yl]-4,5,6,7-tetrahydrobenzothiophene-3-carboxamide; 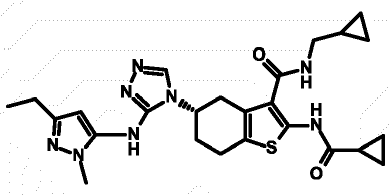
(5S)-2-(cyclopropanecarbonylamino)-N-(cyclopropylmethyl)-5-[3-[(5-ethyl-2-methyl-pyrazol-3-yl)amino]-1,2,4-triazol-4-yl]-4,5,6,7-tetrahydrobenzothiophene-3-carboxamide; 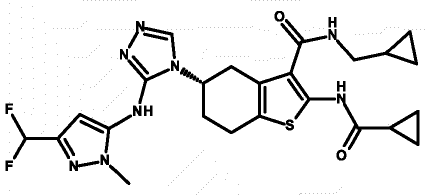
(5S)-2-(Cyclopropanecarbonylamino)-N-(cyclopropylmethyl)-5-[3-[[5-(difluoromethyl)-2-methyl-pyrazol-3-yl]amino]-1,2,4-triazol-4-yl]-4,5,6,7-tetrahydrobenzothiophene-3-carboxamide; and 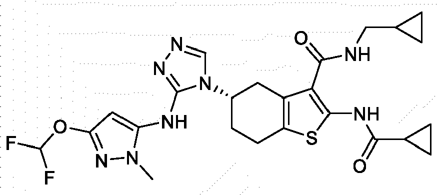
(5S)-2-(cyclopropanecarbonylamino)-N-(cyclopropylmethyl)-5-[3-[[5-(difluoromethoxy)-2-methylpyrazol-3-yl]amino]-1,2,4-triazol-4-yl]-4,5,6,7-tetrahydro-1-benzothiophene-3-carboxamide.

15. A compound according to claim 1 or 2 or a pharmaceutically acceptable salt thereof, wherein the compound is **characterized by** formula (XXV) or (XXVI)

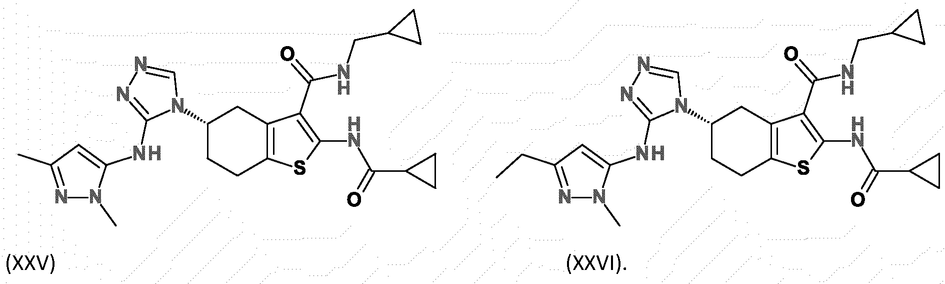

16. A compound according to claim 1 or 2 or a pharmaceutically acceptable salt thereof, wherein the compound is **characterized by** formula (XXVII) or (XXVIII)

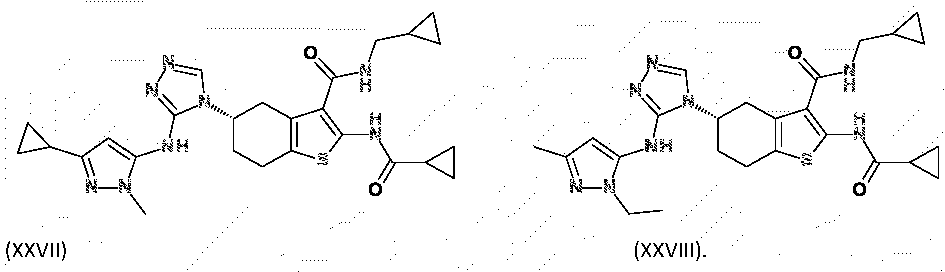

17. A compound according to claim 1 or 2 or a pharmaceutically acceptable salt thereof, wherein the compound is **characterized by** formula (XXIX) or (XXX)

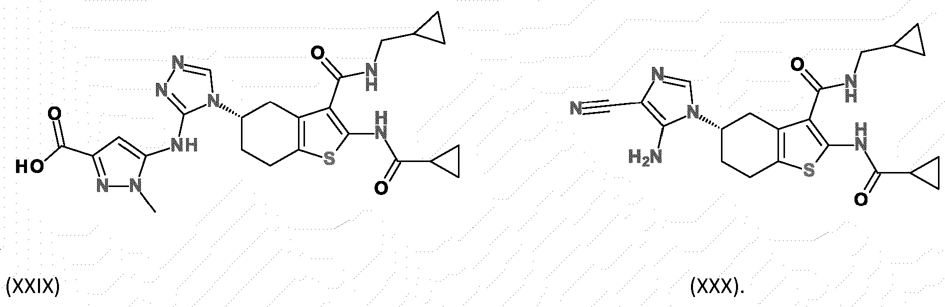

18. A compound according to claim 1 or 2 or a pharmaceutically acceptable salt thereof, wherein the compound is **characterized by** formula (XXXII)

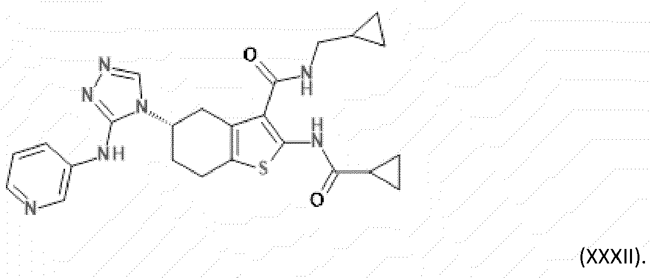

19. A compound according to claim 1 or 2 or a pharmaceutically acceptable salt thereof, wherein the compound is **characterized by** formula (XXXIII) or (XXXIV)

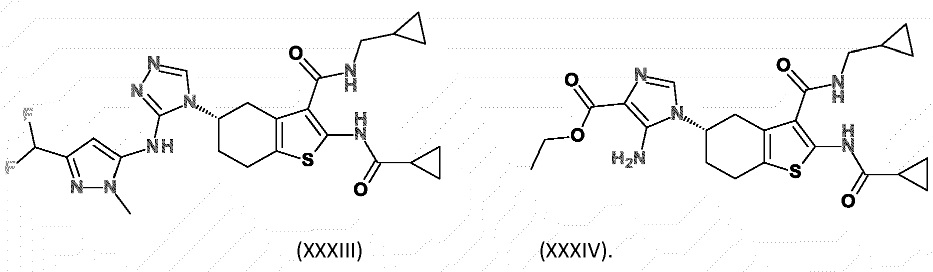

20. A compound according to claim 1 or 2 or a pharmaceutically acceptable salt thereof, wherein the compound is **characterized by** formula (XXXV) or (XXXVI)

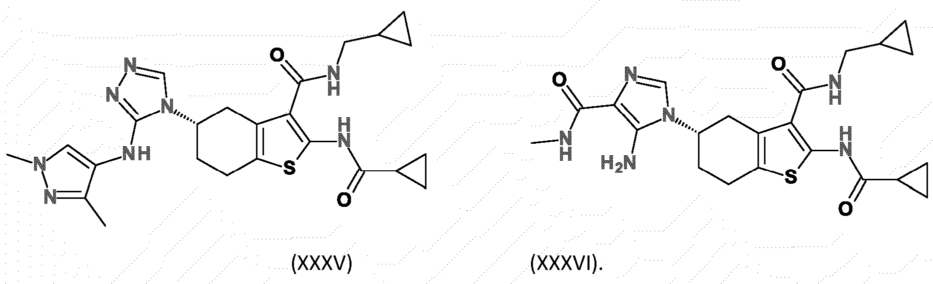

21. A pharmaceutical composition comprising a compound according to any one of claims 1 to 20 or a pharmaceutically acceptable salt thereof.

22. A compound according to any one of claims 1 to 20 or a pharmaceutically acceptable salt thereof for use in a method for the treatment or prevention of disorders caused by IgE.

23. A compound for use according to claim 22 or a pharmaceutically acceptable salt thereof, wherein the disorder is allergy, non-allergic mast cell responses, type 1 hypersensitivity, urticaria, or familiar sinus inflammation.

24. A compound for use according to claim 22 or a pharmaceutically acceptable salt thereof, wherein the disorder is airway constriction in asthma, local inflammation in eczema, increased mucus secretion in allergic rhinitis, urticaria, or increased vascular permeability.

25. A compound for use according to claim 22 or a pharmaceutically acceptable salt thereof, wherein the disorder is eosinophilic granulomatosis with polyangiitis (also known as "Churg Strauss syndrome"), aspirin exacerbated respiratory disease, or cutaneous T-cell lymphoma.

## Patentansprüche

1. Verbindungen der Formel (I) und pharmazeutisch unbedenkliche Salze davon: 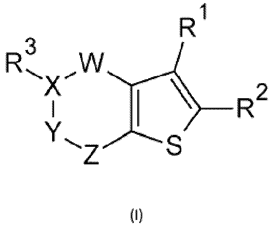 wobei,
X CH, N oder C-R⁴ darstellt, wobei, wenn X C-R⁴ ist, R³ und R⁴ miteinander verknüpft sein können, um einen 4-6-gliedrigen Cycloalkyl- oder heterocyclischen Ring auszubilden,
wobei ein beliebiges davon optional durch einen oder mehrere Substituenten substituiert sein kann, ausgewählt aus Halogen, =O, OH, SMe, C₁-C₂-Alkoxy, O-C₁-C₂-Haloalkyl, C₁-C₂-Alkyl, C₁-C₂-Heteroalkyl, C₁-C₂-Haloalkyl, NH₂, NHMe, NMe₂, Aryl und Heteroaryl, substituiert sein kann, wobei Aryl und Heteroaryl optional durch einen oder mehrere Substituenten, ausgewählt aus Halogen, OH, C₁-C₆-Alkyl, C₁-C₆-Alkoxy und C₁-C₂-Haloalkyl, substituiert sind;
wenn X N darstellt, dann W, Y und Z C sind oder eines von W, Y und Z eine Einfachbindung ist, wobei die anderen zwei C sind;
wenn X CH oder C-R⁴ darstellt, dann W, Y und Z C sind, oder eines von W, Y oder Z eine Einfachbindung, O, S, S(O), S(O) oder N ist, wobei die anderen zwei C sind;
wenn W, Y oder Z C sind, jedes C durch 1 oder 2 Gruppen substituiert sein kann, die unabhängig voneinander aus H, Halogen, SF₅, =O, =NOH, -OH, -CN, -C(O)Me, -C(O)OH, - C(O)NH₂, NH-C(O)O-C₁-C₄-Alkyl, -NO₂, NH₂, NHMe, NMe₂, SH, SMe, SOMe, SO₂Me, C₁-C₄-Alkyl, C₃-C₄-Cycloalkyl, C₁-C₄-Alkoxy, Heterocyclus, C₁-C₄-Heteroalkyl, die miteinander verknüpft sein können, um einen 3-6-gliedrigen Cycloalkyl- oder 4- bis 6-gliedrigen heterocyclischen Ring auszubilden,
wobei ein beliebiges von diesen optional durch einen oder mehrere Substituenten substituiert sein kann, ausgewählt aus Halogen, =O, OH, SMe, C₁-C₂-Alkoxy, O-C₁-C₂-Haloalkyl, C₁-C₂-Alkyl, C₁-C₂-Heteroalkyl, C₁-C₂-Haloalkyl, NH₂, NHMe, Nme₂, die miteinander verknüpft sein können, um einen 3-6-gliedrigen Cycloalkyl- oder einen 4-6-gliedrigen heterocyclischen Ring auszubilden;
wobei,
wenn W, Y oder Z N ist, dieses N unabhängig durch H, C₁-C₄-Alkyl, C₃-C₄-Cycloalkyl, Heterocyclus, -C(O)Me, -C(O)NH₂, -C(O)OH, -S(O)Me, -S(O)₂Me, -S(O)₂NH₂ substituiert sein kann, von denen ein beliebiges optional durch einen oder mehrere Substituenten substituiert sein kann, ausgewählt aus Halogen, OH, SMe, C₁-C₂-Alkoxy, O-C₁-C₂-Haloalkyl, C₁-C₂-Alkyl, C₁-C₂-Heteroalkyl, C₁-C₂-Haloalkyl, NH₂, NHMe, NMe₂;
und W, X, Y und Z direkt oder über Substituenten miteinander verknüpft sein können, um einen Ring auszubilden;
R1 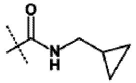 darstellt;
R2 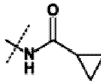 darstellt;
R³ aus H, -OH, C₁-C₈-Alkyl, -NR^{d}R^{c}, -NH-R¹⁴-NH-R^{a}, -NMe-R¹⁴-NH-R^{a}, -NH-R¹⁴-NMe-R^{a}, -CO-NR^{d}R^{c}, -NH-COO-R^{c}, -NH-CO-R^{c}, -COOH, -COO-C₁-C₈-Alkyl, -CO-C₁-C₈-Alkyl, -C₁-C₈-Alkylen-COO-C₁-C₈-Alkyl, -C₁-C₈-Alkylen-NR^{a}, -COO-C₁-C₈-Alkyl, -C₁-C₈-Alkylen-NR^{d}R^{c}, -C₁-C₈-Alkylen-NH-R¹⁴-NH-R^{a}, Heteroaryl, -CN, -CH=N-OH, -CF₃, Alkylamino, Amino, -NH-SO-C₁-C₈-Alkyl, -NH-SO₂-C₁-C₈-Alkyl, Heteroalkyl-Amino, Aryl, Heterocyclus, C₃-C₆-Cycloalkyl, -NHS(O)(=NH)Me, -SO₂-C₁-C₈-Alkyl, -NH-C(CH₃)=N-CN, -NH-CH=N-CN, -NH-S(=O)(=N)-C₁-C₂-Alkyl ausgewählt ist,
wobei ein beliebiges von diesen optional durch einen oder mehrere Substituenten substituiert sein kann, ausgewählt aus Halogen, -OH, =O, -COOH, -COH, -CN, SMe, -NR^{d}R^{c}, -OR^{c}, C₁-C₂-Alkoxy, O-C₁-C₂-Haloalkyl, C₁-C₂-Alkyl, C₁-C₂-Heteroalkyl, C₁-C₂-Haloalkyl, C₁-C₂-Alkylen-O-C₁-C₂-Alkyl, C₁-C₂-Alkylen-C₃-C₄-Cycloalkyl, Heterocyclus, C₃-C₆-Cycloalkyl, Aryl, Heteroaryl, C₃-C₆-Cycloalkyl-Amino, Heterocyclo-Amino, Aryl-Amino, Heteroaryl-Amino, -CONR^{d}R^{c}, -NHCO-R^{a}, -NHCOO-C₁-C₄-Alkyl, -CO-C₁-C₂-Alkyl, -COO-R^{a}, die miteinander verknüpft sein können, um einen Ring auszubilden, und wobei -CONR^{d}R^{c} und - NR^{d}R^{c} miteinander verknüpft sein können, um einen siebengliedrigen Ring auszubilden, der mit einer sechsgliedrigen Arylgruppe verschmolzen ist;
wobei Aryl, Heteroaryl, Heterocyclus und Cycloalkyl optional durch einen oder mehrere Substituenten substituiert sind, ausgewählt aus Halogenoxo, Cyano, OH, -NOOH, C₁-C₆-Alkyl, C₁-C₆-Alkoxy, Halo-C₁-C₆-Alkoxy, C₃-C₆-Cycloalkyl-C₁-C₃-Alkoxy, -O-CO-C₁-C₆-Alkyl, C₃-C₆-Cycloalkyl, C₁-C₂-Haloalkyl, C₂-C₄-Heterocyclus, Acetyl, Acetyl-Phenyl, - CONR^{d}R_{c}, COO-C₁-C₈-Alkyl, -S(O)NH-C₁-C₂-Alkyl, -S(O)n-C₁-C₂-Alkyl, Heteroaryl, das optional durch eine oder mehrere Halogengruppen substituiert sein kann, C₁-C₂-Alkyl, wobei n gleich 0, 1 oder 2 ist;
R⁴ aus Halogen, OH, CN, NO₂, NH₂, NHMe, NMe₂, SH, SMe, SOMe, SO2Me, C₁-C₄-Alkyl, C₃-C₄-Cycloalkyl, C₁-C₃-Alkoxy, C₁-C₃-Heteroalkyl, Heterocyclus ausgewählt ist,
wobei ein beliebiges von diesen optional durch einen oder mehrere Substituenten substituiert sein kann, ausgewählt aus Halogen, =O, OH, SMe, C₁-C₂-Alkoxy, O-C₁-C₂-Haloalkyl, C₁-C₂-Alkyl, C₁-C₂-Heteroalkyl, C₁-C₂-Haloalkyl, NH₂, NHMe, Nme₂, die miteinander verknüpft sein können, um einen Ring auszubilden;
R¹⁴ C=O, C=S, C=N-OH, C=NR^{b}, C=CH-NO₂, SO₂ und C₃-C₆-Cycloalkenyl ist, das durch 1 oder 2 =O substituiert sein kann;
R^{a} aus H, C₁-C₈-Alkyl, Heteroalkyl, C₁-C₈-Alkoxy, Heterocyclus, C₃-C₈-Cycloalkyl, Heteroaryl und Aryl ausgewählt ist,
wobei ein beliebiges von diesen optional durch einen oder mehrere Substituenten substituiert sein kann, ausgewählt aus Halogen, =O, OH, SMe, C₁-C₂-Alkoxy, -O-C₁-C₂-Haloalkyl, C₁-C₂-Alkyl, C₃-C₆-Cycloalkyl, C₃-C₆-Heterocycloalkyl, C₁-C₂-Heteroalkyl, C₁-C₂-Haloalkyl, Aryl, Heteroaryl, NH₂, NHMe, NMe₂, die miteinander verknüpft sein können, um einen Ring auszubilden;
R^{b} aus CN, Aryl, Heteroaryl, -NO₂, -O-C₁-C₂-Alkyl, -CO-O-C₁-C₂-Alkyl ausgewählt ist, wobei Aryl und Heteroaryl optional durch ein oder mehrere C₁-C₂-Alkyle substituiert sind; R^{c} aus H, Aryl, Heteroaryl, C₃-C₆-Cycloalkenyl, C₃-C₈-Cycloalkyl, Heterocyclus, C₁-C₈-Alkyl, C₁-C₈-Heteroalkyl, C₁-C₄-Alkylen-C₃-C₆-Cycloalkyl ausgewählt ist;
wobei ein beliebiges von diesen optional durch einen oder mehrere Substituenten substituiert sein kann, ausgewählt aus C(O)(R^{g}), C(O)N(R^{f})(R^{g}), Halogen, SFs, OH, =O, -COH, -COOH, -CO-O-R^{a}, CN, SMe, SO₂Me, SO₂R^{g}, C₁-C₄-Alkoxy, O-C₁-C₄-Haloalkyl, C₁-C₄-Alkyl, C₁-C₂-Heteroalkyl, C₁-C₄-Haloalkyl, N(R^{f})(R^{g}), N(R^{f})SO₂(R^{g}), SO₂NH₂, SO₂NHMe, SO₂NMe₂, S(O)(NR^{f})R^{g}, C₃-C₆-Cycloalkyl, Heterocyclus, C₁-C₄-Alkylen-C₃-C₆-Cycloalkyl, Aryl und Heteroaryl, die miteinander verknüpft sein können, um einen Ring auszubilden,
wobei Aryl, Heteroaryl, Heterocyclus und Cycloalkyl optional durch einen oder mehrere Substituenten substituiert sein können, ausgewählt aus Halogen, =O, =NH, =NMe, OH, C₁-C₂-Alkyl, C₁-C₂-Alkoxy, -COOH, -COO-C₁-C₂-Alkyl, SO₂R^{a}, COR^{a}, SOR^{a}, S(O)(NH)R^{a}, CONHR^{a}, die miteinander verknüpft sein können, um einen Ring auszubilden;
R^{d} H oder C₁-C₄-Alkyl darstellt
und R^{c} und R^{d} miteinander verknüpft sein können, um einen Heterocyclus auszubilden;
R^{f} aus H, Aryl, Heteroaryl, C₃-C₆-Cycloalkenyl, C₃-C₈-Cycloalkyl, Heterocyclus, C₁-C₈-Alkyl, C₁-C₈-Heteroalkyl, C₁-C₄-Alkylen-C₃-C₆-Cycloalkyl ausgewählt ist,
wobei ein beliebiges von diesen optional durch einen oder mehrere Substituenten substituiert sein kann, ausgewählt aus Halogen, =O, =NH, =NMe, OH, Aryl, Heteroaryl, C₁-C₂-Alkyl, C₁-C₂-Haloalkyl, C₁-C₂-Alkoxy, O-C₁-C₂-Haloalkyl, -COOH, -COO-C₁-C₂-Alkyl, SO₂R^{a}, COR^{a}, SOR^{a}, S(O)(NH)R^{a}, CONHR^{a};
R^{g} aus H, Aryl, Heteroaryl, C₃-C₆-Cycloalkenyl, C₃-C₈-Cycloalkyl, Heterocyclus, C₁-C₈-Alkyl, C₁-C₈-Heteroalkyl, C₁-C₄-Alkylen-C₃-C₆-Cycloalkyl ausgewählt ist,
wobei ein beliebiges von diesen optional durch einen oder mehrere Substituenten substituiert sein kann, ausgewählt aus Halogen, =O, =NH, =NMe, OH, Aryl, Heteroaryl, C₁-C₂-Alkyl, C₁-C₂-Haloalkyl, C₁-C₂-Alkoxy, O-C₁-C₂-Haloalkyl, -COOH, -COO-C₁-C₂-Alkyl, SO₂R^{a}, COR^{a}, SOR^{a}, S(O)(NH)R^{a}, CONHR^{a}
R^{f} und R^{g} miteinander verknüpft sein können, um einen Ring auszubilden.

2. Verbindung nach Anspruch 1 oder ein pharmazeutisch unbedenkliches Salz davon, 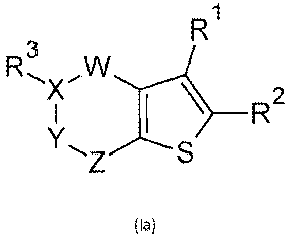 wobei,
X CH, N oder C-R⁴ darstellt, wobei, wenn X C-R⁴ ist, R³ und R⁴ miteinander verknüpft sein können, um einen 4-6-gliedrigen Cycloalkyl- oder heterocyclischen Ring auszubilden,
wobei ein beliebiges davon optional durch einen oder mehrere Substituenten substituiert sein kann, ausgewählt aus Halogen, =O, OH, SMe, C₁-C₂-Alkoxy, O-C₁-C₂-Haloalkyl, C₁-C₂-Alkyl, C₁-C₂-Heteroalkyl, C₁-C₂-Haloalkyl, NH₂, NHMe, NMe₂, Aryl und Heteroaryl, substituiert sein kann, wobei Aryl und Heteroaryl optional durch einen oder mehrere Substituenten, ausgewählt aus Halogen, OH, C₁-C₆-Alkyl, C₁-C₆-Alkoxy und C₁-C₂-Haloalkyl, substituiert sind;
wenn X N darstellt, dann W, Y und Z C sind oder eines von W, Y und Z eine Einfachbindung ist, wobei die anderen zwei C sind;
wenn X CH oder C-R⁴ darstellt, dann W, Y und Z C sind, oder eines von W, Y oder Z eine Einfachbindung, O, S, S(O), S(O) oder N ist, wobei die anderen zwei C sind;
wenn W, Y oder Z C sind, jedes C durch 1 oder 2 Gruppen substituiert sein kann, die unabhängig voneinander aus H, Halogen, SF₅, =O, =NOH, -OH, -CN, -C(O)Me, -C(O)OH, - C(O)NH₂, NH-C(O)O-C₁-C₄-Alkyl, -NO₂, NH₂, NHMe, NMe₂, SH, SMe, SOMe, SO₂Me, C₁-C₄-Alkyl, C₃-C₄-Cycloalkyl, C₁-C₄-Alkoxy, Heterocyclus, C₁-C₄-Heteroalkyl ausgewählt sind, die miteinander verknüpft sein können, um einen 3-6-gliedrigen Cycloalkyl- oder einen 4-6-gliedrigen heterocyclischen Ring auszubilden,
wobei ein beliebiges von diesen optional durch einen oder mehrere Substituenten substituiert sein kann, ausgewählt aus Halogen, =O, OH, SMe, C₁-C₂-Alkoxy, O-C₁-C₂-Haloalkyl, C₁-C₂-Alkyl, C₁-C₂-Heteroalkyl, C₁-C₂-Haloalkyl, NH₂, NHMe, Nme₂, die miteinander verknüpft sein können, um einen 3-6-gliedrigen Cycloalkyl- oder einen 4-6-gliedrigen heterocyclischen Ring auszubilden;
wobei,
wenn W, Y oder Z N ist, dieses N unabhängig durch H, C₁-C₄-Alkyl, C₃-C₄-Cycloalkyl, Heterocyclus, -C(O)Me, -C(O)NH₂, -C(O)OH, -S(O)Me, -S(O)₂Me, -S(O)₂NH₂ substituiert sein kann, von denen ein beliebiges optional durch einen oder mehrere Substituenten substituiert sein kann, ausgewählt aus Halogen, OH, SMe, C₁-C₂-Alkoxy, O-C₁-C₂-Haloalkyl, C₁-C₂-Alkyl, C₁-C₂-Heteroalkyl, C₁-C₂-Haloalkyl, NH₂, NHMe, NMe₂;
und W, X, Y und Z direkt oder über Substituenten miteinander verknüpft sein können, um einen Ring auszubilden;
R1 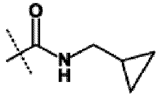 darstellt;
R2 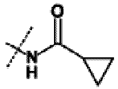 darstellt;
R³ aus H, -OH, C₁-C₈-Alkyl, -NR^{d}R^{c}, -NH-R¹⁴-NH-R^{a}, -NMe-R¹⁴-NH-R^{a}, -NH-R¹⁴-NMe-R^{a}, -CO-NR^{d}R^{c}, -NH-COO-R^{c}, -NH-CO-R^{c}, -COOH, -COO-C₁-C₈-Alkyl, -CO-C₁-C₈-Alkyl, -C₁-C₈-Alkylen-COO-C₁-C₈-Alkyl, -C₁-C₈-Alkylen-NR^{a}, -COO-C₁-C₈-Alkyl, -C₁-C₈-Alkylen-NR^{d}R^{c}, -C₁-C₈-Alkylen-NH-R¹⁴-NH-R^{a}, Heteroaryl, -CN, -CH=N-OH, -CF₃, Alkylamino, Amino, -NH-SO-C₁-C₈-Alkyl, -NH-SO₂-C₁-C₈-Alkyl, Heteroalkyl-Amino, Aryl, Heterocyclus, C₃-C₆-Cycloalkyl, -NHS(O)(=NH)Me, -SO₂-C₁-C₈-Alkyl, -NH-C(CH₃)=N-CN, -NH-CH=N-CN, -NH-S(=O)(=N)-C₁-C₂-Alkyl ausgewählt ist,
wobei ein beliebiges von diesen optional durch einen oder mehrere Substituenten substituiert sein kann, ausgewählt aus Halogen, -OH, =O, -COOH, -COH, -CN, SMe, - NR^{d}R^{c}, -OR^{c}, C₁-C₂-Alkoxy, O-C₁-C₂-Haloalkyl, C₁-C₂-Alkyl, C₁-C₂-Heteroalkyl, C₁-C₂-Haloalkyl, C₁-C₂-Alkylen-O-C₁-C₂-Alkyl, C₁-C₂-Alkylen-C₃-C₄-Cycloalkyl, Heterocyclus, C₃-C₆-Cycloalkyl, Aryl, Heteroaryl, C₃-C₆-Cycloalkyl-Amino, Heterocyclus-Amino, Aryl-Amino, Heteroaryl-Amino, -CONR^{d}R^{c}, -NHCO-R^{a}, -NHCOO-C₁-C₄-Alkyl, -CO-C₁-C₂-Alkyl, -COO-R^{a}, die miteinander verknüpft sein können, um einen Ring auszubilden,
wobei Aryl, Heteroaryl, Heterocyclus und Cycloalkyl optional durch einen oder mehrere Substituenten substituiert sind, ausgewählt aus Halogen, OH, C₁-C₆-Alkyl, C₁-C₆-Alkoxy, -O-CO-C₁-C₆-Alkyl, C₃-C₆-Cycloalkyl, C₁-C₂-Haloalkyl und C₂-C₄-Heterocyclus;
R⁴ aus Halogen, OH, CN, NO₂, NH₂, NHMe, NMe₂, SH, SMe, SOMe, SO2Me, C₁-C₄-Alkyl, C₃-C₄-Cycloalkyl, C₁-C₃-Alkoxy, C₁-C₃-Heteroalkyl, Heterocyclus ausgewählt ist,
wobei ein beliebiges von diesen optional durch einen oder mehrere Substituenten substituiert sein kann, ausgewählt aus Halogen, =O, OH, SMe, C₁-C₂-Alkoxy, O-C₁-C₂-Haloalkyl, C₁-C₂-Alkyl, C₁-C₂-Heteroalkyl, C₁-C₂-Haloalkyl, NH₂, NHMe, Nme₂, die miteinander verknüpft sein können, um einen Ring auszubilden;
R¹⁴ C=O, C=S, C=N-OH, C=NR^{b}, C=CH-NO₂, SO₂ und C₃-C₆-Cycloalkenyl ist, das durch 1 oder 2 =O substituiert sein kann;
R^{a} aus H, C₁-C₈-Alkyl, Heteroalkyl, C₁-C₈-Alkoxy, Heterocyclus, C₃-C₈-Cycloalkyl, Heteroaryl und Aryl ausgewählt ist,
wobei ein beliebiges von diesen optional durch einen oder mehrere Substituenten substituiert sein kann, ausgewählt aus Halogen, =O, OH, SMe, C₁-C₂-Alkoxy, -O-C₁-C₂-Haloalkyl, C₁-C₂-Alkyl, C₃-C₆-Cycloalkyl, C₁-C₂-Heteroalkyl, C₁-C₂-Haloalkyl, Aryl, Heteroaryl, NH₂, NHMe, NMe₂, die miteinander verknüpft sein können, um einen Ring auszubilden;
R^{b} aus CN, Aryl, Heteroaryl, -NO₂, -O-CC₁-C₂-Alkyl, -CO-O-C₁-C₂-Alkyl ausgewählt ist,
wobei Aryl und Heteroaryl optional durch ein oder mehrere C₁-C₂-Alkyle substituiert sind;
R^{c} aus H, Aryl, Heteroaryl, C₃-C₆-Cycloalkenyl, C₃-C₈-Cycloalkyl, Heterocyclus, C₁-C₈-Alkyl, C₁-C₈-Heteroalkyl, C₁-C₄-Alkylen-C₃-C₆-Cycloalkyl ausgewählt ist;
wobei ein beliebiges von diesen optional durch einen oder mehrere Substituenten substituiert sein kann, ausgewählt aus C(O)(R^{g}), C(O)N(R^{f})(R^{g}), Halogen, SFs, OH, =O, - COH, -COOH, -CO-O-R^{a}, CN, SMe, SO₂Me, SO₂R^{g}, C₁-C₄-Alkoxy, O-C₁-C₄-Haloalkyl, C₁-C₄-Alkyl, C₁-C₂-Heteroalkyl, C₁-C₄-Haloalkyl, N(R^{f})(R^{g}), N(R^{f})SO₂(R^{g}), SO₂NH₂, SO₂NHMe, SO₂NMe₂, S(O)(NR^{f})R^{g}, C₃-C₆-Cycloalkyl, Heterocyclus, C₁-C₄-Alkylen-C₃-C₆-Cycloalkyl, Aryl und Heteroaryl, die miteinander verknüpft sein können, um einen Ring auszubilden,
wobei Aryl, Heteroaryl, Heterocyclus und Cycloalkyl optional durch einen oder mehrere Substituenten substituiert sind, ausgewählt aus Halogen, =O, =NH, =NMe, OH, C₁-C₂-Alkyl, C₁-C₂-Alkoxy, -COOH, -COO-C₁-C₂-Alkyl, SO₂R^{a}, COR^{a}, SOR^{a}, S(O)(NH)R^{a}, CONHR^{a}, die miteinander verknüpft sein können, um einen Ring auszubilden;
R^{d} H oder C₁-C₄-Alkyl darstellt
und R^{c} und R^{d} miteinander verknüpft sein können, um einen Heterocyclus auszubilden;
R^{f} aus H, Aryl, Heteroaryl, C₃-C₆-Cycloalkenyl, C₃-C₈-Cycloalkyl, Heterocyclus, C₁-C₈-Alkyl, C₁-C₈-Heteroalkyl, C₁-C₄-Alkylen-C₃-C₆-Cycloalkyl ausgewählt ist,
wobei ein beliebiges von diesen optional durch einen oder mehrere Substituenten substituiert sein kann, ausgewählt aus Halogen, =O, =NH, =NMe, OH, Aryl, Heteroaryl, C₁-C₂-Alkyl, C₁-C₂-Haloalkyl, C₁-C₂-Alkoxy, O-C₁-C₂-Haloalkyl, -COOH, -COO-C₁-C₂-Alkyl, SO₂R^{a}, COR^{a}, SOR^{a}, S(O)(NH)R^{a}, CONHR^{a};
R^{g} aus H, Aryl, Heteroaryl, C₃-C₆-Cycloalkenyl, C₃-C₈-Cycloalkyl, Heterocyclus, C₁-C₈-Alkyl, C₁-C₈-Heteroalkyl, C₁-C₄-Alkylen-C₃-C₆-Cycloalkyl ausgewählt ist,
wobei ein beliebiges von diesen optional durch einen oder mehrere Substituenten substituiert sein kann, ausgewählt aus Halogen, =O, =NH, =NMe, OH, Aryl, Heteroaryl, C₁-C₂-Alkyl, C₁-C₂-Haloalkyl, C₁-C₂-Alkoxy, O-C₁-C₂-Haloalkyl, -COOH, -COO-C₁-C₂-Alkyl, SO₂R^{a}, COR^{a}, SOR^{a}, S(O)(NH)R^{a}, CONHR^{a}
R^{f} und R^{g} miteinander verknüpft sein können, um einen Ring auszubilden.

3. Verbindung nach Anspruch 1 oder 2 oder ein pharmazeutisch unbedenkliches Salz davon, die eine Verbindung der Formel (II) ist: 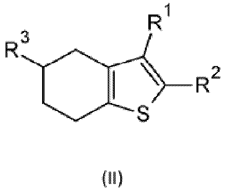 wobei R¹, R² und R³ wie in Anspruch 1 definiert sind.

4. Verbindung nach einem der Ansprüche 1 oder 2 oder ein pharmazeutisch unbedenkliches Salz davon, wobei X C oder N ist; Z C, N, O oder S ist; Y C oder S ist.

5. Verbindung nach einem der Ansprüche 1 oder 2 oder ein pharmazeutisch unbedenkliches Salz davon, wobei X, Y, W C ist und Z N ist, wobei N durch eine Gruppe substituiert ist, die aus Methylsulfonyl, Acetyl, Ethylcarbamoyl und Cyano ausgewählt ist.

6. Verbindung nach einem der Ansprüche 1 oder 2 oder ein pharmazeutisch akzeptables Salz davon, wobei X, Y, W, Z C ist und wobei Z durch eine Gruppe substituiert ist, die aus OH, Methyl, Ethylcarbamoyl, Cyano, Oxo, tert-Butoxycarbonylamino, 1H-Pyrazol-3-yl ausgewählt ist.

7. Verbindung nach Anspruch 1 oder 2 oder ein pharmazeutisch unbedenkliches Salz davon, wobei X, Y, W, Z C ist und wobei W durch eine Methylgruppe substituiert ist und R³ H oder eine Methylgruppe ist.

8. Verbindung nach einem der Ansprüche 1 bis 4 oder ein pharmazeutisch unbedenkliches Salz davon, wobei X C oder N ist und R³ aus H, OH, Methyl, Amino, Methylsulfonyl, Acetyl, 2-Methoxyacetyl, 2-Phenylacetyl, 2-(tert-butoxycarbonylamino)ethyl, 2-Phenylethyl, Pyrimidin-2-ylamino, Methansufonamido, (4-Methoxyphenyl)carbamothioylamino, (4-Methoxyphenyl)carbamoylamino, Ethylcarbamoyl, [*N*'-Cyano-*N*-(p-tolyl)carbamimidoyl]amino, [*N*'-Cyano-*N*-ethyl-carbamimidoyl]amino, (1-Oxoisoindolin-5-yl)carbamoyl, (3-Sulfamoylphenyl)carbamoyl, (3-Methylsulfonylphenyl)carbamoyl, 3-Isoxazol-5-ylanilino, (2-Ethoxy-3,4-dioxo-cyclobuten-1-yl)amino, Ethylcarbonyl, Carboxy, (1-Oxoisoindolin-5-yl)aminocarbonyl, [(5-Oxopyrrolidin-3-yl)phenyl]aminocarbonyl, [3-(3-Methyl-5-oxo-4H-pyrazol-1-yl)phenyl]carbonylamino, (1H-Indazol-4-yl)carbonylamino, 1H-Imidazo[4,5-b]pyrazin-2-yl, Indan-2-yl, [2-(4-Methoxyphenyl)ethylaminocarbonyl, Chinoxalin-6-yl, (Thiazol-2-yl)aminocarbonyl, (1H-Tetrazol-5-yl)aminocarbonyl, (4-Pyridyl)aminocarbonyl, Pyrazin-2-ylaminocarbonyl, (3-pyridyl)aminocarbonyl, 3-Hydroxyazetidine-1-carbonyl, 1,1-Dioxo-1,4-thiazinan-4-carbonyl, 2-(4-Hydroxyphenyl)ethylaminocarbonyl, Hydroxyiminomethyl, [(4-Ethyl-1,2,4-triazol-3-yl)amino]methyl, (4-Methylanilino)methyl, 2-Amino-3-pyridyl, 5-(p-Tolyl)-1H-imidazol-2-yl, Cyano, Trifluormethyl, 4-Methyloxazol-2-yl, Aminomethyl, (p-Tolylcarbamoylamino)methyl, 3-Methyl-1,2,4-oxadiazol-5-yl, 5-Methyl-1,3,4-oxadiazol-2-yl, Pyrrolidin-1-carbonyl, 3-Pyridylcarbamothioylamino, 3-(3-Pyridylamino)-1,2,4-triazol-4-yl, 1H-Pyrazol-3-yl, 5-Amino-4-cyano-imidazol-1-yl, 3-(2,2-Dimethylpropylamino)-1,2,4-triazol-4-yl, 3-[(2,5-Dimethylpyrazol-3-yl)amino]-1,2,4-triazol-4-yl, 4-(2,5-Dimethylpyrazol-3-yl)-1,2,4-triazol-3-yl]amino, [(2,5-Dimethylpyrazol-3-yl)amino]-1,2,4-triazol-4-yl, N'-Cyano-N-phenyl-carbamimidoylamino, 3-(Isobutylamino)-1,2,4-triazol-4-yl, 3-[(1-Methylcyclopropyl)methylamino]-1,2,4-triazol-4-yl, 5-Acetamidoimidazol-1-yl, 3-[(5-Methyl-3-pyridyl)amino]-1,2,4-triazol-4-yl, 3-(4-Pyridylamino)-1,2,4-triazol-4-yl, N'-Cyano-N-(3,4-dimethoxyphenyl)carbamimidoyl]amino, 4-Ethoxycarbony-5-(ethylamino)imidazol-1-yl, 3-(2-morpholinoethylamino)-1,2,4-triazol-4-yl, (5-Oxo-4H-1,2,4-oxadiazol-3-yl)amino, 2-(Ethylamino)-3,4-dioxo-cyclobuten-1-yl]amino, [N'-Cyano-N-(2,2,2-trifluorethyl)carbamimidoyl]amino, 3-(Ethylamino)-1,2,4-triazol-4-yl, (N-Ethyl-N'-nitro-carbamimidoyl)amino, 3-(3-Pyridylamino)-1,2,4-triazol-4-yl, 3-[(1-Oxidopyridin-1-ium-3-yl)amino, [N'-Nitro-N-(p-tolyl)carbamimidoyl]amino, 2-(3-Pyridylamino)imidazol-1-yl, 3-(3-Pyridylamino)-1,2,4-triazol-4-yl, 3-(Cyclopropylmethylamino, (2,5-Dimethylpyrazol-3-yl)carbamothioylamino, 3-(Pyrimidin-5-ylamino)-1,2,4-triazol-4-yl, 2-(Cyclopropylmethylamino)imidazol-1-yl, [4-(Ethylamino)-1,1-dioxo-1,2,5-thiadiazol-3-yl]amino, [1-(4-Methylanilino)-2-nitro-vinyl]amino, (N'-Cyano-N-ethyl-carbamimidoyl)-methyl-amino, [2-(4-Methylanilino)-3,4-dioxo-cyclobuten-1-yl]amino, 3-(2-Pyridylamino)-1,2,4-triazol-4-yl, 2-Acetamidoimidazol-1-yl, 5-(N-tert-Butyloxycarbonylamino)-triazol-1-yl, 5-(3-Pyridylamino)triazol-1-yl, Ethylcarbamothioylamino, 3-[[(3S)-Tetrahydrofuran-3-yl]amino]-1,2,4-triazol-4-yl, [(E)-N-Ethyl-N'-nitro-carbamimidoyl]amino, 2-(Ethylamino)imidazol-1-yl, 3-[(3,5-Dimethylisoxazol-4-yl )amino, [[N'-Ethoxycarbonyl-N-(p-tolyl)carbamimidoyl]amino], [2-[(2-Methyl-3-pyridyl)amino]imidazol-1-yl, 1-[(Ethylamino)-2-nitro-vinyl]amino, (4-Methyl-6-oxo-1H-pyrimidin-2-yl)amino, 5-Amino-4-(methylcarbamoyl)imidazol-1-yl, 5-Amino-4-carbamoyl-imidazol-1-yl, 2-Anilinoimidazol-1-yl, Ethylcarbamoylamino, (4-Oxo-1H-pyridin-2-yl)amino, [6-(5-Methyl-1,3,4-oxadiazol-2-yl)-3-pyridyl]amino, 5-(Ethylamino)tetrazol-1-yl, 1H-1,2,4-triazol-3-ylamino, 4-(1-Methylpyrazol-4-yl)-1,2,4-triazol-3-yl]amino, 4-(Ethyloxy-carbonyl)imidazol-1-yl, [4-(4-Fluorphenyl)-1,2,4-triazol-3-yl]amino, 1H-Imidazol-2-ylamino, N'-Methoxy-N-(p-tolyl)carbamimidoyl]amino, [4-(4-Methylanilino)-1,1-dioxo-1,2,5-thiadiazol-3-yl]amino, [1-(3-Pyridyl)imidazol-2-yl]amino, 5-Aminotriazol-1-yl, (2-Methoxyphenyl)carbamoylamino, 4-Pyridin-carbonylamino, Benzylamino, 1H-Benzimidazol-2-ylamino, [N'-Hydroxycarbamimidoyl]amino, [N'-(1-Methylpyrazol-3-yl)-N-(p-tolyl)carbamimidoyl]amino, 3-[(3,5-Dimethylpyrazin-2-yl)amino, 3-(Ethylamino)-5-(methoxymethyl)-1,2,4-triazol-4-yl, [5-(3-Pyridyl)-1H-1,2,4-triazol-3-yl]amino, p-Tolylcarbonylamino, Chinazolin-2-ylamino, 1,3-Benzoxazol-2-ylamino, 3-(Ethylamino )-5-(hydroxymethyl)-1,2,4-triazol-4-yl, 2-Aminoimidazol-1-yl, 3-Amino-5-oxo-1,2,4-oxadiazol-4-yl, 3-(Ethylamino)-5-methyl-1,2,4-triazol-4-yl, (2-Ethyl-3,4-dioxo-cyclobuten-1-yl)amino, 1,3,4-Oxadiazol-2-ylamino, 5-Oxo-1-(p-tolyl)-4H-imidazol-2-yl]amino, [2-[Ethyl(methyl)amino]-3,4-dioxo-cyclobuten-1-yl]amino, (Methylsulfonimidoyl)amino, [N-Cyano-C-methyl-carbonimidoyl]amino, Tetrazol-1-yl, Methansulfinamido, Morpholino, 3-(Ethylamino)-5-(trifluormethyl)-1,2,4-triazol-4-yl, (4-Oxo-1H-pyrimidin-2-yl)amino, Isopropylsulfamoylamino, N'-Cyano-N-morpholino-carbamimidoyl]amino, Acetamido, Methanesulfonamido, [N'-Cyano-N-methoxy-carbamimidoyl]amino, (4-Methoxyphenyl)carbamoylamino, [4-Oxo-6-(trifluormethyl)-1H-pyrimidin-2-yl]amino, [4-(Trifluormethoxy)phenyl]carbamoylamino, 3-(2-Methylpropanoylamino)-1,2,4-triazol-4-yl, 3-[(2,5-Dimethylpyrazol-3-yl)-methyl-amino]-1,2,4-triazol-4-yl, 5-Amino-4-(5-methyl-1,3,4-oxadiazol-2-yl)imidazol-1-yl, 3-[(3-Methyl-1H-pyrazol-5-yl)amino]-1,2,4-triazol-4-yl, 3-[(1,3-Dimethylpyrazol-4-yl)amino]-1,2,4-triazol-4-yl, -[(5-Cyclopropyl-2-methyl-pyrazol-3-yl)amino]-1,2,4-triazol-4-yl, 3-[(5-Isopropyl-2-methyl-pyrazol-3-yl)amino]-1,2,4-triazol-4-yl, 3-[[2-Methyl-5-(trifluormethyl)pyrazo1-3-yl]amino]-1,2,4-triazol-4-yl, 3-[(2-Isopropyl-5-methyl-pyrazol-3-yl)amino, 3-[(5-Methyl-2-tetrahydrofuran-3-yl-pyrazol-3-yl)amino]-1,2,4-triazol-4-yl, 3-[(2-Methylpyrazol-3-yl)amino]-1,2,4-triazol-4-yl, 3-[(2-Ethyl-5-methyl-pyrazol-3-yl)amino]-1,2,4-triazol-4-yl, 3-[(2-Cyclopropyl-5-methyl-pyrazo1-3-yl)amino]-1,2,4-triazol-4-yl, 3-[(3-Methoxy-1-methyl-pyrazol-4-yl)amino]-1,2,4-triazol-4-yl, 3-[(5-Ethyl-2-methyl-pyrazol-3-yl)amino]-1,2,4-triazol-4-yl, 3-[[5-(Difluormethyl)-2-methyl-pyrazol-3-yl]amino, 3-[(1,4-Dimethyl pyrazol-3-yl)amino]-1,2,4-triazol-4-yl, [3-[(5-Methoxycarbonyl-2-methyl-pyrazol-3-yl)amino]-1,2,4-triazol-4-yl], 2-[[2-Methyl-5-(trifluormethyl)pyrazol-3-yl]amino]imidazol-1-yl, [3-[(5-Carboxy-2-methyl-pyrazol-3-yl)amino]-1,2,4-triazol-4-yl] ausgewählt ist.

9. Verbindungen nach Anspruch 1 oder ein pharmazeutisch unbedenkliches Salz davon, wobei W, X, Y, Z C ist und Z optional durch eine Methylgruppe substituiert ist.

10. Verbindungen nach den Ansprüchen 1 bis 4 oder ein pharmazeutisch unbedenkliches Salz davon, wobei R³ aus (1,1-Dioxothiolan-3-yl)amino-1,2,4-triazol-4-yl, 3-[3-[Methylsulfonimidoyl]anilino]-1,2,4-triazol-4-yl, 3-[(1-Acetyl-3-methylpyrrolidin-3-yl)amino]-4H-1,2,4-triazol-4-yl, 3-{[1-(6-Methylpyridazin-3-yl)pyrrolidin-3-yl]amino}-4H-1,2,4-triazol-4-yl, 3-[[-1-Pyrazin-2-ylpyrrolidin-3-yl]amino]-1,2,4-triazol-4-yl, 3-[(4-Methyl-1-methylsulfonyl-pyrrolidin-3-yl)amino]-1,2,4-triazol-4-yl, 3-[(1-Oxo-2,3-dihydro-1H-isoindol-4-yl)amino]-4H-1,2,4-triazol-4-yl, 3-[[-1-(6-Fluorpyridazin-3-yl)pyrrolidin-3-yl]amino]-1,2,4-triazol-4-yl, 3-[[1-Pyrimidin-2-ylpyrrolidin-3-yl]amino]-1,2,4-triazol-4-yl, 5-Amino-4-(2-methoxyethoxycarbonyl)imidazol-1-yl, 5-Amino-4-(2-morpholinethoxycarbonyl)imidazol-1-yl, 3-(3-Methylsulfanylanilino)-1,2,4-triazol-4-yl, 3-(3-Methylsulfonylanilino)-1,2,4-triazol-4-yl, 3-(3-Methylsulfinylanilino)-1,2,4-triazol-4-yl, 3-[3-(Methylsulfonimidoyl)anilino]-1,2,4-triazol-4-yl, 3-[(6-Methylsulfinyl-2-pyridyl)amino]-1,2,4-triazol-4-yl, 3-[(6-Methylsulfonyl-2-pyridyl)amino]-1,2,4-triazol-4-yl, 8-Methoxy-4-oxo-5,10-dihydroimidazo[4,5-c][1,5]benzodiazepin-1-yl, 8-Methoxy-5-methyl-4-oxo-10H-imidazo[4,5-c][1,5]benzodiazepin-1-yl, 5-Amino-4-[(2S)-2-methylpyrrolidin-1-carbonyl]imidazol-1-yl, 5-Amino-4-[(3R)-3-hydroxypyrrolidin-1-carbonyl]imidazol-1-yl, 5-Amino-4-[(2S)-2-methylpyrrolidin-1-carbonyl]imidazol-1-yl, 5-[[4-(Dihydroxyamino)-2,5-dimethyl-3H-pyrazol-3-yl]amino]-4-ethoxycarbonyl-imidazol-1-yl, 5-[[4-(Dihydroxyamino)-2,5-dimethyl-3H-pyrazol-3-yl]amino]-4-ethoxycarbonyl-imidazol-1-yl, 3-(2-Methyl-3-methylsulfinyl-anilino)-1,2,4-triazol-4-yl, 3-(2-Methyl-3-methylsulfonyl-anilino)-1,2,4-triazol-4-yl, 3-[(2-Methyl-5-oxazol-2-yl-pyrazol-3-yl)amino]-1,2,4-triazol-4-yl, 1-Acetylpyrrolidin-3-yl]amino]-1,2,4-triazol-4-yl, 1-Methylsulfonylpyrrolidin-3-yl]amino]-1,2,4-triazol-4-yl, 3-[(2-Methylcyclohexyl)amino]-1,2,4-triazol-4-yl, 3-[(5-Oxopyrrolidin-3-yl)amino]-1,2,4-triazol-4-yl, 3-[(2-Oxo-4-piperidyl)amino]-1,2,4-triazol-4-yl, 3-[(1-Acetyl-2-methyl-pyrrolidin-3-yl)amino]-1,2,4-triazol-4-yl, 3-[(2-Methyl-1-methylsulfonyl-pyrrolidin-3-yl)amino]-1,2,4-triazol-4-yl, -[3-[[3-(6-Methyl-3-pyridyl)cyclobutyl]amino]-1,2,4-triazol-4-yl, 3-[[5-(Difluormethoxy)-2-methyl-pyrazol-3-yl]amino]-1,2,4-triazol-4-yl, [[1-Benzoyl-4,4-difluor-pyrrolidin-3-yl]amino]-1,2,4-triazol-4-yl, 3-[(1-Acetyl-4-piperidyl)amino]-1,2,4-triazol-4-yl, 3-[(1-Methoxycarbonylpyrrolidin-3-yl)amino]-1,2,4-triazol-4-yl, 3-[[1-(Ethylcarbamoyl)pyrrolidin-3-yl]amino]-1,2,4-triazol-4-yl, 3-{[2-(Pyrimidin-2-yl)-2-azaspiro[4.4]nonan-7-yl]amino}-4H-1,2,4-triazol-4-yl, 3-[[2-Methyl-5-(trifluormethoxy)pyrazol-3-yl]amino]-1,2,4-triazol-4-yl, 3-[[5-[Chlor(difluor)methoxy]-2-methyl-pyrazol-3-yl]amino]-1,2,4-triazol-4-yl, 3-{[1-Methyl-3-(5-methyl-1,3,4-oxadiazol-2-yl)-1H-pyrazol-5-yl]amino}-4H-1,2,4-triazol-4-yl, 3-[(5-Methoxy-3-methyl-pyrazin-2-yl)amino]-1,2,4-triazol-4-yl, (5-Methylthiazolo[5,4-b]pyridin-2-yl)amino, 3-[(5-Cyano-2-methyl-3-pyridyl)amino]-1,2,4-triazol-4-yl, 3-[(6-Cyano-2-methyl-3-pyridyl)amino]-1,2,4-triazol-4-yl, 3-[(1-Methyl-2-oxo-4-pyridyl)amino]-1,2,4-triazol-4-yl, 3-[(6-Chlor-2-methyl-3-pyridyl)amino]-1,2,4-triazol-4-yl, 3-[[2-Methyl-6-(trifluormethyl)-3-pyridyl]amino]-1,2,4-triazol-4-yl, 3-[(3-Methylpyridazin-4-yl)amino]-1,2,4-triazol-4-yl, 3-[(5-Chlor-2-methyl-pyrazol-3-yl)amino]-1,2,4-triazol-4-yl, 3-[(3-Methylisothiazol-4-yl)amino]-1,2,4-triazol-4-yl, 3-[(3-Methylisoxazol-4-yl)amino]-1,2,4-triazol-4-yl, 3-[[5-(Cyclopropylmethoxy)-2-methyl-pyrazol-3-yl]amino]-1,2,4-triazol-4-yl, 3-[(5-Ethoxy-2-methyl-pyrazol-3-yl)amino]-1,2,4-triazol-4-yl, 3-[(2,5-Dimethylpyrazol-3-yl)amino]-1,2,4-triazol-4-yl, (5-Amino-4-ethoxycarbonyl-imidazol-1-yl), 3-[[5-(Difluormethoxy)-2-methylpyrazol-3-yl]amino]-1,2,4-triazol-4-yl, 3-[[5-(Difluormethyl)-2-methylpyrazol-3-yl]amino]-1,2,4-triazol-4-yl, 3-[(5-Methoxy-2-methylpyrazol-3-yl)amino]-1,2,4-triazol-4-yl, 3-[[5-(Difluormethyl)-2-(trideuteriomethyl)pyrazol-3-yl]amino, 3-[(5-Cyano-2-methyl-pyrazol-3-yl)amino]-1,2,4-triazol-4-yl, 3-[(2,2-Difluor-1,3-benzodioxol-5-yl)amino]-1,2,4-triazol-4-yl, 3-[(5-Methoxy-2-methyl-pyrazol-3-yl)amino]-1,2,4-triazol-4-yl, 3-[[5-(Difluormethyl)-2-(trideuteriomethyl)pyrazol-3-yl]amino]-1,2,4-triazol-4-yl, 3-[(6-Fluor-2-methyl-3-pyridyl)amino]-1,2,4-triazol-4-yl, 3-[[5-(Cyclopropoxy)-2-methyl-pyrazol-3-yl]amino]-1,2,4-triazol-4-yl, 3-[[2-Methyl-5-(2,2,2-trifluorethoxy)pyrazol-3-yl]amino]-1,2,4-triazol-4-yl, 3-[[6-(Trifluormethyl)-3-pyridyl]amino]-1,2,4-triazol-4-yl, 3-[(6-Methoxy-3-pyridyl)amino]-1,2,4-triazol-4-yl, 3-[(2-Methoxy-4-pyridyl)amino]-1,2,4-triazol-4-yl, 3-[(6-Chlor-3-pyridyl)amino]-1,2,4-triazol-4-yl, 3-[(2-Chlor-4-pyridyl)amino]-1,2,4-triazol-4-yl, 3-[(6-Methoxy-2-methyl-3-pyridyl)amino]-1,2,4-triazol-4-yl, Thiazolo[5,4-b]pyridin-2-ylamino, 3-[(3-Methyltriazol-4-yl)amino]-1,2,4-triazol-4-yl.

11. Verbindung nach Anspruch 1 oder ein pharmazeutisch unbedenkliches Salz davon, wobei die Verbindung ausgewählt ist aus:
2-(Cyclopropancarbonylamino)-*N*-(cyclopropylmethyl)-4,5,6,7-tetrahydrothieno[3,2-*c*]pyridin-3-carboxamid; Hydrochlorid;
2-(Cyclopropancarbonylamino)-*N*-(cyclopropylmethyl)-5-methylsulfonyl-6,7-dihydro-4*H-*thieno[3,2-*c*]pyridin-3-carboxamid;
5-Acetyl-2-(Cyclopropancarbonylamino)-*N*-(cyclopropylmethyl)-6,7-dihydro-4*H*-thieno[3,2-*c*]pyridin-3-carboxamid;
2-(Cyclopropancarbonylamino)-*N*-(cyclopropylmethyl)-7-methylsulfonyl-5,6-dihydro-4*H-*thieno[2,3-*b*]pyridin-3-carboxamid;
7-Acetyl-2-( Cyclopropancarbonylamino)-*N*-(cyclopropylmethyl)-5,6-dihydro-4*H-*thieno[2,3-*b*]pyridin-3-carboxamid;
2-(Cyclopropancarbonylamino)-*N*-(cyclopropylmethyl)-5-(2-methoxyacetyl)-6,7-dihydro-4*H*-thieno[3,2-*c*]pyridin-3-carboxamid;
2-(Cyclopropancarbonylamino)-*N*-(cyclopropylmethyl)-5-(2-phenylacetyl)-6,7-dihydro-4*H-*thieno[3,2-*c*]pyridin-3-carboxamid;
*Tert*-Butyl-*N*-[2-[2-(cyclopropanecarbonylamino)-3-(cyclopropylmethylcarbamoyl)-6,7-dihydro-4*H*-thieno[3,2-*c*]pyridin-5-yl]ethyl]carbamat;
2-(Cyclopropancarbonylamino)-*N*-(cyclopropylmethyl)-5-(2-phenylethyl)-6,7-dihydro-4*H-*thieno[3,2-*c*]pyridin-3-carboxamid;
Methyl-2-[2-(Cyclopropancarbonylamino)-3-(cyclopropylmethylcarbamoyl)-6,7-dihydro-4*H-*thieno[3,2-*c*]pyridin-5-yl]acetat;
(4*R*)-2-(Cyclopropancarbonylamino)-*N*-(cyclopropylmethyl)-4-methyl-5,6-dihydro-4*H-*cyclopenta[*b*]thiophen-3-carboxamid;
(4*S*)-2-(Cyclopropancarbonylamino)-*N*-(cyclopropylmethyl)-4-methyl-5,6-dihydro-4*H-*cyclopenta[*b*]thiophen-3-carboxamid;
Ethyl-2-(cyclopropanecarbonylamino)-3-(cyclopropylmethylcarbamoyl)-4,5,6,7-tetrahydrobenzothiophen-5-carboxylat;
2-(Cyclopropancarbonylamino)-3-(cyclopropylmethylcarbamoyl)-4,5,6,7-tetrahydrobenzothiophen-5-carbonsäure;
2-(Cyclopropancarbonylamino)-N3-(cyclopropylmethyl)-N5-(1-oxoisoindolin-5-yl)-4,5,6,7-tetrahydrobenzothiophen-3,5-dicarboxamid;
2-(Cyclopropancarbonylamino)-N3-(cyclopropylmethyl)-N5-[3-(5-oxopyrrolidin-3-yl)phenyl]-4,5,6,7-tetrahydrobenzothiophen-3,5-dicarboxamid;
2-(Cyclopropancarbonylamino)-N3-(cyclopropylmethyl)-N5-[3-(3-methyl-5-oxo-4H-pyrazol-1-yl)phenyl]-4,5,6,7-tetrahydrobenzothiophen-3,5-dicarboxamid;
2-(Cyclopropancarbonylamino)-N3-(cyclopropylmethyl)-N5-(1H-indazol-4-yl)-4,5,6,7-tetrahydrobenzothiophen-3,5-dicarboxamid; 2,2,2-Trifluoressigsäure;
2-(Cyclopropancarbonylamino)-N3-(cyclopropylmethyl)-N5-(1H-indazol-5-yl)-4,5,6,7-tetrahydrobenzothiophen-3,5-dicarboxamid; 2,2,2-Trifluoressigsäure;
2-(Cyclopropancarbonylamino)-N-(cyclopropylmethyl)-5-(1H-imidazo[4,5-b]pyrazin-2-yl)-4,5,6,7-tetrahydrobenzothiophen-3-carboxamid;
2-(Cyclopropancarbonylamino)-N3-(cyclopropylmethyl)-N5-indan-2-yl-4,5,6,7-tetrahydrobenzothiophen-3,5-dicarboxamid;
2-(Cyclopropancarbonylamino)-N3-(cyclopropylmethyl)-N5-[2-(4-methoxyphenyl)ethyl]-4,5,6,7-tetrahydrobenzothiophen-3,5-dicarboxamid;
2-(Cyclopropancarbonylamino)-N3-(cyclopropylmethyl)-N5-chinoxalin-6-yl-4,5,6,7-tetrahydrobenzothiophen-3,5-dicarboxamid;
2-(Cyclopropancarbonylamino)-N3-(cyclopropylmethyl)-N5-thiazol-2-yl-4,5,6,7-tetrahydrobenzothiophen-3,5-dicarboxamid;
2-(Cyclopropancarbonylamino)-N3-(cyclopropylmethyl)-N5-(1H-tetrazol-5-yl)-4,5,6,7-tetrahydrobenzothiophen-3,5-dicarboxamid;
2-(Cyclopropancarbonylamino)-N3-(cyclopropylmethyl)-N5-(4-Pyridyl)-4,5,6,7-5-tetrahydrobenzothiophen-3,5-dicarboxamid;
2-(Cyclopropancarbonylamino)-N3-(cyclopropylmethyl)-N5-pyrazin-2-yl-4,5,6,7-tetrahydrobenzothiophen-3,5-dicarboxamid;
2-(Cyclopropancarbonylamino)-N3-(cyclopropylmethyl)-N5-(3-pyridyl)-4,5,6,7-tetrahydrobenzothiophen-3,5-dicarboxamid;
2-(Cyclopropancarbonylamino)-N-(cyclopropylmethyl)-5-(3-hydroxyazetidin-1-carbonyl)-4,5,6,7-tetrahydrobenzothiophen-3-carboxamid;
2-(Cyclopropancarbonylamino)-N-(cyclopropylmethyl)-5-(1,1-dioxo-1,4-thiazinan-4-carbonyl)-4,5,6,7-tetrahydrobenzothiophen-3-carboxamid;
2-(Cyclopropancarbonylamino)-N3-(cyclopropylmethyl)-N5-[2-(4-hydroxyphenyl)ethyl]-4,5,6,7-tetrahydrobenzothiophen-3,5-dicarboxamid;
2-(Cyclopropancarbonylamino)-N-(cyclopropylmethyl)-5-(hydroxyiminomethyl)-4,5,6,7-tetrahydrobenzothiophen-3-carboxamid;
2-(Cyclopropancarbonylamino)-N-(cyclopropylmethyl)-5-[[(4-ethyl-1,2,4-triazol-3-yl)amino]methyl]-4,5,6,7-tetrahydrobenzothiophen-3-carboxamid;
2-(Cyclopropancarbonylamino)-N-(cyclopropylmethyl)-5-hydroxy-5-[(4-methylanilino)methyl]-6,7-dihydro-4H-benzothiophen-3-carboxamid;
2-(Cyclopropancarbonylamino)-N-(cyclopropylmethyl)-2'-oxo-3'-(p-tolyl)spiro[6,7-dihydro-4H-benzothiophen-5,5'-oxazolidin]-3-carboxamid;
5-(2-Amino-3-pyridyl)-2-(cyclopropancarbonylamino)-N-(cyclopropylmethyl)-4,5,6,7-tetrahydrobenzothiophen-3-carboxamid;
2-(Cyclopropancarbonylamino)-N-(cyclopropylmethyl)-5-[5-(p-tolyl)-1H-imidazol-2-yl]-4,5,6,7-tetrahydrobenzothiophen-3-carboxamid;
5-Cyano-2-(cyclopropanecarbonylamino)-N-(cyclopropylmethyl)-4,5,6,7-tetrahydrobenzothiophen-3-carboxamid;
7-Cyano-2-(cyclopropancarbonylamino)-N-(cyclopropylmethyl)-4,5,6,7-tetrahydrobenzothiophen-3-carboxamid;
(5R)-2-(Cyclopropancarbonylamino)-N-(cyclopropylmethyl)-5-(trifluormethyl)-4,5,6,7-tetrahydrobenzothiophen-3-carboxamid;
(5S)-2-(Cyclopropancarbonylamino)-N-(cyclopropylmethyl)-5-(trifluormethyl)-4,5,6,7-tetrahydrobenzothiophen-3-carboxamid;
2-(Cyclopropancarbonylamino)-N-(cyclopropylmethyl)-5-(4-methyloxazol-2-yl)-4,5,6,7-tetrahydrobenzothiophen-3-carboxamid;
5-(Aminomethyl)-2-(cyclopropancarbonylamino)-N-(cyclopropylmethyl)-5-hydroxy-6,7-dihydro-4H-benzothiophen-3-carboxamid;
2-(Cyclopropancarbonylamino)-N-(cyclopropylmethyl)-5-hydroxy-5-[(p-tolylcarbamoylamino)methyl]-6,7-dihydro-4H-benzothiophen-3-carboxamid;
2-(Cyclopropancarbonylamino)-N-(cyclopropylmethyl)-5-(3-methyl-1,2,4-oxadiazol-5-yl)-4,5,6,7-tetrahydrobenzothiophen-3-carboxamid;
2-(Cyclopropancarbonylamino)-N-(cyclopropylmethyl)-5-(5-methyl-1,3,4-oxadiazol-2-yl)-4,5,6,7-tetrahydrobenzothiophen-3-carboxamid;
2-(Cyclopropancarbonylamino)-N-(cyclopropylmethyl)-5-(pyrrolidin-1-carbonyl)-4,5,6,7-tetrahydrobenzothiophen-3-carboxamid;
2-(Cyclopropancarbonylamino)-N-(cyclopropylmethyl)-5-hydroxy-5-methyl-6,7-dihydro-4H-benzothiophen-3-carboxamid;
2-(Cyclopropancarbonylamino)-N-(cyclopropylmethyl)-4-methyl-4,5,6,7-tetrahydrobenzothiophen-3-carboxamid;
2-(Cyclopropancarbonylamino)-N-(cyclopropylmethyl)-5,7-dimethyl-4,5,6,7-tetrahydrobenzothiophen-3-carboxamid;
2-(Cyclopropancarbonylamino)-N-(cyclopropylmethyl)-5-methyl-4,5,6,7-tetrahydrobenzothiophen-3-carboxamid;
2-(Cyclopropancarbonylamino)-N-(cyclopropylmethyl)-7-methyl-4,5,6,7-tetrahydrobenzothiophen-3-carboxamid;
2-(Cyclopropancarbonylamino)-N-(cyclopropylmethyl)-5,5-dimethyl-6,7-dihydro-4H-benzothiophen-3-carboxamid;
2-(Cyclopropancarbonylamino)-N-(cyclopropylmethyl)-5-methyl-7-oxo-5,6-dihydro-4H-benzothiophen-3-carboxamid;
2'-(Cyclopropancarbonylamino)-N-(cyclopropylmethyl)spiro[1,3-dioxolan-2,5'-6,7-dihydro-4H-benzothiophen]-3'-carboxamid;
2-(Cyclopropancarbonylamino)-N-(cyclopropylmethyl)-4,5-dimethyl-4,5,6,7-tetrahydrobenzothiophen-3-carboxamid;
2-(Cyclopropancarbonylamino)-N-(cyclopropylmethyl)-2',5'-dioxo-spiro[6,7-dihydro-4H-benzothiophen-5,4'-imidazolidin]-3-carboxamid;
tert-Butyl-N-[2-(cyclopropancarbonylamino)-3-(cyclopropylmethylcarbamoyl)-4,5,6,7-tetrahydrobenzothiophen-7-yl]carbamat;
2-(Cyclopropancarbonylamino)-N3-(cyclopropylmethyl)-N7-ethyl-4,5,6,7-tetrahydrobenzothiophen-3,7-dicarboxamid;
2-(Cyclopropancarbonylamino)-N-(cyclopropylmethyl)-5-(1H-pyrazol-3-yl)-4,5,6,7-tetrahydrobenzothiophen-3-carboxamid;
2-(Cyclopropancarbonylamino)-N-(cyclopropylmethyl)-7-(1H-pyrazol-3-yl)-4,5,6,7-tetrahydrobenzothiophen-3-carboxamid;
(5S)-5-(5-Amino-4-cyano-imidazol-1-yl)-2-(cyclopropancarbonylamino)-N-(cyclopropylmethyl)-4,5,6,7-tetrahydrobenzothiophen-3-carboxamid;
(5S)-2-(Cyclopropancarbonylamino)-N-(cyclopropylmethyl)-5-[3-(2,2-dimethylpropylamino)-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[(2,5-dimethylpyrazol-3-yl)amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[[4-(2,5-dimethylpyrazol-3-yl)-1,2,4-triazol-3-yl]amino]-4,5,6,7-tetrahydrobenzothiophen-3-carboxamid;
5-Amino-2-(cyclopropancarbonylamino)-N-(cyclopropylmethyl)-4,5,6,7-tetrahydrobenzothiophen-3-carboxamid;
5-[[N'-Cyano-N-phenyl-carbamimidoyl]amino]-2-(cyclopropancarbonylamino)-N-(cyclopropylmethyl)-4,5,6,7-tetrahydrobenzothiophen-3-carboxamid;
(5S)-2-(Cyclopropancarbonylamino)-N-(cyclopropylmethyl)-5-[3-(isobutylamino)-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[(1-methylcyclopropyl)methylamino]-1,2,4-triazol-4-yl]-4,5,6,7-tetrahydrobenzothiophen-3-carboxamid;
(5S)-5-(5-Acetamidoimidazol-1-yl)-2-(cyclopropancarbonylamino)-N-(cyclopropylmethyl)-4,5,6,7-tetrahydrobenzothiophen-3-carboxamid;
(5S)-2-(Cyclopropancarbonylamino)-N-(cyclopropylmethyl)-5-[3-[(5-methyl-3-pyridyl)amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-(4-pyridylamino)-1,2,4-triazol-4-yl]-4,5,6,7-tetrahydrobenzothiophen-3-carboxamid;
5-[[N'-Cyano-N-(3,4-dimethoxyphenyl)carbamimidoyl]amino]-2-(cyclopropancarbonylamino)-N-(cyclopropylmethyl)-4,5,6,7-tetrahydrobenzothiophen-3-carboxamid;
Ethyl-5-amino-1-[(5S)-2-(cyclopropancarbonylamino)-3-(cyclopropylmethylcarbamoyl)-4,5,6,7-tetrahydrobenzothiophen-5-yl]imidazol-4-carboxylat;
(5S)-2-(Cyclopropancarbonylamino)-N-(cyclopropylmethyl)-5-[3-(2-morpholinoethylamino)-1,2,4-triazol-4-yl]-4,5,6,7-tetrahydrobenzothiophen-3-carboxamid;
2-(Cyclopropancarbonylamino)-N-(cyclopropylmethyl)-5-[(5-oxo-4H-1,2,4-oxadiazol-3-yl)amino]-4,5,6,7-tetrahydrobenzothiophen-3-carboxamid;
2-(Cyclopropancarbonylamino)-N-(cyclopropylmethyl)-5-[[2-(ethylamino)-3,4-dioxo-cyclobuten-1-yl]amino]-4,5,6,7-tetrahydrobenzothiophen-3-carboxamid;
5-[[N'-Cyano-N-(2,2,2-trifluorethyl)carbamimidoyl]amino]-2-(cyclopropancarbonylamino)-N-(cyclopropylmethyl)-4,5,6,7-tetrahydrobenzothiophen-3-carboxamid;
(5S)-2-(Cyclopropancarbonylamino)-N-(cyclopropylmethyl)-5-[3-(ethylamino)-1,2,4-triazol-4-yl]-4,5,6,7-tetrahydrobenzothiophen-3-carboxamid;
2-(Cyclopropancarbonylamino)-N-(cyclopropylmethyl)-5-[(N-ethyl-N'-nitro-carbamimidoyl)amino]-4,5,6,7-tetrahydrobenzothiophen-3-carboxamid;
(5S)-2-(Cyclopropancarbonylamino)-N-(cyclopropylmethyl)-5-[3-(3-pyridylamino)-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[(1-oxidopyridin-1-ium-3-yl)amino]-1,2,4-triazol-4-yl]-4,5,6,7-tetrahydrobenzothiophen-3-carboxamid;
2-(Cyclopropancarbonylamino)-N-(cyclopropylmethyl)-5-[[N'-nitro-N-(p-tolyl)carbamimidoyl]amino]-4,5,6,7-tetrahydrobenzothiophen-3-carboxamid;
(5S)-2-(Cyclopropancarbonylamino)-N-(cyclopropylmethyl)-5-[2-(3-pyridylamino)imidazol-1-yl]-4,5,6,7-tetrahydrobenzothiophen-3-carboxamid;
(5S)-2-(Cyclopropancarbonylamino)-N-(cyclopropylmethyl)-5-[3-(cyclopropylmethylamino)-1,2,4-triazol-4-yl]-4,5,6,7-tetrahydrobenzothiophen-3-carboxamid;
(5S)-2-(Cyclopropancarbonylamino)-N-(cyclopropylmethyl)-5-[(2,5-dimethylpyrazol-3-yl)carbamothioylamino]-4,5,6,7-tetrahydrobenzothiophen-3-carboxamid;
(5S)-2-(Cyclopropancarbonylamino)-N-(cyclopropylmethyl)-5-[3-(pyrimidin-5-ylamino)-1,2,4-triazol-4-yl]-4,5,6,7-tetrahydrobenzothiophen-3-carboxamid;
(5S)-2-(Cyclopropancarbonylamino)-N-(cyclopropylmethyl)-5-[2-(cyclopropylmethylamino)imidazol-1-yl]-4,5,6,7-tetrahydrobenzothiophen-3-carboxamid;
2-(Cyclopropancarbonylamino)-N-(cyclopropylmethyl)-5-[[4-(ethylamino)-1,1-dioxo-1,2,5-thiadiazol-3-yl]amino]-4,5,6,7-tetrahydrobenzothiophen-3-carboxamid;
2-(Cyclopropancarbonylamino)-N-(cyclopropylmethyl)-5-[[1-(4-methylanilino)-2-nitro-vinyl]amino]-4,5,6,7-tetrahydrobenzothiophen-3-carboxamid;
(5S)-5-[(N'-Cyano-N-ethyl-carbamimidoyl)-methyl-amino]-2-(cyclopropancarbonylamino)-N-(cyclopropylmethyl)-4,5,6,7-tetrahydrobenzothiophen-3-carboxamid;
2-(Cyclopropancarbonylamino)-N-(cyclopropylmethyl)-5-[[2-(4-methylanilino)-3,4-dioxo-cyclobuten-1-yl]amino]-4,5,6,7-tetrahydrobenzothiophen-3-carboxamid;
(5S)-2-(Cyclopropancarbonylamino)-N-(cyclopropylmethyl)-5-[3-(2-pyridylamino)-1,2,4-triazol-4-yl]-4,5,6,7-tetrahydrobenzothiophen-3-carboxamid;
(5S)-5-(2-Acetamidoimidazol-1-yl)-2-(cyclopropancarbonylamino)-N-(cyclopropylmethyl)-4,5,6,7-tetrahydrobenzothiophen-3-carboxamid;
*tert*-Butyl-N-[3-[2-(cyclopropancarbonylamino)-3-(cyclopropylmethylcarbamoyl)-4,5,6,7-tetrahydrobenzothiophen-5-yl]triazol-4-yl]carbamat;
2-(Cyclopropancarbonylamino)-N-(cyclopropylmethyl)-5-[5-(3-pyridylamino)triazol-1-yl]-4,5,6,7-tetrahydrobenzothiophen-3-carboxamid;
2-(Cyclopropancarbonylamino)-N-(cyclopropylmethyl)-5-(ethylcarbamothioylamino)-4,5,6,7-tetrahydrobenzothiophen-3-carboxamid;
(5S)-2-(Cyclopropancarbonylamino)-N-(cyclopropylmethyl)-5-[3-[[(3S)-tetrahydrofuran-3-yl]amino]-1,2,4-triazol-4-yl]-4,5,6,7-tetrahydrobenzothiophen-3-carboxamid;
5-[[N'-Cyano-N-ethyl-carbamimidoyl]amino]-2-(cyclopropancarbonylamino)-N-(cyclopropylmethyl)-4,5,6,7-tetrahydrobenzothiophen-3-carboxamid;
(5S)-2-(Cyclopropancarbonylamino)-N-(cyclopropylmethyl)-5-[[(E)-N-ethyl-N'-nitro-carbamimidoyl]amino]-4,5,6,7-tetrahydrobenzothiophen-3-carboxamid;
(5R)-2-(Cyclopropancarbonylamino)-N-(cyclopropylmethyl)-5-[[(E)-N-ethyl-N'-nitro-carbamimidoyl]amino]-4,5,6,7-tetrahydrobenzothiophen-3-carboxamid;
(5S)-2-(Cyclopropanecarbonylamino)-N-(cyclopropylmethyl)-5-[2-(ethylamino)imidazol-1-yl]-4,5,6,7-tetrahydrobenzothiophen-3-carboxamid;
(5S)-2-(Cyclopropancarbonylamino)-N-(cyclopropylmethyl)-5-[3-[(3,5-dimethylisoxazol-4-yl)amino]-1,2,4-triazol-4-yl]-4,5,6,7-tetrahydrobenzothiophen-3-carboxamid;
Ethyl-N-[[[2-(cyclopropancarbonylamino)-3-(cyclopropylmethylcarbamoyl)-4,5,6,7-tetrahydrobenzothiophen-5-yl]amino]-(4-methylanilino)methylen]carbamat;
(5S)-2-(Cyclopropancarbonylamino)-N-(cyclopropylmethyl)-5-[2-[(2-methyl-3-20-pyridyl)amino]imidazol-1-yl]-4,5,6,7-tetrahydrobenzothiophen-3-carboxamid;
2-(Cyclopropancarbonylamino)-N-(cyclopropylmethyl)-5-[[1-(ethylamino)-2-nitro-vinyl]amino]-4,5,6,7-tetrahydrobenzothiophen-3-carboxamid;
2-(Cyclopropancarbonylamino)-N-(cyclopropylmethyl)-5-[(4-methyl-6-oxo-1H-pyrimidin-2-yl)amino]-4,5,6,7-tetrahydrobenzothiophen-3-carboxamid;
5-Amino-l-[(5S)-2-cyclopropanamido-3-[(cyclopropylmethyl)carbamoyl]-4,5,6,7-tetrahydro-1-benzothiophen-5-yl]-N-methyl-1H-imidazol-4-carboxamid;
5-Amino-l-[(5S)-2-cyclopropanamido-3-[(cyclopropylmethyl)carbamoyl]-4,5,6,7-tetrahydro-1-benzothiophen-5-yl]-1H-imidazol-4-carboxamid;
5-(2-Anilinoimidazol-1-yl)-2-(cyclopropancarbonylamino)-N-(cyclopropylmethyl)-4,5,6,7-tetrahydrobenzothiophen-3-carboxamid;
(5S)-5-[[N'-Cyano-N-ethyl-carbamimidoyl]amino]-2-(cyclopropancarbonylamino)-N-(cyclopropylmethyl)-4,5,6,7-tetrahydrobenzothiophen-3-carboxamid;
2-(Cyclopropancarbonylamino)-N-(cyclopropylmethyl)-5-(ethylcarbamoylamino)-4,5,6,7-tetrahydrobenzothiophen-3-carboxamid;
2-(Cyclopropancarbonylamino)-N-(cyclopropylmethyl)-5-[(4-oxo-1H-pyridin-2-yl)amino]-4,5,6,7-tetrahydrobenzothiophen-3-carboxamid;
(5S)-2-(Cyclopropancarbonylamino)-N-(cyclopropylmethyl)-5-[[6-(5-methyl-1,3,4-oxadiazol-2-yl)-3-pyridyl]amino]-4,5,6,7-tetrahydrobenzothiophen-3-carboxamid;
2-(Cyclopropancarbonylamino)-N-(cyclopropylmethyl)-5-[5-(ethylamino)tetrazol-1-yl]-4,5,6,7-tetrahydrobenzothiophen-3-carboxamid;
2-(Cyclopropancarbonylamino)-N-(cyclopropylmethyl)-5-(1H-1,2,4-triazol-3-ylamino)-4,5,6,7-tetrahydrobenzothiophen-3-carboxamid;
(5R)-2-(Cyclopropancarbonylamino)-N-(cyclopropylmethyl)-5-[3-[(2,5-dimethylpyrazol-3-yl)amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[[4-(1-methylpyrazol-4-yl)-1,2,4-triazol-3-yl]amino]-4,5,6,7-tetrahydrobenzothiophen-3-carboxamid;
Ethyl-1-[(5S)-2-cyclopropanamido-3-[(cyclopropylmethyl)carbamoyl]-4,5,6,7-tetrahydro-1-benzothiophen-5-yl]-1H-imidazol-4-carboxylat;
2-(Cyclopropancarbonylamino)-N-(Cyclopropylmethyl)-5-[[4-(4-fluorphenyl)-1,2,4-triazol-3-yl]amino]-4,5,6,7-tetrahydrobenzothiophen-3-carboxamid;
(5S)-2-(Cyclopropancarbonylamino)-N-(cyclopropylmethyl)-5-(1H-imidazol-2-ylamino)-4,5,6,7-tetrahydrobenzothiophen-3-carboxamid;
2-(Cyclopropancarbonylamino)-N-(cyclopropylmethyl)-5-[[N'-methoxy-N-(p-tolyl)carbamimidoyl]amino]-4,5,6,7-tetrahydrobenzothiophen-3-carboxamid;
2-(Cyclopropancarbonylamino)-N-(cyclopropylmethyl)-5-[[4-(4-methylanilino)-1,1-dioxo-1,2,5-thiadiazol-3-yl]amino]-4,5,6,7-tetrahydrobenzothiophen-3-carboxamid;
(5S)-2-(Cyclopropancarbonylamino)-N-(cyclopropylmethyl)-5-[[1-(3-pyridyl)imidazol-2-yl]amino]-4,5,6,7-tetrahydrobenzothiophen-3-carboxamid;
5-(5-Aminotriazol-1-yl)-2-(cyclopropanecarbonylamino)-N-(cyclopropylmethyl)-4,5,6,7-tetrahydrobenzothiophen-3-carboxamid;
2-(Cyclopropancarbonylamino)-N-(cyclopropylmethyl)-5-[(2-methoxyphenyl)carbamoylamino]-4,5,6,7-tetrahydrobenzothiophen-3-carboxamid;
N-[2-(Cyclopropancarbonylamino)-3-(cyclopropylmethylcarbamoyl)-4,5,6,7-tetrahydrobenzothiophen-5-yl]-pyridin-4-carboxamid;
5-(Benzylamino)-2-(cyclopropancarbonylamino)-N-(cyclopropylmethyl)-4,5,6,7-tetrahydrobenzothiophen-3-carboxamid;
5-(1H-Benzimidazol-2-ylamino)-2-(cyclopropancarbonylamino)-N-(cyclopropylmethyl)-4,5,6,7-tetrahydrobenzothiophen-3-carboxamid;
2-(Cyclopropancarbonylamino)-N-(cyclopropylmethyl)-5-[[N'-hydroxycarbamimidoyl]amino]-4,5,6,7-tetrahydrobenzothiophen-3-carboxamid;
2-(Cyclopropancarbonylamino)-N-(cyclopropylmethyl)-5-[[N'-(1-methylpyrazol-3-yl)-N-(p-tolyl)carbamimidoyl]amino]-4,5,6,7-tetrahydrobenzothiophen-3-carboxamid; Ameisensäure;
(5S)-2-(Cyclopropancarbonylamino)-N-(cyclopropylmethyl)-5-[3-[(3,5-dimethylpyrazin-2-yl)amino]-1,2,4-triazol-4-yl]-4,5,6,7-tetrahydrobenzothiophen-3-carboxamid;
2-(Cyclopropancarbonylamino)-N-(cyclopropylmethyl)-5-[3-(ethylamino)-5-(methoxymethyl)-1,2,4-triazol-4-yl]-4,5,6,7-tetrahydrobenzothiophen-3-carboxamid;
2-(Cyclopropancarbonylamino)-N-(cyclopropylmethyl)-5-[[5-(3-pyridyl)-1H-1,2,4-triazol-3-yl]amino]-4,5,6,7-tetrahydrobenzothiophen-3-carboxamid;
p-Tolyl-N-[2-(Cyclopropanecarbonylamino)-3-(cyclopropylmethylcarbamoyl)-4,5,6,7-tetrahydrobenzothiophen-5-yl]carbamat;
2-(Cyclopropancarbonylamino)-N-(cyclopropylmethyl)-5-(chinazolin-2-ylamino)-4,5,6,7-tetrahydrobenzothiophen-3-carboxamid;
5-(1,3-Benzoxazol-2-ylamino)-2-(cyclopropancarbonylamino)-N-(cyclopropylmethyl)-4,5,6,7-tetrahydrobenzothiophen-3-carboxamid;
2-(Cyclopropancarbonylamino)-N-(cyclopropylmethyl)-5-[3-(ethylamino)-5-(hydroxymethyl)-1,2,4-triazol-4-yl]-4,5,6,7-tetrahydrobenzothiophen-3-carboxamid;
(5S)-5-(2-Aminoimidazol-1-yl)-2-(cyclopropancarbonylamino)-N-(cyclopropylmethyl)-4,5,6,7-tetrahydrobenzothiophen-3-carboxamid;
5-(3-Amino-5-oxo-1,2,4-oxadiazol-4-yl)-2-(cyclopropancarbonylamino)-N-(cyclopropylmethyl)-4,5,6,7-tetrahydrobenzothiophen-3-carboxamid;
2-(Cyclopropancarbonylamino)-N-(cyclopropylmethyl)-5-[3-(ethylamino)-5-methyl-1,2,4-triazol-4-yl]-4,5,6,7-tetrahydrobenzothiophen-3-carboxamid;
(5R)-2-(Cyclopropancarbonylamino)-N-(cyclopropylmethyl)-5-[(2-ethyl-3,4-dioxo-cyclobuten-1-yl)amino]-4,5,6,7-tetrahydrobenzothiophen-3-carboxamid;
(5S)-2-(Cyclopropancarbonylamino)-N-(cyclopropylmethyl)-5-[(2-ethyl-3,4-dioxo-cyclobuten-1-yl)amino]-4,5,6,7-tetrahydrobenzothiophen-3-carboxamid;
2-(Cyclopropancarbonylamino)-N-(cyclopropylmethyl)-5-(pyrimidin-2-ylamino)-4,5,6,7-tetrahydrobenzothiophen-3-carboxamid;
2-(Cyclopropancarbonylamino)-N-(cyclopropylmethyl)-5-(1,3,4-oxadiazol-2-ylamino)-4,5,6,7-tetrahydrobenzothiophen-3-carboxamid;
2-(Cyclopropancarbonylamino)-N-(cyclopropylmethyl)-5-[[5-oxo-1-(p-tolyl)-4H-imidazol-2-yl]amino]-4,5,6,7-tetrahydrobenzothiophen-3-carboxamid;
2-(Cyclopropancarbonylamino)-N-(cyclopropylmethyl)-5-[[2-[ethyl(methyl)amino]-3,4-dioxo-cyclobuten-1-yl]amino]-4,5,6,7-tetrahydrobenzothiophen-3-carboxamid;
2-(Cyclopropancarbonylamino)-N-(cyclopropylmethyl)-5-[(methylsulfonimidoyl)amino]-4,5,6,7-tetrahydrobenzothiophen-3-carboxamid;
5-[[N-Cyano-C-methyl-carbonimidoyl]amino]-2-(cyclopropancarbonylamino)-N-(cyclopropylmethyl)-4,5,6,7-tetrahydrobenzothiophen-3-carboxamid;
2-(Cyclopropancarbonylamino)-N-(cyclopropylmethyl)-5-(tetrazol-1-yl)-4,5,6,7-tetrahydrobenzothiophen-3-carboxamid;
2-(Cyclopropancarbonylamino)-N-(cyclopropylmethyl)-5-(methansulfinamido)-4,5,6,7-tetrahydrobenzothiophen-3-carboxamid;
2-(Cyclopropancarbonylamino)-N-(cyclopropylmethyl)-5-morpholino-4,5,6,7-tetrahydrobenzothiophen-3-carboxamid;
2-(Cyclopropancarbonylamino)-N-(cyclopropylmethyl)-5-[3-(ethylamino)-5-(trifluormethyl)-1,2,4-triazol-4-yl]-4,5,6,7-tetrahydrobenzothiophen-3-carboxamid;
2-(Cyclopropancarbonylamino)-N-(cyclopropylmethyl)-5-[(4-oxo-1H-pyrimidin-2-yl)amino]-4,5,6,7-tetrahydrobenzothiophen-3-carboxamid;
2-(Cyclopropancarbonylamino)-N-(cyclopropylmethyl)-5-(isopropylsulfamoylamino)-4,5,6,7-tetrahydrobenzothiophen-3-carboxamid;
5-[[N'-Cyano-N-morpholino-carbamimidoyl]amino]-2-(cyclopropancarbonylamino)-N-(cyclopropylmethyl)-4,5,6,7-tetrahydrobenzothiophen-3-carboxamid;
5-Acetamido-2-(cyclopropancarbonylamino)-N-(cyclopropylmethyl)-4,5,6,7-tetrahydrobenzothiophen-3-carboxamid;
2-(Cyclopropancarbonylamino)-N-(cyclopropylmethyl)-5-(methansulfonamido)-4,5,6,7-tetrahydrobenzothiophen-3-carboxamid;
5-[[N'-Cyano-N-methoxy-carbamimidoyl]amino]-2-(cyclopropancarbonylamino)-N-(cyclopropylmethyl)-4,5,6,7-tetrahydrobenzothiophen-3-carboxamid;
2-(Cyclopropancarbonylamino)-N-(cyclopropylmethyl)-5-[[4-oxo-6-(trifluormethyl)-1H-pyrimidin-2-yl]amino]-4,5,6,7-tetrahydrobenzothiophen-3-carboxamid;
(5S)-2-(Cyclopropancarbonylamino)-N-(cyclopropylmethyl)-5-[3-(2-methylpropanoylamino)-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[(2,5-dimethylpyrazol-3-yl)-methyl-amino]-1,2,4-triazol-4-yl]-4,5,6,7-tetrahydrobenzothiophen-3-carboxamid;
(5S)-5-[5-Amino-4-(5-methyl-1,3,4-oxadiazol-2-yl)imidazol-1-yl]-2-(cyclopropancarbonylamino)-N-(cyclopropylmethyl)-4,5,6,7-tetrahydrobenzothiophen-3-carboxamid;
(5S)-2-(Cyclopropancarbonylamino)-N-(cyclopropylmethyl)-5-[3-[(3-methyl-1H-pyrazol-5-yl)amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[(1,3-dimethylpyrazol-4-yl)amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[(5-cyclopropyl-2-methyl-pyrazol-3-yl)amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[(5-isopropyl-2-methyl-pyrazol-3-20-yl)amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[[2-methyl-5-(trifluormethyl)pyrazol-3-yl]amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[(2-isopropyl-5-methyl-pyrazol-3-yl)amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[(5-methyl-2-tetrahydrofuran-3-yl-pyrazol-3-yl)-amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[(2-methylpyrazol-3-yl)amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[(2-ethyl-5-methyl-pyrazol-3-yl)amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[(2-cyclopropyl-5-methyl-pyrazol-3-yl)amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[(3-methoxy-1-methyl-pyrazol-4-5 yl)amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[(5-ethyl-2-methyl-pyrazol-3-yl)amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[[5-(difluormethyl)-2-methyl-pyrazol-3-yl]amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[(1,4-dimethylpyrazol-3-yl)amino]-1,2,4-triazol-4-yl]-4,5,6,7-tetrahydrobenzothiophen-3-carboxamid;
Methyl-5-[[4-[(5S)-2-(cyclopropanecarbonylamino)-3-(cyclopropylmethylcarbamoyl)-4,5,6,7-tetrahydrobenzothiophen-5-yl]-1,2,4-triazol-3-yl]amino]-1-methyl-pyrazol-3-carboxylat;
(5S)-2-(Cyclopropancarbonylamino)-N-(cyclopropylmethyl)-5-[2-[[2-methyl-5-(trifluormethyl)pyrazol-3-yl]amino]imidazol-1-yl]-4,5,6,7-tetrahydrobenzothiophen-3-carboxamid;
5-[[4-[(5S)-2-(Cyclopropancarbonylamino)-3-(cyclopropylmethylcarbamoyl)-4,5,6,7-tetrahydrobenzothiophen-5-yl]-1,2,4-triazol-3-yl]amino]-1-methyl-pyrazol-3-carbonsäure; deren Enantiomeren und pharmazeutisch unbedenklichen Salzen davon.

12. Verbindung nach Anspruch 1 oder ein pharmazeutisch unbedenkliches Salz davon, wobei die Verbindung ausgewählt ist aus:
(5S)-2-( Cyclopropancarbonylamino)-N-(cyclopropylmethyl)-5-[3-[[(3S*)-1,1-dioxothiolan-3-yl]amino]-1,2,4-triazol-4-yl]-4,5,6,7-tetrahydrobenzothiophen-3-carboxamid [*oder R];
(5S)-2-Cyclopropanamido-N-(cyclopropylmethyl)-5-[3-({3-[imino(methyl)oxo-lambda6-sulfanyl]-2-methylphenyl}amino)-4H-1,2,4-triazol-4-yl]-4,5,6,7-tetrahydro-1-benzothiophen-3-carboxamid;
(5S)-2-(Cyclopropancarbonylamino)-N-(cyclopropylmethyl)-5-[3-[2-methyl-3-[(S)-methylsulfonimidoyl]anilino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[2-methyl-3-[(R)-methylsulfonimidoyl]anilino]-1,2,4-triazol-4-yl]-4,5,6,7-tetrahydrobenzothiophen-3-carboxamid;
(5S)-5-{3-[(1-Acetyl-3-methylpyrrolidin-3-yl)amino]-4H-1,2,4-triazol-4-yl}-2-cyclopropanamido-N-(cyclopropylmethyl)-4,5,6,7-tetrahydro-1-benzothiophen-3-carboxamid;
(5S)-5-[3-[[(3S*)-1-Acetyl-3-methyl-pyrrolidin-3-yl]amino]-1,2,4-triazol-4-yl]-2-(cyclopropancarbonylamino)-N-(cyclopropylmethyl)-4,5,6,7-tetrahydrobenzothiophen-3-carboxamid [* oder R];
(5S)-2-Cyclopropanamido-N-(cyclopropylmethyl)-5-(3-{[(3S)-1-(6-methylpyridazin-3-yl)pyrrolidin-3-yl]amino}-4H-1,2,4-triazol-4-yl)-4,5,6,7-tetrahydro-1-benzothiophen-3-carboxamid;
(5S)-2-(Cyclopropancarbonylamino)-N-(cyclopropylmethyl)-5-[3-[[(3S)-1-pyrazin-2-ylpyrrolidin-3-yl]amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[(4-methyl-1-methylsulfonyl-pyrrolidin-3-yl)amino]-1,2,4-triazol-4-yl]-4,5,6,7-tetrahydrobenzothiophen-3-carboxamid;
(5S)-2-Cyclopropanamido-N-(cyclopropylmethyl)-5-{3-[(1-oxo-2,3-dihydro-1H-isoindol-4-yl)amino]-4H-1,2,4-triazol-4-yl}-4,5,6,7-tetrahydro-1-benzothiophen-3-carboxamid;
(5S)-2-(Cyclopropancarbonylamino)-N-(cyclopropylmethyl)-5-[3-[[(3S)-1-(6-fluorpyridazin-3-yl)pyrrolidin-3-yl]amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[[(3S)-1-pyrimidin-2-ylpyrrolidin-3-yl]amino]-1,2,4-triazol-4-yl]-4,5,6,7-tetrahydro-1-benzothiophen-3-carboxamid;
2-Methoxyethyl-5-amino-1-[(5S)-2-(cyclopropancarbonylamino)-3-(cyclopropylmethylcarbamoyl)-4,5,6,7-tetrahydro-1-benzothiophen-5-yl]imidazol-4-carboxylat;
2-Morpholin-4-ylethyl-5-amino-1-[(5S)-2-(cyclopropancarbonylamino)-3-(cyclopropylmethylcarbamoyl)-4,5,6,7-tetrahydro-1-benzothiophen-5-yl]imidazol-4-carboxylat;
(5S)-2-(Cyclopropancarbonylamino)-N-(cyclopropylmethyl)-5-[3-(3-methylsulfanylanilino)-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-(3-methylsulfonylanilino)-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-(3-methylsulfinylanilino)-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[3-(methylsulfonimidoyl)anilino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[3-[(S)-methylsulfonimidoyl]anilino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[3-[(R)-methylsulfonimidoyl]anilino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-(8-methoxy-4-oxo-5,10-dihydroimidazo[4,5-c][1,5]benzodiazepin-1-yl)-4,5,6,7-tetrahydrobenzothiophen-3-carboxamid;
(5S)-2-(Cyclopropancarbonylamino)-N-(cyclopropylmethyl)-5-(8-methoxy-5-methyl-4-oxo-10H-imidazo[4,5-c][1,5]benzodiazepin-1-yl)-4,5,6,7-tetrahydrobenzothiophen-3-carboxamid;
(5S)-5-[5-Amino-4-[(3R)-3-hydroxypyrrolidin-1-carbonyl]imidazol-1-yl]-2-(cyclopropancarbonylamino)-N-(cyclopropylmethyl)-4,5,6,7-tetrahydrobenzothiophen-3-carboxamid;
(5S)-5-[5-Amino-4-[(2S)-2-Methylpyrrolidin-1-carbonyl]imidazol-1-yl]-2-(cyclopropancarbonylamino)-N-(cyclopropylmethyl)-4,5,6,7-tetrahydrobenzothiophen-3-carboxamid;
Ethyl-1-[(5S)-2-(cyclopropancarbonylamino)-3-(cyclopropylmethylcarbamoyl)-4,5,6,7-tetrahydrobenzothiophen-5-yl]-5-[(2,5-dimethyl-4-nitro-pyrazol-3-yl)amino]imidazol-4-carboxylat;
(5S)-2-(Cyclopropancarbonylamino)-N-(cyclopropylmethyl)-5-[3-(2-methyl-3-methylsulfinyl-anilino)-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-(2-methyl-3-methylsulfonyl-anilino)-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[(2-methyl-5-oxazol-2-yl-pyrazol-3-yl)amino]-1,2,4-triazol-4-yl]-4,5,6,7-tetrahydrobenzothiophen-3-carboxamid;
(5S)-5-[3-[[(3S)-1-Acetylpyrrolidin-3-yl]amino]-1,2,4-triazol-4-yl]-2-(cyclopropancarbonylamino)-N-(cyclopropylmethyl)-4,5,6,7-tetrahydrobenzothiophen-3-carboxamid;
(5S)-2-(Cyclopropancarbonylamino)-N-(cyclopropylmethyl)-5-[3-[[(3S)-1-methylsulfonylpyrrolidin-3-yl]amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[[(3R)-1-methylsulfonylpyrrolidin-3-yl]amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[(2-methylcyclohexyl)amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[(5-oxopyrrolidin-3-yl)amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[[(3R*)-5-oxopyrrolidin-3-yl]amino]-1,2,4-triazol-4-yl]-4,5,6,7-tetrahydrobenzothiophen-3-carboxamid (* oder S);
(5S)-2-(Cyclopropancarbonylamino)-N-(cyclopropylmethyl)-5-[3-[(2-oxo-4-piperidyl)amino]-1,2,4-triazol-4-yl]-4,5,6,7-tetrahydrobenzothiophen-3-carboxamid;
(5S)-5-[3-[(1-Acetyl-2-methyl-pyrrolidin-3-yl)amino]-1,2,4-triazol-4-yl]-2-(cyclopropancarbonylamino)-N-(cyclopropylmethyl)-4,5,6,7-tetrahydrobenzothiophen-3-carboxamid;
(5S)-2-(Cyclopropancarbonylamino)-N-(cyclopropylmethyl)-5-[3-[(2-methyl-1-methylsulfonyl-pyrrolidin-3-yl)amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[[3-(6-methyl-3-pyridyl)cyclobutyl]amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[[3-(6-methyl-3-pyridyl)cyclobutyl]amino]-1,2,4-triazol-4-yl]-4,5,6,7-tetrahydrobenzothiophen-3-carboxamid;
(5S)-5-[3-[[(3S*)-1-Benzoyl-4,4-difluor-pyrrolidin-3-yl]amino]-1,2,4-triazol-4-yl]-2-(cyclopropancarbonylamino)-N-(cyclopropylmethyl)-4,5,6,7-tetrahydrobenzothiophen-3-carboxamid [* oder R];
(5S)-5-[3-[(1-Acetyl-4-piperidyl)amino]-1,2,4-triazol-4-yl]-2-(cyclopropancarbonylamino)-N-(cyclopropylmethyl)-4,5,6,7-tetrahydrobenzothiophen-3-carboxamid;
Methyl-(3S)-3-[[4-[(5S)-2-(cyclopropancarbonylamino)-3-(cyclopropylmethylcarbamoyl)-4,5,6,7-tetrahydrobenzothiophen-5-yl]-1,2,4-triazol-3-yl]amino]pyrrolidin-1-carboxylat;
(3S)-3-[[4-[(5S)-2-(Cyclopropancarbonylamino)-3-(cyclopropylmethylcarbamoyl)-4,5,6,7-tetrahydrobenzothiophen-5-yl]-1,2,4-triazol-3-yl]amino]-N-ethyl-pyrrolidin-1-carboxamid;
(5S)-2-Cyclopropanamido-N-(cyclopropylmethyl)-5-(3-{[2-(pyrimidin-2-yl)-2-azaspiro[4.4]nonan-7-yl]amino}-4H-1,2,4-triazol-4-yl)-4,5,6,7-tetrahydro-1-benzothiophen-3-carboxamid;
(5S)-2-(Cyclopropancarbonylamino)-N-(cyclopropylmethyl)-5-[3-[[2-methyl-5-(trifluormethoxy)pyrazol-3-yl]amino]-1,2,4-triazol-4-yl]-4,5,6,7-tetrahydrobenzothiophen-3-carboxamid;
(5S)-5-[3-[[5-[Chlor(difluor)methoxy]-2-methyl-pyrazol-3-yl]amino]-1,2,4-triazol-4-yl]-2-(cyclopropancarbonylamino)-N-(cyclopropylmethyl)-4,5,6,7-tetrahydrobenzothiophen-3-carboxamid;
(5S)-2-Cyclopropanamido-N-(cyclopropylmethyl)-5-(3-{[1-methyl-3-(5-methyl-1,3,4-oxadiazol-2-yl)-1H-pyrazol-5-yl]amino }-4H-1,2,4-triazol-4-yl)-4,5,6,7-tetrahydro-1-benzothiophen-3-carboxamid;
(5S)-2-(Cyclopropancarbonylamino)-N-(cyclopropylmethyl)-5-[3-[(5-methoxy-3-methyl-pyrazin-2-yl)amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[(5-methylthiazolo[5,4-b]pyridin-2-yl)amino]-4,5,6,7-tetrahydrobenzothiophen-3-carboxamid;
(5S)-5-[3-[(5-Cyano-2-methyl-3-pyridyl)amino]-1,2,4-triazol-4-yl]-2-(cyclopropancarbonylamino)-N-(cyclopropylmethyl)-4,5,6,7-tetrahydrobenzothiophen-3-carboxamid;
(5S)-5-[3-[(6-Cyano-2-methyl-3-pyridyl)amino]-1,2,4-triazol-4-yl]-2-(cyclopropancarbonylamino)-N-(cyclopropylmethyl)-4,5,6,7-tetrahydrobenzothiophen-3-carboxamid;
(5S)-2-(Cyclopropancarbonylamino)-N-(cyclopropylmethyl)-5-[3-[(1-methyl-2-oxo-4-pyridyl)amino]-1,2,4-triazol-4-yl]-4,5,6,7-tetrahydrobenzothiophen-3-carboxamid;
(5S)-5-[3-[(6-Chlor-2-methyl-3-pyridyl)amino]-1,2,4-triazol-4-yl]-2-(cyclopropancarbonylamino)-N-(cyclopropylmethyl)-4,5,6,7-tetrahydrobenzothiophen-3-carboxamid;
(5S)-2-(Cyclopropancarbonylamino)-N-(cyclopropylmethyl)-5-[3-[[2-methyl-6-(trifluormethyl)-3-pyridyl]amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[(3-methylpyridazin-4-yl)amino]-1,2,4-triazol-4-yl]-4,5,6,7-tetrahydrobenzothiophen-3-carboxamid;
(5S)-5-[3-[(5-Chlor-2-methyl-pyrazol-3-yl)amino]-1,2,4-triazol-4-yl]-2-(cyclopropancarbonylamino)-N-(cyclopropylmethyl)-4,5,6,7-tetrahydrobenzothiophen-3-carboxamid;
(5S)-2-(Cyclopropancarbonylamino)-N-(cyclopropylmethyl)-5-[3-[(3-methylisothiazol-4-yl)amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[(3-methylisoxazol-4-yl)amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[(5-methoxy-2-methylpyrazol-3-yl)amino]-1,2,4-triazol-4-yl]-4,5,6,7-tetrahydro-1-benzothiophen-3-carboxamid;
(5S)-2-(Cyclopropancarbonylamino)-N-(cyclopropylmethyl)-5-[3-[[5-(difluormethoxy)-2-methylpyrazol-3-yl]amino]-1,2,4-triazol-4-yl]-4,5,6,7-tetrahydro-1-benzothiophen-3-carboxamid;
(5S)-2-(Cyclopropancarbonylamino)-N-(cyclopropylmethyl)-5-[3-[[5-(difluormethyl)-2-(trideuteriomethyl)pyrazol-3-yl]amino]-1,2,4-triazol-4-yl]-4,5,6,7-tetrahydrobenzothiophen-3-carboxamid;
(5S)-5-[3-[(5-Cyano-2-methyl-pyrazol-3-yl)amino]-1,2,4-triazol-4-yl]-2-(cyclopropancarbonylamino)-N-(cyclopropylmethyl)-4,5,6,7-tetrahydrobenzothiophen-3-carboxamid;
(5S)-2-(Cyclopropancarbonylamino)-N-(cyclopropylmethyl)-5-[3-[(2,2-difluor-1,3-benzodioxol-5-yl)amino]-1,2,4-triazol-4-yl]-4,5,6,7-tetrahydrobenzothiophen-3-carboxamid;
(4RS,5RS)-2-(Cyclopropancarbonylamino)-N-(cyclopropylmethyl)-4-methyl-5-[3-[[2-methyl-5-(trifluormethyl)pyrazol-3-yl]amino]-1,2,4-triazol-4-yl]-4,5,6,7-tetrahydrobenzothiophen-3-carboxamid;
(4R*,5R*)-2-(Cyclopropancarbonylamino)-N-(cyclopropylmethyl)-4-methyl-5-[3-[[2-methyl-5-(trifluormethyl)pyrazol-3-yl]amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[[6-(difluormethyl)-3-pyridyl]amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[(6-fluor-2-methyl-3-pyridyl)amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[[6-(trifluormethyl)-3-pyridyl]amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[(6-methoxy-3-pyridyl)amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[(2-methoxy-4-pyridyl)amino]-1,2,4-triazol-4-yl]-4,5,6,7-tetrahydrobenzothiophen-3-carboxamid;
(5S)-5-[3-[(6-Chlor-3-pyridyl)amino]-1,2,4-triazol-4-yl]-2-(cyclopropancarbonylamino)-N-(cyclopropylmethyl)-4,5,6,7-tetrahydrobenzothiophen-3-carboxamid;
(5S)-5-[3-[(2-Chlor-4-pyridyl)amino]-1,2,4-triazol-4-yl]-2-(cyclopropancarbonylamino)-N-(cyclopropylmethyl)-4,5,6,7-tetrahydrobenzothiophen-3-carboxamid;
(5S)-2-(Cyclopropancarbonylamino)-N-(cyclopropylmethyl)-5-[3-[(6-methoxy-2-methyl-3-pyridyl)amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[(2-fluor-3-pyridyl)amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[(3-methyltriazol-4-yl)amino]-1,2,4-triazol-4-yl]-4,5,6,7-tetrahydrobenzothiophen-3-carboxamid;
(3R*)-6-(Cyclopropancarbonylamino)-N-(cyclopropylmethyl)-3-[3-[[2-methyl-5-(trifluormethyl)pyrazol-3-yl]amino]-1,2,4-triazol-4-yl]-3,4-dihydro-2H-thieno[2,3-b]pyran-5-carboxamid (* oder S).

13. Verbindung nach Anspruch 1 oder ein pharmazeutisch unbedenkliches Salz davon, wobei die Verbindung ausgewählt ist aus:
(5S)-2-(Cyclopropancarbonylamino)-N-(cyclopropylmethyl)-5-[3-[(2,5-dimethylpyrazol-3-yl)amino]-1,2,4-triazol-4-yl]-4,5,6,7-tetrahydrobenzothiophen-3-carboxamid;
Ethyl-5-amino-1-[(5S)-2-(cyclopropancarbonylamino)-3-(cyclopropylmethylcarbamoyl)-4,5,6,7-tetrahydrobenzothiophen-5-yl]imidazol-4-carboxylat;
(5S)-2-(Cyclopropancarbonylamino)-N-(cyclopropylmethyl)-5-[3-[(5-cyclopropyl-2-methyl-pyrazol-3-yl)amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[(5-isopropyl-2-methyl-pyrazol-3-yl)amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[[2-methyl-5-(trifluormethyl)pyrazol-3-yl]amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[(5-ethyl-2-methyl-pyrazol-3-yl)amino]-1,2,4-triazol-4-yl]-4,5,6,7-tetrahydrobenzothiophen-3-carboxamid;
(5S)-2-(Cyclopropancarbonylamino)-N-(cyclopropylmethyl)-5-[3-[[5-(difluormethyl)-2-methyl-pyrazol-3-yl]amino]-1,2,4-triazol-4-yl]-4,5,6,7-tetrahydrobenzothiophen-3-carboxamid.

14. Verbindung nach Anspruch 1 oder ein pharmazeutisch akzeptables Salz davon, wobei die Verbindung ausgewählt ist aus: 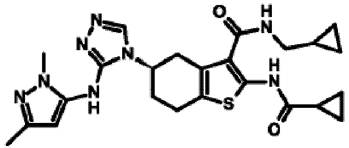
(5S)-2-(Cyclopropancarbonylamino)-N-(Cyclopropylmethyl)-5-[3-[(2,5-Dimethylpyrazol-3-yl)amino]-1,2,4-triazol-4-yl]-4,5,6,7-tetrahydrobenzothiophen-3-carboxamid; 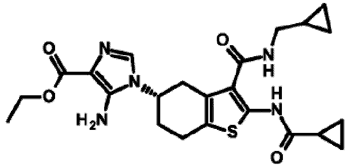
Ethyl-5-amino-1-[(5S)-2-(Cyclopropancarbonylamino)-3-(cyclopropylmethylcarbamoyl)-4,5,6,7-tetrahydrobenzothiophen-5-yl]imidazol-4-carboxylat; 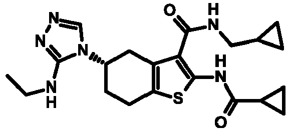
(5S)-2-(Cyclopropancarbonylamino)-N-(cyclopropylmethyl)-5-[3-(ethylamino)-1,2,4-triazol-4-yl]-4,5,6,7-tetrahydrobenzothiophen-3-carboxamid, 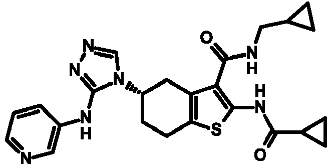
(5S)-2-(Cyclopropancarbonylamino)-N-(cyclopropylmethyl)-5-[3-(3-pyridylamino)-1,2,4-triazol-4-yl]-4,5,6,7-tetrahydrobenzothiophen-3-carboxamid; 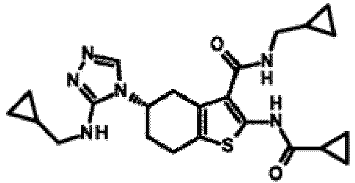
(5S)-2-(Cyclopropancarbonylamino)-N-(cyclopropylmethyl)-5-[3-(cyclopropylmethylamino)-1,2,4-triazol-4-yl]-4,5,6,7-tetrahydrobenzothiophen-3-carboxamid; 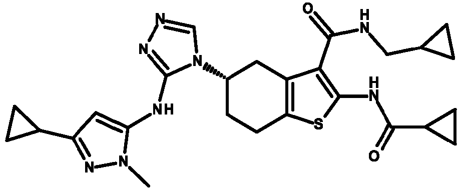
(5S)-2-(Cyclopropancarbonylamino)-N-(cyclopropylmethyl)-5-[3-[(5-cyclopropyl-2-methyl-pyrazol-3-yl)amino]-1,2,4-triazol-4-yl]-4,5,6,7-tetrahydrobenzothiophen-3-carboxamid; 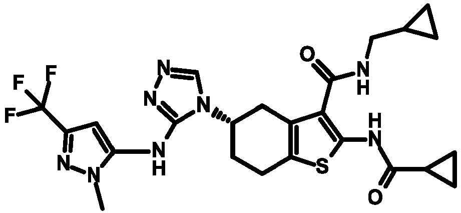
(5S)-2-(Cyclopropancarbonylamino)-N-(cyclopropylmethyl)-5-[3-[[2-methyl-5-(trifluormethyl)pyrazol-3-yl]amino]-1,2,4-triazol-4-yl]-4,5,6,7-tetrahydrobenzothiophen-3-carboxamid; 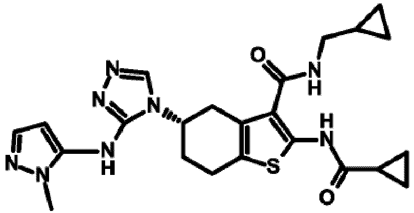
(5S)-2-(Cyclopropancarbonylamino)-N-(cyclopropylmethyl)-5-[3-[(2-methylpyrazol-3-yl)amino]-1,2,4-triazol-4-yl]-4,5,6,7-tetrahydrobenzothiophen-3-carboxamid; 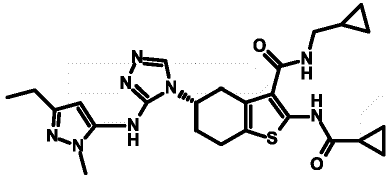
(5S)-2-(Cyclopropancarbonylamino)-N-(cyclopropylmethyl)-5-[3-[(5-ethyl-2-methyl-pyrazol-3-yl)amino]-1,2,4-triazol-4-yl]-4,5,6,7-tetrahydrobenzothiophen-3-carboxamid; 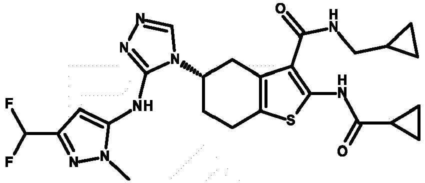
(5S)-2-(Cyclopropancarbonylamino)-N-(cyclopropylmethyl)-5-[3-[[5-(Difluormethyl)-2-methyl-pyrazol-3-yl]amino]-1,2,4-triazol-4-yl]-4,5,6,7-tetrahydrobenzothiophen-3-carboxamid; und 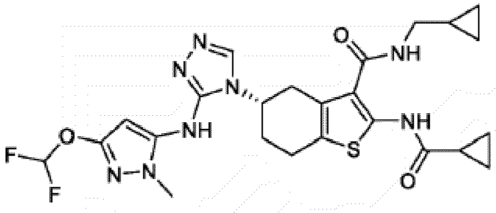
(5S)-2-(Cyclopropancarbonylamino)-N-(cyclopropylmethyl)-5-[3-[[5-(difluormethoxy)-2-methylpyrazol-3-yl]amino]-1,2,4-triazol-4-yl]-4,5,6,7-tetrahydro-1-benzothiophen-3-carboxamid.

15. Verbindung nach Anspruch 1 oder 2 oder ein pharmazeutisch unbedenkliches Salz davon, wobei die Verbindung durch die Formel (XXV) oder (XXVI) 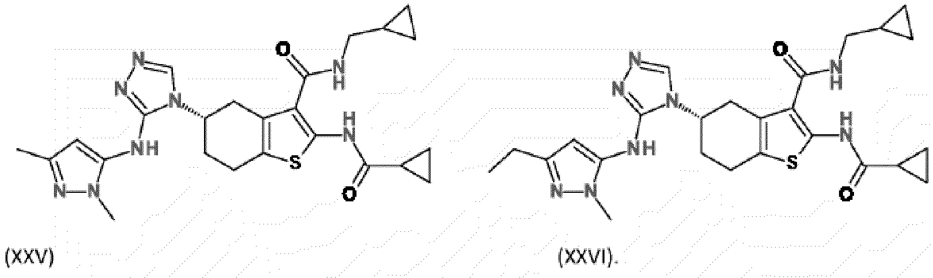 gekennzeichnet ist.

16. Verbindung nach Anspruch 1 oder 2 oder ein pharmazeutisch unbedenkliches Salz davon, wobei die Verbindung durch die Formel (XXVII) oder (XXVIII) 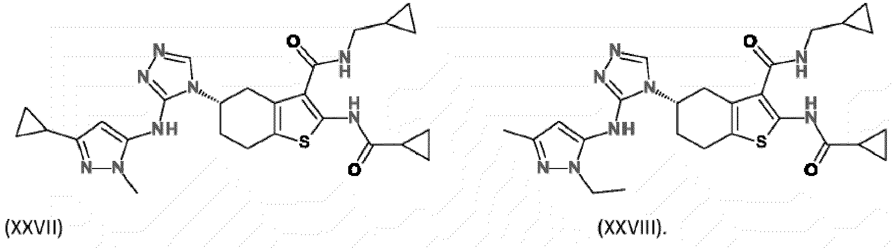 gekennzeichnet ist.

17. Verbindung nach Anspruch 1 oder 2 oder ein pharmazeutisch unbedenkliches Salz davon, wobei die Verbindung durch die Formel (XXIX) oder (XXX) 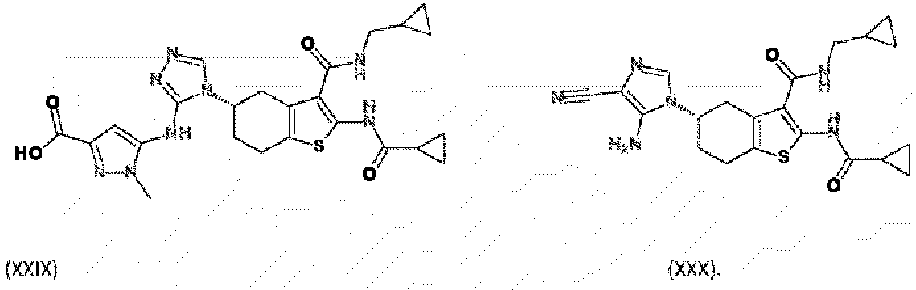 gekennzeichnet ist.

18. Verbindung nach Anspruch 1 oder 2 oder ein pharmazeutisch unbedenkliches Salz davon, wobei die Verbindung durch die Formel (XXXII) 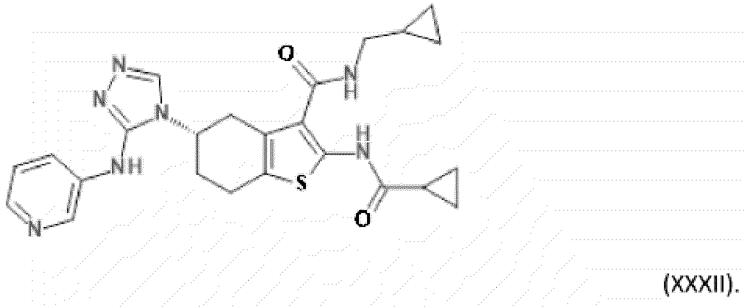 gekennzeichnet ist.

19. Verbindung nach Anspruch 1 oder 2 oder ein pharmazeutisch unbedenkliches Salz davon, wobei die Verbindung durch die Formel (XXXIII) oder (XXXIV) 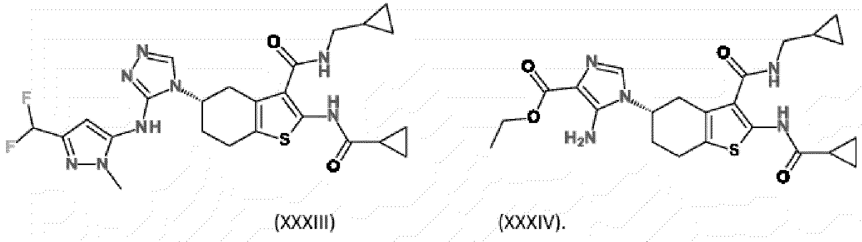 gekennzeichnet ist.

20. Verbindung nach Anspruch 1 oder 2 oder ein pharmazeutisch unbedenkliches Salz davon, wobei die Verbindung durch die Formel (XXXV) oder (XXXVI) 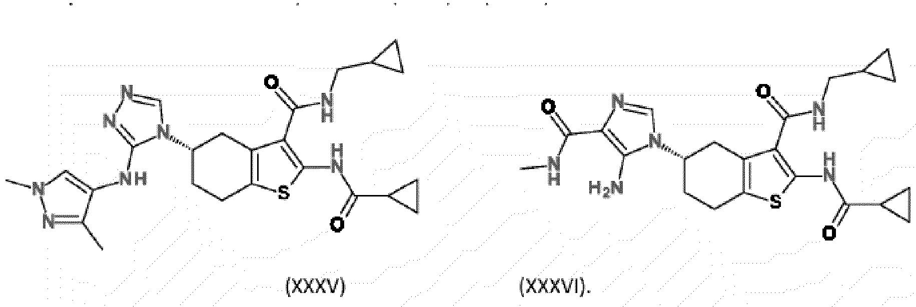 gekennzeichnet ist.

21. Pharmazeutische Zusammensetzung, die eine Verbindung nach einem der Ansprüche 1 bis 20 oder ein pharmazeutisch unbedenkliches Salz davon umfasst.

22. Verbindung nach einem der Ansprüche 1 bis 20 oder ein pharmazeutisch unbedenkliches Salz davon für die Verwendung in einem Verfahren für die Behandlung oder Vorbeugung von durch IgE verursachten Erkrankungen.

23. Verbindung für die Verwendung nach Anspruch 22 oder ein pharmazeutisch unbedenkliches Salz davon, wobei die Erkrankung eine Allergie, nicht allergische Mastzellreaktionen, eine Überempfindlichkeit des Typs 1, Urtikaria oder eine familiäre Nebenhöhlenentzündung ist.

24. Verbindung für die Verwendung nach Anspruch 22 oder ein pharmazeutisch unbedenkliches Salz davon, wobei die Erkrankung eine Verengung der Atemwege bei Asthma, eine lokale Entzündung bei Ekzemen, eine erhöhte Schleimsekretion bei allergischer Rhinitis, Urtikaria oder eine erhöhte Gefäßpermeabilität ist.

25. Verbindung für die Verwendung nach Anspruch 22 oder ein pharmazeutisch unbedenkliches Salz davon, wobei die Erkrankung eosinophile Granulomatose mit Polyangiitis (auch bekannt als "Churg-Strauss-Syndrom"), durch Aspirin verschlimmerte Atemwegserkrankung oder kutanes T-Zell-Lymphom ist.

## Revendications

1. Composés de formule (I) et sels pharmaceutiquement acceptables de ceux-ci : 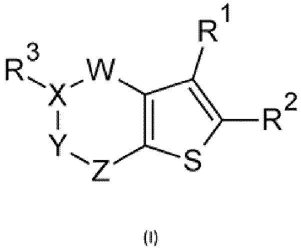 dans laquelle formule
X représente CH, N ou C-R⁴, et lorsque X est C-R⁴, R³ et R⁴ peuvent être liés pour former un noyau cycloalkyle ou hétérocyclique de 4 à 6 chaînons,
dont l'un quelconque peut être éventuellement substitué par un ou plusieurs substituants choisis parmi : halogène, =O, OH, SMe, alcoxy en C₁-C₂, -O-(halogénoalkyle en -C₁-C₂), alkyle en C₁-C₂, hétéroalkyle en C₁-C₂, halogénoalkyle en C₁-C₂, NH₂, NHMe, NMe₂, aryle et hétéroaryle, l'aryle et l'hétéroaryle étant éventuellement substitués par un ou plusieurs substituants choisis parmi : halogène, OH, alkyle en C₁-C₆, alcoxy en C₁-C₆ et halogénoalkyle en C₁-C₂ ;
lorsque X représente N, alors W, Y et Z sont C, ou un constituant parmi W, Y et Z est une liaison simple et les deux autres sont C ;
lorsque X représente CH ou C-R⁴, alors W, Y et Z sont C, ou un constituant parmi W, Y et Z est une liaison simple, O, S, S(O), S(O)₂ ou N et les deux autres sont C ;
lorsque W, Y ou Z sont C, chaque C peut être substitué par 1 ou 2 groupes qui sont indépendamment choisis parmi : H, halogène, SF₅, =O, =NOH, -OH, -CN, - C(O)Me, -C(O)OH, -C(O)NH₂, NH-C(O)O-(alkyle en C₁-C₄), -NO₂, NH₂, NHMe, NMe₂, SH, SMe, SOMe, SO₂Me, alkyle en C₁-C₄, cycloalkyle en C₃-C₄, alcoxy en C₁-C₄, hétérocycle, hétéroalkyle en C₁-C₄, lesquels peuvent être liés pour former un noyau cycloalkyle de 3 à 6 chaînons ou un noyau hétérocycle de 4 à 6 chaînons,
dont l'un quelconque peut être éventuellement substitué par un ou plusieurs substituants choisis parmi : halogène, =O, OH, SMe, alcoxy en C₁-C₂, O-(halogénoalkyle en C₁-C₂), alkyle en C₁-C₂, hétéroalkyle en C₁-C₂, halogénoalkyle en C₁-C₂, NH₂, NHMe, NMe₂, lesquels peuvent être liés pour former un noyau cycloalkyle de 3 à 6 chaînons ou un noyau hétérocycle de 4 à 6 chaînons,
étant entendu que
lorsque W, Y ou Z est N, ledit N peut être indépendamment substitué par H, alkyle en C₁-C₄, cycloalkyle en C₃-C₄, hétérocycle, -C(O)Me, -C(O)NH₂, -C(O)OH, - S(O)Me, -S(O)₂Me, -S(O)₂NH₂, dont l'un quelconque peut être éventuellement substitué par un ou plusieurs substituants choisis parmi : halogène, OH, SMe, alcoxy en C₁-C₂, O-(halogénoalkyle en C₁-C₂), alkyle en C₁-C₂, hétéroalkyle en C₁-C₂, halogénoalkyle en C₁-C₂, NH₂, NHMe, NMe₂,
et W, X, Y et Z peuvent être liés directement ou par le biais de substituants pour former un noyau ;
R¹ **représente** 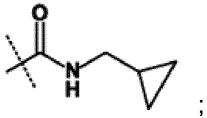
R² représente 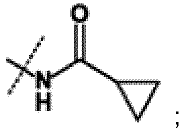
R³ est choisi parmi : H, -OH, alkyle en C₁-C₈, -NR^{d}R^{c}, -NH-R¹⁴-NH-R^{a}, -NMe-R¹⁴-NH-R^{a}, -NH-R¹⁴-NMe-R^{a}, -CO-NR^{d}R^{c}, -NH-COO-R^{c}, -NH-CO-R^{c}, -COOH, -COO-(alkyle en C₁-C₈), -CO-(alkyle en C₁-C₈), -(alkylène en C₁-C₈)-COO-(alkyle en C₁-C₈), - (alkylène en C₁-C₈)-NR^{a}-COO-(alkyle en C₁-C₈), -(alkylène en C₁-C₈)-NR^{d}R^{c}, - (alkylène en C₁-C₈)-NH-R¹⁴-NH-R^{a}, hétéroaryle, -CN, -CH=N-OH, -CF₃, alkylamino, amino, -NH-SO-(alkyle en C₁-C₈), -NH-SO₂-(alkyle en C₁-C₈), hétéroalkyl-amino, aryle, hétérocycle, cycloalkyle en C₃-C₆, -NHS(O)(=NH)Me, -SO₂-(alkyle en C₁-C₈), -NH-C(CH₃)=N-CN, -NH-CH=N-CN, -NH-S(=O)(=N)-(alkyle C₁-C₂),
dont l'un quelconque peut être éventuellement substitué par un ou plusieurs substituants choisis parmi : halogène, -OH, =O, -COOH, -COH, -CN, SMe, -NR^{d}R^{c}, - OR^{c}, alcoxy en C₁-C₂, O-(halogénoalkyle en C₁-C₂), alkyle en C₁-C₂, hétéroalkyle en C₁-C₂, halogénoalkyle en C₁-C₂, (alkylène en C₁-C₂)-O-(alkyle en C₁-C₂), (alkylène en C₁-C₂)-(cycloalkyle en C₃-C₄), hétérocycle, cycloalkyle en C₃-C₆, aryle, hétéroaryle, (cycloalkyl en C₃-C₆)-amino, hétérocyclo-amino, aryl-amino, hétéroaryl-amino, - CONR^{d}R^{c}, -NHCO-R^{a}, -NHCOO-(alkyle en C₁-C₄), -CO-(alkyle en C₁-C₂), -COO-R^{a}, qui peuvent être liés pour former un noyau, et -CONR^{d}R^{c} et -NR^{d}R^{c} pouvant être liés pour former un noyau à sept chaînons fusionné à un groupe aryle à six chaînons,
aryle, hétéroaryle, hétérocycle et cycloalkyle étant éventuellement substitués par un ou plusieurs substituants choisis parmi : halogène oxo, cyano, OH, -NOOH, alkyle en C₁-C₆, alcoxy en C₁-C₆, halogéno-(alcoxy en C₁-C₆), (cycloalkyle en C₃-C₆)-(alcoxy en C₁-C₃), -O-CO-(alkyle en C₁-C₆), cycloalkyle en C₃-C₆, halogénoalkyle en C₁-C₂, hétérocycle en C₂-C₄, acétyle, acétyl-phényle, -CONR^{d}R^{c}, COO-(alkyle en C₁-C₈), -S(O)NH-(alkyle en C₁-C₂), -S(O)ₙ-(alkyle en C₁-C₂), hétéroaryle pouvant être éventuellement substitué par un ou plusieurs groupes halogène, alkyle en C₁-C₂,
n valant 0, 1 ou 2 ;
R⁴ est choisi parmi : halogène, OH, CN, NO₂, NH₂, NHMe, NMe₂, SH, SMe, SOMe, SO₂Me, alkyle en C₁-C₄, cycloalkyle en C₃-C₄, alcoxy en C₁-C₃, hétéroalkyle en C₁-C₃, hétérocycle,
dont l'un quelconque peut être éventuellement substitué par un ou plusieurs substituants choisis parmi : halogène, =O, OH, SMe, alcoxy en C₁-C₂, O-(halogénoalkyle en C₁-C₂), alkyle en C₁-C₂, hétéroalkyle en C₁-C₂, halogénoalkyle en C₁-C₂, NH₂, NHMe, NMe₂, qui peuvent être liés pour former un noyau ;
R¹⁴ est C=O, C=S, C=N-OH, C=NR^{b}, C=CH-NO₂, SO₂, et cycloalcényle en C₃-C₆ qui peut être substitué par 1 ou 2 =O,
R^{a} est choisi parmi : H, alkyle en C₁-C₈, hétéroalkyle, alcoxy en C₁-C₈, hétérocycle, cycloalkyle en C₃-C₈, hétéroaryle et aryle,
dont l'un quelconque peut être éventuellement substitué par un ou plusieurs substituants choisis parmi : halogène, =O, OH, SMe, alcoxy en C₁-C₂, -O-(halogénoalkyle en -C₁-C₂), alkyle en C₁-C₂, cycloalkyle en C₃-C₆, hétérocycloalkyle en C₃-C₆, hétéroalkyle en C₁-C₂, halogénoalkyle en C₁-C₂, aryle, hétéroaryle, NH₂, NHMe, NMe₂ qui peuvent être liés pour former un noyau,
R^{b} est choisi parmi : CN, aryle, hétéroaryle, -NO₂, -O-(alkyle en -C₁-C₂), -CO-O-(alkyle en C₁-C₂),
aryle et hétéroaryle étant éventuellement substitués par un ou plusieurs alkyle en C₁-C₂,
R^{c} est choisi parmi : H, aryle, hétéroaryle, cycloalcényle en C₃-C₆, cycloalkyle en C₃-C₈, hétérocycle, alkyle en C₁-C₈, hétéroalkyle en C₁-C₈, (alkylène en C₁-C₄)-(cycloalkyle en C₃-C₆),
dont l'un quelconque peut être éventuellement substitué par un ou plusieurs substituants choisis parmi : C(O)(R^{g}), C(O)N(R^{f})(R^{g}), halogène, SF₅, OH, =O, -COH, -COOH, -CO-O-R^{a}, CN, SMe, SO₂Me, SO₂R^{g}, alcoxy en C₁-C₄, O-(halogénoalkyle en C₁-C₄), alkyle en C₁-C₄, hétéroalkyle en C₁-C₂, halogénoalkyle en C₁-C₄, N(R^{f})(R^{g}), N(R^{f})SO₂(R^{g}), SO₂NH₂, SO₂NHMe, SO₂NMe₂, S(O)(NR^{f})R^{g}, cycloalkyle en C₃-C₆, hétérocycle, (alkylène en C₁-C₄)-(cycloalkyle en C₃-C₆), aryle et hétéroaryle, qui peuvent être liés pour former un noyau,
aryle, hétéroaryle, hétérocycle et cycloalkyle étant éventuellement substitués par un ou plusieurs substituants choisis parmi : halogène, =O, =NH, =NMe, OH, alkyle en C₁-C₂, alcoxy en C₁-C₂, -COOH, -COO-(alkyle en C₁-C₂), SO₂R^{a}, COR^{a}, SOR^{a}, S(O)(NH)R^{a}, CONHR^{a}, qui peuvent être liés pour former un noyau,
R^{d} représente H ou alkyle en C₁-C₄,
et R^{c} et R^{d} peuvent être liés pour former un hétérocycle ;
R^{f} est choisi parmi : H, aryle, hétéroaryle, cycloalcényle en C₃-C₆, cycloalkyle en C₃-C₈, hétérocycle, alkyle en C₁-C₈, hétéroalkyle en C₁-C₈, (alkylène en C₁-C₄)-(cycloalkyle en C₃-C₆),
dont l'un quelconque peut être éventuellement substitué par un ou plusieurs substituants choisis parmi : halogène, =O, =NH, =NMe, OH, aryle, hétéroaryle, alkyle en C₁-C₂, halogénoalkyle en C₁-C₂, alcoxy en C₁-C₂, O-(halogénoalkyle en C₁-C₂), - COOH, -COO-(alkyle en C₁-C₂), SO₂R^{a}, COR^{a}, SOR^{a}, S(O)(NH)R^{a}, CONHR^{a} ;
R^{g} est choisi parmi : H, aryle, hétéroaryle, cycloalcényle en C₃-C₆, cycloalkyle en C₃-C₈, hétérocycle, alkyle en C₁-C₈, hétéroalkyle en C₁-C₈, (alkylène en C₁-C₄)-(cycloalkyle en C₃-C₆),
dont l'un quelconque peut être éventuellement substitué par un ou plusieurs substituants choisis parmi : halogène, =O, =NH, =NMe, OH, aryle, hétéroaryle, alkyle en C₁-C₂, halogénoalkyle en C₁-C₂, alcoxy en C₁-C₂, O-(halogénoalkyle en C₁-C₂), - COOH, -COO-(alkyle en C₁-C₂), SO₂R^{a}, COR^{a}, SOR^{a}, S(O)(NH)R^{a}, CONHR^{a},
R^{f} et R^{g} peuvent être liés pour former un noyau.

2. Composé selon la revendication 1 ou sel pharmaceutiquement acceptable de celui-ci, consistant en un composé de formule (la) : 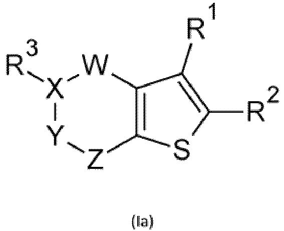 dans laquelle
X représente CH, N ou C-R⁴, et lorsque X est C-R⁴, R³ et R⁴ peuvent être liés pour former un noyau cycloalkyle ou hétérocyclique de 4 à 6 chaînons,
dont l'un quelconque peut être éventuellement substitué par un ou plusieurs substituants choisis parmi : halogène, =O, OH, SMe, alcoxy en C₁-C₂, O-(halogénoalkyle en C₁-C₂), alkyle en C₁-C₂, hétéroalkyle en C₁-C₂, halogénoalkyle en C₁-C₂, NH₂, NHMe, NMe₂, aryle et hétéroaryle, lesdits aryle et hétéroaryle étant éventuellement substitués par un ou plusieurs substituants choisis parmi : halogène, OH, alkyle en C₁-C₆, alcoxy en C₁-C₆ et halogénoalkyle en C₁-C₂,
lorsque X représente N, alors W, Y et Z sont C, ou un constituant parmi W, Y et Z est une liaison simple et les deux autres sont C,
lorsque X représente CH ou C-R⁴, alors W, Y et Z sont C, ou un constituant parmi W, Y et Z est une liaison simple, O, S, S(O), S(O)₂ ou N, et les deux autres sont C,
lorsque W, Y ou Z sont C, chaque C peut être substitué par 1 ou 2 groupes qui sont indépendamment choisis parmi : H, halogène, SF₅, =O, =NOH, -OH, -CN, - C(O)Me, -C(O)OH, -C(O)NH₂, NH-C(O)O-(alkyle en C₁-C₄), -NO₂, NH₂, NHMe, NMe₂, SH, SMe, SOMe, SO₂Me, alkyle en C₁-C₄, cycloalkyle en C₃-C₄, alcoxy en C₁-C₄, hétérocycle, hétéroalkyle en C₁-C₄, qui peuvent être liés pour former un noyau cycloalkyle de 3 à 6 chaînons ou un noyau hétérocycle de 4 à 6 chaînons,
dont l'un quelconque peut être éventuellement substitué par un ou plusieurs substituants choisis parmi : halogène, =O, OH, SMe, alcoxy en C₁-C₂, O-(halogénoalkyle en C₁-C₂), alkyle en C₁-C₂, hétéroalkyle en C₁-C₂, halogénoalkyle en C₁-C₂, NH₂, NHMe, NMe₂, qui peuvent être liés pour former un noyau cycloalkyle de 3 à 6 chaînons ou un noyau hétérocycle de 4 à 6 chaînons,
étant entendu que
lorsque W, Y ou Z est N, ledit N peut être indépendamment substitué par H, alkyle en C₁-C₄, cycloalkyle en C₃-C₄, hétérocycle, -C(O)Me, -C(O)NH₂, -C(O)OH, - S(O)Me, -S(O)₂Me, -S(O)₂NH₂, dont l'un quelconque peut être éventuellement substitué par un ou plusieurs substituants choisis parmi : halogène, OH, SMe, alcoxy en C₁-C₂, O-(halogénoalkyle en C₁-C₂), alkyle en C₁-C₂, hétéroalkyle en C₁-C₂, halogénoalkyle en C₁-C₂, NH₂, NHMe, NMe₂,
et W, X, Y et Z peuvent être liés directement ou par le biais de substituants pour former un noyau,
R¹ représente 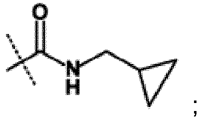
R² représente 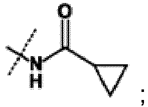
R³ est choisi parmi : H, -OH, alkyle en C₁-C₈, -NR^{d}R^{c}, -NH-R¹⁴-NH-R^{a}, -NMe-R¹⁴-NH-R^{a}, -NH-R¹⁴-NMe-R^{a}, -CO-NR^{d}R^{c} -NH-COO-R^{c}, -NH-CO-R^{c}, -COOH, -COO-(alkyle en C₁-C₈), -CO-(alkyle en C₁-C₈), -(alkylène en C₁-C₈)-COO-(alkyle en C₁-C₈), -(alkylène en C₁-C₈)-NR^{a}-COO-(alkyle en C₁-C₈), -(alkylène en C₁-C₈)-NR^{d}R^{c}, -(alkylène en C₁-C₈)-NH-R¹⁴-NH-R^{a}, hétéroaryle, -CN, -CH=N-OH, -CF₃, alkylamino, amino, -NH-SO-(alkyle en C₁-C₈), -NH-SO₂-(alkyle en C₁-C₈), hétéroalkyl-amino, aryle, hétérocycle, cycloalkyle en C₃-C₆, -NHS(O)(=NH)Me, -SO₂-(alkyle en C₁-C₈), -NH-C(CH₃)=N-CN, -NH-CH=N-CN, -NH-S(=O)(=N)-(alkyle en C₁-C₂),
dont l'un quelconque peut être éventuellement substitué par un ou plusieurs substituants choisis parmi : halogène, -OH, =O, -COOH, -COH, -CN, SMe, -NR^{d}R^{c}, - OR^{c}, alcoxy en C₁-C₂, O-(halogénoalkyle en C₁-C₂), alkyle en C₁-C₂, hétéroalkyle en C₁-C₂, halogénoalkyle en C₁-C₂, (alkylène en C₁-C₂)-O-(alkyle en C₁-C₂), (alkylène en C₁-C₂)-(cycloalkyle en C₃-C₄), hétérocycle, cycloalkyle en C₃-C₆, aryle, hétéroaryle, (cycloalkyl en C₃-C₆)-amino, hétérocycle-amino, aryl-amino, hétéroaryl-amino, - CONR^{d}R^{c}, -NHCO-R^{a}, -NHCOO-(alkyle en C₁-C₄), -CO-(alkyle en C₁-C₂), -COO-R^{a}, qui peuvent être liés pour former un noyau,
aryle, hétéroaryle, hétérocycle et cycloalkyle étant éventuellement substitués par un ou plusieurs substituants choisis parmi : halogène, OH, alkyle en C₁-C₆, alcoxy en C₁-C₆, -O-CO-(alkyle en C₁-C₆), cycloalkyle en C₃-C₆, halogénoalkyle en C₁-C₂ et hétérocycle en C₂-C₄ ;
R⁴ est choisi parmi : halogène, OH, CN, NO₂, NH₂, NHMe, NMe₂, SH, SMe, SOMe, SO₂Me, alkyle en C₁-C₄, cycloalkyle en C₃-C₄, alcoxy en C₁-C₃, hétéroalkyle en C₁-C₃, hétérocycle,
dont l'un quelconque peut être éventuellement substitué par un ou plusieurs substituants choisis parmi : halogène, =O, OH, SMe, alcoxy en C₁-C₂, O-(halogénoalkyle en C₁-C₂), alkyle en C₁-C₂, hétéroalkyle en C₁-C₂, halogénoalkyle en C₁-C₂ NH₂, NHMe, NMe₂, qui peuvent être liés pour former un noyau ;
R¹⁴ est C=O, C=S, C=N-OH, C=NR^{b}, C=CH-NO₂, SO₂, et cycloalcényle en C₃-C₆ qui peut être substitué par 1 ou 2 =O ;
R^{a} est choisi parmi : H, alkyle en C₁-C₈, hétéroalkyle, alcoxy en C₁-C₈, hétérocycle, cycloalkyle en C₃-C₈, hétéroaryle et aryle,
dont l'un quelconque peut être éventuellement substitué par un ou plusieurs substituants choisis parmi : halogène, =O, OH, SMe, alcoxy en C₁-C₂, -O-(halogénoalkyle en C₁-C₂), alkyle en C₁-C₂, cycloalkyle en C₃-C₆, hétéroalkyle en C₁-C₂, halogénoalkyle en C₁-C₂, aryle, hétéroaryle, NH₂, NHMe, NMe₂ qui peuvent être liés pour former un noyau ;
R^{b} est choisi parmi : CN, aryle, hétéroaryle, -NO₂, -O-(alkyle en C₁-C₂), -CO-O-(alkyle en C₁-C₂),
avec aryle et hétéroaryle éventuellement substitués par un ou plusieurs alkyle en C₁-C₂ ;
R^{c} est choisi parmi : H, aryle, hétéroaryle, cycloalcényle en C₃-C₆, cycloalkyle en C₃-C₈, hétérocycle, alkyle en C₁-C₈, hétéroalkyle en C₁-C₈, (alkylène en C₁-C₄)-(cycloalkyle en C₃-C₆),
dont l'un quelconque peut être éventuellement substitué par un ou plusieurs substituants choisis parmi : C(O)(R^{g}), C(O)N(R^{f})(R^{g}), halogène, SF₅, OH, =O, -COH, -COOH, -CO-O-R^{a}, CN, SMe, SO₂Me, SO₂R^{g}, alcoxy en C₁-C₄, O-(halogénoalkyle en C₁-C₄), alkyle en C₁-C₄, hétéroalkyle en C₁-C₂, halogénoalkyle en C₁-C₄, N(R^{f})(R^{g}), N(R^{f})SO₂(R^{g}), SO₂NH₂, SO₂NHMe, SO₂NMe₂, S(O)(NR^{f})R^{g}, cycloalkyle en C₃-C₆, hétérocycle, (alkylène en C₁-C₄)-(cycloalkyle en C₃-C₆), aryle et hétéroaryle, qui peuvent être liés pour former un noyau,
aryle, hétéroaryle, hétérocycle et cycloalkyle étant éventuellement substitués par un ou plusieurs substituants choisis parmi : halogène, =O, =NH, =NMe, OH, alkyle en C₁-C₂, alcoxy en C₁-C₂, -COOH, -COO-(alkyle en C₁-C₂), SO₂R^{a}, COR^{a}, SOR^{a} S(O)(NH)R^{a}, CONHR^{a}, qui peuvent être liés pour former un noyau ;
R^{d} représente H ou alkyle en C₁-C₄,
et R^{c} et R^{d} peuvent être liés pour former un hétérocycle ;
R^{f} est choisi parmi : H, aryle, hétéroaryle, cycloalcényle en C₃-C₆, cycloalkyle en C₃-C₆, hétérocycle, alkyle en C₁-C₈, hétéroalkyle en C₁-C₈, (alkylène en C₁-C₄)-(cycloalkyle en C₃-C₆),
dont l'un quelconque peut être éventuellement substitué par un ou plusieurs substituants choisis parmi : halogène, =O, =NH, =NMe, OH, aryle, hétéroaryle, alkyle en C₁-C₂, halogénoalkyle en C₁-C₂, alcoxy en C₁-C₂, O-(halogénoalkyle en C₁-C₂), - COOH, -COO-(alkyle en C₁-C₂), SO₂R^{a}, COR^{a}, SOR^{a}, S(O)(NH)R^{a}, CONHR^{a} ;
R^{g} est choisi parmi : H, aryle, hétéroaryle, cycloalcényle en C₃-C₆, cycloalkyle en C₃-C₈, hétérocycle, alkyle en C₁-C₈, hétéroalkyle en C₁-C₈, (alkylène en C₁-C₄)-(cycloalkyle en C₃-C₆),
dont l'un quelconque peut être éventuellement substitué par un ou plusieurs substituants choisis parmi : halogène, =O, =NH, =NMe, OH, aryle, hétéroaryle, alkyle en C₁-C₂, halogénoalkyle en C₁-C₂, alcoxy en C₁-C₂, O-(halogénoalkyle en C₁-C₂), - COOH, -COO-(alkyle en C₁-C₂), SO₂R^{a}, COR^{a}, SOR^{a}, S(O)(NH)R^{a}, CONHR^{a},
R^{f} et R^{g} peuvent être liés pour former un noyau.

3. Composé selon la revendication 1 ou 2, ou sel pharmaceutiquement acceptable de celui-ci, consistant en un composé de formule (II) : 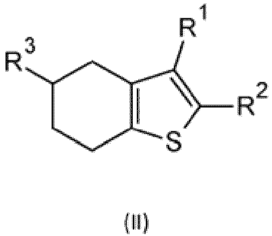 dans laquelle R¹, R² et R³ sont tels que définis dans la revendication 1.

4. Composé selon la revendication 1 ou 2, ou sel pharmaceutiquement acceptable de celui-ci, dans lequel X est C ou N, Z est C, N, O ou S, Y est C ou S.

5. Composé selon la revendication 1 ou 2, ou sel pharmaceutiquement acceptable de celui-ci, dans lequel X, Y, W est C et Z est N, N étant substitué par un groupe choisi parmi : méthylsulfonyle, acétyle, éthylcarbamoyle et cyano.

6. Composé selon la revendication 1 ou 2, ou sel pharmaceutiquement acceptable de celui-ci, dans lequel X, Y, W, Z est C et dans lequel Z est substitué par un groupe choisi parmi : OH, méthyle, éthylcarbamoyle, cyano, oxo, tert-butoxycarbonylamino, 1H-pyrazol-3-yle.

7. Composé selon la revendication 1 ou 2, ou sel pharmaceutiquement acceptable de celui-ci, dans lequel X, Y, W, Z est C, et dans lequel W est substitué par un groupe méthyle et R³ est H ou un groupe méthyle.

8. Composé selon l'une quelconque des revendications 1 à 4 ou sel pharmaceutiquement acceptable de celui-ci, dans lequel X est C ou N et R³ est choisi parmi : H, OH, méthyle, amino, méthylsulfonyle, acétyle, 2-méthoxyacétyle, 2-phénylacétyle, 2-(tert-butoxycarbonylamino)éthyle, 2-phényléthyle, pyrimidin-2-ylamino, méthanesulfonamido, (4-méthoxyphényl)carbamothioylamino, (4-méthoxyphényl)carbamoylamino, éthylcarbamoyle, [*N'*-cyano-N-(p-tolyl)carbamimidoyl]amino, [*N'*-cyano-*N*-éthyl-carbamimidoyl]amino, (1-oxoisoindolin-5-yl)carbamoyle, (3-sulfamoylphényl)carbamoyle, (3-méthylsulfonylphényl)carbamoyle, 3-isoxazol-5-ylanilino, (2-éthoxy-3,4-dioxo-cyclobutén-1-yl)amino, éthylcarbonyle, carboxy, (1-oxoisoindolin-5-yl)aminocarbonyle, [(5-oxopyrrolidin-3-yl)phényl]aminocarbonyle, [3-(3-méthyl-5-oxo-4H-pyrazol-1-yl)phényl]carbonylamino, (1H-indazol-4-yl)carbonylamino, 1H-imidazo[4,5-b]pyrazin-2-yle, indan-2-yle, [2-(4-méthoxyphényl)éthylaminocarbonyle, quinoxalin-6-yle, (thiazol-2-yl)aminocarbonyle, (1H-tétrazol-5-yl)aminocarbonyle, (4-pyridyl)aminocarbonyle, pyrazin-2-ylaminocarbonyle, (3-pyridyl)aminocarbonyle, 3-hydroxyazétidine-1-carbonyle, 1,1-dioxo-1,4-thiazinane-4-carbonyle, 2-(4-hydroxyphényl)éthylaminocarbonyle, hydroxyiminométhyle, [(4-éthyl-1,2,4-triazol-3-yl)amino]méthyle, (4-méthylanilino)méthyle, 2-amino-3-pyridyle, 5-(p-tolyl)-1H-imidazol-2-yle, cyano, trifluorométhyle, 4-méthyloxazol-2-yle, aminométhyle, (p-tolylcarbamoylamino)méthyle, 3-méthyl-1,2,4-oxadiazol-5-yle, 5-méthyl-1,3,4-oxadiazol-2-yle, pyrrolidine-1-carbonyle, 3-pyridylcarbamothioylamino, 3-(3-pyridylamino)-1,2,4-triazol-4-yle, 1H-pyrazol-3-yle, 5-amino-4-cyano-imidazol-1-yle, 3-(2,2-diméthylpropylamino)-1,2,4-triazol-4-yle, 3-[(2,5-diméthylpyrazol-3-yl)amino]-1,2,4-triazol-4-yle, 4-(2,5-diméthylpyrazol-3-yl)-1,2,4-triazol-3-yl]amino, [(2,5-diméthylpyrazol-3-yl)amino]-1,2,4-triazol-4-yle, N'-cyano-N-phényl-carbamimidoylamino, 3-(isobutylamino)-1,2,4-triazol-4-yle, 3-[(1-méthylcyclopropyl)méthylamino]-1,2,4-triazol-4-yle, 5-acétamidoimidazol-1-yle, 3-[(5-méthyl-3-pyridyl)amino]-1,2,4-triazol-4-yle, 3-(4-pyridylamino)-1,2,4-triazol-4-yle, N'-cyano-N-(3,4-diméthoxyphényl)carbamimidoyl]amino, 4-éthoxycarbonyl-5-(éthylamino)imidazole-1-yle, 3-(2-morpholinoéthylamino)-1,2,4-triazol-4-yle, (5-oxo-4H-1,2,4-oxadiazol-3-yl)amino, 2-(éthylamino)-3,4-dioxo-cyclobutén-1-yl]amino, [N'-cyano-N-(2,2,2-trifluoroéthyl)carbamimidoyl]amino, 3-(éthylamino)-1,2,4-triazol-4-yle, (N-éthyl-N'-nitro-carbamimidoyl)amino, 3-(3-pyridylamino)-1,2,4-triazol-4-yle, 3-[(1-oxidopyridin-1-ium-3-yl)amino, [N'-nitro-N-(p-tolyl)carbamimidoyl]amino, 2-(3-pyridylamino)imidazol-1-yle, 3-(3-pyridylamino)-1,2,4-triazol-4-yle, 3-(cyclopropylméthylamino, (2,5-diméthylpyrazol-3-yl)carbamothioylamino, 3-(pyrimidin-5-ylamino)-1,2,4-triazol-4-yle, 2-(cyclopropylméthylamino)imidazol-1-yle, [4-(éthylamino)-1,1-dioxo-1,2,5-thiadiazol-3-yl]amino, [1-(4-méthylanilino)-2-nitro-vinyl]amino, (N'-cyano-N-éthyl-carbamimidoyl)méthyl-amino, [2-(4-méthylanilino)-3,4-dioxo-cyclobutén-1-yl]amino, 3-(2-pyridylamino)-1,2,4-triazol-4-yle, 2-acétamidoimidazol-1-yle, 5-(N-tert-butyloxycarbonylamino)-triazol-1-yle, 5-(3-pyridylamino)triazol-1-yle, éthylcarbamothioylamino, 3-[[(3S)-tétrahydrofuran-3-yl]amino]-1,2,4-triazol-4-yle, [(E)-N-éthyl-N'-nitro-carbamimidoyl]amino, 2-(éthylamino)imidazol-1-yle, 3-[(3,5-diméthylisoxazol-4-yl)amino], [[N'-éthoxycarbonyl-N-(p-tolyl)carbamimidoyl]amino], [2-[(2-méthyl-3-pyridyl)amino]imidazol-1-yle, 1-[(éthylamino)-2-nitro-vinyl]amino, (4-méthyl-6-oxo-1H-pyrimidin-2-yl)amino, 5-amino-4-(méthylcarbamoyl)imidazol-1-yle, 5-amino-4-carbamoyl-imidazol-1-yle, 2-anilinoimidazol-1-yle, éthylcarbamoylamino, (4-oxo-1H-pyridin-2-yl)amino, [6-(5-méthyl-1,3,4-oxadiazol-2-yl)-3-pyridyl]amino, 5-(éthylamino)tétrazol-1-yle, 1H-1,2,4-triazol-3-ylamino, 4-(1-méthylpyrazol-4-yl)-1,2,4-triazol-3-yl]amino, 4-(éthyloxycarbonyl)imidazol-1-yle, [4-(4-fluorophényl)-1,2,4-triazol-3-yl]amino, 1H-imidazol-2-ylamino, N'-méthoxy-N-(p-tolyl)carbamimidoyl]amino, [4-(4-méthylanilino)-1,1-dioxo-1,2,5-thiadiazol-3-yl]amino, [1-(3-pyridyl)imidazol-2-yl]amino, 5-aminotriazol-1-yle, (2-méthoxyphényl)carbamoylamino, 4-pyridine-carbonylamino, benzylamino, 1H-benzimidazol-2-ylamino, [N'-hydroxycarbamimidoyl]amino, [N'-(1-méthylpyrazol-3-yl)-N-(p-tolyl)carbamimidoyl]amino, 3-[(3,5-diméthylpyrazin-2-yl)amino, 3-(éthylamino)-5-(méthoxyméthyl)-1,2,4-triazol-4-yle, [5-(3-pyridyl)-1H-1,2,4-triazol-3-yl]amino, p-tolyl-carbonylamino, quinazolin-2-ylamino, 1,3-benzoxazol-2-ylamino, 3-(éthylamino)-5-(hydroxyméthyl)-1,2,4-triazol-4-yle, 2-aminoimidazol-1-yle, 3-amino-5-oxo-1,2,4-oxadiazol-4-yle, 3-(éthylamino)-5-méthyl-1,2,4-triazol-4-yle, (2-éthyl-3,4-dioxo-cyclobutén-1-yl)amino, 1,3,4-oxadiazol-2-ylamino, 5-oxo-1-(p-tolyl)-4H-imidazol-2-yl]amino, [2-[éthyl(méthyl)amino]-3,4-dioxo-cyclobutén-1-yl]amino, (méthylsulfonimidoyl)amino, [N-cyano-C-méthyl-carbonimidoyl]amino, tétrazol-1-yle, méthanesulfinamido, morpholino, 3-(éthylamino)-5-(trifluorométhyl)-1,2,4-triazol-4-yle, (4-oxo-1H-pyrimidin-2-yl)amino, isopropylsulfamoylamino, *N'-*cyano-*N*-morpholino-carbamimidoyl]amino, acétamido, méthanesulfonamido, [N'-cyano-N-méthoxy-carbamimidoyl]amino, (4-méthoxyphényl)carbamoylamino, [4-oxo-6-(trifluorométhyl)-1H-pyrimidin-2-yl]amino, [4-(trifluorométhoxy)phényl]carbamoylamino, 3-(2-méthylpropanoylamino)-1,2,4-triazol-4-yle, 3-[(2,5-diméthylpyrazol-3-y)-méthyl-amino]-1,2,4-triazol-4-yle, 5-amino-4-(5-méthyl-1,3,4-oxadiazol-2-yl)imidazol-1-yle, 3-[(3-méthyl-1H-pyrazol-5-yl)amino]-1,2,4-triazol-4-yle, 3-[(1,3-diméthylpyrazol-4-yl)amino]-1,2,4-triazol-4-yle, -[(5-cyclopropyl-2-méthyl-pyrazol-3-yl)amino]-1,2,4-triazol-4-yle, 3-[(5-isopropyl-2-méthyl-pyrazol-3-yl)amino]-1,2,4-triazol-4-yle, 3-[[2-méthyl-5-(trifluorométhyl)pyrazoI-3-yl]amino]-1,2,4-triazol-4-yle, 3-[(2-isopropyl-5-méthylpyrazol-3-yl)amino, 3-[(5-méthyl-2-tétrahydrofuran-3-yl-pyrazol-3-yl)amino]-1,2,4-triazol-4-yle, 3-[(2-méthylpyrazol-3-yl)amino]-1,2,4-triazol-4-yle, 3-[(2-éthyl-5-méthyl-pyrazol-3-yl)amino]-1,2,4-triazol-4-yle, 3-[(2-cyclopropyl-5-méthyl-pyrazol-3-yl)amino]-1,2,4-triazol-4-yle, 3-[(3-méthoxy-1-méthyl-pyrazol-4-yl)amino]-1,2,4-triazol-4-yle, 3-[(5-éthyl-2-méthyl-pyrazol-3-yl)amino]-1,2,4-triazol-4-yle, 3-[[5-(difluorométhyl)-2-méthyl-pyrazol-3-yl]amino, 3-[(1,4-diméthylpyrazol-3-yl)amino]-1,2,4-triazol-4-yle, [3-[(5-méthoxycarbonyl-2-méthyl-pyrazol-3-yl)amino]-1,2,4-triazol-4-yle], 2-[[2-méthyl-5-(trifluorométhyl)pyrazol-3-yl]amino]imidazol-1-yle, [3-[(5-carboxy-2-méthyl-pyrazol-3-yl)amino]-1,2,4-triazol-4-yle].

9. Composés selon la revendication 1, ou sel pharmaceutiquement acceptable de ceux-ci, dans lesquels W, X, Y, Z est C et Z est éventuellement substitué par un groupe méthyle.

10. Composés selon les revendications 1 à 4, ou sel pharmaceutiquement acceptable de ceux-ci, dans lesquels
R³ est choisi parmi : (1,1-dioxothiolan-3-yl)amino-1,2,4-triazol-4-yle, 3-[3-[méthylsulfonimidoyl]anilino]-1,2,4-triazol-4-yle, 3-[(1-acétyl-3-méthylpyrrolidin-3-yl)amino]-4H-1,2,4-triazol-4-yle, 3-{[1-(6-méthylpyridazin-3-yl)pyrrolidin-3-yl]amino}-4H-1,2,4-triazol-4-yle, 3-[[-1-pyrazin-2-ylpyrrolidin-3-yl]amino]-1,2,4-triazol-4-yle, 3-[(4-méthyl-1-méthylsulfonylpyrrolidin-3-yl)amino]-1,2,4-triazol-4-yle, 3-[(1-oxo-2,3-dihydro-1H-isoindol-4-yl)amino]-4H-1,2,4-triazol-4-yle, 3-[[-1-(6-fluoropyridazin-3-yl)pyrrolidin-3-yl]amino]-1,2,4-triazol-4-yle, 3-[[1-pyrimidin-2-ylpyrrolidin-3-yl]amino]-1,2,4-triazol-4-yle, 5-amino-4-(2-méthoxyéthoxycarbonyl)imidazol-1-yle, 5-amino-4-(2-morpholinéthoxycarbonyl)imidazol-1-yle, 3-(3-méthylsulfanylanilino)-1,2,4-triazol-4-yle, 3-(3-méthylsulfonylanilino)-1,2,4-triazol-4-yle, 3-(3-méthylsulfinylanilino)-1,2,4-triazol-4-yle, 3-[3-(méthylsulfonimidoyl)anilino]-1,2,4-triazol-4-yle, 3-[(6-méthylsulfinyl-2-pyridyl)amino]-1,2,4-triazol-4-yle, 3-[(6-méthylsulfonyl-2-pyridyl)amino]-1,2,4-triazol-4-yle, 8-méthoxy-4-oxo-5,10-dihydroimidazo[4,5-c][1,5]benzodiazépin-1-yle, 8-méthoxy-5-méthyl-4-oxo-10H-imidazo[4,5-c][1,5]benzodiazépin-1-yle, 5-amino-4-[(2S)-2-méthylpyrrolidine-1-carbonyl]imidazol-1-yle, 5-amino-4-[(3R)-3-hydroxypyrrolidine-1-carbonyl]imidazol-1-yle, 5-amino-4-[(2S)-2-méthylpyrrolidine-1-carbonyl]imidazol-1-yle, 5-[[4-(dihydroxyamino)-2,5-diméthyl-3H-pyrazol-3-yl]amino]-4-éthoxycarbonyl-imidazol-1-yle, 5-[[4-(dihydroxyamino)-2,5-diméthyl-3H-pyrazol-3-yl]amino]-4-éthoxycarbonyl-imidazol-1-yle, 3-(2-méthyl-3-méthylsulfinyl-anilino)-1,2,4-triazol-4-yle, 3-(2-méthyl-3-méthylsulfonyl-anilino)-1,2,4-triazol-4-yle, 3-[(2-méthyl-5-oxazol-2-yl-pyrazol-3-yl)amino]1,2,4-triazol-4-yle, 1-acétylpyrrolidin-3-yl]amino]-1,2,4triazol-4-yle, 1-méthylsulfonylpyrrolidin-3-yl)amino]-1,2,4-triazol-4-yle, 3-[(2-méthylcyclohexyl)amino]-1,2,4-triazol-4-yle, 3-[(5-oxopyrrolidin-3-yl)amino]-1,2,4-triazol-4-yle, 3-[(2-oxo-4-pipéridyl)amino]-1,2,4-triazol-4-yle, 3-[(1-acétyl-2-méthyl-pyrrolidin-3-yl)amino]-1,2,4-triazol-4-yle, 3-[(2-méthyl-1-méthylsulfonyl-pyrrolidin-3-yl)amino]-1,2,4-triazol-4-yle, -[3-[[3-(6-méthyl-3-pyridyl)cyclobutyl]amino]-1,2,4-triazol-4-yle, 3-[[5-(difluorométhoxy)-2-méthyl-pyrazol-3-yl]amino]-1,2,4-triazol-4-yle, [[1-benzoyl-4,4-difluoro-pyrrolidin-3-yl]amino]-1,2,4-triazol-4-yle, 3-[(1-acétyl-4-pipéridyl)amino]-1,2,4-triazol-4-yle, 3-[(1-méthoxycarbonylpyrrolidin-3-yl)amino]-1,2,4-triazol-4-yle, 3-[[1-(éthylcarbamoyl)pyrrolidin-3-yl]amino]-1,2,4-triazol-4-yle, 3-{[2-(pyrimidin-2-yl)-2-azaspiro[4.4]nonan-7-yl]amino}-4H-1,2,4-triazol-4-yle, 3-[[2-méthyl-5-(trifluorométhoxy)pyrazol-3-yl]amino]-1,2,4-triazol-4-yle, 3-[[5-[chloro(difluoro)méthoxy]-2-méthyl-pyrazol-3-yl]amino]-1,2,4-triazol-4-yle, 3-{[1-méthyl-3-(5-méthyl-1,3,4-oxadiazol-2-yl)-1H-pyrazol-5-yl]amino}-4H-1,2,4-triazol-4-yle, 3-[(5-méthoxy-3-méthyl-pyrazin-2-yl)amino]-1,2,4-triazol-4-yle, (5-méthylthiazolo[5,4-b]pyridin-2-yl)amino, 3-[(5-cyano-2-méthyl-3-pyridyl)amino]-1,2,4-triazol-4-yle, 3-[(6-cyano-2-méthyl-3-pyridyl)amino]-1,2,4-triazol-4-yle, 3-[(1-méthyl-2-oxo-4-pyridyl)amino]-1,2,4-triazol-4-yle, 3-[(6-chloro-2-méthyl-3-pyridyl)amino]-1,2,4-triazol-4-yle, 3-[[2-méthyl-6-(trifluorométhyl)-3-pyridyl]amino]-1,2,4-triazol-4-yle, 3-[(3-méthylpyridazin-4-yl)amino]-1,2,4-triazol-4-yle, 3-[(5-chloro-2-méthyl-pyrazol-3-yl)amino]-1,2,4-triazol-4-yle, 3-[(3-méthylisothiazol-4-yl)amino]-1,2,4-triazol-4-yle, 3-[(3-méthylisoxazol-4-yl)amino]-1,2,4-triazol-4-yle, 3-[[5-(cyclopropylméthoxy)-2-méthyl-pyrazol-3-yl]amino]-1,2,4-triazol-4-yle, 3-[(5-éthoxy-2-méthyl-pyrazol-3-yl)amino]-1,2,4-triazol-4-yle, 3-[(2,5-diméthylpyrazol-3-yl)amino]-1,2,4-triazol-4-yle, (5-amino-4-éthoxycarbonyl-imidazol-1-yle), 3-[[5-(difluorométhoxy)-2-méthylpyrazol-3-yl]amino]-1,2,4-triazol-4-yle, 3-[[5-(difluorométhyl)-2-méthylpyrazol-3-yl]amino]-1,2,4-triazol-4-yle, 3-[(5-méthoxy-2-méthylpyrazol-3-yl)amino]-1,2,4-triazol-4-yle, 3-[[5-(difluorométhyl)-2-(trideutériométhyl)pyrazol-3-yl]amino], 3-[(5-cyano-2-méthyl-pyrazol-3-yl)amino]-1,2,4-triazol-4-yle, 3-[(2,2-difluoro-1,3-benzodioxol-5-yl)amino]-1,2,4-triazol-4-yle, 3-[(5-méthoxy-2-méthyl-pyrazol-3-yl)amino]-1,2,4-triazol-4-yle, 3-[[5-(difluorométhyl)-2-(trideutériométhyl)pyrazol-3-yl]amino]-1,2,4-triazol-4-yle, 3-[(6-fluoro-2-méthyl-3-pyridyl)amino]-1,2,4-triazol-4-yle, 3-[[5-(cyclopropoxy)-2-méthyl-pyrazol-3-yl]amino]-1,2,4-triazol-4-yle, 3-[[2-méthyl-5-(2,2,2-trifluoroéthoxy)pyrazol-3-yl]amino]-1,2,4-triazol-4-yle, 3-[[6-(trifluorométhyl)-3-pyridyl]amino]-1,2,4-triazol-4-yle, 3-[(6-méthoxy-3-pyridyl)amino]-1,2,4-triazol-4-yle, 3-[(2-méthoxy-4-pyridyl)amino]-1,2,4-triazol-4-yle, 3-[(6-chloro-3-pyridyl)amino]-1,2,4-triazol-4-yle, 3-[(2-chloro-4-pyridyl)amino]-1,2,4-triazol-4-yle, 3-[(6-méthoxy-2-méthyl-3-pyridyl)amino]-1,2,4-triazol-4-yle, thiazolo[5,4-b]pyridin-2-ylamino, 3-[(3-méthyltriazol-4-yl)amino]-1,2,4-triazol-4-yle.

11. Composé selon la revendication 1 ou sel pharmaceutiquement acceptable de celui-ci, ledit composé étant choisi parmi :
chlorhydrate de 2-(cyclopropanecarbonylamino)-*N*-(cyclopropylméthyl)-4,5,6,7-tétrahydrothiéno[3,2-*c*]pyridine-3-carboxamide,
2-(cyclopropanecarbonylamino)-*N*-(cyclopropylméthyl)-5-méthylsulfonyl-6,7-dihydro-4*H*-thiéno[3,2-*c*]pyridine-3-carboxamide,
5-acétyl-2-(cyclopropanecarbonylamino)-*N*-(cyclopropylméthyl)-6,7-dihydro-4*H-*thiéno[3,2-*c*]pyridine-3-carboxamide,
2-(cyclopropanecarbonylamino)-*N*-(cyclopropylméthyl)-7-méthylsulfonyl-5,6-dihydro-4*H*-thiéno[2,3-*b*]pyridine-3-carboxamide,
7-acétyl-2-(cyclopropanecarbonylamino)-*N*-(cyclopropylméthyl)-5,6-dihydro-4*H-*thiéno[2,3-*b*]pyridine-3-carboxamide,
2-(cyclopropanecarbonylamino)-*N*-(cyclopropylméthyl)-5-(2-méthoxyacétyl)-6,7-dihydro-4*H*-thiéno[3,2-*c*]pyridine-3-carboxamide,
2-(cyclopropanecarbonylamino)-*N*-(cyclopropylméthyl)-5-(2-phénylacétyl)-6,7-dihydro-4*H*-thiéno[3,2-*c*]pyridine-3-carboxamide,
*N*-[2-[2-(cyclopropanecarbonylamino)-3-(cyclopropylméthylcarbamoyl)-6,7-dihydro-4*H*-thiéno[3,2-*c*]pyridin-5-yl]éthyl]carbamate de *tert*-butyle,
2-(cyclopropanecarbonylamino)-*N*-(cyclopropylméthyl)-5-(2-phényléthyl)-6,7-dihydro-4*H*-thiéno[3,2-*c*]pyridine-3-carboxamide,
2-[2-(cyclopropanecarbonylamino)-3-(cyclopropylméthylcarbamoyl)-6,7-dihydro-4*H-*thiéno[3,2-*c*]pyridin-5-yl]acétate de méthyle,
(*4R*)-2-(cyclopropanecarbonylamino)-*N*-(cyclopropylméthyl)-4-méthyl-5,6-dihydro-4*H*-cyclopenta[*b*]thiophène-3-carboxamide,
(*4S*)-2-(cyclopropanecarbonylamino)-*N*-(cyclopropylméthyl)-4-méthyl-5,6-dihydro-4*H*-cyclopenta[*b*]thiophène-3-carboxamide,
2-(cyclopropanecarbonylamino)-3-(cyclopropylméthylcarbamoyl)-4,5,6,7-tétrahydrobenzothiophène-5-carboxylate d'éthyle,
acide 2-(cyclopropanecarbonylamino)-3-(cyclopropylméthylcarbamoyl)-4,5,6,7-tétrahydrobenzothiophène-5-carboxylique,
2-(cyclopropanecarbonylamino)-N3-(cyclopropylméthyl)-N5-(1-oxoisoindolin-5-yl)-4,5,6,7-tétrahydrobenzothiophène-3,5-dicarboxamide,
2-(cyclopropanecarbonylamino)-N3-(cyclopropylméthyl)-N5-[3-(5-oxopyrrolidin-3-yl)phényl]-4,5,6,7-tétrahydrobenzothiophène-3,5-dicarboxamide,
2-(cyclopropanecarbonylamino)-N3-(cyclopropylméthyl)-N5-[3-(3-méthyl-5-oxo-4H-pyrazol-1-yl)phényl]-4,5,6,7-tétrahydrobenzothiophène-3,5-dicarboxamide,
2-(cyclopropanecarbonylamino)-N3-(cyclopropylméthyl)-N5-(1H-indazol-4-yl)-4,5,6,7-tétrahydrobenzothiophène-3,5-dicarboxamide, acide 2,2,2-trifluoroacétique,
2-(cyclopropanecarbonylamino)-N3-(cyclopropylméthyl)-N5-(1H-indazol-5-yl)-4,5,6,7-tétrahydrobenzothiophène-3,5-dicarboxamide, acide 2,2,2-trifluoroacétique,
2-(cyclopropanecarbonylamino)-N-(cyclopropylméthyl)-5-(1H-imidazo[4,5-b]pyrazin-2-yl)-4,5,6,7-tétrahydrobenzothiophène-3-carboxamide,
2-(cyclopropanecarbonylamino)-N3-(cyclopropylméthyl)-N5-indan-2-yl-4,5,6,7-tétrahydrobenzothiophène-3,5-dicarboxamide,
2-(cyclopropanecarbonylamino)-N3-(cyclopropylméthyl)-N5-[2-(4-méthoxyphényl)éthyl]-4,5,6,7-tétrahydrobenzothiophène-3,5-dicarboxamide,
2-(cyclopropanecarbonylamino)-N3-(cyclopropylméthyl)-N5-quinoxalin-6-yl-4,5,6,7-tétrahydrobenzothiophène-3,5-dicarboxamide,
2-(cyclopropanecarbonylamino)-N3-(cyclopropylméthyl)-N5-thiazol-2-yl-4,5,6,7-tétrahydrobenzothiophène-3,5-dicarboxamide,
2-(cyclopropanecarbonylamino)-N3-(cyclopropylméthyl)-N5-(1H-tétrazol-5-yl)-4,5,6,7-tétrahydrobenzothiophène-3,5-dicarboxamide,
2-(cyclopropanecarbonylamino)-N3-(cyclopropylméthyl)-N5-(4-pyridyl)-4,5,6,7-tétrahydrobenzothiophène-3,5-dicarboxamide,
2-(cyclopropanecarbonylamino)-N3-(cyclopropylméthyl)-N5-pyrazin-2-yl-4,5,6,7-tétrahydrobenzothiophène-3,5-dicarboxamide,
2-(cyclopropanecarbonylamino)-N3-(cyclopropylméthyl)-N5-(3-pyridyl)-4,5,6,7-tétrahydrobenzothiophène-3,5-dicarboxamide,
2-(cyclopropanecarbonylamino)-N-(cyclopropylméthyl)-5-(3-hydroxyazétidine-1-carbonyl)-4,5,6,7-tétrahydrobenzothiophène-3-carboxamide,
2-(cyclopropanecarbonylamino)-N-(cyclopropylméthyl)-5-(1,1-dioxo-1,4-thiazinane-4-carbonyl)-4,5,6,7-tétrahydrobenzothiophène-3-carboxamide,
2-(cyclopropanecarbonylamino)-N3-(cyclopropylméthyl)-N5-[2-(4-hydroxyphényl)éthyl]-4,5,6,7-tétrahydrobenzothiophène-3,5-dicarboxamide,
2-(cyclopropanecarbonylamino)-N-(cyclopropylméthyl)-5-(hydroxyiminométhyl)-4,5,6,7-tétrahydrobenzothiophène-3-carboxamide,
2-(cyclopropanecarbonylamino)-N-(cyclopropylméthyl)-5-[[(4-éthyl-1,2,4-triazol-3-yl)amino]méthyl]-4,5,6,7-tétrahydrobenzothiophène-3-carboxamide,
2-(cyclopropanecarbonylamino)-N-(cyclopropylméthyl)-5-hydroxy-5-[(4-méthylanilino)méthyl]-6,7-dihydro-4H-benzothiophène-3-carboxamide,
2-(cyclopropanecarbonylamino)-N-(cyclopropylméthyl)-2'-oxo-3'-(p-tolyl)spiro[6,7-dihydro-4H-benzothiophène-5,5'-oxazolidine]-3-carboxamide,
5-(2-amino-3-pyridyl)-2-(cyclopropanecarbonylamino)-N-(cyclopropylméthyl)-4,5,6,7-tétrahydrobenzothiophène-3-carboxamide,
2-(cyclopropanecarbonylamino)-N-(cyclopropylméthyl)-5-[5-(p-tolyl)-1H-imidazol-2-yl]-4,5,6,7-tétrahydrobenzothiophène-3-carboxamide,
5-cyano-2-(cyclopropanecarbonylamino)-N-(cyclopropylméthyl)-4,5,6,7-tétrahydrobenzothiophène-3-carboxamide,
7-cyano-2-(cyclopropanecarbonylamino)-N-(cyclopropylméthyl)-4,5,6,7-tétrahydrobenzothiophène-3-carboxamide,
(5R)-2-(cyclopropanecarbonylamino)-N-(cyclopropylméthyl)-5-(trifluorométhyl)-4,5,6,7-tétrahydrobenzothiophène-3-carboxamide,
(5S)-2-(cyclopropanecarbonylamino)-N-(cyclopropylméthyl)-5-(trifluorométhyl)-4,5,6,7-tétrahydrobenzothiophène-3-carboxamide,
2-(cyclopropanecarbonylamino)-N-(cyclopropylméthyl)-5-(4-méthyloxazol-2-yl)-4,5,6,7-tétrahydrobenzothiophène-3-carboxamide,
5-(aminométhyl)-2-(cyclopropanecarbonylamino)-N-(cyclopropylméthyl)-5-hydroxy-6,7-dihydro-4H-benzothiophène-3-carboxamide,
2-(cyclopropanecarbonylamino)-N-(cyclopropylméthyl)-5-hydroxy-5-[(p-tolylcarbamoylamino)méthyl]-6,7-dihydro-4H-benzothiophène-3-carboxamide,
2-(cyclopropanecarbonylamino)-N-(cyclopropylméthyl)-5-(3-méthyl-1,2,4-oxadiazol-5-yl)-4,5,6,7-tétrahydrobenzothiophène-3-carboxamide,
2-(cyclopropanecarbonylamino)-N-(cyclopropylméthyl)-5-(5-méthyl-1,3,4-oxadiazol-2-yl)-4,5,6,7-tétrahydrobenzothiophène-3-carboxamide,
2-(cyclopropanecarbonylamino)-N-(cyclopropylméthyl)-5-(pyrrolidine-1-carbonyl)-4,5,6,7-tétrahydrobenzothiophène-3-carboxamide,
2-(cyclopropanecarbonylamino)-N-(cyclopropylméthyl)-5-hydroxy-5-méthyl-6,7-dihydro-4H-benzothiophène-3-carboxamide,
2-(cyclopropanecarbonylamino)-N-(cyclopropylméthyl)-4-méthyl-4,5,6,7-tétrahydrobenzothiophène-3-carboxamide,
2-(cyclopropanecarbonylamino)-N-(cyclopropylméthyl)-5,7-diméthyl-4,5,6,7-tétrahydrobenzothiophène-3-carboxamide,
2-(cyclopropanecarbonylamino)-N-(cyclopropylméthyl)-5-méthyl-4,5,6,7-tétrahydrobenzothiophène-3-carboxamide,
2-(cyclopropanecarbonylamino)-N-(cyclopropylméthyl)-7-méthyl-4,5,6,7-tétrahydrobenzothiophène-3-carboxamide,
2-(cyclopropanecarbonylamino)-N-(cyclopropylméthyl)-5,5-diméthyl-6,7-dihydro-4H-benzothiophène-3-carboxamide,
2-(cyclopropanecarbonylamino)-N-(cyclopropylméthyl)-5-méthyl-7-oxo-5,6-dihydro-4H-benzothiophène-3-carboxamide,
2'-(cyclopropanecarbonylamino)-N-(cyclopropylméthyl)spiro[1,3-dioxolane-2,5'-6,7-dihydro-4H-benzothiophène]-3'-carboxamide,
2-(cyclopropanecarbonylamino)-N-(cyclopropylméthyl)-4,5-diméthyl-4,5,6,7-tétrahydrobenzothiophène-3-carboxamide,
2-(cyclopropanecarbonylamino)-N-(cyclopropylméthyl)-2',5'-dioxo-spiro[6,7-dihydro-4H-benzothiophène-5,4'-imidazolidine]-3-carboxamide,
N-[2-(cyclopropanecarbonylamino)-3-(cyclopropylméthylcarbamoyl)-4,5,6,7-tétrahydrobenzothiophén-7-yl]carbamate de tert-butyle,
2-(cyclopropanecarbonylamino)-N3-(cyclopropylméthyl)-N7-éthyl-4,5,6,7-tétrahydrobenzothiophène-3,7-dicarboxamide,
2-(cyclopropanecarbonylamino)-N-(cyclopropylméthyl)-5-(1H-pyrazol-3-yl)-4,5,6,7-tétrahydrobenzothiophène-3-carboxamide,
2-(cyclopropanecarbonylamino)-N-(cyclopropylméthyl)-7-(1H-pyrazol-3-yl)-4,5,6,7-tétrahydrobenzothiophène-3-carboxamide,
(5S)-5-(5-amino-4-cyano-imidazol-1-yl)-2-(cyclopropanecarbonylamino)-N-(cyclopropylméthyl)-4,5,6,7-tétrahydrobenzothiophène-3-carboxamide,
(5S)-2-(cyclopropanecarbonylamino)-N-(cyclopropylméthyl)-5-[3-(2,2-diméthylpropylamino)-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[(2,5-diméthylpyrazol-3-yl)amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[[4-(2,5-diméthylpyrazol-3-yl)-1,2,4-triazol-3-yl]amino]-4,5,6,7-tétrahydrobenzothiophène-3-carboxamide,
5-amino-2-(cyclopropanecarbonylamino)-N-(cyclopropylméthyl)-4,5,6,7-tétrahydrobenzothiophène-3-carboxamide,
5-[[N'-cyano-N-phényl-carbamimidoyl]amino]-2-(cyclopropanecarbonylamino)-N-(cyclopropylméthyl)-4,5,6,7-tétrahydrobenzothiophène-3-carboxamide,
(5S)-2-(cyclopropanecarbonylamino)-N-(cyclopropylméthyl)-5-[3-(isobutylamino)-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[(1-méthylcyclopropyl)méthylamino]-1,2,4-triazol-4-yl]-4,5,6,7-tétrahydrobenzothiophène-3-carboxamide,
(5S)-5-(5-acétamidoimidazol-1-yl)-2-(cyclopropanecarbonylamino)-N-(cyclopropylméthyl)-4,5,6,7-tétrahydrobenzothiophène-3-carboxamide,
(5S)-2-(cyclopropanecarbonylamino)-N-(cyclopropylméthyl)-5-[3-[(5-méthyl-3-pyridyl)amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-(4-pyridylamino)-1,2,4-triazol-4-yl]-4,5,6,7-tétrahydrobenzothiophène-3-carboxamide,
5-[[N'-cyano-N-(3,4-diméthoxyphényl)carbamimidoyl]amino]-2-(cyclopropanecarbonylamino)-N-(cyclopropylméthyl)-4,5,6,7-tétrahydrobenzothiophène-3-carboxamide,
5-amino-1-[(5S)-2-(cyclopropanecarbonylamino)-3-(cyclopropylméthylcarbamoyl)-4,5,6,7-tétrahydrobenzothiophén-5-yl]imidazole-4-carboxylate d'éthyle,
(5S)-2-(cyclopropanecarbonylamino)-N-(cyclopropylméthyl)-5-[3-(2-morpholinoéthylamino)-1,2,4-triazol-4-yl]-4,5,6,7-tétrahydrobenzothiophène-3-carboxamide,
2-(cyclopropanecarbonylamino)-N-(cyclopropylméthyl)-5-[(5-oxo-4H-1,2,4-oxadiazol-3-yl)amino]-4,5,6,7-tétrahydrobenzothiophène-3-carboxamide,
2-(cyclopropanecarbonylamino)-N-(cyclopropylméthyl)-5-[[2-(éthylamino)-3,4-dioxo-cyclobutén-1-yl]amino]-4,5,6,7-tétrahydrobenzothiophène-3-carboxamide,
5-[[N'-cyano-N-(2,2,2-trifluoroéthyl)carbamimidoyl]amino]-2-(cyclopropanecarbonylamino)-N-(cyclopropylméthyl)-4,5,6,7-tétrahydrobenzothiophène-3-carboxamide,
(5S)-2-(cyclopropanecarbonylamino)-N-(cyclopropylméthyl)-5-[3-(isobutylamino)-1,2,4-triazol-4-yl]-4,5,6,7-tétrahydrobenzothiophène-3-carboxamide,
2-(cyclopropanecarbonylamino)-N-(cyclopropylméthyl)-5-[(N-éthyl-N'-nitro-carbamimidoyl)amino]-4,5,6,7-tétrahydrobenzothiophène-3-carboxamide,
(5S)-2-(cyclopropanecarbonylamino)-N-(cyclopropylméthyl)-5-[3-(3-pyridylamino)-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[(1-oxidopyridin-1-ium-3-yl)amino]-1,2,4-triazol-4-yl]-4,5,6,7-tétrahydrobenzothiophène-3-carboxamide,
2-(cyclopropanecarbonylamino)-N-(cyclopropylméthyl)-5-[[N'-nitro-N-(p-tolyl)carbamimidoyl]amino]-4,5,6,7-tétrahydrobenzothiophène-3-carboxamide,
(5S)-2-(cyclopropanecarbonylamino)-N-(cyclopropylméthyl)-5-[2-(3-pyridylamino)imidazol-1-yl]-4,5,6,7-tétrahydrobenzothiophène-3-carboxamide,
(5S)-2-(cyclopropanecarbonylamino)-N-(cyclopropylméthyl)-5-[3-(cyclopropylméthylamino)-1,2,4-triazol-4-yl]-4,5,6,7-tétrahydrobenzothiophène-3-carboxamide,
(5S)-2-(cyclopropanecarbonylamino)-N-(cyclopropylméthyl)-5-[(2,5-diméthylpyrazol-3-yl)carbamothioylamino]-4,5,6,7-tétrahydrobenzothiophène-3-carboxamide,
(5S)-2-(cyclopropanecarbonylamino)-N-(cyclopropylméthyl)-5-[3-(pyrimidin-5-ylamino)-1,2,4-triazol-4-yl]-4,5,6,7-tétrahydrobenzothiophène-3-carboxamide,
(5S)-2-(cyclopropanecarbonylamino)-N-(cyclopropylméthyl)-5-[2-(cyclopropylméthylamino)imidazol-1-yl]-4,5,6,7-tétrahydrobenzothiophène-3-carboxamide,
2-(cyclopropanecarbonylamino)-N-(cyclopropylméthyl)-5-[[4-(éthylamino)-1,1-dioxo-1,2,5-thiadiazol-3-yl]amino]-4,5,6,7-tétrahydrobenzothiophène-3-carboxamide,
2-(cyclopropanecarbonylamino)-N-(cyclopropylméthyl)-5-[[1-(4-méthylanilino)-2-nitro-vinyl]amino]-4,5,6,7-tétrahydrobenzothiophène-3-carboxamide,
(5S)-5-[(N'-cyano-N-éthyl-carbamimidoyl)-méthyl-amino]-2-(cyclopropanecarbonylamino)-N-(cyclopropylméthyl)-4,5,6,7-tétrahydrobenzothiophène-3-carboxamide,
2-(cyclopropanecarbonylamino)-N-(cyclopropylméthyl)-5-[[2-(4-méthylanilino)-3,4-dioxocyclobutén-1-yl]amino]-4,5,6,7-tétrahydrobenzothiophène-3-carboxamide,
(5S)-2-(cyclopropanecarbonylamino)-N-(cyclopropylméthyl)-5-[3-(2-pyridylamino)-1,2,4-triazol-4-yl]-4,5,6,7-tétrahydrobenzothiophène-3-carboxamide,
(5S)-5-(2-acétamidoimidazol-1-yl)-2-(cyclopropanecarbonylamino)-N-(cyclopropylméthyl)-4,5,6,7-tétrahydrobenzothiophène-3-carboxamide,
N-[3-[2-(cyclopropanecarbonylamino)-3-(cyclopropylméthylcarbamoyl)-4,5,6,7-tétrahydrobenzothiophén-5-yl]triazol-4-yl]carbamate de *tert*-butyle,
2-(cyclopropanecarbonylamino)-N-(cyclopropylméthyl)-5-[5-(3-pyridylamino)triazol-1-yl]-4,5,6,7-tétrahydrobenzothiophène-3-carboxamide,
2-(cyclopropanecarbonylamino)-N-(cyclopropylméthyl)-5-(éthylcarbamothioylamino)-4,5,6,7-tétrahydrobenzothiophène-3-carboxamide,
(5S)-2-(cyclopropanecarbonylamino)-N-(cyclopropylméthyl)-5-[3-[[(3S)-tétrahydrofuran-3-yl]amino]-1,2,4-triazol-4-yl]-4,5,6,7-tétrahydrobenzothiophène-3-carboxamide,
5-[[N'-cyano-N-éthyl-carbamimidoyl]amino]-2-(cyclopropanecarbonylamino)-N-(cyclopropylméthyl)-4,5,6,7-tétrahydrobenzothiophène-3-carboxamide,
(5S)-2-(cyclopropanecarbonylamino)-N-(cyclopropylméthyl)-5-[[(E)-N-éthyl-N'-nitro-carbamimidoyl]amino]-4,5,6,7-tétrahydrobenzothiophène-3-carboxamide,
(5R)-2-(cyclopropanecarbonylamino)-N-(cyclopropylméthyl)-5-[[(E)-N-éthyl-N'-nitrocarbamimidoyl]amino]-4,5,6,7-tétrahydrobenzothiophène-3-carboxamide,
(5S)-2-(cyclopropanecarbonylamino)-N-(cyclopropylméthyl)-5-[2-(éthylamino)imidazol-1-yl]-4,5,6,7-tétrahydrobenzothiophène-3-carboxamide,
(5S)-2-(cyclopropanecarbonylamino)-N-(cyclopropylméthyl)-5-[3-[(3,5-diméthylisoxazol-4-yl)amino]-1,2,4-triazol-4-yl]-4,5,6,7-tétrahydrobenzothiophène-3-carboxamide,
N-[[[2-(cyclopropanecarbonylamino)-3-(cyclopropylméthylcarbamoyl)-4,5,6,7-tétrahydrobenzothiophén-5-yl]amino]-(4-méthylanilino)méthylène]carbamate d'éthyle,
(5S)-2-(cyclopropanecarbonylamino)-N-(cyclopropylméthyl)-5-[2-[(2-méthyl-3-pyridyl)amino]imidazol-1-yl]-4,5,6,7-tétrahydrobenzothiophène-3-carboxamide,
2-(cyclopropanecarbonylamino)-N-(cyclopropylméthyl)-5-[[1-(éthylamino)-2-nitro-vinyl]amino]-4,5,6,7-tétrahydrobenzothiophène-3-carboxamide,
2-(cyclopropanecarbonylamino)-N-(cyclopropylméthyl)-5-[(4-méthyl-6-oxo-1H-pyrimidin-2-yl)amino]-4,5,6,7-tétrahydrobenzothiophène-3-carboxamide,
5-amino-1-[(5S)-2-cyclopropaneamido-3-[(cyclopropylméthyl)carbamoyl]-4,5,6,7-tétrahydro-1-benzothiophén-5-yl]-N-méthyl-1H-imidazole-4-carboxamide,
5-amino-1-[(5S)-2-cyclopropaneamido-3-[(cyclopropylméthyl)carbamoyl]-4,5,6,7-tétrahydro-1-benzothiophén-5-yl]-1H-imidazole-4-carboxamide,
5-(2-anilinoimidazol-1-yl)-2-(cyclopropanecarbonylamino)-N-(cyclopropylméthyl)-4,5,6,7-tétrahydrobenzothiophène-3-carboxamide,
(5S)-5-[[N'-cyano-N-éthyl-carbamimidoyl]amino]-2-(cyclopropanecarbonylamino)-N-(cyclopropylméthyl)-4,5,6,7-tétrahydrobenzothiophène-3-carboxamide,
2-(cyclopropanecarbonylamino)-N-(cyclopropylméthyl)-5-(éthylcarbamoylamino)-4,5,6,7-tétrahydrobenzothiophène-3-carboxamide,
2-(cyclopropanecarbonylamino)-N-(cyclopropylméthyl)-5-[(4-oxo-1H-pyridin-2-yl)amino]-4,5,6,7-tétrahydrobenzothiophène-3-carboxamide,
(5S)-2-(cyclopropanecarbonylamino)-N-(cyclopropylméthyl)-5-[[6-(5-méthyl-1,3,4-oxadiazol-2-yl)-3-pyridyl]amino]-4,5,6,7-tétrahydrobenzothiophène-3-carboxamide,
2-(cyclopropanecarbonylamino)-N-(cyclopropylméthyl)-5-[5-(éthylamino)tétrazol-1-yl]-4,5,6,7-tétrahydrobenzothiophène-3-carboxamide,
2-(cyclopropanecarbonylamino)-N-(cyclopropylméthyl)-5-(1H-1,2,4-triazol-3-ylamino)-4,5,6,7-tétrahydrobenzothiophène-3-carboxamide,
(5R)-2-(cyclopropanecarbonylamino)-N-(cyclopropylméthyl)-5-[3-[(2,5-diméthylpyrazol-3-yl)amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[[4-(1-méthylpyrazol-4-yl)-1,2,4-triazol-3-yl]amino]-4,5,6,7-tétrahydrobenzothiophène-3-carboxamide,
1-[(5S)-2-cyclopropaneamido-3-[(cyclopropylméthyl)carbamoyl]-4,5,6,7-tétrahydro-1-benzothiophén-5-yl]-1H-imidazole-4-carboxylate d'éthyle,
2-(cyclopropanecarbonylamino)-N-(cyclopropylméthyl)-5-[[4-(4-fluorophényl)-1,2,4-triazol-3-yl]amino]-4,5,6,7-tétrahydrobenzothiophène-3-carboxamide,
(5S)-2-(cyclopropanecarbonylamino)-N-(cyclopropylméthyl)-5-(1H-imidazol-2-ylamino)-4,5,6,7-tétrahydrobenzothiophène-3-carboxamide,
2-(cyclopropanecarbonylamino)-N-(cyclopropylméthyl)-5-[[N'-méthoxy-N-(p-tolyl)carbamimidoyl]amino]-4,5,6,7-tétrahydrobenzothiophène-3-carboxamide,
2-(cyclopropanecarbonylamino)-N-(cyclopropylméthyl)-5-[[4-(4-méthylanilino)-1,1-dioxo-1,2,5-thiadiazol-3-yl]amino]-4,5,6,7-tétrahydrobenzothiophène-3-carboxamide,
(5S)-2-(cyclopropanecarbonylamino)-N-(cyclopropylméthyl)-5-[[1-(3-pyridyl)imidazol-2-yl]amino]-4,5,6,7-tétrahydrobenzothiophène-3-carboxamide,
5-(5-aminotriazol-1-yl)-2-(cyclopropanecarbonylamino)-N-(cyclopropylméthyl)-4,5,6,7-tétrahydrobenzothiophène-3-carboxamide,
2-(cyclopropanecarbonylamino)-N-(cyclopropylméthyl)-5-[(2-méthoxyphényl)carbamoylamino]-4,5,6,7-tétrahydrobenzothiophène-3-carboxamide,
N-[2-(cyclopropanecarbonylamino)-3-(cyclopropylméthylcarbamoyl)-4,5,6,7-tétrahydrobenzothiophén-5-yl]pyridine-4-carboxamide,
5-(benzylamino)-2-(cyclopropanecarbonylamino)-N-(cyclopropylméthyl)-4,5,6,7-tétrahydrobenzothiophène-3-carboxamide,
5-(1H-benzimidazol-2-ylamino)-2-(cyclopropanecarbonylamino)-N-(cyclopropylméthyl)-4,5,6,7-tétrahydrobenzothiophène-3-carboxamide,
2-(cyclopropanecarbonylamino)-N-(cyclopropylméthyl)-5-[[N'-hydroxycarbamimidoyl]amino]-4,5,6,7-tétrahydrobenzothiophène-3-carboxamide,
2-(cyclopropanecarbonylamino)-N-(cyclopropylméthyl)-5-[[N'-(1-méthylpyrazol-3-yl)-N-(p-tolyl)carbamimidoyl]amino]-4,5,6,7-tétrahydrobenzothiophène-3-carboxamide, acide formique,
(5S)-2-(cyclopropanecarbonylamino)-N-(cyclopropylméthyl)-5-[3-[(3,5-diméthylpyrazin-2-yl)amino]-1,2,4-triazol-4-yl]-4,5,6,7-tétrahydrobenzothiophène-3-carboxamide,
2-(cyclopropanecarbonylamino)-N-(cyclopropylméthyl)-5-[3-(éthylamino)-5-(méthoxyméthyl)-1,2,4-triazol-4-yl]-4,5,6,7-tétrahydrobenzothiophène-3-carboxamide,
2-(cyclopropanecarbonylamino)-N-(cyclopropylméthyl)-5-[[5-(3-pyridyl)-1H-1,2,4-triazol-3-yl]amino]-4,5,6,7-tétrahydrobenzothiophène-3-carboxamide,
N-[2-(cyclopropanecarbonylamino)-3-(cyclopropylméthylcarbamoyl)-4,5,6,7-tétrahydrobenzothiophén-5-yl]carbamate de p-tolyle,
2-(cyclopropanecarbonylamino)-N-(cyclopropylméthyl)-5-(quinazolin-2-ylamino)-4,5,6,7-tétrahydrobenzothiophène-3-carboxamide,
5-(1,3-benzoxazol-2-ylamino)-2-(cyclopropanecarbonylamino)-N-(cyclopropylméthyl)-4,5,6,7-tétrahydrobenzothiophène-3-carboxamide,
2-(cyclopropanecarbonylamino)-N-(cyclopropylméthyl)-5-[3-(éthylamino)-5-(hydroxyméthyl)-1,2,4-triazol-4-yl]-4,5,6,7-tétrahydrobenzothiophène-3-carboxamide,
(5S)-5-(2-aminoimidazol-1-yl)-2-(cyclopropanecarbonylamino)-N-(cyclopropylméthyl)-4,5,6,7-tétrahydrobenzothiophène-3-carboxamide,
5-(3-amino-5-oxo-1,2,4-oxadiazol-4-yl)-2-(cyclopropanecarbonylamino)-N-(cyclopropylméthyl)-4,5,6,7-tétrahydrobenzothiophène-3-carboxamide,
2-(cyclopropanecarbonylamino)-N-(cyclopropylméthyl)-5-[3-(éthylamino)-5-méthyl-1,2,4-triazol-4-yl]-4,5,6,7-tétrahydrobenzothiophène-3-carboxamide,
(5R)-2-(cyclopropanecarbonylamino)-N-(cyclopropylméthyl)-5-[(2-éthyl-3,4-dioxo-cyclobutén-1-yl)amino]-4,5,6,7-tétrahydrobenzothiophène-3-carboxamide,
(5S)-2-(cyclopropanecarbonylamino)-N-(cyclopropylméthyl)-5-[(2-éthyl-3,4-dioxo-cyclobutén-1-yl)amino]-4,5,6,7-tétrahydrobenzothiophène-3-carboxamide,
2-(cyclopropanecarbonylamino)-N-(cyclopropylméthyl)-5-(pyrimidin-2-ylamino)-4,5,6,7-tétrahydrobenzothiophène-3-carboxamide,
2-(cyclopropanecarbonylamino)-N-(cyclopropylméthyl)-5-(1,3,4-oxadiazol-2-ylamino)-4,5,6,7-tétrahydrobenzothiophène-3-carboxamide,
2-(cyclopropanecarbonylamino)-N-(cyclopropylméthyl)-5-[[5-oxo-1-(p-tolyl)-4H-imidazol-2-yl]amino]-4,5,6,7-tétrahydrobenzothiophène-3-carboxamide,
2-(cyclopropanecarbonylamino)-N-(cyclopropylméthyl)-5-[[2-[éthyl(méthyl)amino]-3,4-dioxocyclobutén-1-yl]amino]-4,5,6,7-tétrahydrobenzothiophène-3-carboxamide,
2-(cyclopropanecarbonylamino)-N-(cyclopropylméthyl)-5-[(méthylsulfonimidoyl)amino]-4,5,6,7-tétrahydrobenzothiophène-3-carboxamide,
5-[[N-cyano-C-méthyl-carbonimidoyl]amino]-2-(cyclopropanecarbonylamino)-N-(cyclopropylméthyl)-4,5,6,7-tétrahydrobenzothiophène-3-carboxamide,
2-(cyclopropanecarbonylamino)-N-(cyclopropylméthyl)-5-(tétrazol-1-yl)-4,5,6,7-tétrahydrobenzothiophène-3-carboxamide,
2-(cyclopropanecarbonylamino)-N-(cyclopropylméthyl)-5-(méthanesulfinamido)-4,5,6,7-tétrahydrobenzothiophène-3-carboxamide,
2-(cyclopropanecarbonylamino)-N-(cyclopropylméthyl)-5-morpholino-4,5,6,7-tétrahydrobenzothiophène-3-carboxamide,
2-(cyclopropanecarbonylamino)-N-(cyclopropylméthyl)-5-[3-(éthylamino)-5-(trifluorométhyl)-1,2,4-triazol-4-yl]-4,5,6,7-tétrahydrobenzothiophène-3-carboxamide,
2-(cyclopropanecarbonylamino)-N-(cyclopropylméthyl)-5-[(4-oxo-1H-pyrimidin-2-yl)amino]-4,5,6,7-tétrahydrobenzothiophène-3-carboxamide,
2-(cyclopropanecarbonylamino)-N-(cyclopropylméthyl)-5-(isopropylsulfamoylamino)-4,5,6,7-tétrahydrobenzothiophène-3-carboxamide,
5-[[*N*'-cyano-*N*-morpholino-carbamimidoyl]amino]-2-(cyclopropanecarbonylamino)-*N-*(cyclopropylméthyl)-4,5,6,7-tétrahydrobenzothiophène-3-carboxamide,
5-acétamido-2-(cyclopropanecarbonylamino)-N-(cyclopropylméthyl)-4,5,6,7-tétrahydrobenzothiophène-3-carboxamide,
2-(cyclopropanecarbonylamino)-N-(cyclopropylméthyl)-5-(méthanesulfonamido)-4,5,6,7-tétrahydrobenzothiophène-3-carboxamide,
5-[[N'-cyano-N-méthoxy-carbamimidoyl]amino]-2-(cyclopropanecarbonylamino)-N-(cyclopropylméthyl)-4,5,6,7-tétrahydrobenzothiophène-3-carboxamide,
2-(cyclopropanecarbonylamino)-N-(cyclopropylméthyl)-5-[[4-oxo-6-(trifluorométhyl)-1H-pyrimidin-2-yl]amino]-4,5,6,7-tétrahydrobenzothiophène-3-carboxamide,
(5S)-2-(cyclopropanecarbonylamino)-N-(cyclopropylméthyl)-5-[3-(2-méthylpropanoylamino)-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[(2,5-diméthylpyrazol-3-yl)-méthyl-amino]-1,2,4-triazol-4-yl]-4,5,6,7-tétrahydrobenzothiophène-3-carboxamide,
(5S)-5-[5-amino-4-(5-méthyl-1,3,4-oxadiazol-2-yl)imidazol-1-yl]-2-(cyclopropanecarbonylamino)-N-(cyclopropylméthyl)-4,5,6,7-tétrahydrobenzothiophène-3-carboxamide,
(5S)-2-(cyclopropanecarbonylamino)-N-(cyclopropylméthyl)-5-[3-[(3-méthyl-1H-pyrazol-5-yl)amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[(1,3-diméthylpyrazol-4-yl)amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[(5-cyclopropyl-2-méthyl-pyrazol-3-yl)amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[(5-isopropyl-2-méthyl-pyrazol-3-yl)amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[[2-méthyl-5-(trifluorométhyl)pyrazol-3-yl]amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[(2-isopropyl-5-méthyl-pyrazol-3-yl)amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[(5-méthyl-2-tétrahydrofuran-3-yl-pyrazol-3-yl)amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[(2-méthylpyrazol-3-yl)amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[(2-éthyl-5-méthyl-pyrazol-3-yl)amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[(2-cyclopropyl-5-méthyl-pyrazol-3-yl)amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[(3-méthoxy-1-méthyl-pyrazol-4-yl)amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[(5-éthyl-2-méthyl-pyrazol-3-yl)amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[[5-(difluorométhyl)-2-méthyl-pyrazol-3-yl]amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[(1,4-diméthylpyrazol-3-yl)amino]-1,2,4-triazol-4-yl]-4,5,6,7-tétrahydrobenzothiophène-3-carboxamide,
5-[[4-[(5S)-2-(cyclopropanecarbonylamino)-3-(cyclopropylméthylcarbamoyl)-4,5,6,7-tétrahydrobenzothiophén-5-yl]-1,2,4-triazol-3-yl]amino]-1-méthyl-pyrazole-3-carboxylate de méthyle,
(5S)-2-(cyclopropanecarbonylamino)-N-(cyclopropylméthyl)-5-[2-[[2-méthyl-5-(trifluorométhyl)pyrazol-3-yl]amino]imidazol-1-yl]-4,5,6,7-tétrahydrobenzothiophène-3-carboxamide,
acide 5-[[4-[(5S)-2-(cyclopropanecarbonylamino)-3-(cyclopropylméthylcarbamoyl)-4,5,6,7-tétrahydrobenzothiophén-5-yl]-1,2,4-triazol-3-yl]amino]-1-méthyl-pyrazole-3-carboxylique,
leurs énantiomères et leurs sels pharmaceutiquement acceptables.

12. Composé selon la revendication 1 ou sel pharmaceutiquement acceptable de celui-ci, ledit composé étant choisi parmi :
(5S)-2-(cyclopropanecarbonylamino)-N-(cyclopropylméthyl)-5-[3-[[(3S*)-1,1-dioxothiolan-3-yl]amino]-1,2,4-triazol-4-yl]-4,5,6,7-tétrahydrobenzothiophène-3-carboxamide [* ou R],
(5S)-2-cyclopropaneamido-N-(cyclopropylméthyl)-5-[3-({3-[imino(méthyl)oxo-lambda6-sulfanyl]-2-méthylphényl}amino)-4H-1,2,4-triazol-4-yl]-4,5,6,7-tétrahydro-1-benzothiophène-3-carboxamide,
(5S)-2-(cyclopropanecarbonylamino)-N-(cyclopropylméthyl)-5-[3-[2-méthyl-3-[(S)-méthylsulfonimidoyl]anilino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[2-méthyl-3-[(R)-méthylsulfonimidoyl]anilino]-1,2,4-triazol-4-yl]-4,5,6,7-tétrahydrobenzothiophène-3-carboxamide,
(5S)-5-{3-[(1-acétyl-3-méthylpyrrolidin-3-yl)amino]-4H-1,2,4-triazol-4-yl}-2-cyclopropaneamido-N-(cyclopropylméthyl)-4,5,6,7-tétrahydro-1-benzothiophène-3-carboxamide,
(5S)-5-[3-[[(3S*)-1-acétyl-3-méthylpyrrolidin-3-yl]amino]-1,2,4-triazol-4-yl]-2-(cyclopropanecarbonylamino)-N-(cyclopropylméthyl)-4,5,6,7-tétrahydrobenzothiophène-3-carboxamide [* ou R],
(5S)-2-cyclopropaneamido-N-(cyclopropylméthyl)-5-(3-{[(3S)-1-(6-méthylpyridazin-3-yl)pyrrolidin-3-yl]amino}-4H-1,2,4-triazol-4-yl)-4,5,6,7-tétrahydro-1-benzothiophène-3-carboxamide,
(5S)-2-(cyclopropanecarbonylamino)-N-(cyclopropylméthyl)-5-[3-[[(3S)-1-pyrazin-2-ylpyrrolidin-3-yl]amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[(4-méthyl-1-méthylsulfonylpyrrolidin-3-yl)amino]-1,2,4-triazol-4-yl]-4,5,6,7-tétrahydrobenzothiophène-3-carboxamide,
(5S)-2-cyclopropaneamido-N-(cyclopropylméthyl)-5-{3-[(1-oxo-2,3-dihydro-1H-isoindol-4-yl)amino]-4H-1,2,4-triazol-4-yl}-4,5,6,7-tétrahydro-1-benzothiophène-3-carboxamide,
(5S)-2-(cyclopropanecarbonylamino)-N-(cyclopropylméthyl)-5-[3-[[(3S)-1-(6-fluoropyridazin-3-yl)pyrrolidin-3-yl]amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[[(3S)-1-pyrimidin-2-ylpyrrolidin-3-yl]amino]-1,2,4-triazol-4-yl]-4,5,6,7-tétrahydro-1-benzothiophène-3-carboxamide,
5-amino-1-[(5S)-2-(cyclopropanecarbonylamino)-3-(cyclopropylméthylcarbamoyl)-4,5,6,7-tétrahydro-1-benzothiophén-5-yl]imidazole-4-carboxylate de 2-méthoxyéthyle,
5-amino-1-[(5S)-2-(cyclopropanecarbonylamino)-3-(cyclopropylméthylcarbamoyl)-4,5,6,7-tétrahydro-1-benzothiophén-5-yl]imidazole-4-carboxylate de 2-morpholin-4-yléthyle,
(5S)-2-(cyclopropanecarbonylamino)-N-(cyclopropylméthyl)-5-[3-(3-méthylsulfanylanilino)-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-(3-méthylsulfonylanilino)-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-(3-méthylsulfinylanilino)-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[3-(méthylsulfonimidoyl)anilino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[3-[(S)-méthylsulfonimidoyl]anilino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[3-[(R)-méthylsulfonimidoyl]anilino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-(8-méthoxy-4-oxo-5,10-dihydroimidazo[4,5-c][1,5]benzodiazépin-1-yl)-4,5,6,7-tétrahydrobenzothiophène-3-carboxamide,
(5S)-2-(cyclopropanecarbonylamino)-N-(cyclopropylméthyl)-5-(8-méthoxy-5-méthyl-4-oxo-10H-imidazo[4,5-c][1,5]benzodiazépin-1-yl)-4,5,6,7-tétrahydrobenzothiophène-3-carboxamide,
(5S)-5-[5-amino-4-[(3R)-3-hydroxypyrrolidine-1-carbonyl]imidazol-1-yl]-2-(cyclopropanecarbonylamino)-N-(cyclopropylméthyl)-4,5,6,7-tétrahydrobenzothiophène-3-carboxamide,
(5S)-5-[5-amino-4-[(2S)-2-méthylpyrrolidine-1-carbonyl]imidazol-1-yl]-2-(cyclopropanecarbonylamino)-N-(cyclopropylméthyl)-4,5,6,7-tétrahydrobenzothiophène-3-carboxamide,
1-[(5S)-2-(cyclopropanecarbonylamino)-3-(cyclopropylméthylcarbamoyl)-4,5,6,7-tétrahydrobenzothiophén-5-yl]-5-[(2,5-diméthyl-4-nitro-pyrazol-3-yl)amino]imidazole-4-carboxylate d'éthyle,
(5S)-2-(cyclopropanecarbonylamino)-N-(cyclopropylméthyl)-5-[3-(2-méthyl-3-méthylsulfinyl-anilino)-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-(2-méthyl-3-méthylsulfonyl-anilino)-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[(2-méthyl-5-oxazol-2-yl-pyrazol-3-yl)amino]-1,2,4-triazol-4-yl]-4,5,6,7-tétrahydrobenzothiophène-3-carboxamide,
(5S)-5-[3-[[(3S)-1-acétylpyrrolidin-3-yl]amino]-1,2,4-triazol-4-yl]-2-(cyclopropanecarbonylamino)-N-(cyclopropylméthyl)-4,5,6,7-tétrahydrobenzothiophène-3-carboxamide,
(5S)-2-(cyclopropanecarbonylamino)-N-(cyclopropylméthyl)-5-[3-[[(3S)-1-méthylsulfonylpyrrolidin-3-yl]amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[[(3R)-1-méthylsulfonylpyrrolidin-3-yl]amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[(2-méthylcyclohexyl)amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[(5-oxopyrrolidin-3-yl)amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[[(3R*)-5-oxopyrrolidin-3-yl]amino]-1,2,4-triazol-4-yl]-4,5,6,7-tétrahydrobenzothiophène-3-carboxamide (*ou S),
(5S)-2-(cyclopropanecarbonylamino)-N-(cyclopropylméthyl)-5-[3-[(2-oxo-4-pipéridyl)amino]-1,2,4-triazol-4-yl]-4,5,6,7-tétrahydrobenzothiophène-3-carboxamide,
(5S)-5-[3-[(1-acétyl-2-méthyl-pyrrolidin-3-yl)amino]-1,2,4-triazol-4-yl]-2-(cyclopropanecarbonylamino)-N-(cyclopropylméthyl)-4,5,6,7-tétrahydrobenzothiophène-3-carboxamide,
(5S)-2-(cyclopropanecarbonylamino)-N-(cyclopropylméthyl)-5-[3-[(2-méthyl-1-méthylsulfonylpyrrolidin-3-yl)amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[[3-(6-méthyl-3-pyridyl)cyclobutyl]amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[[3-(6-méthyl-3-pyridyl)cyclobutyl]amino]-1,2,4-triazol-4-yl]-4,5,6,7-tétrahydrobenzothiophène-3-carboxamide,
(5S)-5-[3-[[(3S*)-1-benzoyl-4,4-difluoro-pyrrolidin-3-yl]amino]-1,2,4-triazol-4-yl]-2-(cyclopropanecarbonylamino)-N-(cyclopropylméthyl)-4,5,6,7-tétrahydrobenzothiophène-3-carboxamide [* ou R],
(5S)-5-[3-[(1-acétyl-4-pipéridyl)amino]-1,2,4-triazol-4-yl]-2-(cyclopropanecarbonylamino)-N-(cyclopropylméthyl)-4,5,6,7-tétrahydrobenzothiophène-3-carboxamide,
(3S)-3-[[4-[(5S)-2-(cyclopropanecarbonylamino)-3-(cyclopropylméthylcarbamoyl)-4,5,6,7-tétrahydrobenzothiophén-5-yl]-1,2,4-triazol-3-yl]amino]pyrrolidine-1-carboxylate de méthyle,
(3S)-3-[[4-[(5S)-2-(cyclopropanecarbonylamino)-3-(cyclopropylméthylcarbamoyl)-4,5,6,7-tétrahydrobenzothiophén-5-yl]-1,2,4-triazol-3-yl]amino]-N-éthyl-pyrrolidine-1-carboxamide,
(5S)-2-cyclopropaneamido-N-(cyclopropylméthyl)-5-(3-{[2-(pyrimidin-2-yl)-2-azaspiro[4.4]nonan-7-yl]amino}-4H-1,2,4-triazol-4-yl)-4,5,6,7-tétrahydro-1-benzothiophène-3-carboxamide,
(5S)-2-(cyclopropanecarbonylamino)-N-(cyclopropylméthyl)-5-[3-[[2-méthyl-5-(trifluorométhoxy)pyrazol-3-yl]amino]-1,2,4-triazol-4-yl]-4,5,6,7-tétrahydrobenzothiophène-3-carboxamide,
(5S)-5-[3-[[5-[chloro(difluoro)méthoxy]-2-méthyl-pyrazol-3-yl]amino]-1,2,4-triazol-4-yl]-2-(cyclopropanecarbonylamino)-N-(cyclopropylméthyl)-4,5,6,7-tétrahydrobenzothiophène-3-carboxamide,
(5S)-2-cyclopropaneamido-N-(cyclopropylméthyl)-5-(3-{[1-méthyl-3-(5-méthyl-1,3,4-oxadiazol-2-yl)-1H-pyrazol-5-yl]amino}-4H-1,2,4-triazol-4-yl)-4,5,6,7-tétrahydro-1-benzothiophène-3-carboxamide,
(5S)-2-(cyclopropanecarbonylamino)-N-(cyclopropylméthyl)-5-[3-[(5-méthoxy-3-méthyl-pyrazin-2-yl)amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[(5-méthylthiazolo[5,4-b]pyridin-2-yl)amino]-4,5,6,7-tétrahydrobenzothiophène-3-carboxamide,
(5S)-5-[3-[(5-cyano-2-méthyl-3-pyridyl)amino]-1,2,4-triazol-4-yl]-2-(cyclopropanecarbonylamino)-N-(cyclopropylméthyl)-4,5,6,7-tétrahydrobenzothiophène-3-carboxamide,
(5S)-5-[3-[(6-cyano-2-méthyl-3-pyridyl)amino]-1,2,4-triazol-4-yl]-2-(cyclopropanecarbonylamino)-N-(cyclopropylméthyl)-4,5,6,7-tétrahydrobenzothiophène-3-carboxamide,
(5S)-2-(cyclopropanecarbonylamino)-N-(cyclopropylméthyl)-5-[3-[(1-méthyl-2-oxo-4-pyridyl)amino]-1,2,4-triazol-4-yl]-4,5,6,7-tétrahydrobenzothiophène-3-carboxamide,
(5S)-5-[3-[(6-chloro-2-méthyl-3-pyridyl)amino]-1,2,4-triazol-4-yl]-2-(cyclopropanecarbonylamino)-N-(cyclopropylméthyl)-4,5,6,7-tétrahydrobenzothiophène-3-carboxamide,
(5S)-2-(cyclopropanecarbonylamino)-N-(cyclopropylméthyl)-5-[3-[[2-méthyl-6-(trifluorométhyl)-3-pyridyl]amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[(3-méthylpyridazin-4-yl)amino]-1,2,4-triazol-4-yl]-4,5,6,7-tétrahydrobenzothiophène-3-carboxamide,
(5S)-5-[3-[(5-chloro-2-méthyl-pyrazol-3-yl)amino]-1,2,4-triazol-4-yl]-2-(cyclopropanecarbonylamino)N-(cyclopropylméthyl)-4,5,6,7-tétrahydrobenzothiophène-3-carboxamide,
(5S)-2-(cyclopropanecarbonylamino)-N-(cyclopropylméthyl)-5-[3-[(3-méthylisothiazol-4-yl)amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[(3-méthylisoxazol-4-yl)amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[(5-méthoxy-2-méthylpyrazol-3-yl)amino]-1,2,4-triazol-4-yl]-4,5,6,7-tétrahydro-1-benzothiophène-3-carboxamide,
(5S)-2-(cyclopropanecarbonylamino)-N-(cyclopropylméthyl)-5-[3-[[5-(difluorométhoxy)-2-méthylpyrazol-3-yl]amino]-1,2,4-triazol-4-yl]-4,5,6,7-tétrahydro-1-benzothiophène-3-carboxamide,
(5S)-2-(cyclopropanecarbonylamino)-N-(cyclopropylméthyl)-5-[3-[[5-(difluorométhyl)-2-(trideutériométhyl)pyrazol-3-yl]amino]-1,2,4-triazol-4-yl]-4,5,6,7-tétrahydrobenzothiophène-3-carboxamide,
(5S)-5-[3-[(5-cyano-2-méthyl-pyrazol-3-yl)amino]-1,2,4-triazol-4-yl]-2-(cyclopropanecarbonylamino)-N-(cyclopropylméthyl)-4,5,6,7-tétrahydrobenzothiophène-3-carboxamide,
(5S)-2-(cyclopropanecarbonylamino)-N-(cyclopropylméthyl)-5-[3-[(2,2-difluoro-1,3-benzodioxol-5-yl)amino]-1,2,4-triazol-4-yl]-4,5,6,7-tétrahydrobenzothiophène-3-carboxamide,
(4RS,5RS)-2-(cyclopropanecarbonylamino)-N-(cyclopropylméthyl)-4-méthyl-5-[3-[[2-méthyl-5-(trifluorométhyl)pyrazol-3-yl]amino]-1,2,4-triazol-4-yl]-4,5,6,7-tétrahydrobenzothiophène-3-carboxamide,
(4R*,5R*)-2-(cyclopropanecarbonylamino)-N-(cyclopropylméthyl)-4-méthyl-5-[3-[[2-méthyl-5-(trifluorométhyl)pyrazolo-3-yl]amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[[6-(difluorométhyl)-3-pyridyl]amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[(6-fluoro-2-méthyl-3-pyridyl)amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[[6-(trifluorométhyl)-3-pyridyl]amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[(6-méthoxy-3-pyridyl)amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[(2-méthoxy-4-pyridyl)amino]-1,2,4-triazol-4-yl]-4,5,6,7-tétrahydrobenzothiophène-3-carboxamide,
(5S)-5-[3-[(6-chloro-3-pyridyl)amino]-1,2,4-triazol-4-yl]-2-(cyclopropanecarbonylamino)-N-(cyclopropylméthyl)-4,5,6,7-tétrahydrobenzothiophène-3-carboxamide,
(5S)-5-[3-[(2-chloro-4-pyridyl)amino]-1,2,4-triazol-4-yl]-2-(cyclopropanecarbonylamino)-N-(cyclopropylméthyl)-4,5,6,7-tétrahydrobenzothiophène-3-carboxamide,
(5S)-2-(cyclopropanecarbonylamino)-N-(cyclopropylméthyl)-5-[3-[(6-méthoxy-2-méthyl-3-pyridyl)amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[(2-fluoro-3-pyridyl)amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[(3-méthyltriazol-4-yl)amino]-1,2,4-triazol-4-yl]-4,5,6,7-tétrahydrobenzothiophène-3-carboxamide,
(3R*)-6-(cyclopropanecarbonylamino)-N-(cyclopropylméthyl)-3-[3-[[2-méthyl-5-(trifluorométhyl)pyrazol-3-yl]amino]-1,2,4-triazol-4-yl]-3,4-dihydro-2H-thiéno[2,3-b]pyrane-5-carboxamide (* ou S).

13. Composé selon la revendication 1 ou sel pharmaceutiquement acceptable de celui-ci, ledit composé étant choisi parmi :
(5S)-2-(cyclopropanecarbonylamino)-N-(cyclopropylméthyl)-5-[3-[(2,5-diméthylpyrazol-3-yl)amino]-1,2,4-triazol-4-yl]-4,5,6,7-tétrahydrobenzothiophène-3-carboxamide,
5-amino-1-[(5S)-2-(cyclopropanecarbonylamino)-3-(cyclopropylméthylcarbamoyl)-4,5,6,7-tétrahydrobenzothiophén-5-yl]imidazole-4-carboxylate d'éthyle,
(5S)-2-(cyclopropanecarbonylamino)-N-(cyclopropylméthyl)-5-[3-[(5-cyclopropyl-2-méthyl-pyrazol-3-yl)amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[(5-isopropyl-2-méthyl-pyrazol-3-yl)amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[[2-méthyl-5-(trifluorométhyl)pyrazol-3-yl]amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[(5-éthyl-2-méthyl-pyrazol-3-yl)amino]-1,2,4-triazol-4-yl]-4,5,6,7-tétrahydrobenzothiophène-3-carboxamide,
(5S)-2-(cyclopropanecarbonylamino)-N-(cyclopropylméthyl)-5-[3-[[5-(difluorométhyl)-2-méthylpyrazol-3-yl]amino]-1,2,4-triazol-4-yl]-4,5,6,7-tétrahydrobenzothiophène-3-carboxamide.

14. Composé selon la revendication 1 ou sel pharmaceutiquement acceptable de celui-ci, ledit composé étant choisi parmi : 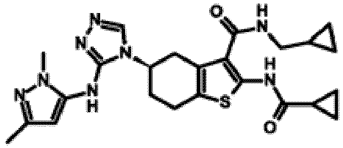
(5S)-2-(cyclopropanecarbonylamino)-N-(cyclopropylméthyl)-5-[3-[(2,5-diméthylpyrazol-3-yl)amino]-1,2,4-triazol-4-yl]-4,5,6,7-tétrahydrobenzothiophène-3-carboxamide, 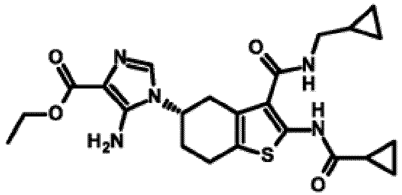
5-amino-1-[(5S)-2-(cyclopropanecarbonylamino)-3-(cyclopropylméthylcarbamoyl)-4,5,6,7-tétrahydrobenzothiophén-5-yl]imidazole-4-carboxylate d'éthyle, 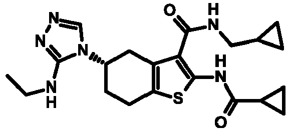
(5S)-2-(cyclopropanecarbonylamino)-N-(cyclopropylméthyl)-5-[3-(isobutylamino)-1,2,4-triazol-4-yl]-4,5,6,7-tétrahydrobenzothiophène-3-carboxamide, 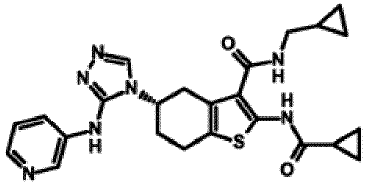
(5S)-2-(cyclopropanecarbonylamino)-N-(cyclopropylméthyl)-5-[3-(3-pyridylamino)-1,2,4-triazol-4-yl]-4,5,6,7-tétrahydrobenzothiophène-3-carboxamide, 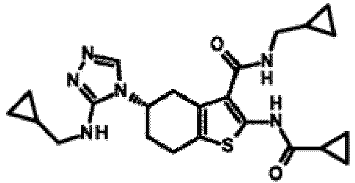
(5S)-2-(cyclopropanecarbonylamino)-N-(cyclopropylméthyl)-5-[3-(cyclopropylméthylamino)-1,2,4-triazol-4-yl]-4,5,6,7-tétrahydrobenzothiophène-3-carboxamide, 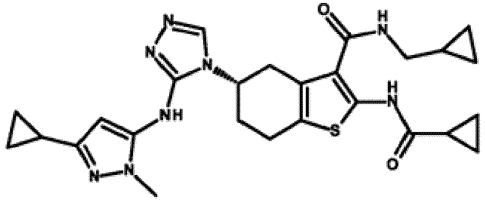
(5S)-2-(cyclopropanecarbonylamino)-N-(cyclopropylméthyl)-5-[3-[(5-cyclopropyl-2-méthyl-pyrazol-3-yl)amino]-1,2,4-triazol-4-yl]-4,5,6,7-tétrahydrobenzothiophène-3-carboxamide, 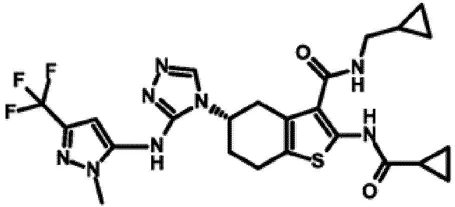
(5S)-2-(cyclopropanecarbonylamino)-N-(cyclopropylméthyl)-5-[3-[[2-méthyl-5-(trifluorométhyl)pyrazol-3-yl]amino]-1,2,4-triazol-4-yl]-4,5,6,7-tétrahydrobenzothiophène-3-carboxamide, 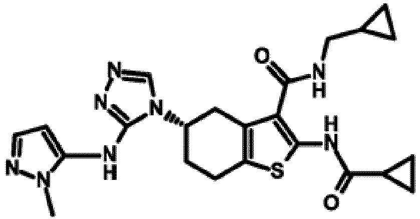
(5S)-2-(cyclopropanecarbonylamino)-N-(cyclopropylméthyl)-5-[3-[(2-méthylpyrazol-3-yl)amino]-1,2,4-triazol-4-yl]-4,5,6,7-tétrahydrobenzothiophène-3-carboxamide, 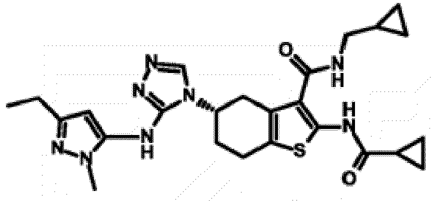
(5S)-2-(cyclopropanecarbonylamino)-N-(cyclopropylméthyl)-5-[3-[(5-éthyl-2-méthyl-pyrazol-3-yl)amino]-1,2,4-triazol-4-yl]-4,5,6,7-tétrahydrobenzothiophène-3-carboxamide, 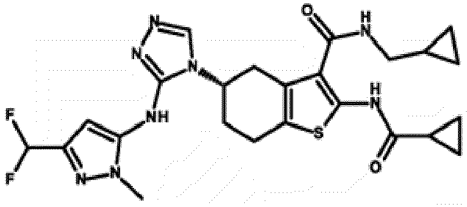
(5S)-2-(cyclopropanecarbonylamino)-N-(cyclopropylméthyl)-5-[3-[[5-(difluorométhyl)-2-méthylpyrazol-3-yl]amino]-1,2,4-triazol-4-yl]-4,5,6,7-tétrahydrobenzothiophène-3-carboxamide, et 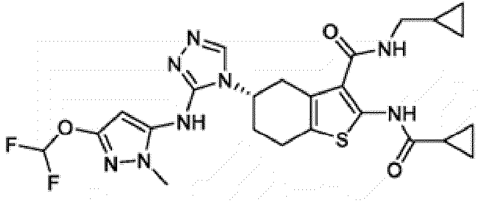
(5S)-2-(cyclopropanecarbonylamino)-N-(cyclopropylméthyl)-5-[3-[[5-(difluorométhoxy)-2-méthylpyrazol-3-yl]amino]-1,2,4-triazol-4-yl]-4,5,6,7-tétrahydro-1-benzothiophène-3-carboxamide.

15. Composé selon la revendication 1 ou 2, ou sel pharmaceutiquement acceptable de celui-ci, le composé étant **caractérisé par** la formule (XXV) ou (XXVI)

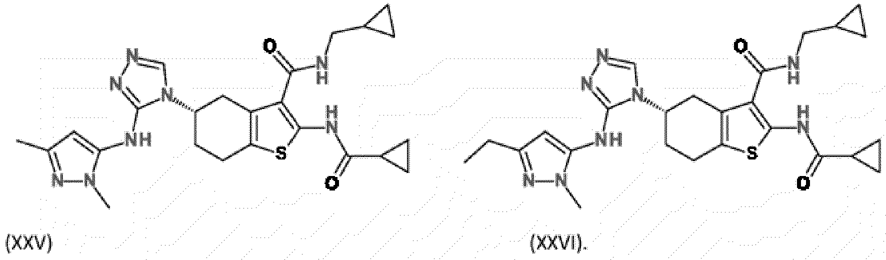

16. Composé selon la revendication 1 ou 2, ou sel pharmaceutiquement acceptable de celui-ci, le composé étant **caractérisé par** la formule (XXVII) ou (XXVIII)

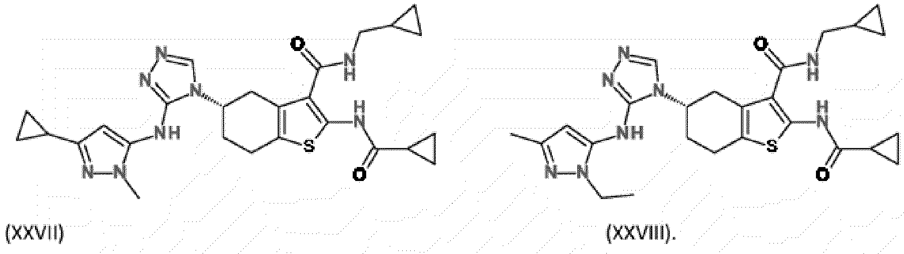

17. Composé selon la revendication 1 ou 2, ou sel pharmaceutiquement acceptable de celui-ci, le composé étant **caractérisé par** la formule (XXIX) ou (XXX)

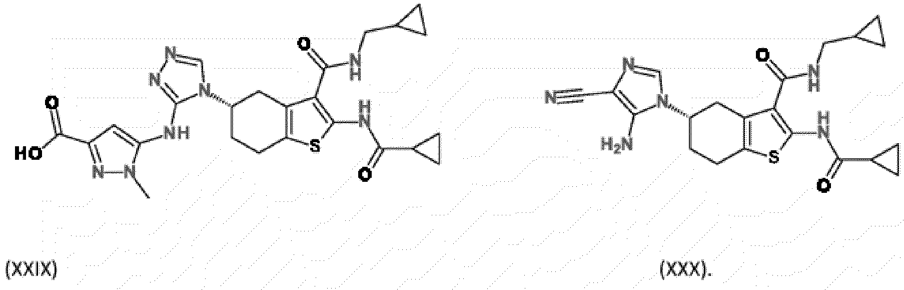

18. Composé selon la revendication 1 ou 2, ou sel pharmaceutiquement acceptable de celui-ci, le composé étant **caractérisé par** la formule (XXXII)

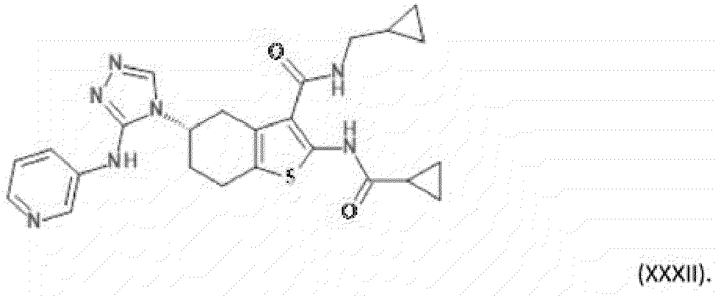

19. Composé selon la revendication 1 ou 2, ou sel pharmaceutiquement acceptable de celui-ci, le composé étant **caractérisé par** la formule (XXXIII) ou (XXXIV)

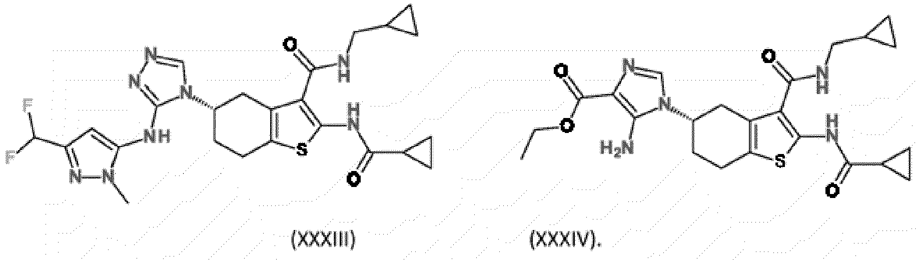

20. Composé selon la revendication 1 ou 2, ou sel pharmaceutiquement acceptable de celui-ci, le composé étant **caractérisé par** la formule (XXXV) ou (XXXVI)

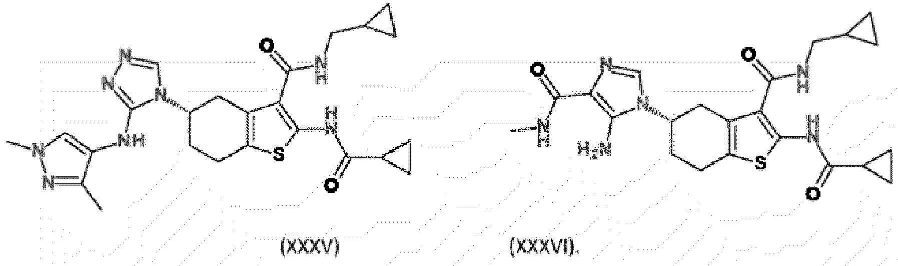

21. Composition pharmaceutique comprenant un composé selon l'une quelconque des revendications 1 à 20 ou un sel pharmaceutiquement acceptable de celui-ci.

22. Composé selon l'une quelconque des revendications 1 à 20 ou sel pharmaceutiquement acceptable de celui-ci, destiné à être utilisé dans un procédé de traitement ou de prévention des troubles causés par les IgE.

23. Composé destiné à être utilisé selon la revendication 22, ou sel pharmaceutiquement acceptable de celui-ci, le trouble étant une allergie, des réactions mastocytaires non allergiques, une hypersensibilité de type 1, de l'urticaire ou une inflammation sinusale familiale.

24. Composé destiné à être utilisé selon la revendication 22, ou sel pharmaceutiquement acceptable de celui-ci, le trouble étant une constriction des voies respiratoires dans le cas de l'asthme, une inflammation locale dans le cas de l'eczéma, une sécrétion accrue de mucus dans le cas de la rhinite allergique, de l'urticaire ou une perméabilité vasculaire accrue.

25. Composé destiné à être utilisé selon la revendication 22, ou sel pharmaceutiquement acceptable de celui-ci, le trouble étant une granulomatose éosinophilique avec polyangéite (également appelée « syndrome de Churg-Strauss »), une maladie respiratoire exacerbée par l'aspirine ou un lymphome cutané à cellules T.
